# EUROPEAN PATENT APPLICATION

(11) **EP 1 683 859 A2**
(43) Date of publication of application: **26.07.2006**
(21) Application number: 06110391.7
(22) Date of filing: 23.06.2000
(51) Int. Cl.: C12N 9/02, C07K 14/34

(54) **Putative gene coding for Catechol 1,2-Dioxygenase from Corynebacterium glutamicum**

(30) Priority: 25.06.1999 US 141031 P; 08.07.1999 DE 19931636; 09.07.1999 DE 19932125; 09.07.1999 DE 19932126; 09.07.1999 DE 19932127; 09.07.1999 DE 19932128; 09.07.1999 DE 19932129; 09.07.1999 DE 19932226; 14.07.1999 DE 19932920; 14.07.1999 DE 19932922; 14.07.1999 DE 19932924; 14.07.1999 DE 19932928; 14.07.1999 DE 19932930; 14.07.1999 DE 19932933; 14.07.1999 DE 19932935; 14.07.1999 DE 19932973; 14.07.1999 DE 19933002; 14.07.1999 DE 19933003; 14.07.1999 DE 19933005; 14.07.1999 DE 19933006; 31.08.1999 DE 19941378; 31.08.1999 DE 19941379; 31.08.1999 DE 19941390; 31.08.1999 DE 19941391; 03.09.1999 DE 19942088
(62) Divisional of application: 00938991.7
(71) Applicant: BASF Aktiengesellschaft, 67056 Ludwigshafen (DE)
(72) Inventor: Pompejus, Markus, BASF Aktiengesellschaft, Yongsan-gu, Seoul 140-210 (KR); Kröger, Burkhard, 67117 Limburgerhof (DE); Schröder, Hartwig, 69226 Nußloch (DE); Zelder, Oskar, 67346 Speyer (DE); Haberhauer, Gregor, 67117 Limburgerhof (DE)

(57) **Abstract**

Isolated nucleic acid molecules, designated HA nucleic acid molecules, which encode novel HA proteins from Corynebacterium glutamicum are described. The invention also provides antisense nucleic acid molecules. recombinant expression vectors containing HA nucleic acid molecules, and host cells into which the expression vectors have been introduced. The invention still further provides isolated HA proteins, mutated HA proteins, fusion proteins, antigenic peptides and methods for the improvement of production of a desired compound from C. glutamicum based on genetic engineering of HA genes in this organism.

SEQUENCE LISTING

<110> BASF Aktiengesellschaft

<120> CORYNEBACTERIUM GLUTAMICUM GENES ENCODING PROTEINS
      INVOLVED IN HOMEOSTASIS AND ADAPTATION

<130> PF 0000050163 EP-2

<140> PCT/IB00/00911
<141> 2000-06-23

<150> US60/141,031
<151> 1999-06-25

<150> DE 19931636.8
<151> 1999-07-08

<150> DE 19932125.6
<151> 1999-07-09

<150> DE 19932126.4
<151> 1999-07-09

<150> DE 19932127.2
<151> 1999-07-09

<150> DE 19932128.0
<151> 1999-07-09

<150> DE 19932129.9
<151> 1999-07-09

<150> DE 19932226.0
<151> 1999-07-09

<150> DE 19932920.6
<151> 1999-07-14

<150> DE 19932922.2
<151> 1999-07-14

<150> DE 19932924.9
<151> 1999-07-14

<150> DE 19932928.1
<151> 1999-07-14

<150> DE 19932930.3
<151> 1999-07-14

<150> DE 19932933.8
<151> 1999-07-14

<150> DE 19932935.4
<151> 1999-07-14

<150> DE 19932973.7
<151> 1999-07-14

<150> DE 19933002.6
<151> 1999-07-14

<150> DE 19933003.4
<151> 1999-07-14

<150> DE 19933005.0
<151> 1999-07-14

<150> DE 19933006.9
<151> 1999-07-14

<150> DE 19941378.9
<151> 1999-08-31

<150> DE 19941379.7
<151> 1999-08-31

<150> DE 19941390.8

<151> 1999-08-31

<150> DE 19941391.6
<151> 1999-08-31

<150> DE 19942088.2
<151> 1999-09-03

<160> 442

<210> 1
<211> 314
<212> DNA
<213> Corynebacterium glutamicum

<220>
<221> CDS
<222> (1)..(291)
<223> RXA02548

<400> 1
cca ccg atc tac ttc tcc cac gac cgc gaa gtt ttc gag cgc gac ggc     48
Pro Pro Ile Tyr Phe Ser His Asp Arg Glu Val Phe Glu Arg Asp Gly
1               5                   10                  15

atg tgg ctg acc gca ggc gag tgg ggt gga cca aag aag ggc gag gag     96
Met Trp Leu Thr Ala Gly Glu Trp Gly Gly Pro Lys Lys Gly Glu Glu
            20                  25                  30

atc gtc acc aag act gtc cgc tac cgc acc gtc ggc gat atg tcc tgc     144
Ile Val Thr Lys Thr Val Arg Tyr Arg Thr Val Gly Asp Met Ser Cys
        35                  40                  45

acc ggt gct gtg ctc tcc gaa gcc cgc acc att gac gat gtg atc gaa     192
Thr Gly Ala Val Leu Ser Glu Ala Arg Thr Ile Asp Asp Val Ile Glu
    50                  55                  60

gag atc gcc acc tcc acc ctt acc gaa cgt ggc gca acc cgc gcc gat     240
Glu Ile Ala Thr Ser Thr Leu Thr Glu Arg Gly Ala Thr Arg Ala Asp
65                  70                  75                  80

gac cgc ctc agc gaa tcc gca atg gaa gac cgc aag aag gaa ggc tac     288
Asp Arg Leu Ser Glu Ser Ala Met Glu Asp Arg Lys Lys Glu Gly Tyr
                85                  90                  95

ttc tgatgactgc tccaaccttg aat                                       314
Phe

<210> 2
<211> 97
<212> PRT
<213> Corynebacterium glutamicum

<400> 2
Pro Pro Ile Tyr Phe Ser His Asp Arg Glu Val Phe Glu Arg Asp Gly
1               5                   10                  15

Met Trp Leu Thr Ala Gly Glu Trp Gly Gly Pro Lys Lys Gly Glu Glu
            20                  25                  30

Ile Val Thr Lys Thr Val Arg Tyr Arg Thr Val Gly Asp Met Ser Cys
        35                  40                  45

Thr Gly Ala Val Leu Ser Glu Ala Arg Thr Ile Asp Asp Val Ile Glu
    50                  55                  60

Glu Ile Ala Thr Ser Thr Leu Thr Glu Arg Gly Ala Thr Arg Ala Asp
65                  70                  75                  80

Asp Arg Leu Ser Glu Ser Ala Met Glu Asp Arg Lys Lys Glu Gly Tyr
                85                  90                  95

Phe

<210> 3
<211> 980
<212> DNA
<213> Corynebacterium glutamicum

<220>
<221> CDS
<222> (1)..(957)
<223> RXN00249

<400> 3
acc ggc gtc tcc acc tcc cag gtt gta gtt ttg ctt gtc gac gcc cgc     48
Thr Gly Val Ser Thr Ser Gln Val Val Val Leu Leu Val Asp Ala Arg
1               5                   10                  15

cac ggc gtc gtc gag cag acc cgc cgc cac ctg tcc gta tcg gct ctg     96
His Gly Val Val Glu Gln Thr Arg Arg His Leu Ser Val Ser Ala Leu
            20                  25                  30

ctg ggc gta cgc acg gtg atc ctc gca gtc aac aaa att gac ctt gtt     144
Leu Gly Val Arg Thr Val Ile Leu Ala Val Asn Lys Ile Asp Leu Val
        35                  40                  45

gat tac agc gaa gaa gtc ttc cgc aac att gaa aag gaa ttc gtt ggc     192
Asp Tyr Ser Glu Glu Val Phe Arg Asn Ile Glu Lys Glu Phe Val Gly
    50                  55                  60

ctg gca tct gca ctt gat gtc aca gac acc cac gtt gtt cca atc tct     240
Leu Ala Ser Ala Leu Asp Val Thr Asp Thr His Val Val Pro Ile Ser
65                  70                  75                  80

gcg ctc aag ggc gac aac gtt gca gaa cct tcc acc cac atg gat tgg     288
Ala Leu Lys Gly Asp Asn Val Ala Glu Pro Ser Thr His Met Asp Trp
                85                  90                  95

tac acc gga cca acc gtg ctg gaa atc ctg gaa aac gta gaa gtt tcc     336
Tyr Thr Gly Pro Thr Val Leu Glu Ile Leu Glu Asn Val Glu Val Ser
            100                 105                 110

cac ggc cgt gca cac gac ctg ggc ttc cgc ttc cca atc cag tac gtc     384
His Gly Arg Ala His Asp Leu Gly Phe Arg Phe Pro Ile Gln Tyr Val
        115                 120                 125

atc cgc gag cac gcc acc gac tac cgt ggc tac gcc ggc acc atc aac     432
Ile Arg Glu His Ala Thr Asp Tyr Arg Gly Tyr Ala Gly Thr Ile Asn
    130                 135                 140

gct ggt tcc gtc tcc gtg ggc gat acc gtg tac cta cct gaa ggc cgc     480
Ala Gly Ser Val Ser Val Gly Asp Thr Val Tyr Leu Pro Glu Gly Arg
145                 150                 155                 160

acc acc cag gtc acc cac atc gat tcc gct gac gga tcc ctc cag acc     528
Thr Thr Gln Val Thr His Ile Asp Ser Ala Asp Gly Ser Leu Gln Thr
                165                 170                 175

gca tca gtt gga gaa gcc gtt gtc ctg cgc cta gcc cag gaa atc gac     576
Ala Ser Val Gly Glu Ala Val Val Leu Arg Leu Ala Gln Glu Ile Asp
            180                 185                 190

ctc atc cgc ggc gaa ctc atc gct ggc gaa gac cgc cca gaa tcc gtt     624
Leu Ile Arg Gly Glu Leu Ile Ala Gly Glu Asp Arg Pro Glu Ser Val
        195                 200                 205

cgc tcc ttc aac gcc act gtt gtt ggc ttg gcc gat cgc acc atc aaa     672
Arg Ser Phe Asn Ala Thr Val Val Gly Leu Ala Asp Arg Thr Ile Lys
    210                 215                 220

cca ggt gca gca gtc aag gtt cgc tac ggc acc gag ctg gtc cgc gga     720
Pro Gly Ala Ala Val Lys Val Arg Tyr Gly Thr Glu Leu Val Arg Gly
225                 230                 235                 240

cgc gtc gca gcc atc gaa cga gtc ctc gac atc gac ggc gtc aac gac     768
Arg Val Ala Ala Ile Glu Arg Val Leu Asp Ile Asp Gly Val Asn Asp
                245                 250                 255

aac gaa gca cca gaa acc tac ggc ctc aac gac atc gca cac gtg cgc     816
Asn Glu Ala Pro Glu Thr Tyr Gly Leu Asn Asp Ile Ala His Val Arg
            260                 265                 270

atc gac gtt gca ggc gaa ctc gaa gtt gaa gat tac gct gcc cgc ggc     864
Ile Asp Val Ala Gly Glu Leu Glu Val Glu Asp Tyr Ala Ala Arg Gly
        275                 280                 285

gcc atc gga tcc ttc ctc ctc atc gac caa tcc tcc ggc gat acc ctc     912
Ala Ile Gly Ser Phe Leu Leu Ile Asp Gln Ser Ser Gly Asp Thr Leu
    290                 295                 300

gca gct ggc ttg gtt ggc cac cgc cta cgc aat aac tgg tcg atc         957
Ala Ala Gly Leu Val Gly His Arg Leu Arg Asn Asn Trp Ser Ile
305                 310                 315

tagaccagtg tcttaggcaa gac                                           980


<210> 4
<211> 319
<212> PRT
<213> Corynebacterium glutamicum

<400> 4
Thr Gly Val Ser Thr Ser Gln Val Val Val Leu Leu Val Asp Ala Arg
  1               5                  10                  15

His Gly Val Val Glu Gln Thr Arg Arg His Leu Ser Val Ser Ala Leu
             20                  25                  30

Leu Gly Val Arg Thr Val Ile Leu Ala Val Asn Lys Ile Asp Leu Val
         35                  40                  45

Asp Tyr Ser Glu Glu Val Phe Arg Asn Ile Glu Lys Glu Phe Val Gly
     50                  55                  60

Leu Ala Ser Ala Leu Asp Val Thr Asp Thr His Val Val Pro Ile Ser
 65                  70                  75                  80

Ala Leu Lys Gly Asp Asn Val Ala Glu Pro Ser Thr His Met Asp Trp
                 85                  90                  95

Tyr Thr Gly Pro Thr Val Leu Glu Ile Leu Glu Asn Val Glu Val Ser
            100                 105                 110

His Gly Arg Ala His Asp Leu Gly Phe Arg Phe Pro Ile Gln Tyr Val
        115                 120                 125

Ile Arg Glu His Ala Thr Asp Tyr Arg Gly Tyr Ala Gly Thr Ile Asn
    130                 135                 140

Ala Gly Ser Val Ser Val Gly Asp Thr Val Tyr Leu Pro Glu Gly Arg
145                 150                 155                 160

Thr Thr Gln Val Thr His Ile Asp Ser Ala Asp Gly Ser Leu Gln Thr
                165                 170                 175

Ala Ser Val Gly Glu Ala Val Val Leu Arg Leu Ala Gln Glu Ile Asp
            180                 185                 190

Leu Ile Arg Gly Glu Leu Ile Ala Gly Glu Asp Arg Pro Glu Ser Val
        195                 200                 205

Arg Ser Phe Asn Ala Thr Val Val Gly Leu Ala Asp Arg Thr Ile Lys
    210                 215                 220

Pro Gly Ala Ala Val Lys Val Arg Tyr Gly Thr Glu Leu Val Arg Gly
225                 230                 235                 240

Arg Val Ala Ala Ile Glu Arg Val Leu Asp Ile Asp Gly Val Asn Asp
                245                 250                 255

Asn Glu Ala Pro Glu Thr Tyr Gly Leu Asn Asp Ile Ala His Val Arg
            260                 265                 270

Ile Asp Val Ala Gly Glu Leu Glu Val Glu Asp Tyr Ala Ala Arg Gly

275                 280                 285

Ala Ile Gly Ser Phe Leu Leu Ile Asp Gln Ser Ser Gly Asp Thr Leu
    290                 295                 300

Ala Ala Gly Leu Val Gly His Arg Leu Arg Asn Asn Trp Ser Ile
305                 310                 315


<210> 5
<211> 977
<212> DNA
<213> Corynebacterium glutamicum


<220>
<221> CDS
<222> (1)..(954)
<223> FRXA00249

<400> 5
ggt gtt ttc acc ttc caa ggt gta gtt ttg ctt gtc gac gcc cgc cac      48
Gly Val Phe Thr Phe Gln Gly Val Val Leu Leu Val Asp Ala Arg His
  1               5                  10                  15

ggc gtc gtc gag cag acc cgc cgc cac ctg tcc gta tcg gct ctg ctg      96
Gly Val Val Glu Gln Thr Arg Arg His Leu Ser Val Ser Ala Leu Leu
             20                  25                  30

ggc gta cgc acg gtg atc ctc gca gtc aac aaa att gac ctt gtt gat     144
Gly Val Arg Thr Val Ile Leu Ala Val Asn Lys Ile Asp Leu Val Asp
         35                  40                  45

tac agc gaa gaa gtc ttc cgc aac att gaa aag gaa ttc gtt ggc ctg     192
Tyr Ser Glu Glu Val Phe Arg Asn Ile Glu Lys Glu Phe Val Gly Leu
     50                  55                  60

gca tct gca ctt gat gtc aca gac acc cac gtt gtt cca atc tct gcg     240
Ala Ser Ala Leu Asp Val Thr Asp Thr His Val Val Pro Ile Ser Ala
 65                  70                  75                  80

ctc aag ggc gac aac gtt gca gaa cct tcc acc cac atg gat tgg tac     288
Leu Lys Gly Asp Asn Val Ala Glu Pro Ser Thr His Met Asp Trp Tyr
                 85                  90                  95

acc gga cca acc gtg ctg gaa atc ctg gaa aac gta gaa gtt tcc cac     336
Thr Gly Pro Thr Val Leu Glu Ile Leu Glu Asn Val Glu Val Ser His
            100                 105                 110

ggc cgt gca cac gac ctg ggc ttc cgc ttc cca atc cag tac gtc atc     384
Gly Arg Ala His Asp Leu Gly Phe Arg Phe Pro Ile Gln Tyr Val Ile
        115                 120                 125

cgc gag cac gcc acc gac tac cgt ggc tac gcc ggc acc atc aac gct     432
Arg Glu His Ala Thr Asp Tyr Arg Gly Tyr Ala Gly Thr Ile Asn Ala
    130                 135                 140

ggt tcc gtc tcc gtg ggc gat acc gtg tac cta cct gaa ggc cgc acc     480
Gly Ser Val Ser Val Gly Asp Thr Val Tyr Leu Pro Glu Gly Arg Thr
145                 150                 155                 160

acc cag gtc acc cac atc gat tcc gct gac gga tcc ctc cag acc gca     528
Thr Gln Val Thr His Ile Asp Ser Ala Asp Gly Ser Leu Gln Thr Ala
                165                 170                 175

tca gtt gga gaa gcc gtt gtc ctg cgc cta gcc cag gaa atc gac ctc     576
Ser Val Gly Glu Ala Val Val Leu Arg Leu Ala Gln Glu Ile Asp Leu
            180                 185                 190

atc cgc ggc gaa ctc atc gct ggc gaa gac cgc cca gaa tcc gtt cgc     624
Ile Arg Gly Glu Leu Ile Ala Gly Glu Asp Arg Pro Glu Ser Val Arg
        195                 200                 205

tcc ttc aac gcc act gtt gtt ggc ttg gcc gat cgc acc atc aaa cca     672
Ser Phe Asn Ala Thr Val Val Gly Leu Ala Asp Arg Thr Ile Lys Pro
    210                 215                 220

ggt gca gca gtc aag gtt cgc tac ggc acc gag ctg gtc cgc gga cgc     720
Gly Ala Ala Val Lys Val Arg Tyr Gly Thr Glu Leu Val Arg Gly Arg
225                 230                 235                 240

gtc gca gcc atc gaa cga gtc ctc gac atc gac ggc gtc aac gac aac     768
Val Ala Ala Ile Glu Arg Val Leu Asp Ile Asp Gly Val Asn Asp Asn
                245                 250                 255

gaa gca cca gaa acc tac ggc ctc aac gac atc gca cac gtg cgc atc     816
Glu Ala Pro Glu Thr Tyr Gly Leu Asn Asp Ile Ala His Val Arg Ile
            260                 265                 270

gac gtt gca ggc gaa ctc gaa gtt gaa gat tac gct gcc cgc ggc gcc     864
Asp Val Ala Gly Glu Leu Glu Val Glu Asp Tyr Ala Ala Arg Gly Ala
        275                 280                 285

atc gga tcc ttc ctc ctc atc gac caa tcc tcc ggc gat acc ctc gca     912
Ile Gly Ser Phe Leu Leu Ile Asp Gln Ser Ser Gly Asp Thr Leu Ala
    290                 295                 300

gct ggc ttg gtt ggc cac cgc cta cgc aat aac tgg tcg atc             954
Ala Gly Leu Val Gly His Arg Leu Arg Asn Asn Trp Ser Ile
305                 310                 315

tagaccagtg tcttaggcaa gac                                           977


<210> 6
<211> 318
<212> PRT
<213> Corynebacterium glutamicum

<400> 6
Gly Val Phe Thr Phe Gln Gly Val Val Leu Leu Val Asp Ala Arg His
1               5                   10                  15

Gly Val Val Glu Gln Thr Arg Arg His Leu Ser Val Ser Ala Leu Leu
            20                  25                  30

Gly Val Arg Thr Val Ile Leu Ala Val Asn Lys Ile Asp Leu Val Asp
        35                  40                  45

Tyr Ser Glu Glu Val Phe Arg Asn Ile Glu Lys Glu Phe Val Gly Leu
    50                  55                  60

Ala Ser Ala Leu Asp Val Thr Asp Thr His Val Val Pro Ile Ser Ala
65                  70                  75                  80

Leu Lys Gly Asp Asn Val Ala Glu Pro Ser Thr His Met Asp Trp Tyr
                85                  90                  95

Thr Gly Pro Thr Val Leu Glu Ile Leu Glu Asn Val Glu Val Ser His
            100                 105                 110

Gly Arg Ala His Asp Leu Gly Phe Arg Phe Pro Ile Gln Tyr Val Ile
        115                 120                 125

Arg Glu His Ala Thr Asp Tyr Arg Gly Tyr Ala Gly Thr Ile Asn Ala
    130                 135                 140

Gly Ser Val Ser Val Gly Asp Thr Val Tyr Leu Pro Glu Gly Arg Thr
145                 150                 155                 160

Thr Gln Val Thr His Ile Asp Ser Ala Asp Gly Ser Leu Gln Thr Ala
                165                 170                 175

Ser Val Gly Glu Ala Val Val Leu Arg Leu Ala Gln Glu Ile Asp Leu
            180                 185                 190

Ile Arg Gly Glu Leu Ile Ala Gly Glu Asp Arg Pro Glu Ser Val Arg
        195                 200                 205

Ser Phe Asn Ala Thr Val Val Gly Leu Ala Asp Arg Thr Ile Lys Pro
    210                 215                 220

Gly Ala Ala Val Lys Val Arg Tyr Gly Thr Glu Leu Val Arg Gly Arg
225                 230                 235                 240

Val Ala Ala Ile Glu Arg Val Leu Asp Ile Asp Gly Val Asn Asp Asn
                245                 250                 255

Glu Ala Pro Glu Thr Tyr Gly Leu Asn Asp Ile Ala His Val Arg Ile
            260                 265                 270

Asp Val Ala Gly Glu Leu Glu Val Glu Asp Tyr Ala Ala Arg Gly Ala
        275                 280                 285

Ile Gly Ser Phe Leu Leu Ile Asp Gln Ser Ser Gly Asp Thr Leu Ala
    290                 295                 300

Ala Gly Leu Val Gly His Arg Leu Arg Asn Asn Trp Ser Ile
305                 310                 315


<210> 7
<211> 954
<212> DNA
<213> Corynebacterium glutamicum

<220>
<221> CDS
<222> (101)..(931)
<223> RXA01073

<400> 7
taaccgactc cagcactaaa ctccaaaccc ttggcccgca ccgccaaagt ttagcgcgcc 60

ccaagacacc accgcgccat gtttgcctag gattaggtac atg aca aac act caa   115
                                           Met Thr Asn Thr Gln
                                           1               5

acc gag atc att aat gaa cta aag gtg agc cca gca atc gac gtg gcc   163
Thr Glu Ile Ile Asn Glu Leu Lys Val Ser Pro Ala Ile Asp Val Ala
                10                  15                  20

aag gaa gtt gaa ttc cgt gtg cag ttc ctc gtc gat tac ctg cgg gct   211
Lys Glu Val Glu Phe Arg Val Gln Phe Leu Val Asp Tyr Leu Arg Ala
            25                  30                  35

tcc cat aca aaa ggc ttt gtt ctt ggt att tca ggt ggc cag gat tcc   259
Ser His Thr Lys Gly Phe Val Leu Gly Ile Ser Gly Gly Gln Asp Ser
        40                  45                  50

act ctt gcg gga cga ctc acg cag ctg gca gta gag cgc att cgt gcg   307
Thr Leu Ala Gly Arg Leu Thr Gln Leu Ala Val Glu Arg Ile Arg Ala
    55                  60                  65

gaa gaa aac agc acg gat tat gtc ttc tac gca gtt cgc ctc ccc tac   355
Glu Glu Asn Ser Thr Asp Tyr Val Phe Tyr Ala Val Arg Leu Pro Tyr
70                  75                  80                  85

gcg atc cag gca gat gag gac gat gcg caa gtt gca ttg gaa ttc atc   403
Ala Ile Gln Ala Asp Glu Asp Asp Ala Gln Val Ala Leu Glu Phe Ile
                90                  95                  100

gca cct gac aag agc gtg acc gtc aac gtt aaa gac gca acg gac gcc   451
Ala Pro Asp Lys Ser Val Thr Val Asn Val Lys Asp Ala Thr Asp Ala
            105                 110                 115

acc gaa gca act gtt gca gct gct ttg gaa ctt cct gag ctg acc gac   499
Thr Glu Ala Thr Val Ala Ala Ala Leu Glu Leu Pro Glu Leu Thr Asp
        120                 125                 130

ttc aat cgg ggc aat att aaa gct cgc caa cgc atg gtt gcc cag tac   547
Phe Asn Arg Gly Asn Ile Lys Ala Arg Gln Arg Met Val Ala Gln Tyr
    135                 140                 145

gca atc gca ggc cag ttg ggc ttg ctg gtt att ggc act gat cac gcg   595
Ala Ile Ala Gly Gln Leu Gly Leu Leu Val Ile Gly Thr Asp His Ala
150                 155                 160                 165

gct gaa aac gtc acg ggg ttc ttc acc aaa ttc ggt gat ggc gca gct   643
Ala Glu Asn Val Thr Gly Phe Phe Thr Lys Phe Gly Asp Gly Ala Ala
                170                 175                 180

gac ctg ctt cct ttg gca ggt ttg agc aag cgt caa gga gct gcc att     691
Asp Leu Leu Pro Leu Ala Gly Leu Ser Lys Arg Gln Gly Ala Ala Ile
            185                 190                 195

ctg gag cac ctg ggt gca cct tca agc acg tgg acc aag gtt cct acc     739
Leu Glu His Leu Gly Ala Pro Ser Ser Thr Trp Thr Lys Val Pro Thr
        200                 205                 210

gct gat ttg gaa gag gat cgc cca gcg ttg cca gat gag gaa gca ctt     787
Ala Asp Leu Glu Glu Asp Arg Pro Ala Leu Pro Asp Glu Glu Ala Leu
    215                 220                 225

ggt gtg tcg tat gcg gac atc gat aat tac ctg gaa aac aag ccc gat     835
Gly Val Ser Tyr Ala Asp Ile Asp Asn Tyr Leu Glu Asn Lys Pro Asp
230                 235                 240                 245

gtc agt gaa aaa gcc cag cag cgc att gag cac ctg tgg aag gtg ggc     883
Val Ser Glu Lys Ala Gln Gln Arg Ile Glu His Leu Trp Lys Val Gly
                250                 255                 260

cag cac aag cgc cac ctc cct gct acc ccg cag gaa aat tgg tgg cgt     931
Gln His Lys Arg His Leu Pro Ala Thr Pro Gln Glu Asn Trp Trp Arg
            265                 270                 275

taatccaaca gtttgagtgt cgc                                           954


<210> 8
<211> 277
<212> PRT
<213> Corynebacterium glutamicum

<400> 8
Met Thr Asn Thr Gln Thr Glu Ile Ile Asn Glu Leu Lys Val Ser Pro
1               5                   10                  15

Ala Ile Asp Val Ala Lys Glu Val Glu Phe Arg Val Gln Phe Leu Val
            20                  25                  30

Asp Tyr Leu Arg Ala Ser His Thr Lys Gly Phe Val Leu Gly Ile Ser
        35                  40                  45

Gly Gly Gln Asp Ser Thr Leu Ala Gly Arg Leu Thr Gln Leu Ala Val
    50                  55                  60

Glu Arg Ile Arg Ala Glu Glu Asn Ser Thr Asp Tyr Val Phe Tyr Ala
65                  70                  75                  80

Val Arg Leu Pro Tyr Ala Ile Gln Ala Asp Glu Asp Asp Ala Gln Val
                85                  90                  95

Ala Leu Glu Phe Ile Ala Pro Asp Lys Ser Val Thr Val Asn Val Lys
            100                 105                 110

Asp Ala Thr Asp Ala Thr Glu Ala Thr Val Ala Ala Ala Leu Glu Leu
        115                 120                 125

Pro Glu Leu Thr Asp Phe Asn Arg Gly Asn Ile Lys Ala Arg Gln Arg
    130                 135                 140

Met Val Ala Gln Tyr Ala Ile Ala Gly Gln Leu Gly Leu Leu Val Ile
145                 150                 155                 160

Gly Thr Asp His Ala Ala Glu Asn Val Thr Gly Phe Phe Thr Lys Phe
                165                 170                 175

Gly Asp Gly Ala Ala Asp Leu Leu Pro Leu Ala Gly Leu Ser Lys Arg
            180                 185                 190

Gln Gly Ala Ala Ile Leu Glu His Leu Gly Ala Pro Ser Ser Thr Trp
        195                 200                 205

Thr Lys Val Pro Thr Ala Asp Leu Glu Glu Asp Arg Pro Ala Leu Pro
    210                 215                 220

Asp Glu Glu Ala Leu Gly Val Ser Tyr Ala Asp Ile Asp Asn Tyr Leu

225                 230                 235                 240

Glu Asn Lys Pro Asp Val Ser Glu Lys Ala Gln Gln Arg Ile Glu His
                245                 250                 255

Leu Trp Lys Val Gly Gln His Lys Arg His Leu Pro Ala Thr Pro Gln
            260                 265                 270

Glu Asn Trp Trp Arg
        275


<210> 9
<211> 609
<212> DNA
<213> Corynebacterium glutamicum

<220>
<221> CDS
<222> (101)..(586)
<223> RXN02913

<400> 9
tccctgacat ccaggttgaa gcaacgtttg atgacggcac caagctcgtc accgtgcaca 60

atcccatccg ataacccttg atgtttttag gagttttgtc atg atc cca ggc gag   115
                                            Met Ile Pro Gly Glu
                                            1               5

tac atc ctg tcc agc gaa tca ctc acc gga aat gtt ggg cgc gag gcc   163
Tyr Ile Leu Ser Ser Glu Ser Leu Thr Gly Asn Val Gly Arg Glu Ala
                10                  15                  20

aaa acc atc gaa atc atc aac acc ggt gat agg cct gtg cag att ggt   211
Lys Thr Ile Glu Ile Ile Asn Thr Gly Asp Arg Pro Val Gln Ile Gly
            25                  30                  35

tcg cat ttc cac ttc gct gaa gta aac ccc agc atc agc ttt gat cgc   259
Ser His Phe His Phe Ala Glu Val Asn Pro Ser Ile Ser Phe Asp Arg
        40                  45                  50

agt gaa ggt tac ggc ttc cgc ctt gat att cca tcc ggc acc gcg gtg   307
Ser Glu Gly Tyr Gly Phe Arg Leu Asp Ile Pro Ser Gly Thr Ala Val
    55                  60                  65

cgc ctc gag cca ggc gat gcc cgc acc gtc aac ctt gtt gcc att ggc   355
Arg Leu Glu Pro Gly Asp Ala Arg Thr Val Asn Leu Val Ala Ile Gly
70                  75                  80                  85

ggt gac cgc att gtt gca ggt ttc cga gat ctc gtc gat ggg ccg ttg   403
Gly Asp Arg Ile Val Ala Gly Phe Arg Asp Leu Val Asp Gly Pro Leu
                90                  95                  100

gag gac ctc aaa gtc aac gtg tgg gag gga cgc gaa gac ggt tgg cgt   451
Glu Asp Leu Lys Val Asn Val Trp Glu Gly Arg Glu Asp Gly Trp Arg
            105                 110                 115

cgt tcc tca gct gct ggt gat gct cca caa gaa ttg cca cag gtc gaa   499
Arg Ser Ser Ala Ala Gly Asp Ala Pro Gln Glu Leu Pro Gln Val Glu
        120                 125                 130

gct gct gaa cgt ggc cgg aaa cta gat gac gcc act gat gtg gac aca   547
Ala Ala Glu Arg Gly Arg Lys Leu Asp Asp Ala Thr Asp Val Asp Thr
    135                 140                 145

aat gtg ggc aca gaa gaa ggc ttt gaa gaa ggt cga aat taaatgagtt   596
Asn Val Gly Thr Glu Glu Gly Phe Glu Glu Gly Arg Asn
150                 155                 160

ttgagatttc ccg                                                    609


<210> 10
<211> 162
<212> PRT
<213> Corynebacterium glutamicum

<400> 10
Met Ile Pro Gly Glu Tyr Ile Leu Ser Ser Glu Ser Leu Thr Gly Asn
1               5                   10                  15

Val Gly Arg Glu Ala Lys Thr Ile Glu Ile Ile Asn Thr Gly Asp Arg
            20                  25                  30

Pro Val Gln Ile Gly Ser His Phe His Phe Ala Glu Val Asn Pro Ser
        35                  40                  45

Ile Ser Phe Asp Arg Ser Glu Gly Tyr Gly Phe Arg Leu Asp Ile Pro
    50                  55                  60

Ser Gly Thr Ala Val Arg Leu Glu Pro Gly Asp Ala Arg Thr Val Asn
65                  70                  75                  80

Leu Val Ala Ile Gly Gly Asp Arg Ile Val Ala Gly Phe Arg Asp Leu
                85                  90                  95

Val Asp Gly Pro Leu Glu Asp Leu Lys Val Asn Val Trp Glu Gly Arg
            100                 105                 110

Glu Asp Gly Trp Arg Arg Ser Ser Ala Ala Gly Asp Ala Pro Gln Glu
        115                 120                 125

Leu Pro Gln Val Glu Ala Ala Glu Arg Gly Arg Lys Leu Asp Asp Ala
    130                 135                 140

Thr Asp Val Asp Thr Asn Val Gly Thr Glu Glu Gly Phe Glu Glu Gly
145                 150                 155                 160

Arg Asn



<210> 11
<211> 220
<212> DNA
<213> Corynebacterium glutamicum

<220>
<221> CDS
<222> (101)..(220)
<223> FRXA02264

<400> 11
tccctgacat ccaggttgaa gcaacgtttg atgacggcac caagctcgtc accgtgcaca 60

atcccatccg ataacccttg atgtttttag gagttttgtc atg atc cca ggc gag    115
                                            Met Ile Pro Gly Glu
                                            1               5

tac atc ctg tcc agc gaa tca ctc acc gga aat gtt ggg cgc gag gcc    163
Tyr Ile Leu Ser Ser Glu Ser Leu Thr Gly Asn Val Gly Arg Glu Ala
                10                  15                  20

aaa acc atc gaa atc atc aac acc ggt gat agg cct gtg cag att ggt    211
Lys Thr Ile Glu Ile Ile Asn Thr Gly Asp Arg Pro Val Gln Ile Gly
            25                  30                  35

tcg cat ttc                                                        220
Ser His Phe
        40


<210> 12
<211> 40
<212> PRT
<213> Corynebacterium glutamicum

<400> 12
Met Ile Pro Gly Glu Tyr Ile Leu Ser Ser Glu Ser Leu Thr Gly Asn
1               5                   10                  15

Val Gly Arg Glu Ala Lys Thr Ile Glu Ile Ile Asn Thr Gly Asp Arg
            20                  25                  30

Pro Val Gln Ile Gly Ser His Phe
        35                  40

<210> 13
<211> 1833
<212> DNA
<213> Corynebacterium glutamicum

<220>
<221> CDS
<222> (101)..(1810)
<223> RXN02274

<400> 13
acaggtcgaa gctgctgaac gtggccggaa actagatgac gccactgatg tggacacaaa 60

tgtgggcaca gaagaaggct ttgaagaagg tcgaaattaa atg agt ttt gag att   115
                                            Met Ser Phe Glu Ile
                                            1               5

tcc cgc aag cag tac acc gac ctt tat ggt cca acc gtt ggc gat tca   163
Ser Arg Lys Gln Tyr Thr Asp Leu Tyr Gly Pro Thr Val Gly Asp Ser
                10                  15                  20

gta cgt ctt gct gat act gag ctt ttt ctc tgt gtg gaa aaa gat tac   211
Val Arg Leu Ala Asp Thr Glu Leu Phe Leu Cys Val Glu Lys Asp Tyr
            25                  30                  35

gca gca atc ggc gaa gaa gta gca ttc ggc ggt ggc aag gtc att cgt   259
Ala Ala Ile Gly Glu Glu Val Ala Phe Gly Gly Gly Lys Val Ile Arg
        40                  45                  50

gat ggc atg ggc caa aat ggc acc ttg gtt cgc gat gta gat att ccc   307
Asp Gly Met Gly Gln Asn Gly Thr Leu Val Arg Asp Val Asp Ile Pro
    55                  60                  65

gat acc gtc atc acc aac gtc atc gtc ctt gac tat acg ggt gtg tac   355
Asp Thr Val Ile Thr Asn Val Ile Val Leu Asp Tyr Thr Gly Val Tyr
70                  75                  80                  85

aaa gct gac gtt gcg ctt cga gat ggc aaa atc ttc cga atc gga aag   403
Lys Ala Asp Val Ala Leu Arg Asp Gly Lys Ile Phe Arg Ile Gly Lys
                90                  95                  100

gcc gga aac ccg aat gtc atg gaa aac gtc gac atc gtc atc ggc gtt   451
Ala Gly Asn Pro Asn Val Met Glu Asn Val Asp Ile Val Ile Gly Val
            105                 110                 115

gcc acc gac atc att gct ggt gaa ggc aaa atc ctt acc gca ggt ggc   499
Ala Thr Asp Ile Ile Ala Gly Glu Gly Lys Ile Leu Thr Ala Gly Gly
        120                 125                 130

atc gac acg cac gtg cac ttc ttg ggc aca gac cag gtc aac act gca   547
Ile Asp Thr His Val His Phe Leu Gly Thr Asp Gln Val Asn Thr Ala
    135                 140                 145

tta gca tca ggt atc acc acg atg atc ggt gga ggc acc ggc cca agc   595
Leu Ala Ser Gly Ile Thr Thr Met Ile Gly Gly Gly Thr Gly Pro Ser
150                 155                 160                 165

cag gcg tcg atg gct aca act gtc acg cca ggt cag tgg aat acc tac   643
Gln Ala Ser Met Ala Thr Thr Val Thr Pro Gly Gln Trp Asn Thr Tyr
                170                 175                 180

aac atg ctt agt gct ttt gaa ggc atg ccc atg aac ttt ggc att ttg   691
Asn Met Leu Ser Ala Phe Glu Gly Met Pro Met Asn Phe Gly Ile Leu
            185                 190                 195

ggt aaa ggc cat ggt tct tcc aaa tct ccg ctg gct gag cag gtt cgt   739
Gly Lys Gly His Gly Ser Ser Lys Ser Pro Leu Ala Glu Gln Val Arg
        200                 205                 210

gcg ggt gca atc ggt ctg aaa att cac gag gac tgg ggt gcc aca cca   787
Ala Gly Ala Ile Gly Leu Lys Ile His Glu Asp Trp Gly Ala Thr Pro

215                 220                 225

tcg tcg atc aac act gcc cta gaa gta gcc gat gac atg gac atc cag      835
Ser Ser Ile Asn Thr Ala Leu Glu Val Ala Asp Asp Met Asp Ile Gln
230                 235                 240                 245

gtg gca ctc cac tcc gat acc ttg aat gag gcc ggt ttt gtg gaa gac      883
Val Ala Leu His Ser Asp Thr Leu Asn Glu Ala Gly Phe Val Glu Asp
                250                 255                 260

acc att gaa gcc att gcg ggc cga gtc atc cat acc ttc cac acc gaa      931
Thr Ile Glu Ala Ile Ala Gly Arg Val Ile His Thr Phe His Thr Glu
            265                 270                 275

ggt gct ggt ggt gga cac gct cct gac cta atc cga gtg gct gct ctg      979
Gly Ala Gly Gly Gly His Ala Pro Asp Leu Ile Arg Val Ala Ala Leu
        280                 285                 290

cca aac gtg ttg cct gca tcc acc aac cca acg ctc cca tac acc cga     1027
Pro Asn Val Leu Pro Ala Ser Thr Asn Pro Thr Leu Pro Tyr Thr Arg
    295                 300                 305

aac act gtt gaa gag cac ctg gac atg gtg atg gtt gcc cac cac ctc     1075
Asn Thr Val Glu Glu His Leu Asp Met Val Met Val Ala His His Leu
310                 315                 320                 325

aac cca gat att cca gaa gac gtg gct ttt gcg gat tcc cga att cgt     1123
Asn Pro Asp Ile Pro Glu Asp Val Ala Phe Ala Asp Ser Arg Ile Arg
                330                 335                 340

gcc gaa acg att gca gcc gaa gat gtg ctt cac gat atg ggt atc ttc     1171
Ala Glu Thr Ile Ala Ala Glu Asp Val Leu His Asp Met Gly Ile Phe
            345                 350                 355

tct atc acc tct tcg gat tcc cag gcg atg ggc cga gta gga gag acc     1219
Ser Ile Thr Ser Ser Asp Ser Gln Ala Met Gly Arg Val Gly Glu Thr
        360                 365                 370

atc acg cgc acg tgg cag gtc gcc gac cat atg aaa cgc acc cgt gga     1267
Ile Thr Arg Thr Trp Gln Val Ala Asp His Met Lys Arg Thr Arg Gly
    375                 380                 385

tca cta acg gga gat gct cca tac aac gac aac aac cgc ttg cgt cga     1315
Ser Leu Thr Gly Asp Ala Pro Tyr Asn Asp Asn Asn Arg Leu Arg Arg
390                 395                 400                 405

ttc atc gca aaa tac acc atc aac cct gcg att gcg cac ggt gtg gat     1363
Phe Ile Ala Lys Tyr Thr Ile Asn Pro Ala Ile Ala His Gly Val Asp
                410                 415                 420

tat gtt gtt cgt tca gtg gag gaa ggc aag ttc gct gac ctc gtg ctg     1411
Tyr Val Val Arg Ser Val Glu Glu Gly Lys Phe Ala Asp Leu Val Leu
            425                 430                 435

tgg gat cca aag ttc ttt ggt gtg aaa cct gat ctg gtg atc aag ggt     1459
Trp Asp Pro Lys Phe Phe Gly Val Lys Pro Asp Leu Val Ile Lys Gly
        440                 445                 450

ggg ttg atg gtc aat tcc ctc atg ggt gat tcc aac ggt tcc att cca     1507
Gly Leu Met Val Asn Ser Leu Met Gly Asp Ser Asn Gly Ser Ile Pro
    455                 460                 465

act ccg cag ccc cgc acc ctg cgc aat act tgg ggt gcg ttt ggc cag     1555
Thr Pro Gln Pro Arg Thr Leu Arg Asn Thr Trp Gly Ala Phe Gly Gln
470                 475                 480                 485

gca gtt tcc aga agc tcc att aca ttc cta tcc cag gac gct atc gat     1603
Ala Val Ser Arg Ser Ser Ile Thr Phe Leu Ser Gln Asp Ala Ile Asp
                490                 495                 500

gca aat gtt cct gat ctg ctg aat ctg agg aag cag atc cgg ggc gtt     1651
Ala Asn Val Pro Asp Leu Leu Asn Leu Arg Lys Gln Ile Arg Gly Val
            505                 510                 515

cga ggt gta agg aat ctg acc aaa cga gac atg aaa ctc aat gca gaa     1699
Arg Gly Val Arg Asn Leu Thr Lys Arg Asp Met Lys Leu Asn Ala Glu
        520                 525                 530

atg cct gat att cgt gtc gat cca gag acc tac cag gtg ttt gtc aac     1747
Met Pro Asp Ile Arg Val Asp Pro Glu Thr Tyr Gln Val Phe Val Asn
    535                 540                 545

ggt gag ttg atc acc agc aag cca gca gag aca gtg cca atg gca cgt     1795
Gly Glu Leu Ile Thr Ser Lys Pro Ala Glu Thr Val Pro Met Ala Arg
550                 555                 560                 565

cgc tac ttc ttg ttc taatccgcca acaaggaagg aag                       1833
Arg Tyr Phe Leu Phe
                570


<210> 14
<211> 570
<212> PRT
<213> Corynebacterium glutamicum

<400> 14
Met Ser Phe Glu Ile Ser Arg Lys Gln Tyr Thr Asp Leu Tyr Gly Pro
  1               5                  10                  15

Thr Val Gly Asp Ser Val Arg Leu Ala Asp Thr Glu Leu Phe Leu Cys
             20                  25                  30

Val Glu Lys Asp Tyr Ala Ala Ile Gly Glu Glu Val Ala Phe Gly Gly
         35                  40                  45

Gly Lys Val Ile Arg Asp Gly Met Gly Gln Asn Gly Thr Leu Val Arg
     50                  55                  60

Asp Val Asp Ile Pro Asp Thr Val Ile Thr Asn Val Ile Val Leu Asp
 65                  70                  75                  80

Tyr Thr Gly Val Tyr Lys Ala Asp Val Ala Leu Arg Asp Gly Lys Ile
                 85                  90                  95

Phe Arg Ile Gly Lys Ala Gly Asn Pro Asn Val Met Glu Asn Val Asp
            100                 105                 110

Ile Val Ile Gly Val Ala Thr Asp Ile Ile Ala Gly Glu Gly Lys Ile
        115                 120                 125

Leu Thr Ala Gly Gly Ile Asp Thr His Val His Phe Leu Gly Thr Asp
    130                 135                 140

Gln Val Asn Thr Ala Leu Ala Ser Gly Ile Thr Thr Met Ile Gly Gly
145                 150                 155                 160

Gly Thr Gly Pro Ser Gln Ala Ser Met Ala Thr Thr Val Thr Pro Gly
                165                 170                 175

Gln Trp Asn Thr Tyr Asn Met Leu Ser Ala Phe Glu Gly Met Pro Met
            180                 185                 190

Asn Phe Gly Ile Leu Gly Lys Gly His Gly Ser Ser Lys Ser Pro Leu
        195                 200                 205

Ala Glu Gln Val Arg Ala Gly Ala Ile Gly Leu Lys Ile His Glu Asp
    210                 215                 220

Trp Gly Ala Thr Pro Ser Ser Ile Asn Thr Ala Leu Glu Val Ala Asp
225                 230                 235                 240

Asp Met Asp Ile Gln Val Ala Leu His Ser Asp Thr Leu Asn Glu Ala
                245                 250                 255

Gly Phe Val Glu Asp Thr Ile Glu Ala Ile Ala Gly Arg Val Ile His
            260                 265                 270

Thr Phe His Thr Glu Gly Ala Gly Gly Gly His Ala Pro Asp Leu Ile
        275                 280                 285

Arg Val Ala Ala Leu Pro Asn Val Leu Pro Ala Ser Thr Asn Pro Thr
    290                 295                 300

Leu Pro Tyr Thr Arg Asn Thr Val Glu Glu His Leu Asp Met Val Met
305                 310                 315                 320

Val Ala His His Leu Asn Pro Asp Ile Pro Glu Asp Val Ala Phe Ala
                325                 330                 335

Asp Ser Arg Ile Arg Ala Glu Thr Ile Ala Ala Glu Asp Val Leu His
            340                 345                 350

Asp Met Gly Ile Phe Ser Ile Thr Ser Ser Asp Ser Gln Ala Met Gly
        355                 360                 365

Arg Val Gly Glu Thr Ile Thr Arg Thr Trp Gln Val Ala Asp His Met
    370                 375                 380

Lys Arg Thr Arg Gly Ser Leu Thr Gly Asp Ala Pro Tyr Asn Asp Asn
385                 390                 395                 400

Asn Arg Leu Arg Arg Phe Ile Ala Lys Tyr Thr Ile Asn Pro Ala Ile
                405                 410                 415

Ala His Gly Val Asp Tyr Val Val Arg Ser Val Glu Glu Gly Lys Phe
            420                 425                 430

Ala Asp Leu Val Leu Trp Asp Pro Lys Phe Phe Gly Val Lys Pro Asp
        435                 440                 445

Leu Val Ile Lys Gly Gly Leu Met Val Asn Ser Leu Met Gly Asp Ser
    450                 455                 460

Asn Gly Ser Ile Pro Thr Pro Gln Pro Arg Thr Leu Arg Asn Thr Trp
465                 470                 475                 480

Gly Ala Phe Gly Gln Ala Val Ser Arg Ser Ser Ile Thr Phe Leu Ser
                485                 490                 495

Gln Asp Ala Ile Asp Ala Asn Val Pro Asp Leu Leu Asn Leu Arg Lys
            500                 505                 510

Gln Ile Arg Gly Val Arg Gly Val Arg Asn Leu Thr Lys Arg Asp Met
        515                 520                 525

Lys Leu Asn Ala Glu Met Pro Asp Ile Arg Val Asp Pro Glu Thr Tyr
    530                 535                 540

Gln Val Phe Val Asn Gly Glu Leu Ile Thr Ser Lys Pro Ala Glu Thr
545                 550                 555                 560

Val Pro Met Ala Arg Arg Tyr Phe Leu Phe
                565                 570


<210> 15
<211> 1625
<212> DNA
<213> Corynebacterium glutamicum

<220>
<221> CDS
<222> (1)..(1602)
<223> FRXA02274

<400> 15
tac gca gca atc ggc gaa gaa gta gca ttc ggc ggt ggc aag gtc att      48
Tyr Ala Ala Ile Gly Glu Glu Val Ala Phe Gly Gly Gly Lys Val Ile
  1               5                  10                  15

cgt gat ggc atg ggc caa aat ggc acc ttg gtt cgc gat gta gat att      96
Arg Asp Gly Met Gly Gln Asn Gly Thr Leu Val Arg Asp Val Asp Ile
             20                  25                  30

ccc gat acc gtc atc acc aac gtc atc gtc ctt gac tat acg ggt gtg     144
Pro Asp Thr Val Ile Thr Asn Val Ile Val Leu Asp Tyr Thr Gly Val
         35                  40                  45

tac aaa gct gac gtt gcg ctt cga gat ggc aaa atc ttc cga atc gga     192
Tyr Lys Ala Asp Val Ala Leu Arg Asp Gly Lys Ile Phe Arg Ile Gly

50                  55                  60

aag gcc gga aac ccg aat gtc atg gaa aac gtc gac atc gtc atc ggc     240
Lys Ala Gly Asn Pro Asn Val Met Glu Asn Val Asp Ile Val Ile Gly
 65                  70                  75                  80

gtt gcc acc gac atc att gct ggt gaa ggc aaa atc ctt acc gca ggt     288
Val Ala Thr Asp Ile Ile Ala Gly Glu Gly Lys Ile Leu Thr Ala Gly
                 85                  90                  95

ggc atc gac acg cac gtg cac ttc ttg ggc aca gac cag gtc aac act     336
Gly Ile Asp Thr His Val His Phe Leu Gly Thr Asp Gln Val Asn Thr
            100                 105                 110

gca tta gca tca ggt atc acc acg atg atc ggt gga ggc acc ggc cca     384
Ala Leu Ala Ser Gly Ile Thr Thr Met Ile Gly Gly Gly Thr Gly Pro
        115                 120                 125

agc cag gcg tcg atg gct aca act gtc acg cca ggt cag tgg aat acc     432
Ser Gln Ala Ser Met Ala Thr Thr Val Thr Pro Gly Gln Trp Asn Thr
    130                 135                 140

tac aac atg ctt agt gct ttt gaa ggc atg ccc atg aac ttt ggc att     480
Tyr Asn Met Leu Ser Ala Phe Glu Gly Met Pro Met Asn Phe Gly Ile
145                 150                 155                 160

ttg ggt aaa ggc cat ggt tct tcc aaa tct ccg ctg gct gag cag gtt     528
Leu Gly Lys Gly His Gly Ser Ser Lys Ser Pro Leu Ala Glu Gln Val
                165                 170                 175

cgt gcg ggt gca atc ggt ctg aaa att cac gag gac tgg ggt gcc aca     576
Arg Ala Gly Ala Ile Gly Leu Lys Ile His Glu Asp Trp Gly Ala Thr
            180                 185                 190

cca tcg tcg atc aac act gcc cta gaa gta gcc gat gac atg gac atc     624

Pro Ser Ser Ile Asn Thr Ala Leu Glu Val Ala Asp Asp Met Asp Ile
        195                 200                 205

cag gtg gca ctc cac tcc gat acc ttg aat gag gcc ggt ttt gtg gaa     672
Gln Val Ala Leu His Ser Asp Thr Leu Asn Glu Ala Gly Phe Val Glu
    210                 215                 220

gac acc att gaa gcc att gcg ggc cga gtc atc cat acc ttc cac acc     720
Asp Thr Ile Glu Ala Ile Ala Gly Arg Val Ile His Thr Phe His Thr
225                 230                 235                 240

gaa ggt gct ggt ggt gga cac gct cct gac cta atc cga gtg gct gct     768
Glu Gly Ala Gly Gly Gly His Ala Pro Asp Leu Ile Arg Val Ala Ala
                245                 250                 255

ctg cca aac gtg ttg cct gca tcc acc aac cca acg ctc cca tac acc     816
Leu Pro Asn Val Leu Pro Ala Ser Thr Asn Pro Thr Leu Pro Tyr Thr
            260                 265                 270

cga aac act gtt gaa gag cac ctg gac atg gtg atg gtt gcc cac cac     864
Arg Asn Thr Val Glu Glu His Leu Asp Met Val Met Val Ala His His
        275                 280                 285

ctc aac cca gat att cca gaa gac gtg gct ttt gcg gat tcc cga att     912
Leu Asn Pro Asp Ile Pro Glu Asp Val Ala Phe Ala Asp Ser Arg Ile
    290                 295                 300

cgt gcc gaa acg att gca gcc gaa gat gtg ctt cac gat atg ggt atc     960
Arg Ala Glu Thr Ile Ala Ala Glu Asp Val Leu His Asp Met Gly Ile
305                 310                 315                 320

ttc tct atc acc tct tcg gat tcc cag gcg atg ggc cga gta gga gag    1008
Phe Ser Ile Thr Ser Ser Asp Ser Gln Ala Met Gly Arg Val Gly Glu
                325                 330                 335

acc atc acg cgc acg tgg cag gtc gcc gac cat atg aaa cgc acc cgt    1056
Thr Ile Thr Arg Thr Trp Gln Val Ala Asp His Met Lys Arg Thr Arg
            340                 345                 350

gga tca cta acg gga gat gct cca tac aac gac aac aac cgc ttg cgt    1104
Gly Ser Leu Thr Gly Asp Ala Pro Tyr Asn Asp Asn Asn Arg Leu Arg

355                 360                 365

cga ttc atc gca aaa tac acc atc aac cct gcg att gcg cac ggt gtg      1152
Arg Phe Ile Ala Lys Tyr Thr Ile Asn Pro Ala Ile Ala His Gly Val
    370                 375                 380

gat tat gtt gtt cgt tca gtg gag gaa ggc aag ttc gct gac ctc gtg      1200
Asp Tyr Val Val Arg Ser Val Glu Glu Gly Lys Phe Ala Asp Leu Val
385                 390                 395                 400

ctg tgg gat cca aag ttc ttt ggt gtg aaa cct gat ctg gtg atc aag      1248
Leu Trp Asp Pro Lys Phe Phe Gly Val Lys Pro Asp Leu Val Ile Lys
                405                 410                 415

ggt ggg ttg atg gtc aat tcc ctc atg ggt gat tcc aac ggt tcc att      1296
Gly Gly Leu Met Val Asn Ser Leu Met Gly Asp Ser Asn Gly Ser Ile
            420                 425                 430

cca act ccg cag ccc cgc acc ctg cgc aat act tgg ggt gcg ttt ggc      1344
Pro Thr Pro Gln Pro Arg Thr Leu Arg Asn Thr Trp Gly Ala Phe Gly
        435                 440                 445

cag gca gtt tcc aga agc tcc att aca ttc cta tcc cag gac gct atc      1392
Gln Ala Val Ser Arg Ser Ser Ile Thr Phe Leu Ser Gln Asp Ala Ile
    450                 455                 460

gat gca aat gtt cct gat ctg ctg aat ctg agg aag cag atc cgg ggc      1440
Asp Ala Asn Val Pro Asp Leu Leu Asn Leu Arg Lys Gln Ile Arg Gly
465                 470                 475                 480

gtt cga ggt gta agg aat ctg acc aaa cga gac atg aaa ctc aat gca      1488
Val Arg Gly Val Arg Asn Leu Thr Lys Arg Asp Met Lys Leu Asn Ala
                485                 490                 495

gaa atg cct gat att cgt gtc gat cca gag acc tac cag gtg ttt gtc      1536
Glu Met Pro Asp Ile Arg Val Asp Pro Glu Thr Tyr Gln Val Phe Val
            500                 505                 510

aac ggt gag ttg atc acc agc aag cca gca gag aca gtg cca atg gca      1584
Asn Gly Glu Leu Ile Thr Ser Lys Pro Ala Glu Thr Val Pro Met Ala
        515                 520                 525

cgt cgc tac ttc ttg ttc taatccgcca acaaggaagg aag                    1625
Arg Arg Tyr Phe Leu Phe
    530


<210> 16
<211> 534
<212> PRT
<213> Corynebacterium glutamicum

<400> 16
Tyr Ala Ala Ile Gly Glu Glu Val Ala Phe Gly Gly Gly Lys Val Ile
1               5                   10                  15

Arg Asp Gly Met Gly Gln Asn Gly Thr Leu Val Arg Asp Val Asp Ile
            20                  25                  30

Pro Asp Thr Val Ile Thr Asn Val Ile Val Leu Asp Tyr Thr Gly Val
        35                  40                  45

Tyr Lys Ala Asp Val Ala Leu Arg Asp Gly Lys Ile Phe Arg Ile Gly
    50                  55                  60

Lys Ala Gly Asn Pro Asn Val Met Glu Asn Val Asp Ile Val Ile Gly
65                  70                  75                  80

Val Ala Thr Asp Ile Ile Ala Gly Glu Gly Lys Ile Leu Thr Ala Gly
                85                  90                  95

Gly Ile Asp Thr His Val His Phe Leu Gly Thr Asp Gln Val Asn Thr
            100                 105                 110

Ala Leu Ala Ser Gly Ile Thr Thr Met Ile Gly Gly Gly Thr Gly Pro
        115                 120                 125

Ser Gln Ala Ser Met Ala Thr Thr Val Thr Pro Gly Gln Trp Asn Thr
    130                 135                 140

Tyr Asn Met Leu Ser Ala Phe Glu Gly Met Pro Met Asn Phe Gly Ile
145                 150                 155                 160

Leu Gly Lys Gly His Gly Ser Ser Lys Ser Pro Leu Ala Glu Gln Val
                165                 170                 175

Arg Ala Gly Ala Ile Gly Leu Lys Ile His Glu Asp Trp Gly Ala Thr
            180                 185                 190

Pro Ser Ser Ile Asn Thr Ala Leu Glu Val Ala Asp Asp Met Asp Ile
        195                 200                 205

Gln Val Ala Leu His Ser Asp Thr Leu Asn Glu Ala Gly Phe Val Glu
    210                 215                 220

Asp Thr Ile Glu Ala Ile Ala Gly Arg Val Ile His Thr Phe His Thr
225                 230                 235                 240

Glu Gly Ala Gly Gly Gly His Ala Pro Asp Leu Ile Arg Val Ala Ala
                245                 250                 255

Leu Pro Asn Val Leu Pro Ala Ser Thr Asn Pro Thr Leu Pro Tyr Thr
            260                 265                 270

Arg Asn Thr Val Glu Glu His Leu Asp Met Val Met Val Ala His His
        275                 280                 285

Leu Asn Pro Asp Ile Pro Glu Asp Val Ala Phe Ala Asp Ser Arg Ile

    290                 295                 300

Arg Ala Glu Thr Ile Ala Ala Glu Asp Val Leu His Asp Met Gly Ile
305                 310                 315                 320

Phe Ser Ile Thr Ser Ser Asp Ser Gln Ala Met Gly Arg Val Gly Glu
                325                 330                 335

Thr Ile Thr Arg Thr Trp Gln Val Ala Asp His Met Lys Arg Thr Arg
            340                 345                 350

Gly Ser Leu Thr Gly Asp Ala Pro Tyr Asn Asp Asn Asn Arg Leu Arg
        355                 360                 365

Arg Phe Ile Ala Lys Tyr Thr Ile Asn Pro Ala Ile Ala His Gly Val
    370                 375                 380

Asp Tyr Val Val Arg Ser Val Glu Glu Gly Lys Phe Ala Asp Leu Val
385                 390                 395                 400

Leu Trp Asp Pro Lys Phe Phe Gly Val Lys Pro Asp Leu Val Ile Lys
                405                 410                 415

Gly Gly Leu Met Val Asn Ser Leu Met Gly Asp Ser Asn Gly Ser Ile
            420                 425                 430

Pro Thr Pro Gln Pro Arg Thr Leu Arg Asn Thr Trp Gly Ala Phe Gly
        435                 440                 445

Gln Ala Val Ser Arg Ser Ser Ile Thr Phe Leu Ser Gln Asp Ala Ile
    450                 455                 460

Asp Ala Asn Val Pro Asp Leu Leu Asn Leu Arg Lys Gln Ile Arg Gly
465                 470                 475                 480

Val Arg Gly Val Arg Asn Leu Thr Lys Arg Asp Met Lys Leu Asn Ala
                485                 490                 495

Glu Met Pro Asp Ile Arg Val Asp Pro Glu Thr Tyr Gln Val Phe Val
            500                 505                 510

Asn Gly Glu Leu Ile Thr Ser Lys Pro Ala Glu Thr Val Pro Met Ala
        515                 520                 525

Arg Arg Tyr Phe Leu Phe

    530

<210> 17
<211> 423
<212> DNA
<213> Corynebacterium glutamicum

<220>
<221> CDS
<222> (101)..(400)
<223> RXA02265

<400> 17
tcaaaggaaa gcagtatatt gtcggttttt tagaggctct cccaaatatt gcgttggggg 60

agctaaacta atttctgtta cctgacagaa aggggcaaaa ttg cat atc act cct 115
Leu His Ile Thr Pro
1               5

cgt gaa caa gaa aaa ctg atg atc gtg gtg gcg gct gac ctt gca cgt 163
Arg Glu Gln Glu Lys Leu Met Ile Val Val Ala Ala Asp Leu Ala Arg
                10                  15                  20

cgc cgt aaa gat cgc ggc cta aaa ctt aac cac cca gag gcc gtc gcc 211
Arg Arg Lys Asp Arg Gly Leu Lys Leu Asn His Pro Glu Ala Val Ala
            25                  30                  35

ctc atc acg tat gaa ctg att gaa ggc gcc cgt gac gga cgc aca gtc 259
Leu Ile Thr Tyr Glu Leu Ile Glu Gly Ala Arg Asp Gly Arg Thr Val
        40                  45                  50

gca gac ctt atg agc tgg gga agc acc att ttg act agg gat gat gtc 307
Ala Asp Leu Met Ser Trp Gly Ser Thr Ile Leu Thr Arg Asp Asp Val
    55                  60                  65

tta gaa ggc atc cca gag atg atc cct gac atc cag gtt gaa gca acg 355
Leu Glu Gly Ile Pro Glu Met Ile Pro Asp Ile Gln Val Glu Ala Thr
70                  75                  80                  85

ttt gat gac ggc acc aag ctc gtc acc gtg cac aat ccc atc cga 400
Phe Asp Asp Gly Thr Lys Leu Val Thr Val His Asn Pro Ile Arg
                90                  95                  100

taacccttga tgtttttagg agt 423


<210> 18
<211> 100
<212> PRT
<213> Corynebacterium glutamicum

<400> 18
Leu His Ile Thr Pro Arg Glu Gln Glu Lys Leu Met Ile Val Val Ala
1               5                   10                  15

Ala Asp Leu Ala Arg Arg Arg Lys Asp Arg Gly Leu Lys Leu Asn His
            20                  25                  30

Pro Glu Ala Val Ala Leu Ile Thr Tyr Glu Leu Ile Glu Gly Ala Arg
        35                  40                  45

Asp Gly Arg Thr Val Ala Asp Leu Met Ser Trp Gly Ser Thr Ile Leu
    50                  55                  60

Thr Arg Asp Asp Val Leu Glu Gly Ile Pro Glu Met Ile Pro Asp Ile
65                  70                  75                  80

Gln Val Glu Ala Thr Phe Asp Asp Gly Thr Lys Leu Val Thr Val His
                85                  90                  95

Asn Pro Ile Arg
            100


<210> 19

<211> 972
<212> DNA
<213> Corynebacterium glutamicum

<220>
<221> CDS
<222> (101)..(949)
<223> RXA02278

<400> 19
accattctgc ctgactaatc tgcgcaccga tgatggtttg gataaggtct tggaatggat 60

ccgccatgag gtgatgatgc aggacttgca ggaagcctaa atg aca caa acc caa 115
Met Thr Gln Thr Gln
1               5

cca gtg gga acc ctg cga ctg acc atc gat gat caa gga ccc caa ggt 163
Pro Val Gly Thr Leu Arg Leu Thr Ile Asp Asp Gln Gly Pro Gln Gly
                10                  15                  20

caa agc cgt gcg gtg gag caa ttt cac cag ggt gcg ctt cga gtc atc 211
Gln Ser Arg Ala Val Glu Gln Phe His Gln Gly Ala Leu Arg Val Ile
            25                  30                  35

cgg cca cac tac ttg gat gat tcc gga cag gtt tgc tac acc atc att 259
Arg Pro His Tyr Leu Asp Asp Ser Gly Gln Val Cys Tyr Thr Ile Ile
        40                  45                  50

gcc att ggt ggc gga tac ctg ggc ggc gat gtg tat gag cag caa ttc 307
Ala Ile Gly Gly Gly Tyr Leu Gly Gly Asp Val Tyr Glu Gln Gln Phe
    55                  60                  65

acg atc aaa gac aac gca aaa gct ttg atc acc acg caa tcg gcc acc 355
Thr Ile Lys Asp Asn Ala Lys Ala Leu Ile Thr Thr Gln Ser Ala Thr
70                  75                  80                  85

aag att tat cgc aca ccg caa gga cca gcc acg cag cac acc gaa atc 403
Lys Ile Tyr Arg Thr Pro Gln Gly Pro Ala Thr Gln His Thr Glu Ile
                90                  95                  100

aac gtc ggt gaa aat gct gtg ctg gaa tac ttg gcg gat caa acc atc 451
Asn Val Gly Glu Asn Ala Val Leu Glu Tyr Leu Ala Asp Gln Thr Ile
            105                 110                 115

gcg tac cgg gag gcc acc tat cat caa ttc acc aag gtg gcg ctg cac 499
Ala Tyr Arg Glu Ala Thr Tyr His Gln Phe Thr Lys Val Ala Leu His
        120                 125                 130

ccg agc gca acg ttt gtg atg agc gaa caa atc acc cca ggc tgg cac 547
Pro Ser Ala Thr Phe Val Met Ser Glu Gln Ile Thr Pro Gly Trp His
    135                 140                 145

ccc gac ggc aaa cac ttt gct tac gat gaa atg cgt cta cac acc gaa 595
Pro Asp Gly Lys His Phe Ala Tyr Asp Glu Met Arg Leu His Thr Glu
150                 155                 160                 165

atc acg gac tcc acc aca ggg cga ctc gtg ctc ttg gat aat tta ctg 643
Ile Thr Asp Ser Thr Thr Gly Arg Leu Val Leu Leu Asp Asn Leu Leu
                170                 175                 180

ctc cgg ccg gac tcc cga gag gga agt ttt ggg tgg acg gaa cag tac 691
Leu Arg Pro Asp Ser Arg Glu Gly Ser Phe Gly Trp Thr Glu Gln Tyr
            185                 190                 195

aca cat tca ggg cag atg att gtg atg ggg gaa ggc gtc gat aag cag 739
Thr His Ser Gly Gln Met Ile Val Met Gly Glu Gly Val Asp Lys Gln
        200                 205                 210

ctt gtt gct gag ctg aat gag caa ctt gcc gcg cac cct gat gtg tac 787
Leu Val Ala Glu Leu Asn Glu Gln Leu Ala Ala His Pro Asp Val Tyr
    215                 220                 225

ggc gcc gtc aat ttc tta agc gcg ccg ggc acg tta ctg cgc gga ttt 835
Gly Ala Val Asn Phe Leu Ser Ala Pro Gly Thr Leu Leu Arg Gly Phe
230                 235                 240                 245

att gcg cgc acg ctg agc aac cgc act gag gag ttg att aac ctg cac 883

Ile Ala Arg Thr Leu Ser Asn Arg Thr Glu Glu Leu Ile Asn Leu His
                250                 255                 260

gaa cac att gcg tcg ctg ttg cgc ggg cgg tgg cgc ggg cag gaa ccg     931
Glu His Ile Ala Ser Leu Leu Arg Gly Arg Trp Arg Gly Gln Glu Pro
            265                 270                 275

gtg aat ttg cgg aag tac tagacggcgt cgagaaatcg aag                   972
Val Asn Leu Arg Lys Tyr
        280


<210> 20
<211> 283
<212> PRT
<213> Corynebacterium glutamicum

<400> 20
Met Thr Gln Thr Gln Pro Val Gly Thr Leu Arg Leu Thr Ile Asp Asp
  1               5                  10                  15

Gln Gly Pro Gln Gly Gln Ser Arg Ala Val Glu Gln Phe His Gln Gly
             20                  25                  30

Ala Leu Arg Val Ile Arg Pro His Tyr Leu Asp Asp Ser Gly Gln Val
         35                  40                  45

Cys Tyr Thr Ile Ile Ala Ile Gly Gly Gly Tyr Leu Gly Gly Asp Val
     50                  55                  60

Tyr Glu Gln Gln Phe Thr Ile Lys Asp Asn Ala Lys Ala Leu Ile Thr
 65                  70                  75                  80

Thr Gln Ser Ala Thr Lys Ile Tyr Arg Thr Pro Gln Gly Pro Ala Thr
                 85                  90                  95

Gln His Thr Glu Ile Asn Val Gly Glu Asn Ala Val Leu Glu Tyr Leu
            100                 105                 110

Ala Asp Gln Thr Ile Ala Tyr Arg Glu Ala Thr Tyr His Gln Phe Thr
        115                 120                 125

Lys Val Ala Leu His Pro Ser Ala Thr Phe Val Met Ser Glu Gln Ile
    130                 135                 140

Thr Pro Gly Trp His Pro Asp Gly Lys His Phe Ala Tyr Asp Glu Met
145                 150                 155                 160

Arg Leu His Thr Glu Ile Thr Asp Ser Thr Thr Gly Arg Leu Val Leu
                165                 170                 175

Leu Asp Asn Leu Leu Leu Arg Pro Asp Ser Arg Glu Gly Ser Phe Gly
            180                 185                 190

Trp Thr Glu Gln Tyr Thr His Ser Gly Gln Met Ile Val Met Gly Glu
        195                 200                 205

Gly Val Asp Lys Gln Leu Val Ala Glu Leu Asn Glu Gln Leu Ala Ala
    210                 215                 220

His Pro Asp Val Tyr Gly Ala Val Asn Phe Leu Ser Ala Pro Gly Thr
225                 230                 235                 240

Leu Leu Arg Gly Phe Ile Ala Arg Thr Leu Ser Asn Arg Thr Glu Glu
                245                 250                 255

Leu Ile Asn Leu His Glu His Ile Ala Ser Leu Leu Arg Gly Arg Trp
            260                 265                 270

Arg Gly Gln Glu Pro Val Asn Leu Arg Lys Tyr
        275                 280


<210> 21
<211> 594
<212> DNA
<213> Corynebacterium glutamicum

<220>
<221> CDS
<222> (101)..(571)
<223> RXA02275

<400> 21
gtttgtcaac ggtgagttga tcaccagcaa gccagcagag acagtgccaa tggcacgtcg 60

ctacttcttg ttctaatccg ccaacaagga aggaagatcc atg att atc act gcg 115
Met Ile Ile Thr Ala
1               5

atc gac acc aac atc tac gat gaa ccg gag ttt gtt gaa gga cgc gat 163
Ile Asp Thr Asn Ile Tyr Asp Glu Pro Glu Phe Val Glu Gly Arg Asp
                10                  15                  20

gtc atc ggt gtg cgc ttt gaa gat tta gtt ttg gat aag cgc att caa 211
Val Ile Gly Val Arg Phe Glu Asp Leu Val Leu Asp Lys Arg Ile Gln
            25                  30                  35

cgg gtt gca ctc ccc gga gga gaa gaa ctg ggg ttg cgg tta aac cac 259
Arg Val Ala Leu Pro Gly Gly Glu Glu Leu Gly Leu Arg Leu Asn His
        40                  45                  50

ggg cat ccg att ctg cgt gaa ggt gat gtg ttg aaa gct gat gat aag 307
Gly His Pro Ile Leu Arg Glu Gly Asp Val Leu Lys Ala Asp Asp Lys
    55                  60                  65

acg gta ttt gtg gtg gag att atc ccc acg gat gtt tta gtt atc acg 355
Thr Val Phe Val Val Glu Ile Ile Pro Thr Asp Val Leu Val Ile Thr
70                  75                  80                  85

cca agc gat att cac cag atg gga ttt gtg gcg cac tcc ctg gga aac 403
Pro Ser Asp Ile His Gln Met Gly Phe Val Ala His Ser Leu Gly Asn
                90                  95                  100

agg cac ctg cca gca cag ttt tcc aag cca ggt gaa ttg aca gag aag 451
Arg His Leu Pro Ala Gln Phe Ser Lys Pro Gly Glu Leu Thr Glu Lys
            105                 110                 115

gca gcc atg atc gtg caa tac gat cac acg gtg gtc agc ttc ttg gat 499
Ala Ala Met Ile Val Gln Tyr Asp His Thr Val Val Ser Phe Leu Asp
        120                 125                 130

gag cac ggc atc gag tat cag cgc acc gaa ctt gtt ccg cca att cct 547
Glu His Gly Ile Glu Tyr Gln Arg Thr Glu Leu Val Pro Pro Ile Pro
    135                 140                 145

ttc agg cat agc ggg cac aca cat tgatggatct tgacgctgat ttt 594
Phe Arg His Ser Gly His Thr His
150                 155


<210> 22
<211> 157
<212> PRT
<213> Corynebacterium glutamicum

<400> 22
Met Ile Ile Thr Ala Ile Asp Thr Asn Ile Tyr Asp Glu Pro Glu Phe
1               5                   10                  15

Val Glu Gly Arg Asp Val Ile Gly Val Arg Phe Glu Asp Leu Val Leu
            20                  25                  30

Asp Lys Arg Ile Gln Arg Val Ala Leu Pro Gly Gly Glu Glu Leu Gly
        35                  40                  45

Leu Arg Leu Asn His Gly His Pro Ile Leu Arg Glu Gly Asp Val Leu
    50                  55                  60

Lys Ala Asp Asp Lys Thr Val Phe Val Val Glu Ile Ile Pro Thr Asp
65                  70                  75                  80

Val Leu Val Ile Thr Pro Ser Asp Ile His Gln Met Gly Phe Val Ala
                85                  90                  95

His Ser Leu Gly Asn Arg His Leu Pro Ala Gln Phe Ser Lys Pro Gly
            100                 105                 110

Glu Leu Thr Glu Lys Ala Ala Met Ile Val Gln Tyr Asp His Thr Val
        115                 120                 125

Val Ser Phe Leu Asp Glu His Gly Ile Glu Tyr Gln Arg Thr Glu Leu
    130                 135                 140

Val Pro Pro Ile Pro Phe Arg His Ser Gly His Thr His
145                 150                 155


<210> 23
<211> 801
<212> DNA
<213> Corynebacterium glutamicum

<220>
<221> CDS
<222> (101)..(778)
<223> RXA02276

<400> 23
atcacacggt ggtcagcttc ttggatgagc acggcatcga gtatcagcgc accgaacttg 60

ttccgccaat tcctttcagg catagcgggc acacacattg atg gat ctt gac gct   115
                                            Met Asp Leu Asp Ala
                                            1               5

gat ttt ctg ctg ttg cat tta tcg gat tca gca ctt cca acg gga gcg   163
Asp Phe Leu Leu Leu His Leu Ser Asp Ser Ala Leu Pro Thr Gly Ala
                10                  15                  20

ttt gcg cac tca ttt gga ttt gaa act tat atg gat gca gag cga atc   211
Phe Ala His Ser Phe Gly Phe Glu Thr Tyr Met Asp Ala Glu Arg Ile
            25                  30                  35

acc aat gca gag gag ttc caa gac tgg ctg aaa gtc ctg ctt aag gtg   259
Thr Asn Ala Glu Glu Phe Gln Asp Trp Leu Lys Val Leu Leu Lys Val
        40                  45                  50

caa ttg acc agc tct gat gct ttg gca atg agg atg ttt tac gcc acc   307
Gln Leu Thr Ser Ser Asp Ala Leu Ala Met Arg Met Phe Tyr Ala Thr
    55                  60                  65

ccg acg gtg tct gag ctg aaa cgg ctg gat gag cgc ctt ttt gct gga   355
Pro Thr Val Ser Glu Leu Lys Arg Leu Asp Glu Arg Leu Phe Ala Gly
70                  75                  80                  85

act ccg gcg aga gaa att cgg gaa gct aat gct cga atg ggt acg cgc   403
Thr Pro Ala Arg Glu Ile Arg Glu Ala Asn Ala Arg Met Gly Thr Arg
                90                  95                  100

atg gca gag atc gtg gct gaa acc tac tcc gtg ccc ctg att gtt gag   451
Met Ala Glu Ile Val Ala Glu Thr Tyr Ser Val Pro Leu Ile Val Glu
            105                 110                 115

tat ctc gaa ttg att caa cat cga gag cta tca ggg cac ccg gct ttg   499
Tyr Leu Glu Leu Ile Gln His Arg Glu Leu Ser Gly His Pro Ala Leu
        120                 125                 130

gct ttg gct ctt gcc acc cac agc gcg ggg att gat gtg gat cga gca   547
Ala Leu Ala Leu Ala Thr His Ser Ala Gly Ile Asp Val Asp Arg Ala
    135                 140                 145

atc cac gct cac ctc acg gca acg gtg agt tcg ctg atc caa aat gcg   595
Ile His Ala His Leu Thr Ala Thr Val Ser Ser Leu Ile Gln Asn Ala
150                 155                 160                 165

gtt cgt ggc atc cca ctg ggg caa atg gca ggt cag cgg gtg atg ttc   643
Val Arg Gly Ile Pro Leu Gly Gln Met Ala Gly Gln Arg Val Met Phe
                170                 175                 180

gcc atg cgt gag cat atc ggt gcg gcc gtg aaa cgt agc gcg aac ttg   691
Ala Met Arg Glu His Ile Gly Ala Ala Val Lys Arg Ser Ala Asn Leu

185                 190                 195

gat gag att gat ttc tgt tcg ggt gat cca ggc ttg gat att tca caa     739
Asp Glu Ile Asp Phe Cys Ser Gly Asp Pro Gly Leu Asp Ile Ser Gln
        200                 205                 210

atg gtt cat gaa acc caa cgc gca cga cta ttt atg agt taagaaggag     788
Met Val His Glu Thr Gln Arg Ala Arg Leu Phe Met Ser
    215                 220                 225

aaaagaaaca tgg                                                     801


<210> 24
<211> 226
<212> PRT
<213> Corynebacterium glutamicum

<400> 24
Met Asp Leu Asp Ala Asp Phe Leu Leu Leu His Leu Ser Asp Ser Ala
1               5                   10                  15

Leu Pro Thr Gly Ala Phe Ala His Ser Phe Gly Phe Glu Thr Tyr Met
            20                  25                  30

Asp Ala Glu Arg Ile Thr Asn Ala Glu Glu Phe Gln Asp Trp Leu Lys
        35                  40                  45

Val Leu Leu Lys Val Gln Leu Thr Ser Ser Asp Ala Leu Ala Met Arg
    50                  55                  60

Met Phe Tyr Ala Thr Pro Thr Val Ser Glu Leu Lys Arg Leu Asp Glu
65                  70                  75                  80

Arg Leu Phe Ala Gly Thr Pro Ala Arg Glu Ile Arg Glu Ala Asn Ala
                85                  90                  95

Arg Met Gly Thr Arg Met Ala Glu Ile Val Ala Glu Thr Tyr Ser Val
            100                 105                 110

Pro Leu Ile Val Glu Tyr Leu Glu Leu Ile Gln His Arg Glu Leu Ser
        115                 120                 125

Gly His Pro Ala Leu Ala Leu Ala Leu Ala Thr His Ser Ala Gly Ile
    130                 135                 140

Asp Val Asp Arg Ala Ile His Ala His Leu Thr Ala Thr Val Ser Ser
145                 150                 155                 160

Leu Ile Gln Asn Ala Val Arg Gly Ile Pro Leu Gly Gln Met Ala Gly
                165                 170                 175

Gln Arg Val Met Phe Ala Met Arg Glu His Ile Gly Ala Ala Val Lys
            180                 185                 190

Arg Ser Ala Asn Leu Asp Glu Ile Asp Phe Cys Ser Gly Asp Pro Gly
        195                 200                 205

Leu Asp Ile Ser Gln Met Val His Glu Thr Gln Arg Ala Arg Leu Phe
    210                 215                 220

Met Ser
225


<210> 25
<211> 738
<212> DNA
<213> Corynebacterium glutamicum

<220>
<221> CDS
<222> (101)..(715)
<223> RXA02277

<400> 25
attgatttct gttcgggtga tccaggcttg gatatttcac aaatggttca tgaaacccaa 60

cgcgcacgac tatttatgag ttaagaagga gaaaagaaac atg ggt cca atc aga 115
Met Gly Pro Ile Arg
1               5

atc ggc gta ggc ggg ccg gtc ggc gcc gga aaa acg cag ctg gta gag 163
Ile Gly Val Gly Gly Pro Val Gly Ala Gly Lys Thr Gln Leu Val Glu
                10                  15                  20

cgg att acg cga gcg ctt atc gac gaa gtc agc atg gct gca atc act 211
Arg Ile Thr Arg Ala Leu Ile Asp Glu Val Ser Met Ala Ala Ile Thr
            25                  30                  35

aac gat atc tac acc att gaa gac gcc aag att ctt gcc gcc aat gga 259
Asn Asp Ile Tyr Thr Ile Glu Asp Ala Lys Ile Leu Ala Ala Asn Gly
        40                  45                  50

gtg ctg cca gaa gaa cgc att gtt ggc att gaa act gga gga tgc cca 307
Val Leu Pro Glu Glu Arg Ile Val Gly Ile Glu Thr Gly Gly Cys Pro
    55                  60                  65

cac act gcg att cgt gaa gac acc tcc atg aat gat gca gcg atc aaa 355
His Thr Ala Ile Arg Glu Asp Thr Ser Met Asn Asp Ala Ala Ile Lys
70                  75                  80                  85

gac ctt gtg gaa cgc ttc cca gat ctg gaa ctc atc ttt gtg gaa tct 403
Asp Leu Val Glu Arg Phe Pro Asp Leu Glu Leu Ile Phe Val Glu Ser
                90                  95                  100

ggt gga gat aat ctc tct gca acg ttc tcg cca gag ctg gtg gat ttt 451
Gly Gly Asp Asn Leu Ser Ala Thr Phe Ser Pro Glu Leu Val Asp Phe
            105                 110                 115

tcc atc tac atc atc gat gtt gcc caa ggt gag aag atc ccg agg aaa 499
Ser Ile Tyr Ile Ile Asp Val Ala Gln Gly Glu Lys Ile Pro Arg Lys
        120                 125                 130

gct ggc caa ggc atg att aag tcg gat ttg ttt atc att aat aaa act 547

Ala Gly Gln Gly Met Ile Lys Ser Asp Leu Phe Ile Ile Asn Lys Thr
    135                 140                 145

gac ctt gcc cca tat gtt ggt gcc aac cta gat gtc atg gtg gaa gat 595
Asp Leu Ala Pro Tyr Val Gly Ala Asn Leu Asp Val Met Val Glu Asp
150                 155                 160                 165

gcc aaa gca ttc cgc aag aac aaa cca ttc tgc ctg act aat ctg cgc 643
Ala Lys Ala Phe Arg Lys Asn Lys Pro Phe Cys Leu Thr Asn Leu Arg
                170                 175                 180

acc gat gat ggt ttg gat aag gtc ttg gaa tgg atc cgc cat gag gtg 691
Thr Asp Asp Gly Leu Asp Lys Val Leu Glu Trp Ile Arg His Glu Val
            185                 190                 195

atg atg cag gac ttg cag gaa gcc taaatgacac aaacccaacc agt 738
Met Met Gln Asp Leu Gln Glu Ala
        200                 205


<210> 26
<211> 205
<212> PRT
<213> Corynebacterium glutamicum

<400> 26
Met Gly Pro Ile Arg Ile Gly Val Gly Gly Pro Val Gly Ala Gly Lys
1               5                   10                  15

Thr Gln Leu Val Glu Arg Ile Thr Arg Ala Leu Ile Asp Glu Val Ser
            20                  25                  30

Met Ala Ala Ile Thr Asn Asp Ile Tyr Thr Ile Glu Asp Ala Lys Ile
        35                  40                  45

Leu Ala Ala Asn Gly Val Leu Pro Glu Glu Arg Ile Val Gly Ile Glu
    50                  55                  60

Thr Gly Gly Cys Pro His Thr Ala Ile Arg Glu Asp Thr Ser Met Asn
65                  70                  75                  80

Asp Ala Ala Ile Lys Asp Leu Val Glu Arg Phe Pro Asp Leu Glu Leu
                85                  90                  95

Ile Phe Val Glu Ser Gly Gly Asp Asn Leu Ser Ala Thr Phe Ser Pro
            100                 105                 110

Glu Leu Val Asp Phe Ser Ile Tyr Ile Ile Asp Val Ala Gln Gly Glu
        115                 120                 125

Lys Ile Pro Arg Lys Ala Gly Gln Gly Met Ile Lys Ser Asp Leu Phe
    130                 135                 140

Ile Ile Asn Lys Thr Asp Leu Ala Pro Tyr Val Gly Ala Asn Leu Asp
145                 150                 155                 160

Val Met Val Glu Asp Ala Lys Ala Phe Arg Lys Asn Lys Pro Phe Cys
                165                 170                 175

Leu Thr Asn Leu Arg Thr Asp Asp Gly Leu Asp Lys Val Leu Glu Trp
            180                 185                 190

Ile Arg His Glu Val Met Met Gln Asp Leu Gln Glu Ala
        195                 200                 205


<210> 27
<211> 1119
<212> DNA
<213> Corynebacterium glutamicum

<220>
<221> CDS
<222> (101)..(1096)
<223> RXA02603

<400> 27
gcatggtgct tggcgtgcat tacccaaccg atgtgctagc cggcgcgttg ttgggagcag 60

cgaccgcaga ggccgtccat aagatcgaaa gggctacgaa gtg agc gaa cac gcc    115
                                           Val Ser Glu His Ala
                                           1               5

gct gaa cat cac cgc gat acc caa aat ttc tta acc tcc gaa ccg cac    163
Ala Glu His His Arg Asp Thr Gln Asn Phe Leu Thr Ser Glu Pro His
                10                  15                  20

acc acg gca atc gaa gac aac aag aag cgc caa ccg ccg aaa aac ctt    211
Thr Thr Ala Ile Glu Asp Asn Lys Lys Arg Gln Pro Pro Lys Asn Leu
            25                  30                  35

gct gac ggc atg atc aag gcg ctg cgc ccc aag cag tgg gtc aag aac    259
Ala Asp Gly Met Ile Lys Ala Leu Arg Pro Lys Gln Trp Val Lys Asn
        40                  45                  50

gtt ctt gtg cta gca gca cca ctt gct gct ggt gca gat gcg atc ttc    307
Val Leu Val Leu Ala Ala Pro Leu Ala Ala Gly Ala Asp Ala Ile Phe
    55                  60                  65

aac cag cgc acg atc atc gac gtt gct atc gca ttc gta gtg ttc tgc    355
Asn Gln Arg Thr Ile Ile Asp Val Ala Ile Ala Phe Val Val Phe Cys
70                  75                  80                  85

ttc ggt gca tca gcc att tac ttg gtt aat gat gcc cgt gac gtg gaa    403
Phe Gly Ala Ser Ala Ile Tyr Leu Val Asn Asp Ala Arg Asp Val Glu
                90                  95                  100

gct gac cgc gag cac cca acc aag cgt ttc cgc ccc atc gct gca gga    451
Ala Asp Arg Glu His Pro Thr Lys Arg Phe Arg Pro Ile Ala Ala Gly
            105                 110                 115

gtc ctg cca gta gga atg gca tac ggc atg gcc gtg gcg ctc att gca    499
Val Leu Pro Val Gly Met Ala Tyr Gly Met Ala Val Ala Leu Ile Ala
        120                 125                 130

cta tcc atc gga ctg tct ttc ctc gcc acc gac ggc gtg gca ctt gcc     547
Leu Ser Ile Gly Leu Ser Phe Leu Ala Thr Asp Gly Val Ala Leu Ala
    135                 140                 145

tgc gtg att ggc gtg tac att gcg ctg cag ctg gga tac tgc ttc ggt     595
Cys Val Ile Gly Val Tyr Ile Ala Leu Gln Leu Gly Tyr Cys Phe Gly
150                 155                 160                 165

tgg aag cac atg cca gtg atc gat att gcg ctt gtc tcc tcc gga ttc     643
Trp Lys His Met Pro Val Ile Asp Ile Ala Leu Val Ser Ser Gly Phe
                170                 175                 180

atg ctc cgc gca atg gca ggt ggt gtc gca gca ggc atc gag cta tcc     691
Met Leu Arg Ala Met Ala Gly Gly Val Ala Ala Gly Ile Glu Leu Ser
            185                 190                 195

cag tgg ttc ctg cta gtc gct gcg ttt ggt tcc ctg ttc atg gca tct     739
Gln Trp Phe Leu Leu Val Ala Ala Phe Gly Ser Leu Phe Met Ala Ser
        200                 205                 210

gga aag cgc tac gca gaa atc ctt ctg cac gag cgc acc ggc gct aag     787
Gly Lys Arg Tyr Ala Glu Ile Leu Leu His Glu Arg Thr Gly Ala Lys
    215                 220                 225

atc cgc aag tcc ctg gaa agc tac acc ccc acc tac ctg cgc ttc gtt     835
Ile Arg Lys Ser Leu Glu Ser Tyr Thr Pro Thr Tyr Leu Arg Phe Val
230                 235                 240                 245

tgg acc atg gca gca aca gca gtg gtc atg tcc tac gca ctg tgg ggc     883
Trp Thr Met Ala Ala Thr Ala Val Val Met Ser Tyr Ala Leu Trp Gly
                250                 255                 260

ttc gac ctt tcc caa cac tcc acc gac gca ggt ccg tgg tac caa atc     931
Phe Asp Leu Ser Gln His Ser Thr Asp Ala Gly Pro Trp Tyr Gln Ile
            265                 270                 275

tcc atg gtt cca ttc acc atc gcc atc ctg cgc tac gca gcc ggc gta     979
Ser Met Val Pro Phe Thr Ile Ala Ile Leu Arg Tyr Ala Ala Gly Val
        280                 285                 290

gac acc ggc gac ggc ggt gcc cct gac gaa gtg gca ctc agc gac aaa     1027
Asp Thr Gly Asp Gly Gly Ala Pro Asp Glu Val Ala Leu Ser Asp Lys
    295                 300                 305

gtt ctg cag gta cta gcc cta gca tgg gtt ttc tgc atc gtg atg gct     1075
Val Leu Gln Val Leu Ala Leu Ala Trp Val Phe Cys Ile Val Met Ala
310                 315                 320                 325

gtg tac atc atg ccg atg ttt tgaatattta ccaatgaaca tgc               1119
Val Tyr Ile Met Pro Met Phe
                330


<210> 28
<211> 332
<212> PRT
<213> Corynebacterium glutamicum

<400> 28
Val Ser Glu His Ala Ala Glu His His Arg Asp Thr Gln Asn Phe Leu
1               5                   10                  15

Thr Ser Glu Pro His Thr Thr Ala Ile Glu Asp Asn Lys Lys Arg Gln
            20                  25                  30

Pro Pro Lys Asn Leu Ala Asp Gly Met Ile Lys Ala Leu Arg Pro Lys
        35                  40                  45

Gln Trp Val Lys Asn Val Leu Val Leu Ala Ala Pro Leu Ala Ala Gly
    50                  55                  60

Ala Asp Ala Ile Phe Asn Gln Arg Thr Ile Ile Asp Val Ala Ile Ala
65                  70                  75                  80

Phe Val Val Phe Cys Phe Gly Ala Ser Ala Ile Tyr Leu Val Asn Asp
                85                  90                  95

Ala Arg Asp Val Glu Ala Asp Arg Glu His Pro Thr Lys Arg Phe Arg
            100                 105                 110

Pro Ile Ala Ala Gly Val Leu Pro Val Gly Met Ala Tyr Gly Met Ala
        115                 120                 125

Val Ala Leu Ile Ala Leu Ser Ile Gly Leu Ser Phe Leu Ala Thr Asp
    130                 135                 140

Gly Val Ala Leu Ala Cys Val Ile Gly Val Tyr Ile Ala Leu Gln Leu
145                 150                 155                 160

Gly Tyr Cys Phe Gly Trp Lys His Met Pro Val Ile Asp Ile Ala Leu
                165                 170                 175

Val Ser Ser Gly Phe Met Leu Arg Ala Met Ala Gly Gly Val Ala Ala
            180                 185                 190

Gly Ile Glu Leu Ser Gln Trp Phe Leu Leu Val Ala Ala Phe Gly Ser
        195                 200                 205

Leu Phe Met Ala Ser Gly Lys Arg Tyr Ala Glu Ile Leu Leu His Glu
    210                 215                 220

Arg Thr Gly Ala Lys Ile Arg Lys Ser Leu Glu Ser Tyr Thr Pro Thr
225                 230                 235                 240

Tyr Leu Arg Phe Val Trp Thr Met Ala Ala Thr Ala Val Val Met Ser
                245                 250                 255

Tyr Ala Leu Trp Gly Phe Asp Leu Ser Gln His Ser Thr Asp Ala Gly
            260                 265                 270

Pro Trp Tyr Gln Ile Ser Met Val Pro Phe Thr Ile Ala Ile Leu Arg
        275                 280                 285

Tyr Ala Ala Gly Val Asp Thr Gly Asp Gly Gly Ala Pro Asp Glu Val
    290                 295                 300

Ala Leu Ser Asp Lys Val Leu Gln Val Leu Ala Leu Ala Trp Val Phe
305                 310                 315                 320

Cys Ile Val Met Ala Val Tyr Ile Met Pro Met Phe
                325                 330


<210> 29
<211> 2004
<212> DNA
<213> Corynebacterium glutamicum


<220>
<221> CDS
<222> (101)..(1981)
<223> RXA01385

<400> 29
atcacacttt cattcgcagt gtgatctgaa ctacatttct ggttactgta cggaacacgc 60

tccgtgaatg agataggaaa tcccctcgaa aggaccagac atg cag ttt cat tat    115
                                          Met Gln Phe His Tyr
                                          1               5

gaa gga tac gca acc ggt gac cca atg gag atg cgc gcg gaa ggt agc    163
Glu Gly Tyr Ala Thr Gly Asp Pro Met Glu Met Arg Ala Glu Gly Ser
                10                  15                  20

gga atc aac cgc ccg gac gat ctc ccc gag gtc atg gat gtt ctc atc    211
Gly Ile Asn Arg Pro Asp Asp Leu Pro Glu Val Met Asp Val Leu Ile
            25                  30                  35

gtt ggt gca ggt ccg gct ggc acc atc gca gcg gct cag ctt tcc cga    259
Val Gly Ala Gly Pro Ala Gly Thr Ile Ala Ala Ala Gln Leu Ser Arg
        40                  45                  50

ttc ccc aat gtg acc acc cgc ctc gta gag aga agc gac cgt cgc ctc    307

Phe Pro Asn Val Thr Thr Arg Leu Val Glu Arg Ser Asp Arg Arg Leu
55                  60                  65

gaa cta gcc aat gca gat ggc gtg cac tcc cga acc att gaa act ttc     355
Glu Leu Ala Asn Ala Asp Gly Val His Ser Arg Thr Ile Glu Thr Phe
70                  75                  80                  85

cag gca ttt ggt ttc gcc cac gag atc ctc gcc gaa gct cat gaa atc     403
Gln Ala Phe Gly Phe Ala His Glu Ile Leu Ala Glu Ala His Glu Ile
                90                  95                  100

acc gac atg gcg ttc tgg aag ccg gac ccg caa aac cct cgt gag atc     451
Thr Asp Met Ala Phe Trp Lys Pro Asp Pro Gln Asn Pro Arg Glu Ile
            105                 110                 115

att cgc gac aac agc acc cgc gag ctg cca cag cac atc agt gaa ttt     499
Ile Arg Asp Asn Ser Thr Arg Glu Leu Pro Gln His Ile Ser Glu Phe
        120                 125                 130

ccg atg gcg ttg ctc acc cag acc cgc atc atc gac cac ttc aac cgg     547
Pro Met Ala Leu Leu Thr Gln Thr Arg Ile Ile Asp His Phe Asn Arg
    135                 140                 145

ttc atg aag aac tcc cca acc agg atg aag cct gac tat gga tac gag     595
Phe Met Lys Asn Ser Pro Thr Arg Met Lys Pro Asp Tyr Gly Tyr Glu
150                 155                 160                 165

ttc gtg gac ttt gaa gta gaa gaa gac gca gaa tat ccg gta att gtc     643
Phe Val Asp Phe Glu Val Glu Glu Asp Ala Glu Tyr Pro Val Ile Val
                170                 175                 180

acc ctc cgc cgc acc agt ggc gag caa act ggc gaa ttg gtc acc gtc     691
Thr Leu Arg Arg Thr Ser Gly Glu Gln Thr Gly Glu Leu Val Thr Val
            185                 190                 195

cga acc aag tac ctg gtc ggt gcc gat ggt gca cga agc caa gtg cgc     739
Arg Thr Lys Tyr Leu Val Gly Ala Asp Gly Ala Arg Ser Gln Val Arg
        200                 205                 210

aaa tca ctg gga tac cga ctc caa ggt aag cag gct aac cac gct tgg     787
Lys Ser Leu Gly Tyr Arg Leu Gln Gly Lys Gln Ala Asn His Ala Trp
    215                 220                 225

ggt gtg atg gat att cac gcg aac acc gag ttc ccc gac gtg cgc aag     835
Gly Val Met Asp Ile His Ala Asn Thr Glu Phe Pro Asp Val Arg Lys
230                 235                 240                 245

aag tgc acc atc aaa tct gat tcg ggt cgc acc atc ttg ctc atc cca     883
Lys Cys Thr Ile Lys Ser Asp Ser Gly Arg Thr Ile Leu Leu Ile Pro
                250                 255                 260

cgt gag ggt ggc ttc ctc ttc cgt ctc tac gtt gac ctg ggc gaa gta     931
Arg Glu Gly Gly Phe Leu Phe Arg Leu Tyr Val Asp Leu Gly Glu Val
            265                 270                 275

cct gat gat ggc agc aag gct gtt cgt gat acc cca ctc cag gat gtc     979
Pro Asp Asp Gly Ser Lys Ala Val Arg Asp Thr Pro Leu Gln Asp Val
        280                 285                 290

atc gac acc gcg aac cag atc atg gct cca ttc acc ctc gac gtg aaa     1027
Ile Asp Thr Ala Asn Gln Ile Met Ala Pro Phe Thr Leu Asp Val Lys
    295                 300                 305

aac gtt gtg tgg aac tcc atc tac gag gta ggc cac cgc gtc gca gac     1075
Asn Val Val Trp Asn Ser Ile Tyr Glu Val Gly His Arg Val Ala Asp
310                 315                 320                 325

cat ttc gat gac cgt gtt tca gaa aaa acc tcg agc gaa cac cca cgc     1123
His Phe Asp Asp Arg Val Ser Glu Lys Thr Ser Ser Glu His Pro Arg
                330                 335                 340

att ttc att gct ggc gac gcc tgc cac acc cac agc gct aag gct ggc     1171
Ile Phe Ile Ala Gly Asp Ala Cys His Thr His Ser Ala Lys Ala Gly
            345                 350                 355

cag ggc atg aac gtg tcc atg cag gac gga ttc aac ctt ggc tgg aag     1219
Gln Gly Met Asn Val Ser Met Gln Asp Gly Phe Asn Leu Gly Trp Lys

360                 365                 370

ctt gga cat gtg gcc agc gga aat agc cca cgc gaa cta ctt cag acc      1267
Leu Gly His Val Ala Ser Gly Asn Ser Pro Arg Glu Leu Leu Gln Thr
    375                 380                 385

tac gct gaa gag cgc gaa gac att gcc tac aag ctc atc gag tac gac      1315
Tyr Ala Glu Glu Arg Glu Asp Ile Ala Tyr Lys Leu Ile Glu Tyr Asp
390                 395                 400                 405

aag aac tgg tca aca ctc atg gca aag cca agc agc gaa atg ggc agt      1363
Lys Asn Trp Ser Thr Leu Met Ala Lys Pro Ser Ser Glu Met Gly Ser
                410                 415                 420

gcc caa gac ctt gag gat ttc tac cgc gcg aac tct gag ttc aat gcc      1411
Ala Gln Asp Leu Glu Asp Phe Tyr Arg Ala Asn Ser Glu Phe Asn Ala
            425                 430                 435

ggc tac atg acc cac tat cct cct tct tcc atc aca atg gat ggc agc      1459
Gly Tyr Met Thr His Tyr Pro Pro Ser Ser Ile Thr Met Asp Gly Ser
        440                 445                 450

aac caa gat ctg gca aag ggc tac cca att ggc cga cgc ttc aag tca      1507
Asn Gln Asp Leu Ala Lys Gly Tyr Pro Ile Gly Arg Arg Phe Lys Ser
    455                 460                 465

gcg atg gtt ggt cga gtc tgc gac ttc acc gaa aca cac ctc ggt cac      1555
Ala Met Val Gly Arg Val Cys Asp Phe Thr Glu Thr His Leu Gly His
470                 475                 480                 485

caa gca aca gcc gac gga cgc atg cgc gca tac gtc ttc gca gga tcc      1603
Gln Ala Thr Ala Asp Gly Arg Met Arg Ala Tyr Val Phe Ala Gly Ser
                490                 495                 500

gat gca ctt aac ggc gag ggt tct gag cta gac cgc tgg gca gaa tgg      1651
Asp Ala Leu Asn Gly Glu Gly Ser Glu Leu Asp Arg Trp Ala Glu Trp
            505                 510                 515

gca gag gcg aac ctt gac ccc acg ctt gtc gac gcc aag gtg att tac      1699
Ala Glu Ala Asn Leu Asp Pro Thr Leu Val Asp Ala Lys Val Ile Tyr
        520                 525                 530

caa agc cct tat acc gag ctc gac acc cgc cag gtt cca tcc gtg ttc      1747
Gln Ser Pro Tyr Thr Glu Leu Asp Thr Arg Gln Val Pro Ser Val Phe
    535                 540                 545

aaa cct gca gtc ggg atc ttc gaa ctg acc aat gtg gaa aac tcc ttc      1795
Lys Pro Ala Val Gly Ile Phe Glu Leu Thr Asn Val Glu Asn Ser Phe
550                 555                 560                 565

ggt atc acc acg gac tcc gac atc ttt gat agt cgc gag atc tcc cgc      1843
Gly Ile Thr Thr Asp Ser Asp Ile Phe Asp Ser Arg Glu Ile Ser Arg
                570                 575                 580

gat ggt gtc gtg gtg gta gtc cga cca gac caa tac gtt tcc gga atc      1891
Asp Gly Val Val Val Val Val Arg Pro Asp Gln Tyr Val Ser Gly Ile
            585                 590                 595

ttc cca ctc act gat acc caa ggg ctt ggc gaa ttc ctc acc gga tac      1939
Phe Pro Leu Thr Asp Thr Gln Gly Leu Gly Glu Phe Leu Thr Gly Tyr
        600                 605                 610

ttc ccc aaa atg aaa ggc gca cat cag cta atc aac gcg aac              1981
Phe Pro Lys Met Lys Gly Ala His Gln Leu Ile Asn Ala Asn
    615                 620                 625

taaggcacag ctgttaaaac agt                                            2004


<210> 30
<211> 627
<212> PRT
<213> Corynebacterium glutamicum

<400> 30
   Met Gln Phe His Tyr Glu Gly Tyr Ala Thr Gly Asp Pro Met Glu Met
   1               5                   10                  15
   Arg Ala Glu Gly Ser Gly Ile Asn Arg Pro Asp Asp Leu Pro Glu Val
               20                  25                  30
   Met Asp Val Leu Ile Val Gly Ala Gly Pro Ala Gly Thr Ile Ala Ala
           35                  40                  45
   Ala Gln Leu Ser Arg Phe Pro Asn Val Thr Thr Arg Leu Val Glu Arg
       50                  55                  60
   Ser Asp Arg Arg Leu Glu Leu Ala Asn Ala Asp Gly Val His Ser Arg
   65                  70                  75                  80
   Thr Ile Glu Thr Phe Gln Ala Phe Gly Phe Ala His Glu Ile Leu Ala
                   85                  90                  95
   Glu Ala His Glu Ile Thr Asp Met Ala Phe Trp Lys Pro Asp Pro Gln
               100                 105                 110
   Asn Pro Arg Glu Ile Ile Arg Asp Asn Ser Thr Arg Glu Leu Pro Gln
           115                 120                 125
   His Ile Ser Glu Phe Pro Met Ala Leu Leu Thr Gln Thr Arg Ile Ile
       130                 135                 140
   Asp His Phe Asn Arg Phe Met Lys Asn Ser Pro Thr Arg Met Lys Pro
   145                 150                 155                 160
   Asp Tyr Gly Tyr Glu Phe Val Asp Phe Glu Val Glu Glu Asp Ala Glu
                   165                 170                 175
   Tyr Pro Val Ile Val Thr Leu Arg Arg Thr Ser Gly Glu Gln Thr Gly
               180                 185                 190
   Glu Leu Val Thr Val Arg Thr Lys Tyr Leu Val Gly Ala Asp Gly Ala
           195                 200                 205
   Arg Ser Gln Val Arg Lys Ser Leu Gly Tyr Arg Leu Gln Gly Lys Gln
       210                 215                 220
   Ala Asn His Ala Trp Gly Val Met Asp Ile His Ala Asn Thr Glu Phe
   225                 230                 235                 240
   Pro Asp Val Arg Lys Lys Cys Thr Ile Lys Ser Asp Ser Gly Arg Thr
                   245                 250                 255
   Ile Leu Leu Ile Pro Arg Glu Gly Gly Phe Leu Phe Arg Leu Tyr Val
               260                 265                 270
   Asp Leu Gly Glu Val Pro Asp Asp Gly Ser Lys Ala Val Arg Asp Thr
           275                 280                 285
   Pro Leu Gln Asp Val Ile Asp Thr Ala Asn Gln Ile Met Ala Pro Phe
       290                 295                 300
   Thr Leu Asp Val Lys Asn Val Val Trp Asn Ser Ile Tyr Glu Val Gly
   305                 310                 315                 320
   His Arg Val Ala Asp His Phe Asp Asp Arg Val Ser Glu Lys Thr Ser
                   325                 330                 335
   Ser Glu His Pro Arg Ile Phe Ile Ala Gly Asp Ala Cys His Thr His
               340                 345                 350
   Ser Ala Lys Ala Gly Gln Gly Met Asn Val Ser Met Gln Asp Gly Phe
           355                 360                 365
   Asn Leu Gly Trp Lys Leu Gly His Val Ala Ser Gly Asn Ser Pro Arg
       370                 375                 380
   Glu Leu Leu Gln Thr Tyr Ala Glu Glu Arg Glu Asp Ile Ala Tyr Lys
   385                 390                 395                 400
   Leu Ile Glu Tyr Asp Lys Asn Trp Ser Thr Leu Met Ala Lys Pro Ser
                   405                 410                 415
   Ser Glu Met Gly Ser Ala Gln Asp Leu Glu Asp Phe Tyr Arg Ala Asn
               420                 425                 430
   Ser Glu Phe Asn Ala Gly Tyr Met Thr His Tyr Pro Pro Ser Ser Ile
           435                 440                 445
   Thr Met Asp Gly Ser Asn Gln Asp Leu Ala Lys Gly Tyr Pro Ile Gly
       450                 455                 460
   Arg Arg Phe Lys Ser Ala Met Val Gly Arg Val Cys Asp Phe Thr Glu
   465                 470                 475                 480
   Thr His Leu Gly His Gln Ala Thr Ala Asp Gly Arg Met Arg Ala Tyr
                   485                 490                 495
   Val Phe Ala Gly Ser Asp Ala Leu Asn Gly Glu Gly Ser Glu Leu Asp
               500                 505                 510
   Arg Trp Ala Glu Trp Ala Glu Ala Asn Leu Asp Pro Thr Leu Val Asp
           515                 520                 525
   Ala Lys Val Ile Tyr Gln Ser Pro Tyr Thr Glu Leu Asp Thr Arg Gln
       530                 535                 540
   Val Pro Ser Val Phe Lys Pro Ala Val Gly Ile Phe Glu Leu Thr Asn
   545                 550                 555                 560
   Val Glu Asn Ser Phe Gly Ile Thr Thr Asp Ser Asp Ile Phe Asp Ser
                   565                 570                 575
   Arg Glu Ile Ser Arg Asp Gly Val Val Val Val Val Arg Pro Asp Gln
               580                 585                 590
   Tyr Val Ser Gly Ile Phe Pro Leu Thr Asp Thr Gln Gly Leu Gly Glu
           595                 600                 605
   Phe Leu Thr Gly Tyr Phe Pro Lys Met Lys Gly Ala His Gln Leu Ile
       610                 615                 620
   Asn Ala Asn
   625


<210> 31
<211> 915
<212> DNA
<213> Corynebacterium glutamicum

<220>
<221> CDS
<222> (101)..(892)
<223> RXN00675

<400> 31
gtggaagtac tcacctcaag gtggccggaa ccgcatgcat tgtgcggtgc cggccacctt 60

tggttttaac agtttaattt gaagaaagag acgtggaagc atg ggt ttc cgt tcc    115
                                           Met Gly Phe Arg Ser
                                           1               5

aag aag aag gtt att gcg gca aag acc gcc gct gag ctg gac gcg atg    163
Lys Lys Lys Val Ile Ala Ala Lys Thr Ala Ala Glu Leu Asp Ala Met
                10                  15                  20

cag gcg gcg ggt gag atc gtc ggc aag gct ttg cag gct gtg cgc gct    211
Gln Ala Ala Gly Glu Ile Val Gly Lys Ala Leu Gln Ala Val Arg Ala
            25                  30                  35

gag gct aaa gct ggc atg agc acg tgg gat ctg gat cag atc gcg gag    259
Glu Ala Lys Ala Gly Met Ser Thr Trp Asp Leu Asp Gln Ile Ala Glu
        40                  45                  50

cag gtt atc cgc gat gct ggc gcc gtt cct aca ttc ctg ggt tac cag    307
Gln Val Ile Arg Asp Ala Gly Ala Val Pro Thr Phe Leu Gly Tyr Gln
    55                  60                  65

ggt ttt ccg gca tca gtg tgc gct tcg gtc aat gag gtg att gtt cac    355

Gly Phe Pro Ala Ser Val Cys Ala Ser Val Asn Glu Val Ile Val His
70                  75                  80                  85

ggc att cca tcc aag gag acc atc ttg gag gaa ggc gat ctg gtg tcc     403
Gly Ile Pro Ser Lys Glu Thr Ile Leu Glu Glu Gly Asp Leu Val Ser
                90                  95                  100

atc gac tgc ggc gca acc ttt gat ggt tgg gtc ggc gat tcc gcg tgg     451
Ile Asp Cys Gly Ala Thr Phe Asp Gly Trp Val Gly Asp Ser Ala Trp
            105                 110                 115

agc ttc ggc atc ggc gag ctg gac gag gac gtc cag ggt ctc aac ttg     499
Ser Phe Gly Ile Gly Glu Leu Asp Glu Asp Val Gln Gly Leu Asn Leu
        120                 125                 130

gct acc gag tgg gtc ctc atg gaa ggc atg aag gcc atg gtt cca ggc     547
Ala Thr Glu Trp Val Leu Met Glu Gly Met Lys Ala Met Val Pro Gly
    135                 140                 145

aac cgt ttg acc gat gtc tcc cac gct ctc gag gtc gca acc cgc aag     595
Asn Arg Leu Thr Asp Val Ser His Ala Leu Glu Val Ala Thr Arg Lys
150                 155                 160                 165

gct gag tcc aag ttc ggc gtc gcg ctc ggc atc gtc gat ggc tac ggc     643
Ala Glu Ser Lys Phe Gly Val Ala Leu Gly Ile Val Asp Gly Tyr Gly
                170                 175                 180

gga cac ggc att ggc cgc cac atg cac gag gag cca tac ttg gct aat     691
Gly His Gly Ile Gly Arg His Met His Glu Glu Pro Tyr Leu Ala Asn
            185                 190                 195

gag ggc aag gcc ggc aag ggc cct gtg att cag gag ggc tcc gtg ctc     739
Glu Gly Lys Ala Gly Lys Gly Pro Val Ile Gln Glu Gly Ser Val Leu
        200                 205                 210

gcc att gag cct atg ctc acc ctc ggc acc gaa gat tcc gca gtg ctg     787
Ala Ile Glu Pro Met Leu Thr Leu Gly Thr Glu Asp Ser Ala Val Leu
    215                 220                 225

gaa gat gat tgg act gtc gtg act ctc gac ggt tca tgg gca tca cac     835
Glu Asp Asp Trp Thr Val Val Thr Leu Asp Gly Ser Trp Ala Ser His
230                 235                 240                 245

tgg gag cac acc gtt gca gcc acc aag ggc ggc ccg cgc atc ctc acg     883
Trp Glu His Thr Val Ala Ala Thr Lys Gly Gly Pro Arg Ile Leu Thr
                250                 255                 260

ccg cgt tat taaaatgatg cttttcgacg cat                               915
Pro Arg Tyr

<210> 32
<211> 264
<212> PRT
<213> Corynebacterium glutamicum

<400> 32
Met Gly Phe Arg Ser Lys Lys Lys Val Ile Ala Ala Lys Thr Ala Ala
1               5                   10                  15

Glu Leu Asp Ala Met Gln Ala Ala Gly Glu Ile Val Gly Lys Ala Leu
            20                  25                  30

Gln Ala Val Arg Ala Glu Ala Lys Ala Gly Met Ser Thr Trp Asp Leu

        35                  40                  45

Asp Gln Ile Ala Glu Gln Val Ile Arg Asp Ala Gly Ala Val Pro Thr
    50                  55                  60

Phe Leu Gly Tyr Gln Gly Phe Pro Ala Ser Val Cys Ala Ser Val Asn
65                  70                  75                  80

Glu Val Ile Val His Gly Ile Pro Ser Lys Glu Thr Ile Leu Glu Glu
                85                  90                  95

Gly Asp Leu Val Ser Ile Asp Cys Gly Ala Thr Phe Asp Gly Trp Val
            100                 105                 110

Gly Asp Ser Ala Trp Ser Phe Gly Ile Gly Glu Leu Asp Glu Asp Val
        115                 120                 125

Gln Gly Leu Asn Leu Ala Thr Glu Trp Val Leu Met Glu Gly Met Lys
    130                 135                 140

Ala Met Val Pro Gly Asn Arg Leu Thr Asp Val Ser His Ala Leu Glu
145                 150                 155                 160

Val Ala Thr Arg Lys Ala Glu Ser Lys Phe Gly Val Ala Leu Gly Ile
                165                 170                 175

Val Asp Gly Tyr Gly Gly His Gly Ile Gly Arg His Met His Glu Glu
            180                 185                 190

Pro Tyr Leu Ala Asn Glu Gly Lys Ala Gly Lys Gly Pro Val Ile Gln
        195                 200                 205

Glu Gly Ser Val Leu Ala Ile Glu Pro Met Leu Thr Leu Gly Thr Glu
    210                 215                 220

Asp Ser Ala Val Leu Glu Asp Asp Trp Thr Val Val Thr Leu Asp Gly
225                 230                 235                 240

Ser Trp Ala Ser His Trp Glu His Thr Val Ala Ala Thr Lys Gly Gly
                245                 250                 255

Pro Arg Ile Leu Thr Pro Arg Tyr
            260


<210> 33
<211> 506
<212> DNA
<213> Corynebacterium glutamicum

<220>
<221> CDS
<222> (1)..(483)
<223> FRXA00675

<400> 33
tgc ggc gca acc ttt gat ggt tgg gtc ggc gat tcc gcg tgg agc ttc      48
Cys Gly Ala Thr Phe Asp Gly Trp Val Gly Asp Ser Ala Trp Ser Phe
  1               5                  10                  15

ggc atc ggc gag ctg gac gag gac gtc cag ggt ctc aac ttg gct acc      96
Gly Ile Gly Glu Leu Asp Glu Asp Val Gln Gly Leu Asn Leu Ala Thr
             20                  25                  30

gag tgg gtc ctc atg gaa ggc atg aag gcc atg gtt cca ggc aac cgt     144
Glu Trp Val Leu Met Glu Gly Met Lys Ala Met Val Pro Gly Asn Arg
         35                  40                  45

ttg acc gat gtc tcc cac gct ctc gag gtc gca acc cgc aag gct gag     192
Leu Thr Asp Val Ser His Ala Leu Glu Val Ala Thr Arg Lys Ala Glu
     50                  55                  60

tcc aag ttc ggc gtc gcg ctc ggc atc gtc gat ggc tac ggc gga cac     240
Ser Lys Phe Gly Val Ala Leu Gly Ile Val Asp Gly Tyr Gly Gly His
 65                  70                  75                  80

ggc att ggc cgc cac atg cac gag gag cca tac ttg gct aat gag ggc     288
Gly Ile Gly Arg His Met His Glu Glu Pro Tyr Leu Ala Asn Glu Gly
                 85                  90                  95

aag gcc ggc aag ggc cct gtg att cag gag ggc tcc gtg ctc gcc att     336
Lys Ala Gly Lys Gly Pro Val Ile Gln Glu Gly Ser Val Leu Ala Ile
            100                 105                 110

gag cct atg ctc acc ctc ggc acc gaa gat tcc gca gtg ctg gaa gat     384
Glu Pro Met Leu Thr Leu Gly Thr Glu Asp Ser Ala Val Leu Glu Asp
        115                 120                 125

gat tgg act gtc gtg act ctc gac ggt tca tgg gca tca cac tgg gag     432
Asp Trp Thr Val Val Thr Leu Asp Gly Ser Trp Ala Ser His Trp Glu
    130                 135                 140

cac acc gtt gca gcc acc aag ggc ggc ccg cgc atc ctc acg ccg cgt     480
His Thr Val Ala Ala Thr Lys Gly Gly Pro Arg Ile Leu Thr Pro Arg
145                 150                 155                 160

tat taaaatgatg cttttcgacg cat                                       506
Tyr

<210> 34
<211> 161
<212> PRT
<213> Corynebacterium glutamicum

<400> 34
Cys Gly Ala Thr Phe Asp Gly Trp Val Gly Asp Ser Ala Trp Ser Phe
  1               5                  10                  15

Gly Ile Gly Glu Leu Asp Glu Asp Val Gln Gly Leu Asn Leu Ala Thr
             20                  25                  30

Glu Trp Val Leu Met Glu Gly Met Lys Ala Met Val Pro Gly Asn Arg
         35                  40                  45

Leu Thr Asp Val Ser His Ala Leu Glu Val Ala Thr Arg Lys Ala Glu
     50                  55                  60

Ser Lys Phe Gly Val Ala Leu Gly Ile Val Asp Gly Tyr Gly Gly His
 65                  70                  75                  80

Gly Ile Gly Arg His Met His Glu Glu Pro Tyr Leu Ala Asn Glu Gly
                 85                  90                  95

Lys Ala Gly Lys Gly Pro Val Ile Gln Glu Gly Ser Val Leu Ala Ile
            100                 105                 110

Glu Pro Met Leu Thr Leu Gly Thr Glu Asp Ser Ala Val Leu Glu Asp
        115                 120                 125

Asp Trp Thr Val Val Thr Leu Asp Gly Ser Trp Ala Ser His Trp Glu
    130                 135                 140

His Thr Val Ala Ala Thr Lys Gly Gly Pro Arg Ile Leu Thr Pro Arg
145                 150                 155                 160

Tyr

<210> 35
<211> 996
<212> DNA
<213> Corynebacterium glutamicum

<220>
<221> CDS
<222> (101)..(973)
<223> RXA01609

<400> 35
gttgcagtca gtgaagctga agagcagcca gtcggctaaa aaatagcctc catccaaccc 60

gctttacctg tcatcgcttc aggcgactag catggtgacc atg tct aaa atg cgc    115
                                            Met Ser Lys Met Arg
                                              1               5

gca cca ctt gta ccc gga att cct acc cca atc agg gaa gta cct gca    163
Ala Pro Leu Val Pro Gly Ile Pro Thr Pro Ile Arg Glu Val Pro Ala
                 10                  15                  20

cat att gaa cgt cca gaa tat gtg tgg aag gac gaa gtc caa gaa gca    211
His Ile Glu Arg Pro Glu Tyr Val Trp Lys Asp Glu Val Gln Glu Ala

25                  30                  35

atc ggt gag cct ttt gtg cag gcc cct gag gtc atc gag aag atg cgt     259
Ile Gly Glu Pro Phe Val Gln Ala Pro Glu Val Ile Glu Lys Met Arg
        40                  45                  50

gag aca tct cgc atc gct gca aac tca ctg aaa atc gcg ggc gaa gcc     307
Glu Thr Ser Arg Ile Ala Ala Asn Ser Leu Lys Ile Ala Gly Glu Ala
    55                  60                  65

gtc aag cca ggc gtg acc act gat gaa ctt gat cgc att gtg cat gag     355
Val Lys Pro Gly Val Thr Thr Asp Glu Leu Asp Arg Ile Val His Glu
 70                  75                  80                  85

tac acc tgc gat atg ggc gca tac cct tca gat ctt ggt tac cgg gga     403
Tyr Thr Cys Asp Met Gly Ala Tyr Pro Ser Asp Leu Gly Tyr Arg Gly
                90                  95                 100

ttc acc aag tcc tca tgc att tcc ctc aat gag atc gtg tgc cac ggt     451
Phe Thr Lys Ser Ser Cys Ile Ser Leu Asn Glu Ile Val Cys His Gly
            105                 110                 115

att cct gat tcc acc gtc att gaa gag ggc gat att gtt aac atc gat     499
Ile Pro Asp Ser Thr Val Ile Glu Glu Gly Asp Ile Val Asn Ile Asp
        120                 125                 130

gtc acc gcg ttc aag cac ggc gtc cac ggc gac tgc aat gcc acc ttc     547
Val Thr Ala Phe Lys His Gly Val His Gly Asp Cys Asn Ala Thr Phe
    135                 140                 145

tta gcg ggt gat gtt tct gaa gaa cac cgc ctg ctg gtt gag cgc acc     595
Leu Ala Gly Asp Val Ser Glu Glu His Arg Leu Leu Val Glu Arg Thr
150                 155                 160                 165

gaa gaa gcc atg atg cgt tcc atc cgt gca gca aag cct gga cgt gaa     643
Glu Glu Ala Met Met Arg Ser Ile Arg Ala Ala Lys Pro Gly Arg Glu
                170                 175                 180

atc aac gtc att ggg cgt gtc att gag tct tac gcc aag cgt ttt ggc     691
Ile Asn Val Ile Gly Arg Val Ile Glu Ser Tyr Ala Lys Arg Phe Gly
            185                 190                 195

tac aac gtg gtc cgc gat ttc acc gga cac ggc atc ggc cca act ttc     739
Tyr Asn Val Val Arg Asp Phe Thr Gly His Gly Ile Gly Pro Thr Phe
        200                 205                 210

cac aac ggc ctt gtg gtg ctg cac tac gac aac act cag tac cgc gat     787
His Asn Gly Leu Val Val Leu His Tyr Asp Asn Thr Gln Tyr Arg Asp
    215                 220                 225

ctg ctc gtg cca ggc atg acc ttg acc atc gag cca atg atc aac ctt     835
Leu Leu Val Pro Gly Met Thr Leu Thr Ile Glu Pro Met Ile Asn Leu
230                 235                 240                 245

ggt tcc ctc gac tac gag atc tgg gaa gat gat tgg act gtc caa aac     883
Gly Ser Leu Asp Tyr Glu Ile Trp Glu Asp Asp Trp Thr Val Gln Asn
                250                 255                 260

gtt gac cgt aag ttc agc gcg cag ttc gag cac acc att gtc atc acc     931
Val Asp Arg Lys Phe Ser Ala Gln Phe Glu His Thr Ile Val Ile Thr
            265                 270                 275

gaa gac ggc aat gag atc ctc acc ctc cca gac gat tcc gtc             973
Glu Asp Gly Asn Glu Ile Leu Thr Leu Pro Asp Asp Ser Val
        280                 285                 290

taaaaacgcc taggccacaa gcc                                           996


<210> 36
<211> 291
<212> PRT
<213> Corynebacterium glutamicum

<400> 36
Met Ser Lys Met Arg Ala Pro Leu Val Pro Gly Ile Pro Thr Pro Ile
  1               5                  10                  15

Arg Glu Val Pro Ala His Ile Glu Arg Pro Glu Tyr Val Trp Lys Asp
            20                  25                  30

Glu Val Gln Glu Ala Ile Gly Glu Pro Phe Val Gln Ala Pro Glu Val
        35                  40                  45

Ile Glu Lys Met Arg Glu Thr Ser Arg Ile Ala Ala Asn Ser Leu Lys
    50                  55                  60

Ile Ala Gly Glu Ala Val Lys Pro Gly Val Thr Thr Asp Glu Leu Asp
65                  70                  75                  80

Arg Ile Val His Glu Tyr Thr Cys Asp Met Gly Ala Tyr Pro Ser Asp
                85                  90                  95

Leu Gly Tyr Arg Gly Phe Thr Lys Ser Ser Cys Ile Ser Leu Asn Glu
            100                 105                 110

Ile Val Cys His Gly Ile Pro Asp Ser Thr Val Ile Glu Glu Gly Asp
        115                 120                 125

Ile Val Asn Ile Asp Val Thr Ala Phe Lys His Gly Val His Gly Asp
    130                 135                 140

Cys Asn Ala Thr Phe Leu Ala Gly Asp Val Ser Glu Glu His Arg Leu
145                 150                 155                 160

Leu Val Glu Arg Thr Glu Glu Ala Met Met Arg Ser Ile Arg Ala Ala
                165                 170                 175

Lys Pro Gly Arg Glu Ile Asn Val Ile Gly Arg Val Ile Glu Ser Tyr
            180                 185                 190

Ala Lys Arg Phe Gly Tyr Asn Val Val Arg Asp Phe Thr Gly His Gly
        195                 200                 205

Ile Gly Pro Thr Phe His Asn Gly Leu Val Val Leu His Tyr Asp Asn
    210                 215                 220

Thr Gln Tyr Arg Asp Leu Leu Val Pro Gly Met Thr Leu Thr Ile Glu
225                 230                 235                 240

Pro Met Ile Asn Leu Gly Ser Leu Asp Tyr Glu Ile Trp Glu Asp Asp
                245                 250                 255

Trp Thr Val Gln Asn Val Asp Arg Lys Phe Ser Ala Gln Phe Glu His
            260                 265                 270

Thr Ile Val Ile Thr Glu Asp Gly Asn Glu Ile Leu Thr Leu Pro Asp
        275                 280                 285

Asp Ser Val
    290


<210> 37
<211> 1644
<212> DNA
<213> Corynebacterium glutamicum

<220>
<221> CDS
<222> (101)..(1621)
<223> RXA01358

<400> 37
gagggtaaac cttatactcc gattcgtttt gatgtggtgg caatcgtttt ggatccgcac 60

acggggcgcc cagagatcac tgtttatgag gatgtggaac atg gcg ctc ggt aga 115
Met Ala Leu Gly Arg
1               5

act ata tcc act gcg caa tta ggt gtg cag gca aaa atc gtt cgt gtg 163
Thr Ile Ser Thr Ala Gln Leu Gly Val Gln Ala Lys Ile Val Arg Val
                10                  15                  20

gag gct aat gtt ggc cca gga ttg cct ggt acc tac att gtt gga tta 211
Glu Ala Asn Val Gly Pro Gly Leu Pro Gly Thr Tyr Ile Val Gly Leu
            25                  30                  35

gca gat act gcc att tca gaa tct cgg gat cgt att aaa act gcg gtg 259
Ala Asp Thr Ala Ile Ser Glu Ser Arg Asp Arg Ile Lys Thr Ala Val
        40                  45                  50

caa aac agt ggc ttg atg tgg cca aag acc aaa gtg atc att aac ctc 307
Gln Asn Ser Gly Leu Met Trp Pro Lys Thr Lys Val Ile Ile Asn Leu
    55                  60                  65

tcg ccg gct tcc atg cgt aaa caa ggt tcg cag tgt gat ctc gcg atg 355
Ser Pro Ala Ser Met Arg Lys Gln Gly Ser Gln Cys Asp Leu Ala Met
70                  75                  80                  85

acc gtt gca gtt ctc gtt gcc cat ggc tct aac ccc aaa gcg aag ttt 403
Thr Val Ala Val Leu Val Ala His Gly Ser Asn Pro Lys Ala Lys Phe
                90                  95                  100

cat gcg cag aac acg tta ttt ctg ggt gag gtg gcg ctt gat gga acc 451
His Ala Gln Asn Thr Leu Phe Leu Gly Glu Val Ala Leu Asp Gly Thr
            105                 110                 115

ttg ctc cct gtt act gga gtt ctt cca gcg ctg ttg gcc gcg aag gag 499
Leu Leu Pro Val Thr Gly Val Leu Pro Ala Leu Leu Ala Ala Lys Glu
        120                 125                 130

gaa ggt att ggc aag att gtg atc ccg gag gga aat gcc caa gaa gca 547
Glu Gly Ile Gly Lys Ile Val Ile Pro Glu Gly Asn Ala Gln Glu Ala
    135                 140                 145

gga ctg gtt gag gat cct tca gtc ttt ttg gca cat tcc atc gac cag 595
Gly Leu Val Glu Asp Pro Ser Val Phe Leu Ala His Ser Ile Asp Gln
150                 155                 160                 165

gtc ttg cga tgg ctt gat ggg gaa gag gcg ttg cct cag cct gga cta 643
Val Leu Arg Trp Leu Asp Gly Glu Glu Ala Leu Pro Gln Pro Gly Leu
                170                 175                 180

ttc aat gat gaa aat agc ctc aaa ctg cct gat atg cgt gat gtg gtg 691
Phe Asn Asp Glu Asn Ser Leu Lys Leu Pro Asp Met Arg Asp Val Val
            185                 190                 195

gga caa cca gaa gct agg ttt gct gca gaa gta gct gct gcc ggt ggt 739
Gly Gln Pro Glu Ala Arg Phe Ala Ala Glu Val Ala Ala Ala Gly Gly
        200                 205                 210

cac cac atg ctg atg att ggc cct ccc ggt tct gga aaa tcc atg atc 787
His His Met Leu Met Ile Gly Pro Pro Gly Ser Gly Lys Ser Met Ile
    215                 220                 225

gcc gag cgg att ccc agc ttg ctt ccc gaa ctg tcg ccc caa caa atg 835
Ala Glu Arg Ile Pro Ser Leu Leu Pro Glu Leu Ser Pro Gln Gln Met
230                 235                 240                 245

atc gag gcg acg gca gtg cat tcc gtt gtg ggg cga acc ttt tca ggg 883
Ile Glu Ala Thr Ala Val His Ser Val Val Gly Arg Thr Phe Ser Gly
                250                 255                 260

ccg gtg tcg agg gct ccg ttt att tcc cca cac cac aat gtc agc aag 931
Pro Val Ser Arg Ala Pro Phe Ile Ser Pro His His Asn Val Ser Lys
            265                 270                 275

gct gct ctg ctc gga ggt ggc tcg gga tct cct ctt ccg ggt gct atc 979
Ala Ala Leu Leu Gly Gly Gly Ser Gly Ser Pro Leu Pro Gly Ala Ile
        280                 285                 290

agc ctg gcg cat cat ggt gtg ttg ttc ctg gat gag gtt agt gag att 1027
Ser Leu Ala His His Gly Val Leu Phe Leu Asp Glu Val Ser Glu Ile
    295                 300                 305

cca gcg tca atc ctt gat tct ttg agg act cca ttg gaa tac ggc tcg 1075
Pro Ala Ser Ile Leu Asp Ser Leu Arg Thr Pro Leu Glu Tyr Gly Ser
310                 315                 320                 325

atc cgc atc atc aga tcc cgc cat gat gtc acc ttc ccc gca cag ttc 1123

Ile Arg Ile Ile Arg Ser Arg His Asp Val Thr Phe Pro Ala Gln Phe
                330                 335                 340

cag ctc atc ctc gcg gcc aat ccg tgt aga tgc ggt gca gaa cag cct      1171
Gln Leu Ile Leu Ala Ala Asn Pro Cys Arg Cys Gly Ala Glu Gln Pro
            345                 350                 355

caa gaa tgt gtc tgt tct ggc tca gct cgc gcg acg tac ctt aat aat      1219
Gln Glu Cys Val Cys Ser Gly Ser Ala Arg Ala Thr Tyr Leu Asn Asn
        360                 365                 370

ctt tcg ggt ccg ttg agg gat cgc ttg gac atg gtt gtt gcc acc cac      1267
Leu Ser Gly Pro Leu Arg Asp Arg Leu Asp Met Val Val Ala Thr His
    375                 380                 385

tct aaa ggt gca gtg ctg cgt agt gat gac gtt gag gca tct gct ccc      1315
Ser Lys Gly Ala Val Leu Arg Ser Asp Asp Val Glu Ala Ser Ala Pro
390                 395                 400                 405

att gct gat cgg gtg gca caa gct cgt gag agg gca gct ttc cga tgg      1363
Ile Ala Asp Arg Val Ala Gln Ala Arg Glu Arg Ala Ala Phe Arg Trp
                410                 415                 420

cgc cgt tct gga ctg gga aat ctt gtt aat gca cac gta gat cca cac      1411
Arg Arg Ser Gly Leu Gly Asn Leu Val Asn Ala His Val Asp Pro His
            425                 430                 435

ttc ttg cgg agg aat ttt gcc gca aca gaa gac gcc atg gtg tac ctc      1459
Phe Leu Arg Arg Asn Phe Ala Ala Thr Glu Asp Ala Met Val Tyr Leu
        440                 445                 450

ggc gcg ttc ctg gcg gaa gga aca atc tcc caa cgt ggc tgc gat cgg      1507
Gly Ala Phe Leu Ala Glu Gly Thr Ile Ser Gln Arg Gly Cys Asp Arg
    455                 460                 465

gcc ata aaa ctg ggt tgg acc ttg tgc gat ttg gat ggg gaa cag cag      1555
Ala Ile Lys Leu Gly Trp Thr Leu Cys Asp Leu Asp Gly Glu Gln Gln
470                 475                 480                 485

ccc aat ctt gac cat att gcg cga gcc atg gag ctt cgg ggc act aca      1603
Pro Asn Leu Asp His Ile Ala Arg Ala Met Glu Leu Arg Gly Thr Thr
                490                 495                 500

tac agt gag gta gca gca tgattgactc ccgcttgctg gca                    1644
Tyr Ser Glu Val Ala Ala
            505


<210> 38
<211> 507
<212> PRT
<213> Corynebacterium glutamicum


<400> 38
Met Ala Leu Gly Arg Thr Ile Ser Thr Ala Gln Leu Gly Val Gln Ala
  1               5                  10                  15

Lys Ile Val Arg Val Glu Ala Asn Val Gly Pro Gly Leu Pro Gly Thr
             20                  25                  30

Tyr Ile Val Gly Leu Ala Asp Thr Ala Ile Ser Glu Ser Arg Asp Arg
         35                  40                  45

Ile Lys Thr Ala Val Gln Asn Ser Gly Leu Met Trp Pro Lys Thr Lys
     50                  55                  60

Val Ile Ile Asn Leu Ser Pro Ala Ser Met Arg Lys Gln Gly Ser Gln
 65                  70                  75                  80

Cys Asp Leu Ala Met Thr Val Ala Val Leu Val Ala His Gly Ser Asn
                 85                  90                  95

Pro Lys Ala Lys Phe His Ala Gln Asn Thr Leu Phe Leu Gly Glu Val
            100                 105                 110

Ala Leu Asp Gly Thr Leu Leu Pro Val Thr Gly Val Leu Pro Ala Leu
        115                 120                 125

Leu Ala Ala Lys Glu Glu Gly Ile Gly Lys Ile Val Ile Pro Glu Gly
    130                 135                 140

Asn Ala Gln Glu Ala Gly Leu Val Glu Asp Pro Ser Val Phe Leu Ala
145                 150                 155                 160

His Ser Ile Asp Gln Val Leu Arg Trp Leu Asp Gly Glu Glu Ala Leu
                165                 170                 175

Pro Gln Pro Gly Leu Phe Asn Asp Glu Asn Ser Leu Lys Leu Pro Asp
            180                 185                 190

Met Arg Asp Val Val Gly Gln Pro Glu Ala Arg Phe Ala Ala Glu Val
        195                 200                 205

Ala Ala Ala Gly Gly His His Met Leu Met Ile Gly Pro Pro Gly Ser
    210                 215                 220

Gly Lys Ser Met Ile Ala Glu Arg Ile Pro Ser Leu Leu Pro Glu Leu
225                 230                 235                 240

Ser Pro Gln Gln Met Ile Glu Ala Thr Ala Val His Ser Val Val Gly
                245                 250                 255

Arg Thr Phe Ser Gly Pro Val Ser Arg Ala Pro Phe Ile Ser Pro His
            260                 265                 270

His Asn Val Ser Lys Ala Ala Leu Leu Gly Gly Gly Ser Gly Ser Pro
        275                 280                 285

Leu Pro Gly Ala Ile Ser Leu Ala His His Gly Val Leu Phe Leu Asp
    290                 295                 300

Glu Val Ser Glu Ile Pro Ala Ser Ile Leu Asp Ser Leu Arg Thr Pro
305                 310                 315                 320

Leu Glu Tyr Gly Ser Ile Arg Ile Ile Arg Ser Arg His Asp Val Thr
                325                 330                 335

Phe Pro Ala Gln Phe Gln Leu Ile Leu Ala Ala Asn Pro Cys Arg Cys
            340                 345                 350

Gly Ala Glu Gln Pro Gln Glu Cys Val Cys Ser Gly Ser Ala Arg Ala
        355                 360                 365

Thr Tyr Leu Asn Asn Leu Ser Gly Pro Leu Arg Asp Arg Leu Asp Met
    370                 375                 380

Val Val Ala Thr His Ser Lys Gly Ala Val Leu Arg Ser Asp Asp Val
385                 390                 395                 400

Glu Ala Ser Ala Pro Ile Ala Asp Arg Val Ala Gln Ala Arg Glu Arg
                405                 410                 415

Ala Ala Phe Arg Trp Arg Arg Ser Gly Leu Gly Asn Leu Val Asn Ala
            420                 425                 430

His Val Asp Pro His Phe Leu Arg Arg Asn Phe Ala Ala Thr Glu Asp
        435                 440                 445

Ala Met Val Tyr Leu Gly Ala Phe Leu Ala Glu Gly Thr Ile Ser Gln
    450                 455                 460

Arg Gly Cys Asp Arg Ala Ile Lys Leu Gly Trp Thr Leu Cys Asp Leu
465                 470                 475                 480

Asp Gly Glu Gln Gln Pro Asn Leu Asp His Ile Ala Arg Ala Met Glu
                485                 490                 495

Leu Arg Gly Thr Thr Tyr Ser Glu Val Ala Ala
            500                 505

<210> 39

<211> 1173
<212> DNA
<213> Corynebacterium glutamicum

<220>
<221> CDS
<222> (101)..(1150)
<223> RXA01458

<400> 39
gccattagag ttcatattta ccaatctaca aggcaactat ttcctattgc tacgatccga 60

acaacgctgt aagcgaacac aggttgtagg gtggatattc gtg aat cgc cga atc 115
Val Asn Arg Arg Ile
1               5

aag act ctg acg tgg ggt gct atc cct ttg gtg ctg ctg gca tcg ttg 163
Lys Thr Leu Thr Trp Gly Ala Ile Pro Leu Val Leu Leu Ala Ser Leu
                10                  15                  20

gta agc att gac cat att ccg gga aca aac atc aac ttg agc gtg cct 211
Val Ser Ile Asp His Ile Pro Gly Thr Asn Ile Asn Leu Ser Val Pro
            25                  30                  35

tat gcc gct gaa ggc cca ggt cct acg atc aat acg ctt ggt cag gtc 259
Tyr Ala Ala Glu Gly Pro Gly Pro Thr Ile Asn Thr Leu Gly Gln Val
        40                  45                  50

gac ggc gag gat gtt gtg tcc atc agt agt gct gat ctg gat gag acc 307
Asp Gly Glu Asp Val Val Ser Ile Ser Ser Ala Asp Leu Asp Glu Thr
    55                  60                  65

gaa ggt aac ctg aac atg acc act gtg tcg gtt cgt tcc ggc atg aca 355
Glu Gly Asn Leu Asn Met Thr Thr Val Ser Val Arg Ser Gly Met Thr
70                  75                  80                  85

ttg tcg cag gta att tcc cga tgg ctg ttt acc gat gac aca atc gtt 403
Leu Ser Gln Val Ile Ser Arg Trp Leu Phe Thr Asp Asp Thr Ile Val
                90                  95                  100

ccc atc gag cag gtt ttc cct ccc ggc caa tcc acc gag gaa gtc gaa 451
Pro Ile Glu Gln Val Phe Pro Pro Gly Gln Ser Thr Glu Glu Val Glu
            105                 110                 115

gaa tcc aac cgc acc gcg ttc atc tct tcg gag tct tcc gca acg atc 499
Glu Ser Asn Arg Thr Ala Phe Ile Ser Ser Glu Ser Ser Ala Thr Ile
        120                 125                 130

gcc gcg atg aat tac ctc aac att ccc gtc gaa gtt gaa gtt gca gaa 547
Ala Ala Met Asn Tyr Leu Asn Ile Pro Val Glu Val Glu Val Ala Glu
    135                 140                 145

gtc ctc acc gac agc gcc gca acc gga att ttc gaa ccc ggc gac aaa 595
Val Leu Thr Asp Ser Ala Ala Thr Gly Ile Phe Glu Pro Gly Asp Lys
150                 155                 160                 165

ctt ctc agc atc gac ggc acc gca atc tcc act ccc ggc gat gca caa 643
Leu Leu Ser Ile Asp Gly Thr Ala Ile Ser Thr Pro Gly Asp Ala Gln
                170                 175                 180

acc atc gtg cga tcg aaa gct ccc ggc gat gag atc acg att tcc tac 691
Thr Ile Val Arg Ser Lys Ala Pro Gly Asp Glu Ile Thr Ile Ser Tyr
            185                 190                 195

gag aga aac gat gcg gaa tct caa gca acc atc act ttg agg gaa cac 739
Glu Arg Asn Asp Ala Glu Ser Gln Ala Thr Ile Thr Leu Arg Glu His
        200                 205                 210

ccg gat gat tct tcg gtg gcg ttg ttg ggt att tca atg ttg tcg gtg 787
Pro Asp Asp Ser Ser Val Ala Leu Leu Gly Ile Ser Met Leu Ser Val
    215                 220                 225

cct tcg agc gcg att gag gtt gat tac aac ttg gaa gat atc ggt ggt 835
Pro Ser Ser Ala Ile Glu Val Asp Tyr Asn Leu Glu Asp Ile Gly Gly
230                 235                 240                 245

ccg agc gct ggc atg atg ttt tcg ttg gcg gtc gtc gat aag ctt tcg 883

Pro Ser Ala Gly Met Met Phe Ser Leu Ala Val Val Asp Lys Leu Ser
                250                 255                 260

cct ggc gcg ctg aat ggt ggc aag ttt gtc gct ggc act ggc acc atc     931
Pro Gly Ala Leu Asn Gly Gly Lys Phe Val Ala Gly Thr Gly Thr Ile
            265                 270                 275

gcg gag gac ggg tcg gtg ggc ccg att ggc ggt att gcg cac aag gtg     979
Ala Glu Asp Gly Ser Val Gly Pro Ile Gly Gly Ile Ala His Lys Val
        280                 285                 290

cgc gct gcg gag gac gcg ggc gcg gaa gtg ttt ttg agc cct gcg gac     1027
Arg Ala Ala Glu Asp Ala Gly Ala Glu Val Phe Leu Ser Pro Ala Asp
    295                 300                 305

aat tgc gcg gag gcg atg agt gcg aag cct cag gat atg acg atc ttg     1075
Asn Cys Ala Glu Ala Met Ser Ala Lys Pro Gln Asp Met Thr Ile Leu
310                 315                 320                 325

aag gtg gat tcg ttg tct cag gca atc gat cag atg gct gcc tac aac     1123
Lys Val Asp Ser Leu Ser Gln Ala Ile Asp Gln Met Ala Ala Tyr Asn
                330                 335                 340

gag ggc tct gat ttc cag acg tgt ggc tagtttttag acgctgaggt           1170
Glu Gly Ser Asp Phe Gln Thr Cys Gly
            345                 350

ttc                                                                 1173


<210> 40
<211> 350
<212> PRT
<213> Corynebacterium glutamicum

<400> 40
Val Asn Arg Arg Ile Lys Thr Leu Thr Trp Gly Ala Ile Pro Leu Val
1               5                   10                  15

Leu Leu Ala Ser Leu Val Ser Ile Asp His Ile Pro Gly Thr Asn Ile
            20                  25                  30

Asn Leu Ser Val Pro Tyr Ala Ala Glu Gly Pro Gly Pro Thr Ile Asn
        35                  40                  45

Thr Leu Gly Gln Val Asp Gly Glu Asp Val Val Ser Ile Ser Ser Ala
    50                  55                  60

Asp Leu Asp Glu Thr Glu Gly Asn Leu Asn Met Thr Thr Val Ser Val
65                  70                  75                  80

Arg Ser Gly Met Thr Leu Ser Gln Val Ile Ser Arg Trp Leu Phe Thr
                85                  90                  95

Asp Asp Thr Ile Val Pro Ile Glu Gln Val Phe Pro Pro Gly Gln Ser
            100                 105                 110

Thr Glu Glu Val Glu Glu Ser Asn Arg Thr Ala Phe Ile Ser Ser Glu
        115                 120                 125

Ser Ser Ala Thr Ile Ala Ala Met Asn Tyr Leu Asn Ile Pro Val Glu
    130                 135                 140

Val Glu Val Ala Glu Val Leu Thr Asp Ser Ala Ala Thr Gly Ile Phe
145                 150                 155                 160

Glu Pro Gly Asp Lys Leu Leu Ser Ile Asp Gly Thr Ala Ile Ser Thr
                165                 170                 175

Pro Gly Asp Ala Gln Thr Ile Val Arg Ser Lys Ala Pro Gly Asp Glu
            180                 185                 190

Ile Thr Ile Ser Tyr Glu Arg Asn Asp Ala Glu Ser Gln Ala Thr Ile
        195                 200                 205

Thr Leu Arg Glu His Pro Asp Asp Ser Ser Val Ala Leu Leu Gly Ile
    210                 215                 220

Ser Met Leu Ser Val Pro Ser Ser Ala Ile Glu Val Asp Tyr Asn Leu
225                 230                 235                 240

Glu Asp Ile Gly Gly Pro Ser Ala Gly Met Met Phe Ser Leu Ala Val
                245                 250                 255

Val Asp Lys Leu Ser Pro Gly Ala Leu Asn Gly Gly Lys Phe Val Ala
            260                 265                 270

Gly Thr Gly Thr Ile Ala Glu Asp Gly Ser Val Gly Pro Ile Gly Gly
        275                 280                 285

Ile Ala His Lys Val Arg Ala Ala Glu Asp Ala Gly Ala Glu Val Phe
    290                 295                 300

Leu Ser Pro Ala Asp Asn Cys Ala Glu Ala Met Ser Ala Lys Pro Gln
305                 310                 315                 320

Asp Met Thr Ile Leu Lys Val Asp Ser Leu Ser Gln Ala Ile Asp Gln
                325                 330                 335

Met Ala Ala Tyr Asn Glu Gly Ser Asp Phe Gln Thr Cys Gly
            340                 345                 350


<210> 41
<211> 1119
<212> DNA
<213> Corynebacterium glutamicum

<220>
<221> CDS
<222> (101)..(1096)
<223> RXA01654

<400> 41
cttttgctgt tatgaaagtg aactaaagat atctatgcgc agaccggtct cacgccgcgc 60

cattttttgca acatctgttt tggttgcggg ggtgagcatc atg tca cct tcg gcc    115
                                            Met Ser Pro Ser Ala
                                            1               5

aac gca gct gag gct ccg gca tcg gaa tgg gtg aat acg aca gcg atc    163
Asn Ala Ala Glu Ala Pro Ala Ser Glu Trp Val Asn Thr Thr Ala Ile
                10                  15                  20

gta gat caa gcg aat gct cag ttg tcg cag ttt ggc gtg agt ctt gac    211
Val Asp Gln Ala Asn Ala Gln Leu Ser Gln Phe Gly Val Ser Leu Asp
            25                  30                  35

cga agt gca gca gaa ctt ttt gat gat cag gca aac tcc caa att gat    259
Arg Ser Ala Ala Glu Leu Phe Asp Asp Gln Ala Asn Ser Gln Ile Asp
        40                  45                  50

gca gcg ctt tca ccg tat gcc gat aag gtt cca acc tct ggc ggc cag    307
Ala Ala Leu Ser Pro Tyr Ala Asp Lys Val Pro Thr Ser Gly Gly Gln
    55                  60                  65

gta gtc gag caa agt ctt cag gtt gtg gag cag gaa gtt caa aag gca    355
Val Val Glu Gln Ser Leu Gln Val Val Glu Gln Glu Val Gln Lys Ala
70                  75                  80                  85

ctg ccc aac tat gaa atc cgt acc gat ctg caa tcc cag gtg atg ggt    403
Leu Pro Asn Tyr Glu Ile Arg Thr Asp Leu Gln Ser Gln Val Met Gly
                90                  95                  100

gca act cta gga gag gtg ctg cac cga gtt cct gga tca tgg ttt gat    451
Ala Thr Leu Gly Glu Val Leu His Arg Val Pro Gly Ser Trp Phe Asp
            105                 110                 115

gcg cca gca gtt cct gaa gaa tcc agg att gta gag gaa cag ggt aaa    499
Ala Pro Ala Val Pro Glu Glu Ser Arg Ile Val Glu Glu Gln Gly Lys
        120                 125                 130

tcc ctg tat ggg ccc ggt acc ccg atc tat ctc aac gga aat tcc atg    547
Ser Leu Tyr Gly Pro Gly Thr Pro Ile Tyr Leu Asn Gly Asn Ser Met

135                 140                 145

tgc acg ctt gcg gtg act gga act gat gca gat ggg cgc aag atc ggt     595
Cys Thr Leu Ala Val Thr Gly Thr Asp Ala Asp Gly Arg Lys Ile Gly
150                 155                 160                 165

atc act gca ggt cac tgt gga aaa tcg ggc gat gca gtc cgt tcg gct     643
Ile Thr Ala Gly His Cys Gly Lys Ser Gly Asp Ala Val Arg Ser Ala
                170                 175                 180

gac tcc ttc tgg gtc ggc gat acc gga aca gtg gtg tac aac gcg cct     691
Asp Ser Phe Trp Val Gly Asp Thr Gly Thr Val Val Tyr Asn Ala Pro
            185                 190                 195

aac gct gac tac tcc gtg atc gag ttc ggt tcc aat gca gag ttg agc     739
Asn Ala Asp Tyr Ser Val Ile Glu Phe Gly Ser Asn Ala Glu Leu Ser
        200                 205                 210

aat acc tac aac ggt gtc acc gcg aat gct gtc ggc ggt ggc gtg act     787
Asn Thr Tyr Asn Gly Val Thr Ala Asn Ala Val Gly Gly Gly Val Thr
    215                 220                 225

aat ggc caa gaa gta tgc aaa aac gga gtt gct act ggc tac acc tgt     835
Asn Gly Gln Glu Val Cys Lys Asn Gly Val Ala Thr Gly Tyr Thr Cys
230                 235                 240                 245

ggt ttg gtg tgg act gct gat gag cgc atg acg atg tct cag gtg tgt     883
Gly Leu Val Trp Thr Ala Asp Glu Arg Met Thr Met Ser Gln Val Cys
                250                 255                 260

gcg ggt cgt ggt gat tcg ggt gct ccg ctg att gca gat ggt cgt gtg     931
Ala Gly Arg Gly Asp Ser Gly Ala Pro Leu Ile Ala Asp Gly Arg Val
            265                 270                 275

gtt ggt ctt gta tct ggt ggt gta att cct gat tac aac ctg gca tgc     979
Val Gly Leu Val Ser Gly Gly Val Ile Pro Asp Tyr Asn Leu Ala Cys
        280                 285                 290

gcc act ccg ttg cag gga cct ttc ttc atg cca acg ctg tca gtg aac     1027
Ala Thr Pro Leu Gln Gly Pro Phe Phe Met Pro Thr Leu Ser Val Asn
    295                 300                 305

atg gat act gtc cta act gat ttg gat tcg cag gat ctt ccc ggt cga     1075
Met Asp Thr Val Leu Thr Asp Leu Asp Ser Gln Asp Leu Pro Gly Arg
310                 315                 320                 325

ggt ttt cag cca act gct gga tagaatttag aaaatccgcc gtt               1119
Gly Phe Gln Pro Thr Ala Gly
                330


<210> 42
<211> 332
<212> PRT
<213> Corynebacterium glutamicum

<400> 42
Met Ser Pro Ser Ala Asn Ala Ala Glu Ala Pro Ala Ser Glu Trp Val
1               5                   10                  15

Asn Thr Thr Ala Ile Val Asp Gln Ala Asn Ala Gln Leu Ser Gln Phe
            20                  25                  30

Gly Val Ser Leu Asp Arg Ser Ala Ala Glu Leu Phe Asp Asp Gln Ala
        35                  40                  45

Asn Ser Gln Ile Asp Ala Ala Leu Ser Pro Tyr Ala Asp Lys Val Pro
    50                  55                  60

Thr Ser Gly Gly Gln Val Val Glu Gln Ser Leu Gln Val Val Glu Gln
65                  70                  75                  80

Glu Val Gln Lys Ala Leu Pro Asn Tyr Glu Ile Arg Thr Asp Leu Gln
                85                  90                  95

Ser Gln Val Met Gly Ala Thr Leu Gly Glu Val Leu His Arg Val Pro
            100                 105                 110

Gly Ser Trp Phe Asp Ala Pro Ala Val Pro Glu Glu Ser Arg Ile Val
        115                 120                 125

Glu Glu Gln Gly Lys Ser Leu Tyr Gly Pro Gly Thr Pro Ile Tyr Leu
    130                 135                 140

Asn Gly Asn Ser Met Cys Thr Leu Ala Val Thr Gly Thr Asp Ala Asp
145                 150                 155                 160

Gly Arg Lys Ile Gly Ile Thr Ala Gly His Cys Gly Lys Ser Gly Asp
                165                 170                 175

Ala Val Arg Ser Ala Asp Ser Phe Trp Val Gly Asp Thr Gly Thr Val
            180                 185                 190

Val Tyr Asn Ala Pro Asn Ala Asp Tyr Ser Val Ile Glu Phe Gly Ser
        195                 200                 205

Asn Ala Glu Leu Ser Asn Thr Tyr Asn Gly Val Thr Ala Asn Ala Val
    210                 215                 220

Gly Gly Gly Val Thr Asn Gly Gln Glu Val Cys Lys Asn Gly Val Ala
225                 230                 235                 240

Thr Gly Tyr Thr Cys Gly Leu Val Trp Thr Ala Asp Glu Arg Met Thr
                245                 250                 255

Met Ser Gln Val Cys Ala Gly Arg Gly Asp Ser Gly Ala Pro Leu Ile
            260                 265                 270

Ala Asp Gly Arg Val Val Gly Leu Val Ser Gly Gly Val Ile Pro Asp
        275                 280                 285

Tyr Asn Leu Ala Cys Ala Thr Pro Leu Gln Gly Pro Phe Phe Met Pro
    290                 295                 300

Thr Leu Ser Val Asn Met Asp Thr Val Leu Thr Asp Leu Asp Ser Gln
305                 310                 315                 320

Asp Leu Pro Gly Arg Gly Phe Gln Pro Thr Ala Gly
                325                 330


<210> 43
<211> 2049
<212> DNA
<213> Corynebacterium glutamicum

<220>
<221> CDS
<222> (101)..(2026)
<223> RXN01868

<400> 43
tgacaggcta ccttctgggg tggacatgat ccccaacgct caacccactt gtggcaccaa 60

ccacaaaccc tgtggcggta aatcccctag agtaggccac atg aag gat ctt tat   115
                                           Met Lys Asp Leu Tyr
                                           1               5

cgc ttt gtc aat ggc ctg tgg ctt gac acc cac atc att ccc gac gat   163
Arg Phe Val Asn Gly Leu Trp Leu Asp Thr His Ile Ile Pro Asp Asp
                10                  15                  20

cgc gcg gtg gac ggc acg ttc cac aag ctg cgc gat gat gct gaa gaa   211
Arg Ala Val Asp Gly Thr Phe His Lys Leu Arg Asp Asp Ala Glu Glu
            25                  30                  35

gac gtc cat gag atc gtc aag gaa gac act gga cgc gca ggc aca ctt   259
Asp Val His Glu Ile Val Lys Glu Asp Thr Gly Arg Ala Gly Thr Leu
        40                  45                  50

tat gcc tca ttt atg gat act gac gcc atc aac gct gct ggt gtt gca   307
Tyr Ala Ser Phe Met Asp Thr Asp Ala Ile Asn Ala Ala Gly Val Ala
    55                  60                  65

ccg ctc gat gcg gat ctg aac agg ctg tct gtt gct aac tca tcg ttt 355
Pro Leu Asp Ala Asp Leu Asn Arg Leu Ser Val Ala Asn Ser Ser Phe
70                  75                  80                  85

ttc gca gct gct ctc ggc gaa ctg gac cgt gaa ggc gtt ggc gcg cca 403
Phe Ala Ala Ala Leu Gly Glu Leu Asp Arg Glu Gly Val Gly Ala Pro
                90                  95                  100

gta ggt ttc tgg gtg gag aag gat tct tcc tcc aac gaa tcc gtc gcc 451
Val Gly Phe Trp Val Glu Lys Asp Ser Ser Ser Asn Glu Ser Val Ala
            105                 110                 115

tat gtc atc cag tcc ggc ctc ggc ctg ccc gat gag gct tat tac cgc 499
Tyr Val Ile Gln Ser Gly Leu Gly Leu Pro Asp Glu Ala Tyr Tyr Arg
        120                 125                 130

gag gag gca cac gcc gaa act ctc gcg gcc tac aaa gag cac gtt gag 547
Glu Glu Ala His Ala Glu Thr Leu Ala Ala Tyr Lys Glu His Val Glu
    135                 140                 145

cgc atg ctc ggc tac ttg gat aac agc cgc ctc ttc ggt ctg tcg gct 595
Arg Met Leu Gly Tyr Leu Asp Asn Ser Arg Leu Phe Gly Leu Ser Ala
150                 155                 160                 165

gct tcc gct gcc gca cga att gtc gcc ctg gaa acg gaa atc gct gct 643
Ala Ser Ala Ala Ala Arg Ile Val Ala Leu Glu Thr Glu Ile Ala Ala
                170                 175                 180

ggc cac tgg gat gtc gtg aag acc cgc gac gcc gta gcc acc tac aac 691
Gly His Trp Asp Val Val Lys Thr Arg Asp Ala Val Ala Thr Tyr Asn
            185                 190                 195

ccc acc gaa ctc ggc gcg ctg cca cca aag gtc cgc acg ctg ctc agt 739
Pro Thr Glu Leu Gly Ala Leu Pro Pro Lys Val Arg Thr Leu Leu Ser
        200                 205                 210

tcc gca ggc ctc ccg gac cag cgc ctg gta tcg atg atg ccg tca tac 787
Ser Ala Gly Leu Pro Asp Gln Arg Leu Val Ser Met Met Pro Ser Tyr
    215                 220                 225

ctc gac cac ctc aac ggc ttg ctt gtc gac gac cgc ctc ccc gat tgg 835
Leu Asp His Leu Asn Gly Leu Leu Val Asp Asp Arg Leu Pro Asp Trp
230                 235                 240                 245

cag cta tgg gca acc tgg cac atc ttg agg tct cga gca gga ctg ttg 883
Gln Leu Trp Ala Thr Trp His Ile Leu Arg Ser Arg Ala Gly Leu Leu
                250                 255                 260

acc gag gaa att agc caa gca aac ttc gac ttc tat ggc acc aaa ctg 931
Thr Glu Glu Ile Ser Gln Ala Asn Phe Asp Phe Tyr Gly Thr Lys Leu
            265                 270                 275

tcc ggc gcc acc gag caa aaa gat cga tgg aag cgt gct gtc ggc ctg 979
Ser Gly Ala Thr Glu Gln Lys Asp Arg Trp Lys Arg Ala Val Gly Leu
        280                 285                 290

gca gag cgc atg gtg ggc gag gaa atc ggg caa cga ttc gtc gaa agg 1027
Ala Glu Arg Met Val Gly Glu Glu Ile Gly Gln Arg Phe Val Glu Arg
    295                 300                 305

cat ttt cct gca agc tcc aag gag cac atg ctt gag ctc gtc gac tac 1075
His Phe Pro Ala Ser Ser Lys Glu His Met Leu Glu Leu Val Asp Tyr
310                 315                 320                 325

ctg gtt gcc gcc tac cgt gat cgc att tcc aac ctc gaa tgg atg acg 1123
Leu Val Ala Ala Tyr Arg Asp Arg Ile Ser Asn Leu Glu Trp Met Thr
                330                 335                 340

ccc gcc acc cgc gag cgt gcc ctg gaa aag ttg ggc aaa ttc aac gcg 1171
Pro Ala Thr Arg Glu Arg Ala Leu Glu Lys Leu Gly Lys Phe Asn Ala
            345                 350                 355

aaa atc ggc tac ccc gac aag tgg cgc tcc tac gaa ggc ctc gaa ttc 1219
Lys Ile Gly Tyr Pro Asp Lys Trp Arg Ser Tyr Glu Gly Leu Glu Phe
        360                 365                 370

ggc tcc gac ctg gtg gac aac tcc cgc aag ggc tcc gcg ttc ctc cat 1267

Gly Ser Asp Leu Val Asp Asn Ser Arg Lys Gly Ser Ala Phe Leu His
    375                 380                 385

gac tat gag ctg ggc aag atc ggc aaa cca gcc gac cgc gac gaa tgg     1315
Asp Tyr Glu Leu Gly Lys Ile Gly Lys Pro Ala Asp Arg Asp Glu Trp
390                 395                 400                 405

gtc acc acc cca caa acc gtc aac gcc ttc tac aac ccc gtg gtc aac     1363
Val Thr Thr Pro Gln Thr Val Asn Ala Phe Tyr Asn Pro Val Val Asn
                410                 415                 420

gac atc acc ttc ccc gca gcc atc ctg cgc gca cca ttc ttc gac ccc     1411
Asp Ile Thr Phe Pro Ala Ala Ile Leu Arg Ala Pro Phe Phe Asp Pro
            425                 430                 435

gaa gca gaa gcc gca gaa aac ttc ggt gca atc ggt gct gtg atc gga     1459
Glu Ala Glu Ala Ala Glu Asn Phe Gly Ala Ile Gly Ala Val Ile Gly
        440                 445                 450

cac gaa atc ggc cac ggc ttt gac gat caa ggc agc caa tac gac ggc     1507
His Glu Ile Gly His Gly Phe Asp Asp Gln Gly Ser Gln Tyr Asp Gly
    455                 460                 465

gac ggc aac ctc aac tcc tgg tgg acc gac gaa gac cgc tcc gca ttc     1555
Asp Gly Asn Leu Asn Ser Trp Trp Thr Asp Glu Asp Arg Ser Ala Phe
470                 475                 480                 485

gag cag ctc acc tca cgt ctg gtc acc caa ttc agc gga ctc gtc cct     1603
Glu Gln Leu Thr Ser Arg Leu Val Thr Gln Phe Ser Gly Leu Val Pro
                490                 495                 500

gcc gtc ctg acc tct gaa gga atc gac acc gac ggc gtc aac ggt gaa     1651
Ala Val Leu Thr Ser Glu Gly Ile Asp Thr Asp Gly Val Asn Gly Glu
            505                 510                 515

ttc act ctc ggc gaa aac atc ggt gac ctc ggc gga ttg ggc atc gct     1699
Phe Thr Leu Gly Glu Asn Ile Gly Asp Leu Gly Gly Leu Gly Ile Ala
        520                 525                 530

gtc gtt gcc tac gaa aag tac ctc gca gac cgt ggc caa acc ttt gaa     1747
Val Val Ala Tyr Glu Lys Tyr Leu Ala Asp Arg Gly Gln Thr Phe Glu
    535                 540                 545

acc tca cca gtc caa aaa ttc gaa gca gaa ggc gcc gag gaa ggc ctg     1795
Thr Ser Pro Val Gln Lys Phe Glu Ala Glu Gly Ala Glu Glu Gly Leu
550                 555                 560                 565

gcc gag caa gaa ttc aac ggt ctc caa cgc ctc ttc ctg tcc tgg gct     1843
Ala Glu Gln Glu Phe Asn Gly Leu Gln Arg Leu Phe Leu Ser Trp Ala
                570                 575                 580

cgc gtg tgg cgc acc aaa atc cgc cca cag atg gcc gtc caa tac ctg     1891
Arg Val Trp Arg Thr Lys Ile Arg Pro Gln Met Ala Val Gln Tyr Leu
            585                 590                 595

gcc atc gac cca cac tcc cct gca gaa ttc cgc tgc aat gtc atc gcc     1939
Ala Ile Asp Pro His Ser Pro Ala Glu Phe Arg Cys Asn Val Ile Ala
        600                 605                 610

gga aac gtc gct gaa ttc tac gaa gca ttc gac gtc ccc gaa gat gca     1987
Gly Asn Val Ala Glu Phe Tyr Glu Ala Phe Asp Val Pro Glu Asp Ala
    615                 620                 625

cct gtg tac atc aag cca gaa gag cgc cta gct atc tgg tagttgttag     2036
Pro Val Tyr Ile Lys Pro Glu Glu Arg Leu Ala Ile Trp
630                 635                 640

ttggtattga aaa                                                      2049


<210> 44
<211> 642
<212> PRT
<213> Corynebacterium glutamicum

<400> 44
Met Lys Asp Leu Tyr Arg Phe Val Asn Gly Leu Trp Leu Asp Thr His

1               5                   10                  15

Ile Ile Pro Asp Asp Arg Ala Val Asp Gly Thr Phe His Lys Leu Arg
            20                  25                  30

Asp Asp Ala Glu Glu Asp Val His Glu Ile Val Lys Glu Asp Thr Gly
        35                  40                  45

Arg Ala Gly Thr Leu Tyr Ala Ser Phe Met Asp Thr Asp Ala Ile Asn
    50                  55                  60

Ala Ala Gly Val Ala Pro Leu Asp Ala Asp Leu Asn Arg Leu Ser Val
65                  70                  75                  80

Ala Asn Ser Ser Phe Phe Ala Ala Ala Leu Gly Glu Leu Asp Arg Glu
                85                  90                  95

Gly Val Gly Ala Pro Val Gly Phe Trp Val Glu Lys Asp Ser Ser Ser
            100                 105                 110

Asn Glu Ser Val Ala Tyr Val Ile Gln Ser Gly Leu Gly Leu Pro Asp
        115                 120                 125

Glu Ala Tyr Tyr Arg Glu Glu Ala His Ala Glu Thr Leu Ala Ala Tyr
    130                 135                 140

Lys Glu His Val Glu Arg Met Leu Gly Tyr Leu Asp Asn Ser Arg Leu
145                 150                 155                 160

Phe Gly Leu Ser Ala Ala Ser Ala Ala Ala Arg Ile Val Ala Leu Glu
                165                 170                 175

Thr Glu Ile Ala Ala Gly His Trp Asp Val Val Lys Thr Arg Asp Ala
            180                 185                 190

Val Ala Thr Tyr Asn Pro Thr Glu Leu Gly Ala Leu Pro Pro Lys Val
        195                 200                 205

Arg Thr Leu Leu Ser Ser Ala Gly Leu Pro Asp Gln Arg Leu Val Ser
    210                 215                 220

Met Met Pro Ser Tyr Leu Asp His Leu Asn Gly Leu Leu Val Asp Asp
225                 230                 235                 240

Arg Leu Pro Asp Trp Gln Leu Trp Ala Thr Trp His Ile Leu Arg Ser
                245                 250                 255

Arg Ala Gly Leu Leu Thr Glu Glu Ile Ser Gln Ala Asn Phe Asp Phe
            260                 265                 270

Tyr Gly Thr Lys Leu Ser Gly Ala Thr Glu Gln Lys Asp Arg Trp Lys
        275                 280                 285

Arg Ala Val Gly Leu Ala Glu Arg Met Val Gly Glu Glu Ile Gly Gln
    290                 295                 300

Arg Phe Val Glu Arg His Phe Pro Ala Ser Ser Lys Glu His Met Leu
305                 310                 315                 320

Glu Leu Val Asp Tyr Leu Val Ala Ala Tyr Arg Asp Arg Ile Ser Asn
                325                 330                 335

Leu Glu Trp Met Thr Pro Ala Thr Arg Glu Arg Ala Leu Glu Lys Leu
            340                 345                 350

Gly Lys Phe Asn Ala Lys Ile Gly Tyr Pro Asp Lys Trp Arg Ser Tyr
        355                 360                 365

Glu Gly Leu Glu Phe Gly Ser Asp Leu Val Asp Asn Ser Arg Lys Gly
    370                 375                 380

Ser Ala Phe Leu His Asp Tyr Glu Leu Gly Lys Ile Gly Lys Pro Ala
385                 390                 395                 400

Asp Arg Asp Glu Trp Val Thr Thr Pro Gln Thr Val Asn Ala Phe Tyr
                405                 410                 415

Asn Pro Val Val Asn Asp Ile Thr Phe Pro Ala Ala Ile Leu Arg Ala
            420                 425                 430

Pro Phe Phe Asp Pro Glu Ala Glu Ala Ala Glu Asn Phe Gly Ala Ile
        435                 440                 445

Gly Ala Val Ile Gly His Glu Ile Gly His Gly Phe Asp Asp Gln Gly
    450                 455                 460

Ser Gln Tyr Asp Gly Asp Gly Asn Leu Asn Ser Trp Trp Thr Asp Glu
465                 470                 475                 480

Asp Arg Ser Ala Phe Glu Gln Leu Thr Ser Arg Leu Val Thr Gln Phe
                485                 490                 495

Ser Gly Leu Val Pro Ala Val Leu Thr Ser Glu Gly Ile Asp Thr Asp
            500                 505                 510

Gly Val Asn Gly Glu Phe Thr Leu Gly Glu Asn Ile Gly Asp Leu Gly
        515                 520                 525

Gly Leu Gly Ile Ala Val Val Ala Tyr Glu Lys Tyr Leu Ala Asp Arg
    530                 535                 540

Gly Gln Thr Phe Glu Thr Ser Pro Val Gln Lys Phe Glu Ala Glu Gly
545                 550                 555                 560

Ala Glu Glu Gly Leu Ala Glu Gln Glu Phe Asn Gly Leu Gln Arg Leu
                565                 570                 575

Phe Leu Ser Trp Ala Arg Val Trp Arg Thr Lys Ile Arg Pro Gln Met

            580                 585                 590

Ala Val Gln Tyr Leu Ala Ile Asp Pro His Ser Pro Ala Glu Phe Arg
        595                 600                 605

Cys Asn Val Ile Ala Gly Asn Val Ala Glu Phe Tyr Glu Ala Phe Asp
    610                 615                 620

Val Pro Glu Asp Ala Pro Val Tyr Ile Lys Pro Glu Glu Arg Leu Ala
625                 630                 635                 640

Ile Trp



<210> 45
<211> 1734
<212> DNA
<213> Corynebacterium glutamicum

<220>
<221> CDS
<222> (101)..(1711)
<223> FRXA01868

<400> 45
atgcggatct gaacaggctg tctgttgcta actcatcgtt ttcgcagctg ctctcggcga 60

actggaccgt gaaggcgttg gcgcgccagt aggtttctgg gtg gag aag gat tct    115
                                            Val Glu Lys Asp Ser
                                            1               5

tcc tcc aac gaa tcc gtc gcc tat gtc atc cag tcc ggc ctc ggc ctg    163
Ser Ser Asn Glu Ser Val Ala Tyr Val Ile Gln Ser Gly Leu Gly Leu
                10                  15                  20

ccc gat gag gct tat tac cgc gag gag gca cac gcc gaa act ctc gcg    211
Pro Asp Glu Ala Tyr Tyr Arg Glu Glu Ala His Ala Glu Thr Leu Ala
            25                  30                  35

gcc tac aaa gag cac gtt gag cgc atg ctc ggc tac ttg gat aac agc    259
Ala Tyr Lys Glu His Val Glu Arg Met Leu Gly Tyr Leu Asp Asn Ser
        40                  45                  50

cgc ctc ttc ggt ctg tcg gct gct tcc gct gcc gca cga att gtc gcc    307

Arg Leu Phe Gly Leu Ser Ala Ala Ser Ala Ala Ala Arg Ile Val Ala
    55                  60                  65

ctg gaa acg gaa atc gct gct ggc cac tgg gat gtc gtg aag acc cgc 355
Leu Glu Thr Glu Ile Ala Ala Gly His Trp Asp Val Val Lys Thr Arg
70                  75                  80                  85

gac gcc gta gcc acc tac aac ccc acc gaa ctc ggc gcg ctg cca cca 403
Asp Ala Val Ala Thr Tyr Asn Pro Thr Glu Leu Gly Ala Leu Pro Pro
                90                  95                  100

aag gtc cgc acg ctg ctc agt tcc gca ggc ctc ccg gac cag cgc ctg 451
Lys Val Arg Thr Leu Leu Ser Ser Ala Gly Leu Pro Asp Gln Arg Leu
            105                 110                 115

gta tcg atg atg ccg tca tac ctc gac cac ctc aac ggc ttg ctt gtc 499
Val Ser Met Met Pro Ser Tyr Leu Asp His Leu Asn Gly Leu Leu Val
        120                 125                 130

gac gac cgc ctc ccc gat tgg cag cta tgg gca acc tgg cac atc ttg 547
Asp Asp Arg Leu Pro Asp Trp Gln Leu Trp Ala Thr Trp His Ile Leu
    135                 140                 145

agg tct cga gca gga ctg ttg acc gag gaa att agc caa gca aac ttc 595
Arg Ser Arg Ala Gly Leu Leu Thr Glu Glu Ile Ser Gln Ala Asn Phe
150                 155                 160                 165

gac ttc tat ggc acc aaa ctg tcc ggc gcc acc gag caa aaa gat cga 643
Asp Phe Tyr Gly Thr Lys Leu Ser Gly Ala Thr Glu Gln Lys Asp Arg
                170                 175                 180

tgg aag cgt gct gtc ggc ctg gca gag cgc atg gtg ggc gag gaa atc 691
Trp Lys Arg Ala Val Gly Leu Ala Glu Arg Met Val Gly Glu Glu Ile
            185                 190                 195

ggg caa cga ttc gtc gaa agg cat ttt cct gca agc tcc aag gag cac 739
Gly Gln Arg Phe Val Glu Arg His Phe Pro Ala Ser Ser Lys Glu His
        200                 205                 210

atg ctt gag ctc gtc gac tac ctg gtt gcc gcc tac cgt gat cgc att 787
Met Leu Glu Leu Val Asp Tyr Leu Val Ala Ala Tyr Arg Asp Arg Ile
    215                 220                 225

tcc aac ctc gaa tgg atg acg ccc gcc acc cgc gag cgt gcc ctg gaa 835
Ser Asn Leu Glu Trp Met Thr Pro Ala Thr Arg Glu Arg Ala Leu Glu
230                 235                 240                 245

aag ttg ggc aaa ttc aac gcg aaa atc ggc tac ccc gac aag tgg cgc 883
Lys Leu Gly Lys Phe Asn Ala Lys Ile Gly Tyr Pro Asp Lys Trp Arg
                250                 255                 260

tcc tac gaa ggc ctc gaa ttc ggc tcc gac ctg gtg gac aac tcc cgc 931
Ser Tyr Glu Gly Leu Glu Phe Gly Ser Asp Leu Val Asp Asn Ser Arg
            265                 270                 275

aag ggc tcc gcg ttc ctc cat gac tat gag ctg ggc aag atc ggc aaa 979
Lys Gly Ser Ala Phe Leu His Asp Tyr Glu Leu Gly Lys Ile Gly Lys
        280                 285                 290

cca gcc gac cgc gac gaa tgg gtc acc acc cca caa acc gtc aac gcc 1027
Pro Ala Asp Arg Asp Glu Trp Val Thr Thr Pro Gln Thr Val Asn Ala
    295                 300                 305

ttc tac aac ccc gtg gtc aac gac atc acc ttc ccc gca gcc atc ctg 1075
Phe Tyr Asn Pro Val Val Asn Asp Ile Thr Phe Pro Ala Ala Ile Leu
310                 315                 320                 325

cgc gca cca ttc ttc gac ccc gaa gca gaa gcc gca gaa aac ttc ggt 1123
Arg Ala Pro Phe Phe Asp Pro Glu Ala Glu Ala Ala Glu Asn Phe Gly
                330                 335                 340

gca atc ggt gct gtg atc gga cac gaa atc ggc cac ggc ttt gac gat 1171
Ala Ile Gly Ala Val Ile Gly His Glu Ile Gly His Gly Phe Asp Asp
            345                 350                 355

caa ggc agc caa tac gac ggc gac ggc aac ctc aac tcc tgg tgg acc 1219
Gln Gly Ser Gln Tyr Asp Gly Asp Gly Asn Leu Asn Ser Trp Trp Thr

360                 365                 370

gac gaa gac cgc tcc gca ttc gag cag ctc acc tca cgt ctg gtc acc      1267
Asp Glu Asp Arg Ser Ala Phe Glu Gln Leu Thr Ser Arg Leu Val Thr
    375                 380                 385

caa ttc agc gga ctc gtc cct gcc gtc ctg acc tct gaa gga atc gac      1315
Gln Phe Ser Gly Leu Val Pro Ala Val Leu Thr Ser Glu Gly Ile Asp
390                 395                 400                 405

acc gac ggc gtc aac ggt gaa ttc act ctc ggc gaa aac atc ggt gac      1363
Thr Asp Gly Val Asn Gly Glu Phe Thr Leu Gly Glu Asn Ile Gly Asp
                410                 415                 420

ctc ggc gga ttg ggc atc gct gtc gtt gcc tac gaa aag tac ctc gca      1411
Leu Gly Gly Leu Gly Ile Ala Val Val Ala Tyr Glu Lys Tyr Leu Ala
            425                 430                 435

gac cgt ggc caa acc ttt gaa acc tca cca gtc caa aaa ttc gaa gca      1459
Asp Arg Gly Gln Thr Phe Glu Thr Ser Pro Val Gln Lys Phe Glu Ala
        440                 445                 450

gaa ggc gcc gag gaa ggc ctg gcc gag caa gaa ttc aac ggt ctc caa      1507
Glu Gly Ala Glu Glu Gly Leu Ala Glu Gln Glu Phe Asn Gly Leu Gln
    455                 460                 465

cgc ctc ttc ctg tcc tgg gct cgc gtg tgg cgc acc aaa atc cgc cca      1555
Arg Leu Phe Leu Ser Trp Ala Arg Val Trp Arg Thr Lys Ile Arg Pro
470                 475                 480                 485

cag atg gcc gtc caa tac ctg gcc atc gac cca cac tcc cct gca gaa      1603
Gln Met Ala Val Gln Tyr Leu Ala Ile Asp Pro His Ser Pro Ala Glu
                490                 495                 500

ttc cgc tgc aat gtc atc gcc gga aac gtc gct gaa ttc tac gaa gca      1651
Phe Arg Cys Asn Val Ile Ala Gly Asn Val Ala Glu Phe Tyr Glu Ala
            505                 510                 515

ttc gac gtc ccc gaa gat gca cct gtg tac atc aag cca gaa gag cgc      1699
Phe Asp Val Pro Glu Asp Ala Pro Val Tyr Ile Lys Pro Glu Glu Arg
        520                 525                 530

cta gct atc tgg tagttgttag ttggtattga aaa                            1734
Leu Ala Ile Trp
    535


<210> 46
<211> 537
<212> PRT
<213> Corynebacterium glutamicum

<400> 46
Val Glu Lys Asp Ser Ser Ser Asn Glu Ser Val Ala Tyr Val Ile Gln
1               5                   10                  15

Ser Gly Leu Gly Leu Pro Asp Glu Ala Tyr Tyr Arg Glu Glu Ala His
            20                  25                  30

Ala Glu Thr Leu Ala Ala Tyr Lys Glu His Val Glu Arg Met Leu Gly
        35                  40                  45

Tyr Leu Asp Asn Ser Arg Leu Phe Gly Leu Ser Ala Ala Ser Ala Ala
    50                  55                  60

Ala Arg Ile Val Ala Leu Glu Thr Glu Ile Ala Ala Gly His Trp Asp
65                  70                  75                  80

Val Val Lys Thr Arg Asp Ala Val Ala Thr Tyr Asn Pro Thr Glu Leu
                85                  90                  95

Gly Ala Leu Pro Pro Lys Val Arg Thr Leu Leu Ser Ser Ala Gly Leu
            100                 105                 110

Pro Asp Gln Arg Leu Val Ser Met Met Pro Ser Tyr Leu Asp His Leu
        115                 120                 125

Asn Gly Leu Leu Val Asp Asp Arg Leu Pro Asp Trp Gln Leu Trp Ala
    130                 135                 140

Thr Trp His Ile Leu Arg Ser Arg Ala Gly Leu Leu Thr Glu Glu Ile
145                 150                 155                 160

Ser Gln Ala Asn Phe Asp Phe Tyr Gly Thr Lys Leu Ser Gly Ala Thr
                165                 170                 175

Glu Gln Lys Asp Arg Trp Lys Arg Ala Val Gly Leu Ala Glu Arg Met
            180                 185                 190

Val Gly Glu Glu Ile Gly Gln Arg Phe Val Glu Arg His Phe Pro Ala
        195                 200                 205

Ser Ser Lys Glu His Met Leu Glu Leu Val Asp Tyr Leu Val Ala Ala
    210                 215                 220

Tyr Arg Asp Arg Ile Ser Asn Leu Glu Trp Met Thr Pro Ala Thr Arg
225                 230                 235                 240

Glu Arg Ala Leu Glu Lys Leu Gly Lys Phe Asn Ala Lys Ile Gly Tyr
                245                 250                 255

Pro Asp Lys Trp Arg Ser Tyr Glu Gly Leu Glu Phe Gly Ser Asp Leu
            260                 265                 270

Val Asp Asn Ser Arg Lys Gly Ser Ala Phe Leu His Asp Tyr Glu Leu
        275                 280                 285

Gly Lys Ile Gly Lys Pro Ala Asp Arg Asp Glu Trp Val Thr Thr Pro
    290                 295                 300

Gln Thr Val Asn Ala Phe Tyr Asn Pro Val Val Asn Asp Ile Thr Phe
305                 310                 315                 320

Pro Ala Ala Ile Leu Arg Ala Pro Phe Phe Asp Pro Glu Ala Glu Ala
                325                 330                 335

Ala Glu Asn Phe Gly Ala Ile Gly Ala Val Ile Gly His Glu Ile Gly
            340                 345                 350

His Gly Phe Asp Asp Gln Gly Ser Gln Tyr Asp Gly Asp Gly Asn Leu
        355                 360                 365

Asn Ser Trp Trp Thr Asp Glu Asp Arg Ser Ala Phe Glu Gln Leu Thr
    370                 375                 380

Ser Arg Leu Val Thr Gln Phe Ser Gly Leu Val Pro Ala Val Leu Thr
385                 390                 395                 400

Ser Glu Gly Ile Asp Thr Asp Gly Val Asn Gly Glu Phe Thr Leu Gly
                405                 410                 415

Glu Asn Ile Gly Asp Leu Gly Gly Leu Gly Ile Ala Val Val Ala Tyr
            420                 425                 430

Glu Lys Tyr Leu Ala Asp Arg Gly Gln Thr Phe Glu Thr Ser Pro Val
        435                 440                 445

Gln Lys Phe Glu Ala Glu Gly Ala Glu Glu Gly Leu Ala Glu Gln Glu
    450                 455                 460

Phe Asn Gly Leu Gln Arg Leu Phe Leu Ser Trp Ala Arg Val Trp Arg
465                 470                 475                 480

Thr Lys Ile Arg Pro Gln Met Ala Val Gln Tyr Leu Ala Ile Asp Pro
                485                 490                 495

His Ser Pro Ala Glu Phe Arg Cys Asn Val Ile Ala Gly Asn Val Ala
            500                 505                 510

Glu Phe Tyr Glu Ala Phe Asp Val Pro Glu Asp Ala Pro Val Tyr Ile
        515                 520                 525

Lys Pro Glu Glu Arg Leu Ala Ile Trp
    530                 535

<210> 47
<211> 426
<212> DNA
<213> Corynebacterium glutamicum

<220>
<221> CDS
<222> (101)..(403)
<223> FRXA01869

<400> 47
tgacaggcta ccttctgggg tggacatgat ccccaacgct caacccactt gtggcaccaa 60

ccacaaaccc tgtggcggta aatcccctag agtaggccac atg aag gat ctt tat 115
Met Lys Asp Leu Tyr
1               5

cgc ttt gtc aat ggc ctg tgg ctt gac acc cac atc att ccc gac gat 163
Arg Phe Val Asn Gly Leu Trp Leu Asp Thr His Ile Ile Pro Asp Asp
                10                  15                  20

cgc gcg gtg gac ggc acg ttc cac aag ctg cgc gat gat gct gaa gaa 211
Arg Ala Val Asp Gly Thr Phe His Lys Leu Arg Asp Asp Ala Glu Glu
            25                  30                  35

gac gtc cat gag atc gtc aag gaa gac act gga cgc gca ggc aca ctt 259
Asp Val His Glu Ile Val Lys Glu Asp Thr Gly Arg Ala Gly Thr Leu
        40                  45                  50

tat gcc tca ttt atg gat act gac gcc atc aac gct gct ggt gtt gca 307
Tyr Ala Ser Phe Met Asp Thr Asp Ala Ile Asn Ala Ala Gly Val Ala
    55                  60                  65

ccg ctc gat gcg gat ctg aac agg ctg tct gtt gct aac tca tcg ttt 355
Pro Leu Asp Ala Asp Leu Asn Arg Leu Ser Val Ala Asn Ser Ser Phe
70                  75                  80                  85

tcg cag ctg ctc tcg gcg aac tgg acc gtg aag gcg ttg gcg cgc cag 403
Ser Gln Leu Leu Ser Ala Asn Trp Thr Val Lys Ala Leu Ala Arg Gln
                90                  95                  100

taggtttctg ggtggagaag gat 426


<210> 48
<211> 101
<212> PRT
<213> Corynebacterium glutamicum

<400> 48
Met Lys Asp Leu Tyr Arg Phe Val Asn Gly Leu Trp Leu Asp Thr His
1               5                   10                  15

Ile Ile Pro Asp Asp Arg Ala Val Asp Gly Thr Phe His Lys Leu Arg
            20                  25                  30

Asp Asp Ala Glu Glu Asp Val His Glu Ile Val Lys Glu Asp Thr Gly
        35                  40                  45

Arg Ala Gly Thr Leu Tyr Ala Ser Phe Met Asp Thr Asp Ala Ile Asn
    50                  55                  60

Ala Ala Gly Val Ala Pro Leu Asp Ala Asp Leu Asn Arg Leu Ser Val
65                  70                  75                  80

Ala Asn Ser Ser Phe Ser Gln Leu Leu Ser Ala Asn Trp Thr Val Lys
                85                  90                  95

Ala Leu Ala Arg Gln
            100


<210> 49
<211> 2898
<212> DNA

<213> Corynebacterium glutamicum

<220>
<221> CDS
<222> (101)..(2875)
<223> RXN03028

<400> 49
   ctaattccaa gccgagctga aaaagtctgg aagttttgcc caataagggc gttaaagtgg 60
   gtgaaagcga atttagaaat aaagaattaa ggggagagac atg ttc gag agg ttt 115
   Met Phe Glu Arg Phe
   1 5
   acc gat cgt gca cgc cgc gtg att gtg ctc gcg cag gaa gag gcg cgc 163
   Thr Asp Arg Ala Arg Arg Val Ile Val Leu Ala Gln Glu Glu Ala Arg
   10 15 20
   atg ctc aac cac aat tac atc ggc acg gag cac att ctc ctc ggc ctc 211
   Met Leu Asn His Asn Tyr Ile Gly Thr Glu His Ile Leu Leu Gly Leu
   25 30 35
   att cac gag ggc gag ggc gtt gca gcc aag gct ttg gaa tcc atg gga 259
   Ile His Glu Gly Glu Gly Val Ala Ala Lys Ala Leu Glu Ser Met Gly
   40 45 50
   att tcc ctg gac gcc gtc cgc cag gaa gtc gaa gag att atc ggc cag 307
   Ile Ser Leu Asp Ala Val Arg Gln Glu Val Glu Glu Ile Ile Gly Gln
   55 60 65
   ggc tcc cag ccc acc acc ggc cac att cct ttt act cca cgt gcc aag 355
   Gly Ser Gln Pro Thr Thr Gly His Ile Pro Phe Thr Pro Arg Ala Lys
   70 75 80 85
   aag gtc ctg gag ctc agc ctc cgc gaa ggc cta caa atg gga cac aag 403
   Lys Val Leu Glu Leu Ser Leu Arg Glu Gly Leu Gln Met Gly His Lys
   90 95 100
   tac atc ggt act gag ttc ctg ctt ctc ggt ttg atc cgt gag ggc gag 451
   Tyr Ile Gly Thr Glu Phe Leu Leu Leu Gly Leu Ile Arg Glu Gly Glu
   105 110 115
   ggc gtt gct gcc cag gtc ctg gtc aag ctt ggt gct gat ctg cca cgc 499
   Gly Val Ala Ala Gln Val Leu Val Lys Leu Gly Ala Asp Leu Pro Arg
   120 125 130
   gtg cgt cag caa gtt att cag ctt ctc tcc ggc tac gaa ggt ggc cag 547
   Val Arg Gln Gln Val Ile Gln Leu Leu Ser Gly Tyr Glu Gly Gly Gln
   135 140 145
   ggc gga tcc cca gag ggc ggc cag ggc gcc cct act ggc ggt gac gct 595
   Gly Gly Ser Pro Glu Gly Gly Gln Gly Ala Pro Thr Gly Gly Asp Ala
   150 155 160 165
   gtt ggt gca gga gct gct cct ggc ggt cgt cca tct tcg ggc agc cca 643
   Val Gly Ala Gly Ala Ala Pro Gly Gly Arg Pro Ser Ser Gly Ser Pro
   170 175 180
   ggc gag cgt tct acc tct ttg gtc ctt gac cag ttc ggc cgc aac ctc 691
   Gly Glu Arg Ser Thr Ser Leu Val Leu Asp Gln Phe Gly Arg Asn Leu
   185 190 195
   acc cag gct gca aag gac ggc aag ctg gat cca gtt gtt ggt cgc gat 739
   Thr Gln Ala Ala Lys Asp Gly Lys Leu Asp Pro Val Val Gly Arg Asp
   200 205 210
   aag gaa atc gag cgc atc atg cag gtg ctc tcc cgt cgt acc aag aac 787
   Lys Glu Ile Glu Arg Ile Met Gln Val Leu Ser Arg Arg Thr Lys Asn
   215 220 225
   aac cca gtt ctt att ggt gag cca ggt gtt ggt aag acc gca gtt gtt 835
   Asn Pro Val Leu Ile Gly Glu Pro Gly Val Gly Lys Thr Ala Val Val
   230 235 240 245
   gaa ggt ctt gca cta gac att gtt aac ggc aag gtt cca gag acc ctc 883
   Glu Gly Leu Ala Leu Asp Ile Val Asn Gly Lys Val Pro Glu Thr Leu
   250 255 260
   aag gac aag cag gtt tac tcc ctt gac tta ggt tcc ctg gtt gca ggt 931
   Lys Asp Lys Gln Val Tyr Ser Leu Asp Leu Gly Ser Leu Val Ala Gly
   265 270 275
   tcc cgt tac cgc ggt gac ttc gaa gag cga ctg aag aag gtc ctc aag 979
   Ser Arg Tyr Arg Gly Asp Phe Glu Glu Arg Leu Lys Lys Val Leu Lys
   280 285 290
   gag att aac cag cgc ggc gac atc atc ctg ttt atc gat gag atc cac 1027
   Glu Ile Asn Gln Arg Gly Asp Ile Ile Leu Phe Ile Asp Glu Ile His
   295 300 305
   acc ctc gtg ggt gca ggt gca gca gaa ggc gca atc gac gct gcc tcc 1075
   Thr Leu Val Gly Ala Gly Ala Ala Glu Gly Ala Ile Asp Ala Ala Ser
   310 315 320 325
   ctg ctt aag cca aag ctt gcc cgc ggt gaa ctg cag acc att ggt gca 1123
   Leu Leu Lys Pro Lys Leu Ala Arg Gly Glu Leu Gln Thr Ile Gly Ala
   330 335 340
   acc acc ctg gat gag tac cgt aag cac att gaa aag gac gca gct ctt 1171
   Thr Thr Leu Asp Glu Tyr Arg Lys His Ile Glu Lys Asp Ala Ala Leu
   345 350 355
   gag cgt cgt ttc cag cca gtg cag gtt cca gag cct tcg gtt gat ctc 1219
   Glu Arg Arg Phe Gln Pro Val Gln Val Pro Glu Pro Ser Val Asp Leu
   360 365 370
   acc gtt gag atc ttg aag ggt ctg cgc gac cgc tac gaa gct cac cac 1267
   Thr Val Glu Ile Leu Lys Gly Leu Arg Asp Arg Tyr Glu Ala His His
   375 380 385
   cgc gta tcc atc acc gat ggt gct ctt act gca gca gct cag ctt gct 1315
   Arg Val Ser Ile Thr Asp Gly Ala Leu Thr Ala Ala Ala Gln Leu Ala
   390 395 400 405
   gat cgc tac atc aac gac cgc ttc ttg cca gat aag gcc gtt gac ctc 1363
   Asp Arg Tyr Ile Asn Asp Arg Phe Leu Pro Asp Lys Ala Val Asp Leu
   410 415 420
   atc gat gag gct ggc gcc cgc atg cgc atc aag cgc atg acc gca cct 1411
   Ile Asp Glu Ala Gly Ala Arg Met Arg Ile Lys Arg Met Thr Ala Pro
   425 430 435
   tcc tcc ctc cgc gag gtt gat gag cgt atc gct gat gtt cgc cgt gag 1459
   Ser Ser Leu Arg Glu Val Asp Glu Arg Ile Ala Asp Val Arg Arg Glu
   440 445 450
   aag gaa gca gcg atc gat gct cag gac ttt gag aag gca gca ggt ctt 1507
   Lys Glu Ala Ala Ile Asp Ala Gln Asp Phe Glu Lys Ala Ala Gly Leu
   455 460 465
   cgc gat aag gag cgc aag ctc ggc gaa gag cgt tca gag aag gaa aag 1555
   Arg Asp Lys Glu Arg Lys Leu Gly Glu Glu Arg Ser Glu Lys Glu Lys
   470 475 480 485
   cag tgg cgc tcc ggc gac ctc gag gac atc gct gag gtt ggc gaa gag 1603
   Gln Trp Arg Ser Gly Asp Leu Glu Asp Ile Ala Glu Val Gly Glu Glu
   490 495 500
   cag atc gca gaa gta ctg gcc aac tgg act ggt att cct gtc ttc aag 1651
   Gln Ile Ala Glu Val Leu Ala Asn Trp Thr Gly Ile Pro Val Phe Lys
   505 510 515
   ctc acc gaa gct gaa tct tca cgc ctg ctc aac atg gaa gaa gag ttg 1699
   Leu Thr Glu Ala Glu Ser Ser Arg Leu Leu Asn Met Glu Glu Glu Leu
   520 525 530
   cac aag cgc atc atc gga cag gat gaa gct gtc aag gct gtc tcc cgt 1747
   His Lys Arg Ile Ile Gly Gln Asp Glu Ala Val Lys Ala Val Ser Arg
   535 540 545
   gcg atc cgt cgt acc cgt gca ggt ctg aag gat cct aag cgt cct tcc 1795
   Ala Ile Arg Arg Thr Arg Ala Gly Leu Lys Asp Pro Lys Arg Pro Ser
   550 555 560 565
   ggc tcc ttc atc ttc gct ggt cca tcc ggc gtt ggt aag acc gag ctg     1843
   Gly Ser Phe Ile Phe Ala Gly Pro Ser Gly Val Gly Lys Thr Glu Leu
                   570                 575                 580
   tcc aag gct ctc gca gga ttc ctc ttc ggt gac gat gat tcc ctc atc     1891
   Ser Lys Ala Leu Ala Gly Phe Leu Phe Gly Asp Asp Asp Ser Leu Ile
               585                 590                 595
   caa atc gac atg ggt gag ttc cac gac cgc ttc acc gcg tcc cga ctt     1939
   Gln Ile Asp Met Gly Glu Phe His Asp Arg Phe Thr Ala Ser Arg Leu
           600                 605                 610
   ttc ggt gcc cct ccg gga tac gtt ggc tac gaa gaa ggt ggc cag ctg     1987
   Phe Gly Ala Pro Pro Gly Tyr Val Gly Tyr Glu Glu Gly Gly Gln Leu
       615                 620                 625
   acc gag aag gtt cgc cgt aag cca ttc tcc gtt gtg ctt ttc gac gaa     2035
   Thr Glu Lys Val Arg Arg Lys Pro Phe Ser Val Val Leu Phe Asp Glu
   630                 635                 640                 645
   atc gag aag gcc cac aag gag atc tac aac acc ttg ctg cag gtg ttg     2083
   Ile Glu Lys Ala His Lys Glu Ile Tyr Asn Thr Leu Leu Gln Val Leu
                   650                 655                 660
   gaa gat ggt cgc ctt acc gat ggt cag gga cgc atc gtg gac ttc aag     2131
   Glu Asp Gly Arg Leu Thr Asp Gly Gln Gly Arg Ile Val Asp Phe Lys
               665                 670                 675
   aac acc gtc ctg atc ttc acc tcc aac ctg ggc acc gct gac atc tcc     2179
   Asn Thr Val Leu Ile Phe Thr Ser Asn Leu Gly Thr Ala Asp Ile Ser
           680                 685                 690
   aag gct gtt ggc ctg ggc ttc tcc gga tcc tcc gag act gac agc gat     2227
   Lys Ala Val Gly Leu Gly Phe Ser Gly Ser Ser Glu Thr Asp Ser Asp
       695                 700                 705
   gct cag tac gac cgc atg aag aac aag gtc cac gac gag ctg aag aag     2275
   Ala Gln Tyr Asp Arg Met Lys Asn Lys Val His Asp Glu Leu Lys Lys
   710                 715                 720                 725
   cac ttc cgc cct gag ttc ctg aac cgt att gat gag atc gtg gtc ttc     2323
   His Phe Arg Pro Glu Phe Leu Asn Arg Ile Asp Glu Ile Val Val Phe
                   730                 735                 740
   cac cag ctc acc aag gat cag atc gtt cag atg gtc gac ctt ctt atc     2371
   His Gln Leu Thr Lys Asp Gln Ile Val Gln Met Val Asp Leu Leu Ile
               745                 750                 755
   ggt cgc gtt tcc aac gca ctg gct gag aag gac atg agc atc gaa ctg     2419
   Gly Arg Val Ser Asn Ala Leu Ala Glu Lys Asp Met Ser Ile Glu Leu
           760                 765                 770
   act gag aag gcc aag gac ctc ctg gct aac cga ggc ttc gat cca gtt     2467
   Thr Glu Lys Ala Lys Asp Leu Leu Ala Asn Arg Gly Phe Asp Pro Val
       775                 780                 785
   ctg ggt gca cga cca ttg cgt cgc acc atc cag cgc gaa att gaa gac     2515
   Leu Gly Ala Arg Pro Leu Arg Arg Thr Ile Gln Arg Glu Ile Glu Asp
   790                 795                 800                 805
   cag atg tcc gag aag atc ctc ttc ggt gaa atc ggc gca ggc gag atc     2563
   Gln Met Ser Glu Lys Ile Leu Phe Gly Glu Ile Gly Ala Gly Glu Ile
                   810                 815                 820
   gtc acc gtt gac gtc gaa ggc tgg gac ggc gag tcc aag gac acc gac     2611
   Val Thr Val Asp Val Glu Gly Trp Asp Gly Glu Ser Lys Asp Thr Asp
               825                 830                 835
   cgt gcg aag ttc acc ttc aca cca cgt cca aag cca atg cca gaa ggt     2659
   Arg Ala Lys Phe Thr Phe Thr Pro Arg Pro Lys Pro Met Pro Glu Gly
           840                 845                 850
   aag ttc tct gag atc tct gtc gag gct gcg gaa gca att caa gat gta     2707
   Lys Phe Ser Glu Ile Ser Val Glu Ala Ala Glu Ala Ile Gln Asp Val
       855                 860                 865
   gat tct gca gct gac ggc gat gtc cca gaa acc gat tca ctt tcc gac     2755
   Asp Ser Ala Ala Asp Gly Asp Val Pro Glu Thr Asp Ser Leu Ser Asp
   870                 875                 880                 885
   att gac ctt gaa acc ctt gaa aag ttt gag gaa gat gta gaa aac ggc     2803
   Ile Asp Leu Glu Thr Leu Glu Lys Phe Glu Glu Asp Val Glu Asn Gly
                   890                 895                 900
   acc gac att gat cag gtg tcc ggt gac tac tac ggc acc gat gat cag     2851
   Thr Asp Ile Asp Gln Val Ser Gly Asp Tyr Tyr Gly Thr Asp Asp Gln
               905                 910                 915
   gga ggc act gct cca agc aag gag tagcaacctt ttgaaaaagg gcc           2898
   Gly Gly Thr Ala Pro Ser Lys Glu
           920                 925

<210> 50
<211> 925
<212> PRT
<213> Corynebacterium glutamicum

<400> 50
Met Phe Glu Arg Phe Thr Asp Arg Ala Arg Arg Val Ile Val Leu Ala
  1               5                  10                  15

Gln Glu Glu Ala Arg Met Leu Asn His Asn Tyr Ile Gly Thr Glu His
             20                  25                  30

Ile Leu Leu Gly Leu Ile His Glu Gly Glu Gly Val Ala Ala Lys Ala
         35                  40                  45

Leu Glu Ser Met Gly Ile Ser Leu Asp Ala Val Arg Gln Glu Val Glu
     50                  55                  60

Glu Ile Ile Gly Gln Gly Ser Gln Pro Thr Thr Gly His Ile Pro Phe
 65                  70                  75                  80

Thr Pro Arg Ala Lys Lys Val Leu Glu Leu Ser Leu Arg Glu Gly Leu
                 85                  90                  95

Gln Met Gly His Lys Tyr Ile Gly Thr Glu Phe Leu Leu Leu Gly Leu
            100                 105                 110

Ile Arg Glu Gly Glu Gly Val Ala Ala Gln Val Leu Val Lys Leu Gly
        115                 120                 125

Ala Asp Leu Pro Arg Val Arg Gln Gln Val Ile Gln Leu Leu Ser Gly
    130                 135                 140

Tyr Glu Gly Gly Gln Gly Gly Ser Pro Glu Gly Gly Gln Gly Ala Pro
145                 150                 155                 160

Thr Gly Gly Asp Ala Val Gly Ala Gly Ala Ala Pro Gly Gly Arg Pro
                165                 170                 175

Ser Ser Gly Ser Pro Gly Glu Arg Ser Thr Ser Leu Val Leu Asp Gln
            180                 185                 190

Phe Gly Arg Asn Leu Thr Gln Ala Ala Lys Asp Gly Lys Leu Asp Pro
        195                 200                 205

Val Val Gly Arg Asp Lys Glu Ile Glu Arg Ile Met Gln Val Leu Ser
    210                 215                 220

Arg Arg Thr Lys Asn Asn Pro Val Leu Ile Gly Glu Pro Gly Val Gly
225                 230                 235                 240

Lys Thr Ala Val Val Glu Gly Leu Ala Leu Asp Ile Val Asn Gly Lys
                245                 250                 255

Val Pro Glu Thr Leu Lys Asp Lys Gln Val Tyr Ser Leu Asp Leu Gly
            260                 265                 270

Ser Leu Val Ala Gly Ser Arg Tyr Arg Gly Asp Phe Glu Glu Arg Leu
        275                 280                 285

Lys Lys Val Leu Lys Glu Ile Asn Gln Arg Gly Asp Ile Ile Leu Phe

290                 295                 300

Ile Asp Glu Ile His Thr Leu Val Gly Ala Gly Ala Ala Glu Gly Ala
305                 310                 315                 320

Ile Asp Ala Ala Ser Leu Leu Lys Pro Lys Leu Ala Arg Gly Glu Leu
                325                 330                 335

Gln Thr Ile Gly Ala Thr Thr Leu Asp Glu Tyr Arg Lys His Ile Glu
            340                 345                 350

Lys Asp Ala Ala Leu Glu Arg Arg Phe Gln Pro Val Gln Val Pro Glu
        355                 360                 365

Pro Ser Val Asp Leu Thr Val Glu Ile Leu Lys Gly Leu Arg Asp Arg
    370                 375                 380

Tyr Glu Ala His His Arg Val Ser Ile Thr Asp Gly Ala Leu Thr Ala
385                 390                 395                 400

Ala Ala Gln Leu Ala Asp Arg Tyr Ile Asn Asp Arg Phe Leu Pro Asp
                405                 410                 415

Lys Ala Val Asp Leu Ile Asp Glu Ala Gly Ala Arg Met Arg Ile Lys
            420                 425                 430

Arg Met Thr Ala Pro Ser Ser Leu Arg Glu Val Asp Glu Arg Ile Ala
        435                 440                 445

Asp Val Arg Arg Glu Lys Glu Ala Ala Ile Asp Ala Gln Asp Phe Glu
    450                 455                 460

Lys Ala Ala Gly Leu Arg Asp Lys Glu Arg Lys Leu Gly Glu Glu Arg
465                 470                 475                 480

Ser Glu Lys Glu Lys Gln Trp Arg Ser Gly Asp Leu Glu Asp Ile Ala
                485                 490                 495

Glu Val Gly Glu Glu Gln Ile Ala Glu Val Leu Ala Asn Trp Thr Gly
            500                 505                 510

Ile Pro Val Phe Lys Leu Thr Glu Ala Glu Ser Ser Arg Leu Leu Asn
        515                 520                 525

Met Glu Glu Glu Leu His Lys Arg Ile Ile Gly Gln Asp Glu Ala Val
    530                 535                 540

Lys Ala Val Ser Arg Ala Ile Arg Arg Thr Arg Ala Gly Leu Lys Asp
545                 550                 555                 560

Pro Lys Arg Pro Ser Gly Ser Phe Ile Phe Ala Gly Pro Ser Gly Val
                565                 570                 575

Gly Lys Thr Glu Leu Ser Lys Ala Leu Ala Gly Phe Leu Phe Gly Asp
            580                 585                 590

Asp Asp Ser Leu Ile Gln Ile Asp Met Gly Glu Phe His Asp Arg Phe
        595                 600                 605

Thr Ala Ser Arg Leu Phe Gly Ala Pro Pro Gly Tyr Val Gly Tyr Glu
    610                 615                 620

Glu Gly Gly Gln Leu Thr Glu Lys Val Arg Arg Lys Pro Phe Ser Val
625                 630                 635                 640

Val Leu Phe Asp Glu Ile Glu Lys Ala His Lys Glu Ile Tyr Asn Thr
                645                 650                 655

Leu Leu Gln Val Leu Glu Asp Gly Arg Leu Thr Asp Gly Gln Gly Arg
            660                 665                 670

Ile Val Asp Phe Lys Asn Thr Val Leu Ile Phe Thr Ser Asn Leu Gly
        675                 680                 685

Thr Ala Asp Ile Ser Lys Ala Val Gly Leu Gly Phe Ser Gly Ser Ser
    690                 695                 700

Glu Thr Asp Ser Asp Ala Gln Tyr Asp Arg Met Lys Asn Lys Val His
705                 710                 715                 720

Asp Glu Leu Lys Lys His Phe Arg Pro Glu Phe Leu Asn Arg Ile Asp
                725                 730                 735

Glu Ile Val Val Phe His Gln Leu Thr Lys Asp Gln Ile Val Gln Met
            740                 745                 750

Val Asp Leu Leu Ile Gly Arg Val Ser Asn Ala Leu Ala Glu Lys Asp
        755                 760                 765

Met Ser Ile Glu Leu Thr Glu Lys Ala Lys Asp Leu Leu Ala Asn Arg
    770                 775                 780

Gly Phe Asp Pro Val Leu Gly Ala Arg Pro Leu Arg Arg Thr Ile Gln
785                 790                 795                 800

Arg Glu Ile Glu Asp Gln Met Ser Glu Lys Ile Leu Phe Gly Glu Ile
                805                 810                 815

Gly Ala Gly Glu Ile Val Thr Val Asp Val Glu Gly Trp Asp Gly Glu
            820                 825                 830

Ser Lys Asp Thr Asp Arg Ala Lys Phe Thr Phe Thr Pro Arg Pro Lys
        835                 840                 845

Pro Met Pro Glu Gly Lys Phe Ser Glu Ile Ser Val Glu Ala Ala Glu
    850                 855                 860

Ala Ile Gln Asp Val Asp Ser Ala Ala Asp Gly Asp Val Pro Glu Thr
865                 870                 875                 880

Asp Ser Leu Ser Asp Ile Asp Leu Glu Thr Leu Glu Lys Phe Glu Glu
                885                 890                 895

Asp Val Glu Asn Gly Thr Asp Ile Asp Gln Val Ser Gly Asp Tyr Tyr
            900                 905                 910

Gly Thr Asp Asp Gln Gly Gly Thr Ala Pro Ser Lys Glu
        915                 920                 925


<210> 51
<211> 1104
<212> DNA
<213> Corynebacterium glutamicum

<220>
<221> CDS
<222> (101)..(1081)
<223> FRXA02470

<400> 51
ctaattccaa gccgagctga aaaagtctgg aagttttgcc caataagggc gttaaagtgg 60

gtgaaagcga atttagaaat aaagaattaa ggggagagac atg ttc gag agg ttt 115
                                           Met Phe Glu Arg Phe
                                           1               5

acc gat cgt gca cgc cgc gtg att gtg ctc gcg cag gaa gag gcg cgc 163
Thr Asp Arg Ala Arg Arg Val Ile Val Leu Ala Gln Glu Glu Ala Arg
                10                  15                  20

atg ctc aac cac aat tac atc ggc acg gag cac att ctc ctc ggc ctc 211
Met Leu Asn His Asn Tyr Ile Gly Thr Glu His Ile Leu Leu Gly Leu
            25                  30                  35

att cac gag ggc gag ggc gtt gca gcc aag gct ttg gaa tcc atg gga 259
Ile His Glu Gly Glu Gly Val Ala Ala Lys Ala Leu Glu Ser Met Gly
        40                  45                  50

att tcc ctg gac gcc gtc cgc cag gaa gtc gaa gag att atc ggc cag 307
Ile Ser Leu Asp Ala Val Arg Gln Glu Val Glu Glu Ile Ile Gly Gln
    55                  60                  65

ggc tcc cag ccc acc acc ggc cac att cct ttt act cca cgt gcc aag 355

Gly Ser Gln Pro Thr Thr Gly His Ile Pro Phe Thr Pro Arg Ala Lys
 70                  75                  80                  85

aag gtc ctg gag ctc agc ctc cgc gaa ggc cta caa atg gga cac aag     403
Lys Val Leu Glu Leu Ser Leu Arg Glu Gly Leu Gln Met Gly His Lys
                90                  95                  100

tac atc ggt act gag ttc ctg ctt ctc ggt ttg atc cgt gag ggc gag     451
Tyr Ile Gly Thr Glu Phe Leu Leu Leu Gly Leu Ile Arg Glu Gly Glu
            105                 110                 115

ggc gtt gct gcc cag gtc ctg gtc aag ctt ggt gct gat ctg cca cgc     499
Gly Val Ala Ala Gln Val Leu Val Lys Leu Gly Ala Asp Leu Pro Arg
        120                 125                 130

gtg cgt cag caa gtt att cag ctt ctc tcc ggc tac gaa ggt ggc cag     547
Val Arg Gln Gln Val Ile Gln Leu Leu Ser Gly Tyr Glu Gly Gly Gln
    135                 140                 145

ggc gga tcc cca gag ggc ggc cag ggc gcc cct act ggc ggt gac gct     595
Gly Gly Ser Pro Glu Gly Gly Gln Gly Ala Pro Thr Gly Gly Asp Ala
150                 155                 160                 165

gtt ggt gca gga gct gct cct ggc ggt cgt cca tct tcg ggc agc cca     643
Val Gly Ala Gly Ala Ala Pro Gly Gly Arg Pro Ser Ser Gly Ser Pro
                170                 175                 180

ggc gag cgt tct acc tct ttg gtc ctt gac cag ttc ggc cgc aac ctc     691
Gly Glu Arg Ser Thr Ser Leu Val Leu Asp Gln Phe Gly Arg Asn Leu
            185                 190                 195

acc cag gct gca aag gac ggc aag ctg gat cca gtt gtt ggt cgc gat     739
Thr Gln Ala Ala Lys Asp Gly Lys Leu Asp Pro Val Val Gly Arg Asp
        200                 205                 210

aag gaa atc gag cgc atc atg cag gtg ctc tcc cgt cgt acc aag aac     787
Lys Glu Ile Glu Arg Ile Met Gln Val Leu Ser Arg Arg Thr Lys Asn
    215                 220                 225

aac cca gtt ctt att ggt gag cca ggt gtt ggt aag acc gca gtt gtt     835
Asn Pro Val Leu Ile Gly Glu Pro Gly Val Gly Lys Thr Ala Val Val
230                 235                 240                 245

gaa ggt ctt gca cta gac att gtt aac ggc aag gtt cca gag acc ctc     883
Glu Gly Leu Ala Leu Asp Ile Val Asn Gly Lys Val Pro Glu Thr Leu
                250                 255                 260

aag gac aag cag gtt tac tcc ctt gac tta ggt tcc ctg gtt gca ggt     931
Lys Asp Lys Gln Val Tyr Ser Leu Asp Leu Gly Ser Leu Val Ala Gly
            265                 270                 275

tcc cgt tac cgc ggt gac ttc gaa gag cga ctg aag aag gtc ctc aag     979
Ser Arg Tyr Arg Gly Asp Phe Glu Glu Arg Leu Lys Lys Val Leu Lys
        280                 285                 290

gag att aac cag cgc ggc gac atc atc ctg ttt atc gat gag atc cac     1027
Glu Ile Asn Gln Arg Gly Asp Ile Ile Leu Phe Ile Asp Glu Ile His
    295                 300                 305

acc ctc gtg ggt gca ggt gca gca cga agg cgc aat cga cgc tgc ctc     1075
Thr Leu Val Gly Ala Gly Ala Ala Arg Arg Arg Asn Arg Arg Cys Leu
310                 315                 320                 325

cct gct taagccaaag cttgcccgcg gtg                                   1104
Pro Ala

<210> 52
<211> 327
<212> PRT
<213> Corynebacterium glutamicum

<400> 52
Met Phe Glu Arg Phe Thr Asp Arg Ala Arg Arg Val Ile Val Leu Ala
1               5                   10                  15

Gln Glu Glu Ala Arg Met Leu Asn His Asn Tyr Ile Gly Thr Glu His
            20                  25                  30

Ile Leu Leu Gly Leu Ile His Glu Gly Glu Gly Val Ala Ala Lys Ala
        35                  40                  45

Leu Glu Ser Met Gly Ile Ser Leu Asp Ala Val Arg Gln Glu Val Glu
    50                  55                  60

Glu Ile Ile Gly Gln Gly Ser Gln Pro Thr Thr Gly His Ile Pro Phe
65                  70                  75                  80

Thr Pro Arg Ala Lys Lys Val Leu Glu Leu Ser Leu Arg Glu Gly Leu
                85                  90                  95

Gln Met Gly His Lys Tyr Ile Gly Thr Glu Phe Leu Leu Leu Gly Leu
            100                 105                 110

Ile Arg Glu Gly Glu Gly Val Ala Ala Gln Val Leu Val Lys Leu Gly
        115                 120                 125

Ala Asp Leu Pro Arg Val Arg Gln Gln Val Ile Gln Leu Leu Ser Gly
    130                 135                 140

Tyr Glu Gly Gly Gln Gly Gly Ser Pro Glu Gly Gly Gln Gly Ala Pro
145                 150                 155                 160

Thr Gly Gly Asp Ala Val Gly Ala Gly Ala Ala Pro Gly Gly Arg Pro
                165                 170                 175

Ser Ser Gly Ser Pro Gly Glu Arg Ser Thr Ser Leu Val Leu Asp Gln
            180                 185                 190

Phe Gly Arg Asn Leu Thr Gln Ala Ala Lys Asp Gly Lys Leu Asp Pro
        195                 200                 205

Val Val Gly Arg Asp Lys Glu Ile Glu Arg Ile Met Gln Val Leu Ser
    210                 215                 220

Arg Arg Thr Lys Asn Asn Pro Val Leu Ile Gly Glu Pro Gly Val Gly
225                 230                 235                 240

Lys Thr Ala Val Val Glu Gly Leu Ala Leu Asp Ile Val Asn Gly Lys
                245                 250                 255

Val Pro Glu Thr Leu Lys Asp Lys Gln Val Tyr Ser Leu Asp Leu Gly
            260                 265                 270

Ser Leu Val Ala Gly Ser Arg Tyr Arg Gly Asp Phe Glu Glu Arg Leu
        275                 280                 285

Lys Lys Val Leu Lys Glu Ile Asn Gln Arg Gly Asp Ile Ile Leu Phe
    290                 295                 300

Ile Asp Glu Ile His Thr Leu Val Gly Ala Gly Ala Ala Arg Arg Arg
305                 310                 315                 320

Asn Arg Arg Cys Leu Pro Ala
                325


<210> 53
<211> 1956
<212> DNA
<213> Corynebacterium glutamicum

<220>
<221> CDS
<222> (101)..(1933)
<223> FRXA02471

<400> 53
ggtgacttcg aagagcgact gaagaaggtc ctcaaggaga ttaaccagcg cggcgacatc 60

atcctgttta tcgatgagat ccacaccctc gtgggtgcag gtg cag cac gaa ggc   115
                                           Val Gln His Glu Gly
                                           1               5

gca atc gac gct gcc tcc ctg ctt aag cca aag ctt gcc cgc ggt gaa     163
Ala Ile Asp Ala Ala Ser Leu Leu Lys Pro Lys Leu Ala Arg Gly Glu
                10                  15                  20

ctg cag acc att ggt gca acc acc ctg gat gag tac cgt aag cac att     211
Leu Gln Thr Ile Gly Ala Thr Thr Leu Asp Glu Tyr Arg Lys His Ile
            25                  30                  35

gaa aag gac gca gct ctt gag cgt cgt ttc cag cca gtg cag gtt cca     259
Glu Lys Asp Ala Ala Leu Glu Arg Arg Phe Gln Pro Val Gln Val Pro
        40                  45                  50

gag cct tcg gtt gat ctc acc gtt gag atc ttg aag ggt ctg cgc gac     307
Glu Pro Ser Val Asp Leu Thr Val Glu Ile Leu Lys Gly Leu Arg Asp
    55                  60                  65

cgc tac gaa gct cac cac cgc gta tcc atc acc gat ggt gct ctt act     355
Arg Tyr Glu Ala His His Arg Val Ser Ile Thr Asp Gly Ala Leu Thr
70                  75                  80                  85

gca gca gct cag ctt gct gat cgc tac atc aac gac cgc ttc ttg cca     403
Ala Ala Ala Gln Leu Ala Asp Arg Tyr Ile Asn Asp Arg Phe Leu Pro
                90                  95                  100

gat aag gcc gtt gac ctc atc gat gag gct ggc gcc cgc atg cgc atc     451
Asp Lys Ala Val Asp Leu Ile Asp Glu Ala Gly Ala Arg Met Arg Ile
            105                 110                 115

aag cgc atg acc gca cct tcc tcc ctc cgc gag gtt gat gag cgt atc     499
Lys Arg Met Thr Ala Pro Ser Ser Leu Arg Glu Val Asp Glu Arg Ile
        120                 125                 130

gct gat gtt cgc cgt gag aag gaa gca gcg atc gat gct cag gac ttt     547
Ala Asp Val Arg Arg Glu Lys Glu Ala Ala Ile Asp Ala Gln Asp Phe
    135                 140                 145

gag aag gca gca ggt ctt cgc gat aag gag cgc aag ctc ggc gaa gag     595
Glu Lys Ala Ala Gly Leu Arg Asp Lys Glu Arg Lys Leu Gly Glu Glu
150                 155                 160                 165

cgt tca gag aag gaa aag cag tgg cgc tcc ggc gac ctc gag gac atc     643
Arg Ser Glu Lys Glu Lys Gln Trp Arg Ser Gly Asp Leu Glu Asp Ile
                170                 175                 180

gct gag gtt ggc gaa gag cag atc gca gaa gta ctg gcc aac tgg act     691
Ala Glu Val Gly Glu Glu Gln Ile Ala Glu Val Leu Ala Asn Trp Thr
            185                 190                 195

ggt att cct gtc ttc aag ctc acc gaa gct gaa tct tca cgc ctg ctc     739
Gly Ile Pro Val Phe Lys Leu Thr Glu Ala Glu Ser Ser Arg Leu Leu
        200                 205                 210

aac atg gaa gaa gag ttg cac aag cgc atc atc gga cag gat gaa gct     787
Asn Met Glu Glu Glu Leu His Lys Arg Ile Ile Gly Gln Asp Glu Ala
    215                 220                 225

gtc aag gct gtc tcc cgt gcg atc cgt cgt acc cgt gca ggt ctg aag     835
Val Lys Ala Val Ser Arg Ala Ile Arg Arg Thr Arg Ala Gly Leu Lys
230                 235                 240                 245

gat cct aag cgt cct tcc ggc tcc ttc atc ttc gct ggt cca tcc ggc     883
Asp Pro Lys Arg Pro Ser Gly Ser Phe Ile Phe Ala Gly Pro Ser Gly
                250                 255                 260

gtt ggt aag acc gag ctg tcc aag gct ctc gca gga ttc ctc ttc ggt     931
Val Gly Lys Thr Glu Leu Ser Lys Ala Leu Ala Gly Phe Leu Phe Gly
            265                 270                 275

gac gat gat tcc ctc atc caa atc gac atg ggt gag ttc cac gac cgc     979
Asp Asp Asp Ser Leu Ile Gln Ile Asp Met Gly Glu Phe His Asp Arg
        280                 285                 290

ttc acc gcg tcc cga ctt ttc ggt gcc cct ccg gga tac gtt ggc tac     1027
Phe Thr Ala Ser Arg Leu Phe Gly Ala Pro Pro Gly Tyr Val Gly Tyr
    295                 300                 305

gaa gaa ggt ggc cag ctg acc gag aag gtt cgc cgt aag cca ttc tcc     1075
Glu Glu Gly Gly Gln Leu Thr Glu Lys Val Arg Arg Lys Pro Phe Ser
310                 315                 320                 325

gtt gtg ctt ttc gac gaa atc gag aag gcc cac aag gag atc tac aac     1123
Val Val Leu Phe Asp Glu Ile Glu Lys Ala His Lys Glu Ile Tyr Asn
                330                 335                 340

acc ttg ctg cag gtg ttg gaa gat ggt cgc ctt acc gat ggt cag gga     1171
Thr Leu Leu Gln Val Leu Glu Asp Gly Arg Leu Thr Asp Gly Gln Gly
            345                 350                 355

cgc atc gtg gac ttc aag aac acc gtc ctg atc ttc acc tcc aac ctg     1219
Arg Ile Val Asp Phe Lys Asn Thr Val Leu Ile Phe Thr Ser Asn Leu
        360                 365                 370

ggc acc gct gac atc tcc aag gct gtt ggc ctg ggc ttc tcc gga tcc     1267
Gly Thr Ala Asp Ile Ser Lys Ala Val Gly Leu Gly Phe Ser Gly Ser
    375                 380                 385

tcc gag act gac agc gat gct cag tac gac cgc atg aag aac aag gtc     1315
Ser Glu Thr Asp Ser Asp Ala Gln Tyr Asp Arg Met Lys Asn Lys Val
390                 395                 400                 405

cac gac gag ctg aag aag cac ttc cgc cct gag ttc ctg aac cgt att     1363
His Asp Glu Leu Lys Lys His Phe Arg Pro Glu Phe Leu Asn Arg Ile
                410                 415                 420

gat gag atc gtg gtc ttc cac cag ctc acc aag gat cag atc gtt cag     1411
Asp Glu Ile Val Val Phe His Gln Leu Thr Lys Asp Gln Ile Val Gln
            425                 430                 435

atg gtc gac ctt ctt atc ggt cgc gtt tcc aac gca ctg gct gag aag     1459
Met Val Asp Leu Leu Ile Gly Arg Val Ser Asn Ala Leu Ala Glu Lys
        440                 445                 450

gac atg agc atc gaa ctg act gag aag gcc aag gac ctc ctg gct aac     1507
Asp Met Ser Ile Glu Leu Thr Glu Lys Ala Lys Asp Leu Leu Ala Asn
    455                 460                 465

cga ggc ttc gat cca gtt ctg ggt gca cga cca ttg cgt cgc acc atc     1555
Arg Gly Phe Asp Pro Val Leu Gly Ala Arg Pro Leu Arg Arg Thr Ile
470                 475                 480                 485

cag cgc gaa att gaa gac cag atg tcc gag aag atc ctc ttc ggt gaa     1603
Gln Arg Glu Ile Glu Asp Gln Met Ser Glu Lys Ile Leu Phe Gly Glu
                490                 495                 500

atc ggc gca ggc gag atc gtc acc gtt gac gtc gaa ggc tgg gac ggc     1651
Ile Gly Ala Gly Glu Ile Val Thr Val Asp Val Glu Gly Trp Asp Gly
            505                 510                 515

gag tcc aag gac acc gac cgt gcg aag ttc acc ttc aca cca cgt cca     1699
Glu Ser Lys Asp Thr Asp Arg Ala Lys Phe Thr Phe Thr Pro Arg Pro
        520                 525                 530

aag cca atg cca gaa ggt aag ttc tct gag atc tct gtc gag gct gcg     1747
Lys Pro Met Pro Glu Gly Lys Phe Ser Glu Ile Ser Val Glu Ala Ala
    535                 540                 545

gaa gca att caa gat gta gat tct gca gct gac ggc gat gtc cca gaa     1795
Glu Ala Ile Gln Asp Val Asp Ser Ala Ala Asp Gly Asp Val Pro Glu
550                 555                 560                 565

acc gat tca ctt tcc gac att gac ctt gaa acc ctt gaa aag ttt gag     1843
Thr Asp Ser Leu Ser Asp Ile Asp Leu Glu Thr Leu Glu Lys Phe Glu
                570                 575                 580

gaa gat gta gaa aac ggc acc gac att gat cag gtg tcc ggt gac tac     1891
Glu Asp Val Glu Asn Gly Thr Asp Ile Asp Gln Val Ser Gly Asp Tyr
            585                 590                 595

tac ggc acc gat gat cag gga ggc act gct cca agc aag gag             1933
Tyr Gly Thr Asp Asp Gln Gly Gly Thr Ala Pro Ser Lys Glu
        600                 605                 610

tagcaacctt ttgaaaaagg gcc                                           1956

<210> 54
<211> 611
<212> PRT
<213> Corynebacterium glutamicum

<400> 54
Val Gln His Glu Gly Ala Ile Asp Ala Ala Ser Leu Leu Lys Pro Lys
1               5                   10                  15

Leu Ala Arg Gly Glu Leu Gln Thr Ile Gly Ala Thr Thr Leu Asp Glu
            20                  25                  30

Tyr Arg Lys His Ile Glu Lys Asp Ala Ala Leu Glu Arg Arg Phe Gln
        35                  40                  45

Pro Val Gln Val Pro Glu Pro Ser Val Asp Leu Thr Val Glu Ile Leu
    50                  55                  60

Lys Gly Leu Arg Asp Arg Tyr Glu Ala His His Arg Val Ser Ile Thr
65                  70                  75                  80

Asp Gly Ala Leu Thr Ala Ala Ala Gln Leu Ala Asp Arg Tyr Ile Asn
                85                  90                  95

Asp Arg Phe Leu Pro Asp Lys Ala Val Asp Leu Ile Asp Glu Ala Gly
            100                 105                 110

Ala Arg Met Arg Ile Lys Arg Met Thr Ala Pro Ser Ser Leu Arg Glu
        115                 120                 125

Val Asp Glu Arg Ile Ala Asp Val Arg Arg Glu Lys Glu Ala Ala Ile
    130                 135                 140

Asp Ala Gln Asp Phe Glu Lys Ala Ala Gly Leu Arg Asp Lys Glu Arg
145                 150                 155                 160

Lys Leu Gly Glu Glu Arg Ser Glu Lys Glu Lys Gln Trp Arg Ser Gly
                165                 170                 175

Asp Leu Glu Asp Ile Ala Glu Val Gly Glu Glu Gln Ile Ala Glu Val
            180                 185                 190

Leu Ala Asn Trp Thr Gly Ile Pro Val Phe Lys Leu Thr Glu Ala Glu
        195                 200                 205

Ser Ser Arg Leu Leu Asn Met Glu Glu Glu Leu His Lys Arg Ile Ile
    210                 215                 220

Gly Gln Asp Glu Ala Val Lys Ala Val Ser Arg Ala Ile Arg Arg Thr
225                 230                 235                 240

Arg Ala Gly Leu Lys Asp Pro Lys Arg Pro Ser Gly Ser Phe Ile Phe
                245                 250                 255

Ala Gly Pro Ser Gly Val Gly Lys Thr Glu Leu Ser Lys Ala Leu Ala
            260                 265                 270

Gly Phe Leu Phe Gly Asp Asp Asp Ser Leu Ile Gln Ile Asp Met Gly
        275                 280                 285

Glu Phe His Asp Arg Phe Thr Ala Ser Arg Leu Phe Gly Ala Pro Pro
    290                 295                 300

Gly Tyr Val Gly Tyr Glu Glu Gly Gly Gln Leu Thr Glu Lys Val Arg
305                 310                 315                 320

Arg Lys Pro Phe Ser Val Val Leu Phe Asp Glu Ile Glu Lys Ala His
                325                 330                 335

Lys Glu Ile Tyr Asn Thr Leu Leu Gln Val Leu Glu Asp Gly Arg Leu
            340                 345                 350

Thr Asp Gly Gln Gly Arg Ile Val Asp Phe Lys Asn Thr Val Leu Ile
        355                 360                 365

Phe Thr Ser Asn Leu Gly Thr Ala Asp Ile Ser Lys Ala Val Gly Leu
    370                 375                 380

Gly Phe Ser Gly Ser Ser Glu Thr Asp Ser Asp Ala Gln Tyr Asp Arg
385                 390                 395                 400

Met Lys Asn Lys Val His Asp Glu Leu Lys Lys His Phe Arg Pro Glu
                405                 410                 415

Phe Leu Asn Arg Ile Asp Glu Ile Val Val Phe His Gln Leu Thr Lys
            420                 425                 430

Asp Gln Ile Val Gln Met Val Asp Leu Leu Ile Gly Arg Val Ser Asn
        435                 440                 445

Ala Leu Ala Glu Lys Asp Met Ser Ile Glu Leu Thr Glu Lys Ala Lys
    450                 455                 460

Asp Leu Leu Ala Asn Arg Gly Phe Asp Pro Val Leu Gly Ala Arg Pro
465                 470                 475                 480

Leu Arg Arg Thr Ile Gln Arg Glu Ile Glu Asp Gln Met Ser Glu Lys
                485                 490                 495

Ile Leu Phe Gly Glu Ile Gly Ala Gly Glu Ile Val Thr Val Asp Val
            500                 505                 510

Glu Gly Trp Asp Gly Glu Ser Lys Asp Thr Asp Arg Ala Lys Phe Thr
        515                 520                 525

Phe Thr Pro Arg Pro Lys Pro Met Pro Glu Gly Lys Phe Ser Glu Ile
    530                 535                 540

Ser Val Glu Ala Ala Glu Ala Ile Gln Asp Val Asp Ser Ala Ala Asp
545                 550                 555                 560

Gly Asp Val Pro Glu Thr Asp Ser Leu Ser Asp Ile Asp Leu Glu Thr
                565                 570                 575

Leu Glu Lys Phe Glu Glu Asp Val Glu Asn Gly Thr Asp Ile Asp Gln
            580                 585                 590

Val Ser Gly Asp Tyr Tyr Gly Thr Asp Asp Gln Gly Gly Thr Ala Pro
        595                 600                 605

Ser Lys Glu
    610


<210> 55
<211> 1446
<212> DNA
<213> Corynebacterium glutamicum

<220>
<221> CDS
<222> (101)..(1423)
<223> RXA02630

<400> 55
gcgggttcga aaatgtcgat gattaaacca ctaaagagct cacaggaagt gttcagacta 60

cttagagtga cgccccagcc acagggttca taatcaaatc atg aca aat caa ttc    115
                                            Met Thr Asn Gln Phe
                                            1               5

ccc aca aac aac ggt gag aac ccg gac cgt gca tcg gaa act cca tca    163
Pro Thr Asn Asn Gly Glu Asn Pro Asp Arg Ala Ser Glu Thr Pro Ser
                10                  15                  20

gaa acc aac tcc ttc gaa cat gtg cgt agt tca tat ccg cag tgg ggt    211
Glu Thr Asn Ser Phe Glu His Val Arg Ser Ser Tyr Pro Gln Trp Gly
            25                  30                  35

aac act gct tcc aat caa aac ccc tat cct ggt gcg ggc ttc ggc tct    259
Asn Thr Ala Ser Asn Gln Asn Pro Tyr Pro Gly Ala Gly Phe Gly Ser
        40                  45                  50

gaa caa aac act caa caa gga aat gag caa caa gct cca gcc tgg acc    307
Glu Gln Asn Thr Gln Gln Gly Asn Glu Gln Gln Ala Pro Ala Trp Thr
    55                  60                  65

agt tgg gat aat cag cct cta agc aca gat gta aag cca gct aaa gaa    355
Ser Trp Asp Asn Gln Pro Leu Ser Thr Asp Val Lys Pro Ala Lys Glu
70                  75                  80                  85

aag cga aaa gtt ggc atc gga acg gca ctc gcg tta atg ctt gtt ggt    403
Lys Arg Lys Val Gly Ile Gly Thr Ala Leu Ala Leu Met Leu Val Gly
                90                  95                  100

tct att gct acc ggt agc gtt gtg ggt gtt gca gca acc cag ctt ggt    451
Ser Ile Ala Thr Gly Ser Val Val Gly Val Ala Ala Thr Gln Leu Gly
            105                 110                 115

tcg gac tct tca acc cca gtt aat gct ctt gag cag ccc agc gtg cag    499
Ser Asp Ser Ser Thr Pro Val Asn Ala Leu Glu Gln Pro Ser Val Gln
        120                 125                 130

cgc acc act aat gct gaa cca ggt tca gcg gaa cag gtt gct gcc gca    547
Arg Thr Thr Asn Ala Glu Pro Gly Ser Ala Glu Gln Val Ala Ala Ala
    135                 140                 145

gtt ttg cct tct gtc gtc tct att cag gcc att act agg acg tct gct    595
Val Leu Pro Ser Val Val Ser Ile Gln Ala Ile Thr Arg Thr Ser Ala
150                 155                 160                 165

tct gag ggc tct gga tcc att att tcc tct gat ggt tac gtc atg acc    643
Ser Glu Gly Ser Gly Ser Ile Ile Ser Ser Asp Gly Tyr Val Met Thr
                170                 175                 180

aat aat cac gtc gtg gca ggc att gaa caa tct ggt gtg tta gaa gta    691
Asn Asn His Val Val Ala Gly Ile Glu Gln Ser Gly Val Leu Glu Val
            185                 190                 195

agt ttc tcc gat gga act aca gcg caa gct gat ttt att gct ggt gat    739
Ser Phe Ser Asp Gly Thr Thr Ala Gln Ala Asp Phe Ile Ala Gly Asp
        200                 205                 210

cct tcc aca gat att gct gtg atc aag att agg gat gtg tcc aac ctt    787
Pro Ser Thr Asp Ile Ala Val Ile Lys Ile Arg Asp Val Ser Asn Leu
    215                 220                 225

cca gtt atg agc ttt gga gat tcg gac gca tta ggc gtt gga caa agt    835
Pro Val Met Ser Phe Gly Asp Ser Asp Ala Leu Gly Val Gly Gln Ser
230                 235                 240                 245

gtg atg gct gtt ggt tct cca ctg ggt ctg agc tcc act gtg acc acc    883
Val Met Ala Val Gly Ser Pro Leu Gly Leu Ser Ser Thr Val Thr Thr
                250                 255                 260

ggt att gtg tcg gcc gtg aac cgt cct gtg cga gct tct ggt gat ggc    931
Gly Ile Val Ser Ala Val Asn Arg Pro Val Arg Ala Ser Gly Asp Gly
            265                 270                 275

gga gag tcg tcc ctc atc gat gct atc cag acc gat gct gcg atc aac    979
Gly Glu Ser Ser Leu Ile Asp Ala Ile Gln Thr Asp Ala Ala Ile Asn
        280                 285                 290

cct ggt aac tct ggt ggt ccg ctg gtt gat atg gat ggc aac ctc att    1027
Pro Gly Asn Ser Gly Gly Pro Leu Val Asp Met Asp Gly Asn Leu Ile
    295                 300                 305

ggc atg aat tcg gta att gca tcg att tcg agc acc agc gat tcc gca    1075
Gly Met Asn Ser Val Ile Ala Ser Ile Ser Ser Thr Ser Asp Ser Ala
310                 315                 320                 325

ggt tcc att ggt ctt ggt ttt tct atc cca tcc aac ttt gcc aag cgc    1123
Gly Ser Ile Gly Leu Gly Phe Ser Ile Pro Ser Asn Phe Ala Lys Arg
                330                 335                 340

gtg gcc gat caa ttg atc agc acc ggc cag gta act cag ccg atg atc    1171
Val Ala Asp Gln Leu Ile Ser Thr Gly Gln Val Thr Gln Pro Met Ile
            345                 350                 355

ggt gtg cag gtt ggc act gac aac tca gtg aca ggc gct gtg att gcc     1219
Gly Val Gln Val Gly Thr Asp Asn Ser Val Thr Gly Ala Val Ile Ala
        360                 365                 370

agt gtt caa gat ggt gga ccg gcc gca gat gct gga ctt cag cca ggc     1267
Ser Val Gln Asp Gly Gly Pro Ala Ala Asp Ala Gly Leu Gln Pro Gly
    375                 380                 385

gat atc gtg acc aag ctc aat gat cga gtt att gat agc cca gac tcc     1315
Asp Ile Val Thr Lys Leu Asn Asp Arg Val Ile Asp Ser Pro Asp Ser
390                 395                 400                 405

ttg atc gct gct gtt cgt tcg cat gat ttt ggc gaa acc gtc act tta     1363
Leu Ile Ala Ala Val Arg Ser His Asp Phe Gly Glu Thr Val Thr Leu
                410                 415                 420

aca att aca cag cca gat acc tcg cag agc cgg gag gta gag gtt act     1411
Thr Ile Thr Gln Pro Asp Thr Ser Gln Ser Arg Glu Val Glu Val Thr
            425                 430                 435

ctg acg agt gag taggtttaaa agagttaatc tgc                           1446
Leu Thr Ser Glu
        440


<210> 56
<211> 441
<212> PRT
<213> Corynebacterium glutamicum

<400> 56
Met Thr Asn Gln Phe Pro Thr Asn Asn Gly Glu Asn Pro Asp Arg Ala
1               5                   10                  15

Ser Glu Thr Pro Ser Glu Thr Asn Ser Phe Glu His Val Arg Ser Ser
            20                  25                  30

Tyr Pro Gln Trp Gly Asn Thr Ala Ser Asn Gln Asn Pro Tyr Pro Gly
        35                  40                  45

Ala Gly Phe Gly Ser Glu Gln Asn Thr Gln Gln Gly Asn Glu Gln Gln
    50                  55                  60

Ala Pro Ala Trp Thr Ser Trp Asp Asn Gln Pro Leu Ser Thr Asp Val
65                  70                  75                  80

Lys Pro Ala Lys Glu Lys Arg Lys Val Gly Ile Gly Thr Ala Leu Ala
                85                  90                  95

Leu Met Leu Val Gly Ser Ile Ala Thr Gly Ser Val Val Gly Val Ala
            100                 105                 110

Ala Thr Gln Leu Gly Ser Asp Ser Ser Thr Pro Val Asn Ala Leu Glu
        115                 120                 125

Gln Pro Ser Val Gln Arg Thr Thr Asn Ala Glu Pro Gly Ser Ala Glu
    130                 135                 140

Gln Val Ala Ala Ala Val Leu Pro Ser Val Val Ser Ile Gln Ala Ile
145                 150                 155                 160

Thr Arg Thr Ser Ala Ser Glu Gly Ser Gly Ser Ile Ile Ser Ser Asp
                165                 170                 175

Gly Tyr Val Met Thr Asn Asn His Val Val Ala Gly Ile Glu Gln Ser
            180                 185                 190

Gly Val Leu Glu Val Ser Phe Ser Asp Gly Thr Thr Ala Gln Ala Asp
        195                 200                 205

Phe Ile Ala Gly Asp Pro Ser Thr Asp Ile Ala Val Ile Lys Ile Arg
    210                 215                 220

Asp Val Ser Asn Leu Pro Val Met Ser Phe Gly Asp Ser Asp Ala Leu
225                 230                 235                 240

Gly Val Gly Gln Ser Val Met Ala Val Gly Ser Pro Leu Gly Leu Ser

245                 250                 255

Ser Thr Val Thr Thr Gly Ile Val Ser Ala Val Asn Arg Pro Val Arg
            260                 265                 270

Ala Ser Gly Asp Gly Gly Glu Ser Ser Leu Ile Asp Ala Ile Gln Thr
        275                 280                 285

Asp Ala Ala Ile Asn Pro Gly Asn Ser Gly Gly Pro Leu Val Asp Met
    290                 295                 300

Asp Gly Asn Leu Ile Gly Met Asn Ser Val Ile Ala Ser Ile Ser Ser
305                 310                 315                 320

Thr Ser Asp Ser Ala Gly Ser Ile Gly Leu Gly Phe Ser Ile Pro Ser
                325                 330                 335

Asn Phe Ala Lys Arg Val Ala Asp Gln Leu Ile Ser Thr Gly Gln Val
            340                 345                 350

Thr Gln Pro Met Ile Gly Val Gln Val Gly Thr Asp Asn Ser Val Thr
        355                 360                 365

Gly Ala Val Ile Ala Ser Val Gln Asp Gly Gly Pro Ala Ala Asp Ala
    370                 375                 380

Gly Leu Gln Pro Gly Asp Ile Val Thr Lys Leu Asn Asp Arg Val Ile
385                 390                 395                 400

Asp Ser Pro Asp Ser Leu Ile Ala Ala Val Arg Ser His Asp Phe Gly
                405                 410                 415

Glu Thr Val Thr Leu Thr Ile Thr Gln Pro Asp Thr Ser Gln Ser Arg
            420                 425                 430

Glu Val Glu Val Thr Leu Thr Ser Glu
        435                 440


<210> 57
<211> 518
<212> DNA
<213> Corynebacterium glutamicum

<220>
<221> CDS
<222> (1)..(495)
<223> RXA02834

<400> 57
gat tct aaa ggt aga agt gtt gac ttt aaa aat acc att atc atc atg      48
Asp Ser Lys Gly Arg Ser Val Asp Phe Lys Asn Thr Ile Ile Ile Met
1               5                   10                  15

act agt aat att ggt tca caa gta tta ctt gaa aat gta aaa gat gct      96
Thr Ser Asn Ile Gly Ser Gln Val Leu Leu Glu Asn Val Lys Asp Ala
            20                  25                  30

ggt gaa att agt gat gat aca gag aaa gca gtt atg gac agt cta cat      144
Gly Glu Ile Ser Asp Asp Thr Glu Lys Ala Val Met Asp Ser Leu His
        35                  40                  45

gca tac ttc aaa cct gaa ata tta aat cgt atg gat gac atc gtg tta      192
Ala Tyr Phe Lys Pro Glu Ile Leu Asn Arg Met Asp Asp Ile Val Leu
    50                  55                  60

ttt aaa cca tta tca gtt aat gat atg agt atg att gta gat aaa att      240
Phe Lys Pro Leu Ser Val Asn Asp Met Ser Met Ile Val Asp Lys Ile
65                  70                  75                  80

tta aca caa tta aat atg aga tta tta gat caa cat atc tca att gaa      288
Leu Thr Gln Leu Asn Met Arg Leu Leu Asp Gln His Ile Ser Ile Glu
                85                  90                  95

gtg aca gaa gaa gcg aaa aaa tgg cta ggt gaa gaa gcg tat gaa cca      336
Val Thr Glu Glu Ala Lys Lys Trp Leu Gly Glu Glu Ala Tyr Glu Pro
            100                 105                 110

caa ttt ggt gca aga cca tta aaa cgc ttt gtt caa cga caa ata gaa     384
Gln Phe Gly Ala Arg Pro Leu Lys Arg Phe Val Gln Arg Gln Ile Glu
        115                 120                 125

act cca att gca cgt atg atg att aaa gaa agt cta cct gaa ggt aca     432
Thr Pro Ile Ala Arg Met Met Ile Lys Glu Ser Leu Pro Glu Gly Thr
    130                 135                 140

ata att aaa gta gat tta aat gac aat aaa gaa ctt gat ttt aag gtt     480
Ile Ile Lys Val Asp Leu Asn Asp Asn Lys Glu Leu Asp Phe Lys Val
145                 150                 155                 160

gtt aaa cct acg tct taatctagca aaatattaat ttg                       518
Val Lys Pro Thr Ser
                165


<210> 58
<211> 165
<212> PRT
<213> Corynebacterium glutamicum

<400> 58
Asp Ser Lys Gly Arg Ser Val Asp Phe Lys Asn Thr Ile Ile Ile Met
  1               5                  10                  15

Thr Ser Asn Ile Gly Ser Gln Val Leu Leu Glu Asn Val Lys Asp Ala
             20                  25                  30

Gly Glu Ile Ser Asp Asp Thr Glu Lys Ala Val Met Asp Ser Leu His
         35                  40                  45

Ala Tyr Phe Lys Pro Glu Ile Leu Asn Arg Met Asp Asp Ile Val Leu
     50                  55                  60

Phe Lys Pro Leu Ser Val Asn Asp Met Ser Met Ile Val Asp Lys Ile
 65                  70                  75                  80

Leu Thr Gln Leu Asn Met Arg Leu Leu Asp Gln His Ile Ser Ile Glu
                 85                  90                  95

Val Thr Glu Glu Ala Lys Lys Trp Leu Gly Glu Glu Ala Tyr Glu Pro
            100                 105                 110

Gln Phe Gly Ala Arg Pro Leu Lys Arg Phe Val Gln Arg Gln Ile Glu
        115                 120                 125

Thr Pro Ile Ala Arg Met Met Ile Lys Glu Ser Leu Pro Glu Gly Thr
    130                 135                 140

Ile Ile Lys Val Asp Leu Asn Asp Asn Lys Glu Leu Asp Phe Lys Val
145                 150                 155                 160

Val Lys Pro Thr Ser
                165


<210> 59
<211> 1314
<212> DNA
<213> Corynebacterium glutamicum

<220>
<221> CDS
<222> (101)..(1291)
<223> RXA00112

<400> 59
gcgttaggct gacgggctcc ggtggcctaa aatcgaagcg atctaaaaat ttggcagtta 60

atattatcga ttttcgaaag cgtagaacac caaaccattg ttg agc ccc agc ctg     115
                                            Leu Ser Pro Ser Leu
                                              1               5

gtc gtc gat gcc gtc atc gtc ctc gtt atg gca ttc gcc ctg tgg ggt     163
Val Val Asp Ala Val Ile Val Leu Val Met Ala Phe Ala Leu Trp Gly

                    10                  15                  20

ggt tgg cgt caa ggc gcc ttc acc tcg ctg ctg tcc acc gtc ggc gtc      211
Gly Trp Arg Gln Gly Ala Phe Thr Ser Leu Leu Ser Thr Val Gly Val
                25                  30                  35

gtt tct ggc ctg gta gtt ggc gca gca gca gct cca ttt gtc atg ggt      259
Val Ser Gly Leu Val Val Gly Ala Ala Ala Ala Pro Phe Val Met Gly
            40                  45                  50

ctc acc gat tcc acc gcg ctt cgc ttc ctc ctg gcg atc ggc acc gtg      307
Leu Thr Asp Ser Thr Ala Leu Arg Phe Leu Leu Ala Ile Gly Thr Val
    55                  60                  65

gtg ctg ctg gtt ggt ttg gga aat ctc atc ggc gcc cac ttg ggt gct      355
Val Leu Leu Val Gly Leu Gly Asn Leu Ile Gly Ala His Leu Gly Ala
70                  75                  80                  85

gcg att aga gac aac atc aaa ttc cga agt tcc agg acc tta gat tct      403
Ala Ile Arg Asp Asn Ile Lys Phe Arg Ser Ser Arg Thr Leu Asp Ser
                90                  95                  100

ggg ctc ggc gcc att ttc caa gta ttg gcc acc ttg atc gtg gtg tgg      451
Gly Leu Gly Ala Ile Phe Gln Val Leu Ala Thr Leu Ile Val Val Trp
            105                 110                 115

ctc gtc gca att ccc ctg gcc aca ggc ctc ccc gga act gtc gcc agc      499
Leu Val Ala Ile Pro Leu Ala Thr Gly Leu Pro Gly Thr Val Ala Ser
        120                 125                 130

gga att aga gac tcc cgc atc ctg ggc ttt gta gac aaa tac acc ccg      547
Gly Ile Arg Asp Ser Arg Ile Leu Gly Phe Val Asp Lys Tyr Thr Pro
    135                 140                 145

caa ggc cta gat acc ctg ccc tcc aaa atc gct gcg atg ctc agc gaa      595
Gln Gly Leu Asp Thr Leu Pro Ser Lys Ile Ala Ala Met Leu Ser Glu
150                 155                 160                 165

tcc ggc ctc cca cca ctg att tcc ccc ttc acc ggc gga tcc tcg gtg      643
Ser Gly Leu Pro Pro Leu Ile Ser Pro Phe Thr Gly Gly Ser Ser Val
                170                 175                 180

gaa gtg gac gcc ccc gaa atc aac gtc acc aac gtt gac cta gtc gaa      691
Glu Val Asp Ala Pro Glu Ile Asn Val Thr Asn Val Asp Leu Val Glu
            185                 190                 195

gca atg cgc ccg tcc gtc atc cac gtg atg ggt gac gcc caa gaa tgc      739
Ala Met Arg Pro Ser Val Ile His Val Met Gly Asp Ala Gln Glu Cys
        200                 205                 210

agc cgc cga ctc atg ggt tct ggc ttt gtg gca tcc ccc gac tac gtt      787
Ser Arg Arg Leu Met Gly Ser Gly Phe Val Ala Ser Pro Asp Tyr Val
    215                 220                 225

gtg acc aac gcc cac gtt gtt gca ggt acc tcc acc gtc agc ctg gat      835
Val Thr Asn Ala His Val Val Ala Gly Thr Ser Thr Val Ser Leu Asp
230                 235                 240                 245

acc atg atc gga acc cgc tcc gca gag gta gtg ttc tac gac ccg aac      883
Thr Met Ile Gly Thr Arg Ser Ala Glu Val Val Phe Tyr Asp Pro Asn
                250                 255                 260

ctg gac atc gca gtc ctt tac agc cct gac ctc ggc ttg gat cca ctg      931
Leu Asp Ile Ala Val Leu Tyr Ser Pro Asp Leu Gly Leu Asp Pro Leu
            265                 270                 275

ccg tgg gca tcc act ccg cta gac act ggc gat gaa gca atc gtc atg      979
Pro Trp Ala Ser Thr Pro Leu Asp Thr Gly Asp Glu Ala Ile Val Met
        280                 285                 290

gga ttc cca cag tcc gga cct ttc aac gcc tcc cca gcc agg gtc cgc     1027
Gly Phe Pro Gln Ser Gly Pro Phe Asn Ala Ser Pro Ala Arg Val Arg
    295                 300                 305

gaa cgc atc atg atc acc ggc agc aac att tac gcc aac ggc cag cac     1075
Glu Arg Ile Met Ile Thr Gly Ser Asn Ile Tyr Ala Asn Gly Gln His
310                 315                 320                 325

gaa cgc gaa gcc tat tca gtc cgc gga tcc atc caa tct gga aac tcc     1123
Glu Arg Glu Ala Tyr Ser Val Arg Gly Ser Ile Gln Ser Gly Asn Ser
                330                 335                 340

ggc ggc cca atg acc aac gaa atg ggt gaa gtg gtt ggt gtt gtc ttc     1171
Gly Gly Pro Met Thr Asn Glu Met Gly Glu Val Val Gly Val Val Phe
            345                 350                 355

ggc gca gcg atc gac ggc tcc gat acc ggt tac gtt ctc act gcc gaa     1219
Gly Ala Ala Ile Asp Gly Ser Asp Thr Gly Tyr Val Leu Thr Ala Glu
        360                 365                 370

gag gta cag gag cgg atc ggc gac atc acc gcg ctg act cag cct gtc     1267
Glu Val Gln Glu Arg Ile Gly Asp Ile Thr Ala Leu Thr Gln Pro Val
    375                 380                 385

gat acg atg cag tgc gcg gtt tct tagtcgtcgg gagctaggac cag           1314
Asp Thr Met Gln Cys Ala Val Ser
390                 395


<210> 60
<211> 397
<212> PRT
<213> Corynebacterium glutamicum

<400> 60
Leu Ser Pro Ser Leu Val Val Asp Ala Val Ile Val Leu Val Met Ala
1               5                   10                  15

Phe Ala Leu Trp Gly Gly Trp Arg Gln Gly Ala Phe Thr Ser Leu Leu
            20                  25                  30

Ser Thr Val Gly Val Val Ser Gly Leu Val Val Gly Ala Ala Ala Ala
        35                  40                  45

Pro Phe Val Met Gly Leu Thr Asp Ser Thr Ala Leu Arg Phe Leu Leu
    50                  55                  60

Ala Ile Gly Thr Val Val Leu Leu Val Gly Leu Gly Asn Leu Ile Gly
65                  70                  75                  80

Ala His Leu Gly Ala Ala Ile Arg Asp Asn Ile Lys Phe Arg Ser Ser
                85                  90                  95

Arg Thr Leu Asp Ser Gly Leu Gly Ala Ile Phe Gln Val Leu Ala Thr
            100                 105                 110

Leu Ile Val Val Trp Leu Val Ala Ile Pro Leu Ala Thr Gly Leu Pro
        115                 120                 125

Gly Thr Val Ala Ser Gly Ile Arg Asp Ser Arg Ile Leu Gly Phe Val
    130                 135                 140

Asp Lys Tyr Thr Pro Gln Gly Leu Asp Thr Leu Pro Ser Lys Ile Ala
145                 150                 155                 160

Ala Met Leu Ser Glu Ser Gly Leu Pro Pro Leu Ile Ser Pro Phe Thr
                165                 170                 175

Gly Gly Ser Ser Val Glu Val Asp Ala Pro Glu Ile Asn Val Thr Asn
            180                 185                 190

Val Asp Leu Val Glu Ala Met Arg Pro Ser Val Ile His Val Met Gly
        195                 200                 205

Asp Ala Gln Glu Cys Ser Arg Arg Leu Met Gly Ser Gly Phe Val Ala
    210                 215                 220

Ser Pro Asp Tyr Val Val Thr Asn Ala His Val Val Ala Gly Thr Ser
225                 230                 235                 240

Thr Val Ser Leu Asp Thr Met Ile Gly Thr Arg Ser Ala Glu Val Val
                245                 250                 255

Phe Tyr Asp Pro Asn Leu Asp Ile Ala Val Leu Tyr Ser Pro Asp Leu

260                 265                 270

Gly Leu Asp Pro Leu Pro Trp Ala Ser Thr Pro Leu Asp Thr Gly Asp
        275                 280                 285

Glu Ala Ile Val Met Gly Phe Pro Gln Ser Gly Pro Phe Asn Ala Ser
    290                 295                 300

Pro Ala Arg Val Arg Glu Arg Ile Met Ile Thr Gly Ser Asn Ile Tyr
305                 310                 315                 320

Ala Asn Gly Gln His Glu Arg Glu Ala Tyr Ser Val Arg Gly Ser Ile
                325                 330                 335

Gln Ser Gly Asn Ser Gly Gly Pro Met Thr Asn Glu Met Gly Glu Val
            340                 345                 350

Val Gly Val Val Phe Gly Ala Ala Ile Asp Gly Ser Asp Thr Gly Tyr
        355                 360                 365

Val Leu Thr Ala Glu Glu Val Gln Glu Arg Ile Gly Asp Ile Thr Ala
    370                 375                 380

Leu Thr Gln Pro Val Asp Thr Met Gln Cys Ala Val Ser
385                 390                 395


<210> 61
<211> 729
<212> DNA
<213> Corynebacterium glutamicum

<220>
<221> CDS
<222> (101)..(706)
<223> RXA00566

<400> 61
cttgttgacc acgtgattac cttggctgaa ggcccaatca gcaactaggc gcacggaaaa 60

ctttaaagga gagaataaga ttatgagcag cggattccaa atg cca acg tcc cgt    115
                                           Met Pro Thr Ser Arg
                                           1               5

tac gtg ctg cct tcc ttc att gag cag tcc gca tac ggc acc aaa gag    163
Tyr Val Leu Pro Ser Phe Ile Glu Gln Ser Ala Tyr Gly Thr Lys Glu
                10                  15                  20

acc aac cct tac gca aaa ctc ttt gaa gag cgc atc atc ttc ctg ggc    211
Thr Asn Pro Tyr Ala Lys Leu Phe Glu Glu Arg Ile Ile Phe Leu Gly
            25                  30                  35

acc cag gtc gac gac acc tct gca aac gac atc atg gcg cag ctc ctt    259
Thr Gln Val Asp Asp Thr Ser Ala Asn Asp Ile Met Ala Gln Leu Leu
        40                  45                  50

gtc ctc gaa ggc atg gac cca gac cgc gat atc acc ctg tac atc aac    307
Val Leu Glu Gly Met Asp Pro Asp Arg Asp Ile Thr Leu Tyr Ile Asn
    55                  60                  65

tca cct ggt gga tcc ttc acc gcg ttg atg gca att tac gac acc atg    355
Ser Pro Gly Gly Ser Phe Thr Ala Leu Met Ala Ile Tyr Asp Thr Met
70                  75                  80                  85

cag tac gtc cgc cca gac gtt cag acc gtc tgc ctt ggt cag gca gca    403
Gln Tyr Val Arg Pro Asp Val Gln Thr Val Cys Leu Gly Gln Ala Ala
                90                  95                  100

tcc gca gcc gca gtt ctt ctt gca gcc ggt gca cca ggt aag cgc gct    451
Ser Ala Ala Ala Val Leu Leu Ala Ala Gly Ala Pro Gly Lys Arg Ala
            105                 110                 115

gtt ctt cct aac tcc cgc gtg ctg atc cac cag cca gca acc cag ggc    499
Val Leu Pro Asn Ser Arg Val Leu Ile His Gln Pro Ala Thr Gln Gly
        120                 125                 130

acc cag ggt cag gtt tct gac ctc gag atc cag gca gct gaa atc gag    547

Thr Gln Gly Gln Val Ser Asp Leu Glu Ile Gln Ala Ala Glu Ile Glu
    135                 140                 145

cgc atg cgt cgt ttg atg gaa acc acc ttg gct gag cac acc ggc aag     595
Arg Met Arg Arg Leu Met Glu Thr Thr Leu Ala Glu His Thr Gly Lys
150                 155                 160                 165

acc gcg gag cag atc cgc atc gat acc gac cgt gac aag atc ctc acc     643
Thr Ala Glu Gln Ile Arg Ile Asp Thr Asp Arg Asp Lys Ile Leu Thr
                170                 175                 180

gct gag gaa gca ctc gag tat ggc atc gtt gac cag gtc ttc gat tac     691
Ala Glu Glu Ala Leu Glu Tyr Gly Ile Val Asp Gln Val Phe Asp Tyr
            185                 190                 195

cgc aag ctc aag cgc tagagttttt taaagattcg ggt                       729
Arg Lys Leu Lys Arg
        200


<210> 62
<211> 202
<212> PRT
<213> Corynebacterium glutamicum

<400> 62
Met Pro Thr Ser Arg Tyr Val Leu Pro Ser Phe Ile Glu Gln Ser Ala
  1               5                  10                  15

Tyr Gly Thr Lys Glu Thr Asn Pro Tyr Ala Lys Leu Phe Glu Glu Arg
             20                  25                  30

Ile Ile Phe Leu Gly Thr Gln Val Asp Asp Thr Ser Ala Asn Asp Ile
         35                  40                  45

Met Ala Gln Leu Leu Val Leu Glu Gly Met Asp Pro Asp Arg Asp Ile
     50                  55                  60

Thr Leu Tyr Ile Asn Ser Pro Gly Gly Ser Phe Thr Ala Leu Met Ala
 65                  70                  75                  80

Ile Tyr Asp Thr Met Gln Tyr Val Arg Pro Asp Val Gln Thr Val Cys
                 85                  90                  95

Leu Gly Gln Ala Ala Ser Ala Ala Ala Val Leu Leu Ala Ala Gly Ala
            100                 105                 110

Pro Gly Lys Arg Ala Val Leu Pro Asn Ser Arg Val Leu Ile His Gln
        115                 120                 125

Pro Ala Thr Gln Gly Thr Gln Gly Gln Val Ser Asp Leu Glu Ile Gln
    130                 135                 140

Ala Ala Glu Ile Glu Arg Met Arg Arg Leu Met Glu Thr Thr Leu Ala
145                 150                 155                 160

Glu His Thr Gly Lys Thr Ala Glu Gln Ile Arg Ile Asp Thr Asp Arg
                165                 170                 175

Asp Lys Ile Leu Thr Ala Glu Glu Ala Leu Glu Tyr Gly Ile Val Asp
            180                 185                 190

Gln Val Phe Asp Tyr Arg Lys Leu Lys Arg
        195                 200


<210> 63
<211> 714
<212> DNA
<213> Corynebacterium glutamicum

<220>
<221> CDS
<222> (101)..(691)
<223> RXA00567

<400> 63

cgaacagagg cggtttcatg gaaataccgc cgggtagtct ggtgacattg aaccaaatga 60

acgtacccaa gatttaagaa tgtaggagtt gactgttttc atg agc gat att cgt 115
Met Ser Asp Ile Arg
1               5

atg gca gcc cag ggt ggg cct ggt ttc gga aat gac gtc ttt gat cgc 163
Met Ala Ala Gln Gly Gly Pro Gly Phe Gly Asn Asp Val Phe Asp Arg
                10                  15                  20

ctc atg agt gag cgc atc att ttc ctg gga agc cag gta gac gat gag 211
Leu Met Ser Glu Arg Ile Ile Phe Leu Gly Ser Gln Val Asp Asp Glu
            25                  30                  35

att gca aac aag cta tgc gct cag atc ctg ctg ctg tcc gct gag gat 259
Ile Ala Asn Lys Leu Cys Ala Gln Ile Leu Leu Leu Ser Ala Glu Asp
        40                  45                  50

cca acc agg gac atc tcc ctg tac att aac tcc cca ggt ggc tcc gtc 307
Pro Thr Arg Asp Ile Ser Leu Tyr Ile Asn Ser Pro Gly Gly Ser Val
    55                  60                  65

acc gca ggc atg gca att tat gac acc atg aaa tac tcc cca tgc gac 355
Thr Ala Gly Met Ala Ile Tyr Asp Thr Met Lys Tyr Ser Pro Cys Asp
70                  75                  80                  85

atc gca acc tac ggc atg ggc ctg gca gca tcc atg ggc cag ttc ctg 403
Ile Ala Thr Tyr Gly Met Gly Leu Ala Ala Ser Met Gly Gln Phe Leu
                90                  95                  100

ctt tcc ggt ggc act aag ggc aag cgt ttc gca ttg cca cac gca cgt 451
Leu Ser Gly Gly Thr Lys Gly Lys Arg Phe Ala Leu Pro His Ala Arg
            105                 110                 115

atc atg atg cac cag cct tcc gct ggt gtt ggt ggt acc gca gct gat 499
Ile Met Met His Gln Pro Ser Ala Gly Val Gly Gly Thr Ala Ala Asp
        120                 125                 130

atc gct atc cag gct gag cag ttc gca gcc acc aag cgt gaa atg gcc 547
Ile Ala Ile Gln Ala Glu Gln Phe Ala Ala Thr Lys Arg Glu Met Ala
    135                 140                 145

cag ctg atc gct gag cac acc ggc cag acc ttt gag cag atc tcc aag 595
Gln Leu Ile Ala Glu His Thr Gly Gln Thr Phe Glu Gln Ile Ser Lys
150                 155                 160                 165

gat tcc gat cgt gac cgc tgg ttc act gca cag gaa gct aag gat tac 643
Asp Ser Asp Arg Asp Arg Trp Phe Thr Ala Gln Glu Ala Lys Asp Tyr
                170                 175                 180

gga ctt gtt gac cac gtg att acc ttg gct gaa ggc cca atc agc aac 691
Gly Leu Val Asp His Val Ile Thr Leu Ala Glu Gly Pro Ile Ser Asn
            185                 190                 195

taggcgcacg gaaaacttta aag 714

<210> 64
<211> 197
<212> PRT
<213> Corynebacterium glutamicum

<400> 64
Met Ser Asp Ile Arg Met Ala Ala Gln Gly Gly Pro Gly Phe Gly Asn
1               5                   10                  15

Asp Val Phe Asp Arg Leu Met Ser Glu Arg Ile Ile Phe Leu Gly Ser
            20                  25                  30

Gln Val Asp Asp Glu Ile Ala Asn Lys Leu Cys Ala Gln Ile Leu Leu
        35                  40                  45

Leu Ser Ala Glu Asp Pro Thr Arg Asp Ile Ser Leu Tyr Ile Asn Ser
    50                  55                  60

Pro Gly Gly Ser Val Thr Ala Gly Met Ala Ile Tyr Asp Thr Met Lys
65                  70                  75                  80

Tyr Ser Pro Cys Asp Ile Ala Thr Tyr Gly Met Gly Leu Ala Ala Ser
                85                  90                  95

Met Gly Gln Phe Leu Leu Ser Gly Gly Thr Lys Gly Lys Arg Phe Ala
            100                 105                 110

Leu Pro His Ala Arg Ile Met Met His Gln Pro Ser Ala Gly Val Gly
        115                 120                 125

Gly Thr Ala Ala Asp Ile Ala Ile Gln Ala Glu Gln Phe Ala Ala Thr
    130                 135                 140

Lys Arg Glu Met Ala Gln Leu Ile Ala Glu His Thr Gly Gln Thr Phe
145                 150                 155                 160

Glu Gln Ile Ser Lys Asp Ser Asp Arg Asp Arg Trp Phe Thr Ala Gln
                165                 170                 175

Glu Ala Lys Asp Tyr Gly Leu Val Asp His Val Ile Thr Leu Ala Glu
            180                 185                 190

Gly Pro Ile Ser Asn
        195


<210> 65
<211> 1875
<212> DNA
<213> Corynebacterium glutamicum

<220>
<221> CDS
<222> (101)..(1852)
<223> RXN03094

<400> 65
atagatatta gagagttaaa taatggcgct tgacctgcag gaaattgaga tcaacactga 60

ttgtgtaggt tggcgcccaa caaagaaagg gcgttgaaag atg agt tca ttc aat    115
                                            Met Ser Ser Phe Asn
                                            1               5

cca act acc aaa acc aat gaa gcc atg cag gct gct ctt cag cag gca    163
Pro Thr Thr Lys Thr Asn Glu Ala Met Gln Ala Ala Leu Gln Gln Ala
                10                  15                  20

tcc tcg gct ggc aac cct gat att cgt cca gct cac ctg ttg gct gcc    211
Ser Ser Ala Gly Asn Pro Asp Ile Arg Pro Ala His Leu Leu Ala Ala
            25                  30                  35

atc ttg gag caa act gat ggc gta gca gcg cca gtc ctc atg gct act    259
Ile Leu Glu Gln Thr Asp Gly Val Ala Ala Pro Val Leu Met Ala Thr
        40                  45                  50

ggt gtg gat cct aag gag atc ctc gca gag gcc aag aag ttg gtt gct    307
Gly Val Asp Pro Lys Glu Ile Leu Ala Glu Ala Lys Lys Leu Val Ala
    55                  60                  65

tct tac ccc aag gct tct ggc gcc aat atg gct aat cca aac ttc aac    355
Ser Tyr Pro Lys Ala Ser Gly Ala Asn Met Ala Asn Pro Asn Phe Asn
70                  75                  80                  85

cgg gat gcc ctc aat gcg ttc act gca gct cag gag ctt gcc ggt gag    403
Arg Asp Ala Leu Asn Ala Phe Thr Ala Ala Gln Glu Leu Ala Gly Glu
                90                  95                  100

ttg ggc gat gag tac gtc tca acc gaa gta ctt ctt gcc ggt atc gct    451
Leu Gly Asp Glu Tyr Val Ser Thr Glu Val Leu Leu Ala Gly Ile Ala
            105                 110                 115

cgc gga aag tct gat gct gcg gat ctg ttg acc aac aag ggt gca acc    499
Arg Gly Lys Ser Asp Ala Ala Asp Leu Leu Thr Asn Lys Gly Ala Thr
        120                 125                 130

tat gac gcc atc aaa gag gct ttc cct tcg gtt cgt gga tct cag cgt    547
Tyr Asp Ala Ile Lys Glu Ala Phe Pro Ser Val Arg Gly Ser Gln Arg

135                 140                 145

gtc acc act cag gat cca gag gga cag ttc cag gct ttg gaa aag tac      595
Val Thr Thr Gln Asp Pro Glu Gly Gln Phe Gln Ala Leu Glu Lys Tyr
150                 155                 160                 165

tcc act gac ctg acc aag ctt gct cgt gaa ggc aag att gat cct gtt      643
Ser Thr Asp Leu Thr Lys Leu Ala Arg Glu Gly Lys Ile Asp Pro Val
                170                 175                 180

att ggc cgt gac cag gaa att cgt cgc gtc gtt cag gtg ctt agc cgt      691
Ile Gly Arg Asp Gln Glu Ile Arg Arg Val Val Gln Val Leu Ser Arg
            185                 190                 195

cgt acc aag aac aac cct gtt ctg atc ggt gag cca ggt gtc ggt aaa      739
Arg Thr Lys Asn Asn Pro Val Leu Ile Gly Glu Pro Gly Val Gly Lys
        200                 205                 210

acc gcc atc gtg gaa ggc ctt gca cgc cgc atc gtt gct ggt gac gtt      787
Thr Ala Ile Val Glu Gly Leu Ala Arg Arg Ile Val Ala Gly Asp Val
    215                 220                 225

cca gaa tcc ctc aag ggc aaa act ctg atc agt ctt gat ctt ggt tcc      835
Pro Glu Ser Leu Lys Gly Lys Thr Leu Ile Ser Leu Asp Leu Gly Ser
230                 235                 240                 245

atg gtt gcc ggc gct aag tat cgc ggt gaa ttc gag gag cga ctg aag      883
Met Val Ala Gly Ala Lys Tyr Arg Gly Glu Phe Glu Glu Arg Leu Lys
                250                 255                 260

gct gtt ctg gat gag atc aag gga gct aac ggc gaa gtc gtt acc ttc      931
Ala Val Leu Asp Glu Ile Lys Gly Ala Asn Gly Glu Val Val Thr Phe
            265                 270                 275

atc gat gag ctg cac acc atc gtc ggc gct ggt gct tcg ggt gaa tcc      979
Ile Asp Glu Leu His Thr Ile Val Gly Ala Gly Ala Ser Gly Glu Ser
        280                 285                 290

gcc atg gat gcc gga aac atg att aag cca ctg ctt gcc cgc ggt gag      1027
Ala Met Asp Ala Gly Asn Met Ile Lys Pro Leu Leu Ala Arg Gly Glu
    295                 300                 305

ctg cgc ttg gtt ggt gcc acc acg ctg aat gag tac cgc aag tac atc      1075
Leu Arg Leu Val Gly Ala Thr Thr Leu Asn Glu Tyr Arg Lys Tyr Ile
310                 315                 320                 325

gaa aag gac gct gcc ctg gag cgt agg ttc cag cag gtt tat gtc ggt      1123
Glu Lys Asp Ala Ala Leu Glu Arg Arg Phe Gln Gln Val Tyr Val Gly
                330                 335                 340

gag cca acg gta gaa gat gcc atc ggt att ctt cgt gga ttg aag gaa      1171
Glu Pro Thr Val Glu Asp Ala Ile Gly Ile Leu Arg Gly Leu Lys Glu
            345                 350                 355

cgc tac gag gtc cat cac ggt gtc cgc atc cag gac tcc gca ctg gtc      1219
Arg Tyr Glu Val His His Gly Val Arg Ile Gln Asp Ser Ala Leu Val
        360                 365                 370

gcc gca gct gaa ctc tca aac cgc tat atc acc agc cgt ttc ctt cct      1267
Ala Ala Ala Glu Leu Ser Asn Arg Tyr Ile Thr Ser Arg Phe Leu Pro
    375                 380                 385

gat aag gct att gac tta gtt gat gag gca gca tca cgc ctg cgc atg      1315
Asp Lys Ala Ile Asp Leu Val Asp Glu Ala Ala Ser Arg Leu Arg Met
390                 395                 400                 405

gag att gat tct tca cct cag gaa atc gat gag ctg gag cgt atc gtc      1363
Glu Ile Asp Ser Ser Pro Gln Glu Ile Asp Glu Leu Glu Arg Ile Val
                410                 415                 420

cgc cgc ctc gag atc gaa gag atg gcg ctg tcc aag gaa tcc gat gca      1411
Arg Arg Leu Glu Ile Glu Glu Met Ala Leu Ser Lys Glu Ser Asp Ala
            425                 430                 435

gct tcc aag gaa cgt cta gaa aag ctg cgc tcg gaa ctt gct gat gaa      1459
Ala Ser Lys Glu Arg Leu Glu Lys Leu Arg Ser Glu Leu Ala Asp Glu
        440                 445                 450

cgc gaa aag ctc tct gag ttg aag gct cgt tgg cag aat gag aaa act     1507
Arg Glu Lys Leu Ser Glu Leu Lys Ala Arg Trp Gln Asn Glu Lys Thr
    455                 460                 465

gct att gac gat gtc cgg gag atg aaa gaa gag ctg gaa gcg ctg cgt     1555
Ala Ile Asp Asp Val Arg Glu Met Lys Glu Glu Leu Glu Ala Leu Arg
470                 475                 480                 485

tct gag tcg gat att gca aaa cgt gac ggc aat tat tgt cgt gtc gca     1603
Ser Glu Ser Asp Ile Ala Lys Arg Asp Gly Asn Tyr Cys Arg Val Ala
                490                 495                 500

aag ctt cgc tac ggc cga atc cct gag ctg gaa aag cag atc gag gat     1651
Lys Leu Arg Tyr Gly Arg Ile Pro Glu Leu Glu Lys Gln Ile Glu Asp
            505                 510                 515

gca gaa tcc aag gtc gag gtc aat gaa aat gcc atg ctc act gag gag     1699
Ala Glu Ser Lys Val Glu Val Asn Glu Asn Ala Met Leu Thr Glu Glu
        520                 525                 530

gtc acg cca gac acg atc gcc gat gtg gtt tcc gca tgg acg ggc att     1747
Val Thr Pro Asp Thr Ile Ala Asp Val Val Ser Ala Trp Thr Gly Ile
    535                 540                 545

cct gca ggc aag atg atg cag ggt gag acc gag aag ctg ctc aac atg     1795
Pro Ala Gly Lys Met Met Gln Gly Glu Thr Glu Lys Leu Leu Asn Met
550                 555                 560                 565

gag cgc gtc ttg ggc aac cgt gtg gtc ggt cag cta gaa agc ggt aac     1843
Glu Arg Val Leu Gly Asn Arg Val Val Gly Gln Leu Glu Ser Gly Asn
                570                 575                 580

tgc agt gtc tgacgcggtg cgtcgttcac gcg                               1875
Cys Ser Val

<210> 66
<211> 584
<212> PRT
<213> Corynebacterium glutamicum

<400> 66
Met Ser Ser Phe Asn Pro Thr Thr Lys Thr Asn Glu Ala Met Gln Ala
1               5                   10                  15

Ala Leu Gln Gln Ala Ser Ser Ala Gly Asn Pro Asp Ile Arg Pro Ala
            20                  25                  30

His Leu Leu Ala Ala Ile Leu Glu Gln Thr Asp Gly Val Ala Ala Pro
        35                  40                  45

Val Leu Met Ala Thr Gly Val Asp Pro Lys Glu Ile Leu Ala Glu Ala
    50                  55                  60

Lys Lys Leu Val Ala Ser Tyr Pro Lys Ala Ser Gly Ala Asn Met Ala
65                  70                  75                  80

Asn Pro Asn Phe Asn Arg Asp Ala Leu Asn Ala Phe Thr Ala Ala Gln
                85                  90                  95

Glu Leu Ala Gly Glu Leu Gly Asp Glu Tyr Val Ser Thr Glu Val Leu
            100                 105                 110

Leu Ala Gly Ile Ala Arg Gly Lys Ser Asp Ala Ala Asp Leu Leu Thr
        115                 120                 125

Asn Lys Gly Ala Thr Tyr Asp Ala Ile Lys Glu Ala Phe Pro Ser Val
    130                 135                 140

Arg Gly Ser Gln Arg Val Thr Thr Gln Asp Pro Glu Gly Gln Phe Gln
145                 150                 155                 160

Ala Leu Glu Lys Tyr Ser Thr Asp Leu Thr Lys Leu Ala Arg Glu Gly
                165                 170                 175

Lys Ile Asp Pro Val Ile Gly Arg Asp Gln Glu Ile Arg Arg Val Val
            180                 185                 190

Gln Val Leu Ser Arg Arg Thr Lys Asn Asn Pro Val Leu Ile Gly Glu
        195                 200                 205

Pro Gly Val Gly Lys Thr Ala Ile Val Glu Gly Leu Ala Arg Arg Ile
    210                 215                 220

Val Ala Gly Asp Val Pro Glu Ser Leu Lys Gly Lys Thr Leu Ile Ser
225                 230                 235                 240

Leu Asp Leu Gly Ser Met Val Ala Gly Ala Lys Tyr Arg Gly Glu Phe
                245                 250                 255

Glu Glu Arg Leu Lys Ala Val Leu Asp Glu Ile Lys Gly Ala Asn Gly
            260                 265                 270

Glu Val Val Thr Phe Ile Asp Glu Leu His Thr Ile Val Gly Ala Gly
        275                 280                 285

Ala Ser Gly Glu Ser Ala Met Asp Ala Gly Asn Met Ile Lys Pro Leu
    290                 295                 300

Leu Ala Arg Gly Glu Leu Arg Leu Val Gly Ala Thr Thr Leu Asn Glu
305                 310                 315                 320

Tyr Arg Lys Tyr Ile Glu Lys Asp Ala Ala Leu Glu Arg Arg Phe Gln
                325                 330                 335

Gln Val Tyr Val Gly Glu Pro Thr Val Glu Asp Ala Ile Gly Ile Leu
            340                 345                 350

Arg Gly Leu Lys Glu Arg Tyr Glu Val His His Gly Val Arg Ile Gln
        355                 360                 365

Asp Ser Ala Leu Val Ala Ala Ala Glu Leu Ser Asn Arg Tyr Ile Thr
    370                 375                 380

Ser Arg Phe Leu Pro Asp Lys Ala Ile Asp Leu Val Asp Glu Ala Ala
385                 390                 395                 400

Ser Arg Leu Arg Met Glu Ile Asp Ser Ser Pro Gln Glu Ile Asp Glu
                405                 410                 415

Leu Glu Arg Ile Val Arg Arg Leu Glu Ile Glu Glu Met Ala Leu Ser
            420                 425                 430

Lys Glu Ser Asp Ala Ala Ser Lys Glu Arg Leu Glu Lys Leu Arg Ser
        435                 440                 445

Glu Leu Ala Asp Glu Arg Glu Lys Leu Ser Glu Leu Lys Ala Arg Trp
    450                 455                 460

Gln Asn Glu Lys Thr Ala Ile Asp Asp Val Arg Glu Met Lys Glu Glu
465                 470                 475                 480

Leu Glu Ala Leu Arg Ser Glu Ser Asp Ile Ala Lys Arg Asp Gly Asn
                485                 490                 495

Tyr Cys Arg Val Ala Lys Leu Arg Tyr Gly Arg Ile Pro Glu Leu Glu
            500                 505                 510

Lys Gln Ile Glu Asp Ala Glu Ser Lys Val Glu Val Asn Glu Asn Ala
        515                 520                 525

Met Leu Thr Glu Glu Val Thr Pro Asp Thr Ile Ala Asp Val Val Ser
    530                 535                 540

Ala Trp Thr Gly Ile Pro Ala Gly Lys Met Met Gln Gly Glu Thr Glu
545                 550                 555                 560

Lys Leu Leu Asn Met Glu Arg Val Leu Gly Asn Arg Val Val Gly Gln
                565                 570                 575

Leu Glu Ser Gly Asn Cys Ser Val
            580

<210> 67
<211> 1816
<212> DNA
<213> Corynebacterium glutamicum

<220>
<221> CDS
<222> (101)..(1816)
<223> FRXA01668

<400> 67
atagatatta gagagttaaa taatggcgct tgacctgcag gaaattgaga tcaacactga 60

ttgtgtaggt tggcgcccaa caaagaaagg gcgttgaaag atg agt tca ttc aat 115
Met Ser Ser Phe Asn
1               5

cca act acc aaa acc aat gaa gcc atg cag gct gct ctt cag cag gca 163
Pro Thr Thr Lys Thr Asn Glu Ala Met Gln Ala Ala Leu Gln Gln Ala
                10                  15                  20

tcc tcg gct ggc aac cct gat att cgt cca gct cac ctg ttg gct gcc 211
Ser Ser Ala Gly Asn Pro Asp Ile Arg Pro Ala His Leu Leu Ala Ala
            25                  30                  35

atc ttg gag caa act gat ggc gta gca gcg cca gtc ctc atg gct act 259
Ile Leu Glu Gln Thr Asp Gly Val Ala Ala Pro Val Leu Met Ala Thr
        40                  45                  50

ggt gtg gat cct aag gag atc ctc gca gag gcc aag aag ttg gtt gct 307
Gly Val Asp Pro Lys Glu Ile Leu Ala Glu Ala Lys Lys Leu Val Ala
    55                  60                  65

tct tac ccc aag gct tct ggc gcc aat atg gct aat cca aac ttc aac 355
Ser Tyr Pro Lys Ala Ser Gly Ala Asn Met Ala Asn Pro Asn Phe Asn
70                  75                  80                  85

cgg gat gcc ctc aat gcg ttc act gca gct cag gag ctt gcc ggt gag 403
Arg Asp Ala Leu Asn Ala Phe Thr Ala Ala Gln Glu Leu Ala Gly Glu
                90                  95                  100

ttg ggc gat gag tac gtc tca acc gaa gta ctt ctt gcc ggt atc gct 451
Leu Gly Asp Glu Tyr Val Ser Thr Glu Val Leu Leu Ala Gly Ile Ala
            105                 110                 115

cgc gga aag tct gat gct gcg gat ctg ttg acc aac aag ggt gca acc 499
Arg Gly Lys Ser Asp Ala Ala Asp Leu Leu Thr Asn Lys Gly Ala Thr
        120                 125                 130

tat gac gcc atc aaa gag gct ttc cct tcg gtt cgt gga tct cag cgt 547
Tyr Asp Ala Ile Lys Glu Ala Phe Pro Ser Val Arg Gly Ser Gln Arg
    135                 140                 145

gtc acc act cag gat cca gag gga cag ttc cag gct ttg gaa aag tac 595
Val Thr Thr Gln Asp Pro Glu Gly Gln Phe Gln Ala Leu Glu Lys Tyr
150                 155                 160                 165

tcc act gac ctg acc aag ctt gct cgt gaa ggc aag att gat cct gtt 643
Ser Thr Asp Leu Thr Lys Leu Ala Arg Glu Gly Lys Ile Asp Pro Val
                170                 175                 180

att ggc cgt gac cag gaa att cgt cgc gtc gtt cag gtg ctt agc cgt 691
Ile Gly Arg Asp Gln Glu Ile Arg Arg Val Val Gln Val Leu Ser Arg
            185                 190                 195

cgt acc aag aac aac cct gtt ctg atc ggt gag cca ggt gtc ggt aaa 739
Arg Thr Lys Asn Asn Pro Val Leu Ile Gly Glu Pro Gly Val Gly Lys
        200                 205                 210

acc gcc atc gtg gaa ggc ctt gca cgc cgc atc gtt gct ggt gac gtt 787
Thr Ala Ile Val Glu Gly Leu Ala Arg Arg Ile Val Ala Gly Asp Val
    215                 220                 225

cca gaa tcc ctc aag ggc aaa act ctg atc agt ctt gat ctt ggt tcc 835
Pro Glu Ser Leu Lys Gly Lys Thr Leu Ile Ser Leu Asp Leu Gly Ser

230                 235                 240                 245

atg gtt gcc ggc gct aag tat cgc ggt gaa ttc gag gag cga ctg aag      883
Met Val Ala Gly Ala Lys Tyr Arg Gly Glu Phe Glu Glu Arg Leu Lys
                250                 255                 260

gct gtt ctg gat gag atc aag gga gct aac ggc gaa gtc gtt acc ttc      931
Ala Val Leu Asp Glu Ile Lys Gly Ala Asn Gly Glu Val Val Thr Phe
            265                 270                 275

atc gat gag ctg cac acc atc gtc ggc gct ggt gct tcg ggt gaa tcc      979
Ile Asp Glu Leu His Thr Ile Val Gly Ala Gly Ala Ser Gly Glu Ser
        280                 285                 290

gcc atg gat gcc gga aac atg att aag cca ctg ctt gcc cgc ggt gag     1027
Ala Met Asp Ala Gly Asn Met Ile Lys Pro Leu Leu Ala Arg Gly Glu
    295                 300                 305

ctg cgc ttg gtt ggt gcc acc acg ctg aat gag tac cgc aag tac atc     1075
Leu Arg Leu Val Gly Ala Thr Thr Leu Asn Glu Tyr Arg Lys Tyr Ile
310                 315                 320                 325

gaa aag gac gct gcc ctg gag cgt agg ttc cag cag gtt tat gtc ggt     1123
Glu Lys Asp Ala Ala Leu Glu Arg Arg Phe Gln Gln Val Tyr Val Gly
                330                 335                 340

gag cca acg gta gaa gat gcc atc ggt att ctt cgt gga ttg aag gaa     1171
Glu Pro Thr Val Glu Asp Ala Ile Gly Ile Leu Arg Gly Leu Lys Glu
            345                 350                 355

cgc tac gag gtc cat cac ggt gtc cgc atc cag gac tcc gca ctg gtc     1219
Arg Tyr Glu Val His His Gly Val Arg Ile Gln Asp Ser Ala Leu Val
        360                 365                 370

gcc gca gct gaa ctc tca aac cgc tat atc acc agc cgt ttc ctt cct     1267
Ala Ala Ala Glu Leu Ser Asn Arg Tyr Ile Thr Ser Arg Phe Leu Pro
    375                 380                 385

gat aag gct att gac tta gtt gat gag gca gca tca cgc ctg cgc atg     1315
Asp Lys Ala Ile Asp Leu Val Asp Glu Ala Ala Ser Arg Leu Arg Met
390                 395                 400                 405

gag att gat tct tca cct cag gaa atc gat gag ctg gag cgt atc gtc     1363
Glu Ile Asp Ser Ser Pro Gln Glu Ile Asp Glu Leu Glu Arg Ile Val
                410                 415                 420

cgc cgc ctc gag atc gaa gag atg gcg ctg tcc aag gaa tcc gat gca     1411
Arg Arg Leu Glu Ile Glu Glu Met Ala Leu Ser Lys Glu Ser Asp Ala
            425                 430                 435

gct tcc aag gaa cgt cta gaa aag ctg cgc tcg gaa ctt gct gat gaa     1459
Ala Ser Lys Glu Arg Leu Glu Lys Leu Arg Ser Glu Leu Ala Asp Glu
        440                 445                 450

cgc gaa aag ctc tct gag ttg aag gct cgt tgg cag aat gag aaa act     1507
Arg Glu Lys Leu Ser Glu Leu Lys Ala Arg Trp Gln Asn Glu Lys Thr
    455                 460                 465

gct att gac gat gtc cgg gag atg aaa gaa gag ctg gaa gcg ctg cgt     1555
Ala Ile Asp Asp Val Arg Glu Met Lys Glu Glu Leu Glu Ala Leu Arg
470                 475                 480                 485

tct gag tcg gat att gca aaa cgt gac ggc aat tat tgt cgt gtc gca     1603
Ser Glu Ser Asp Ile Ala Lys Arg Asp Gly Asn Tyr Cys Arg Val Ala
                490                 495                 500

aag ctt cgc tac ggc cga atc cct gag ctg gaa aag cag atc gag gat     1651
Lys Leu Arg Tyr Gly Arg Ile Pro Glu Leu Glu Lys Gln Ile Glu Asp
            505                 510                 515

gca gaa tcc aag gtc gag gtc aat gaa aat gcc atg ctc act gag gag     1699
Ala Glu Ser Lys Val Glu Val Asn Glu Asn Ala Met Leu Thr Glu Glu
        520                 525                 530

gtc acg cca gac acg atc gcc gat gtg gtt tcc gca tgg acg ggc att     1747
Val Thr Pro Asp Thr Ile Ala Asp Val Val Ser Ala Trp Thr Gly Ile
    535                 540                 545

cct gca ggc aag atg atg cag ggt gag acc gag aag ctg ctc aac atg     1795
Pro Ala Gly Lys Met Met Gln Gly Glu Thr Glu Lys Leu Leu Asn Met
550                 555                 560                 565

gag cgc gtc ttg ggc aac ccg                                         1816
Glu Arg Val Leu Gly Asn Pro
                570


<210> 68
<211> 572
<212> PRT
<213> Corynebacterium glutamicum

<400> 68
Met Ser Ser Phe Asn Pro Thr Thr Lys Thr Asn Glu Ala Met Gln Ala
  1               5                  10                  15

Ala Leu Gln Gln Ala Ser Ser Ala Gly Asn Pro Asp Ile Arg Pro Ala
             20                  25                  30

His Leu Leu Ala Ala Ile Leu Glu Gln Thr Asp Gly Val Ala Ala Pro
         35                  40                  45

Val Leu Met Ala Thr Gly Val Asp Pro Lys Glu Ile Leu Ala Glu Ala
     50                  55                  60

Lys Lys Leu Val Ala Ser Tyr Pro Lys Ala Ser Gly Ala Asn Met Ala
 65                  70                  75                  80

Asn Pro Asn Phe Asn Arg Asp Ala Leu Asn Ala Phe Thr Ala Ala Gln
                 85                  90                  95

Glu Leu Ala Gly Glu Leu Gly Asp Glu Tyr Val Ser Thr Glu Val Leu
            100                 105                 110

Leu Ala Gly Ile Ala Arg Gly Lys Ser Asp Ala Ala Asp Leu Leu Thr
        115                 120                 125

Asn Lys Gly Ala Thr Tyr Asp Ala Ile Lys Glu Ala Phe Pro Ser Val
    130                 135                 140

Arg Gly Ser Gln Arg Val Thr Thr Gln Asp Pro Glu Gly Gln Phe Gln
145                 150                 155                 160

Ala Leu Glu Lys Tyr Ser Thr Asp Leu Thr Lys Leu Ala Arg Glu Gly
                165                 170                 175

Lys Ile Asp Pro Val Ile Gly Arg Asp Gln Glu Ile Arg Arg Val Val
            180                 185                 190

Gln Val Leu Ser Arg Arg Thr Lys Asn Asn Pro Val Leu Ile Gly Glu
        195                 200                 205

Pro Gly Val Gly Lys Thr Ala Ile Val Glu Gly Leu Ala Arg Arg Ile
    210                 215                 220

Val Ala Gly Asp Val Pro Glu Ser Leu Lys Gly Lys Thr Leu Ile Ser
225                 230                 235                 240

Leu Asp Leu Gly Ser Met Val Ala Gly Ala Lys Tyr Arg Gly Glu Phe
                245                 250                 255

Glu Glu Arg Leu Lys Ala Val Leu Asp Glu Ile Lys Gly Ala Asn Gly
            260                 265                 270

Glu Val Val Thr Phe Ile Asp Glu Leu His Thr Ile Val Gly Ala Gly
        275                 280                 285

Ala Ser Gly Glu Ser Ala Met Asp Ala Gly Asn Met Ile Lys Pro Leu
    290                 295                 300

Leu Ala Arg Gly Glu Leu Arg Leu Val Gly Ala Thr Thr Leu Asn Glu
305                 310                 315                 320

Tyr Arg Lys Tyr Ile Glu Lys Asp Ala Ala Leu Glu Arg Arg Phe Gln

325                 330                 335

Gln Val Tyr Val Gly Glu Pro Thr Val Glu Asp Ala Ile Gly Ile Leu
            340                 345                 350

Arg Gly Leu Lys Glu Arg Tyr Glu Val His His Gly Val Arg Ile Gln
        355                 360                 365

Asp Ser Ala Leu Val Ala Ala Ala Glu Leu Ser Asn Arg Tyr Ile Thr
    370                 375                 380

Ser Arg Phe Leu Pro Asp Lys Ala Ile Asp Leu Val Asp Glu Ala Ala
385                 390                 395                 400

Ser Arg Leu Arg Met Glu Ile Asp Ser Ser Pro Gln Glu Ile Asp Glu
                405                 410                 415

Leu Glu Arg Ile Val Arg Arg Leu Glu Ile Glu Glu Met Ala Leu Ser
            420                 425                 430

Lys Glu Ser Asp Ala Ala Ser Lys Glu Arg Leu Glu Lys Leu Arg Ser
        435                 440                 445

Glu Leu Ala Asp Glu Arg Glu Lys Leu Ser Glu Leu Lys Ala Arg Trp
    450                 455                 460

Gln Asn Glu Lys Thr Ala Ile Asp Asp Val Arg Glu Met Lys Glu Glu
465                 470                 475                 480

Leu Glu Ala Leu Arg Ser Glu Ser Asp Ile Ala Lys Arg Asp Gly Asn
                485                 490                 495

Tyr Cys Arg Val Ala Lys Leu Arg Tyr Gly Arg Ile Pro Glu Leu Glu
            500                 505                 510

Lys Gln Ile Glu Asp Ala Glu Ser Lys Val Glu Val Asn Glu Asn Ala
        515                 520                 525

Met Leu Thr Glu Glu Val Thr Pro Asp Thr Ile Ala Asp Val Val Ser
    530                 535                 540

Ala Trp Thr Gly Ile Pro Ala Gly Lys Met Met Gln Gly Glu Thr Glu
545                 550                 555                 560

Lys Leu Leu Asn Met Glu Arg Val Leu Gly Asn Pro
                565                 570


<210> 69
<211> 1401
<212> DNA
<213> Corynebacterium glutamicum

<220>
<221> CDS
<222> (101)..(1378)
<223> RXN01120

<400> 69
acaggtaaag cgctaagatg gaacaaccca ttgccaatat tgttggttag agttgtacgc 60

agtaaatctt ttcaatcgtg gaagcgggtc tcacagtcta atg gca cgt atg cag   115
                                           Met Ala Arg Met Gln
                                           1               5

gaa agc gcc gat ctg ctc aaa tgt tcc ttc tgc gga aag agc caa aag   163
Glu Ser Ala Asp Leu Leu Lys Cys Ser Phe Cys Gly Lys Ser Gln Lys
                10                  15                  20

cag gta aaa aaa ctc atc gcg ggt ggc gcc gta tat atc tgt gat gag   211
Gln Val Lys Lys Leu Ile Ala Gly Gly Ala Val Tyr Ile Cys Asp Glu
            25                  30                  35

tgc att gag ctg tgc aac gag att att gaa gaa gaa ctc ggt caa gct   259
Cys Ile Glu Leu Cys Asn Glu Ile Ile Glu Glu Glu Leu Gly Gln Ala
        40                  45                  50

caa cac gac gag cag gag cgc aac gag ctc ccc aag ccg tcg gag att     307
Gln His Asp Glu Gln Glu Arg Asn Glu Leu Pro Lys Pro Ser Glu Ile
     55                  60                  65

tca gcc ttc ctt gat act tat gtc atc ggg cag gac cca gca aaa cgt     355
Ser Ala Phe Leu Asp Thr Tyr Val Ile Gly Gln Asp Pro Ala Lys Arg
 70                  75                  80                  85

atc ctg tcg gtt gcg gtg tac aac cat tac aag cgt ctc cgc gca tcg     403
Ile Leu Ser Val Ala Val Tyr Asn His Tyr Lys Arg Leu Arg Ala Ser
                 90                  95                 100

gaa acc atc ggt cgt cgc agg aat gac gag cct gaa acc gaa ctg gtt     451
Glu Thr Ile Gly Arg Arg Arg Asn Asp Glu Pro Glu Thr Glu Leu Val
            105                 110                 115

aag tcc aat att ttg atg ctc ggc ccc act ggc tcc ggc aag act ttc     499
Lys Ser Asn Ile Leu Met Leu Gly Pro Thr Gly Ser Gly Lys Thr Phe
        120                 125                 130

ctt gcc cag act ttg gca aag ctg ctg gat gtt cct ttt gct atc gcg     547
Leu Ala Gln Thr Leu Ala Lys Leu Leu Asp Val Pro Phe Ala Ile Ala
    135                 140                 145

gat gcc acc tca ctg acc gag gct ggt tat gtg ggc gag gat gtg gaa     595
Asp Ala Thr Ser Leu Thr Glu Ala Gly Tyr Val Gly Glu Asp Val Glu
150                 155                 160                 165

aac atc ttg ctc aag ctg ctt cag gct gct gat ttt gat gtg gaa cgt     643
Asn Ile Leu Leu Lys Leu Leu Gln Ala Ala Asp Phe Asp Val Glu Arg
                170                 175                 180

gca cag cgc ggc atc att tac atc gat gaa gtg gac aag att tcc cgc     691
Ala Gln Arg Gly Ile Ile Tyr Ile Asp Glu Val Asp Lys Ile Ser Arg
            185                 190                 195

aag tct gaa aac cca tcg atc act cgc gat gtt tcc ggt gaa ggc gtg     739
Lys Ser Glu Asn Pro Ser Ile Thr Arg Asp Val Ser Gly Glu Gly Val
        200                 205                 210

cag cag gca ctg ctg aaa att ttg gaa ggc act gtc gcc gca atc cca     787
Gln Gln Ala Leu Leu Lys Ile Leu Glu Gly Thr Val Ala Ala Ile Pro
    215                 220                 225

ccg cag gga gga cgc aag cac ccc aac cag gat ttc atc cag ctg gat     835
Pro Gln Gly Gly Arg Lys His Pro Asn Gln Asp Phe Ile Gln Leu Asp
230                 235                 240                 245

acc acc aac att ttg ttc atc gtt gct ggt gcg ttc tct ggt ctg gag     883
Thr Thr Asn Ile Leu Phe Ile Val Ala Gly Ala Phe Ser Gly Leu Glu
                250                 255                 260

aag gtc atc gcg gac cgc aat ggc aag aaa ggc ttg ggc ttc ggt gtg     931
Lys Val Ile Ala Asp Arg Asn Gly Lys Lys Gly Leu Gly Phe Gly Val
            265                 270                 275

gag gtc tct tcc aag aag gaa gaa gcc aac att gtg gat atc ttc aag     979
Glu Val Ser Ser Lys Lys Glu Glu Ala Asn Ile Val Asp Ile Phe Lys
        280                 285                 290

gat gtc ctc cct gag gac ctg gtg aag ttt ggt ctc atc cca gaa ttc    1027
Asp Val Leu Pro Glu Asp Leu Val Lys Phe Gly Leu Ile Pro Glu Phe
    295                 300                 305

att ggg cgt ctg cca gtc gtt gcc acc gta tcc aac ctg gat cag aaa    1075
Ile Gly Arg Leu Pro Val Val Ala Thr Val Ser Asn Leu Asp Gln Lys
310                 315                 320                 325

tct ctg gtc aag gtt ctc acg gag cct cgt aac tca ttg gtg aag cag    1123
Ser Leu Val Lys Val Leu Thr Glu Pro Arg Asn Ser Leu Val Lys Gln
                330                 335                 340

tat cga cgt ctg ttt gaa atg gat gac gct gtg ttg acc ttt act gat    1171
Tyr Arg Arg Leu Phe Glu Met Asp Asp Ala Val Leu Thr Phe Thr Asp
            345                 350                 355

gat gct ttg gag gag atc gct aat cag gca ctc gag cgc aaa act ggc    1219

Asp Ala Leu Glu Glu Ile Ala Asn Gln Ala Leu Glu Arg Lys Thr Gly
        360                 365                 370

gcc cgt ggc ctg cgc gcg atc atg gaa gag atc ctg gtt ccg atc atg      1267
Ala Arg Gly Leu Arg Ala Ile Met Glu Glu Ile Leu Val Pro Ile Met
    375                 380                 385

tat gac ctc cca gac cgt aaa gac gtt ggc gaa gtc atc atc aac ggt      1315
Tyr Asp Leu Pro Asp Arg Lys Asp Val Gly Glu Val Ile Ile Asn Gly
390                 395                 400                 405

gcc gtt gcc cgt ggc gaa gcc gaa cca gag atg ttg gaa gct gtc gca      1363
Ala Val Ala Arg Gly Glu Ala Glu Pro Glu Met Leu Glu Ala Val Ala
                410                 415                 420

gaa gaa aag acc gcg tagttggcag gagttatcac cgg                        1401
Glu Glu Lys Thr Ala
            425


<210> 70
<211> 426
<212> PRT
<213> Corynebacterium glutamicum

<400> 70
Met Ala Arg Met Gln Glu Ser Ala Asp Leu Leu Lys Cys Ser Phe Cys
  1               5                  10                  15

Gly Lys Ser Gln Lys Gln Val Lys Lys Leu Ile Ala Gly Gly Ala Val
             20                  25                  30

Tyr Ile Cys Asp Glu Cys Ile Glu Leu Cys Asn Glu Ile Ile Glu Glu
         35                  40                  45

Glu Leu Gly Gln Ala Gln His Asp Glu Gln Glu Arg Asn Glu Leu Pro
     50                  55                  60

Lys Pro Ser Glu Ile Ser Ala Phe Leu Asp Thr Tyr Val Ile Gly Gln
 65                  70                  75                  80

Asp Pro Ala Lys Arg Ile Leu Ser Val Ala Val Tyr Asn His Tyr Lys
                 85                  90                  95

Arg Leu Arg Ala Ser Glu Thr Ile Gly Arg Arg Arg Asn Asp Glu Pro
            100                 105                 110

Glu Thr Glu Leu Val Lys Ser Asn Ile Leu Met Leu Gly Pro Thr Gly
        115                 120                 125

Ser Gly Lys Thr Phe Leu Ala Gln Thr Leu Ala Lys Leu Leu Asp Val
    130                 135                 140

Pro Phe Ala Ile Ala Asp Ala Thr Ser Leu Thr Glu Ala Gly Tyr Val
145                 150                 155                 160

Gly Glu Asp Val Glu Asn Ile Leu Leu Lys Leu Leu Gln Ala Ala Asp
                165                 170                 175

Phe Asp Val Glu Arg Ala Gln Arg Gly Ile Ile Tyr Ile Asp Glu Val
            180                 185                 190

Asp Lys Ile Ser Arg Lys Ser Glu Asn Pro Ser Ile Thr Arg Asp Val
        195                 200                 205

Ser Gly Glu Gly Val Gln Gln Ala Leu Leu Lys Ile Leu Glu Gly Thr
    210                 215                 220

Val Ala Ala Ile Pro Pro Gln Gly Gly Arg Lys His Pro Asn Gln Asp
225                 230                 235                 240

Phe Ile Gln Leu Asp Thr Thr Asn Ile Leu Phe Ile Val Ala Gly Ala
                245                 250                 255

Phe Ser Gly Leu Glu Lys Val Ile Ala Asp Arg Asn Gly Lys Lys Gly
            260                 265                 270

Leu Gly Phe Gly Val Glu Val Ser Ser Lys Lys Glu Glu Ala Asn Ile
        275                 280                 285

Val Asp Ile Phe Lys Asp Val Leu Pro Glu Asp Leu Val Lys Phe Gly
    290                 295                 300

Leu Ile Pro Glu Phe Ile Gly Arg Leu Pro Val Val Ala Thr Val Ser
305                 310                 315                 320

Asn Leu Asp Gln Lys Ser Leu Val Lys Val Leu Thr Glu Pro Arg Asn
                325                 330                 335

Ser Leu Val Lys Gln Tyr Arg Arg Leu Phe Glu Met Asp Asp Ala Val
            340                 345                 350

Leu Thr Phe Thr Asp Asp Ala Leu Glu Glu Ile Ala Asn Gln Ala Leu
        355                 360                 365

Glu Arg Lys Thr Gly Ala Arg Gly Leu Arg Ala Ile Met Glu Glu Ile
    370                 375                 380

Leu Val Pro Ile Met Tyr Asp Leu Pro Asp Arg Lys Asp Val Gly Glu
385                 390                 395                 400

Val Ile Ile Asn Gly Ala Val Ala Arg Gly Glu Ala Glu Pro Glu Met
                405                 410                 415

Leu Glu Ala Val Ala Glu Glu Lys Thr Ala
            420                 425


<210> 71
<211> 1401
<212> DNA
<213> Corynebacterium glutamicum

<220>
<221> CDS
<222> (101)..(1378)
<223> FRXA01120

<400> 71
acaggtaaag cgctaagatg gaacaaccca ttgccaatat tgttggttag agttgtacgc 60

agtaaatctt ttcaatcgtg gaagcgggtc tcacagtcta atg gca cgt atg cag   115
                                            Met Ala Arg Met Gln
                                            1               5

gaa agc gcc gat ctg ctc aaa tgt tcc ttc tgc gga aag agc caa aag   163
Glu Ser Ala Asp Leu Leu Lys Cys Ser Phe Cys Gly Lys Ser Gln Lys
                10                  15                  20

cag gta aaa aaa ctc atc gcg ggt ggc gcc gta tat atc tgt gat gag   211
Gln Val Lys Lys Leu Ile Ala Gly Gly Ala Val Tyr Ile Cys Asp Glu
            25                  30                  35

tgc att gag ctg tgc aac gag att att gaa gaa gaa ctc ggt caa gct   259
Cys Ile Glu Leu Cys Asn Glu Ile Ile Glu Glu Glu Leu Gly Gln Ala
        40                  45                  50

caa cac gac gag cag gag cgc aac gag ctc ccc aag ccg tcg gag att   307
Gln His Asp Glu Gln Glu Arg Asn Glu Leu Pro Lys Pro Ser Glu Ile
    55                  60                  65

tca gcc ttc ctt gat act tat gtc atc ggg cag gac cca gca aaa cgt   355
Ser Ala Phe Leu Asp Thr Tyr Val Ile Gly Gln Asp Pro Ala Lys Arg
70                  75                  80                  85

atc ctg tcg gtt gcg gtg tac aac cat tac aag cgt ctc cgc gca tcg   403
Ile Leu Ser Val Ala Val Tyr Asn His Tyr Lys Arg Leu Arg Ala Ser
                90                  95                  100

gaa acc atc ggt cgt cgc agg aat gac gag cct gaa acc gaa ctg gtt   451
Glu Thr Ile Gly Arg Arg Arg Asn Asp Glu Pro Glu Thr Glu Leu Val
            105                 110                 115

aag tcc aat att ttg atg ctc ggc ccc act ggc tcc ggc aag act ttc   499

Lys Ser Asn Ile Leu Met Leu Gly Pro Thr Gly Ser Gly Lys Thr Phe
        120                 125                 130

ctt gcc cag act ttg gca aag ctg ctg gat gtt cct ttt gct atc gcg     547
Leu Ala Gln Thr Leu Ala Lys Leu Leu Asp Val Pro Phe Ala Ile Ala
    135                 140                 145

gat gcc acc tca ctg acc gag gct ggt tat gtg ggc gag gat gtg gaa     595
Asp Ala Thr Ser Leu Thr Glu Ala Gly Tyr Val Gly Glu Asp Val Glu
150                 155                 160                 165

aac atc ttg ctc aag ctg ctt cag gct gct gat ttt gat gtg gaa cgt     643
Asn Ile Leu Leu Lys Leu Leu Gln Ala Ala Asp Phe Asp Val Glu Arg
                170                 175                 180

gca cag cgc ggc atc att tac atc gat gaa gtg gac aag att tcc cgc     691
Ala Gln Arg Gly Ile Ile Tyr Ile Asp Glu Val Asp Lys Ile Ser Arg
            185                 190                 195

aag tct gaa aac cca tcg atc act cgc gat gtt tcc ggt gaa ggc gtg     739
Lys Ser Glu Asn Pro Ser Ile Thr Arg Asp Val Ser Gly Glu Gly Val
        200                 205                 210

cag cag gca ctg ctg aaa att ttg gaa ggc act gtc gcc gca atc cca     787
Gln Gln Ala Leu Leu Lys Ile Leu Glu Gly Thr Val Ala Ala Ile Pro
    215                 220                 225

ccg cag gga gga cgc aag cac ccc aac cag gat ttc atc cag ctg gat     835
Pro Gln Gly Gly Arg Lys His Pro Asn Gln Asp Phe Ile Gln Leu Asp
230                 235                 240                 245

acc acc aac att ttg ttc atc gtt gct ggt gcg ttc tct ggt ctg gag     883
Thr Thr Asn Ile Leu Phe Ile Val Ala Gly Ala Phe Ser Gly Leu Glu
                250                 255                 260

aag gtc atc gcg gac cgc aat ggc aag aaa ggc ttg ggc ttc ggt gtg     931
Lys Val Ile Ala Asp Arg Asn Gly Lys Lys Gly Leu Gly Phe Gly Val
            265                 270                 275

gag gtc tct tcc aag aag gaa gaa gcc aac att gtg gat atc ttc aag     979
Glu Val Ser Ser Lys Lys Glu Glu Ala Asn Ile Val Asp Ile Phe Lys
        280                 285                 290

gat gtc ctc cct gag gac ctg gtg aag ttt ggt ctc atc cca gaa ttc     1027
Asp Val Leu Pro Glu Asp Leu Val Lys Phe Gly Leu Ile Pro Glu Phe
    295                 300                 305

att ggg cgt ctg cca gtc gtt gcc acc gta tcc aac ctg gat cag aaa     1075
Ile Gly Arg Leu Pro Val Val Ala Thr Val Ser Asn Leu Asp Gln Lys
310                 315                 320                 325

tct ctg gtc aag gtt ctc acg gag cct cgt aac tca ttg gtg aag cag     1123
Ser Leu Val Lys Val Leu Thr Glu Pro Arg Asn Ser Leu Val Lys Gln
                330                 335                 340

tat cga cgt ctg ttt gaa atg gat gac gct gtg ttg acc ttt act gat     1171
Tyr Arg Arg Leu Phe Glu Met Asp Asp Ala Val Leu Thr Phe Thr Asp
            345                 350                 355

gat gct ttg gag gag atc gct aat cag gca ctc gag cgc aaa act ggc     1219
Asp Ala Leu Glu Glu Ile Ala Asn Gln Ala Leu Glu Arg Lys Thr Gly
        360                 365                 370

gcc cgt ggc ctg cgc gcg atc atg gaa gag atc ctg gtt ccg atc atg     1267
Ala Arg Gly Leu Arg Ala Ile Met Glu Glu Ile Leu Val Pro Ile Met
    375                 380                 385

tat gac ctc cca gac cgt aaa gac gtt ggc gaa gtc atc atc aac ggt     1315
Tyr Asp Leu Pro Asp Arg Lys Asp Val Gly Glu Val Ile Ile Asn Gly
390                 395                 400                 405

gcc gtt gcc cgt ggc gaa gcc gaa cca gag atg ttg gaa gct gtc gca     1363
Ala Val Ala Arg Gly Glu Ala Glu Pro Glu Met Leu Glu Ala Val Ala
                410                 415                 420

gaa gaa aag acc gcg tagttggcag gagttatcac cgg                       1401
Glu Glu Lys Thr Ala

            425

<210> 72
<211> 426
<212> PRT
<213> Corynebacterium glutamicum

<400> 72
Met Ala Arg Met Gln Glu Ser Ala Asp Leu Leu Lys Cys Ser Phe Cys
1               5                   10                  15

Gly Lys Ser Gln Lys Gln Val Lys Lys Leu Ile Ala Gly Gly Ala Val
            20                  25                  30

Tyr Ile Cys Asp Glu Cys Ile Glu Leu Cys Asn Glu Ile Ile Glu Glu
        35                  40                  45

Glu Leu Gly Gln Ala Gln His Asp Glu Gln Glu Arg Asn Glu Leu Pro
    50                  55                  60

Lys Pro Ser Glu Ile Ser Ala Phe Leu Asp Thr Tyr Val Ile Gly Gln
65                  70                  75                  80

Asp Pro Ala Lys Arg Ile Leu Ser Val Ala Val Tyr Asn His Tyr Lys
                85                  90                  95

Arg Leu Arg Ala Ser Glu Thr Ile Gly Arg Arg Arg Asn Asp Glu Pro
            100                 105                 110

Glu Thr Glu Leu Val Lys Ser Asn Ile Leu Met Leu Gly Pro Thr Gly
        115                 120                 125

Ser Gly Lys Thr Phe Leu Ala Gln Thr Leu Ala Lys Leu Leu Asp Val
    130                 135                 140

Pro Phe Ala Ile Ala Asp Ala Thr Ser Leu Thr Glu Ala Gly Tyr Val
145                 150                 155                 160

Gly Glu Asp Val Glu Asn Ile Leu Leu Lys Leu Leu Gln Ala Ala Asp
                165                 170                 175

Phe Asp Val Glu Arg Ala Gln Arg Gly Ile Ile Tyr Ile Asp Glu Val
            180                 185                 190

Asp Lys Ile Ser Arg Lys Ser Glu Asn Pro Ser Ile Thr Arg Asp Val
        195                 200                 205

Ser Gly Glu Gly Val Gln Gln Ala Leu Leu Lys Ile Leu Glu Gly Thr
    210                 215                 220

Val Ala Ala Ile Pro Pro Gln Gly Gly Arg Lys His Pro Asn Gln Asp
225                 230                 235                 240

Phe Ile Gln Leu Asp Thr Thr Asn Ile Leu Phe Ile Val Ala Gly Ala
                245                 250                 255

Phe Ser Gly Leu Glu Lys Val Ile Ala Asp Arg Asn Gly Lys Lys Gly
            260                 265                 270

Leu Gly Phe Gly Val Glu Val Ser Ser Lys Lys Glu Glu Ala Asn Ile
        275                 280                 285

Val Asp Ile Phe Lys Asp Val Leu Pro Glu Asp Leu Val Lys Phe Gly
    290                 295                 300

Leu Ile Pro Glu Phe Ile Gly Arg Leu Pro Val Val Ala Thr Val Ser
305                 310                 315                 320

Asn Leu Asp Gln Lys Ser Leu Val Lys Val Leu Thr Glu Pro Arg Asn
                325                 330                 335

Ser Leu Val Lys Gln Tyr Arg Arg Leu Phe Glu Met Asp Asp Ala Val
            340                 345                 350

Leu Thr Phe Thr Asp Asp Ala Leu Glu Glu Ile Ala Asn Gln Ala Leu
        355                 360                 365

Glu Arg Lys Thr Gly Ala Arg Gly Leu Arg Ala Ile Met Glu Glu Ile
    370                 375                 380

Leu Val Pro Ile Met Tyr Asp Leu Pro Asp Arg Lys Asp Val Gly Glu
385                 390                 395                 400

Val Ile Ile Asn Gly Ala Val Ala Arg Gly Glu Ala Glu Pro Glu Met
                405                 410                 415

Leu Glu Ala Val Ala Glu Glu Lys Thr Ala
            420                 425


<210> 73
<211> 1065
<212> DNA
<213> Corynebacterium glutamicum

<220>
<221> CDS
<222> (101)..(1042)
<223> RXA00744

<400> 73
tctgagtcgg tagaagtatt acccagtgac ttaagtttct tagatttttt tgagcaacag 60

cgaccagcca cgttagtgtg gtcgagtaga ggatagctac atg ggg aac tgg gca    115
                                           Met Gly Asn Trp Ala
                                           1               5

gag att act gat gaa att tct aag att tac caa gat aat cag tac aag    163
Glu Ile Thr Asp Glu Ile Ser Lys Ile Tyr Gln Asp Asn Gln Tyr Lys
                10                  15                  20

att aga caa ata aat gat gtt gac gca gta agc gat aaa cgt aga gaa    211
Ile Arg Gln Ile Asn Asp Val Asp Ala Val Ser Asp Lys Arg Arg Glu
            25                  30                  35

gcg cta caa gca ctg ttt gaa cat act ggt cga aat gta atc gtc tat    259
Ala Leu Gln Ala Leu Phe Glu His Thr Gly Arg Asn Val Ile Val Tyr
        40                  45                  50

tat tca gcg tgg tta gaa aat ggt cga cga ttt tcc ggg caa tct acg    307
Tyr Ser Ala Trp Leu Glu Asn Gly Arg Arg Phe Ser Gly Gln Ser Thr
    55                  60                  65

gat ttt tcg gta aat gat act gat aaa aac agt ttt atg act gcg ctc    355
Asp Phe Ser Val Asn Asp Thr Asp Lys Asn Ser Phe Met Thr Ala Leu
70                  75                  80                  85

cat aag ttg gat cag agt aaa ggt ctc gat ctt atc ctc cac act ccg    403
His Lys Leu Asp Gln Ser Lys Gly Leu Asp Leu Ile Leu His Thr Pro
                90                  95                  100

ggt gga gat gtt gct gcg aca gag tcg tta gta gat tac att cac gca    451
Gly Gly Asp Val Ala Ala Thr Glu Ser Leu Val Asp Tyr Ile His Ala
            105                 110                 115

ctc ttt ggt caa gat ttc aga gtc att gtc ccc caa ctc gca atg tca    499
Leu Phe Gly Gln Asp Phe Arg Val Ile Val Pro Gln Leu Ala Met Ser
        120                 125                 130

gca gga aca atg atc gca ctt tcg tcc aaa gag att gtt atg ggg aag    547
Ala Gly Thr Met Ile Ala Leu Ser Ser Lys Glu Ile Val Met Gly Lys
    135                 140                 145

cat tct agt ctt ggc ccc att gat cct cag ttt aac ggc cta ccg gca    595
His Ser Ser Leu Gly Pro Ile Asp Pro Gln Phe Asn Gly Leu Pro Ala
150                 155                 160                 165

cac ggg tta ttg gaa gaa ttt gag caa gcg aag aaa gag gtc tct gag    643
His Gly Leu Leu Glu Glu Phe Glu Gln Ala Lys Lys Glu Val Ser Glu
                170                 175                 180

aat ccg cag act gct cat ata tgg cag gtg atc ttg aat aaa tac aac    691
Asn Pro Gln Thr Ala His Ile Trp Gln Val Ile Leu Asn Lys Tyr Asn

185                 190                 195

ccc acg atg ttg ggt gaa gct aaa aaa gct att cag tgg tcc aac tcg     739
Pro Thr Met Leu Gly Glu Ala Lys Lys Ala Ile Gln Trp Ser Asn Ser
        200                 205                 210

atg gtt aag cag tgg ctt gaa aag ggt atg ttt tta gac gag cct gac     787
Met Val Lys Gln Trp Leu Glu Lys Gly Met Phe Leu Asp Glu Pro Asp
    215                 220                 225

aaa gaa gaa aaa gcc act cgc gct atc aaa gag ctc gct gat cat tcc     835
Lys Glu Glu Lys Ala Thr Arg Ala Ile Lys Glu Leu Ala Asp His Ser
230                 235                 240                 245

gtt act ctt gcg cat aat cga cac att tcg gtc agt aaa gca ctt gag     883
Val Thr Leu Ala His Asn Arg His Ile Ser Val Ser Lys Ala Leu Glu
                250                 255                 260

ctg gga ttg aat atc aaa gaa ctt gag agc gat cca aag ctt caa gat     931
Leu Gly Leu Asn Ile Lys Glu Leu Glu Ser Asp Pro Lys Leu Gln Asp
            265                 270                 275

tta gtt ctt act ctt cac cac ctg tcc gtt att gct gcg caa cga gga     979
Leu Val Leu Thr Leu His His Leu Ser Val Ile Ala Ala Gln Arg Gly
        280                 285                 290

cca tta att aag ttt gtc gtc aat cat gac aac cgt ggc act ttt ctg     1027
Pro Leu Ile Lys Phe Val Val Asn His Asp Asn Arg Gly Thr Phe Leu
    295                 300                 305

cag ggg cat gaa aac taattaagtg atgcaatagt cta                       1065
Gln Gly His Glu Asn
310


<210> 74
<211> 314
<212> PRT
<213> Corynebacterium glutamicum

<400> 74
Met Gly Asn Trp Ala Glu Ile Thr Asp Glu Ile Ser Lys Ile Tyr Gln
1               5                   10                  15

Asp Asn Gln Tyr Lys Ile Arg Gln Ile Asn Asp Val Asp Ala Val Ser
            20                  25                  30

Asp Lys Arg Arg Glu Ala Leu Gln Ala Leu Phe Glu His Thr Gly Arg
        35                  40                  45

Asn Val Ile Val Tyr Tyr Ser Ala Trp Leu Glu Asn Gly Arg Arg Phe
    50                  55                  60

Ser Gly Gln Ser Thr Asp Phe Ser Val Asn Asp Thr Asp Lys Asn Ser
65                  70                  75                  80

Phe Met Thr Ala Leu His Lys Leu Asp Gln Ser Lys Gly Leu Asp Leu
                85                  90                  95

Ile Leu His Thr Pro Gly Gly Asp Val Ala Ala Thr Glu Ser Leu Val
            100                 105                 110

Asp Tyr Ile His Ala Leu Phe Gly Gln Asp Phe Arg Val Ile Val Pro
        115                 120                 125

Gln Leu Ala Met Ser Ala Gly Thr Met Ile Ala Leu Ser Ser Lys Glu
    130                 135                 140

Ile Val Met Gly Lys His Ser Ser Leu Gly Pro Ile Asp Pro Gln Phe
145                 150                 155                 160

Asn Gly Leu Pro Ala His Gly Leu Leu Glu Glu Phe Glu Gln Ala Lys
                165                 170                 175

Lys Glu Val Ser Glu Asn Pro Gln Thr Ala His Ile Trp Gln Val Ile
            180                 185                 190

Leu Asn Lys Tyr Asn Pro Thr Met Leu Gly Glu Ala Lys Lys Ala Ile
        195                 200                 205

Gln Trp Ser Asn Ser Met Val Lys Gln Trp Leu Glu Lys Gly Met Phe
    210                 215                 220

Leu Asp Glu Pro Asp Lys Glu Glu Lys Ala Thr Arg Ala Ile Lys Glu
225                 230                 235                 240

Leu Ala Asp His Ser Val Thr Leu Ala His Asn Arg His Ile Ser Val
                245                 250                 255

Ser Lys Ala Leu Glu Leu Gly Leu Asn Ile Lys Glu Leu Glu Ser Asp
            260                 265                 270

Pro Lys Leu Gln Asp Leu Val Leu Thr Leu His His Leu Ser Val Ile
        275                 280                 285

Ala Ala Gln Arg Gly Pro Leu Ile Lys Phe Val Val Asn His Asp Asn
    290                 295                 300

Arg Gly Thr Phe Leu Gln Gly His Glu Asn
305                 310


<210> 75
<211> 957
<212> DNA
<213> Corynebacterium glutamicum

<220>
<221> CDS
<222> (101)..(934)
<223> RXA00844

<400> 75
ggtgacggcc aaaggcgcgc gcattgcgcg ccccaaaatc gacgtcattg acagcatctt 60

gtgacaacat tttgtagagc aaccatctag actgttcttt atg tct tct gcg tca    115
                                            Met Ser Ser Ala Ser
                                            1               5

ttt acc acc aaa gca ctg tcc gta ctc gca gct tta acg gct gcg tct    163
Phe Thr Thr Lys Ala Leu Ser Val Leu Ala Ala Leu Thr Ala Ala Ser
                10                  15                  20

gcc ccc tta gtg gcg gcg tca cct gca cat gct ttg gca aat gct cgc    211
Ala Pro Leu Val Ala Ala Ser Pro Ala His Ala Leu Ala Asn Ala Arg
            25                  30                  35

aac gtt acg ggt tca agc acc act tca gat tca att gtt cgt ctg cac    259
Asn Val Thr Gly Ser Ser Thr Thr Ser Asp Ser Ile Val Arg Leu His
        40                  45                  50

atc ggt aac act gca tgt aca gga acc atg atc acc cca acg tgg gcg    307
Ile Gly Asn Thr Ala Cys Thr Gly Thr Met Ile Thr Pro Thr Trp Ala
    55                  60                  65

atc acc gcc cgc cac tgt atc cct gag ggc ggt att gcc ggt gca gct    355
Ile Thr Ala Arg His Cys Ile Pro Glu Gly Gly Ile Ala Gly Ala Ala
70                  75                  80                  85

att ggt tca agc act ttg agc caa ttt cag cag gtg tcc caa gcg atc    403
Ile Gly Ser Ser Thr Leu Ser Gln Phe Gln Gln Val Ser Gln Ala Ile
                90                  95                  100

ttg cac cct act gcg gac tta gct ctc gtt gag ctt ccc aat cag gca    451
Leu His Pro Thr Ala Asp Leu Ala Leu Val Glu Leu Pro Asn Gln Ala
            105                 110                 115

agt tcc aac acg gtt gat ctc tac ggt gca cac gtg cag cct ggt gaa    499
Ser Ser Asn Thr Val Asp Leu Tyr Gly Ala His Val Gln Pro Gly Glu
        120                 125                 130

aat ggt caa gca gcc ggc tgg ggt ggg tac tct gcc ttt ggc caa aat    547
Asn Gly Gln Ala Ala Gly Trp Gly Gly Tyr Ser Ala Phe Gly Gln Asn
    135                 140                 145

gtt gca cag caa gcc gat gtg caa att caa cgc agg gta gtc aat gtg     595
Val Ala Gln Gln Ala Asp Val Gln Ile Gln Arg Arg Val Val Asn Val
150                 155                 160                 165

cca agc ccc gac cgc acc gct gtg ctg ctt gaa ggc act gtt tct aac     643
Pro Ser Pro Asp Arg Thr Ala Val Leu Leu Glu Gly Thr Val Ser Asn
                170                 175                 180

ggt cgt ctc gta cca ggc gat tcc ggc gga cct ttg tac atc aat ggt     691
Gly Arg Leu Val Pro Gly Asp Ser Gly Gly Pro Leu Tyr Ile Asn Gly
            185                 190                 195

caa ctg gct ggt gtg ctc agc atg tcc act gac gta gaa aac gat gca     739
Gln Leu Ala Gly Val Leu Ser Met Ser Thr Asp Val Glu Asn Asp Ala
        200                 205                 210

cta gac ggc acc gtc ggc tgg tac atc ccc gtt gct gaa cac gcc gag     787
Leu Asp Gly Thr Val Gly Trp Tyr Ile Pro Val Ala Glu His Ala Glu
    215                 220                 225

tgg atc gcc tac tac acc ggc aag cac att gcc ccc att gct ggt gcg     835
Trp Ile Ala Tyr Tyr Thr Gly Lys His Ile Ala Pro Ile Ala Gly Ala
230                 235                 240                 245

ccc gca gaa ctt gtt gac gcc acc gcc aac ccc acc ttc atc cct gct     883
Pro Ala Glu Leu Val Asp Ala Thr Ala Asn Pro Thr Phe Ile Pro Ala
                250                 255                 260

cca cag cct ttc acc ggt tca tcc atc ggt ggt tgg gcg ctg ggc agc     931
Pro Gln Pro Phe Thr Gly Ser Ser Ile Gly Gly Trp Ala Leu Gly Ser
            265                 270                 275

tcc tagaatatgc tgatctccct gct                                       957
Ser

<210> 76
<211> 278
<212> PRT
<213> Corynebacterium glutamicum

<400> 76
Met Ser Ser Ala Ser Phe Thr Thr Lys Ala Leu Ser Val Leu Ala Ala
1               5                   10                  15

Leu Thr Ala Ala Ser Ala Pro Leu Val Ala Ala Ser Pro Ala His Ala
            20                  25                  30

Leu Ala Asn Ala Arg Asn Val Thr Gly Ser Ser Thr Thr Ser Asp Ser
        35                  40                  45

Ile Val Arg Leu His Ile Gly Asn Thr Ala Cys Thr Gly Thr Met Ile
    50                  55                  60

Thr Pro Thr Trp Ala Ile Thr Ala Arg His Cys Ile Pro Glu Gly Gly
65                  70                  75                  80

Ile Ala Gly Ala Ala Ile Gly Ser Ser Thr Leu Ser Gln Phe Gln Gln
                85                  90                  95

Val Ser Gln Ala Ile Leu His Pro Thr Ala Asp Leu Ala Leu Val Glu
            100                 105                 110

Leu Pro Asn Gln Ala Ser Ser Asn Thr Val Asp Leu Tyr Gly Ala His
        115                 120                 125

Val Gln Pro Gly Glu Asn Gly Gln Ala Ala Gly Trp Gly Gly Tyr Ser
    130                 135                 140

Ala Phe Gly Gln Asn Val Ala Gln Gln Ala Asp Val Gln Ile Gln Arg
145                 150                 155                 160

Arg Val Val Asn Val Pro Ser Pro Asp Arg Thr Ala Val Leu Leu Glu
                165                 170                 175

Gly Thr Val Ser Asn Gly Arg Leu Val Pro Gly Asp Ser Gly Gly Pro
            180                 185                 190

Leu Tyr Ile Asn Gly Gln Leu Ala Gly Val Leu Ser Met Ser Thr Asp
        195                 200                 205

Val Glu Asn Asp Ala Leu Asp Gly Thr Val Gly Trp Tyr Ile Pro Val
    210                 215                 220

Ala Glu His Ala Glu Trp Ile Ala Tyr Tyr Thr Gly Lys His Ile Ala
225                 230                 235                 240

Pro Ile Ala Gly Ala Pro Ala Glu Leu Val Asp Ala Thr Ala Asn Pro
                245                 250                 255

Thr Phe Ile Pro Ala Pro Gln Pro Phe Thr Gly Ser Ser Ile Gly Gly
            260                 265                 270

Trp Ala Leu Gly Ser Ser
        275


<210> 77
<211> 958
<212> DNA
<213> Corynebacterium glutamicum

<220>
<221> CDS
<222> (101)..(958)
<223> RXA01151

<400> 77
ccaggcgttt ggctgatggc gtggagcctc ctcgtccgca gcgtaaggca cgtcgataaa 60

aagacctagt tggagggcgt aaagggttag agtggtgacc atg agt tca cca act    115
                                            Met Ser Ser Pro Thr
                                            1               5

gat tct tcg ccc tct aat tct ttt agc gac ttc aac cgg gag gaa cag    163
Asp Ser Ser Pro Ser Asn Ser Phe Ser Asp Phe Asn Arg Glu Glu Gln
                10                  15                  20

tcc cgg tta tct gat gag gtg cgc cag ctc aag cgc acc aac tct gat    211
Ser Arg Leu Ser Asp Glu Val Arg Gln Leu Lys Arg Thr Asn Ser Asp
            25                  30                  35

ctt ggg gca cgt aat gcc aag ctc gcg gag atg ctg aag tcg tct cgg    259
Leu Gly Ala Arg Asn Ala Lys Leu Ala Glu Met Leu Lys Ser Ser Arg
        40                  45                  50

gat aaa ttg tct gtg ctg ttt tct cag ttg gag gat atg gct cag ccg    307
Asp Lys Leu Ser Val Leu Phe Ser Gln Leu Glu Asp Met Ala Gln Pro
    55                  60                  65

cca tcg gtg tat ggc act ttc ttg gaa acc gcg aaa gac ggt tct aat    355
Pro Ser Val Tyr Gly Thr Phe Leu Glu Thr Ala Lys Asp Gly Ser Asn
70                  75                  80                  85

gcg gag atc ttt gct ggt gga cgt cgc atg cgt gtg gct gtt tct cct    403
Ala Glu Ile Phe Ala Gly Gly Arg Arg Met Arg Val Ala Val Ser Pro
                90                  95                  100

atg ctg tgt gcc gcg gat ttg atg ccg ggt gtg cag gtt cgt ttg ggt    451
Met Leu Cys Ala Ala Asp Leu Met Pro Gly Val Gln Val Arg Leu Gly
            105                 110                 115

gaa ggc aat caa gtt ctt gag gcc tgt gat ttt gaa caa acc ggt gaa    499
Glu Gly Asn Gln Val Leu Glu Ala Cys Asp Phe Glu Gln Thr Gly Glu
        120                 125                 130

tta gcc acg ttg atg gaa atg att ggc cgg gat cgt gct ttg gtt tca    547
Leu Ala Thr Leu Met Glu Met Ile Gly Arg Asp Arg Ala Leu Val Ser
    135                 140                 145

gat cgc tcg ggg gag gag cgc gtc gtc aag ctt gct ggt ccg ttg atg    595
Asp Arg Ser Gly Glu Glu Arg Val Val Lys Leu Ala Gly Pro Leu Met

150                 155                 160                 165

gat cgc acc gca aag ctg ccg cgc ccc ggt gac acc ctg ctt gtt gac      643
Asp Arg Thr Ala Lys Leu Pro Arg Pro Gly Asp Thr Leu Leu Val Asp
                170                 175                 180

cgc aaa gcg ggc tac gct ttt gag gcg att gcc aag acg gaa att tcg      691
Arg Lys Ala Gly Tyr Ala Phe Glu Ala Ile Ala Lys Thr Glu Ile Ser
            185                 190                 195

agg ctt gcg ctg gaa gag gcg cca gat gtg tct tat cag gat att ggt      739
Arg Leu Ala Leu Glu Glu Ala Pro Asp Val Ser Tyr Gln Asp Ile Gly
        200                 205                 210

ggc ttg gat gat cag att gaa ttg att caa gat gcc gtt gag ctg cca      787
Gly Leu Asp Asp Gln Ile Glu Leu Ile Gln Asp Ala Val Glu Leu Pro
    215                 220                 225

ttt ttg cac ccg gag atg tac cgc gcc tac aac ctg cat cca cca aag      835
Phe Leu His Pro Glu Met Tyr Arg Ala Tyr Asn Leu His Pro Pro Lys
230                 235                 240                 245

ggc gtg ctg ctg tac ggc cct ccc ggc tgt gga aag acg ctg att gct      883
Gly Val Leu Leu Tyr Gly Pro Pro Gly Cys Gly Lys Thr Leu Ile Ala
                250                 255                 260

aag gct gtg gct aat tct ttg gcc aac cgc atc ggt gag act ggc acc      931
Lys Ala Val Ala Asn Ser Leu Ala Asn Arg Ile Gly Glu Thr Gly Thr
            265                 270                 275

tcg tac ttc atc aac gtc aag ggg cca                                  958
Ser Tyr Phe Ile Asn Val Lys Gly Pro
        280                 285


<210> 78
<211> 286
<212> PRT
<213> Corynebacterium glutamicum

<400> 78
Met Ser Ser Pro Thr Asp Ser Ser Pro Ser Asn Ser Phe Ser Asp Phe
1               5                   10                  15

Asn Arg Glu Glu Gln Ser Arg Leu Ser Asp Glu Val Arg Gln Leu Lys
            20                  25                  30

Arg Thr Asn Ser Asp Leu Gly Ala Arg Asn Ala Lys Leu Ala Glu Met
        35                  40                  45

Leu Lys Ser Ser Arg Asp Lys Leu Ser Val Leu Phe Ser Gln Leu Glu
    50                  55                  60

Asp Met Ala Gln Pro Pro Ser Val Tyr Gly Thr Phe Leu Glu Thr Ala
65                  70                  75                  80

Lys Asp Gly Ser Asn Ala Glu Ile Phe Ala Gly Gly Arg Arg Met Arg
                85                  90                  95

Val Ala Val Ser Pro Met Leu Cys Ala Ala Asp Leu Met Pro Gly Val
            100                 105                 110

Gln Val Arg Leu Gly Glu Gly Asn Gln Val Leu Glu Ala Cys Asp Phe
        115                 120                 125

Glu Gln Thr Gly Glu Leu Ala Thr Leu Met Glu Met Ile Gly Arg Asp
    130                 135                 140

Arg Ala Leu Val Ser Asp Arg Ser Gly Glu Glu Arg Val Val Lys Leu
145                 150                 155                 160

Ala Gly Pro Leu Met Asp Arg Thr Ala Lys Leu Pro Arg Pro Gly Asp
                165                 170                 175

Thr Leu Leu Val Asp Arg Lys Ala Gly Tyr Ala Phe Glu Ala Ile Ala
            180                 185                 190

Lys Thr Glu Ile Ser Arg Leu Ala Leu Glu Glu Ala Pro Asp Val Ser
        195                 200                 205

Tyr Gln Asp Ile Gly Gly Leu Asp Asp Gln Ile Glu Leu Ile Gln Asp
    210                 215                 220

Ala Val Glu Leu Pro Phe Leu His Pro Glu Met Tyr Arg Ala Tyr Asn
225                 230                 235                 240

Leu His Pro Pro Lys Gly Val Leu Leu Tyr Gly Pro Pro Gly Cys Gly
                245                 250                 255

Lys Thr Leu Ile Ala Lys Ala Val Ala Asn Ser Leu Ala Asn Arg Ile
            260                 265                 270

Gly Glu Thr Gly Thr Ser Tyr Phe Ile Asn Val Lys Gly Pro
        275                 280                 285


<210> 79
<211> 735
<212> DNA
<213> Corynebacterium glutamicum

<220>
<221> CDS
<222> (101)..(712)
<223> RXA02317

<400> 79
gaattttccg gtgttaaaat gtgcggtgag cttggcgttg gcggaggacc agtgtgggga 60

gacgtcgaaa agcgaattca tggccccatc ttgccttaaa atg gcg cac atg cgc    115
                                            Met Ala His Met Arg
                                            1               5

tta ctg ctg acc tcc ttt ggc cat gat cat att cgg gat ttt gta cgc    163
Leu Leu Leu Thr Ser Phe Gly His Asp His Ile Arg Asp Phe Val Arg
                10                  15                  20

ggt acc gtg gcg tat atc cct gat gcg acc agg ctt ttt gct gat agt    211
Gly Thr Val Ala Tyr Ile Pro Asp Ala Thr Arg Leu Phe Ala Asp Ser
            25                  30                  35

ccc gag gct gct cct ttt atg gag acg gag cga aat atg ctg cgc gag    259
Pro Glu Ala Ala Pro Phe Met Glu Thr Glu Arg Asn Met Leu Arg Glu
        40                  45                  50

cac ggc ttg agc att cgt gag ctg ccg att tcc acg tcg act ccg gag    307
His Gly Leu Ser Ile Arg Glu Leu Pro Ile Ser Thr Ser Thr Pro Glu
    55                  60                  65

gaa gtg gat cgg gtg ctt ggt gag gtt gat ggg gtg tat gtg gcg ggc    355
Glu Val Asp Arg Val Leu Gly Glu Val Asp Gly Val Tyr Val Ala Gly
70                  75                  80                  85

ggt gag act ttt gat ctg atg tgg ctg ctg cgt tcc aca ggc aat gat    403
Gly Glu Thr Phe Asp Leu Met Trp Leu Leu Arg Ser Thr Gly Asn Asp
                90                  95                  100

gag gtg ttg att aag cat gtt cgc gct ggt cta ccg tat att gga acg    451
Glu Val Leu Ile Lys His Val Arg Ala Gly Leu Pro Tyr Ile Gly Thr
            105                 110                 115

agc gcc ggc gcg gta att gca ggt cct tcg att gaa ccg atc agc ttt    499
Ser Ala Gly Ala Val Ile Ala Gly Pro Ser Ile Glu Pro Ile Ser Phe
        120                 125                 130

ttg gat agc ccc gat gtc gcg ccg aat tta agc gac tat tca ggt cta    547
Leu Asp Ser Pro Asp Val Ala Pro Asn Leu Ser Asp Tyr Ser Gly Leu
    135                 140                 145

ggc ctg tgc gag cat gtc gtg gtg ccc cat gct ggt ggc acg atc ccg    595
Gly Leu Cys Glu His Val Val Val Pro His Ala Gly Gly Thr Ile Pro
150                 155                 160                 165

caa ttt ccc atc gat gtg ttt gcg gaa acc gtg cgc acc tac ggc gcc    643

Gln Phe Pro Ile Asp Val Phe Ala Glu Thr Val Arg Thr Tyr Gly Ala
                170                 175                 180

gaa ttc ccg ctg gtc ctg ctt aaa gat gga cag gca ctg ctt atc gac     691
Glu Phe Pro Leu Val Leu Leu Lys Asp Gly Gln Ala Leu Leu Ile Asp
        185                 190                 195

gac cac ggc gtc cac cta att taggatggtt ccccatgagc acc               735
Asp His Gly Val His Leu Ile
    200

<210> 80
<211> 204
<212> PRT
<213> Corynebacterium glutamicum

<400> 80
Met Ala His Met Arg Leu Leu Leu Thr Ser Phe Gly His Asp His Ile
  1               5                  10                  15

Arg Asp Phe Val Arg Gly Thr Val Ala Tyr Ile Pro Asp Ala Thr Arg
             20                  25                  30

Leu Phe Ala Asp Ser Pro Glu Ala Ala Pro Phe Met Glu Thr Glu Arg
         35                  40                  45

Asn Met Leu Arg Glu His Gly Leu Ser Ile Arg Glu Leu Pro Ile Ser
     50                  55                  60

Thr Ser Thr Pro Glu Glu Val Asp Arg Val Leu Gly Glu Val Asp Gly
 65                  70                  75                  80

Val Tyr Val Ala Gly Gly Glu Thr Phe Asp Leu Met Trp Leu Leu Arg
                 85                  90                  95

Ser Thr Gly Asn Asp Glu Val Leu Ile Lys His Val Arg Ala Gly Leu
            100                 105                 110

Pro Tyr Ile Gly Thr Ser Ala Gly Ala Val Ile Ala Gly Pro Ser Ile
        115                 120                 125

Glu Pro Ile Ser Phe Leu Asp Ser Pro Asp Val Ala Pro Asn Leu Ser
    130                 135                 140

Asp Tyr Ser Gly Leu Gly Leu Cys Glu His Val Val Val Pro His Ala
145                 150                 155                 160

Gly Gly Thr Ile Pro Gln Phe Pro Ile Asp Val Phe Ala Glu Thr Val
                165                 170                 175

Arg Thr Tyr Gly Ala Glu Phe Pro Leu Val Leu Leu Lys Asp Gly Gln
            180                 185                 190

Ala Leu Leu Ile Asp Asp His Gly Val His Leu Ile
        195                 200

<210> 81
<211> 774
<212> DNA
<213> Corynebacterium glutamicum

<220>
<221> CDS
<222> (101)..(751)
<223> RXA02644

<400> 81
cgtggggaag tggagcccac actgaaaagc tttgcgaggt caacatggcc accaggctag 60

tgaaaatgct gccggttggc gagctaagat gtgagatgct atg gca cat ctc acg     115
                                           Met Ala His Leu Thr
                                             1               5

caa tac caa ctc cct cag gcc ggt caa gtc ttt gag cat gaa cta gag     163
Gln Tyr Gln Leu Pro Gln Ala Gly Gln Val Phe Glu His Glu Leu Glu

10                  15                  20

atc aag cgc tcg cga ttt ctg acc tat atc acg cgt gtg caa gat cag     211
Ile Lys Arg Ser Arg Phe Leu Thr Tyr Ile Thr Arg Val Gln Asp Gln
                25                  30                  35

gag cag gct cgc gaa ttt att cac tcc atc aag gag ctg tat ccg gat     259
Glu Gln Ala Arg Glu Phe Ile His Ser Ile Lys Glu Leu Tyr Pro Asp
            40                  45                  50

gcg cgt cat cat tgc agt gcc ttt att ttc cat gtg gat ggc tca aat     307
Ala Arg His His Cys Ser Ala Phe Ile Phe His Val Asp Gly Ser Asn
        55                  60                  65

gat gtg gag cgt tcc tct gat gat ggc gaa cct tcc ggt acc gcg gga     355
Asp Val Glu Arg Ser Ser Asp Asp Gly Glu Pro Ser Gly Thr Ala Gly
 70                  75                  80                  85

aaa ccc atg cta gag gcg ttg cgt ggc tcg gga atg aaa gat att gcc     403
Lys Pro Met Leu Glu Ala Leu Arg Gly Ser Gly Met Lys Asp Ile Ala
                 90                  95                 100

gct gtg gtg gtg cgt tat ttc ggt ggc gta aaa ctg ggc act ggc gga     451
Ala Val Val Val Arg Tyr Phe Gly Gly Val Lys Leu Gly Thr Gly Gly
            105                 110                 115

tta gtt aat gcc tac acc aac gcg gtg acg gag ctt cta cct gag gtt     499
Leu Val Asn Ala Tyr Thr Asn Ala Val Thr Glu Leu Leu Pro Glu Val
        120                 125                 130

ttg caa gtc acc cgc tct gtt cgg gag att ttc aag att gac ctt ccg     547
Leu Gln Val Thr Arg Ser Val Arg Glu Ile Phe Lys Ile Asp Leu Pro
    135                 140                 145

cat tct gat gcg ggg cgc att gaa gcg aat ctg cgc ggc atg ggc atc     595
His Ser Asp Ala Gly Arg Ile Glu Ala Asn Leu Arg Gly Met Gly Ile
150                 155                 160                 165

atc att act gac act gaa tat ggt gca gaa gtc aca tac acc ttg gct     643
Ile Ile Thr Asp Thr Glu Tyr Gly Ala Glu Val Thr Tyr Thr Leu Ala
                170                 175                 180

tta ttg cct ggt gaa cag gct gcg gtg gaa tct caa ttg tca tcc atg     691
Leu Leu Pro Gly Glu Gln Ala Ala Val Glu Ser Gln Leu Ser Ser Met
            185                 190                 195

atg ggt gca gaa att gaa ttg aaa gaa tcc ggg cac atg tgg gtg gaa     739
Met Gly Ala Glu Ile Glu Leu Lys Glu Ser Gly His Met Trp Val Glu
        200                 205                 210

tcc ccg agt gac tagtgcggtg taagagcact aga                           774
Ser Pro Ser Asp
    215


<210> 82
<211> 217
<212> PRT
<213> Corynebacterium glutamicum

<400> 82
Met Ala His Leu Thr Gln Tyr Gln Leu Pro Gln Ala Gly Gln Val Phe
1               5                   10                  15

Glu His Glu Leu Glu Ile Lys Arg Ser Arg Phe Leu Thr Tyr Ile Thr
            20                  25                  30

Arg Val Gln Asp Gln Glu Gln Ala Arg Glu Phe Ile His Ser Ile Lys
        35                  40                  45

Glu Leu Tyr Pro Asp Ala Arg His His Cys Ser Ala Phe Ile Phe His
    50                  55                  60

Val Asp Gly Ser Asn Asp Val Glu Arg Ser Ser Asp Asp Gly Glu Pro
65                  70                  75                  80

Ser Gly Thr Ala Gly Lys Pro Met Leu Glu Ala Leu Arg Gly Ser Gly

85                  90                  95

Met Lys Asp Ile Ala Ala Val Val Val Arg Tyr Phe Gly Gly Val Lys
            100                 105                 110

Leu Gly Thr Gly Gly Leu Val Asn Ala Tyr Thr Asn Ala Val Thr Glu
        115                 120                 125

Leu Leu Pro Glu Val Leu Gln Val Thr Arg Ser Val Arg Glu Ile Phe
    130                 135                 140

Lys Ile Asp Leu Pro His Ser Asp Ala Gly Arg Ile Glu Ala Asn Leu
145                 150                 155                 160

Arg Gly Met Gly Ile Ile Ile Thr Asp Thr Glu Tyr Gly Ala Glu Val
                165                 170                 175

Thr Tyr Thr Leu Ala Leu Leu Pro Gly Glu Gln Ala Ala Val Glu Ser
            180                 185                 190

Gln Leu Ser Ser Met Met Gly Ala Glu Ile Glu Leu Lys Glu Ser Gly
        195                 200                 205

His Met Trp Val Glu Ser Pro Ser Asp
    210                 215


<210> 83
<211> 1411
<212> DNA
<213> Corynebacterium glutamicum

<220>
<221> CDS
<222> (101)..(1411)
<223> RXN02820

<400> 83
caacgaaaca aatgcaagcc cccaatcatg ggtttctacc aattaaattt tctttcagaa 60

aatatctccc cacataaaag ttccttgata ggctcgagag atg aaa gtg acc caa    115
                                           Met Lys Val Thr Gln
                                           1               5

agc aca ttc ctt aaa tcg gta gct gcg ttc act gtc gca gcc tta acc    163
Ser Thr Phe Leu Lys Ser Val Ala Ala Phe Thr Val Ala Ala Leu Thr
                10                  15                  20

ctg acc atc tct tcg tgt tcc agc ggt gaa gac acc tcc gca agc tcc    211
Leu Thr Ile Ser Ser Cys Ser Ser Gly Glu Asp Thr Ser Ala Ser Ser
            25                  30                  35

acg gat act gaa aac tcc tca acc caa gca gca gcg tct ccc cca ctt    259
Thr Asp Thr Glu Asn Ser Ser Thr Gln Ala Ala Ala Ser Pro Pro Leu
        40                  45                  50

gcg cct tgt gaa ctt ccc gcc gac gct tct gct gaa gag gaa gta gaa    307
Ala Pro Cys Glu Leu Pro Ala Asp Ala Ser Ala Glu Glu Glu Val Glu
    55                  60                  65

ggc act cac aca ggt gaa gat att tct gtt gcc ccg gaa atc ggt acc    355
Gly Thr His Thr Gly Glu Asp Ile Ser Val Ala Pro Glu Ile Gly Thr
70                  75                  80                  85

ggc tac cgc gag ggc atg acc cct gtt caa acc caa ggt tat gcg gtg    403
Gly Tyr Arg Glu Gly Met Thr Pro Val Gln Thr Gln Gly Tyr Ala Val
                90                  95                  100

gca act gca aac ccc atc gct tct gaa gca gcc tgc gcg gtg tta aga    451
Ala Thr Ala Asn Pro Ile Ala Ser Glu Ala Ala Cys Ala Val Leu Arg
            105                 110                 115

gaa ggc ggc act gca gct gat gct ctt gtc acc gcg cag ttt gtt ttg    499
Glu Gly Gly Thr Ala Ala Asp Ala Leu Val Thr Ala Gln Phe Val Leu
        120                 125                 130

gga ctg acg gaa ccg cag tcg tct ggc ctt ggt ggt ggc gga tac att    547

Gly Leu Thr Glu Pro Gln Ser Ser Gly Leu Gly Gly Gly Tyr Ile
    135                 140                 145

ctg tac tac gac gcc gaa gcc aat gcg gtg aca gcc att gat ggc cgt     595
Leu Tyr Tyr Asp Ala Glu Ala Asn Ala Val Thr Ala Ile Asp Gly Arg
150                 155                 160                 165

gaa aca gcg cca gtt gct gct gat gaa aac tat ctc att cat gtt tct     643
Glu Thr Ala Pro Val Ala Ala Asp Glu Asn Tyr Leu Ile His Val Ser
                170                 175                 180

gca gag gat caa acg gca cct gtt cct gat gcc cga cgt tcc ggc agg     691
Ala Glu Asp Gln Thr Ala Pro Val Pro Asp Ala Arg Arg Ser Gly Arg
            185                 190                 195

tca att ggt gtg cca gga atc gtg gca gcc ctt gga cag ctg cat gat     739
Ser Ile Gly Val Pro Gly Ile Val Ala Ala Leu Gly Gln Leu His Asp
        200                 205                 210

tca ttc gga aag acc tcc tgg cag gac gtg ctg aca act ccg cag cag     787
Ser Phe Gly Lys Thr Ser Trp Gln Asp Val Leu Thr Thr Pro Gln Gln
    215                 220                 225

ctc gca act gat ggt ttt tcc atc agc cct cgc atg tca gca tca att     835
Leu Ala Thr Asp Gly Phe Ser Ile Ser Pro Arg Met Ser Ala Ser Ile
230                 235                 240                 245

gct aac tcc gct gag gat ctc tcc cac gat ccg gaa gct gcc gca tat     883
Ala Asn Ser Ala Glu Asp Leu Ser His Asp Pro Glu Ala Ala Ala Tyr
                250                 255                 260

ttc ctt gat gaa aac ggt gat gcg aag gca ccc ggc aca ctt tta caa     931
Phe Leu Asp Glu Asn Gly Asp Ala Lys Ala Pro Gly Thr Leu Leu Gln
            265                 270                 275

aac cct gac tat gca gaa acg att cgt ctc atc tct gaa ggt ggc ccc     979
Asn Pro Asp Tyr Ala Glu Thr Ile Arg Leu Ile Ser Glu Gly Gly Pro
        280                 285                 290

gat gcg ttc tac acg ggt gag att gca gca gac atc gtg gaa cgc gcc     1027
Asp Ala Phe Tyr Thr Gly Glu Ile Ala Ala Asp Ile Val Glu Arg Ala
    295                 300                 305

acc cgt gag gtt gac ggt ttc aca cca tca ctg atg agc acg gca gat     1075
Thr Arg Glu Val Asp Gly Phe Thr Pro Ser Leu Met Ser Thr Ala Asp
310                 315                 320                 325

ttg gct gcc tac act ccg gaa act cgt gaa gct ttg tgt gct ccc tac     1123
Leu Ala Ala Tyr Thr Pro Glu Thr Arg Glu Ala Leu Cys Ala Pro Tyr
                330                 335                 340

cgc gac aag att gtt tgt ggc atg cca ccg tca tca tcg ggt ggc gtc     1171
Arg Asp Lys Ile Val Cys Gly Met Pro Pro Ser Ser Ser Gly Gly Val
            345                 350                 355

aca gtg atg gaa acc ctg ggt atc ttg aac aac ttt gat ctc gcc caa     1219
Thr Val Met Glu Thr Leu Gly Ile Leu Asn Asn Phe Asp Leu Ala Gln
        360                 365                 370

tac cca ccc act gag gtt ggt ttg gat ggc gga ttg cca aat gcg gaa     1267
Tyr Pro Pro Thr Glu Val Gly Leu Asp Gly Gly Leu Pro Asn Ala Glu
    375                 380                 385

gct gtt cac ctg att tca gag gct gag cgc ctg gct tat gct gat cgc     1315
Ala Val His Leu Ile Ser Glu Ala Glu Arg Leu Ala Tyr Ala Asp Arg
390                 395                 400                 405

gat gct tac atc ggt gat cct gct ttc gtg gaa gtt cca gca ggt ggt     1363
Asp Ala Tyr Ile Gly Asp Pro Ala Phe Val Glu Val Pro Ala Gly Gly
                410                 415                 420

gtc caa cag tgg atc aac cat gtc cac acg ggc gaa cac tcc aaa ctt     1411
Val Gln Gln Trp Ile Asn His Val His Thr Gly Glu His Ser Lys Leu
            425                 430                 435

<210> 84

<211> 437
<212> PRT
<213> Corynebacterium glutamicum

<400> 84
Met Lys Val Thr Gln Ser Thr Phe Leu Lys Ser Val Ala Ala Phe Thr
1               5                   10                  15

Val Ala Ala Leu Thr Leu Thr Ile Ser Ser Cys Ser Ser Gly Glu Asp
            20                  25                  30

Thr Ser Ala Ser Ser Thr Asp Thr Glu Asn Ser Ser Thr Gln Ala Ala
        35                  40                  45

Ala Ser Pro Pro Leu Ala Pro Cys Glu Leu Pro Ala Asp Ala Ser Ala
    50                  55                  60

Glu Glu Glu Val Glu Gly Thr His Thr Gly Glu Asp Ile Ser Val Ala
65                  70                  75                  80

Pro Glu Ile Gly Thr Gly Tyr Arg Glu Gly Met Thr Pro Val Gln Thr
                85                  90                  95

Gln Gly Tyr Ala Val Ala Thr Ala Asn Pro Ile Ala Ser Glu Ala Ala
            100                 105                 110

Cys Ala Val Leu Arg Glu Gly Gly Thr Ala Ala Asp Ala Leu Val Thr
        115                 120                 125

Ala Gln Phe Val Leu Gly Leu Thr Glu Pro Gln Ser Ser Gly Leu Gly
    130                 135                 140

Gly Gly Gly Tyr Ile Leu Tyr Tyr Asp Ala Glu Ala Asn Ala Val Thr
145                 150                 155                 160

Ala Ile Asp Gly Arg Glu Thr Ala Pro Val Ala Ala Asp Glu Asn Tyr
                165                 170                 175

Leu Ile His Val Ser Ala Glu Asp Gln Thr Ala Pro Val Pro Asp Ala
            180                 185                 190

Arg Arg Ser Gly Arg Ser Ile Gly Val Pro Gly Ile Val Ala Ala Leu
        195                 200                 205

Gly Gln Leu His Asp Ser Phe Gly Lys Thr Ser Trp Gln Asp Val Leu
    210                 215                 220

Thr Thr Pro Gln Gln Leu Ala Thr Asp Gly Phe Ser Ile Ser Pro Arg
225                 230                 235                 240

Met Ser Ala Ser Ile Ala Asn Ser Ala Glu Asp Leu Ser His Asp Pro
                245                 250                 255

Glu Ala Ala Ala Tyr Phe Leu Asp Glu Asn Gly Asp Ala Lys Ala Pro
            260                 265                 270

Gly Thr Leu Leu Gln Asn Pro Asp Tyr Ala Glu Thr Ile Arg Leu Ile
        275                 280                 285

Ser Glu Gly Gly Pro Asp Ala Phe Tyr Thr Gly Glu Ile Ala Ala Asp
    290                 295                 300

Ile Val Glu Arg Ala Thr Arg Glu Val Asp Gly Phe Thr Pro Ser Leu
305                 310                 315                 320

Met Ser Thr Ala Asp Leu Ala Ala Tyr Thr Pro Glu Thr Arg Glu Ala
                325                 330                 335

Leu Cys Ala Pro Tyr Arg Asp Lys Ile Val Cys Gly Met Pro Pro Ser
            340                 345                 350

Ser Ser Gly Gly Val Thr Val Met Glu Thr Leu Gly Ile Leu Asn Asn
        355                 360                 365

Phe Asp Leu Ala Gln Tyr Pro Pro Thr Glu Val Gly Leu Asp Gly Gly
    370                 375                 380

Leu Pro Asn Ala Glu Ala Val His Leu Ile Ser Glu Ala Glu Arg Leu
385                 390                 395                 400

Ala Tyr Ala Asp Arg Asp Ala Tyr Ile Gly Asp Pro Ala Phe Val Glu
                405                 410                 415

Val Pro Ala Gly Gly Val Gln Gln Trp Ile Asn His Val His Thr Gly
            420                 425                 430

Glu His Ser Lys Leu
        435


<210> 85
<211> 507
<212> DNA
<213> Corynebacterium glutamicum

<220>
<221> CDS
<222> (1)..(507)
<223> FRXA02820

<400> 85
gct aac tcc gct gag gat ctc tcc cac gat ccg gaa gct gcc gca tat      48
Ala Asn Ser Ala Glu Asp Leu Ser His Asp Pro Glu Ala Ala Ala Tyr
  1               5                  10                  15

ttc ctt gat gaa aac ggt gat gcg aag gca ccc ggc aca ctt tta caa      96
Phe Leu Asp Glu Asn Gly Asp Ala Lys Ala Pro Gly Thr Leu Leu Gln
             20                  25                  30

aac cct gac tat gca gaa acg att cgt ctc atc tct gaa ggt ggc ccc     144
Asn Pro Asp Tyr Ala Glu Thr Ile Arg Leu Ile Ser Glu Gly Gly Pro
         35                  40                  45

gat gcg ttc tac acg ggt gag att gca gca gac atc gtg gaa cgc gcc     192
Asp Ala Phe Tyr Thr Gly Glu Ile Ala Ala Asp Ile Val Glu Arg Ala
     50                  55                  60

acc cgt gag gtt gac ggt ttc aca cca tca ctg atg agc acg gca gat     240
Thr Arg Glu Val Asp Gly Phe Thr Pro Ser Leu Met Ser Thr Ala Asp
 65                  70                  75                  80

ttg gct gcc tac act ccg gaa act cgt gaa gct ttg tgt gct ccc tac     288
Leu Ala Ala Tyr Thr Pro Glu Thr Arg Glu Ala Leu Cys Ala Pro Tyr
                 85                  90                  95

cgc gac aag att gtt tgt ggc atg cca ccg tca tca tcg ggt ggc gtc     336
Arg Asp Lys Ile Val Cys Gly Met Pro Pro Ser Ser Ser Gly Gly Val
            100                 105                 110

aca gtg atg gaa acc ctg ggt atc ttg aac aac ttt gat ctc gcc caa     384
Thr Val Met Glu Thr Leu Gly Ile Leu Asn Asn Phe Asp Leu Ala Gln
        115                 120                 125

tac cca ccc act gag gtt ggt ttg gat ggc gga ttg cca aat gcg gaa     432
Tyr Pro Pro Thr Glu Val Gly Leu Asp Gly Gly Leu Pro Asn Ala Glu
    130                 135                 140

gct gtt cac ctg att tca gag gct gag cgc ctg gct tat gct gat cgc     480
Ala Val His Leu Ile Ser Glu Ala Glu Arg Leu Ala Tyr Ala Asp Arg
145                 150                 155                 160

gat gct tac atc ggt gat cct gct ttc                                 507
Asp Ala Tyr Ile Gly Asp Pro Ala Phe
                165


<210> 86
<211> 169
<212> PRT
<213> Corynebacterium glutamicum

<400> 86
Ala Asn Ser Ala Glu Asp Leu Ser His Asp Pro Glu Ala Ala Ala Tyr
  1               5                  10                  15

Phe Leu Asp Glu Asn Gly Asp Ala Lys Ala Pro Gly Thr Leu Leu Gln
            20                  25                  30

Asn Pro Asp Tyr Ala Glu Thr Ile Arg Leu Ile Ser Glu Gly Gly Pro
        35                  40                  45

Asp Ala Phe Tyr Thr Gly Glu Ile Ala Ala Asp Ile Val Glu Arg Ala
    50                  55                  60

Thr Arg Glu Val Asp Gly Phe Thr Pro Ser Leu Met Ser Thr Ala Asp
65                  70                  75                  80

Leu Ala Ala Tyr Thr Pro Glu Thr Arg Glu Ala Leu Cys Ala Pro Tyr
                85                  90                  95

Arg Asp Lys Ile Val Cys Gly Met Pro Pro Ser Ser Ser Gly Gly Val
            100                 105                 110

Thr Val Met Glu Thr Leu Gly Ile Leu Asn Asn Phe Asp Leu Ala Gln
        115                 120                 125

Tyr Pro Pro Thr Glu Val Gly Leu Asp Gly Gly Leu Pro Asn Ala Glu
    130                 135                 140

Ala Val His Leu Ile Ser Glu Ala Glu Arg Leu Ala Tyr Ala Asp Arg
145                 150                 155                 160

Asp Ala Tyr Ile Gly Asp Pro Ala Phe
                165


<210> 87
<211> 604
<212> DNA
<213> Corynebacterium glutamicum

<220>
<221> CDS
<222> (101)..(604)
<223> FRXA02000

<400> 87
caacgaaaca aatgcaagcc cccaatcatg ggtttctacc aattaaattt tctttcagaa 60

aatatctccc cacataaaag ttccttgata ggctcgagag atg aaa gtg acc caa    115
                                           Met Lys Val Thr Gln
                                           1               5

agc aca ttc ctt aaa tcg gta gct gcg ttc act gtc gca gcc tta acc    163
Ser Thr Phe Leu Lys Ser Val Ala Ala Phe Thr Val Ala Ala Leu Thr
                10                  15                  20

ctg acc atc tct tcg tgt tcc agc ggt gaa gac acc tcc gca agc tcc    211
Leu Thr Ile Ser Ser Cys Ser Ser Gly Glu Asp Thr Ser Ala Ser Ser
            25                  30                  35

acg gat act gaa aac tcc tca acc caa gca gca gcg tct ccc cca ctt    259
Thr Asp Thr Glu Asn Ser Ser Thr Gln Ala Ala Ala Ser Pro Pro Leu
        40                  45                  50

gcg cct tgt gaa ctt ccc gcc gac gct tct gct gaa gag gaa gta gaa    307
Ala Pro Cys Glu Leu Pro Ala Asp Ala Ser Ala Glu Glu Glu Val Glu
    55                  60                  65

ggc act cac aca ggt gaa gat att tct gtt gcc ccg gaa atc ggt acc    355
Gly Thr His Thr Gly Glu Asp Ile Ser Val Ala Pro Glu Ile Gly Thr
70                  75                  80                  85

ggc tac cgc gag ggc atg acc cct gtt caa acc caa ggt tat gcg gtg    403
Gly Tyr Arg Glu Gly Met Thr Pro Val Gln Thr Gln Gly Tyr Ala Val
                90                  95                  100

gca act gca aac ccc atc gct tct gaa gca gcc tgc gcg gtg tta aga    451
Ala Thr Ala Asn Pro Ile Ala Ser Glu Ala Ala Cys Ala Val Leu Arg
            105                 110                 115

gaa ggc ggc act gca gct gat gct ctt gtc acc gcg cag ttt gtt ttg     499
Glu Gly Gly Thr Ala Ala Asp Ala Leu Val Thr Ala Gln Phe Val Leu
        120                 125                 130

gga ctg acg gaa ccg cag tcg tct ggc ctt ggt ggt ggc gga tac att     547
Gly Leu Thr Glu Pro Gln Ser Ser Gly Leu Gly Gly Gly Gly Tyr Ile
    135                 140                 145

ctg tac tac gac gcc gaa gcc aat gcg gtg aca gcc att gat ggc cgt     595
Leu Tyr Tyr Asp Ala Glu Ala Asn Ala Val Thr Ala Ile Asp Gly Arg
150                 155                 160                 165

gaa aca gcg                                                         604
Glu Thr Ala

<210> 88
<211> 168
<212> PRT
<213> Corynebacterium glutamicum

<400> 88
Met Lys Val Thr Gln Ser Thr Phe Leu Lys Ser Val Ala Ala Phe Thr
  1               5                  10                  15

Val Ala Ala Leu Thr Leu Thr Ile Ser Ser Cys Ser Ser Gly Glu Asp
             20                  25                  30

Thr Ser Ala Ser Ser Thr Asp Thr Glu Asn Ser Ser Thr Gln Ala Ala
         35                  40                  45

Ala Ser Pro Pro Leu Ala Pro Cys Glu Leu Pro Ala Asp Ala Ser Ala
     50                  55                  60

Glu Glu Glu Val Glu Gly Thr His Thr Gly Glu Asp Ile Ser Val Ala
 65                  70                  75                  80

Pro Glu Ile Gly Thr Gly Tyr Arg Glu Gly Met Thr Pro Val Gln Thr
                 85                  90                  95

Gln Gly Tyr Ala Val Ala Thr Ala Asn Pro Ile Ala Ser Glu Ala Ala
            100                 105                 110

Cys Ala Val Leu Arg Glu Gly Gly Thr Ala Ala Asp Ala Leu Val Thr
        115                 120                 125

Ala Gln Phe Val Leu Gly Leu Thr Glu Pro Gln Ser Ser Gly Leu Gly
    130                 135                 140

Gly Gly Gly Tyr Ile Leu Tyr Tyr Asp Ala Glu Ala Asn Ala Val Thr
145                 150                 155                 160

Ala Ile Asp Gly Arg Glu Thr Ala
                165

<210> 89
<211> 824
<212> DNA
<213> Corynebacterium glutamicum

<220>
<221> CDS
<222> (1)..(801)
<223> RXN03178

<400> 89
ccc acc acc gtg gtc acg ggc acg atg gaa gcc gcc aac atc gag ggc      48
Pro Thr Thr Val Val Thr Gly Thr Met Glu Ala Ala Asn Ile Glu Gly
  1               5                  10                  15

tcc cgc gtg ggt gtc ggc gag gcc ggc cag tat acc gtt gat cag ctg      96
Ser Arg Val Gly Val Gly Glu Ala Gly Gln Tyr Thr Val Asp Gln Leu
             20                  25                  30

ctg cac ggt ctt ctt tta gcc agc ggt aac gat gcg gcg tat atg ttg     144

Leu His Gly Leu Leu Leu Ala Ser Gly Asn Asp Ala Ala Tyr Met Leu
        35                  40                  45

gct cag gaa ctt ggt ggg gat caa gca acc ctg gag aaa gta aac gcg 192
Ala Gln Glu Leu Gly Gly Asp Gln Ala Thr Leu Glu Lys Val Asn Ala
    50                  55                  60

ctg gcc aag gag ttg ggc act caa gac acc ttc gtt gcc act tat tcc 240
Leu Ala Lys Glu Leu Gly Thr Gln Asp Thr Phe Val Ala Thr Tyr Ser
65                  70                  75                  80

ggt ttg gat gcg ccg gga atg tcg acc tcc gca tac gac atg tca ttg 288
Gly Leu Asp Ala Pro Gly Met Ser Thr Ser Ala Tyr Asp Met Ser Leu
                85                  90                  95

att tat cag cat gcg tgg cag aac ccg gtt ttc gag tcg att atc tcc 336
Ile Tyr Gln His Ala Trp Gln Asn Pro Val Phe Glu Ser Ile Ile Ser
            100                 105                 110

acc gat cac att gat ttc cct ggt tgg ggc gac aat gag ggt ttc caa 384
Thr Asp His Ile Asp Phe Pro Gly Trp Gly Asp Asn Glu Gly Phe Gln
        115                 120                 125

gtc tgg aac gat aac gcc ttg ttc atg aac gat cct gat ggc atc ggc 432
Val Trp Asn Asp Asn Ala Leu Phe Met Asn Asp Pro Asp Gly Ile Gly
    130                 135                 140

ggc aag acc ggc tac acc gac gac gcg aac cac acc ttt gtc ggc ggt 480
Gly Lys Thr Gly Tyr Thr Asp Asp Ala Asn His Thr Phe Val Gly Gly
145                 150                 155                 160

ctc gat cgg ggt ggt cgc cgc ctc gcc gcc gta ctc ttg gat tcc acc 528
Leu Asp Arg Gly Gly Arg Arg Leu Ala Ala Val Leu Leu Asp Ser Thr
                165                 170                 175

gtc agc gac att cgt ccg tgg gaa caa gca cga ttg ctt atc gac gcc 576
Val Ser Asp Ile Arg Pro Trp Glu Gln Ala Arg Leu Leu Ile Asp Ala
            180                 185                 190

tcc ctc ccc atc acg ccg ggg tcc ggc gtg ggc cag ctg ggc tcc ggc 624
Ser Leu Pro Ile Thr Pro Gly Ser Gly Val Gly Gln Leu Gly Ser Gly
        195                 200                 205

agc gcg aac gat gtg gca ccg gcg acc cca gaa tta cca gaa ccc acc 672
Ser Ala Asn Asp Val Ala Pro Ala Thr Pro Glu Leu Pro Glu Pro Thr
    210                 215                 220

gac aac ctg act tca ggt gag ggt ggg tcg cag aac acg ctt ctt aag 720
Asp Asn Leu Thr Ser Gly Glu Gly Gly Ser Gln Asn Thr Leu Leu Lys
225                 230                 235                 240

ctc gtg gtg ccc atc gga atc atc gtg ctg ttg cta atc gcc gca cta 768
Leu Val Val Pro Ile Gly Ile Ile Val Leu Leu Leu Ile Ala Ala Leu
                245                 250                 255

gcg tgg aca ttc aga tct ccc aag aaa aag aac taggtgttct tcttcacgac 821
Ala Trp Thr Phe Arg Ser Pro Lys Lys Lys Asn
            260                 265

ctc 824

<210> 90
<211> 267
<212> PRT
<213> Corynebacterium glutamicum

<400> 90
Pro Thr Thr Val Val Thr Gly Thr Met Glu Ala Ala Asn Ile Glu Gly
1               5                   10                  15

Ser Arg Val Gly Val Gly Glu Ala Gly Gln Tyr Thr Val Asp Gln Leu
            20                  25                  30

Leu His Gly Leu Leu Leu Ala Ser Gly Asn Asp Ala Ala Tyr Met Leu
        35                  40                  45

Ala Gln Glu Leu Gly Gly Asp Gln Ala Thr Leu Glu Lys Val Asn Ala
    50                  55                  60

Leu Ala Lys Glu Leu Gly Thr Gln Asp Thr Phe Val Ala Thr Tyr Ser
65                  70                  75                  80

Gly Leu Asp Ala Pro Gly Met Ser Thr Ser Ala Tyr Asp Met Ser Leu
                85                  90                  95

Ile Tyr Gln His Ala Trp Gln Asn Pro Val Phe Glu Ser Ile Ile Ser
            100                 105                 110

Thr Asp His Ile Asp Phe Pro Gly Trp Gly Asp Asn Glu Gly Phe Gln
        115                 120                 125

Val Trp Asn Asp Asn Ala Leu Phe Met Asn Asp Pro Asp Gly Ile Gly
    130                 135                 140

Gly Lys Thr Gly Tyr Thr Asp Asp Ala Asn His Thr Phe Val Gly Gly
145                 150                 155                 160

Leu Asp Arg Gly Gly Arg Arg Leu Ala Ala Val Leu Leu Asp Ser Thr
                165                 170                 175

Val Ser Asp Ile Arg Pro Trp Glu Gln Ala Arg Leu Leu Ile Asp Ala
            180                 185                 190

Ser Leu Pro Ile Thr Pro Gly Ser Gly Val Gly Gln Leu Gly Ser Gly
        195                 200                 205

Ser Ala Asn Asp Val Ala Pro Ala Thr Pro Glu Leu Pro Glu Pro Thr
    210                 215                 220

Asp Asn Leu Thr Ser Gly Glu Gly Gly Ser Gln Asn Thr Leu Leu Lys
225                 230                 235                 240

Leu Val Val Pro Ile Gly Ile Ile Val Leu Leu Leu Ile Ala Ala Leu
                245                 250                 255

Ala Trp Thr Phe Arg Ser Pro Lys Lys Lys Asn
            260                 265


<210> 91
<211> 749
<212> DNA
<213> Corynebacterium glutamicum

<220>
<221> CDS
<222> (1)..(726)
<223> FRXA02859

<400> 91
cag tat acc gtt gat cag ctg ctg cac ggt ctt ctt tta gcc agc ggt      48
Gln Tyr Thr Val Asp Gln Leu Leu His Gly Leu Leu Leu Ala Ser Gly
1               5                   10                  15

aac gat gcg gcg tat ctg ttg gct cag gaa ctt ggt ggg gat caa gca      96
Asn Asp Ala Ala Tyr Leu Leu Ala Gln Glu Leu Gly Gly Asp Gln Ala
            20                  25                  30

acc ctg gag aaa gta aac gcg ctg gcc aag gag ttg ggc act caa gac     144
Thr Leu Glu Lys Val Asn Ala Leu Ala Lys Glu Leu Gly Thr Gln Asp
        35                  40                  45

acc ttc gtt gcc act tat tcc ggt ttg gat gcg ccg gga atg tcg acc     192
Thr Phe Val Ala Thr Tyr Ser Gly Leu Asp Ala Pro Gly Met Ser Thr
    50                  55                  60

tcc gca tac gac atg tca ttg att tat cag cat gcg tgg cag aac ccg     240
Ser Ala Tyr Asp Met Ser Leu Ile Tyr Gln His Ala Trp Gln Asn Pro
65                  70                  75                  80

gtt ttc gag tcg att atc tcc acc gat cac att gat ttc cct ggt tgg     288
Val Phe Glu Ser Ile Ile Ser Thr Asp His Ile Asp Phe Pro Gly Trp
                85                  90                  95

ggc gac aat gag ggt ttc caa gtc tgg aac gat aac gcc ttg ttc atg     336
Gly Asp Asn Glu Gly Phe Gln Val Trp Asn Asp Asn Ala Leu Phe Met
            100                 105                 110

aac gat cct gat ggc atc ggc ggc aag acc ggc tac acc gac gac gcg     384
Asn Asp Pro Asp Gly Ile Gly Gly Lys Thr Gly Tyr Thr Asp Asp Ala
        115                 120                 125

aac cac acc ttt gtc ggc ggt ctc gat cgg ggt ggt cgc cgc ctc gcc     432
Asn His Thr Phe Val Gly Gly Leu Asp Arg Gly Gly Arg Arg Leu Ala
    130                 135                 140

gcc gta ctc ttg gat tcc acc gtc agc gac att cgt ccg tgg gaa caa     480
Ala Val Leu Leu Asp Ser Thr Val Ser Asp Ile Arg Pro Trp Glu Gln
145                 150                 155                 160

gca cga ttg ctt atc gac gcc tcc ctc ccc atc acg ccg ggg tcc ggc     528
Ala Arg Leu Leu Ile Asp Ala Ser Leu Pro Ile Thr Pro Gly Ser Gly
                165                 170                 175

gtg ggc cag ctg ggc tcc ggc agc gcg aac gat gtg gca ccg gcg acc     576
Val Gly Gln Leu Gly Ser Gly Ser Ala Asn Asp Val Ala Pro Ala Thr
            180                 185                 190

cca gaa tta cca gaa ccc acc gac aac ctg act tca ggt gag ggt ggg     624
Pro Glu Leu Pro Glu Pro Thr Asp Asn Leu Thr Ser Gly Glu Gly Gly
        195                 200                 205

tcg cag aac acg ctg ctt aag ctc gtg gtg ccc atc gga atc atc gtg     672
Ser Gln Asn Thr Leu Leu Lys Leu Val Val Pro Ile Gly Ile Ile Val
    210                 215                 220

ctg ttg cta atc gcc gca cta gcg tgg aca ttc aga tct ccc aag aaa     720
Leu Leu Leu Ile Ala Ala Leu Ala Trp Thr Phe Arg Ser Pro Lys Lys
225                 230                 235                 240

aag aac taggtgttct tcttcacgac ctc                                   749
Lys Asn



<210> 92
<211> 242
<212> PRT
<213> Corynebacterium glutamicum

<400> 92
Gln Tyr Thr Val Asp Gln Leu Leu His Gly Leu Leu Leu Ala Ser Gly
  1               5                  10                  15

Asn Asp Ala Ala Tyr Leu Leu Ala Gln Glu Leu Gly Gly Asp Gln Ala
            20                  25                  30

Thr Leu Glu Lys Val Asn Ala Leu Ala Lys Glu Leu Gly Thr Gln Asp
        35                  40                  45

Thr Phe Val Ala Thr Tyr Ser Gly Leu Asp Ala Pro Gly Met Ser Thr
    50                  55                  60

Ser Ala Tyr Asp Met Ser Leu Ile Tyr Gln His Ala Trp Gln Asn Pro
 65                  70                  75                  80

Val Phe Glu Ser Ile Ile Ser Thr Asp His Ile Asp Phe Pro Gly Trp
                 85                  90                  95

Gly Asp Asn Glu Gly Phe Gln Val Trp Asn Asp Asn Ala Leu Phe Met
            100                 105                 110

Asn Asp Pro Asp Gly Ile Gly Gly Lys Thr Gly Tyr Thr Asp Asp Ala
        115                 120                 125

Asn His Thr Phe Val Gly Gly Leu Asp Arg Gly Gly Arg Arg Leu Ala
    130                 135                 140

Ala Val Leu Leu Asp Ser Thr Val Ser Asp Ile Arg Pro Trp Glu Gln
145                 150                 155                 160

Ala Arg Leu Leu Ile Asp Ala Ser Leu Pro Ile Thr Pro Gly Ser Gly
                165                 170                 175

Val Gly Gln Leu Gly Ser Gly Ser Ala Asn Asp Val Ala Pro Ala Thr
            180                 185                 190

Pro Glu Leu Pro Glu Pro Thr Asp Asn Leu Thr Ser Gly Glu Gly Gly
        195                 200                 205

Ser Gln Asn Thr Leu Leu Lys Leu Val Val Pro Ile Gly Ile Ile Val
    210                 215                 220

Leu Leu Leu Ile Ala Ala Leu Ala Trp Thr Phe Arg Ser Pro Lys Lys
225                 230                 235                 240

Lys Asn

<210> 93
<211> 1212
<212> DNA
<213> Corynebacterium glutamicum

<220>
<221> CDS
<222> (101)..(1189)
<223> RXA00137

<400> 93
tttacagagt gcttatgagg caatcagcca ctaagtgttg agtaatctac tagtttggac 60

tagaagttac ccactttcag tgaattttta aggagagaac atg gct ttg gct gat    115
                                            Met Ala Leu Ala Asp
                                            1               5

acc cga ttt gcc act cgt cgt cgc gca ctt gcc gca aaa ctg gca gct    163
Thr Arg Phe Ala Thr Arg Arg Arg Ala Leu Ala Ala Lys Leu Ala Ala
                10                  15                  20

caa cgg atc gac tca att ttg gtg aca agc ccg atc cat gtt cgc tat    211
Gln Arg Ile Asp Ser Ile Leu Val Thr Ser Pro Ile His Val Arg Tyr
            25                  30                  35

ctc agc gga ttc acc ggc tcc aac ggc gca ctg atc gtg aac aaa gat    259
Leu Ser Gly Phe Thr Gly Ser Asn Gly Ala Leu Ile Val Asn Lys Asp
        40                  45                  50

ctc tcc gcg cag atc tgc acc gac ggt cgc tac acc acc cag atc gca    307
Leu Ser Ala Gln Ile Cys Thr Asp Gly Arg Tyr Thr Thr Gln Ile Ala
    55                  60                  65

gaa gaa gtc ccg gac atc gag gcg ctg att gag cgt gcc tcg gca acg    355
Glu Glu Val Pro Asp Ile Glu Ala Leu Ile Glu Arg Ala Ser Ala Thr
70                  75                  80                  85

acg ctg cta gcg cag gtc gaa ggg ccg cgt cgt ata gca atc gaa gcc    403
Thr Leu Leu Ala Gln Val Glu Gly Pro Arg Arg Ile Ala Ile Glu Ala
                90                  95                  100

gca caa acc acc ctg gac cag cta gac agc ctg cgt gaa gca acc cag    451
Ala Gln Thr Thr Leu Asp Gln Leu Asp Ser Leu Arg Glu Ala Thr Gln
            105                 110                 115

gaa gac gtc gag ctg atc ccc gtg tca ggt gtt gtg gaa tcc att cgc    499
Glu Asp Val Glu Leu Ile Pro Val Ser Gly Val Val Glu Ser Ile Arg
        120                 125                 130

ctg acc aaa gac agc ttc gaa ctc gac cgc ctc cgc gat gtc gca gcg    547
Leu Thr Lys Asp Ser Phe Glu Leu Asp Arg Leu Arg Asp Val Ala Ala
    135                 140                 145

ctg gct tcc caa gca ttc gaa gat tta ctc gca gca gga gaa ctc gcc    595
Leu Ala Ser Gln Ala Phe Glu Asp Leu Leu Ala Ala Gly Glu Leu Ala
150                 155                 160                 165

gaa ggc cga tca gag cgc caa gtc gcc gcc gat ctg gaa tac cgc atg     643
Glu Gly Arg Ser Glu Arg Gln Val Ala Ala Asp Leu Glu Tyr Arg Met
                170                 175                 180

cgc ctg ttg gga gca gaa cgc ccc agc ttc gac acc atc gtg gcc tct     691
Arg Leu Leu Gly Ala Glu Arg Pro Ser Phe Asp Thr Ile Val Ala Ser
            185                 190                 195

gga cct aac tcc gcg aaa cca cac cac ggc gca ggc gac cgc atc ctc     739
Gly Pro Asn Ser Ala Lys Pro His His Gly Ala Gly Asp Arg Ile Leu
        200                 205                 210

cag cgc ggc gat cta gtc acc atc gat ttc ggc gca cac gca cgc gga     787
Gln Arg Gly Asp Leu Val Thr Ile Asp Phe Gly Ala His Ala Arg Gly
    215                 220                 225

ttc aac tcc gat atg acc cgc acc ctc gtt atg ggc gaa gca ggg gag     835
Phe Asn Ser Asp Met Thr Arg Thr Leu Val Met Gly Glu Ala Gly Glu
230                 235                 240                 245

ttc gaa gca gaa atc tac gac atc gtc ctg cgc tcc caa ctc gct ggt     883
Phe Glu Ala Glu Ile Tyr Asp Ile Val Leu Arg Ser Gln Leu Ala Gly
                250                 255                 260

gtt gaa gca gcc tac tca ggc gcc aac ctc ttc gac atc gac gca gca     931
Val Glu Ala Ala Tyr Ser Gly Ala Asn Leu Phe Asp Ile Asp Ala Ala
            265                 270                 275

tgc cgc aaa atc atc gaa gac gca ggc tac ggc gaa tac ttc gtg cac     979
Cys Arg Lys Ile Ile Glu Asp Ala Gly Tyr Gly Glu Tyr Phe Val His
        280                 285                 290

tcc acc ggc cac ggc atc gga ctt gaa gtc cac gaa gcc cca agc gca     1027
Ser Thr Gly His Gly Ile Gly Leu Glu Val His Glu Ala Pro Ser Ala
    295                 300                 305

tcc aaa acc tca caa gga gtc cta gaa acc ggc tcc aca ctg acc atc     1075
Ser Lys Thr Ser Gln Gly Val Leu Glu Thr Gly Ser Thr Leu Thr Ile
310                 315                 320                 325

gaa ccc gga att tac gtc ccc gga aag ggc ggc gta cgc atc gaa gac     1123
Glu Pro Gly Ile Tyr Val Pro Gly Lys Gly Gly Val Arg Ile Glu Asp
                330                 335                 340

acc ctg att att acc tca gga gca ccg gaa atc atc acc aag gtg agt     1171
Thr Leu Ile Ile Thr Ser Gly Ala Pro Glu Ile Ile Thr Lys Val Ser
            345                 350                 355

aag gac ctc atc gtg gtg taatctaggt gagctaatcg gtc                   1212
Lys Asp Leu Ile Val Val
        360


<210> 94
<211> 363
<212> PRT
<213> Corynebacterium glutamicum

<400> 94
Met Ala Leu Ala Asp Thr Arg Phe Ala Thr Arg Arg Arg Ala Leu Ala
1               5                   10                  15

Ala Lys Leu Ala Ala Gln Arg Ile Asp Ser Ile Leu Val Thr Ser Pro
            20                  25                  30

Ile His Val Arg Tyr Leu Ser Gly Phe Thr Gly Ser Asn Gly Ala Leu
        35                  40                  45

Ile Val Asn Lys Asp Leu Ser Ala Gln Ile Cys Thr Asp Gly Arg Tyr
    50                  55                  60

Thr Thr Gln Ile Ala Glu Glu Val Pro Asp Ile Glu Ala Leu Ile Glu
65                  70                  75                  80

Arg Ala Ser Ala Thr Thr Leu Leu Ala Gln Val Glu Gly Pro Arg Arg
                85                  90                  95

Ile Ala Ile Glu Ala Ala Gln Thr Thr Leu Asp Gln Leu Asp Ser Leu
            100                 105                 110

Arg Glu Ala Thr Gln Glu Asp Val Glu Leu Ile Pro Val Ser Gly Val
        115                 120                 125

Val Glu Ser Ile Arg Leu Thr Lys Asp Ser Phe Glu Leu Asp Arg Leu
    130                 135                 140

Arg Asp Val Ala Ala Leu Ala Ser Gln Ala Phe Glu Asp Leu Leu Ala
145                 150                 155                 160

Ala Gly Glu Leu Ala Glu Gly Arg Ser Glu Arg Gln Val Ala Ala Asp
                165                 170                 175

Leu Glu Tyr Arg Met Arg Leu Leu Gly Ala Glu Arg Pro Ser Phe Asp
            180                 185                 190

Thr Ile Val Ala Ser Gly Pro Asn Ser Ala Lys Pro His His Gly Ala
        195                 200                 205

Gly Asp Arg Ile Leu Gln Arg Gly Asp Leu Val Thr Ile Asp Phe Gly
    210                 215                 220

Ala His Ala Arg Gly Phe Asn Ser Asp Met Thr Arg Thr Leu Val Met
225                 230                 235                 240

Gly Glu Ala Gly Glu Phe Glu Ala Glu Ile Tyr Asp Ile Val Leu Arg
                245                 250                 255

Ser Gln Leu Ala Gly Val Glu Ala Ala Tyr Ser Gly Ala Asn Leu Phe
            260                 265                 270

Asp Ile Asp Ala Ala Cys Arg Lys Ile Ile Glu Asp Ala Gly Tyr Gly
        275                 280                 285

Glu Tyr Phe Val His Ser Thr Gly His Gly Ile Gly Leu Glu Val His
    290                 295                 300

Glu Ala Pro Ser Ala Ser Lys Thr Ser Gln Gly Val Leu Glu Thr Gly
305                 310                 315                 320

Ser Thr Leu Thr Ile Glu Pro Gly Ile Tyr Val Pro Gly Lys Gly Gly
                325                 330                 335

Val Arg Ile Glu Asp Thr Leu Ile Ile Thr Ser Gly Ala Pro Glu Ile
            340                 345                 350

Ile Thr Lys Val Ser Lys Asp Leu Ile Val Val
        355                 360


<210> 95
<211> 1404
<212> DNA
<213> Corynebacterium glutamicum

<220>
<221> CDS
<222> (101)..(1381)
<223> RXN00499

<400> 95
tgccaacagg ggatatgcca ctgtgtaccc cacggcgatg atcggtaaaa tcctcggcgc 60

gcagatattg ttcttgctgc tctaaggtga tttttgggca gtg gtg ggg gtg gtg   115
                                          Val Val Gly Val Val
                                          1               5

tcc acc cct gcg cgt aac ctg gga agc atg act aaa aca ctt ggt tcc   163
Ser Thr Pro Ala Arg Asn Leu Gly Ser Met Thr Lys Thr Leu Gly Ser
                10                  15                  20

ctt cag ctg gaa gaa atc acg ctg acc ctc cct ctg act gaa gat gtg   211
Leu Gln Leu Glu Glu Ile Thr Leu Thr Leu Pro Leu Thr Glu Asp Val
            25                  30                  35

gcc gat gaa cgc acc att gat gtg ttc gca cgc att gcc aca cgc gtc     259
Ala Asp Glu Arg Thr Ile Asp Val Phe Ala Arg Ile Ala Thr Arg Val
        40                  45                  50

ggt ggg gaa gac ctt cca tat tta gta ttc ctg cag ggt ggg cct ggc     307
Gly Gly Glu Asp Leu Pro Tyr Leu Val Phe Leu Gln Gly Gly Pro Gly
    55                  60                  65

aat gaa gct cca cgt cca agc ctt aat ccc ctc aac ccc aat tgg ttg     355
Asn Glu Ala Pro Arg Pro Ser Leu Asn Pro Leu Asn Pro Asn Trp Leu
70                  75                  80                  85

ggc gtg gcc ttg gag gaa tac cgc gtg gtc atg ttg gat caa cgt ggc     403
Gly Val Ala Leu Glu Glu Tyr Arg Val Val Met Leu Asp Gln Arg Gly
                90                  95                  100

acc ggc cgt tcc acc cca gtg ggt aat gat att ttg gaa aaa ccc aca     451
Thr Gly Arg Ser Thr Pro Val Gly Asn Asp Ile Leu Glu Lys Pro Thr
            105                 110                 115

gca gaa gta gtg gag tac tta tcc cac ctg cgc gca gat ggc att gtg     499
Ala Glu Val Val Glu Tyr Leu Ser His Leu Arg Ala Asp Gly Ile Val
        120                 125                 130

cga gat gct gaa gcc ctg cgt aag cat ttg ggt gtg aat cag tgg aac     547
Arg Asp Ala Glu Ala Leu Arg Lys His Leu Gly Val Asn Gln Trp Asn
    135                 140                 145

ctt tta ggc cag tcc ttc gga ggt ttc acc acc ctg cat tac ttg tcc     595
Leu Leu Gly Gln Ser Phe Gly Gly Phe Thr Thr Leu His Tyr Leu Ser
150                 155                 160                 165

cgg cac gcc gat tcc ttg gac aac gtg ttt att acc ggc ggt ctc agc     643
Arg His Ala Asp Ser Leu Asp Asn Val Phe Ile Thr Gly Gly Leu Ser
                170                 175                 180

gct att gat cgc cca gca gaa gac gtg tat gcc aac tgt tac aac cgc     691
Ala Ile Asp Arg Pro Ala Glu Asp Val Tyr Ala Asn Cys Tyr Asn Arg
            185                 190                 195

atg cgc cga aac tct gag gaa ttc tac cgt cgc ttc ccg caa tta cgg     739
Met Arg Arg Asn Ser Glu Glu Phe Tyr Arg Arg Phe Pro Gln Leu Arg
        200                 205                 210

gaa act ttc cga ggg ttg gtt aat cgt gct cgc gcc ggg gag att gtg     787
Glu Thr Phe Arg Gly Leu Val Asn Arg Ala Arg Ala Gly Glu Ile Val
    215                 220                 225

ctt ccc acc ggc gaa gtt gtg tca gaa acc agg ctg cga tcc ctt ggt     835
Leu Pro Thr Gly Glu Val Val Ser Glu Thr Arg Leu Arg Ser Leu Gly
230                 235                 240                 245

cac ttg ttg ggt agc aat gac ggc tgg ttt gat ctg tac aac ctg ctg     883
His Leu Leu Gly Ser Asn Asp Gly Trp Phe Asp Leu Tyr Asn Leu Leu
                250                 255                 260

gaa tta gat ccc acc tcc aac gct ttt gtc cat gac ctg gca gga ctt     931
Glu Leu Asp Pro Thr Ser Asn Ala Phe Val His Asp Leu Ala Gly Leu
            265                 270                 275

ttg cct ttc ggc aac cgc aac cca att tat tac gtg ctc cat gag tcc     979
Leu Pro Phe Gly Asn Arg Asn Pro Ile Tyr Tyr Val Leu His Glu Ser
        280                 285                 290

tct tac gcc gac ggt gtg gtg aca aat tgg gca gca gag cgt gtg ctt     1027
Ser Tyr Ala Asp Gly Val Val Thr Asn Trp Ala Ala Glu Arg Val Leu
    295                 300                 305

cca gag gat ttc cgc gag gat cca aca ctg ctc acc ggt gag cac gtg     1075
Pro Glu Asp Phe Arg Glu Asp Pro Thr Leu Leu Thr Gly Glu His Val
310                 315                 320                 325

ttc cag gag tgg aca gac acc gtg ccg tcg ctc aag ccg tgg aag gac     1123
Phe Gln Glu Trp Thr Asp Thr Val Pro Ser Leu Lys Pro Trp Lys Asp
                330                 335                 340

gtt gcc ctg gca ttg gct cag cag gaa tgg ccc aag ctt tat gat gcg     1171

Val Ala Leu Ala Leu Ala Gln Gln Glu Trp Pro Lys Leu Tyr Asp Ala
            345                 350                 355

aag gca ttg gaa aac tca cag gcc aag ggc gct gca gca gtg tat gcc      1219
Lys Ala Leu Glu Asn Ser Gln Ala Lys Gly Ala Ala Ala Val Tyr Ala
        360                 365                 370

aat gac gtt ttc gtc cca gtg gat tac tct ctg gaa acc gca caa cac      1267
Asn Asp Val Phe Val Pro Val Asp Tyr Ser Leu Glu Thr Ala Gln His
    375                 380                 385

ctg ccc ggt gtg cag ctg ttt atc acc agc cag cat gaa cac aat gga      1315
Leu Pro Gly Val Gln Leu Phe Ile Thr Ser Gln His Glu His Asn Gly
390                 395                 400                 405

ctt cgt gcc agc tca ggc gca gta ctg aag cac ctt ttc gat ctg gcc      1363
Leu Arg Ala Ser Ser Gly Ala Val Leu Lys His Leu Phe Asp Leu Ala
                410                 415                 420

cac ggc cga gag gta cgc tgattcctcg tgttagtact agc                    1404
His Gly Arg Glu Val Arg
            425


<210> 96
<211> 427
<212> PRT
<213> Corynebacterium glutamicum

<400> 96
Val Val Gly Val Val Ser Thr Pro Ala Arg Asn Leu Gly Ser Met Thr
1               5                   10                  15

Lys Thr Leu Gly Ser Leu Gln Leu Glu Glu Ile Thr Leu Thr Leu Pro
            20                  25                  30

Leu Thr Glu Asp Val Ala Asp Glu Arg Thr Ile Asp Val Phe Ala Arg
        35                  40                  45

Ile Ala Thr Arg Val Gly Gly Glu Asp Leu Pro Tyr Leu Val Phe Leu
    50                  55                  60

Gln Gly Gly Pro Gly Asn Glu Ala Pro Arg Pro Ser Leu Asn Pro Leu
65                  70                  75                  80

Asn Pro Asn Trp Leu Gly Val Ala Leu Glu Glu Tyr Arg Val Val Met
                85                  90                  95

Leu Asp Gln Arg Gly Thr Gly Arg Ser Thr Pro Val Gly Asn Asp Ile
            100                 105                 110

Leu Glu Lys Pro Thr Ala Glu Val Val Glu Tyr Leu Ser His Leu Arg
        115                 120                 125

Ala Asp Gly Ile Val Arg Asp Ala Glu Ala Leu Arg Lys His Leu Gly
    130                 135                 140

Val Asn Gln Trp Asn Leu Leu Gly Gln Ser Phe Gly Gly Phe Thr Thr
145                 150                 155                 160

Leu His Tyr Leu Ser Arg His Ala Asp Ser Leu Asp Asn Val Phe Ile
                165                 170                 175

Thr Gly Gly Leu Ser Ala Ile Asp Arg Pro Ala Glu Asp Val Tyr Ala
            180                 185                 190

Asn Cys Tyr Asn Arg Met Arg Arg Asn Ser Glu Glu Phe Tyr Arg Arg
        195                 200                 205

Phe Pro Gln Leu Arg Glu Thr Phe Arg Gly Leu Val Asn Arg Ala Arg
    210                 215                 220

Ala Gly Glu Ile Val Leu Pro Thr Gly Glu Val Val Ser Glu Thr Arg
225                 230                 235                 240

Leu Arg Ser Leu Gly His Leu Leu Gly Ser Asn Asp Gly Trp Phe Asp
                245                 250                 255

Leu Tyr Asn Leu Leu Glu Leu Asp Pro Thr Ser Asn Ala Phe Val His
            260                 265                 270

Asp Leu Ala Gly Leu Leu Pro Phe Gly Asn Arg Asn Pro Ile Tyr Tyr
        275                 280                 285

Val Leu His Glu Ser Ser Tyr Ala Asp Gly Val Val Thr Asn Trp Ala
    290                 295                 300

Ala Glu Arg Val Leu Pro Glu Asp Phe Arg Glu Asp Pro Thr Leu Leu
305                 310                 315                 320

Thr Gly Glu His Val Phe Gln Glu Trp Thr Asp Thr Val Pro Ser Leu
                325                 330                 335

Lys Pro Trp Lys Asp Val Ala Leu Ala Leu Ala Gln Gln Glu Trp Pro
            340                 345                 350

Lys Leu Tyr Asp Ala Lys Ala Leu Glu Asn Ser Gln Ala Lys Gly Ala
        355                 360                 365

Ala Ala Val Tyr Ala Asn Asp Val Phe Val Pro Val Asp Tyr Ser Leu
    370                 375                 380

Glu Thr Ala Gln His Leu Pro Gly Val Gln Leu Phe Ile Thr Ser Gln
385                 390                 395                 400

His Glu His Asn Gly Leu Arg Ala Ser Ser Gly Ala Val Leu Lys His
                405                 410                 415

Leu Phe Asp Leu Ala His Gly Arg Glu Val Arg
            420                 425


<210> 97
<211> 980
<212> DNA
<213> Corynebacterium glutamicum

<220>
<221> CDS
<222> (1)..(957)
<223> FRXA00499

<400> 97
ggt aat gat att ttg gaa aaa ccc aca gca gaa gta gtg gag tac tta      48
Gly Asn Asp Ile Leu Glu Lys Pro Thr Ala Glu Val Val Glu Tyr Leu
  1               5                  10                  15

tcc cac ctg cgc gca gat ggc att gtg cga gat gct gaa gcc ctg cgt      96
Ser His Leu Arg Ala Asp Gly Ile Val Arg Asp Ala Glu Ala Leu Arg
             20                  25                  30

aag cat ttg ggt gtg aat cag tgg aac ctt tta ggc cag tcc ttc gga     144
Lys His Leu Gly Val Asn Gln Trp Asn Leu Leu Gly Gln Ser Phe Gly
         35                  40                  45

ggt ttc acc acc ctg cat tac ttg tcc cgg cac gcc gat tcc ttg gac     192
Gly Phe Thr Thr Leu His Tyr Leu Ser Arg His Ala Asp Ser Leu Asp
     50                  55                  60

aac gtg ttt att acc ggc ggt ctc agc gct att gat cgc cca gca gaa     240
Asn Val Phe Ile Thr Gly Gly Leu Ser Ala Ile Asp Arg Pro Ala Glu
 65                  70                  75                  80

gac gtg tat gcc aac tgt tac aac cgc atg cgc cga aac tct gag gaa     288
Asp Val Tyr Ala Asn Cys Tyr Asn Arg Met Arg Arg Asn Ser Glu Glu
                 85                  90                  95

ttc tac cgt cgc ttc ccg caa tta cgg gaa act ttc cga ggg ttg gtt     336
Phe Tyr Arg Arg Phe Pro Gln Leu Arg Glu Thr Phe Arg Gly Leu Val
            100                 105                 110

aat cgt gct cgc gcc ggg gag att gtg ctt ccc acc ggc gaa gtt gtg     384
Asn Arg Ala Arg Ala Gly Glu Ile Val Leu Pro Thr Gly Glu Val Val
        115                 120                 125

tca gaa acc agg ctg cga tcc ctt ggt cac ttg ttg ggt agc aat gac     432
Ser Glu Thr Arg Leu Arg Ser Leu Gly His Leu Leu Gly Ser Asn Asp
    130                 135                 140

ggc tgg ttt gat ctg tac aac ctg ctg gaa tta gat ccc acc tcc aac     480
Gly Trp Phe Asp Leu Tyr Asn Leu Leu Glu Leu Asp Pro Thr Ser Asn
145                 150                 155                 160

gct ttt gtc cat gac ctg gca gga ctt ttg cct ttc ggc aac cgc aac     528
Ala Phe Val His Asp Leu Ala Gly Leu Leu Pro Phe Gly Asn Arg Asn
                165                 170                 175

cca att tat tac gtg ctc cat gag tcc tct tac gcc gac ggt gtg gtg     576
Pro Ile Tyr Tyr Val Leu His Glu Ser Ser Tyr Ala Asp Gly Val Val
            180                 185                 190

aca aat tgg gca gca gag cgt gtg ctt cca gag gat ttc cgc gag gat     624
Thr Asn Trp Ala Ala Glu Arg Val Leu Pro Glu Asp Phe Arg Glu Asp
        195                 200                 205

cca aca ctg ctc acc ggt gag cac gtg ttc cag gag tgg aca gac acc     672
Pro Thr Leu Leu Thr Gly Glu His Val Phe Gln Glu Trp Thr Asp Thr
    210                 215                 220

gtg ccg tcg ctc aag ccg tgg aag gac gtt gcc ctg gca ttg gct cag     720
Val Pro Ser Leu Lys Pro Trp Lys Asp Val Ala Leu Ala Leu Ala Gln
225                 230                 235                 240

cag gaa tgg ccc aag ctt tat gat gcg aag gca ttg gaa aac tca cag     768
Gln Glu Trp Pro Lys Leu Tyr Asp Ala Lys Ala Leu Glu Asn Ser Gln
                245                 250                 255

gcc aag ggc gct gca gca gtg tat gcc aat gac gtt ttc gtc cca gtg     816
Ala Lys Gly Ala Ala Ala Val Tyr Ala Asn Asp Val Phe Val Pro Val
            260                 265                 270

gat tac tct ctg gaa acc gca caa cac ctg ccc ggt gtg cag ctg ttt     864
Asp Tyr Ser Leu Glu Thr Ala Gln His Leu Pro Gly Val Gln Leu Phe
        275                 280                 285

atc acc agc cag cat gaa cac aat gga ctt cgt gcc agc tca ggc gca     912
Ile Thr Ser Gln His Glu His Asn Gly Leu Arg Ala Ser Ser Gly Ala
    290                 295                 300

gta ctg aag cac ctt ttc gat ctg gcc cac ggc cga gag gta cgc         957
Val Leu Lys His Leu Phe Asp Leu Ala His Gly Arg Glu Val Arg
305                 310                 315

tgattcctcg tgttagtact agc                                           980


<210> 98
<211> 319
<212> PRT
<213> Corynebacterium glutamicum

<400> 98
Gly Asn Asp Ile Leu Glu Lys Pro Thr Ala Glu Val Val Glu Tyr Leu
1               5                   10                  15

Ser His Leu Arg Ala Asp Gly Ile Val Arg Asp Ala Glu Ala Leu Arg
            20                  25                  30

Lys His Leu Gly Val Asn Gln Trp Asn Leu Leu Gly Gln Ser Phe Gly
        35                  40                  45

Gly Phe Thr Thr Leu His Tyr Leu Ser Arg His Ala Asp Ser Leu Asp
    50                  55                  60

Asn Val Phe Ile Thr Gly Gly Leu Ser Ala Ile Asp Arg Pro Ala Glu
65                  70                  75                  80

Asp Val Tyr Ala Asn Cys Tyr Asn Arg Met Arg Arg Asn Ser Glu Glu
                85                  90                  95

Phe Tyr Arg Arg Phe Pro Gln Leu Arg Glu Thr Phe Arg Gly Leu Val

100                 105                 110

Asn Arg Ala Arg Ala Gly Glu Ile Val Leu Pro Thr Gly Glu Val Val
        115                 120                 125

Ser Glu Thr Arg Leu Arg Ser Leu Gly His Leu Leu Gly Ser Asn Asp
    130                 135                 140

Gly Trp Phe Asp Leu Tyr Asn Leu Leu Glu Leu Asp Pro Thr Ser Asn
145                 150                 155                 160

Ala Phe Val His Asp Leu Ala Gly Leu Leu Pro Phe Gly Asn Arg Asn
                165                 170                 175

Pro Ile Tyr Tyr Val Leu His Glu Ser Ser Tyr Ala Asp Gly Val Val
            180                 185                 190

Thr Asn Trp Ala Ala Glu Arg Val Leu Pro Glu Asp Phe Arg Glu Asp
        195                 200                 205

Pro Thr Leu Leu Thr Gly Glu His Val Phe Gln Glu Trp Thr Asp Thr
    210                 215                 220

Val Pro Ser Leu Lys Pro Trp Lys Asp Val Ala Leu Ala Leu Ala Gln
225                 230                 235                 240

Gln Glu Trp Pro Lys Leu Tyr Asp Ala Lys Ala Leu Glu Asn Ser Gln
                245                 250                 255

Ala Lys Gly Ala Ala Ala Val Tyr Ala Asn Asp Val Phe Val Pro Val
            260                 265                 270

Asp Tyr Ser Leu Glu Thr Ala Gln His Leu Pro Gly Val Gln Leu Phe
        275                 280                 285

Ile Thr Ser Gln His Glu His Asn Gly Leu Arg Ala Ser Ser Gly Ala
    290                 295                 300

Val Leu Lys His Leu Phe Asp Leu Ala His Gly Arg Glu Val Arg
305                 310                 315


<210> 99
<211> 1788
<212> DNA
<213> Corynebacterium glutamicum

<220>
<221> CDS
<222> (101)..(1765)
<223> RXN00877

<400> 99
aaataatggg gctcgccggt gatggttccc gccgggcatt caacggtgac ggaagaggtg 60

gcagacatga tgaaaactct agcaactagt atcggtcact atg act gtt gaa cac    115
                                           Met Thr Val Glu His
                                           1               5

ctg ctc aag ccc agc acc ttg ccc tac cag ctg ccc gat ttc gca gcg    163
Leu Leu Lys Pro Ser Thr Leu Pro Tyr Gln Leu Pro Asp Phe Ala Ala
                10                  15                  20

atc aag gtg gct gat ttc ccg ccc gcc ttc gaa ctc gca tta gct gaa    211
Ile Lys Val Ala Asp Phe Pro Pro Ala Phe Glu Leu Ala Leu Ala Glu
            25                  30                  35

cac gat gct gaa att aca gcg atc gct acc aat gag gac gct cct acc    259
His Asp Ala Glu Ile Thr Ala Ile Ala Thr Asn Glu Asp Ala Pro Thr
        40                  45                  50

tgg gag aac acc att gag gcc ctg gaa cgc gca ggc ctg tcc ctc aac    307
Trp Glu Asn Thr Ile Glu Ala Leu Glu Arg Ala Gly Leu Ser Leu Asn
    55                  60                  65

cgc gtc gcc gcc gta ttc ttc aac ttg cag ggc acc gat tcc tcc cct    355
Arg Val Ala Ala Val Phe Phe Asn Leu Gln Gly Thr Asp Ser Ser Pro

70                    75                    80                    85

gaa atg gat gaa atc gca gcc act atc gcg ccg aaa ctc tcc gcg cat     403
Glu Met Asp Glu Ile Ala Ala Thr Ile Ala Pro Lys Leu Ser Ala His
                90                    95                    100

tcg gat gcg att ttc cac aat gct gcg ctt ttc gcg cgc att gag gcc     451
Ser Asp Ala Ile Phe His Asn Ala Ala Leu Phe Ala Arg Ile Glu Ala
            105                   110                   115

gta gaa gca ccg gcc gac gag gaa tcg caa cgc ctg ttg tcc cac acc     499
Val Glu Ala Pro Ala Asp Glu Glu Ser Gln Arg Leu Leu Ser His Thr
        120                   125                   130

aag cgc gct ttt cga cgt cgc ggt gca gca ctc aac gcc gac ggc aag     547
Lys Arg Ala Phe Arg Arg Arg Gly Ala Ala Leu Asn Ala Asp Gly Lys
    135                   140                   145

gcc cga ctg agc acc atc aac cag cgc cta tcg gca ctg tcc gaa cag     595
Ala Arg Leu Ser Thr Ile Asn Gln Arg Leu Ser Ala Leu Ser Glu Gln
150                   155                   160                   165

ttc ggc cgc aac ctg ctt cag gac acc cgc gat ctg gcg gtc aac ttt     643
Phe Gly Arg Asn Leu Leu Gln Asp Thr Arg Asp Leu Ala Val Asn Phe
                170                   175                   180

gaa gaa tct gaa ctt gcc ggt ttt agc gaa gcc cgc ata tcc gcc gcc     691
Glu Glu Ser Glu Leu Ala Gly Phe Ser Glu Ala Arg Ile Ser Ala Ala
            185                   190                   195

gct gac tac gca gca gca gtt ggc acc gaa ggc tac gtg gtt cca ctg     739
Ala Asp Tyr Ala Ala Ala Val Gly Thr Glu Gly Tyr Val Val Pro Leu
        200                   205                   210

gaa ctg ccc acc gtg cag tca gag cag gca gta tta acc gaa tcc gcc     787
Glu Leu Pro Thr Val Gln Ser Glu Gln Ala Val Leu Thr Glu Ser Ala
    215                   220                   225

tcg cgt gca aag ctt tat gaa gcc tcc cag aag cgt ggc gcc agc ctg     835
Ser Arg Ala Lys Leu Tyr Glu Ala Ser Gln Lys Arg Gly Ala Ser Leu
230                   235                   240                   245

aac aag gac gtg ctg ctc gaa acc gtg cgt ctg cgt gct gaa cgc gcc     883
Asn Lys Asp Val Leu Leu Glu Thr Val Arg Leu Arg Ala Glu Arg Ala
                250                   255                   260

aca ctt tta ggc tac gac acc cac gcc gat tac gtc atc gaa gaa gaa     931
Thr Leu Leu Gly Tyr Asp Thr His Ala Asp Tyr Val Ile Glu Glu Glu
            265                   270                   275

acc gcc gat gac gtc gca gcc gtg cgc gcc ttg ctt tat gat ctc gcc     979
Thr Ala Asp Asp Val Ala Ala Val Arg Ala Leu Leu Tyr Asp Leu Ala
        280                   285                   290

cca gcc gcc tct gcc aat gcg aaa gcc gaa tac aaa ctc tcc gca gaa     1027
Pro Ala Ala Ser Ala Asn Ala Lys Ala Glu Tyr Lys Leu Ser Ala Glu
    295                   300                   305

gaa gca gaa gaa cac ggc caa aaa gtc ggc gca gct gac tgg agc ttc     1075
Glu Ala Glu Glu His Gly Gln Lys Val Gly Ala Ala Asp Trp Ser Phe
310                   315                   320                   325

tgg gaa gcc aaa gtc cgc gcc cgc gac tac gcc ctg gac gaa acc gaa     1123
Trp Glu Ala Lys Val Arg Ala Arg Asp Tyr Ala Leu Asp Glu Thr Glu
                330                   335                   340

ctg cgc aac tac ttc cca ttg aac caa gta ctc cgt gac ggc gtc ttc     1171
Leu Arg Asn Tyr Phe Pro Leu Asn Gln Val Leu Arg Asp Gly Val Phe
            345                   350                   355

ttc gct gct aac cgc ctc tac gga atc acc gtg gaa cca cgc cct gac     1219
Phe Ala Ala Asn Arg Leu Tyr Gly Ile Thr Val Glu Pro Arg Pro Asp
        360                   365                   370

ctg cgc ggt tac gcc gag ggc gtg gac gtc tgg gaa gtc ctc gat tct     1267
Leu Arg Gly Tyr Ala Glu Gly Val Asp Val Trp Glu Val Leu Asp Ser
    375                   380                   385

gac ggc tcc ggc atc ggc ctg atc ctt acc gac tac tac ggc cga cca     1315
Asp Gly Ser Gly Ile Gly Leu Ile Leu Thr Asp Tyr Tyr Gly Arg Pro
390                 395                 400                 405

tcc aag cgg ggc ggc gct tgg atg tcc agc ttt gtc gac caa tcc gag     1363
Ser Lys Arg Gly Gly Ala Trp Met Ser Ser Phe Val Asp Gln Ser Glu
                410                 415                 420

ctg cta ggc acc aag cca gtc gtg gtc aac gtt atg ggt att acc aaa     1411
Leu Leu Gly Thr Lys Pro Val Val Val Asn Val Met Gly Ile Thr Lys
            425                 430                 435

cca acc acc ggc gaa gca cta ctc agc ctc gat gaa gta acc acc atc     1459
Pro Thr Thr Gly Glu Ala Leu Leu Ser Leu Asp Glu Val Thr Thr Ile
        440                 445                 450

ttc cac gaa ttc ggc cac ggc ctg cac ggc ttg ctg tcc aag gtg cgc     1507
Phe His Glu Phe Gly His Gly Leu His Gly Leu Leu Ser Lys Val Arg
    455                 460                 465

tac cca agc ttc tcc gga acc tcc gtg ccc cgc gac tac gta gaa ttc     1555
Tyr Pro Ser Phe Ser Gly Thr Ser Val Pro Arg Asp Tyr Val Glu Phe
470                 475                 480                 485

ccc tcc cag atc aac gaa aac tgg gca ttc gac cct gca gta gtc cgc     1603
Pro Ser Gln Ile Asn Glu Asn Trp Ala Phe Asp Pro Ala Val Val Arg
                490                 495                 500

aac tac gcc cgc cac gtg gac acc ggc gac atc att cca gac tcc ctg     1651
Asn Tyr Ala Arg His Val Asp Thr Gly Asp Ile Ile Pro Asp Ser Leu
            505                 510                 515

ctt gag gca gtg gaa gca tgt ggc att tca gac aga gtg gtg gaa cat     1699
Leu Glu Ala Val Glu Ala Cys Gly Ile Ser Asp Arg Val Val Glu His
        520                 525                 530

gtg agt act tgt ccc cat cta tta tcg acc tgc cct gtc tct ccc tgt     1747
Val Ser Thr Cys Pro His Leu Leu Ser Thr Cys Pro Val Ser Pro Cys
    535                 540                 545

cca cag cgg atg ccg cac tagtcaatga cattgaccaa tta                   1788
Pro Gln Arg Met Pro His
550                 555


<210> 100
<211> 555
<212> PRT
<213> Corynebacterium glutamicum

<400> 100
Met Thr Val Glu His Leu Leu Lys Pro Ser Thr Leu Pro Tyr Gln Leu
1               5                   10                  15

Pro Asp Phe Ala Ala Ile Lys Val Ala Asp Phe Pro Pro Ala Phe Glu
            20                  25                  30

Leu Ala Leu Ala Glu His Asp Ala Glu Ile Thr Ala Ile Ala Thr Asn
        35                  40                  45

Glu Asp Ala Pro Thr Trp Glu Asn Thr Ile Glu Ala Leu Glu Arg Ala
    50                  55                  60

Gly Leu Ser Leu Asn Arg Val Ala Ala Val Phe Phe Asn Leu Gln Gly
65                  70                  75                  80

Thr Asp Ser Ser Pro Glu Met Asp Glu Ile Ala Ala Thr Ile Ala Pro
                85                  90                  95

Lys Leu Ser Ala His Ser Asp Ala Ile Phe His Asn Ala Ala Leu Phe
            100                 105                 110

Ala Arg Ile Glu Ala Val Glu Ala Pro Ala Asp Glu Glu Ser Gln Arg
        115                 120                 125

Leu Leu Ser His Thr Lys Arg Ala Phe Arg Arg Arg Gly Ala Ala Leu

130                 135                 140

Asn Ala Asp Gly Lys Ala Arg Leu Ser Thr Ile Asn Gln Arg Leu Ser
145                 150                 155                 160

Ala Leu Ser Glu Gln Phe Gly Arg Asn Leu Leu Gln Asp Thr Arg Asp
                165                 170                 175

Leu Ala Val Asn Phe Glu Glu Ser Glu Leu Ala Gly Phe Ser Glu Ala
            180                 185                 190

Arg Ile Ser Ala Ala Ala Asp Tyr Ala Ala Ala Val Gly Thr Glu Gly
        195                 200                 205

Tyr Val Val Pro Leu Glu Leu Pro Thr Val Gln Ser Glu Gln Ala Val
    210                 215                 220

Leu Thr Glu Ser Ala Ser Arg Ala Lys Leu Tyr Glu Ala Ser Gln Lys
225                 230                 235                 240

Arg Gly Ala Ser Leu Asn Lys Asp Val Leu Leu Glu Thr Val Arg Leu
                245                 250                 255

Arg Ala Glu Arg Ala Thr Leu Leu Gly Tyr Asp Thr His Ala Asp Tyr
            260                 265                 270

Val Ile Glu Glu Glu Thr Ala Asp Asp Val Ala Ala Val Arg Ala Leu
        275                 280                 285

Leu Tyr Asp Leu Ala Pro Ala Ala Ser Ala Asn Ala Lys Ala Glu Tyr
    290                 295                 300

Lys Leu Ser Ala Glu Glu Ala Glu Glu His Gly Gln Lys Val Gly Ala
305                 310                 315                 320

Ala Asp Trp Ser Phe Trp Glu Ala Lys Val Arg Ala Arg Asp Tyr Ala
                325                 330                 335

Leu Asp Glu Thr Glu Leu Arg Asn Tyr Phe Pro Leu Asn Gln Val Leu
            340                 345                 350

Arg Asp Gly Val Phe Phe Ala Ala Asn Arg Leu Tyr Gly Ile Thr Val
        355                 360                 365

Glu Pro Arg Pro Asp Leu Arg Gly Tyr Ala Glu Gly Val Asp Val Trp
    370                 375                 380

Glu Val Leu Asp Ser Asp Gly Ser Gly Ile Gly Leu Ile Leu Thr Asp
385                 390                 395                 400

Tyr Tyr Gly Arg Pro Ser Lys Arg Gly Gly Ala Trp Met Ser Ser Phe
                405                 410                 415

Val Asp Gln Ser Glu Leu Leu Gly Thr Lys Pro Val Val Val Asn Val
            420                 425                 430

Met Gly Ile Thr Lys Pro Thr Thr Gly Glu Ala Leu Leu Ser Leu Asp
        435                 440                 445

Glu Val Thr Thr Ile Phe His Glu Phe Gly His Gly Leu His Gly Leu
    450                 455                 460

Leu Ser Lys Val Arg Tyr Pro Ser Phe Ser Gly Thr Ser Val Pro Arg
465                 470                 475                 480

Asp Tyr Val Glu Phe Pro Ser Gln Ile Asn Glu Asn Trp Ala Phe Asp
                485                 490                 495

Pro Ala Val Val Arg Asn Tyr Ala Arg His Val Asp Thr Gly Asp Ile
            500                 505                 510

Ile Pro Asp Ser Leu Leu Glu Ala Val Glu Ala Cys Gly Ile Ser Asp
        515                 520                 525

Arg Val Val Glu His Val Ser Thr Cys Pro His Leu Leu Ser Thr Cys
    530                 535                 540

Pro Val Ser Pro Cys Pro Gln Arg Met Pro His
545                 550                 555

<210> 101
<211> 1088
<212> DNA
<213> Corynebacterium glutamicum

<220>
<221> CDS
<222> (1)..(1065)
<223> FRXA00877

<400> 101
gca gca gca gtt ggc acc gaa ggc tac gtg gtt cca ctg gaa ctg ccc      48
Ala Ala Ala Val Gly Thr Glu Gly Tyr Val Val Pro Leu Glu Leu Pro
1               5                   10                  15

acc gtg cag tca gag cag gca gta tta acc gaa tcc gcc tcg cgt gca      96
Thr Val Gln Ser Glu Gln Ala Val Leu Thr Glu Ser Ala Ser Arg Ala
            20                  25                  30

aag ctt tat gaa gcc tcc cag aag cgt ggc gcc agc ctg aac aag gac      144
Lys Leu Tyr Glu Ala Ser Gln Lys Arg Gly Ala Ser Leu Asn Lys Asp
        35                  40                  45

gtg ctg ctc gaa acc gtg cgt ctg cgt gct gaa cgc gcc aca ctt tta      192
Val Leu Leu Glu Thr Val Arg Leu Arg Ala Glu Arg Ala Thr Leu Leu
    50                  55                  60

ggc tac gac acc cac gcc gat tac gtc atc gaa gaa gaa acc gcc gat      240
Gly Tyr Asp Thr His Ala Asp Tyr Val Ile Glu Glu Glu Thr Ala Asp
65                  70                  75                  80

gac gtc gca gcc gtg cgc gcc ttg ctt tat gat ctc gcc cca gcc gcc      288
Asp Val Ala Ala Val Arg Ala Leu Leu Tyr Asp Leu Ala Pro Ala Ala
                85                  90                  95

tct gcc aat gcg aaa gcc gaa tac aaa ctc tcc gca gaa gaa gca gaa      336
Ser Ala Asn Ala Lys Ala Glu Tyr Lys Leu Ser Ala Glu Glu Ala Glu
            100                 105                 110

gaa cac ggc caa aaa gtc ggc gca gct gac tgg agc ttc tgg gaa gcc      384
Glu His Gly Gln Lys Val Gly Ala Ala Asp Trp Ser Phe Trp Glu Ala
        115                 120                 125

aaa gtc cgc gcc cgc gac tac gcc ctg gac gaa acc gaa ctg cgc aac      432
Lys Val Arg Ala Arg Asp Tyr Ala Leu Asp Glu Thr Glu Leu Arg Asn
    130                 135                 140

tac ttc cca ttg aac caa gta ctc cgt gac ggc gtc ttc ttc gct gct      480
Tyr Phe Pro Leu Asn Gln Val Leu Arg Asp Gly Val Phe Phe Ala Ala
145                 150                 155                 160

aac cgc ctc tac gga atc acc gtg gaa cca cgc cct gac ctg cgc ggt      528
Asn Arg Leu Tyr Gly Ile Thr Val Glu Pro Arg Pro Asp Leu Arg Gly
                165                 170                 175

tac gcc gag ggc gtg gac gtc tgg gaa gtc ctc gat tct gac ggc tcc      576
Tyr Ala Glu Gly Val Asp Val Trp Glu Val Leu Asp Ser Asp Gly Ser
            180                 185                 190

ggc atc ggc ctg atc ctt acc gac tac tac ggc cga cca tcc aag cgg      624
Gly Ile Gly Leu Ile Leu Thr Asp Tyr Tyr Gly Arg Pro Ser Lys Arg
        195                 200                 205

ggc ggc gct tgg atg tcc agc ttt gtc gac caa tcc gag ctg cta ggc      672
Gly Gly Ala Trp Met Ser Ser Phe Val Asp Gln Ser Glu Leu Leu Gly
    210                 215                 220

acc aag cca gtc gtg gtc aac gtt atg ggt att acc aaa cca acc acc      720
Thr Lys Pro Val Val Val Asn Val Met Gly Ile Thr Lys Pro Thr Thr
225                 230                 235                 240

ggc gaa gca cta ctc agc ctc gat gaa gta acc acc atc ttc cac gaa      768
Gly Glu Ala Leu Leu Ser Leu Asp Glu Val Thr Thr Ile Phe His Glu

245                 250                 255

ttc ggc cac ggc ctg cac ggc ttg ctg tcc aag gtg cgc tac cca agc      816
Phe Gly His Gly Leu His Gly Leu Leu Ser Lys Val Arg Tyr Pro Ser
            260                 265                 270

ttc tcc gga acc tcc gtg ccc cgc gac tac gta gaa ttc ccc tcc cag      864
Phe Ser Gly Thr Ser Val Pro Arg Asp Tyr Val Glu Phe Pro Ser Gln
        275                 280                 285

atc aac gaa aac tgg gca ttc gac cct gca gta gtc cgc aac tac gcc      912
Ile Asn Glu Asn Trp Ala Phe Asp Pro Ala Val Val Arg Asn Tyr Ala
    290                 295                 300

cgc cac gtg gac acc ggc gac atc att cca gac tcc ctg ctt gag gca      960
Arg His Val Asp Thr Gly Asp Ile Ile Pro Asp Ser Leu Leu Glu Ala
305                 310                 315                 320

gtg gaa gca tgt ggc att tca gac aga gtg gtg gaa cat gtg agt act     1008
Val Glu Ala Cys Gly Ile Ser Asp Arg Val Val Glu His Val Ser Thr
                325                 330                 335

tgt ccc cat cta tta tcg acc tgc cct gtc tct ccc tgt cca cag cgg     1056
Cys Pro His Leu Leu Ser Thr Cys Pro Val Ser Pro Cys Pro Gln Arg
            340                 345                 350

atg ccg cac tagtcaatga cattgaccaa tta                               1088
Met Pro His
        355


<210> 102
<211> 355
<212> PRT
<213> Corynebacterium glutamicum

<400> 102
Ala Ala Ala Val Gly Thr Glu Gly Tyr Val Val Pro Leu Glu Leu Pro
1               5                   10                  15

Thr Val Gln Ser Glu Gln Ala Val Leu Thr Glu Ser Ala Ser Arg Ala
            20                  25                  30

Lys Leu Tyr Glu Ala Ser Gln Lys Arg Gly Ala Ser Leu Asn Lys Asp
        35                  40                  45

Val Leu Leu Glu Thr Val Arg Leu Arg Ala Glu Arg Ala Thr Leu Leu
    50                  55                  60

Gly Tyr Asp Thr His Ala Asp Tyr Val Ile Glu Glu Glu Thr Ala Asp
65                  70                  75                  80

Asp Val Ala Ala Val Arg Ala Leu Leu Tyr Asp Leu Ala Pro Ala Ala
                85                  90                  95

Ser Ala Asn Ala Lys Ala Glu Tyr Lys Leu Ser Ala Glu Glu Ala Glu
            100                 105                 110

Glu His Gly Gln Lys Val Gly Ala Ala Asp Trp Ser Phe Trp Glu Ala
        115                 120                 125

Lys Val Arg Ala Arg Asp Tyr Ala Leu Asp Glu Thr Glu Leu Arg Asn
    130                 135                 140

Tyr Phe Pro Leu Asn Gln Val Leu Arg Asp Gly Val Phe Phe Ala Ala
145                 150                 155                 160

Asn Arg Leu Tyr Gly Ile Thr Val Glu Pro Arg Pro Asp Leu Arg Gly
                165                 170                 175

Tyr Ala Glu Gly Val Asp Val Trp Glu Val Leu Asp Ser Asp Gly Ser
            180                 185                 190

Gly Ile Gly Leu Ile Leu Thr Asp Tyr Tyr Gly Arg Pro Ser Lys Arg
        195                 200                 205

Gly Gly Ala Trp Met Ser Ser Phe Val Asp Gln Ser Glu Leu Leu Gly

210                 215                 220

Thr Lys Pro Val Val Val Asn Val Met Gly Ile Thr Lys Pro Thr Thr
225                 230                 235                 240

Gly Glu Ala Leu Leu Ser Leu Asp Glu Val Thr Thr Ile Phe His Glu
                245                 250                 255

Phe Gly His Gly Leu His Gly Leu Leu Ser Lys Val Arg Tyr Pro Ser
            260                 265                 270

Phe Ser Gly Thr Ser Val Pro Arg Asp Tyr Val Glu Phe Pro Ser Gln
        275                 280                 285

Ile Asn Glu Asn Trp Ala Phe Asp Pro Ala Val Val Arg Asn Tyr Ala
    290                 295                 300

Arg His Val Asp Thr Gly Asp Ile Ile Pro Asp Ser Leu Leu Glu Ala
305                 310                 315                 320

Val Glu Ala Cys Gly Ile Ser Asp Arg Val Val Glu His Val Ser Thr
                325                 330                 335

Cys Pro His Leu Leu Ser Thr Cys Pro Val Ser Pro Cys Pro Gln Arg
            340                 345                 350

Met Pro His
        355


<210> 103
<211> 2724
<212> DNA
<213> Corynebacterium glutamicum

<220>
<221> CDS
<222> (101)..(2701)
<223> RXN01014

<400> 103
   tcttaaagtt ttctagcaat ccacactagg cgcgaactat cgtggtgtca ttgcgcacct 60
   tctaagggta gcgccccctc aaatttcaag gagcattaaa ttg acg tcc act aat   115
                                              Leu Thr Ser Thr Asn
                                                1               5
   ctc acc cga cag gaa gct tcg gat cgt tcg agg tta ctg agt gta gaa    163
   Leu Thr Arg Gln Glu Ala Ser Asp Arg Ser Arg Leu Leu Ser Val Glu
                   10                  15                  20
   aac tat gac att gca ctt gat ctc aac aac ggt gat gag ttt ttt agt    211
   Asn Tyr Asp Ile Ala Leu Asp Leu Asn Asn Gly Asp Glu Phe Phe Ser
               25                  30                  35
   tcc tcc acc gtt gtc agc ttc act gtc agg aag gct ggc gat acc ttt    259
   Ser Ser Thr Val Val Ser Phe Thr Val Arg Lys Ala Gly Asp Thr Phe
           40                  45                  50
   att gat ctc cgc gca gca agc gtt gag gag gtt cgc ctg gac aat gtg    307
   Ile Asp Leu Arg Ala Ala Ser Val Glu Glu Val Arg Leu Asp Asn Val
       55                  60                  65
   tcc atc aaa gat gag gct cta acc ctt ggc aag aac ggc tac gac gag    355
   Ser Ile Lys Asp Glu Ala Leu Thr Leu Gly Lys Asn Gly Tyr Asp Glu
   70                  75                  80                  85
   acg ttc ggc atc gcc ctg aag ggt ctt act ccc ggc gcg cac acc ttg    403
   Thr Phe Gly Ile Ala Leu Lys Gly Leu Thr Pro Gly Ala His Thr Leu
                   90                  95                  100
   cgg gta acg gcg tct atc ccc tat tcc cgc acc ggt gaa ggc ctg cac    451
   Arg Val Thr Ala Ser Ile Pro Tyr Ser Arg Thr Gly Glu Gly Leu His
               105                 110                 115
   cgc atg gtg gat cca gca gac aat gag gtg tat ttg tac acc cag ttt    499
   Arg Met Val Asp Pro Ala Asp Asn Glu Val Tyr Leu Tyr Thr Gln Phe
   120 125 130
   gag acc gcc gat gcc aag cgt atg ttc gcg tgt ttc gat cag cca gac 547
   Glu Thr Ala Asp Ala Lys Arg Met Phe Ala Cys Phe Asp Gln Pro Asp
   135 140 145
   ctc aag gct acc tat gat ctg aac atc aaa act cct aag ggt tgg aag 595
   Leu Lys Ala Thr Tyr Asp Leu Asn Ile Lys Thr Pro Lys Gly Trp Lys
   150 155 160 165
   atc att tcc aac tct gag cag cag gtt tcc act cag cac act gat tac 643
   Ile Ile Ser Asn Ser Glu Gln Gln Val Ser Thr Gln His Thr Asp Tyr
   170 175 180
   gat acc cac att tcc cga gtg gac tat ccc ctc tcc acc tac ctg att 691
   Asp Thr His Ile Ser Arg Val Asp Tyr Pro Leu Ser Thr Tyr Leu Ile
   185 190 195
   gcg gtg tgc gcg ggt cgt tac cac gag gtg tgc gat gtc tgg aag ggt 739
   Ala Val Cys Ala Gly Arg Tyr His Glu Val Cys Asp Val Trp Lys Gly
   200 205 210
   acg ctc acc cac cat gca gaa aca cct gcc gat cag cca act gag ctg 787
   Thr Leu Thr His His Ala Glu Thr Pro Ala Asp Gln Pro Thr Glu Leu
   215 220 225
   act gtt ccg ctt gct ctc tac tgc cgc agt tct ttg gct aaa gat ctt 835
   Thr Val Pro Leu Ala Leu Tyr Cys Arg Ser Ser Leu Ala Lys Asp Leu
   230 235 240 245
   gat gcg gtg cgt ctg ttt acc gaa acg aag cag ggc ttt gat tgg tac 883
   Asp Ala Val Arg Leu Phe Thr Glu Thr Lys Gln Gly Phe Asp Trp Tyr
   250 255 260
   cac cgc aac ttc ggt gtg gcg tac cca ttc ggc aag tac gat cag atc 931
   His Arg Asn Phe Gly Val Ala Tyr Pro Phe Gly Lys Tyr Asp Gln Ile
   265 270 275
   ttc gtc cct gaa ttt aat gct ggc gcg atg gag aac gcc ggc gct gtc 979
   Phe Val Pro Glu Phe Asn Ala Gly Ala Met Glu Asn Ala Gly Ala Val
   280 285 290
   acc atc cgc gat gag tac gtt ttt gca tcc aag gca acc cgt tac cgc 1027
   Thr Ile Arg Asp Glu Tyr Val Phe Ala Ser Lys Ala Thr Arg Tyr Arg
   295 300 305
   tac gag cgc cgc gct gaa acc atc ctt cac gag ctc gct cac atg tgg 1075
   Tyr Glu Arg Arg Ala Glu Thr Ile Leu His Glu Leu Ala His Met Trp
   310 315 320 325
   ttc ggt gtg ctg gtg acc atg cag tgg tgg gat gat ctg tgg ctc aac 1123
   Phe Gly Val Leu Val Thr Met Gln Trp Trp Asp Asp Leu Trp Leu Asn
   330 335 340
   gag tcc ttc gcc act tgg tcc gcg gca att tct cag gct gag gaa act 1171
   Glu Ser Phe Ala Thr Trp Ser Ala Ala Ile Ser Gln Ala Glu Glu Thr
   345 350 355
   gaa tac aac act gca tgg gtg act ttc gcc aat gtg gag aag tcg tgg 1219
   Glu Tyr Asn Thr Ala Trp Val Thr Phe Ala Asn Val Glu Lys Ser Trp
   360 365 370
   gcg tac cag cag gat cag ctg cct tcc acc cac ccg gtg ttc tct gac 1267
   Ala Tyr Gln Gln Asp Gln Leu Pro Ser Thr His Pro Val Phe Ser Asp
   375 380 385
   gga tac gac att gag act gtc gac cag aac ttc gac ggc atc acc tac 1315
   Gly Tyr Asp Ile Glu Thr Val Asp Gln Asn Phe Asp Gly Ile Thr Tyr
   390 395 400 405
   gca aag ggc gcc tcg gtg ctc aag cag ctg cag gca tac gtt ggc cgt 1363
   Ala Lys Gly Ala Ser Val Leu Lys Gln Leu Gln Ala Tyr Val Gly Arg
   410 415 420
   gag gaa ttc ctg gca ggc gta cgc agg cac ttt gcc aac cac gca tgg 1411
   Glu Glu Phe Leu Ala Gly Val Arg Arg His Phe Ala Asn His Ala Trp
   425 430 435
   ggc aac gcc agc ttt gat gat ctg ctc ggc gcc ctc gag cag tcc tcc 1459
   Gly Asn Ala Ser Phe Asp Asp Leu Leu Gly Ala Leu Glu Gln Ser Ser
   440 445 450
   ggc cgc gac ctc tcc gac tgg gca aac cag tgg ctc aag acc acc ggc 1507
   Gly Arg Asp Leu Ser Asp Trp Ala Asn Gln Trp Leu Lys Thr Thr Gly
   455 460 465
   atc aac acc ctc ggc gca aag ttc acc acc gac aac ggc aaa tac acc 1555
   Ile Asn Thr Leu Gly Ala Lys Phe Thr Thr Asp Asn Gly Lys Tyr Thr
   470 475 480 485
   tcc ttc tcc gtc acc cag acc ggc gcc gcg ccg ggt gcc ggt gag ctg 1603
   Ser Phe Ser Val Thr Gln Thr Gly Ala Ala Pro Gly Ala Gly Glu Leu
   490 495 500
   cgg act cac cgc atc gcg gtg ggt ctt tat aag ctt gtc gac gga tcc 1651
   Arg Thr His Arg Ile Ala Val Gly Leu Tyr Lys Leu Val Asp Gly Ser
   505 510 515
   ctc aac cgc tac gca cga gta gaa ctt gac tgc agt ggc gcg tcg aca 1699
   Leu Asn Arg Tyr Ala Arg Val Glu Leu Asp Cys Ser Gly Ala Ser Thr
   520 525 530
   agc gtt gaa gag atc gtt gga ctt gag cag gct gac ttc gtg ctg gtc 1747
   Ser Val Glu Glu Ile Val Gly Leu Glu Gln Ala Asp Phe Val Leu Val
   535 540 545
   aac gat gat gat ctg acg tat gcg ctg ctg gat ctg gat gat gat tca 1795
   Asn Asp Asp Asp Leu Thr Tyr Ala Leu Leu Asp Leu Asp Asp Asp Ser
   550 555 560 565
   cgc aat ttt gtc atc gac aat att gat aag ttc agc gac cct atg cct 1843
   Arg Asn Phe Val Ile Asp Asn Ile Asp Lys Phe Ser Asp Pro Met Pro
   570 575 580
   cgc acg ctg gtg tgg tcc gct gcg tgg gag atg act cgc gct ggt cag 1891
   Arg Thr Leu Val Trp Ser Ala Ala Trp Glu Met Thr Arg Ala Gly Gln
   585 590 595
   atg aag gct cgt gat ttc atc gcg ctg gtt gct cgt ggc gct gct gcg 1939
   Met Lys Ala Arg Asp Phe Ile Ala Leu Val Ala Arg Gly Ala Ala Ala
   600 605 610
   gaa act gaa att gct gtg ctg gag cgc att ctc gcg cag gct acc tct 1987
   Glu Thr Glu Ile Ala Val Leu Glu Arg Ile Leu Ala Gln Ala Thr Ser
   615 620 625
   gcg ctg aag agc tac gcc gac cca gcg tgg gca gaa gca act gga aat 2035
   Ala Leu Lys Ser Tyr Ala Asp Pro Ala Trp Ala Glu Ala Thr Gly Asn
   630 635 640 645
   gac ctg ctg gcc gat gct ttc ctt gag ggt gct cgc tcc gca gaa cca 2083
   Asp Leu Leu Ala Asp Ala Phe Leu Glu Gly Ala Arg Ser Ala Glu Pro
   650 655 660
   gac tcc gac act cag ttg gcg ttc att cag gct ctg gca aaa gca acg 2131
   Asp Ser Asp Thr Gln Leu Ala Phe Ile Gln Ala Leu Ala Lys Ala Thr
   665 670 675
   ctc aat gat gct gct gcc gat tac ttc cgc gac att ctt gcc ggc aac 2179
   Leu Asn Asp Ala Ala Ala Asp Tyr Phe Arg Asp Ile Leu Ala Gly Asn
   680 685 690
   gtc gaa ggc ctg acc gtg gat cct gac ctg cgt tgg tgg gca ctg act 2227
   Val Glu Gly Leu Thr Val Asp Pro Asp Leu Arg Trp Trp Ala Leu Thr
   695 700 705
   gcg ctt atc gcc cgt ggt gac atc gag gct gtc gaa gat gca atc gcc 2275
   Ala Leu Ile Ala Arg Gly Asp Ile Glu Ala Val Glu Asp Ala Ile Ala
   710 715 720 725
   gct gaa ctt tcc cgc gac aac tcc agt gcc tcc ttc ctc gca tca ctt 2323
   Ala Glu Leu Ser Arg Asp Asn Ser Ser Ala Ser Phe Leu Ala Ser Leu
   730 735 740
   cga gcc ggt gcc gct gtg aac act gaa gaa gtg aag gct gct gca tac     2371
   Arg Ala Gly Ala Ala Val Asn Thr Glu Glu Val Lys Ala Ala Ala Tyr
               745                 750                 755
   aag cat gtc acg gca gtt gat agt ggc cta tcc aac ctg gag ctg cgc     2419
   Lys His Val Thr Ala Val Asp Ser Gly Leu Ser Asn Leu Glu Leu Arg
           760                 765                 770
   cac aag att gaa ggc ctc aca ttc act ggc tct tct gaa ctg ctg caa     2467
   His Lys Ile Glu Gly Leu Thr Phe Thr Gly Ser Ser Glu Leu Leu Gln
       775                 780                 785
   gcc tac aac gag cag tac ttc gaa atc ctt gat gat gtg tgg gcg aac     2515
   Ala Tyr Asn Glu Gln Tyr Phe Glu Ile Leu Asp Asp Val Trp Ala Asn
   790                 795                 800                 805
   ttc tcc ggc gaa atg gca cag cag atc gtc ctc gga ctg ttc cct tca     2563
   Phe Ser Gly Glu Met Ala Gln Gln Ile Val Leu Gly Leu Phe Pro Ser
                   810                 815                 820
   tgg aac gtt tcc gaa gag ggt ctc aag cgt acc gac gag ttt ctt gat     2611
   Trp Asn Val Ser Glu Glu Gly Leu Lys Arg Thr Asp Glu Phe Leu Asp
               825                 830                 835
   ggc gaa cat gtc gca ggc atc aag cga att gtt tcc gaa tcc ctc gac     2659
   Gly Glu His Val Ala Gly Ile Lys Arg Ile Val Ser Glu Ser Leu Asp
           840                 845                 850
   cgc act gcc cgt gct ctg cgc aac cgt gcg gca gat gct gcg             2701
   Arg Thr Ala Arg Ala Leu Arg Asn Arg Ala Ala Asp Ala Ala
       855                 860                 865
   taagtaaaag attctcaatc cca                                           2724


<210> 104
<211> 867
<212> PRT
<213> Corynebacterium glutamicum

<400> 104
Leu Thr Ser Thr Asn Leu Thr Arg Gln Glu Ala Ser Asp Arg Ser Arg
1               5                   10                  15

Leu Leu Ser Val Glu Asn Tyr Asp Ile Ala Leu Asp Leu Asn Asn Gly
            20                  25                  30

Asp Glu Phe Phe Ser Ser Ser Thr Val Val Ser Phe Thr Val Arg Lys
        35                  40                  45

Ala Gly Asp Thr Phe Ile Asp Leu Arg Ala Ala Ser Val Glu Glu Val
    50                  55                  60

Arg Leu Asp Asn Val Ser Ile Lys Asp Glu Ala Leu Thr Leu Gly Lys
65                  70                  75                  80

Asn Gly Tyr Asp Glu Thr Phe Gly Ile Ala Leu Lys Gly Leu Thr Pro
                85                  90                  95

Gly Ala His Thr Leu Arg Val Thr Ala Ser Ile Pro Tyr Ser Arg Thr
            100                 105                 110

Gly Glu Gly Leu His Arg Met Val Asp Pro Ala Asp Asn Glu Val Tyr
        115                 120                 125

Leu Tyr Thr Gln Phe Glu Thr Ala Asp Ala Lys Arg Met Phe Ala Cys
    130                 135                 140

Phe Asp Gln Pro Asp Leu Lys Ala Thr Tyr Asp Leu Asn Ile Lys Thr
145                 150                 155                 160

Pro Lys Gly Trp Lys Ile Ile Ser Asn Ser Glu Gln Gln Val Ser Thr
                165                 170                 175

Gln His Thr Asp Tyr Asp Thr His Ile Ser Arg Val Asp Tyr Pro Leu
            180                 185                 190

Ser Thr Tyr Leu Ile Ala Val Cys Ala Gly Arg Tyr His Glu Val Cys
        195                 200                 205

Asp Val Trp Lys Gly Thr Leu Thr His His Ala Glu Thr Pro Ala Asp
    210                 215                 220

Gln Pro Thr Glu Leu Thr Val Pro Leu Ala Leu Tyr Cys Arg Ser Ser
225                 230                 235                 240

Leu Ala Lys Asp Leu Asp Ala Val Arg Leu Phe Thr Glu Thr Lys Gln
                245                 250                 255

Gly Phe Asp Trp Tyr His Arg Asn Phe Gly Val Ala Tyr Pro Phe Gly
            260                 265                 270

Lys Tyr Asp Gln Ile Phe Val Pro Glu Phe Asn Ala Gly Ala Met Glu
        275                 280                 285

Asn Ala Gly Ala Val Thr Ile Arg Asp Glu Tyr Val Phe Ala Ser Lys
    290                 295                 300

Ala Thr Arg Tyr Arg Tyr Glu Arg Arg Ala Glu Thr Ile Leu His Glu
305                 310                 315                 320

Leu Ala His Met Trp Phe Gly Val Leu Val Thr Met Gln Trp Trp Asp
                325                 330                 335

Asp Leu Trp Leu Asn Glu Ser Phe Ala Thr Trp Ser Ala Ala Ile Ser
            340                 345                 350

Gln Ala Glu Glu Thr Glu Tyr Asn Thr Ala Trp Val Thr Phe Ala Asn
        355                 360                 365

Val Glu Lys Ser Trp Ala Tyr Gln Gln Asp Gln Leu Pro Ser Thr His
    370                 375                 380

Pro Val Phe Ser Asp Gly Tyr Asp Ile Glu Thr Val Asp Gln Asn Phe
385                 390                 395                 400

Asp Gly Ile Thr Tyr Ala Lys Gly Ala Ser Val Leu Lys Gln Leu Gln
                405                 410                 415

Ala Tyr Val Gly Arg Glu Glu Phe Leu Ala Gly Val Arg Arg His Phe
            420                 425                 430

Ala Asn His Ala Trp Gly Asn Ala Ser Phe Asp Asp Leu Leu Gly Ala
        435                 440                 445

Leu Glu Gln Ser Ser Gly Arg Asp Leu Ser Asp Trp Ala Asn Gln Trp
    450                 455                 460

Leu Lys Thr Thr Gly Ile Asn Thr Leu Gly Ala Lys Phe Thr Thr Asp
465                 470                 475                 480

Asn Gly Lys Tyr Thr Ser Phe Ser Val Thr Gln Thr Gly Ala Ala Pro
                485                 490                 495

Gly Ala Gly Glu Leu Arg Thr His Arg Ile Ala Val Gly Leu Tyr Lys
            500                 505                 510

Leu Val Asp Gly Ser Leu Asn Arg Tyr Ala Arg Val Glu Leu Asp Cys
        515                 520                 525

Ser Gly Ala Ser Thr Ser Val Glu Glu Ile Val Gly Leu Glu Gln Ala
    530                 535                 540

Asp Phe Val Leu Val Asn Asp Asp Asp Leu Thr Tyr Ala Leu Leu Asp
545                 550                 555                 560

Leu Asp Asp Asp Ser Arg Asn Phe Val Ile Asp Asn Ile Asp Lys Phe
                565                 570                 575

Ser Asp Pro Met Pro Arg Thr Leu Val Trp Ser Ala Ala Trp Glu Met
            580                 585                 590

Thr Arg Ala Gly Gln Met Lys Ala Arg Asp Phe Ile Ala Leu Val Ala
        595                 600                 605

Arg Gly Ala Ala Ala Glu Thr Glu Ile Ala Val Leu Glu Arg Ile Leu
    610                 615                 620

Ala Gln Ala Thr Ser Ala Leu Lys Ser Tyr Ala Asp Pro Ala Trp Ala
625                 630                 635                 640

Glu Ala Thr Gly Asn Asp Leu Leu Ala Asp Ala Phe Leu Glu Gly Ala
                645                 650                 655

Arg Ser Ala Glu Pro Asp Ser Asp Thr Gln Leu Ala Phe Ile Gln Ala
            660                 665                 670

Leu Ala Lys Ala Thr Leu Asn Asp Ala Ala Ala Asp Tyr Phe Arg Asp
        675                 680                 685

Ile Leu Ala Gly Asn Val Glu Gly Leu Thr Val Asp Pro Asp Leu Arg
    690                 695                 700

Trp Trp Ala Leu Thr Ala Leu Ile Ala Arg Gly Asp Ile Glu Ala Val
705                 710                 715                 720

Glu Asp Ala Ile Ala Ala Glu Leu Ser Arg Asp Asn Ser Ser Ala Ser
                725                 730                 735

Phe Leu Ala Ser Leu Arg Ala Gly Ala Ala Val Asn Thr Glu Glu Val
            740                 745                 750

Lys Ala Ala Ala Tyr Lys His Val Thr Ala Val Asp Ser Gly Leu Ser
        755                 760                 765

Asn Leu Glu Leu Arg His Lys Ile Glu Gly Leu Thr Phe Thr Gly Ser
    770                 775                 780

Ser Glu Leu Leu Gln Ala Tyr Asn Glu Gln Tyr Phe Glu Ile Leu Asp
785                 790                 795                 800

Asp Val Trp Ala Asn Phe Ser Gly Glu Met Ala Gln Gln Ile Val Leu
                805                 810                 815

Gly Leu Phe Pro Ser Trp Asn Val Ser Glu Glu Gly Leu Lys Arg Thr
            820                 825                 830

Asp Glu Phe Leu Asp Gly Glu His Val Ala Gly Ile Lys Arg Ile Val
        835                 840                 845

Ser Glu Ser Leu Asp Arg Thr Ala Arg Ala Leu Arg Asn Arg Ala Ala
    850                 855                 860

Asp Ala Ala
865


<210> 105
<211> 1578
<212> DNA
<213> Corynebacterium glutamicum

<220>
<221> CDS
<222> (1)..(1578)
<223> FRXA01014

<400> 105
gat gat ctg tgg ctc aac gag tcc ttc gcc act tgg tcc gcg gca att      48
Asp Asp Leu Trp Leu Asn Glu Ser Phe Ala Thr Trp Ser Ala Ala Ile
  1               5                  10                  15

tct cag gct gag gaa act gaa tac aac act gca tgg gtg act ttc gcc      96
Ser Gln Ala Glu Glu Thr Glu Tyr Asn Thr Ala Trp Val Thr Phe Ala
             20                  25                  30

aat gtg gag aag tcg tgg gcg tac cag cag gat cag ctg cct tcc acc     144
Asn Val Glu Lys Ser Trp Ala Tyr Gln Gln Asp Gln Leu Pro Ser Thr
         35                  40                  45

cac ccg gtg ttc tct gac gga tac gac att gag act gtc gac cag aac     192

His Pro Val Phe Ser Asp Gly Tyr Asp Ile Glu Thr Val Asp Gln Asn
    50                  55                  60

ttc gac ggc atc acc tac gca aag ggc gcc tcg gtg ctc aag cag ctg     240
Phe Asp Gly Ile Thr Tyr Ala Lys Gly Ala Ser Val Leu Lys Gln Leu
65                  70                  75                  80

cag gca tac gtt ggc cgt gag gaa ttc ctg gca ggc gta cgc agg cac     288
Gln Ala Tyr Val Gly Arg Glu Glu Phe Leu Ala Gly Val Arg Arg His
                85                  90                  95

ttt gcc aac cac gca tgg ggc aac gcc agc ttt gat gat ctg ctc ggc     336
Phe Ala Asn His Ala Trp Gly Asn Ala Ser Phe Asp Asp Leu Leu Gly
            100                 105                 110

gcc ctc gag cag tcc tcc ggc cgc gac ctc tcc gac tgg gca aac cag     384
Ala Leu Glu Gln Ser Ser Gly Arg Asp Leu Ser Asp Trp Ala Asn Gln
        115                 120                 125

tgg ctc aag acc acc ggc atc aac acc ctc ggc gca aag ttc acc acc     432
Trp Leu Lys Thr Thr Gly Ile Asn Thr Leu Gly Ala Lys Phe Thr Thr
    130                 135                 140

gac aac ggc aaa tac acc tcc ttc tcc gtc acc cag acc ggc gcc gcg     480
Asp Asn Gly Lys Tyr Thr Ser Phe Ser Val Thr Gln Thr Gly Ala Ala
145                 150                 155                 160

ccg ggt gcc ggt gag ctg cgg act cac cgc atc gcg gtg ggt ctt tat     528
Pro Gly Ala Gly Glu Leu Arg Thr His Arg Ile Ala Val Gly Leu Tyr
                165                 170                 175

aag ctt gtc gac gga tcc ctc aac cgc tac gca cga gta gaa ctt gac     576
Lys Leu Val Asp Gly Ser Leu Asn Arg Tyr Ala Arg Val Glu Leu Asp
            180                 185                 190

tgc agt ggc gcg tcg aca agc gtt gaa gag atc gtt gga ctt gag cag     624
Cys Ser Gly Ala Ser Thr Ser Val Glu Glu Ile Val Gly Leu Glu Gln
        195                 200                 205

gct gac ttc gtg ctg gtc aac gat gat gat ctg acg tat gcg ctg ctg     672
Ala Asp Phe Val Leu Val Asn Asp Asp Asp Leu Thr Tyr Ala Leu Leu
    210                 215                 220

gat ctg gat gat gat tca cgc aat ttt gtc atc gac aat att gat aag     720
Asp Leu Asp Asp Asp Ser Arg Asn Phe Val Ile Asp Asn Ile Asp Lys
225                 230                 235                 240

ttc agc gac cct atg cct cgc acg ctg gtg tgg tcc gct gcg tgg gag     768
Phe Ser Asp Pro Met Pro Arg Thr Leu Val Trp Ser Ala Ala Trp Glu
                245                 250                 255

atg act cgc gct ggt cag atg aag gct cgt gat ttc atc gcg ctg gtt     816
Met Thr Arg Ala Gly Gln Met Lys Ala Arg Asp Phe Ile Ala Leu Val
            260                 265                 270

gct cgt ggc gct gct gcg gaa act gaa att gct gtg ctg gag cgc att     864
Ala Arg Gly Ala Ala Ala Glu Thr Glu Ile Ala Val Leu Glu Arg Ile
        275                 280                 285

ctc gcg cag gct acc tct gcg ctg aag agc tac gcc gac cca gcg tgg     912
Leu Ala Gln Ala Thr Ser Ala Leu Lys Ser Tyr Ala Asp Pro Ala Trp
    290                 295                 300

gca gaa gca act gga aat gac ctg ctg gcc gat gct ttc ctt gag ggt     960
Ala Glu Ala Thr Gly Asn Asp Leu Leu Ala Asp Ala Phe Leu Glu Gly
305                 310                 315                 320

gct cgc tcc gca gaa cca gac tcc gac act cag ttg gcg ttc att cag     1008
Ala Arg Ser Ala Glu Pro Asp Ser Asp Thr Gln Leu Ala Phe Ile Gln
                325                 330                 335

gct ctg gca aaa gca acg ctc aat gat gct gct gcc gat tac ttc cgc     1056
Ala Leu Ala Lys Ala Thr Leu Asn Asp Ala Ala Ala Asp Tyr Phe Arg
            340                 345                 350

gac att ctt gcc ggc aac gtc gaa ggc ctg acc gtg gat cct gac ctg     1104
Asp Ile Leu Ala Gly Asn Val Glu Gly Leu Thr Val Asp Pro Asp Leu

355                 360                 365

cgt tgg tgg gca ctg act gcg ctt atc gcc cgt ggt gac atc gag gct      1152
Arg Trp Trp Ala Leu Thr Ala Leu Ile Ala Arg Gly Asp Ile Glu Ala
    370                 375                 380

gtc gaa gat gca atc gcc gct gaa ctt tcc cgc gac aac tcc agt gcc      1200
Val Glu Asp Ala Ile Ala Ala Glu Leu Ser Arg Asp Asn Ser Ser Ala
385                 390                 395                 400

tcc ttc ctc gca tca ctt cga gcc ggt gcc gct gtg aac act gaa gaa      1248
Ser Phe Leu Ala Ser Leu Arg Ala Gly Ala Ala Val Asn Thr Glu Glu
                405                 410                 415

gtg aag gct gct gca tac aag cat gtc ccg gca gtt gat agt ggc cta      1296
Val Lys Ala Ala Ala Tyr Lys His Val Pro Ala Val Asp Ser Gly Leu
            420                 425                 430

tcc aac ctg gag ctg cgc cac aag att gaa ggc ctc aca ttc act ggc      1344
Ser Asn Leu Glu Leu Arg His Lys Ile Glu Gly Leu Thr Phe Thr Gly
        435                 440                 445

tct ttt gaa ctg ctg caa gcc tac aac gag cag tac ttc gaa atc ctt      1392
Ser Phe Glu Leu Leu Gln Ala Tyr Asn Glu Gln Tyr Phe Glu Ile Leu
    450                 455                 460

gat gat gtg tgg gcg aac ttc tcc ggc gaa atg gca cag cag atc gtc      1440
Asp Asp Val Trp Ala Asn Phe Ser Gly Glu Met Ala Gln Gln Ile Val
465                 470                 475                 480

ctc gga ctg ttc cct tca tgg aac gtt tcc gaa gag ggt ctc aag cgt      1488
Leu Gly Leu Phe Pro Ser Trp Asn Val Ser Glu Glu Gly Leu Lys Arg
                485                 490                 495

acc gac gag ttt ctt gat ggc gaa cat gtc gca ggc atc aag cga att      1536
Thr Asp Glu Phe Leu Asp Gly Glu His Val Ala Gly Ile Lys Arg Ile
            500                 505                 510

gtt tcc gaa tcc ctc gac cgc act gcc cgt gct ctg cgc aac              1578
Val Ser Glu Ser Leu Asp Arg Thr Ala Arg Ala Leu Arg Asn
        515                 520                 525


<210> 106
<211> 526
<212> PRT
<213> Corynebacterium glutamicum

<400> 106
Asp Asp Leu Trp Leu Asn Glu Ser Phe Ala Thr Trp Ser Ala Ala Ile
1               5                   10                  15

Ser Gln Ala Glu Glu Thr Glu Tyr Asn Thr Ala Trp Val Thr Phe Ala
            20                  25                  30

Asn Val Glu Lys Ser Trp Ala Tyr Gln Gln Asp Gln Leu Pro Ser Thr
        35                  40                  45

His Pro Val Phe Ser Asp Gly Tyr Asp Ile Glu Thr Val Asp Gln Asn
    50                  55                  60

Phe Asp Gly Ile Thr Tyr Ala Lys Gly Ala Ser Val Leu Lys Gln Leu
65                  70                  75                  80

Gln Ala Tyr Val Gly Arg Glu Glu Phe Leu Ala Gly Val Arg Arg His
                85                  90                  95

Phe Ala Asn His Ala Trp Gly Asn Ala Ser Phe Asp Asp Leu Leu Gly
            100                 105                 110

Ala Leu Glu Gln Ser Ser Gly Arg Asp Leu Ser Asp Trp Ala Asn Gln
        115                 120                 125

Trp Leu Lys Thr Thr Gly Ile Asn Thr Leu Gly Ala Lys Phe Thr Thr
    130                 135                 140

Asp Asn Gly Lys Tyr Thr Ser Phe Ser Val Thr Gln Thr Gly Ala Ala

145                 150                 155                 160

Pro Gly Ala Gly Glu Leu Arg Thr His Arg Ile Ala Val Gly Leu Tyr
                165                 170                 175

Lys Leu Val Asp Gly Ser Leu Asn Arg Tyr Ala Arg Val Glu Leu Asp
            180                 185                 190

Cys Ser Gly Ala Ser Thr Ser Val Glu Glu Ile Val Gly Leu Glu Gln
        195                 200                 205

Ala Asp Phe Val Leu Val Asn Asp Asp Asp Leu Thr Tyr Ala Leu Leu
    210                 215                 220

Asp Leu Asp Asp Asp Ser Arg Asn Phe Val Ile Asp Asn Ile Asp Lys
225                 230                 235                 240

Phe Ser Asp Pro Met Pro Arg Thr Leu Val Trp Ser Ala Ala Trp Glu
                245                 250                 255

Met Thr Arg Ala Gly Gln Met Lys Ala Arg Asp Phe Ile Ala Leu Val
            260                 265                 270

Ala Arg Gly Ala Ala Ala Glu Thr Glu Ile Ala Val Leu Glu Arg Ile
        275                 280                 285

Leu Ala Gln Ala Thr Ser Ala Leu Lys Ser Tyr Ala Asp Pro Ala Trp
    290                 295                 300

Ala Glu Ala Thr Gly Asn Asp Leu Leu Ala Asp Ala Phe Leu Glu Gly
305                 310                 315                 320

Ala Arg Ser Ala Glu Pro Asp Ser Asp Thr Gln Leu Ala Phe Ile Gln
                325                 330                 335

Ala Leu Ala Lys Ala Thr Leu Asn Asp Ala Ala Ala Asp Tyr Phe Arg
            340                 345                 350

Asp Ile Leu Ala Gly Asn Val Glu Gly Leu Thr Val Asp Pro Asp Leu
        355                 360                 365

Arg Trp Trp Ala Leu Thr Ala Leu Ile Ala Arg Gly Asp Ile Glu Ala
    370                 375                 380

Val Glu Asp Ala Ile Ala Ala Glu Leu Ser Arg Asp Asn Ser Ser Ala
385                 390                 395                 400

Ser Phe Leu Ala Ser Leu Arg Ala Gly Ala Ala Val Asn Thr Glu Glu
                405                 410                 415

Val Lys Ala Ala Ala Tyr Lys His Val Pro Ala Val Asp Ser Gly Leu
            420                 425                 430

Ser Asn Leu Glu Leu Arg His Lys Ile Glu Gly Leu Thr Phe Thr Gly
        435                 440                 445

Ser Phe Glu Leu Leu Gln Ala Tyr Asn Glu Gln Tyr Phe Glu Ile Leu
    450                 455                 460

Asp Asp Val Trp Ala Asn Phe Ser Gly Glu Met Ala Gln Gln Ile Val
465                 470                 475                 480

Leu Gly Leu Phe Pro Ser Trp Asn Val Ser Glu Glu Gly Leu Lys Arg
                485                 490                 495

Thr Asp Glu Phe Leu Asp Gly Glu His Val Ala Gly Ile Lys Arg Ile
            500                 505                 510

Val Ser Glu Ser Leu Asp Arg Thr Ala Arg Ala Leu Arg Asn
        515                 520                 525

<210> 107
<211> 964
<212> DNA
<213> Corynebacterium glutamicum

<220>
<221> CDS
<222> (101)..(964)
<223> FRXA01018

<400> 107
tcttaaagtt ttctagcaat ccacactagg cgcgaactat cgtggtgtca ttgcgcacct 60

tctaagggta gcgccccctc aaatttcaag gagcattaaa ttg acg tcc act aat 115
Leu Thr Ser Thr Asn
1               5

ctc acc cga cag gaa gct tcg gat cgt tcg agg tta ctg agt gta gaa 163
Leu Thr Arg Gln Glu Ala Ser Asp Arg Ser Arg Leu Leu Ser Val Glu
                10                  15                  20

aac tat gac att gca ctt gat ctc aac aac ggt gat gag ttt ttt agt 211
Asn Tyr Asp Ile Ala Leu Asp Leu Asn Asn Gly Asp Glu Phe Phe Ser
            25                  30                  35

tcc tcc acc gtt gtc agc ttc act gtc agg aag gct ggc gat acc ttt 259
Ser Ser Thr Val Val Ser Phe Thr Val Arg Lys Ala Gly Asp Thr Phe
        40                  45                  50

att gat ctc cgc gca gca agc gtt gag gag gtt cgc ctg gac aat gtg 307
Ile Asp Leu Arg Ala Ala Ser Val Glu Glu Val Arg Leu Asp Asn Val
    55                  60                  65

tcc atc aaa gat gag gct cta acc ctt ggc aag aac ggc tac gac gag 355
Ser Ile Lys Asp Glu Ala Leu Thr Leu Gly Lys Asn Gly Tyr Asp Glu
70                  75                  80                  85

acg ttc ggc atc gcc ctg aag ggt ctt act ccc ggc gcg cac acc ttg 403
Thr Phe Gly Ile Ala Leu Lys Gly Leu Thr Pro Gly Ala His Thr Leu
                90                  95                  100

cgg gta acg gcg tct atc ccc tat tcc cgc acc ggt gaa ggc ctg cac 451
Arg Val Thr Ala Ser Ile Pro Tyr Ser Arg Thr Gly Glu Gly Leu His
            105                 110                 115

cgc atg gtg gat cca gca gac aat gag gtg tat ttg tac acc cag ttt 499
Arg Met Val Asp Pro Ala Asp Asn Glu Val Tyr Leu Tyr Thr Gln Phe
        120                 125                 130

gag acc gcc gat gcc aag cgt atg ttc gcg tgt ttc gat cag cca gac 547
Glu Thr Ala Asp Ala Lys Arg Met Phe Ala Cys Phe Asp Gln Pro Asp
    135                 140                 145

ctc aag gct acc tat gat ctg aac atc aaa act cct aag ggt tgg aag 595
Leu Lys Ala Thr Tyr Asp Leu Asn Ile Lys Thr Pro Lys Gly Trp Lys
150                 155                 160                 165

atc att tcc aac tct gag cag cag gtt tcc act cag cac act gat tac 643
Ile Ile Ser Asn Ser Glu Gln Gln Val Ser Thr Gln His Thr Asp Tyr
                170                 175                 180

gat acc cac att tcc cga gtg gac tat ccc ctc tcc acc tac ctg att 691
Asp Thr His Ile Ser Arg Val Asp Tyr Pro Leu Ser Thr Tyr Leu Ile
            185                 190                 195

gcg gtg tgc gcg ggt cgt tac cac gag gtg tgc gat gtc tgg aag ggt 739
Ala Val Cys Ala Gly Arg Tyr His Glu Val Cys Asp Val Trp Lys Gly
        200                 205                 210

acg ctc acc cac cat gca gaa aca cct gcc gat cag cca act gag ctg 787
Thr Leu Thr His His Ala Glu Thr Pro Ala Asp Gln Pro Thr Glu Leu
    215                 220                 225

act gtt ccg ctt gct ctc tac tgc cgc agt tct ttg gct aaa gat ctt 835
Thr Val Pro Leu Ala Leu Tyr Cys Arg Ser Ser Leu Ala Lys Asp Leu
230                 235                 240                 245

gat gcg gtg cgt ctg ttt acc gaa acg aag cag ggc ttt gat tgg tac 883
Asp Ala Val Arg Leu Phe Thr Glu Thr Lys Gln Gly Phe Asp Trp Tyr
                250                 255                 260

cac cgc aac ttc ggt gtg gcg tac cca ttc ggc aag tac gat cag atc 931

His Arg Asn Phe Gly Val Ala Tyr Pro Phe Gly Lys Tyr Asp Gln Ile
            265                 270                 275

ttc gtc cct gaa ttt aat gct ggc gcg atg gag                         964
Phe Val Pro Glu Phe Asn Ala Gly Ala Met Glu
        280                 285


<210> 108
<211> 288
<212> PRT
<213> Corynebacterium glutamicum

<400> 108
Leu Thr Ser Thr Asn Leu Thr Arg Gln Glu Ala Ser Asp Arg Ser Arg
1               5                   10                  15

Leu Leu Ser Val Glu Asn Tyr Asp Ile Ala Leu Asp Leu Asn Asn Gly
            20                  25                  30

Asp Glu Phe Phe Ser Ser Ser Thr Val Val Ser Phe Thr Val Arg Lys
        35                  40                  45

Ala Gly Asp Thr Phe Ile Asp Leu Arg Ala Ala Ser Val Glu Glu Val
    50                  55                  60

Arg Leu Asp Asn Val Ser Ile Lys Asp Glu Ala Leu Thr Leu Gly Lys
65                  70                  75                  80

Asn Gly Tyr Asp Glu Thr Phe Gly Ile Ala Leu Lys Gly Leu Thr Pro
                85                  90                  95

Gly Ala His Thr Leu Arg Val Thr Ala Ser Ile Pro Tyr Ser Arg Thr
            100                 105                 110

Gly Glu Gly Leu His Arg Met Val Asp Pro Ala Asp Asn Glu Val Tyr
        115                 120                 125

Leu Tyr Thr Gln Phe Glu Thr Ala Asp Ala Lys Arg Met Phe Ala Cys
    130                 135                 140

Phe Asp Gln Pro Asp Leu Lys Ala Thr Tyr Asp Leu Asn Ile Lys Thr
145                 150                 155                 160

Pro Lys Gly Trp Lys Ile Ile Ser Asn Ser Glu Gln Gln Val Ser Thr
                165                 170                 175

Gln His Thr Asp Tyr Asp Thr His Ile Ser Arg Val Asp Tyr Pro Leu
            180                 185                 190

Ser Thr Tyr Leu Ile Ala Val Cys Ala Gly Arg Tyr His Glu Val Cys
        195                 200                 205

Asp Val Trp Lys Gly Thr Leu Thr His His Ala Glu Thr Pro Ala Asp
    210                 215                 220

Gln Pro Thr Glu Leu Thr Val Pro Leu Ala Leu Tyr Cys Arg Ser Ser
225                 230                 235                 240

Leu Ala Lys Asp Leu Asp Ala Val Arg Leu Phe Thr Glu Thr Lys Gln
                245                 250                 255

Gly Phe Asp Trp Tyr His Arg Asn Phe Gly Val Ala Tyr Pro Phe Gly
            260                 265                 270

Lys Tyr Asp Gln Ile Phe Val Pro Glu Phe Asn Ala Gly Ala Met Glu
        275                 280                 285


<210> 109
<211> 1383
<212> DNA
<213> Corynebacterium glutamicum

<220>
<221> CDS
<222> (101)..(1360)
<223> RXA01147

<400> 109
ataatgatca cctactttaa cggcttcagg tgacattgtg gattcgcatt gtggattcgg 60

gggcccgcgc tgtttccaag aatttggcta cccttgttct atg cat gta act gac 115
Met His Val Thr Asp
1               5

gat ttc tta agt ttt att gcc cta agc cca agt tcc tat cac gcg gcc 163
Asp Phe Leu Ser Phe Ile Ala Leu Ser Pro Ser Ser Tyr His Ala Ala
                10                  15                  20

gcg gcg gtg gag cgc agg ttg ctc cat gag ggg ttc att cgt cag gaa 211
Ala Ala Val Glu Arg Arg Leu Leu His Glu Gly Phe Ile Arg Gln Glu
            25                  30                  35

gat acc gat gaa tgg gat gcc cgc cct ggt ggg cat gtg acg gtg cgt 259
Asp Thr Asp Glu Trp Asp Ala Arg Pro Gly Gly His Val Thr Val Arg
        40                  45                  50

ggg gga gca gta gtg gcg tgg tgg gtg cct gag gat gct tcg cca gat 307
Gly Gly Ala Val Val Ala Trp Trp Val Pro Glu Asp Ala Ser Pro Asp
    55                  60                  65

tcc ggg ttc cgc atc att ggg tca cat act gat tca ccg ggt ttc aag 355
Ser Gly Phe Arg Ile Ile Gly Ser His Thr Asp Ser Pro Gly Phe Lys
70                  75                  80                  85

tta aag ccc cgt ggg gat ctt tcc tca cac ggt tgg cag cag gcc ggc 403
Leu Lys Pro Arg Gly Asp Leu Ser Ser His Gly Trp Gln Gln Ala Gly
                90                  95                  100

gtc gag gtt tac ggc gga ccg atc ctg cca agc tgg ctg gat cgc gag 451
Val Glu Val Tyr Gly Gly Pro Ile Leu Pro Ser Trp Leu Asp Arg Glu
            105                 110                 115

ctg gcc tta gca ggc cgc att gtg ctt gcc gac ggg tcc gtc aag ctt 499
Leu Ala Leu Ala Gly Arg Ile Val Leu Ala Asp Gly Ser Val Lys Leu
        120                 125                 130

gtc aac acc ggc ccg att ctg cgc atc ccg cac gtg gct att cat ttg 547
Val Asn Thr Gly Pro Ile Leu Arg Ile Pro His Val Ala Ile His Leu
    135                 140                 145

gac cgt act gtt aat tcc caa ctc acc ctt aat cca cag cgt cac ctg 595
Asp Arg Thr Val Asn Ser Gln Leu Thr Leu Asn Pro Gln Arg His Leu
150                 155                 160                 165

cag cct gtg ttt gct gtt ggt gag ccc gac gta tca att ctg gat gtc 643
Gln Pro Val Phe Ala Val Gly Glu Pro Asp Val Ser Ile Leu Asp Val
                170                 175                 180

att gct ggt gct gcg gta gtg gat cct gca gat att gtc agc cat gat 691
Ile Ala Gly Ala Ala Val Val Asp Pro Ala Asp Ile Val Ser His Asp
            185                 190                 195

ctg atc acg gtg gct acc caa gat gct gaa gta ttt ggc gca cat ggg 739
Leu Ile Thr Val Ala Thr Gln Asp Ala Glu Val Phe Gly Ala His Gly
        200                 205                 210

gat ttc ttg gcg tct ggt cgc ctg gat aac ctg agc agc gtg cat cca 787
Asp Phe Leu Ala Ser Gly Arg Leu Asp Asn Leu Ser Ser Val His Pro
    215                 220                 225

tcc atg act gca ttg att gcg gct tcg caa tct gac gat act ggt tcg 835
Ser Met Thr Ala Leu Ile Ala Ala Ser Gln Ser Asp Asp Thr Gly Ser
230                 235                 240                 245

gat att ttg gtt ctt gct gca ttc gat cat gaa gaa gtg gga agt aat 883
Asp Ile Leu Val Leu Ala Ala Phe Asp His Glu Glu Val Gly Ser Asn
                250                 255                 260

tcc acc tcg ggt gcc ggg ggc ccc ctg ttg gag gat gtg ctc aac cgt 931

Ser Thr Ser Gly Ala Gly Gly Pro Leu Leu Glu Asp Val Leu Asn Arg
            265                 270                 275

act gct cgt gcg ttg ggt gca gat gaa gat gag cga cgc cgg atg ttt      979
Thr Ala Arg Ala Leu Gly Ala Asp Glu Asp Glu Arg Arg Arg Met Phe
        280                 285                 290

aac cgt tcc acc atg gtc tca gct gac gcg gca cac tcc att cac ccc      1027
Asn Arg Ser Thr Met Val Ser Ala Asp Ala Ala His Ser Ile His Pro
    295                 300                 305

aac ttc ccc gag aag cat gat caa gct aat tac ccc atc att ggt aaa      1075
Asn Phe Pro Glu Lys His Asp Gln Ala Asn Tyr Pro Ile Ile Gly Lys
310                 315                 320                 325

ggt cct gta ttg aag gtc aac gcc aac cag cgc tac acc tcc gat gca      1123
Gly Pro Val Leu Lys Val Asn Ala Asn Gln Arg Tyr Thr Ser Asp Ala
                330                 335                 340

gtc act tca ggc atg tgg atc agg gca tgt cag att gcc ggt gtg cca      1171
Val Thr Ser Gly Met Trp Ile Arg Ala Cys Gln Ile Ala Gly Val Pro
            345                 350                 355

cac cag gtg ttt gcc ggc aac aac gat gtg ccg tgt ggt tcc acc atc      1219
His Gln Val Phe Ala Gly Asn Asn Asp Val Pro Cys Gly Ser Thr Ile
        360                 365                 370

ggc ccg atc agt gcg act cgc ctg ggt atc gat tct gtc gat gtc ggt      1267
Gly Pro Ile Ser Ala Thr Arg Leu Gly Ile Asp Ser Val Asp Val Gly
    375                 380                 385

att cca ttg ctg tcc atg cac tcc gca cgc gaa atg gcc gga gtg aag      1315
Ile Pro Leu Leu Ser Met His Ser Ala Arg Glu Met Ala Gly Val Lys
390                 395                 400                 405

gat ctg atg tgg ttt gaa caa gcc ctg gaa gcc tat ctg gta aat          1360
Asp Leu Met Trp Phe Glu Gln Ala Leu Glu Ala Tyr Leu Val Asn
                410                 415                 420

taacgccgag ttcaatcaag aca                                            1383


<210> 110
<211> 420
<212> PRT
<213> Corynebacterium glutamicum

<400> 110
Met His Val Thr Asp Asp Phe Leu Ser Phe Ile Ala Leu Ser Pro Ser
1               5                   10                  15

Ser Tyr His Ala Ala Ala Ala Val Glu Arg Arg Leu Leu His Glu Gly
            20                  25                  30

Phe Ile Arg Gln Glu Asp Thr Asp Glu Trp Asp Ala Arg Pro Gly Gly
        35                  40                  45

His Val Thr Val Arg Gly Gly Ala Val Val Ala Trp Trp Val Pro Glu
    50                  55                  60

Asp Ala Ser Pro Asp Ser Gly Phe Arg Ile Ile Gly Ser His Thr Asp
65                  70                  75                  80

Ser Pro Gly Phe Lys Leu Lys Pro Arg Gly Asp Leu Ser Ser His Gly
                85                  90                  95

Trp Gln Gln Ala Gly Val Glu Val Tyr Gly Gly Pro Ile Leu Pro Ser
            100                 105                 110

Trp Leu Asp Arg Glu Leu Ala Leu Ala Gly Arg Ile Val Leu Ala Asp
        115                 120                 125

Gly Ser Val Lys Leu Val Asn Thr Gly Pro Ile Leu Arg Ile Pro His
    130                 135                 140

Val Ala Ile His Leu Asp Arg Thr Val Asn Ser Gln Leu Thr Leu Asn
145                 150                 155                 160

Pro Gln Arg His Leu Gln Pro Val Phe Ala Val Gly Glu Pro Asp Val
                165                 170                 175

Ser Ile Leu Asp Val Ile Ala Gly Ala Ala Val Val Asp Pro Ala Asp
            180                 185                 190

Ile Val Ser His Asp Leu Ile Thr Val Ala Thr Gln Asp Ala Glu Val
        195                 200                 205

Phe Gly Ala His Gly Asp Phe Leu Ala Ser Gly Arg Leu Asp Asn Leu
    210                 215                 220

Ser Ser Val His Pro Ser Met Thr Ala Leu Ile Ala Ala Ser Gln Ser
225                 230                 235                 240

Asp Asp Thr Gly Ser Asp Ile Leu Val Leu Ala Ala Phe Asp His Glu
                245                 250                 255

Glu Val Gly Ser Asn Ser Thr Ser Gly Ala Gly Gly Pro Leu Leu Glu
            260                 265                 270

Asp Val Leu Asn Arg Thr Ala Arg Ala Leu Gly Ala Asp Glu Asp Glu
        275                 280                 285

Arg Arg Arg Met Phe Asn Arg Ser Thr Met Val Ser Ala Asp Ala Ala
    290                 295                 300

His Ser Ile His Pro Asn Phe Pro Glu Lys His Asp Gln Ala Asn Tyr
305                 310                 315                 320

Pro Ile Ile Gly Lys Gly Pro Val Leu Lys Val Asn Ala Asn Gln Arg
                325                 330                 335

Tyr Thr Ser Asp Ala Val Thr Ser Gly Met Trp Ile Arg Ala Cys Gln
            340                 345                 350

Ile Ala Gly Val Pro His Gln Val Phe Ala Gly Asn Asn Asp Val Pro
        355                 360                 365

Cys Gly Ser Thr Ile Gly Pro Ile Ser Ala Thr Arg Leu Gly Ile Asp
    370                 375                 380

Ser Val Asp Val Gly Ile Pro Leu Leu Ser Met His Ser Ala Arg Glu
385                 390                 395                 400

Met Ala Gly Val Lys Asp Leu Met Trp Phe Glu Gln Ala Leu Glu Ala
                405                 410                 415

Tyr Leu Val Asn
            420


<210> 111
<211> 1260
<212> DNA
<213> Corynebacterium glutamicum

<220>
<221> CDS
<222> (101)..(1237)
<223> RXA01161

<400> 111
tttttcgtga tcaacaatcc gctggcatag cgtccagcag atttgattct gacagtgtgg 60

tttgatcgca cacctgccta ggctactagg gttggagact atg agt gat cct tca    115
                                           Met Ser Asp Pro Ser
                                           1               5

aca aac aat ttc ccc aca tcg gta tat gca cag cgt ctt gcg gat gca    163
Thr Asn Asn Phe Pro Thr Ser Val Tyr Ala Gln Arg Leu Ala Asp Ala
                10                  15                  20

caa gaa ggc gca cgc aag gct ggc ttg aac ggt ttg atc atc ggt aca    211
Gln Glu Gly Ala Arg Lys Ala Gly Leu Asn Gly Leu Ile Ile Gly Thr
            25                  30                  35

ggc gca gaa ctt gcg tat cta acc ggc agc tgg atc tcc acc cat gag     259
Gly Ala Glu Leu Ala Tyr Leu Thr Gly Ser Trp Ile Ser Thr His Glu
        40                  45                  50

cgt cta acc gct ttg gtg atc ccc agc gaa gga acc gca acc att gtt     307
Arg Leu Thr Ala Leu Val Ile Pro Ser Glu Gly Thr Ala Thr Ile Val
    55                  60                  65

ctt ccc gct gta gac cgc gga gac tta gca ctg tct gct att cca gga     355
Leu Pro Ala Val Asp Arg Gly Asp Leu Ala Leu Ser Ala Ile Pro Gly
70                  75                  80                  85

cta gac atc aat gtg gcc gga tgg gtt gat ggc gat aat gcc cat gag     403
Leu Asp Ile Asn Val Ala Gly Trp Val Asp Gly Asp Asn Ala His Glu
                90                  95                  100

ttg gcc gta gat gct ctc ggt gtt tca gag ttc gaa gca ttg ggt att     451
Leu Ala Val Asp Ala Leu Gly Val Ser Glu Phe Glu Ala Leu Gly Ile
            105                 110                 115

ggt tcc tcc atc acg gca gat cac ctg att cct atc cag aac ctg gtg     499
Gly Ser Ser Ile Thr Ala Asp His Leu Ile Pro Ile Gln Asn Leu Val
        120                 125                 130

ggc tcc acc tgc cgc atg gag ttg gca gtt caa gtg ctg aaa gaa ttg     547
Gly Ser Thr Cys Arg Met Glu Leu Ala Val Gln Val Leu Lys Glu Leu
    135                 140                 145

ttt gtc tct aaa gac gaa gca gag atc gag cag ctt cgc ggc gca ggt     595
Phe Val Ser Lys Asp Glu Ala Glu Ile Glu Gln Leu Arg Gly Ala Gly
150                 155                 160                 165

gca gcc att gac cgt gtc cac gcc aaa gtc ccg gag ctt ctt caa gac     643
Ala Ala Ile Asp Arg Val His Ala Lys Val Pro Glu Leu Leu Gln Asp
                170                 175                 180

gga cgc acc gaa gca gag gtt gca gca cag ctc aac gat ctc atc ttg     691
Gly Arg Thr Glu Ala Glu Val Ala Ala Gln Leu Asn Asp Leu Ile Leu
            185                 190                 195

gaa gag cac tct gag gtg gac ttc gtg att gtg gga tcc gct gaa aac     739
Glu Glu His Ser Glu Val Asp Phe Val Ile Val Gly Ser Ala Glu Asn
        200                 205                 210

ggc gcg aac cct cac cac ggt ttc tct gac cga gtc ctc cgc aat ggc     787
Gly Ala Asn Pro His His Gly Phe Ser Asp Arg Val Leu Arg Asn Gly
    215                 220                 225

gac atc gtg gtg gtt gat ata gga ggc acc ttc ggc cct ggt tac cac     835
Asp Ile Val Val Val Asp Ile Gly Gly Thr Phe Gly Pro Gly Tyr His
230                 235                 240                 245

tct gac tgc aca cgc acc tac att gtg ggc gga aac cct gac gat gcg     883
Ser Asp Cys Thr Arg Thr Tyr Ile Val Gly Gly Asn Pro Asp Asp Ala
                250                 255                 260

gat cca gag ttc gct aag ttc tac caa gtg ctc tac gaa gca cag ctc     931
Asp Pro Glu Phe Ala Lys Phe Tyr Gln Val Leu Tyr Glu Ala Gln Leu
            265                 270                 275

gca gcc gtt gcg cat gtt cgc cct ggc gtt act gca gaa tca gtg gac     979
Ala Ala Val Ala His Val Arg Pro Gly Val Thr Ala Glu Ser Val Asp
        280                 285                 290

gct gtt gct cgc gat cac att gct gcg gct gga tac ggc gaa tac ttc     1027
Ala Val Ala Arg Asp His Ile Ala Ala Ala Gly Tyr Gly Glu Tyr Phe
    295                 300                 305

att cac cgc aca gga cac ggc att ggt cta tcc acc cat gag gag cca     1075
Ile His Arg Thr Gly His Gly Ile Gly Leu Ser Thr His Glu Glu Pro
310                 315                 320                 325

ttc atc atg gcg ggt aac tca ctc gtg ttg gaa gcc gga atg gcc ttt     1123
Phe Ile Met Ala Gly Asn Ser Leu Val Leu Glu Ala Gly Met Ala Phe
                330                 335                 340

tcc att gag cct ggc atc tac att gaa gga atc cac gga gcg cgc atc     1171
Ser Ile Glu Pro Gly Ile Tyr Ile Glu Gly Ile His Gly Ala Arg Ile
            345                 350                 355

gaa gac atc gtt gtg gtg aat gaa gac ggt tgt gaa acc ctc aac aac     1219
Glu Asp Ile Val Val Val Asn Glu Asp Gly Cys Glu Thr Leu Asn Asn
        360                 365                 370

cag ccc aag gaa ctg cgt tgagcattct tctcctaggc gga                   1260
Gln Pro Lys Glu Leu Arg
    375


<210> 112
<211> 379
<212> PRT
<213> Corynebacterium glutamicum

<400> 112
Met Ser Asp Pro Ser Thr Asn Asn Phe Pro Thr Ser Val Tyr Ala Gln
  1               5                  10                  15

Arg Leu Ala Asp Ala Gln Glu Gly Ala Arg Lys Ala Gly Leu Asn Gly
             20                  25                  30

Leu Ile Ile Gly Thr Gly Ala Glu Leu Ala Tyr Leu Thr Gly Ser Trp
         35                  40                  45

Ile Ser Thr His Glu Arg Leu Thr Ala Leu Val Ile Pro Ser Glu Gly
     50                  55                  60

Thr Ala Thr Ile Val Leu Pro Ala Val Asp Arg Gly Asp Leu Ala Leu
 65                  70                  75                  80

Ser Ala Ile Pro Gly Leu Asp Ile Asn Val Ala Gly Trp Val Asp Gly
                 85                  90                  95

Asp Asn Ala His Glu Leu Ala Val Asp Ala Leu Gly Val Ser Glu Phe
            100                 105                 110

Glu Ala Leu Gly Ile Gly Ser Ser Ile Thr Ala Asp His Leu Ile Pro
        115                 120                 125

Ile Gln Asn Leu Val Gly Ser Thr Cys Arg Met Glu Leu Ala Val Gln
    130                 135                 140

Val Leu Lys Glu Leu Phe Val Ser Lys Asp Glu Ala Glu Ile Glu Gln
145                 150                 155                 160

Leu Arg Gly Ala Gly Ala Ala Ile Asp Arg Val His Ala Lys Val Pro
                165                 170                 175

Glu Leu Leu Gln Asp Gly Arg Thr Glu Ala Glu Val Ala Ala Gln Leu
            180                 185                 190

Asn Asp Leu Ile Leu Glu Glu His Ser Glu Val Asp Phe Val Ile Val
        195                 200                 205

Gly Ser Ala Glu Asn Gly Ala Asn Pro His His Gly Phe Ser Asp Arg
    210                 215                 220

Val Leu Arg Asn Gly Asp Ile Val Val Val Asp Ile Gly Gly Thr Phe
225                 230                 235                 240

Gly Pro Gly Tyr His Ser Asp Cys Thr Arg Thr Tyr Ile Val Gly Gly
                245                 250                 255

Asn Pro Asp Asp Ala Asp Pro Glu Phe Ala Lys Phe Tyr Gln Val Leu
            260                 265                 270

Tyr Glu Ala Gln Leu Ala Ala Val Ala His Val Arg Pro Gly Val Thr
        275                 280                 285

Ala Glu Ser Val Asp Ala Val Ala Arg Asp His Ile Ala Ala Ala Gly
    290                 295                 300

Tyr Gly Glu Tyr Phe Ile His Arg Thr Gly His Gly Ile Gly Leu Ser

305                 310                 315                 320

Thr His Glu Glu Pro Phe Ile Met Ala Gly Asn Ser Leu Val Leu Glu
                325                 330                 335

Ala Gly Met Ala Phe Ser Ile Glu Pro Gly Ile Tyr Ile Glu Gly Ile
            340                 345                 350

His Gly Ala Arg Ile Glu Asp Ile Val Val Val Asn Glu Asp Gly Cys
        355                 360                 365

Glu Thr Leu Asn Asn Gln Pro Lys Glu Leu Arg
    370                 375


<210> 113
<211> 980
<212> DNA
<213> Corynebacterium glutamicum

<220>
<221> CDS
<222> (1)..(957)
<223> RXN01181

<400> 113
tct gta ctg ctc gct cgc gac ttg gtg aac acc cct tca tca cac ctg      48
Ser Val Leu Leu Ala Arg Asp Leu Val Asn Thr Pro Ser Ser His Leu
  1               5                  10                  15

tac cca gag tcc tac tca gta att gca tcc aac gaa gcg tcc aag cac      96
Tyr Pro Glu Ser Tyr Ser Val Ile Ala Ser Asn Glu Ala Ser Lys His
             20                  25                  30

ggc ttg cag acc acc atc ctg gat gag aag cag ctt gct gat caa ggt     144
Gly Leu Gln Thr Thr Ile Leu Asp Glu Lys Gln Leu Ala Asp Gln Gly
         35                  40                  45

ttc ggc ggc atc ctc gca gtc ggt aac ggc tcc tcc cgc aag cct cgt     192
Phe Gly Gly Ile Leu Ala Val Gly Asn Gly Ser Ser Arg Lys Pro Arg
     50                  55                  60

ctg ctg cgc atc gat tgg aag cca cgc aag gct aag aag tcg atc gct     240
Leu Leu Arg Ile Asp Trp Lys Pro Arg Lys Ala Lys Lys Ser Ile Ala
 65                  70                  75                  80

ttg gtt ggc aag ggc atc acc ttt gac acc ggc gga att tcc atc aag     288
Leu Val Gly Lys Gly Ile Thr Phe Asp Thr Gly Gly Ile Ser Ile Lys
                 85                  90                  95

cct ggc gca agc atg gag aac atg atc tcc gac atg ggt gga tcc gca     336
Pro Gly Ala Ser Met Glu Asn Met Ile Ser Asp Met Gly Gly Ser Ala
            100                 105                 110

tcc gta ttg gcc acc att atc gct gca gct cgt ttg aac ctg tcg atc     384
Ser Val Leu Ala Thr Ile Ile Ala Ala Ala Arg Leu Asn Leu Ser Ile
        115                 120                 125

aac gtc ttc gcg ttc cta cca atg gct gag aac atg cca tcc ggt gac     432
Asn Val Phe Ala Phe Leu Pro Met Ala Glu Asn Met Pro Ser Gly Asp
    130                 135                 140

gct ttc cgc ccc ggc gat gtc atc act cat ttc ggt ggt atc acc tcc     480
Ala Phe Arg Pro Gly Asp Val Ile Thr His Phe Gly Gly Ile Thr Ser
145                 150                 155                 160

gaa atc ttg aac acc gac gct gaa ggc cgc ctc att ctg gca gat gcc     528
Glu Ile Leu Asn Thr Asp Ala Glu Gly Arg Leu Ile Leu Ala Asp Ala
                165                 170                 175

att gct tac gct tct gaa gat aag cct gac tac ctc att gat gcg gca     576
Ile Ala Tyr Ala Ser Glu Asp Lys Pro Asp Tyr Leu Ile Asp Ala Ala
            180                 185                 190

acc ctg act ggt gct caa tta gtc gct tta ggc ctg cgg act tca ggt     624
Thr Leu Thr Gly Ala Gln Leu Val Ala Leu Gly Leu Arg Thr Ser Gly
        195                 200                 205

gtc atg ggt acc gat gag ttc cgc gac agc gtt gcc aag act ggc cgc     672
Val Met Gly Thr Asp Glu Phe Arg Asp Ser Val Ala Lys Thr Gly Arg
    210                 215                 220

gag gtt ggc gag caa gca tgg gca atg cct ctt cct gaa gag ctc gat     720
Glu Val Gly Glu Gln Ala Trp Ala Met Pro Leu Pro Glu Glu Leu Asp
225                 230                 235                 240

gag cag gtt aag tcc cct gtc gct gac ctg cgc aat gtc acc aat tcc     768
Glu Gln Val Lys Ser Pro Val Ala Asp Leu Arg Asn Val Thr Asn Ser
                245                 250                 255

cgt ttc gca gga atg tct gct gcg ggt cgt tac ttg cag gaa ttc gtt     816
Arg Phe Ala Gly Met Ser Ala Ala Gly Arg Tyr Leu Gln Glu Phe Val
            260                 265                 270

ggt gcc gac atc gag tgg gct cac gtc gat atc gct ggc cct gca tac     864
Gly Ala Asp Ile Glu Trp Ala His Val Asp Ile Ala Gly Pro Ala Tyr
        275                 280                 285

aac act gct ggt gaa ttc ggt tac acg cca aag cgc gca acc gga caa     912
Asn Thr Ala Gly Glu Phe Gly Tyr Thr Pro Lys Arg Ala Thr Gly Gln
    290                 295                 300

cca gtg cgc acc ttc gtt cag gtt ctg aag gat ctg tcg gaa agc         957
Pro Val Arg Thr Phe Val Gln Val Leu Lys Asp Leu Ser Glu Ser
305                 310                 315

taaacgctag ttaaagatca gga                                           980


<210> 114
<211> 319
<212> PRT
<213> Corynebacterium glutamicum

<400> 114
Ser Val Leu Leu Ala Arg Asp Leu Val Asn Thr Pro Ser Ser His Leu
  1               5                  10                  15

Tyr Pro Glu Ser Tyr Ser Val Ile Ala Ser Asn Glu Ala Ser Lys His
             20                  25                  30

Gly Leu Gln Thr Thr Ile Leu Asp Glu Lys Gln Leu Ala Asp Gln Gly
         35                  40                  45

Phe Gly Gly Ile Leu Ala Val Gly Asn Gly Ser Ser Arg Lys Pro Arg
     50                  55                  60

Leu Leu Arg Ile Asp Trp Lys Pro Arg Lys Ala Lys Lys Ser Ile Ala
 65                  70                  75                  80

Leu Val Gly Lys Gly Ile Thr Phe Asp Thr Gly Gly Ile Ser Ile Lys
                 85                  90                  95

Pro Gly Ala Ser Met Glu Asn Met Ile Ser Asp Met Gly Gly Ser Ala
            100                 105                 110

Ser Val Leu Ala Thr Ile Ile Ala Ala Ala Arg Leu Asn Leu Ser Ile
        115                 120                 125

Asn Val Phe Ala Phe Leu Pro Met Ala Glu Asn Met Pro Ser Gly Asp
    130                 135                 140

Ala Phe Arg Pro Gly Asp Val Ile Thr His Phe Gly Gly Ile Thr Ser
145                 150                 155                 160

Glu Ile Leu Asn Thr Asp Ala Glu Gly Arg Leu Ile Leu Ala Asp Ala
                165                 170                 175

Ile Ala Tyr Ala Ser Glu Asp Lys Pro Asp Tyr Leu Ile Asp Ala Ala
            180                 185                 190

Thr Leu Thr Gly Ala Gln Leu Val Ala Leu Gly Leu Arg Thr Ser Gly
        195                 200                 205

Val Met Gly Thr Asp Glu Phe Arg Asp Ser Val Ala Lys Thr Gly Arg
    210                 215                 220

Glu Val Gly Glu Gln Ala Trp Ala Met Pro Leu Pro Glu Glu Leu Asp
225                 230                 235                 240

Glu Gln Val Lys Ser Pro Val Ala Asp Leu Arg Asn Val Thr Asn Ser
                245                 250                 255

Arg Phe Ala Gly Met Ser Ala Ala Gly Arg Tyr Leu Gln Glu Phe Val
            260                 265                 270

Gly Ala Asp Ile Glu Trp Ala His Val Asp Ile Ala Gly Pro Ala Tyr
        275                 280                 285

Asn Thr Ala Gly Glu Phe Gly Tyr Thr Pro Lys Arg Ala Thr Gly Gln
    290                 295                 300

Pro Val Arg Thr Phe Val Gln Val Leu Lys Asp Leu Ser Glu Ser
305                 310                 315


<210> 115
<211> 980
<212> DNA
<213> Corynebacterium glutamicum

<220>
<221> CDS
<222> (1)..(957)
<223> FRXA01181

<400> 115
tct gta ctg ctc gct cgc gac ttg gtg aac acc cct tca tca cac ctg      48
Ser Val Leu Leu Ala Arg Asp Leu Val Asn Thr Pro Ser Ser His Leu
  1               5                  10                  15

tac cca gag tcc tac tca gta att gca tcc aac gaa gcg tcc aag cac      96
Tyr Pro Glu Ser Tyr Ser Val Ile Ala Ser Asn Glu Ala Ser Lys His
             20                  25                  30

ggc ttg cag acc acc atc ctg gat gag aag cag ctt gct gat caa ggt     144
Gly Leu Gln Thr Thr Ile Leu Asp Glu Lys Gln Leu Ala Asp Gln Gly
         35                  40                  45

ttc ggc ggc atc ctc gca gtc ggt aac ggc tcc tcc cgc aag cct cgt     192
Phe Gly Gly Ile Leu Ala Val Gly Asn Gly Ser Ser Arg Lys Pro Arg
     50                  55                  60

ctg ctg cgc atc gat tgg aag cca cgc aag gct aag aag tcg atc gct     240
Leu Leu Arg Ile Asp Trp Lys Pro Arg Lys Ala Lys Lys Ser Ile Ala
 65                  70                  75                  80

ttg gtt ggc aag ggc atc acc ttt gac acc ggc gga att tcc atc aag     288
Leu Val Gly Lys Gly Ile Thr Phe Asp Thr Gly Gly Ile Ser Ile Lys
                 85                  90                  95

cct ggc gca agc atg gag aac atg atc tcc gac atg ggt gga tcc gca     336
Pro Gly Ala Ser Met Glu Asn Met Ile Ser Asp Met Gly Gly Ser Ala
            100                 105                 110

tcc gta ttg gcc acc att atc gct gca gct cgt ttg aac ctg tcg atc     384
Ser Val Leu Ala Thr Ile Ile Ala Ala Ala Arg Leu Asn Leu Ser Ile
        115                 120                 125

aac gtc ttc gcg ttc cta cca atg gct gag aac atg cca tcc ggt gac     432
Asn Val Phe Ala Phe Leu Pro Met Ala Glu Asn Met Pro Ser Gly Asp
    130                 135                 140

gct ttc cgc ccc ggc gat gtc atc act cat ttc ggt ggt atc acc tcc     480
Ala Phe Arg Pro Gly Asp Val Ile Thr His Phe Gly Gly Ile Thr Ser
145                 150                 155                 160

gaa atc ttg aac acc gac gct gaa ggc cgc ctc att ctg gca gat gcc     528
Glu Ile Leu Asn Thr Asp Ala Glu Gly Arg Leu Ile Leu Ala Asp Ala
                165                 170                 175

att gct tac gct tct gaa gat aag cct gac tac ctc att gat gcg gca     576
Ile Ala Tyr Ala Ser Glu Asp Lys Pro Asp Tyr Leu Ile Asp Ala Ala
            180                 185                 190

acc ctg act ggt gct caa tta gtc gct tta ggc ctg cgg act tca ggt     624
Thr Leu Thr Gly Ala Gln Leu Val Ala Leu Gly Leu Arg Thr Ser Gly
        195                 200                 205

gtc atg ggt acc gat gag ttc cgc gac agc gtt gcc aag act ggc cgc     672
Val Met Gly Thr Asp Glu Phe Arg Asp Ser Val Ala Lys Thr Gly Arg
    210                 215                 220

gag gtt ggc gag caa gca tgg gca atg cct ctt cct gaa gag ctc gat     720
Glu Val Gly Glu Gln Ala Trp Ala Met Pro Leu Pro Glu Glu Leu Asp
225                 230                 235                 240

gag cag gtt aag tcc cct gtc gct gac ctg cgc aat gtc acc aat tcc     768
Glu Gln Val Lys Ser Pro Val Ala Asp Leu Arg Asn Val Thr Asn Ser
                245                 250                 255

cgt ttc gca gga atg tct gct gcg ggt cgt tac ttg cag gaa ttc gtt     816
Arg Phe Ala Gly Met Ser Ala Ala Gly Arg Tyr Leu Gln Glu Phe Val
            260                 265                 270

ggt gcc gac atc gag tgg gct cac gtc gat atc gct ggc cct gca tac     864
Gly Ala Asp Ile Glu Trp Ala His Val Asp Ile Ala Gly Pro Ala Tyr
        275                 280                 285

aac act gct ggt gaa ttc ggt tac acg cca aag cgc gca acc gga caa     912
Asn Thr Ala Gly Glu Phe Gly Tyr Thr Pro Lys Arg Ala Thr Gly Gln
    290                 295                 300

cca gtg cgc acc ttc gtt cag gtt ctg aag gat ctg tcg gaa agc         957
Pro Val Arg Thr Phe Val Gln Val Leu Lys Asp Leu Ser Glu Ser
305                 310                 315

taaacgctag ttaaagatca gga                                           980


<210> 116
<211> 319
<212> PRT
<213> Corynebacterium glutamicum

<400> 116
Ser Val Leu Leu Ala Arg Asp Leu Val Asn Thr Pro Ser Ser His Leu
1               5                   10                  15

Tyr Pro Glu Ser Tyr Ser Val Ile Ala Ser Asn Glu Ala Ser Lys His
            20                  25                  30

Gly Leu Gln Thr Thr Ile Leu Asp Glu Lys Gln Leu Ala Asp Gln Gly
        35                  40                  45

Phe Gly Gly Ile Leu Ala Val Gly Asn Gly Ser Ser Arg Lys Pro Arg
    50                  55                  60

Leu Leu Arg Ile Asp Trp Lys Pro Arg Lys Ala Lys Lys Ser Ile Ala
65                  70                  75                  80

Leu Val Gly Lys Gly Ile Thr Phe Asp Thr Gly Gly Ile Ser Ile Lys
                85                  90                  95

Pro Gly Ala Ser Met Glu Asn Met Ile Ser Asp Met Gly Gly Ser Ala
            100                 105                 110

Ser Val Leu Ala Thr Ile Ile Ala Ala Ala Arg Leu Asn Leu Ser Ile
        115                 120                 125

Asn Val Phe Ala Phe Leu Pro Met Ala Glu Asn Met Pro Ser Gly Asp
    130                 135                 140

Ala Phe Arg Pro Gly Asp Val Ile Thr His Phe Gly Gly Ile Thr Ser
145                 150                 155                 160

Glu Ile Leu Asn Thr Asp Ala Glu Gly Arg Leu Ile Leu Ala Asp Ala
                165                 170                 175

Ile Ala Tyr Ala Ser Glu Asp Lys Pro Asp Tyr Leu Ile Asp Ala Ala
            180                 185                 190

Thr Leu Thr Gly Ala Gln Leu Val Ala Leu Gly Leu Arg Thr Ser Gly
        195                 200                 205

Val Met Gly Thr Asp Glu Phe Arg Asp Ser Val Ala Lys Thr Gly Arg
    210                 215                 220

Glu Val Gly Glu Gln Ala Trp Ala Met Pro Leu Pro Glu Glu Leu Asp
225                 230                 235                 240

Glu Gln Val Lys Ser Pro Val Ala Asp Leu Arg Asn Val Thr Asn Ser
                245                 250                 255

Arg Phe Ala Gly Met Ser Ala Ala Gly Arg Tyr Leu Gln Glu Phe Val
            260                 265                 270

Gly Ala Asp Ile Glu Trp Ala His Val Asp Ile Ala Gly Pro Ala Tyr
        275                 280                 285

Asn Thr Ala Gly Glu Phe Gly Tyr Thr Pro Lys Arg Ala Thr Gly Gln
    290                 295                 300

Pro Val Arg Thr Phe Val Gln Val Leu Lys Asp Leu Ser Glu Ser
305                 310                 315


<210> 117
<211> 2127
<212> DNA
<213> Corynebacterium glutamicum

<220>
<221> CDS
<222> (101)..(2104)
<223> RXN01277

<400> 117
tactactcgg ttcacgttta cgtcggctga tccaattgga ggcgccctcg gaagccgcct 60

taaaaaacct gccggtcaaa agatcactaa cctgaacttc atg act gat tac acg   115
                                            Met Thr Asp Tyr Thr
                                            1               5

ttc ctc gaa gac att gac acc ccg gaa gcg ctc gcg tgg gcg gaa aaa   163
Phe Leu Glu Asp Ile Asp Thr Pro Glu Ala Leu Ala Trp Ala Glu Lys
                10                  15                  20

tgg tcg ggg gaa agc gtc gaa aag cta aaa agc cca gcc aag gac gcc   211
Trp Ser Gly Glu Ser Val Glu Lys Leu Lys Ser Pro Ala Lys Asp Ala
            25                  30                  35

ctg gaa gcc agg ctg ctg gct gcg ttg gac acc gat gat cgc att gcc   259
Leu Glu Ala Arg Leu Leu Ala Ala Leu Asp Thr Asp Asp Arg Ile Ala
        40                  45                  50

tac gtg agc cgg cgc ggt gag aag ctg tac aac ttt tgg cgg gac gcg   307
Tyr Val Ser Arg Arg Gly Glu Lys Leu Tyr Asn Phe Trp Arg Asp Ala
    55                  60                  65

cag cat ccg cgt gga gtg tgg cgc acg acc acg ttg gag tcg tat gaa   355
Gln His Pro Arg Gly Val Trp Arg Thr Thr Thr Leu Glu Ser Tyr Glu
70                  75                  80                  85

agt gac cag ccg gag tgg gac gtg ctc att gat gtg gat gcg ttg gcg   403
Ser Asp Gln Pro Glu Trp Asp Val Leu Ile Asp Val Asp Ala Leu Ala
                90                  95                  100

gag gat gag ggc gaa aac tgg gta tgg aag ggc gcg gtt gtg cgc tcg   451
Glu Asp Glu Gly Glu Asn Trp Val Trp Lys Gly Ala Val Val Arg Ser
            105                 110                 115

ccg gag ttt gat cgg gcg ttg gtg aag ttc tcg cgg ggc ggg gct gat   499
Pro Glu Phe Asp Arg Ala Leu Val Lys Phe Ser Arg Gly Gly Ala Asp
        120                 125                 130

gcg acg gtg att agg gag ttt gat ctg gcc acg gct gct ttc gtg gat     547
Ala Thr Val Ile Arg Glu Phe Asp Leu Ala Thr Ala Ala Phe Val Asp
    135                 140                 145

gat tcg ccg ttt gaa ttg aag gag gcg aag tcc gat gtc acg tgg gtt     595
Asp Ser Pro Phe Glu Leu Lys Glu Ala Lys Ser Asp Val Thr Trp Val
150                 155                 160                 165

gat ctg gat acg ttg ctg gtg ggc acg gat acc ggc gag ggg tca ctg     643
Asp Leu Asp Thr Leu Leu Val Gly Thr Asp Thr Gly Glu Gly Ser Leu
                170                 175                 180

acg gat tct ggg tac ccg gcg cgg gtg ctc acg tgg aag cgt ggg act     691
Thr Asp Ser Gly Tyr Pro Ala Arg Val Leu Thr Trp Lys Arg Gly Thr
            185                 190                 195

ccg ctt gag cag gcg gag ttg ttc ttt gag ggg tcg cgt cag gat gtg     739
Pro Leu Glu Gln Ala Glu Leu Phe Phe Glu Gly Ser Arg Gln Asp Val
        200                 205                 210

gcg act cat gcg tgg cgg gat tca aca cct ggt ttt gag cgg acg ttt     787
Ala Thr His Ala Trp Arg Asp Ser Thr Pro Gly Phe Glu Arg Thr Phe
    215                 220                 225

gtg tca agg tcg ttg gat ttc tat aat tcg gag acg tcg ctg gaa acc     835
Val Ser Arg Ser Leu Asp Phe Tyr Asn Ser Glu Thr Ser Leu Glu Thr
230                 235                 240                 245

gag ggt ggc ctg gtc aag ctt gat gtg ccg acc gat tgc gat gtc att     883
Glu Gly Gly Leu Val Lys Leu Asp Val Pro Thr Asp Cys Asp Val Ile
                250                 255                 260

gtg aag aag cag tgg att ttt gtg agt cct cgg acg gat ttc gct ggg     931
Val Lys Lys Gln Trp Ile Phe Val Ser Pro Arg Thr Asp Phe Ala Gly
            265                 270                 275

att cca gca ggt ggc ttg gga gtg ctg ctg tta aag gag ttc ctt gag     979
Ile Pro Ala Gly Gly Leu Gly Val Leu Leu Leu Lys Glu Phe Leu Glu
        280                 285                 290

ggc ggg cgc gat ttt cag cct gtg ttt acg cct act gag tcg acg tcg     1027
Gly Gly Arg Asp Phe Gln Pro Val Phe Thr Pro Thr Glu Ser Thr Ser
    295                 300                 305

ctg cag gga ttg gcc acg aca aag aat ttc ctg gtt tta acg ctc ctt     1075
Leu Gln Gly Leu Ala Thr Thr Lys Asn Phe Leu Val Leu Thr Leu Leu
310                 315                 320                 325

aat aat gtc tcc aca gaa atc gtc aca gtg ccg ctc aat gat ccg aca     1123
Asn Asn Val Ser Thr Glu Ile Val Thr Val Pro Leu Asn Asp Pro Thr
                330                 335                 340

acg gag cat gaa cac att gac ctc cca gag cat gtc acc gcg cat gtg     1171
Thr Glu His Glu His Ile Asp Leu Pro Glu His Val Thr Ala His Val
            345                 350                 355

gtt gct acc tcc ccg ttg gat ggc gat gaa att tgg gtg cag gca gcg     1219
Val Ala Thr Ser Pro Leu Asp Gly Asp Glu Ile Trp Val Gln Ala Ala
        360                 365                 370

agt ttc acc gaa gcg cca acg ttg ctg cgt gcg gag ctg cct ggt gcg     1267
Ser Phe Thr Glu Ala Pro Thr Leu Leu Arg Ala Glu Leu Pro Gly Ala
    375                 380                 385

ctt gag gct gtg aag aag gcg ccg ttg cag ttt gaa aat gct ggt cag     1315
Leu Glu Ala Val Lys Lys Ala Pro Leu Gln Phe Glu Asn Ala Gly Gln
390                 395                 400                 405

gag act cgt cag cat tgg gca acc tcg gcg gat gga acg aag att ccg     1363
Glu Thr Arg Gln His Trp Ala Thr Ser Ala Asp Gly Thr Lys Ile Pro
                410                 415                 420

tac ttt att aca gga gcc ttc gag gag gaa cca caa aac acc ctg gtc     1411
Tyr Phe Ile Thr Gly Ala Phe Glu Glu Glu Pro Gln Asn Thr Leu Val
            425                 430                 435

cac gcc tac ggc ggc ttc gag gtt tcc ctt acc cca agc cac tcc ccg     1459
His Ala Tyr Gly Gly Phe Glu Val Ser Leu Thr Pro Ser His Ser Pro
            440                 445                 450

acc cgc ggc atc gca tgg ttg gaa aag ggc tac tac ttt gtg gaa gcc     1507
Thr Arg Gly Ile Ala Trp Leu Glu Lys Gly Tyr Tyr Phe Val Glu Ala
    455                 460                 465

aac ctg cgt ggt ggc ggt gaa ttc ggt ccg gaa tgg cat tcg cag gca     1555
Asn Leu Arg Gly Gly Gly Glu Phe Gly Pro Glu Trp His Ser Gln Ala
470                 475                 480                 485

acc aag ctg aac cgc atg aag gtg tgg gag gat cac cgc gcg gtg ctc     1603
Thr Lys Leu Asn Arg Met Lys Val Trp Glu Asp His Arg Ala Val Leu
                490                 495                 500

gcc gac ctt gtg gag cgc ggc tac gca acg ccg gag cag att gcg att     1651
Ala Asp Leu Val Glu Arg Gly Tyr Ala Thr Pro Glu Gln Ile Ala Ile
            505                 510                 515

cgt ggc gga tcc aac ggt ggt ttg ctg aca agt ggc gcg tta act cag     1699
Arg Gly Gly Ser Asn Gly Gly Leu Leu Thr Ser Gly Ala Leu Thr Gln
        520                 525                 530

tac cca gaa gca ttc ggt gcg gca gtt gtg cag gtg ccg ttg gct gat     1747
Tyr Pro Glu Ala Phe Gly Ala Ala Val Val Gln Val Pro Leu Ala Asp
    535                 540                 545

atg ttg cgc tat cac acc tgg tca gcg ggt gct tcg tgg atg gcg gag     1795
Met Leu Arg Tyr His Thr Trp Ser Ala Gly Ala Ser Trp Met Ala Glu
550                 555                 560                 565

tac ggc aac cct gac gat ccg gag gaa cgg gcg gtg att gag cag tac     1843
Tyr Gly Asn Pro Asp Asp Pro Glu Glu Arg Ala Val Ile Glu Gln Tyr
                570                 575                 580

tcg ccg gtg cag gcg gtg gtg ggc gtc gag aag cga att tat cca ccc     1891
Ser Pro Val Gln Ala Val Val Gly Val Glu Lys Arg Ile Tyr Pro Pro
            585                 590                 595

gca ttg gtg acg acc tca acc cgg gac gac cgc gtc cac ccc gcg cac     1939
Ala Leu Val Thr Thr Ser Thr Arg Asp Asp Arg Val His Pro Ala His
        600                 605                 610

gcg cgc ctt ttt gct caa gct ttg ctt gat gcg ggc cag gcc gtg gat     1987
Ala Arg Leu Phe Ala Gln Ala Leu Leu Asp Ala Gly Gln Ala Val Asp
    615                 620                 625

tac tac gaa aac acc gag ggc ggc cat gcc ggc gcg gcg gat aac aag     2035
Tyr Tyr Glu Asn Thr Glu Gly Gly His Ala Gly Ala Ala Asp Asn Lys
630                 635                 640                 645

cag acc gcg ttt gtg gaa tcg ctg atc tac acc tgg atc gag aag act     2083
Gln Thr Ala Phe Val Glu Ser Leu Ile Tyr Thr Trp Ile Glu Lys Thr
                650                 655                 660

ttg gat cag cag ggt agc att taatacctat gattatgcga agg               2127
Leu Asp Gln Gln Gly Ser Ile
            665

<210> 118
<211> 668
<212> PRT
<213> Corynebacterium glutamicum

<400> 118
Met Thr Asp Tyr Thr Phe Leu Glu Asp Ile Asp Thr Pro Glu Ala Leu
  1               5                  10                  15

Ala Trp Ala Glu Lys Trp Ser Gly Glu Ser Val Glu Lys Leu Lys Ser
             20                  25                  30

Pro Ala Lys Asp Ala Leu Glu Ala Arg Leu Leu Ala Ala Leu Asp Thr
         35                  40                  45

Asp Asp Arg Ile Ala Tyr Val Ser Arg Arg Gly Glu Lys Leu Tyr Asn

50                  55                  60

Phe Trp Arg Asp Ala Gln His Pro Arg Gly Val Trp Arg Thr Thr Thr
65                  70                  75                  80

Leu Glu Ser Tyr Glu Ser Asp Gln Pro Glu Trp Asp Val Leu Ile Asp
                85                  90                  95

Val Asp Ala Leu Ala Glu Asp Glu Gly Glu Asn Trp Val Trp Lys Gly
            100                 105                 110

Ala Val Val Arg Ser Pro Glu Phe Asp Arg Ala Leu Val Lys Phe Ser
        115                 120                 125

Arg Gly Gly Ala Asp Ala Thr Val Ile Arg Glu Phe Asp Leu Ala Thr
    130                 135                 140

Ala Ala Phe Val Asp Asp Ser Pro Phe Glu Leu Lys Glu Ala Lys Ser
145                 150                 155                 160

Asp Val Thr Trp Val Asp Leu Asp Thr Leu Leu Val Gly Thr Asp Thr
                165                 170                 175

Gly Glu Gly Ser Leu Thr Asp Ser Gly Tyr Pro Ala Arg Val Leu Thr
            180                 185                 190

Trp Lys Arg Gly Thr Pro Leu Glu Gln Ala Glu Leu Phe Phe Glu Gly
        195                 200                 205

Ser Arg Gln Asp Val Ala Thr His Ala Trp Arg Asp Ser Thr Pro Gly
    210                 215                 220

Phe Glu Arg Thr Phe Val Ser Arg Ser Leu Asp Phe Tyr Asn Ser Glu
225                 230                 235                 240

Thr Ser Leu Glu Thr Glu Gly Gly Leu Val Lys Leu Asp Val Pro Thr
                245                 250                 255

Asp Cys Asp Val Ile Val Lys Lys Gln Trp Ile Phe Val Ser Pro Arg
            260                 265                 270

Thr Asp Phe Ala Gly Ile Pro Ala Gly Gly Leu Gly Val Leu Leu Leu
        275                 280                 285

Lys Glu Phe Leu Glu Gly Gly Arg Asp Phe Gln Pro Val Phe Thr Pro
    290                 295                 300

Thr Glu Ser Thr Ser Leu Gln Gly Leu Ala Thr Thr Lys Asn Phe Leu
305                 310                 315                 320

Val Leu Thr Leu Leu Asn Asn Val Ser Thr Glu Ile Val Thr Val Pro
                325                 330                 335

Leu Asn Asp Pro Thr Thr Glu His Glu His Ile Asp Leu Pro Glu His
            340                 345                 350

Val Thr Ala His Val Val Ala Thr Ser Pro Leu Asp Gly Asp Glu Ile
        355                 360                 365

Trp Val Gln Ala Ala Ser Phe Thr Glu Ala Pro Thr Leu Leu Arg Ala
    370                 375                 380

Glu Leu Pro Gly Ala Leu Glu Ala Val Lys Lys Ala Pro Leu Gln Phe
385                 390                 395                 400

Glu Asn Ala Gly Gln Glu Thr Arg Gln His Trp Ala Thr Ser Ala Asp
                405                 410                 415

Gly Thr Lys Ile Pro Tyr Phe Ile Thr Gly Ala Phe Glu Glu Glu Pro
            420                 425                 430

Gln Asn Thr Leu Val His Ala Tyr Gly Gly Phe Glu Val Ser Leu Thr
        435                 440                 445

Pro Ser His Ser Pro Thr Arg Gly Ile Ala Trp Leu Glu Lys Gly Tyr
    450                 455                 460

Tyr Phe Val Glu Ala Asn Leu Arg Gly Gly Gly Glu Phe Gly Pro Glu
465                 470                 475                 480

Trp His Ser Gln Ala Thr Lys Leu Asn Arg Met Lys Val Trp Glu Asp
                485                 490                 495

His Arg Ala Val Leu Ala Asp Leu Val Glu Arg Gly Tyr Ala Thr Pro
            500                 505                 510

Glu Gln Ile Ala Ile Arg Gly Gly Ser Asn Gly Gly Leu Leu Thr Ser
        515                 520                 525

Gly Ala Leu Thr Gln Tyr Pro Glu Ala Phe Gly Ala Ala Val Val Gln
    530                 535                 540

Val Pro Leu Ala Asp Met Leu Arg Tyr His Thr Trp Ser Ala Gly Ala
545                 550                 555                 560

Ser Trp Met Ala Glu Tyr Gly Asn Pro Asp Asp Pro Glu Glu Arg Ala
                565                 570                 575

Val Ile Glu Gln Tyr Ser Pro Val Gln Ala Val Val Gly Val Glu Lys
            580                 585                 590

Arg Ile Tyr Pro Pro Ala Leu Val Thr Thr Ser Thr Arg Asp Asp Arg
        595                 600                 605

Val His Pro Ala His Ala Arg Leu Phe Ala Gln Ala Leu Leu Asp Ala
    610                 615                 620

Gly Gln Ala Val Asp Tyr Tyr Glu Asn Thr Glu Gly Gly His Ala Gly
625                 630                 635                 640

Ala Ala Asp Asn Lys Gln Thr Ala Phe Val Glu Ser Leu Ile Tyr Thr
                645                 650                 655

Trp Ile Glu Lys Thr Leu Asp Gln Gln Gly Ser Ile
            660                 665


<210> 119
<211> 1812
<212> DNA
<213> Corynebacterium glutamicum

<220>
<221> CDS
<222> (101)..(1789)
<223> FRXA01277

<400> 119
tactactcgg ttcacgttta cgtcggctga tccaattgga ggcgccctcg gaagccgcct 60

taaaaaacct gccggtcaaa agatcactaa cctgaacttc atg act gat tac acg    115
                                            Met Thr Asp Tyr Thr
                                            1               5

ttc ctc gaa gac att gac acc ccg gaa gcg ctc gcg tgg gcg gaa aaa    163
Phe Leu Glu Asp Ile Asp Thr Pro Glu Ala Leu Ala Trp Ala Glu Lys
                10                  15                  20

tgg tcg ggg gaa agc gtc gaa aag cta aaa agc cca gcc aag gac gcc    211
Trp Ser Gly Glu Ser Val Glu Lys Leu Lys Ser Pro Ala Lys Asp Ala
            25                  30                  35

ctg gaa gcc agg ctg ctg gct gcg ttg gac acc gat gat cgc att gcc    259
Leu Glu Ala Arg Leu Leu Ala Ala Leu Asp Thr Asp Asp Arg Ile Ala
        40                  45                  50

tac gtg agc cgg cgc ggt gag aag ctg tac aac ttt tgg cgg gac gcg    307
Tyr Val Ser Arg Arg Gly Glu Lys Leu Tyr Asn Phe Trp Arg Asp Ala
    55                  60                  65

cag cat ccg cgt gga gtg tgg cgc acg acc acg ttg gag tcg tat gaa    355
Gln His Pro Arg Gly Val Trp Arg Thr Thr Thr Leu Glu Ser Tyr Glu
70                  75                  80                  85

agt gac cag ccg gag tgg gac gtg ctc att gat gtg gat gcg ttg gcg     403
Ser Asp Gln Pro Glu Trp Asp Val Leu Ile Asp Val Asp Ala Leu Ala
                90                  95                  100

gag gat gag ggc gaa aac tgg gta tgg aag ggc gcg gtt gtg cgc tcg     451
Glu Asp Glu Gly Glu Asn Trp Val Trp Lys Gly Ala Val Val Arg Ser
            105                 110                 115

ccg gag ttt gat cgg gcg ttg gtg aag ttc tcg cgg ggc ggg gct gat     499
Pro Glu Phe Asp Arg Ala Leu Val Lys Phe Ser Arg Gly Gly Ala Asp
        120                 125                 130

gcg acg gtg att agg gag ttt gat ctg gcc acg gct gct ttc gtg gat     547
Ala Thr Val Ile Arg Glu Phe Asp Leu Ala Thr Ala Ala Phe Val Asp
    135                 140                 145

gat tcg ccg ttt gaa ttg aag gag gcg aag tcc gat gtc acg tgg gtt     595
Asp Ser Pro Phe Glu Leu Lys Glu Ala Lys Ser Asp Val Thr Trp Val
150                 155                 160                 165

gat ctg gat acg ttg ctg gtg ggc acg gat acc ggc gag ggg tca ctg     643
Asp Leu Asp Thr Leu Leu Val Gly Thr Asp Thr Gly Glu Gly Ser Leu
                170                 175                 180

acg gat tct ggg tac ccg gcg cgg gtg ctc acg tgg aag cgt ggg act     691
Thr Asp Ser Gly Tyr Pro Ala Arg Val Leu Thr Trp Lys Arg Gly Thr
            185                 190                 195

ccg ctt gag cag gcg gag ttg ttc ttt gag ggg tcg cgt cag gat gtg     739
Pro Leu Glu Gln Ala Glu Leu Phe Phe Glu Gly Ser Arg Gln Asp Val
        200                 205                 210

gcg act cat gcg tgg cgg gat tca aca cct ggt ttt gag cgg acg ttt     787
Ala Thr His Ala Trp Arg Asp Ser Thr Pro Gly Phe Glu Arg Thr Phe
    215                 220                 225

gtg tca agg tcg ttg gat ttc tat aat tcg gag acg tcg ctg gaa acc     835
Val Ser Arg Ser Leu Asp Phe Tyr Asn Ser Glu Thr Ser Leu Glu Thr
230                 235                 240                 245

gag ggt ggc ctg gtc aag ctt gat gtg ccg acc gat tgc gat gtc att     883
Glu Gly Gly Leu Val Lys Leu Asp Val Pro Thr Asp Cys Asp Val Ile
                250                 255                 260

gtg aag aag cag tgg att ttt gtg agt cct cgg acg gat ttc gct ggg     931
Val Lys Lys Gln Trp Ile Phe Val Ser Pro Arg Thr Asp Phe Ala Gly
            265                 270                 275

att cca gca ggt ggc ttg gga gtg ctg ctg tta aag gag ttc ctt gag     979
Ile Pro Ala Gly Gly Leu Gly Val Leu Leu Leu Lys Glu Phe Leu Glu
        280                 285                 290

ggc ggg cgc gat ttt cag cct gtg ttt acg cct act gag tcg acg tcg     1027
Gly Gly Arg Asp Phe Gln Pro Val Phe Thr Pro Thr Glu Ser Thr Ser
    295                 300                 305

ctg cag gga ttg gcc acg aca aag aat ttc ctg gtt tta acg ctc ctt     1075
Leu Gln Gly Leu Ala Thr Thr Lys Asn Phe Leu Val Leu Thr Leu Leu
310                 315                 320                 325

aat aat gtc tcc aca gaa atc gtc aca gtg ccg ctc aat gat ccg aca     1123
Asn Asn Val Ser Thr Glu Ile Val Thr Val Pro Leu Asn Asp Pro Thr
                330                 335                 340

acg gag cat gaa cac att gac ctc cca gag cat gtc acc gcg cat gtg     1171
Thr Glu His Glu His Ile Asp Leu Pro Glu His Val Thr Ala His Val
            345                 350                 355

gtt gct acc tcc ccg ttg gat ggc gat gaa att tgg gtg cag gca gcg     1219
Val Ala Thr Ser Pro Leu Asp Gly Asp Glu Ile Trp Val Gln Ala Ala
        360                 365                 370

agt ttc acc gaa gcg cca acg ttg ctg cgt gcg gag ctg cct ggt gcg     1267
Ser Phe Thr Glu Ala Pro Thr Leu Leu Arg Ala Glu Leu Pro Gly Ala
    375                 380                 385

ctt gag gct gtg aag aag gcg ccg ttg cag ttt gaa aat gct ggt cag     1315

Leu Glu Ala Val Lys Lys Ala Pro Leu Gln Phe Glu Asn Ala Gly Gln
390                 395                 400                 405

gag act cgt cag cat tgg gca acc tcg gcg gat gga acg aag att ccg     1363
Glu Thr Arg Gln His Trp Ala Thr Ser Ala Asp Gly Thr Lys Ile Pro
                410                 415                 420

tac ttt att aca gga gcc ttc gag gag gaa cca caa aac acc ctg gtc     1411
Tyr Phe Ile Thr Gly Ala Phe Glu Glu Glu Pro Gln Asn Thr Leu Val
            425                 430                 435

cac gcc tac ggc ggc ttc gag gtt tcc ctt acc cca agc cac tcc ccg     1459
His Ala Tyr Gly Gly Phe Glu Val Ser Leu Thr Pro Ser His Ser Pro
        440                 445                 450

acc cgc ggc atc gca tgg ttg gaa aag ggc tac tac ttt gtg gaa gcc     1507
Thr Arg Gly Ile Ala Trp Leu Glu Lys Gly Tyr Tyr Phe Val Glu Ala
    455                 460                 465

aac ctg cgt ggt ggc ggt gaa ttc ggt ccg gaa tgg cat tcg cag gca     1555
Asn Leu Arg Gly Gly Gly Glu Phe Gly Pro Glu Trp His Ser Gln Ala
470                 475                 480                 485

acc aag ctg aac cgc atg aag gtg tgg gag gat cac cgc gcg gtg ctc     1603
Thr Lys Leu Asn Arg Met Lys Val Trp Glu Asp His Arg Ala Val Leu
                490                 495                 500

gcc gac ctt gtg gag cgc ggc tac gca acg ccg gag cag att gcg att     1651
Ala Asp Leu Val Glu Arg Gly Tyr Ala Thr Pro Glu Gln Ile Ala Ile
            505                 510                 515

cgt ggc gga tcc aac ggt ggt ttg ctg aca agt ggc gcg tta act cag     1699
Arg Gly Gly Ser Asn Gly Gly Leu Leu Thr Ser Gly Ala Leu Thr Gln
        520                 525                 530

tac cca gaa gca ttc ggt gcg gca gtt gtg cag gtg ccg ttg gct gat     1747
Tyr Pro Glu Ala Phe Gly Ala Ala Val Val Gln Val Pro Leu Ala Asp
    535                 540                 545

atg ttg cgc tat cac acc tgg tca gcg ggt acc tcg tgg atg             1789
Met Leu Arg Tyr His Thr Trp Ser Ala Gly Thr Ser Trp Met
550                 555                 560

taggtgtcgg caaccatggg aac                                           1812


<210> 120
<211> 563
<212> PRT
<213> Corynebacterium glutamicum

<400> 120
Met Thr Asp Tyr Thr Phe Leu Glu Asp Ile Asp Thr Pro Glu Ala Leu
1               5                   10                  15

Ala Trp Ala Glu Lys Trp Ser Gly Glu Ser Val Glu Lys Leu Lys Ser
            20                  25                  30

Pro Ala Lys Asp Ala Leu Glu Ala Arg Leu Leu Ala Ala Leu Asp Thr
        35                  40                  45

Asp Asp Arg Ile Ala Tyr Val Ser Arg Arg Gly Glu Lys Leu Tyr Asn
    50                  55                  60

Phe Trp Arg Asp Ala Gln His Pro Arg Gly Val Trp Arg Thr Thr Thr
65                  70                  75                  80

Leu Glu Ser Tyr Glu Ser Asp Gln Pro Glu Trp Asp Val Leu Ile Asp
                85                  90                  95

Val Asp Ala Leu Ala Glu Asp Glu Gly Glu Asn Trp Val Trp Lys Gly
            100                 105                 110

Ala Val Val Arg Ser Pro Glu Phe Asp Arg Ala Leu Val Lys Phe Ser
        115                 120                 125

Arg Gly Gly Ala Asp Ala Thr Val Ile Arg Glu Phe Asp Leu Ala Thr

130                 135                 140

Ala Ala Phe Val Asp Asp Ser Pro Phe Glu Leu Lys Glu Ala Lys Ser
145                 150                 155                 160

Asp Val Thr Trp Val Asp Leu Asp Thr Leu Leu Val Gly Thr Asp Thr
                165                 170                 175

Gly Glu Gly Ser Leu Thr Asp Ser Gly Tyr Pro Ala Arg Val Leu Thr
            180                 185                 190

Trp Lys Arg Gly Thr Pro Leu Glu Gln Ala Glu Leu Phe Phe Glu Gly
        195                 200                 205

Ser Arg Gln Asp Val Ala Thr His Ala Trp Arg Asp Ser Thr Pro Gly
    210                 215                 220

Phe Glu Arg Thr Phe Val Ser Arg Ser Leu Asp Phe Tyr Asn Ser Glu
225                 230                 235                 240

Thr Ser Leu Glu Thr Glu Gly Gly Leu Val Lys Leu Asp Val Pro Thr
                245                 250                 255

Asp Cys Asp Val Ile Val Lys Lys Gln Trp Ile Phe Val Ser Pro Arg
            260                 265                 270

Thr Asp Phe Ala Gly Ile Pro Ala Gly Gly Leu Gly Val Leu Leu Leu
        275                 280                 285

Lys Glu Phe Leu Glu Gly Gly Arg Asp Phe Gln Pro Val Phe Thr Pro
    290                 295                 300

Thr Glu Ser Thr Ser Leu Gln Gly Leu Ala Thr Thr Lys Asn Phe Leu
305                 310                 315                 320

Val Leu Thr Leu Leu Asn Asn Val Ser Thr Glu Ile Val Thr Val Pro
                325                 330                 335

Leu Asn Asp Pro Thr Thr Glu His Glu His Ile Asp Leu Pro Glu His
            340                 345                 350

Val Thr Ala His Val Val Ala Thr Ser Pro Leu Asp Gly Asp Glu Ile
        355                 360                 365

Trp Val Gln Ala Ala Ser Phe Thr Glu Ala Pro Thr Leu Leu Arg Ala
    370                 375                 380

Glu Leu Pro Gly Ala Leu Glu Ala Val Lys Lys Ala Pro Leu Gln Phe
385                 390                 395                 400

Glu Asn Ala Gly Gln Glu Thr Arg Gln His Trp Ala Thr Ser Ala Asp
                405                 410                 415

Gly Thr Lys Ile Pro Tyr Phe Ile Thr Gly Ala Phe Glu Glu Glu Pro
            420                 425                 430

Gln Asn Thr Leu Val His Ala Tyr Gly Gly Phe Glu Val Ser Leu Thr
        435                 440                 445

Pro Ser His Ser Pro Thr Arg Gly Ile Ala Trp Leu Glu Lys Gly Tyr
    450                 455                 460

Tyr Phe Val Glu Ala Asn Leu Arg Gly Gly Gly Glu Phe Gly Pro Glu
465                 470                 475                 480

Trp His Ser Gln Ala Thr Lys Leu Asn Arg Met Lys Val Trp Glu Asp
                485                 490                 495

His Arg Ala Val Leu Ala Asp Leu Val Glu Arg Gly Tyr Ala Thr Pro
            500                 505                 510

Glu Gln Ile Ala Ile Arg Gly Gly Ser Asn Gly Gly Leu Leu Thr Ser
        515                 520                 525

Gly Ala Leu Thr Gln Tyr Pro Glu Ala Phe Gly Ala Ala Val Val Gln
    530                 535                 540

Val Pro Leu Ala Asp Met Leu Arg Tyr His Thr Trp Ser Ala Gly Thr
545                 550                 555                 560

Ser Trp Met

<210> 121
<211> 526
<212> DNA
<213> Corynebacterium glutamicum

<220>
<221> CDS
<222> (101)..(526)
<223> RXA01914

<400> 121
ggttttgagg aatggctagg cttgttaaaa gttagtttca atttgatgcc tcccccaacc 60

aaagcggaga cacaacttca acgagaggac tcagctttca atg gcg aaa aat gcc    115
                                            Met Ala Lys Asn Ala
                                            1               5

tac agc aca aca gca cca acc aag gtg tcc aag gat gcc act ctt cca    163
Tyr Ser Thr Thr Ala Pro Thr Lys Val Ser Lys Asp Ala Thr Leu Pro
                10                  15                  20

gtt cgt gga acg gtc gct gaa ctc aag ctc gaa aag aag ttg cca aag    211
Val Arg Gly Thr Val Ala Glu Leu Lys Leu Glu Lys Lys Leu Pro Lys
            25                  30                  35

aag att gat gcc atc atc gtc gcg att ttt gaa ggc gaa gat tcc atc    259
Lys Ile Asp Ala Ile Ile Val Ala Ile Phe Glu Gly Glu Asp Ser Ile
        40                  45                  50

gaa ctc gcc ggc ggc gaa atc ctc gat ttc atc ttc agt acc gag cag    307
Glu Leu Ala Gly Gly Glu Ile Leu Asp Phe Ile Phe Ser Thr Glu Gln
    55                  60                  65

cag gcc gac atc ctc act cag ctc gaa gct gtc ggc gca aag gcc acc    355
Gln Ala Asp Ile Leu Thr Gln Leu Glu Ala Val Gly Ala Lys Ala Thr
70                  75                  80                  85

gca aac agc atc acc cgc gtc cca ggc acc gac gtt gcg cct gtc att    403
Ala Asn Ser Ile Thr Arg Val Pro Gly Thr Asp Val Ala Pro Val Ile
                90                  95                  100

gcg gtt ggt ttg ggc aag gct gat ttg ctt gac gac gag acc ctc cgc    451
Ala Val Gly Leu Gly Lys Ala Asp Leu Leu Asp Asp Glu Thr Leu Arg
            105                 110                 115

cgc gct tcc ggc acg gcg gcc cgc tcc ctc ggt ggt ttt gaa aat gtc    499
Arg Ala Ser Gly Thr Ala Ala Arg Ser Leu Gly Gly Phe Glu Asn Val
        120                 125                 130

gcc acc acc att ggc gat ttg gga ctt                                526
Ala Thr Thr Ile Gly Asp Leu Gly Leu
    135                 140

<210> 122
<211> 142
<212> PRT
<213> Corynebacterium glutamicum

<400> 122
Met Ala Lys Asn Ala Tyr Ser Thr Thr Ala Pro Thr Lys Val Ser Lys
1               5                   10                  15

Asp Ala Thr Leu Pro Val Arg Gly Thr Val Ala Glu Leu Lys Leu Glu
            20                  25                  30

Lys Lys Leu Pro Lys Lys Ile Asp Ala Ile Ile Val Ala Ile Phe Glu
        35                  40                  45

Gly Glu Asp Ser Ile Glu Leu Ala Gly Gly Glu Ile Leu Asp Phe Ile

50                  55                  60

Phe Ser Thr Glu Gln Gln Ala Asp Ile Leu Thr Gln Leu Glu Ala Val
65                  70                  75                  80

Gly Ala Lys Ala Thr Ala Asn Ser Ile Thr Arg Val Pro Gly Thr Asp
                85                  90                  95

Val Ala Pro Val Ile Ala Val Gly Leu Gly Lys Ala Asp Leu Leu Asp
            100                 105                 110

Asp Glu Thr Leu Arg Arg Ala Ser Gly Thr Ala Ala Arg Ser Leu Gly
        115                 120                 125

Gly Phe Glu Asn Val Ala Thr Thr Ile Gly Asp Leu Gly Leu
    130                 135                 140


<210> 123
<211> 1497
<212> DNA
<213> Corynebacterium glutamicum

<220>
<221> CDS
<222> (101)..(1474)
<223> RXA02048

<400> 123
gcgcggcgga taacaagcag accgcgtttg tggaatcgct gatctacacc tggatcgaaa 60

agactttgga tcagcagggt agcatttaat acctatgatt atg cga agg ctg cgc   115
                                            Met Arg Arg Leu Arg
                                            1               5

tcc acc ccg gtc cct ggt aca cgc gat tcc tac aca gga att gat ttc   163
Ser Thr Pro Val Pro Gly Thr Arg Asp Ser Tyr Thr Gly Ile Asp Phe
                10                  15                  20

aac tta ggc ttc cac atc cga cgc tac gag ctt gat ctc acc tac cgc   211
Asn Leu Gly Phe His Ile Arg Arg Tyr Glu Leu Asp Leu Thr Tyr Arg
            25                  30                  35

gta gca ccc aac ctg ctc atg ggc acc gca acg ctg cac atg gat aat   259
Val Ala Pro Asn Leu Leu Met Gly Thr Ala Thr Leu His Met Asp Asn
        40                  45                  50

tac cgt gcg ctc gac gcg ctg acc ctg gac ctc ggc ggc agc ctg cgc   307
Tyr Arg Ala Leu Asp Ala Leu Thr Leu Asp Leu Gly Gly Ser Leu Arg
    55                  60                  65

gtg gaa aaa gtc acc gcc aaa ggc acc gcc ggc acc cac atc caa gtc   355
Val Glu Lys Val Thr Ala Lys Gly Thr Ala Gly Thr His Ile Gln Val
70                  75                  80                  85

gcg cgc ttc cgc cac gcc ggc cgc aaa ctg cgc atc acc ttc cgc aac   403
Ala Arg Phe Arg His Ala Gly Arg Lys Leu Arg Ile Thr Phe Arg Asn
                90                  95                  100

caa atc ccg gtt gac cag gaa ttt tca ctc acc atc cgc tac cgc ggc   451
Gln Ile Pro Val Asp Gln Glu Phe Ser Leu Thr Ile Arg Tyr Arg Gly
            105                 110                 115

aac ccg cgc ccc ctg cgc agc gaa tgg ggc atg atc ggc tgg gaa gag   499
Asn Pro Arg Pro Leu Arg Ser Glu Trp Gly Met Ile Gly Trp Glu Glu
        120                 125                 130

ctc gac aac ggc gcc ctc gtc gcc gcc cag cca aac ggc gcg ccg agc   547
Leu Asp Asn Gly Ala Leu Val Ala Ala Gln Pro Asn Gly Ala Pro Ser
    135                 140                 145

tgg ttc ccc tgc gac gac acg ccc gac gag aag gcg ctt ttc gac gtc   595
Trp Phe Pro Cys Asp Asp Thr Pro Asp Glu Lys Ala Leu Phe Asp Val
150                 155                 160                 165

cac ttc cac acc gac aac gga tac gcc gcc att atc acc ggt gat tta   643
His Phe His Thr Asp Asn Gly Tyr Ala Ala Ile Ile Thr Gly Asp Leu

170                 175                 180

atc tca aaa cac gtc agt ggc agc atg acc acc tgg cac tac caa tcc     691
Ile Ser Lys His Val Ser Gly Ser Met Thr Thr Trp His Tyr Gln Ser
            185                 190                 195

cgc gaa ccc atg gcc acc tac ctc gca gcc gtc cac gtc gga gaa tac     739
Arg Glu Pro Met Ala Thr Tyr Leu Ala Ala Val His Val Gly Glu Tyr
        200                 205                 210

gac act gta tcc ctg ggc gtt tcg gaa tcg ggc gtt gtg gtg gag gcg     787
Asp Thr Val Ser Leu Gly Val Ser Glu Ser Gly Val Val Val Glu Ala
    215                 220                 225

tat gtg cct gtg ggg gat gcg gcc ttg cgg gct cgg att ttg gag gac     835
Tyr Val Pro Val Gly Asp Ala Ala Leu Arg Ala Arg Ile Leu Glu Asp
230                 235                 240                 245

ttt gcc aaa caa gtc gac atg tta gac gcc tac gaa aaa ctc ttc ggc     883
Phe Ala Lys Gln Val Asp Met Leu Asp Ala Tyr Glu Lys Leu Phe Gly
                250                 255                 260

ccc tac cca ttc cgc agc tac cgc gta gtc atc acc gaa gac gaa ctc     931
Pro Tyr Pro Phe Arg Ser Tyr Arg Val Val Ile Thr Glu Asp Glu Leu
            265                 270                 275

gaa atc cca ctc gaa gcc caa ggc ctc tcc agc ttc gga gcc aac cac     979
Glu Ile Pro Leu Glu Ala Gln Gly Leu Ser Ser Phe Gly Ala Asn His
        280                 285                 290

gcc acc ggc gaa gga acc tgg gaa cga ctc atc gcc cac gaa ctc tcc     1027
Ala Thr Gly Glu Gly Thr Trp Glu Arg Leu Ile Ala His Glu Leu Ser
    295                 300                 305

cac cag tgg ttt ggc aac tca ctc ggc ctc gcc caa tgg aac gac atc     1075
His Gln Trp Phe Gly Asn Ser Leu Gly Leu Ala Gln Trp Asn Asp Ile
310                 315                 320                 325

tgg ctc aac gaa ggc ttc gcc tgt tac gcg gaa tgg ctc tgg ttt gag     1123
Trp Leu Asn Glu Gly Phe Ala Cys Tyr Ala Glu Trp Leu Trp Phe Glu
                330                 335                 340

gca gct gga gtt aag tcg gct gcg gaa agt gcg ttg gaa ttc tat cga     1171
Ala Ala Gly Val Lys Ser Ala Ala Glu Ser Ala Leu Glu Phe Tyr Arg
            345                 350                 355

ggc ctg gag gcg ctg ccg aag gat att ttg ctg gcc aac ccc ggc gcg     1219
Gly Leu Glu Ala Leu Pro Lys Asp Ile Leu Leu Ala Asn Pro Gly Ala
        360                 365                 370

aag gat atg ttc gac gac cgc gtc tac aag cgc ggc gct ctg act gtc     1267
Lys Asp Met Phe Asp Asp Arg Val Tyr Lys Arg Gly Ala Leu Thr Val
    375                 380                 385

cat gca ttg cgg gaa ttg ctt ggc gat gat gca ttc ttc aaa gct gtg     1315
His Ala Leu Arg Glu Leu Leu Gly Asp Asp Ala Phe Phe Lys Ala Val
390                 395                 400                 405

cgc tcc tac gtt gcc gaa ggc cga cac gga ctc gtt gaa ccc cgc gac     1363
Arg Ser Tyr Val Ala Glu Gly Arg His Gly Leu Val Glu Pro Arg Asp
                410                 415                 420

ctg aaa cga cac ctc tac gca gtc tcc aca gac cac gca gct tta gat     1411
Leu Lys Arg His Leu Tyr Ala Val Ser Thr Asp His Ala Ala Leu Asp
            425                 430                 435

gca gtg tgg cag tcc tgg ctt cgc gat ctg gag ttg ccg gag ttt cct     1459
Ala Val Trp Gln Ser Trp Leu Arg Asp Leu Glu Leu Pro Glu Phe Pro
        440                 445                 450

tct ggt ggt ttg gac tagtgcgcta tctgacgctg gcc                       1497
Ser Gly Gly Leu Asp
    455

<210> 124
<211> 458

<212> PRT
<213> Corynebacterium glutamicum

<400> 124
Met Arg Arg Leu Arg Ser Thr Pro Val Pro Gly Thr Arg Asp Ser Tyr
1               5                   10                  15

Thr Gly Ile Asp Phe Asn Leu Gly Phe His Ile Arg Arg Tyr Glu Leu
            20                  25                  30

Asp Leu Thr Tyr Arg Val Ala Pro Asn Leu Leu Met Gly Thr Ala Thr
        35                  40                  45

Leu His Met Asp Asn Tyr Arg Ala Leu Asp Ala Leu Thr Leu Asp Leu
    50                  55                  60

Gly Gly Ser Leu Arg Val Glu Lys Val Thr Ala Lys Gly Thr Ala Gly
65                  70                  75                  80

Thr His Ile Gln Val Ala Arg Phe Arg His Ala Gly Arg Lys Leu Arg
                85                  90                  95

Ile Thr Phe Arg Asn Gln Ile Pro Val Asp Gln Glu Phe Ser Leu Thr
            100                 105                 110

Ile Arg Tyr Arg Gly Asn Pro Arg Pro Leu Arg Ser Glu Trp Gly Met
        115                 120                 125

Ile Gly Trp Glu Glu Leu Asp Asn Gly Ala Leu Val Ala Ala Gln Pro
    130                 135                 140

Asn Gly Ala Pro Ser Trp Phe Pro Cys Asp Asp Thr Pro Asp Glu Lys
145                 150                 155                 160

Ala Leu Phe Asp Val His Phe His Thr Asp Asn Gly Tyr Ala Ala Ile
                165                 170                 175

Ile Thr Gly Asp Leu Ile Ser Lys His Val Ser Gly Ser Met Thr Thr
            180                 185                 190

Trp His Tyr Gln Ser Arg Glu Pro Met Ala Thr Tyr Leu Ala Ala Val
        195                 200                 205

His Val Gly Glu Tyr Asp Thr Val Ser Leu Gly Val Ser Glu Ser Gly
    210                 215                 220

Val Val Val Glu Ala Tyr Val Pro Val Gly Asp Ala Ala Leu Arg Ala
225                 230                 235                 240

Arg Ile Leu Glu Asp Phe Ala Lys Gln Val Asp Met Leu Asp Ala Tyr
                245                 250                 255

Glu Lys Leu Phe Gly Pro Tyr Pro Phe Arg Ser Tyr Arg Val Val Ile
            260                 265                 270

Thr Glu Asp Glu Leu Glu Ile Pro Leu Glu Ala Gln Gly Leu Ser Ser
        275                 280                 285

Phe Gly Ala Asn His Ala Thr Gly Glu Gly Thr Trp Glu Arg Leu Ile
    290                 295                 300

Ala His Glu Leu Ser His Gln Trp Phe Gly Asn Ser Leu Gly Leu Ala
305                 310                 315                 320

Gln Trp Asn Asp Ile Trp Leu Asn Glu Gly Phe Ala Cys Tyr Ala Glu
                325                 330                 335

Trp Leu Trp Phe Glu Ala Ala Gly Val Lys Ser Ala Ala Glu Ser Ala
            340                 345                 350

Leu Glu Phe Tyr Arg Gly Leu Glu Ala Leu Pro Lys Asp Ile Leu Leu
        355                 360                 365

Ala Asn Pro Gly Ala Lys Asp Met Phe Asp Asp Arg Val Tyr Lys Arg
    370                 375                 380

Gly Ala Leu Thr Val His Ala Leu Arg Glu Leu Leu Gly Asp Asp Ala

385                 390                 395                 400

Phe Phe Lys Ala Val Arg Ser Tyr Val Ala Glu Gly Arg His Gly Leu
                405                 410                 415

Val Glu Pro Arg Asp Leu Lys Arg His Leu Tyr Ala Val Ser Thr Asp
            420                 425                 430

His Ala Ala Leu Asp Ala Val Trp Gln Ser Trp Leu Arg Asp Leu Glu
        435                 440                 445

Leu Pro Glu Phe Pro Ser Gly Gly Leu Asp
    450                 455


<210> 125
<211> 906
<212> DNA
<213> Corynebacterium glutamicum

<220>
<221> CDS
<222> (101)..(883)
<223> RXN00621

<400> 125
aaatgaatcc gggtttttca gtttcggggt gcaaatcaga atgtcgccaa tggcgaacac 60

acgagcgtgc agaagatgtg cgtgactaag atcgggggct atg tct gaa cgc cta    115
                                           Met Ser Glu Arg Leu
                                           1               5

aac gct ccg caa gca cca atc cat ccc atc acc cga acc cac cac ggt    163
Asn Ala Pro Gln Ala Pro Ile His Pro Ile Thr Arg Thr His His Gly
                10                  15                  20

att gat ttc gta gac aac tat gaa tgg ctg agg gat aaa gaa tcc caa    211
Ile Asp Phe Val Asp Asn Tyr Glu Trp Leu Arg Asp Lys Glu Ser Gln
            25                  30                  35

gaa acc ttg gac tac ctg gag gcg gag aat gcg ttc acc aag cag gag    259
Glu Thr Leu Asp Tyr Leu Glu Ala Glu Asn Ala Phe Thr Lys Gln Glu
        40                  45                  50

act gaa cag cta gcc aca ctg cgg gac aac atc tat gaa gag att aag    307
Thr Glu Gln Leu Ala Thr Leu Arg Asp Asn Ile Tyr Glu Glu Ile Lys
    55                  60                  65

tca cgc gtt aaa gaa acc gac atg tcc atc cca gtg cgt gcc gga aag    355
Ser Arg Val Lys Glu Thr Asp Met Ser Ile Pro Val Arg Ala Gly Lys
70                  75                  80                  85

cac tgg tat tac tct cgc act gaa gaa ggc aag agc tac ggc tat tcc    403
His Trp Tyr Tyr Ser Arg Thr Glu Glu Gly Lys Ser Tyr Gly Tyr Ser
                90                  95                  100

tgc cgc att cca gtg act gaa ggg tcg gat gca tgg acc cct cct gtt    451
Cys Arg Ile Pro Val Thr Glu Gly Ser Asp Ala Trp Thr Pro Pro Val
            105                 110                 115

atc cct gag ggt gag cca gcg cag ggt gaa acc atc atc atg gat gcc    499
Ile Pro Glu Gly Glu Pro Ala Gln Gly Glu Thr Ile Ile Met Asp Ala
        120                 125                 130

aac gag ttg gca gaa ggc cac gaa ttc ttc tcc atg ggt gca tca tct    547
Asn Glu Leu Ala Glu Gly His Glu Phe Phe Ser Met Gly Ala Ser Ser
    135                 140                 145

gtc acc acc tct ggc cgc tac ctt gcg tat tcc acc gat gtc acg ggc    595
Val Thr Thr Ser Gly Arg Tyr Leu Ala Tyr Ser Thr Asp Val Thr Gly
150                 155                 160                 165

gaa gag cgc ttt acg ttg cgc atc aag gat cta gaa act ggc gag ctg    643
Glu Glu Arg Phe Thr Leu Arg Ile Lys Asp Leu Glu Thr Gly Glu Leu
                170                 175                 180

ctt cct gat acc ctg act ggc att ttc tac ggt gct act tgg gtg ggg    691

Leu Pro Asp Thr Leu Thr Gly Ile Phe Tyr Gly Ala Thr Trp Val Gly
            185                 190                 195

gag gag tac ctc ttt tac cag cgc gtt gat gat gcg tgg cgt cca gat      739
Glu Glu Tyr Leu Phe Tyr Gln Arg Val Asp Asp Ala Trp Arg Pro Asp
        200                 205                 210

act gtg tgg cgc cac aag gtg ggt acc ccg gtt gaa gaa gac gtg ttg      787
Thr Val Trp Arg His Lys Val Gly Thr Pro Val Glu Glu Asp Val Leu
    215                 220                 225

gtg tac cac gag cct gat gaa cgt tat tcc acc tgg gtg ggc acc act      835
Val Tyr His Glu Pro Asp Glu Arg Tyr Ser Thr Trp Val Gly Thr Thr
230                 235                 240                 245

cgt tca gaa aaa gtt cat cct ttt tgg ttg cgc ctc caa gat cac ctc      883
Arg Ser Glu Lys Val His Pro Phe Trp Leu Arg Leu Gln Asp His Leu
                250                 255                 260

tgaagtacgc gtgcttcctt tcg                                            906


<210> 126
<211> 261
<212> PRT
<213> Corynebacterium glutamicum

<400> 126
Met Ser Glu Arg Leu Asn Ala Pro Gln Ala Pro Ile His Pro Ile Thr
1               5                   10                  15

Arg Thr His His Gly Ile Asp Phe Val Asp Asn Tyr Glu Trp Leu Arg
            20                  25                  30

Asp Lys Glu Ser Gln Glu Thr Leu Asp Tyr Leu Glu Ala Glu Asn Ala
        35                  40                  45

Phe Thr Lys Gln Glu Thr Glu Gln Leu Ala Thr Leu Arg Asp Asn Ile
    50                  55                  60

Tyr Glu Glu Ile Lys Ser Arg Val Lys Glu Thr Asp Met Ser Ile Pro
65                  70                  75                  80

Val Arg Ala Gly Lys His Trp Tyr Tyr Ser Arg Thr Glu Glu Gly Lys
                85                  90                  95

Ser Tyr Gly Tyr Ser Cys Arg Ile Pro Val Thr Glu Gly Ser Asp Ala
            100                 105                 110

Trp Thr Pro Pro Val Ile Pro Glu Gly Glu Pro Ala Gln Gly Glu Thr
        115                 120                 125

Ile Ile Met Asp Ala Asn Glu Leu Ala Glu Gly His Glu Phe Phe Ser
    130                 135                 140

Met Gly Ala Ser Ser Val Thr Thr Ser Gly Arg Tyr Leu Ala Tyr Ser
145                 150                 155                 160

Thr Asp Val Thr Gly Glu Glu Arg Phe Thr Leu Arg Ile Lys Asp Leu
                165                 170                 175

Glu Thr Gly Glu Leu Leu Pro Asp Thr Leu Thr Gly Ile Phe Tyr Gly
            180                 185                 190

Ala Thr Trp Val Gly Glu Glu Tyr Leu Phe Tyr Gln Arg Val Asp Asp
        195                 200                 205

Ala Trp Arg Pro Asp Thr Val Trp Arg His Lys Val Gly Thr Pro Val
    210                 215                 220

Glu Glu Asp Val Leu Val Tyr His Glu Pro Asp Glu Arg Tyr Ser Thr
225                 230                 235                 240

Trp Val Gly Thr Thr Arg Ser Glu Lys Val His Pro Phe Trp Leu Arg
                245                 250                 255

Leu Gln Asp His Leu

            260

<210> 127
<211> 906
<212> DNA
<213> Corynebacterium glutamicum

<220>
<221> CDS
<222> (101)..(883)
<223> FRXA00621

<400> 127
aaatgaatcc gggtttttca gtttcggggt gcaaatcaga atgtcgccaa tggcgaacac 60

acgagcgtgc agaagatgtg cgtgactaag atcgggggct atg tct gaa cgc cta 115
Met Ser Glu Arg Leu
1               5

aac gct ccg caa gca cca atc cat ccc atc acc cga acc cac cac ggt 163
Asn Ala Pro Gln Ala Pro Ile His Pro Ile Thr Arg Thr His His Gly
                10                  15                  20

att gat ttc gta gac aac tat gaa tgg ctg agg gat aaa gaa tcc caa 211
Ile Asp Phe Val Asp Asn Tyr Glu Trp Leu Arg Asp Lys Glu Ser Gln
            25                  30                  35

gaa acc ttg gac tac ctg gag gcg gag aat gcg ttc acc aag cag gag 259
Glu Thr Leu Asp Tyr Leu Glu Ala Glu Asn Ala Phe Thr Lys Gln Glu
        40                  45                  50

act gaa cag cta gcc aca ctg cgg gac aac atc tat gaa gag att aag 307
Thr Glu Gln Leu Ala Thr Leu Arg Asp Asn Ile Tyr Glu Glu Ile Lys
    55                  60                  65

tca cgc gtt aaa gaa acc gac atg tcc atc cca gtg cgt gcc gga aag 355
Ser Arg Val Lys Glu Thr Asp Met Ser Ile Pro Val Arg Ala Gly Lys
70                  75                  80                  85

cac tgg tat tac tct cgc act gaa gaa ggc aag agc tac ggc tat tcc 403
His Trp Tyr Tyr Ser Arg Thr Glu Glu Gly Lys Ser Tyr Gly Tyr Ser
                90                  95                  100

tgc cgc att cca gtg act gaa ggg tcg gat gca tgg acc cct cct gtt 451
Cys Arg Ile Pro Val Thr Glu Gly Ser Asp Ala Trp Thr Pro Pro Val
            105                 110                 115

atc cct gag ggt gag cca gcg cag ggt gaa acc atc atc atg gat gcc 499
Ile Pro Glu Gly Glu Pro Ala Gln Gly Glu Thr Ile Ile Met Asp Ala
        120                 125                 130

aac gag ttg gca gaa ggc cac gaa ttc ttc tcc atg ggt gca tca tct 547
Asn Glu Leu Ala Glu Gly His Glu Phe Phe Ser Met Gly Ala Ser Ser
    135                 140                 145

gtc acc acc tct ggc cgc tac ctt gcg tat tcc acc gat gtc acg ggc 595
Val Thr Thr Ser Gly Arg Tyr Leu Ala Tyr Ser Thr Asp Val Thr Gly
150                 155                 160                 165

gaa gag cgc ttt acg ttg cgc atc aag gat cta gaa act ggc gag ctg 643
Glu Glu Arg Phe Thr Leu Arg Ile Lys Asp Leu Glu Thr Gly Glu Leu
                170                 175                 180

ctt cct gat acc ctg act ggc att ttc tac ggt gct act tgg gtg ggg 691
Leu Pro Asp Thr Leu Thr Gly Ile Phe Tyr Gly Ala Thr Trp Val Gly
            185                 190                 195

gag gag tac ctc ttt tac cag cgc gtt gat gat gcg tgg cgt cca gat 739
Glu Glu Tyr Leu Phe Tyr Gln Arg Val Asp Asp Ala Trp Arg Pro Asp
        200                 205                 210

act gtg tgg cgc cac aag gtg ggt acc ccg gtt gaa gaa gac gtg ttg 787
Thr Val Trp Arg His Lys Val Gly Thr Pro Val Glu Glu Asp Val Leu
    215                 220                 225

gtg tac cac gag cct gat gaa cgt tat tcc acc tgg gtg ggc acc act 835

Val Tyr His Glu Pro Asp Glu Arg Tyr Ser Thr Trp Val Gly Thr Thr
230                 235                 240                 245

cgt tca gaa aaa gtt cat cct ttt tgg ttg cgc ctc caa gat cac ctc     883
Arg Ser Glu Lys Val His Pro Phe Trp Leu Arg Leu Gln Asp His Leu
                250                 255                 260

tgaagtacgc gtgcttcctt tcg                                           906


<210> 128
<211> 261
<212> PRT
<213> Corynebacterium glutamicum

<400> 128
Met Ser Glu Arg Leu Asn Ala Pro Gln Ala Pro Ile His Pro Ile Thr
1               5                   10                  15

Arg Thr His His Gly Ile Asp Phe Val Asp Asn Tyr Glu Trp Leu Arg
            20                  25                  30

Asp Lys Glu Ser Gln Glu Thr Leu Asp Tyr Leu Glu Ala Glu Asn Ala
        35                  40                  45

Phe Thr Lys Gln Glu Thr Glu Gln Leu Ala Thr Leu Arg Asp Asn Ile
    50                  55                  60

Tyr Glu Glu Ile Lys Ser Arg Val Lys Glu Thr Asp Met Ser Ile Pro
65                  70                  75                  80

Val Arg Ala Gly Lys His Trp Tyr Tyr Ser Arg Thr Glu Glu Gly Lys
                85                  90                  95

Ser Tyr Gly Tyr Ser Cys Arg Ile Pro Val Thr Glu Gly Ser Asp Ala
            100                 105                 110

Trp Thr Pro Pro Val Ile Pro Glu Gly Glu Pro Ala Gln Gly Glu Thr
        115                 120                 125

Ile Ile Met Asp Ala Asn Glu Leu Ala Glu Gly His Glu Phe Phe Ser
    130                 135                 140

Met Gly Ala Ser Ser Val Thr Thr Ser Gly Arg Tyr Leu Ala Tyr Ser
145                 150                 155                 160

Thr Asp Val Thr Gly Glu Glu Arg Phe Thr Leu Arg Ile Lys Asp Leu
                165                 170                 175

Glu Thr Gly Glu Leu Leu Pro Asp Thr Leu Thr Gly Ile Phe Tyr Gly
            180                 185                 190

Ala Thr Trp Val Gly Glu Glu Tyr Leu Phe Tyr Gln Arg Val Asp Asp
        195                 200                 205

Ala Trp Arg Pro Asp Thr Val Trp Arg His Lys Val Gly Thr Pro Val
    210                 215                 220

Glu Glu Asp Val Leu Val Tyr His Glu Pro Asp Glu Arg Tyr Ser Thr
225                 230                 235                 240

Trp Val Gly Thr Thr Arg Ser Glu Lys Val His Pro Phe Trp Leu Arg
                245                 250                 255

Leu Gln Asp His Leu
            260


<210> 129
<211> 1539
<212> DNA
<213> Corynebacterium glutamicum

<220>
<221> CDS
<222> (101)..(1516)
<223> RXN00622

<400> 129
ttttaccagc gcgttgatga tgcgtggcgt ccagatactg tgtggcgcca caaggtgggt 60

accccggttg aagaagacgt gttggtgtac cacgagcctg atg aac gtt att cca 115
Met Asn Val Ile Pro
1               5

cct ggg tgg gca cca ctc gtt cag aaa aag ttc atc ctt ttt ggt tgc 163
Pro Gly Trp Ala Pro Leu Val Gln Lys Lys Phe Ile Leu Phe Gly Cys
                10                  15                  20

gcc tcc aag atc acc tct gaa gta cgc gtg ctt cct ttc gac cag cca 211
Ala Ser Lys Ile Thr Ser Glu Val Arg Val Leu Pro Phe Asp Gln Pro
            25                  30                  35

gag ggc acc cct gag gtg ctg att ccg cgc gcg gag ggt gtg gaa tac 259
Glu Gly Thr Pro Glu Val Leu Ile Pro Arg Ala Glu Gly Val Glu Tyr
        40                  45                  50

gac gtc gat cat gca gtc gta gac ggc tcc gat att tgg ttg gtc aca 307
Asp Val Asp His Ala Val Val Asp Gly Ser Asp Ile Trp Leu Val Thr
    55                  60                  65

cac aac gcc gag ggc ccg aac ttt tcg gtg ggg tgg gct ggc gtc gac 355
His Asn Ala Glu Gly Pro Asn Phe Ser Val Gly Trp Ala Gly Val Asp
70                  75                  80                  85

aag ctc aat tct ttg gac gcg ctg gcg cca ctc gtc gcg cac aag gat 403
Lys Leu Asn Ser Leu Asp Ala Leu Ala Pro Leu Val Ala His Lys Asp
                90                  95                  100

gac gtg cgc att gag ggt gtc gat acc tac cgc gat ttc atc atc ctg 451
Asp Val Arg Ile Glu Gly Val Asp Thr Tyr Arg Asp Phe Ile Ile Leu
            105                 110                 115

ggc tac agg tcc ggc gcg atc ggc cag gtc gcg atc atg aag ctt atc 499
Gly Tyr Arg Ser Gly Ala Ile Gly Gln Val Ala Ile Met Lys Leu Ile
        120                 125                 130

gac gga acc ttc ggc gat ttc caa cag ctg gaa ttt gac gag gaa atc 547
Asp Gly Thr Phe Gly Asp Phe Gln Gln Leu Glu Phe Asp Glu Glu Ile
    135                 140                 145

tac acc gtc gca tcg ggc gga aac cca gaa tgg gac gcc ccc gtc att 595
Tyr Thr Val Ala Ser Gly Gly Asn Pro Glu Trp Asp Ala Pro Val Ile
150                 155                 160                 165

cgc ctt tct tac gga tca ttc acc acc ccg gcg cag ctg ttt aac tac 643
Arg Leu Ser Tyr Gly Ser Phe Thr Thr Pro Ala Gln Leu Phe Asn Tyr
                170                 175                 180

tgg att gaa tcc ggc gaa cgc acg ctg ctg aag cag cag gaa gtg ctc 691
Trp Ile Glu Ser Gly Glu Arg Thr Leu Leu Lys Gln Gln Glu Val Leu
            185                 190                 195

ggc gga tac aag ccg tca gac tat gtg gcc tcc cga ttg tgg gtc act 739
Gly Gly Tyr Lys Pro Ser Asp Tyr Val Ala Ser Arg Leu Trp Val Thr
        200                 205                 210

gcg aaa gat ggc gcg cag att cca gtg tcc ttg gtg cac cgc acc gac 787
Ala Lys Asp Gly Ala Gln Ile Pro Val Ser Leu Val His Arg Thr Asp
    215                 220                 225

ctg gat gta tcc aag ccc aac ccc acg ttg ctc tac ggc tat ggt tcc 835
Leu Asp Val Ser Lys Pro Asn Pro Thr Leu Leu Tyr Gly Tyr Gly Ser
230                 235                 240                 245

tac gaa tca tcc att gat cca ggc ttc tct atc gcg cgt ttg tca ctg 883
Tyr Glu Ser Ser Ile Asp Pro Gly Phe Ser Ile Ala Arg Leu Ser Leu
                250                 255                 260

atg gat cgt ggc atg att ttt gcg att gcc cac gtt cgt ggc ggt ggc 931
Met Asp Arg Gly Met Ile Phe Ala Ile Ala His Val Arg Gly Gly Gly
            265                 270                 275

gaa atg ggt cgt ggc tgg tac gac aac ggc aaa acc acc acg aag aaa 979

Glu Met Gly Arg Gly Trp Tyr Asp Asn Gly Lys Thr Thr Thr Lys Lys
        280                 285                 290

aac acc ttc acc gac ttc att gat gtt gcc gac gcc ctc atc gag cag      1027
Asn Thr Phe Thr Asp Phe Ile Asp Val Ala Asp Ala Leu Ile Glu Gln
    295                 300                 305

aag att tct gcc cct gaa atg ctg gtt gca gaa ggc ggc tca gct ggt      1075
Lys Ile Ser Ala Pro Glu Met Leu Val Ala Glu Gly Gly Ser Ala Gly
310                 315                 320                 325

ggc atg ctc atg ggc gcc att gcc aac atg gcc ggt gac cgc ttc aag      1123
Gly Met Leu Met Gly Ala Ile Ala Asn Met Ala Gly Asp Arg Phe Lys
                330                 335                 340

gcg atc gaa gcc aac gtg cca ttc gtc gat ccg ctg acc tct atg ctc      1171
Ala Ile Glu Ala Asn Val Pro Phe Val Asp Pro Leu Thr Ser Met Leu
            345                 350                 355

atg ccg gaa ctg cca ctg acg gtt atc gaa tgg gat gag tgg ggc gat      1219
Met Pro Glu Leu Pro Leu Thr Val Ile Glu Trp Asp Glu Trp Gly Asp
        360                 365                 370

cca ctc cac gat aag gac gtc tat gaa tac atg gcg tcg tat gcc cca      1267
Pro Leu His Asp Lys Asp Val Tyr Glu Tyr Met Ala Ser Tyr Ala Pro
    375                 380                 385

tat gaa aac atc gag gca aag aac tac ccc aat atc ttg gcc gta aca      1315
Tyr Glu Asn Ile Glu Ala Lys Asn Tyr Pro Asn Ile Leu Ala Val Thr
390                 395                 400                 405

tcg ctc aac gac acc cga gtg ttg tac gtc gaa cca gcc aaa tgg gta      1363
Ser Leu Asn Asp Thr Arg Val Leu Tyr Val Glu Pro Ala Lys Trp Val
                410                 415                 420

gcg cag ctt cgg gcg act gca acc ggt gga gaa ttc ctt ctg aaa act      1411
Ala Gln Leu Arg Ala Thr Ala Thr Gly Gly Glu Phe Leu Leu Lys Thr
            425                 430                 435

gaa atg gtt gcc gga cac ggc ggt gtg tca gga cgc tac gaa aag tgg      1459
Glu Met Val Ala Gly His Gly Gly Val Ser Gly Arg Tyr Glu Lys Trp
        440                 445                 450

cgt gag act gca ttt gag tac ggc tgg ttg atc aac caa gca acc ggt      1507
Arg Glu Thr Ala Phe Glu Tyr Gly Trp Leu Ile Asn Gln Ala Thr Gly
    455                 460                 465

gtg acc gaa taaaacttgt tcgactagcg aac                                1539
Val Thr Glu
470


<210> 130
<211> 472
<212> PRT
<213> Corynebacterium glutamicum

<400> 130
Met Asn Val Ile Pro Pro Gly Trp Ala Pro Leu Val Gln Lys Lys Phe
1               5                   10                  15

Ile Leu Phe Gly Cys Ala Ser Lys Ile Thr Ser Glu Val Arg Val Leu
            20                  25                  30

Pro Phe Asp Gln Pro Glu Gly Thr Pro Glu Val Leu Ile Pro Arg Ala
        35                  40                  45

Glu Gly Val Glu Tyr Asp Val Asp His Ala Val Val Asp Gly Ser Asp
    50                  55                  60

Ile Trp Leu Val Thr His Asn Ala Glu Gly Pro Asn Phe Ser Val Gly
65                  70                  75                  80

Trp Ala Gly Val Asp Lys Leu Asn Ser Leu Asp Ala Leu Ala Pro Leu
                85                  90                  95

Val Ala His Lys Asp Asp Val Arg Ile Glu Gly Val Asp Thr Tyr Arg

100                 105                 110

Asp Phe Ile Ile Leu Gly Tyr Arg Ser Gly Ala Ile Gly Gln Val Ala
        115                 120                 125

Ile Met Lys Leu Ile Asp Gly Thr Phe Gly Asp Phe Gln Gln Leu Glu
    130                 135                 140

Phe Asp Glu Glu Ile Tyr Thr Val Ala Ser Gly Gly Asn Pro Glu Trp
145                 150                 155                 160

Asp Ala Pro Val Ile Arg Leu Ser Tyr Gly Ser Phe Thr Thr Pro Ala
                165                 170                 175

Gln Leu Phe Asn Tyr Trp Ile Glu Ser Gly Glu Arg Thr Leu Leu Lys
            180                 185                 190

Gln Gln Glu Val Leu Gly Gly Tyr Lys Pro Ser Asp Tyr Val Ala Ser
        195                 200                 205

Arg Leu Trp Val Thr Ala Lys Asp Gly Ala Gln Ile Pro Val Ser Leu
    210                 215                 220

Val His Arg Thr Asp Leu Asp Val Ser Lys Pro Asn Pro Thr Leu Leu
225                 230                 235                 240

Tyr Gly Tyr Gly Ser Tyr Glu Ser Ser Ile Asp Pro Gly Phe Ser Ile
                245                 250                 255

Ala Arg Leu Ser Leu Met Asp Arg Gly Met Ile Phe Ala Ile Ala His
            260                 265                 270

Val Arg Gly Gly Gly Glu Met Gly Arg Gly Trp Tyr Asp Asn Gly Lys
        275                 280                 285

Thr Thr Thr Lys Lys Asn Thr Phe Thr Asp Phe Ile Asp Val Ala Asp
    290                 295                 300

Ala Leu Ile Glu Gln Lys Ile Ser Ala Pro Glu Met Leu Val Ala Glu
305                 310                 315                 320

Gly Gly Ser Ala Gly Gly Met Leu Met Gly Ala Ile Ala Asn Met Ala
                325                 330                 335

Gly Asp Arg Phe Lys Ala Ile Glu Ala Asn Val Pro Phe Val Asp Pro
            340                 345                 350

Leu Thr Ser Met Leu Met Pro Glu Leu Pro Leu Thr Val Ile Glu Trp
        355                 360                 365

Asp Glu Trp Gly Asp Pro Leu His Asp Lys Asp Val Tyr Glu Tyr Met
    370                 375                 380

Ala Ser Tyr Ala Pro Tyr Glu Asn Ile Glu Ala Lys Asn Tyr Pro Asn
385                 390                 395                 400

Ile Leu Ala Val Thr Ser Leu Asn Asp Thr Arg Val Leu Tyr Val Glu
                405                 410                 415

Pro Ala Lys Trp Val Ala Gln Leu Arg Ala Thr Ala Thr Gly Gly Glu
            420                 425                 430

Phe Leu Leu Lys Thr Glu Met Val Ala Gly His Gly Gly Val Ser Gly
        435                 440                 445

Arg Tyr Glu Lys Trp Arg Glu Thr Ala Phe Glu Tyr Gly Trp Leu Ile
    450                 455                 460

Asn Gln Ala Thr Gly Val Thr Glu
465                 470

<210> 131
<211> 1539
<212> DNA
<213> Corynebacterium glutamicum

<220>
<221> CDS
<222> (101)..(1516)
<223> FRXA00622

<400> 131
ttttaccagc gcgttgatga tgcgtggcgt ccagatactg tgtggcgcca caaggtgggt 60

accccggttg aagaagacgt gttggtgtac cacgagcctg atg aac gtt att cca 115
Met Asn Val Ile Pro
1               5

cct ggg tgg gca cca ctc gtt cag aaa aag ttc atc ctt ttt ggt tgc 163
Pro Gly Trp Ala Pro Leu Val Gln Lys Lys Phe Ile Leu Phe Gly Cys
                10                  15                  20

gcc tcc aag atc acc tct gaa gta cgc gtg ctt cct ttc gac cag cca 211
Ala Ser Lys Ile Thr Ser Glu Val Arg Val Leu Pro Phe Asp Gln Pro
            25                  30                  35

gag ggc acc cct gag gtg ctg att ccg cgc gcg gag ggt gtg gaa tac 259
Glu Gly Thr Pro Glu Val Leu Ile Pro Arg Ala Glu Gly Val Glu Tyr
        40                  45                  50

gac gtc gat cat gca gtc gta gac ggc tcc gat att tgg ttg gtc aca 307
Asp Val Asp His Ala Val Val Asp Gly Ser Asp Ile Trp Leu Val Thr
    55                  60                  65

cac aac gcc gag ggc ccg aac ttt tcg gtg ggg tgg gct ggc gtc gac 355
His Asn Ala Glu Gly Pro Asn Phe Ser Val Gly Trp Ala Gly Val Asp
70                  75                  80                  85

aag ctc aat tct ttg gac gcg ctg gcg cca ctc gtc gcg cac aag gat 403
Lys Leu Asn Ser Leu Asp Ala Leu Ala Pro Leu Val Ala His Lys Asp
                90                  95                  100

gac gtg cgc att gag ggt gtc gat acc tac cgc gat ttc atc atc ctg 451
Asp Val Arg Ile Glu Gly Val Asp Thr Tyr Arg Asp Phe Ile Ile Leu
            105                 110                 115

ggc tac agg tcc ggc gcg atc ggc cag gtc gcg atc atg aag ctt atc 499
Gly Tyr Arg Ser Gly Ala Ile Gly Gln Val Ala Ile Met Lys Leu Ile
        120                 125                 130

gac gga acc ttc ggc gat ttc caa cag ctg gaa ttt gac gag gaa atc 547
Asp Gly Thr Phe Gly Asp Phe Gln Gln Leu Glu Phe Asp Glu Glu Ile
    135                 140                 145

tac acc gtc gca tcg ggc gga aac cca gaa tgg gac gcc ccc gtc att 595
Tyr Thr Val Ala Ser Gly Gly Asn Pro Glu Trp Asp Ala Pro Val Ile
150                 155                 160                 165

cgc ctt tct tac gga tca ttc acc acc ccg gcg cag ctg ttt aac tac 643
Arg Leu Ser Tyr Gly Ser Phe Thr Thr Pro Ala Gln Leu Phe Asn Tyr
                170                 175                 180

tgg att gaa tcc ggc gaa cgc acg ctg ctg aag cag cag gaa gtg ctc 691
Trp Ile Glu Ser Gly Glu Arg Thr Leu Leu Lys Gln Gln Glu Val Leu
            185                 190                 195

ggc gga tac aag ccg tca gac tat gtg gcc tcc cga ttg tgg gtc act 739
Gly Gly Tyr Lys Pro Ser Asp Tyr Val Ala Ser Arg Leu Trp Val Thr
        200                 205                 210

gcg aaa gat ggc gcg cag att cca gtg tcc ttg gtg cac cgc acc gac 787
Ala Lys Asp Gly Ala Gln Ile Pro Val Ser Leu Val His Arg Thr Asp
    215                 220                 225

ctg gat gta tcc aag ccc aac ccc acg ttg ctc tac ggc tat ggt tcc 835
Leu Asp Val Ser Lys Pro Asn Pro Thr Leu Leu Tyr Gly Tyr Gly Ser
230                 235                 240                 245

tac gaa tca tcc att gat cca ggc ttc tct atc gcg cgt ttg tca ctg 883
Tyr Glu Ser Ser Ile Asp Pro Gly Phe Ser Ile Ala Arg Leu Ser Leu
                250                 255                 260

atg gat cgt ggc atg att ttt gcg att gcc cac gtt cgt ggc ggt ggc 931

Met Asp Arg Gly Met Ile Phe Ala Ile Ala His Val Arg Gly Gly Gly
            265                 270                 275

gaa atg ggt cgt ggc tgg tac gac aac ggc aaa acc acc acg aag aaa      979
Glu Met Gly Arg Gly Trp Tyr Asp Asn Gly Lys Thr Thr Thr Lys Lys
        280                 285                 290

aac acc ttc acc gac ttc att gat gtt gcc gac gcc ctc atc gag cag      1027
Asn Thr Phe Thr Asp Phe Ile Asp Val Ala Asp Ala Leu Ile Glu Gln
    295                 300                 305

aag att tct gcc cct gaa atg ctg gtt gca gaa ggc ggc tca gct ggt      1075
Lys Ile Ser Ala Pro Glu Met Leu Val Ala Glu Gly Gly Ser Ala Gly
310                 315                 320                 325

ggc atg ctc atg ggc gcc att gcc aac atg gcc ggt gac cgc ttc aag      1123
Gly Met Leu Met Gly Ala Ile Ala Asn Met Ala Gly Asp Arg Phe Lys
                330                 335                 340

gcg atc gaa gcc aac gtg cca ttc gtc gat ccg ctg acc tct atg ctc      1171
Ala Ile Glu Ala Asn Val Pro Phe Val Asp Pro Leu Thr Ser Met Leu
            345                 350                 355

atg ccg gaa ctg cca ctg acg gtt atc gaa tgg gat gag tgg ggc gat      1219
Met Pro Glu Leu Pro Leu Thr Val Ile Glu Trp Asp Glu Trp Gly Asp
        360                 365                 370

cca ctc cac gat aag gac gtc tat gaa tac atg gcg tcg tat gcc cca      1267
Pro Leu His Asp Lys Asp Val Tyr Glu Tyr Met Ala Ser Tyr Ala Pro
    375                 380                 385

tat gaa aac atc gag gca aag aac tac ccc aat atc ttg gcc gta aca      1315
Tyr Glu Asn Ile Glu Ala Lys Asn Tyr Pro Asn Ile Leu Ala Val Thr
390                 395                 400                 405

tcg ctc aac gac acc cga gtg ttg tac gtc gaa cca gcc aaa tgg gta      1363
Ser Leu Asn Asp Thr Arg Val Leu Tyr Val Glu Pro Ala Lys Trp Val
                410                 415                 420

gcg cag ctt cgg gcg act gca acc ggt gga gaa ttc ctt ctg aaa act      1411
Ala Gln Leu Arg Ala Thr Ala Thr Gly Gly Glu Phe Leu Leu Lys Thr
            425                 430                 435

gaa atg gtt gcc gga cac ggc ggt gtg tca gga cgc tac gaa aag tgg      1459
Glu Met Val Ala Gly His Gly Gly Val Ser Gly Arg Tyr Glu Lys Trp
        440                 445                 450

cgt gag act gca ttt gag tac ggc tgg ttg atc aac caa gca acc ggt      1507
Arg Glu Thr Ala Phe Glu Tyr Gly Trp Leu Ile Asn Gln Ala Thr Gly
    455                 460                 465

gtg acc gaa taaaacttgt tcgactagcg aac                                1539
Val Thr Glu
470


<210> 132
<211> 472
<212> PRT
<213> Corynebacterium glutamicum

<400> 132
Met Asn Val Ile Pro Pro Gly Trp Ala Pro Leu Val Gln Lys Lys Phe
  1               5                  10                  15

Ile Leu Phe Gly Cys Ala Ser Lys Ile Thr Ser Glu Val Arg Val Leu
             20                  25                  30

Pro Phe Asp Gln Pro Glu Gly Thr Pro Glu Val Leu Ile Pro Arg Ala
         35                  40                  45

Glu Gly Val Glu Tyr Asp Val Asp His Ala Val Val Asp Gly Ser Asp
     50                  55                  60

Ile Trp Leu Val Thr His Asn Ala Glu Gly Pro Asn Phe Ser Val Gly
 65                  70                  75                  80

Trp Ala Gly Val Asp Lys Leu Asn Ser Leu Asp Ala Leu Ala Pro Leu
                85                  90                  95

Val Ala His Lys Asp Asp Val Arg Ile Glu Gly Val Asp Thr Tyr Arg
            100                 105                 110

Asp Phe Ile Ile Leu Gly Tyr Arg Ser Gly Ala Ile Gly Gln Val Ala
        115                 120                 125

Ile Met Lys Leu Ile Asp Gly Thr Phe Gly Asp Phe Gln Gln Leu Glu
    130                 135                 140

Phe Asp Glu Glu Ile Tyr Thr Val Ala Ser Gly Gly Asn Pro Glu Trp
145                 150                 155                 160

Asp Ala Pro Val Ile Arg Leu Ser Tyr Gly Ser Phe Thr Thr Pro Ala
                165                 170                 175

Gln Leu Phe Asn Tyr Trp Ile Glu Ser Gly Glu Arg Thr Leu Leu Lys
            180                 185                 190

Gln Gln Glu Val Leu Gly Gly Tyr Lys Pro Ser Asp Tyr Val Ala Ser
        195                 200                 205

Arg Leu Trp Val Thr Ala Lys Asp Gly Ala Gln Ile Pro Val Ser Leu
    210                 215                 220

Val His Arg Thr Asp Leu Asp Val Ser Lys Pro Asn Pro Thr Leu Leu
225                 230                 235                 240

Tyr Gly Tyr Gly Ser Tyr Glu Ser Ser Ile Asp Pro Gly Phe Ser Ile
                245                 250                 255

Ala Arg Leu Ser Leu Met Asp Arg Gly Met Ile Phe Ala Ile Ala His
            260                 265                 270

Val Arg Gly Gly Gly Glu Met Gly Arg Gly Trp Tyr Asp Asn Gly Lys
        275                 280                 285

Thr Thr Thr Lys Lys Asn Thr Phe Thr Asp Phe Ile Asp Val Ala Asp
    290                 295                 300

Ala Leu Ile Glu Gln Lys Ile Ser Ala Pro Glu Met Leu Val Ala Glu
305                 310                 315                 320

Gly Gly Ser Ala Gly Gly Met Leu Met Gly Ala Ile Ala Asn Met Ala
                325                 330                 335

Gly Asp Arg Phe Lys Ala Ile Glu Ala Asn Val Pro Phe Val Asp Pro
            340                 345                 350

Leu Thr Ser Met Leu Met Pro Glu Leu Pro Leu Thr Val Ile Glu Trp
        355                 360                 365

Asp Glu Trp Gly Asp Pro Leu His Asp Lys Asp Val Tyr Glu Tyr Met
    370                 375                 380

Ala Ser Tyr Ala Pro Tyr Glu Asn Ile Glu Ala Lys Asn Tyr Pro Asn
385                 390                 395                 400

Ile Leu Ala Val Thr Ser Leu Asn Asp Thr Arg Val Leu Tyr Val Glu
                405                 410                 415

Pro Ala Lys Trp Val Ala Gln Leu Arg Ala Thr Ala Thr Gly Gly Glu
            420                 425                 430

Phe Leu Leu Lys Thr Glu Met Val Ala Gly His Gly Gly Val Ser Gly
        435                 440                 445

Arg Tyr Glu Lys Trp Arg Glu Thr Ala Phe Glu Tyr Gly Trp Leu Ile
    450                 455                 460

Asn Gln Ala Thr Gly Val Thr Glu
465                 470

<210> 133

<211> 1629
<212> DNA
<213> Corynebacterium glutamicum

<220>
<221> CDS
<222> (101)..(1606)
<223> RXN00982

<400> 133
gaaaacaaac gtccttgaag ccgtaatgcc ccgttcgaca ataaaaaggg tagtagcagt 60

tcttgccgcc tcgactgcgc ttagcccctt tttggtatca atg ccc act gca gca 115
Met Pro Thr Ala Ala
1               5

gcg caa gaa aac atc cgc tgg gaa gaa tgc cca cct cag gta gat att 163
Ala Gln Glu Asn Ile Arg Trp Glu Glu Cys Pro Pro Gln Val Asp Ile
                10                  15                  20

gcc tcc gct caa tgt ggc agc atc gac gtg ccc atg cac tat tct gat 211
Ala Ser Ala Gln Cys Gly Ser Ile Asp Val Pro Met His Tyr Ser Asp
            25                  30                  35

ccc tca ctt ggc gat atc agc gtg ggc ttt gtc aag gtc cct gcc caa 259
Pro Ser Leu Gly Asp Ile Ser Val Gly Phe Val Lys Val Pro Ala Gln
        40                  45                  50

ggc gaa aag cac ggc acc atc ttc ggt aac tcc ggt ggc cct ggt ggc 307
Gly Glu Lys His Gly Thr Ile Phe Gly Asn Ser Gly Gly Pro Gly Gly
    55                  60                  65

gat gcc tat agc ttc ttc ggc agc caa tcc atg aac tgg cca gaa gcc 355
Asp Ala Tyr Ser Phe Phe Gly Ser Gln Ser Met Asn Trp Pro Glu Ala
70                  75                  80                  85

atg tac caa aac tac gac ctc gtt gca gtg cag cct cgc gga atg gtc 403
Met Tyr Gln Asn Tyr Asp Leu Val Ala Val Gln Pro Arg Gly Met Val
                90                  95                  100

ggc tcc aca ccg gtt aac tgc gac aac atc gca cca gga tac gat ttc 451
Gly Ser Thr Pro Val Asn Cys Asp Asn Ile Ala Pro Gly Tyr Asp Phe
            105                 110                 115

ctc tcg ctg ctc acc cgc gaa ggc gct ttc gtt aaa gaa tcc tgc gag 499
Leu Ser Leu Leu Thr Arg Glu Gly Ala Phe Val Lys Glu Ser Cys Glu
        120                 125                 130

atc ggc acc ccc ggc tac acc tcc agc ctg acc acc gac aac acc gcc 547
Ile Gly Thr Pro Gly Tyr Thr Ser Ser Leu Thr Thr Asp Asn Thr Ala
    135                 140                 145

aac gac tgg gag cgc gtc cgc caa gca ctt ggc gat gac aag atc tcc 595
Asn Asp Trp Glu Arg Val Arg Gln Ala Leu Gly Asp Asp Lys Ile Ser
150                 155                 160                 165

atc ttc gga ctg tcc tac gga acc tac ctc gga tcg gtc tac gcc acc 643
Ile Phe Gly Leu Ser Tyr Gly Thr Tyr Leu Gly Ser Val Tyr Ala Thr
                170                 175                 180

cgc tac cca cag cac acc gac aag gtt gtc ctc gat tcc gca atg gcg 691
Arg Tyr Pro Gln His Thr Asp Lys Val Val Leu Asp Ser Ala Met Ala
            185                 190                 195

ccc agc ctg gca tgg aac ggc atc atg gcc tcc caa gaa cag ggc tac 739
Pro Ser Leu Ala Trp Asn Gly Ile Met Ala Ser Gln Glu Gln Gly Tyr
        200                 205                 210

aaa aac tcc ctc aac gac ttc ttc acc tgg gtt gca gaa aac aac gac 787
Lys Asn Ser Leu Asn Asp Phe Phe Thr Trp Val Ala Glu Asn Asn Asp
    215                 220                 225

acg tat ggc ctc ggc act acc cca cta gcc gtg tac caa aac tgg tca 835
Thr Tyr Gly Leu Gly Thr Thr Pro Leu Ala Val Tyr Gln Asn Trp Ser
230                 235                 240                 245

aac aag atc gtc gcc gaa acc gga acc aac cca acc gtt gct cca cca 883

Asn Lys Ile Val Ala Glu Thr Gly Thr Asn Pro Thr Val Ala Pro Pro
                250                 255                 260

cca gca caa gtt ggc gat gtc cca cca gca ttc gca tgg gcc ggc caa      931
Pro Ala Gln Val Gly Asp Val Pro Pro Ala Phe Ala Trp Ala Gly Gln
            265                 270                 275

gca ggc gca gac atg atg acc gcc acc aac cca acc tcc gtg caa ctc      979
Ala Gly Ala Asp Met Met Thr Ala Thr Asn Pro Thr Ser Val Gln Leu
        280                 285                 290

cag ggc ctt gcc acc cag ctc cta aac cct gga tcc aac cag tca ctg      1027
Gln Gly Leu Ala Thr Gln Leu Leu Asn Pro Gly Ser Asn Gln Ser Leu
    295                 300                 305

agc cct ctg ctc aac gtc acc cgc gcc tac att cca cag cca tca acc      1075
Ser Pro Leu Leu Asn Val Thr Arg Ala Tyr Ile Pro Gln Pro Ser Thr
310                 315                 320                 325

tgg ccc atg ctc gca ggc gcc atc tca ggg caa aca ccc atc cct gac      1123
Trp Pro Met Leu Ala Gly Ala Ile Ser Gly Gln Thr Pro Ile Pro Asp
                330                 335                 340

gta act gac acc ggc gac gac cca tac gtc atc gaa agc atc aac gcc      1171
Val Thr Asp Thr Gly Asp Asp Pro Tyr Val Ile Glu Ser Ile Asn Ala
            345                 350                 355

agc gtc aac atg cag cgc atg gtc atg tgc aac gaa aac acc gtc gca      1219
Ser Val Asn Met Gln Arg Met Val Met Cys Asn Glu Asn Thr Val Ala
        360                 365                 370

cca gac cca gta gca atg gca cgc atg gcc tgg aca agc atg gtc acc      1267
Pro Asp Pro Val Ala Met Ala Arg Met Ala Trp Thr Ser Met Val Thr
    375                 380                 385

ggc gac gtc ttt gac att tac tcc gtt aaa ttc agc tcc gga caa gcc      1315
Gly Asp Val Phe Asp Ile Tyr Ser Val Lys Phe Ser Ser Gly Gln Ala
390                 395                 400                 405

tgc tcc ggc atc acc cca aca agc ggc cgc cag cca acc gac gga tct      1363
Cys Ser Gly Ile Thr Pro Thr Ser Gly Arg Gln Pro Thr Asp Gly Ser
                410                 415                 420

caa cta gca gtc caa cca cta ctc ctc cag gga acc agc gac cca caa      1411
Gln Leu Ala Val Gln Pro Leu Leu Leu Gln Gly Thr Ser Asp Pro Gln
            425                 430                 435

acc cca tac tgg acc cac aac gag ctt gcc gac gcc atg aac gcc cac      1459
Thr Pro Tyr Trp Thr His Asn Glu Leu Ala Asp Ala Met Asn Ala His
        440                 445                 450

gtg gtc acc gtc aac gga cca gga cac ggc caa tcc atc ggc ggc acc      1507
Val Val Thr Val Asn Gly Pro Gly His Gly Gln Ser Ile Gly Gly Thr
    455                 460                 465

aac caa gca atc aac gac att gtt gtg gac tac ctc cgc acc gga cac      1555
Asn Gln Ala Ile Asn Asp Ile Val Val Asp Tyr Leu Arg Thr Gly His
470                 475                 480                 485

acc gac gcc acc tgg gtc gaa ggc aac aca ccc acc cca att acg gct      1603
Thr Asp Ala Thr Trp Val Glu Gly Asn Thr Pro Thr Pro Ile Thr Ala
                490                 495                 500

ggc taattgcttt ccacttagta gat                                        1629
Gly

<210> 134
<211> 502
<212> PRT
<213> Corynebacterium glutamicum

<400> 134
Met Pro Thr Ala Ala Ala Gln Glu Asn Ile Arg Trp Glu Glu Cys Pro
1               5                   10                  15

Pro Gln Val Asp Ile Ala Ser Ala Gln Cys Gly Ser Ile Asp Val Pro
                20                  25                  30

Met His Tyr Ser Asp Pro Ser Leu Gly Asp Ile Ser Val Gly Phe Val
            35                  40                  45

Lys Val Pro Ala Gln Gly Glu Lys His Gly Thr Ile Phe Gly Asn Ser
    50                  55                  60

Gly Gly Pro Gly Gly Asp Ala Tyr Ser Phe Phe Gly Ser Gln Ser Met
65                  70                  75                  80

Asn Trp Pro Glu Ala Met Tyr Gln Asn Tyr Asp Leu Val Ala Val Gln
                85                  90                  95

Pro Arg Gly Met Val Gly Ser Thr Pro Val Asn Cys Asp Asn Ile Ala
            100                 105                 110

Pro Gly Tyr Asp Phe Leu Ser Leu Leu Thr Arg Glu Gly Ala Phe Val
        115                 120                 125

Lys Glu Ser Cys Glu Ile Gly Thr Pro Gly Tyr Thr Ser Ser Leu Thr
    130                 135                 140

Thr Asp Asn Thr Ala Asn Asp Trp Glu Arg Val Arg Gln Ala Leu Gly
145                 150                 155                 160

Asp Asp Lys Ile Ser Ile Phe Gly Leu Ser Tyr Gly Thr Tyr Leu Gly
                165                 170                 175

Ser Val Tyr Ala Thr Arg Tyr Pro Gln His Thr Asp Lys Val Val Leu
            180                 185                 190

Asp Ser Ala Met Ala Pro Ser Leu Ala Trp Asn Gly Ile Met Ala Ser
        195                 200                 205

Gln Glu Gln Gly Tyr Lys Asn Ser Leu Asn Asp Phe Phe Thr Trp Val
    210                 215                 220

Ala Glu Asn Asn Asp Thr Tyr Gly Leu Gly Thr Thr Pro Leu Ala Val
225                 230                 235                 240

Tyr Gln Asn Trp Ser Asn Lys Ile Val Ala Glu Thr Gly Thr Asn Pro
                245                 250                 255

Thr Val Ala Pro Pro Pro Ala Gln Val Gly Asp Val Pro Pro Ala Phe
            260                 265                 270

Ala Trp Ala Gly Gln Ala Gly Ala Asp Met Met Thr Ala Thr Asn Pro
        275                 280                 285

Thr Ser Val Gln Leu Gln Gly Leu Ala Thr Gln Leu Leu Asn Pro Gly
    290                 295                 300

Ser Asn Gln Ser Leu Ser Pro Leu Leu Asn Val Thr Arg Ala Tyr Ile
305                 310                 315                 320

Pro Gln Pro Ser Thr Trp Pro Met Leu Ala Gly Ala Ile Ser Gly Gln
                325                 330                 335

Thr Pro Ile Pro Asp Val Thr Asp Thr Gly Asp Asp Pro Tyr Val Ile
            340                 345                 350

Glu Ser Ile Asn Ala Ser Val Asn Met Gln Arg Met Val Met Cys Asn
        355                 360                 365

Glu Asn Thr Val Ala Pro Asp Pro Val Ala Met Ala Arg Met Ala Trp
    370                 375                 380

Thr Ser Met Val Thr Gly Asp Val Phe Asp Ile Tyr Ser Val Lys Phe
385                 390                 395                 400

Ser Ser Gly Gln Ala Cys Ser Gly Ile Thr Pro Thr Ser Gly Arg Gln
                405                 410                 415

Pro Thr Asp Gly Ser Gln Leu Ala Val Gln Pro Leu Leu Leu Gln Gly
            420                 425                 430

Thr Ser Asp Pro Gln Thr Pro Tyr Trp Thr His Asn Glu Leu Ala Asp
        435                 440                 445

Ala Met Asn Ala His Val Val Thr Val Asn Gly Pro Gly His Gly Gln
    450                 455                 460

Ser Ile Gly Gly Thr Asn Gln Ala Ile Asn Asp Ile Val Val Asp Tyr
465                 470                 475                 480

Leu Arg Thr Gly His Thr Asp Ala Thr Trp Val Glu Gly Asn Thr Pro
                485                 490                 495

Thr Pro Ile Thr Ala Gly
            500


<210> 135
<211> 1114
<212> DNA
<213> Corynebacterium glutamicum

<220>
<221> CDS
<222> (101)..(1114)
<223> FRXA00977

<400> 135
gaaaacaaac gtccttgaag ccgtaatgcc ccgttcgaca ataaaaaggg tagtagcagt 60

tcttgccgcc tcgactgcgc ttagcccctt tttggtatca atg ccc act gca gca   115
                                            Met Pro Thr Ala Ala
                                            1               5

gcg caa gaa aac atc cgc tgg gaa gaa tgc cca cct cag gta gat att   163
Ala Gln Glu Asn Ile Arg Trp Glu Glu Cys Pro Pro Gln Val Asp Ile
                10                  15                  20

gcc tcc gct caa tgt ggc agc atc gac gtg ccc atg cac tat tct gat   211
Ala Ser Ala Gln Cys Gly Ser Ile Asp Val Pro Met His Tyr Ser Asp
            25                  30                  35

ccc tca ctt ggc gat atc agc gtg ggc ttt gtc aag gtc cct gcc caa   259
Pro Ser Leu Gly Asp Ile Ser Val Gly Phe Val Lys Val Pro Ala Gln
        40                  45                  50

ggc gaa aag cac ggc acc atc ttc ggt aac tcc ggt ggc cct ggt ggc   307
Gly Glu Lys His Gly Thr Ile Phe Gly Asn Ser Gly Gly Pro Gly Gly
    55                  60                  65

gat gcc tat agc ttc ttc ggc agc caa tcc atg aac tgg cca gaa gcc   355
Asp Ala Tyr Ser Phe Phe Gly Ser Gln Ser Met Asn Trp Pro Glu Ala
70                  75                  80                  85

atg tac caa aac tac gac ctc gtt gca gtg cag cct cgc gga atg gtc   403
Met Tyr Gln Asn Tyr Asp Leu Val Ala Val Gln Pro Arg Gly Met Val
                90                  95                  100

ggc tcc aca ccg gtt aac tgc gac aac atc gca cca gga tac gat ttc   451
Gly Ser Thr Pro Val Asn Cys Asp Asn Ile Ala Pro Gly Tyr Asp Phe
            105                 110                 115

ctc tcg ctg ctc acc cgc gaa ggc gct ttc gtt aaa gaa tcc tgc gag   499
Leu Ser Leu Leu Thr Arg Glu Gly Ala Phe Val Lys Glu Ser Cys Glu
        120                 125                 130

atc ggc acc ccc ggc tac acc tcc agc ctg acc acc gac aac acc gcc   547
Ile Gly Thr Pro Gly Tyr Thr Ser Ser Leu Thr Thr Asp Asn Thr Ala
    135                 140                 145

aac gac tgg gag cgc gtc cgc caa gca ctt ggc gat gac aag atc tcc   595
Asn Asp Trp Glu Arg Val Arg Gln Ala Leu Gly Asp Asp Lys Ile Ser
150                 155                 160                 165

atc ttc gga ctg tcc tac gga acc tac ctc gga tcg gtc tac gcc acc   643
Ile Phe Gly Leu Ser Tyr Gly Thr Tyr Leu Gly Ser Val Tyr Ala Thr
                170                 175                 180

cgc tac cca cag cac acc gac aag gtt gtc ctc gat tcc gca atg gcg     691
Arg Tyr Pro Gln His Thr Asp Lys Val Val Leu Asp Ser Ala Met Ala
            185                 190                 195

ccc agc ctg gca tgg aac ggc atc atg gcc tcc caa gaa cag ggc tac     739
Pro Ser Leu Ala Trp Asn Gly Ile Met Ala Ser Gln Glu Gln Gly Tyr
        200                 205                 210

aaa aac tcc ctc aac gac ttc ttc acc tgg gtt gca gaa aac aac gac     787
Lys Asn Ser Leu Asn Asp Phe Phe Thr Trp Val Ala Glu Asn Asn Asp
    215                 220                 225

acg tat ggc ctc ggc act acc cca cta gcc gtg tac caa aac tgg tca     835
Thr Tyr Gly Leu Gly Thr Thr Pro Leu Ala Val Tyr Gln Asn Trp Ser
230                 235                 240                 245

aac aag atc gtc gcc gaa acc gga acc aac cca acc gtt gct cca cca     883
Asn Lys Ile Val Ala Glu Thr Gly Thr Asn Pro Thr Val Ala Pro Pro
                250                 255                 260

cca gca caa gtt ggc gat gtc cca cca gca ttc gca tgg gcc ggc caa     931
Pro Ala Gln Val Gly Asp Val Pro Pro Ala Phe Ala Trp Ala Gly Gln
            265                 270                 275

gca ggc gca gac atg atg acc gcc acc aac cca acc tcc gtg caa ctc     979
Ala Gly Ala Asp Met Met Thr Ala Thr Asn Pro Thr Ser Val Gln Leu
        280                 285                 290

cag ggc ctt gcc acc cag ctc cta aac cct gga tcc aac cag tca ctg     1027
Gln Gly Leu Ala Thr Gln Leu Leu Asn Pro Gly Ser Asn Gln Ser Leu
    295                 300                 305

agc cct ctg ctc aac gtc acc cgc gcc tac att cca cag cca tca acc     1075
Ser Pro Leu Leu Asn Val Thr Arg Ala Tyr Ile Pro Gln Pro Ser Thr
310                 315                 320                 325

tgg ccc atg ctc gca ggc gcc atc tca ggg caa aca ccc                 1114
Trp Pro Met Leu Ala Gly Ala Ile Ser Gly Gln Thr Pro
                330                 335


<210> 136
<211> 338
<212> PRT
<213> Corynebacterium glutamicum

<400> 136
Met Pro Thr Ala Ala Ala Gln Glu Asn Ile Arg Trp Glu Glu Cys Pro
1               5                   10                  15

Pro Gln Val Asp Ile Ala Ser Ala Gln Cys Gly Ser Ile Asp Val Pro
            20                  25                  30

Met His Tyr Ser Asp Pro Ser Leu Gly Asp Ile Ser Val Gly Phe Val
        35                  40                  45

Lys Val Pro Ala Gln Gly Glu Lys His Gly Thr Ile Phe Gly Asn Ser
    50                  55                  60

Gly Gly Pro Gly Gly Asp Ala Tyr Ser Phe Phe Gly Ser Gln Ser Met
65                  70                  75                  80

Asn Trp Pro Glu Ala Met Tyr Gln Asn Tyr Asp Leu Val Ala Val Gln
                85                  90                  95

Pro Arg Gly Met Val Gly Ser Thr Pro Val Asn Cys Asp Asn Ile Ala
            100                 105                 110

Pro Gly Tyr Asp Phe Leu Ser Leu Leu Thr Arg Glu Gly Ala Phe Val
        115                 120                 125

Lys Glu Ser Cys Glu Ile Gly Thr Pro Gly Tyr Thr Ser Ser Leu Thr
    130                 135                 140

Thr Asp Asn Thr Ala Asn Asp Trp Glu Arg Val Arg Gln Ala Leu Gly
145                 150                 155                 160

Asp Asp Lys Ile Ser Ile Phe Gly Leu Ser Tyr Gly Thr Tyr Leu Gly
                165                 170                 175

Ser Val Tyr Ala Thr Arg Tyr Pro Gln His Thr Asp Lys Val Val Leu
            180                 185                 190

Asp Ser Ala Met Ala Pro Ser Leu Ala Trp Asn Gly Ile Met Ala Ser
        195                 200                 205

Gln Glu Gln Gly Tyr Lys Asn Ser Leu Asn Asp Phe Phe Thr Trp Val
    210                 215                 220

Ala Glu Asn Asn Asp Thr Tyr Gly Leu Gly Thr Thr Pro Leu Ala Val
225                 230                 235                 240

Tyr Gln Asn Trp Ser Asn Lys Ile Val Ala Glu Thr Gly Thr Asn Pro
                245                 250                 255

Thr Val Ala Pro Pro Pro Ala Gln Val Gly Asp Val Pro Pro Ala Phe
            260                 265                 270

Ala Trp Ala Gly Gln Ala Gly Ala Asp Met Met Thr Ala Thr Asn Pro
        275                 280                 285

Thr Ser Val Gln Leu Gln Gly Leu Ala Thr Gln Leu Leu Asn Pro Gly
    290                 295                 300

Ser Asn Gln Ser Leu Ser Pro Leu Leu Asn Val Thr Arg Ala Tyr Ile
305                 310                 315                 320

Pro Gln Pro Ser Thr Trp Pro Met Leu Ala Gly Ala Ile Ser Gly Gln
                325                 330                 335

Thr Pro



<210> 137
<211> 269
<212> DNA
<213> Corynebacterium glutamicum

<220>
<221> CDS
<222> (1)..(246)
<223> FRXA00982

<400> 137
tct caa cta gca gtc caa cca cta ctc ctc cag gga acc agc gac cca      48
Ser Gln Leu Ala Val Gln Pro Leu Leu Leu Gln Gly Thr Ser Asp Pro
  1               5                  10                  15

caa acc cca tac tgg acc cac aac gag ctt gcc gac gcc atg aac gcc      96
Gln Thr Pro Tyr Trp Thr His Asn Glu Leu Ala Asp Ala Met Asn Ala
             20                  25                  30

cac gtg gtc acc gtc aac gga cca gga cac ggc caa tcc atc ggc ggc      144
His Val Val Thr Val Asn Gly Pro Gly His Gly Gln Ser Ile Gly Gly
         35                  40                  45

acc aac caa gca atc aac gac att gtt gtg gac tac ctc cgc acc gga      192
Thr Asn Gln Ala Ile Asn Asp Ile Val Val Asp Tyr Leu Arg Thr Gly
     50                  55                  60

cac acc gac gcc acc tgg gtc gaa ggc aac aca ccc acc cca att acg      240
His Thr Asp Ala Thr Trp Val Glu Gly Asn Thr Pro Thr Pro Ile Thr
 65                  70                  75                  80

gct ggc taattgcttt ccacttagta gat                                    269
Ala Gly



<210> 138
<211> 82
<212> PRT

<213> Corynebacterium glutamicum

<400> 138
Ser Gln Leu Ala Val Gln Pro Leu Leu Leu Gln Gly Thr Ser Asp Pro
1               5                   10                  15

Gln Thr Pro Tyr Trp Thr His Asn Glu Leu Ala Asp Ala Met Asn Ala
            20                  25                  30

His Val Val Thr Val Asn Gly Pro Gly His Gly Gln Ser Ile Gly Gly
        35                  40                  45

Thr Asn Gln Ala Ile Asn Asp Ile Val Val Asp Tyr Leu Arg Thr Gly
    50                  55                  60

His Thr Asp Ala Thr Trp Val Glu Gly Asn Thr Pro Thr Pro Ile Thr
65                  70                  75                  80

Ala Gly

<210> 139
<211> 1419
<212> DNA
<213> Corynebacterium glutamicum

<220>
<221> CDS
<222> (101)..(1396)
<223> RXA00152

<400> 139
gtcattgata tccaaggcac gaccgcgatt gtatggaaag aagcctaaat ttttaacaat 60

caaatagtac tggccattcc caactaaaac tggagtaacg atg aca gga cta atc 115
                                          Met Thr Gly Leu Ile
                                          1               5

ctc gcc ata gtt ttc ctg gtc ttt gtc gcc gtc gtg gtg atc aag tcc 163
Leu Ala Ile Val Phe Leu Val Phe Val Ala Val Val Val Ile Lys Ser
                10                  15                  20

ata gcc ctg att ccc cag ggt gaa gcc gcc gtc att gaa cgc ctt ggt 211
Ile Ala Leu Ile Pro Gln Gly Glu Ala Ala Val Ile Glu Arg Leu Gly
            25                  30                  35

agc tac acc cgc acc gtt tca ggt ggc ctg acc ctg ctg gtt cca ttc 259
Ser Tyr Thr Arg Thr Val Ser Gly Gly Leu Thr Leu Leu Val Pro Phe
        40                  45                  50

gtg gac cga gta cgc gca agg atc gac acc cgt gag cgc gtg gtc tca 307
Val Asp Arg Val Arg Ala Arg Ile Asp Thr Arg Glu Arg Val Val Ser
    55                  60                  65

ttc cca ccg cag gct gtt att acc caa gac aac ctg acc gtg gcc atc 355
Phe Pro Pro Gln Ala Val Ile Thr Gln Asp Asn Leu Thr Val Ala Ile
70                  75                  80                  85

gat atc gtg gtg acc ttc caa atc aac gaa cca gag cgc gcc atc tac 403
Asp Ile Val Val Thr Phe Gln Ile Asn Glu Pro Glu Arg Ala Ile Tyr
                90                  95                  100

ggc gtg gac aac tac atc gtc ggt gtg gag cag att tct gta gca aca 451
Gly Val Asp Asn Tyr Ile Val Gly Val Glu Gln Ile Ser Val Ala Thr
            105                 110                 115

ctt cga gac gtt gtc ggt ggc atg acc ctg gaa gaa acc ctc act tca 499
Leu Arg Asp Val Val Gly Gly Met Thr Leu Glu Glu Thr Leu Thr Ser
        120                 125                 130

cgt gac gtg atc aac cgc cgc ctc cgt ggc gag ctc gat gca gca acc 547
Arg Asp Val Ile Asn Arg Arg Leu Arg Gly Glu Leu Asp Ala Ala Thr
    135                 140                 145

acc aaa tgg ggc ctg cgc atc agc cgt gtg gaa cta aag gca att gat 595
Thr Lys Trp Gly Leu Arg Ile Ser Arg Val Glu Leu Lys Ala Ile Asp

150                 155                 160                 165

ccg cca cca tcc atc cag caa tcg atg gaa aag cag atg aag gca gac     643
Pro Pro Pro Ser Ile Gln Gln Ser Met Glu Lys Gln Met Lys Ala Asp
                170                 175                 180

cgt gaa aag cgc gcc acc att ttg acc gca gaa ggt cag cgc gaa gcc     691
Arg Glu Lys Arg Ala Thr Ile Leu Thr Ala Glu Gly Gln Arg Glu Ala
            185                 190                 195

gac atc aaa act gcc gaa ggt gaa aag caa gcc aag atc ctc caa gct     739
Asp Ile Lys Thr Ala Glu Gly Glu Lys Gln Ala Lys Ile Leu Gln Ala
        200                 205                 210

gag ggt gaa aag cac gca tcc atc ctg aac gca gaa gca gaa cgc caa     787
Glu Gly Glu Lys His Ala Ser Ile Leu Asn Ala Glu Ala Glu Arg Gln
    215                 220                 225

gcg atg atc ctg cgc gcc gaa ggt gaa cgc gca gca cgc tac ctc cag     835
Ala Met Ile Leu Arg Ala Glu Gly Glu Arg Ala Ala Arg Tyr Leu Gln
230                 235                 240                 245

gcg cag ggt gaa gcc cga gca atc caa aag gtc aac gca gca atc aag     883
Ala Gln Gly Glu Ala Arg Ala Ile Gln Lys Val Asn Ala Ala Ile Lys
                250                 255                 260

tct gcc aag ttg acc cca gag gtt ctt gct tat caa tac ctc gaa aag     931
Ser Ala Lys Leu Thr Pro Glu Val Leu Ala Tyr Gln Tyr Leu Glu Lys
            265                 270                 275

ctt cct aag atc gca gag ggc aac gcc tcc aag atg tgg gtc atc cca     979
Leu Pro Lys Ile Ala Glu Gly Asn Ala Ser Lys Met Trp Val Ile Pro
        280                 285                 290

agc cag ttc tcc gat tct ctg gaa ggt ttt gcg aag cag ttc ggc gca     1027
Ser Gln Phe Ser Asp Ser Leu Glu Gly Phe Ala Lys Gln Phe Gly Ala
    295                 300                 305

aag gat gca gaa ggt gtc ttc cgc tac gaa cca aac acc gtg gat gaa     1075
Lys Asp Ala Glu Gly Val Phe Arg Tyr Glu Pro Asn Thr Val Asp Glu
310                 315                 320                 325

gaa acc cgc gac atc gca aac gcc gac aac gtg gaa gac tgg ttc tcc     1123
Glu Thr Arg Asp Ile Ala Asn Ala Asp Asn Val Glu Asp Trp Phe Ser
                330                 335                 340

acc gaa tca gac cct gaa atc gca gca gca gtc gcc gca gca aac gcc     1171
Thr Glu Ser Asp Pro Glu Ile Ala Ala Ala Val Ala Ala Ala Asn Ala
            345                 350                 355

gtg gcc aac aag cca gtc gat cca gaa ccc ggt gag atc ctt tcc aag     1219
Val Ala Asn Lys Pro Val Asp Pro Glu Pro Gly Glu Ile Leu Ser Lys
        360                 365                 370

aag acc gca cga cgc gtt gaa cct gaa gca gta ttg gag gct ttg caa     1267
Lys Thr Ala Arg Arg Val Glu Pro Glu Ala Val Leu Glu Ala Leu Gln
    375                 380                 385

aac gga acc act aca caa cct gag gtt gag gca gca cct cct acc gca     1315
Asn Gly Thr Thr Thr Gln Pro Glu Val Glu Ala Ala Pro Pro Thr Ala
390                 395                 400                 405

aac ttc gcc caa gaa ttc cct gca cca cag gca aac cct gaa gat tac     1363
Asn Phe Ala Gln Glu Phe Pro Ala Pro Gln Ala Asn Pro Glu Asp Tyr
                410                 415                 420

tcc gac caa cac cga gag aat cct tac gga aac taatcaggca taagaaaagg 1416
Ser Asp Gln His Arg Glu Asn Pro Tyr Gly Asn
            425                 430

cgg                                                                 1419

<210> 140
<211> 432
<212> PRT
<213> Corynebacterium glutamicum

<400> 140
Met Thr Gly Leu Ile Leu Ala Ile Val Phe Leu Val Phe Val Ala Val
1               5                   10                  15

Val Val Ile Lys Ser Ile Ala Leu Ile Pro Gln Gly Glu Ala Ala Val
            20                  25                  30

Ile Glu Arg Leu Gly Ser Tyr Thr Arg Thr Val Ser Gly Gly Leu Thr
        35                  40                  45

Leu Leu Val Pro Phe Val Asp Arg Val Arg Ala Arg Ile Asp Thr Arg
    50                  55                  60

Glu Arg Val Val Ser Phe Pro Pro Gln Ala Val Ile Thr Gln Asp Asn
65                  70                  75                  80

Leu Thr Val Ala Ile Asp Ile Val Val Thr Phe Gln Ile Asn Glu Pro
                85                  90                  95

Glu Arg Ala Ile Tyr Gly Val Asp Asn Tyr Ile Val Gly Val Glu Gln
            100                 105                 110

Ile Ser Val Ala Thr Leu Arg Asp Val Val Gly Gly Met Thr Leu Glu
        115                 120                 125

Glu Thr Leu Thr Ser Arg Asp Val Ile Asn Arg Arg Leu Arg Gly Glu
    130                 135                 140

Leu Asp Ala Ala Thr Thr Lys Trp Gly Leu Arg Ile Ser Arg Val Glu
145                 150                 155                 160

Leu Lys Ala Ile Asp Pro Pro Pro Ser Ile Gln Gln Ser Met Glu Lys
                165                 170                 175

Gln Met Lys Ala Asp Arg Glu Lys Arg Ala Thr Ile Leu Thr Ala Glu
            180                 185                 190

Gly Gln Arg Glu Ala Asp Ile Lys Thr Ala Glu Gly Glu Lys Gln Ala
        195                 200                 205

Lys Ile Leu Gln Ala Glu Gly Glu Lys His Ala Ser Ile Leu Asn Ala
    210                 215                 220

Glu Ala Glu Arg Gln Ala Met Ile Leu Arg Ala Glu Gly Glu Arg Ala
225                 230                 235                 240

Ala Arg Tyr Leu Gln Ala Gln Gly Glu Ala Arg Ala Ile Gln Lys Val
                245                 250                 255

Asn Ala Ala Ile Lys Ser Ala Lys Leu Thr Pro Glu Val Leu Ala Tyr
            260                 265                 270

Gln Tyr Leu Glu Lys Leu Pro Lys Ile Ala Glu Gly Asn Ala Ser Lys
        275                 280                 285

Met Trp Val Ile Pro Ser Gln Phe Ser Asp Ser Leu Glu Gly Phe Ala
    290                 295                 300

Lys Gln Phe Gly Ala Lys Asp Ala Glu Gly Val Phe Arg Tyr Glu Pro
305                 310                 315                 320

Asn Thr Val Asp Glu Glu Thr Arg Asp Ile Ala Asn Ala Asp Asn Val
                325                 330                 335

Glu Asp Trp Phe Ser Thr Glu Ser Asp Pro Glu Ile Ala Ala Ala Val
            340                 345                 350

Ala Ala Ala Asn Ala Val Ala Asn Lys Pro Val Asp Pro Glu Pro Gly
        355                 360                 365

Glu Ile Leu Ser Lys Lys Thr Ala Arg Arg Val Glu Pro Glu Ala Val
    370                 375                 380

Leu Glu Ala Leu Gln Asn Gly Thr Thr Thr Gln Pro Glu Val Glu Ala
385                 390                 395                 400

Ala Pro Pro Thr Ala Asn Phe Ala Gln Glu Phe Pro Ala Pro Gln Ala
                405                 410                 415

Asn Pro Glu Asp Tyr Ser Asp Gln His Arg Glu Asn Pro Tyr Gly Asn
            420                 425                 430

<210> 141
<211> 1098
<212> DNA
<213> Corynebacterium glutamicum

<220>
<221> CDS
<222> (101)..(1075)
<223> RXA02558

<400> 141
ccctcccgaa attagaaatc cccttttaat gtgatatcac tttctgttaa actgatatca 60

cattcttttt cagcacccca gacttaaaag gagcaccacc atg agc acc ata gaa   115
                                           Met Ser Thr Ile Glu
                                           1               5

gag cgc act cct gga gct gtc gcc aca gaa cca gtg gga cac gaa ggc   163
Glu Arg Thr Pro Gly Ala Val Ala Thr Glu Pro Val Gly His Glu Gly
                10                  15                  20

gca cgc gtc agc att aat gag aag aac gtg tgg tct ttg ggc gca ggt   211
Ala Arg Val Ser Ile Asn Glu Lys Asn Val Trp Ser Leu Gly Ala Gly
            25                  30                  35

cca gca gct ttc gca ctg ctc gca atg att gtg ctc atg att gcc agt   259
Pro Ala Ala Phe Ala Leu Leu Ala Met Ile Val Leu Met Ile Ala Ser
        40                  45                  50

gga gtt ttc ttc gct caa tcc atc aac act tta gaa aac gat ggc ggt   307
Gly Val Phe Phe Ala Gln Ser Ile Asn Thr Leu Glu Asn Asp Gly Gly
    55                  60                  65

gga aca ctt gcg gtt acg gga ctg att gcc agc atc gtc gtt ttc act   355
Gly Thr Leu Ala Val Thr Gly Leu Ile Ala Ser Ile Val Val Phe Thr
70                  75                  80                  85

gtt gca ttg gtg gtc acc ata act tcg gtg aag gtg gtc agc cct gga   403
Val Ala Leu Val Val Thr Ile Thr Ser Val Lys Val Val Ser Pro Gly
                90                  95                  100

cat act ctg act gtg cag ttc ttt gga cga tac atc gga acc ctg cgt   451
His Thr Leu Thr Val Gln Phe Phe Gly Arg Tyr Ile Gly Thr Leu Arg
            105                 110                 115

cga act ggg ttg tct ttc gtt ccc cca ctg tct gtg acg aag aaa gtg   499
Arg Thr Gly Leu Ser Phe Val Pro Pro Leu Ser Val Thr Lys Lys Val
        120                 125                 130

tcc gtg agg gtc cga aac ttt gaa acc aac gaa gcc aaa gtt aat gac   547
Ser Val Arg Val Arg Asn Phe Glu Thr Asn Glu Ala Lys Val Asn Asp
    135                 140                 145

tac aac ggc aac ccc atc aac att gca gcg atc atc gtg tgg cag gta   595
Tyr Asn Gly Asn Pro Ile Asn Ile Ala Ala Ile Ile Val Trp Gln Val
150                 155                 160                 165

gcc gat act gca cag gct agc ttc tct gtg gag gat ttc gaa gag ttc   643
Ala Asp Thr Ala Gln Ala Ser Phe Ser Val Glu Asp Phe Glu Glu Phe
                170                 175                 180

ctg cac cag cag gcc gag tcc gca ctg cgt cac gtg gca acc cag cac   691
Leu His Gln Gln Ala Glu Ser Ala Leu Arg His Val Ala Thr Gln His
            185                 190                 195

ccc tat gat tcc cca gtt gat ggt cgt gtt tcc ttg cgt ggc gct acc   739
Pro Tyr Asp Ser Pro Val Asp Gly Arg Val Ser Leu Arg Gly Ala Thr

200                 205                 210

gat gag gtc agt gaa gaa ctc gca gat gag gtg gca caa cga gca gct     787
Asp Glu Val Ser Glu Glu Leu Ala Asp Glu Val Ala Gln Arg Ala Ala
    215                 220                 225

gtt gca ggt ctt gaa atc gtc gaa gcc cgc atc tct tcc ttg agc tac     835
Val Ala Gly Leu Glu Ile Val Glu Ala Arg Ile Ser Ser Leu Ser Tyr
230                 235                 240                 245

gca ccg gaa att gcc cag gcg atg ctg cag cgc cag cag gct tcc gcg     883
Ala Pro Glu Ile Ala Gln Ala Met Leu Gln Arg Gln Gln Ala Ser Ala
                250                 255                 260

att gtt gat gcc cgc gaa aag atc gtc gag ggc gct gtc acc atg gtg     931
Ile Val Asp Ala Arg Glu Lys Ile Val Glu Gly Ala Val Thr Met Val
            265                 270                 275

gaa acc gca ctt gac cag ctt gag caa cgt gaa att gtg gat ttg gat     979
Glu Thr Ala Leu Asp Gln Leu Glu Gln Arg Glu Ile Val Asp Leu Asp
        280                 285                 290

cca gag cga cgc gcc gcg atg gtt tcc aac ctg ttg gtt gtg ttg tgt     1027
Pro Glu Arg Arg Ala Ala Met Val Ser Asn Leu Leu Val Val Leu Cys
    295                 300                 305

tcc gac acc aat gct cag cca atc gtc aac gcc ggt agc ctc tac caa     1075
Ser Asp Thr Asn Ala Gln Pro Ile Val Asn Ala Gly Ser Leu Tyr Gln
310                 315                 320                 325

taagacaatg gcccgcaaac agg                                           1098


<210> 142
<211> 325
<212> PRT
<213> Corynebacterium glutamicum

<400> 142
Met Ser Thr Ile Glu Glu Arg Thr Pro Gly Ala Val Ala Thr Glu Pro
1               5                   10                  15

Val Gly His Glu Gly Ala Arg Val Ser Ile Asn Glu Lys Asn Val Trp
            20                  25                  30

Ser Leu Gly Ala Gly Pro Ala Ala Phe Ala Leu Leu Ala Met Ile Val
        35                  40                  45

Leu Met Ile Ala Ser Gly Val Phe Phe Ala Gln Ser Ile Asn Thr Leu
    50                  55                  60

Glu Asn Asp Gly Gly Gly Thr Leu Ala Val Thr Gly Leu Ile Ala Ser
65                  70                  75                  80

Ile Val Val Phe Thr Val Ala Leu Val Val Thr Ile Thr Ser Val Lys
                85                  90                  95

Val Val Ser Pro Gly His Thr Leu Thr Val Gln Phe Phe Gly Arg Tyr
            100                 105                 110

Ile Gly Thr Leu Arg Arg Thr Gly Leu Ser Phe Val Pro Pro Leu Ser
        115                 120                 125

Val Thr Lys Lys Val Ser Val Arg Val Arg Asn Phe Glu Thr Asn Glu
    130                 135                 140

Ala Lys Val Asn Asp Tyr Asn Gly Asn Pro Ile Asn Ile Ala Ala Ile
145                 150                 155                 160

Ile Val Trp Gln Val Ala Asp Thr Ala Gln Ala Ser Phe Ser Val Glu
                165                 170                 175

Asp Phe Glu Glu Phe Leu His Gln Gln Ala Glu Ser Ala Leu Arg His
            180                 185                 190

Val Ala Thr Gln His Pro Tyr Asp Ser Pro Val Asp Gly Arg Val Ser
        195                 200                 205

Leu Arg Gly Ala Thr Asp Glu Val Ser Glu Glu Leu Ala Asp Glu Val
    210                 215                 220

Ala Gln Arg Ala Ala Val Ala Gly Leu Glu Ile Val Glu Ala Arg Ile
225                 230                 235                 240

Ser Ser Leu Ser Tyr Ala Pro Glu Ile Ala Gln Ala Met Leu Gln Arg
                245                 250                 255

Gln Gln Ala Ser Ala Ile Val Asp Ala Arg Glu Lys Ile Val Glu Gly
            260                 265                 270

Ala Val Thr Met Val Glu Thr Ala Leu Asp Gln Leu Glu Gln Arg Glu
        275                 280                 285

Ile Val Asp Leu Asp Pro Glu Arg Arg Ala Ala Met Val Ser Asn Leu
    290                 295                 300

Leu Val Val Leu Cys Ser Asp Thr Asn Ala Gln Pro Ile Val Asn Ala
305                 310                 315                 320

Gly Ser Leu Tyr Gln
                325


<210> 143
<211> 798
<212> DNA
<213> Corynebacterium glutamicum

<220>
<221> CDS
<222> (101)..(775)
<223> RXA00500

<400> 143
caccagccag catgaacaca atggacttcg tgccagctca ggcgcagtac tgaagcacct 60

tttcgatctg gcccacggcc gagaggtacg ctgattcctc gtg tta gta cta gcc    115
                                          Val Leu Val Leu Ala
                                          1               5

cta gac acc tca acc cct gac ctg atc gtc ggc gtc gtc gac tcc gac    163
Leu Asp Thr Ser Thr Pro Asp Leu Ile Val Gly Val Val Asp Ser Asp
                10                  15                  20

acc gga aac acc cgc gcc gaa acc atc atc gag gac acc cgc gca cac    211
Thr Gly Asn Thr Arg Ala Glu Thr Ile Ile Glu Asp Thr Arg Ala His
            25                  30                  35

aac gag cag ctc acg ccc acc gtc cag aag acg ctt ctc gac gcc aac    259
Asn Glu Gln Leu Thr Pro Thr Val Gln Lys Thr Leu Leu Asp Ala Asn
        40                  45                  50

ttg agc ttt tca gat atc gac gcg atc gtc gtg ggt tgc ggc ccg gga    307
Leu Ser Phe Ser Asp Ile Asp Ala Ile Val Val Gly Cys Gly Pro Gly
    55                  60                  65

ccg ttc act gga ctt cga gta ggc atg gtg tcc ggc gca gcg ttc ggt    355
Pro Phe Thr Gly Leu Arg Val Gly Met Val Ser Gly Ala Ala Phe Gly
70                  75                  80                  85

gat gcc ctg gga atc cct gtc tat gga gtc tgc tca ctc gac gcg atc    403
Asp Ala Leu Gly Ile Pro Val Tyr Gly Val Cys Ser Leu Asp Ala Ile
                90                  95                  100

gct cac aat att ggt gca cgc aac atc ccg cac gca tta gtt gcc act    451
Ala His Asn Ile Gly Ala Arg Asn Ile Pro His Ala Leu Val Ala Thr
            105                 110                 115

gat gcg cgc cgc cgt gaa atc tac tgg gca acc tac cgc tcc ggc gaa    499
Asp Ala Arg Arg Arg Glu Ile Tyr Trp Ala Thr Tyr Arg Ser Gly Glu
        120                 125                 130

cgt gat cag gga cca gat gtc atc gca cca gca aac atc cag atc agc    547
Arg Asp Gln Gly Pro Asp Val Ile Ala Pro Ala Asn Ile Gln Ile Ser

135                 140                 145

ggc gct gta gac acc att tcg att cct gag cac ctg gtg gaa aaa ctc      595
Gly Ala Val Asp Thr Ile Ser Ile Pro Glu His Leu Val Glu Lys Leu
150                 155                 160                 165

cca gaa gaa ctc cag aat gtc acc atg cat agc ggc aaa cct gcc ccc      643
Pro Glu Glu Leu Gln Asn Val Thr Met His Ser Gly Lys Pro Ala Pro
                170                 175                 180

gca agc ttg gtg gca gtg gct gat ttc agt gtg gaa cca caa cca ttg      691
Ala Ser Leu Val Ala Val Ala Asp Phe Ser Val Glu Pro Gln Pro Leu
            185                 190                 195

gtt cct ctt tac ctg cgc cgc cca gat gcc aaa gaa cca aaa cca aaa      739
Val Pro Leu Tyr Leu Arg Arg Pro Asp Ala Lys Glu Pro Lys Pro Lys
        200                 205                 210

cct aaa tct gca gcc atc ccc gag gtg gat ctt tca tgagtgaaca           785
Pro Lys Ser Ala Ala Ile Pro Glu Val Asp Leu Ser
    215                 220                 225

attcgagcta cgg                                                       798


<210> 144
<211> 225
<212> PRT
<213> Corynebacterium glutamicum

<400> 144
Val Leu Val Leu Ala Leu Asp Thr Ser Thr Pro Asp Leu Ile Val Gly
  1               5                  10                  15

Val Val Asp Ser Asp Thr Gly Asn Thr Arg Ala Glu Thr Ile Ile Glu
             20                  25                  30

Asp Thr Arg Ala His Asn Glu Gln Leu Thr Pro Thr Val Gln Lys Thr
         35                  40                  45

Leu Leu Asp Ala Asn Leu Ser Phe Ser Asp Ile Asp Ala Ile Val Val
     50                  55                  60

Gly Cys Gly Pro Gly Pro Phe Thr Gly Leu Arg Val Gly Met Val Ser
 65                  70                  75                  80

Gly Ala Ala Phe Gly Asp Ala Leu Gly Ile Pro Val Tyr Gly Val Cys
                 85                  90                  95

Ser Leu Asp Ala Ile Ala His Asn Ile Gly Ala Arg Asn Ile Pro His
            100                 105                 110

Ala Leu Val Ala Thr Asp Ala Arg Arg Arg Glu Ile Tyr Trp Ala Thr
        115                 120                 125

Tyr Arg Ser Gly Glu Arg Asp Gln Gly Pro Asp Val Ile Ala Pro Ala
    130                 135                 140

Asn Ile Gln Ile Ser Gly Ala Val Asp Thr Ile Ser Ile Pro Glu His
145                 150                 155                 160

Leu Val Glu Lys Leu Pro Glu Glu Leu Gln Asn Val Thr Met His Ser
                165                 170                 175

Gly Lys Pro Ala Pro Ala Ser Leu Val Ala Val Ala Asp Phe Ser Val
            180                 185                 190

Glu Pro Gln Pro Leu Val Pro Leu Tyr Leu Arg Arg Pro Asp Ala Lys
        195                 200                 205

Glu Pro Lys Pro Lys Pro Lys Ser Ala Ala Ile Pro Glu Val Asp Leu
    210                 215                 220

Ser
225

<210> 145
<211> 630
<212> DNA
<213> Corynebacterium glutamicum

<220>
<221> CDS
<222> (101)..(607)
<223> RXA00501

<400> 145
tggaaccaca accattggtt cctctttacc tgcgccgccc agatgccaaa gaaccaaaac 60

caaaacctaa atctgcagcc atccccgagg tggatctttc atg agt gaa caa ttc 115
Met Ser Glu Gln Phe
1               5

gag cta cgg gaa ctc cgc agg gaa gac gcg ggg cgc tgc gcc gac ctg 163
Glu Leu Arg Glu Leu Arg Arg Glu Asp Ala Gly Arg Cys Ala Asp Leu
                10                  15                  20

gag caa atc ctg ttc cca ggt gat aac ccc tgg cca cgt gat gtc ttt 211
Glu Gln Ile Leu Phe Pro Gly Asp Asn Pro Trp Pro Arg Asp Val Phe
            25                  30                  35

gcc gtg gag ttt tcc cac ccc acc aat ttc tac atc ggc gct ttc gac 259
Ala Val Glu Phe Ser His Pro Thr Asn Phe Tyr Ile Gly Ala Phe Asp
        40                  45                  50

gaa gga tac ttg gtg gcg tac gca ggt ctt gcc atg atg gga cct gcg 307
Glu Gly Tyr Leu Val Ala Tyr Ala Gly Leu Ala Met Met Gly Pro Ala
    55                  60                  65

gat gat cca gag ttt gaa atc cac acc att ggt gtc gat ccg gaa ttc 355
Asp Asp Pro Glu Phe Glu Ile His Thr Ile Gly Val Asp Pro Glu Phe
70                  75                  80                  85

caa aga aaa ggc ttg gga cgc gta ctc atg gat caa atg atg cat gca 403
Gln Arg Lys Gly Leu Gly Arg Val Leu Met Asp Gln Met Met His Ala
                90                  95                  100

gcg gac agc cac gac ggt cca gtt ttc ttg gaa gtc cgc acc gac aac 451
Ala Asp Ser His Asp Gly Pro Val Phe Leu Glu Val Arg Thr Asp Asn
            105                 110                 115

gta ccc gcg att tcc atg tac gag gct ttc ggc ttt aaa acc ttg gcc 499
Val Pro Ala Ile Ser Met Tyr Glu Ala Phe Gly Phe Lys Thr Leu Ala
        120                 125                 130

gtg cgc aaa aac tac tac cgg cca tcc gga gct gac gcc tac acc atg 547
Val Arg Lys Asn Tyr Tyr Arg Pro Ser Gly Ala Asp Ala Tyr Thr Met
    135                 140                 145

caa cgc cca cgc ttg agc gat cgc aaa gat caa cag aca gac aca gag 595
Gln Arg Pro Arg Leu Ser Asp Arg Lys Asp Gln Gln Thr Asp Thr Glu
150                 155                 160                 165

ggg aca ccc agc taaaccatga tcgttttggg aat 630
Gly Thr Pro Ser

<210> 146
<211> 169
<212> PRT
<213> Corynebacterium glutamicum

<400> 146
Met Ser Glu Gln Phe Glu Leu Arg Glu Leu Arg Arg Glu Asp Ala Gly
1               5                   10                  15

Arg Cys Ala Asp Leu Glu Gln Ile Leu Phe Pro Gly Asp Asn Pro Trp
            20                  25                  30

Pro Arg Asp Val Phe Ala Val Glu Phe Ser His Pro Thr Asn Phe Tyr
        35                  40                  45

Ile Gly Ala Phe Asp Glu Gly Tyr Leu Val Ala Tyr Ala Gly Leu Ala
    50                  55                  60

Met Met Gly Pro Ala Asp Asp Pro Glu Phe Glu Ile His Thr Ile Gly
65                  70                  75                  80

Val Asp Pro Glu Phe Gln Arg Lys Gly Leu Gly Arg Val Leu Met Asp
                85                  90                  95

Gln Met Met His Ala Ala Asp Ser His Asp Gly Pro Val Phe Leu Glu
            100                 105                 110

Val Arg Thr Asp Asn Val Pro Ala Ile Ser Met Tyr Glu Ala Phe Gly
        115                 120                 125

Phe Lys Thr Leu Ala Val Arg Lys Asn Tyr Tyr Arg Pro Ser Gly Ala
    130                 135                 140

Asp Ala Tyr Thr Met Gln Arg Pro Arg Leu Ser Asp Arg Lys Asp Gln
145                 150                 155                 160

Gln Thr Asp Thr Glu Gly Thr Pro Ser
                165


<210> 147
<211> 1155
<212> DNA
<213> Corynebacterium glutamicum

<220>
<221> CDS
<222> (101)..(1132)
<223> RXA00502

<400> 147
ctaccggcca tccggagctg acgcctacac catgcaacgc ccacgcttga gcgatcgcaa 60

agatcaacag acagacacag aggggacacc cagctaaacc atg atc gtt ttg gga   115
                                            Met Ile Val Leu Gly
                                            1               5

att gaa agc tcc tgc gat gaa aca ggc gta ggc gta gtc aaa ctt gac   163
Ile Glu Ser Ser Cys Asp Glu Thr Gly Val Gly Val Val Lys Leu Asp
                10                  15                  20

ggc gaa gga aac cta gag atc ctc gcc gac tca gtg gcc tcc tcc atg   211
Gly Glu Gly Asn Leu Glu Ile Leu Ala Asp Ser Val Ala Ser Ser Met
            25                  30                  35

caa gaa cat gcc cgc ttt ggt ggc gtc gtg cca gaa atc gcc tcc cgg   259
Gln Glu His Ala Arg Phe Gly Gly Val Val Pro Glu Ile Ala Ser Arg
        40                  45                  50

gcg cac ctg gaa tct atg gtc ccc gtg atg cgt gaa gcg ttg agg cag   307
Ala His Leu Glu Ser Met Val Pro Val Met Arg Glu Ala Leu Arg Gln
    55                  60                  65

gcg ggc gtc gac agg cca gat gct gtg gct gca acc gtg ggc cct ggt   355
Ala Gly Val Asp Arg Pro Asp Ala Val Ala Ala Thr Val Gly Pro Gly
70                  75                  80                  85

ttg gcg ggc gcg ctg ctc gtt gga gcc agc gct gcg aag gcg tat gcc   403
Leu Ala Gly Ala Leu Leu Val Gly Ala Ser Ala Ala Lys Ala Tyr Ala
                90                  95                  100

gct gcg tgg gga gtt ccg ttt tac gcg gtc aac cac ctg ggc gga cac   451
Ala Ala Trp Gly Val Pro Phe Tyr Ala Val Asn His Leu Gly Gly His
            105                 110                 115

gtc gcc gtg gcc aat ctg gaa ggt gaa act ctt cca cac gcg gtg gct   499
Val Ala Val Ala Asn Leu Glu Gly Glu Thr Leu Pro His Ala Val Ala
        120                 125                 130

ttg ctg gtt tcc ggc gga cac act caa ttg ttg gaa gtc gac gcg gtg   547
Leu Leu Val Ser Gly Gly His Thr Gln Leu Leu Glu Val Asp Ala Val
    135                 140                 145

gga tta ccc atg aag gaa ttg gga tcc acc ctc gac gat gcc gct ggc     595
Gly Leu Pro Met Lys Glu Leu Gly Ser Thr Leu Asp Asp Ala Ala Gly
150                 155                 160                 165

gaa gcc tat gac aaa gtc tca agg ctg ttg gga ttg ggc tac cca ggc     643
Glu Ala Tyr Asp Lys Val Ser Arg Leu Leu Gly Leu Gly Tyr Pro Gly
                170                 175                 180

ggc ccc atc att gat aaa ttg gcg cgc cgg ggt aat cca gag gcc att     691
Gly Pro Ile Ile Asp Lys Leu Ala Arg Arg Gly Asn Pro Glu Ala Ile
            185                 190                 195

gct ttc ccc cgc gga ttg atg aaa aag tcg gat tct cgg cat gat ttc     739
Ala Phe Pro Arg Gly Leu Met Lys Lys Ser Asp Ser Arg His Asp Phe
        200                 205                 210

agc ttt tcc ggt ttg aaa acc tcc gtt gcc cgc tac gtg gaa gct gcg     787
Ser Phe Ser Gly Leu Lys Thr Ser Val Ala Arg Tyr Val Glu Ala Ala
    215                 220                 225

gaa aga aac ggt gaa gtt att tcc gtg gag gac gtc tgc gca tca ttc     835
Glu Arg Asn Gly Glu Val Ile Ser Val Glu Asp Val Cys Ala Ser Phe
230                 235                 240                 245

caa gaa gcg gtg tgt gat gtg ttg acg ttt aag gcc gtg cgt gcg tgc     883
Gln Glu Ala Val Cys Asp Val Leu Thr Phe Lys Ala Val Arg Ala Cys
                250                 255                 260

cgc gat gtc ggt gcg aag gtg ctg ctg ttg ggt gga gga gtg gct gcc     931
Arg Asp Val Gly Ala Lys Val Leu Leu Leu Gly Gly Gly Val Ala Ala
            265                 270                 275

aac tct cgt ctg cgg gag ctt gct caa gaa cgt tgc gat aaa gcc gac     979
Asn Ser Arg Leu Arg Glu Leu Ala Gln Glu Arg Cys Asp Lys Ala Asp
        280                 285                 290

atc gaa ctc cgg gtt cct cgt ttc aat ttg tgc acc gat aat ggt gtc     1027
Ile Glu Leu Arg Val Pro Arg Phe Asn Leu Cys Thr Asp Asn Gly Val
    295                 300                 305

atg att gca gcg ttg gcg gct caa aga atc cac gaa ggt gcc caa gaa     1075
Met Ile Ala Ala Leu Ala Ala Gln Arg Ile His Glu Gly Ala Gln Glu
310                 315                 320                 325

tca cca att tcg gtc gga act gat cct tct ttg tcc gtt gag acc cca     1123
Ser Pro Ile Ser Val Gly Thr Asp Pro Ser Leu Ser Val Glu Thr Pro
                330                 335                 340

cag gtg ttt taaacattta gtattagttc cat                               1155
Gln Val Phe

<210> 148
<211> 344
<212> PRT
<213> Corynebacterium glutamicum

<400> 148
Met Ile Val Leu Gly Ile Glu Ser Ser Cys Asp Glu Thr Gly Val Gly
1               5                   10                  15

Val Val Lys Leu Asp Gly Glu Gly Asn Leu Glu Ile Leu Ala Asp Ser
            20                  25                  30

Val Ala Ser Ser Met Gln Glu His Ala Arg Phe Gly Gly Val Val Pro
        35                  40                  45

Glu Ile Ala Ser Arg Ala His Leu Glu Ser Met Val Pro Val Met Arg
    50                  55                  60

Glu Ala Leu Arg Gln Ala Gly Val Asp Arg Pro Asp Ala Val Ala Ala
65                  70                  75                  80

Thr Val Gly Pro Gly Leu Ala Gly Ala Leu Leu Val Gly Ala Ser Ala
                85                  90                  95

Ala Lys Ala Tyr Ala Ala Ala Trp Gly Val Pro Phe Tyr Ala Val Asn
            100                 105                 110

His Leu Gly Gly His Val Ala Val Ala Asn Leu Glu Gly Glu Thr Leu
        115                 120                 125

Pro His Ala Val Ala Leu Leu Val Ser Gly Gly His Thr Gln Leu Leu
    130                 135                 140

Glu Val Asp Ala Val Gly Leu Pro Met Lys Glu Leu Gly Ser Thr Leu
145                 150                 155                 160

Asp Asp Ala Ala Gly Glu Ala Tyr Asp Lys Val Ser Arg Leu Leu Gly
                165                 170                 175

Leu Gly Tyr Pro Gly Gly Pro Ile Ile Asp Lys Leu Ala Arg Arg Gly
            180                 185                 190

Asn Pro Glu Ala Ile Ala Phe Pro Arg Gly Leu Met Lys Lys Ser Asp
        195                 200                 205

Ser Arg His Asp Phe Ser Phe Ser Gly Leu Lys Thr Ser Val Ala Arg
    210                 215                 220

Tyr Val Glu Ala Ala Glu Arg Asn Gly Glu Val Ile Ser Val Glu Asp
225                 230                 235                 240

Val Cys Ala Ser Phe Gln Glu Ala Val Cys Asp Val Leu Thr Phe Lys
                245                 250                 255

Ala Val Arg Ala Cys Arg Asp Val Gly Ala Lys Val Leu Leu Leu Gly
            260                 265                 270

Gly Gly Val Ala Ala Asn Ser Arg Leu Arg Glu Leu Ala Gln Glu Arg
        275                 280                 285

Cys Asp Lys Ala Asp Ile Glu Leu Arg Val Pro Arg Phe Asn Leu Cys
    290                 295                 300

Thr Asp Asn Gly Val Met Ile Ala Ala Leu Ala Ala Gln Arg Ile His
305                 310                 315                 320

Glu Gly Ala Gln Glu Ser Pro Ile Ser Val Gly Thr Asp Pro Ser Leu
                325                 330                 335

Ser Val Glu Thr Pro Gln Val Phe
            340


<210> 149
<211> 888
<212> DNA
<213> Corynebacterium glutamicum

<220>
<221> CDS
<222> (101)..(865)
<223> RXN02589

<400> 149
gcctaaattg cagcgagagg tctaaaaggt agtgctctag ggattcatcc aaactcacga 60

atattgaagt tttaaagttg aacaggaaaa ataacaaata atg tct att tct gat   115
                                           Met Ser Ile Ser Asp
                                           1               5

aat tcc cgc gat caa tta gga gaa ctg cca gct ggt cgg cct ctc caa   163
Asn Ser Arg Asp Gln Leu Gly Glu Leu Pro Ala Gly Arg Pro Leu Gln
                10                  15                  20

tcc gat ttt gat aat gac ctc gac tac cca cgt cta ggc agt gtc acg   211
Ser Asp Phe Asp Asn Asp Leu Asp Tyr Pro Arg Leu Gly Ser Val Thr
            25                  30                  35

ttt agg cgt ggc acc ctc act gaa aac cag caa acc atg tgg gat gaa   259
Phe Arg Arg Gly Thr Leu Thr Glu Asn Gln Gln Thr Met Trp Asp Glu

40                  45                  50

aag tgg cct gaa ctg ggt cgc gtc ctc gaa gat gag ctg att gat gtt     307
Lys Trp Pro Glu Leu Gly Arg Val Leu Glu Asp Glu Leu Ile Asp Val
    55                  60                  65

gat gcg tgg ttc ggg cgc gaa ggc gca aaa acc atc gta gag atc ggc     355
Asp Ala Trp Phe Gly Arg Glu Gly Ala Lys Thr Ile Val Glu Ile Gly
70                  75                  80                  85

tct ggc act gga act tcg act gct gcc atg gct cca ctt gag gct gat     403
Ser Gly Thr Gly Thr Ser Thr Ala Ala Met Ala Pro Leu Glu Ala Asp
                90                  95                  100

acc aac att gtc gcc gtc gaa cta tac aag ccg ggc ttg gcc aag ttg     451
Thr Asn Ile Val Ala Val Glu Leu Tyr Lys Pro Gly Leu Ala Lys Leu
            105                 110                 115

atg ggc tct gtt gtc cgt gga gag atc gac aac gtg cgc atg gtc cgc     499
Met Gly Ser Val Val Arg Gly Glu Ile Asp Asn Val Arg Met Val Arg
        120                 125                 130

gga gac ggc atc gag gtg ctc aac cgc atg ttt gcc gat ggg tcc ctg     547
Gly Asp Gly Ile Glu Val Leu Asn Arg Met Phe Ala Asp Gly Ser Leu
    135                 140                 145

gac ggc atc cgc gta tac ttc ccg gac cct tgg cca aag gcg cgc cac     595
Asp Gly Ile Arg Val Tyr Phe Pro Asp Pro Trp Pro Lys Ala Arg His
150                 155                 160                 165

aac aag cgc cgc atc atc cag tct ggt ccg ctg aac ctg ttt gca aag     643
Asn Lys Arg Arg Ile Ile Gln Ser Gly Pro Leu Asn Leu Phe Ala Lys
                170                 175                 180

aag ctc aag cca ggt gga gtt ctg cac gtt gct acc gac cac gct gat     691
Lys Leu Lys Pro Gly Gly Val Leu His Val Ala Thr Asp His Ala Asp
            185                 190                 195

tac gca gag tgg atc aat gag cta gtt gag gtc gaa cca ctg ctt gag     739
Tyr Ala Glu Trp Ile Asn Glu Leu Val Glu Val Glu Pro Leu Leu Glu
        200                 205                 210

tac aaa ggc tgg cca tgg gag gaa tgc cct cag ctg act gac cgt cag     787
Tyr Lys Gly Trp Pro Trp Glu Glu Cys Pro Gln Leu Thr Asp Arg Gln
    215                 220                 225

gtc atc acc aag ttt gaa ggc aaa ggc ttg gaa aaa gat cac gtg atc     835
Val Ile Thr Lys Phe Glu Gly Lys Gly Leu Glu Lys Asp His Val Ile
230                 235                 240                 245

aat gag tac ttg tgg cag aag gtg caa aac taatgtctga tgtgcatgag     885
Asn Glu Tyr Leu Trp Gln Lys Val Gln Asn
                250                 255

gtc                                                                 888


<210> 150
<211> 255
<212> PRT
<213> Corynebacterium glutamicum

<400> 150
Met Ser Ile Ser Asp Asn Ser Arg Asp Gln Leu Gly Glu Leu Pro Ala
1               5                   10                  15

Gly Arg Pro Leu Gln Ser Asp Phe Asp Asn Asp Leu Asp Tyr Pro Arg
            20                  25                  30

Leu Gly Ser Val Thr Phe Arg Arg Gly Thr Leu Thr Glu Asn Gln Gln
        35                  40                  45

Thr Met Trp Asp Glu Lys Trp Pro Glu Leu Gly Arg Val Leu Glu Asp
    50                  55                  60

Glu Leu Ile Asp Val Asp Ala Trp Phe Gly Arg Glu Gly Ala Lys Thr
65                  70                  75                  80

Ile Val Glu Ile Gly Ser Gly Thr Gly Thr Ser Thr Ala Ala Met Ala
                85                  90                  95

Pro Leu Glu Ala Asp Thr Asn Ile Val Ala Val Glu Leu Tyr Lys Pro
            100                 105                 110

Gly Leu Ala Lys Leu Met Gly Ser Val Val Arg Gly Glu Ile Asp Asn
        115                 120                 125

Val Arg Met Val Arg Gly Asp Gly Ile Glu Val Leu Asn Arg Met Phe
    130                 135                 140

Ala Asp Gly Ser Leu Asp Gly Ile Arg Val Tyr Phe Pro Asp Pro Trp
145                 150                 155                 160

Pro Lys Ala Arg His Asn Lys Arg Arg Ile Ile Gln Ser Gly Pro Leu
                165                 170                 175

Asn Leu Phe Ala Lys Lys Leu Lys Pro Gly Gly Val Leu His Val Ala
            180                 185                 190

Thr Asp His Ala Asp Tyr Ala Glu Trp Ile Asn Glu Leu Val Glu Val
        195                 200                 205

Glu Pro Leu Leu Glu Tyr Lys Gly Trp Pro Trp Glu Glu Cys Pro Gln
    210                 215                 220

Leu Thr Asp Arg Gln Val Ile Thr Lys Phe Glu Gly Lys Gly Leu Glu
225                 230                 235                 240

Lys Asp His Val Ile Asn Glu Tyr Leu Trp Gln Lys Val Gln Asn
                245                 250                 255


<210> 151
<211> 888
<212> DNA
<213> Corynebacterium glutamicum

<220>
<221> CDS
<222> (101)..(865)
<223> FRXA02589

<400> 151
gcctaaattg cagcgagagg tctaaaaggt agtgctctag ggattcatcc aaactcacga 60

atattgaagt tttaaagttg aacaggaaaa ataacaaata atg tct att tct gat    115
                                           Met Ser Ile Ser Asp
                                           1               5

aat tcc cgc gat caa tta gga gaa ctg cca gct ggt cgg cct ctc caa    163
Asn Ser Arg Asp Gln Leu Gly Glu Leu Pro Ala Gly Arg Pro Leu Gln
                10                  15                  20

tcc gat ttt gat aat gac ctc gac tac cca cgt cta ggc agt gtc acg    211
Ser Asp Phe Asp Asn Asp Leu Asp Tyr Pro Arg Leu Gly Ser Val Thr
            25                  30                  35

ttt agg cgt ggc acc ctc act gaa aac cag caa acc atg tgg gat gaa    259
Phe Arg Arg Gly Thr Leu Thr Glu Asn Gln Gln Thr Met Trp Asp Glu
        40                  45                  50

aag tgg cct gaa ctg ggt cgc gtc ctc gaa gat gag ctg att gat gtt    307
Lys Trp Pro Glu Leu Gly Arg Val Leu Glu Asp Glu Leu Ile Asp Val
    55                  60                  65

gat gcg tgg ttc ggg cgc gaa ggc gca aaa acc atc gta gag atc ggc    355
Asp Ala Trp Phe Gly Arg Glu Gly Ala Lys Thr Ile Val Glu Ile Gly
70                  75                  80                  85

tct ggc act gga act tcg act gct gcc atg gct cca ctt gag gct gat    403
Ser Gly Thr Gly Thr Ser Thr Ala Ala Met Ala Pro Leu Glu Ala Asp
                90                  95                  100

acc aac att gtc gcc gtc gaa cta tac aag ccg ggc ttg gcc aag ttg    451

Thr Asn Ile Val Ala Val Glu Leu Tyr Lys Pro Gly Leu Ala Lys Leu
            105                 110                 115

atg ggc tct gtt gtc cgt gga gag atc gac aac gtg cgc atg gtc cgc      499
Met Gly Ser Val Val Arg Gly Glu Ile Asp Asn Val Arg Met Val Arg
        120                 125                 130

gga gac ggc atc gag gtg ctc aac cgc atg ttt gcc gat ggg tcc ctg      547
Gly Asp Gly Ile Glu Val Leu Asn Arg Met Phe Ala Asp Gly Ser Leu
    135                 140                 145

gac ggc atc cgc gta tac ttc ccg gac cct tgg cca aag gcg cgc cac      595
Asp Gly Ile Arg Val Tyr Phe Pro Asp Pro Trp Pro Lys Ala Arg His
150                 155                 160                 165

aac aag cgc cgc atc atc cag tct ggt ccg ctg aac ctg ttt gca aag      643
Asn Lys Arg Arg Ile Ile Gln Ser Gly Pro Leu Asn Leu Phe Ala Lys
                170                 175                 180

aag ctc aag cca ggt gga gtt ctg cac gtt gct acc gac cac gct gat      691
Lys Leu Lys Pro Gly Gly Val Leu His Val Ala Thr Asp His Ala Asp
            185                 190                 195

tac gca gag tgg atc aat gag cta gtt gag gtc gaa cca ctg ctt gag      739
Tyr Ala Glu Trp Ile Asn Glu Leu Val Glu Val Glu Pro Leu Leu Glu
        200                 205                 210

tac aaa ggc tgg cca tgg gag gaa tgc cct cag ctg act gac cgt cag      787
Tyr Lys Gly Trp Pro Trp Glu Glu Cys Pro Gln Leu Thr Asp Arg Gln
    215                 220                 225

gtc atc acc aag ttt gaa ggc aaa ggc ttg gaa aaa gat cac gtg atc      835
Val Ile Thr Lys Phe Glu Gly Lys Gly Leu Glu Lys Asp His Val Ile
230                 235                 240                 245

aat gag tac ttg tgg cag aag gtg caa aac taatgtctga tgtgcatgag      885
Asn Glu Tyr Leu Trp Gln Lys Val Gln Asn
                250                 255

gtc                                                                  888


<210> 152
<211> 255
<212> PRT
<213> Corynebacterium glutamicum

<400> 152
Met Ser Ile Ser Asp Asn Ser Arg Asp Gln Leu Gly Glu Leu Pro Ala
1               5                   10                  15

Gly Arg Pro Leu Gln Ser Asp Phe Asp Asn Asp Leu Asp Tyr Pro Arg
            20                  25                  30

Leu Gly Ser Val Thr Phe Arg Arg Gly Thr Leu Thr Glu Asn Gln Gln
        35                  40                  45

Thr Met Trp Asp Glu Lys Trp Pro Glu Leu Gly Arg Val Leu Glu Asp
    50                  55                  60

Glu Leu Ile Asp Val Asp Ala Trp Phe Gly Arg Glu Gly Ala Lys Thr
65                  70                  75                  80

Ile Val Glu Ile Gly Ser Gly Thr Gly Thr Ser Thr Ala Ala Met Ala
                85                  90                  95

Pro Leu Glu Ala Asp Thr Asn Ile Val Ala Val Glu Leu Tyr Lys Pro
            100                 105                 110

Gly Leu Ala Lys Leu Met Gly Ser Val Val Arg Gly Glu Ile Asp Asn
        115                 120                 125

Val Arg Met Val Arg Gly Asp Gly Ile Glu Val Leu Asn Arg Met Phe
    130                 135                 140

Ala Asp Gly Ser Leu Asp Gly Ile Arg Val Tyr Phe Pro Asp Pro Trp
145                 150                 155                 160

Pro Lys Ala Arg His Asn Lys Arg Arg Ile Ile Gln Ser Gly Pro Leu
                165                 170                 175

Asn Leu Phe Ala Lys Lys Leu Lys Pro Gly Gly Val Leu His Val Ala
            180                 185                 190

Thr Asp His Ala Asp Tyr Ala Glu Trp Ile Asn Glu Leu Val Glu Val
        195                 200                 205

Glu Pro Leu Leu Glu Tyr Lys Gly Trp Pro Trp Glu Glu Cys Pro Gln
    210                 215                 220

Leu Thr Asp Arg Gln Val Ile Thr Lys Phe Glu Gly Lys Gly Leu Glu
225                 230                 235                 240

Lys Asp His Val Ile Asn Glu Tyr Leu Trp Gln Lys Val Gln Asn
                245                 250                 255


<210> 153
<211> 948
<212> DNA
<213> Corynebacterium glutamicum

<220>
<221> CDS
<222> (101)..(925)
<223> RXA00226

<400> 153
ccgcctgcgg tgtcagcgga gcgcgcccgg cgtctgaaaa ctgcacttgg tgagaacgtg 60

attgttcatg atgtcacctg ttccatcggt acggaggggc atg aac tta tcg atg   115
                                            Met Asn Leu Ser Met
                                            1               5

ccg gcc ttc gct acc tgg gtt ctg atc cta gat ttc tca cgc acc ctc   163
Pro Ala Phe Ala Thr Trp Val Leu Ile Leu Asp Phe Ser Arg Thr Leu
                10                  15                  20

atg gca gcc cac aat ctc cag ggc aaa aac gcc ctg att ttc cgc gcc   211
Met Ala Ala His Asn Leu Gln Gly Lys Asn Ala Leu Ile Phe Arg Ala
            25                  30                  35

gac gcg ctc cag ccc gca agc agg gga gcc gac gtc atc atc gcg gac   259
Asp Ala Leu Gln Pro Ala Ser Arg Gly Ala Asp Val Ile Ile Ala Asp
        40                  45                  50

cct gcc aga cgc gcc ggg ggc aag cgc att aca aat ccg gca cag ctc   307
Pro Ala Arg Arg Ala Gly Gly Lys Arg Ile Thr Asn Pro Ala Gln Leu
    55                  60                  65

ctg cca cct ctg cct tcg ctt ctc gac gcc tgg atc aac caa cca ctc   355
Leu Pro Pro Leu Pro Ser Leu Leu Asp Ala Trp Ile Asn Gln Pro Leu
70                  75                  80                  85

gcc gtt aaa tgt gcc ccc ggc ctt gat ttt tcg gaa tgg cca ggt ctc   403
Ala Val Lys Cys Ala Pro Gly Leu Asp Phe Ser Glu Trp Pro Gly Leu
                90                  95                  100

gtc agt att gcc agc gtt gat gga ggc gtg aaa gaa gca tgc ctc tac   451
Val Ser Ile Ala Ser Val Asp Gly Gly Val Lys Glu Ala Cys Leu Tyr
            105                 110                 115

act acg gat ctg gca gat ggg gaa act cgc gaa gct atc gtg atc aaa   499
Thr Thr Asp Leu Ala Asp Gly Glu Thr Arg Glu Ala Ile Val Ile Lys
        120                 125                 130

gat ggg ctc att gac cgc atc acc aac ttt gaa gac gat gcc acg gga   547
Asp Gly Leu Ile Asp Arg Ile Thr Asn Phe Glu Asp Asp Ala Thr Gly
    135                 140                 145

caa gac ctt gcg gct gca cct ggt gag ttc atc atc gac cca gac ggt   595
Gln Asp Leu Ala Ala Ala Pro Gly Glu Phe Ile Ile Asp Pro Asp Gly
150                 155                 160                 165

gcc atc gtg cgc gcc ggg ttg gtt cgc cac tat gca gtg cgt gag cag     643
Ala Ile Val Arg Ala Gly Leu Val Arg His Tyr Ala Val Arg Glu Gln
                170                 175                 180

ctg tgg atg ttg gat gag cgg atc gca tac ctt acg ggc aat cgg att     691
Leu Trp Met Leu Asp Glu Arg Ile Ala Tyr Leu Thr Gly Asn Arg Ile
            185                 190                 195

cca gag ggt acc agc ggt ttt agg ttt att gaa gag gtt ccg ctg aag     739
Pro Glu Gly Thr Ser Gly Phe Arg Phe Ile Glu Glu Val Pro Leu Lys
        200                 205                 210

aag ctg aaa tcg gcg atg gca gca cat gat gcg ggg gcg gtt gaa att     787
Lys Leu Lys Ser Ala Met Ala Ala His Asp Ala Gly Ala Val Glu Ile
    215                 220                 225

tta gtg cgt ggt gtt gat gtt gat cct gat cag ttg cgg aaa aga ttg     835
Leu Val Arg Gly Val Asp Val Asp Pro Asp Gln Leu Arg Lys Arg Leu
230                 235                 240                 245

cag ctg aag ggt acc aag gcg atg tct gtg gtg atc act cga att ggc     883
Gln Leu Lys Gly Thr Lys Ala Met Ser Val Val Ile Thr Arg Ile Gly
                250                 255                 260

agc cga ggg gtt gca ttg att tgt ggt cct cgc gag cgc gcc             925
Ser Arg Gly Val Ala Leu Ile Cys Gly Pro Arg Glu Arg Ala
            265                 270                 275

taaagccgat gcaaataaaa ttg                                           948


<210> 154
<211> 275
<212> PRT
<213> Corynebacterium glutamicum

<400> 154
Met Asn Leu Ser Met Pro Ala Phe Ala Thr Trp Val Leu Ile Leu Asp
1               5                   10                  15

Phe Ser Arg Thr Leu Met Ala Ala His Asn Leu Gln Gly Lys Asn Ala
            20                  25                  30

Leu Ile Phe Arg Ala Asp Ala Leu Gln Pro Ala Ser Arg Gly Ala Asp
        35                  40                  45

Val Ile Ile Ala Asp Pro Ala Arg Arg Ala Gly Gly Lys Arg Ile Thr
    50                  55                  60

Asn Pro Ala Gln Leu Leu Pro Pro Leu Pro Ser Leu Leu Asp Ala Trp
65                  70                  75                  80

Ile Asn Gln Pro Leu Ala Val Lys Cys Ala Pro Gly Leu Asp Phe Ser
                85                  90                  95

Glu Trp Pro Gly Leu Val Ser Ile Ala Ser Val Asp Gly Gly Val Lys
            100                 105                 110

Glu Ala Cys Leu Tyr Thr Thr Asp Leu Ala Asp Gly Glu Thr Arg Glu
        115                 120                 125

Ala Ile Val Ile Lys Asp Gly Leu Ile Asp Arg Ile Thr Asn Phe Glu
    130                 135                 140

Asp Asp Ala Thr Gly Gln Asp Leu Ala Ala Ala Pro Gly Glu Phe Ile
145                 150                 155                 160

Ile Asp Pro Asp Gly Ala Ile Val Arg Ala Gly Leu Val Arg His Tyr
                165                 170                 175

Ala Val Arg Glu Gln Leu Trp Met Leu Asp Glu Arg Ile Ala Tyr Leu
            180                 185                 190

Thr Gly Asn Arg Ile Pro Glu Gly Thr Ser Gly Phe Arg Phe Ile Glu
        195                 200                 205

Glu Val Pro Leu Lys Lys Leu Lys Ser Ala Met Ala Ala His Asp Ala

210                 215                 220

Gly Ala Val Glu Ile Leu Val Arg Gly Val Asp Val Asp Pro Asp Gln
225                 230                 235                 240

Leu Arg Lys Arg Leu Gln Leu Lys Gly Thr Lys Ala Met Ser Val Val
                245                 250                 255

Ile Thr Arg Ile Gly Ser Arg Gly Val Ala Leu Ile Cys Gly Pro Arg
            260                 265                 270

Glu Arg Ala
        275


<210> 155
<211> 924
<212> DNA
<213> Corynebacterium glutamicum

<220>
<221> CDS
<222> (101)..(901)
<223> RXN01885

<400> 155
gtggcgtcgc agggatgttc ctgcggcacc atttttgctg aggtggaact cacggattaa 60

acacggattt ttctaaggtt aatcaagtaa ggtttacctt atg act acg aaa cct   115
                                           Met Thr Thr Lys Pro
                                           1               5

atc atc cca gaa tca acc cac tcc gca gaa cgt gct ggt gga cat tgg   163
Ile Ile Pro Glu Ser Thr His Ser Ala Glu Arg Ala Gly Gly His Trp
                10                  15                  20

atc ctt gcc agg ctt gga aag aaa gtg ctg cgc cct gga ggt cgt gaa   211
Ile Leu Ala Arg Leu Gly Lys Lys Val Leu Arg Pro Gly Gly Arg Glu
            25                  30                  35

aca acg cag ttc ctg ctg gag aac ctt tct ttg acc ggt gct acc gtg   259
Thr Thr Gln Phe Leu Leu Glu Asn Leu Ser Leu Thr Gly Ala Thr Val
        40                  45                  50

gtg gaa ttt gct cca gga ctt ggc gtg act gca cgt gac atc ctt ggc   307
Val Glu Phe Ala Pro Gly Leu Gly Val Thr Ala Arg Asp Ile Leu Gly
    55                  60                  65

aag ggt ccg gct cgc tac atc gga gtg gat agc gac gcg gat gca tgc   355
Lys Gly Pro Ala Arg Tyr Ile Gly Val Asp Ser Asp Ala Asp Ala Cys
70                  75                  80                  85

gcg aat gta cgt gcg atc tta cct gct ggt cct cac gag gtg cgc aat   403
Ala Asn Val Arg Ala Ile Leu Pro Ala Gly Pro His Glu Val Arg Asn
                90                  95                  100

aca aat gcc acc gat act ggc ctt gaa agc gac tcg ttt gat gtt gtc   451
Thr Asn Ala Thr Asp Thr Gly Leu Glu Ser Asp Ser Phe Asp Val Val
            105                 110                 115

atc ggc gaa gcg atg ttg acc atg cag acc gat aag cac aag ttg gag   499
Ile Gly Glu Ala Met Leu Thr Met Gln Thr Asp Lys His Lys Leu Glu
        120                 125                 130

ctg atg cgc gag gca gct cga att ctg aaa cca ggc ggg ctg tac ggc   547
Leu Met Arg Glu Ala Ala Arg Ile Leu Lys Pro Gly Gly Leu Tyr Gly
    135                 140                 145

att cac gag ctg tcg ctg gtg cct gac aat gtc tcc act gcg gtg aaa   595
Ile His Glu Leu Ser Leu Val Pro Asp Asn Val Ser Thr Ala Val Lys
150                 155                 160                 165

gag gat att gct aag gcg ctg gct cgt tcc atc aaa gtc aat gcc cgc   643
Glu Asp Ile Ala Lys Ala Leu Ala Arg Ser Ile Lys Val Asn Ala Arg
                170                 175                 180

ccc atc acg gtg ccg gaa tgg gct gcg ttg gcg cgt gag gca ggg ttc   691

Pro Ile Thr Val Pro Glu Trp Ala Ala Leu Ala Arg Glu Ala Gly Phe
                185                 190                 195

gat gtg att aat att cgc caa gcc gac atg gcc ctt cta tcc ctc aag     739
Asp Val Ile Asn Ile Arg Gln Ala Asp Met Ala Leu Leu Ser Leu Lys
        200                 205                 210

cgg aac ctg aag gat gaa ggg cta aaa ggt gtc ttc acg att gtg agg     787
Arg Asn Leu Lys Asp Glu Gly Leu Lys Gly Val Phe Thr Ile Val Arg
    215                 220                 225

aac gtg att agc caa ccg gat ctg cgc aag cga gtg ctc gga atg cga     835
Asn Val Ile Ser Gln Pro Asp Leu Arg Lys Arg Val Leu Gly Met Arg
230                 235                 240                 245

aag act ttc acc gag cat aaa gat cac tta ggt gcg gtt ggc atc att     883
Lys Thr Phe Thr Glu His Lys Asp His Leu Gly Ala Val Gly Ile Ile
                250                 255                 260

ttg cag aag aga gcc caa tagggatctg aaatggaggg gtg                   924
Leu Gln Lys Arg Ala Gln
                265


<210> 156
<211> 267
<212> PRT
<213> Corynebacterium glutamicum

<400> 156
Met Thr Thr Lys Pro Ile Ile Pro Glu Ser Thr His Ser Ala Glu Arg
  1               5                  10                  15

Ala Gly Gly His Trp Ile Leu Ala Arg Leu Gly Lys Lys Val Leu Arg
                 20                  25                  30

Pro Gly Gly Arg Glu Thr Thr Gln Phe Leu Leu Glu Asn Leu Ser Leu
         35                  40                  45

Thr Gly Ala Thr Val Val Glu Phe Ala Pro Gly Leu Gly Val Thr Ala
     50                  55                  60

Arg Asp Ile Leu Gly Lys Gly Pro Ala Arg Tyr Ile Gly Val Asp Ser
 65                  70                  75                  80

Asp Ala Asp Ala Cys Ala Asn Val Arg Ala Ile Leu Pro Ala Gly Pro
                 85                  90                  95

His Glu Val Arg Asn Thr Asn Ala Thr Asp Thr Gly Leu Glu Ser Asp
                100                 105                 110

Ser Phe Asp Val Val Ile Gly Glu Ala Met Leu Thr Met Gln Thr Asp
        115                 120                 125

Lys His Lys Leu Glu Leu Met Arg Glu Ala Ala Arg Ile Leu Lys Pro
    130                 135                 140

Gly Gly Leu Tyr Gly Ile His Glu Leu Ser Leu Val Pro Asp Asn Val
145                 150                 155                 160

Ser Thr Ala Val Lys Glu Asp Ile Ala Lys Ala Leu Ala Arg Ser Ile
                165                 170                 175

Lys Val Asn Ala Arg Pro Ile Thr Val Pro Glu Trp Ala Ala Leu Ala
                180                 185                 190

Arg Glu Ala Gly Phe Asp Val Ile Asn Ile Arg Gln Ala Asp Met Ala
        195                 200                 205

Leu Leu Ser Leu Lys Arg Asn Leu Lys Asp Glu Gly Leu Lys Gly Val
    210                 215                 220

Phe Thr Ile Val Arg Asn Val Ile Ser Gln Pro Asp Leu Arg Lys Arg
225                 230                 235                 240

Val Leu Gly Met Arg Lys Thr Phe Thr Glu His Lys Asp His Leu Gly
                245                 250                 255

Ala Val Gly Ile Ile Leu Gln Lys Arg Ala Gln
            260                 265

<210> 157
<211> 924
<212> DNA
<213> Corynebacterium glutamicum

<220>
<221> CDS
<222> (101)..(901)
<223> FRXA01885

<400> 157
gtggcgtcgc agggatgttc ctgcggcacc atttttgctg aggtggaact cacggattaa 60

acacggattt ttctaaggtt aatcaagtaa ggtttacctt atg act acg aaa cct    115
                                           Met Thr Thr Lys Pro
                                           1               5

atc atc cca gaa tca acc cac tcc gca gaa cgt gct ggt gga cat tgg    163
Ile Ile Pro Glu Ser Thr His Ser Ala Glu Arg Ala Gly Gly His Trp
                10                  15                  20

atc ctt gcc agg ctt gga aag aaa gtg ctg cgc cct gga ggt cgt gaa    211
Ile Leu Ala Arg Leu Gly Lys Lys Val Leu Arg Pro Gly Gly Arg Glu
            25                  30                  35

aca acg cag ttc ctg ctg gag aac ctt tct ttg acc ggt gct acc gtg    259
Thr Thr Gln Phe Leu Leu Glu Asn Leu Ser Leu Thr Gly Ala Thr Val
        40                  45                  50

gtg gaa ttt gct cca gga ctt ggc gtg act gca cgt gac atc ctt ggc    307
Val Glu Phe Ala Pro Gly Leu Gly Val Thr Ala Arg Asp Ile Leu Gly
    55                  60                  65

aag ggt ccg gct cgc tac atc gga gtg gat agc gac gcg gat gca tgc    355
Lys Gly Pro Ala Arg Tyr Ile Gly Val Asp Ser Asp Ala Asp Ala Cys
70                  75                  80                  85

gcg aat gta cgt gcg atc tta cct gct ggt cct cac gag gtg cgc aat    403
Ala Asn Val Arg Ala Ile Leu Pro Ala Gly Pro His Glu Val Arg Asn
                90                  95                  100

aca aat gcc acc gat act ggc ctt gaa agc gac tcg ttt gat gtt gtc    451
Thr Asn Ala Thr Asp Thr Gly Leu Glu Ser Asp Ser Phe Asp Val Val
            105                 110                 115

atc ggc gaa gcg atg ttg acc atg cag acc gat aag cac aag ttg gag    499
Ile Gly Glu Ala Met Leu Thr Met Gln Thr Asp Lys His Lys Leu Glu
        120                 125                 130

ctg atg cgc gag gca gct cga att ctg aaa cca ggc ggg ctg tac ggc    547
Leu Met Arg Glu Ala Ala Arg Ile Leu Lys Pro Gly Gly Leu Tyr Gly
    135                 140                 145

att cac gag ctg tcg ctg gtg cct gac aat gtc tcc act gcg gtg aaa    595
Ile His Glu Leu Ser Leu Val Pro Asp Asn Val Ser Thr Ala Val Lys
150                 155                 160                 165

gag gat att gct aag gcg ctg gct cgt tcc atc aaa gtc aat gcc cgc    643
Glu Asp Ile Ala Lys Ala Leu Ala Arg Ser Ile Lys Val Asn Ala Arg
                170                 175                 180

ccc atc acg gtg ccg gaa tgg gct gcg ttg gcg cgt gag gca ggg ttc    691
Pro Ile Thr Val Pro Glu Trp Ala Ala Leu Ala Arg Glu Ala Gly Phe
            185                 190                 195

gat gtg att aat att cgc caa gcc gac atg gcc ctt cta tcc ctc aag    739
Asp Val Ile Asn Ile Arg Gln Ala Asp Met Ala Leu Leu Ser Leu Lys
        200                 205                 210

cgg aac ctg aag gat gaa ggg cta aaa ggt gtc ttc acg att gtg agg    787
Arg Asn Leu Lys Asp Glu Gly Leu Lys Gly Val Phe Thr Ile Val Arg
    215                 220                 225

aac gtg att agc caa ccg gat ctg cgc aag cga gtg ctc gga atg cga     835
Asn Val Ile Ser Gln Pro Asp Leu Arg Lys Arg Val Leu Gly Met Arg
230                 235                 240                 245

aag act ttc acc gag cat aaa gat cac tta ggt gcg gtt ggc atc att     883
Lys Thr Phe Thr Glu His Lys Asp His Leu Gly Ala Val Gly Ile Ile
                250                 255                 260

ttg cag aag aga gcc caa tagggatctg aaatggaggg gtg                   924
Leu Gln Lys Arg Ala Gln
            265


<210> 158
<211> 267
<212> PRT
<213> Corynebacterium glutamicum

<400> 158
Met Thr Thr Lys Pro Ile Ile Pro Glu Ser Thr His Ser Ala Glu Arg
  1               5                  10                  15

Ala Gly Gly His Trp Ile Leu Ala Arg Leu Gly Lys Lys Val Leu Arg
             20                  25                  30

Pro Gly Gly Arg Glu Thr Thr Gln Phe Leu Leu Glu Asn Leu Ser Leu
         35                  40                  45

Thr Gly Ala Thr Val Val Glu Phe Ala Pro Gly Leu Gly Val Thr Ala
     50                  55                  60

Arg Asp Ile Leu Gly Lys Gly Pro Ala Arg Tyr Ile Gly Val Asp Ser
 65                  70                  75                  80

Asp Ala Asp Ala Cys Ala Asn Val Arg Ala Ile Leu Pro Ala Gly Pro
                 85                  90                  95

His Glu Val Arg Asn Thr Asn Ala Thr Asp Thr Gly Leu Glu Ser Asp
            100                 105                 110

Ser Phe Asp Val Val Ile Gly Glu Ala Met Leu Thr Met Gln Thr Asp
        115                 120                 125

Lys His Lys Leu Glu Leu Met Arg Glu Ala Ala Arg Ile Leu Lys Pro
    130                 135                 140

Gly Gly Leu Tyr Gly Ile His Glu Leu Ser Leu Val Pro Asp Asn Val
145                 150                 155                 160

Ser Thr Ala Val Lys Glu Asp Ile Ala Lys Ala Leu Ala Arg Ser Ile
                165                 170                 175

Lys Val Asn Ala Arg Pro Ile Thr Val Pro Glu Trp Ala Ala Leu Ala
            180                 185                 190

Arg Glu Ala Gly Phe Asp Val Ile Asn Ile Arg Gln Ala Asp Met Ala
        195                 200                 205

Leu Leu Ser Leu Lys Arg Asn Leu Lys Asp Glu Gly Leu Lys Gly Val
    210                 215                 220

Phe Thr Ile Val Arg Asn Val Ile Ser Gln Pro Asp Leu Arg Lys Arg
225                 230                 235                 240

Val Leu Gly Met Arg Lys Thr Phe Thr Glu His Lys Asp His Leu Gly
                245                 250                 255

Ala Val Gly Ile Ile Leu Gln Lys Arg Ala Gln
            260                 265


<210> 159
<211> 894
<212> DNA
<213> Corynebacterium glutamicum

<220>
<221> CDS
<222> (101)..(871)
<223> RXA02592

<400> 159
aaacaccacc attgtttcct ccatcgacga tgccatcacc accattttgc gatggatgaa 60

cggcgaagac atccgcgacc tcaactggac ccgcgcataa atg gcc tca ttt ccg 115
Met Ala Ser Phe Pro
1               5

gag ctt ccg gct ctt cgt cgc ttg gct acc ttg ggc agg tcg tgg ggt 163
Glu Leu Pro Ala Leu Arg Arg Leu Ala Thr Leu Gly Arg Ser Trp Gly
                10                  15                  20

tta ctg tct gat ttc aaa tac gaa caa acc cga cct gac atc ttt tac 211
Leu Leu Ser Asp Phe Lys Tyr Glu Gln Thr Arg Pro Asp Ile Phe Tyr
            25                  30                  35

gga aac ctg gcc ctc gat acc tcg agt ctg gtg gcg gct ttg tct gaa 259
Gly Asn Leu Ala Leu Asp Thr Ser Ser Leu Val Ala Ala Leu Ser Glu
        40                  45                  50

gat att tct ggc gcc gga tta aat gac ctg aaa gtt ctc gac gtc ggc 307
Asp Ile Ser Gly Ala Gly Leu Asn Asp Leu Lys Val Leu Asp Val Gly
    55                  60                  65

ggc gga ccc gga tac ttc gcc gaa gcc ttt gag aca ctg ggc gcc acc 355
Gly Gly Pro Gly Tyr Phe Ala Glu Ala Phe Glu Thr Leu Gly Ala Thr
70                  75                  80                  85

tac ttc tcc gtc gaa ccc gac gtt ggc gaa atg tcc gca gct ggc atc 403
Tyr Phe Ser Val Glu Pro Asp Val Gly Glu Met Ser Ala Ala Gly Ile
                90                  95                  100

gac gtc cac gga tca gtc cgc gga tcc ggc ctc gac ctg ccg ttt ctt 451
Asp Val His Gly Ser Val Arg Gly Ser Gly Leu Asp Leu Pro Phe Leu
            105                 110                 115

ccc gat tcc ttt gac gtg gtg tac tcc tcc aac gtt gca gaa cat gtc 499
Pro Asp Ser Phe Asp Val Val Tyr Ser Ser Asn Val Ala Glu His Val
        120                 125                 130

tcc gca ccg tgg gaa ttg gga gaa gaa atg ctc cgc gtc acc cgc agc 547
Ser Ala Pro Trp Glu Leu Gly Glu Glu Met Leu Arg Val Thr Arg Ser
    135                 140                 145

ggc ggc ctg gca atc ctg agc tac acc att tgg tta ggg ccc ttc ggc 595
Gly Gly Leu Ala Ile Leu Ser Tyr Thr Ile Trp Leu Gly Pro Phe Gly
150                 155                 160                 165

ggc cat gaa acc gga ctg tgg gaa cac tac gtt ggc gga gaa ttt gcc 643
Gly His Glu Thr Gly Leu Trp Glu His Tyr Val Gly Gly Glu Phe Ala
                170                 175                 180

cgc gat cgc tac acg aag aaa cac ggg cac ccg cct aag aac gtt ttc 691
Arg Asp Arg Tyr Thr Lys Lys His Gly His Pro Pro Lys Asn Val Phe
            185                 190                 195

ggg gag tca ctg ttt aat gtg tcc tgc cgg gag ggg ctg gaa tgg gga 739
Gly Glu Ser Leu Phe Asn Val Ser Cys Arg Glu Gly Leu Glu Trp Gly
        200                 205                 210

gcc tcc gtg ggc aat gcg gaa ttg gtt gcc gct ttt ccc cgc tac cac 787
Ala Ser Val Gly Asn Ala Glu Leu Val Ala Ala Phe Pro Arg Tyr His
    215                 220                 225

ccg tat tgg gtc tgg tgg atg gtt aaa gtc cca gtg ctc cga gaa ttc 835
Pro Tyr Trp Val Trp Trp Met Val Lys Val Pro Val Leu Arg Glu Phe
230                 235                 240                 245

gcg gta agt aac ttg gtg ttg gtg ttt aaa aag cac tgaggttttg 881
Ala Val Ser Asn Leu Val Leu Val Phe Lys Lys His
                250                 255

aggaattcat cgc 894

<210> 160
<211> 257
<212> PRT
<213> Corynebacterium glutamicum

<400> 160
Met Ala Ser Phe Pro Glu Leu Pro Ala Leu Arg Arg Leu Ala Thr Leu
1               5                   10                  15

Gly Arg Ser Trp Gly Leu Leu Ser Asp Phe Lys Tyr Glu Gln Thr Arg
            20                  25                  30

Pro Asp Ile Phe Tyr Gly Asn Leu Ala Leu Asp Thr Ser Ser Leu Val
        35                  40                  45

Ala Ala Leu Ser Glu Asp Ile Ser Gly Ala Gly Leu Asn Asp Leu Lys
    50                  55                  60

Val Leu Asp Val Gly Gly Gly Pro Gly Tyr Phe Ala Glu Ala Phe Glu
65                  70                  75                  80

Thr Leu Gly Ala Thr Tyr Phe Ser Val Glu Pro Asp Val Gly Glu Met
                85                  90                  95

Ser Ala Ala Gly Ile Asp Val His Gly Ser Val Arg Gly Ser Gly Leu
            100                 105                 110

Asp Leu Pro Phe Leu Pro Asp Ser Phe Asp Val Val Tyr Ser Ser Asn
        115                 120                 125

Val Ala Glu His Val Ser Ala Pro Trp Glu Leu Gly Glu Glu Met Leu
    130                 135                 140

Arg Val Thr Arg Ser Gly Gly Leu Ala Ile Leu Ser Tyr Thr Ile Trp
145                 150                 155                 160

Leu Gly Pro Phe Gly Gly His Glu Thr Gly Leu Trp Glu His Tyr Val
                165                 170                 175

Gly Gly Glu Phe Ala Arg Asp Arg Tyr Thr Lys Lys His Gly His Pro
            180                 185                 190

Pro Lys Asn Val Phe Gly Glu Ser Leu Phe Asn Val Ser Cys Arg Glu
        195                 200                 205

Gly Leu Glu Trp Gly Ala Ser Val Gly Asn Ala Glu Leu Val Ala Ala
    210                 215                 220

Phe Pro Arg Tyr His Pro Tyr Trp Val Trp Trp Met Val Lys Val Pro
225                 230                 235                 240

Val Leu Arg Glu Phe Ala Val Ser Asn Leu Val Leu Val Phe Lys Lys
                245                 250                 255

His

<210> 161
<211> 720
<212> DNA
<213> Corynebacterium glutamicum

<220>
<221> CDS
<222> (101)..(697)
<223> RXN01795

<400> 161
agaccatatt gaagacctcg aagctgttga gcctggctac atcgtcaagc ctcgcctgta 60

caacttcgct gaatacggtg tcccacaatt ccgcgaacgt gtg ctc att gtt ggc   115
                                            Val Leu Ile Val Gly
                                            1               5

att cgc cgt gac acc ggc ttt gat ttc aag cac cca gct cct acc cat     163
Ile Arg Arg Asp Thr Gly Phe Asp Phe Lys His Pro Ala Pro Thr His
                10                  15                  20

ggc cct cgc ggt gac atg ccg tat aag act gcc ggc gaa gcg ctc aaa     211
Gly Pro Arg Gly Asp Met Pro Tyr Lys Thr Ala Gly Glu Ala Leu Lys
                25                  30                  35

ggc gtg aag gat gtc ccc aca aac aac aac cac atg aag atc atg cct     259
Gly Val Lys Asp Val Pro Thr Asn Asn Asn His Met Lys Ile Met Pro
            40                  45                  50

cgc acc gtt gaa gtg ctt aag cgc atc cct gag ggc gaa aac ttc acc     307
Arg Thr Val Glu Val Leu Lys Arg Ile Pro Glu Gly Glu Asn Phe Thr
    55                  60                  65

gcg atc ccc aaa gat gac ccc tac tac gtc aag ggc atg att agt cac     355
Ala Ile Pro Lys Asp Asp Pro Tyr Tyr Val Lys Gly Met Ile Ser His
70                  75                  80                  85

gtt tac cgt cgc ttg cac cgt gat gag cca tcc aaa acc ctt atc gcc     403
Val Tyr Arg Arg Leu His Arg Asp Glu Pro Ser Lys Thr Leu Ile Ala
                90                  95                  100

ggt ggc ggc ggg ggt aca tgg gga tac cat tat gaa aaa aat cga gca     451
Gly Gly Gly Gly Gly Thr Trp Gly Tyr His Tyr Glu Lys Asn Arg Ala
            105                 110                 115

ttg acc aac cgc gag cgg gct aga att caa tcg ttc ccc gat gac ttt     499
Leu Thr Asn Arg Glu Arg Ala Arg Ile Gln Ser Phe Pro Asp Asp Phe
        120                 125                 130

gag ttt ttg gga tca aac acc gaa gtc cgc cgc caa atc ggt aat gct     547
Glu Phe Leu Gly Ser Asn Thr Glu Val Arg Arg Gln Ile Gly Asn Ala
    135                 140                 145

gtt cct cct gta ggt atg cac gct gtg ggt gag cga ctg atg aac ctg     595
Val Pro Pro Val Gly Met His Ala Val Gly Glu Arg Leu Met Asn Leu
150                 155                 160                 165

tac acc ggg aat tac act ccc gtc gat cta gag gaa cag cac gcg tac     643
Tyr Thr Gly Asn Tyr Thr Pro Val Asp Leu Glu Glu Gln His Ala Tyr
                170                 175                 180

ctg cag acg ctc tcc att aag gaa cgt ctc gcg ctg gct gat cag gaa     691
Leu Gln Thr Leu Ser Ile Lys Glu Arg Leu Ala Leu Ala Asp Gln Glu
            185                 190                 195

gct gat taagtagata tatgaagccc acc                                   720
Ala Asp

<210> 162
<211> 199
<212> PRT
<213> Corynebacterium glutamicum

<400> 162
Val Leu Ile Val Gly Ile Arg Arg Asp Thr Gly Phe Asp Phe Lys His
1               5                   10                  15

Pro Ala Pro Thr His Gly Pro Arg Gly Asp Met Pro Tyr Lys Thr Ala
            20                  25                  30

Gly Glu Ala Leu Lys Gly Val Lys Asp Val Pro Thr Asn Asn Asn His
        35                  40                  45

Met Lys Ile Met Pro Arg Thr Val Glu Val Leu Lys Arg Ile Pro Glu
    50                  55                  60

Gly Glu Asn Phe Thr Ala Ile Pro Lys Asp Asp Pro Tyr Tyr Val Lys
65                  70                  75                  80

Gly Met Ile Ser His Val Tyr Arg Arg Leu His Arg Asp Glu Pro Ser
                85                  90                  95

Lys Thr Leu Ile Ala Gly Gly Gly Gly Gly Thr Trp Gly Tyr His Tyr
            100                 105                 110

Glu Lys Asn Arg Ala Leu Thr Asn Arg Glu Arg Ala Arg Ile Gln Ser
        115                 120                 125

Phe Pro Asp Asp Phe Glu Phe Leu Gly Ser Asn Thr Glu Val Arg Arg
    130                 135                 140

Gln Ile Gly Asn Ala Val Pro Pro Val Gly Met His Ala Val Gly Glu
145                 150                 155                 160

Arg Leu Met Asn Leu Tyr Thr Gly Asn Tyr Thr Pro Val Asp Leu Glu
                165                 170                 175

Glu Gln His Ala Tyr Leu Gln Thr Leu Ser Ile Lys Glu Arg Leu Ala
            180                 185                 190

Leu Ala Asp Gln Glu Ala Asp
        195


<210> 163
<211> 535
<212> DNA
<213> Corynebacterium glutamicum

<220>
<221> CDS
<222> (101)..(535)
<223> FRXA01795

<400> 163
agaccatatt gaagacctcg aagctgttga gcctggctac atcgtcaagc ctcgcctgta 60

caacttcgct gaatacggtg tcccacaatt ccgcgaacgt gtg ctc att gtt ggc    115
                                           Val Leu Ile Val Gly
                                           1               5

att cgc cgt gac acc ggc ttt gat ttc aag cac cca gct cct acc cat    163
Ile Arg Arg Asp Thr Gly Phe Asp Phe Lys His Pro Ala Pro Thr His
                10                  15                  20

ggc cct cgc ggt gac atg ccg tat aag act gcc ggc gaa gcg ctc aaa    211
Gly Pro Arg Gly Asp Met Pro Tyr Lys Thr Ala Gly Glu Ala Leu Lys
            25                  30                  35

ggc gtg aag gat gtc ccc aca aac aac aac cac atg aag atc atg cct    259
Gly Val Lys Asp Val Pro Thr Asn Asn Asn His Met Lys Ile Met Pro
        40                  45                  50

cgc acc gtt gaa gtg ctt aag cgc atc cct gag ggc gaa aac ttc acc    307
Arg Thr Val Glu Val Leu Lys Arg Ile Pro Glu Gly Glu Asn Phe Thr
    55                  60                  65

gcg atc ccc aaa gat gac ccc tac tac gtc aag ggc atg att agt cac    355
Ala Ile Pro Lys Asp Asp Pro Tyr Tyr Val Lys Gly Met Ile Ser His
70                  75                  80                  85

gtt tac cgt cgc ttg cac cgt gat gag cca tcc aaa acc ctt atc gcc    403
Val Tyr Arg Arg Leu His Arg Asp Glu Pro Ser Lys Thr Leu Ile Ala
                90                  95                  100

ggt ggc ggc ggg ggt aca tgg gga tac cat tat gaa aaa aat cga gca    451
Gly Gly Gly Gly Gly Thr Trp Gly Tyr His Tyr Glu Lys Asn Arg Ala
            105                 110                 115

ttg acc aac cgc gag cgg gct aga att caa tcg ttc ccc gat gac ttt    499
Leu Thr Asn Arg Glu Arg Ala Arg Ile Gln Ser Phe Pro Asp Asp Phe
        120                 125                 130

gag ttt ttg gga tca aac acc caa gtc cgc cgc caa                    535
Glu Phe Leu Gly Ser Asn Thr Gln Val Arg Arg Gln
    135                 140                 145


<210> 164

<211> 145
<212> PRT
<213> Corynebacterium glutamicum

<400> 164
Val Leu Ile Val Gly Ile Arg Arg Asp Thr Gly Phe Asp Phe Lys His
1               5                   10                  15

Pro Ala Pro Thr His Gly Pro Arg Gly Asp Met Pro Tyr Lys Thr Ala
            20                  25                  30

Gly Glu Ala Leu Lys Gly Val Lys Asp Val Pro Thr Asn Asn Asn His
        35                  40                  45

Met Lys Ile Met Pro Arg Thr Val Glu Val Leu Lys Arg Ile Pro Glu
    50                  55                  60

Gly Glu Asn Phe Thr Ala Ile Pro Lys Asp Asp Pro Tyr Tyr Val Lys
65                  70                  75                  80

Gly Met Ile Ser His Val Tyr Arg Arg Leu His Arg Asp Glu Pro Ser
                85                  90                  95

Lys Thr Leu Ile Ala Gly Gly Gly Gly Gly Thr Trp Gly Tyr His Tyr
            100                 105                 110

Glu Lys Asn Arg Ala Leu Thr Asn Arg Glu Arg Ala Arg Ile Gln Ser
        115                 120                 125

Phe Pro Asp Asp Phe Glu Phe Leu Gly Ser Asn Thr Gln Val Arg Arg
    130                 135                 140

Gln
145


<210> 165
<211> 1614
<212> DNA
<213> Corynebacterium glutamicum

<220>
<221> CDS
<222> (101)..(1591)
<223> RXA01214

<400> 165
gttgatatga tgtggggtgt ttatgaaaat ttgtttgagg gagtgaaggc gcatgttgtt 60

gccagagttg aatcgtcgga ctttttcaa aggggccggg gtg ctg gca gca acg    115
                                          Val Leu Ala Ala Thr
                                          1               5

gtg gtg ggt gcg cag gtg ctg gtg gcg tgt tcc tca gat gat gtg cgt    163
Val Val Gly Ala Gln Val Leu Val Ala Cys Ser Ser Asp Asp Val Arg
                10                  15                  20

ggt tat ggg gga gag ccg cgg acg ttg cct att cca cca gca gat tta    211
Gly Tyr Gly Gly Glu Pro Arg Thr Leu Pro Ile Pro Pro Ala Asp Leu
            25                  30                  35

ggt acg cgt gag gga tct agc gtg cac ttt gcc ctg gag gct cag act    259
Gly Thr Arg Glu Gly Ser Ser Val His Phe Ala Leu Glu Ala Gln Thr
        40                  45                  50

ggg gag agt cag att ttg ccg gat gtc aca acg aag acg tgg ggt ttc    307
Gly Glu Ser Gln Ile Leu Pro Asp Val Thr Thr Lys Thr Trp Gly Phe
    55                  60                  65

aat ggc act cat ttg ggg ccg acg ttg gtg gtg aag aaa ggt gat gac    355
Asn Gly Thr His Leu Gly Pro Thr Leu Val Val Lys Lys Gly Asp Asp
70                  75                  80                  85

gtc cac gtt gat gtg ata aac aat ttg gat gaa atg acc act gtg cac    403
Val His Val Asp Val Ile Asn Asn Leu Asp Glu Met Thr Thr Val His
                90                  95                  100

tgg cat ggc atg aag ttg ccg gcg att gct gat ggt ggt ccg cac tca     451
Trp His Gly Met Lys Leu Pro Ala Ile Ala Asp Gly Gly Pro His Ser
        105                 110                 115

ccg atc ggg cct ggg cag acg tgg tca cca acg tgg act gtg gcc aat     499
Pro Ile Gly Pro Gly Gln Thr Trp Ser Pro Thr Trp Thr Val Ala Asn
        120                 125                 130

gat gca gcc act ttg tgg tac cac ccg cac act cat ggc ctg aca ggt     547
Asp Ala Ala Thr Leu Trp Tyr His Pro His Thr His Gly Leu Thr Gly
    135                 140                 145

ttg cat gcg tac cgt ggt ttg gcg ggg atg atc att gtg gaa gat gaa     595
Leu His Ala Tyr Arg Gly Leu Ala Gly Met Ile Ile Val Glu Asp Glu
150                 155                 160                 165

gca aca gac aag ctg gat ctg cca cgc gag tac ggt gtg gac gat att     643
Ala Thr Asp Lys Leu Asp Leu Pro Arg Glu Tyr Gly Val Asp Asp Ile
                170                 175                 180

ccg ctg gtt tta atg gat cac cgc ttc tta gaa gac ggt tcc ctt gat     691
Pro Leu Val Leu Met Asp His Arg Phe Leu Glu Asp Gly Ser Leu Asp
            185                 190                 195

gag gaa gac ctc ccc gat ctt ggg ctg ttg ggc gat acc ccc act gcc     739
Glu Glu Asp Leu Pro Asp Leu Gly Leu Leu Gly Asp Thr Pro Thr Ala
        200                 205                 210

aat ggc att acc aat gcg cac ttt gat gcc acc acg cgc cgg gtt cgg     787
Asn Gly Ile Thr Asn Ala His Phe Asp Ala Thr Thr Arg Arg Val Arg
    215                 220                 225

ttc cgc gtg ctc aac ggc tcc aat atg cgg ttc tat aac ttg gcg ttt     835
Phe Arg Val Leu Asn Gly Ser Asn Met Arg Phe Tyr Asn Leu Ala Phe
230                 235                 240                 245

tca gac acg cgc acc ttc caa gtc att gcc agc gat tcc ggt ttg ctg     883
Ser Asp Thr Arg Thr Phe Gln Val Ile Ala Ser Asp Ser Gly Leu Leu
                250                 255                 260

gat gaa cct caa gac cgc acc acc ttg gct att ggc cca ggc gag cgg     931
Asp Glu Pro Gln Asp Arg Thr Thr Leu Ala Ile Gly Pro Gly Glu Arg
            265                 270                 275

tgg gaa atc gtc gtg gag cta gag ccc ggc gag gac gtc acc ttg gaa     979
Trp Glu Ile Val Val Glu Leu Glu Pro Gly Glu Asp Val Thr Leu Glu
        280                 285                 290

tct gta ggt ttt gag gac aac tac ggc gtc cct gat gat gag ttc gtg     1027
Ser Val Gly Phe Glu Asp Asn Tyr Gly Val Pro Asp Asp Glu Phe Val
    295                 300                 305

ccc gat ttc ggc atg tca gat tcc ttc cag ctg ctc acc atc acc ggc     1075
Pro Asp Phe Gly Met Ser Asp Ser Phe Gln Leu Leu Thr Ile Thr Gly
310                 315                 320                 325

cct tcc gat gat gct gcg caa gca cct gct ttg ccg ggc gtg ctg gtg     1123
Pro Ser Asp Asp Ala Ala Gln Ala Pro Ala Leu Pro Gly Val Leu Val
                330                 335                 340

aaa tcc acc gaa cct gac gtc atc gat gcc act gaa cgc acc ttc atc     1171
Lys Ser Thr Glu Pro Asp Val Ile Asp Ala Thr Glu Arg Thr Phe Ile
            345                 350                 355

atg aac acc ttc tcc atc aac gat cta cag atg gac atg cag cgc gtt     1219
Met Asn Thr Phe Ser Ile Asn Asp Leu Gln Met Asp Met Gln Arg Val
        360                 365                 370

gac gtg gtg att gac cat gac cag cca gaa gtg tgg att gtc acc aac     1267
Asp Val Val Ile Asp His Asp Gln Pro Glu Val Trp Ile Val Thr Asn
    375                 380                 385

gac aac tcc gac tgg ccc cac aac ttc cat gtc cac gac gcc cgg ttt     1315
Asp Asn Ser Asp Trp Pro His Asn Phe His Val His Asp Ala Arg Phe
390                 395                 400                 405

aag gtg ctg aaa ttt gaa ggc acc gac gta gag ctc ttc aac gac ggc     1363

Lys Val Leu Lys Phe Glu Gly Thr Asp Val Glu Leu Phe Asn Asp Gly
                410                 415                 420

tgg aaa gac acc gtc ggc ctg cca ccg gga gca acc gca act tta gcc      1411
Trp Lys Asp Thr Val Gly Leu Pro Pro Gly Ala Thr Ala Thr Leu Ala
            425                 430                 435

gtg gaa ttt ggc cac tac cca gac ccg caa tgg ccc tac atg tat cac      1459
Val Glu Phe Gly His Tyr Pro Asp Pro Gln Trp Pro Tyr Met Tyr His
        440                 445                 450

tgc cac atg ctc tac cac gag gat caa ggc atg atg ggg cag ttc gtc      1507
Cys His Met Leu Tyr His Glu Asp Gln Gly Met Met Gly Gln Phe Val
    455                 460                 465

atc gtg gag cca ggc gac gag ccg gcg gcg gtg ctg ggg tcg ggc acg      1555
Ile Val Glu Pro Gly Asp Glu Pro Ala Ala Val Leu Gly Ser Gly Thr
470                 475                 480                 485

ggc tcc agc att gac tcc gcc ggc gga cat gcg cac taggggcgtg           1601
Gly Ser Ser Ile Asp Ser Ala Gly Gly His Ala His
                490                 495

gggcggcgtc gat                                                       1614


<210> 166
<211> 497
<212> PRT
<213> Corynebacterium glutamicum

<400> 166
Val Leu Ala Ala Thr Val Val Gly Ala Gln Val Leu Val Ala Cys Ser
1               5                   10                  15

Ser Asp Asp Val Arg Gly Tyr Gly Gly Glu Pro Arg Thr Leu Pro Ile
            20                  25                  30

Pro Pro Ala Asp Leu Gly Thr Arg Glu Gly Ser Ser Val His Phe Ala
        35                  40                  45

Leu Glu Ala Gln Thr Gly Glu Ser Gln Ile Leu Pro Asp Val Thr Thr
    50                  55                  60

Lys Thr Trp Gly Phe Asn Gly Thr His Leu Gly Pro Thr Leu Val Val
65                  70                  75                  80

Lys Lys Gly Asp Asp Val His Val Asp Val Ile Asn Asn Leu Asp Glu
                85                  90                  95

Met Thr Thr Val His Trp His Gly Met Lys Leu Pro Ala Ile Ala Asp
            100                 105                 110

Gly Gly Pro His Ser Pro Ile Gly Pro Gly Gln Thr Trp Ser Pro Thr
        115                 120                 125

Trp Thr Val Ala Asn Asp Ala Ala Thr Leu Trp Tyr His Pro His Thr
    130                 135                 140

His Gly Leu Thr Gly Leu His Ala Tyr Arg Gly Leu Ala Gly Met Ile
145                 150                 155                 160

Ile Val Glu Asp Glu Ala Thr Asp Lys Leu Asp Leu Pro Arg Glu Tyr
                165                 170                 175

Gly Val Asp Asp Ile Pro Leu Val Leu Met Asp His Arg Phe Leu Glu
            180                 185                 190

Asp Gly Ser Leu Asp Glu Glu Asp Leu Pro Asp Leu Gly Leu Leu Gly
        195                 200                 205

Asp Thr Pro Thr Ala Asn Gly Ile Thr Asn Ala His Phe Asp Ala Thr
    210                 215                 220

Thr Arg Arg Val Arg Phe Arg Val Leu Asn Gly Ser Asn Met Arg Phe
225                 230                 235                 240

Tyr Asn Leu Ala Phe Ser Asp Thr Arg Thr Phe Gln Val Ile Ala Ser
                245                 250                 255

Asp Ser Gly Leu Leu Asp Glu Pro Gln Asp Arg Thr Thr Leu Ala Ile
            260                 265                 270

Gly Pro Gly Glu Arg Trp Glu Ile Val Val Glu Leu Glu Pro Gly Glu
        275                 280                 285

Asp Val Thr Leu Glu Ser Val Gly Phe Glu Asp Asn Tyr Gly Val Pro
    290                 295                 300

Asp Asp Glu Phe Val Pro Asp Phe Gly Met Ser Asp Ser Phe Gln Leu
305                 310                 315                 320

Leu Thr Ile Thr Gly Pro Ser Asp Asp Ala Ala Gln Ala Pro Ala Leu
                325                 330                 335

Pro Gly Val Leu Val Lys Ser Thr Glu Pro Asp Val Ile Asp Ala Thr
            340                 345                 350

Glu Arg Thr Phe Ile Met Asn Thr Phe Ser Ile Asn Asp Leu Gln Met
        355                 360                 365

Asp Met Gln Arg Val Asp Val Val Ile Asp His Asp Gln Pro Glu Val
    370                 375                 380

Trp Ile Val Thr Asn Asp Asn Ser Asp Trp Pro His Asn Phe His Val
385                 390                 395                 400

His Asp Ala Arg Phe Lys Val Leu Lys Phe Glu Gly Thr Asp Val Glu
                405                 410                 415

Leu Phe Asn Asp Gly Trp Lys Asp Thr Val Gly Leu Pro Pro Gly Ala
            420                 425                 430

Thr Ala Thr Leu Ala Val Glu Phe Gly His Tyr Pro Asp Pro Gln Trp
        435                 440                 445

Pro Tyr Met Tyr His Cys His Met Leu Tyr His Glu Asp Gln Gly Met
    450                 455                 460

Met Gly Gln Phe Val Ile Val Glu Pro Gly Asp Glu Pro Ala Ala Val
465                 470                 475                 480

Leu Gly Ser Gly Thr Gly Ser Ser Ile Asp Ser Ala Gly Gly His Ala
                485                 490                 495

His

<210> 167
<211> 588
<212> DNA
<213> Corynebacterium glutamicum

<220>
<221> CDS
<222> (1)..(588)
<223> RXA01250

<400> 167
ctg gat atc ggc ggc atc gaa gcc aag acg tgg gga tac gtc tct gac      48
Leu Asp Ile Gly Gly Ile Glu Ala Lys Thr Trp Gly Tyr Val Ser Asp
  1               5                  10                  15

acc ggg gat gcg gcc att gag gcc acc gcc ggc gac gtc ctc cag gtc      96
Thr Gly Asp Ala Ala Ile Glu Ala Thr Ala Gly Asp Val Leu Gln Val
             20                  25                  30

gat atc acc aat gac ctg cct gag agc acc tcc atc cac tgg cat ggc     144
Asp Ile Thr Asn Asp Leu Pro Glu Ser Thr Ser Ile His Trp His Gly
         35                  40                  45

atc gca ctc cac aac gca gcc gac ggt gtg ccc ggc atg acc cag gac     192
Ile Ala Leu His Asn Ala Ala Asp Gly Val Pro Gly Met Thr Gln Asp

50                  55                  60

ccc att gaa cct ggc gag tct ttc tcc tat gtt ttt gaa gtc ccc cac      240
Pro Ile Glu Pro Gly Glu Ser Phe Ser Tyr Val Phe Glu Val Pro His
65                  70                  75                  80

ggt ggc acc tac ttc tac cat tcc cac acc ggc ctg cag ctt gat cgc      288
Gly Gly Thr Tyr Phe Tyr His Ser His Thr Gly Leu Gln Leu Asp Arg
                85                  90                  95

ggc ctc cac gcc cca ctg atc atc cgt gac ccg caa gac gct gag gac      336
Gly Leu His Ala Pro Leu Ile Ile Arg Asp Pro Gln Asp Ala Glu Asp
            100                 105                 110

cag gac gtc gag tgg acc atc gtg ctc gac gac tgg gtc gat ggc att      384
Gln Asp Val Glu Trp Thr Ile Val Leu Asp Asp Trp Val Asp Gly Ile
        115                 120                 125

cag ggc act ccc gac gat gag ctc gac aag ctc acc gga atg ggt tcg      432
Gln Gly Thr Pro Asp Asp Glu Leu Asp Lys Leu Thr Gly Met Gly Ser
    130                 135                 140

ggc gac cat aac ggg agg atg gga atg gga ggt cac ggc cag atg atg      480
Gly Asp His Asn Gly Arg Met Gly Met Gly Gly His Gly Gln Met Met
145                 150                 155                 160

cac ggc acc ccg gac cgg gta ctg ggc ggg gat gtc ggc gat gtg atg      528
His Gly Thr Pro Asp Arg Val Leu Gly Gly Asp Val Gly Asp Val Met
                165                 170                 175

tat ccg cac tac ctc atc aac gga cgt atc ccc cgt gct cac cgg acc      576
Tyr Pro His Tyr Leu Ile Asn Gly Arg Ile Pro Arg Ala His Arg Thr
            180                 185                 190

ttc gag gct cgc                                                      588
Phe Glu Ala Arg
        195


<210> 168
<211> 196
<212> PRT
<213> Corynebacterium glutamicum

<400> 168
Leu Asp Ile Gly Gly Ile Glu Ala Lys Thr Trp Gly Tyr Val Ser Asp
1               5                   10                  15

Thr Gly Asp Ala Ala Ile Glu Ala Thr Ala Gly Asp Val Leu Gln Val
            20                  25                  30

Asp Ile Thr Asn Asp Leu Pro Glu Ser Thr Ser Ile His Trp His Gly
        35                  40                  45

Ile Ala Leu His Asn Ala Ala Asp Gly Val Pro Gly Met Thr Gln Asp
    50                  55                  60

Pro Ile Glu Pro Gly Glu Ser Phe Ser Tyr Val Phe Glu Val Pro His
65                  70                  75                  80

Gly Gly Thr Tyr Phe Tyr His Ser His Thr Gly Leu Gln Leu Asp Arg
                85                  90                  95

Gly Leu His Ala Pro Leu Ile Ile Arg Asp Pro Gln Asp Ala Glu Asp
            100                 105                 110

Gln Asp Val Glu Trp Thr Ile Val Leu Asp Asp Trp Val Asp Gly Ile
        115                 120                 125

Gln Gly Thr Pro Asp Asp Glu Leu Asp Lys Leu Thr Gly Met Gly Ser
    130                 135                 140

Gly Asp His Asn Gly Arg Met Gly Met Gly Gly His Gly Gln Met Met
145                 150                 155                 160

His Gly Thr Pro Asp Arg Val Leu Gly Gly Asp Val Gly Asp Val Met
                165                 170                 175

Tyr Pro His Tyr Leu Ile Asn Gly Arg Ile Pro Arg Ala His Arg Thr
            180                 185                 190

Phe Glu Ala Arg
        195


<210> 169
<211> 744
<212> DNA
<213> Corynebacterium glutamicum

<220>
<221> CDS
<222> (101)..(721)
<223> RXA02477

<400> 169
cgagcagggc tgtttgaaaa gctgtaaatg acatgaccta aatgattgta ctgactggca 60

ctttaggtca tatgtcacac cgagtggaat aataaagctt atg cct ttg cgt aat   115
                                           Met Pro Leu Arg Asn
                                           1               5

gtt gat aga act ccg ccc gca gta tgg gaa gca ttg ctt gcc gga aac   163
Val Asp Arg Thr Pro Pro Ala Val Trp Glu Ala Leu Leu Ala Gly Asn
                10                  15                  20

gaa aga ttc atc agt ttc aac gaa gat cga cca aac cag gac gcc ccg   211
Glu Arg Phe Ile Ser Phe Asn Glu Asp Arg Pro Asn Gln Asp Ala Pro
            25                  30                  35

cgc aga aga gaa ctt cgc aat gga caa acg cct gca gct gtt gtt att   259
Arg Arg Arg Glu Leu Arg Asn Gly Gln Thr Pro Ala Ala Val Val Ile
        40                  45                  50

tcc tgt tca gat tct cga gtg cca gtt gag att att ttt gac gtc ggt   307
Ser Cys Ser Asp Ser Arg Val Pro Val Glu Ile Ile Phe Asp Val Gly
    55                  60                  65

ctc ggt gac ctc ttt gtt gtc cgt act gcc gga gaa atc ctc gac caa   355
Leu Gly Asp Leu Phe Val Val Arg Thr Ala Gly Glu Ile Leu Asp Gln
70                  75                  80                  85

gca gtg ctt gcg tcc atc gaa tac gcc act gaa tcc atc ggc gtt cca   403
Ala Val Leu Ala Ser Ile Glu Tyr Ala Thr Glu Ser Ile Gly Val Pro
                90                  95                  100

ttg gtt atc gtc atg ggc cac gaa tcc tgt ggt gca gtt gca gca act   451
Leu Val Ile Val Met Gly His Glu Ser Cys Gly Ala Val Ala Ala Thr
            105                 110                 115

gca gca gca ctt gaa ggc ggt gca ctt ccc gga ggc tac caa cga gtt   499
Ala Ala Ala Leu Glu Gly Gly Ala Leu Pro Gly Gly Tyr Gln Arg Val
        120                 125                 130

ttg gtt gaa aag gtt gca cca tcc att cta gaa gcc aag gca gag ggc   547
Leu Val Glu Lys Val Ala Pro Ser Ile Leu Glu Ala Lys Ala Glu Gly
    135                 140                 145

ctg agc tcc atc aag gaa ttc gag gaa cac cac gtt gtg gca acg gta   595
Leu Ser Ser Ile Lys Glu Phe Glu Glu His His Val Val Ala Thr Val
150                 155                 160                 165

aac caa ctg ttg tcc cgt tct cca gag att cat cag aag gtc gaa acc   643
Asn Gln Leu Leu Ser Arg Ser Pro Glu Ile His Gln Lys Val Glu Thr
                170                 175                 180

ggt gag ttg gga atc att ggt ttg cgc tac cga ctc tct gac ggt cgt   691
Gly Glu Leu Gly Ile Ile Gly Leu Arg Tyr Arg Leu Ser Asp Gly Arg
            185                 190                 195

act gaa cct gta att agc aag aac gtg ggt tagttttcgg tctgagattg     741
Thr Glu Pro Val Ile Ser Lys Asn Val Gly
        200                 205

cct                                                                 744

<210> 170
<211> 207
<212> PRT
<213> Corynebacterium glutamicum

<400> 170
Met Pro Leu Arg Asn Val Asp Arg Thr Pro Pro Ala Val Trp Glu Ala
1               5                   10                  15

Leu Leu Ala Gly Asn Glu Arg Phe Ile Ser Phe Asn Glu Asp Arg Pro
            20                  25                  30

Asn Gln Asp Ala Pro Arg Arg Arg Glu Leu Arg Asn Gly Gln Thr Pro
        35                  40                  45

Ala Ala Val Val Ile Ser Cys Ser Asp Ser Arg Val Pro Val Glu Ile
    50                  55                  60

Ile Phe Asp Val Gly Leu Gly Asp Leu Phe Val Val Arg Thr Ala Gly
65                  70                  75                  80

Glu Ile Leu Asp Gln Ala Val Leu Ala Ser Ile Glu Tyr Ala Thr Glu
                85                  90                  95

Ser Ile Gly Val Pro Leu Val Ile Val Met Gly His Glu Ser Cys Gly
            100                 105                 110

Ala Val Ala Ala Thr Ala Ala Ala Leu Glu Gly Gly Ala Leu Pro Gly
        115                 120                 125

Gly Tyr Gln Arg Val Leu Val Glu Lys Val Ala Pro Ser Ile Leu Glu
    130                 135                 140

Ala Lys Ala Glu Gly Leu Ser Ser Ile Lys Glu Phe Glu Glu His His
145                 150                 155                 160

Val Val Ala Thr Val Asn Gln Leu Leu Ser Arg Ser Pro Glu Ile His
                165                 170                 175

Gln Lys Val Glu Thr Gly Glu Leu Gly Ile Ile Gly Leu Arg Tyr Arg
            180                 185                 190

Leu Ser Asp Gly Arg Thr Glu Pro Val Ile Ser Lys Asn Val Gly
        195                 200                 205


<210> 171
<211> 618
<212> DNA
<213> Corynebacterium glutamicum

<220>
<221> CDS
<222> (101)..(595)
<223> RXN00833

<400> 171
agctttttgc atgtgtcata tcgtaccgtt tgcataggcc tgttcgcgct tggtgaacct 60

tttctagcac caaaacaaaa ctctccctag tatggggtcc atg gct aaa aca cat   115
                                            Met Ala Lys Thr His
                                            1               5

ttt caa ggc aac gaa act gct acc tcc ggc gaa ctg cca cag gtc ggc   163
Phe Gln Gly Asn Glu Thr Ala Thr Ser Gly Glu Leu Pro Gln Val Gly
                10                  15                  20

gac aac ctc gca gag ttc aac ctc gtc aac acc gaa ctg ggc gag gtc   211
Asp Asn Leu Ala Glu Phe Asn Leu Val Asn Thr Glu Leu Gly Glu Val
            25                  30                  35

tcc tca aag gac ttc cag ggc cgc aag ctt gtc ctg aac atc ttc cca   259
Ser Ser Lys Asp Phe Gln Gly Arg Lys Leu Val Leu Asn Ile Phe Pro
        40                  45                  50

tcc gtt gac acc ggc gtt tgt gca aca tca gtc cgc aag ttc aac gag     307
Ser Val Asp Thr Gly Val Cys Ala Thr Ser Val Arg Lys Phe Asn Glu
     55                  60                  65

gca gca gca agc ctg gaa aac acc acc gtg ctg tgc atc tcc aag gat     355
Ala Ala Ala Ser Leu Glu Asn Thr Thr Val Leu Cys Ile Ser Lys Asp
 70                  75                  80                  85

ctt cca ttc gca ctg ggc cgt ttc tgc tcc gca gaa ggc atc gag aac     403
Leu Pro Phe Ala Leu Gly Arg Phe Cys Ser Ala Glu Gly Ile Glu Asn
                 90                  95                 100

gtc acc cca gta tcc gca ttc cgt tcc acc ttc ggt gaa gac aac ggc     451
Val Thr Pro Val Ser Ala Phe Arg Ser Thr Phe Gly Glu Asp Asn Gly
            105                 110                 115

atc gtg ctc gaa ggc tca cca ctt aag ggt ctt ctt gca cgc agc gtc     499
Ile Val Leu Glu Gly Ser Pro Leu Lys Gly Leu Leu Ala Arg Ser Val
        120                 125                 130

atc gtc gtc gat gaa aac ggc aag gtt gct tac acc cag ttg gtt gat     547
Ile Val Val Asp Glu Asn Gly Lys Val Ala Tyr Thr Gln Leu Val Asp
    135                 140                 145

gag atc ttc act gaa cct gat tac gac gct gca ctt gct ggg ctg aac     595
Glu Ile Phe Thr Glu Pro Asp Tyr Asp Ala Ala Leu Ala Gly Leu Asn
150                 155                 160                 165

taatttactt cgctcagggg aat                                           618


<210> 172
<211> 165
<212> PRT
<213> Corynebacterium glutamicum

<400> 172
Met Ala Lys Thr His Phe Gln Gly Asn Glu Thr Ala Thr Ser Gly Glu
  1               5                  10                  15

Leu Pro Gln Val Gly Asp Asn Leu Ala Glu Phe Asn Leu Val Asn Thr
             20                  25                  30

Glu Leu Gly Glu Val Ser Ser Lys Asp Phe Gln Gly Arg Lys Leu Val
         35                  40                  45

Leu Asn Ile Phe Pro Ser Val Asp Thr Gly Val Cys Ala Thr Ser Val
     50                  55                  60

Arg Lys Phe Asn Glu Ala Ala Ala Ser Leu Glu Asn Thr Thr Val Leu
 65                  70                  75                  80

Cys Ile Ser Lys Asp Leu Pro Phe Ala Leu Gly Arg Phe Cys Ser Ala
                 85                  90                  95

Glu Gly Ile Glu Asn Val Thr Pro Val Ser Ala Phe Arg Ser Thr Phe
            100                 105                 110

Gly Glu Asp Asn Gly Ile Val Leu Glu Gly Ser Pro Leu Lys Gly Leu
        115                 120                 125

Leu Ala Arg Ser Val Ile Val Val Asp Glu Asn Gly Lys Val Ala Tyr
    130                 135                 140

Thr Gln Leu Val Asp Glu Ile Phe Thr Glu Pro Asp Tyr Asp Ala Ala
145                 150                 155                 160

Leu Ala Gly Leu Asn
                165


<210> 173
<211> 469
<212> DNA
<213> Corynebacterium glutamicum

<220>
<221> CDS
<222> (101)..(469)
<223> FRXA00833

<400> 173
agctttttgc atgtgtcata tcgtaccgtt tgcataggcc tgttcgcgct tggtgaacct 60

tttctagcac caaaacaaaa ctctccctag tatggggtcc atg gct aaa aca cat 115
Met Ala Lys Thr His
1               5

ttt caa ggc aac gaa act gct acc tcc ggc gaa ctg cca cag gtc ggc 163
Phe Gln Gly Asn Glu Thr Ala Thr Ser Gly Glu Leu Pro Gln Val Gly
                10                  15                  20

gac aac ctc gca gag ttc aac ctc gtc aac acc gaa ctg ggc gag gtc 211
Asp Asn Leu Ala Glu Phe Asn Leu Val Asn Thr Glu Leu Gly Glu Val
            25                  30                  35

tcc tca aag gac ttc cag ggc cgc aag ctt gtc ctg aac atc ttc cca 259
Ser Ser Lys Asp Phe Gln Gly Arg Lys Leu Val Leu Asn Ile Phe Pro
        40                  45                  50

tcc gtt gac acc ggc gtt tgt gca aca tca gtc cgc aag ttc aac gag 307
Ser Val Asp Thr Gly Val Cys Ala Thr Ser Val Arg Lys Phe Asn Glu
    55                  60                  65

gca gca gca agc ctg gaa aac acc acc gtg ctg tgc atc tcc aag gat 355
Ala Ala Ala Ser Leu Glu Asn Thr Thr Val Leu Cys Ile Ser Lys Asp
70                  75                  80                  85

ctt cca ttc gca ctg ggc cgt ttc tgc tcc gca gaa ggc atc gag aac 403
Leu Pro Phe Ala Leu Gly Arg Phe Cys Ser Ala Glu Gly Ile Glu Asn
                90                  95                  100

gtc acc cca gta tcc gca ttc cgt tcc acc ttc ggt gaa gac aac ggc 451
Val Thr Pro Val Ser Ala Phe Arg Ser Thr Phe Gly Glu Asp Asn Gly
            105                 110                 115

atc gtg ctc gaa ggc tca 469
Ile Val Leu Glu Gly Ser
        120


<210> 174
<211> 123
<212> PRT
<213> Corynebacterium glutamicum

<400> 174
Met Ala Lys Thr His Phe Gln Gly Asn Glu Thr Ala Thr Ser Gly Glu
1               5                   10                  15

Leu Pro Gln Val Gly Asp Asn Leu Ala Glu Phe Asn Leu Val Asn Thr
            20                  25                  30

Glu Leu Gly Glu Val Ser Ser Lys Asp Phe Gln Gly Arg Lys Leu Val
        35                  40                  45

Leu Asn Ile Phe Pro Ser Val Asp Thr Gly Val Cys Ala Thr Ser Val
    50                  55                  60

Arg Lys Phe Asn Glu Ala Ala Ala Ser Leu Glu Asn Thr Thr Val Leu
65                  70                  75                  80

Cys Ile Ser Lys Asp Leu Pro Phe Ala Leu Gly Arg Phe Cys Ser Ala
                85                  90                  95

Glu Gly Ile Glu Asn Val Thr Pro Val Ser Ala Phe Arg Ser Thr Phe
            100                 105                 110

Gly Glu Asp Asn Gly Ile Val Leu Glu Gly Ser
        115                 120


<210> 175

<211> 1146
<212> DNA
<213> Corynebacterium glutamicum

<220>
<221> CDS
<222> (101)..(1123)
<223> RXA01224

<400> 175
ttggcggcgg gaagttcagg cttgggggca aacagtgctt ggattttaga caaaaaactc 60

acggaagtca tcctatggca ggcgcgccta ggatggtgcc atg agc atc ctt gac 115
Met Ser Ile Leu Asp
1               5

acg ttg aaa act ccc gtg att gtc gcc ccg atg gct ggc ggc ccg tcc 163
Thr Leu Lys Thr Pro Val Ile Val Ala Pro Met Ala Gly Gly Pro Ser
                10                  15                  20

act ccc gcg ttg gtc aat gca gca gca gag gca ggt tcc ctc ggg ttc 211
Thr Pro Ala Leu Val Asn Ala Ala Ala Glu Ala Gly Ser Leu Gly Phe
            25                  30                  35

ttg gct ggt ggc gtc atg cct ctt gag cag ctg aaa cag gaa ttg tca 259
Leu Ala Gly Gly Val Met Pro Leu Glu Gln Leu Lys Gln Glu Leu Ser
        40                  45                  50

gag gta aaa ggc gtc ttt ggc gtc aac ctg ttt cgc ccg cag acg gat 307
Glu Val Lys Gly Val Phe Gly Val Asn Leu Phe Arg Pro Gln Thr Asp
    55                  60                  65

gcg cct aag cct tca gac att gat gag ctg gcg gga ttg ttg tcc tcg 355
Ala Pro Lys Pro Ser Asp Ile Asp Glu Leu Ala Gly Leu Leu Ser Ser
70                  75                  80                  85

gcg ttt cgg caa ttt ggc ctc gat gag ccg acg gtg cct acg ccg gat 403
Ala Phe Arg Gln Phe Gly Leu Asp Glu Pro Thr Val Pro Thr Pro Asp
                90                  95                  100

ttg agc aat ggg tgg gag gct aaa ttt gag gcc gtt ctt gcc gct aag 451
Leu Ser Asn Gly Trp Glu Ala Lys Phe Glu Ala Val Leu Ala Ala Lys
            105                 110                 115

ccc gcc gtt ttt tcc tgc acc ttt ggt att ttt agc gct gaa gaa ttc 499
Pro Ala Val Phe Ser Cys Thr Phe Gly Ile Phe Ser Ala Glu Glu Phe
        120                 125                 130

gcc cgg atc aaa gcc acc gga att gag gcg tgg gtg acg gtg acc aat 547
Ala Arg Ile Lys Ala Thr Gly Ile Glu Ala Trp Val Thr Val Thr Asn
    135                 140                 145

ccg gag gac gcg ctg gct gcg cag aaa gct ggc gcc aac gcg ctt gtc 595
Pro Glu Asp Ala Leu Ala Ala Gln Lys Ala Gly Ala Asn Ala Leu Val
150                 155                 160                 165

gtg caa ggc ccc gag gcg ggt ggg cac cgc tct acc tgg tcc att gaa 643
Val Gln Gly Pro Glu Ala Gly Gly His Arg Ser Thr Trp Ser Ile Glu
                170                 175                 180

gtg gag ccg gac gag cgc gac ctg aaa acc ctc ctc gca gct gtc aaa 691
Val Glu Pro Asp Glu Arg Asp Leu Lys Thr Leu Leu Ala Ala Val Lys
            185                 190                 195

caa gcg ggc gtt tac ctc ccg ctc atc gca gcc ggc ggc ctt tca acc 739
Gln Ala Gly Val Tyr Leu Pro Leu Ile Ala Ala Gly Gly Leu Ser Thr
        200                 205                 210

tcc gca gac gtg gca gca att tta gaa gcc ggc gcc agc gct gcc tcc 787
Ser Ala Asp Val Ala Ala Ile Leu Glu Ala Gly Ala Ser Ala Ala Ser
    215                 220                 225

tgt ggt tcc gcc ttt ttg ctt agc gac gaa gcc ggc acc agc tca ctt 835
Cys Gly Ser Ala Phe Leu Leu Ser Asp Glu Ala Gly Thr Ser Ser Leu
230                 235                 240                 245

aac cgc gag atc ttg gac gcc gcc cca gca ctt ggt ttg gaa tcg gtg 883

Asn Arg Glu Ile Leu Asp Ala Ala Pro Ala Leu Gly Leu Glu Ser Val
                250                 255                 260

tca tct cgc gca ttt tcg ggc cgt tat gcc agg gga gtg gaa acc agg     931
Ser Ser Arg Ala Phe Ser Gly Arg Tyr Ala Arg Gly Val Glu Thr Arg
            265                 270                 275

ttc acc cgt tcg aac gag ggg tta ccc ccg ttg tac cca tac ctc aac     979
Phe Thr Arg Ser Asn Glu Gly Leu Pro Pro Leu Tyr Pro Tyr Leu Asn
        280                 285                 290

cca atg atc aca tct tta cgt aag gtg gcg gga agt gca ggg aac tgg     1027
Pro Met Ile Thr Ser Leu Arg Lys Val Ala Gly Ser Ala Gly Asn Trp
    295                 300                 305

gat tac gcc tac tgc ctg gta gga gtc ggc ctg gaa tcg att gcg aag     1075
Asp Tyr Ala Tyr Cys Leu Val Gly Val Gly Leu Glu Ser Ile Ala Lys
310                 315                 320                 325

ggt agt gca aag cag ata ctg gaa tca tta aca cct tcc gct ttg ggc     1123
Gly Ser Ala Lys Gln Ile Leu Glu Ser Leu Thr Pro Ser Ala Leu Gly
                330                 335                 340

taatgttggg gggagtgctt tca                                           1146


<210> 176
<211> 341
<212> PRT
<213> Corynebacterium glutamicum

<400> 176
Met Ser Ile Leu Asp Thr Leu Lys Thr Pro Val Ile Val Ala Pro Met
  1               5                  10                  15

Ala Gly Gly Pro Ser Thr Pro Ala Leu Val Asn Ala Ala Ala Glu Ala
             20                  25                  30

Gly Ser Leu Gly Phe Leu Ala Gly Gly Val Met Pro Leu Glu Gln Leu
         35                  40                  45

Lys Gln Glu Leu Ser Glu Val Lys Gly Val Phe Gly Val Asn Leu Phe
     50                  55                  60

Arg Pro Gln Thr Asp Ala Pro Lys Pro Ser Asp Ile Asp Glu Leu Ala
 65                  70                  75                  80

Gly Leu Leu Ser Ser Ala Phe Arg Gln Phe Gly Leu Asp Glu Pro Thr
                 85                  90                  95

Val Pro Thr Pro Asp Leu Ser Asn Gly Trp Glu Ala Lys Phe Glu Ala
            100                 105                 110

Val Leu Ala Ala Lys Pro Ala Val Phe Ser Cys Thr Phe Gly Ile Phe
        115                 120                 125

Ser Ala Glu Glu Phe Ala Arg Ile Lys Ala Thr Gly Ile Glu Ala Trp
    130                 135                 140

Val Thr Val Thr Asn Pro Glu Asp Ala Leu Ala Ala Gln Lys Ala Gly
145                 150                 155                 160

Ala Asn Ala Leu Val Val Gln Gly Pro Glu Ala Gly Gly His Arg Ser
                165                 170                 175

Thr Trp Ser Ile Glu Val Glu Pro Asp Glu Arg Asp Leu Lys Thr Leu
            180                 185                 190

Leu Ala Ala Val Lys Gln Ala Gly Val Tyr Leu Pro Leu Ile Ala Ala
        195                 200                 205

Gly Gly Leu Ser Thr Ser Ala Asp Val Ala Ala Ile Leu Glu Ala Gly
    210                 215                 220

Ala Ser Ala Ala Ser Cys Gly Ser Ala Phe Leu Leu Ser Asp Glu Ala
225                 230                 235                 240

Gly Thr Ser Ser Leu Asn Arg Glu Ile Leu Asp Ala Ala Pro Ala Leu
                245                 250                 255

Gly Leu Glu Ser Val Ser Ser Arg Ala Phe Ser Gly Arg Tyr Ala Arg
            260                 265                 270

Gly Val Glu Thr Arg Phe Thr Arg Ser Asn Glu Gly Leu Pro Pro Leu
        275                 280                 285

Tyr Pro Tyr Leu Asn Pro Met Ile Thr Ser Leu Arg Lys Val Ala Gly
    290                 295                 300

Ser Ala Gly Asn Trp Asp Tyr Ala Tyr Cys Leu Val Gly Val Gly Leu
305                 310                 315                 320

Glu Ser Ile Ala Lys Gly Ser Ala Lys Gln Ile Leu Glu Ser Leu Thr
                325                 330                 335

Pro Ser Ala Leu Gly
            340


<210> 177
<211> 516
<212> DNA
<213> Corynebacterium glutamicum

<220>
<221> CDS
<222> (101)..(493)
<223> RXA01182

<400> 177
gttaaaacgg aaactaatac cccaaaggat accgattcaa tttgtgatgt gtggtgttcg 60

ggtcatatca agctaaacag atgcccccta caataggctt gtg ttc aat tta ttt    115
                                           Val Phe Asn Leu Phe
                                           1               5

ggt cgt aaa act cct cgc tct aac ctc cgc cca cca cgc ggt ccg ggc    163
Gly Arg Lys Thr Pro Arg Ser Asn Leu Arg Pro Pro Arg Gly Pro Gly
                10                  15                  20

gat act gtg cgc ccg gaa gat tta aaa ttc ttg atg caa tgg gtg cag    211
Asp Thr Val Arg Pro Glu Asp Leu Lys Phe Leu Met Gln Trp Val Gln
            25                  30                  35

gat aag cca ttt gtt gag gca ttc gtt gaa ccg gaa acg ctg gtc aat    259
Asp Lys Pro Phe Val Glu Ala Phe Val Glu Pro Glu Thr Leu Val Asn
        40                  45                  50

gag atg tct gtc gtt ttg gtt gat gct cat ggg gtt ttt gtc cgc cga    307
Glu Met Ser Val Val Leu Val Asp Ala His Gly Val Phe Val Arg Arg
    55                  60                  65

agg atc ggc ggt ccc aaa ggg att gat gtt atc gcg aaa aag ctc ggc    355
Arg Ile Gly Gly Pro Lys Gly Ile Asp Val Ile Ala Lys Lys Leu Gly
70                  75                  80                  85

gtt ccg gtt tat gat gtt gag gag acc ggt tac ccc caa agg atg cgc    403
Val Pro Val Tyr Asp Val Glu Glu Thr Gly Tyr Pro Gln Arg Met Arg
                90                  95                  100

gaa cgc att gaa tat gag cgc atc tta aga aag cgt gag gaa caa aaa    451
Glu Arg Ile Glu Tyr Glu Arg Ile Leu Arg Lys Arg Glu Glu Gln Lys
            105                 110                 115

gct cgc cgc gct aaa ttt gag cgc ggc gag aat cct gat ctt            493
Ala Arg Arg Ala Lys Phe Glu Arg Gly Glu Asn Pro Asp Leu
        120                 125                 130

taactagcgt ttagctttcc gac                                          516


<210> 178
<211> 131
<212> PRT

<213> Corynebacterium glutamicum

<400> 178
Val Phe Asn Leu Phe Gly Arg Lys Thr Pro Arg Ser Asn Leu Arg Pro
1               5                   10                  15

Pro Arg Gly Pro Gly Asp Thr Val Arg Pro Glu Asp Leu Lys Phe Leu
            20                  25                  30

Met Gln Trp Val Gln Asp Lys Pro Phe Val Glu Ala Phe Val Glu Pro
        35                  40                  45

Glu Thr Leu Val Asn Glu Met Ser Val Val Leu Val Asp Ala His Gly
    50                  55                  60

Val Phe Val Arg Arg Arg Ile Gly Gly Pro Lys Gly Ile Asp Val Ile
65                  70                  75                  80

Ala Lys Lys Leu Gly Val Pro Val Tyr Asp Val Glu Glu Thr Gly Tyr
                85                  90                  95

Pro Gln Arg Met Arg Glu Arg Ile Glu Tyr Glu Arg Ile Leu Arg Lys
            100                 105                 110

Arg Glu Glu Gln Lys Ala Arg Arg Ala Lys Phe Glu Arg Gly Glu Asn
        115                 120                 125

Pro Asp Leu
    130


<210> 179
<211> 834
<212> DNA
<213> Corynebacterium glutamicum

<220>
<221> CDS
<222> (101)..(811)
<223> RXA02531

<400> 179
cacttcgctc cccaaggtac atccccgatg ccacttcttg gagccatcat cggtgccacc 60

aaacacattg aagtgggcac tggagtagtg gatatgcgtt atg aaa atc cct ttg   115
                                           Met Lys Ile Pro Leu
                                           1               5

tat atg gcc gag gaa gca gct gct ctc aat ctg ctt gcc gac ggc cga   163
Tyr Met Ala Glu Glu Ala Ala Ala Leu Asn Leu Leu Ala Asp Gly Arg
                10                  15                  20

cta gcc ctc gga gtt tcc agg gga tca ccc gaa cca gcc gag aag ggt   211
Leu Ala Leu Gly Val Ser Arg Gly Ser Pro Glu Pro Ala Glu Lys Gly
            25                  30                  35

tgg gaa gct ttc ggc tac gac ggc ggt gat gat cct aaa gct gca ggc   259
Trp Glu Ala Phe Gly Tyr Asp Gly Gly Asp Asp Pro Lys Ala Ala Gly
        40                  45                  50

atg gca cgg gag aaa ttc ctt cgc ttc ctc gat gcc atc gat ggt cgc   307
Met Ala Arg Glu Lys Phe Leu Arg Phe Leu Asp Ala Ile Asp Gly Arg
    55                  60                  65

ccc atg tcc atc gct tcc gag aat caa tac cca cgc ctc tac cat ccg   355
Pro Met Ser Ile Ala Ser Glu Asn Gln Tyr Pro Arg Leu Tyr His Pro
70                  75                  80                  85

ggc act ccc ctg ccg atc ttc ccg cat gat ctt gac ttg ggt aaa tcc   403
Gly Thr Pro Leu Pro Ile Phe Pro His Asp Leu Asp Leu Gly Lys Ser
                90                  95                  100

att tgg tgg ggc gcc ggt tcc cac aac acc gcc gaa caa gca gca cgc   451
Ile Trp Trp Gly Ala Gly Ser His Asn Thr Ala Glu Gln Ala Ala Arg
            105                 110                 115

gat ggc gtt aac ttg atg agc tcc acc ctc gtc gcc gaa gcc acc ggc   499

Asp Gly Val Asn Leu Met Ser Ser Thr Leu Val Ala Glu Ala Thr Gly
        120                 125                 130

caa tcc ttc ggg gat ctg caa gcc gat caa atc gcg ttc tac cgc caa     547
Gln Ser Phe Gly Asp Leu Gln Ala Asp Gln Ile Ala Phe Tyr Arg Gln
    135                 140                 145

gct tgg aaa gaa gcc gga cac gat tgg acc cca cgt gtg tct gtc tcc     595
Ala Trp Lys Glu Ala Gly His Asp Trp Thr Pro Arg Val Ser Val Ser
150                 155                 160                 165

agg tcc atc ttt ccg atc gtc acc gac cgc gac cgt gag ctt ttc gga     643
Arg Ser Ile Phe Pro Ile Val Thr Asp Arg Asp Arg Glu Leu Phe Gly
                170                 175                 180

ctt cag gga caa ggc ggt gac caa gta gga atc ctg gat gat acc cga     691
Leu Gln Gly Gln Gly Gly Asp Gln Val Gly Ile Leu Asp Asp Thr Arg
            185                 190                 195

tcc acg ttc ggt cgc agc tac gcc gga agt ccc gat gaa ctc atc gac     739
Ser Thr Phe Gly Arg Ser Tyr Ala Gly Ser Pro Asp Glu Leu Ile Asp
        200                 205                 210

cag ctc caa gga aga caa agc tgt gat gga agc cga cac ctt gat gct     787
Gln Leu Gln Gly Arg Gln Ser Cys Asp Gly Ser Arg His Leu Asp Ala
    215                 220                 225

cac cgc ccc caa cca aat ggg tgt tgagatcaac gcgtcgatcc tga          834
His Arg Pro Gln Pro Asn Gly Cys
230                 235


<210> 180
<211> 237
<212> PRT
<213> Corynebacterium glutamicum

<400> 180
Met Lys Ile Pro Leu Tyr Met Ala Glu Glu Ala Ala Ala Leu Asn Leu
1               5                   10                  15

Leu Ala Asp Gly Arg Leu Ala Leu Gly Val Ser Arg Gly Ser Pro Glu
            20                  25                  30

Pro Ala Glu Lys Gly Trp Glu Ala Phe Gly Tyr Asp Gly Gly Asp Asp
        35                  40                  45

Pro Lys Ala Ala Gly Met Ala Arg Glu Lys Phe Leu Arg Phe Leu Asp
    50                  55                  60

Ala Ile Asp Gly Arg Pro Met Ser Ile Ala Ser Glu Asn Gln Tyr Pro
65                  70                  75                  80

Arg Leu Tyr His Pro Gly Thr Pro Leu Pro Ile Phe Pro His Asp Leu
                85                  90                  95

Asp Leu Gly Lys Ser Ile Trp Trp Gly Ala Gly Ser His Asn Thr Ala
            100                 105                 110

Glu Gln Ala Ala Arg Asp Gly Val Asn Leu Met Ser Ser Thr Leu Val
        115                 120                 125

Ala Glu Ala Thr Gly Gln Ser Phe Gly Asp Leu Gln Ala Asp Gln Ile
    130                 135                 140

Ala Phe Tyr Arg Gln Ala Trp Lys Glu Ala Gly His Asp Trp Thr Pro
145                 150                 155                 160

Arg Val Ser Val Ser Arg Ser Ile Phe Pro Ile Val Thr Asp Arg Asp
                165                 170                 175

Arg Glu Leu Phe Gly Leu Gln Gly Gln Gly Gly Asp Gln Val Gly Ile
            180                 185                 190

Leu Asp Asp Thr Arg Ser Thr Phe Gly Arg Ser Tyr Ala Gly Ser Pro
        195                 200                 205

Asp Glu Leu Ile Asp Gln Leu Gln Gly Arg Gln Ser Cys Asp Gly Ser
    210                 215                 220

Arg His Leu Asp Ala His Arg Pro Gln Pro Asn Gly Cys
225                 230                 235

<210> 181
<211> 1614
<212> DNA
<213> Corynebacterium glutamicum

<220>
<221> CDS
<222> (101)..(1591)
<223> RXN00689

<400> 181
acagggaaat cctcccagaa ttaatcaccg aagctgcaca ccagatggct actgcagacc 60

tcaatcgtgc aaaggccctg ttaagaacgg atgcgatccg atg aat gct gca acc    115
                                            Met Asn Ala Ala Thr
                                            1               5

agg cgt gct tct ctg caa ctc ccc tat acc cat gtc gat gat ttc tac    163
Arg Arg Ala Ser Leu Gln Leu Pro Tyr Thr His Val Asp Asp Phe Tyr
                10                  15                  20

atc aac ggt tcc tgg gtt aaa gca gaa gga aca caa cgc aac ccc gta    211
Ile Asn Gly Ser Trp Val Lys Ala Glu Gly Thr Gln Arg Asn Pro Val
            25                  30                  35

gtt gat cct gcg gtc ggt caa gaa tgg gga tct gtt cca gaa gca acc    259
Val Asp Pro Ala Val Gly Gln Glu Trp Gly Ser Val Pro Glu Ala Thr
        40                  45                  50

gca tct gaa ttg gac tct gcg gtg gga gct gca cgt aca gcg cta aag    307
Ala Ser Glu Leu Asp Ser Ala Val Gly Ala Ala Arg Thr Ala Leu Lys
    55                  60                  65

tcg tgg agt gca ctt aca ggt gcg gaa cga aca ggc tac ctc ctg aaa    355
Ser Trp Ser Ala Leu Thr Gly Ala Glu Arg Thr Gly Tyr Leu Leu Lys
70                  75                  80                  85

atc gcg acg gaa att gaa tcc cgt tct gaa gct cta gca ctt act aat    403
Ile Ala Thr Glu Ile Glu Ser Arg Ser Glu Ala Leu Ala Leu Thr Asn
                90                  95                  100

acc cgc gaa aat ggt tcc ccc att tcc gag acc cgt gga gct gcg tcc    451
Thr Arg Glu Asn Gly Ser Pro Ile Ser Glu Thr Arg Gly Ala Ala Ser
            105                 110                 115

aat gca gca gga att ttc cgt tac ttt gcc act ctc gcg cct tgg tta    499
Asn Ala Ala Gly Ile Phe Arg Tyr Phe Ala Thr Leu Ala Pro Trp Leu
        120                 125                 130

gac ggc gaa gac atc cgc cca ttt cct gcc ggt agc gcc gaa tcc atc    547
Asp Gly Glu Asp Ile Arg Pro Phe Pro Ala Gly Ser Ala Glu Ser Ile
    135                 140                 145

gtg gat aaa gat ccc atc ggt gtc tgc gca ctc atc gcc cca tgg aat    595
Val Asp Lys Asp Pro Ile Gly Val Cys Ala Leu Ile Ala Pro Trp Asn
150                 155                 160                 165

ttc ccg atc aac ctt gta gtc atc aaa ctg gca cca gca ctt ctt gcc    643
Phe Pro Ile Asn Leu Val Val Ile Lys Leu Ala Pro Ala Leu Leu Ala
                170                 175                 180

ggc tgt acc gtc atc atc aaa cca gcc tcc ccc acc cca ctg tcg atc    691
Gly Cys Thr Val Ile Ile Lys Pro Ala Ser Pro Thr Pro Leu Ser Ile
            185                 190                 195

cgt ttc atc atc gaa gcc atc gaa gcc gcc gga gtg cca gca ggc gta    739
Arg Phe Ile Ile Glu Ala Ile Glu Ala Ala Gly Val Pro Ala Gly Val
        200                 205                 210

gtc aac cta ctc acc ggt tca ggg cgt ttc ggt gat gcc ctt gtc cgc    787

Val Asn Leu Leu Thr Gly Ser Gly Arg Phe Gly Asp Ala Leu Val Arg
    215                 220                 225

cac ccc gga gta gac aag gta gcg ttt acc gga tca acg cct gtt gga     835
His Pro Gly Val Asp Lys Val Ala Phe Thr Gly Ser Thr Pro Val Gly
230                 235                 240                 245

aag aag atc gct gcc gcc tgc gga gaa cta ctc cga cca gtg act tta     883
Lys Lys Ile Ala Ala Ala Cys Gly Glu Leu Leu Arg Pro Val Thr Leu
                250                 255                 260

gag cta ggc gga aaa tct tcc gcg att atc ctt cct gat gca gac atg     931
Glu Leu Gly Gly Lys Ser Ser Ala Ile Ile Leu Pro Asp Ala Asp Met
            265                 270                 275

tca gta ctc tcg acg cgg ttg att cga tcc tgt atg cgc aac act gga     979
Ser Val Leu Ser Thr Arg Leu Ile Arg Ser Cys Met Arg Asn Thr Gly
        280                 285                 290

caa acc tgc tac atc agt acc cgg att att gcc cct agc tca cgc tat     1027
Gln Thr Cys Tyr Ile Ser Thr Arg Ile Ile Ala Pro Ser Ser Arg Tyr
    295                 300                 305

gcg gaa gtc gta caa aca gtg gca agc act atc gct gca ggt aga caa     1075
Ala Glu Val Val Gln Thr Val Ala Ser Thr Ile Ala Ala Gly Arg Gln
310                 315                 320                 325

ggt gac ccc tat gat gaa gaa acg gtt ttt ggg cca gtt gcc agc gcc     1123
Gly Asp Pro Tyr Asp Glu Glu Thr Val Phe Gly Pro Val Ala Ser Ala
                330                 335                 340

tct cag tac tca acc gtc atg tct tac att gac tcc gca cga gag gaa     1171
Ser Gln Tyr Ser Thr Val Met Ser Tyr Ile Asp Ser Ala Arg Glu Glu
            345                 350                 355

ggt gca cga gtg gtt gca ggt gga acc cgg tca atc agc ctt tct gaa     1219
Gly Ala Arg Val Val Ala Gly Gly Thr Arg Ser Ile Ser Leu Ser Glu
        360                 365                 370

ggt tta gaa tca ggc gag ttt atc caa cca acc gtg ttt gcc gat gtc     1267
Gly Leu Glu Ser Gly Glu Phe Ile Gln Pro Thr Val Phe Ala Asp Val
    375                 380                 385

acc ccc gac atg cgg ata tca cgc gaa gaa atc ttc ggc cct gtt att     1315
Thr Pro Asp Met Arg Ile Ser Arg Glu Glu Ile Phe Gly Pro Val Ile
390                 395                 400                 405

tcc atc cta aag tac gac gat aca aac ggt gtt tcc gaa gca atc gca     1363
Ser Ile Leu Lys Tyr Asp Asp Thr Asn Gly Val Ser Glu Ala Ile Ala
                410                 415                 420

cta gcc aac aac acg aaa ttc ggt ctc ggt ggc ttg gta ttt ggt gcg     1411
Leu Ala Asn Asn Thr Lys Phe Gly Leu Gly Gly Leu Val Phe Gly Ala
            425                 430                 435

gat gag gaa caa gca cta gaa gtc gcc cgt caa gtg gat tct ggt tcc     1459
Asp Glu Glu Gln Ala Leu Glu Val Ala Arg Gln Val Asp Ser Gly Ser
        440                 445                 450

gta ggc atc aac ttc ttc ggt tcc aac cat tcc gcc cca ttt gga gga     1507
Val Gly Ile Asn Phe Phe Gly Ser Asn His Ser Ala Pro Phe Gly Gly
    455                 460                 465

cgc cac gaa tcc ggt atg gga gtg gaa tac ggc atc gaa ggc ctc agt     1555
Arg His Glu Ser Gly Met Gly Val Glu Tyr Gly Ile Glu Gly Leu Ser
470                 475                 480                 485

gct tac ctg aca tac aag agt att cac cga acc att tagttactga          1601
Ala Tyr Leu Thr Tyr Lys Ser Ile His Arg Thr Ile
                490                 495

aagttctcag cta                                                      1614


<210> 182
<211> 497
<212> PRT

<213> Corynebacterium glutamicum

<400> 182
Met Asn Ala Ala Thr Arg Arg Ala Ser Leu Gln Leu Pro Tyr Thr His
1               5                   10                  15

Val Asp Asp Phe Tyr Ile Asn Gly Ser Trp Val Lys Ala Glu Gly Thr
            20                  25                  30

Gln Arg Asn Pro Val Val Asp Pro Ala Val Gly Gln Glu Trp Gly Ser
        35                  40                  45

Val Pro Glu Ala Thr Ala Ser Glu Leu Asp Ser Ala Val Gly Ala Ala
    50                  55                  60

Arg Thr Ala Leu Lys Ser Trp Ser Ala Leu Thr Gly Ala Glu Arg Thr
65                  70                  75                  80

Gly Tyr Leu Leu Lys Ile Ala Thr Glu Ile Glu Ser Arg Ser Glu Ala
                85                  90                  95

Leu Ala Leu Thr Asn Thr Arg Glu Asn Gly Ser Pro Ile Ser Glu Thr
            100                 105                 110

Arg Gly Ala Ala Ser Asn Ala Ala Gly Ile Phe Arg Tyr Phe Ala Thr
        115                 120                 125

Leu Ala Pro Trp Leu Asp Gly Glu Asp Ile Arg Pro Phe Pro Ala Gly
    130                 135                 140

Ser Ala Glu Ser Ile Val Asp Lys Asp Pro Ile Gly Val Cys Ala Leu
145                 150                 155                 160

Ile Ala Pro Trp Asn Phe Pro Ile Asn Leu Val Val Ile Lys Leu Ala
                165                 170                 175

Pro Ala Leu Leu Ala Gly Cys Thr Val Ile Ile Lys Pro Ala Ser Pro
            180                 185                 190

Thr Pro Leu Ser Ile Arg Phe Ile Ile Glu Ala Ile Glu Ala Ala Gly
        195                 200                 205

Val Pro Ala Gly Val Val Asn Leu Leu Thr Gly Ser Gly Arg Phe Gly
    210                 215                 220

Asp Ala Leu Val Arg His Pro Gly Val Asp Lys Val Ala Phe Thr Gly
225                 230                 235                 240

Ser Thr Pro Val Gly Lys Lys Ile Ala Ala Ala Cys Gly Glu Leu Leu
                245                 250                 255

Arg Pro Val Thr Leu Glu Leu Gly Gly Lys Ser Ser Ala Ile Ile Leu
            260                 265                 270

Pro Asp Ala Asp Met Ser Val Leu Ser Thr Arg Leu Ile Arg Ser Cys
        275                 280                 285

Met Arg Asn Thr Gly Gln Thr Cys Tyr Ile Ser Thr Arg Ile Ile Ala
    290                 295                 300

Pro Ser Ser Arg Tyr Ala Glu Val Val Gln Thr Val Ala Ser Thr Ile
305                 310                 315                 320

Ala Ala Gly Arg Gln Gly Asp Pro Tyr Asp Glu Glu Thr Val Phe Gly
                325                 330                 335

Pro Val Ala Ser Ala Ser Gln Tyr Ser Thr Val Met Ser Tyr Ile Asp
            340                 345                 350

Ser Ala Arg Glu Glu Gly Ala Arg Val Val Ala Gly Gly Thr Arg Ser
        355                 360                 365

Ile Ser Leu Ser Glu Gly Leu Glu Ser Gly Glu Phe Ile Gln Pro Thr
    370                 375                 380

Val Phe Ala Asp Val Thr Pro Asp Met Arg Ile Ser Arg Glu Glu Ile
385                 390                 395                 400

Phe Gly Pro Val Ile Ser Ile Leu Lys Tyr Asp Asp Thr Asn Gly Val
                405                 410                 415

Ser Glu Ala Ile Ala Leu Ala Asn Asn Thr Lys Phe Gly Leu Gly Gly
            420                 425                 430

Leu Val Phe Gly Ala Asp Glu Glu Gln Ala Leu Glu Val Ala Arg Gln
        435                 440                 445

Val Asp Ser Gly Ser Val Gly Ile Asn Phe Phe Gly Ser Asn His Ser
    450                 455                 460

Ala Pro Phe Gly Gly Arg His Glu Ser Gly Met Gly Val Glu Tyr Gly
465                 470                 475                 480

Ile Glu Gly Leu Ser Ala Tyr Leu Thr Tyr Lys Ser Ile His Arg Thr
                485                 490                 495

Ile

<210> 183
<211> 750
<212> DNA
<213> Corynebacterium glutamicum

<220>
<221> CDS
<222> (101)..(727)
<223> FRXA00689

<400> 183
actccgacca gtgactttag agctaggcgg aaaatcttcc gcgattatcc ttcctgatgc 60

agacatgtca gtactctcga cgcggttgat tcgatcctgt atg cgc aac act gga    115
                                           Met Arg Asn Thr Gly
                                           1               5

caa acc tgc tac atc agt acc cgg att att gcc cct agc tca cgc tat    163
Gln Thr Cys Tyr Ile Ser Thr Arg Ile Ile Ala Pro Ser Ser Arg Tyr
                10                  15                  20

gcg gaa gtc gta caa aca gtg gca agc act atc gct gca ggt aga caa    211
Ala Glu Val Val Gln Thr Val Ala Ser Thr Ile Ala Ala Gly Arg Gln
            25                  30                  35

ggt gac ccc tat gat gaa gaa acg gtt ttt ggg cca gtt gcc agc gcc    259
Gly Asp Pro Tyr Asp Glu Glu Thr Val Phe Gly Pro Val Ala Ser Ala
        40                  45                  50

tct cag tac tca acc gtc atg tct tac att gac tcc gca cga gag gaa    307
Ser Gln Tyr Ser Thr Val Met Ser Tyr Ile Asp Ser Ala Arg Glu Glu
    55                  60                  65

ggt gca cga gtg gtt gca ggt gga acc cgg tca atc agc ctt tct gaa    355
Gly Ala Arg Val Val Ala Gly Gly Thr Arg Ser Ile Ser Leu Ser Glu
70                  75                  80                  85

ggt tta gaa tca ggc gag ttt atc caa cca acc gtg ttt gcc gat gtc    403
Gly Leu Glu Ser Gly Glu Phe Ile Gln Pro Thr Val Phe Ala Asp Val
                90                  95                  100

acc ccc gac atg cgg ata tca cgc gaa gaa atc ttc ggc cct gtt att    451
Thr Pro Asp Met Arg Ile Ser Arg Glu Glu Ile Phe Gly Pro Val Ile
            105                 110                 115

tcc atc cta aag tac gac gat aca aac ggt gtt tcc gaa gca atc gca    499
Ser Ile Leu Lys Tyr Asp Asp Thr Asn Gly Val Ser Glu Ala Ile Ala
        120                 125                 130

cta gcc aac aac acg aaa ttc ggt ctc ggt ggc ttg gta ttt ggt gcg    547
Leu Ala Asn Asn Thr Lys Phe Gly Leu Gly Gly Leu Val Phe Gly Ala
    135                 140                 145

gat gag gaa caa gca cta gaa gtc gcc cgt caa gtg gat tct ggt tcc    595

Asp Glu Glu Gln Ala Leu Glu Val Ala Arg Gln Val Asp Ser Gly Ser
150                 155                 160                 165

gta ggc atc aac ttc ttc ggt tcc aac cat tcc gcc cca ttt gga gga     643
Val Gly Ile Asn Phe Phe Gly Ser Asn His Ser Ala Pro Phe Gly Gly
                170                 175                 180

cgc cac gaa tcc ggt atg gga gtg gaa tac ggc atc gaa ggc ctc agt     691
Arg His Glu Ser Gly Met Gly Val Glu Tyr Gly Ile Glu Gly Leu Ser
            185                 190                 195

gct tac ctg aca tac aag agt att cac cga acc att tagttactga          737
Ala Tyr Leu Thr Tyr Lys Ser Ile His Arg Thr Ile
        200                 205

aagttctcag cta                                                      750


<210> 184
<211> 209
<212> PRT
<213> Corynebacterium glutamicum

<400> 184
Met Arg Asn Thr Gly Gln Thr Cys Tyr Ile Ser Thr Arg Ile Ile Ala
  1               5                  10                  15

Pro Ser Ser Arg Tyr Ala Glu Val Val Gln Thr Val Ala Ser Thr Ile
             20                  25                  30

Ala Ala Gly Arg Gln Gly Asp Pro Tyr Asp Glu Glu Thr Val Phe Gly
         35                  40                  45

Pro Val Ala Ser Ala Ser Gln Tyr Ser Thr Val Met Ser Tyr Ile Asp
     50                  55                  60

Ser Ala Arg Glu Glu Gly Ala Arg Val Val Ala Gly Gly Thr Arg Ser
 65                  70                  75                  80

Ile Ser Leu Ser Glu Gly Leu Glu Ser Gly Glu Phe Ile Gln Pro Thr
                 85                  90                  95

Val Phe Ala Asp Val Thr Pro Asp Met Arg Ile Ser Arg Glu Glu Ile
            100                 105                 110

Phe Gly Pro Val Ile Ser Ile Leu Lys Tyr Asp Asp Thr Asn Gly Val
        115                 120                 125

Ser Glu Ala Ile Ala Leu Ala Asn Asn Thr Lys Phe Gly Leu Gly Gly
    130                 135                 140

Leu Val Phe Gly Ala Asp Glu Glu Gln Ala Leu Glu Val Ala Arg Gln
145                 150                 155                 160

Val Asp Ser Gly Ser Val Gly Ile Asn Phe Phe Gly Ser Asn His Ser
                165                 170                 175

Ala Pro Phe Gly Gly Arg His Glu Ser Gly Met Gly Val Glu Tyr Gly
            180                 185                 190

Ile Glu Gly Leu Ser Ala Tyr Leu Thr Tyr Lys Ser Ile His Arg Thr
        195                 200                 205

Ile



<210> 185
<211> 878
<212> DNA
<213> Corynebacterium glutamicum

<220>
<221> CDS
<222> (1)..(855)
<223> RXN03128

<400> 185
aac gga ctc gcc att ccc gac att gga ttt ggt gta ttc caa acc cca     48
Asn Gly Leu Ala Ile Pro Asp Ile Gly Phe Gly Val Phe Gln Thr Pro
1               5                   10                  15

ccc gat gaa acc cga aac tcc gtt aac gct gct ctt gaa gcc ggc tat     96
Pro Asp Glu Thr Arg Asn Ser Val Asn Ala Ala Leu Glu Ala Gly Tyr
            20                  25                  30

cgc cac atc gac acc gcc gcc gca tac ggc aat gaa cgt gaa gtc ggt     144
Arg His Ile Asp Thr Ala Ala Ala Tyr Gly Asn Glu Arg Glu Val Gly
        35                  40                  45

gaa gca atc gca gca tcc ggc att ggc cgc gac gag atc acc atc gaa     192
Glu Ala Ile Ala Ala Ser Gly Ile Gly Arg Asp Glu Ile Thr Ile Glu
    50                  55                  60

acc aaa atc tgg gtg acc gac tac ggc ttc gag gaa act ctc cac gca     240
Thr Lys Ile Trp Val Thr Asp Tyr Gly Phe Glu Glu Thr Leu His Ala
65                  70                  75                  80

ttc gac aag gcc aca ggc aag ctt ggt gtc gat aca ctg gac att ttg     288
Phe Asp Lys Ala Thr Gly Lys Leu Gly Val Asp Thr Leu Asp Ile Leu
                85                  90                  95

atc ttg cac cag gca gtg cca agc agc ttt gat cgc acc atc gcc gcc     336
Ile Leu His Gln Ala Val Pro Ser Ser Phe Asp Arg Thr Ile Ala Ala
            100                 105                 110

tac aag gcg cta gag aag ctg ctt ttc gac ggc gcg gtg cgg gca atc     384
Tyr Lys Ala Leu Glu Lys Leu Leu Phe Asp Gly Ala Val Arg Ala Ile
        115                 120                 125

gga gtc agt aat ttc atg cca gag cac ctg gac aaa ctc ctt ttg gaa     432
Gly Val Ser Asn Phe Met Pro Glu His Leu Asp Lys Leu Leu Leu Glu
    130                 135                 140

acc tcc att gtc cca gct ctg aac caa atc gaa tgc cac ccc tac ttc     480
Thr Ser Ile Val Pro Ala Leu Asn Gln Ile Glu Cys His Pro Tyr Phe
145                 150                 155                 160

cag cag cgt gac gtg ctt gcc cgc aat gag cag ctt ggc att ttg act     528
Gln Gln Arg Asp Val Leu Ala Arg Asn Glu Gln Leu Gly Ile Leu Thr
                165                 170                 175

cag gcg tgg tca cca atc ggt ggc atc acc ttc tac cgc gac gga cag     576
Gln Ala Trp Ser Pro Ile Gly Gly Ile Thr Phe Tyr Arg Asp Gly Gln
            180                 185                 190

ctt cca agc act cta gaa aat gag gtc atc gct gga atc gcc gca gaa     624
Leu Pro Ser Thr Leu Glu Asn Glu Val Ile Ala Gly Ile Ala Ala Glu
        195                 200                 205

gtt ggc aaa aca cca gct caa gta atg ctg cgc tgg cac cta cag cgt     672
Val Gly Lys Thr Pro Ala Gln Val Met Leu Arg Trp His Leu Gln Arg
    210                 215                 220

aga cgt cac gca att cca aag tct gtg acc cca tca cgc att gtg gaa     720
Arg Arg His Ala Ile Pro Lys Ser Val Thr Pro Ser Arg Ile Val Glu
225                 230                 235                 240

aac ttt gag atc ttt gat ttc gaa ctc tcc gat gag caa cta cag caa     768
Asn Phe Glu Ile Phe Asp Phe Glu Leu Ser Asp Glu Gln Leu Gln Gln
                245                 250                 255

atc gat gcc ctc aac acc gat ctg cgc ggt ggc cca gaa cca gag aac     816
Ile Asp Ala Leu Asn Thr Asp Leu Arg Gly Gly Pro Glu Pro Glu Asn
            260                 265                 270

atc acc atg gaa aac tac tac cga gaa atc cca gaa gcc taaaggccct     865
Ile Thr Met Glu Asn Tyr Tyr Arg Glu Ile Pro Glu Ala
        275                 280                 285

tagaggcgaa tgt                                                     878

<210> 186

<211> 285
<212> PRT
<213> Corynebacterium glutamicum

<400> 186
Asn Gly Leu Ala Ile Pro Asp Ile Gly Phe Gly Val Phe Gln Thr Pro
1               5                   10                  15

Pro Asp Glu Thr Arg Asn Ser Val Asn Ala Ala Leu Glu Ala Gly Tyr
            20                  25                  30

Arg His Ile Asp Thr Ala Ala Ala Tyr Gly Asn Glu Arg Glu Val Gly
        35                  40                  45

Glu Ala Ile Ala Ala Ser Gly Ile Gly Arg Asp Glu Ile Thr Ile Glu
    50                  55                  60

Thr Lys Ile Trp Val Thr Asp Tyr Gly Phe Glu Glu Thr Leu His Ala
65                  70                  75                  80

Phe Asp Lys Ala Thr Gly Lys Leu Gly Val Asp Thr Leu Asp Ile Leu
                85                  90                  95

Ile Leu His Gln Ala Val Pro Ser Ser Phe Asp Arg Thr Ile Ala Ala
            100                 105                 110

Tyr Lys Ala Leu Glu Lys Leu Leu Phe Asp Gly Ala Val Arg Ala Ile
        115                 120                 125

Gly Val Ser Asn Phe Met Pro Glu His Leu Asp Lys Leu Leu Leu Glu
    130                 135                 140

Thr Ser Ile Val Pro Ala Leu Asn Gln Ile Glu Cys His Pro Tyr Phe
145                 150                 155                 160

Gln Gln Arg Asp Val Leu Ala Arg Asn Glu Gln Leu Gly Ile Leu Thr
                165                 170                 175

Gln Ala Trp Ser Pro Ile Gly Gly Ile Thr Phe Tyr Arg Asp Gly Gln
            180                 185                 190

Leu Pro Ser Thr Leu Glu Asn Glu Val Ile Ala Gly Ile Ala Ala Glu
        195                 200                 205

Val Gly Lys Thr Pro Ala Gln Val Met Leu Arg Trp His Leu Gln Arg
    210                 215                 220

Arg Arg His Ala Ile Pro Lys Ser Val Thr Pro Ser Arg Ile Val Glu
225                 230                 235                 240

Asn Phe Glu Ile Phe Asp Phe Glu Leu Ser Asp Glu Gln Leu Gln Gln
                245                 250                 255

Ile Asp Ala Leu Asn Thr Asp Leu Arg Gly Gly Pro Glu Pro Glu Asn
            260                 265                 270

Ile Thr Met Glu Asn Tyr Tyr Arg Glu Ile Pro Glu Ala
        275                 280                 285


<210> 187
<211> 522
<212> DNA
<213> Corynebacterium glutamicum

<220>
<221> CDS
<222> (1)..(522)
<223> FRXA02192

<400> 187
att ccc gac att gga ttt ggt gtc ttc caa acc cca ccc gat gaa acc      48
Ile Pro Asp Ile Gly Phe Gly Val Phe Gln Thr Pro Pro Asp Glu Thr
1               5                   10                  15

cga aac tcc gtt aac gct gct ctt gaa gcc ggc tat cgc cac atc gac      96
Arg Asn Ser Val Asn Ala Ala Leu Glu Ala Gly Tyr Arg His Ile Asp

20                  25                  30

acc gcg gcc gca tac ggc aat gaa cgt gaa gtc ggt gaa gca atc gca      144
Thr Ala Ala Ala Tyr Gly Asn Glu Arg Glu Val Gly Glu Ala Ile Ala
        35                  40                  45

gca tcc ggc att ggc cgc gac gag atc acc atc gaa acc aaa atc tgg      192
Ala Ser Gly Ile Gly Arg Asp Glu Ile Thr Ile Glu Thr Lys Ile Trp
    50                  55                  60

gtg acc gac tac ggc ttc gag gaa act ctc cac gca ttc gac aag gcc      240
Val Thr Asp Tyr Gly Phe Glu Glu Thr Leu His Ala Phe Asp Lys Ala
65                  70                  75                  80

aca ggc aag ctt ggt gtc gat aca ctg gac att ttg atc ttg cac cag      288
Thr Gly Lys Leu Gly Val Asp Thr Leu Asp Ile Leu Ile Leu His Gln
                85                  90                  95

gca gtg cca agc agc ttt gat cgc acc atc gcc gcc tac aag gcg cta      336
Ala Val Pro Ser Ser Phe Asp Arg Thr Ile Ala Ala Tyr Lys Ala Leu
            100                 105                 110

gag aag ctg ctt ttc gac ggc gcg gtg cgg gca atc gga gtc agt aat      384
Glu Lys Leu Leu Phe Asp Gly Ala Val Arg Ala Ile Gly Val Ser Asn
        115                 120                 125

ttc atg cca gag cac ctg gac aaa ctc ctt ttg gaa acc tcc att gtc      432
Phe Met Pro Glu His Leu Asp Lys Leu Leu Leu Glu Thr Ser Ile Val
    130                 135                 140

cca gct ctg aac caa atc gaa tgc cac ccc tac ttc cag cag cgt gac      480
Pro Ala Leu Asn Gln Ile Glu Cys His Pro Tyr Phe Gln Gln Arg Asp
145                 150                 155                 160

gtg ctt gcc cgc aat gag cag ctt ggc att ttg act cag gcg              522
Val Leu Ala Arg Asn Glu Gln Leu Gly Ile Leu Thr Gln Ala
                165                 170


<210> 188
<211> 174
<212> PRT
<213> Corynebacterium glutamicum

<400> 188
Ile Pro Asp Ile Gly Phe Gly Val Phe Gln Thr Pro Pro Asp Glu Thr
1               5                   10                  15

Arg Asn Ser Val Asn Ala Ala Leu Glu Ala Gly Tyr Arg His Ile Asp
            20                  25                  30

Thr Ala Ala Ala Tyr Gly Asn Glu Arg Glu Val Gly Glu Ala Ile Ala
        35                  40                  45

Ala Ser Gly Ile Gly Arg Asp Glu Ile Thr Ile Glu Thr Lys Ile Trp
    50                  55                  60

Val Thr Asp Tyr Gly Phe Glu Glu Thr Leu His Ala Phe Asp Lys Ala
65                  70                  75                  80

Thr Gly Lys Leu Gly Val Asp Thr Leu Asp Ile Leu Ile Leu His Gln
                85                  90                  95

Ala Val Pro Ser Ser Phe Asp Arg Thr Ile Ala Ala Tyr Lys Ala Leu
            100                 105                 110

Glu Lys Leu Leu Phe Asp Gly Ala Val Arg Ala Ile Gly Val Ser Asn
        115                 120                 125

Phe Met Pro Glu His Leu Asp Lys Leu Leu Leu Glu Thr Ser Ile Val
    130                 135                 140

Pro Ala Leu Asn Gln Ile Glu Cys His Pro Tyr Phe Gln Gln Arg Asp
145                 150                 155                 160

Val Leu Ala Arg Asn Glu Gln Leu Gly Ile Leu Thr Gln Ala
                165                 170

<210> 189
<211> 1039
<212> DNA
<213> Corynebacterium glutamicum

<220>
<221> CDS
<222> (101)..(1039)
<223> RXA02351

<400> 189
tgacacttta cagactggtt ttcaactaat gacaccgaaa gaaatacacc tcaacctttt 60

tgctttcggt gccgggcacc acgcggcggc gtggcgagcg gtg gag gga agc gtc 115
Val Glu Gly Ser Val
1               5

gaa aag ctg ggt tta att tcc tgg tgg gag gaa ctc gcg cgc acc gct 163
Glu Lys Leu Gly Leu Ile Ser Trp Trp Glu Glu Leu Ala Arg Thr Ala
                10                  15                  20

gag cgg ggc aag ctg gat gcg gtc ttt ttg gcc gat ggg cag gcg att 211
Glu Arg Gly Lys Leu Asp Ala Val Phe Leu Ala Asp Gly Gln Ala Ile
            25                  30                  35

aat ccg gtc ggt ctg gag aat ggg ccg ggc tgg ttt ttg gag ccg gtg 259
Asn Pro Val Gly Leu Glu Asn Gly Pro Gly Trp Phe Leu Glu Pro Val
        40                  45                  50

acc gcg ttg act gcg atg gcg cgg gcg acg aac aat att ggg ttg atc 307
Thr Ala Leu Thr Ala Met Ala Arg Ala Thr Asn Asn Ile Gly Leu Ile
    55                  60                  65

agc aca att tcc agt acg ttt tgg cag ccg ttt cat gcg gcg cgg atg 355
Ser Thr Ile Ser Ser Thr Phe Trp Gln Pro Phe His Ala Ala Arg Met
70                  75                  80                  85

atc gcc agc ttg gat cat att tcg ggt ggg cgt gct gga atc aat gtg 403
Ile Ala Ser Leu Asp His Ile Ser Gly Gly Arg Ala Gly Ile Asn Val
                90                  95                  100

gtg aca tcg atg acc gat gcg gag gcg cgt aac cac ggg atg gat gcg 451
Val Thr Ser Met Thr Asp Ala Glu Ala Arg Asn His Gly Met Asp Ala
            105                 110                 115

ttg ccg ggt cac gat gtt cgc tat gcg cgc gct gcg gaa ttt att gaa 499
Leu Pro Gly His Asp Val Arg Tyr Ala Arg Ala Ala Glu Phe Ile Glu
        120                 125                 130

acc atc act gcg ctg tgg gat tct tgg cct gcg gaa agt ttg gtg atg 547
Thr Ile Thr Ala Leu Trp Asp Ser Trp Pro Ala Glu Ser Leu Val Met
    135                 140                 145

gat cgt gct gga aaa ttt gcg gac tcc tcg ctc att aaa tct atc gat 595
Asp Arg Ala Gly Lys Phe Ala Asp Ser Ser Leu Ile Lys Ser Ile Asp
150                 155                 160                 165

cat gat ggt gag ttc ttc caa gtc gct ggt ccg ctg aat atc ccc agt 643
His Asp Gly Glu Phe Phe Gln Val Ala Gly Pro Leu Asn Ile Pro Ser
                170                 175                 180

cct ccg cag ggt cga ccc gta ctt ttt cag gct gga tcc tca ccg caa 691
Pro Pro Gln Gly Arg Pro Val Leu Phe Gln Ala Gly Ser Ser Pro Gln
            185                 190                 195

gga cgg gaa atc gct gcg aaa tac gcc gag gca att tac tct gtg gcg 739
Gly Arg Glu Ile Ala Ala Lys Tyr Ala Glu Ala Ile Tyr Ser Val Ala
        200                 205                 210

tgg gat ttg gag caa gcg caa gat tat cgc tct gat att cat gct cgt 787
Trp Asp Leu Glu Gln Ala Gln Asp Tyr Arg Ser Asp Ile His Ala Arg
    215                 220                 225

gcc act gcc cag ggt cgc gag ccc atg ccg gtg ctt cct ggt ttg gtg 835
Ala Thr Ala Gln Gly Arg Glu Pro Met Pro Val Leu Pro Gly Leu Val

230                 235                 240                 245

act ttt gtt ggc acg acc gtg gaa gaa gcg cgt gca aaa cag cag gct      883
Thr Phe Val Gly Thr Thr Val Glu Glu Ala Arg Ala Lys Gln Gln Ala
            250                 255                 260

ctt aat gcg ttg ctg ccg gtc aaa gac tca cta aat cag ttg agt ttc      931
Leu Asn Ala Leu Leu Pro Val Lys Asp Ser Leu Asn Gln Leu Ser Phe
        265                 270                 275

ttt gtg ggt caa gat tgc tcg acg tgg gat ttg gat gca cct ccc cca      979
Phe Val Gly Gln Asp Cys Ser Thr Trp Asp Leu Asp Ala Pro Pro Pro
    280                 285                 290

cca ctg cca ccg cta gaa gag ttt tcc ggt cct aaa ggc agg tac gaa      1027
Pro Leu Pro Pro Leu Glu Glu Phe Ser Gly Pro Lys Gly Arg Tyr Glu
    295                 300                 305

acg gtc ctg cgg                                                      1039
Thr Val Leu Arg
310


<210> 190
<211> 313
<212> PRT
<213> Corynebacterium glutamicum

<400> 190
Val Glu Gly Ser Val Glu Lys Leu Gly Leu Ile Ser Trp Trp Glu Glu
1               5                   10                  15

Leu Ala Arg Thr Ala Glu Arg Gly Lys Leu Asp Ala Val Phe Leu Ala
            20                  25                  30

Asp Gly Gln Ala Ile Asn Pro Val Gly Leu Glu Asn Gly Pro Gly Trp
        35                  40                  45

Phe Leu Glu Pro Val Thr Ala Leu Thr Ala Met Ala Arg Ala Thr Asn
    50                  55                  60

Asn Ile Gly Leu Ile Ser Thr Ile Ser Ser Thr Phe Trp Gln Pro Phe
65                  70                  75                  80

His Ala Ala Arg Met Ile Ala Ser Leu Asp His Ile Ser Gly Gly Arg
                85                  90                  95

Ala Gly Ile Asn Val Val Thr Ser Met Thr Asp Ala Glu Ala Arg Asn
            100                 105                 110

His Gly Met Asp Ala Leu Pro Gly His Asp Val Arg Tyr Ala Arg Ala
        115                 120                 125

Ala Glu Phe Ile Glu Thr Ile Thr Ala Leu Trp Asp Ser Trp Pro Ala
    130                 135                 140

Glu Ser Leu Val Met Asp Arg Ala Gly Lys Phe Ala Asp Ser Ser Leu
145                 150                 155                 160

Ile Lys Ser Ile Asp His Asp Gly Glu Phe Phe Gln Val Ala Gly Pro
                165                 170                 175

Leu Asn Ile Pro Ser Pro Pro Gln Gly Arg Pro Val Leu Phe Gln Ala
            180                 185                 190

Gly Ser Ser Pro Gln Gly Arg Glu Ile Ala Ala Lys Tyr Ala Glu Ala
        195                 200                 205

Ile Tyr Ser Val Ala Trp Asp Leu Glu Gln Ala Gln Asp Tyr Arg Ser
    210                 215                 220

Asp Ile His Ala Arg Ala Thr Ala Gln Gly Arg Glu Pro Met Pro Val
225                 230                 235                 240

Leu Pro Gly Leu Val Thr Phe Val Gly Thr Thr Val Glu Glu Ala Arg
                245                 250                 255

Ala Lys Gln Gln Ala Leu Asn Ala Leu Leu Pro Val Lys Asp Ser Leu
        260                 265                 270

Asn Gln Leu Ser Phe Phe Val Gly Gln Asp Cys Ser Thr Trp Asp Leu
        275                 280                 285

Asp Ala Pro Pro Pro Pro Leu Pro Pro Leu Glu Glu Phe Ser Gly Pro
    290                 295                 300

Lys Gly Arg Tyr Glu Thr Val Leu Arg
305                 310


<210> 191
<211> 924
<212> DNA
<213> Corynebacterium glutamicum

<220>
<221> CDS
<222> (101)..(901)
<223> RXN00905

<400> 191
cgctgcccct ctatgctgct cctagttacc cctgcacaaa tagcggtttt tctcacgcat 60

tctgcatcga gtcgggtcga cgtatataag gtggaaaggc atg acc caa ttc gaa    115
                                            Met Thr Gln Phe Glu
                                            1               5

aac gcg caa gta ctt aaa gag aac atc gaa aac caa cgc gag cag atc    163
Asn Ala Gln Val Leu Lys Glu Asn Ile Glu Asn Gln Arg Glu Gln Ile
                10                  15                  20

ttt acc cag ttg aaa gaa att gtg tct ttc aac tcc gtg cac agc gat    211
Phe Thr Gln Leu Lys Glu Ile Val Ser Phe Asn Ser Val His Ser Asp
            25                  30                  35

cca aac cta ctg gag gac tac gcc ggc gcg aaa gaa tgg gta aaa gaa    259
Pro Asn Leu Leu Glu Asp Tyr Ala Gly Ala Lys Glu Trp Val Lys Glu
        40                  45                  50

aca ctg acc aac gca ggt ctc acc gtc agc gaa ttc gct gcc gaa gat    307
Thr Leu Thr Asn Ala Gly Leu Thr Val Ser Glu Phe Ala Ala Glu Asp
    55                  60                  65

gga acc acc aac ttc atc ggc acc cgc aag ggc tcc gaa ggt gca cca    355
Gly Thr Thr Asn Phe Ile Gly Thr Arg Lys Gly Ser Glu Gly Ala Pro
70                  75                  80                  85

aag gta ctg ctg tac agc cac ttc gac gtt gtc cca tcc ggc cct ttg    403
Lys Val Leu Leu Tyr Ser His Phe Asp Val Val Pro Ser Gly Pro Leu
                90                  95                  100

gat ctc tgg gac acc aat cct ttt gaa ctc acc gag cgc gac gct ggc    451
Asp Leu Trp Asp Thr Asn Pro Phe Glu Leu Thr Glu Arg Asp Ala Gly
            105                 110                 115

cac ggc acc cgc tgg tac ggc cgc ggc gcc gct gac tgc aag ggc aac    499
His Gly Thr Arg Trp Tyr Gly Arg Gly Ala Ala Asp Cys Lys Gly Asn
        120                 125                 130

ctg gtc atg cac ctc gca gca ctg cgc gcc gtc gaa gcc agc ggc gac    547
Leu Val Met His Leu Ala Ala Leu Arg Ala Val Glu Ala Ser Gly Asp
    135                 140                 145

acc aca ctc aac ctc acc tac gtg gtc gag ggc tcc gag gaa atg gga    595
Thr Thr Leu Asn Leu Thr Tyr Val Val Glu Gly Ser Glu Glu Met Gly
150                 155                 160                 165

ggc gga gcg ctc agc gcg ctc atc aag gac aag cct gag ctt ttc gac    643
Gly Gly Ala Leu Ser Ala Leu Ile Lys Asp Lys Pro Glu Leu Phe Asp
                170                 175                 180

gca gat gtc atc ttg att gca gac agc gga aac gct tcc gtg ggc acc    691
Ala Asp Val Ile Leu Ile Ala Asp Ser Gly Asn Ala Ser Val Gly Thr
            185                 190                 195

cca acc ttg acc act acc ctg cgc ggt ggc gga cag gtc acc gtc acc     739
Pro Thr Leu Thr Thr Thr Leu Arg Gly Gly Gly Gln Val Thr Val Thr
        200                 205                 210

gtg gac acc ctt gaa ggc gct gtt cac tcc ggc cag aac ggt ggc gct     787
Val Asp Thr Leu Glu Gly Ala Val His Ser Gly Gln Asn Gly Gly Ala
    215                 220                 225

gcc cca gat gct gtt gct gct ctc gtg cgc gtt ctg gat act ttg cgc     835
Ala Pro Asp Ala Val Ala Ala Leu Val Arg Val Leu Asp Thr Leu Arg
230                 235                 240                 245

gat gaa cac gga cgc acc gtt atc gac ggc tgt caa cac cac cgc aaa     883
Asp Glu His Gly Arg Thr Val Ile Asp Gly Cys Gln His His Arg Lys
                250                 255                 260

ctg gaa ggg cga gcc tta tgatccagag actttccgca gcg                   924
Leu Glu Gly Arg Ala Leu
            265


<210> 192
<211> 267
<212> PRT
<213> Corynebacterium glutamicum

<400> 192
Met Thr Gln Phe Glu Asn Ala Gln Val Leu Lys Glu Asn Ile Glu Asn
1               5                   10                  15

Gln Arg Glu Gln Ile Phe Thr Gln Leu Lys Glu Ile Val Ser Phe Asn
            20                  25                  30

Ser Val His Ser Asp Pro Asn Leu Leu Glu Asp Tyr Ala Gly Ala Lys
        35                  40                  45

Glu Trp Val Lys Glu Thr Leu Thr Asn Ala Gly Leu Thr Val Ser Glu
    50                  55                  60

Phe Ala Ala Glu Asp Gly Thr Thr Asn Phe Ile Gly Thr Arg Lys Gly
65                  70                  75                  80

Ser Glu Gly Ala Pro Lys Val Leu Leu Tyr Ser His Phe Asp Val Val
                85                  90                  95

Pro Ser Gly Pro Leu Asp Leu Trp Asp Thr Asn Pro Phe Glu Leu Thr
            100                 105                 110

Glu Arg Asp Ala Gly His Gly Thr Arg Trp Tyr Gly Arg Gly Ala Ala
        115                 120                 125

Asp Cys Lys Gly Asn Leu Val Met His Leu Ala Ala Leu Arg Ala Val
    130                 135                 140

Glu Ala Ser Gly Asp Thr Thr Leu Asn Leu Thr Tyr Val Val Glu Gly
145                 150                 155                 160

Ser Glu Glu Met Gly Gly Gly Ala Leu Ser Ala Leu Ile Lys Asp Lys
                165                 170                 175

Pro Glu Leu Phe Asp Ala Asp Val Ile Leu Ile Ala Asp Ser Gly Asn
            180                 185                 190

Ala Ser Val Gly Thr Pro Thr Leu Thr Thr Thr Leu Arg Gly Gly Gly
        195                 200                 205

Gln Val Thr Val Thr Val Asp Thr Leu Glu Gly Ala Val His Ser Gly
    210                 215                 220

Gln Asn Gly Gly Ala Ala Pro Asp Ala Val Ala Ala Leu Val Arg Val
225                 230                 235                 240

Leu Asp Thr Leu Arg Asp Glu His Gly Arg Thr Val Ile Asp Gly Cys
                245                 250                 255

Gln His His Arg Lys Leu Glu Gly Arg Ala Leu

260                 265


<210> 193
<211> 716
<212> DNA
<213> Corynebacterium glutamicum

<220>
<221> CDS
<222> (1)..(693)
<223> FRXA00905

<400> 193
gat cca aac cta ctg gag gac tac gcc ggc gcg aaa gaa tgg gta aaa      48
Asp Pro Asn Leu Leu Glu Asp Tyr Ala Gly Ala Lys Glu Trp Val Lys
1               5                   10                  15

gaa aca ctg acc aac gca ggt ctc acc gtc agc gaa ttc gct gcc gaa      96
Glu Thr Leu Thr Asn Ala Gly Leu Thr Val Ser Glu Phe Ala Ala Glu
            20                  25                  30

gat gga acc acc aac ttc atc ggc acc cgc aag ggc tcc gaa ggt gca      144
Asp Gly Thr Thr Asn Phe Ile Gly Thr Arg Lys Gly Ser Glu Gly Ala
        35                  40                  45

cca aag gta ctg ctg tac agc cac ttc gac gtt gtc cca tcc ggc cct      192
Pro Lys Val Leu Leu Tyr Ser His Phe Asp Val Val Pro Ser Gly Pro
    50                  55                  60

ttg gat ctc tgg gac acc aat cct ttt gaa ctc acc gag cgc gac gct      240
Leu Asp Leu Trp Asp Thr Asn Pro Phe Glu Leu Thr Glu Arg Asp Ala
65                  70                  75                  80

ggc cac ggc acc cgc tgg tac ggc cgc ggc gcc gct gac tgc aag ggc      288
Gly His Gly Thr Arg Trp Tyr Gly Arg Gly Ala Ala Asp Cys Lys Gly
                85                  90                  95

aac ctg gtc atg cac ctc gca gca ctg cgc gcc gtc gaa gcc agc ggc      336
Asn Leu Val Met His Leu Ala Ala Leu Arg Ala Val Glu Ala Ser Gly
            100                 105                 110

gac acc aca ctc aac ctc acc tac gtg gtc gag ggc tcc gag gaa atg      384
Asp Thr Thr Leu Asn Leu Thr Tyr Val Val Glu Gly Ser Glu Glu Met
        115                 120                 125

gga ggc gga gcg ctc agc gcg ctc atc aag gac aag cct gag ctt ttc      432
Gly Gly Gly Ala Leu Ser Ala Leu Ile Lys Asp Lys Pro Glu Leu Phe
    130                 135                 140

gac gca gat gtc atc ttg att gca gac agc gga aac gct tcc gtg ggc      480
Asp Ala Asp Val Ile Leu Ile Ala Asp Ser Gly Asn Ala Ser Val Gly
145                 150                 155                 160

acc cca acc ttg acc act acc ctg cgc ggt ggc gga cag gtc acc gtc      528
Thr Pro Thr Leu Thr Thr Thr Leu Arg Gly Gly Gly Gln Val Thr Val
                165                 170                 175

acc gtg gac acc ctt gaa ggc gct gtt cac tcc ggc cag aac ggt ggc      576
Thr Val Asp Thr Leu Glu Gly Ala Val His Ser Gly Gln Asn Gly Gly
            180                 185                 190

gct gcc cca gat gct gtt gct gct ctc gtg cgc gtt ctg gat act ttg      624
Ala Ala Pro Asp Ala Val Ala Ala Leu Val Arg Val Leu Asp Thr Leu
        195                 200                 205

cgc gat gaa cac gga cgc acc gtt atc gac ggc tgt caa cac cac cgc      672
Arg Asp Glu His Gly Arg Thr Val Ile Asp Gly Cys Gln His His Arg
    210                 215                 220

aaa ctg gaa ggg cga gcc tta tgatccagag actttccgca gcg                716
Lys Leu Glu Gly Arg Ala Leu
225                 230


<210> 194
<211> 231

<212> PRT
<213> Corynebacterium glutamicum

<400> 194
Asp Pro Asn Leu Leu Glu Asp Tyr Ala Gly Ala Lys Glu Trp Val Lys
1               5                   10                  15

Glu Thr Leu Thr Asn Ala Gly Leu Thr Val Ser Glu Phe Ala Ala Glu
            20                  25                  30

Asp Gly Thr Thr Asn Phe Ile Gly Thr Arg Lys Gly Ser Glu Gly Ala
        35                  40                  45

Pro Lys Val Leu Leu Tyr Ser His Phe Asp Val Val Pro Ser Gly Pro
    50                  55                  60

Leu Asp Leu Trp Asp Thr Asn Pro Phe Glu Leu Thr Glu Arg Asp Ala
65                  70                  75                  80

Gly His Gly Thr Arg Trp Tyr Gly Arg Gly Ala Ala Asp Cys Lys Gly
                85                  90                  95

Asn Leu Val Met His Leu Ala Ala Leu Arg Ala Val Glu Ala Ser Gly
            100                 105                 110

Asp Thr Thr Leu Asn Leu Thr Tyr Val Val Glu Gly Ser Glu Glu Met
        115                 120                 125

Gly Gly Gly Ala Leu Ser Ala Leu Ile Lys Asp Lys Pro Glu Leu Phe
    130                 135                 140

Asp Ala Asp Val Ile Leu Ile Ala Asp Ser Gly Asn Ala Ser Val Gly
145                 150                 155                 160

Thr Pro Thr Leu Thr Thr Thr Leu Arg Gly Gly Gly Gln Val Thr Val
                165                 170                 175

Thr Val Asp Thr Leu Glu Gly Ala Val His Ser Gly Gln Asn Gly Gly
            180                 185                 190

Ala Ala Pro Asp Ala Val Ala Ala Leu Val Arg Val Leu Asp Thr Leu
        195                 200                 205

Arg Asp Glu His Gly Arg Thr Val Ile Asp Gly Cys Gln His His Arg
    210                 215                 220

Lys Leu Glu Gly Arg Ala Leu
225                 230


<210> 195
<211> 627
<212> DNA
<213> Corynebacterium glutamicum

<220>
<221> CDS
<222> (101)..(604)
<223> RXA00906

<400> 195
accgtggaca cccttgaagg cgctgttcac tccggccaga acggtggcgc tgccccagat 60

gctgttgctg ctctcgtgcg cgttctggat actttgcgcg atg aac acg gac gca    115
                                            Met Asn Thr Asp Ala
                                            1               5

ccg tta tcg acg gct gtc aac acc acc gca aac tgg aag ggc gag cct    163
Pro Leu Ser Thr Ala Val Asn Thr Thr Ala Asn Trp Lys Gly Glu Pro
                10                  15                  20

tat gat cca gag act ttc cgc agc gat gcc ggc atc ctc gac ggt gta    211
Tyr Asp Pro Glu Thr Phe Arg Ser Asp Ala Gly Ile Leu Asp Gly Val
            25                  30                  35

gac atc atg ggc gac ggc gac aac cca gca agc atg ctg tgg tcc agg    259
Asp Ile Met Gly Asp Gly Asp Asn Pro Ala Ser Met Leu Trp Ser Arg

40                  45                  50

cct gca atc tcc atc acc gga ttc act tcc acc cca gtg gca gaa gca     307
Pro Ala Ile Ser Ile Thr Gly Phe Thr Ser Thr Pro Val Ala Glu Ala
    55                  60                  65

ctc aac gca gtg ccc gca acg gcg tcc gcc aag cta aac ctt cgc gtg     355
Leu Asn Ala Val Pro Ala Thr Ala Ser Ala Lys Leu Asn Leu Arg Val
70                  75                  80                  85

cca gca ggc ctg gaa gca aac gat gtg gcc gag aag ctg aag cag cac     403
Pro Ala Gly Leu Glu Ala Asn Asp Val Ala Glu Lys Leu Lys Gln His
                90                  95                  100

ctg atc aat cac aca cct tgg ggc gca aag atc acg gtg gag atc gat     451
Leu Ile Asn His Thr Pro Trp Gly Ala Lys Ile Thr Val Glu Ile Asp
            105                 110                 115

gac att aac caa ccg ttc tcc acc gat att acc ggc cct gca atg tcc     499
Asp Ile Asn Gln Pro Phe Ser Thr Asp Ile Thr Gly Pro Ala Met Ser
        120                 125                 130

acc ctg gcg tcc tgc ctg agc gct gcg tac gag ggc aag gat ctt gtc     547
Thr Leu Ala Ser Cys Leu Ser Ala Ala Tyr Glu Gly Lys Asp Leu Val
    135                 140                 145

acc gaa ggc agc ggc gga tcc att cca ctg tgt acc gaa ctg att gag     595
Thr Glu Gly Ser Gly Gly Ser Ile Pro Leu Cys Thr Glu Leu Ile Glu
150                 155                 160                 165

gtc aac cca taagcagaat tggcactcta cgg                               627
Val Asn Pro



<210> 196
<211> 168
<212> PRT
<213> Corynebacterium glutamicum

<400> 196
Met Asn Thr Asp Ala Pro Leu Ser Thr Ala Val Asn Thr Thr Ala Asn
1               5                   10                  15

Trp Lys Gly Glu Pro Tyr Asp Pro Glu Thr Phe Arg Ser Asp Ala Gly
            20                  25                  30

Ile Leu Asp Gly Val Asp Ile Met Gly Asp Gly Asp Asn Pro Ala Ser
        35                  40                  45

Met Leu Trp Ser Arg Pro Ala Ile Ser Ile Thr Gly Phe Thr Ser Thr
    50                  55                  60

Pro Val Ala Glu Ala Leu Asn Ala Val Pro Ala Thr Ala Ser Ala Lys
65                  70                  75                  80

Leu Asn Leu Arg Val Pro Ala Gly Leu Glu Ala Asn Asp Val Ala Glu
                85                  90                  95

Lys Leu Lys Gln His Leu Ile Asn His Thr Pro Trp Gly Ala Lys Ile
            100                 105                 110

Thr Val Glu Ile Asp Asp Ile Asn Gln Pro Phe Ser Thr Asp Ile Thr
        115                 120                 125

Gly Pro Ala Met Ser Thr Leu Ala Ser Cys Leu Ser Ala Ala Tyr Glu
    130                 135                 140

Gly Lys Asp Leu Val Thr Glu Gly Ser Gly Gly Ser Ile Pro Leu Cys
145                 150                 155                 160

Thr Glu Leu Ile Glu Val Asn Pro
                165


<210> 197
<211> 246

<212> DNA
<213> Corynebacterium glutamicum

<220>
<221> CDS
<222> (101)..(223)
<223> RXA00907

<400> 197
cctgagcgct gcgtacgagg gcaaggatct tgtcaccgaa ggcagcggcg gatccattcc 60

actgtgtacc gaactgattg aggtcaaccc ataagcagaa ttg gca ctc tac ggt 115
Leu Ala Leu Tyr Gly
1               5

gtg gaa gaa ccc ctc acc gtt atc cac tcc gct aat gaa tct gtt gac 163
Val Glu Glu Pro Leu Thr Val Ile His Ser Ala Asn Glu Ser Val Asp
                10                  15                  20

ccc aat gag att cgc gat atc gcc acc gca gaa gca ttg ttc ctg ctc 211
Pro Asn Glu Ile Arg Asp Ile Ala Thr Ala Glu Ala Leu Phe Leu Leu
            25                  30                  35

aac tac acc aag tagacccaaa agcaggcgtt aac 246
Asn Tyr Thr Lys
        40


<210> 198
<211> 41
<212> PRT
<213> Corynebacterium glutamicum

<400> 198
Leu Ala Leu Tyr Gly Val Glu Glu Pro Leu Thr Val Ile His Ser Ala
1               5                   10                  15

Asn Glu Ser Val Asp Pro Asn Glu Ile Arg Asp Ile Ala Thr Ala Glu
            20                  25                  30

Ala Leu Phe Leu Leu Asn Tyr Thr Lys
        35                  40


<210> 199
<211> 1386
<212> DNA
<213> Corynebacterium glutamicum

<220>
<221> CDS
<222> (101)..(1363)
<223> RXA02101

<400> 199
gccatggaat gctccgttga acgcaacagc cttaaataca atcccctcct ataagccaag 60

agttttagtg tcgctgcgca ggtactctac tatctaatcc atg agc cgc att tca 115
Met Ser Arg Ile Ser
1               5

gaa ctt cta aac aat cat ggt gtt gat ctg tcg tgg caa gag gcc gca 163
Glu Leu Leu Asn Asn His Gly Val Asp Leu Ser Trp Gln Glu Ala Ala
                10                  15                  20

tat cag gat ttc cac gaa cat cct gag ctc tcc ggc ttc gaa tca gag 211
Tyr Gln Asp Phe His Glu His Pro Glu Leu Ser Gly Phe Glu Ser Glu
            25                  30                  35

acc gca gat cgc att cag aaa tac ctc gag cgt ttt gat tgt gag gtg 259
Thr Ala Asp Arg Ile Gln Lys Tyr Leu Glu Arg Phe Asp Cys Glu Val
        40                  45                  50

att cca aat gtt ggc ggt tac ggc att ctg gcc gtg ttc cga aat ggg 307
Ile Pro Asn Val Gly Gly Tyr Gly Ile Leu Ala Val Phe Arg Asn Gly
    55                  60                  65

tcg aca gat cct ggt gcc cct gtt gcg tta atg cgc gca gat ttc gat 355
Ser Thr Asp Pro Gly Ala Pro Val Ala Leu Met Arg Ala Asp Phe Asp
70                  75                  80                  85

ggc ctt ccc gtc aag gaa atc acc gga gtt ccg ttt gct tcc act cgt 403
Gly Leu Pro Val Lys Glu Ile Thr Gly Val Pro Phe Ala Ser Thr Arg
                90                  95                  100

atg cgt ccg cat gat ggg gca aat gtc cat gtc atg cac gca tgc ggc 451
Met Arg Pro His Asp Gly Ala Asn Val His Val Met His Ala Cys Gly
            105                 110                 115

cac gat gtc cac gtc acc gcg ctg ctt ggt gcg tgt gcc att tta gat 499
His Asp Val His Val Thr Ala Leu Leu Gly Ala Cys Ala Ile Leu Asp
        120                 125                 130

gag cgt cgc gat gca tgg gaa ggc acg ttc atc gcg ttg ttc cag cca 547
Glu Arg Arg Asp Ala Trp Glu Gly Thr Phe Ile Ala Leu Phe Gln Pro
    135                 140                 145

tcg gag gaa aac tcc caa ggc gct aac aag atg gtc gcc ggc ggt tta 595
Ser Glu Glu Asn Ser Gln Gly Ala Asn Lys Met Val Ala Gly Gly Leu
150                 155                 160                 165

gtt gat ctg atc cca cgc cct gat gtg tgc ttt ggc cag cat gta gtc 643
Val Asp Leu Ile Pro Arg Pro Asp Val Cys Phe Gly Gln His Val Val
                170                 175                 180

ccc ggt gct gca gga acc gtg atg agc atg cct ggc ggt gct ctc gct 691
Pro Gly Ala Ala Gly Thr Val Met Ser Met Pro Gly Gly Ala Leu Ala
            185                 190                 195

gcc tgc gat tcc att gaa atc cgc att cag ggt cgc agc gcc cat ggt 739
Ala Cys Asp Ser Ile Glu Ile Arg Ile Gln Gly Arg Ser Ala His Gly
        200                 205                 210

tcc atg cct cat aat tcc atc gat ccc act tat gtt gca gcg atg att 787
Ser Met Pro His Asn Ser Ile Asp Pro Thr Tyr Val Ala Ala Met Ile
    215                 220                 225

gtc gtg cga ctc caa gga atc gtg ggc cgc gag gtt tct cca gag gat 835
Val Val Arg Leu Gln Gly Ile Val Gly Arg Glu Val Ser Pro Glu Asp
230                 235                 240                 245

ttc gcc gtt att tct gtg ggc acc ctc cag tcg ggc aac acc aac aac 883
Phe Ala Val Ile Ser Val Gly Thr Leu Gln Ser Gly Asn Thr Asn Asn
                250                 255                 260

acc att cca gca agt gct cgt ttg gtg ttg aac tgc cgt ttc tac aac 931
Thr Ile Pro Ala Ser Ala Arg Leu Val Leu Asn Cys Arg Phe Tyr Asn
            265                 270                 275

gac aaa gtc aag cac aag gtc tac cga gcc atc gaa cgt gtt gtc cgt 979
Asp Lys Val Lys His Lys Val Tyr Arg Ala Ile Glu Arg Val Val Arg
        280                 285                 290

ggt gaa tgc ctt gct tcc ggt att gag gaa gaa cct gtc att gag tac 1027
Gly Glu Cys Leu Ala Ser Gly Ile Glu Glu Glu Pro Val Ile Glu Tyr
    295                 300                 305

ttc gcc cac ggt gat ctc acc aac aac acc cct gtt gtc ttc gat act 1075
Phe Ala His Gly Asp Leu Thr Asn Asn Thr Pro Val Val Phe Asp Thr
310                 315                 320                 325

gtg cgc cct gtc ttc gac gat gtt ttc ggc gag gat tct att gac gct 1123
Val Arg Pro Val Phe Asp Asp Val Phe Gly Glu Asp Ser Ile Asp Ala
                330                 335                 340

tac cgg tgg act gcg tcg gag gat ttc ccc tcc att cct aag gca ttc 1171
Tyr Arg Trp Thr Ala Ser Glu Asp Phe Pro Ser Ile Pro Lys Ala Phe
            345                 350                 355

aac agc cct tac ctg tac tgg acg att ggt gtc acg ccg cgc gat cag 1219
Asn Ser Pro Tyr Leu Tyr Trp Thr Ile Gly Val Thr Pro Arg Asp Gln
        360                 365                 370

tgg aca gaa gcc gta gaa aga gac cgc gtg gca tcg gat gtg cca gcc 1267

Trp Thr Glu Ala Val Glu Arg Asp Arg Val Ala Ser Asp Val Pro Ala
    375                 380                 385

aat cac atg gga gat ttc ctc cct gat tat gcg ccg acg atg tcc gct    1315
Asn His Met Gly Asp Phe Leu Pro Asp Tyr Ala Pro Thr Met Ser Ala
390                 395                 400                 405

gcc acc cgc gca gcc gca gcc gcg ctg ctg acc tac ttg gga act aac    1363
Ala Thr Arg Ala Ala Ala Ala Ala Leu Leu Thr Tyr Leu Gly Thr Asn
                410                 415                 420

taatcatcta gttttctgcg acg                                          1386


<210> 200
<211> 421
<212> PRT
<213> Corynebacterium glutamicum

<400> 200
Met Ser Arg Ile Ser Glu Leu Leu Asn Asn His Gly Val Asp Leu Ser
  1               5                  10                  15

Trp Gln Glu Ala Ala Tyr Gln Asp Phe His Glu His Pro Glu Leu Ser
             20                  25                  30

Gly Phe Glu Ser Glu Thr Ala Asp Arg Ile Gln Lys Tyr Leu Glu Arg
         35                  40                  45

Phe Asp Cys Glu Val Ile Pro Asn Val Gly Gly Tyr Gly Ile Leu Ala
     50                  55                  60

Val Phe Arg Asn Gly Ser Thr Asp Pro Gly Ala Pro Val Ala Leu Met
 65                  70                  75                  80

Arg Ala Asp Phe Asp Gly Leu Pro Val Lys Glu Ile Thr Gly Val Pro
                 85                  90                  95

Phe Ala Ser Thr Arg Met Arg Pro His Asp Gly Ala Asn Val His Val
            100                 105                 110

Met His Ala Cys Gly His Asp Val His Val Thr Ala Leu Leu Gly Ala
        115                 120                 125

Cys Ala Ile Leu Asp Glu Arg Arg Asp Ala Trp Glu Gly Thr Phe Ile
    130                 135                 140

Ala Leu Phe Gln Pro Ser Glu Glu Asn Ser Gln Gly Ala Asn Lys Met
145                 150                 155                 160

Val Ala Gly Gly Leu Val Asp Leu Ile Pro Arg Pro Asp Val Cys Phe
                165                 170                 175

Gly Gln His Val Val Pro Gly Ala Ala Gly Thr Val Met Ser Met Pro
            180                 185                 190

Gly Gly Ala Leu Ala Ala Cys Asp Ser Ile Glu Ile Arg Ile Gln Gly
        195                 200                 205

Arg Ser Ala His Gly Ser Met Pro His Asn Ser Ile Asp Pro Thr Tyr
    210                 215                 220

Val Ala Ala Met Ile Val Val Arg Leu Gln Gly Ile Val Gly Arg Glu
225                 230                 235                 240

Val Ser Pro Glu Asp Phe Ala Val Ile Ser Val Gly Thr Leu Gln Ser
                245                 250                 255

Gly Asn Thr Asn Asn Thr Ile Pro Ala Ser Ala Arg Leu Val Leu Asn
            260                 265                 270

Cys Arg Phe Tyr Asn Asp Lys Val Lys His Lys Val Tyr Arg Ala Ile
        275                 280                 285

Glu Arg Val Val Arg Gly Glu Cys Leu Ala Ser Gly Ile Glu Glu Glu
    290                 295                 300

Pro Val Ile Glu Tyr Phe Ala His Gly Asp Leu Thr Asn Asn Thr Pro
305                 310                 315                 320

Val Val Phe Asp Thr Val Arg Pro Val Phe Asp Asp Val Phe Gly Glu
                325                 330                 335

Asp Ser Ile Asp Ala Tyr Arg Trp Thr Ala Ser Glu Asp Phe Pro Ser
            340                 345                 350

Ile Pro Lys Ala Phe Asn Ser Pro Tyr Leu Tyr Trp Thr Ile Gly Val
        355                 360                 365

Thr Pro Arg Asp Gln Trp Thr Glu Ala Val Glu Arg Asp Arg Val Ala
    370                 375                 380

Ser Asp Val Pro Ala Asn His Met Gly Asp Phe Leu Pro Asp Tyr Ala
385                 390                 395                 400

Pro Thr Met Ser Ala Ala Thr Arg Ala Ala Ala Ala Ala Leu Leu Thr
                405                 410                 415

Tyr Leu Gly Thr Asn
            420


<210> 201
<211> 1389
<212> DNA
<213> Corynebacterium glutamicum

<220>
<221> CDS
<222> (101)..(1366)
<223> RXN02565

<400> 201
ggaaattcga tacagtgcga tgacgcgata ttagaaagaa aaagatgcgc tttacgacga 60

aaccctcacc ctccttcagg aacttatccg caacgcctgc gtg aat gat cta acc    115
                                            Val Asn Asp Leu Thr
                                            1               5

cca gat tca ggt cag gaa att aga aac gcg gaa agc cta gaa cgt ttc    163
Pro Asp Ser Gly Gln Glu Ile Arg Asn Ala Glu Ser Leu Glu Arg Phe
                10                  15                  20

ttt gaa gga acc ccc aac gtt aaa atc acc aag ctg gaa ccg cat ccg    211
Phe Glu Gly Thr Pro Asn Val Lys Ile Thr Lys Leu Glu Pro His Pro
            25                  30                  35

ggc cgg acc tca att atc gtg act gtt cca ggc agc gat cca gat gct    259
Gly Arg Thr Ser Ile Ile Val Thr Val Pro Gly Ser Asp Pro Asp Ala
        40                  45                  50

gag cct tta aca ctg ctt gga cat act gat gtt gtg cct gtt gat ctg    307
Glu Pro Leu Thr Leu Leu Gly His Thr Asp Val Val Pro Val Asp Leu
    55                  60                  65

cct aaa tgg act aaa gat cca ttc ggt gcg gag att tcg gat gga cag    355
Pro Lys Trp Thr Lys Asp Pro Phe Gly Ala Glu Ile Ser Asp Gly Gln
70                  75                  80                  85

att tgg ggt aga ggg tcc gtc gat atg ctc ttt att acc gca acc caa    403
Ile Trp Gly Arg Gly Ser Val Asp Met Leu Phe Ile Thr Ala Thr Gln
                90                  95                  100

gcg gcc gtc acc cgt caa gta gcc cgt gaa ggc ggc ctg cgt ggc acg    451
Ala Ala Val Thr Arg Gln Val Ala Arg Glu Gly Gly Leu Arg Gly Thr
            105                 110                 115

ctg aca ttc gtt ggc gtt gct gat gag gaa gcc cgc ggc gga ctc gga    499
Leu Thr Phe Val Gly Val Ala Asp Glu Glu Ala Arg Gly Gly Leu Gly
        120                 125                 130

gcg aag tgg ctt tcc gaa gaa cac caa aac ctc ttc agc tgg aaa aac    547
Ala Lys Trp Leu Ser Glu Glu His Gln Asn Leu Phe Ser Trp Lys Asn
    135                 140                 145

tgc ctc tcc gaa tcc ggt gga tcg cac ctt cca gtc cac gac ggc agc     595
Cys Leu Ser Glu Ser Gly Gly Ser His Leu Pro Val His Asp Gly Ser
150                 155                 160                 165

gac gca gta gta att aac gtt gga gaa aaa ggt gca gct caa cgt cgt     643
Asp Ala Val Val Ile Asn Val Gly Glu Lys Gly Ala Ala Gln Arg Arg
                170                 175                 180

att cac gtc aat ggc gat gct ggt cat ggt tcc att cct ttc gac cgt     691
Ile His Val Asn Gly Asp Ala Gly His Gly Ser Ile Pro Phe Asp Arg
            185                 190                 195

gac agc gct att gtc aag atc ggt gaa gtc gcc cgc cga atc gct gcc     739
Asp Ser Ala Ile Val Lys Ile Gly Glu Val Ala Arg Arg Ile Ala Ala
        200                 205                 210

gcc gat ctg aag gta gcc aag gac gat atc tgg caa ggc ttc gtc caa     787
Ala Asp Leu Lys Val Ala Lys Asp Asp Ile Trp Gln Gly Phe Val Gln
    215                 220                 225

gcg cac cgt ttc gac cca gaa acg gag cag gcg ctt ctt agc ggg acc     835
Ala His Arg Phe Asp Pro Glu Thr Glu Gln Ala Leu Leu Ser Gly Thr
230                 235                 240                 245

tcc cct gag gcc tac gca gag ttc ggc gga ctc tcc cgc ttc gcc cac     883
Ser Pro Glu Ala Tyr Ala Glu Phe Gly Gly Leu Ser Arg Phe Ala His
                250                 255                 260

gcg gtg tct cat ctc acg atc gcc caa act gtg gtt cgt gca ggt caa     931
Ala Val Ser His Leu Thr Ile Ala Gln Thr Val Val Arg Ala Gly Gln
            265                 270                 275

gcc atc aat gta ttg cca tcg cat gcg tac ttg gaa ctg gat atc cgt     979
Ala Ile Asn Val Leu Pro Ser His Ala Tyr Leu Glu Leu Asp Ile Arg
        280                 285                 290

acc ctt cca ggc caa acc aat gac tat gtt gat gac acc ctg cgt gct     1027
Thr Leu Pro Gly Gln Thr Asn Asp Tyr Val Asp Asp Thr Leu Arg Ala
    295                 300                 305

gct ctg ggc gat ctt gcc gat gaa gta gaa atc gaa cac ctc atc tct     1075
Ala Leu Gly Asp Leu Ala Asp Glu Val Glu Ile Glu His Leu Ile Ser
310                 315                 320                 325

gaa gaa gca acg gtg agc cca act gat tcc agg ttg tat aac acc ttg     1123
Glu Glu Ala Thr Val Ser Pro Thr Asp Ser Arg Leu Tyr Asn Thr Leu
                330                 335                 340

gaa aaa gtt ctt ggt gat ttc ttc ccc gat gcg cct gtg gtc cca att     1171
Glu Lys Val Leu Gly Asp Phe Phe Pro Asp Ala Pro Val Val Pro Ile
            345                 350                 355

att tcc tct ggt ggc tct gac ctg cgc ttt ggt cgt cga cta ggc ggt     1219
Ile Ser Ser Gly Gly Ser Asp Leu Arg Phe Gly Arg Arg Leu Gly Gly
        360                 365                 370

gtt ggt tat ggt ttt gca gtt cat gca cgt gaa cga act ttg gcg gaa     1267
Val Gly Tyr Gly Phe Ala Val His Ala Arg Glu Arg Thr Leu Ala Glu
    375                 380                 385

gca atg ggg caa ctt cac tcc cat gac gag gcg ctg tac ctg gaa gat     1315
Ala Met Gly Gln Leu His Ser His Asp Glu Ala Leu Tyr Leu Glu Asp
390                 395                 400                 405

ctt gaa ctg act gtt cgg ggt tat gac tcc gtc gtg cgt gaa ttc cta     1363
Leu Glu Leu Thr Val Arg Gly Tyr Asp Ser Val Val Arg Glu Phe Leu
                410                 415                 420

ggc taaaaacatg aagcaggagt ctt                                       1389
Gly

<210> 202
<211> 422
<212> PRT

<213> Corynebacterium glutamicum

<400> 202
Val Asn Asp Leu Thr Pro Asp Ser Gly Gln Glu Ile Arg Asn Ala Glu
1               5                   10                  15

Ser Leu Glu Arg Phe Phe Glu Gly Thr Pro Asn Val Lys Ile Thr Lys
            20                  25                  30

Leu Glu Pro His Pro Gly Arg Thr Ser Ile Ile Val Thr Val Pro Gly
        35                  40                  45

Ser Asp Pro Asp Ala Glu Pro Leu Thr Leu Leu Gly His Thr Asp Val
    50                  55                  60

Val Pro Val Asp Leu Pro Lys Trp Thr Lys Asp Pro Phe Gly Ala Glu
65                  70                  75                  80

Ile Ser Asp Gly Gln Ile Trp Gly Arg Gly Ser Val Asp Met Leu Phe
                85                  90                  95

Ile Thr Ala Thr Gln Ala Ala Val Thr Arg Gln Val Ala Arg Glu Gly
            100                 105                 110

Gly Leu Arg Gly Thr Leu Thr Phe Val Gly Val Ala Asp Glu Glu Ala
        115                 120                 125

Arg Gly Gly Leu Gly Ala Lys Trp Leu Ser Glu Glu His Gln Asn Leu
    130                 135                 140

Phe Ser Trp Lys Asn Cys Leu Ser Glu Ser Gly Gly Ser His Leu Pro
145                 150                 155                 160

Val His Asp Gly Ser Asp Ala Val Val Ile Asn Val Gly Glu Lys Gly
                165                 170                 175

Ala Ala Gln Arg Arg Ile His Val Asn Gly Asp Ala Gly His Gly Ser
            180                 185                 190

Ile Pro Phe Asp Arg Asp Ser Ala Ile Val Lys Ile Gly Glu Val Ala
        195                 200                 205

Arg Arg Ile Ala Ala Ala Asp Leu Lys Val Ala Lys Asp Asp Ile Trp
    210                 215                 220

Gln Gly Phe Val Gln Ala His Arg Phe Asp Pro Glu Thr Glu Gln Ala
225                 230                 235                 240

Leu Leu Ser Gly Thr Ser Pro Glu Ala Tyr Ala Glu Phe Gly Gly Leu
                245                 250                 255

Ser Arg Phe Ala His Ala Val Ser His Leu Thr Ile Ala Gln Thr Val
            260                 265                 270

Val Arg Ala Gly Gln Ala Ile Asn Val Leu Pro Ser His Ala Tyr Leu
        275                 280                 285

Glu Leu Asp Ile Arg Thr Leu Pro Gly Gln Thr Asn Asp Tyr Val Asp
    290                 295                 300

Asp Thr Leu Arg Ala Ala Leu Gly Asp Leu Ala Asp Glu Val Glu Ile
305                 310                 315                 320

Glu His Leu Ile Ser Glu Glu Ala Thr Val Ser Pro Thr Asp Ser Arg
                325                 330                 335

Leu Tyr Asn Thr Leu Glu Lys Val Leu Gly Asp Phe Phe Pro Asp Ala
            340                 345                 350

Pro Val Val Pro Ile Ile Ser Ser Gly Gly Ser Asp Leu Arg Phe Gly
        355                 360                 365

Arg Arg Leu Gly Gly Val Gly Tyr Gly Phe Ala Val His Ala Arg Glu
    370                 375                 380

Arg Thr Leu Ala Glu Ala Met Gly Gln Leu His Ser His Asp Glu Ala
385                 390                 395                 400

Leu Tyr Leu Glu Asp Leu Glu Leu Thr Val Arg Gly Tyr Asp Ser Val
                405                 410                 415

Val Arg Glu Phe Leu Gly
            420


<210> 203
<211> 365
<212> DNA
<213> Corynebacterium glutamicum

<220>
<221> CDS
<222> (1)..(342)
<223> FRXA02565

<400> 203
gct gct ctg ggc gat ctt gcc gat gaa gta gaa atc gaa cac ctc atc      48
Ala Ala Leu Gly Asp Leu Ala Asp Glu Val Glu Ile Glu His Leu Ile
  1               5                  10                  15

tct gaa gaa gca acg gtg agc cca act gat tcc agg ttg tat aac acc      96
Ser Glu Glu Ala Thr Val Ser Pro Thr Asp Ser Arg Leu Tyr Asn Thr
             20                  25                  30

ttg gaa aaa gtt ctt ggt gat ttc ttc ccc gat gcg cct gtg gtc cca     144
Leu Glu Lys Val Leu Gly Asp Phe Phe Pro Asp Ala Pro Val Val Pro
         35                  40                  45

att att tcc tct ggt ggc tct gac ctg cgc ttt ggt cgt cga cta ggc     192
Ile Ile Ser Ser Gly Gly Ser Asp Leu Arg Phe Gly Arg Arg Leu Gly
     50                  55                  60

ggt gtt ggt tat ggt ttt gca gtt cat gca cgt gaa cga act ttg gcg     240
Gly Val Gly Tyr Gly Phe Ala Val His Ala Arg Glu Arg Thr Leu Ala
 65                  70                  75                  80

gaa gca atg ggg caa ctt cac tcc cat gac gag gcg ctg tac ctg gaa     288
Glu Ala Met Gly Gln Leu His Ser His Asp Glu Ala Leu Tyr Leu Glu
                 85                  90                  95

gat ctt gaa ctg act gtt cgg ggt tat gac tcc gtc gtg cgt gaa ttc     336
Asp Leu Glu Leu Thr Val Arg Gly Tyr Asp Ser Val Val Arg Glu Phe
            100                 105                 110

cta ggc taaaaacatg aagcaggagt ctt                                   365
Leu Gly



<210> 204
<211> 114
<212> PRT
<213> Corynebacterium glutamicum

<400> 204
Ala Ala Leu Gly Asp Leu Ala Asp Glu Val Glu Ile Glu His Leu Ile
  1               5                  10                  15

Ser Glu Glu Ala Thr Val Ser Pro Thr Asp Ser Arg Leu Tyr Asn Thr
             20                  25                  30

Leu Glu Lys Val Leu Gly Asp Phe Phe Pro Asp Ala Pro Val Val Pro
         35                  40                  45

Ile Ile Ser Ser Gly Gly Ser Asp Leu Arg Phe Gly Arg Arg Leu Gly
     50                  55                  60

Gly Val Gly Tyr Gly Phe Ala Val His Ala Arg Glu Arg Thr Leu Ala
 65                  70                  75                  80

Glu Ala Met Gly Gln Leu His Ser His Asp Glu Ala Leu Tyr Leu Glu
                 85                  90                  95

Asp Leu Glu Leu Thr Val Arg Gly Tyr Asp Ser Val Val Arg Glu Phe

            100                 105                 110

Leu Gly

<210> 205
<211> 738
<212> DNA
<213> Corynebacterium glutamicum

<220>
<221> CDS
<222> (1)..(738)
<223> FRXA02567

<400> 205
ctt atc cgc aac gcc tgc gtg aat gat cta acc cca gat tca ggt cag 48
Leu Ile Arg Asn Ala Cys Val Asn Asp Leu Thr Pro Asp Ser Gly Gln
1               5                   10                  15

gaa att aga aac gcg gaa agc cta gaa cgt ttc ttt gaa gga acc ccc 96
Glu Ile Arg Asn Ala Glu Ser Leu Glu Arg Phe Phe Glu Gly Thr Pro
            20                  25                  30

aac gtt aaa atc acc aag ctg gaa ccg cat ccg ggc cgg acc tca att 144
Asn Val Lys Ile Thr Lys Leu Glu Pro His Pro Gly Arg Thr Ser Ile
        35                  40                  45

atc gtg act gtt cca ggc agc gat cca gat gct gag cct tta aca ctg 192
Ile Val Thr Val Pro Gly Ser Asp Pro Asp Ala Glu Pro Leu Thr Leu
    50                  55                  60

ctt gga cat act gat gtt gtg cct gtt gat ctg cct aaa tgg act aaa 240
Leu Gly His Thr Asp Val Val Pro Val Asp Leu Pro Lys Trp Thr Lys
65                  70                  75                  80

gat cca ttc ggt gcg gag att tcg gat gga cag att tgg ggt aga ggg 288
Asp Pro Phe Gly Ala Glu Ile Ser Asp Gly Gln Ile Trp Gly Arg Gly
                85                  90                  95

tcc gtc gat atg ctc ttt att acc gca acc caa gcg gcc gtc acc cgt 336
Ser Val Asp Met Leu Phe Ile Thr Ala Thr Gln Ala Ala Val Thr Arg
            100                 105                 110

caa gta gcc cgt gaa ggc ggc ctg cgt ggc acg ctg aca ttc gtt ggc 384
Gln Val Ala Arg Glu Gly Gly Leu Arg Gly Thr Leu Thr Phe Val Gly
        115                 120                 125

gtt gct gat gag gaa gcc cgc ggc gga ctc gga gcg aag tgg ctt tcc 432
Val Ala Asp Glu Glu Ala Arg Gly Gly Leu Gly Ala Lys Trp Leu Ser
    130                 135                 140

gaa gaa cac caa aac ctc ttc agc tgg aaa aac tgc ctc tcc gaa tcc 480
Glu Glu His Gln Asn Leu Phe Ser Trp Lys Asn Cys Leu Ser Glu Ser
145                 150                 155                 160

ggt gga tcg cac ctt cca gtc cac gac ggc agc gac gca gta gta att 528
Gly Gly Ser His Leu Pro Val His Asp Gly Ser Asp Ala Val Val Ile
                165                 170                 175

aac gtt gga gaa aaa ggt gca gct caa cgt cgt att cac gtc aat ggc 576
Asn Val Gly Glu Lys Gly Ala Ala Gln Arg Arg Ile His Val Asn Gly
            180                 185                 190

gat gct ggt cat ggt tcc att cct ttc gac cgt gac agc gct att gtc 624
Asp Ala Gly His Gly Ser Ile Pro Phe Asp Arg Asp Ser Ala Ile Val
        195                 200                 205

aag atc ggt gaa gtc gcc cgc cga atc gct gcc gcc gat ctg aag gta 672
Lys Ile Gly Glu Val Ala Arg Arg Ile Ala Ala Ala Asp Leu Lys Val
    210                 215                 220

gcc aag gac gat atc tgg caa ggc ttc gtc caa gcg cac cgt ttc gac 720
Ala Lys Asp Asp Ile Trp Gln Gly Phe Val Gln Ala His Arg Phe Asp
225                 230                 235                 240

cca gaa acg gag cag gcg                                              738
Pro Glu Thr Glu Gln Ala
                245


<210> 206
<211> 246
<212> PRT
<213> Corynebacterium glutamicum

<400> 206
Leu Ile Arg Asn Ala Cys Val Asn Asp Leu Thr Pro Asp Ser Gly Gln
1               5                   10                  15

Glu Ile Arg Asn Ala Glu Ser Leu Glu Arg Phe Phe Glu Gly Thr Pro
            20                  25                  30

Asn Val Lys Ile Thr Lys Leu Glu Pro His Pro Gly Arg Thr Ser Ile
        35                  40                  45

Ile Val Thr Val Pro Gly Ser Asp Pro Asp Ala Glu Pro Leu Thr Leu
    50                  55                  60

Leu Gly His Thr Asp Val Val Pro Val Asp Leu Pro Lys Trp Thr Lys
65                  70                  75                  80

Asp Pro Phe Gly Ala Glu Ile Ser Asp Gly Gln Ile Trp Gly Arg Gly
                85                  90                  95

Ser Val Asp Met Leu Phe Ile Thr Ala Thr Gln Ala Ala Val Thr Arg
            100                 105                 110

Gln Val Ala Arg Glu Gly Gly Leu Arg Gly Thr Leu Thr Phe Val Gly
        115                 120                 125

Val Ala Asp Glu Glu Ala Arg Gly Gly Leu Gly Ala Lys Trp Leu Ser
    130                 135                 140

Glu Glu His Gln Asn Leu Phe Ser Trp Lys Asn Cys Leu Ser Glu Ser
145                 150                 155                 160

Gly Gly Ser His Leu Pro Val His Asp Gly Ser Asp Ala Val Val Ile
                165                 170                 175

Asn Val Gly Glu Lys Gly Ala Ala Gln Arg Arg Ile His Val Asn Gly
            180                 185                 190

Asp Ala Gly His Gly Ser Ile Pro Phe Asp Arg Asp Ser Ala Ile Val
        195                 200                 205

Lys Ile Gly Glu Val Ala Arg Arg Ile Ala Ala Ala Asp Leu Lys Val
    210                 215                 220

Ala Lys Asp Asp Ile Trp Gln Gly Phe Val Gln Ala His Arg Phe Asp
225                 230                 235                 240

Pro Glu Thr Glu Gln Ala
                245


<210> 207
<211> 1308
<212> DNA
<213> Corynebacterium glutamicum

<220>
<221> CDS
<222> (101)..(1285)
<223> RXN03077

<400> 207
accccgatcc tttgttttcg tgggatcact attagactcg actctaccgc gctgcaggtt 60

ttcctgatac gcctgcggac aaaacagaaa ggtatttcac gtg atg gaa att ggt   115
                                           Val Met Glu Ile Gly
                                           1               5

gtg cag gtt gcc tca tgg atg gac cgc cac cat gac gag gtc ata aag     163
Val Gln Val Ala Ser Trp Met Asp Arg His His Asp Glu Val Ile Lys
                10                  15                  20

tgg cgc agg cat ttg cac agc cat cct gag ctc tcc cac atg gaa tac     211
Trp Arg Arg His Leu His Ser His Pro Glu Leu Ser His Met Glu Tyr
            25                  30                  35

cgc acg act gag tat ttg gcc tcg gtt ctg aaa gat cac ggc atg gaa     259
Arg Thr Thr Glu Tyr Leu Ala Ser Val Leu Lys Asp His Gly Met Glu
        40                  45                  50

cca cac ctg ttc cca gga acc ggt ttg atg gtg gat atc gga cca gaa     307
Pro His Leu Phe Pro Gly Thr Gly Leu Met Val Asp Ile Gly Pro Glu
    55                  60                  65

ggg gac tcc cgc ctg gcg ttt cgc gct gat atc gat gcc ctt ccg ctg     355
Gly Asp Ser Arg Leu Ala Phe Arg Ala Asp Ile Asp Ala Leu Pro Leu
70                  75                  80                  85

ctt gaa tca acc ggc tta gag ttc tct tcc aca gcc act ggc gtt gcg     403
Leu Glu Ser Thr Gly Leu Glu Phe Ser Ser Thr Ala Thr Gly Val Ala
                90                  95                  100

cat gcc tgc gga cat gac gtg cac acg gtg atc gct ttg gca ctt gcc     451
His Ala Cys Gly His Asp Val His Thr Val Ile Ala Leu Ala Leu Ala
            105                 110                 115

tgt gca ctg aac acc atc gaa ctg ccc atc ggc att cgg gtg att ttc     499
Cys Ala Leu Asn Thr Ile Glu Leu Pro Ile Gly Ile Arg Val Ile Phe
        120                 125                 130

cag ccg gca gaa gaa gtc atg act ggt ggc gca acg gac gtc att gcc     547
Gln Pro Ala Glu Glu Val Met Thr Gly Gly Ala Thr Asp Val Ile Ala
    135                 140                 145

cac ggt ggc ctt gat ggt gtg gat gcg att tac gcc atc cac gtt gaa     595
His Gly Gly Leu Asp Gly Val Asp Ala Ile Tyr Ala Ile His Val Glu
150                 155                 160                 165

ccc aaa ttg aag gtc ggt cgc gtc ggt gta cgc gct ggc gcg att act     643
Pro Lys Leu Lys Val Gly Arg Val Gly Val Arg Ala Gly Ala Ile Thr
                170                 175                 180

tct gcc tca gat gtg atc gaa atc aga gtc aag ggt gaa gga gga cat     691
Ser Ala Ser Asp Val Ile Glu Ile Arg Val Lys Gly Glu Gly Gly His
            185                 190                 195

agc gca cgt cca cac ctc tcc gct gat gtt gtt tac gcc ttg agc aaa     739
Ser Ala Arg Pro His Leu Ser Ala Asp Val Val Tyr Ala Leu Ser Lys
        200                 205                 210

ttg gtc gtt gat ctt ccc ggt ttg ctg tcc agg cgc gtc gat cca cgc     787
Leu Val Val Asp Leu Pro Gly Leu Leu Ser Arg Arg Val Asp Pro Arg
    215                 220                 225

acc ggc acc gtg ctt gtt ttc ggc acc atc aac gcc ggc tat gcg ccc     835
Thr Gly Thr Val Leu Val Phe Gly Thr Ile Asn Ala Gly Tyr Ala Pro
230                 235                 240                 245

aac gcg atc cca gat tcc ggc atc gtg tca ggc acc ttg cgt aca gcc     883
Asn Ala Ile Pro Asp Ser Gly Ile Val Ser Gly Thr Leu Arg Thr Ala
                250                 255                 260

gac atc tct acc tgg cgt gac atg cgt ccg ctt atc tct gag ctg gtg     931
Asp Ile Ser Thr Trp Arg Asp Met Arg Pro Leu Ile Ser Glu Leu Val
            265                 270                 275

gaa cag gtg ctc gca ccc acc gga gtc acc cat gaa ctg atc tac aat     979
Glu Gln Val Leu Ala Pro Thr Gly Val Thr His Glu Leu Ile Tyr Asn
        280                 285                 290

ccg ggt gtt cca cca gtg ctt aac gac gat gtc gcc acc gct ttg ttg     1027
Pro Gly Val Pro Pro Val Leu Asn Asp Asp Val Ala Thr Ala Leu Leu
    295                 300                 305

gca agc gca gca cgc gac atg gac aca caa tct gtt gtc caa gcg ccg     1075

Ala Ser Ala Ala Arg Asp Met Asp Thr Gln Ser Val Val Gln Ala Pro
310                 315                 320                 325

cag tca tcc ggt gga gaa gac ttc tcg tgg tac ctt gaa cac gtc cca      1123
Gln Ser Ser Gly Gly Glu Asp Phe Ser Trp Tyr Leu Glu His Val Pro
                330                 335                 340

gga tca atg gcc cgg ttg ggt tgc tgg ccg ggg cac gga ccc aag caa      1171
Gly Ser Met Ala Arg Leu Gly Cys Trp Pro Gly His Gly Pro Lys Gln
            345                 350                 355

gac ctc cat caa agt gac ctg gtt gtg gat gag cga gcc atc gga gtt      1219
Asp Leu His Gln Ser Asp Leu Val Val Asp Glu Arg Ala Ile Gly Val
        360                 365                 370

ggc gtc agg ctc ttt ggc tcc ctt gtg cag cag tac agt agc cga tct      1267
Gly Val Arg Leu Phe Gly Ser Leu Val Gln Gln Tyr Ser Ser Arg Ser
    375                 380                 385

gaa gct ttc tta aat tcc taatgggggt agtgtgtagg gct                    1308
Glu Ala Phe Leu Asn Ser
390                 395


<210> 208
<211> 395
<212> PRT
<213> Corynebacterium glutamicum

<400> 208
Val Met Glu Ile Gly Val Gln Val Ala Ser Trp Met Asp Arg His His
  1               5                  10                  15

Asp Glu Val Ile Lys Trp Arg Arg His Leu His Ser His Pro Glu Leu
             20                  25                  30

Ser His Met Glu Tyr Arg Thr Thr Glu Tyr Leu Ala Ser Val Leu Lys
         35                  40                  45

Asp His Gly Met Glu Pro His Leu Phe Pro Gly Thr Gly Leu Met Val
     50                  55                  60

Asp Ile Gly Pro Glu Gly Asp Ser Arg Leu Ala Phe Arg Ala Asp Ile
 65                  70                  75                  80

Asp Ala Leu Pro Leu Leu Glu Ser Thr Gly Leu Glu Phe Ser Ser Thr
                 85                  90                  95

Ala Thr Gly Val Ala His Ala Cys Gly His Asp Val His Thr Val Ile
            100                 105                 110

Ala Leu Ala Leu Ala Cys Ala Leu Asn Thr Ile Glu Leu Pro Ile Gly
        115                 120                 125

Ile Arg Val Ile Phe Gln Pro Ala Glu Glu Val Met Thr Gly Gly Ala
    130                 135                 140

Thr Asp Val Ile Ala His Gly Gly Leu Asp Gly Val Asp Ala Ile Tyr
145                 150                 155                 160

Ala Ile His Val Glu Pro Lys Leu Lys Val Gly Arg Val Gly Val Arg
                165                 170                 175

Ala Gly Ala Ile Thr Ser Ala Ser Asp Val Ile Glu Ile Arg Val Lys
            180                 185                 190

Gly Glu Gly Gly His Ser Ala Arg Pro His Leu Ser Ala Asp Val Val
        195                 200                 205

Tyr Ala Leu Ser Lys Leu Val Val Asp Leu Pro Gly Leu Leu Ser Arg
    210                 215                 220

Arg Val Asp Pro Arg Thr Gly Thr Val Leu Val Phe Gly Thr Ile Asn
225                 230                 235                 240

Ala Gly Tyr Ala Pro Asn Ala Ile Pro Asp Ser Gly Ile Val Ser Gly
                245                 250                 255

Thr Leu Arg Thr Ala Asp Ile Ser Thr Trp Arg Asp Met Arg Pro Leu
            260                 265                 270

Ile Ser Glu Leu Val Glu Gln Val Leu Ala Pro Thr Gly Val Thr His
        275                 280                 285

Glu Leu Ile Tyr Asn Pro Gly Val Pro Pro Val Leu Asn Asp Asp Val
    290                 295                 300

Ala Thr Ala Leu Leu Ala Ser Ala Ala Arg Asp Met Asp Thr Gln Ser
305                 310                 315                 320

Val Val Gln Ala Pro Gln Ser Ser Gly Gly Glu Asp Phe Ser Trp Tyr
                325                 330                 335

Leu Glu His Val Pro Gly Ser Met Ala Arg Leu Gly Cys Trp Pro Gly
            340                 345                 350

His Gly Pro Lys Gln Asp Leu His Gln Ser Asp Leu Val Val Asp Glu
        355                 360                 365

Arg Ala Ile Gly Val Gly Val Arg Leu Phe Gly Ser Leu Val Gln Gln
    370                 375                 380

Tyr Ser Ser Arg Ser Glu Ala Phe Leu Asn Ser
385                 390                 395


<210> 209
<211> 1308
<212> DNA
<213> Corynebacterium glutamicum

<220>
<221> CDS
<222> (101)..(1285)
<223> FRXA02855

<400> 209
accccgatcc tttgttttcg tgggatcact attagactcg actctaccgc gctgcaggtt 60

ttcctgatac gcctgcggac aaaacagaaa ggtatttcac gtg atg gaa att ggt    115
                                           Val Met Glu Ile Gly
                                           1               5

gtg cag gtt gcc tca tgg atg gac cgc cac cat gac gag gtc ata aag    163
Val Gln Val Ala Ser Trp Met Asp Arg His His Asp Glu Val Ile Lys
                10                  15                  20

tgg cgc agg cat ttg cac agc cat cct gag ctc tcc cac atg gaa tac    211
Trp Arg Arg His Leu His Ser His Pro Glu Leu Ser His Met Glu Tyr
            25                  30                  35

cgc acg act gag tat ttg gcc tcg gtt ctg aaa gat cac ggc atg gaa    259
Arg Thr Thr Glu Tyr Leu Ala Ser Val Leu Lys Asp His Gly Met Glu
        40                  45                  50

cca cac ctg ttc cca gga acc ggt ttg atg gtg gat atc gga cca gaa    307
Pro His Leu Phe Pro Gly Thr Gly Leu Met Val Asp Ile Gly Pro Glu
    55                  60                  65

ggg gac tcc cgc ctg gcg ttt cgc gct gat atc gat gcc ctt ccg ctg    355
Gly Asp Ser Arg Leu Ala Phe Arg Ala Asp Ile Asp Ala Leu Pro Leu
70                  75                  80                  85

ctt gaa tca acc ggc tta gag ttc tct tcc aca gcc act ggc gtt gcg    403
Leu Glu Ser Thr Gly Leu Glu Phe Ser Ser Thr Ala Thr Gly Val Ala
                90                  95                  100

cat gcc tgc gga cat gac gtg cac acg gtg atc gct ttg gca ctt gcc    451
His Ala Cys Gly His Asp Val His Thr Val Ile Ala Leu Ala Leu Ala
            105                 110                 115

tgt gca ctg aac acc atc gaa ctg ccc atc ggc att cgg gtg att ttc    499
Cys Ala Leu Asn Thr Ile Glu Leu Pro Ile Gly Ile Arg Val Ile Phe
        120                 125                 130

cag ccg gca gaa gaa gtc atg act ggt ggc gca acg gac gtc att gcc     547
Gln Pro Ala Glu Glu Val Met Thr Gly Gly Ala Thr Asp Val Ile Ala
    135                 140                 145

cac ggt ggc ctt gat ggt gtg gat gcg att tac gcc atc cac gtt gaa     595
His Gly Gly Leu Asp Gly Val Asp Ala Ile Tyr Ala Ile His Val Glu
150                 155                 160                 165

ccc aaa ttg aag gtc ggt cgc gtc ggt gta cgc gct ggc gcg att act     643
Pro Lys Leu Lys Val Gly Arg Val Gly Val Arg Ala Gly Ala Ile Thr
                170                 175                 180

tct gcc tca gat gtg atc gaa atc aga gtc aag ggt gaa gga gga cat     691
Ser Ala Ser Asp Val Ile Glu Ile Arg Val Lys Gly Glu Gly Gly His
            185                 190                 195

agc gca cgt cca cac ctc tcc gct gat gtt gtt tac gcc ttg agc aaa     739
Ser Ala Arg Pro His Leu Ser Ala Asp Val Val Tyr Ala Leu Ser Lys
        200                 205                 210

ttg gtc gtt gat ctt ccc ggt ttg ctg tcc agg cgc gtc gat cca cgc     787
Leu Val Val Asp Leu Pro Gly Leu Leu Ser Arg Arg Val Asp Pro Arg
    215                 220                 225

acc ggc acc gtg ctt gtt ttc ggc acc atc aac gcc ggc tat gcg ccc     835
Thr Gly Thr Val Leu Val Phe Gly Thr Ile Asn Ala Gly Tyr Ala Pro
230                 235                 240                 245

aac gcg atc cca gat tcc ggc atc gtg tca ggc acc ttg cgt aca gcc     883
Asn Ala Ile Pro Asp Ser Gly Ile Val Ser Gly Thr Leu Arg Thr Ala
                250                 255                 260

gac atc tct acc tgg cgt gac atg cgt ccg ctt atc tct gag ctg gtg     931
Asp Ile Ser Thr Trp Arg Asp Met Arg Pro Leu Ile Ser Glu Leu Val
            265                 270                 275

gaa cag gtg ctc gca ccc acc gga gtc acc cat gaa ctg atc tac aat     979
Glu Gln Val Leu Ala Pro Thr Gly Val Thr His Glu Leu Ile Tyr Asn
        280                 285                 290

ccg ggt gtt cca cca gtg ctt aac gac gat gtc gcc acc gct ttg ttg     1027
Pro Gly Val Pro Pro Val Leu Asn Asp Asp Val Ala Thr Ala Leu Leu
    295                 300                 305

gca agc gca gca cgc gac atg gac aca caa tct gtt gtc caa gcg ccg     1075
Ala Ser Ala Ala Arg Asp Met Asp Thr Gln Ser Val Val Gln Ala Pro
310                 315                 320                 325

cag tca tcc ggt gga gaa gac ttc tcg tgg tac ctt gaa cac gtc cca     1123
Gln Ser Ser Gly Gly Glu Asp Phe Ser Trp Tyr Leu Glu His Val Pro
                330                 335                 340

gga tca atg gcc cgg ttg ggt tgc tgg ccg ggg cac gga ccc aag caa     1171
Gly Ser Met Ala Arg Leu Gly Cys Trp Pro Gly His Gly Pro Lys Gln
            345                 350                 355

gac ctc cat caa agt gac ctg gtt gtg gat gag cga gcc atc gga gtt     1219
Asp Leu His Gln Ser Asp Leu Val Val Asp Glu Arg Ala Ile Gly Val
        360                 365                 370

ggc gtc agg ctc ttt ggc tcc ctt gtg cag cag tac agt agc cga tct     1267
Gly Val Arg Leu Phe Gly Ser Leu Val Gln Gln Tyr Ser Ser Arg Ser
    375                 380                 385

gaa gct ttc tta aat tcc taatgggggt agtgtgtagg gct                   1308
Glu Ala Phe Leu Asn Ser
390                 395


<210> 210
<211> 395
<212> PRT
<213> Corynebacterium glutamicum

<400> 210
Val Met Glu Ile Gly Val Gln Val Ala Ser Trp Met Asp Arg His His

1               5                   10                  15

Asp Glu Val Ile Lys Trp Arg Arg His Leu His Ser His Pro Glu Leu
                20                  25                  30

Ser His Met Glu Tyr Arg Thr Thr Glu Tyr Leu Ala Ser Val Leu Lys
            35                  40                  45

Asp His Gly Met Glu Pro His Leu Phe Pro Gly Thr Gly Leu Met Val
    50                  55                  60

Asp Ile Gly Pro Glu Gly Asp Ser Arg Leu Ala Phe Arg Ala Asp Ile
65                  70                  75                  80

Asp Ala Leu Pro Leu Leu Glu Ser Thr Gly Leu Glu Phe Ser Ser Thr
                85                  90                  95

Ala Thr Gly Val Ala His Ala Cys Gly His Asp Val His Thr Val Ile
            100                 105                 110

Ala Leu Ala Leu Ala Cys Ala Leu Asn Thr Ile Glu Leu Pro Ile Gly
        115                 120                 125

Ile Arg Val Ile Phe Gln Pro Ala Glu Glu Val Met Thr Gly Gly Ala
    130                 135                 140

Thr Asp Val Ile Ala His Gly Gly Leu Asp Gly Val Asp Ala Ile Tyr
145                 150                 155                 160

Ala Ile His Val Glu Pro Lys Leu Lys Val Gly Arg Val Gly Val Arg
                165                 170                 175

Ala Gly Ala Ile Thr Ser Ala Ser Asp Val Ile Glu Ile Arg Val Lys
            180                 185                 190

Gly Glu Gly Gly His Ser Ala Arg Pro His Leu Ser Ala Asp Val Val
        195                 200                 205

Tyr Ala Leu Ser Lys Leu Val Val Asp Leu Pro Gly Leu Leu Ser Arg
    210                 215                 220

Arg Val Asp Pro Arg Thr Gly Thr Val Leu Val Phe Gly Thr Ile Asn
225                 230                 235                 240

Ala Gly Tyr Ala Pro Asn Ala Ile Pro Asp Ser Gly Ile Val Ser Gly
                245                 250                 255

Thr Leu Arg Thr Ala Asp Ile Ser Thr Trp Arg Asp Met Arg Pro Leu
            260                 265                 270

Ile Ser Glu Leu Val Glu Gln Val Leu Ala Pro Thr Gly Val Thr His
        275                 280                 285

Glu Leu Ile Tyr Asn Pro Gly Val Pro Pro Val Leu Asn Asp Asp Val
    290                 295                 300

Ala Thr Ala Leu Leu Ala Ser Ala Ala Arg Asp Met Asp Thr Gln Ser
305                 310                 315                 320

Val Val Gln Ala Pro Gln Ser Ser Gly Gly Glu Asp Phe Ser Trp Tyr
                325                 330                 335

Leu Glu His Val Pro Gly Ser Met Ala Arg Leu Gly Cys Trp Pro Gly
            340                 345                 350

His Gly Pro Lys Gln Asp Leu His Gln Ser Asp Leu Val Val Asp Glu
        355                 360                 365

Arg Ala Ile Gly Val Gly Val Arg Leu Phe Gly Ser Leu Val Gln Gln
    370                 375                 380

Tyr Ser Ser Arg Ser Glu Ala Phe Leu Asn Ser
385                 390                 395

<210> 211
<211> 1509

<212> DNA
<213> Corynebacterium glutamicum

<220>
<221> CDS
<222> (101)..(1486)
<223> RXA00026

<400> 211
ccctttctgg ctagcctggg ctacattgtt ggcaatttgg ttctacgccg atttgccgct 60

tggacctggc tctgcgatct tcctcgaagg ataagttttc atg agt act gac aat 115
Met Ser Thr Asp Asn
1               5

ttt tct cca caa gtt ccg tcg act gtg tat ttg gat tac atg gag caa 163
Phe Ser Pro Gln Val Pro Ser Thr Val Tyr Leu Asp Tyr Met Glu Gln
                10                  15                  20

ggg att gcc gcg cgc aaa gcg gag gca gaa tct aac gcc agc acg aag 211
Gly Ile Ala Ala Arg Lys Ala Glu Ala Glu Ser Asn Ala Ser Thr Lys
            25                  30                  35

ggg gag agc ccg gat tat cca ggc cag cag gtt att tgg cgc ctg atc 259
Gly Glu Ser Pro Asp Tyr Pro Gly Gln Gln Val Ile Trp Arg Leu Ile
        40                  45                  50

cag gaa gca ggg gag tcg ttg cgt gat gaa ctg cgc aca ctg gct ttc 307
Gln Glu Ala Gly Glu Ser Leu Arg Asp Glu Leu Arg Thr Leu Ala Phe
    55                  60                  65

acg ctg cac gac cat ccg gaa gaa gcg ttc gag gag gtg ttc gcc acc 355
Thr Leu His Asp His Pro Glu Glu Ala Phe Glu Glu Val Phe Ala Thr
70                  75                  80                  85

gag gaa atc aca aaa ctt ctg caa aat cat ggt ttt gag gtt cag agt 403
Glu Glu Ile Thr Lys Leu Leu Gln Asn His Gly Phe Glu Val Gln Ser
                90                  95                  100

gga gtt tat ggt gtt aaa acc gct cta gaa act agt ttt gaa acc cct 451
Gly Val Tyr Gly Val Lys Thr Ala Leu Glu Thr Ser Phe Glu Thr Pro
            105                 110                 115

ggt tat gat cca gcg cag cac cca agc att gcg atc ttg gcg gaa tac 499
Gly Tyr Asp Pro Ala Gln His Pro Ser Ile Ala Ile Leu Ala Glu Tyr
        120                 125                 130

gat gcc ctt cca gag atc ggc cat gca tgc ggg cac aat atc atc gca 547
Asp Ala Leu Pro Glu Ile Gly His Ala Cys Gly His Asn Ile Ile Ala
    135                 140                 145

gca gct ggt gtt ggc gca ttt tta gct gtc acc aac atg atc aaa act 595
Ala Ala Gly Val Gly Ala Phe Leu Ala Val Thr Asn Met Ile Lys Thr
150                 155                 160                 165

gcc gaa gtg aaa ggc gtg gat cac ctc gac ttt gaa ggc cgg atc gtg 643
Ala Glu Val Lys Gly Val Asp His Leu Asp Phe Glu Gly Arg Ile Val
                170                 175                 180

ctg ttg gga aca cct gct gag gag ggg cat tcc ggc aag gaa tac atg 691
Leu Leu Gly Thr Pro Ala Glu Glu Gly His Ser Gly Lys Glu Tyr Met
            185                 190                 195

atc cga aat ggc gca ttc gat ggc att gat gcg tcg att atg atg cac 739
Ile Arg Asn Gly Ala Phe Asp Gly Ile Asp Ala Ser Ile Met Met His
        200                 205                 210

ccc ttt ggc ttc gat ctg gcg gag cat gtt tgg gtg ggc aga cgt acc 787
Pro Phe Gly Phe Asp Leu Ala Glu His Val Trp Val Gly Arg Arg Thr
    215                 220                 225

atg acg gcg acg ttc cac ggt gtc tct gca cac gcg tct tcg cag cct 835
Met Thr Ala Thr Phe His Gly Val Ser Ala His Ala Ser Ser Gln Pro
230                 235                 240                 245

ttc atg ggt aaa aat gcc ctc gac gct gca agt ttg gcg tac cag ggc 883
Phe Met Gly Lys Asn Ala Leu Asp Ala Ala Ser Leu Ala Tyr Gln Gly

                    250                     255                     260

ttc gga gtt ttg cgt cag caa atg cca ccg agc gac cgc ctt cac gcc      931
Phe Gly Val Leu Arg Gln Gln Met Pro Pro Ser Asp Arg Leu His Ala
            265                 270                 275

att att acg gaa ggc gga aac cgg cca agc atc att cca gac act gca      979
Ile Ile Thr Glu Gly Gly Asn Arg Pro Ser Ile Ile Pro Asp Thr Ala
        280                 285                 290

acg atg tcg ctg tac gtg cgt tct ttg ttg ccg gaa gca ctc aaa gac     1027
Thr Met Ser Leu Tyr Val Arg Ser Leu Leu Pro Glu Ala Leu Lys Asp
    295                 300                 305

ata tcg aaa cgc gtg gat gat gtg ctc gat ggg gcg gcc ttg atg gcg     1075
Ile Ser Lys Arg Val Asp Asp Val Leu Asp Gly Ala Ala Leu Met Ala
310                 315                 320                 325

ggg gtt ggc gtc gaa aag caa tgg gat gtg cac cca gct agc ttg ccc     1123
Gly Val Gly Val Glu Lys Gln Trp Asp Val His Pro Ala Ser Leu Pro
                330                 335                 340

gtg cgc aac aat cat gtg ttg gcg cgg cgt tgg gca aaa acg cag aat     1171
Val Arg Asn Asn His Val Leu Ala Arg Arg Trp Ala Lys Thr Gln Asn
            345                 350                 355

ctg cgt ggt cga acg gcg ctt tcg gag ggt att ttg ccc gac act ctg     1219
Leu Arg Gly Arg Thr Ala Leu Ser Glu Gly Ile Leu Pro Asp Thr Leu
        360                 365                 370

gca gca tcg act gat ttt ggc aat gtc tcg cac ctg gtt ccg ggc att     1267
Ala Ala Ser Thr Asp Phe Gly Asn Val Ser His Leu Val Pro Gly Ile
    375                 380                 385

cat ccg atg gtg aaa att tct ccg gaa aac gtt gcg ctc cac acc aag     1315
His Pro Met Val Lys Ile Ser Pro Glu Asn Val Ala Leu His Thr Lys
390                 395                 400                 405

gaa ttc gcc gct tat gcg cgc acg gaa gag gcc atc gac gca gcc gtc     1363
Glu Phe Ala Ala Tyr Ala Arg Thr Glu Glu Ala Ile Asp Ala Ala Val
                410                 415                 420

gac gcc gca atc ggg ctg gcg caa gtc gcc gtt gac gcg ctt gca gat     1411
Asp Ala Ala Ile Gly Leu Ala Gln Val Ala Val Asp Ala Leu Ala Asp
            425                 430                 435

ccg caa atg ctt atc gac gcg acc ctc gag ttc acc aac tcc ggc gac     1459
Pro Gln Met Leu Ile Asp Ala Thr Leu Glu Phe Thr Asn Ser Gly Asp
        440                 445                 450

gta ctt aaa gta ggg gac tat ttg gct taggcaacga ctccgaaacc           1506
Val Leu Lys Val Gly Asp Tyr Leu Ala
    455                 460

ttc                                                                 1509


<210> 212
<211> 462
<212> PRT
<213> Corynebacterium glutamicum

<400> 212
Met Ser Thr Asp Asn Phe Ser Pro Gln Val Pro Ser Thr Val Tyr Leu
1               5                   10                  15

Asp Tyr Met Glu Gln Gly Ile Ala Ala Arg Lys Ala Glu Ala Glu Ser
            20                  25                  30

Asn Ala Ser Thr Lys Gly Glu Ser Pro Asp Tyr Pro Gly Gln Gln Val
        35                  40                  45

Ile Trp Arg Leu Ile Gln Glu Ala Gly Glu Ser Leu Arg Asp Glu Leu
    50                  55                  60

Arg Thr Leu Ala Phe Thr Leu His Asp His Pro Glu Glu Ala Phe Glu
65                  70                  75                  80

Glu Val Phe Ala Thr Glu Glu Ile Thr Lys Leu Leu Gln Asn His Gly
                85                  90                  95

Phe Glu Val Gln Ser Gly Val Tyr Gly Val Lys Thr Ala Leu Glu Thr
            100                 105                 110

Ser Phe Glu Thr Pro Gly Tyr Asp Pro Ala Gln His Pro Ser Ile Ala
        115                 120                 125

Ile Leu Ala Glu Tyr Asp Ala Leu Pro Glu Ile Gly His Ala Cys Gly
    130                 135                 140

His Asn Ile Ile Ala Ala Ala Gly Val Gly Ala Phe Leu Ala Val Thr
145                 150                 155                 160

Asn Met Ile Lys Thr Ala Glu Val Lys Gly Val Asp His Leu Asp Phe
                165                 170                 175

Glu Gly Arg Ile Val Leu Leu Gly Thr Pro Ala Glu Glu Gly His Ser
            180                 185                 190

Gly Lys Glu Tyr Met Ile Arg Asn Gly Ala Phe Asp Gly Ile Asp Ala
        195                 200                 205

Ser Ile Met Met His Pro Phe Gly Phe Asp Leu Ala Glu His Val Trp
    210                 215                 220

Val Gly Arg Arg Thr Met Thr Ala Thr Phe His Gly Val Ser Ala His
225                 230                 235                 240

Ala Ser Ser Gln Pro Phe Met Gly Lys Asn Ala Leu Asp Ala Ala Ser
                245                 250                 255

Leu Ala Tyr Gln Gly Phe Gly Val Leu Arg Gln Gln Met Pro Pro Ser
            260                 265                 270

Asp Arg Leu His Ala Ile Ile Thr Glu Gly Gly Asn Arg Pro Ser Ile
        275                 280                 285

Ile Pro Asp Thr Ala Thr Met Ser Leu Tyr Val Arg Ser Leu Leu Pro
    290                 295                 300

Glu Ala Leu Lys Asp Ile Ser Lys Arg Val Asp Asp Val Leu Asp Gly
305                 310                 315                 320

Ala Ala Leu Met Ala Gly Val Gly Val Glu Lys Gln Trp Asp Val His
                325                 330                 335

Pro Ala Ser Leu Pro Val Arg Asn Asn His Val Leu Ala Arg Arg Trp
            340                 345                 350

Ala Lys Thr Gln Asn Leu Arg Gly Arg Thr Ala Leu Ser Glu Gly Ile
        355                 360                 365

Leu Pro Asp Thr Leu Ala Ala Ser Thr Asp Phe Gly Asn Val Ser His
    370                 375                 380

Leu Val Pro Gly Ile His Pro Met Val Lys Ile Ser Pro Glu Asn Val
385                 390                 395                 400

Ala Leu His Thr Lys Glu Phe Ala Ala Tyr Ala Arg Thr Glu Glu Ala
                405                 410                 415

Ile Asp Ala Ala Val Asp Ala Ala Ile Gly Leu Ala Gln Val Ala Val
            420                 425                 430

Asp Ala Leu Ala Asp Pro Gln Met Leu Ile Asp Ala Thr Leu Glu Phe
        435                 440                 445

Thr Asn Ser Gly Asp Val Leu Lys Val Gly Asp Tyr Leu Ala
    450                 455                 460

<210> 213
<211> 954
<212> DNA

<213> Corynebacterium glutamicum

<220>
<221> CDS
<222> (101)..(931)
<223> RXA01971

<400> 213
aggtcttgtt tatttcggct actgattcag tagctgcgct ccgataggat tcttagtttt 60

cagttcagta tctttgagcc acggctagaa tgtgaatcct atg tct aag aag aag 115
Met Ser Lys Lys Lys
1               5

cct cgc ccc att ccg gtt cct gcc caa ttt atc cct ggt ctc att gat 163
Pro Arg Pro Ile Pro Val Pro Ala Gln Phe Ile Pro Gly Leu Ile Asp
                10                  15                  20

gcg cat aca cat ttg gca tcg tgt gga gga gat ctt gca ggg ttg gtg 211
Ala His Thr His Leu Ala Ser Cys Gly Gly Asp Leu Ala Gly Leu Val
            25                  30                  35

gaa agg gcc aag gag gcg ggc gtc gaa aag ctt tgt acc gtc ggt gat 259
Glu Arg Ala Lys Glu Ala Gly Val Glu Lys Leu Cys Thr Val Gly Asp
        40                  45                  50

ggt ttg gct gag gcc gag ctt gcg ctg gag gcc gcg caa cag ttt ggc 307
Gly Leu Ala Glu Ala Glu Leu Ala Leu Glu Ala Ala Gln Gln Phe Gly
    55                  60                  65

aat gtg ttt gct gcg tgt gcg att cat ccg acg aag gct gat cag ttg 355
Asn Val Phe Ala Ala Cys Ala Ile His Pro Thr Lys Ala Asp Gln Leu
70                  75                  80                  85

gat ggg gct gcg cgt gcg cgg ctg acg cag atg gcg gcg gat ccg aat 403
Asp Gly Ala Ala Arg Ala Arg Leu Thr Gln Met Ala Ala Asp Pro Asn
                90                  95                  100

tgt gtg gcc att ggt gag act ggt ttg gat tcg tat tgg atc aag cac 451
Cys Val Ala Ile Gly Glu Thr Gly Leu Asp Ser Tyr Trp Ile Lys His
            105                 110                 115

gat cca gag gac acg gcg gcg ttg gat gtg caa gag gag gcg ctg cgc 499
Asp Pro Glu Asp Thr Ala Ala Leu Asp Val Gln Glu Glu Ala Leu Arg
        120                 125                 130

tgg cat att gat ttg gca att agt gcg gat aag ccg ttg atg att cac 547
Trp His Ile Asp Leu Ala Ile Ser Ala Asp Lys Pro Leu Met Ile His
    135                 140                 145

aat cgt gag gcg gat gct gat ttg atg cga gtg ttg gcg gat gct cca 595
Asn Arg Glu Ala Asp Ala Asp Leu Met Arg Val Leu Ala Asp Ala Pro
150                 155                 160                 165

cct cca aaa gat acg att ctg cat tgt ttt tct tcg ccg ttg gac gtg 643
Pro Pro Lys Asp Thr Ile Leu His Cys Phe Ser Ser Pro Leu Asp Val
                170                 175                 180

gcg aag gaa gcg ttg gat cgt gga tat gtg ttg agt ttt gcg ggc aat 691
Ala Lys Glu Ala Leu Asp Arg Gly Tyr Val Leu Ser Phe Ala Gly Asn
            185                 190                 195

gtg acg ttt aag cgt aat gag gag ttg cgg gag gct gct cgt att gcg 739
Val Thr Phe Lys Arg Asn Glu Glu Leu Arg Glu Ala Ala Arg Ile Ala
        200                 205                 210

ccg att tcc cag att ttg att gaa acc gat gcg ccg tat atg acg ccg 787
Pro Ile Ser Gln Ile Leu Ile Glu Thr Asp Ala Pro Tyr Met Thr Pro
    215                 220                 225

gag ccg ttt cgg ggg agt agg aat gag ccg tcg ttg att ggt cat acg 835
Glu Pro Phe Arg Gly Ser Arg Asn Glu Pro Ser Leu Ile Gly His Thr
230                 235                 240                 245

gcg cta tgc att gcg gag gtt cgg ggg atg gct gtg gag gat gtt gcg 883
Ala Leu Cys Ile Ala Glu Val Arg Gly Met Ala Val Glu Asp Val Ala
                250                 255                 260

gcg gct ttg aat gag aat ttt gat cgc gtt tat ggg gtc aca aat cta     931
Ala Ala Leu Asn Glu Asn Phe Asp Arg Val Tyr Gly Val Thr Asn Leu
            265                 270                 275

taacgtgagg tagctcacag tca                                           954


<210> 214
<211> 277
<212> PRT
<213> Corynebacterium glutamicum

<400> 214
Met Ser Lys Lys Lys Pro Arg Pro Ile Pro Val Pro Ala Gln Phe Ile
1               5                   10                  15

Pro Gly Leu Ile Asp Ala His Thr His Leu Ala Ser Cys Gly Gly Asp
            20                  25                  30

Leu Ala Gly Leu Val Glu Arg Ala Lys Glu Ala Gly Val Glu Lys Leu
        35                  40                  45

Cys Thr Val Gly Asp Gly Leu Ala Glu Ala Glu Leu Ala Leu Glu Ala
    50                  55                  60

Ala Gln Gln Phe Gly Asn Val Phe Ala Ala Cys Ala Ile His Pro Thr
65                  70                  75                  80

Lys Ala Asp Gln Leu Asp Gly Ala Ala Arg Ala Arg Leu Thr Gln Met
                85                  90                  95

Ala Ala Asp Pro Asn Cys Val Ala Ile Gly Glu Thr Gly Leu Asp Ser
            100                 105                 110

Tyr Trp Ile Lys His Asp Pro Glu Asp Thr Ala Ala Leu Asp Val Gln
        115                 120                 125

Glu Glu Ala Leu Arg Trp His Ile Asp Leu Ala Ile Ser Ala Asp Lys
    130                 135                 140

Pro Leu Met Ile His Asn Arg Glu Ala Asp Ala Asp Leu Met Arg Val
145                 150                 155                 160

Leu Ala Asp Ala Pro Pro Pro Lys Asp Thr Ile Leu His Cys Phe Ser
                165                 170                 175

Ser Pro Leu Asp Val Ala Lys Glu Ala Leu Asp Arg Gly Tyr Val Leu
            180                 185                 190

Ser Phe Ala Gly Asn Val Thr Phe Lys Arg Asn Glu Glu Leu Arg Glu
        195                 200                 205

Ala Ala Arg Ile Ala Pro Ile Ser Gln Ile Leu Ile Glu Thr Asp Ala
    210                 215                 220

Pro Tyr Met Thr Pro Glu Pro Phe Arg Gly Ser Arg Asn Glu Pro Ser
225                 230                 235                 240

Leu Ile Gly His Thr Ala Leu Cys Ile Ala Glu Val Arg Gly Met Ala
                245                 250                 255

Val Glu Asp Val Ala Ala Ala Leu Asn Glu Asn Phe Asp Arg Val Tyr
            260                 265                 270

Gly Val Thr Asn Leu
        275


<210> 215
<211> 954
<212> DNA
<213> Corynebacterium glutamicum

<220>
<221> CDS
<222> (101)..(931)

<223> RXA01802

<400> 215
ggaattctgg acaaaagtgt tcactacgtt agacatgaga accagtgtgg cacatcacag 60

gaaatcttcg cgggtgttta gacaacccgg atgtgacaga atg ggc gat caa gac 115
Met Gly Asp Gln Asp
1               5

ata atc gga aag gaa tcc aaa caa atg gac ttt cgc ctc gtc gcg aca 163
Ile Ile Gly Lys Glu Ser Lys Gln Met Asp Phe Arg Leu Val Ala Thr
                10                  15                  20

gac atg gac ggc aca ctt tta aac acc cac cac gaa gtc cca gag aaa 211
Asp Met Asp Gly Thr Leu Leu Asn Thr His His Glu Val Pro Glu Lys
            25                  30                  35

ttt tgg gac atc ctg gaa caa atg cgt gcc aaa gga atc gcc ttc gca 259
Phe Trp Asp Ile Leu Glu Gln Met Arg Ala Lys Gly Ile Ala Phe Ala
        40                  45                  50

cca gcc agc ggc cgt caa tta gcc acc ttg caa aaa caa ttc ggg cac 307
Pro Ala Ser Gly Arg Gln Leu Ala Thr Leu Gln Lys Gln Phe Gly His
    55                  60                  65

gcg ggt gaa ccc att tct tac atc gca gaa aac ggc acc gtg gta gtc 355
Ala Gly Glu Pro Ile Ser Tyr Ile Ala Glu Asn Gly Thr Val Val Val
70                  75                  80                  85

cac gac ggc gaa att atc tcc ctg acc acc atc gac tcc gac acc gta 403
His Asp Gly Glu Ile Ile Ser Leu Thr Thr Ile Asp Ser Asp Thr Val
                90                  95                  100

cac tcc atc atc gat gcc gtg cgc gca tcc gac atc gat atg gga gta 451
His Ser Ile Ile Asp Ala Val Arg Ala Ser Asp Ile Asp Met Gly Val
            105                 110                 115

gtg gtc tgc cga cca gaa cgc gcc tac gtc gaa cgc aac gac gaa gct 499
Val Val Cys Arg Pro Glu Arg Ala Tyr Val Glu Arg Asn Asp Glu Ala
        120                 125                 130

ttc cgc gcc gaa ggc ctg aaa tac tac gtc tcc atc gag gaa gtc caa 547
Phe Arg Ala Glu Gly Leu Lys Tyr Tyr Val Ser Ile Glu Glu Val Gln
    135                 140                 145

gac ctc cac gaa gca gtc aac aat gaa gta atc aag gta gcg atc ttt 595
Asp Leu His Glu Ala Val Asn Asn Glu Val Ile Lys Val Ala Ile Phe
150                 155                 160                 165

aca ttc caa gat gcc gaa aag gac tgt gcc ccc atc atc cgc gca gcc 643
Thr Phe Gln Asp Ala Glu Lys Asp Cys Ala Pro Ile Ile Arg Ala Ala
                170                 175                 180

tcc ccc aac gcc aac gtt gtt gtc tcc ggc cag cac tgg gtc gat gtc 691
Ser Pro Asn Ala Asn Val Val Val Ser Gly Gln His Trp Val Asp Val
            185                 190                 195

atg gat cct tca gcc aac aag ggc caa gct ttg gct gct ctc cgc gat 739
Met Asp Pro Ser Ala Asn Lys Gly Gln Ala Leu Ala Ala Leu Arg Asp
        200                 205                 210

gcc ctc gga ttg gaa gaa tcc caa act ctc gtg ttt ggc gac tac ctc 787
Ala Leu Gly Leu Glu Glu Ser Gln Thr Leu Val Phe Gly Asp Tyr Leu
    215                 220                 225

aac gac act gaa ttg atc aag gcc gcc ggc aag tct tac gcc atg tcc 835
Asn Asp Thr Glu Leu Ile Lys Ala Ala Gly Lys Ser Tyr Ala Met Ser
230                 235                 240                 245

aat gcc cac ccg gac att ttg gaa ttg gcc gac gaa att gca cca tcc 883
Asn Ala His Pro Asp Ile Leu Glu Leu Ala Asp Glu Ile Ala Pro Ser
                250                 255                 260

aac att gaa gag ggc gtt att gtg gtg ctg gag aag ttg ctt aac ggt 931
Asn Ile Glu Glu Gly Val Ile Val Val Leu Glu Lys Leu Leu Asn Gly
            265                 270                 275

taacgattgc aggcagcagg ttc                                          954

<210> 216
<211> 277
<212> PRT
<213> Corynebacterium glutamicum

<400> 216
Met Gly Asp Gln Asp Ile Ile Gly Lys Glu Ser Lys Gln Met Asp Phe
1               5                   10                  15

Arg Leu Val Ala Thr Asp Met Asp Gly Thr Leu Leu Asn Thr His His
            20                  25                  30

Glu Val Pro Glu Lys Phe Trp Asp Ile Leu Glu Gln Met Arg Ala Lys
        35                  40                  45

Gly Ile Ala Phe Ala Pro Ala Ser Gly Arg Gln Leu Ala Thr Leu Gln
    50                  55                  60

Lys Gln Phe Gly His Ala Gly Glu Pro Ile Ser Tyr Ile Ala Glu Asn
65                  70                  75                  80

Gly Thr Val Val Val His Asp Gly Glu Ile Ile Ser Leu Thr Thr Ile
                85                  90                  95

Asp Ser Asp Thr Val His Ser Ile Ile Asp Ala Val Arg Ala Ser Asp
            100                 105                 110

Ile Asp Met Gly Val Val Val Cys Arg Pro Glu Arg Ala Tyr Val Glu
        115                 120                 125

Arg Asn Asp Glu Ala Phe Arg Ala Glu Gly Leu Lys Tyr Tyr Val Ser
    130                 135                 140

Ile Glu Glu Val Gln Asp Leu His Glu Ala Val Asn Asn Glu Val Ile
145                 150                 155                 160

Lys Val Ala Ile Phe Thr Phe Gln Asp Ala Glu Lys Asp Cys Ala Pro
                165                 170                 175

Ile Ile Arg Ala Ala Ser Pro Asn Ala Asn Val Val Val Ser Gly Gln
            180                 185                 190

His Trp Val Asp Val Met Asp Pro Ser Ala Asn Lys Gly Gln Ala Leu
        195                 200                 205

Ala Ala Leu Arg Asp Ala Leu Gly Leu Glu Glu Ser Gln Thr Leu Val
    210                 215                 220

Phe Gly Asp Tyr Leu Asn Asp Thr Glu Leu Ile Lys Ala Ala Gly Lys
225                 230                 235                 240

Ser Tyr Ala Met Ser Asn Ala His Pro Asp Ile Leu Glu Leu Ala Asp
                245                 250                 255

Glu Ile Ala Pro Ser Asn Ile Glu Glu Gly Val Ile Val Val Leu Glu
            260                 265                 270

Lys Leu Leu Asn Gly
        275


<210> 217
<211> 1066
<212> DNA
<213> Corynebacterium glutamicum

<220>
<221> CDS
<222> (101)..(1066)
<223> RXN00866

<400> 217
gcatcaacgt aggagatcct cgacttccaa ttatggctcc aaatgagcag gaacttgagg 60

ctctccgaga agacatgaaa aaagctggag ttctataaat atg aat gat tcc cga 115
Met Asn Asp Ser Arg
1               5

aat cgc ggc cgg aag gtt acc cgc aag gcg ggc cca cca gaa gct ggt 163
Asn Arg Gly Arg Lys Val Thr Arg Lys Ala Gly Pro Pro Glu Ala Gly
                10                  15                  20

cag gaa aac cat ctg gat acc cct gtc ttt cag gca cca gat gct tcc 211
Gln Glu Asn His Leu Asp Thr Pro Val Phe Gln Ala Pro Asp Ala Ser
            25                  30                  35

tct aac cag agc gct gta aaa gct gag acc gcc gga aac gac aat cgg 259
Ser Asn Gln Ser Ala Val Lys Ala Glu Thr Ala Gly Asn Asp Asn Arg
        40                  45                  50

gat gct gcg caa ggt gct caa gga tcc caa gat tct cag ggt tcc cag 307
Asp Ala Ala Gln Gly Ala Gln Gly Ser Gln Asp Ser Gln Gly Ser Gln
    55                  60                  65

aac gct caa ggt tcc cag aac cgc gag tcc gga aac aac aac cgc aac 355
Asn Ala Gln Gly Ser Gln Asn Arg Glu Ser Gly Asn Asn Asn Arg Asn
70                  75                  80                  85

cgt tcc aac aac aac cgt cgc ggt ggt cgt gga cgt cgt gga tcc gga 403
Arg Ser Asn Asn Asn Arg Arg Gly Gly Arg Gly Arg Arg Gly Ser Gly
                90                  95                  100

aac gcc aat gag ggc gcg aac aac aac agc ggt aac cag aac cgt cag 451
Asn Ala Asn Glu Gly Ala Asn Asn Asn Ser Gly Asn Gln Asn Arg Gln
            105                 110                 115

ggc gga aac cgt ggc aac cgc ggt ggc gga cgc cga aac gtt gtt aag 499
Gly Gly Asn Arg Gly Asn Arg Gly Gly Gly Arg Arg Asn Val Val Lys
        120                 125                 130

tcg atg cag ggt gcg gat ctg acc cag cgc ctg cca gag cca cca aag 547
Ser Met Gln Gly Ala Asp Leu Thr Gln Arg Leu Pro Glu Pro Pro Lys
    135                 140                 145

gca ccg gca aac ggt ctg cgt att tac gca ctt ggt ggc att tcc gaa 595
Ala Pro Ala Asn Gly Leu Arg Ile Tyr Ala Leu Gly Gly Ile Ser Glu
150                 155                 160                 165

atc ggt cgc aac atg acc gtg ttt gag tac aac aac cgt ctg ctc atc 643
Ile Gly Arg Asn Met Thr Val Phe Glu Tyr Asn Asn Arg Leu Leu Ile
                170                 175                 180

gtg gac tgt ggt gtg ctc ttc cca tct tca ggt gag cca ggc gtt gac 691
Val Asp Cys Gly Val Leu Phe Pro Ser Ser Gly Glu Pro Gly Val Asp
            185                 190                 195

ctg att ctt cct gac ttc ggc cca att gag gat cac ctg cac cgc gtc 739
Leu Ile Leu Pro Asp Phe Gly Pro Ile Glu Asp His Leu His Arg Val
        200                 205                 210

gat gca ttg gtg gtt act cac gga cac gaa gac cac att ggt gct att 787
Asp Ala Leu Val Val Thr His Gly His Glu Asp His Ile Gly Ala Ile
    215                 220                 225

ccc tgg ctg ctg aag ctg cgc aac gat atc cca atc ttg gca tcc cgt 835
Pro Trp Leu Leu Lys Leu Arg Asn Asp Ile Pro Ile Leu Ala Ser Arg
230                 235                 240                 245

ttc acc ttg gct ctg att gca gct aag tgt aag gaa cac cgt cag cgt 883
Phe Thr Leu Ala Leu Ile Ala Ala Lys Cys Lys Glu His Arg Gln Arg
                250                 255                 260

ccg aag ctg atc gag gtc aac gag cag tcc aat gag gac cgc gga ccg 931
Pro Lys Leu Ile Glu Val Asn Glu Gln Ser Asn Glu Asp Arg Gly Pro
            265                 270                 275

ttc aac att cgc ttc tgg gct gtt aac cac tcc atc cca gac tgc ctt 979
Phe Asn Ile Arg Phe Trp Ala Val Asn His Ser Ile Pro Asp Cys Leu
        280                 285                 290

ggt ctt gct atc aag act cct gct ggt ttg gtc atc cac acc ggt gac 1027

Gly Leu Ala Ile Lys Thr Pro Ala Gly Leu Val Ile His Thr Gly Asp
    295                 300                 305

atc aag ctg gat cag act cct cct gat gga cgc cca act                1066
Ile Lys Leu Asp Gln Thr Pro Pro Asp Gly Arg Pro Thr
310                 315                 320


<210> 218
<211> 322
<212> PRT
<213> Corynebacterium glutamicum

<400> 218
Met Asn Asp Ser Arg Asn Arg Gly Arg Lys Val Thr Arg Lys Ala Gly
  1               5                  10                  15

Pro Pro Glu Ala Gly Gln Glu Asn His Leu Asp Thr Pro Val Phe Gln
             20                  25                  30

Ala Pro Asp Ala Ser Ser Asn Gln Ser Ala Val Lys Ala Glu Thr Ala
         35                  40                  45

Gly Asn Asp Asn Arg Asp Ala Ala Gln Gly Ala Gln Gly Ser Gln Asp
     50                  55                  60

Ser Gln Gly Ser Gln Asn Ala Gln Gly Ser Gln Asn Arg Glu Ser Gly
 65                  70                  75                  80

Asn Asn Asn Arg Asn Arg Ser Asn Asn Asn Arg Arg Gly Gly Arg Gly
                 85                  90                  95

Arg Arg Gly Ser Gly Asn Ala Asn Glu Gly Ala Asn Asn Asn Ser Gly
            100                 105                 110

Asn Gln Asn Arg Gln Gly Gly Asn Arg Gly Asn Arg Gly Gly Gly Arg
        115                 120                 125

Arg Asn Val Val Lys Ser Met Gln Gly Ala Asp Leu Thr Gln Arg Leu
    130                 135                 140

Pro Glu Pro Pro Lys Ala Pro Ala Asn Gly Leu Arg Ile Tyr Ala Leu
145                 150                 155                 160

Gly Gly Ile Ser Glu Ile Gly Arg Asn Met Thr Val Phe Glu Tyr Asn
                165                 170                 175

Asn Arg Leu Leu Ile Val Asp Cys Gly Val Leu Phe Pro Ser Ser Gly
            180                 185                 190

Glu Pro Gly Val Asp Leu Ile Leu Pro Asp Phe Gly Pro Ile Glu Asp
        195                 200                 205

His Leu His Arg Val Asp Ala Leu Val Val Thr His Gly His Glu Asp
    210                 215                 220

His Ile Gly Ala Ile Pro Trp Leu Leu Lys Leu Arg Asn Asp Ile Pro
225                 230                 235                 240

Ile Leu Ala Ser Arg Phe Thr Leu Ala Leu Ile Ala Ala Lys Cys Lys
                245                 250                 255

Glu His Arg Gln Arg Pro Lys Leu Ile Glu Val Asn Glu Gln Ser Asn
            260                 265                 270

Glu Asp Arg Gly Pro Phe Asn Ile Arg Phe Trp Ala Val Asn His Ser
        275                 280                 285

Ile Pro Asp Cys Leu Gly Leu Ala Ile Lys Thr Pro Ala Gly Leu Val
    290                 295                 300

Ile His Thr Gly Asp Ile Lys Leu Asp Gln Thr Pro Pro Asp Gly Arg
305                 310                 315                 320

Pro Thr

<210> 219
<211> 1045
<212> DNA
<213> Corynebacterium glutamicum

<220>
<221> CDS
<222> (101)..(1045)
<223> FRXA00866

<400> 219
gcatcaacgt aggagatcct cgacttccaa ttatggctcc aaatgagcag gaacttgagg 60

ctctccgaga agacatgaaa aaagctggag ttctataaat atg aat gat tcc cga 115
Met Asn Asp Ser Arg
1               5

aat cgc ggc cgg aag gtt acc cgc aag gcg ggc cca cca gaa gct ggt 163
Asn Arg Gly Arg Lys Val Thr Arg Lys Ala Gly Pro Pro Glu Ala Gly
                10                  15                  20

cag gaa aac cat ctg gat acc cct gtc ttt cag gca cca gat gct tcc 211
Gln Glu Asn His Leu Asp Thr Pro Val Phe Gln Ala Pro Asp Ala Ser
            25                  30                  35

tct aac cag agc gct gta aaa gct gag acc gcc gga aac gac aat cgg 259
Ser Asn Gln Ser Ala Val Lys Ala Glu Thr Ala Gly Asn Asp Asn Arg
        40                  45                  50

gat gct gcg caa ggt gct caa gga tcc caa gat tct cag ggt tcc cag 307
Asp Ala Ala Gln Gly Ala Gln Gly Ser Gln Asp Ser Gln Gly Ser Gln
    55                  60                  65

aac gct caa ggt tcc cag aac cgc gag tcc gga aac aac aac cgc aac 355
Asn Ala Gln Gly Ser Gln Asn Arg Glu Ser Gly Asn Asn Asn Arg Asn
70                  75                  80                  85

cgt tcc aac aac aac cgt cgc ggt ggt cgt gga cgt cgt gga tcc gga 403
Arg Ser Asn Asn Asn Arg Arg Gly Gly Arg Gly Arg Arg Gly Ser Gly
                90                  95                  100

aac gcc aat gag ggc gcg aac aac aac agc ggt aac cag aac cgt cag 451
Asn Ala Asn Glu Gly Ala Asn Asn Asn Ser Gly Asn Gln Asn Arg Gln
            105                 110                 115

ggc gga aac cgt ggc aac cgc ggt ggc gga cgc cga aac gtt gtt aag 499
Gly Gly Asn Arg Gly Asn Arg Gly Gly Gly Arg Arg Asn Val Val Lys
        120                 125                 130

tcg atg cag ggt gcg gat ctg acc cag cgc ctg cca gag cca cca aag 547
Ser Met Gln Gly Ala Asp Leu Thr Gln Arg Leu Pro Glu Pro Pro Lys
    135                 140                 145

gca ccg gca aac ggt ctg cgt att tac gca ctt ggt ggc att tcc gaa 595
Ala Pro Ala Asn Gly Leu Arg Ile Tyr Ala Leu Gly Gly Ile Ser Glu
150                 155                 160                 165

atc ggt cgc aac atg acc gtg ttt gag tac aac aac cgt ctg ctc atc 643
Ile Gly Arg Asn Met Thr Val Phe Glu Tyr Asn Asn Arg Leu Leu Ile
                170                 175                 180

gtg gac tgt ggt gtg ctc ttc cca tct tca ggt gag cca ggc gtt gac 691
Val Asp Cys Gly Val Leu Phe Pro Ser Ser Gly Glu Pro Gly Val Asp
            185                 190                 195

ctg att ctt cct gac ttc ggc cca att gag gat cac ctg cac cgc gtc 739
Leu Ile Leu Pro Asp Phe Gly Pro Ile Glu Asp His Leu His Arg Val
        200                 205                 210

gat gca ttg gtg gtt act cac gga cac gaa gac cac att ggt gct att 787
Asp Ala Leu Val Val Thr His Gly His Glu Asp His Ile Gly Ala Ile
    215                 220                 225

ccc tgg ctg ctg aag ctg cgc aac gat atc cca atc ttg gca tcc cgt 835
Pro Trp Leu Leu Lys Leu Arg Asn Asp Ile Pro Ile Leu Ala Ser Arg
230                 235                 240                 245

ttc acc ttg gct ctg att gca gct aag tgt aag gaa cac cgt cag cgt     883
Phe Thr Leu Ala Leu Ile Ala Ala Lys Cys Lys Glu His Arg Gln Arg
                250                 255                 260

ccg aag ctg atc gag gtc aac gag cag tcc aat gag gac cgc gga ccg     931
Pro Lys Leu Ile Glu Val Asn Glu Gln Ser Asn Glu Asp Arg Gly Pro
            265                 270                 275

ttc aac att cgc ttc tgg gct gtt aac cac tcc atc cca gac tgc ctt     979
Phe Asn Ile Arg Phe Trp Ala Val Asn His Ser Ile Pro Asp Cys Leu
        280                 285                 290

ggt ctt gct atc aag act cct gct ggt ttg gtc atc cac acc ggt gac    1027
Gly Leu Ala Ile Lys Thr Pro Ala Gly Leu Val Ile His Thr Gly Asp
    295                 300                 305

atc aag ctg gat cag act                                            1045
Ile Lys Leu Asp Gln Thr
310                 315


<210> 220
<211> 315
<212> PRT
<213> Corynebacterium glutamicum

<400> 220
Met Asn Asp Ser Arg Asn Arg Gly Arg Lys Val Thr Arg Lys Ala Gly
1               5                   10                  15

Pro Pro Glu Ala Gly Gln Glu Asn His Leu Asp Thr Pro Val Phe Gln
            20                  25                  30

Ala Pro Asp Ala Ser Ser Asn Gln Ser Ala Val Lys Ala Glu Thr Ala
        35                  40                  45

Gly Asn Asp Asn Arg Asp Ala Ala Gln Gly Ala Gln Gly Ser Gln Asp
    50                  55                  60

Ser Gln Gly Ser Gln Asn Ala Gln Gly Ser Gln Asn Arg Glu Ser Gly
65                  70                  75                  80

Asn Asn Asn Arg Asn Arg Ser Asn Asn Asn Arg Arg Gly Gly Arg Gly
                85                  90                  95

Arg Arg Gly Ser Gly Asn Ala Asn Glu Gly Ala Asn Asn Asn Ser Gly
            100                 105                 110

Asn Gln Asn Arg Gln Gly Gly Asn Arg Gly Asn Arg Gly Gly Gly Arg
        115                 120                 125

Arg Asn Val Val Lys Ser Met Gln Gly Ala Asp Leu Thr Gln Arg Leu
    130                 135                 140

Pro Glu Pro Pro Lys Ala Pro Ala Asn Gly Leu Arg Ile Tyr Ala Leu
145                 150                 155                 160

Gly Gly Ile Ser Glu Ile Gly Arg Asn Met Thr Val Phe Glu Tyr Asn
                165                 170                 175

Asn Arg Leu Leu Ile Val Asp Cys Gly Val Leu Phe Pro Ser Ser Gly
            180                 185                 190

Glu Pro Gly Val Asp Leu Ile Leu Pro Asp Phe Gly Pro Ile Glu Asp
        195                 200                 205

His Leu His Arg Val Asp Ala Leu Val Val Thr His Gly His Glu Asp
    210                 215                 220

His Ile Gly Ala Ile Pro Trp Leu Leu Lys Leu Arg Asn Asp Ile Pro
225                 230                 235                 240

Ile Leu Ala Ser Arg Phe Thr Leu Ala Leu Ile Ala Ala Lys Cys Lys
                245                 250                 255

Glu His Arg Gln Arg Pro Lys Leu Ile Glu Val Asn Glu Gln Ser Asn

260                 265                 270

Glu Asp Arg Gly Pro Phe Asn Ile Arg Phe Trp Ala Val Asn His Ser
        275                 280                 285

Ile Pro Asp Cys Leu Gly Leu Ala Ile Lys Thr Pro Ala Gly Leu Val
    290                 295                 300

Ile His Thr Gly Asp Ile Lys Leu Asp Gln Thr
305                 310                 315


<210> 221
<211> 789
<212> DNA
<213> Corynebacterium glutamicum

<220>
<221> CDS
<222> (101)..(766)
<223> RXA02410

<400> 221
tatgagactg accatccttg gaagctctgg tagcgtgccc gctccaggta accccgcatc 60

cggatatctg ttaacttctc cggacgcccc tgccgtgatt atg gac atg ggc cca   115
                                            Met Asp Met Gly Pro
                                            1               5

ggt gtc ctt gca gca gtt caa gaa att caa gat cct gct gat gcg cat   163
Gly Val Leu Ala Ala Val Gln Glu Ile Gln Asp Pro Ala Asp Ala His
                10                  15                  20

gtt att ttc tcc cat ttg cac acc gat cac tgc gct gat ttt gcg tcc   211
Val Ile Phe Ser His Leu His Thr Asp His Cys Ala Asp Phe Ala Ser
            25                  30                  35

ttg atg gtg tgg cgc agg ttc cac cca acg ctg gcc gcc aag agc cgc   259
Leu Met Val Trp Arg Arg Phe His Pro Thr Leu Ala Ala Lys Ser Arg
        40                  45                  50

aat ctt ttg ttt gga cct gaa gat acc ccc aac agg ctt ggt cgt ttg   307
Asn Leu Leu Phe Gly Pro Glu Asp Thr Pro Asn Arg Leu Gly Arg Leu
    55                  60                  65

agc tcc gat gag cct gat ggc gtt gac gat atg tca gat act ttt gct   355
Ser Ser Asp Glu Pro Asp Gly Val Asp Asp Met Ser Asp Thr Phe Ala
70                  75                  80                  85

ttc gac gcc tgg gaa gag cgc aag cca gag ctc att gat aat ttc acg   403
Phe Asp Ala Trp Glu Glu Arg Lys Pro Glu Leu Ile Asp Asn Phe Thr
                90                  95                  100

gtc acg ccg ttc cgc gtt gtg cac ccc att gag acc tac gcg ctt cgc   451
Val Thr Pro Phe Arg Val Val His Pro Ile Glu Thr Tyr Ala Leu Arg
            105                 110                 115

gta gag gag cac cgc acc ggc gcc tca att acg tat tcc ggt gac agc   499
Val Glu Glu His Arg Thr Gly Ala Ser Ile Thr Tyr Ser Gly Asp Ser
        120                 125                 130

gcg tac acc gaa gcg ctt atc gac gcc gcc cgc aac gtt gac att ttc   547
Ala Tyr Thr Glu Ala Leu Ile Asp Ala Ala Arg Asn Val Asp Ile Phe
    135                 140                 145

ttg tgc gag gca act tgg ggc acc tct tgc gat gac aaa gca cca gga   595
Leu Cys Glu Ala Thr Trp Gly Thr Ser Cys Asp Asp Lys Ala Pro Gly
150                 155                 160                 165

atg cat atg tgt ggc caa gac gcc gga aga att gcg gca gca gct ggc   643
Met His Met Cys Gly Gln Asp Ala Gly Arg Ile Ala Ala Ala Ala Gly
                170                 175                 180

gta aag aaa ctg att atc act cat gtt cca cca tgg att gat gca gag   691
Val Lys Lys Leu Ile Ile Thr His Val Pro Pro Trp Ile Asp Ala Glu
            185                 190                 195

gcc aca gtg gca gca gct gcg gaa cac ttt gat ggt cct atc gaa ttg     739
Ala Thr Val Ala Ala Ala Ala Glu His Phe Asp Gly Pro Ile Glu Leu
        200                 205                 210

gca cga tca gga atg gtt atc gag ttt tagtccgttt gtactaataa           786
Ala Arg Ser Gly Met Val Ile Glu Phe
    215                 220

ggt                                                                 789


<210> 222
<211> 222
<212> PRT
<213> Corynebacterium glutamicum

<400> 222
Met Asp Met Gly Pro Gly Val Leu Ala Ala Val Gln Glu Ile Gln Asp
  1               5                  10                  15

Pro Ala Asp Ala His Val Ile Phe Ser His Leu His Thr Asp His Cys
             20                  25                  30

Ala Asp Phe Ala Ser Leu Met Val Trp Arg Arg Phe His Pro Thr Leu
         35                  40                  45

Ala Ala Lys Ser Arg Asn Leu Leu Phe Gly Pro Glu Asp Thr Pro Asn
     50                  55                  60

Arg Leu Gly Arg Leu Ser Ser Asp Glu Pro Asp Gly Val Asp Asp Met
 65                  70                  75                  80

Ser Asp Thr Phe Ala Phe Asp Ala Trp Glu Glu Arg Lys Pro Glu Leu
                 85                  90                  95

Ile Asp Asn Phe Thr Val Thr Pro Phe Arg Val Val His Pro Ile Glu
            100                 105                 110

Thr Tyr Ala Leu Arg Val Glu Glu His Arg Thr Gly Ala Ser Ile Thr
        115                 120                 125

Tyr Ser Gly Asp Ser Ala Tyr Thr Glu Ala Leu Ile Asp Ala Ala Arg
    130                 135                 140

Asn Val Asp Ile Phe Leu Cys Glu Ala Thr Trp Gly Thr Ser Cys Asp
145                 150                 155                 160

Asp Lys Ala Pro Gly Met His Met Cys Gly Gln Asp Ala Gly Arg Ile
                165                 170                 175

Ala Ala Ala Ala Gly Val Lys Lys Leu Ile Ile Thr His Val Pro Pro
            180                 185                 190

Trp Ile Asp Ala Glu Ala Thr Val Ala Ala Ala Ala Glu His Phe Asp
        195                 200                 205

Gly Pro Ile Glu Leu Ala Arg Ser Gly Met Val Ile Glu Phe
    210                 215                 220


<210> 223
<211> 455
<212> DNA
<213> Corynebacterium glutamicum

<220>
<221> CDS
<222> (1)..(432)
<223> RXA00961

<400> 223
cta gag aac tgg cgt atc ggc cgc atg ttg ctg ctt ggc gac gcc gcc      48
Leu Glu Asn Trp Arg Ile Gly Arg Met Leu Leu Leu Gly Asp Ala Ala
  1               5                  10                  15

cac gca ccc ctc cag tac ctc gcc tca ggc gcg gtc atg gcc atg gaa      96
His Ala Pro Leu Gln Tyr Leu Ala Ser Gly Ala Val Met Ala Met Glu

20                  25                  30

gac gcc gag gct gtc gcc ctc ttc gct gcc gac gct gcg cgt gct ggc     144
Asp Ala Glu Ala Val Ala Leu Phe Ala Ala Asp Ala Ala Arg Ala Gly
        35                  40                  45

aac ctc gat tgg gaa gag gta ctc gca gag gtg gaa gct gaa cgc cga     192
Asn Leu Asp Trp Glu Glu Val Leu Ala Glu Val Glu Ala Glu Arg Arg
    50                  55                  60

cca cgc tgc agc cgc atc caa acc gta ggc cgt ttc tgg gga gag ctc     240
Pro Arg Cys Ser Arg Ile Gln Thr Val Gly Arg Phe Trp Gly Glu Leu
65                  70                  75                  80

tgg cat gtg gaa ggc acc gca cgt ctc atc cgc aac gaa gtt ttc cgc     288
Trp His Val Glu Gly Thr Ala Arg Leu Ile Arg Asn Glu Val Phe Arg
                85                  90                  95

caa gca gac cgc aat ggc tgg ttc atc tat gca gac tgg ctg tgg ggt     336
Gln Ala Asp Arg Asn Gly Trp Phe Ile Tyr Ala Asp Trp Leu Trp Gly
            100                 105                 110

tac gat gca tcc aag cgt gcc cac atc gcc aac cct gag ctc gga gaa     384
Tyr Asp Ala Ser Lys Arg Ala His Ile Ala Asn Pro Glu Leu Gly Glu
        115                 120                 125

atg cca caa gca ctg aag gaa tgg cgc tac gcc ctc ctc gaa cag aaa     432
Met Pro Gln Ala Leu Lys Glu Trp Arg Tyr Ala Leu Leu Glu Gln Lys
    130                 135                 140

tagcagcctc acctgttaag gga                                           455


<210> 224
<211> 144
<212> PRT
<213> Corynebacterium glutamicum

<400> 224
Leu Glu Asn Trp Arg Ile Gly Arg Met Leu Leu Leu Gly Asp Ala Ala
1               5                   10                  15

His Ala Pro Leu Gln Tyr Leu Ala Ser Gly Ala Val Met Ala Met Glu
            20                  25                  30

Asp Ala Glu Ala Val Ala Leu Phe Ala Ala Asp Ala Ala Arg Ala Gly
        35                  40                  45

Asn Leu Asp Trp Glu Glu Val Leu Ala Glu Val Glu Ala Glu Arg Arg
    50                  55                  60

Pro Arg Cys Ser Arg Ile Gln Thr Val Gly Arg Phe Trp Gly Glu Leu
65                  70                  75                  80

Trp His Val Glu Gly Thr Ala Arg Leu Ile Arg Asn Glu Val Phe Arg
                85                  90                  95

Gln Ala Asp Arg Asn Gly Trp Phe Ile Tyr Ala Asp Trp Leu Trp Gly
            100                 105                 110

Tyr Asp Ala Ser Lys Arg Ala His Ile Ala Asn Pro Glu Leu Gly Glu
        115                 120                 125

Met Pro Gln Ala Leu Lys Glu Trp Arg Tyr Ala Leu Leu Glu Gln Lys
    130                 135                 140


<210> 225
<211> 1116
<212> DNA
<213> Corynebacterium glutamicum

<220>
<221> CDS

<222> (101)..(1093)
<223> RXA00111

<400> 225
ccgagaagct ggagaaggcc aacaagcgtg gcctctacac ctccgcgtcc ttccacagcc 60

ccggcgccat cactggcgac cactaaaaaa ggagacttcg atg gcc ttt ttt agc 115
Met Ala Phe Phe Ser
1               5

ttt tcg acg tct ccc ctc acc cgc ctc atc ccc ggc agc cgc tcc aaa 163
Phe Ser Thr Ser Pro Leu Thr Arg Leu Ile Pro Gly Ser Arg Ser Lys
                10                  15                  20

gcc aca ggc gcc aaa cgg cgc ctg agc agc aca atc gcg tcg att gaa 211
Ala Thr Gly Ala Lys Arg Arg Leu Ser Ser Thr Ile Ala Ser Ile Glu
            25                  30                  35

cgc tcc ccc ggc atc att gcc cta gac gga ccg ttc acc cac gat cac 259
Arg Ser Pro Gly Ile Ile Ala Leu Asp Gly Pro Phe Thr His Asp His
        40                  45                  50

gtc tcc gta cgt ggc att cgc ctc cat tta gca gag gca ggc tcc ccc 307
Val Ser Val Arg Gly Ile Arg Leu His Leu Ala Glu Ala Gly Ser Pro
    55                  60                  65

acc aaa ccc ctg gtt ctt ctg atc cac ggg gct ttc ggc ggt tgg tac 355
Thr Lys Pro Leu Val Leu Leu Ile His Gly Ala Phe Gly Gly Trp Tyr
70                  75                  80                  85

gac tac cgc gaa gtc atc ggc cca ctc gca gat gcc ggc ttc cac gtc 403
Asp Tyr Arg Glu Val Ile Gly Pro Leu Ala Asp Ala Gly Phe His Val
                90                  95                  100

gcc gcc atc gat cta cgc ggc tac ggc atg tcc gac aaa ccc cca aca 451
Ala Ala Ile Asp Leu Arg Gly Tyr Gly Met Ser Asp Lys Pro Pro Thr
            105                 110                 115

ggc tac gac ctc cgc cac gca gcc gga gaa ctc agc agc gtt atc gca 499
Gly Tyr Asp Leu Arg His Ala Ala Gly Glu Leu Ser Ser Val Ile Ala
        120                 125                 130

gct ctc ggc cac gat gac gca ctt ctt gtc ggc tcc gac acc ggc gcc 547
Ala Leu Gly His Asp Asp Ala Leu Leu Val Gly Ser Asp Thr Gly Ala
    135                 140                 145

agc atc gcc tgg gct atc gct tcc atg tac ccc gaa cgg gtc cgc ggc 595
Ser Ile Ala Trp Ala Ile Ala Ser Met Tyr Pro Glu Arg Val Arg Gly
150                 155                 160                 165

cta att tcc ctc ggc gcg atc cac ccc ctt gac atg cga cgc gcc atc 643
Leu Ile Ser Leu Gly Ala Ile His Pro Leu Asp Met Arg Arg Ala Ile
                170                 175                 180

cga cga aaa ccc cac cta cac gtc tct gac ctc agc cga ctt gct cct 691
Arg Arg Lys Pro His Leu His Val Ser Asp Leu Ser Arg Leu Ala Pro
            185                 190                 195

ttt cgg ttg ccc tca ttc ctg cat aac ctc ttc cac ttc gga atc acc 739
Phe Arg Leu Pro Ser Phe Leu His Asn Leu Phe His Phe Gly Ile Thr
        200                 205                 210

agc gaa gct cga cgt gag atc gtc aac aac acg tcc tcg tcc tac cag 787
Ser Glu Ala Arg Arg Glu Ile Val Asn Asn Thr Ser Ser Ser Tyr Gln
    215                 220                 225

cgc agc aac gca ttc aca gag aca gtg ctc ctc cgc aaa aaa gca cta 835
Arg Ser Asn Ala Phe Thr Glu Thr Val Leu Leu Arg Lys Lys Ala Leu
230                 235                 240                 245

tcg atc gac cac acc atc acc ccg atc atc cgc acc aac cgc tac ctc 883
Ser Ile Asp His Thr Ile Thr Pro Ile Ile Arg Thr Asn Arg Tyr Leu
                250                 255                 260

gtt ggg tcg atc ccc agc aaa aca gtc tcc gca ccg gtg tgg ctg ctc 931
Val Gly Ser Ile Pro Ser Lys Thr Val Ser Ala Pro Val Trp Leu Leu
            265                 270                 275

aga acc aac act cga cgc tgg gaa cat cta gcc aat act gcg cgc act     979
Arg Thr Asn Thr Arg Arg Trp Glu His Leu Ala Asn Thr Ala Arg Thr
        280                 285                 290

cga acg aca ggg cca ttc acc acc atc gcg atc ccc ggc ggc tac gaa     1027
Arg Thr Thr Gly Pro Phe Thr Thr Ile Ala Ile Pro Gly Gly Tyr Glu
    295                 300                 305

ctc ccc tac ctc gag aac cct tcc gaa ttt gca gca acc atc gca gag     1075
Leu Pro Tyr Leu Glu Asn Pro Ser Glu Phe Ala Ala Thr Ile Ala Glu
310                 315                 320                 325

ttc gcg cgc acc acg ttt taagcactgt ggctgaggcg ctg                   1116
Phe Ala Arg Thr Thr Phe
                330


<210> 226
<211> 331
<212> PRT
<213> Corynebacterium glutamicum

<400> 226
Met Ala Phe Phe Ser Phe Ser Thr Ser Pro Leu Thr Arg Leu Ile Pro
1               5                   10                  15

Gly Ser Arg Ser Lys Ala Thr Gly Ala Lys Arg Arg Leu Ser Ser Thr
            20                  25                  30

Ile Ala Ser Ile Glu Arg Ser Pro Gly Ile Ile Ala Leu Asp Gly Pro
        35                  40                  45

Phe Thr His Asp His Val Ser Val Arg Gly Ile Arg Leu His Leu Ala
    50                  55                  60

Glu Ala Gly Ser Pro Thr Lys Pro Leu Val Leu Leu Ile His Gly Ala
65                  70                  75                  80

Phe Gly Gly Trp Tyr Asp Tyr Arg Glu Val Ile Gly Pro Leu Ala Asp
                85                  90                  95

Ala Gly Phe His Val Ala Ala Ile Asp Leu Arg Gly Tyr Gly Met Ser
            100                 105                 110

Asp Lys Pro Pro Thr Gly Tyr Asp Leu Arg His Ala Ala Gly Glu Leu
        115                 120                 125

Ser Ser Val Ile Ala Ala Leu Gly His Asp Asp Ala Leu Leu Val Gly
    130                 135                 140

Ser Asp Thr Gly Ala Ser Ile Ala Trp Ala Ile Ala Ser Met Tyr Pro
145                 150                 155                 160

Glu Arg Val Arg Gly Leu Ile Ser Leu Gly Ala Ile His Pro Leu Asp
                165                 170                 175

Met Arg Arg Ala Ile Arg Arg Lys Pro His Leu His Val Ser Asp Leu
            180                 185                 190

Ser Arg Leu Ala Pro Phe Arg Leu Pro Ser Phe Leu His Asn Leu Phe
        195                 200                 205

His Phe Gly Ile Thr Ser Glu Ala Arg Arg Glu Ile Val Asn Asn Thr
    210                 215                 220

Ser Ser Ser Tyr Gln Arg Ser Asn Ala Phe Thr Glu Thr Val Leu Leu
225                 230                 235                 240

Arg Lys Lys Ala Leu Ser Ile Asp His Thr Ile Thr Pro Ile Ile Arg
                245                 250                 255

Thr Asn Arg Tyr Leu Val Gly Ser Ile Pro Ser Lys Thr Val Ser Ala
            260                 265                 270

Pro Val Trp Leu Leu Arg Thr Asn Thr Arg Arg Trp Glu His Leu Ala
        275                 280                 285

Asn Thr Ala Arg Thr Arg Thr Thr Gly Pro Phe Thr Thr Ile Ala Ile
    290                 295                 300

Pro Gly Gly Tyr Glu Leu Pro Tyr Leu Glu Asn Pro Ser Glu Phe Ala
305                 310                 315                 320

Ala Thr Ile Ala Glu Phe Ala Arg Thr Thr Phe
                325                 330


<210> 227
<211> 1020
<212> DNA
<213> Corynebacterium glutamicum

<220>
<221> CDS
<222> (101)..(997)
<223> RXA01932

<400> 227
tttctaacct gcatccaagc ctaggtggaa ttgagatgac gcgtcgtaga gatcgaaaac 60

tcaaccaaat ttcttgccta aggcttctag gattgtcgtt atg ctc ctt cac cca    115
                                           Met Leu Leu His Pro
                                           1               5

gat gcg cag ttt tat atc gat acc ttg ccc act ctc agc gcg gag gag    163
Asp Ala Gln Phe Tyr Ile Asp Thr Leu Pro Thr Leu Ser Ala Glu Glu
                10                  15                  20

cag gtg agt ttt ggt aaa gac gct cct gtt tca gag gct gat gca acc    211
Gln Val Ser Phe Gly Lys Asp Ala Pro Val Ser Glu Ala Asp Ala Thr
            25                  30                  35

cat gtg gcg aca gat caa gat att gct ggg gtg ccg gtg agg gtt tat    259
His Val Ala Thr Asp Gln Asp Ile Ala Gly Val Pro Val Arg Val Tyr
        40                  45                  50

acg cct tta tct ggg gct ggg gat ttg ccg tgt ttg gtg tac ttc cac    307
Thr Pro Leu Ser Gly Ala Gly Asp Leu Pro Cys Leu Val Tyr Phe His
    55                  60                  65

ggc ggt ggc tgg tcc ggc ggc acc ctc aac atg atc gat gcc acg gtt    355
Gly Gly Gly Trp Ser Gly Gly Thr Leu Asn Met Ile Asp Ala Thr Val
70                  75                  80                  85

cac tct cta gtg gtt ggc ctg ccg atc atc gcc atc agc gtg gac tac    403
His Ser Leu Val Val Gly Leu Pro Ile Ile Ala Ile Ser Val Asp Tyr
                90                  95                  100

cga ctt gca ccc gca cac cca ttt cca gcg gct atc gac gac gcg ttt    451
Arg Leu Ala Pro Ala His Pro Phe Pro Ala Ala Ile Asp Asp Ala Phe
            105                 110                 115

gca gtg gtc agt gcc gta ttg gat ggg gtg tct ggg ctg agt att gat    499
Ala Val Val Ser Ala Val Leu Asp Gly Val Ser Gly Leu Ser Ile Asp
        120                 125                 130

act tcc cga gtg gca att ggc ggt gac agt gcc ggt gga aat att gcc    547
Thr Ser Arg Val Ala Ile Gly Gly Asp Ser Ala Gly Gly Asn Ile Ala
    135                 140                 145

gcg gtt act gca caa cag ctg cgt gaa cgg gct gtg ggt tct act cct    595
Ala Val Thr Ala Gln Gln Leu Arg Glu Arg Ala Val Gly Ser Thr Pro
150                 155                 160                 165

gta ttg gct cac cag gtg ctt att ttt ccg gta act gat gtt tcc act    643
Val Leu Ala His Gln Val Leu Ile Phe Pro Val Thr Asp Val Ser Thr
                170                 175                 180

aca tct acg ccg agc tat ctc aca ttt ggc aaa gat tgc tac ctg aca    691
Thr Ser Thr Pro Ser Tyr Leu Thr Phe Gly Lys Asp Cys Tyr Leu Thr
            185                 190                 195

aag gac gcg atg gaa cgc tac atc gaa caa tat gcc gat ggg cac gac    739

Lys Asp Ala Met Glu Arg Tyr Ile Glu Gln Tyr Ala Asp Gly His Asp
        200                 205                 210

cgc acc gac cct cga ctc tca ccg cta ctg gca tct gat ttg agc gac 787
Arg Thr Asp Pro Arg Leu Ser Pro Leu Leu Ala Ser Asp Leu Ser Asp
    215                 220                 225

ctc cca ccc acc acc att gtg tac ggc gaa tgc gac gtg tta gcc cat 835
Leu Pro Pro Thr Thr Ile Val Tyr Gly Glu Cys Asp Val Leu Ala His
230                 235                 240                 245

gaa gtg cga gcc tat gga caa gct cta cta gag gct gga aat tcc gtg 883
Glu Val Arg Ala Tyr Gly Gln Ala Leu Leu Glu Ala Gly Asn Ser Val
                250                 255                 260

acg atg act gaa ttc aaa gga cag atc cac gcc ttt att aac cta ggg 931
Thr Met Thr Glu Phe Lys Gly Gln Ile His Ala Phe Ile Asn Leu Gly
            265                 270                 275

gga atc agt tcc gat gcg cgg gct gct cga cga ctc atc cgc gcc gaa 979
Gly Ile Ser Ser Asp Ala Arg Ala Ala Arg Arg Leu Ile Arg Ala Glu
        280                 285                 290

ttg gaa gca gca ctt tgt taaaggttga gatttaacat tcg 1020
Leu Glu Ala Ala Leu Cys
    295

<210> 228
<211> 299
<212> PRT
<213> Corynebacterium glutamicum

<400> 228
Met Leu Leu His Pro Asp Ala Gln Phe Tyr Ile Asp Thr Leu Pro Thr
1               5                   10                  15

Leu Ser Ala Glu Glu Gln Val Ser Phe Gly Lys Asp Ala Pro Val Ser
            20                  25                  30

Glu Ala Asp Ala Thr His Val Ala Thr Asp Gln Asp Ile Ala Gly Val
        35                  40                  45

Pro Val Arg Val Tyr Thr Pro Leu Ser Gly Ala Gly Asp Leu Pro Cys
    50                  55                  60

Leu Val Tyr Phe His Gly Gly Gly Trp Ser Gly Gly Thr Leu Asn Met
65                  70                  75                  80

Ile Asp Ala Thr Val His Ser Leu Val Val Gly Leu Pro Ile Ile Ala
                85                  90                  95

Ile Ser Val Asp Tyr Arg Leu Ala Pro Ala His Pro Phe Pro Ala Ala
            100                 105                 110

Ile Asp Asp Ala Phe Ala Val Val Ser Ala Val Leu Asp Gly Val Ser
        115                 120                 125

Gly Leu Ser Ile Asp Thr Ser Arg Val Ala Ile Gly Gly Asp Ser Ala
    130                 135                 140

Gly Gly Asn Ile Ala Ala Val Thr Ala Gln Gln Leu Arg Glu Arg Ala
145                 150                 155                 160

Val Gly Ser Thr Pro Val Leu Ala His Gln Val Leu Ile Phe Pro Val
                165                 170                 175

Thr Asp Val Ser Thr Thr Ser Thr Pro Ser Tyr Leu Thr Phe Gly Lys
            180                 185                 190

Asp Cys Tyr Leu Thr Lys Asp Ala Met Glu Arg Tyr Ile Glu Gln Tyr
        195                 200                 205

Ala Asp Gly His Asp Arg Thr Asp Pro Arg Leu Ser Pro Leu Leu Ala
    210                 215                 220

Ser Asp Leu Ser Asp Leu Pro Pro Thr Thr Ile Val Tyr Gly Glu Cys

225                 230                 235                 240

Asp Val Leu Ala His Glu Val Arg Ala Tyr Gly Gln Ala Leu Leu Glu
                245                 250                 255

Ala Gly Asn Ser Val Thr Met Thr Glu Phe Lys Gly Gln Ile His Ala
            260                 265                 270

Phe Ile Asn Leu Gly Gly Ile Ser Ser Asp Ala Arg Ala Ala Arg Arg
        275                 280                 285

Leu Ile Arg Ala Glu Leu Glu Ala Ala Leu Cys
    290                 295


<210> 229
<211> 1131
<212> DNA
<213> Corynebacterium glutamicum

<220>
<221> CDS
<222> (101)..(1108)
<223> RXA02574

<400> 229
tgtgctcctt gcgggctgcg cagaagagcc ggaacagcaa aaagcaataa gccgcttatc 60

gacgtccccc tccacccctc ccgcaccgac cgcggaggat ttg gcg cgc gcg caa   115
                                            Leu Ala Arg Ala Gln
                                            1               5

atc cct gaa cag caa cgc gac caa gtc gcg tcg ctg atg atg gtt gga   163
Ile Pro Glu Gln Gln Arg Asp Gln Val Ala Ser Leu Met Met Val Gly
                10                  15                  20

gtt gcg aat tat gat cag gca ttg gat gcg ctc aat cag ggg gtg ggt   211
Val Ala Asn Tyr Asp Gln Ala Leu Asp Ala Leu Asn Gln Gly Val Gly
            25                  30                  35

ggc atc ttt att ggt tcc tgg aca gat gaa aat ctg ctc acg gaa cct   259
Gly Ile Phe Ile Gly Ser Trp Thr Asp Glu Asn Leu Leu Thr Glu Pro
        40                  45                  50

ggc cgt aat att gag gcg ctc cgc gaa gcc gtc ggc agg gat ttc tcc   307
Gly Arg Asn Ile Glu Ala Leu Arg Glu Ala Val Gly Arg Asp Phe Ser
    55                  60                  65

gtc agc atc gac ttc gaa ggc ggc cgc gtc cag cgt gcc acc aat att   355
Val Ser Ile Asp Phe Glu Gly Gly Arg Val Gln Arg Ala Thr Asn Ile
70                  75                  80                  85

ctt ggt gat ttc ccc tca ccg cgc gtg atg gcg caa acc atg acg ccg   403
Leu Gly Asp Phe Pro Ser Pro Arg Val Met Ala Gln Thr Met Thr Pro
                90                  95                  100

gaa caa gta gaa gat ctc gca gaa atc cta ggc act ggt tta gct gca   451
Glu Gln Val Glu Asp Leu Ala Glu Ile Leu Gly Thr Gly Leu Ala Ala
            105                 110                 115

cat ggt gtg aca gtt aac ttt gca cct gtt gta gat gta gat gct tgg   499
His Gly Val Thr Val Asn Phe Ala Pro Val Val Asp Val Asp Ala Trp
        120                 125                 130

ggt ctc ccc gtc gtt ggc gat cgt tcc ttt tcc aac gac cca gcc gta   547
Gly Leu Pro Val Val Gly Asp Arg Ser Phe Ser Asn Asp Pro Ala Val
    135                 140                 145

gca gct act tat gcc aca gct ttt gca aag ggc tta agc aaa gta gga   595
Ala Ala Thr Tyr Ala Thr Ala Phe Ala Lys Gly Leu Ser Lys Val Gly
150                 155                 160                 165

att acc cca gta ttc aaa cat ttc cca ggt cac ggt cgt gca agt ggc   643
Ile Thr Pro Val Phe Lys His Phe Pro Gly His Gly Arg Ala Ser Gly
                170                 175                 180

gat tcg cac acc caa gat gtg gtg acc ccc gca ctt gat gag ctt aaa   691

Asp Ser His Thr Gln Asp Val Val Thr Pro Ala Leu Asp Glu Leu Lys
            185                 190                 195

act tac gac ctc atc cct tat ggt caa gca ctt tct gaa act gac gga 739
Thr Tyr Asp Leu Ile Pro Tyr Gly Gln Ala Leu Ser Glu Thr Asp Gly
        200                 205                 210

gcc gtc atg gtg ggc cac atg att gtt cca ggt ctt ggc acc gac gga 787
Ala Val Met Val Gly His Met Ile Val Pro Gly Leu Gly Thr Asp Gly
    215                 220                 225

gtt cca tcc tct atc gac ccc gcc acc tat caa ctg ctc cgc agt ggc 835
Val Pro Ser Ser Ile Asp Pro Ala Thr Tyr Gln Leu Leu Arg Ser Gly
230                 235                 240                 245

gat tac cca ggt ggc gtg cct ttc gat ggc gtg atc tac acc gac gat 883
Asp Tyr Pro Gly Gly Val Pro Phe Asp Gly Val Ile Tyr Thr Asp Asp
                250                 255                 260

ctc tct gga atg agt gcc att tcc gcc acc cat tca ccc gca gaa gca 931
Leu Ser Gly Met Ser Ala Ile Ser Ala Thr His Ser Pro Ala Glu Ala
            265                 270                 275

gtg ctt gcc tcc ctc aaa gca ggc gca gac caa gca cta tgg atc gac 979
Val Leu Ala Ser Leu Lys Ala Gly Ala Asp Gln Ala Leu Trp Ile Asp
        280                 285                 290

tat ggg tcg ttg ggc tcc gcg att gat cgc gtt gat gct gcc gtt agc 1027
Tyr Gly Ser Leu Gly Ser Ala Ile Asp Arg Val Asp Ala Ala Val Ser
    295                 300                 305

agc ggt gaa tac cct caa gaa caa atg ctg gca tct gcg tta aga gtc 1075
Ser Gly Glu Tyr Pro Gln Glu Gln Met Leu Ala Ser Ala Leu Arg Val
310                 315                 320                 325

caa ttg ctc tac atc aca cgt ctc gaa caa aag tgaagttacc agtccgtaac 1128
Gln Leu Leu Tyr Ile Thr Arg Leu Glu Gln Lys
                330                 335

ccc 1131

<210> 230
<211> 336
<212> PRT
<213> Corynebacterium glutamicum

<400> 230
Leu Ala Arg Ala Gln Ile Pro Glu Gln Gln Arg Asp Gln Val Ala Ser
1               5                   10                  15

Leu Met Met Val Gly Val Ala Asn Tyr Asp Gln Ala Leu Asp Ala Leu
            20                  25                  30

Asn Gln Gly Val Gly Gly Ile Phe Ile Gly Ser Trp Thr Asp Glu Asn
        35                  40                  45

Leu Leu Thr Glu Pro Gly Arg Asn Ile Glu Ala Leu Arg Glu Ala Val
    50                  55                  60

Gly Arg Asp Phe Ser Val Ser Ile Asp Phe Glu Gly Gly Arg Val Gln
65                  70                  75                  80

Arg Ala Thr Asn Ile Leu Gly Asp Phe Pro Ser Pro Arg Val Met Ala
                85                  90                  95

Gln Thr Met Thr Pro Glu Gln Val Glu Asp Leu Ala Glu Ile Leu Gly
            100                 105                 110

Thr Gly Leu Ala Ala His Gly Val Thr Val Asn Phe Ala Pro Val Val
        115                 120                 125

Asp Val Asp Ala Trp Gly Leu Pro Val Val Gly Asp Arg Ser Phe Ser
    130                 135                 140

Asn Asp Pro Ala Val Ala Ala Thr Tyr Ala Thr Ala Phe Ala Lys Gly
145                 150                 155                 160

Leu Ser Lys Val Gly Ile Thr Pro Val Phe Lys His Phe Pro Gly His
                165                 170                 175

Gly Arg Ala Ser Gly Asp Ser His Thr Gln Asp Val Val Thr Pro Ala
            180                 185                 190

Leu Asp Glu Leu Lys Thr Tyr Asp Leu Ile Pro Tyr Gly Gln Ala Leu
        195                 200                 205

Ser Glu Thr Asp Gly Ala Val Met Val Gly His Met Ile Val Pro Gly
    210                 215                 220

Leu Gly Thr Asp Gly Val Pro Ser Ser Ile Asp Pro Ala Thr Tyr Gln
225                 230                 235                 240

Leu Leu Arg Ser Gly Asp Tyr Pro Gly Gly Val Pro Phe Asp Gly Val
                245                 250                 255

Ile Tyr Thr Asp Asp Leu Ser Gly Met Ser Ala Ile Ser Ala Thr His
            260                 265                 270

Ser Pro Ala Glu Ala Val Leu Ala Ser Leu Lys Ala Gly Ala Asp Gln
        275                 280                 285

Ala Leu Trp Ile Asp Tyr Gly Ser Leu Gly Ser Ala Ile Asp Arg Val
    290                 295                 300

Asp Ala Ala Val Ser Ser Gly Glu Tyr Pro Gln Glu Gln Met Leu Ala
305                 310                 315                 320

Ser Ala Leu Arg Val Gln Leu Leu Tyr Ile Thr Arg Leu Glu Gln Lys
                325                 330                 335

<210> 231
<211> 1599
<212> DNA
<213> Corynebacterium glutamicum

<220>
<221> CDS
<222> (101)..(1576)
<223> RXN00983

<400> 231
   gtgagaaaac agtggctcaa atatcgacat cttctactca cagttcaacc tgtcgtggct 60
   ggaggccggc tgcattggtg tcgacgccga tgaaacgtcc gtg act gca ggt gaa    115
                                              Val Thr Ala Gly Glu
                                               1               5
   acc acc act atg aat gtc acg ttg acc aat cct ttc gac aac gca att    163
   Thr Thr Thr Met Asn Val Thr Leu Thr Asn Pro Phe Asp Asn Ala Ile
                   10                  15                  20
   ttt gac cga gca gtc tcc ctt gaa cgt ccc gaa gga tgg caa gct gag    211
   Phe Asp Arg Ala Val Ser Leu Glu Arg Pro Glu Gly Trp Gln Ala Glu
               25                  30                  35
   gat gtt cgt gtg tcg atc cca tct gga gaa tct gtc aca atc cca gtc    259
   Asp Val Arg Val Ser Ile Pro Ser Gly Glu Ser Val Thr Ile Pro Val
           40                  45                  50
   cag gtc aca gca ccg ctg gta gcc gac aac ggt gaa ctt cca gtg gag    307
   Gln Val Thr Ala Pro Leu Val Ala Asp Asn Gly Glu Leu Pro Val Glu
       55                  60                  65
   gtg tcc att ctt gat gga gca gac cgc tac acg ggt cgt ctc aat ctc    355
   Val Ser Ile Leu Asp Gly Ala Asp Arg Tyr Thr Gly Arg Leu Asn Leu
   70                  75                  80                  85
   act gtt cag ggt ggg caa gaa cct gca cca act tca gtg aag gtg agc    403
   Thr Val Gln Gly Gly Gln Glu Pro Ala Pro Thr Ser Val Lys Val Ser
                       90                  95                  100
   att cca aat ctc aag gac act tat gta gca ggg gag aag atc agc att     451
   Ile Pro Asn Leu Lys Asp Thr Tyr Val Ala Gly Glu Lys Ile Ser Ile
               105                 110                 115
   aac ttt gcg gtc aac aac ccg ttt gac gtt acg gtt aat tcg gtg cca     499
   Asn Phe Ala Val Asn Asn Pro Phe Asp Val Thr Val Asn Ser Val Pro
           120                 125                 130
   agc ctg ggg gaa ggc gag aac tgg atg cct gca aac cta cgc gga ttt     547
   Ser Leu Gly Glu Gly Glu Asn Trp Met Pro Ala Asn Leu Arg Gly Phe
       135                 140                 145
   gat cca gag cag ggt act ccc aac tgt cgt tac aag aat tta ggc gcg     595
   Asp Pro Glu Gln Gly Thr Pro Asn Cys Arg Tyr Lys Asn Leu Gly Ala
   150                 155                 160                 165
   aat aag agc tat gac tgc acc aca act acc tat gaa gtc agc gat ttg     643
   Asn Lys Ser Tyr Asp Cys Thr Thr Thr Thr Tyr Glu Val Ser Asp Leu
                   170                 175                 180
   gat gta gaa cgc gga tac gtg gat att cca acg gta tgg acg ttt act     691
   Asp Val Glu Arg Gly Tyr Val Asp Ile Pro Thr Val Trp Thr Phe Thr
               185                 190                 195
   aac tcc gca ggc gaa acg gta tgg tcc aaa aac gtt gat gtg cct cga     739
   Asn Ser Ala Gly Glu Thr Val Trp Ser Lys Asn Val Asp Val Pro Arg
           200                 205                 210
   gtt gaa ctc aat gga aca cag gat gct gtc act gat gca atc gta acg     787
   Val Glu Leu Asn Gly Thr Gln Asp Ala Val Thr Asp Ala Ile Val Thr
       215                 220                 225
   gtt gat ccc atc aac cca gtt cat tcc aac ggc cag agc caa act gtt     835
   Val Asp Pro Ile Asn Pro Val His Ser Asn Gly Gln Ser Gln Thr Val
   230                 235                 240                 245
   gag gtc cag gct aat gtc acc tca gag gga gat ctg cca gct gga tct     883
   Glu Val Gln Ala Asn Val Thr Ser Glu Gly Asp Leu Pro Ala Gly Ser
                   250                 255                 260
   aag gtg gcc ttt tat cta gat tca tcg ccc att gat acc gca gct gtt     931
   Lys Val Ala Phe Tyr Leu Asp Ser Ser Pro Ile Asp Thr Ala Ala Val
               265                 270                 275
   gat gcg gaa ggg cat gcc agc atc tcg att gat gtg gac aac atc gca     979
   Asp Ala Glu Gly His Ala Ser Ile Ser Ile Asp Val Asp Asn Ile Ala
           280                 285                 290
   agc gag cag cct gaa cgc aca ttt gag gtt cgc gcc cga ctc gtc gtt     1027
   Ser Glu Gln Pro Glu Arg Thr Phe Glu Val Arg Ala Arg Leu Val Val
       295                 300                 305
   cca gaa gat gca cca cga tca atc gcg cgt gat gcc ttg gca cgt ttt     1075
   Pro Glu Asp Ala Pro Arg Ser Ile Ala Arg Asp Ala Leu Ala Arg Phe
   310                 315                 320                 325
   aca gtc ctg tct gaa caa gtg cag cag aac tcc ttg gtg atc atg aat     1123
   Thr Val Leu Ser Glu Gln Val Gln Gln Asn Ser Leu Val Ile Met Asn
                   330                 335                 340
   cat cca gat gtg ttt tct gat gga caa aca aag act att gtc atc gca     1171
   His Pro Asp Val Phe Ser Asp Gly Gln Thr Lys Thr Ile Val Ile Ala
               345                 350                 355
   gcg aag gcg aca gca cac gat gga tcg ccg gtg gct atc ggt act ctc     1219
   Ala Lys Ala Thr Ala His Asp Gly Ser Pro Val Ala Ile Gly Thr Leu
           360                 365                 370
   att gca ttt cgc gtc aac ggt att gag cgg gac gtg gtt cca act aac     1267
   Ile Ala Phe Arg Val Asn Gly Ile Glu Arg Asp Val Val Pro Thr Asn
       375                 380                 385
   gcg caa gga aca gca aag ctt cag cta gac ctc aag cca gta aat act     1315
   Ala Gln Gly Thr Ala Lys Leu Gln Leu Asp Leu Lys Pro Val Asn Thr
   390                 395                 400                 405
   gaa gac gag gaa tat gaa gta aca gtt gaa gcc gag ctg gat gaa ttg     1363
   Glu Asp Glu Glu Tyr Glu Val Thr Val Glu Ala Glu Leu Asp Glu Leu
                   410                 415                 420
   act gct cag acc acg ttc aaa gta ctt gct ggt gag gaa gag gaa ccc     1411
   Thr Ala Gln Thr Thr Phe Lys Val Leu Ala Gly Glu Glu Glu Glu Pro
               425                 430                 435
   acc agc acc gaa gaa caa ccg tca gaa act gag cag cct tct gaa cct     1459
   Thr Ser Thr Glu Glu Gln Pro Ser Glu Thr Glu Gln Pro Ser Glu Pro
           440                 445                 450
   gaa gag gaa tcg act ggt gtt gct gga agc tct aac ggt ggc agt ttt     1507
   Glu Glu Glu Ser Thr Gly Val Ala Gly Ser Ser Asn Gly Gly Ser Phe
       455                 460                 465
   gtc gcg ctt tta gcg ctg ctg gca gcg ctt ggt ggc atc gtc ggt gca     1555
   Val Ala Leu Leu Ala Leu Leu Ala Ala Leu Gly Gly Ile Val Gly Ala
   470                 475                 480                 485
   gtc ctc gga ttg ctt aag ttg taggtggctg ggggcgtcga aaa               1599
   Val Leu Gly Leu Leu Lys Leu
               490


<210> 232
<211> 492
<212> PRT
<213> Corynebacterium glutamicum

<400> 232
Val Thr Ala Gly Glu Thr Thr Thr Met Asn Val Thr Leu Thr Asn Pro
1               5                   10                  15

Phe Asp Asn Ala Ile Phe Asp Arg Ala Val Ser Leu Glu Arg Pro Glu
            20                  25                  30

Gly Trp Gln Ala Glu Asp Val Arg Val Ser Ile Pro Ser Gly Glu Ser
        35                  40                  45

Val Thr Ile Pro Val Gln Val Thr Ala Pro Leu Val Ala Asp Asn Gly
    50                  55                  60

Glu Leu Pro Val Glu Val Ser Ile Leu Asp Gly Ala Asp Arg Tyr Thr
65                  70                  75                  80

Gly Arg Leu Asn Leu Thr Val Gln Gly Gly Gln Glu Pro Ala Pro Thr
                85                  90                  95

Ser Val Lys Val Ser Ile Pro Asn Leu Lys Asp Thr Tyr Val Ala Gly
            100                 105                 110

Glu Lys Ile Ser Ile Asn Phe Ala Val Asn Asn Pro Phe Asp Val Thr
        115                 120                 125

Val Asn Ser Val Pro Ser Leu Gly Glu Gly Glu Asn Trp Met Pro Ala
    130                 135                 140

Asn Leu Arg Gly Phe Asp Pro Glu Gln Gly Thr Pro Asn Cys Arg Tyr
145                 150                 155                 160

Lys Asn Leu Gly Ala Asn Lys Ser Tyr Asp Cys Thr Thr Thr Thr Tyr
                165                 170                 175

Glu Val Ser Asp Leu Asp Val Glu Arg Gly Tyr Val Asp Ile Pro Thr
            180                 185                 190

Val Trp Thr Phe Thr Asn Ser Ala Gly Glu Thr Val Trp Ser Lys Asn
        195                 200                 205

Val Asp Val Pro Arg Val Glu Leu Asn Gly Thr Gln Asp Ala Val Thr
    210                 215                 220

Asp Ala Ile Val Thr Val Asp Pro Ile Asn Pro Val His Ser Asn Gly
225                 230                 235                 240

Gln Ser Gln Thr Val Glu Val Gln Ala Asn Val Thr Ser Glu Gly Asp
                245                 250                 255

Leu Pro Ala Gly Ser Lys Val Ala Phe Tyr Leu Asp Ser Ser Pro Ile
            260                 265                 270

Asp Thr Ala Ala Val Asp Ala Glu Gly His Ala Ser Ile Ser Ile Asp
        275                 280                 285

Val Asp Asn Ile Ala Ser Glu Gln Pro Glu Arg Thr Phe Glu Val Arg
    290                 295                 300

Ala Arg Leu Val Val Pro Glu Asp Ala Pro Arg Ser Ile Ala Arg Asp
305                 310                 315                 320

Ala Leu Ala Arg Phe Thr Val Leu Ser Glu Gln Val Gln Gln Asn Ser
                325                 330                 335

Leu Val Ile Met Asn His Pro Asp Val Phe Ser Asp Gly Gln Thr Lys
            340                 345                 350

Thr Ile Val Ile Ala Ala Lys Ala Thr Ala His Asp Gly Ser Pro Val
        355                 360                 365

Ala Ile Gly Thr Leu Ile Ala Phe Arg Val Asn Gly Ile Glu Arg Asp
    370                 375                 380

Val Val Pro Thr Asn Ala Gln Gly Thr Ala Lys Leu Gln Leu Asp Leu
385                 390                 395                 400

Lys Pro Val Asn Thr Glu Asp Glu Glu Tyr Glu Val Thr Val Glu Ala
                405                 410                 415

Glu Leu Asp Glu Leu Thr Ala Gln Thr Thr Phe Lys Val Leu Ala Gly
            420                 425                 430

Glu Glu Glu Glu Pro Thr Ser Thr Glu Glu Gln Pro Ser Glu Thr Glu
        435                 440                 445

Gln Pro Ser Glu Pro Glu Glu Glu Ser Thr Gly Val Ala Gly Ser Ser
    450                 455                 460

Asn Gly Gly Ser Phe Val Ala Leu Leu Ala Leu Leu Ala Ala Leu Gly
465                 470                 475                 480

Gly Ile Val Gly Ala Val Leu Gly Leu Leu Lys Leu
                485                 490


<210> 233
<211> 1297
<212> DNA
<213> Corynebacterium glutamicum

<220>
<221> CDS
<222> (101)..(1297)
<223> FRXA00983

<400> 233
gtgagaaaac agtggctcaa atatcgacat cttctactca cagttcaacc tgtcgtggct 60

ggaggccggc tgcattggtg tcgacgccga tgaaacgtcc gtg act gca ggt gaa    115
                                            Val Thr Ala Gly Glu
                                            1               5

acc acc act atg aat gtc acg ttg acc aat cct ttc gac aac gca att    163
Thr Thr Thr Met Asn Val Thr Leu Thr Asn Pro Phe Asp Asn Ala Ile
                10                  15                  20

ttt gac cga gca gtc tcc ctt gaa cgt ccc gaa gga tgg caa gct gag    211
Phe Asp Arg Ala Val Ser Leu Glu Arg Pro Glu Gly Trp Gln Ala Glu
            25                  30                  35

gat gtt cgt gtg tcg atc cca tct gga gaa tct gtc aca atc cca gtc    259
Asp Val Arg Val Ser Ile Pro Ser Gly Glu Ser Val Thr Ile Pro Val
        40                  45                  50

cag gtc aca gca ccg ctg gta gcc gac aac ggt gaa ctt cca gtg gag     307
Gln Val Thr Ala Pro Leu Val Ala Asp Asn Gly Glu Leu Pro Val Glu
     55                  60                  65

gtg tcc att ctt gat gga gca gac cgc tac acg ggt cgt ctc aat ctc     355
Val Ser Ile Leu Asp Gly Ala Asp Arg Tyr Thr Gly Arg Leu Asn Leu
 70                  75                  80                  85

act gtt cag ggt ggg caa gaa cct gca cca act tca gtg aag gtg agc     403
Thr Val Gln Gly Gly Gln Glu Pro Ala Pro Thr Ser Val Lys Val Ser
                 90                  95                 100

att cca aat ctc aag gac act tat gta gca ggg gag aag atc agc att     451
Ile Pro Asn Leu Lys Asp Thr Tyr Val Ala Gly Glu Lys Ile Ser Ile
            105                 110                 115

aac ttt gcg gtc aac aac ccg ttt gac gtt acg gtt aat tcg gtg cca     499
Asn Phe Ala Val Asn Asn Pro Phe Asp Val Thr Val Asn Ser Val Pro
        120                 125                 130

agc ctg ggg gaa ggc gag aac tgg atg cct gca aac cta cgc gga ttt     547
Ser Leu Gly Glu Gly Glu Asn Trp Met Pro Ala Asn Leu Arg Gly Phe
    135                 140                 145

gat cca gag cag ggt act ccc aac tgt cgt tac aag aat tta ggc gcg     595
Asp Pro Glu Gln Gly Thr Pro Asn Cys Arg Tyr Lys Asn Leu Gly Ala
150                 155                 160                 165

aat aag agc tat gac tgc acc aca act acc tat gaa gtc agc gat ttg     643
Asn Lys Ser Tyr Asp Cys Thr Thr Thr Thr Tyr Glu Val Ser Asp Leu
                170                 175                 180

gat gta gaa cgc gga tac gtg gat att cca acg gta tgg acg ttt act     691
Asp Val Glu Arg Gly Tyr Val Asp Ile Pro Thr Val Trp Thr Phe Thr
            185                 190                 195

aac tcc gca ggc gaa acg gta tgg tcc aaa aac gtt gat gtg cct cga     739
Asn Ser Ala Gly Glu Thr Val Trp Ser Lys Asn Val Asp Val Pro Arg
        200                 205                 210

gtt gaa ctc aat gga aca cag gat gct gtc act gat gca atc gta acg     787
Val Glu Leu Asn Gly Thr Gln Asp Ala Val Thr Asp Ala Ile Val Thr
    215                 220                 225

gtt gat ccc atc aac cca gtt cat tcc aac ggc cag agc caa act gtt     835
Val Asp Pro Ile Asn Pro Val His Ser Asn Gly Gln Ser Gln Thr Val
230                 235                 240                 245

gag gtc cag gct aat gtc acc tca gag gga gat ctg cca gct gga tct     883
Glu Val Gln Ala Asn Val Thr Ser Glu Gly Asp Leu Pro Ala Gly Ser
                250                 255                 260

aag gtg gcc ttt tat cta gat tca tcg ccc att gat acc gca gct gtt     931
Lys Val Ala Phe Tyr Leu Asp Ser Ser Pro Ile Asp Thr Ala Ala Val
            265                 270                 275

gat gcg gaa ggg cat gcc agc atc tcg att gat gtg gac aac atc gca     979
Asp Ala Glu Gly His Ala Ser Ile Ser Ile Asp Val Asp Asn Ile Ala
        280                 285                 290

agc gag cag cct gaa cgc aca ttt gag gtt cgc gcc cga ctc gtc gtt    1027
Ser Glu Gln Pro Glu Arg Thr Phe Glu Val Arg Ala Arg Leu Val Val
    295                 300                 305

cca gaa gat gca cca cga tca atc gcg cgt gat gcc ttg gca cgt ttt    1075
Pro Glu Asp Ala Pro Arg Ser Ile Ala Arg Asp Ala Leu Ala Arg Phe
310                 315                 320                 325

aca gtc ctg tct gaa caa gtg cag cag aac tcc ttg gtg atc atg aat    1123
Thr Val Leu Ser Glu Gln Val Gln Gln Asn Ser Leu Val Ile Met Asn
                330                 335                 340

cat cca gat gtg ttt tct gat gga caa aca aag act att gtc atc gca    1171
His Pro Asp Val Phe Ser Asp Gly Gln Thr Lys Thr Ile Val Ile Ala
            345                 350                 355

gcg aag gcg aca gca cac gat gga tcg ccg gcg gct atc ggt act ctc     1219
Ala Lys Ala Thr Ala His Asp Gly Ser Pro Ala Ala Ile Gly Thr Leu
        360                 365                 370

att gca ttt cgc gtc aac ggt att gag cgg gac gtg gtt cca act aac     1267
Ile Ala Phe Arg Val Asn Gly Ile Glu Arg Asp Val Val Pro Thr Asn
    375                 380                 385

gcg caa gga aca gca aag ctt cag cta gac                             1297
Ala Gln Gly Thr Ala Lys Leu Gln Leu Asp
390                 395


<210> 234
<211> 399
<212> PRT
<213> Corynebacterium glutamicum

<400> 234
Val Thr Ala Gly Glu Thr Thr Thr Met Asn Val Thr Leu Thr Asn Pro
1               5                   10                  15

Phe Asp Asn Ala Ile Phe Asp Arg Ala Val Ser Leu Glu Arg Pro Glu
            20                  25                  30

Gly Trp Gln Ala Glu Asp Val Arg Val Ser Ile Pro Ser Gly Glu Ser
        35                  40                  45

Val Thr Ile Pro Val Gln Val Thr Ala Pro Leu Val Ala Asp Asn Gly
    50                  55                  60

Glu Leu Pro Val Glu Val Ser Ile Leu Asp Gly Ala Asp Arg Tyr Thr
65                  70                  75                  80

Gly Arg Leu Asn Leu Thr Val Gln Gly Gly Gln Glu Pro Ala Pro Thr
                85                  90                  95

Ser Val Lys Val Ser Ile Pro Asn Leu Lys Asp Thr Tyr Val Ala Gly
            100                 105                 110

Glu Lys Ile Ser Ile Asn Phe Ala Val Asn Asn Pro Phe Asp Val Thr
        115                 120                 125

Val Asn Ser Val Pro Ser Leu Gly Glu Gly Glu Asn Trp Met Pro Ala
    130                 135                 140

Asn Leu Arg Gly Phe Asp Pro Glu Gln Gly Thr Pro Asn Cys Arg Tyr
145                 150                 155                 160

Lys Asn Leu Gly Ala Asn Lys Ser Tyr Asp Cys Thr Thr Thr Thr Tyr
                165                 170                 175

Glu Val Ser Asp Leu Asp Val Glu Arg Gly Tyr Val Asp Ile Pro Thr
            180                 185                 190

Val Trp Thr Phe Thr Asn Ser Ala Gly Glu Thr Val Trp Ser Lys Asn
        195                 200                 205

Val Asp Val Pro Arg Val Glu Leu Asn Gly Thr Gln Asp Ala Val Thr
    210                 215                 220

Asp Ala Ile Val Thr Val Asp Pro Ile Asn Pro Val His Ser Asn Gly
225                 230                 235                 240

Gln Ser Gln Thr Val Glu Val Gln Ala Asn Val Thr Ser Glu Gly Asp
                245                 250                 255

Leu Pro Ala Gly Ser Lys Val Ala Phe Tyr Leu Asp Ser Ser Pro Ile
            260                 265                 270

Asp Thr Ala Ala Val Asp Ala Glu Gly His Ala Ser Ile Ser Ile Asp
        275                 280                 285

Val Asp Asn Ile Ala Ser Glu Gln Pro Glu Arg Thr Phe Glu Val Arg
    290                 295                 300

Ala Arg Leu Val Val Pro Glu Asp Ala Pro Arg Ser Ile Ala Arg Asp

305                 310                 315                 320

Ala Leu Ala Arg Phe Thr Val Leu Ser Glu Gln Val Gln Gln Asn Ser
                325                 330                 335

Leu Val Ile Met Asn His Pro Asp Val Phe Ser Asp Gly Gln Thr Lys
            340                 345                 350

Thr Ile Val Ile Ala Ala Lys Ala Thr Ala His Asp Gly Ser Pro Ala
        355                 360                 365

Ala Ile Gly Thr Leu Ile Ala Phe Arg Val Asn Gly Ile Glu Arg Asp
    370                 375                 380

Val Val Pro Thr Asn Ala Gln Gly Thr Ala Lys Leu Gln Leu Asp
385                 390                 395


<210> 235
<211> 440
<212> DNA
<213> Corynebacterium glutamicum

<220>
<221> CDS
<222> (1)..(417)
<223> RXA00984

<400> 235
caa cgt ggt acc cca gtg ctc ctt ggg gaa act cca tgg atg aaa aca    48
Gln Arg Gly Thr Pro Val Leu Leu Gly Glu Thr Pro Trp Met Lys Thr
  1               5                  10                  15

aaa atc gtg gaa ctc agc gat gga acc ctg atg aac aac agt cgt tca    96
Lys Ile Val Glu Leu Ser Asp Gly Thr Leu Met Asn Asn Ser Arg Ser
             20                  25                  30

tca gga gcc gat act tac cgc aag gtg tct tat tcc acc gac ggc ggc    144
Ser Gly Ala Asp Thr Tyr Arg Lys Val Ser Tyr Ser Thr Asp Gly Gly
         35                  40                  45

gtc act tgg acc gag cca act ctt gat acc cag ctg ccg gat cct cgc    192
Val Thr Trp Thr Glu Pro Thr Leu Asp Thr Gln Leu Pro Asp Pro Arg
     50                  55                  60

aac aat gct tcc ctg att cga gta ttc ccg aca gca cct gag gga agt    240
Asn Asn Ala Ser Leu Ile Arg Val Phe Pro Thr Ala Pro Glu Gly Ser
 65                  70                  75                  80

gcg cag gca aag gtt ctg ctg ttc tcc aac act gcc acc acg agt ggc    288
Ala Gln Ala Lys Val Leu Leu Phe Ser Asn Thr Ala Thr Thr Ser Gly
                 85                  90                  95

cgc acc aat ggc acc gtc cgc atg tcg tgt gat gat ggt cag acc tgg    336
Arg Thr Asn Gly Thr Val Arg Met Ser Cys Asp Asp Gly Gln Thr Trp
            100                 105                 110

ccg gtg tct aag gtg ttt gaa cca gga gca atc caa tat acc tcg atg    384
Pro Val Ser Lys Val Phe Glu Pro Gly Ala Ile Gln Tyr Thr Ser Met
        115                 120                 125

gca acg ctt ccc aac ggt gac atc ggc atg ctg tgagaaaaca gtggctcaaa 437
Ala Thr Leu Pro Asn Gly Asp Ile Gly Met Leu
    130                 135

tat                                                                440


<210> 236
<211> 139
<212> PRT
<213> Corynebacterium glutamicum

<400> 236
Gln Arg Gly Thr Pro Val Leu Leu Gly Glu Thr Pro Trp Met Lys Thr
  1               5                  10                  15

Lys Ile Val Glu Leu Ser Asp Gly Thr Leu Met Asn Asn Ser Arg Ser
            20                  25                  30

Ser Gly Ala Asp Thr Tyr Arg Lys Val Ser Tyr Ser Thr Asp Gly Gly
        35                  40                  45

Val Thr Trp Thr Glu Pro Thr Leu Asp Thr Gln Leu Pro Asp Pro Arg
    50                  55                  60

Asn Asn Ala Ser Leu Ile Arg Val Phe Pro Thr Ala Pro Glu Gly Ser
65                  70                  75                  80

Ala Gln Ala Lys Val Leu Leu Phe Ser Asn Thr Ala Thr Thr Ser Gly
                85                  90                  95

Arg Thr Asn Gly Thr Val Arg Met Ser Cys Asp Asp Gly Gln Thr Trp
            100                 105                 110

Pro Val Ser Lys Val Phe Glu Pro Gly Ala Ile Gln Tyr Thr Ser Met
        115                 120                 125

Ala Thr Leu Pro Asn Gly Asp Ile Gly Met Leu
    130                 135


<210> 237
<211> 832
<212> DNA
<213> Corynebacterium glutamicum

<220>
<221> CDS
<222> (101)..(832)
<223> RXN02513

<400> 237
acagcaccgt tttgtaggat aagaaaatcc cgcacacaac ccgtcctggt gggtgaagtg 60

ggggaggcat gtctatgccc ccaattagac atctgacatc atg ctt cca atc tgg   115
                                            Met Leu Pro Ile Trp
                                            1               5

atg ggt ctt cca ttc aag aaa gca ggt gct ttg tct cgg cgt aaa gca   163
Met Gly Leu Pro Phe Lys Lys Ala Gly Ala Leu Ser Arg Arg Lys Ala
                10                  15                  20

gta ttc tca gcg ctt ggt gca gcc gca ctc atg ggc gca gca cta ccc   211
Val Phe Ser Ala Leu Gly Ala Ala Ala Leu Met Gly Ala Ala Leu Pro
            25                  30                  35

acc atc cca acg gcc caa gct caa aca ccc acg ggc tac gga ttc gat   259
Thr Ile Pro Thr Ala Gln Ala Gln Thr Pro Thr Gly Tyr Gly Phe Asp
        40                  45                  50

gca aca gca agc atc agc gaa gaa cca gag ttt tca aca caa caa ctc   307
Ala Thr Ala Ser Ile Ser Glu Glu Pro Glu Phe Ser Thr Gln Gln Leu
    55                  60                  65

gct gac ggc gga act ctc gga ttt gat tgc tac cgc atc cca tcg ctt   355
Ala Asp Gly Gly Thr Leu Gly Phe Asp Cys Tyr Arg Ile Pro Ser Leu
70                  75                  80                  85

ggc gtc gca ccc aac ggc aac gtc ctc gca tcg tgg gat ggt cgc cca   403
Gly Val Ala Pro Asn Gly Asn Val Leu Ala Ser Trp Asp Gly Arg Pro
                90                  95                  100

aac aac tgt tca gat gct cca caa ccc aac tcc atc gtg ggc aag gta   451
Asn Asn Cys Ser Asp Ala Pro Gln Pro Asn Ser Ile Val Gly Lys Val
            105                 110                 115

tcg acc gac aac gga gca acc tgg ggc gaa cag cac gac att tcc gca   499
Ser Thr Asp Asn Gly Ala Thr Trp Gly Glu Gln His Asp Ile Ser Ala
        120                 125                 130

ggt atc acc gcc gaa ccc aaa act ggc tat tcc gat ccc agc atc gtt   547
Gly Ile Thr Ala Glu Pro Lys Thr Gly Tyr Ser Asp Pro Ser Ile Val
    135                 140                 145

gtg gac tgg gag agg ggc gat gtc ttt aac ttc cac gtg aag tca ttc     595
Val Asp Trp Glu Arg Gly Asp Val Phe Asn Phe His Val Lys Ser Phe
150                 155                 160                 165

gat gca gga tac ttc acc tcc caa cca ggc acg gac ccg gat gat cgc     643
Asp Ala Gly Tyr Phe Thr Ser Gln Pro Gly Thr Asp Pro Asp Asp Arg
                170                 175                 180

aac gtt gcc cat gtt gcc tac gcc aaa tca tca gat aac ggc tca acc     691
Asn Val Ala His Val Ala Tyr Ala Lys Ser Ser Asp Asn Gly Ser Thr
            185                 190                 195

tgg gtt gca gac acc gtc att act gat caa gtg gtt gct cat gac acc     739
Trp Val Ala Asp Thr Val Ile Thr Asp Gln Val Val Ala His Asp Thr
        200                 205                 210

tgg gac agc cga ttt gcc aca tcc gga aac ggc atc caa ctg caa tac     787
Trp Asp Ser Arg Phe Ala Thr Ser Gly Asn Gly Ile Gln Leu Gln Tyr
    215                 220                 225

ggc gcg tac aag gga cga ttg gtc cag cca tcg gta act cgc atg         832
Gly Ala Tyr Lys Gly Arg Leu Val Gln Pro Ser Val Thr Arg Met
230                 235                 240


<210> 238
<211> 244
<212> PRT
<213> Corynebacterium glutamicum

<400> 238
Met Leu Pro Ile Trp Met Gly Leu Pro Phe Lys Lys Ala Gly Ala Leu
1               5                   10                  15

Ser Arg Arg Lys Ala Val Phe Ser Ala Leu Gly Ala Ala Ala Leu Met
            20                  25                  30

Gly Ala Ala Leu Pro Thr Ile Pro Thr Ala Gln Ala Gln Thr Pro Thr
        35                  40                  45

Gly Tyr Gly Phe Asp Ala Thr Ala Ser Ile Ser Glu Glu Pro Glu Phe
    50                  55                  60

Ser Thr Gln Gln Leu Ala Asp Gly Gly Thr Leu Gly Phe Asp Cys Tyr
65                  70                  75                  80

Arg Ile Pro Ser Leu Gly Val Ala Pro Asn Gly Asn Val Leu Ala Ser
                85                  90                  95

Trp Asp Gly Arg Pro Asn Asn Cys Ser Asp Ala Pro Gln Pro Asn Ser
            100                 105                 110

Ile Val Gly Lys Val Ser Thr Asp Asn Gly Ala Thr Trp Gly Glu Gln
        115                 120                 125

His Asp Ile Ser Ala Gly Ile Thr Ala Glu Pro Lys Thr Gly Tyr Ser
    130                 135                 140

Asp Pro Ser Ile Val Val Asp Trp Glu Arg Gly Asp Val Phe Asn Phe
145                 150                 155                 160

His Val Lys Ser Phe Asp Ala Gly Tyr Phe Thr Ser Gln Pro Gly Thr
                165                 170                 175

Asp Pro Asp Asp Arg Asn Val Ala His Val Ala Tyr Ala Lys Ser Ser
            180                 185                 190

Asp Asn Gly Ser Thr Trp Val Ala Asp Thr Val Ile Thr Asp Gln Val
        195                 200                 205

Val Ala His Asp Thr Trp Asp Ser Arg Phe Ala Thr Ser Gly Asn Gly
    210                 215                 220

Ile Gln Leu Gln Tyr Gly Ala Tyr Lys Gly Arg Leu Val Gln Pro Ser
225                 230                 235                 240

Val Thr Arg Met

<210> 239
<211> 824
<212> DNA
<213> Corynebacterium glutamicum

<220>
<221> CDS
<222> (93)..(824)
<223> FRXA02513

<400> 239
gtcatgtagg ataagaaaat cccgcacaca acccgtcctg gtggggtaag tgagggaggc 60

atgtctatgc ccccaattag acatctgaca tc atg ctt cca atc tgg atg ggt 113
Met Leu Pro Ile Trp Met Gly
1               5

ctt cca ttc aag aaa gca ggg gct ttg tct cgg cgt aaa gca gta ttc 161
Leu Pro Phe Lys Lys Ala Gly Ala Leu Ser Arg Arg Lys Ala Val Phe
        10                  15                  20

tca gcg ctt ggt gca gac gca ctc atg ggc gca gca cta ccc acc atc 209
Ser Ala Leu Gly Ala Asp Ala Leu Met Gly Ala Ala Leu Pro Thr Ile
    25                  30                  35

cca acg gcc caa gct caa aca ccc acg ggc tac gga ttc gat gca aca 257
Pro Thr Ala Gln Ala Gln Thr Pro Thr Gly Tyr Gly Phe Asp Ala Thr
40                  45                  50                  55

gca agc atc agc gaa gaa cca gag ttt tca aca caa caa ctc gct gac 305
Ala Ser Ile Ser Glu Glu Pro Glu Phe Ser Thr Gln Gln Leu Ala Asp
                60                  65                  70

ggc gga act ctc gga ttt gat tgc tac cgc atc cca tcg ctt ggc gtc 353
Gly Gly Thr Leu Gly Phe Asp Cys Tyr Arg Ile Pro Ser Leu Gly Val
            75                  80                  85

gca ccc aac ggc aac gtc ctc gca tcg tgg gat ggt cgc cca aac aac 401
Ala Pro Asn Gly Asn Val Leu Ala Ser Trp Asp Gly Arg Pro Asn Asn
        90                  95                  100

tgt tca gat gct cca caa ccc aac tcc atc gtg ggc aag gta tcg acc 449
Cys Ser Asp Ala Pro Gln Pro Asn Ser Ile Val Gly Lys Val Ser Thr
    105                 110                 115

gac aac gga gca acc tgg ggc gaa cag cac gac att tcc gca ggt atc 497
Asp Asn Gly Ala Thr Trp Gly Glu Gln His Asp Ile Ser Ala Gly Ile
120                 125                 130                 135

acc gcc gaa ccc aaa act ggc tat tcc gat ccc agc atc gtt gtg gac 545
Thr Ala Glu Pro Lys Thr Gly Tyr Ser Asp Pro Ser Ile Val Val Asp
                140                 145                 150

tgg gag agg ggc gat gtc ttt aac ttc cac gtg aag tca ttc gat gca 593
Trp Glu Arg Gly Asp Val Phe Asn Phe His Val Lys Ser Phe Asp Ala
            155                 160                 165

gga tac ttc acc tcc caa cca ggc acg gac ccg gat gat cgc aac gtt 641
Gly Tyr Phe Thr Ser Gln Pro Gly Thr Asp Pro Asp Asp Arg Asn Val
        170                 175                 180

gcc cat gtt gcc tac gcc aaa tca tca gat aac ggc tca acc tgg gtt 689
Ala His Val Ala Tyr Ala Lys Ser Ser Asp Asn Gly Ser Thr Trp Val
    185                 190                 195

gca gac acc gtc att act gat caa gtg gtt gct cat gac acc tgg gac 737
Ala Asp Thr Val Ile Thr Asp Gln Val Val Ala His Asp Thr Trp Asp
200                 205                 210                 215

agc cga ttt gcc aca tcc gga aac ggc atc caa ctg caa tac ggc gcg 785
Ser Arg Phe Ala Thr Ser Gly Asn Gly Ile Gln Leu Gln Tyr Gly Ala
                220                 225                 230

tac aag gga cga ttg gtc cag cca tcg gta act cgc atg                824
Tyr Lys Gly Arg Leu Val Gln Pro Ser Val Thr Arg Met
            235                 240

<210> 240
<211> 244
<212> PRT
<213> Corynebacterium glutamicum

<400> 240
Met Leu Pro Ile Trp Met Gly Leu Pro Phe Lys Lys Ala Gly Ala Leu
1               5                   10                  15

Ser Arg Arg Lys Ala Val Phe Ser Ala Leu Gly Ala Asp Ala Leu Met
            20                  25                  30

Gly Ala Ala Leu Pro Thr Ile Pro Thr Ala Gln Ala Gln Thr Pro Thr
        35                  40                  45

Gly Tyr Gly Phe Asp Ala Thr Ala Ser Ile Ser Glu Glu Pro Glu Phe
    50                  55                  60

Ser Thr Gln Gln Leu Ala Asp Gly Gly Thr Leu Gly Phe Asp Cys Tyr
65                  70                  75                  80

Arg Ile Pro Ser Leu Gly Val Ala Pro Asn Gly Asn Val Leu Ala Ser
                85                  90                  95

Trp Asp Gly Arg Pro Asn Asn Cys Ser Asp Ala Pro Gln Pro Asn Ser
            100                 105                 110

Ile Val Gly Lys Val Ser Thr Asp Asn Gly Ala Thr Trp Gly Glu Gln
        115                 120                 125

His Asp Ile Ser Ala Gly Ile Thr Ala Glu Pro Lys Thr Gly Tyr Ser
    130                 135                 140

Asp Pro Ser Ile Val Val Asp Trp Glu Arg Gly Asp Val Phe Asn Phe
145                 150                 155                 160

His Val Lys Ser Phe Asp Ala Gly Tyr Phe Thr Ser Gln Pro Gly Thr
                165                 170                 175

Asp Pro Asp Asp Arg Asn Val Ala His Val Ala Tyr Ala Lys Ser Ser
            180                 185                 190

Asp Asn Gly Ser Thr Trp Val Ala Asp Thr Val Ile Thr Asp Gln Val
        195                 200                 205

Val Ala His Asp Thr Trp Asp Ser Arg Phe Ala Thr Ser Gly Asn Gly
    210                 215                 220

Ile Gln Leu Gln Tyr Gly Ala Tyr Lys Gly Arg Leu Val Gln Pro Ser
225                 230                 235                 240

Val Thr Arg Met

<210> 241
<211> 733
<212> DNA
<213> Corynebacterium glutamicum

<220>
<221> CDS
<222> (101)..(733)
<223> RXA00903

<400> 241
gcactcggaa tcgtcgtcct agaaaagaaa gacgcctaaa atgccctcta aaatatcgcg 60

gccctattac caagtagatg tattcagctc cgagccgttc atg gga aac ccg ctt   115
                                            Met Gly Asn Pro Leu
                                            1               5

gct gtc atc gcc gat gct gat gac tta agt gcc gaa caa atg gcc cga     163
Ala Val Ile Ala Asp Ala Asp Asp Leu Ser Ala Glu Gln Met Ala Arg
                10                  15                  20

atc gct agg tgg aca aac ctc tca gaa acc aca ttt ctt tta aag cca     211
Ile Ala Arg Trp Thr Asn Leu Ser Glu Thr Thr Phe Leu Leu Lys Pro
                25                  30                  35

acc caa gaa ggt gct gac tac cgg gta cgc att ttc acc cca acc ggt     259
Thr Gln Glu Gly Ala Asp Tyr Arg Val Arg Ile Phe Thr Pro Thr Gly
        40                  45                  50

gag ctc ccc ttc gct gga cac cca aca ctc gga acc gcc cac gtg ttt     307
Glu Leu Pro Phe Ala Gly His Pro Thr Leu Gly Thr Ala His Val Phe
    55                  60                  65

agg gaa ctg cac ggt gaa cag gga acc cag ttg gtt cag gaa tgt gtc     355
Arg Glu Leu His Gly Glu Gln Gly Thr Gln Leu Val Gln Glu Cys Val
 70                  75                  80                  85

gcc ggt tta gtt gct gtg cgc gct att gac ggg cca gca agt gga ttg     403
Ala Gly Leu Val Ala Val Arg Ala Ile Asp Gly Pro Ala Ser Gly Leu
                90                  95                  100

gct ttt cag gct cca ccc aca ctc aaa gac ggg cca ttg gat gct tcc     451
Ala Phe Gln Ala Pro Pro Thr Leu Lys Asp Gly Pro Leu Asp Ala Ser
            105                 110                 115

gac cta gac gca gct tgt gag gct tta gga atc agc ccc gac ttc att     499
Asp Leu Asp Ala Ala Cys Glu Ala Leu Gly Ile Ser Pro Asp Phe Ile
        120                 125                 130

cga gcc cac caa tgg gta gac aac ggc ccc ggc tgg gca gta gtg gag     547
Arg Ala His Gln Trp Val Asp Asn Gly Pro Gly Trp Ala Val Val Glu
    135                 140                 145

cta ccg agc gcc caa cac gta ttg gat ctg gaa ccc gat ttc agt gca     595
Leu Pro Ser Ala Gln His Val Leu Asp Leu Glu Pro Asp Phe Ser Ala
150                 155                 160                 165

cat cca aca ttg aaa ctc gga gtg att ggg gcc tat ccc gaa ggg gct     643
His Pro Thr Leu Lys Leu Gly Val Ile Gly Ala Tyr Pro Glu Gly Ala
                170                 175                 180

ccc cac gcc ttt gaa gta cgg gca ttc gct caa gga atc ggt gaa gac     691
Pro His Ala Phe Glu Val Arg Ala Phe Ala Gln Gly Ile Gly Glu Asp
            185                 190                 195

cca gtt aca gga agc ctc aat gca ttc att gcg cag tgg cta             733
Pro Val Thr Gly Ser Leu Asn Ala Phe Ile Ala Gln Trp Leu
        200                 205                 210


<210> 242
<211> 211
<212> PRT
<213> Corynebacterium glutamicum

<400> 242
Met Gly Asn Pro Leu Ala Val Ile Ala Asp Ala Asp Asp Leu Ser Ala
1               5                   10                  15

Glu Gln Met Ala Arg Ile Ala Arg Trp Thr Asn Leu Ser Glu Thr Thr
            20                  25                  30

Phe Leu Leu Lys Pro Thr Gln Glu Gly Ala Asp Tyr Arg Val Arg Ile
        35                  40                  45

Phe Thr Pro Thr Gly Glu Leu Pro Phe Ala Gly His Pro Thr Leu Gly
    50                  55                  60

Thr Ala His Val Phe Arg Glu Leu His Gly Glu Gln Gly Thr Gln Leu
65                  70                  75                  80

Val Gln Glu Cys Val Ala Gly Leu Val Ala Val Arg Ala Ile Asp Gly
                85                  90                  95

Pro Ala Ser Gly Leu Ala Phe Gln Ala Pro Pro Thr Leu Lys Asp Gly
            100                 105                 110

Pro Leu Asp Ala Ser Asp Leu Asp Ala Ala Cys Glu Ala Leu Gly Ile
        115                 120                 125

Ser Pro Asp Phe Ile Arg Ala His Gln Trp Val Asp Asn Gly Pro Gly
    130                 135                 140

Trp Ala Val Val Glu Leu Pro Ser Ala Gln His Val Leu Asp Leu Glu
145                 150                 155                 160

Pro Asp Phe Ser Ala His Pro Thr Leu Lys Leu Gly Val Ile Gly Ala
                165                 170                 175

Tyr Pro Glu Gly Ala Pro His Ala Phe Glu Val Arg Ala Phe Ala Gln
            180                 185                 190

Gly Ile Gly Glu Asp Pro Val Thr Gly Ser Leu Asn Ala Phe Ile Ala
        195                 200                 205

Gln Trp Leu
    210


<210> 243
<211> 1146
<212> DNA
<213> Corynebacterium glutamicum

<220>
<221> CDS
<222> (101)..(1123)
<223> RXA01224

<400> 243
ttggcggcgg gaagttcagg cttgggggca aacagtgctt ggattttaga caaaaaactc 60

acggaagtca tcctatggca ggcgcgccta ggatggtgcc atg agc atc ctt gac    115
                                           Met Ser Ile Leu Asp
                                           1               5

acg ttg aaa act ccc gtg att gtc gcc ccg atg gct ggc ggc ccg tcc    163
Thr Leu Lys Thr Pro Val Ile Val Ala Pro Met Ala Gly Gly Pro Ser
                10                  15                  20

act ccc gcg ttg gtc aat gca gca gca gag gca ggt tcc ctc ggg ttc    211
Thr Pro Ala Leu Val Asn Ala Ala Ala Glu Ala Gly Ser Leu Gly Phe
            25                  30                  35

ttg gct ggt ggc gtc atg cct ctt gag cag ctg aaa cag gaa ttg tca    259
Leu Ala Gly Gly Val Met Pro Leu Glu Gln Leu Lys Gln Glu Leu Ser
        40                  45                  50

gag gta aaa ggc gtc ttt ggc gtc aac ctg ttt cgc ccg cag acg gat    307
Glu Val Lys Gly Val Phe Gly Val Asn Leu Phe Arg Pro Gln Thr Asp
    55                  60                  65

gcg cct aag cct tca gac att gat gag ctg gcg gga ttg ttg tcc tcg    355
Ala Pro Lys Pro Ser Asp Ile Asp Glu Leu Ala Gly Leu Leu Ser Ser
70                  75                  80                  85

gcg ttt cgg caa ttt ggc ctc gat gag ccg acg gtg cct acg ccg gat    403
Ala Phe Arg Gln Phe Gly Leu Asp Glu Pro Thr Val Pro Thr Pro Asp
                90                  95                  100

ttg agc aat ggg tgg gag gct aaa ttt gag gcc gtt ctt gcc gct aag    451
Leu Ser Asn Gly Trp Glu Ala Lys Phe Glu Ala Val Leu Ala Ala Lys
            105                 110                 115

ccc gcc gtt ttt tcc tgc acc ttt ggt att ttt agc gct gaa gaa ttc    499
Pro Ala Val Phe Ser Cys Thr Phe Gly Ile Phe Ser Ala Glu Glu Phe
        120                 125                 130

gcc cgg atc aaa gcc acc gga att gag gcg tgg gtg acg gtg acc aat    547
Ala Arg Ile Lys Ala Thr Gly Ile Glu Ala Trp Val Thr Val Thr Asn
    135                 140                 145

ccg gag gac gcg ctg gct gcg cag aaa gct ggc gcc aac gcg ctt gtc     595
Pro Glu Asp Ala Leu Ala Ala Gln Lys Ala Gly Ala Asn Ala Leu Val
150                 155                 160                 165

gtg caa ggc ccc gag gcg ggt ggg cac cgc tct acc tgg tcc att gaa     643
Val Gln Gly Pro Glu Ala Gly Gly His Arg Ser Thr Trp Ser Ile Glu
                170                 175                 180

gtg gag ccg gac gag cgc gac ctg aaa acc ctc ctc gca gct gtc aaa     691
Val Glu Pro Asp Glu Arg Asp Leu Lys Thr Leu Leu Ala Ala Val Lys
            185                 190                 195

caa gcg ggc gtt tac ctc ccg ctc atc gca gcc ggc ggc ctt tca acc     739
Gln Ala Gly Val Tyr Leu Pro Leu Ile Ala Ala Gly Gly Leu Ser Thr
        200                 205                 210

tcc gca gac gtg gca gca att tta gaa gcc ggc gcc agc gct gcc tcc     787
Ser Ala Asp Val Ala Ala Ile Leu Glu Ala Gly Ala Ser Ala Ala Ser
    215                 220                 225

tgt ggt tcc gcc ttt ttg ctt agc gac gaa gcc ggc acc agc tca ctt     835
Cys Gly Ser Ala Phe Leu Leu Ser Asp Glu Ala Gly Thr Ser Ser Leu
230                 235                 240                 245

aac cgc gag atc ttg gac gcc gcc cca gca ctt ggt ttg gaa tcg gtg     883
Asn Arg Glu Ile Leu Asp Ala Ala Pro Ala Leu Gly Leu Glu Ser Val
                250                 255                 260

tca tct cgc gca ttt tcg ggc cgt tat gcc agg gga gtg gaa acc agg     931
Ser Ser Arg Ala Phe Ser Gly Arg Tyr Ala Arg Gly Val Glu Thr Arg
            265                 270                 275

ttc acc cgt tcg aac gag ggg tta ccc ccg ttg tac cca tac ctc aac     979
Phe Thr Arg Ser Asn Glu Gly Leu Pro Pro Leu Tyr Pro Tyr Leu Asn
        280                 285                 290

cca atg atc aca tct tta cgt aag gtg gcg gga agt gca ggg aac tgg     1027
Pro Met Ile Thr Ser Leu Arg Lys Val Ala Gly Ser Ala Gly Asn Trp
    295                 300                 305

gat tac gcc tac tgc ctg gta gga gtc ggc ctg gaa tcg att gcg aag     1075
Asp Tyr Ala Tyr Cys Leu Val Gly Val Gly Leu Glu Ser Ile Ala Lys
310                 315                 320                 325

ggt agt gca aag cag ata ctg gaa tca tta aca cct tcc gct ttg ggc     1123
Gly Ser Ala Lys Gln Ile Leu Glu Ser Leu Thr Pro Ser Ala Leu Gly
                330                 335                 340

taatgttggg gggagtgctt tca                                           1146


<210> 244
<211> 341
<212> PRT
<213> Corynebacterium glutamicum

<400> 244
Met Ser Ile Leu Asp Thr Leu Lys Thr Pro Val Ile Val Ala Pro Met
  1               5                  10                  15

Ala Gly Gly Pro Ser Thr Pro Ala Leu Val Asn Ala Ala Ala Glu Ala
             20                  25                  30

Gly Ser Leu Gly Phe Leu Ala Gly Gly Val Met Pro Leu Glu Gln Leu
         35                  40                  45

Lys Gln Glu Leu Ser Glu Val Lys Gly Val Phe Gly Val Asn Leu Phe
     50                  55                  60

Arg Pro Gln Thr Asp Ala Pro Lys Pro Ser Asp Ile Asp Glu Leu Ala
 65                  70                  75                  80

Gly Leu Leu Ser Ser Ala Phe Arg Gln Phe Gly Leu Asp Glu Pro Thr
                 85                  90                  95

Val Pro Thr Pro Asp Leu Ser Asn Gly Trp Glu Ala Lys Phe Glu Ala

100                 105                 110

Val Leu Ala Ala Lys Pro Ala Val Phe Ser Cys Thr Phe Gly Ile Phe
        115                 120                 125

Ser Ala Glu Glu Phe Ala Arg Ile Lys Ala Thr Gly Ile Glu Ala Trp
    130                 135                 140

Val Thr Val Thr Asn Pro Glu Asp Ala Leu Ala Ala Gln Lys Ala Gly
145                 150                 155                 160

Ala Asn Ala Leu Val Val Gln Gly Pro Glu Ala Gly Gly His Arg Ser
                165                 170                 175

Thr Trp Ser Ile Glu Val Glu Pro Asp Glu Arg Asp Leu Lys Thr Leu
            180                 185                 190

Leu Ala Ala Val Lys Gln Ala Gly Val Tyr Leu Pro Leu Ile Ala Ala
        195                 200                 205

Gly Gly Leu Ser Thr Ser Ala Asp Val Ala Ala Ile Leu Glu Ala Gly
    210                 215                 220

Ala Ser Ala Ala Ser Cys Gly Ser Ala Phe Leu Leu Ser Asp Glu Ala
225                 230                 235                 240

Gly Thr Ser Ser Leu Asn Arg Glu Ile Leu Asp Ala Ala Pro Ala Leu
                245                 250                 255

Gly Leu Glu Ser Val Ser Ser Arg Ala Phe Ser Gly Arg Tyr Ala Arg
            260                 265                 270

Gly Val Glu Thr Arg Phe Thr Arg Ser Asn Glu Gly Leu Pro Pro Leu
        275                 280                 285

Tyr Pro Tyr Leu Asn Pro Met Ile Thr Ser Leu Arg Lys Val Ala Gly
    290                 295                 300

Ser Ala Gly Asn Trp Asp Tyr Ala Tyr Cys Leu Val Gly Val Gly Leu
305                 310                 315                 320

Glu Ser Ile Ala Lys Gly Ser Ala Lys Gln Ile Leu Glu Ser Leu Thr
                325                 330                 335

Pro Ser Ala Leu Gly
            340


<210> 245
<211> 723
<212> DNA
<213> Corynebacterium glutamicum

<220>
<221> CDS
<222> (101)..(700)
<223> RXA01571

<400> 245
aaactacctg ctgagagctt tgtaatttac ggtgtggttg tggaggggtg cgtcgagaag 60

cgctcgtagg cgcttttgat tttcggtag gctaactggg gtg agt atc tca gta    115
                                            Val Ser Ile Ser Val
                                            1               5

aaa gca cta caa aag tcc ggc cca gaa gca cct ttc gag gtc aag atc    163
Lys Ala Leu Gln Lys Ser Gly Pro Glu Ala Pro Phe Glu Val Lys Ile
                10                  15                  20

att gaa cgc cgt gac cca cgc gca gat gat gtg gtt att gat atc aaa    211
Ile Glu Arg Arg Asp Pro Arg Ala Asp Asp Val Val Ile Asp Ile Lys
            25                  30                  35

gct gcg ggc atc tgc cac agc gat atc cac acc atc cgc aac gaa tgg    259
Ala Ala Gly Ile Cys His Ser Asp Ile His Thr Ile Arg Asn Glu Trp
        40                  45                  50

ggc gag gcg cac ttc ccg ctc acc gtc ggc cac gaa atc gca ggc gtt     307
Gly Glu Ala His Phe Pro Leu Thr Val Gly His Glu Ile Ala Gly Val
    55                  60                  65

gtc tct gcg gtt gga tcc gat gta acc aaa tgg aaa gtc ggc gac cgc     355
Val Ser Ala Val Gly Ser Asp Val Thr Lys Trp Lys Val Gly Asp Arg
 70                  75                  80                  85

gtg ggc gtc ggc tgc ctc gtt aac tcc tgc ggc gaa tgc gaa cag tgc     403
Val Gly Val Gly Cys Leu Val Asn Ser Cys Gly Glu Cys Glu Gln Cys
                 90                  95                 100

gtc gca gga ttt gaa aac aac tgc ctt cgc gga aac gtc gga acc tac     451
Val Ala Gly Phe Glu Asn Asn Cys Leu Arg Gly Asn Val Gly Thr Tyr
            105                 110                 115

aac tct aac gac gtc gac ggc acc atc acc caa ggc ggc tac gct gaa     499
Asn Ser Asn Asp Val Asp Gly Thr Ile Thr Gln Gly Gly Tyr Ala Glu
        120                 125                 130

aag gta gtg gtc aac gaa cgt ttc ctg tgc agc atc cca gag gaa ctt     547
Lys Val Val Val Asn Glu Arg Phe Leu Cys Ser Ile Pro Glu Glu Leu
    135                 140                 145

aac ttc gat gtc gca gca cca ctg ctg tgc gca ggc atc acc acc tac     595
Asn Phe Asp Val Ala Ala Pro Leu Leu Cys Ala Gly Ile Thr Thr Tyr
150                 155                 160                 165

tcc cca atc gct cgc tgg aac gtt aaa gaa ggc gac aaa gta gca gtc     643
Ser Pro Ile Ala Arg Trp Asn Val Lys Glu Gly Asp Lys Val Ala Val
                170                 175                 180

atg ggc ctc ggc ggg act cgg aca cat ggg tgt cca gat cgc tgc agc     691
Met Gly Leu Gly Gly Thr Arg Thr His Gly Cys Pro Asp Arg Cys Ser
            185                 190                 195

caa ggg tgc tgaggttacc gttctgtccc gtt                               723
Gln Gly Cys
        200


<210> 246
<211> 200
<212> PRT
<213> Corynebacterium glutamicum

<400> 246
Val Ser Ile Ser Val Lys Ala Leu Gln Lys Ser Gly Pro Glu Ala Pro
  1               5                  10                  15

Phe Glu Val Lys Ile Ile Glu Arg Arg Asp Pro Arg Ala Asp Asp Val
             20                  25                  30

Val Ile Asp Ile Lys Ala Ala Gly Ile Cys His Ser Asp Ile His Thr
         35                  40                  45

Ile Arg Asn Glu Trp Gly Glu Ala His Phe Pro Leu Thr Val Gly His
     50                  55                  60

Glu Ile Ala Gly Val Val Ser Ala Val Gly Ser Asp Val Thr Lys Trp
 65                  70                  75                  80

Lys Val Gly Asp Arg Val Gly Val Gly Cys Leu Val Asn Ser Cys Gly
                 85                  90                  95

Glu Cys Glu Gln Cys Val Ala Gly Phe Glu Asn Asn Cys Leu Arg Gly
            100                 105                 110

Asn Val Gly Thr Tyr Asn Ser Asn Asp Val Asp Gly Thr Ile Thr Gln
        115                 120                 125

Gly Gly Tyr Ala Glu Lys Val Val Val Asn Glu Arg Phe Leu Cys Ser
    130                 135                 140

Ile Pro Glu Glu Leu Asn Phe Asp Val Ala Ala Pro Leu Leu Cys Ala
145                 150                 155                 160

Gly Ile Thr Thr Tyr Ser Pro Ile Ala Arg Trp Asn Val Lys Glu Gly
                165                 170                 175

Asp Lys Val Ala Val Met Gly Leu Gly Gly Thr Arg Thr His Gly Cys
            180                 185                 190

Pro Asp Arg Cys Ser Gln Gly Cys
        195                 200


<210> 247
<211> 1338
<212> DNA
<213> Corynebacterium glutamicum

<220>
<221> CDS
<222> (101)..(1315)
<223> RXN02478

<400> 247
gacatcgtcg aagcgctctc cagcggcaac atcgacgatt atcgcagcgc cgtgctcgct 60

cactacgcgc cgtttcgccg catgatttcc aacatgctcg atg cgc act agc ctc    115
                                            Met Arg Thr Ser Leu
                                            1               5

att gcg cgc ggg ttg tac cgc att ccc gcg ctg gtc tgg gat cag ggt    163
Ile Ala Arg Gly Leu Tyr Arg Ile Pro Ala Leu Val Trp Asp Gln Gly
                10                  15                  20

ctt tta acg ctt ttc gac gcc cgc ctc agt gtt gac gac ctc ccc gca    211
Leu Leu Thr Leu Phe Asp Ala Arg Leu Ser Val Asp Asp Leu Pro Ala
            25                  30                  35

ccc atc gac gtg gtg tca gcg cga tcc tca gac ggc atc acc tgg acc    259
Pro Ile Asp Val Val Ser Ala Arg Ser Ser Asp Gly Ile Thr Trp Thr
        40                  45                  50

acc cca gaa cca gca atc gtc gaa act gaa cac cgc ggt gtg ggc gat    307
Thr Pro Glu Pro Ala Ile Val Glu Thr Glu His Arg Gly Val Gly Asp
    55                  60                  65

gtc tgc ctt gtc acg ggc gat ctg tgc ttc cac gga ttg tcc aac ctc    355
Val Cys Leu Val Thr Gly Asp Leu Cys Phe His Gly Leu Ser Asn Leu
70                  75                  80                  85

gca gga ttt ttt gag gat ccc acc gac ctt gaa ccc cgg ctg gcg cgc    403
Ala Gly Phe Phe Glu Asp Pro Thr Asp Leu Glu Pro Arg Leu Ala Arg
                90                  95                  100

cgg gat gtg agt ggg tgg acg tcg ata agc atg gcc cac tat ttt gcg    451
Arg Asp Val Ser Gly Trp Thr Ser Ile Ser Met Ala His Tyr Phe Ala
            105                 110                 115

gat gtt gat gcc gcg ttc gcc tcg tcg ggg acg gga ctt gtc ctg gcg    499
Asp Val Asp Ala Ala Phe Ala Ser Ser Gly Thr Gly Leu Val Leu Ala
        120                 125                 130

gat ggg cgg tgg att cag agt ttt gtg gtg cgg cgc ggg cgc gag att    547
Asp Gly Arg Trp Ile Gln Ser Phe Val Val Arg Arg Gly Arg Glu Ile
    135                 140                 145

tcg ctt cgg att ctg cgc agc gat ggc cac atc acc gat att gcc ggc    595
Ser Leu Arg Ile Leu Arg Ser Asp Gly His Ile Thr Asp Ile Ala Gly
150                 155                 160                 165

ggt aac gaa tcc gcg atg acg cag ctg ccg agc ggt cgg att gtg ctg    643
Gly Asn Glu Ser Ala Met Thr Gln Leu Pro Ser Gly Arg Ile Val Leu
                170                 175                 180

cat tcc agg ggg gtg gga cac cgt ctg agc agt gtg tcc gat gat ttc    691
His Ser Arg Gly Val Gly His Arg Leu Ser Ser Val Ser Asp Asp Phe
            185                 190                 195

ggg gag aca ttc act ccg ctg gag cct gtg cct gaa cta atc gac ccc    739
Gly Glu Thr Phe Thr Pro Leu Glu Pro Val Pro Glu Leu Ile Asp Pro

200                 205                 210

ggc tgc aac ggc cac gtg ttc tac tgg aaa gcg gct gga atg ctc gcc     787
Gly Cys Asn Gly His Val Phe Tyr Trp Lys Ala Ala Gly Met Leu Ala
    215                 220                 225

gca acg cac ctg gcg gac cct gat ctg cga cgc cac ttg gtg gtt gat     835
Ala Thr His Leu Ala Asp Pro Asp Leu Arg Arg His Leu Val Val Asp
230                 235                 240                 245

tta tcc agc gac gaa gga gcg acc tgg gcg cat cgc atc acc atc gag     883
Leu Ser Ser Asp Glu Gly Ala Thr Trp Ala His Arg Ile Thr Ile Glu
                250                 255                 260

cgc gaa gaa gcc gcc tat tca acc gct gcg gaa atg ccc aac gga gat     931
Arg Glu Glu Ala Ala Tyr Ser Thr Ala Ala Glu Met Pro Asn Gly Asp
            265                 270                 275

gtt gcc gtg gtg tgg gaa gca gag gga acg cgc gcg ata aaa tgc acg     979
Val Ala Val Val Trp Glu Ala Glu Gly Thr Arg Ala Ile Lys Cys Thr
        280                 285                 290

gtg atc agc gta aat gat att tcg ctg cgg atc gat gag ccc att tcc     1027
Val Ile Ser Val Asn Asp Ile Ser Leu Arg Ile Asp Glu Pro Ile Ser
    295                 300                 305

gat gcc ata tcc ctc cgc cat gtg gtg atc aac gat gac cat gac ggc     1075
Asp Ala Ile Ser Leu Arg His Val Val Ile Asn Asp Asp His Asp Gly
310                 315                 320                 325

atc gaa gtc gca ctg cct gac gca tcg caa tgg ggt gaa ggt gta ttc     1123
Ile Glu Val Ala Leu Pro Asp Ala Ser Gln Trp Gly Glu Gly Val Phe
                330                 335                 340

aaa att gtg tcc aat cca gac gcg agc acc caa aaa atc cgc act cga     1171
Lys Ile Val Ser Asn Pro Asp Ala Ser Thr Gln Lys Ile Arg Thr Arg
            345                 350                 355

ggc aag ccc gcg cga cag acc ctg gaa att ggg gat gaa ttg gtt ttt     1219
Gly Lys Pro Ala Arg Gln Thr Leu Glu Ile Gly Asp Glu Leu Val Phe
        360                 365                 370

gat atc cgc aag ggt gga gaa gtg gct tac ggc gtc acg gtt cct tat     1267
Asp Ile Arg Lys Gly Gly Glu Val Ala Tyr Gly Val Thr Val Pro Tyr
    375                 380                 385

gat ggt cgc tcg ttg ggg gaa gtt aaa cag gat ttt gga gtg ggg ctg     1315
Asp Gly Arg Ser Leu Gly Glu Val Lys Gln Asp Phe Gly Val Gly Leu
390                 395                 400                 405

tagaggccga tttgcggtcc ttt                                           1338


<210> 248
<211> 405
<212> PRT
<213> Corynebacterium glutamicum

<400> 248
Met Arg Thr Ser Leu Ile Ala Arg Gly Leu Tyr Arg Ile Pro Ala Leu
1               5                   10                  15

Val Trp Asp Gln Gly Leu Leu Thr Leu Phe Asp Ala Arg Leu Ser Val
            20                  25                  30

Asp Asp Leu Pro Ala Pro Ile Asp Val Val Ser Ala Arg Ser Ser Asp
        35                  40                  45

Gly Ile Thr Trp Thr Thr Pro Glu Pro Ala Ile Val Glu Thr Glu His
    50                  55                  60

Arg Gly Val Gly Asp Val Cys Leu Val Thr Gly Asp Leu Cys Phe His
65                  70                  75                  80

Gly Leu Ser Asn Leu Ala Gly Phe Phe Glu Asp Pro Thr Asp Leu Glu
                85                  90                  95

Pro Arg Leu Ala Arg Arg Asp Val Ser Gly Trp Thr Ser Ile Ser Met
            100                 105                 110

Ala His Tyr Phe Ala Asp Val Asp Ala Ala Phe Ala Ser Ser Gly Thr
        115                 120                 125

Gly Leu Val Leu Ala Asp Gly Arg Trp Ile Gln Ser Phe Val Val Arg
    130                 135                 140

Arg Gly Arg Glu Ile Ser Leu Arg Ile Leu Arg Ser Asp Gly His Ile
145                 150                 155                 160

Thr Asp Ile Ala Gly Gly Asn Glu Ser Ala Met Thr Gln Leu Pro Ser
                165                 170                 175

Gly Arg Ile Val Leu His Ser Arg Gly Val Gly His Arg Leu Ser Ser
            180                 185                 190

Val Ser Asp Asp Phe Gly Glu Thr Phe Thr Pro Leu Glu Pro Val Pro
        195                 200                 205

Glu Leu Ile Asp Pro Gly Cys Asn Gly His Val Phe Tyr Trp Lys Ala
    210                 215                 220

Ala Gly Met Leu Ala Ala Thr His Leu Ala Asp Pro Asp Leu Arg Arg
225                 230                 235                 240

His Leu Val Val Asp Leu Ser Ser Asp Glu Gly Ala Thr Trp Ala His
                245                 250                 255

Arg Ile Thr Ile Glu Arg Glu Glu Ala Ala Tyr Ser Thr Ala Ala Glu
            260                 265                 270

Met Pro Asn Gly Asp Val Ala Val Val Trp Glu Ala Glu Gly Thr Arg
        275                 280                 285

Ala Ile Lys Cys Thr Val Ile Ser Val Asn Asp Ile Ser Leu Arg Ile
    290                 295                 300

Asp Glu Pro Ile Ser Asp Ala Ile Ser Leu Arg His Val Val Ile Asn
305                 310                 315                 320

Asp Asp His Asp Gly Ile Glu Val Ala Leu Pro Asp Ala Ser Gln Trp
                325                 330                 335

Gly Glu Gly Val Phe Lys Ile Val Ser Asn Pro Asp Ala Ser Thr Gln
            340                 345                 350

Lys Ile Arg Thr Arg Gly Lys Pro Ala Arg Gln Thr Leu Glu Ile Gly
        355                 360                 365

Asp Glu Leu Val Phe Asp Ile Arg Lys Gly Gly Glu Val Ala Tyr Gly
    370                 375                 380

Val Thr Val Pro Tyr Asp Gly Arg Ser Leu Gly Glu Val Lys Gln Asp
385                 390                 395                 400

Phe Gly Val Gly Leu
                405


<210> 249
<211> 1213
<212> DNA
<213> Corynebacterium glutamicum

<220>
<221> CDS
<222> (101)..(1213)
<223> RXN00343

<400> 249
ttcggtagaa tgggtaggtt gtcgtgcttg aggtgtggtg gataaccacc tctacaacac 60

cacccaagct ctgttagaaa aaattgagga agcagtctaa atg aaa cac caa tat   115
                                            Met Lys His Gln Tyr
                                            1               5

gat gtc atc gtt gtc ggt tcc ggc gct ggc gga tta tca gct gca gtc 163
Asp Val Ile Val Val Gly Ser Gly Ala Gly Gly Leu Ser Ala Ala Val
                10                  15                  20

agt gca gct tac ggc ggt aag aaa gtc gct gta att gaa aag gcc tca 211
Ser Ala Ala Tyr Gly Gly Lys Lys Val Ala Val Ile Glu Lys Ala Ser
            25                  30                  35

gta ctc ggt gga gcc acc acc tgg tcc ggc ggt tgg gct tgg act cct 259
Val Leu Gly Gly Ala Thr Thr Trp Ser Gly Gly Trp Ala Trp Thr Pro
        40                  45                  50

gga acc agc ctt gcg cgc aaa gac gga gta gtg gaa tcc aaa gaa gaa 307
Gly Thr Ser Leu Ala Arg Lys Asp Gly Val Val Glu Ser Lys Glu Glu
    55                  60                  65

ttc caa acc tac ctg caa gcg gta gtg ggg gag tac tac caa gaa gac 355
Phe Gln Thr Tyr Leu Gln Ala Val Val Gly Glu Tyr Tyr Gln Glu Asp
70                  75                  80                  85

aac atc tcc gcc ttc ttg gac gca gcc cct gaa atg gtc gat ttc ttt 403
Asn Ile Ser Ala Phe Leu Asp Ala Ala Pro Glu Met Val Asp Phe Phe
                90                  95                  100

gaa aaa aac acc gac ctg cag tgg acc ccc ggc gcg aaa atc aac gac 451
Glu Lys Asn Thr Asp Leu Gln Trp Thr Pro Gly Ala Lys Ile Asn Asp
            105                 110                 115

atc tac ggc aac ctc ccc ggt gct ggc act gga cac cgc tcc gtt ggg 499
Ile Tyr Gly Asn Leu Pro Gly Ala Gly Thr Gly His Arg Ser Val Gly
        120                 125                 130

cca aaa cca ttc aac gga cgc aaa gta ccc aag agt gtt ctt cca aaa 547
Pro Lys Pro Phe Asn Gly Arg Lys Val Pro Lys Ser Val Leu Pro Lys
    135                 140                 145

ctg cgc cac cag ctg tat gaa acc tcc ttc ctg gga atg ggc atc atg 595
Leu Arg His Gln Leu Tyr Glu Thr Ser Phe Leu Gly Met Gly Ile Met
150                 155                 160                 165

gct ggg cct gac ctg acg aaa ttc ctc tct gct tca cag ttc gat cca 643
Ala Gly Pro Asp Leu Thr Lys Phe Leu Ser Ala Ser Gln Phe Asp Pro
                170                 175                 180

cgt ggt tgg gta cat gcc gcc agg cgc gtc atc gtg cac atg tgg gac 691
Arg Gly Trp Val His Ala Ala Arg Arg Val Ile Val His Met Trp Asp
            185                 190                 195

atg gtc gtg cac aaa cgc aat atg cag atg gtc aac ggt gca gca ctc 739
Met Val Val His Lys Arg Asn Met Gln Met Val Asn Gly Ala Ala Leu
        200                 205                 210

acc gct cga ctg gct acc tct gca gac aag ctg ggc gtt gat ctc ctg 787
Thr Ala Arg Leu Ala Thr Ser Ala Asp Lys Leu Gly Val Asp Leu Leu
    215                 220                 225

gtc aat cac tcc gca gtg tcg ttg aat tac aaa aac gac cgc gtt acc 835
Val Asn His Ser Ala Val Ser Leu Asn Tyr Lys Asn Asp Arg Val Thr
230                 235                 240                 245

ggc gtg aaa gta caa acc cca cag ggc ttg gta gat ttc gaa gcc act 883
Gly Val Lys Val Gln Thr Pro Gln Gly Leu Val Asp Phe Glu Ala Thr
                250                 255                 260

gcc ggc gtc gtg ctc gcc act ggt gga ttc ccc aac aac gtt gac ctg 931
Ala Gly Val Val Leu Ala Thr Gly Gly Phe Pro Asn Asn Val Asp Leu
            265                 270                 275

cgc aag gaa ctc ttc cca cgc acc cca tca ggt caa gaa cac tgg acc 979
Arg Lys Glu Leu Phe Pro Arg Thr Pro Ser Gly Gln Glu His Trp Thr
        280                 285                 290

ctc gcg cca gca gaa acc acc ggc gac gga cta tcc atg gct cgg gaa 1027
Leu Ala Pro Ala Glu Thr Thr Gly Asp Gly Leu Ser Met Ala Arg Glu
    295                 300                 305

atc ggt gca ggt ttt gtc aac gac ctg aaa tcc cca gca gca tgg tgc     1075
Ile Gly Ala Gly Phe Val Asn Asp Leu Lys Ser Pro Ala Ala Trp Cys
310                 315                 320                 325

cct gtt tca ttg gtc cca tac ttc aac gga aaa gtc ggc acc ttc ccc     1123
Pro Val Ser Leu Val Pro Tyr Phe Asn Gly Lys Val Gly Thr Phe Pro
                330                 335                 340

cac atc atg gac cgc gca aaa cca ggc tcc atc ggt gtt gtc tcc aca     1171
His Ile Met Asp Arg Ala Lys Pro Gly Ser Ile Gly Val Val Ser Thr
            345                 350                 355

ggt aag cga ttc gtc aat gaa gcc aac ggc tac tac gac tac             1213
Gly Lys Arg Phe Val Asn Glu Ala Asn Gly Tyr Tyr Asp Tyr
        360                 365                 370

<210> 250
<211> 371
<212> PRT
<213> Corynebacterium glutamicum

<400> 250
Met Lys His Gln Tyr Asp Val Ile Val Val Gly Ser Gly Ala Gly Gly
  1               5                  10                  15

Leu Ser Ala Ala Val Ser Ala Ala Tyr Gly Gly Lys Lys Val Ala Val
             20                  25                  30

Ile Glu Lys Ala Ser Val Leu Gly Gly Ala Thr Thr Trp Ser Gly Gly
         35                  40                  45

Trp Ala Trp Thr Pro Gly Thr Ser Leu Ala Arg Lys Asp Gly Val Val
     50                  55                  60

Glu Ser Lys Glu Glu Phe Gln Thr Tyr Leu Gln Ala Val Val Gly Glu
 65                  70                  75                  80

Tyr Tyr Gln Glu Asp Asn Ile Ser Ala Phe Leu Asp Ala Ala Pro Glu
                 85                  90                  95

Met Val Asp Phe Phe Glu Lys Asn Thr Asp Leu Gln Trp Thr Pro Gly
            100                 105                 110

Ala Lys Ile Asn Asp Ile Tyr Gly Asn Leu Pro Gly Ala Gly Thr Gly
        115                 120                 125

His Arg Ser Val Gly Pro Lys Pro Phe Asn Gly Arg Lys Val Pro Lys
    130                 135                 140

Ser Val Leu Pro Lys Leu Arg His Gln Leu Tyr Glu Thr Ser Phe Leu
145                 150                 155                 160

Gly Met Gly Ile Met Ala Gly Pro Asp Leu Thr Lys Phe Leu Ser Ala
                165                 170                 175

Ser Gln Phe Asp Pro Arg Gly Trp Val His Ala Ala Arg Arg Val Ile
            180                 185                 190

Val His Met Trp Asp Met Val Val His Lys Arg Asn Met Gln Met Val
        195                 200                 205

Asn Gly Ala Ala Leu Thr Ala Arg Leu Ala Thr Ser Ala Asp Lys Leu
    210                 215                 220

Gly Val Asp Leu Leu Val Asn His Ser Ala Val Ser Leu Asn Tyr Lys
225                 230                 235                 240

Asn Asp Arg Val Thr Gly Val Lys Val Gln Thr Pro Gln Gly Leu Val
                245                 250                 255

Asp Phe Glu Ala Thr Ala Gly Val Val Leu Ala Thr Gly Gly Phe Pro
            260                 265                 270

Asn Asn Val Asp Leu Arg Lys Glu Leu Phe Pro Arg Thr Pro Ser Gly
        275                 280                 285

Gln Glu His Trp Thr Leu Ala Pro Ala Glu Thr Thr Gly Asp Gly Leu
    290                 295                 300

Ser Met Ala Arg Glu Ile Gly Ala Gly Phe Val Asn Asp Leu Lys Ser
305                 310                 315                 320

Pro Ala Ala Trp Cys Pro Val Ser Leu Val Pro Tyr Phe Asn Gly Lys
                325                 330                 335

Val Gly Thr Phe Pro His Ile Met Asp Arg Ala Lys Pro Gly Ser Ile
            340                 345                 350

Gly Val Val Ser Thr Gly Lys Arg Phe Val Asn Glu Ala Asn Gly Tyr
        355                 360                 365

Tyr Asp Tyr
    370


<210> 251
<211> 1083
<212> DNA
<213> Corynebacterium glutamicum

<220>
<221> CDS
<222> (101)..(1060)
<223> RXN01555

<400> 251
ctggttttgt gccgaggatc agccagcaag ttaatgatcc ttacatggcg ctgttgttgg 60

cgcggtagtc aatcatgggg gagtatccca ccgtatccgc gtg aac aag ggc gtg    115
                                           Val Asn Lys Gly Val
                                           1               5

gtg ctg gta gca ggt gga ttc tcc cag aat cca gaa ctg cgc atg aag    163
Val Leu Val Ala Gly Gly Phe Ser Gln Asn Pro Glu Leu Arg Met Lys
                10                  15                  20

tac atg cca gaa ccc acc cca cag ttc tcc cgc acc aac gaa agc gcc    211
Tyr Met Pro Glu Pro Thr Pro Gln Phe Ser Arg Thr Asn Glu Ser Ala
            25                  30                  35

acc ggc gac acc atg gcc ctt gct gcg aaa gtg gga gca cgc cta ggc    259
Thr Gly Asp Thr Met Ala Leu Ala Ala Lys Val Gly Ala Arg Leu Gly
        40                  45                  50

gac gac aac ggt gaa aac gca ctg tgg ttc cca tcg tcc atc ggc acc    307
Asp Asp Asn Gly Glu Asn Ala Leu Trp Phe Pro Ser Ser Ile Gly Thr
    55                  60                  65

cgc gcc gac gga tcc acc gcg gtg tac cca cac att tgg gac cgt ggc    355
Arg Ala Asp Gly Ser Thr Ala Val Tyr Pro His Ile Trp Asp Arg Gly
70                  75                  80                  85

cgc ctc gga gtc atc gca gtc aac gca gca ggc gag cgt ttc gtc gat    403
Arg Leu Gly Val Ile Ala Val Asn Ala Ala Gly Glu Arg Phe Val Asp
                90                  95                  100

gaa tcc gtc tcc tac cac cgc ttc gtg cgc gcc atg tac gaa tcc aac    451
Glu Ser Val Ser Tyr His Arg Phe Val Arg Ala Met Tyr Glu Ser Asn
            105                 110                 115

aaa acc acc ccg act gtt tca gcc tgg ctc att gtt gat tcc cac acc    499
Lys Thr Thr Pro Thr Val Ser Ala Trp Leu Ile Val Asp Ser His Thr
        120                 125                 130

ctg gca aaa tac ggc ctc ggc atg atc acc atg cca cac ctg cct aaa    547
Leu Ala Lys Tyr Gly Leu Gly Met Ile Thr Met Pro His Leu Pro Lys
    135                 140                 145

ctc gct ctg caa aaa tac atc gac tcc gga tac ctg cac gca gga tca    595
Leu Ala Leu Gln Lys Tyr Ile Asp Ser Gly Tyr Leu His Ala Gly Ser
150                 155                 160                 165

tcc ttg gat gaa ttg gca cgc agc att ggt gtg gac gct cgc ggc ctg    643

Ser Leu Asp Glu Leu Ala Arg Ser Ile Gly Val Asp Ala Arg Gly Leu
                170                 175                 180

gaa caa acc gtc aaa cgc tac aat acc ttc gct aaa acg ggt atc gac     691
Glu Gln Thr Val Lys Arg Tyr Asn Thr Phe Ala Lys Thr Gly Ile Asp
            185                 190                 195

gaa gac ttc cac aag ggc gaa ctc ctc ttc ggt caa gcc gcc ggc gat     739
Glu Asp Phe His Lys Gly Glu Leu Leu Phe Gly Gln Ala Ala Gly Asp
        200                 205                 210

cca gac aac aag cca aac ccc aac gtc gga cca atc aag aag gga ccg     787
Pro Asp Asn Lys Pro Asn Pro Asn Val Gly Pro Ile Lys Lys Gly Pro
    215                 220                 225

ttc tac gca atc gct gta gtc cca acc cct ctg gcc act gcc ttt ggc     835
Phe Tyr Ala Ile Ala Val Val Pro Thr Pro Leu Ala Thr Ala Phe Gly
230                 235                 240                 245

atc agc atc aac ccc aac gga cag gtt gtt agt gaa gat ggg gag ccc     883
Ile Ser Ile Asn Pro Asn Gly Gln Val Val Ser Glu Asp Gly Glu Pro
                250                 255                 260

atc att gga ctg tac tcc gca gga aat gat gcc caa tct gtc atg gct     931
Ile Ile Gly Leu Tyr Ser Ala Gly Asn Asp Ala Gln Ser Val Met Ala
            265                 270                 275

tct gaa tat cct ggt gct ggt tca cag gtt ggt tcc gga atg acc ttt     979
Ser Glu Tyr Pro Gly Ala Gly Ser Gln Val Gly Ser Gly Met Thr Phe
        280                 285                 290

ggt tgg atc gca gca cag cac gcg gtg ggg aaa gcg gga aaa tcc gga     1027
Gly Trp Ile Ala Ala Gln His Ala Val Gly Lys Ala Gly Lys Ser Gly
    295                 300                 305

gga gct aag gca gga tat gcc gcg tct tct aag taattgcttg gtgggttgct 1080
Gly Ala Lys Ala Gly Tyr Ala Ala Ser Ser Lys
310                 315                 320

tac                                                                 1083


<210> 252
<211> 320
<212> PRT
<213> Corynebacterium glutamicum

<400> 252
Val Asn Lys Gly Val Val Leu Val Ala Gly Gly Phe Ser Gln Asn Pro
  1               5                  10                  15

Glu Leu Arg Met Lys Tyr Met Pro Glu Pro Thr Pro Gln Phe Ser Arg
             20                  25                  30

Thr Asn Glu Ser Ala Thr Gly Asp Thr Met Ala Leu Ala Ala Lys Val
         35                  40                  45

Gly Ala Arg Leu Gly Asp Asp Asn Gly Glu Asn Ala Leu Trp Phe Pro
     50                  55                  60

Ser Ser Ile Gly Thr Arg Ala Asp Gly Ser Thr Ala Val Tyr Pro His
 65                  70                  75                  80

Ile Trp Asp Arg Gly Arg Leu Gly Val Ile Ala Val Asn Ala Ala Gly
                 85                  90                  95

Glu Arg Phe Val Asp Glu Ser Val Ser Tyr His Arg Phe Val Arg Ala
            100                 105                 110

Met Tyr Glu Ser Asn Lys Thr Thr Pro Thr Val Ser Ala Trp Leu Ile
        115                 120                 125

Val Asp Ser His Thr Leu Ala Lys Tyr Gly Leu Gly Met Ile Thr Met
    130                 135                 140

Pro His Leu Pro Lys Leu Ala Leu Gln Lys Tyr Ile Asp Ser Gly Tyr
145                 150                 155                 160

Leu His Ala Gly Ser Ser Leu Asp Glu Leu Ala Arg Ser Ile Gly Val
                165                 170                 175

Asp Ala Arg Gly Leu Glu Gln Thr Val Lys Arg Tyr Asn Thr Phe Ala
            180                 185                 190

Lys Thr Gly Ile Asp Glu Asp Phe His Lys Gly Glu Leu Leu Phe Gly
        195                 200                 205

Gln Ala Ala Gly Asp Pro Asp Asn Lys Pro Asn Pro Asn Val Gly Pro
    210                 215                 220

Ile Lys Lys Gly Pro Phe Tyr Ala Ile Ala Val Val Pro Thr Pro Leu
225                 230                 235                 240

Ala Thr Ala Phe Gly Ile Ser Ile Asn Pro Asn Gly Gln Val Val Ser
                245                 250                 255

Glu Asp Gly Glu Pro Ile Ile Gly Leu Tyr Ser Ala Gly Asn Asp Ala
            260                 265                 270

Gln Ser Val Met Ala Ser Glu Tyr Pro Gly Ala Gly Ser Gln Val Gly
        275                 280                 285

Ser Gly Met Thr Phe Gly Trp Ile Ala Ala Gln His Ala Val Gly Lys
    290                 295                 300

Ala Gly Lys Ser Gly Gly Ala Lys Ala Gly Tyr Ala Ala Ser Ser Lys
305                 310                 315                 320

<210> 253
<211> 1428
<212> DNA
<213> Corynebacterium glutamicum

<220>
<221> CDS
<222> (101)..(1405)
<223> RXN01166

<400> 253
accgtaccca cagacacacc agaattaaca gaaacagact gaaaaacaac atcgctcgac 60

atgcgcgtaa tcctaacccg cgcacactaa tgtggccgat atg ggc tac acc aac    115
                                            Met Gly Tyr Thr Asn
                                            1               5

ctc aac gac aca cgg gtc ttg cgc gcc ggg tca tgt gat gcc tgg tgg    163
Leu Asn Asp Thr Arg Val Leu Arg Ala Gly Ser Cys Asp Ala Trp Trp
                10                  15                  20

cgc acg atg tct ccg cta gtg cag cag gga agt gag gca gtc ttt cgg    211
Arg Thr Met Ser Pro Leu Val Gln Gln Gly Ser Glu Ala Val Phe Arg
            25                  30                  35

cgc atc atg ggt ctc tcg cgg cgt cct gat cgg aaa cct ggc ttt gac    259
Arg Ile Met Gly Leu Ser Arg Arg Pro Asp Arg Lys Pro Gly Phe Asp
        40                  45                  50

gat gtc cca cat ttc ggc gca gct gtt cga gtt ccc ggt cta aaa cac    307
Asp Val Pro His Phe Gly Ala Ala Val Arg Val Pro Gly Leu Lys His
    55                  60                  65

ggc acg ttg gtc aat gct gca ccc ttg aaa gtt ttg ggc gca cgg ggc    355
Gly Thr Leu Val Asn Ala Ala Pro Leu Lys Val Leu Gly Ala Arg Gly
70                  75                  80                  85

gag ccc aac ccc gcg agt tcg tac cgt ttt gaa tac atc acc ggt gat    403
Glu Pro Asn Pro Ala Ser Ser Tyr Arg Phe Glu Tyr Ile Thr Gly Asp
                90                  95                  100

tcc gca ggt cga gcc atc act gcg acc ggc gct gtc ctc ttt tcc aca    451

Ser Ala Gly Arg Ala Ile Thr Ala Thr Gly Ala Val Leu Phe Ser Thr
            105                 110                 115

cgc ccc tgg aca acc ggc ccg cgt ccc gcg atc gcc atg gct cca tcc     499
Arg Pro Trp Thr Thr Gly Pro Arg Pro Ala Ile Ala Met Ala Pro Ser
        120                 125                 130

acc caa ggc gtc gca cag cac tgc gat ccc tcc cac acc tgc gcc atc     547
Thr Gln Gly Val Ala Gln His Cys Asp Pro Ser His Thr Cys Ala Ile
    135                 140                 145

gga ctc aac gca ttc tat gac aaa ccc ttc gac gca atc att gct tac     595
Gly Leu Asn Ala Phe Tyr Asp Lys Pro Phe Asp Ala Ile Ile Ala Tyr
150                 155                 160                 165

gaa ctc ccc gtc atc ctc tgg ttt cta gct cac gga ctt gac gtt gtg     643
Glu Leu Pro Val Ile Leu Trp Phe Leu Ala His Gly Leu Asp Val Val
                170                 175                 180

ttc atc gat tac ccc cgc gac ccc gca acc ggc gtc caa tac tat tgc     691
Phe Ile Asp Tyr Pro Arg Asp Pro Ala Thr Gly Val Gln Tyr Tyr Cys
            185                 190                 195

gat tcc atc gct gca gct aaa tcg ctt ctc gac gcc gtc ctc gcc tcc     739
Asp Ser Ile Ala Ala Ala Lys Ser Leu Leu Asp Ala Val Leu Ala Ser
        200                 205                 210

aga caa ctc ggc ctt tca ccg gaa gca ccg ctt ggc ctg tgg gga ttc     787
Arg Gln Leu Gly Leu Ser Pro Glu Ala Pro Leu Gly Leu Trp Gly Phe
    215                 220                 225

tcc caa gga ggc ggc gcc act ggc tgg gct gca caa ttg cag gat tac     835
Ser Gln Gly Gly Gly Ala Thr Gly Trp Ala Ala Gln Leu Gln Asp Tyr
230                 235                 240                 245

gca cct gat gtc cgc cca aag gca gcg gtc gtg ggc gct cca cca gtg     883
Ala Pro Asp Val Arg Pro Lys Ala Ala Val Val Gly Ala Pro Pro Val
                250                 255                 260

gat ctc ttc cgc gtc ttg gac act gtc gac ggc gga ttg ctc acc gga     931
Asp Leu Phe Arg Val Leu Asp Thr Val Asp Gly Gly Leu Leu Thr Gly
            265                 270                 275

gtg att gcc tac gcc atc gcg gga ctt gca gtg aac tct tca gag atg     979
Val Ile Ala Tyr Ala Ile Ala Gly Leu Ala Val Asn Ser Ser Glu Met
        280                 285                 290

ttt gag gaa atc atg tcg gtg tta aat gaa cgc gga gtc agt gat gtg     1027
Phe Glu Glu Ile Met Ser Val Leu Asn Glu Arg Gly Val Ser Asp Val
    295                 300                 305

ctg aaa aat atc acc agc tgc gcg gga ggt tcc ttg ttg gcc agt ggc     1075
Leu Lys Asn Ile Thr Ser Cys Ala Gly Gly Ser Leu Leu Ala Ser Gly
310                 315                 320                 325

tac tcg tct tcc cgc ggg tgg aca cat cag ggc acg ccg ctg gca gac     1123
Tyr Ser Ser Ser Arg Gly Trp Thr His Gln Gly Thr Pro Leu Ala Asp
                330                 335                 340

att ctg gac gat ctg cca ctt gtt gtc gct gag ttt ggg aag caa aag     1171
Ile Leu Asp Asp Leu Pro Leu Val Val Ala Glu Phe Gly Lys Gln Lys
            345                 350                 355

ctg ggt cgt gtg gcg cca gaa atc cca gtg ctg ttg tgg ggc tct aaa     1219
Leu Gly Arg Val Ala Pro Glu Ile Pro Val Leu Leu Trp Gly Ser Lys
        360                 365                 370

aat gat gat gtc att ccc att gat ccc att agg gaa ttg cgt gat agc     1267
Asn Asp Asp Val Ile Pro Ile Asp Pro Ile Arg Glu Leu Arg Asp Ser
    375                 380                 385

tgg gcg gac aag ggt acg cca ttg acc tgg cat gaa tcc caa gcg ccg     1315
Trp Ala Asp Lys Gly Thr Pro Leu Thr Trp His Glu Ser Gln Ala Pro
390                 395                 400                 405

cgt gtg cca gga cgc aca ggt ctc aac cat ttc ggg ccc tat ttt aga     1363
Arg Val Pro Gly Arg Thr Gly Leu Asn His Phe Gly Pro Tyr Phe Arg

410                 415                 420

aac ctg gaa aag tac tcg gga tgg ctc ata gat cat ctt gtc          1405
Asn Leu Glu Lys Tyr Ser Gly Trp Leu Ile Asp His Leu Val
            425                 430                 435

tgagtgccgt tttaaaggct cgg                                        1428

<210> 254
<211> 435
<212> PRT
<213> Corynebacterium glutamicum

<400> 254
Met Gly Tyr Thr Asn Leu Asn Asp Thr Arg Val Leu Arg Ala Gly Ser
1               5                   10                  15

Cys Asp Ala Trp Trp Arg Thr Met Ser Pro Leu Val Gln Gln Gly Ser
            20                  25                  30

Glu Ala Val Phe Arg Arg Ile Met Gly Leu Ser Arg Arg Pro Asp Arg
        35                  40                  45

Lys Pro Gly Phe Asp Asp Val Pro His Phe Gly Ala Ala Val Arg Val
    50                  55                  60

Pro Gly Leu Lys His Gly Thr Leu Val Asn Ala Ala Pro Leu Lys Val
65                  70                  75                  80

Leu Gly Ala Arg Gly Glu Pro Asn Pro Ala Ser Ser Tyr Arg Phe Glu
                85                  90                  95

Tyr Ile Thr Gly Asp Ser Ala Gly Arg Ala Ile Thr Ala Thr Gly Ala
            100                 105                 110

Val Leu Phe Ser Thr Arg Pro Trp Thr Thr Gly Pro Arg Pro Ala Ile
        115                 120                 125

Ala Met Ala Pro Ser Thr Gln Gly Val Ala Gln His Cys Asp Pro Ser
    130                 135                 140

His Thr Cys Ala Ile Gly Leu Asn Ala Phe Tyr Asp Lys Pro Phe Asp
145                 150                 155                 160

Ala Ile Ile Ala Tyr Glu Leu Pro Val Ile Leu Trp Phe Leu Ala His
                165                 170                 175

Gly Leu Asp Val Val Phe Ile Asp Tyr Pro Arg Asp Pro Ala Thr Gly
            180                 185                 190

Val Gln Tyr Tyr Cys Asp Ser Ile Ala Ala Ala Lys Ser Leu Leu Asp
        195                 200                 205

Ala Val Leu Ala Ser Arg Gln Leu Gly Leu Ser Pro Glu Ala Pro Leu
    210                 215                 220

Gly Leu Trp Gly Phe Ser Gln Gly Gly Gly Ala Thr Gly Trp Ala Ala
225                 230                 235                 240

Gln Leu Gln Asp Tyr Ala Pro Asp Val Arg Pro Lys Ala Ala Val Val
                245                 250                 255

Gly Ala Pro Pro Val Asp Leu Phe Arg Val Leu Asp Thr Val Asp Gly
            260                 265                 270

Gly Leu Leu Thr Gly Val Ile Ala Tyr Ala Ile Ala Gly Leu Ala Val
        275                 280                 285

Asn Ser Ser Glu Met Phe Glu Glu Ile Met Ser Val Leu Asn Glu Arg
    290                 295                 300

Gly Val Ser Asp Val Leu Lys Asn Ile Thr Ser Cys Ala Gly Gly Ser
305                 310                 315                 320

Leu Leu Ala Ser Gly Tyr Ser Ser Ser Arg Gly Trp Thr His Gln Gly
                325                 330                 335

Thr Pro Leu Ala Asp Ile Leu Asp Asp Leu Pro Leu Val Val Ala Glu
            340                 345                 350

Phe Gly Lys Gln Lys Leu Gly Arg Val Ala Pro Glu Ile Pro Val Leu
        355                 360                 365

Leu Trp Gly Ser Lys Asn Asp Asp Val Ile Pro Ile Asp Pro Ile Arg
    370                 375                 380

Glu Leu Arg Asp Ser Trp Ala Asp Lys Gly Thr Pro Leu Thr Trp His
385                 390                 395                 400

Glu Ser Gln Ala Pro Arg Val Pro Gly Arg Thr Gly Leu Asn His Phe
                405                 410                 415

Gly Pro Tyr Phe Arg Asn Leu Glu Lys Tyr Ser Gly Trp Leu Ile Asp
            420                 425                 430

His Leu Val
        435


<210> 255
<211> 1281
<212> DNA
<213> Corynebacterium glutamicum

<220>
<221> CDS
<222> (101)..(1258)
<223> RXN02001

<400> 255
gcggtttcgt catggataag gactgtgttc gggaccattg cgatactcgt gtcaaaaggc 60

gatagtccag catagaccgt gctttatcga aggtgaaccc atg ccc gtt atc aat    115
                                           Met Pro Val Ile Asn
                                           1               5

agt atc gcc agt ttt tcc gac gag atg acc cgc tgg cgg cgt cac ctg    163
Ser Ile Ala Ser Phe Ser Asp Glu Met Thr Arg Trp Arg Arg His Leu
                10                  15                  20

cat caa aac ccc gaa atc agc ttt gat tgt gtg gaa act gcg gcc ttc    211
His Gln Asn Pro Glu Ile Ser Phe Asp Cys Val Glu Thr Ala Ala Phe
            25                  30                  35

gtg gcc gag cag ctg cgc agc ttc ggg gtg gat gaa att cac acc ggc    259
Val Ala Glu Gln Leu Arg Ser Phe Gly Val Asp Glu Ile His Thr Gly
        40                  45                  50

atc gcg aaa acc ggt atc atc gcc ctg att cac ggg cgc gag gct ggc    307
Ile Ala Lys Thr Gly Ile Ile Ala Leu Ile His Gly Arg Glu Ala Gly
    55                  60                  65

ccc gtc gtc ggc ctg cgc gcc gat atg gac gcg ctg ccg ctg acc gag    355
Pro Val Val Gly Leu Arg Ala Asp Met Asp Ala Leu Pro Leu Thr Glu
70                  75                  80                  85

att acc ggc gtc gac tat gcc tcg acc acc ccc gga aaa atg cac gcc    403
Ile Thr Gly Val Asp Tyr Ala Ser Thr Thr Pro Gly Lys Met His Ala
                90                  95                  100

tgc ggc cac gac ggc cac acg acc atg ctg ctg ggc gcc gcc aaa tat    451
Cys Gly His Asp Gly His Thr Thr Met Leu Leu Gly Ala Ala Lys Tyr
            105                 110                 115

ctg gcc gag acg cgc aat ttc gca ggt acc gtc gcg ctg atc ttc cag    499
Leu Ala Glu Thr Arg Asn Phe Ala Gly Thr Val Ala Leu Ile Phe Gln
        120                 125                 130

cct gcg gaa gaa aac ggc ggc ggc gcg ggc gtt atg gtc gat gaa ggc    547
Pro Ala Glu Glu Asn Gly Gly Gly Ala Gly Val Met Val Asp Glu Gly
    135                 140                 145

gtc ctc gac cgc ttt gcc atc gcc gaa gtc tac gcc ctg cac aac cag    595

Val Leu Asp Arg Phe Ala Ile Ala Glu Val Tyr Ala Leu His Asn Gln
150                 155                 160                 165

ccc ggc ctg ccg ctt ggc cat ttt atg acg aca gcc ggc ccg atc atg     643
Pro Gly Leu Pro Leu Gly His Phe Met Thr Thr Ala Gly Pro Ile Met
                170                 175                 180

gcc gct gtc gac acg ttc gac atc aac att acc gga cgc ggc ggc cac     691
Ala Ala Val Asp Thr Phe Asp Ile Asn Ile Thr Gly Arg Gly Gly His
            185                 190                 195

ggt gcc aaa ccg cac caa acc cgc gac ccc atc gtc gca gcc gtc gga     739
Gly Ala Lys Pro His Gln Thr Arg Asp Pro Ile Val Ala Ala Val Gly
        200                 205                 210

att gtc caa gcg ttt caa acg ata gtc agc cgg aat cac aat ccg gtc     787
Ile Val Gln Ala Phe Gln Thr Ile Val Ser Arg Asn His Asn Pro Val
    215                 220                 225

gag gac ctt gtc gtg tcg gtc acg caa atc cac acc ggc agc gcc gat     835
Glu Asp Leu Val Val Ser Val Thr Gln Ile His Thr Gly Ser Ala Asp
230                 235                 240                 245

aat atc atc ccc gaa acc gcc tat atc aac ggc act gtc cgc acc ttc     883
Asn Ile Ile Pro Glu Thr Ala Tyr Ile Asn Gly Thr Val Arg Thr Phe
                250                 255                 260

aac aaa gac gtg cag gcc atg gtc atc acg cgg atg gaa gaa atc gtc     931
Asn Lys Asp Val Gln Ala Met Val Ile Thr Arg Met Glu Glu Ile Val
            265                 270                 275

gcg ggc caa gct gca gcc tat ggg gtc gag gcg acg ctg acc tac aac     979
Ala Gly Gln Ala Ala Ala Tyr Gly Val Glu Ala Thr Leu Thr Tyr Asn
        280                 285                 290

cgc aac tat ccc gcc acc att aac gac gcc gcc aaa gcc gcc atc gct     1027
Arg Asn Tyr Pro Ala Thr Ile Asn Asp Ala Ala Lys Ala Ala Ile Ala
    295                 300                 305

gcc gaa gtc gcg ggc gag gtc ggc ctc ggg gtc aac ccg aac ggc tcg     1075
Ala Glu Val Ala Gly Glu Val Gly Leu Gly Val Asn Pro Asn Gly Ser
310                 315                 320                 325

cgc ggg atg ggg gcc gag gat ttc tcg tat ttc ctc gaa aag cgc ccg     1123
Arg Gly Met Gly Ala Glu Asp Phe Ser Tyr Phe Leu Glu Lys Arg Pro
                330                 335                 340

ggt gcc tac ctg ttc gtc ggt aat ggc gac agc gcg ggc ctt cac aac     1171
Gly Ala Tyr Leu Phe Val Gly Asn Gly Asp Ser Ala Gly Leu His Asn
            345                 350                 355

ccc gcc tat aat ttc aac gac gag gcc gcg ccc tac ggc gca tcg ttc     1219
Pro Ala Tyr Asn Phe Asn Asp Glu Ala Ala Pro Tyr Gly Ala Ser Phe
        360                 365                 370

ttg gcc cgc atg gca gaa cgc ccc ttg ccg tta aag ggc tgatccatgg     1268
Leu Ala Arg Met Ala Glu Arg Pro Leu Pro Leu Lys Gly
    375                 380                 385

cgctcgaaga tgc                                                      1281


<210> 256
<211> 386
<212> PRT
<213> Corynebacterium glutamicum

<400> 256
Met Pro Val Ile Asn Ser Ile Ala Ser Phe Ser Asp Glu Met Thr Arg
1               5                   10                  15

Trp Arg Arg His Leu His Gln Asn Pro Glu Ile Ser Phe Asp Cys Val
            20                  25                  30

Glu Thr Ala Ala Phe Val Ala Glu Gln Leu Arg Ser Phe Gly Val Asp
        35                  40                  45

Glu Ile His Thr Gly Ile Ala Lys Thr Gly Ile Ile Ala Leu Ile His
    50                  55                  60

Gly Arg Glu Ala Gly Pro Val Val Gly Leu Arg Ala Asp Met Asp Ala
65                  70                  75                  80

Leu Pro Leu Thr Glu Ile Thr Gly Val Asp Tyr Ala Ser Thr Thr Pro
                85                  90                  95

Gly Lys Met His Ala Cys Gly His Asp Gly His Thr Thr Met Leu Leu
            100                 105                 110

Gly Ala Ala Lys Tyr Leu Ala Glu Thr Arg Asn Phe Ala Gly Thr Val
        115                 120                 125

Ala Leu Ile Phe Gln Pro Ala Glu Glu Asn Gly Gly Gly Ala Gly Val
    130                 135                 140

Met Val Asp Glu Gly Val Leu Asp Arg Phe Ala Ile Ala Glu Val Tyr
145                 150                 155                 160

Ala Leu His Asn Gln Pro Gly Leu Pro Leu Gly His Phe Met Thr Thr
                165                 170                 175

Ala Gly Pro Ile Met Ala Ala Val Asp Thr Phe Asp Ile Asn Ile Thr
            180                 185                 190

Gly Arg Gly Gly His Gly Ala Lys Pro His Gln Thr Arg Asp Pro Ile
        195                 200                 205

Val Ala Ala Val Gly Ile Val Gln Ala Phe Gln Thr Ile Val Ser Arg
    210                 215                 220

Asn His Asn Pro Val Glu Asp Leu Val Val Ser Val Thr Gln Ile His
225                 230                 235                 240

Thr Gly Ser Ala Asp Asn Ile Ile Pro Glu Thr Ala Tyr Ile Asn Gly
                245                 250                 255

Thr Val Arg Thr Phe Asn Lys Asp Val Gln Ala Met Val Ile Thr Arg
            260                 265                 270

Met Glu Glu Ile Val Ala Gly Gln Ala Ala Ala Tyr Gly Val Glu Ala
        275                 280                 285

Thr Leu Thr Tyr Asn Arg Asn Tyr Pro Ala Thr Ile Asn Asp Ala Ala
    290                 295                 300

Lys Ala Ala Ile Ala Ala Glu Val Ala Gly Glu Val Gly Leu Gly Val
305                 310                 315                 320

Asn Pro Asn Gly Ser Arg Gly Met Gly Ala Glu Asp Phe Ser Tyr Phe
                325                 330                 335

Leu Glu Lys Arg Pro Gly Ala Tyr Leu Phe Val Gly Asn Gly Asp Ser
            340                 345                 350

Ala Gly Leu His Asn Pro Ala Tyr Asn Phe Asn Asp Glu Ala Ala Pro
        355                 360                 365

Tyr Gly Ala Ser Phe Leu Ala Arg Met Ala Glu Arg Pro Leu Pro Leu
    370                 375                 380

Lys Gly
385


<210> 257
<211> 570
<212> DNA
<213> Corynebacterium glutamicum

<220>
<221> CDS
<222> (101)..(547)
<223> RXN03145

<400> 257
agaggtgttt aaattcccgc ctctgcccca gatttaaaac tatgatgagg gatagcttct 60

caattttcca ggttttccaa aatgaaagag gttcacgcac atg cct act tat act 115
Met Pro Thr Tyr Thr
1               5

tgt tgg tcg caa aga att cgc att tct agg gaa gcc aag caa cgc atc 163
Cys Trp Ser Gln Arg Ile Arg Ile Ser Arg Glu Ala Lys Gln Arg Ile
                10                  15                  20

gct gag gca atc acc gat gcc cac cat gaa tta gcg cat gct ccc aag 211
Ala Glu Ala Ile Thr Asp Ala His His Glu Leu Ala His Ala Pro Lys
            25                  30                  35

tat ttg gtg cag gtg att ttc aat gag gtg gag cct gat tct tat ttc 259
Tyr Leu Val Gln Val Ile Phe Asn Glu Val Glu Pro Asp Ser Tyr Phe
        40                  45                  50

att gcg gcg cag tcg gcg tcg gaa aac cac att tgg gtc caa gca acg 307
Ile Ala Ala Gln Ser Ala Ser Glu Asn His Ile Trp Val Gln Ala Thr
    55                  60                  65

att cgt tcg ggg cgt aca gag aag caa aaa gag gaa ctt ctg ctt cgg 355
Ile Arg Ser Gly Arg Thr Glu Lys Gln Lys Glu Glu Leu Leu Leu Arg
70                  75                  80                  85

ctg aca caa gag atc gcg ctg att ctt ggg atc ccc aat gaa gaa gta 403
Leu Thr Gln Glu Ile Ala Leu Ile Leu Gly Ile Pro Asn Glu Glu Val
                90                  95                  100

tgg gta tat ata ccg gag att cct ggt tcc aat atg acg gaa tat ggc 451
Trp Val Tyr Ile Pro Glu Ile Pro Gly Ser Asn Met Thr Glu Tyr Gly
            105                 110                 115

cgt ctc ctc atg gaa cct ggc gaa gag gag aag tgg ttt aat tcg ctt 499
Arg Leu Leu Met Glu Pro Gly Glu Glu Glu Lys Trp Phe Asn Ser Leu
        120                 125                 130

ccc gaa ggc ctg cgg gaa agg ttg acc gag cta gaa gga tcg tca gaa 547
Pro Glu Gly Leu Arg Glu Arg Leu Thr Glu Leu Glu Gly Ser Ser Glu
    135                 140                 145

tagctctcga ataggccatt tct 570


<210> 258
<211> 149
<212> PRT
<213> Corynebacterium glutamicum

<400> 258
Met Pro Thr Tyr Thr Cys Trp Ser Gln Arg Ile Arg Ile Ser Arg Glu
1               5                   10                  15

Ala Lys Gln Arg Ile Ala Glu Ala Ile Thr Asp Ala His His Glu Leu
            20                  25                  30

Ala His Ala Pro Lys Tyr Leu Val Gln Val Ile Phe Asn Glu Val Glu
        35                  40                  45

Pro Asp Ser Tyr Phe Ile Ala Ala Gln Ser Ala Ser Glu Asn His Ile
    50                  55                  60

Trp Val Gln Ala Thr Ile Arg Ser Gly Arg Thr Glu Lys Gln Lys Glu
65                  70                  75                  80

Glu Leu Leu Leu Arg Leu Thr Gln Glu Ile Ala Leu Ile Leu Gly Ile
                85                  90                  95

Pro Asn Glu Glu Val Trp Val Tyr Ile Pro Glu Ile Pro Gly Ser Asn
            100                 105                 110

Met Thr Glu Tyr Gly Arg Leu Leu Met Glu Pro Gly Glu Glu Glu Lys
        115                 120                 125

Trp Phe Asn Ser Leu Pro Glu Gly Leu Arg Glu Arg Leu Thr Glu Leu

130                 135                 140

Glu Gly Ser Ser Glu
145

<210> 259
<211> 1083
<212> DNA
<213> Corynebacterium glutamicum

<220>
<221> CDS
<222> (101)..(1060)
<223> RXN01466

<400> 259
aatccatgat cccaaactac ctcaaagcgc ttgtaggcta agacttatgg atacacaacg 60

cggctcattg cggggaaaag ctcataaagc aaggctaaag atg acg cca aat ggt    115
                                           Met Thr Pro Asn Gly
                                           1               5

cgc agg caa ctc ctc ctg gag cgt ggc gca gca ttt agc aaa aac cgt    163
Arg Arg Gln Leu Leu Leu Glu Arg Gly Ala Ala Phe Ser Lys Asn Arg
                10                  15                  20

acc ccg ggt cta aaa cac gtc gac cgc cac acc atc gtg gac tcc gac    211
Thr Pro Gly Leu Lys His Val Asp Arg His Thr Ile Val Asp Ser Asp
            25                  30                  35

ggc ctc agc atc cac acg tac atg gtt ggc cat gcc gaa aat gcc acg    259
Gly Leu Ser Ile His Thr Tyr Met Val Gly His Ala Glu Asn Ala Thr
        40                  45                  50

gca acg gtc gtg ttc atc cac ggc ttc acc ctc gcc gcc gaa gtg tat    307
Ala Thr Val Val Phe Ile His Gly Phe Thr Leu Ala Ala Glu Val Tyr
    55                  60                  65

tac atg cag gtc gac tac cta caa acc ttt tac cca aat att aaa agc    355
Tyr Met Gln Val Asp Tyr Leu Gln Thr Phe Tyr Pro Asn Ile Lys Ser
70                  75                  80                  85

gtg ctt atc gac gcc cgc ggc cac ggc gcc acc ggc cag atc cgc cca    403
Val Leu Ile Asp Ala Arg Gly His Gly Ala Thr Gly Gln Ile Arg Pro
                90                  95                  100

gag ctc tgc acc atc gaa gga aca gcg aac gat gtt ctc gca gcc atc    451
Glu Leu Cys Thr Ile Glu Gly Thr Ala Asn Asp Val Leu Ala Ala Ile
            105                 110                 115

cac gaa cac gca ccg acc ggc ccg ctc att ttg gtt ggg cat tcc ctc    499
His Glu His Ala Pro Thr Gly Pro Leu Ile Leu Val Gly His Ser Leu
        120                 125                 130

ggc gga ctc acg gca ctt aac ctg gtt aaa cgg gca gat cac tca ctt    547
Gly Gly Leu Thr Ala Leu Asn Leu Val Lys Arg Ala Asp His Ser Leu
    135                 140                 145

cgg aag agg atc gtc ggc atg gtt cta gtc gcc aca tcg atc gaa tca    595
Arg Lys Arg Ile Val Gly Met Val Leu Val Ala Thr Ser Ile Glu Ser
150                 155                 160                 165

tta tcc acc caa ggt cta cca caa gtc ctg gca tca ccc ctt gcc gac    643
Leu Ser Thr Gln Gly Leu Pro Gln Val Leu Ala Ser Pro Leu Ala Asp
                170                 175                 180

aac atc aaa aac gcc gtc gaa gca gcc ccc aac gat gcc caa aaa ttc    691
Asn Ile Lys Asn Ala Val Glu Ala Ala Pro Asn Asp Ala Gln Lys Phe
            185                 190                 195

cgc caa tac gcc acc aca ttt cta gcc ccc acc ctg gcc acc gca gtc    739
Arg Gln Tyr Ala Thr Thr Phe Leu Ala Pro Thr Leu Ala Thr Ala Val
        200                 205                 210

ttc caa cga gac aca aac gat gaa gtc atc gat ttc cac gcc gcc atg    787
Phe Gln Arg Asp Thr Asn Asp Glu Val Ile Asp Phe His Ala Ala Met

215                 220                 225

atc cac gaa acc ccc ttg gat acc ttc gtc ggt ttc ttc gac gac ctc     835
Ile His Glu Thr Pro Leu Asp Thr Phe Val Gly Phe Phe Asp Asp Leu
230                 235                 240                 245

caa gaa cac gac gaa ctc gat gcc gca cca gca ttg gaa ggc ctc aaa     883
Gln Glu His Asp Glu Leu Asp Ala Ala Pro Ala Leu Glu Gly Leu Lys
                250                 255                 260

ggc tac gtc ctt gcc ggc gaa tta gat gat gtc acc cca att agc caa     931
Gly Tyr Val Leu Ala Gly Glu Leu Asp Asp Val Thr Pro Ile Ser Gln
            265                 270                 275

gcc gac cgc atc tgc gaa gtc tgg ccc ggc gca cgc ctt caa atc gca     979
Ala Asp Arg Ile Cys Glu Val Trp Pro Gly Ala Arg Leu Gln Ile Ala
        280                 285                 290

gaa gga gca ggt cat atg ctt ccg ctt gaa gcg cca gga atc ctc aat    1027
Glu Gly Ala Gly His Met Leu Pro Leu Glu Ala Pro Gly Ile Leu Asn
    295                 300                 305

aat gcg atc ggc aac att ttg gac ggg ctg ggc tgaggaacct ggttcgggcg 1080
Asn Ala Ile Gly Asn Ile Leu Asp Gly Leu Gly
310                 315                 320

tgg                                                                1083

<210> 260
<211> 320
<212> PRT
<213> Corynebacterium glutamicum

<400> 260
Met Thr Pro Asn Gly Arg Arg Gln Leu Leu Leu Glu Arg Gly Ala Ala
  1               5                  10                  15

Phe Ser Lys Asn Arg Thr Pro Gly Leu Lys His Val Asp Arg His Thr
             20                  25                  30

Ile Val Asp Ser Asp Gly Leu Ser Ile His Thr Tyr Met Val Gly His
         35                  40                  45

Ala Glu Asn Ala Thr Ala Thr Val Val Phe Ile His Gly Phe Thr Leu
     50                  55                  60

Ala Ala Glu Val Tyr Tyr Met Gln Val Asp Tyr Leu Gln Thr Phe Tyr
 65                  70                  75                  80

Pro Asn Ile Lys Ser Val Leu Ile Asp Ala Arg Gly His Gly Ala Thr
                 85                  90                  95

Gly Gln Ile Arg Pro Glu Leu Cys Thr Ile Glu Gly Thr Ala Asn Asp
            100                 105                 110

Val Leu Ala Ala Ile His Glu His Ala Pro Thr Gly Pro Leu Ile Leu
        115                 120                 125

Val Gly His Ser Leu Gly Gly Leu Thr Ala Leu Asn Leu Val Lys Arg
    130                 135                 140

Ala Asp His Ser Leu Arg Lys Arg Ile Val Gly Met Val Leu Val Ala
145                 150                 155                 160

Thr Ser Ile Glu Ser Leu Ser Thr Gln Gly Leu Pro Gln Val Leu Ala
                165                 170                 175

Ser Pro Leu Ala Asp Asn Ile Lys Asn Ala Val Glu Ala Ala Pro Asn
            180                 185                 190

Asp Ala Gln Lys Phe Arg Gln Tyr Ala Thr Thr Phe Leu Ala Pro Thr
        195                 200                 205

Leu Ala Thr Ala Val Phe Gln Arg Asp Thr Asn Asp Glu Val Ile Asp
    210                 215                 220

Phe His Ala Ala Met Ile His Glu Thr Pro Leu Asp Thr Phe Val Gly
225                 230                 235                 240

Phe Phe Asp Asp Leu Gln Glu His Asp Glu Leu Asp Ala Ala Pro Ala
                245                 250                 255

Leu Glu Gly Leu Lys Gly Tyr Val Leu Ala Gly Glu Leu Asp Asp Val
            260                 265                 270

Thr Pro Ile Ser Gln Ala Asp Arg Ile Cys Glu Val Trp Pro Gly Ala
        275                 280                 285

Arg Leu Gln Ile Ala Glu Gly Ala Gly His Met Leu Pro Leu Glu Ala
    290                 295                 300

Pro Gly Ile Leu Asn Asn Ala Ile Gly Asn Ile Leu Asp Gly Leu Gly
305                 310                 315                 320

<210> 261
<211> 1137
<212> DNA
<213> Corynebacterium glutamicum

<220>
<221> CDS
<222> (101)..(1114)
<223> RXN01145

<400> 261
   taatgtagtt gtctgcccaa gcgagttaaa ctcccacgat ttacagtggg gggcagacat 60
   cttttcacca aaatttttac gaaaggcgag attttctccc atg gct att gaa ctg    115
                                               Met Ala Ile Glu Leu
                                               1               5
   ctt tat gat gct gac gct gac ctc tcc ttg atc cag ggc cgt aag gtt    163
   Leu Tyr Asp Ala Asp Ala Asp Leu Ser Leu Ile Gln Gly Arg Lys Val
                   10                  15                  20
   gcc atc gtt ggc tac ggc tcc cag ggc cac gca cac tcc cag aac ctc    211
   Ala Ile Val Gly Tyr Gly Ser Gln Gly His Ala His Ser Gln Asn Leu
               25                  30                  35
   cgc gat tct ggc gtt gag gtt gtc att ggt ctg cgc gag ggc tcc aag    259
   Arg Asp Ser Gly Val Glu Val Val Ile Gly Leu Arg Glu Gly Ser Lys
           40                  45                  50
   tcc gca gag aag gca aag gaa gca ggc ttc gag gtc aag acc acc gct    307
   Ser Ala Glu Lys Ala Lys Glu Ala Gly Phe Glu Val Lys Thr Thr Ala
       55                  60                  65
   gag gct gca gct tgg gct gac gtc atc atg ctc ctg gct cca gac acc    355
   Glu Ala Ala Ala Trp Ala Asp Val Ile Met Leu Leu Ala Pro Asp Thr
   70                  75                  80                  85
   tcc cag gca gaa atc ttc acc aac gac atc gag cca aac ctg aac gca    403
   Ser Gln Ala Glu Ile Phe Thr Asn Asp Ile Glu Pro Asn Leu Asn Ala
                   90                  95                  100
   ggc gac gca ctg ctg ttc ggc cac ggc ctg aac att cac ttc gac ctg    451
   Gly Asp Ala Leu Leu Phe Gly His Gly Leu Asn Ile His Phe Asp Leu
               105                 110                 115
   atc aag cca gct gac gac atc atc gtt ggc atg gtt gcg cca aag ggc    499
   Ile Lys Pro Ala Asp Asp Ile Ile Val Gly Met Val Ala Pro Lys Gly
           120                 125                 130
   cca ggc cac ttg gtt cgc cgt cag ttc gtt gat ggc aag ggt gtt cct    547
   Pro Gly His Leu Val Arg Arg Gln Phe Val Asp Gly Lys Gly Val Pro
       135                 140                 145
   tgc ctc atc gca gtc gac cag gac cca acc gga acc gca cag gct ctg    595
   Cys Leu Ile Ala Val Asp Gln Asp Pro Thr Gly Thr Ala Gln Ala Leu
   150                 155                 160                 165
   acc ctg tcc tac gca gca gca atc ggt ggc gca cgc gca ggc gtt atc     643
   Thr Leu Ser Tyr Ala Ala Ala Ile Gly Gly Ala Arg Ala Gly Val Ile
                   170                 175                 180
   cca acc acc ttc gaa gct gag acc gtc acc gac ctc ttc ggc gag cag     691
   Pro Thr Thr Phe Glu Ala Glu Thr Val Thr Asp Leu Phe Gly Glu Gln
               185                 190                 195
   gct gtt ctc tgc ggt ggc acc gag gaa ctg gtc aag gtt ggc ttc gag     739
   Ala Val Leu Cys Gly Gly Thr Glu Glu Leu Val Lys Val Gly Phe Glu
           200                 205                 210
   gtt ctc acc gaa gct ggc tac gag cca gag atg gca tac ttc gag gtt     787
   Val Leu Thr Glu Ala Gly Tyr Glu Pro Glu Met Ala Tyr Phe Glu Val
       215                 220                 225
   ctt cac gag ctc aag ctc atc gtt gac ctc atg ttc gaa ggt ggc atc     835
   Leu His Glu Leu Lys Leu Ile Val Asp Leu Met Phe Glu Gly Gly Ile
   230                 235                 240                 245
   agc aac atg aac tac tct gtt tct gac acc gct gag ttc ggt ggc tac     883
   Ser Asn Met Asn Tyr Ser Val Ser Asp Thr Ala Glu Phe Gly Gly Tyr
                   250                 255                 260
   ctc tcc ggc cca cgc gtc atc gat gca gac acc aag tcc cgc atg aag     931
   Leu Ser Gly Pro Arg Val Ile Asp Ala Asp Thr Lys Ser Arg Met Lys
               265                 270                 275
   gac atc ctg acc gat atc cag gac ggc acc ttc acc aag cgc ctc atc     979
   Asp Ile Leu Thr Asp Ile Gln Asp Gly Thr Phe Thr Lys Arg Leu Ile
           280                 285                 290
   gca aac gtt gag aac ggc aac acc gag ctt gag ggc ctt cgt gct tcc     1027
   Ala Asn Val Glu Asn Gly Asn Thr Glu Leu Glu Gly Leu Arg Ala Ser
       295                 300                 305
   tac aac aac cac cca atc gag gag acc ggc gct aag ctc cgc gac ctc     1075
   Tyr Asn Asn His Pro Ile Glu Glu Thr Gly Ala Lys Leu Arg Asp Leu
   310                 315                 320                 325
   atg agc tgg gtc aag gtt gac gct cgc gca gaa acc gct taagtttcac     1124
   Met Ser Trp Val Lys Val Asp Ala Arg Ala Glu Thr Ala
                   330                 335
   ccctttgacg gct                                                     1137

<210> 262
<211> 338
<212> PRT
<213> Corynebacterium glutamicum

<400> 262
Met Ala Ile Glu Leu Leu Tyr Asp Ala Asp Ala Asp Leu Ser Leu Ile
1               5                   10                  15

Gln Gly Arg Lys Val Ala Ile Val Gly Tyr Gly Ser Gln Gly His Ala
            20                  25                  30

His Ser Gln Asn Leu Arg Asp Ser Gly Val Glu Val Val Ile Gly Leu
        35                  40                  45

Arg Glu Gly Ser Lys Ser Ala Glu Lys Ala Lys Glu Ala Gly Phe Glu
    50                  55                  60

Val Lys Thr Thr Ala Glu Ala Ala Ala Trp Ala Asp Val Ile Met Leu
65                  70                  75                  80

Leu Ala Pro Asp Thr Ser Gln Ala Glu Ile Phe Thr Asn Asp Ile Glu
                85                  90                  95

Pro Asn Leu Asn Ala Gly Asp Ala Leu Leu Phe Gly His Gly Leu Asn
            100                 105                 110

Ile His Phe Asp Leu Ile Lys Pro Ala Asp Asp Ile Ile Val Gly Met

115                 120                 125

Val Ala Pro Lys Gly Pro Gly His Leu Val Arg Arg Gln Phe Val Asp
    130                 135                 140

Gly Lys Gly Val Pro Cys Leu Ile Ala Val Asp Gln Asp Pro Thr Gly
145                 150                 155                 160

Thr Ala Gln Ala Leu Thr Leu Ser Tyr Ala Ala Ala Ile Gly Gly Ala
                165                 170                 175

Arg Ala Gly Val Ile Pro Thr Thr Phe Glu Ala Glu Thr Val Thr Asp
            180                 185                 190

Leu Phe Gly Glu Gln Ala Val Leu Cys Gly Gly Thr Glu Glu Leu Val
        195                 200                 205

Lys Val Gly Phe Glu Val Leu Thr Glu Ala Gly Tyr Glu Pro Glu Met
    210                 215                 220

Ala Tyr Phe Glu Val Leu His Glu Leu Lys Leu Ile Val Asp Leu Met
225                 230                 235                 240

Phe Glu Gly Gly Ile Ser Asn Met Asn Tyr Ser Val Ser Asp Thr Ala
                245                 250                 255

Glu Phe Gly Gly Tyr Leu Ser Gly Pro Arg Val Ile Asp Ala Asp Thr
            260                 265                 270

Lys Ser Arg Met Lys Asp Ile Leu Thr Asp Ile Gln Asp Gly Thr Phe
        275                 280                 285

Thr Lys Arg Leu Ile Ala Asn Val Glu Asn Gly Asn Thr Glu Leu Glu
    290                 295                 300

Gly Leu Arg Ala Ser Tyr Asn Asn His Pro Ile Glu Glu Thr Gly Ala
305                 310                 315                 320

Lys Leu Arg Asp Leu Met Ser Trp Val Lys Val Asp Ala Arg Ala Glu
                325                 330                 335

Thr Ala

<210> 263
<211> 487
<212> DNA
<213> Corynebacterium glutamicum

<220>
<221> CDS
<222> (101)..(487)
<223> RXN03088

<400> 263
tcatggcacc gggacctggc atcgtagcta aggtttcttt tgatgatttc tccgacgtca 60

ccggcggcga tgaactcctc gaattggagg caaagaacta atg ggt caa acc cgc    115
                                           Met Gly Gln Thr Arg
                                           1               5

atc att tcc ggc gac gca cgc ggc cgc aag atc gaa gta cca cca gca    163
Ile Ile Ser Gly Asp Ala Arg Gly Arg Lys Ile Glu Val Pro Pro Ala
                10                  15                  20

ggt acc cgc ccc acc tct gac cgc gca cgc gaa ggt ctc ttc tcc tca    211
Gly Thr Arg Pro Thr Ser Asp Arg Ala Arg Glu Gly Leu Phe Ser Ser
            25                  30                  35

ctg cag gtc cgt ttc gga ttt gag ggc cag cgc gtc ctc gac att ttt    259
Leu Gln Val Arg Phe Gly Phe Glu Gly Gln Arg Val Leu Asp Ile Phe
        40                  45                  50

gcc ggc tcc ggc gca ctc gga ttg gaa gct gcc tcc agg ggt gcc gat    307
Ala Gly Ser Gly Ala Leu Gly Leu Glu Ala Ala Ser Arg Gly Ala Asp
    55                  60                  65

gag gta gtt ctg gtc gag tcg aat cct aag gcc gta gag gta att cga    355
Glu Val Val Leu Val Glu Ser Asn Pro Lys Ala Val Glu Val Ile Arg
 70                 75                  80                  85

cgg aat gtg gac gtc gta aag cat cct cgc gta acc gtc gca gag atg    403
Arg Asn Val Asp Val Val Lys His Pro Arg Val Thr Val Ala Glu Met
                90                  95                  100

aaa gca tcc acc tac ctt gcg tcc gca ccc gat aag ttt ttc acg atg    451
Lys Ala Ser Thr Tyr Leu Ala Ser Ala Pro Asp Lys Phe Phe Thr Met
            105                 110                 115

gtg ctc gcc gac ccg ccc tat gag ctt gcg acg acg                    487
Val Leu Ala Asp Pro Pro Tyr Glu Leu Ala Thr Thr
        120                 125


<210> 264
<211> 129
<212> PRT
<213> Corynebacterium glutamicum

<400> 264
Met Gly Gln Thr Arg Ile Ile Ser Gly Asp Ala Arg Gly Arg Lys Ile
  1               5                  10                  15

Glu Val Pro Pro Ala Gly Thr Arg Pro Thr Ser Asp Arg Ala Arg Glu
             20                  25                  30

Gly Leu Phe Ser Ser Leu Gln Val Arg Phe Gly Phe Glu Gly Gln Arg
         35                  40                  45

Val Leu Asp Ile Phe Ala Gly Ser Gly Ala Leu Gly Leu Glu Ala Ala
     50                  55                  60

Ser Arg Gly Ala Asp Glu Val Val Leu Val Glu Ser Asn Pro Lys Ala
 65                  70                  75                  80

Val Glu Val Ile Arg Arg Asn Val Asp Val Val Lys His Pro Arg Val
                 85                  90                  95

Thr Val Ala Glu Met Lys Ala Ser Thr Tyr Leu Ala Ser Ala Pro Asp
            100                 105                 110

Lys Phe Phe Thr Met Val Leu Ala Asp Pro Pro Tyr Glu Leu Ala Thr
        115                 120                 125

Thr



<210> 265
<211> 639
<212> DNA
<213> Corynebacterium glutamicum

<220>
<221> CDS
<222> (101)..(616)
<223> RXN02952

<400> 265
tccaaaccgc tgtttctgct cgtggcgaat cctggatatt gaagtaattg aaatccgaga 60

cctgatcttt gatctcgcta cctcattcac aagcgccggc atg agc tcc cca gca    115
                                            Met Ser Ser Pro Ala
                                              1               5

ctt gac gct gca aaa cag cgc ctt gct gaa tcc gat ggc ctg atc gct    163
Leu Asp Ala Ala Lys Gln Arg Leu Ala Glu Ser Asp Gly Leu Ile Ala
                 10                  15                  20

gtt acc cca gta ttt acc gcg agc tac tcc ggc atc ttc aag atg ttc    211
Val Thr Pro Val Phe Thr Ala Ser Tyr Ser Gly Ile Phe Lys Met Phe
             25                  30                  35

ttt gat gtc ctg gac ccc aag acc att gtg ggt ctg ccc acc atc att     259
Phe Asp Val Leu Asp Pro Lys Thr Ile Val Gly Leu Pro Thr Ile Ile
        40                  45                  50

gcg gca tct gct gga acg gca cgc cac tca ttg gtt ctc gac cac gcc     307
Ala Ala Ser Ala Gly Thr Ala Arg His Ser Leu Val Leu Asp His Ala
    55                  60                  65

atc cga cca ctg ttt acc tac ttg cga gca gtt gtc gta ccc acc ggc     355
Ile Arg Pro Leu Phe Thr Tyr Leu Arg Ala Val Val Val Pro Thr Gly
 70                  75                  80                  85

gtg ttc gca gcc acg gaa gat ttc ggc act gaa gct ggc gca gac att     403
Val Phe Ala Ala Thr Glu Asp Phe Gly Thr Glu Ala Gly Ala Asp Ile
                 90                  95                 100

gaa cgt cgc gtg aac cgc gca gct ggc gaa tta gcg aca ctc atg ttg     451
Glu Arg Arg Val Asn Arg Ala Ala Gly Glu Leu Ala Thr Leu Met Leu
            105                 110                 115

cag gat tac tcc agt gtg caa ggc ctt ggg ggc gca acc gcg aac caa     499
Gln Asp Tyr Ser Ser Val Gln Gly Leu Gly Gly Ala Thr Ala Asn Gln
        120                 125                 130

gac gct gac ctt tcc ttc cgt cgc acc act ggc gtg acc ccg gga gag     547
Asp Ala Asp Leu Ser Phe Arg Arg Thr Thr Gly Val Thr Pro Gly Glu
    135                 140                 145

aac ttc agc agc ttt gcc gat ctt tct caa agg aca cga cgg aaa cgg     595
Asn Phe Ser Ser Phe Ala Asp Leu Ser Gln Arg Thr Arg Arg Lys Arg
150                 155                 160                 165

cta aat tcg cgg atc tcc gtt taaggcattg aagcatttgg agg               639
Leu Asn Ser Arg Ile Ser Val
                170


<210> 266
<211> 172
<212> PRT
<213> Corynebacterium glutamicum

<400> 266
Met Ser Ser Pro Ala Leu Asp Ala Ala Lys Gln Arg Leu Ala Glu Ser
  1               5                  10                  15

Asp Gly Leu Ile Ala Val Thr Pro Val Phe Thr Ala Ser Tyr Ser Gly
             20                  25                  30

Ile Phe Lys Met Phe Phe Asp Val Leu Asp Pro Lys Thr Ile Val Gly
         35                  40                  45

Leu Pro Thr Ile Ile Ala Ala Ser Ala Gly Thr Ala Arg His Ser Leu
     50                  55                  60

Val Leu Asp His Ala Ile Arg Pro Leu Phe Thr Tyr Leu Arg Ala Val
 65                  70                  75                  80

Val Val Pro Thr Gly Val Phe Ala Ala Thr Glu Asp Phe Gly Thr Glu
                 85                  90                  95

Ala Gly Ala Asp Ile Glu Arg Arg Val Asn Arg Ala Ala Gly Glu Leu
            100                 105                 110

Ala Thr Leu Met Leu Gln Asp Tyr Ser Ser Val Gln Gly Leu Gly Gly
        115                 120                 125

Ala Thr Ala Asn Gln Asp Ala Asp Leu Ser Phe Arg Arg Thr Thr Gly
    130                 135                 140

Val Thr Pro Gly Glu Asn Phe Ser Ser Phe Ala Asp Leu Ser Gln Arg
145                 150                 155                 160

Thr Arg Arg Lys Arg Leu Asn Ser Arg Ile Ser Val
                165                 170

<210> 267
<211> 1044
<212> DNA
<213> Corynebacterium glutamicum

<220>
<221> CDS
<222> (101)..(1021)
<223> RXN00513

<400> 267
cacagcgtac gcacgagctt gaggatcttt cagaactcaa cattgaattg gatgccgata 60

ttttggccaa ggctcctgtg attccggaag gactgttctg atg gcg ggt ttg ttt 115
Met Ala Gly Leu Phe
1               5

tcc tct gct gtt gca cca acg gag cgt cga aaa gca tta cgc gcg gca 163
Ser Ser Ala Val Ala Pro Thr Glu Arg Arg Lys Ala Leu Arg Ala Ala
                10                  15                  20

ctg gct gcg cct gaa att gcc cgc atg cct ggt gca ttc tcc ccg ctg 211
Leu Ala Ala Pro Glu Ile Ala Arg Met Pro Gly Ala Phe Ser Pro Leu
            25                  30                  35

gcg gcg cgc gca atc cag gaa gcc gga ttt gaa ggc gtg tac gtc tcg 259
Ala Ala Arg Ala Ile Gln Glu Ala Gly Phe Glu Gly Val Tyr Val Ser
        40                  45                  50

ggc gcc gtc gtg gcg gct gac ctt gca ttg ccg gat atc ggc ttg acc 307
Gly Ala Val Val Ala Ala Asp Leu Ala Leu Pro Asp Ile Gly Leu Thr
    55                  60                  65

aca ttg acc gaa gtg gcg cac cgc tcc cgg cag atc gca cgc gtg aca 355
Thr Leu Thr Glu Val Ala His Arg Ser Arg Gln Ile Ala Arg Val Thr
70                  75                  80                  85

gac ttg ccc gtg ctg gtc gac gcc gac acc ggc ttc ggc gaa ccc atg 403
Asp Leu Pro Val Leu Val Asp Ala Asp Thr Gly Phe Gly Glu Pro Met
                90                  95                  100

tcc gca gcg cgc acc gtc tcc gaa ctc gaa gat gca ggt gtc gcg ggc 451
Ser Ala Ala Arg Thr Val Ser Glu Leu Glu Asp Ala Gly Val Ala Gly
            105                 110                 115

tgc cac ctg gaa gat caa gtc aac ccc aaa cgc tgt ggg cac ctg gac 499
Cys His Leu Glu Asp Gln Val Asn Pro Lys Arg Cys Gly His Leu Asp
        120                 125                 130

gga aaa gaa gta gtg ggc acg gac atc atg gtt cgt cgc atc gcc gca 547
Gly Lys Glu Val Val Gly Thr Asp Ile Met Val Arg Arg Ile Ala Ala
    135                 140                 145

gct gtc aac gag cgt cgc gat gag caa ttc gtc atc tgc gct cgc acc 595
Ala Val Asn Glu Arg Arg Asp Glu Gln Phe Val Ile Cys Ala Arg Thr
150                 155                 160                 165

gac gcc gcg gga gtg gaa ggc atc gac tcc gcg atc gag cgc gcc aaa 643
Asp Ala Ala Gly Val Glu Gly Ile Asp Ser Ala Ile Glu Arg Ala Lys
                170                 175                 180

gct tac gcg gat gcc ggc gcc gac atg atc ttc acc gaa gcg ctg tac 691
Ala Tyr Ala Asp Ala Gly Ala Asp Met Ile Phe Thr Glu Ala Leu Tyr
            185                 190                 195

agc cct gca gat ttt gaa aaa ttc cgc gcg gcc gtc gac att ccg ctg 739
Ser Pro Ala Asp Phe Glu Lys Phe Arg Ala Ala Val Asp Ile Pro Leu
        200                 205                 210

ctg gcc aac atg acg gaa ttt ggc aaa acc gaa ctt ctg ccc gcg cag 787
Leu Ala Asn Met Thr Glu Phe Gly Lys Thr Glu Leu Leu Pro Ala Gln
    215                 220                 225

ctt ctg gaa gac atc gga tac aac gca gtg atc tac cca gtg acc ctg 835
Leu Leu Glu Asp Ile Gly Tyr Asn Ala Val Ile Tyr Pro Val Thr Leu
230                 235                 240                 245

ctg cgc att gcg atg gga cag gtc gaa caa gct ctc ggc gac att gca     883
Leu Arg Ile Ala Met Gly Gln Val Glu Gln Ala Leu Gly Asp Ile Ala
                250                 255                 260

aac acc gga atc caa acc gac tgg gtc gac cgg atg caa cac cga tcc     931
Asn Thr Gly Ile Gln Thr Asp Trp Val Asp Arg Met Gln His Arg Ser
            265                 270                 275

agg ctg tat gag ctg ctg cgc tac aac gag tac aac gct ttc gac cag     979
Arg Leu Tyr Glu Leu Leu Arg Tyr Asn Glu Tyr Asn Ala Phe Asp Gln
        280                 285                 290

caa gta ttc acc tat tcc gct gac agc tac aag ccc atc ttc             1021
Gln Val Phe Thr Tyr Ser Ala Asp Ser Tyr Lys Pro Ile Phe
    295                 300                 305

taacccgcct atatataagg agt                                           1044


<210> 268
<211> 307
<212> PRT
<213> Corynebacterium glutamicum

<400> 268
Met Ala Gly Leu Phe Ser Ser Ala Val Ala Pro Thr Glu Arg Arg Lys
1               5                   10                  15

Ala Leu Arg Ala Ala Leu Ala Ala Pro Glu Ile Ala Arg Met Pro Gly
            20                  25                  30

Ala Phe Ser Pro Leu Ala Ala Arg Ala Ile Gln Glu Ala Gly Phe Glu
        35                  40                  45

Gly Val Tyr Val Ser Gly Ala Val Val Ala Ala Asp Leu Ala Leu Pro
    50                  55                  60

Asp Ile Gly Leu Thr Thr Leu Thr Glu Val Ala His Arg Ser Arg Gln
65                  70                  75                  80

Ile Ala Arg Val Thr Asp Leu Pro Val Leu Val Asp Ala Asp Thr Gly
                85                  90                  95

Phe Gly Glu Pro Met Ser Ala Ala Arg Thr Val Ser Glu Leu Glu Asp
            100                 105                 110

Ala Gly Val Ala Gly Cys His Leu Glu Asp Gln Val Asn Pro Lys Arg
        115                 120                 125

Cys Gly His Leu Asp Gly Lys Glu Val Val Gly Thr Asp Ile Met Val
    130                 135                 140

Arg Arg Ile Ala Ala Ala Val Asn Glu Arg Arg Asp Glu Gln Phe Val
145                 150                 155                 160

Ile Cys Ala Arg Thr Asp Ala Ala Gly Val Glu Gly Ile Asp Ser Ala
                165                 170                 175

Ile Glu Arg Ala Lys Ala Tyr Ala Asp Ala Gly Ala Asp Met Ile Phe
            180                 185                 190

Thr Glu Ala Leu Tyr Ser Pro Ala Asp Phe Glu Lys Phe Arg Ala Ala
        195                 200                 205

Val Asp Ile Pro Leu Leu Ala Asn Met Thr Glu Phe Gly Lys Thr Glu
    210                 215                 220

Leu Leu Pro Ala Gln Leu Leu Glu Asp Ile Gly Tyr Asn Ala Val Ile
225                 230                 235                 240

Tyr Pro Val Thr Leu Leu Arg Ile Ala Met Gly Gln Val Glu Gln Ala
                245                 250                 255

Leu Gly Asp Ile Ala Asn Thr Gly Ile Gln Thr Asp Trp Val Asp Arg
            260                 265                 270

Met Gln His Arg Ser Arg Leu Tyr Glu Leu Leu Arg Tyr Asn Glu Tyr

275                     280                     285

Asn Ala Phe Asp Gln Gln Val Phe Thr Tyr Ser Ala Asp Ser Tyr Lys
    290                     295                     300

Pro Ile Phe
305


<210> 269
<211> 957
<212> DNA
<213> Corynebacterium glutamicum

<220>
<221> CDS
<222> (101)..(934)
<223> RXN01152

<400> 269
agtgaaggat ctgatgtggt ttgaacaagc cctggaagcc tatctggtaa attaacgccg 60

agttcaatca agacaagcac acagaagaaa gtgagggctc atg ccc tac tca ggt   115
                                           Met Pro Tyr Ser Gly
                                           1               5

ccg ttc caa gca ggc gac cgc gtt cag ctc acc gac gct aaa cgc cgc   163
Pro Phe Gln Ala Gly Asp Arg Val Gln Leu Thr Asp Ala Lys Arg Arg
                10                  15                  20

cat ttc acc atc att ttg gaa cca gga acc acc tac cac acc cac cgt   211
His Phe Thr Ile Ile Leu Glu Pro Gly Thr Thr Tyr His Thr His Arg
            25                  30                  35

gga caa atc gca cac gat gac atc atc ggc gcc gat gag ggc act gtt   259
Gly Gln Ile Ala His Asp Asp Ile Ile Gly Ala Asp Glu Gly Thr Val
        40                  45                  50

gtc cac tcc acc atg ggc tct gat tac ttg tgc ttc cgt cac ctc atg   307
Val His Ser Thr Met Gly Ser Asp Tyr Leu Cys Phe Arg His Leu Met
    55                  60                  65

gtt gat cac gtg ctg agc atg cct cgt ggc gct gca gtt att tat cca   355
Val Asp His Val Leu Ser Met Pro Arg Gly Ala Ala Val Ile Tyr Pro
70                  75                  80                  85

aag gac tct gca cag att ctg gtc gag ggc gat att ttc cct ggc gcc   403
Lys Asp Ser Ala Gln Ile Leu Val Glu Gly Asp Ile Phe Pro Gly Ala
                90                  95                  100

cga gtt ctg gaa gct ggc gct ggt tcc ggt gca ctg tcc atg gcg ctg   451
Arg Val Leu Glu Ala Gly Ala Gly Ser Gly Ala Leu Ser Met Ala Leu
            105                 110                 115

ctt cgt gca gtg ggt gaa aag ggc aat gtc atc tcc tac gaa atc cgt   499
Leu Arg Ala Val Gly Glu Lys Gly Asn Val Ile Ser Tyr Glu Ile Arg
        120                 125                 130

gag gat cac ctg gag tac gca gtc tcc aac gtg gag gag tac ttc ggt   547
Glu Asp His Leu Glu Tyr Ala Val Ser Asn Val Glu Glu Tyr Phe Gly
    135                 140                 145

gag cgt cca gca acc tgg gat cca cgt ctt ggt gac ctg aaa gaa gtc   595
Glu Arg Pro Ala Thr Trp Asp Pro Arg Leu Gly Asp Leu Lys Glu Val
150                 155                 160                 165

acc gtt gag gat ctc ggc gga cct gtt gac cgc atc atc ttg gat atg   643
Thr Val Glu Asp Leu Gly Gly Pro Val Asp Arg Ile Ile Leu Asp Met
                170                 175                 180

ctt gag ccg tgg gaa atg ctg gag acc tgc aag gat ctt ctc atc cct   691
Leu Glu Pro Trp Glu Met Leu Glu Thr Cys Lys Asp Leu Leu Ile Pro
            185                 190                 195

ggt ggt gtg ttt atg acg tat gtg gcg acc gtg cca cag ctg atg aag   739
Gly Gly Val Phe Met Thr Tyr Val Ala Thr Val Pro Gln Leu Met Lys
        200                 205                 210

gtc atg gaa ggc atc cgc gag caa aaa tgc ttc acg gag cca cgc gcg     787
Val Met Glu Gly Ile Arg Glu Gln Lys Cys Phe Thr Glu Pro Arg Ala
    215                 220                 225

tgg gaa tct ttg gtt cgt gat tgg aag gtg gag ggc ttg gca aca cgc     835
Trp Glu Ser Leu Val Arg Asp Trp Lys Val Glu Gly Leu Ala Thr Arg
230                 235                 240                 245

cct gag cac cgc atg aat gcc cac acc gcg ttc ttg gtg ttg acc agg     883
Pro Glu His Arg Met Asn Ala His Thr Ala Phe Leu Val Leu Thr Arg
                250                 255                 260

cgt ttg gct gat ggc gtg gag cct cct cgt ccg cag cgt aag gca cgt     931
Arg Leu Ala Asp Gly Val Glu Pro Pro Arg Pro Gln Arg Lys Ala Arg
            265                 270                 275

cga taaaaagacc tagttggagg gcg                                       957
Arg

<210> 270
<211> 278
<212> PRT
<213> Corynebacterium glutamicum

<400> 270
Met Pro Tyr Ser Gly Pro Phe Gln Ala Gly Asp Arg Val Gln Leu Thr
 1               5                  10                  15

Asp Ala Lys Arg Arg His Phe Thr Ile Ile Leu Glu Pro Gly Thr Thr
            20                  25                  30

Tyr His Thr His Arg Gly Gln Ile Ala His Asp Asp Ile Ile Gly Ala
        35                  40                  45

Asp Glu Gly Thr Val Val His Ser Thr Met Gly Ser Asp Tyr Leu Cys
    50                  55                  60

Phe Arg His Leu Met Val Asp His Val Leu Ser Met Pro Arg Gly Ala
65                  70                  75                  80

Ala Val Ile Tyr Pro Lys Asp Ser Ala Gln Ile Leu Val Glu Gly Asp
                85                  90                  95

Ile Phe Pro Gly Ala Arg Val Leu Glu Ala Gly Ala Gly Ser Gly Ala
            100                 105                 110

Leu Ser Met Ala Leu Leu Arg Ala Val Gly Glu Lys Gly Asn Val Ile
        115                 120                 125

Ser Tyr Glu Ile Arg Glu Asp His Leu Glu Tyr Ala Val Ser Asn Val
    130                 135                 140

Glu Glu Tyr Phe Gly Glu Arg Pro Ala Thr Trp Asp Pro Arg Leu Gly
145                 150                 155                 160

Asp Leu Lys Glu Val Thr Val Glu Asp Leu Gly Gly Pro Val Asp Arg
                165                 170                 175

Ile Ile Leu Asp Met Leu Glu Pro Trp Glu Met Leu Glu Thr Cys Lys
            180                 185                 190

Asp Leu Leu Ile Pro Gly Gly Val Phe Met Thr Tyr Val Ala Thr Val
        195                 200                 205

Pro Gln Leu Met Lys Val Met Glu Gly Ile Arg Glu Gln Lys Cys Phe
    210                 215                 220

Thr Glu Pro Arg Ala Trp Glu Ser Leu Val Arg Asp Trp Lys Val Glu
225                 230                 235                 240

Gly Leu Ala Thr Arg Pro Glu His Arg Met Asn Ala His Thr Ala Phe
                245                 250                 255

Leu Val Leu Thr Arg Arg Leu Ala Asp Gly Val Glu Pro Pro Arg Pro

260                 265                 270

Gln Arg Lys Ala Arg Arg
        275


<210> 271
<211> 2025
<212> DNA
<213> Corynebacterium glutamicum

<220>
<221> CDS
<222> (101)..(2002)
<223> RXN00787

<400> 271
ccagcccgcc caataaataa tttctctctt ctaattgcgg agcctcatat attgagtacg 60

gtattttgaa acaccttcag cccccttttt aggagccaca gtg tct cag cct ctc    115
                                            Val Ser Gln Pro Leu
                                            1               5

agc aag cgt ctc agc ata cga aaa gca ctc gcc agc gcc ttc ata gtt    163
Ser Lys Arg Leu Ser Ile Arg Lys Ala Leu Ala Ser Ala Phe Ile Val
                10                  15                  20

gcg ctg gcg ttt tcg ctt tcc cca gta gcc aaa gcc caa gcc aat gaa    211
Ala Leu Ala Phe Ser Leu Ser Pro Val Ala Lys Ala Gln Ala Asn Glu
            25                  30                  35

act ccg acg atg atc gtg ttg gac aat tca ggc tcc atg aca gct caa    259
Thr Pro Thr Met Ile Val Leu Asp Asn Ser Gly Ser Met Thr Ala Gln
        40                  45                  50

gat gcc ggc gga cag acc cgt atc gat gca gca aaa caa gcc tcc act    307
Asp Ala Gly Gly Gln Thr Arg Ile Asp Ala Ala Lys Gln Ala Ser Thr
    55                  60                  65

cag tta att aat gac atc tcc gac cgc acc gac gta ggt ctg acc tac    355
Gln Leu Ile Asn Asp Ile Ser Asp Arg Thr Asp Val Gly Leu Thr Tyr
70                  75                  80                  85

tac ggc gga aac acc ggc gaa aca gaa gca gac gtt gag atg gga tgc    403
Tyr Gly Gly Asn Thr Gly Glu Thr Glu Ala Asp Val Glu Met Gly Cys
                90                  95                  100

caa gac gtc acc atc ctt ggc ggc ccc tcc cga gga aat gca gac acc    451
Gln Asp Val Thr Ile Leu Gly Gly Pro Ser Arg Gly Asn Ala Asp Thr
            105                 110                 115

tta att gac acg atc aac agc ctg cag cct cga ggc ttc acc ccc atc    499
Leu Ile Asp Thr Ile Asn Ser Leu Gln Pro Arg Gly Phe Thr Pro Ile
        120                 125                 130

ggc aaa gca ctc acc gat acc gcc gcc gag ctc ccc gaa ggc gga aac    547
Gly Lys Ala Leu Thr Asp Thr Ala Ala Glu Leu Pro Glu Gly Gly Asn
    135                 140                 145

att gtg ttg gtc tcc gat ggc atc gcc aac tgc acc cca ccg gat gtc    595
Ile Val Leu Val Ser Asp Gly Ile Ala Asn Cys Thr Pro Pro Asp Val
150                 155                 160                 165

tgc gaa gta gcc caa gaa ctg gct caa agt gga atc aac ctg gtt atc    643
Cys Glu Val Ala Gln Glu Leu Ala Gln Ser Gly Ile Asn Leu Val Ile
                170                 175                 180

aac acc atc gga cta aat gtt gat cca gca gcg cgc gaa gaa ctg gag    691
Asn Thr Ile Gly Leu Asn Val Asp Pro Ala Ala Arg Glu Glu Leu Glu
            185                 190                 195

tgc atc gct gga gtc ggt ggt ggc act tac gcg gat gct tcc gac gcg    739
Cys Ile Ala Gly Val Gly Gly Gly Thr Tyr Ala Asp Ala Ser Asp Ala
        200                 205                 210

cag agc ctt acc gat gcg ctg aca cga gcc gcc agt agg caa tac aac    787
Gln Ser Leu Thr Asp Ala Leu Thr Arg Ala Ala Ser Arg Gln Tyr Asn

215                 220                 225

tct tac acc tcc gat gtg aca aaa att gat ggg gca tcg gaa caa agc     835
Ser Tyr Thr Ser Asp Val Thr Lys Ile Asp Gly Ala Ser Glu Gln Ser
230                 235                 240                 245

gca gcc gta gaa att gat gag gat aca gaa cta ttc ctc acc gac ctg     883
Ala Ala Val Glu Ile Asp Glu Asp Thr Glu Leu Phe Leu Thr Asp Leu
                250                 255                 260

cca caa gaa tcc cgc ttt tgg aaa atc cct gta gag cca ggt gaa acc     931
Pro Gln Glu Ser Arg Phe Trp Lys Ile Pro Val Glu Pro Gly Glu Thr
            265                 270                 275

atc tca gtt tct gcc aac aca gtt acc gac cca aca gta ctc acc atg     979
Ile Ser Val Ser Ala Asn Thr Val Thr Asp Pro Thr Val Leu Thr Met
        280                 285                 290

ggg caa ggc gga atc aag ctt gaa gcc caa ctc cat act gaa gag gct     1027
Gly Gln Gly Gly Ile Lys Leu Glu Ala Gln Leu His Thr Glu Glu Ala
    295                 300                 305

cca caa tac ggc ctg cgt ggt cgg tgc act cgg gtc tca ttt gat aat     1075
Pro Gln Tyr Gly Leu Arg Gly Arg Cys Thr Arg Val Ser Phe Asp Asn
310                 315                 320                 325

ttc aag ccc ggc ctt ggt gta cgc gga atc caa aac gcg tcc gtt gca     1123
Phe Lys Pro Gly Leu Gly Val Arg Gly Ile Gln Asn Ala Ser Val Ala
                330                 335                 340

tca aaa gaa gtg ggc acc aac aac tgt gac acc gat gcc atc tac ctc     1171
Ser Lys Glu Val Gly Thr Asn Asn Cys Asp Thr Asp Ala Ile Tyr Leu
            345                 350                 355

gaa att tct aga agc gga gat tac ctc aac ggg cag gac att cca acg     1219
Glu Ile Ser Arg Ser Gly Asp Tyr Leu Asn Gly Gln Asp Ile Pro Thr
        360                 365                 370

gaa atc acc atc gag cgc ttc gga aaa gta gat gaa tca aca atc gga     1267
Glu Ile Thr Ile Glu Arg Phe Gly Lys Val Asp Glu Ser Thr Ile Gly
    375                 380                 385

aat gtc aca gag gaa cat agc tcc gtc gat ctt acc gag gct gca gca     1315
Asn Val Thr Glu Glu His Ser Ser Val Asp Leu Thr Glu Ala Ala Ala
390                 395                 400                 405

tca gag gca cac cct gtc aca cct ggc cag tgg ttc aca tcg gcc gct     1363
Ser Glu Ala His Pro Val Thr Pro Gly Gln Trp Phe Thr Ser Ala Ala
                410                 415                 420

gat cta gat ccc gca ggt gag aaa gtc tcc tcc atc atc gtt cca gga     1411
Asp Leu Asp Pro Ala Gly Glu Lys Val Ser Ser Ile Ile Val Pro Gly
            425                 430                 435

gaa acc cac ttc tat gcg ctg cct gtc gac tac ggc caa gaa ctg cgc     1459
Glu Thr His Phe Tyr Ala Leu Pro Val Asp Tyr Gly Gln Glu Leu Arg
        440                 445                 450

gca gct gta gaa aca act ttt gac caa atc gac agt tcc gcg ctt ggc     1507
Ala Ala Val Glu Thr Thr Phe Asp Gln Ile Asp Ser Ser Ala Leu Gly
    455                 460                 465

acg cat ctt tat atc caa gcg ttc agc cca aac cgg gca gag ata gag     1555
Thr His Leu Tyr Ile Gln Ala Phe Ser Pro Asn Arg Ala Glu Ile Glu
470                 475                 480                 485

ctc acc aat aga gat acg tca tat gcg gac gac aac ggg ctc aaa act     1603
Leu Thr Asn Arg Asp Thr Ser Tyr Ala Asp Asp Asn Gly Leu Lys Thr
                490                 495                 500

ttt gga ttc ttc acc cca gtg agt gca gca aat ttg ttc gag aaa agt     1651
Phe Gly Phe Phe Thr Pro Val Ser Ala Ala Asn Leu Phe Glu Lys Ser
            505                 510                 515

tct caa ggc ata tcg cta agg agc cca tgg caa ggt ggc acc caa tac     1699
Ser Gln Gly Ile Ser Leu Arg Ser Pro Trp Gln Gly Gly Thr Gln Tyr
        520                 525                 530

ctc gca gtg aca tac cta cca agt ggt caa gat gaa gat gta tcc gca     1747
Leu Ala Val Thr Tyr Leu Pro Ser Gly Gln Asp Glu Asp Val Ser Ala
    535                 540                 545

act gat cag ctg ccc aca ttg gaa tat gaa ctc gtg gca gaa gcg ttt     1795
Thr Asp Gln Leu Pro Thr Leu Glu Tyr Glu Leu Val Ala Glu Ala Phe
550                 555                 560                 565

gga gac cct gtt gac cca ccg gtt ttc gct tca ttg acg gga gca acc     1843
Gly Asp Pro Val Asp Pro Pro Val Phe Ala Ser Leu Thr Gly Ala Thr
                570                 575                 580

cca agc acc tcc acc ccc cca tca gat gtt gcg gaa gat gaa caa atc     1891
Pro Ser Thr Ser Thr Pro Pro Ser Asp Val Ala Glu Asp Glu Gln Ile
            585                 590                 595

tcc gag gca aca gaa gaa gac tca agc agt ttc ccc atc gtg tgg att     1939
Ser Glu Ala Thr Glu Glu Asp Ser Ser Ser Phe Pro Ile Val Trp Ile
        600                 605                 610

ggg ctg ggt gtc att ggc tta ggc ata atc att ggt ttg atc ttt gcg     1987
Gly Leu Gly Val Ile Gly Leu Gly Ile Ile Ile Gly Leu Ile Phe Ala
    615                 620                 625

ctg aga aga aag aat taagccctaa aagataaaga gtc                      2025
Leu Arg Arg Lys Asn
630


<210> 272
<211> 634
<212> PRT
<213> Corynebacterium glutamicum

<400> 272
Val Ser Gln Pro Leu Ser Lys Arg Leu Ser Ile Arg Lys Ala Leu Ala
1               5                   10                  15

Ser Ala Phe Ile Val Ala Leu Ala Phe Ser Leu Ser Pro Val Ala Lys
            20                  25                  30

Ala Gln Ala Asn Glu Thr Pro Thr Met Ile Val Leu Asp Asn Ser Gly
        35                  40                  45

Ser Met Thr Ala Gln Asp Ala Gly Gly Gln Thr Arg Ile Asp Ala Ala
    50                  55                  60

Lys Gln Ala Ser Thr Gln Leu Ile Asn Asp Ile Ser Asp Arg Thr Asp
65                  70                  75                  80

Val Gly Leu Thr Tyr Tyr Gly Gly Asn Thr Gly Glu Thr Glu Ala Asp
                85                  90                  95

Val Glu Met Gly Cys Gln Asp Val Thr Ile Leu Gly Gly Pro Ser Arg
            100                 105                 110

Gly Asn Ala Asp Thr Leu Ile Asp Thr Ile Asn Ser Leu Gln Pro Arg
        115                 120                 125

Gly Phe Thr Pro Ile Gly Lys Ala Leu Thr Asp Thr Ala Ala Glu Leu
    130                 135                 140

Pro Glu Gly Gly Asn Ile Val Leu Val Ser Asp Gly Ile Ala Asn Cys
145                 150                 155                 160

Thr Pro Pro Asp Val Cys Glu Val Ala Gln Glu Leu Ala Gln Ser Gly
                165                 170                 175

Ile Asn Leu Val Ile Asn Thr Ile Gly Leu Asn Val Asp Pro Ala Ala
            180                 185                 190

Arg Glu Glu Leu Glu Cys Ile Ala Gly Val Gly Gly Gly Thr Tyr Ala
        195                 200                 205

Asp Ala Ser Asp Ala Gln Ser Leu Thr Asp Ala Leu Thr Arg Ala Ala
    210                 215                 220

Ser Arg Gln Tyr Asn Ser Tyr Thr Ser Asp Val Thr Lys Ile Asp Gly
225                 230                 235                 240

Ala Ser Glu Gln Ser Ala Ala Val Glu Ile Asp Glu Asp Thr Glu Leu
                245                 250                 255

Phe Leu Thr Asp Leu Pro Gln Glu Ser Arg Phe Trp Lys Ile Pro Val
            260                 265                 270

Glu Pro Gly Glu Thr Ile Ser Val Ser Ala Asn Thr Val Thr Asp Pro
        275                 280                 285

Thr Val Leu Thr Met Gly Gln Gly Gly Ile Lys Leu Glu Ala Gln Leu
    290                 295                 300

His Thr Glu Glu Ala Pro Gln Tyr Gly Leu Arg Gly Arg Cys Thr Arg
305                 310                 315                 320

Val Ser Phe Asp Asn Phe Lys Pro Gly Leu Gly Val Arg Gly Ile Gln
                325                 330                 335

Asn Ala Ser Val Ala Ser Lys Glu Val Gly Thr Asn Asn Cys Asp Thr
            340                 345                 350

Asp Ala Ile Tyr Leu Glu Ile Ser Arg Ser Gly Asp Tyr Leu Asn Gly
        355                 360                 365

Gln Asp Ile Pro Thr Glu Ile Thr Ile Glu Arg Phe Gly Lys Val Asp
    370                 375                 380

Glu Ser Thr Ile Gly Asn Val Thr Glu Glu His Ser Ser Val Asp Leu
385                 390                 395                 400

Thr Glu Ala Ala Ala Ser Glu Ala His Pro Val Thr Pro Gly Gln Trp
                405                 410                 415

Phe Thr Ser Ala Ala Asp Leu Asp Pro Ala Gly Glu Lys Val Ser Ser
            420                 425                 430

Ile Ile Val Pro Gly Glu Thr His Phe Tyr Ala Leu Pro Val Asp Tyr
        435                 440                 445

Gly Gln Glu Leu Arg Ala Ala Val Glu Thr Thr Phe Asp Gln Ile Asp
    450                 455                 460

Ser Ser Ala Leu Gly Thr His Leu Tyr Ile Gln Ala Phe Ser Pro Asn
465                 470                 475                 480

Arg Ala Glu Ile Glu Leu Thr Asn Arg Asp Thr Ser Tyr Ala Asp Asp
                485                 490                 495

Asn Gly Leu Lys Thr Phe Gly Phe Phe Thr Pro Val Ser Ala Ala Asn
            500                 505                 510

Leu Phe Glu Lys Ser Ser Gln Gly Ile Ser Leu Arg Ser Pro Trp Gln
        515                 520                 525

Gly Gly Thr Gln Tyr Leu Ala Val Thr Tyr Leu Pro Ser Gly Gln Asp
    530                 535                 540

Glu Asp Val Ser Ala Thr Asp Gln Leu Pro Thr Leu Glu Tyr Glu Leu
545                 550                 555                 560

Val Ala Glu Ala Phe Gly Asp Pro Val Asp Pro Pro Val Phe Ala Ser
                565                 570                 575

Leu Thr Gly Ala Thr Pro Ser Thr Ser Thr Pro Pro Ser Asp Val Ala
            580                 585                 590

Glu Asp Glu Gln Ile Ser Glu Ala Thr Glu Glu Asp Ser Ser Ser Phe
        595                 600                 605

Pro Ile Val Trp Ile Gly Leu Gly Val Ile Gly Leu Gly Ile Ile Ile
    610                 615                 620

Gly Leu Ile Phe Ala Leu Arg Arg Lys Asn

625                 630


<210> 273
<211> 576
<212> DNA
<213> Corynebacterium glutamicum

<220>
<221> CDS
<222> (101)..(553)
<223> RXN01302

<400> 273
tggggctgcg tggtgttctt catcatcgcc accgctttga cctggatcta ctacgcccgc 60

ccgaacgctc cattcccggg ataaaccgaa aggccaatcc atg act aca act act    115
                                           Met Thr Thr Thr Thr
                                           1               5

tct tct ggg aag tct tct gaa aag atc aac ccc ctc ttc aag ctc ggc    163
Ser Ser Gly Lys Ser Ser Glu Lys Ile Asn Pro Leu Phe Lys Leu Gly
                10                  15                  20

agt ttc cta aga aaa ggc acc gtc ggt tct gaa ggc cag cag att ttc    211
Ser Phe Leu Arg Lys Gly Thr Val Gly Ser Glu Gly Gln Gln Ile Phe
            25                  30                  35

ctt cag ggc gga cgc caa gcc gat gtg ttt tat cgc aac cga tgg gct    259
Leu Gln Gly Gly Arg Gln Ala Asp Val Phe Tyr Arg Asn Arg Trp Ala
        40                  45                  50

ttc gat aaa gtc gtg cgc tcc aca cat ggc gtg aac tgc acg ggc tcc    307
Phe Asp Lys Val Val Arg Ser Thr His Gly Val Asn Cys Thr Gly Ser
    55                  60                  65

tgc tcg tgg aaa gtg tat gta aaa gac ggt gtg atc acc tgg gaa tcc    355
Cys Ser Trp Lys Val Tyr Val Lys Asp Gly Val Ile Thr Trp Glu Ser
70                  75                  80                  85

cag gca gtg gat tac cca act acc ggt gcg gat atg ccc gac aat gaa    403
Gln Ala Val Asp Tyr Pro Thr Thr Gly Ala Asp Met Pro Asp Asn Glu
                90                  95                  100

cca cgt ggc tgc cct cgt ggt gca tca ttt tcc tgg tac acc tac tcc    451
Pro Arg Gly Cys Pro Arg Gly Ala Ser Phe Ser Trp Tyr Thr Tyr Ser
            105                 110                 115

cca acc cgc atc cgc tac cca tac atc ggt ggc gtg cta gtt gat atg    499
Pro Thr Arg Ile Arg Tyr Pro Tyr Ile Gly Gly Val Leu Val Asp Met
        120                 125                 130

tcc gcg aag cca agg aac gcc tgg gcg atc cgg tgc tgg cgt ggc gcg    547
Ser Ala Lys Pro Arg Asn Ala Trp Ala Ile Arg Cys Trp Arg Gly Ala
    135                 140                 145

aca ttg tagaaacccc agaaaagcgc aaa                                  576
Thr Leu
150


<210> 274
<211> 151
<212> PRT
<213> Corynebacterium glutamicum

<400> 274
Met Thr Thr Thr Thr Ser Ser Gly Lys Ser Ser Glu Lys Ile Asn Pro
1               5                   10                  15

Leu Phe Lys Leu Gly Ser Phe Leu Arg Lys Gly Thr Val Gly Ser Glu
            20                  25                  30

Gly Gln Gln Ile Phe Leu Gln Gly Gly Arg Gln Ala Asp Val Phe Tyr
        35                  40                  45

Arg Asn Arg Trp Ala Phe Asp Lys Val Val Arg Ser Thr His Gly Val

50                          55                          60

Asn Cys Thr Gly Ser Cys Ser Trp Lys Val Tyr Val Lys Asp Gly Val
65                          70                          75                          80

Ile Thr Trp Glu Ser Gln Ala Val Asp Tyr Pro Thr Thr Gly Ala Asp
                        85                          90                          95

Met Pro Asp Asn Glu Pro Arg Gly Cys Pro Arg Gly Ala Ser Phe Ser
                100                         105                         110

Trp Tyr Thr Tyr Ser Pro Thr Arg Ile Arg Tyr Pro Tyr Ile Gly Gly
            115                         120                         125

Val Leu Val Asp Met Ser Ala Lys Pro Arg Asn Ala Trp Ala Ile Arg
    130                         135                         140

Cys Trp Arg Gly Ala Thr Leu
145                         150


<210> 275
<211> 466
<212> DNA
<213> Corynebacterium glutamicum

<220>
<221> CDS
<222> (101)..(466)
<223> FRXA01302

<400> 275
tggggctgcg tggtgttctt catcatcgcc accgctttga cctggatcta ctacgcccgc 60

ccgaacgctc cattcccggg ataaaccgaa aggccaatcc atg act aca act act    115
                                            Met Thr Thr Thr Thr
                                            1               5

tct tct ggg aag tct tct gaa aag atc aac ccc ctc ttc aag ctc ggc    163
Ser Ser Gly Lys Ser Ser Glu Lys Ile Asn Pro Leu Phe Lys Leu Gly
                10                  15                  20

agt ttc cta aga aaa ggc acc gtc ggt tct gaa ggc cag cag att ttc    211
Ser Phe Leu Arg Lys Gly Thr Val Gly Ser Glu Gly Gln Gln Ile Phe
            25                  30                  35

ctt cag ggc gga cgc caa gcc gat gtg ttt tat cgc aac cga tgg gct    259
Leu Gln Gly Gly Arg Gln Ala Asp Val Phe Tyr Arg Asn Arg Trp Ala
        40                  45                  50

ttc gat aaa gtc gtg cgc tcc aca cat ggc gtg aac tgc acg ggc tcc    307
Phe Asp Lys Val Val Arg Ser Thr His Gly Val Asn Cys Thr Gly Ser
    55                  60                  65

tgc tcg tgg aaa gtg tat gta aaa gac ggt gtg atc acc tgg gaa tcc    355
Cys Ser Trp Lys Val Tyr Val Lys Asp Gly Val Ile Thr Trp Glu Ser
70                  75                  80                  85

cag gca gtg gat tac cca act acc ggt gcg gat atg ccc gac aat gaa    403
Gln Ala Val Asp Tyr Pro Thr Thr Gly Ala Asp Met Pro Asp Asn Glu
                90                  95                  100

cca cgt ggc tgc cct cgt ggt gca tca ttt tcc tgg tac acc tac tcc    451
Pro Arg Gly Cys Pro Arg Gly Ala Ser Phe Ser Trp Tyr Thr Tyr Ser
            105                 110                 115

cca acc ggc atc cgc                                                466
Pro Thr Gly Ile Arg
        120


<210> 276
<211> 122
<212> PRT
<213> Corynebacterium glutamicum

<400> 276

Met Thr Thr Thr Thr Ser Ser Gly Lys Ser Ser Glu Lys Ile Asn Pro
1               5                   10                  15

Leu Phe Lys Leu Gly Ser Phe Leu Arg Lys Gly Thr Val Gly Ser Glu
            20                  25                  30

Gly Gln Gln Ile Phe Leu Gln Gly Gly Arg Gln Ala Asp Val Phe Tyr
        35                  40                  45

Arg Asn Arg Trp Ala Phe Asp Lys Val Val Arg Ser Thr His Gly Val
    50                  55                  60

Asn Cys Thr Gly Ser Cys Ser Trp Lys Val Tyr Val Lys Asp Gly Val
65                  70                  75                  80

Ile Thr Trp Glu Ser Gln Ala Val Asp Tyr Pro Thr Thr Gly Ala Asp
                85                  90                  95

Met Pro Asp Asn Glu Pro Arg Gly Cys Pro Arg Gly Ala Ser Phe Ser
            100                 105                 110

Trp Tyr Thr Tyr Ser Pro Thr Gly Ile Arg
        115                 120


<210> 277
<211> 2503
<212> DNA
<213> Corynebacterium glutamicum

<220>
<221> CDS
<222> (101)..(2503)
<223> RXN01308

<400> 277
tttcctggta cacctactcc ccaacccgca tccgctaccc atacatcggt ggcgtgctag 60

ttgatatgtc cgcgaagcca aggaacgcct gggcgatccg gtg ctg gcg tgg cgc    115
                                           Val Leu Ala Trp Arg
                                           1               5

gac att gta gaa acc cca gaa aag cgc aaa gca tat gta tcc cag cgg    163
Asp Ile Val Glu Thr Pro Glu Lys Arg Lys Ala Tyr Val Ser Gln Arg
                10                  15                  20

ggc aaa ggt ggc ctc atc cgc gtt cag tat gag gaa gcc atg gag att    211
Gly Lys Gly Gly Leu Ile Arg Val Gln Tyr Glu Glu Ala Met Glu Ile
            25                  30                  35

gct gcg gca gcc cat gtg tac acc atc cgc caa tac ggc ccc gac cgc    259
Ala Ala Ala Ala His Val Tyr Thr Ile Arg Gln Tyr Gly Pro Asp Arg
        40                  45                  50

att cat gga ttc acc gtt att ccc gca atg tcg cag gtg tct tac ggt    307
Ile His Gly Phe Thr Val Ile Pro Ala Met Ser Gln Val Ser Tyr Gly
    55                  60                  65

gct ggt act cgc ttc ttg cag atg atc ggc gga gtg gcg ctg tcc ttc    355
Ala Gly Thr Arg Phe Leu Gln Met Ile Gly Gly Val Ala Leu Ser Phe
70                  75                  80                  85

tac gat tgg tac gcc gac ctc cca cca gca tca cca caa act ttc ggc    403
Tyr Asp Trp Tyr Ala Asp Leu Pro Pro Ala Ser Pro Gln Thr Phe Gly
                90                  95                  100

gat caa act gac gtt ccg gaa tct ggc gac tgg tac aac tcc agc tac    451
Asp Gln Thr Asp Val Pro Glu Ser Gly Asp Trp Tyr Asn Ser Ser Tyr
            105                 110                 115

ctc atg atg tgg ggt tcc aac att ccg gtg acc cgc acg cct gac tcc    499
Leu Met Met Trp Gly Ser Asn Ile Pro Val Thr Arg Thr Pro Asp Ser
        120                 125                 130

cac ttc atg gtg gaa gcc cgc tac aag ggc acc aag gtt gtt gtg gtt    547
His Phe Met Val Glu Ala Arg Tyr Lys Gly Thr Lys Val Val Val Val
    135                 140                 145

tcc ccg gat ttc gct gac tcc acc aaa ttt gct gat gaa tgg gca cgc     595
Ser Pro Asp Phe Ala Asp Ser Thr Lys Phe Ala Asp Glu Trp Ala Arg
150                 155                 160                 165

atc cac cct ggt act gac ggc gca ctc gcc ttt gcc atg ggc cat gtg     643
Ile His Pro Gly Thr Asp Gly Ala Leu Ala Phe Ala Met Gly His Val
                170                 175                 180

atc ttg aag gaa ttc cat gtt gac aag aag acg ccg tac ttc atg gac     691
Ile Leu Lys Glu Phe His Val Asp Lys Lys Thr Pro Tyr Phe Met Asp
            185                 190                 195

tac atg cgc aaa tac acg gac tct cct ttc ctc gtg gaa tta gat gag     739
Tyr Met Arg Lys Tyr Thr Asp Ser Pro Phe Leu Val Glu Leu Asp Glu
        200                 205                 210

cac ggc gat ggc acc tac acc cca ggt aaa ttc ctc act gca gac cgc     787
His Gly Asp Gly Thr Tyr Thr Pro Gly Lys Phe Leu Thr Ala Asp Arg
    215                 220                 225

gca gct gat atc tcc cca gcg ctt gcc gcc act cca aat gcc acc cac     835
Ala Ala Asp Ile Ser Pro Ala Leu Ala Ala Thr Pro Asn Ala Thr His
230                 235                 240                 245

cgt ctc ctt gtg ctg caa aaa gat ggc tca gtt gta gat ccc ggt ggc     883
Arg Leu Leu Val Leu Gln Lys Asp Gly Ser Val Val Asp Pro Gly Gly
                250                 255                 260

act gtc gcg gac cgt tgg ggt gaa gaa ggc atg ggt aag tgg aat ctg     931
Thr Val Ala Asp Arg Trp Gly Glu Glu Gly Met Gly Lys Trp Asn Leu
            265                 270                 275

cgc tta gac ggc gta gat cca gtg atg act att gca gat gta cag act     979
Arg Leu Asp Gly Val Asp Pro Val Met Thr Ile Ala Asp Val Gln Thr
        280                 285                 290

gac acc gaa act gcg gaa gtc ctc ttc ccc cgc ttc gat ctc cca gca     1027
Asp Thr Glu Thr Ala Glu Val Leu Phe Pro Arg Phe Asp Leu Pro Ala
    295                 300                 305

act gcc acc caa gaa ggc ccc att ggt gct ggc acc atc agc cgg ggc     1075
Thr Ala Thr Gln Glu Gly Pro Ile Gly Ala Gly Thr Ile Ser Arg Gly
310                 315                 320                 325

gtt ccc acc atc acg ttg aat ggc cga aag tac acc act gtc ttt gat     1123
Val Pro Thr Ile Thr Leu Asn Gly Arg Lys Tyr Thr Thr Val Phe Asp
                330                 335                 340

gtg ttg ctc gca cac tac ggt gtg aac cgc gaa gag ctc aac ctt cct     1171
Val Leu Leu Ala His Tyr Gly Val Asn Arg Glu Glu Leu Asn Leu Pro
            345                 350                 355

ggt gag tgg cct aag gat ttc cag gat cca gtc atg ggt act cct gcg     1219
Gly Glu Trp Pro Lys Asp Phe Gln Asp Pro Val Met Gly Thr Pro Ala
        360                 365                 370

tgg cag gaa gag ctc acg ggt gtt cct gct aat cag gcg att cgt ttg     1267
Trp Gln Glu Glu Leu Thr Gly Val Pro Ala Asn Gln Ala Ile Arg Leu
    375                 380                 385

ggt cgg gaa ttt gct cag aat gct gat gat tcc aag ggc cgt tcc cag     1315
Gly Arg Glu Phe Ala Gln Asn Ala Asp Asp Ser Lys Gly Arg Ser Gln
390                 395                 400                 405

atc atc atg ggt gct ggt gtg aac cac tac ttc cat gcg gat tct att     1363
Ile Ile Met Gly Ala Gly Val Asn His Tyr Phe His Ala Asp Ser Ile
                410                 415                 420

tat cgc aca ttc ttg gcg ctg acc tct atg tgt ggc acc caa ggt gtt     1411
Tyr Arg Thr Phe Leu Ala Leu Thr Ser Met Cys Gly Thr Gln Gly Val
            425                 430                 435

aac ggt ggc ggt tgg gct cac tac gtt ggt cag gag aaa ctc cgt cca     1459
Asn Gly Gly Gly Trp Ala His Tyr Val Gly Gln Glu Lys Leu Arg Pro
        440                 445                 450

atg aat ggt tgg gca cag tat gcc ttt gct aca gac tgg cag cgt cca     1507
Met Asn Gly Trp Ala Gln Tyr Ala Phe Ala Thr Asp Trp Gln Arg Pro
    455                 460                 465

cca cgt cag atg atc acc act ggt ttc tac tac ctc acc acg gat cag     1555
Pro Arg Gln Met Ile Thr Thr Gly Phe Tyr Tyr Leu Thr Thr Asp Gln
470                 475                 480                 485

tgg agg tat gac aac act cgt gct aat cgt ctg gct tcc cca ctg gct     1603
Trp Arg Tyr Asp Asn Thr Arg Ala Asn Arg Leu Ala Ser Pro Leu Ala
                490                 495                 500

aat cgt ggc acc gtg ggt gac aaa atg acg gcg gat acc ttg gtg gaa     1651
Asn Arg Gly Thr Val Gly Asp Lys Met Thr Ala Asp Thr Leu Val Glu
            505                 510                 515

tcc atg aaa cgt gga tgg atg ccg tca ttc ccg caa ttc aac cgc aat     1699
Ser Met Lys Arg Gly Trp Met Pro Ser Phe Pro Gln Phe Asn Arg Asn
        520                 525                 530

ccc ctc atc ttg agc cag gag gcg gaa gaa aag ggc gtg tct gtt tct     1747
Pro Leu Ile Leu Ser Gln Glu Ala Glu Glu Lys Gly Val Ser Val Ser
    535                 540                 545

gac cat att gtt cag cag ctc acc gat ggt gac ttg cag ttc gcc tgc     1795
Asp His Ile Val Gln Gln Leu Thr Asp Gly Asp Leu Gln Phe Ala Cys
550                 555                 560                 565

gag gat ccg gat gca ccg gaa aac tgg cca cgc att ctg ctt aac tgg     1843
Glu Asp Pro Asp Ala Pro Glu Asn Trp Pro Arg Ile Leu Leu Asn Trp
                570                 575                 580

cgc aca aac cta atg ggc tct tca gct aag ggc acg gag ttt ttc ttg     1891
Arg Thr Asn Leu Met Gly Ser Ser Ala Lys Gly Thr Glu Phe Phe Leu
            585                 590                 595

cgc cat atg ttg ggt gtg gat tct gat gca tct gct gaa gaa aac gcg     1939
Arg His Met Leu Gly Val Asp Ser Asp Ala Ser Ala Glu Glu Asn Ala
        600                 605                 610

ccg gag gat cgt cca agt tcc att gtg tgg agg gat gag gct ccg gaa     1987
Pro Glu Asp Arg Pro Ser Ser Ile Val Trp Arg Asp Glu Ala Pro Glu
    615                 620                 625

gga aag ctc gat ttg atg ctg acc acg gat ttc cgc aac act tcc acc     2035
Gly Lys Leu Asp Leu Met Leu Thr Thr Asp Phe Arg Asn Thr Ser Thr
630                 635                 640                 645

acc ttg gtc tcg gat atc gtg ctg ccg gca gcc acc tgg tat gag aag     2083
Thr Leu Val Ser Asp Ile Val Leu Pro Ala Ala Thr Trp Tyr Glu Lys
                650                 655                 660

cat gat ttg tcc acc acg gat atg cac ccc ttc atc cac tcg ttc aat     2131
His Asp Leu Ser Thr Thr Asp Met His Pro Phe Ile His Ser Phe Asn
            665                 670                 675

gct gcg atc aac cca ccg tgg gag acg cgt act gac tgg gag gtc ttc     2179
Ala Ala Ile Asn Pro Pro Trp Glu Thr Arg Thr Asp Trp Glu Val Phe
        680                 685                 690

cac gat ctc acc aaa gaa ttc tcc tca cag gca gca acc tgg ttg ggc     2227
His Asp Leu Thr Lys Glu Phe Ser Ser Gln Ala Ala Thr Trp Leu Gly
    695                 700                 705

acc caa acc gat gtg atc acc gca ccg att gcc cat gac tcc ccg gat     2275
Thr Gln Thr Asp Val Ile Thr Ala Pro Ile Ala His Asp Ser Pro Asp
710                 715                 720                 725

gag ctc aat atg cct ggc ggt atc gtg cca gat att gat gag gtc ggg     2323
Glu Leu Asn Met Pro Gly Gly Ile Val Pro Asp Ile Asp Glu Val Gly
                730                 735                 740

ctg atc cct ggc aag acg atg gcc aag atc atc ccg gtg gaa cgt gat     2371
Leu Ile Pro Gly Lys Thr Met Ala Lys Ile Ile Pro Val Glu Arg Asp
            745                 750                 755

tac tcc aag gtg tat gaa aag tgg aca cac ttg gga cca ctc acc gcc     2419

Tyr Ser Lys Val Tyr Glu Lys Trp Thr His Leu Gly Pro Leu Thr Ala
        760                 765                 770

aaa gcg ggt acc gga acc cac ggc act gcg ttt aac gtg acc aag caa     2467
Lys Ala Gly Thr Gly Thr His Gly Thr Ala Phe Asn Val Thr Lys Gln
    775                 780                 785

acc gag gag ctg gcg ctg atc aac ggc acc tcc atc                     2503
Thr Glu Glu Leu Ala Leu Ile Asn Gly Thr Ser Ile
790                 795                 800

<210> 278
<211> 801
<212> PRT
<213> Corynebacterium glutamicum

<400> 278
Val Leu Ala Trp Arg Asp Ile Val Glu Thr Pro Glu Lys Arg Lys Ala
  1               5                  10                  15

Tyr Val Ser Gln Arg Gly Lys Gly Gly Leu Ile Arg Val Gln Tyr Glu
             20                  25                  30

Glu Ala Met Glu Ile Ala Ala Ala Ala His Val Tyr Thr Ile Arg Gln
         35                  40                  45

Tyr Gly Pro Asp Arg Ile His Gly Phe Thr Val Ile Pro Ala Met Ser
     50                  55                  60

Gln Val Ser Tyr Gly Ala Gly Thr Arg Phe Leu Gln Met Ile Gly Gly
 65                  70                  75                  80

Val Ala Leu Ser Phe Tyr Asp Trp Tyr Ala Asp Leu Pro Pro Ala Ser
                 85                  90                  95

Pro Gln Thr Phe Gly Asp Gln Thr Asp Val Pro Glu Ser Gly Asp Trp
            100                 105                 110

Tyr Asn Ser Ser Tyr Leu Met Met Trp Gly Ser Asn Ile Pro Val Thr
        115                 120                 125

Arg Thr Pro Asp Ser His Phe Met Val Glu Ala Arg Tyr Lys Gly Thr
    130                 135                 140

Lys Val Val Val Val Ser Pro Asp Phe Ala Asp Ser Thr Lys Phe Ala
145                 150                 155                 160

Asp Glu Trp Ala Arg Ile His Pro Gly Thr Asp Gly Ala Leu Ala Phe
                165                 170                 175

Ala Met Gly His Val Ile Leu Lys Glu Phe His Val Asp Lys Lys Thr
            180                 185                 190

Pro Tyr Phe Met Asp Tyr Met Arg Lys Tyr Thr Asp Ser Pro Phe Leu
        195                 200                 205

Val Glu Leu Asp Glu His Gly Asp Gly Thr Tyr Thr Pro Gly Lys Phe
    210                 215                 220

Leu Thr Ala Asp Arg Ala Ala Asp Ile Ser Pro Ala Leu Ala Ala Thr
225                 230                 235                 240

Pro Asn Ala Thr His Arg Leu Leu Val Leu Gln Lys Asp Gly Ser Val
                245                 250                 255

Val Asp Pro Gly Gly Thr Val Ala Asp Arg Trp Gly Glu Glu Gly Met
            260                 265                 270

Gly Lys Trp Asn Leu Arg Leu Asp Gly Val Asp Pro Val Met Thr Ile
        275                 280                 285

Ala Asp Val Gln Thr Asp Thr Glu Thr Ala Glu Val Leu Phe Pro Arg
    290                 295                 300

Phe Asp Leu Pro Ala Thr Ala Thr Gln Glu Gly Pro Ile Gly Ala Gly
305                 310                 315                 320

Thr Ile Ser Arg Gly Val Pro Thr Ile Thr Leu Asn Gly Arg Lys Tyr
                325                 330                 335

Thr Thr Val Phe Asp Val Leu Leu Ala His Tyr Gly Val Asn Arg Glu
            340                 345                 350

Glu Leu Asn Leu Pro Gly Glu Trp Pro Lys Asp Phe Gln Asp Pro Val
        355                 360                 365

Met Gly Thr Pro Ala Trp Gln Glu Glu Leu Thr Gly Val Pro Ala Asn
    370                 375                 380

Gln Ala Ile Arg Leu Gly Arg Glu Phe Ala Gln Asn Ala Asp Asp Ser
385                 390                 395                 400

Lys Gly Arg Ser Gln Ile Ile Met Gly Ala Gly Val Asn His Tyr Phe
                405                 410                 415

His Ala Asp Ser Ile Tyr Arg Thr Phe Leu Ala Leu Thr Ser Met Cys
            420                 425                 430

Gly Thr Gln Gly Val Asn Gly Gly Gly Trp Ala His Tyr Val Gly Gln
        435                 440                 445

Glu Lys Leu Arg Pro Met Asn Gly Trp Ala Gln Tyr Ala Phe Ala Thr
    450                 455                 460

Asp Trp Gln Arg Pro Pro Arg Gln Met Ile Thr Thr Gly Phe Tyr Tyr
465                 470                 475                 480

Leu Thr Thr Asp Gln Trp Arg Tyr Asp Asn Thr Arg Ala Asn Arg Leu
                485                 490                 495

Ala Ser Pro Leu Ala Asn Arg Gly Thr Val Gly Asp Lys Met Thr Ala
            500                 505                 510

Asp Thr Leu Val Glu Ser Met Lys Arg Gly Trp Met Pro Ser Phe Pro
        515                 520                 525

Gln Phe Asn Arg Asn Pro Leu Ile Leu Ser Gln Glu Ala Glu Glu Lys
    530                 535                 540

Gly Val Ser Val Ser Asp His Ile Val Gln Gln Leu Thr Asp Gly Asp
545                 550                 555                 560

Leu Gln Phe Ala Cys Glu Asp Pro Asp Ala Pro Glu Asn Trp Pro Arg
                565                 570                 575

Ile Leu Leu Asn Trp Arg Thr Asn Leu Met Gly Ser Ser Ala Lys Gly
            580                 585                 590

Thr Glu Phe Phe Leu Arg His Met Leu Gly Val Asp Ser Asp Ala Ser
        595                 600                 605

Ala Glu Glu Asn Ala Pro Glu Asp Arg Pro Ser Ser Ile Val Trp Arg
    610                 615                 620

Asp Glu Ala Pro Glu Gly Lys Leu Asp Leu Met Leu Thr Thr Asp Phe
625                 630                 635                 640

Arg Asn Thr Ser Thr Thr Leu Val Ser Asp Ile Val Leu Pro Ala Ala
                645                 650                 655

Thr Trp Tyr Glu Lys His Asp Leu Ser Thr Thr Asp Met His Pro Phe
            660                 665                 670

Ile His Ser Phe Asn Ala Ala Ile Asn Pro Pro Trp Glu Thr Arg Thr
        675                 680                 685

Asp Trp Glu Val Phe His Asp Leu Thr Lys Glu Phe Ser Ser Gln Ala
    690                 695                 700

Ala Thr Trp Leu Gly Thr Gln Thr Asp Val Ile Thr Ala Pro Ile Ala
705                 710                 715                 720

His Asp Ser Pro Asp Glu Leu Asn Met Pro Gly Gly Ile Val Pro Asp

725                 730                 735

Ile Asp Glu Val Gly Leu Ile Pro Gly Lys Thr Met Ala Lys Ile Ile
            740                 745                 750

Pro Val Glu Arg Asp Tyr Ser Lys Val Tyr Glu Lys Trp Thr His Leu
        755                 760                 765

Gly Pro Leu Thr Ala Lys Ala Gly Thr Gly Thr His Gly Thr Ala Phe
    770                 775                 780

Asn Val Thr Lys Gln Thr Glu Glu Leu Ala Leu Ile Asn Gly Thr Ser
785                 790                 795                 800

Ile

<210> 279
<211> 765
<212> DNA
<213> Corynebacterium glutamicum

<220>
<221> CDS
<222> (85)..(765)
<223> FRXA01307

<400> 279
ttccccaacc cgcatccgct acccatacat cggtggcgtg ctagttgata tgtccgcgaa 60

gccaaggaac gcctgggcga tccg gtg ctg gcg tgg cgc gac att gta gaa acc 114
                           Val Leu Ala Trp Arg Asp Ile Val Glu Thr
                           1               5                   10

cca gaa aag cgc aaa gca tat gta tcc cag cgg ggc aaa ggt ggc ctc    162
Pro Glu Lys Arg Lys Ala Tyr Val Ser Gln Arg Gly Lys Gly Gly Leu
                15                  20                  25

atc cgc gtt cag tat gag gaa gcc atg gag att gct gcg gca gcc cat    210
Ile Arg Val Gln Tyr Glu Glu Ala Met Glu Ile Ala Ala Ala Ala His
            30                  35                  40

gtg tac acc atc cgc caa tac ggc ccc gac cgc att cat gga ttc acc    258
Val Tyr Thr Ile Arg Gln Tyr Gly Pro Asp Arg Ile His Gly Phe Thr
        45                  50                  55

gtt att ccc gca atg tcg cag gtg tct tac ggt gct ggt act cgc ttc    306
Val Ile Pro Ala Met Ser Gln Val Ser Tyr Gly Ala Gly Thr Arg Phe
    60                  65                  70

ttg cag atg atc ggc gga gtg gcg ctg tcc ttc tac gat tgg tac gcc    354
Leu Gln Met Ile Gly Gly Val Ala Leu Ser Phe Tyr Asp Trp Tyr Ala
75                  80                  85                  90

gac ctc cca cca gca tca cca caa act ttc ggc gat caa act gac gtt    402
Asp Leu Pro Pro Ala Ser Pro Gln Thr Phe Gly Asp Gln Thr Asp Val
                95                  100                 105

ccg gaa tct ggc gac tgg tac aac tcc agc tac ctc atg atg tgg ggt    450
Pro Glu Ser Gly Asp Trp Tyr Asn Ser Ser Tyr Leu Met Met Trp Gly
            110                 115                 120

tcc aac att ccg gtg acc cgc acg cct gac tcc cac ttc atg gtg gaa    498
Ser Asn Ile Pro Val Thr Arg Thr Pro Asp Ser His Phe Met Val Glu
        125                 130                 135

gcc cgc tac aag ggc acc aag gtt gtt gtg gtt tcc ccg gat ttc gct    546
Ala Arg Tyr Lys Gly Thr Lys Val Val Val Val Ser Pro Asp Phe Ala
    140                 145                 150

gac tcc acc aaa ttt gct gat gaa tgg gca cgc atc cac cct ggt act    594
Asp Ser Thr Lys Phe Ala Asp Glu Trp Ala Arg Ile His Pro Gly Thr
155                 160                 165                 170

gac ggc gca ctc gcc ttt gcc atg ggc cat gtg atc ttg aag gaa ttc    642
Asp Gly Ala Leu Ala Phe Ala Met Gly His Val Ile Leu Lys Glu Phe

175                 180                 185

cat gtt gac aag aag acg ccg tac ttc atg gac tac atg cgc aaa tac     690
His Val Asp Lys Lys Thr Pro Tyr Phe Met Asp Tyr Met Arg Lys Tyr
            190                 195                 200

acg gac tct cct ttc ctc gtg gaa tta gat gag cac ggc gat ggc acc     738
Thr Asp Ser Pro Phe Leu Val Glu Leu Asp Glu His Gly Asp Gly Thr
        205                 210                 215

tac acc cca ggt aaa ttc ctc act gca                                 765
Tyr Thr Pro Gly Lys Phe Leu Thr Ala
    220                 225

<210> 280
<211> 227
<212> PRT
<213> Corynebacterium glutamicum

<400> 280
Val Leu Ala Trp Arg Asp Ile Val Glu Thr Pro Glu Lys Arg Lys Ala
  1               5                  10                  15

Tyr Val Ser Gln Arg Gly Lys Gly Gly Leu Ile Arg Val Gln Tyr Glu
             20                  25                  30

Glu Ala Met Glu Ile Ala Ala Ala Ala His Val Tyr Thr Ile Arg Gln
         35                  40                  45

Tyr Gly Pro Asp Arg Ile His Gly Phe Thr Val Ile Pro Ala Met Ser
     50                  55                  60

Gln Val Ser Tyr Gly Ala Gly Thr Arg Phe Leu Gln Met Ile Gly Gly
 65                  70                  75                  80

Val Ala Leu Ser Phe Tyr Asp Trp Tyr Ala Asp Leu Pro Pro Ala Ser
                 85                  90                  95

Pro Gln Thr Phe Gly Asp Gln Thr Asp Val Pro Glu Ser Gly Asp Trp
            100                 105                 110

Tyr Asn Ser Ser Tyr Leu Met Met Trp Gly Ser Asn Ile Pro Val Thr
        115                 120                 125

Arg Thr Pro Asp Ser His Phe Met Val Glu Ala Arg Tyr Lys Gly Thr
    130                 135                 140

Lys Val Val Val Val Ser Pro Asp Phe Ala Asp Ser Thr Lys Phe Ala
145                 150                 155                 160

Asp Glu Trp Ala Arg Ile His Pro Gly Thr Asp Gly Ala Leu Ala Phe
                165                 170                 175

Ala Met Gly His Val Ile Leu Lys Glu Phe His Val Asp Lys Lys Thr
            180                 185                 190

Pro Tyr Phe Met Asp Tyr Met Arg Lys Tyr Thr Asp Ser Pro Phe Leu
        195                 200                 205

Val Glu Leu Asp Glu His Gly Asp Gly Thr Tyr Thr Pro Gly Lys Phe
    210                 215                 220

Leu Thr Ala
225

<210> 281
<211> 1206
<212> DNA
<213> Corynebacterium glutamicum

<220>
<221> CDS
<222> (1)..(1206)
<223> FRXA01308

<400> 281
gcc gcc act cca aat gcc acc cac cgt ctc ctt gtg ctg caa aaa gat     48
Ala Ala Thr Pro Asn Ala Thr His Arg Leu Leu Val Leu Gln Lys Asp
 1               5                  10                  15

ggc tca gtt gta gat ccc ggt ggc act gtc gcg gac cgt tgg ggt gaa     96
Gly Ser Val Val Asp Pro Gly Gly Thr Val Ala Asp Arg Trp Gly Glu
                20                  25                  30

gaa ggc atg ggt aag tgg aat ctg cgc tta gac ggc gta gat cca gtg    144
Glu Gly Met Gly Lys Trp Asn Leu Arg Leu Asp Gly Val Asp Pro Val
            35                  40                  45

atg act att gca gat gta cag act gac acc gaa act gcg gaa gtc ctc    192
Met Thr Ile Ala Asp Val Gln Thr Asp Thr Glu Thr Ala Glu Val Leu
        50                  55                  60

ttc ccc cgc ttc gat ctc cca gca act gcc acc caa gaa ggc ccc att    240
Phe Pro Arg Phe Asp Leu Pro Ala Thr Ala Thr Gln Glu Gly Pro Ile
 65                  70                  75                  80

ggt gct ggc acc atc agc cgg ggc gtt ccc acc atc acg ttg aat ggc    288
Gly Ala Gly Thr Ile Ser Arg Gly Val Pro Thr Ile Thr Leu Asn Gly
                 85                  90                  95

cga aag tac acc act gtc ttt gat gtg ttg ctc gca cac tac ggt gtg    336
Arg Lys Tyr Thr Thr Val Phe Asp Val Leu Leu Ala His Tyr Gly Val
            100                 105                 110

aac cgc gaa gag ctc aac ctt cct ggt gag tgg cct aag gat ttc cag    384
Asn Arg Glu Glu Leu Asn Leu Pro Gly Glu Trp Pro Lys Asp Phe Gln
        115                 120                 125

gat cca gtc atg ggt act cct gcg tgg cag gaa gag ctc acg ggt gtt    432
Asp Pro Val Met Gly Thr Pro Ala Trp Gln Glu Glu Leu Thr Gly Val
    130                 135                 140

cct gct aat cag gcg att cgt ttg ggt cgg gaa ttt gct cag aat gct    480
Pro Ala Asn Gln Ala Ile Arg Leu Gly Arg Glu Phe Ala Gln Asn Ala
145                 150                 155                 160

gat gat tcc aag ggc cgt tcc cag atc atc atg ggt gct ggt gtg aac    528
Asp Asp Ser Lys Gly Arg Ser Gln Ile Ile Met Gly Ala Gly Val Asn
                165                 170                 175

cac tac ttc cat gcg gat tct att tat cgc aca ttc ttg gcg ctg acc    576
His Tyr Phe His Ala Asp Ser Ile Tyr Arg Thr Phe Leu Ala Leu Thr
            180                 185                 190

tct atg tgt ggc acc caa ggt gtt aac ggt ggc ggt tgg gct cac tac    624
Ser Met Cys Gly Thr Gln Gly Val Asn Gly Gly Gly Trp Ala His Tyr
        195                 200                 205

gtt ggt cag gag aaa ctc cgt cca atg aat ggt tgg gca cag tat gcc    672
Val Gly Gln Glu Lys Leu Arg Pro Met Asn Gly Trp Ala Gln Tyr Ala
    210                 215                 220

ttt gct aca gac tgg cag cgt cca cca cgt cag atg atc acc act ggt    720
Phe Ala Thr Asp Trp Gln Arg Pro Pro Arg Gln Met Ile Thr Thr Gly
225                 230                 235                 240

ttc tac tac ctc acc acg gat cag tgg agg tat gac aac act cgt gct    768
Phe Tyr Tyr Leu Thr Thr Asp Gln Trp Arg Tyr Asp Asn Thr Arg Ala
                245                 250                 255

aat cgt ctg gct tcc cca ctg gct aat cgt ggc acc gtg ggt gac aaa    816
Asn Arg Leu Ala Ser Pro Leu Ala Asn Arg Gly Thr Val Gly Asp Lys
            260                 265                 270

atg acg gcg gat acc ttg gtg gaa tcc atg aaa cgt gga tgg atg ccg    864
Met Thr Ala Asp Thr Leu Val Glu Ser Met Lys Arg Gly Trp Met Pro
        275                 280                 285

tca ttc ccg caa ttc aac cgc aat ccc ctc atc ttg agc cag gag gcg    912
Ser Phe Pro Gln Phe Asn Arg Asn Pro Leu Ile Leu Ser Gln Glu Ala
    290                 295                 300

gaa gaa aag ggc gtg tct gtt tct gac cat att gtt cag cag ctc acc     960
Glu Glu Lys Gly Val Ser Val Ser Asp His Ile Val Gln Gln Leu Thr
305                 310                 315                 320

gat ggt gac ttg cag ttc gcc tgc gag gat ccg gat gca ccg gaa aac     1008
Asp Gly Asp Leu Gln Phe Ala Cys Glu Asp Pro Asp Ala Pro Glu Asn
                325                 330                 335

tgg cca cgc att ctg ctt aac tgg cgc aca aac cta atg ggc tct tca     1056
Trp Pro Arg Ile Leu Leu Asn Trp Arg Thr Asn Leu Met Gly Ser Ser
            340                 345                 350

gct aag ggc acg gag ttt ttc ttg cgc cat atg ttg ggt gtg gat tct     1104
Ala Lys Gly Thr Glu Phe Phe Leu Arg His Met Leu Gly Val Asp Ser
        355                 360                 365

gat gca tct gct gaa aaa aac gcg ccg gag gat cgt cca agt tcc att     1152
Asp Ala Ser Ala Glu Lys Asn Ala Pro Glu Asp Arg Pro Ser Ser Ile
    370                 375                 380

gtg tgg agg gat gaa gct tcc gaa ggg aag ctc gat ttg atg ctg acc     1200
Val Trp Arg Asp Glu Ala Ser Glu Gly Lys Leu Asp Leu Met Leu Thr
385                 390                 395                 400

acg gat                                                             1206
Thr Asp



<210> 282
<211> 402
<212> PRT
<213> Corynebacterium glutamicum

<400> 282
Ala Ala Thr Pro Asn Ala Thr His Arg Leu Leu Val Leu Gln Lys Asp
1               5                   10                  15

Gly Ser Val Val Asp Pro Gly Gly Thr Val Ala Asp Arg Trp Gly Glu
            20                  25                  30

Glu Gly Met Gly Lys Trp Asn Leu Arg Leu Asp Gly Val Asp Pro Val
        35                  40                  45

Met Thr Ile Ala Asp Val Gln Thr Asp Thr Glu Thr Ala Glu Val Leu
    50                  55                  60

Phe Pro Arg Phe Asp Leu Pro Ala Thr Ala Thr Gln Glu Gly Pro Ile
65                  70                  75                  80

Gly Ala Gly Thr Ile Ser Arg Gly Val Pro Thr Ile Thr Leu Asn Gly
                85                  90                  95

Arg Lys Tyr Thr Thr Val Phe Asp Val Leu Leu Ala His Tyr Gly Val
            100                 105                 110

Asn Arg Glu Glu Leu Asn Leu Pro Gly Glu Trp Pro Lys Asp Phe Gln
        115                 120                 125

Asp Pro Val Met Gly Thr Pro Ala Trp Gln Glu Glu Leu Thr Gly Val
    130                 135                 140

Pro Ala Asn Gln Ala Ile Arg Leu Gly Arg Glu Phe Ala Gln Asn Ala
145                 150                 155                 160

Asp Asp Ser Lys Gly Arg Ser Gln Ile Ile Met Gly Ala Gly Val Asn
                165                 170                 175

His Tyr Phe His Ala Asp Ser Ile Tyr Arg Thr Phe Leu Ala Leu Thr
            180                 185                 190

Ser Met Cys Gly Thr Gln Gly Val Asn Gly Gly Gly Trp Ala His Tyr
        195                 200                 205

Val Gly Gln Glu Lys Leu Arg Pro Met Asn Gly Trp Ala Gln Tyr Ala
    210                 215                 220

Phe Ala Thr Asp Trp Gln Arg Pro Pro Arg Gln Met Ile Thr Thr Gly
225                 230                 235                 240

Phe Tyr Tyr Leu Thr Thr Asp Gln Trp Arg Tyr Asp Asn Thr Arg Ala
                245                 250                 255

Asn Arg Leu Ala Ser Pro Leu Ala Asn Arg Gly Thr Val Gly Asp Lys
            260                 265                 270

Met Thr Ala Asp Thr Leu Val Glu Ser Met Lys Arg Gly Trp Met Pro
        275                 280                 285

Ser Phe Pro Gln Phe Asn Arg Asn Pro Leu Ile Leu Ser Gln Glu Ala
    290                 295                 300

Glu Glu Lys Gly Val Ser Val Ser Asp His Ile Val Gln Gln Leu Thr
305                 310                 315                 320

Asp Gly Asp Leu Gln Phe Ala Cys Glu Asp Pro Asp Ala Pro Glu Asn
                325                 330                 335

Trp Pro Arg Ile Leu Leu Asn Trp Arg Thr Asn Leu Met Gly Ser Ser
            340                 345                 350

Ala Lys Gly Thr Glu Phe Phe Leu Arg His Met Leu Gly Val Asp Ser
        355                 360                 365

Asp Ala Ser Ala Glu Lys Asn Ala Pro Glu Asp Arg Pro Ser Ser Ile
    370                 375                 380

Val Trp Arg Asp Glu Ala Ser Glu Gly Lys Leu Asp Leu Met Leu Thr
385                 390                 395                 400

Thr Asp



<210> 283
<211> 824
<212> DNA
<213> Corynebacterium glutamicum

<220>
<221> CDS
<222> (1)..(801)
<223> RXN01309

<400> 283
att gca gac cac gaa ggt acc cac atc aat tgg gac atg gtc aaa gaa      48
Ile Ala Asp His Glu Gly Thr His Ile Asn Trp Asp Met Val Lys Glu
  1               5                  10                  15

cgt tcc gcc gag gtg atc acc tca ccg gag tgg act ggt tcc aag aag      96
Arg Ser Ala Glu Val Ile Thr Ser Pro Glu Trp Thr Gly Ser Lys Lys
             20                  25                  30

gac gga cgt cgc tac acc gcg ttt tcc atc aac att gaa tac gac aag     144
Asp Gly Arg Arg Tyr Thr Ala Phe Ser Ile Asn Ile Glu Tyr Asp Lys
         35                  40                  45

ccg tgg cac acc ctg tct ggt cgc atg cac tac tac ctc gac cac gat     192
Pro Trp His Thr Leu Ser Gly Arg Met His Tyr Tyr Leu Asp His Asp
     50                  55                  60

tgg ttt att gat tac ggc gag cag ttg cca atc ttt agg cca ccg ttg     240
Trp Phe Ile Asp Tyr Gly Glu Gln Leu Pro Ile Phe Arg Pro Pro Leu
 65                  70                  75                  80

gac aag atc cac atc aat ggt gag gtc ggc cct ggc cag tcg gtc aca     288
Asp Lys Ile His Ile Asn Gly Glu Val Gly Pro Gly Gln Ser Val Thr
                 85                  90                  95

ggc acc gac ggc gaa cca gaa gta acc gtg cgt tat ctg acc acc cac     336
Gly Thr Asp Gly Glu Pro Glu Val Thr Val Arg Tyr Leu Thr Thr His
            100                 105                 110

aac aag tgg tcg att cac tcg cag tac tac gac aat ctg cat gtg ctt     384

Asn Lys Trp Ser Ile His Ser Gln Tyr Tyr Asp Asn Leu His Val Leu
        115                 120                 125

tct att tct cgt ggc ggc cag gtg atc tgg atg tcc aac aag gat gca     432
Ser Ile Ser Arg Gly Gly Gln Val Ile Trp Met Ser Asn Lys Asp Ala
    130                 135                 140

gag aaa ctc ggt atc gct gac aac gat tgg atc gag gct tat aac cgc     480
Glu Lys Leu Gly Ile Ala Asp Asn Asp Trp Ile Glu Ala Tyr Asn Arg
145                 150                 155                 160

aac ggc gtt gtt tct gct cgt gcg att gtc tcc cac cgc att cct gaa     528
Asn Gly Val Val Ser Ala Arg Ala Ile Val Ser His Arg Ile Pro Glu
                165                 170                 175

ggc acc gtg ttt atg aac cac gcg cag gaa cgc acc gct ggc acc ccg     576
Gly Thr Val Phe Met Asn His Ala Gln Glu Arg Thr Ala Gly Thr Pro
            180                 185                 190

ctg aac gag aag tct ggc agg cgc ggc gga act cac aac tct ctt act     624
Leu Asn Glu Lys Ser Gly Arg Arg Gly Gly Thr His Asn Ser Leu Thr
        195                 200                 205

cga atc atg att aag ccg gtc cat gtt gcc ggt ggc tac ggc cac tta     672
Arg Ile Met Ile Lys Pro Val His Val Ala Gly Gly Tyr Gly His Leu
    210                 215                 220

acc tat ggc ttc aac tac atc ggc caa ccg gaa ata acc gcg atg agg     720
Thr Tyr Gly Phe Asn Tyr Ile Gly Gln Pro Glu Ile Thr Ala Met Arg
225                 230                 235                 240

tca cca gaa ttc gtc gcc gct ccc agg agg tgc agt act aat gaa ggt     768
Ser Pro Glu Phe Val Ala Ala Pro Arg Arg Cys Ser Thr Asn Glu Gly
                245                 250                 255

cat ggc tca gat cgc aat gat cat gaa ctt gga taagtgcatt ggctgccaca 821
His Gly Ser Asp Arg Asn Asp His Glu Leu Gly
            260                 265

cgt                                                                 824


<210> 284
<211> 267
<212> PRT
<213> Corynebacterium glutamicum

<400> 284
Ile Ala Asp His Glu Gly Thr His Ile Asn Trp Asp Met Val Lys Glu
  1               5                  10                  15

Arg Ser Ala Glu Val Ile Thr Ser Pro Glu Trp Thr Gly Ser Lys Lys
             20                  25                  30

Asp Gly Arg Arg Tyr Thr Ala Phe Ser Ile Asn Ile Glu Tyr Asp Lys
         35                  40                  45

Pro Trp His Thr Leu Ser Gly Arg Met His Tyr Tyr Leu Asp His Asp
     50                  55                  60

Trp Phe Ile Asp Tyr Gly Glu Gln Leu Pro Ile Phe Arg Pro Pro Leu
 65                  70                  75                  80

Asp Lys Ile His Ile Asn Gly Glu Val Gly Pro Gly Gln Ser Val Thr
                 85                  90                  95

Gly Thr Asp Gly Glu Pro Glu Val Thr Val Arg Tyr Leu Thr Thr His
            100                 105                 110

Asn Lys Trp Ser Ile His Ser Gln Tyr Tyr Asp Asn Leu His Val Leu
        115                 120                 125

Ser Ile Ser Arg Gly Gly Gln Val Ile Trp Met Ser Asn Lys Asp Ala
    130                 135                 140

Glu Lys Leu Gly Ile Ala Asp Asn Asp Trp Ile Glu Ala Tyr Asn Arg
145                 150                 155                 160

Asn Gly Val Val Ser Ala Arg Ala Ile Val Ser His Arg Ile Pro Glu
                165                 170                 175

Gly Thr Val Phe Met Asn His Ala Gln Glu Arg Thr Ala Gly Thr Pro
            180                 185                 190

Leu Asn Glu Lys Ser Gly Arg Arg Gly Gly Thr His Asn Ser Leu Thr
        195                 200                 205

Arg Ile Met Ile Lys Pro Val His Val Ala Gly Gly Tyr Gly His Leu
    210                 215                 220

Thr Tyr Gly Phe Asn Tyr Ile Gly Gln Pro Glu Ile Thr Ala Met Arg
225                 230                 235                 240

Ser Pro Glu Phe Val Ala Ala Pro Arg Arg Cys Ser Thr Asn Glu Gly
                245                 250                 255

His Gly Ser Asp Arg Asn Asp His Glu Leu Gly
            260                 265


<210> 285
<211> 692
<212> DNA
<213> Corynebacterium glutamicum

<220>
<221> CDS
<222> (1)..(669)
<223> FRXA01309

<400> 285
att gca gac cac gaa ggt acc cac atc aat tgg gac atg gtc aaa gaa      48
Ile Ala Asp His Glu Gly Thr His Ile Asn Trp Asp Met Val Lys Glu
  1               5                  10                  15

cgt tcc gcc gag gtg atc acc tca ccg gag tgg act ggt tcc aag aag      96
Arg Ser Ala Glu Val Ile Thr Ser Pro Glu Trp Thr Gly Ser Lys Lys
             20                  25                  30

gac gga cgt cgc tac acc gcg ttt tcc atc aac att gaa tac gac aag     144
Asp Gly Arg Arg Tyr Thr Ala Phe Ser Ile Asn Ile Glu Tyr Asp Lys
         35                  40                  45

ccg tgg cac acc ctg tct ggt cgc atg cac tac tac ctc gac cac gat     192
Pro Trp His Thr Leu Ser Gly Arg Met His Tyr Tyr Leu Asp His Asp
     50                  55                  60

tgg ttt att gat tac ggc gag cag ttg cca atc ttt agg cca ccg ttg     240
Trp Phe Ile Asp Tyr Gly Glu Gln Leu Pro Ile Phe Arg Pro Pro Leu
 65                  70                  75                  80

gac aag atc cac atc aat ggt gag gtc ggc cct ggc cag tcg gtc aca     288
Asp Lys Ile His Ile Asn Gly Glu Val Gly Pro Gly Gln Ser Val Thr
                 85                  90                  95

ggc acc gac ggc gaa cca gaa gta acc gtg cgt tat ctg acc acc cac     336
Gly Thr Asp Gly Glu Pro Glu Val Thr Val Arg Tyr Leu Thr Thr His
            100                 105                 110

aac aag tgg tcg att cac tcg cag tac tac gac aat ctg cat gtg ctt     384
Asn Lys Trp Ser Ile His Ser Gln Tyr Tyr Asp Asn Leu His Val Leu
        115                 120                 125

tct att tct cgt ggc ggc cag gtg atc tgg atg tcc aac aag gat gca     432
Ser Ile Ser Arg Gly Gly Gln Val Ile Trp Met Ser Asn Lys Asp Ala
    130                 135                 140

gag aaa ctc ggt atc gct gac aac gat tgg atc gag gct tat aac cgc     480
Glu Lys Leu Gly Ile Ala Asp Asn Asp Trp Ile Glu Ala Tyr Asn Arg
145                 150                 155                 160

aac ggc gtt gtt tct gct cgt gcg att gtc tcc cac cgc att cct gaa     528
Asn Gly Val Val Ser Ala Arg Ala Ile Val Ser His Arg Ile Pro Glu
                165                 170                 175

ggc acc gtg ttt atg aac cac gcg cag gaa ccc acc gct ggc acc ccg     576
Gly Thr Val Phe Met Asn His Ala Gln Glu Pro Thr Ala Gly Thr Pro
            180                 185                 190

ctg aac gag aag tct ggc agg cgc ggc gga act cac aac tct ctt act     624
Leu Asn Glu Lys Ser Gly Arg Arg Gly Gly Thr His Asn Ser Leu Thr
        195                 200                 205

cga atc atg att aaa acg gtc cat gtt gcc ggt ggc tac ggc act         669
Arg Ile Met Ile Lys Thr Val His Val Ala Gly Gly Tyr Gly Thr
    210                 215                 220

taacctatgg cttaactaca tcg                                           692


<210> 286
<211> 223
<212> PRT
<213> Corynebacterium glutamicum

<400> 286
Ile Ala Asp His Glu Gly Thr His Ile Asn Trp Asp Met Val Lys Glu
  1               5                  10                  15

Arg Ser Ala Glu Val Ile Thr Ser Pro Glu Trp Thr Gly Ser Lys Lys
             20                  25                  30

Asp Gly Arg Arg Tyr Thr Ala Phe Ser Ile Asn Ile Glu Tyr Asp Lys
         35                  40                  45

Pro Trp His Thr Leu Ser Gly Arg Met His Tyr Tyr Leu Asp His Asp
     50                  55                  60

Trp Phe Ile Asp Tyr Gly Glu Gln Leu Pro Ile Phe Arg Pro Pro Leu
 65                  70                  75                  80

Asp Lys Ile His Ile Asn Gly Glu Val Gly Pro Gly Gln Ser Val Thr
                 85                  90                  95

Gly Thr Asp Gly Glu Pro Glu Val Thr Val Arg Tyr Leu Thr Thr His
            100                 105                 110

Asn Lys Trp Ser Ile His Ser Gln Tyr Tyr Asp Asn Leu His Val Leu
        115                 120                 125

Ser Ile Ser Arg Gly Gly Gln Val Ile Trp Met Ser Asn Lys Asp Ala
    130                 135                 140

Glu Lys Leu Gly Ile Ala Asp Asn Asp Trp Ile Glu Ala Tyr Asn Arg
145                 150                 155                 160

Asn Gly Val Val Ser Ala Arg Ala Ile Val Ser His Arg Ile Pro Glu
                165                 170                 175

Gly Thr Val Phe Met Asn His Ala Gln Glu Pro Thr Ala Gly Thr Pro
            180                 185                 190

Leu Asn Glu Lys Ser Gly Arg Arg Gly Gly Thr His Asn Ser Leu Thr
        195                 200                 205

Arg Ile Met Ile Lys Thr Val His Val Ala Gly Gly Tyr Gly Thr
    210                 215                 220


<210> 287
<211> 807
<212> DNA
<213> Corynebacterium glutamicum

<220>
<221> CDS
<222> (101)..(784)
<223> RXA02017

<400> 287
gcctgtggca cgggagctcc cagcggcaag gtgagtctga cctcgtggaa cgttggcgaa 60

cgccccgctg cgatgttccc accaaggaag gactaggcgg atg cgc acc cat act 115
Met Arg Thr His Thr
1               5

ggc aaa att ccg gat cac ttt gtg cct cgc atc tcc atg acg gag gag 163
Gly Lys Ile Pro Asp His Phe Val Pro Arg Ile Ser Met Thr Glu Glu
                10                  15                  20

cag cgg cgc gtg gtg ttc atg ctg aat agt ttg ctg ttg gat tat cca 211
Gln Arg Arg Val Val Phe Met Leu Asn Ser Leu Leu Leu Asp Tyr Pro
            25                  30                  35

gag gag gga ttc gtc gac aag cta aat gcc gtc gag gcg cag ctt gat 259
Glu Glu Gly Phe Val Asp Lys Leu Asn Ala Val Glu Ala Gln Leu Asp
        40                  45                  50

gtc ctt ccg ctc ccc gtc gcg gcg cac gtg gtc gag ttc ctt gac gcg 307
Val Leu Pro Leu Pro Val Ala Ala His Val Val Glu Phe Leu Asp Ala
    55                  60                  65

gca cgc gtc gct ggg cta cgc gcc atg cag gaa gcc tac gtt gag acc 355
Ala Arg Val Ala Gly Leu Arg Ala Met Gln Glu Ala Tyr Val Glu Thr
70                  75                  80                  85

ttt gac cag cgc cga cgc tgc tca ctg ttt ctc acc tac tac gct gtg 403
Phe Asp Gln Arg Arg Arg Cys Ser Leu Phe Leu Thr Tyr Tyr Ala Val
                90                  95                  100

ggc gac acc cgg cag cgc ggc acg gcg atc ctc acc ttc cgt caa acg 451
Gly Asp Thr Arg Gln Arg Gly Thr Ala Ile Leu Thr Phe Arg Gln Thr
            105                 110                 115

ctg caa cag ctc gga ttt gaa tcc gag cgc gac gaa ttg ccc gac cac 499
Leu Gln Gln Leu Gly Phe Glu Ser Glu Arg Asp Glu Leu Pro Asp His
        120                 125                 130

ctc tgc gtc gtg ctt gag gcc gca gcg ctt gct gat tct tcg ctt ttc 547
Leu Cys Val Val Leu Glu Ala Ala Ala Leu Ala Asp Ser Ser Leu Phe
    135                 140                 145

gac gcc gcc acc cag gtg tta tca gct cac cgc gac ggc atc gaa gtg 595
Asp Ala Ala Thr Gln Val Leu Ser Ala His Arg Asp Gly Ile Glu Val
150                 155                 160                 165

ttg cgc gca gcc ctc gac aac ctc gac tcg ccc tac aga tac ctg atc 643
Leu Arg Ala Ala Leu Asp Asn Leu Asp Ser Pro Tyr Arg Tyr Leu Ile
                170                 175                 180

atg tct ttg tgc cag gca ttg cca gaa atc gat gaa gaa acc gcc aac 691
Met Ser Leu Cys Gln Ala Leu Pro Glu Ile Asp Glu Glu Thr Ala Asn
            185                 190                 195

agc tac atg gag ctc atc cgc agc ggt cca cca gca gaa atg gtg ggc 739
Ser Tyr Met Glu Leu Ile Arg Ser Gly Pro Pro Ala Glu Met Val Gly
        200                 205                 210

atc ggc acg cct cta ccg ttc ccc acc tca caa ccg gac att cac 784
Ile Gly Thr Pro Leu Pro Phe Pro Thr Ser Gln Pro Asp Ile His
    215                 220                 225

taggacacac tatgtcaaac ttt 807

<210> 288
<211> 228
<212> PRT
<213> Corynebacterium glutamicum

<400> 288
Met Arg Thr His Thr Gly Lys Ile Pro Asp His Phe Val Pro Arg Ile
1               5                   10                  15

Ser Met Thr Glu Glu Gln Arg Arg Val Val Phe Met Leu Asn Ser Leu
            20                  25                  30

Leu Leu Asp Tyr Pro Glu Glu Gly Phe Val Asp Lys Leu Asn Ala Val

35                  40                  45

Glu Ala Gln Leu Asp Val Leu Pro Leu Pro Val Ala Ala His Val Val
    50                  55                  60

Glu Phe Leu Asp Ala Ala Arg Val Ala Gly Leu Arg Ala Met Gln Glu
65                  70                  75                  80

Ala Tyr Val Glu Thr Phe Asp Gln Arg Arg Arg Cys Ser Leu Phe Leu
                85                  90                  95

Thr Tyr Tyr Ala Val Gly Asp Thr Arg Gln Arg Gly Thr Ala Ile Leu
            100                 105                 110

Thr Phe Arg Gln Thr Leu Gln Gln Leu Gly Phe Glu Ser Glu Arg Asp
        115                 120                 125

Glu Leu Pro Asp His Leu Cys Val Val Leu Glu Ala Ala Ala Leu Ala
    130                 135                 140

Asp Ser Ser Leu Phe Asp Ala Ala Thr Gln Val Leu Ser Ala His Arg
145                 150                 155                 160

Asp Gly Ile Glu Val Leu Arg Ala Ala Leu Asp Asn Leu Asp Ser Pro
                165                 170                 175

Tyr Arg Tyr Leu Ile Met Ser Leu Cys Gln Ala Leu Pro Glu Ile Asp
            180                 185                 190

Glu Glu Thr Ala Asn Ser Tyr Met Glu Leu Ile Arg Ser Gly Pro Pro
        195                 200                 205

Ala Glu Met Val Gly Ile Gly Thr Pro Leu Pro Phe Pro Thr Ser Gln
    210                 215                 220

Pro Asp Ile His
225


<210> 289
<211> 1073
<212> DNA
<213> Corynebacterium glutamicum

<220>
<221> CDS
<222> (1)..(1050)
<223> RXA02018

<400> 289
cgc atc tgc gag cac tgc ctc aac ccc acc tgt gtg tcc tcc tgc cca      48
Arg Ile Cys Glu His Cys Leu Asn Pro Thr Cys Val Ser Ser Cys Pro
1               5                   10                  15

tcc ggt gct atg tac aaa cgc gcc gaa gac ggc atc gtg ctg gtt gac      96
Ser Gly Ala Met Tyr Lys Arg Ala Glu Asp Gly Ile Val Leu Val Asp
            20                  25                  30

cag gat caa tgc cgt ggc tgg cgc atg tgt gtt tcc ggc tgc ccc tac      144
Gln Asp Gln Cys Arg Gly Trp Arg Met Cys Val Ser Gly Cys Pro Tyr
        35                  40                  45

aaa aag gtc tac ttc aat cac aaa tcg ggc aag gcc gaa aag tgt acg      192
Lys Lys Val Tyr Phe Asn His Lys Ser Gly Lys Ala Glu Lys Cys Thr
    50                  55                  60

ctg tgc tat ccg cgc ctc gag gtc ggc cag ccg acc gtg tgc tcc gag      240
Leu Cys Tyr Pro Arg Leu Glu Val Gly Gln Pro Thr Val Cys Ser Glu
65                  70                  75                  80

acg tgc gtg ggt cgc ttg cgc tac ttg ggc gtt ttg ctt tac gac gcc      288
Thr Cys Val Gly Arg Leu Arg Tyr Leu Gly Val Leu Leu Tyr Asp Ala
                85                  90                  95

gac cgt gtc gct gaa gtc gcc gcc acg cca gac gaa aag gat ctt ttc      336
Asp Arg Val Ala Glu Val Ala Ala Thr Pro Asp Glu Lys Asp Leu Phe
            100                 105                 110

gaa gcc caa aag acc ctc ttc cta gat ccc cac gac cca cag gtg atc     384
Glu Ala Gln Lys Thr Leu Phe Leu Asp Pro His Asp Pro Gln Val Ile
        115                 120                 125

gcc gac gcc caa cgc aac ggc atc ccg cac tcc tgg ctc gaa gct gcg     432
Ala Asp Ala Gln Arg Asn Gly Ile Pro His Ser Trp Leu Glu Ala Ala
    130                 135                 140

cag aac tct cca att tac gat ctc atc ttc aaa tac gag gtt gcc ctc     480
Gln Asn Ser Pro Ile Tyr Asp Leu Ile Phe Lys Tyr Glu Val Ala Leu
145                 150                 155                 160

ccg ctt cac cct gaa tac cgc acc ttg ccg atg gtt tgg tac att ccg     528
Pro Leu His Pro Glu Tyr Arg Thr Leu Pro Met Val Trp Tyr Ile Pro
                165                 170                 175

cca cta agc ccc atc gtt gat gag gtg acc gcg tcc ggc aac gac ggc     576
Pro Leu Ser Pro Ile Val Asp Glu Val Thr Ala Ser Gly Asn Asp Gly
            180                 185                 190

gaa gac cac aag atc ctg ctc acc gcg ctg tcc acc atg cgc atc ccg     624
Glu Asp His Lys Ile Leu Leu Thr Ala Leu Ser Thr Met Arg Ile Pro
        195                 200                 205

ctg gaa tac ctg gct gga ttg ttc act gcc ggt gat acc agg ccg gtg     672
Leu Glu Tyr Leu Ala Gly Leu Phe Thr Ala Gly Asp Thr Arg Pro Val
    210                 215                 220

gaa aaa tcc ctc cga cgc cta gcc gcc atg cga tca tat atg cgc gat     720
Glu Lys Ser Leu Arg Arg Leu Ala Ala Met Arg Ser Tyr Met Arg Asp
225                 230                 235                 240

atc agt ttg ggc cgc gaa cct cag gaa gaa atc gca gag gct gtc gga     768
Ile Ser Leu Gly Arg Glu Pro Gln Glu Glu Ile Ala Glu Ala Val Gly
                245                 250                 255

atg acc ggc aag gtg gtg cag gaa atg tat cgc atc ctg gcc att gcc     816
Met Thr Gly Lys Val Val Gln Glu Met Tyr Arg Ile Leu Ala Ile Ala
            260                 265                 270

aag tat gac gat cgc tat gtc atc ccc acc gcc tcc cct gag acc ccg     864
Lys Tyr Asp Asp Arg Tyr Val Ile Pro Thr Ala Ser Pro Glu Thr Pro
        275                 280                 285

cgc gga att tct tcc ctg gat cct ttc ggc gat gtc gat cca gcc cga     912
Arg Gly Ile Ser Ser Leu Asp Pro Phe Gly Asp Val Asp Pro Ala Arg
    290                 295                 300

gcc acc gag cag ctc aac atc ggt ttg ggc gaa ggc gct cca gag gcc     960
Ala Thr Glu Gln Leu Asn Ile Gly Leu Gly Glu Gly Ala Pro Glu Ala
305                 310                 315                 320

tgt ggc acg gga gct ccc agc ggc aag gtg agt ctg acc tcg tgg aac    1008
Cys Gly Thr Gly Ala Pro Ser Gly Lys Val Ser Leu Thr Ser Trp Asn
                325                 330                 335

gtt ggc gaa cgc ccc gct gcg atg ttc cca cca agg aag gac            1050
Val Gly Glu Arg Pro Ala Ala Met Phe Pro Pro Arg Lys Asp
            340                 345                 350

taggcggatg cgcacccata ctg                                          1073

<210> 290
<211> 350
<212> PRT
<213> Corynebacterium glutamicum

<400> 290
Arg Ile Cys Glu His Cys Leu Asn Pro Thr Cys Val Ser Ser Cys Pro
  1               5                  10                  15

Ser Gly Ala Met Tyr Lys Arg Ala Glu Asp Gly Ile Val Leu Val Asp
             20                  25                  30

Gln Asp Gln Cys Arg Gly Trp Arg Met Cys Val Ser Gly Cys Pro Tyr

35                  40                  45

Lys Lys Val Tyr Phe Asn His Lys Ser Gly Lys Ala Glu Lys Cys Thr
    50                  55                  60

Leu Cys Tyr Pro Arg Leu Glu Val Gly Gln Pro Thr Val Cys Ser Glu
65                  70                  75                  80

Thr Cys Val Gly Arg Leu Arg Tyr Leu Gly Val Leu Leu Tyr Asp Ala
                85                  90                  95

Asp Arg Val Ala Glu Val Ala Ala Thr Pro Asp Glu Lys Asp Leu Phe
            100                 105                 110

Glu Ala Gln Lys Thr Leu Phe Leu Asp Pro His Asp Pro Gln Val Ile
        115                 120                 125

Ala Asp Ala Gln Arg Asn Gly Ile Pro His Ser Trp Leu Glu Ala Ala
    130                 135                 140

Gln Asn Ser Pro Ile Tyr Asp Leu Ile Phe Lys Tyr Glu Val Ala Leu
145                 150                 155                 160

Pro Leu His Pro Glu Tyr Arg Thr Leu Pro Met Val Trp Tyr Ile Pro
                165                 170                 175

Pro Leu Ser Pro Ile Val Asp Glu Val Thr Ala Ser Gly Asn Asp Gly
            180                 185                 190

Glu Asp His Lys Ile Leu Leu Thr Ala Leu Ser Thr Met Arg Ile Pro
        195                 200                 205

Leu Glu Tyr Leu Ala Gly Leu Phe Thr Ala Gly Asp Thr Arg Pro Val
    210                 215                 220

Glu Lys Ser Leu Arg Arg Leu Ala Ala Met Arg Ser Tyr Met Arg Asp
225                 230                 235                 240

Ile Ser Leu Gly Arg Glu Pro Gln Glu Glu Ile Ala Glu Ala Val Gly
                245                 250                 255

Met Thr Gly Lys Val Val Gln Glu Met Tyr Arg Ile Leu Ala Ile Ala
            260                 265                 270

Lys Tyr Asp Asp Arg Tyr Val Ile Pro Thr Ala Ser Pro Glu Thr Pro
        275                 280                 285

Arg Gly Ile Ser Ser Leu Asp Pro Phe Gly Asp Val Asp Pro Ala Arg
    290                 295                 300

Ala Thr Glu Gln Leu Asn Ile Gly Leu Gly Glu Gly Ala Pro Glu Ala
305                 310                 315                 320

Cys Gly Thr Gly Ala Pro Ser Gly Lys Val Ser Leu Thr Ser Trp Asn
                325                 330                 335

Val Gly Glu Arg Pro Ala Ala Met Phe Pro Pro Arg Lys Asp
            340                 345                 350


<210> 291
<211> 900
<212> DNA
<213> Corynebacterium glutamicum

<220>
<221> CDS
<222> (101)..(877)
<223> RXA02016

<400> 291
acatggagct catccgcagc ggtccaccag cagaaatggt gggcatcggc acgcctctac 60

cgttccccac ctcacaaccg gacattcact aggacacact atg tca aac ttt gaa   115
                                           Met Ser Asn Phe Glu
                                           1               5

acg ttc ctc tgg gtt gcc tac ccc tgg ctg tgt atc gcc gcc tac atc     163
Thr Phe Leu Trp Val Ala Tyr Pro Trp Leu Cys Ile Ala Ala Tyr Ile
                10                  15                  20

atc ggc att tct tgg cgc tgg cgc gcc gac caa ttc ggt tgg acc acc     211
Ile Gly Ile Ser Trp Arg Trp Arg Ala Asp Gln Phe Gly Trp Thr Thr
                25                  30                  35

cac tcc tcc caa atc tac gaa tcc aaa ctc ctc cgc atc gcc tcc cca     259
His Ser Ser Gln Ile Tyr Glu Ser Lys Leu Leu Arg Ile Ala Ser Pro
        40                  45                  50

ctc ttc cac tgg ggc atg gtg ttc gtg gtg atc ggc cac ctc atg gga     307
Leu Phe His Trp Gly Met Val Phe Val Val Ile Gly His Leu Met Gly
    55                  60                  65

ctt gcc atc ccc aag agc tgg acc caa gct gta gga att tct gac gcc     355
Leu Ala Ile Pro Lys Ser Trp Thr Gln Ala Val Gly Ile Ser Asp Ala
 70                  75                  80                  85

gct tac cac ctc atc gcc acc atc cca ggc acc att gcc ggc atc gct     403
Ala Tyr His Leu Ile Ala Thr Ile Pro Gly Thr Ile Ala Gly Ile Ala
                90                  95                 100

gca gtc ctt gga ctc atc ggc ttg att atc cgt cgc gtg atc aac aaa     451
Ala Val Leu Gly Leu Ile Gly Leu Ile Ile Arg Arg Val Ile Asn Lys
            105                 110                 115

acc gtc ttc ctg tcc acc tca cgc tcc gac aaa gtg atg tat gtg cta     499
Thr Val Phe Leu Ser Thr Ser Arg Ser Asp Lys Val Met Tyr Val Leu
        120                 125                 130

ctc ggc gct gca att ttg tcc ggt ttc atc gcc acc gtc tcc acc cag     547
Leu Gly Ala Ala Ile Leu Ser Gly Phe Ile Ala Thr Val Ser Thr Gln
    135                 140                 145

gtc ttc ggc ggc gca cac ggc tac gac tac cgc gaa acc atc tcc cca     595
Val Phe Gly Gly Ala His Gly Tyr Asp Tyr Arg Glu Thr Ile Ser Pro
150                 155                 160                 165

tgg gtg cgc caa ctg ctc atc ttc aac gct caa cca gag ctc atg gct     643
Trp Val Arg Gln Leu Leu Ile Phe Asn Ala Gln Pro Glu Leu Met Ala
                170                 175                 180

gat gtc cct tgg gaa ttc aag gtc cac atc gtc gct gga ttc acc ctc     691
Asp Val Pro Trp Glu Phe Lys Val His Ile Val Ala Gly Phe Thr Leu
            185                 190                 195

atc gca ctg tgg cca ttc acc cgc cta gtc cac gcg ttc tcc gca cca     739
Ile Ala Leu Trp Pro Phe Thr Arg Leu Val His Ala Phe Ser Ala Pro
        200                 205                 210

gtt gga tac gtc acc cgc ccc tac gtg gtc tat cgc acc cgc gac acc     787
Val Gly Tyr Val Thr Arg Pro Tyr Val Val Tyr Arg Thr Arg Asp Thr
    215                 220                 225

acc tct gaa ccg gca cgc caa aac gtc gcc tgg gaa ccg atc cgc tcg     835
Thr Ser Glu Pro Ala Arg Gln Asn Val Ala Trp Glu Pro Ile Arg Ser
230                 235                 240                 245

gtc aaa aat cag ctc gac aat gac tcg aaa tgg cac ggc gcc             877
Val Lys Asn Gln Leu Asp Asn Asp Ser Lys Trp His Gly Ala
                250                 255

taaattcctc acaagccccc tag                                           900

<210> 292
<211> 259
<212> PRT
<213> Corynebacterium glutamicum

<400> 292
Met Ser Asn Phe Glu Thr Phe Leu Trp Val Ala Tyr Pro Trp Leu Cys
  1               5                  10                  15

Ile Ala Ala Tyr Ile Ile Gly Ile Ser Trp Arg Trp Arg Ala Asp Gln

20                  25                  30

Phe Gly Trp Thr Thr His Ser Ser Gln Ile Tyr Glu Ser Lys Leu Leu
            35                  40                  45

Arg Ile Ala Ser Pro Leu Phe His Trp Gly Met Val Phe Val Val Ile
    50                  55                  60

Gly His Leu Met Gly Leu Ala Ile Pro Lys Ser Trp Thr Gln Ala Val
65                  70                  75                  80

Gly Ile Ser Asp Ala Ala Tyr His Leu Ile Ala Thr Ile Pro Gly Thr
                85                  90                  95

Ile Ala Gly Ile Ala Ala Val Leu Gly Leu Ile Gly Leu Ile Ile Arg
            100                 105                 110

Arg Val Ile Asn Lys Thr Val Phe Leu Ser Thr Ser Arg Ser Asp Lys
        115                 120                 125

Val Met Tyr Val Leu Leu Gly Ala Ala Ile Leu Ser Gly Phe Ile Ala
    130                 135                 140

Thr Val Ser Thr Gln Val Phe Gly Gly Ala His Gly Tyr Asp Tyr Arg
145                 150                 155                 160

Glu Thr Ile Ser Pro Trp Val Arg Gln Leu Leu Ile Phe Asn Ala Gln
                165                 170                 175

Pro Glu Leu Met Ala Asp Val Pro Trp Glu Phe Lys Val His Ile Val
            180                 185                 190

Ala Gly Phe Thr Leu Ile Ala Leu Trp Pro Phe Thr Arg Leu Val His
        195                 200                 205

Ala Phe Ser Ala Pro Val Gly Tyr Val Thr Arg Pro Tyr Val Val Tyr
    210                 215                 220

Arg Thr Arg Asp Thr Thr Ser Glu Pro Ala Arg Gln Asn Val Ala Trp
225                 230                 235                 240

Glu Pro Ile Arg Ser Val Lys Asn Gln Leu Asp Asn Asp Ser Lys Trp
                245                 250                 255

His Gly Ala

<210> 293
<211> 813
<212> DNA
<213> Corynebacterium glutamicum

<220>
<221> CDS
<222> (101)..(790)
<223> RXA00471

<400> 293
acgcatcaaa tctgaaatcg gcgaaggcac cgaagtggca atcaccatgg atgtgtagtt 60

ggtcgtacgc gcgtgtcttc ggggctgtaa cctgaaaggc atg gtt gat gtg ttt 115
                                            Met Val Asp Val Phe
                                            1               5

ttg gtc gat gac cac tcc gtg ttt cgc tcc ggc gtc aaa gca gaa cta 163
Leu Val Asp Asp His Ser Val Phe Arg Ser Gly Val Lys Ala Glu Leu
                10                  15                  20

ggc aac gcc gtc aca gta gtc ggc gaa gca ggg acg gtg gcc gac gcc 211
Gly Asn Ala Val Thr Val Val Gly Glu Ala Gly Thr Val Ala Asp Ala
            25                  30                  35

gta gcc ggc atc aag gca agc aaa cca gag gta gtg ctt ctc gac gtc 259
Val Ala Gly Ile Lys Ala Ser Lys Pro Glu Val Val Leu Leu Asp Val
        40                  45                  50

cac atg ccc gac ggc ggc ggc ctc gca gtg ctc cag cag atc aac gac     307
His Met Pro Asp Gly Gly Gly Leu Ala Val Leu Gln Gln Ile Asn Asp
     55                  60                  65

tcc gat gtg gac acc att ttc ttg gca ctc agt gtc tct gat gct gcg     355
Ser Asp Val Asp Thr Ile Phe Leu Ala Leu Ser Val Ser Asp Ala Ala
 70                  75                  80                  85

gaa gat gtc atc gcc atc atc cgt ggc ggt gcc agg gga tac gtg acc     403
Glu Asp Val Ile Ala Ile Ile Arg Gly Gly Ala Arg Gly Tyr Val Thr
                 90                  95                 100

aaa tca atc tcc ggt gaa gaa ctc atc gaa gcc atc aac cgc gtg aaa     451
Lys Ser Ile Ser Gly Glu Glu Leu Ile Glu Ala Ile Asn Arg Val Lys
            105                 110                 115

tcc ggc gac gca ttc ttc tca cca cgc ctg gca ggc ttt gtc ctc gac     499
Ser Gly Asp Ala Phe Phe Ser Pro Arg Leu Ala Gly Phe Val Leu Asp
        120                 125                 130

gcc ttc gcc gcc ccc gat tcc gca gct ggc gca ggc att gtc gac gca     547
Ala Phe Ala Ala Pro Asp Ser Ala Ala Gly Ala Gly Ile Val Asp Ala
    135                 140                 145

ccc gaa aaa gac gcc gcc gta gaa tcc gga aaa atc ctc gac gac cca     595
Pro Glu Lys Asp Ala Ala Val Glu Ser Gly Lys Ile Leu Asp Asp Pro
150                 155                 160                 165

gtt gtc gac gcc ctc acc cgc cgc gaa ctc gaa gtc ctc cgc cta cta     643
Val Val Asp Ala Leu Thr Arg Arg Glu Leu Glu Val Leu Arg Leu Leu
                170                 175                 180

gcc cgc ggc tac acc tac aaa gaa atc ggc aaa gaa ctg ttc att tcc     691
Ala Arg Gly Tyr Thr Tyr Lys Glu Ile Gly Lys Glu Leu Phe Ile Ser
            185                 190                 195

gtc aaa acc gtg gaa acc cac gcc tca aac att ctg cgg aaa acc caa     739
Val Lys Thr Val Glu Thr His Ala Ser Asn Ile Leu Arg Lys Thr Gln
        200                 205                 210

caa tcc aac cgc cac gcg ttg acc cgg tgg gct cac tcg agg gat ctt     787
Gln Ser Asn Arg His Ala Leu Thr Arg Trp Ala His Ser Arg Asp Leu
    215                 220                 225

gac taatggcggc taaaaagagt ggc                                       813
Asp
230


<210> 294
<211> 230
<212> PRT
<213> Corynebacterium glutamicum

<400> 294
Met Val Asp Val Phe Leu Val Asp Asp His Ser Val Phe Arg Ser Gly
  1               5                  10                  15

Val Lys Ala Glu Leu Gly Asn Ala Val Thr Val Val Gly Glu Ala Gly
             20                  25                  30

Thr Val Ala Asp Ala Val Ala Gly Ile Lys Ala Ser Lys Pro Glu Val
         35                  40                  45

Val Leu Leu Asp Val His Met Pro Asp Gly Gly Gly Leu Ala Val Leu
     50                  55                  60

Gln Gln Ile Asn Asp Ser Asp Val Asp Thr Ile Phe Leu Ala Leu Ser
 65                  70                  75                  80

Val Ser Asp Ala Ala Glu Asp Val Ile Ala Ile Ile Arg Gly Gly Ala
                 85                  90                  95

Arg Gly Tyr Val Thr Lys Ser Ile Ser Gly Glu Glu Leu Ile Glu Ala
            100                 105                 110

Ile Asn Arg Val Lys Ser Gly Asp Ala Phe Phe Ser Pro Arg Leu Ala

115                 120                 125

Gly Phe Val Leu Asp Ala Phe Ala Ala Pro Asp Ser Ala Ala Gly Ala
    130                 135                 140

Gly Ile Val Asp Ala Pro Glu Lys Asp Ala Ala Val Glu Ser Gly Lys
145                 150                 155                 160

Ile Leu Asp Asp Pro Val Val Asp Ala Leu Thr Arg Arg Glu Leu Glu
                165                 170                 175

Val Leu Arg Leu Leu Ala Arg Gly Tyr Thr Tyr Lys Glu Ile Gly Lys
            180                 185                 190

Glu Leu Phe Ile Ser Val Lys Thr Val Glu Thr His Ala Ser Asn Ile
        195                 200                 205

Leu Arg Lys Thr Gln Gln Ser Asn Arg His Ala Leu Thr Arg Trp Ala
    210                 215                 220

His Ser Arg Asp Leu Asp
225                 230


<210> 295
<211> 936
<212> DNA
<213> Corynebacterium glutamicum

<220>
<221> CDS
<222> (101)..(913)
<223> RXA00133

<400> 295
gttacatcag atgaggatgc cctatgggtg tacacatgcg acgggtgtat tgcaggagga 60

aatttgaagg tggataccca gcggattaaa gatgatgaag atg cta ttc gtt cgg    115
                                           Met Leu Phe Val Arg
                                           1               5

cgg ctg aca tcg ctg aaa acc gca aca ggc atc cca gtc acc atg ttc    163
Arg Leu Thr Ser Leu Lys Thr Ala Thr Gly Ile Pro Val Thr Met Phe
                10                  15                  20

gcc act gtg ttg cag gac aat cgc ctg caa att act cag tgg gtt ggg    211
Ala Thr Val Leu Gln Asp Asn Arg Leu Gln Ile Thr Gln Trp Val Gly
            25                  30                  35

ttg cgt acc ccg gct ctg cag aat ctg gtc att gaa cca ggt gtg ggc    259
Leu Arg Thr Pro Ala Leu Gln Asn Leu Val Ile Glu Pro Gly Val Gly
        40                  45                  50

gtt ggt gga cgc gtc gtc gca acc cgt cgt ccg gtt ggt gtg agt gat    307
Val Gly Gly Arg Val Val Ala Thr Arg Arg Pro Val Gly Val Ser Asp
    55                  60                  65

tac acc agg gca aat gtc att tca cat gag aag gat tcc gcg att cag    355
Tyr Thr Arg Ala Asn Val Ile Ser His Glu Lys Asp Ser Ala Ile Gln
70                  75                  80                  85

gat gag ggc ctt cat tcc att gtc gca gtt ccc gtg atc gtg cac cgc    403
Asp Glu Gly Leu His Ser Ile Val Ala Val Pro Val Ile Val His Arg
                90                  95                  100

gaa att cgt ggc gtt ttg tat gtt ggc gtt cac tct gcg gtg cgt ctc    451
Glu Ile Arg Gly Val Leu Tyr Val Gly Val His Ser Ala Val Arg Leu
            105                 110                 115

ggc gac act gtt att gaa gaa gtc acc atg act gcg cgc acg ttg gaa    499
Gly Asp Thr Val Ile Glu Glu Val Thr Met Thr Ala Arg Thr Leu Glu
        120                 125                 130

caa aac ctg gcg atc aac tcc gcg ctt cgc cgc aat ggc gtt cct gat    547
Gln Asn Leu Ala Ile Asn Ser Ala Leu Arg Arg Asn Gly Val Pro Asp
    135                 140                 145

ggt cgc ggt tcc ctc aaa gct aac cgc gtg atg aat ggg gcg gag tgg 595
Gly Arg Gly Ser Leu Lys Ala Asn Arg Val Met Asn Gly Ala Glu Trp
150                 155                 160                 165

gag cag gtt cgt tcc act cat tcc aag ctg cgc atg ctg gca aat cgt 643
Glu Gln Val Arg Ser Thr His Ser Lys Leu Arg Met Leu Ala Asn Arg
                170                 175                 180

gtg acc gat gag gat ctg cgc cgc gat ttg gaa gag ctt tgc gat cag 691
Val Thr Asp Glu Asp Leu Arg Arg Asp Leu Glu Glu Leu Cys Asp Gln
            185                 190                 195

atg gtc acc cca gtc cgc atc aag cag acc acc aag ctg tcc gcg cgt 739
Met Val Thr Pro Val Arg Ile Lys Gln Thr Thr Lys Leu Ser Ala Arg
        200                 205                 210

gag ttg gac gtg ctg gct tgt gtc gcg ctc ggt cac acc aac gtc gaa 787
Glu Leu Asp Val Leu Ala Cys Val Ala Leu Gly His Thr Asn Val Glu
    215                 220                 225

gct gct gaa gag atg ggc atc ggc gcg gaa acc gtc aag agc tac ctg 835
Ala Ala Glu Glu Met Gly Ile Gly Ala Glu Thr Val Lys Ser Tyr Leu
230                 235                 240                 245

cgc tcg gtc atg cgc aag ctc ggc gcc cac acg cgc tac gag gca gtc 883
Arg Ser Val Met Arg Lys Leu Gly Ala His Thr Arg Tyr Glu Ala Val
                250                 255                 260

aac gca gca cgc cgg atc ggc gca ctg cct taaaaagatt ttgctttacg 933
Asn Ala Ala Arg Arg Ile Gly Ala Leu Pro
            265                 270

acg 936

<210> 296
<211> 271
<212> PRT
<213> Corynebacterium glutamicum

<400> 296
Met Leu Phe Val Arg Arg Leu Thr Ser Leu Lys Thr Ala Thr Gly Ile
1               5                   10                  15

Pro Val Thr Met Phe Ala Thr Val Leu Gln Asp Asn Arg Leu Gln Ile
            20                  25                  30

Thr Gln Trp Val Gly Leu Arg Thr Pro Ala Leu Gln Asn Leu Val Ile
        35                  40                  45

Glu Pro Gly Val Gly Val Gly Gly Arg Val Val Ala Thr Arg Arg Pro
    50                  55                  60

Val Gly Val Ser Asp Tyr Thr Arg Ala Asn Val Ile Ser His Glu Lys
65                  70                  75                  80

Asp Ser Ala Ile Gln Asp Glu Gly Leu His Ser Ile Val Ala Val Pro
                85                  90                  95

Val Ile Val His Arg Glu Ile Arg Gly Val Leu Tyr Val Gly Val His
            100                 105                 110

Ser Ala Val Arg Leu Gly Asp Thr Val Ile Glu Glu Val Thr Met Thr
        115                 120                 125

Ala Arg Thr Leu Glu Gln Asn Leu Ala Ile Asn Ser Ala Leu Arg Arg
    130                 135                 140

Asn Gly Val Pro Asp Gly Arg Gly Ser Leu Lys Ala Asn Arg Val Met
145                 150                 155                 160

Asn Gly Ala Glu Trp Glu Gln Val Arg Ser Thr His Ser Lys Leu Arg
                165                 170                 175

Met Leu Ala Asn Arg Val Thr Asp Glu Asp Leu Arg Arg Asp Leu Glu
            180                 185                 190

Glu Leu Cys Asp Gln Met Val Thr Pro Val Arg Ile Lys Gln Thr Thr
        195                 200                 205

Lys Leu Ser Ala Arg Glu Leu Asp Val Leu Ala Cys Val Ala Leu Gly
    210                 215                 220

His Thr Asn Val Glu Ala Ala Glu Glu Met Gly Ile Gly Ala Glu Thr
225                 230                 235                 240

Val Lys Ser Tyr Leu Arg Ser Val Met Arg Lys Leu Gly Ala His Thr
                245                 250                 255

Arg Tyr Glu Ala Val Asn Ala Ala Arg Arg Ile Gly Ala Leu Pro
            260                 265                 270


<210> 297
<211> 759
<212> DNA
<213> Corynebacterium glutamicum

<220>
<221> CDS
<222> (101)..(736)
<223> RXA00650

<400> 297
aaggctagac taaagtacga ttcatctgct catcgatact cttgaaggcg cattttcatt 60

cgaaacgaag tgcgccattg ggaaggacct agttcaaaca atg att cgc gtg ctg   115
                                           Met Ile Arg Val Leu
                                           1               5

ctt gct gat gac cac gaa atc gtg agg ctc gga ctc cga gct gtg ctg   163
Leu Ala Asp Asp His Glu Ile Val Arg Leu Gly Leu Arg Ala Val Leu
                10                  15                  20

gaa agc gcc gag gac att gaa gtg gtg ggc gaa gtc tcc acc gcc gaa   211
Glu Ser Ala Glu Asp Ile Glu Val Val Gly Glu Val Ser Thr Ala Glu
            25                  30                  35

ggt gcg gtg cag gca gcc caa gaa ggc gga atc gac gtc atc ttg atg   259
Gly Ala Val Gln Ala Ala Gln Glu Gly Gly Ile Asp Val Ile Leu Met
        40                  45                  50

gac ctc cga ttc ggc ccc ggc gtc caa gga acc cag gtt tcc aca ggc   307
Asp Leu Arg Phe Gly Pro Gly Val Gln Gly Thr Gln Val Ser Thr Gly
    55                  60                  65

gca gac gcc acc gca gcc atc aag cga aac atc gat aac ccg cca aaa   355
Ala Asp Ala Thr Ala Ala Ile Lys Arg Asn Ile Asp Asn Pro Pro Lys
70                  75                  80                  85

gtc ctg gtc gtg acc aac tac gac acc gac aca gac atc ctc ggc gca   403
Val Leu Val Val Thr Asn Tyr Asp Thr Asp Thr Asp Ile Leu Gly Ala
                90                  95                  100

atc gaa gcc ggc gca ctg ggc tac ctg ctc aaa gac gcc cca ccg agc   451
Ile Glu Ala Gly Ala Leu Gly Tyr Leu Leu Lys Asp Ala Pro Pro Ser
            105                 110                 115

gaa ctc ctg gca gca gta cga tcc gca gca gaa ggt gac tcc aca ctg   499
Glu Leu Leu Ala Ala Val Arg Ser Ala Ala Glu Gly Asp Ser Thr Leu
        120                 125                 130

tca ccc atg gtt gcg aac cgc ctg atg act cgc gtg cgc acc ccc aaa   547
Ser Pro Met Val Ala Asn Arg Leu Met Thr Arg Val Arg Thr Pro Lys
    135                 140                 145

acc tca ctc acc cca cgt gaa ctg gaa gtt ctc aag ctg gtt gcc ggt   595
Thr Ser Leu Thr Pro Arg Glu Leu Glu Val Leu Lys Leu Val Ala Gly
150                 155                 160                 165

gga tcc tcc aac cgc gac att ggc cgt atc ctc ttc ctc tca gaa gcc   643
Gly Ser Ser Asn Arg Asp Ile Gly Arg Ile Leu Phe Leu Ser Glu Ala
                170                 175                 180

acg gtg aaa tcc cac ctc gtg cac atc tac gac aag ctc ggc gtg cgg     691
Thr Val Lys Ser His Leu Val His Ile Tyr Asp Lys Leu Gly Val Arg
            185                 190                 195

tca cgt acc tcc gct gtc gca gcc gca cgt gag cag ggg ctg ctg         736
Ser Arg Thr Ser Ala Val Ala Ala Ala Arg Glu Gln Gly Leu Leu
        200                 205                 210

tagcgggggt tgctgcaagg ctt                                           759


<210> 298
<211> 212
<212> PRT
<213> Corynebacterium glutamicum

<400> 298
Met Ile Arg Val Leu Leu Ala Asp Asp His Glu Ile Val Arg Leu Gly
1               5                   10                  15

Leu Arg Ala Val Leu Glu Ser Ala Glu Asp Ile Glu Val Val Gly Glu
            20                  25                  30

Val Ser Thr Ala Glu Gly Ala Val Gln Ala Ala Gln Glu Gly Gly Ile
        35                  40                  45

Asp Val Ile Leu Met Asp Leu Arg Phe Gly Pro Gly Val Gln Gly Thr
    50                  55                  60

Gln Val Ser Thr Gly Ala Asp Ala Thr Ala Ala Ile Lys Arg Asn Ile
65                  70                  75                  80

Asp Asn Pro Pro Lys Val Leu Val Val Thr Asn Tyr Asp Thr Asp Thr
                85                  90                  95

Asp Ile Leu Gly Ala Ile Glu Ala Gly Ala Leu Gly Tyr Leu Leu Lys
            100                 105                 110

Asp Ala Pro Pro Ser Glu Leu Leu Ala Ala Val Arg Ser Ala Ala Glu
        115                 120                 125

Gly Asp Ser Thr Leu Ser Pro Met Val Ala Asn Arg Leu Met Thr Arg
    130                 135                 140

Val Arg Thr Pro Lys Thr Ser Leu Thr Pro Arg Glu Leu Glu Val Leu
145                 150                 155                 160

Lys Leu Val Ala Gly Gly Ser Ser Asn Arg Asp Ile Gly Arg Ile Leu
                165                 170                 175

Phe Leu Ser Glu Ala Thr Val Lys Ser His Leu Val His Ile Tyr Asp
            180                 185                 190

Lys Leu Gly Val Arg Ser Arg Thr Ser Ala Val Ala Ala Ala Arg Glu
        195                 200                 205

Gln Gly Leu Leu
    210


<210> 299
<211> 655
<212> DNA
<213> Corynebacterium glutamicum

<220>
<221> CDS
<222> (24)..(632)
<223> RXA01189

<400> 299
cacctgcaga aaaggaagct taa atg att tcc att tcc atc gcc gac gac gaa   53
                          Met Ile Ser Ile Ser Ile Ala Asp Asp Glu
                          1               5                   10

gcc ctg atc gca agc tcc ctg gca acc ttg ctc agc ttg gaa ccc gat     101
Ala Leu Ile Ala Ser Ser Leu Ala Thr Leu Leu Ser Leu Glu Pro Asp

15                  20                  25

tta gac gtc cga cct acc gca gga tcc ggt gaa gaa ctc att gaa acg     149
Leu Asp Val Arg Pro Thr Ala Gly Ser Gly Glu Glu Leu Ile Glu Thr
            30                  35                  40

tgg gcg gat cca agc aac cga acc gat gta tgc gtc ctt gac ctt caa     197
Trp Ala Asp Pro Ser Asn Arg Thr Asp Val Cys Val Leu Asp Leu Gln
        45                  50                  55

ctc gga ggc atc gac ggc atc gac acc gcc acc cgg ctc atg gaa acc     245
Leu Gly Gly Ile Asp Gly Ile Asp Thr Ala Thr Arg Leu Met Glu Thr
    60                  65                  70

acc cca gat ttg gcc gtg ctc atc gtg acc agc cac gcc agg ccc cga     293
Thr Pro Asp Leu Ala Val Leu Ile Val Thr Ser His Ala Arg Pro Arg
 75                  80                  85                  90

caa ctc aaa cgc gcg ctt gca gca ggt gtt tta gga ttc ttg ccc aaa     341
Gln Leu Lys Arg Ala Leu Ala Ala Gly Val Leu Gly Phe Leu Pro Lys
                 95                 100                 105

aca tcc acc gca gat gaa ttc gcc acc gca atc cgc acc gtt cac gct     389
Thr Ser Thr Ala Asp Glu Phe Ala Thr Ala Ile Arg Thr Val His Ala
            110                 115                 120

gga cga cgc tac atc gac ccc gaa cta gcc gcc atg acg atc agc gcc     437
Gly Arg Arg Tyr Ile Asp Pro Glu Leu Ala Ala Met Thr Ile Ser Ala
        125                 130                 135

ggt gaa tcc cca tta acc aac cgt gaa gaa gaa gtc ctc gaa cta gca     485
Gly Glu Ser Pro Leu Thr Asn Arg Glu Glu Glu Val Leu Glu Leu Ala
    140                 145                 150

ggc caa gga cta agc gcc gaa gaa att gcg gtg gca gcg cac ctc gcg     533
Gly Gln Gly Leu Ser Ala Glu Glu Ile Ala Val Ala Ala His Leu Ala
155                 160                 165                 170

ccg gga acc acc cgc aac tat tta tcc caa gct atg aca aaa gta ggc     581
Pro Gly Thr Thr Arg Asn Tyr Leu Ser Gln Ala Met Thr Lys Val Gly
                175                 180                 185

gcg cag aat cgc ttt gaa gcg ttc acg cgc gcc agg gaa ttg ggc tgg     629
Ala Gln Asn Arg Phe Glu Ala Phe Thr Arg Ala Arg Glu Leu Gly Trp
            190                 195                 200

ttg tagcttgtgg cttatctcct att                                       655
Leu

<210> 300
<211> 203
<212> PRT
<213> Corynebacterium glutamicum

<400> 300
Met Ile Ser Ile Ser Ile Ala Asp Asp Glu Ala Leu Ile Ala Ser Ser
1               5                   10                  15

Leu Ala Thr Leu Leu Ser Leu Glu Pro Asp Leu Asp Val Arg Pro Thr
            20                  25                  30

Ala Gly Ser Gly Glu Glu Leu Ile Glu Thr Trp Ala Asp Pro Ser Asn
        35                  40                  45

Arg Thr Asp Val Cys Val Leu Asp Leu Gln Leu Gly Gly Ile Asp Gly
    50                  55                  60

Ile Asp Thr Ala Thr Arg Leu Met Glu Thr Thr Pro Asp Leu Ala Val
65                  70                  75                  80

Leu Ile Val Thr Ser His Ala Arg Pro Arg Gln Leu Lys Arg Ala Leu
                85                  90                  95

Ala Ala Gly Val Leu Gly Phe Leu Pro Lys Thr Ser Thr Ala Asp Glu
            100                 105                 110

Phe Ala Thr Ala Ile Arg Thr Val His Ala Gly Arg Arg Tyr Ile Asp
        115                 120                 125

Pro Glu Leu Ala Ala Met Thr Ile Ser Ala Gly Glu Ser Pro Leu Thr
    130                 135                 140

Asn Arg Glu Glu Glu Val Leu Glu Leu Ala Gly Gln Gly Leu Ser Ala
145                 150                 155                 160

Glu Glu Ile Ala Val Ala Ala His Leu Ala Pro Gly Thr Thr Arg Asn
                165                 170                 175

Tyr Leu Ser Gln Ala Met Thr Lys Val Gly Ala Gln Asn Arg Phe Glu
            180                 185                 190

Ala Phe Thr Arg Ala Arg Glu Leu Gly Trp Leu
        195                 200

<210> 301
<211> 753
<212> DNA
<213> Corynebacterium glutamicum

<220>
<221> CDS
<222> (101)..(730)
<223> RXA01607

<400> 301
gggctgaagg gctgggcgga acaataatta ttgaatctac aatcggatcg ggaactggaa 60

tttccgcccg ttttccctat ccacaaaagg accaagataa gtg atc cgt att ctg    115
                                           Val Ile Arg Ile Leu
                                           1               5

ttg gct gat gat cat ccc gtt gtt cgc gca ggc ctt gcc tcc ttg ctg    163
Leu Ala Asp Asp His Pro Val Val Arg Ala Gly Leu Ala Ser Leu Leu
                10                  15                  20

gtg agt gaa gat gat ttt gag ata gtg gac atg gtg ggc acc cca gat    211
Val Ser Glu Asp Asp Phe Glu Ile Val Asp Met Val Gly Thr Pro Asp
            25                  30                  35

gat gcc gtt gcg cgc gcc gcg gaa ggc ggg gtg gat gtg gtg ttg atg    259
Asp Ala Val Ala Arg Ala Ala Glu Gly Gly Val Asp Val Val Leu Met
        40                  45                  50

gat ctg cgt ttt ggt gat caa cca ggc atc gag gtc gcc ggc ggg gta    307
Asp Leu Arg Phe Gly Asp Gln Pro Gly Ile Glu Val Ala Gly Gly Val
    55                  60                  65

gag gca acg cgt cgc atc cgt gcg ctg gac aac ccg cca cag gta ctg    355
Glu Ala Thr Arg Arg Ile Arg Ala Leu Asp Asn Pro Pro Gln Val Leu
70                  75                  80                  85

gtg gtg acc aac tac tcc aca gac ggc gat gtg gtg ggc gca gta tct    403
Val Val Thr Asn Tyr Ser Thr Asp Gly Asp Val Val Gly Ala Val Ser
                90                  95                  100

gct ggt gcc gtg ggg tat ttg ctc aaa gat agc tcc cca gaa gat ctc    451
Ala Gly Ala Val Gly Tyr Leu Leu Lys Asp Ser Ser Pro Glu Asp Leu
            105                 110                 115

att gcc ggt gtt cgc gat gcc gcg cgg gga gaa tca gtg ctt tca aag    499
Ile Ala Gly Val Arg Asp Ala Ala Arg Gly Glu Ser Val Leu Ser Lys
        120                 125                 130

cag gtc gcc agc aag atc atg ggg cgg atg aac aac ccc atg act gct    547
Gln Val Ala Ser Lys Ile Met Gly Arg Met Asn Asn Pro Met Thr Ala
    135                 140                 145

ctc agt gcc aga gaa att gaa gtg ctg tcc ttg gtg gcg caa ggg caa    595
Leu Ser Ala Arg Glu Ile Glu Val Leu Ser Leu Val Ala Gln Gly Gln
150                 155                 160                 165

agc aat aga gaa atc ggc aag aaa ctt ttc ctc act gag gcc acg gtg     643
Ser Asn Arg Glu Ile Gly Lys Lys Leu Phe Leu Thr Glu Ala Thr Val
                170                 175                 180

aaa agt cac atg ggg cat gtg ttc aac aag ctg gat gtc acc tct aga     691
Lys Ser His Met Gly His Val Phe Asn Lys Leu Asp Val Thr Ser Arg
            185                 190                 195

aca gct gcg gta gct gaa gcc aga cag cgc gga att atc tagacgcaca      740
Thr Ala Ala Val Ala Glu Ala Arg Gln Arg Gly Ile Ile
        200                 205                 210

cgtgttggta acc                                                      753


<210> 302
<211> 210
<212> PRT
<213> Corynebacterium glutamicum

<400> 302
Val Ile Arg Ile Leu Leu Ala Asp Asp His Pro Val Val Arg Ala Gly
1               5                   10                  15

Leu Ala Ser Leu Leu Val Ser Glu Asp Asp Phe Glu Ile Val Asp Met
            20                  25                  30

Val Gly Thr Pro Asp Asp Ala Val Ala Arg Ala Ala Glu Gly Gly Val
        35                  40                  45

Asp Val Val Leu Met Asp Leu Arg Phe Gly Asp Gln Pro Gly Ile Glu
    50                  55                  60

Val Ala Gly Gly Val Glu Ala Thr Arg Arg Ile Arg Ala Leu Asp Asn
65                  70                  75                  80

Pro Pro Gln Val Leu Val Val Thr Asn Tyr Ser Thr Asp Gly Asp Val
                85                  90                  95

Val Gly Ala Val Ser Ala Gly Ala Val Gly Tyr Leu Leu Lys Asp Ser
            100                 105                 110

Ser Pro Glu Asp Leu Ile Ala Gly Val Arg Asp Ala Ala Arg Gly Glu
        115                 120                 125

Ser Val Leu Ser Lys Gln Val Ala Ser Lys Ile Met Gly Arg Met Asn
    130                 135                 140

Asn Pro Met Thr Ala Leu Ser Ala Arg Glu Ile Glu Val Leu Ser Leu
145                 150                 155                 160

Val Ala Gln Gly Gln Ser Asn Arg Glu Ile Gly Lys Lys Leu Phe Leu
                165                 170                 175

Thr Glu Ala Thr Val Lys Ser His Met Gly His Val Phe Asn Lys Leu
            180                 185                 190

Asp Val Thr Ser Arg Thr Ala Ala Val Ala Glu Ala Arg Gln Arg Gly
        195                 200                 205

Ile Ile
    210


<210> 303
<211> 1392
<212> DNA
<213> Corynebacterium glutamicum

<220>
<221> CDS
<222> (101)..(1369)
<223> RXN00470

<400> 303
tcataaccag gttgggcaaa agggatgaat ccctggttgt ggtggggctc ctgaaaagta 60

ctcatagact ctattgtgga gtgttgaggc tgataagtga atg ggg gaa agc cct 115
Met Gly Glu Ser Pro
1               5

gaa aag gtg gcg ttc agg gtc ttc cct gat ggt ttg gtg tcg cag ggg 163
Glu Lys Val Ala Phe Arg Val Phe Pro Asp Gly Leu Val Ser Gln Gly
                10                  15                  20

cat gac atg atc gaa gat atg agt aac aca cct gcg cct tat acc ccg 211
His Asp Met Ile Glu Asp Met Ser Asn Thr Pro Ala Pro Tyr Thr Pro
            25                  30                  35

cag cct gcg ggg caa gcg gtg cct tta tat ccc acg ttt acc cgg tca 259
Gln Pro Ala Gly Gln Ala Val Pro Leu Tyr Pro Thr Phe Thr Arg Ser
        40                  45                  50

aga gat ggt cgg gtt gtt gcg ggt gtc gca tcg ggg ctg gca aag cat 307
Arg Asp Gly Arg Val Val Ala Gly Val Ala Ser Gly Leu Ala Lys His
    55                  60                  65

ctt aat gtg tcg gtg ttt tgg gtt cgt gcg ctg ctg att ttt gcg gcg 355
Leu Asn Val Ser Val Phe Trp Val Arg Ala Leu Leu Ile Phe Ala Ala
70                  75                  80                  85

ttg ctg agc ggt gcg ggt ctt ttt gcg tat gcc ttg att tgg att ttt 403
Leu Leu Ser Gly Ala Gly Leu Phe Ala Tyr Ala Leu Ile Trp Ile Phe
                90                  95                  100

acg cgc att gag aaa aag ggg agt ggg gag gcg tcg aca agc aag cgc 451
Thr Arg Ile Glu Lys Lys Gly Ser Gly Glu Ala Ser Thr Ser Lys Arg
            105                 110                 115

tgg gtg tcg tgg tgc ctg gtg ctg ctc gct atc ggt ggt gct gcg gcg 499
Trp Val Ser Trp Cys Leu Val Leu Leu Ala Ile Gly Gly Ala Ala Ala
        120                 125                 130

tcg gtg atg ctg agc acc ggc ttc gcg gtg ggc acg ttg gtg ccc atc 547
Ser Val Met Leu Ser Thr Gly Phe Ala Val Gly Thr Leu Val Pro Ile
    135                 140                 145

ggc gtg gtc ggt gtg ggc ctg ttg atg gtg tgg ctg gcg tat gac cgc 595
Gly Val Val Gly Val Gly Leu Leu Met Val Trp Leu Ala Tyr Asp Arg
150                 155                 160                 165

ggg gtg gaa tcc ggc ccg aat ctg ctg att att gcc acc ggc ggt gtg 643
Gly Val Glu Ser Gly Pro Asn Leu Leu Ile Ile Ala Thr Gly Gly Val
                170                 175                 180

ttg atg ctg gtg gcg atc gtg ctg atc gtg atg aat tgg aac acc cag 691
Leu Met Leu Val Ala Ile Val Leu Ile Val Met Asn Trp Asn Thr Gln
            185                 190                 195

gac ggc ttc gtc atg gcg ctg gtg gcc gtg gtg ctc acg ctg gtg ggt 739
Asp Gly Phe Val Met Ala Leu Val Ala Val Val Leu Thr Leu Val Gly
        200                 205                 210

gtg gct gcg ctg ggc gtt ccg ctg tgg gtg cgg atg tgg gat cag ctg 787
Val Ala Ala Leu Gly Val Pro Leu Trp Val Arg Met Trp Asp Gln Leu
    215                 220                 225

ggc gag gag cgc gcg gaa aaa gcc gca gct gct gag cgc gca gat att 835
Gly Glu Glu Arg Ala Glu Lys Ala Ala Ala Ala Glu Arg Ala Asp Ile
230                 235                 240                 245

gct tcc cgc ctg cat gat tcg gta ctg cag acc ttg gcg ctg att caa 883
Ala Ser Arg Leu His Asp Ser Val Leu Gln Thr Leu Ala Leu Ile Gln
                250                 255                 260

aag cgt gcc gac gac ccc gcc gaa gtc gcc cgc ctg gcc cgc ggg cag 931
Lys Arg Ala Asp Asp Pro Ala Glu Val Ala Arg Leu Ala Arg Gly Gln
            265                 270                 275

gaa cgc gag ctg cgt caa tgg ctg ttt gat tcc caa gat aaa aca cct 979
Glu Arg Glu Leu Arg Gln Trp Leu Phe Asp Ser Gln Asp Lys Thr Pro
        280                 285                 290

caa aca acc ggc act gtc ttt act gcg ttg gag cgc gcc tgc ggt gaa 1027

Gln Thr Thr Gly Thr Val Phe Thr Ala Leu Glu Arg Ala Cys Gly Glu
    295                 300                 305

gtc gag gat att tac gct ctg cgt atc gtg cct gtg acc gtg gga acc      1075
Val Glu Asp Ile Tyr Ala Leu Arg Ile Val Pro Val Thr Val Gly Thr
310                 315                 320                 325

gat gaa gcg ctg act gag aaa acg cag gca gcg gtg atg gca gtc cgc      1123
Asp Glu Ala Leu Thr Glu Lys Thr Gln Ala Ala Val Met Ala Val Arg
                330                 335                 340

gaa gca ctc gtg aac gtg gcc aag cat gcc ggc gtg gaa acc gcc gat      1171
Glu Ala Leu Val Asn Val Ala Lys His Ala Gly Val Glu Thr Ala Asp
            345                 350                 355

gtg tac gcc gaa att atg ctc ggc gaa ctg aac att ttc gtc cgc gac      1219
Val Tyr Ala Glu Ile Met Leu Gly Glu Leu Asn Ile Phe Val Arg Asp
        360                 365                 370

cgc ggt gca gga ttc gac ccc gac aac atc ccc gac ggg cac cac ggg      1267
Arg Gly Ala Gly Phe Asp Pro Asp Asn Ile Pro Asp Gly His His Gly
    375                 380                 385

ctc gcc gaa tcc gtc caa ggc cgc gtc gaa cga gcc ggc gga aaa gta      1315
Leu Ala Glu Ser Val Gln Gly Arg Val Glu Arg Ala Gly Gly Lys Val
390                 395                 400                 405

cgc atc aaa tct gaa atc ggc gaa ggc acc gaa gtg gca atc acc atg      1363
Arg Ile Lys Ser Glu Ile Gly Glu Gly Thr Glu Val Ala Ile Thr Met
                410                 415                 420

gat gtg tagttggtcg tacgcgcgtg tct                                    1392
Asp Val

<210> 304
<211> 423
<212> PRT
<213> Corynebacterium glutamicum

<400> 304
Met Gly Glu Ser Pro Glu Lys Val Ala Phe Arg Val Phe Pro Asp Gly
1               5                   10                  15

Leu Val Ser Gln Gly His Asp Met Ile Glu Asp Met Ser Asn Thr Pro
            20                  25                  30

Ala Pro Tyr Thr Pro Gln Pro Ala Gly Gln Ala Val Pro Leu Tyr Pro
        35                  40                  45

Thr Phe Thr Arg Ser Arg Asp Gly Arg Val Val Ala Gly Val Ala Ser
    50                  55                  60

Gly Leu Ala Lys His Leu Asn Val Ser Val Phe Trp Val Arg Ala Leu
65                  70                  75                  80

Leu Ile Phe Ala Ala Leu Leu Ser Gly Ala Gly Leu Phe Ala Tyr Ala
                85                  90                  95

Leu Ile Trp Ile Phe Thr Arg Ile Glu Lys Lys Gly Ser Gly Glu Ala
            100                 105                 110

Ser Thr Ser Lys Arg Trp Val Ser Trp Cys Leu Val Leu Leu Ala Ile
        115                 120                 125

Gly Gly Ala Ala Ala Ser Val Met Leu Ser Thr Gly Phe Ala Val Gly
    130                 135                 140

Thr Leu Val Pro Ile Gly Val Val Gly Val Gly Leu Leu Met Val Trp
145                 150                 155                 160

Leu Ala Tyr Asp Arg Gly Val Glu Ser Gly Pro Asn Leu Leu Ile Ile
                165                 170                 175

Ala Thr Gly Gly Val Leu Met Leu Val Ala Ile Val Leu Ile Val Met
            180                 185                 190

Asn Trp Asn Thr Gln Asp Gly Phe Val Met Ala Leu Val Ala Val Val
        195                 200                 205

Leu Thr Leu Val Gly Val Ala Ala Leu Gly Val Pro Leu Trp Val Arg
    210                 215                 220

Met Trp Asp Gln Leu Gly Glu Glu Arg Ala Glu Lys Ala Ala Ala Ala
225                 230                 235                 240

Glu Arg Ala Asp Ile Ala Ser Arg Leu His Asp Ser Val Leu Gln Thr
                245                 250                 255

Leu Ala Leu Ile Gln Lys Arg Ala Asp Asp Pro Ala Glu Val Ala Arg
            260                 265                 270

Leu Ala Arg Gly Gln Glu Arg Glu Leu Arg Gln Trp Leu Phe Asp Ser
        275                 280                 285

Gln Asp Lys Thr Pro Gln Thr Thr Gly Thr Val Phe Thr Ala Leu Glu
    290                 295                 300

Arg Ala Cys Gly Glu Val Glu Asp Ile Tyr Ala Leu Arg Ile Val Pro
305                 310                 315                 320

Val Thr Val Gly Thr Asp Glu Ala Leu Thr Glu Lys Thr Gln Ala Ala
                325                 330                 335

Val Met Ala Val Arg Glu Ala Leu Val Asn Val Ala Lys His Ala Gly
            340                 345                 350

Val Glu Thr Ala Asp Val Tyr Ala Glu Ile Met Leu Gly Glu Leu Asn
        355                 360                 365

Ile Phe Val Arg Asp Arg Gly Ala Gly Phe Asp Pro Asp Asn Ile Pro
    370                 375                 380

Asp Gly His His Gly Leu Ala Glu Ser Val Gln Gly Arg Val Glu Arg
385                 390                 395                 400

Ala Gly Gly Lys Val Arg Ile Lys Ser Glu Ile Gly Glu Gly Thr Glu
                405                 410                 415

Val Ala Ile Thr Met Asp Val
            420


<210> 305
<211> 1323
<212> DNA
<213> Corynebacterium glutamicum

<220>
<221> CDS
<222> (101)..(1300)
<223> FRXA00470

<400> 305
tctattgtgg agtgttgagg ctgataagtg aatgggggaa agccctgaaa aggtggcgtt 60

cagggtcttc cctgatggtt tggtgtcgca ggggcatgac atg atc gaa gat atg    115
                                           Met Ile Glu Asp Met
                                           1               5

agt aac aca cct gcg cct tat acc ccg cag cct gcg ggg caa gcg gtg    163
Ser Asn Thr Pro Ala Pro Tyr Thr Pro Gln Pro Ala Gly Gln Ala Val
                10                  15                  20

cct tta tat ccc acg ttt acc cgg tca aga gat ggt cgg gtt gtt gcg    211
Pro Leu Tyr Pro Thr Phe Thr Arg Ser Arg Asp Gly Arg Val Val Ala
            25                  30                  35

ggt gtc gca tcg ggg ctg gca aag cat ctt aat gtg tcg gtg ttt tgg    259
Gly Val Ala Ser Gly Leu Ala Lys His Leu Asn Val Ser Val Phe Trp
        40                  45                  50

gtt cgt gcg ctg ctg att ttt gcg gcg ttg ctg agc ggt gcg ggt ctt    307

Val Arg Ala Leu Leu Ile Phe Ala Ala Leu Leu Ser Gly Ala Gly Leu
    55                  60                  65

ttt gcg tat gcc ttg att tgg att ttt acg cgc att gag aaa aag ggg 355
Phe Ala Tyr Ala Leu Ile Trp Ile Phe Thr Arg Ile Glu Lys Lys Gly
70                  75                  80                  85

agt ggg gag gcg tcg aca agc aag cgc tgg gtg tcg tgg tgc ctg gtg 403
Ser Gly Glu Ala Ser Thr Ser Lys Arg Trp Val Ser Trp Cys Leu Val
                90                  95                  100

ctg ctc gct atc ggt ggt gct gcg gcg tcg gtg atg ctg agc acc ggc 451
Leu Leu Ala Ile Gly Gly Ala Ala Ala Ser Val Met Leu Ser Thr Gly
            105                 110                 115

ttc gcg gtg ggc acg ttg gtg ccc atc ggc gtg gtc ggt gtg ggc ctg 499
Phe Ala Val Gly Thr Leu Val Pro Ile Gly Val Val Gly Val Gly Leu
        120                 125                 130

ttg atg gtg tgg ctg gcg tat gac cgc ggg gtg gaa tcc ggc ccg aat 547
Leu Met Val Trp Leu Ala Tyr Asp Arg Gly Val Glu Ser Gly Pro Asn
    135                 140                 145

ctg ctg att att gcc acc ggc ggt gtg ttg atg ctg gtg gcg atc gtg 595
Leu Leu Ile Ile Ala Thr Gly Gly Val Leu Met Leu Val Ala Ile Val
150                 155                 160                 165

ctg atc gtg atg aat tgg aac acc cag gac ggc ttc gtc atg gcg ctg 643
Leu Ile Val Met Asn Trp Asn Thr Gln Asp Gly Phe Val Met Ala Leu
                170                 175                 180

gtg gcc gtg gtg ctc acg ctg gtg ggt gtg gct gcg ctg ggc gtt ccg 691
Val Ala Val Val Leu Thr Leu Val Gly Val Ala Ala Leu Gly Val Pro
            185                 190                 195

ctg tgg gtg cgg atg tgg gat cag ctg ggc gag gag cgc gcg gaa aaa 739
Leu Trp Val Arg Met Trp Asp Gln Leu Gly Glu Glu Arg Ala Glu Lys
        200                 205                 210

gcc gca gct gct gag cgc gca gat att gct tcc cgc ctg cat gat tcg 787
Ala Ala Ala Ala Glu Arg Ala Asp Ile Ala Ser Arg Leu His Asp Ser
    215                 220                 225

gta ctg cag acc ttg gcg ctg att caa aag cgt gcc gac gac ccc gcc 835
Val Leu Gln Thr Leu Ala Leu Ile Gln Lys Arg Ala Asp Asp Pro Ala
230                 235                 240                 245

gaa gtc gcc cgc ctg gcc cgc ggg cag gaa cgc gag ctg cgt caa tgg 883
Glu Val Ala Arg Leu Ala Arg Gly Gln Glu Arg Glu Leu Arg Gln Trp
                250                 255                 260

ctg ttt gat tcc caa gat aaa aca cct caa aca acc ggc act gtc ttt 931
Leu Phe Asp Ser Gln Asp Lys Thr Pro Gln Thr Thr Gly Thr Val Phe
            265                 270                 275

act gcg ttg gag cgc gcc tgc ggt gaa gtc gag gat att tac gct ctg 979
Thr Ala Leu Glu Arg Ala Cys Gly Glu Val Glu Asp Ile Tyr Ala Leu
        280                 285                 290

cgt atc gtg cct gtg acc gtg gga acc gat gaa gcg ctg act gag aaa 1027
Arg Ile Val Pro Val Thr Val Gly Thr Asp Glu Ala Leu Thr Glu Lys
    295                 300                 305

acg cag gca gcg gtg atg gca gtc cgc gaa gca ctc gtg aac gtg gcc 1075
Thr Gln Ala Ala Val Met Ala Val Arg Glu Ala Leu Val Asn Val Ala
310                 315                 320                 325

aag cat gcc ggc gtg gaa acc gcc gat gtg tac gcc gaa att atg ctc 1123
Lys His Ala Gly Val Glu Thr Ala Asp Val Tyr Ala Glu Ile Met Leu
                330                 335                 340

ggc gaa ctg aac att ttc gtc cgc gac cgc ggt gca gga ttc gac ccc 1171
Gly Glu Leu Asn Ile Phe Val Arg Asp Arg Gly Ala Gly Phe Asp Pro
            345                 350                 355

gac aac atc ccc gac ggg cac cac ggg ctc gcc gaa tcc gtc caa ggc 1219
Asp Asn Ile Pro Asp Gly His His Gly Leu Ala Glu Ser Val Gln Gly

360                 365                 370

cgc gtc gaa cga gcc ggc gga aaa gta cgc atc aaa tct gaa atc ggc    1267
Arg Val Glu Arg Ala Gly Gly Lys Val Arg Ile Lys Ser Glu Ile Gly
    375                 380                 385

gaa ggc acc gaa gtg gca atc acc atg gat gtg tagttggtcg tacgcgcgtg 1320
Glu Gly Thr Glu Val Ala Ile Thr Met Asp Val
390                 395                 400

tct                                                                1323


<210> 306
<211> 400
<212> PRT
<213> Corynebacterium glutamicum

<400> 306
Met Ile Glu Asp Met Ser Asn Thr Pro Ala Pro Tyr Thr Pro Gln Pro
1               5                   10                  15

Ala Gly Gln Ala Val Pro Leu Tyr Pro Thr Phe Thr Arg Ser Arg Asp
            20                  25                  30

Gly Arg Val Val Ala Gly Val Ala Ser Gly Leu Ala Lys His Leu Asn
        35                  40                  45

Val Ser Val Phe Trp Val Arg Ala Leu Leu Ile Phe Ala Ala Leu Leu
    50                  55                  60

Ser Gly Ala Gly Leu Phe Ala Tyr Ala Leu Ile Trp Ile Phe Thr Arg
65                  70                  75                  80

Ile Glu Lys Lys Gly Ser Gly Glu Ala Ser Thr Ser Lys Arg Trp Val
                85                  90                  95

Ser Trp Cys Leu Val Leu Leu Ala Ile Gly Gly Ala Ala Ala Ser Val
            100                 105                 110

Met Leu Ser Thr Gly Phe Ala Val Gly Thr Leu Val Pro Ile Gly Val
        115                 120                 125

Val Gly Val Gly Leu Leu Met Val Trp Leu Ala Tyr Asp Arg Gly Val
    130                 135                 140

Glu Ser Gly Pro Asn Leu Leu Ile Ile Ala Thr Gly Gly Val Leu Met
145                 150                 155                 160

Leu Val Ala Ile Val Leu Ile Val Met Asn Trp Asn Thr Gln Asp Gly
                165                 170                 175

Phe Val Met Ala Leu Val Ala Val Val Leu Thr Leu Val Gly Val Ala
            180                 185                 190

Ala Leu Gly Val Pro Leu Trp Val Arg Met Trp Asp Gln Leu Gly Glu
        195                 200                 205

Glu Arg Ala Glu Lys Ala Ala Ala Ala Glu Arg Ala Asp Ile Ala Ser
    210                 215                 220

Arg Leu His Asp Ser Val Leu Gln Thr Leu Ala Leu Ile Gln Lys Arg
225                 230                 235                 240

Ala Asp Asp Pro Ala Glu Val Ala Arg Leu Ala Arg Gly Gln Glu Arg
                245                 250                 255

Glu Leu Arg Gln Trp Leu Phe Asp Ser Gln Asp Lys Thr Pro Gln Thr
            260                 265                 270

Thr Gly Thr Val Phe Thr Ala Leu Glu Arg Ala Cys Gly Glu Val Glu
        275                 280                 285

Asp Ile Tyr Ala Leu Arg Ile Val Pro Val Thr Val Gly Thr Asp Glu
    290                 295                 300

Ala Leu Thr Glu Lys Thr Gln Ala Ala Val Met Ala Val Arg Glu Ala

305                 310                 315                 320

Leu Val Asn Val Ala Lys His Ala Gly Val Glu Thr Ala Asp Val Tyr
                325                 330                 335

Ala Glu Ile Met Leu Gly Glu Leu Asn Ile Phe Val Arg Asp Arg Gly
            340                 345                 350

Ala Gly Phe Asp Pro Asp Asn Ile Pro Asp Gly His His Gly Leu Ala
        355                 360                 365

Glu Ser Val Gln Gly Arg Val Glu Arg Ala Gly Gly Lys Val Arg Ile
    370                 375                 380

Lys Ser Glu Ile Gly Glu Gly Thr Glu Val Ala Ile Thr Met Asp Val
385                 390                 395                 400

<210> 307
<211> 1119
<212> DNA
<213> Corynebacterium glutamicum

<220>
<221> CDS
<222> (101)..(1096)
<223> RXA00756

<400> 307
attcgctaaa cctgcgcagg atgagactgc cctcgcagaa agcacattcg acgaagccac 60

cgcgtaaaca gtacgtggtg gaagcttgag aggaagacaa gtg aat att gat gtc    115
                                           Val Asn Ile Asp Val
                                           1               5

cag gct tta aaa gcc atc gag tct gaa aaa gga atc cca gtt cca gac    163
Gln Ala Leu Lys Ala Ile Glu Ser Glu Lys Gly Ile Pro Val Pro Asp
                10                  15                  20

ttg ctg cgc acc atc gcc tct gca ctt ttg cat tcg tac atg gat aat    211
Leu Leu Arg Thr Ile Ala Ser Ala Leu Leu His Ser Tyr Met Asp Asn
            25                  30                  35

cgc gaa act gtt gcg tct gcg aac ctg aaa cca cgc gtg gac atc gat    259
Arg Glu Thr Val Ala Ser Ala Asn Leu Lys Pro Arg Val Asp Ile Asp
        40                  45                  50

tcc aca act ggc acg gtc aac gtc atc gtc tca gaa ttc gac gaa aac    307
Ser Thr Thr Gly Thr Val Asn Val Ile Val Ser Glu Phe Asp Glu Asn
    55                  60                  65

gga gag ctc gct tcc gaa tac gac gac acc cca tcc aac ttc gga cga    355
Gly Glu Leu Ala Ser Glu Tyr Asp Asp Thr Pro Ser Asn Phe Gly Arg
70                  75                  80                  85

gtc agc gcc cgc gct gtt cgc gac gcg atc gtt aag tcc ctg cgc gaa    403
Val Ser Ala Arg Ala Val Arg Asp Ala Ile Val Lys Ser Leu Arg Glu
                90                  95                  100

gca gaa gca agc cga gca ttc gat gcg tac gca gat tat gaa ggc acc    451
Ala Glu Ala Ser Arg Ala Phe Asp Ala Tyr Ala Asp Tyr Glu Gly Thr
            105                 110                 115

gtt gtg tcc ggc atc gtt caa gca gat gcc cgc gca gct gaa cgc gga    499
Val Val Ser Gly Ile Val Gln Ala Asp Ala Arg Ala Ala Glu Arg Gly
        120                 125                 130

atc atc atc gtg cag ctg ggt acc gaa gcg gac aac caa gac ggc gtt    547
Ile Ile Ile Val Gln Leu Gly Thr Glu Ala Asp Asn Gln Asp Gly Val
    135                 140                 145

ttg ctc cca gcc gag cag atc cct ggc gaa aag ctc aag cac ggc gac    595
Leu Leu Pro Ala Glu Gln Ile Pro Gly Glu Lys Leu Lys His Gly Asp
150                 155                 160                 165

cgc gtc aag tgc ttc gtc gtt ggc gtg ggc aag ggc aac act gac atc     643
Arg Val Lys Cys Phe Val Val Gly Val Gly Lys Gly Asn Thr Asp Ile
                170                 175                 180

cag atc aac ctg tct cgt act cac cct gag ctg gtg cgc cga ctg ttt     691
Gln Ile Asn Leu Ser Arg Thr His Pro Glu Leu Val Arg Arg Leu Phe
            185                 190                 195

gaa ctg gaa atc cca gaa gtt gct gac gga tcc gtg gaa att gtt gct     739
Glu Leu Glu Ile Pro Glu Val Ala Asp Gly Ser Val Glu Ile Val Ala
        200                 205                 210

atc tcc cgc gaa gcc gga cac cgc tcc aag gtt gct gtt caa gcc aag     787
Ile Ser Arg Glu Ala Gly His Arg Ser Lys Val Ala Val Gln Ala Lys
    215                 220                 225

gtg aag aac ctc aac gcc aag ggc gct tgc att ggc cca cgt gga cag     835
Val Lys Asn Leu Asn Ala Lys Gly Ala Cys Ile Gly Pro Arg Gly Gln
230                 235                 240                 245

cgt gtg tcc aac atc atg cgt gaa ctc ggt gga gaa aaa atc gac atc     883
Arg Val Ser Asn Ile Met Arg Glu Leu Gly Gly Glu Lys Ile Asp Ile
                250                 255                 260

atc gat tac tcc gaa gat cca gca acc ttc gtt gga aat gca ctg gca     931
Ile Asp Tyr Ser Glu Asp Pro Ala Thr Phe Val Gly Asn Ala Leu Ala
            265                 270                 275

cca tcc aag gtt gtc aac gta gag gtc acc gat ctt gaa gct caa acc     979
Pro Ser Lys Val Val Asn Val Glu Val Thr Asp Leu Glu Ala Gln Thr
        280                 285                 290

gcg cgc gta act gtc cct gac tac cag ctt tca cta gca atc ggt aaa     1027
Ala Arg Val Thr Val Pro Asp Tyr Gln Leu Ser Leu Ala Ile Gly Lys
    295                 300                 305

gaa ggt caa aac gcc cgc ttg gct gcc cgc ctg acc ggc tgg aag atc     1075
Glu Gly Gln Asn Ala Arg Leu Ala Ala Arg Leu Thr Gly Trp Lys Ile
310                 315                 320                 325

gac atc cac tct gac atc gat taaaagtcgc ttgaaccggc atg               1119
Asp Ile His Ser Asp Ile Asp
                330


<210> 308
<211> 332
<212> PRT
<213> Corynebacterium glutamicum

<400> 308
Val Asn Ile Asp Val Gln Ala Leu Lys Ala Ile Glu Ser Glu Lys Gly
1               5                   10                  15

Ile Pro Val Pro Asp Leu Leu Arg Thr Ile Ala Ser Ala Leu Leu His
            20                  25                  30

Ser Tyr Met Asp Asn Arg Glu Thr Val Ala Ser Ala Asn Leu Lys Pro
        35                  40                  45

Arg Val Asp Ile Asp Ser Thr Thr Gly Thr Val Asn Val Ile Val Ser
    50                  55                  60

Glu Phe Asp Glu Asn Gly Glu Leu Ala Ser Glu Tyr Asp Asp Thr Pro
65                  70                  75                  80

Ser Asn Phe Gly Arg Val Ser Ala Arg Ala Val Arg Asp Ala Ile Val
                85                  90                  95

Lys Ser Leu Arg Glu Ala Glu Ala Ser Arg Ala Phe Asp Ala Tyr Ala
            100                 105                 110

Asp Tyr Glu Gly Thr Val Val Ser Gly Ile Val Gln Ala Asp Ala Arg
        115                 120                 125

Ala Ala Glu Arg Gly Ile Ile Ile Val Gln Leu Gly Thr Glu Ala Asp

130                 135                 140

Asn Gln Asp Gly Val Leu Leu Pro Ala Glu Gln Ile Pro Gly Glu Lys
145                 150                 155                 160

Leu Lys His Gly Asp Arg Val Lys Cys Phe Val Val Gly Val Gly Lys
                165                 170                 175

Gly Asn Thr Asp Ile Gln Ile Asn Leu Ser Arg Thr His Pro Glu Leu
            180                 185                 190

Val Arg Arg Leu Phe Glu Leu Glu Ile Pro Glu Val Ala Asp Gly Ser
        195                 200                 205

Val Glu Ile Val Ala Ile Ser Arg Glu Ala Gly His Arg Ser Lys Val
    210                 215                 220

Ala Val Gln Ala Lys Val Lys Asn Leu Asn Ala Lys Gly Ala Cys Ile
225                 230                 235                 240

Gly Pro Arg Gly Gln Arg Val Ser Asn Ile Met Arg Glu Leu Gly Gly
                245                 250                 255

Glu Lys Ile Asp Ile Ile Asp Tyr Ser Glu Asp Pro Ala Thr Phe Val
            260                 265                 270

Gly Asn Ala Leu Ala Pro Ser Lys Val Val Asn Val Glu Val Thr Asp
        275                 280                 285

Leu Glu Ala Gln Thr Ala Arg Val Thr Val Pro Asp Tyr Gln Leu Ser
    290                 295                 300

Leu Ala Ile Gly Lys Glu Gly Gln Asn Ala Arg Leu Ala Ala Arg Leu
305                 310                 315                 320

Thr Gly Trp Lys Ile Asp Ile His Ser Asp Ile Asp
                325                 330


<210> 309
<211> 834
<212> DNA
<213> Corynebacterium glutamicum

<220>
<221> CDS
<222> (101)..(811)
<223> RXA00139

<400> 309
gaccggaaac gtactcaagg tagacacccg cgacggttcc tacctctccc gcgttaacaa 60

ctaagattct taaaaccttt aagaatcagc cagaaacatt ttg att gaa cag gaa    115
                                            Leu Ile Glu Gln Glu
                                            1               5

caa aga gaa caa aac gtg agc gag cgt cga caa gat tac aag cga cac    163
Gln Arg Glu Gln Asn Val Ser Glu Arg Arg Gln Asp Tyr Lys Arg His
                10                  15                  20

gga tcc cgc tac aag gcg cgc atg cgt gcc gta gac atc cta ttt gaa    211
Gly Ser Arg Tyr Lys Ala Arg Met Arg Ala Val Asp Ile Leu Phe Glu
            25                  30                  35

gcg gaa tcc cgc gat gtt gat ccc gtg gcc atc atc gat gac cgc cac    259
Ala Glu Ser Arg Asp Val Asp Pro Val Ala Ile Ile Asp Asp Arg His
        40                  45                  50

aag ttg gcg cgc gat acc aac ccc atc gtt gca ccg gta gcg gaa tac    307
Lys Leu Ala Arg Asp Thr Asn Pro Ile Val Ala Pro Val Ala Glu Tyr
    55                  60                  65

acc gaa acc atc atc aat ggc gtt gcc gtt gaa ctc gat acc ctc gat    355
Thr Glu Thr Ile Ile Asn Gly Val Ala Val Glu Leu Asp Thr Leu Asp
70                  75                  80                  85

gtc ttc ctc gcg gaa cac atc gca gaa acc tgg act ctc gga cga ctc    403

Val Phe Leu Ala Glu His Ile Ala Glu Thr Trp Thr Leu Gly Arg Leu
                90                  95                  100

cca tcc gtc gac cgc gca atc ctg cgc gtc gct tcc tgg gaa atg atc     451
Pro Ser Val Asp Arg Ala Ile Leu Arg Val Ala Ser Trp Glu Met Ile
            105                 110                 115

tac aac gcc gac gtt cct gtc acc acc gca atc gtt gaa gcc gtg gaa     499
Tyr Asn Ala Asp Val Pro Val Thr Thr Ala Ile Val Glu Ala Val Glu
        120                 125                 130

att gcc tcc gaa tac tcc gga gac aaa tcc agt gcc tac atc aac gcg     547
Ile Ala Ser Glu Tyr Ser Gly Asp Lys Ser Ser Ala Tyr Ile Asn Ala
    135                 140                 145

aca ctt gac gcc atg gca tca aag gtg gag acc ctc cgc gag cgc gcc     595
Thr Leu Asp Ala Met Ala Ser Lys Val Glu Thr Leu Arg Glu Arg Ala
150                 155                 160                 165

gcc aac cca gaa gca gtt ctg gcg gaa gct tcc gaa tct ctc gat gat     643
Ala Asn Pro Glu Ala Val Leu Ala Glu Ala Ser Glu Ser Leu Asp Asp
                170                 175                 180

gct ccg gtc gcg ccg tgg gat gac tcg gat gct ttg gat gac tcg gat     691
Ala Pro Val Ala Pro Trp Asp Asp Ser Asp Ala Leu Asp Asp Ser Asp
            185                 190                 195

gaa gat ttt gag gct gta gat gct gct gag gtt ttt gag gct gaa gag     739
Glu Asp Phe Glu Ala Val Asp Ala Ala Glu Val Phe Glu Ala Glu Glu
        200                 205                 210

act gta gag gtt tcc gaa gtc gca gaa gac tct gaa gtt tca aag gtt     787
Thr Val Glu Val Ser Glu Val Ala Glu Asp Ser Glu Val Ser Lys Val
    215                 220                 225

tca gaa gaa aag gct gac gag agc taaatctttt ctggctaaca cca           834
Ser Glu Glu Lys Ala Asp Glu Ser
230                 235


<210> 310
<211> 237
<212> PRT
<213> Corynebacterium glutamicum

<400> 310
Leu Ile Glu Gln Glu Gln Arg Glu Gln Asn Val Ser Glu Arg Arg Gln
1               5                   10                  15

Asp Tyr Lys Arg His Gly Ser Arg Tyr Lys Ala Arg Met Arg Ala Val
            20                  25                  30

Asp Ile Leu Phe Glu Ala Glu Ser Arg Asp Val Asp Pro Val Ala Ile
        35                  40                  45

Ile Asp Asp Arg His Lys Leu Ala Arg Asp Thr Asn Pro Ile Val Ala
    50                  55                  60

Pro Val Ala Glu Tyr Thr Glu Thr Ile Ile Asn Gly Val Ala Val Glu
65                  70                  75                  80

Leu Asp Thr Leu Asp Val Phe Leu Ala Glu His Ile Ala Glu Thr Trp
                85                  90                  95

Thr Leu Gly Arg Leu Pro Ser Val Asp Arg Ala Ile Leu Arg Val Ala
            100                 105                 110

Ser Trp Glu Met Ile Tyr Asn Ala Asp Val Pro Val Thr Thr Ala Ile
        115                 120                 125

Val Glu Ala Val Glu Ile Ala Ser Glu Tyr Ser Gly Asp Lys Ser Ser
    130                 135                 140

Ala Tyr Ile Asn Ala Thr Leu Asp Ala Met Ala Ser Lys Val Glu Thr
145                 150                 155                 160

Leu Arg Glu Arg Ala Ala Asn Pro Glu Ala Val Leu Ala Glu Ala Ser

165                 170                 175

Glu Ser Leu Asp Asp Ala Pro Val Ala Pro Trp Asp Asp Ser Asp Ala
            180                 185                 190

Leu Asp Asp Ser Asp Glu Asp Phe Glu Ala Val Asp Ala Ala Glu Val
        195                 200                 205

Phe Glu Ala Glu Glu Thr Val Glu Val Ser Glu Val Ala Glu Asp Ser
    210                 215                 220

Glu Val Ser Lys Val Ser Glu Glu Lys Ala Asp Glu Ser
225                 230                 235


<210> 311
<211> 1458
<212> DNA
<213> Corynebacterium glutamicum

<220>
<221> CDS
<222> (101)..(1435)
<223> RXA01303

<400> 311
aacatgcggg cgcaggtcag agctgttatc ttagtactta tcacagccat agggcgggct 60

tgacggaaag cctttccgcg taaccatgaa gaggcatcac gtg aca caa ctc aac    115
                                           Val Thr Gln Leu Asn
                                           1               5

acc aaa ggc gtt gtt ctg caa ggg tgg gat cca gaa gat cct gaa cat    163
Thr Lys Gly Val Val Leu Gln Gly Trp Asp Pro Glu Asp Pro Glu His
                10                  15                  20

tgg gac tcg aaa att gca tgg cga acc ctg tgg att acc acc ttc tcc    211
Trp Asp Ser Lys Ile Ala Trp Arg Thr Leu Trp Ile Thr Thr Phe Ser
            25                  30                  35

atg att att ggg ttc tgc gtg tgg tat ttg gtt tct gcc atc gct ccc    259
Met Ile Ile Gly Phe Cys Val Trp Tyr Leu Val Ser Ala Ile Ala Pro
        40                  45                  50

cta ctc aat cga att gga ttt gat ctc tca gca ggt cag ctt tat tgg    307
Leu Leu Asn Arg Ile Gly Phe Asp Leu Ser Ala Gly Gln Leu Tyr Trp
    55                  60                  65

ctc gca tct atc ccc ggt ttg gcc ggc gga tta atc cga ttg att tac    355
Leu Ala Ser Ile Pro Gly Leu Ala Gly Gly Leu Ile Arg Leu Ile Tyr
70                  75                  80                  85

atg ttc ctt cca ccg att ctt gga acc cgc aaa ttg gtc gga att tcc    403
Met Phe Leu Pro Pro Ile Leu Gly Thr Arg Lys Leu Val Gly Ile Ser
                90                  95                  100

tcc ggt cta ttt ttg atc ccc atg ttt ggg tgg ttc ctg gct gtc caa    451
Ser Gly Leu Phe Leu Ile Pro Met Phe Gly Trp Phe Leu Ala Val Gln
            105                 110                 115

gat tca agc act ccc tac tgg tgg ctt ctc aca ctc gct gca ctc act    499
Asp Ser Ser Thr Pro Tyr Trp Trp Leu Leu Thr Leu Ala Ala Leu Thr
        120                 125                 130

ggc att ggt ggt ggc gtg ttc tct gga tat atg ccg tcc acg gga tac    547
Gly Ile Gly Gly Gly Val Phe Ser Gly Tyr Met Pro Ser Thr Gly Tyr
    135                 140                 145

ttc ttc ccc aag gca aaa tcg ggc act gcg ctg ggc att cag gca ggt    595
Phe Phe Pro Lys Ala Lys Ser Gly Thr Ala Leu Gly Ile Gln Ala Gly
150                 155                 160                 165

atc ggc aac ctc ggc gtc tcg ata att cag ttc atg ggc cca tgg gtc    643
Ile Gly Asn Leu Gly Val Ser Ile Ile Gln Phe Met Gly Pro Trp Val
                170                 175                 180

atg ggt ttc ggt ctg ctg ggc att ggt ttc ctc acc ccg cag cgc acc    691

Met Gly Phe Gly Leu Leu Gly Ile Gly Phe Leu Thr Pro Gln Arg Thr
            185                 190                 195

att gaa ggc acc acg gtg ttt gtg cac aat gct gcg att gtg ttg gtc      739
Ile Glu Gly Thr Thr Val Phe Val His Asn Ala Ala Ile Val Leu Val
        200                 205                 210

ccg tgg act att ctc gcg gcc gtt tta tcc ttc ctg ttt ctt aaa gat      787
Pro Trp Thr Ile Leu Ala Ala Val Leu Ser Phe Leu Phe Leu Lys Asp
    215                 220                 225

gtc cca gtc acc gca aat ttc cgg caa cag atc gat atc ttt ggc aac      835
Val Pro Val Thr Ala Asn Phe Arg Gln Gln Ile Asp Ile Phe Gly Asn
230                 235                 240                 245

aag aac aca tgg att ttg tcc att atc tac ttg atg aca ttc ggt gcc      883
Lys Asn Thr Trp Ile Leu Ser Ile Ile Tyr Leu Met Thr Phe Gly Ala
                250                 255                 260

ttc gcc ggt ttc gcc gcg cag ttc ggt ctg atc atc aac aac aac ttc      931
Phe Ala Gly Phe Ala Ala Gln Phe Gly Leu Ile Ile Asn Asn Asn Phe
            265                 270                 275

ggc atc gct tcc ccg atg gca gag act tat cca gct gag atg ctt cac      979
Gly Ile Ala Ser Pro Met Ala Glu Thr Tyr Pro Ala Glu Met Leu His
        280                 285                 290

gcc ggt gct acg ttc gcg ttt ctt gga cct ttg att ggt gct ttg gtg      1027
Ala Gly Ala Thr Phe Ala Phe Leu Gly Pro Leu Ile Gly Ala Leu Val
    295                 300                 305

cgt gct gca tgg ggt cca ctg tgt gac aga ttc ggt gga gct atc tgg      1075
Arg Ala Ala Trp Gly Pro Leu Cys Asp Arg Phe Gly Gly Ala Ile Trp
310                 315                 320                 325

acc ttt gtc ggt ggc atc gga atg act atc gcc act gca gct gcc gca      1123
Thr Phe Val Gly Gly Ile Gly Met Thr Ile Ala Thr Ala Ala Ala Ala
                330                 335                 340

atc ttc cta agc aga gcg gag aca cct gat gat ttc tgg cca ttc ctg      1171
Ile Phe Leu Ser Arg Ala Glu Thr Pro Asp Asp Phe Trp Pro Phe Leu
            345                 350                 355

tgg tcc atg ctt gcc ctg ttc ttc ttc acc ggt ctg ggc aat gct ggc      1219
Trp Ser Met Leu Ala Leu Phe Phe Phe Thr Gly Leu Gly Asn Ala Gly
        360                 365                 370

acc ttc aaa caa atg ccc atg att ttg cct aaa cgc caa gca ggt ggc      1267
Thr Phe Lys Gln Met Pro Met Ile Leu Pro Lys Arg Gln Ala Gly Gly
    375                 380                 385

gtg atc ggc tgg acc ggt gcc att ggt gcc ttc ggc ccc ttc att gtc      1315
Val Ile Gly Trp Thr Gly Ala Ile Gly Ala Phe Gly Pro Phe Ile Val
390                 395                 400                 405

ggt gtc ttg ctc tcc ttc act cca act gtc gcg ttc ttc tgg ggc tgc      1363
Gly Val Leu Leu Ser Phe Thr Pro Thr Val Ala Phe Phe Trp Gly Cys
                410                 415                 420

gtg gtg ttc ttc atc atc gcc acc gct ttg acc tgg atc tac tac gcc      1411
Val Val Phe Phe Ile Ile Ala Thr Ala Leu Thr Trp Ile Tyr Tyr Ala
            425                 430                 435

cgc ccg aac gct cca ttc ccg gga taaaccgaaa ggccaatcca tga            1458
Arg Pro Asn Ala Pro Phe Pro Gly
        440                 445

<210> 312
<211> 445
<212> PRT
<213> Corynebacterium glutamicum

<400> 312
Val Thr Gln Leu Asn Thr Lys Gly Val Val Leu Gln Gly Trp Asp Pro
  1               5                  10                  15

Glu Asp Pro Glu His Trp Asp Ser Lys Ile Ala Trp Arg Thr Leu Trp
                20                  25                  30

Ile Thr Thr Phe Ser Met Ile Ile Gly Phe Cys Val Trp Tyr Leu Val
        35                  40                  45

Ser Ala Ile Ala Pro Leu Leu Asn Arg Ile Gly Phe Asp Leu Ser Ala
    50                  55                  60

Gly Gln Leu Tyr Trp Leu Ala Ser Ile Pro Gly Leu Ala Gly Gly Leu
65                  70                  75                  80

Ile Arg Leu Ile Tyr Met Phe Leu Pro Pro Ile Leu Gly Thr Arg Lys
                85                  90                  95

Leu Val Gly Ile Ser Ser Gly Leu Phe Leu Ile Pro Met Phe Gly Trp
            100                 105                 110

Phe Leu Ala Val Gln Asp Ser Ser Thr Pro Tyr Trp Trp Leu Leu Thr
        115                 120                 125

Leu Ala Ala Leu Thr Gly Ile Gly Gly Gly Val Phe Ser Gly Tyr Met
    130                 135                 140

Pro Ser Thr Gly Tyr Phe Phe Pro Lys Ala Lys Ser Gly Thr Ala Leu
145                 150                 155                 160

Gly Ile Gln Ala Gly Ile Gly Asn Leu Gly Val Ser Ile Ile Gln Phe
                165                 170                 175

Met Gly Pro Trp Val Met Gly Phe Gly Leu Leu Gly Ile Gly Phe Leu
            180                 185                 190

Thr Pro Gln Arg Thr Ile Glu Gly Thr Thr Val Phe Val His Asn Ala
        195                 200                 205

Ala Ile Val Leu Val Pro Trp Thr Ile Leu Ala Ala Val Leu Ser Phe
    210                 215                 220

Leu Phe Leu Lys Asp Val Pro Val Thr Ala Asn Phe Arg Gln Gln Ile
225                 230                 235                 240

Asp Ile Phe Gly Asn Lys Asn Thr Trp Ile Leu Ser Ile Ile Tyr Leu
                245                 250                 255

Met Thr Phe Gly Ala Phe Ala Gly Phe Ala Ala Gln Phe Gly Leu Ile
            260                 265                 270

Ile Asn Asn Asn Phe Gly Ile Ala Ser Pro Met Ala Glu Thr Tyr Pro
        275                 280                 285

Ala Glu Met Leu His Ala Gly Ala Thr Phe Ala Phe Leu Gly Pro Leu
    290                 295                 300

Ile Gly Ala Leu Val Arg Ala Ala Trp Gly Pro Leu Cys Asp Arg Phe
305                 310                 315                 320

Gly Gly Ala Ile Trp Thr Phe Val Gly Gly Ile Gly Met Thr Ile Ala
                325                 330                 335

Thr Ala Ala Ala Ala Ile Phe Leu Ser Arg Ala Glu Thr Pro Asp Asp
            340                 345                 350

Phe Trp Pro Phe Leu Trp Ser Met Leu Ala Leu Phe Phe Phe Thr Gly
        355                 360                 365

Leu Gly Asn Ala Gly Thr Phe Lys Gln Met Pro Met Ile Leu Pro Lys
    370                 375                 380

Arg Gln Ala Gly Gly Val Ile Gly Trp Thr Gly Ala Ile Gly Ala Phe
385                 390                 395                 400

Gly Pro Phe Ile Val Gly Val Leu Leu Ser Phe Thr Pro Thr Val Ala
                405                 410                 415

Phe Phe Trp Gly Cys Val Val Phe Phe Ile Ile Ala Thr Ala Leu Thr
            420                 425                 430

Trp Ile Tyr Tyr Ala Arg Pro Asn Ala Pro Phe Pro Gly
        435                 440                 445

<210> 313
<211> 327
<212> DNA
<213> Corynebacterium glutamicum

<220>
<221> CDS
<222> (101)..(304)
<223> RXA01412

<400> 313
cgccttcttt gcagctgaga attaactcac ccttgccctt ataccccata ggggtatagc 60

cttgagggag agagtacttc acctgaaagg ggccagtgac atg gca tta aag aac    115
                                           Met Ala Leu Lys Asn
                                             1               5

tac acc gtt gag ggc atg acc tgc gca cac tgc gtg gca tcg gta act    163
Tyr Thr Val Glu Gly Met Thr Cys Ala His Cys Val Ala Ser Val Thr
                10                  15                  20

gaa gag gta agc gaa gtt aat ggc gtt agc gct gtt gac gtc act cta    211
Glu Glu Val Ser Glu Val Asn Gly Val Ser Ala Val Asp Val Thr Leu
            25                  30                  35

gaa tca gga aac gtc gct gtc agt ggc gaa ggt ttc agc gat gca gag    259
Glu Ser Gly Asn Val Ala Val Ser Gly Glu Gly Phe Ser Asp Ala Glu
        40                  45                  50

atc cag gct gct gta gag gaa gcc ggc tac aag atc gtt gcc tcc        304
Ile Gln Ala Ala Val Glu Glu Ala Gly Tyr Lys Ile Val Ala Ser
    55                  60                  65

taaagcaccc aagaacattt aaa                                          327

<210> 314
<211> 68
<212> PRT
<213> Corynebacterium glutamicum

<400> 314
Met Ala Leu Lys Asn Tyr Thr Val Glu Gly Met Thr Cys Ala His Cys
  1               5                  10                  15

Val Ala Ser Val Thr Glu Glu Val Ser Glu Val Asn Gly Val Ser Ala
            20                  25                  30

Val Asp Val Thr Leu Glu Ser Gly Asn Val Ala Val Ser Gly Glu Gly
        35                  40                  45

Phe Ser Asp Ala Glu Ile Gln Ala Ala Val Glu Glu Ala Gly Tyr Lys
    50                  55                  60

Ile Val Ala Ser
65

<210> 315
<211> 1266
<212> DNA
<213> Corynebacterium glutamicum

<220>
<221> CDS
<222> (101)..(1243)
<223> RXA00773

<400> 315
gcccccaaaa agtgaaagca caccactttc ctagttgcgc cctgctcaca atttgcttca 60

aatattttgc ccaacctgat tcacggggga caatagttag gtg act tta aaa atc    115

Val Thr Leu Lys Ile
1               5

ggc ccc ttt gac ctt gcc tcc cct gtg gtt cta gcc ccc atg gct ggt     163
Gly Pro Phe Asp Leu Ala Ser Pro Val Val Leu Ala Pro Met Ala Gly
                10                  15                  20

gta acc aac gtt gct ttc cgc acg ctg tgc cgt gaa cag gaa atg caa     211
Val Thr Asn Val Ala Phe Arg Thr Leu Cys Arg Glu Gln Glu Met Gln
            25                  30                  35

cgc acg gga aca atc tcg ggg ctg tac gtc tgt gaa atg gtg act gcg     259
Arg Thr Gly Thr Ile Ser Gly Leu Tyr Val Cys Glu Met Val Thr Ala
        40                  45                  50

cgt gct ctt gtt gag cgc aat gag aaa acc atg cac atg acc acc ttc     307
Arg Ala Leu Val Glu Arg Asn Glu Lys Thr Met His Met Thr Thr Phe
    55                  60                  65

gcg ccg gat gaa aat ccc cga agc ttg cag ctg tac acg gtt gac ccg     355
Ala Pro Asp Glu Asn Pro Arg Ser Leu Gln Leu Tyr Thr Val Asp Pro
70                  75                  80                  85

aag tac acc tac gaa gcg gcg aag atg atc gtt gat gaa aac ttg gcg     403
Lys Tyr Thr Tyr Glu Ala Ala Lys Met Ile Val Asp Glu Asn Leu Ala
                90                  95                  100

gat cat att gat atg aac ttt ggc tgc ccg gtt cca aag gtc acg cgc     451
Asp His Ile Asp Met Asn Phe Gly Cys Pro Val Pro Lys Val Thr Arg
            105                 110                 115

cgg ggt ggc ggt tct gcg att cct tac aag cgc cgt ttg ttt gaa aac     499
Arg Gly Gly Gly Ser Ala Ile Pro Tyr Lys Arg Arg Leu Phe Glu Asn
        120                 125                 130

atc gtt tcc gcg gct gtg aag gct acg gaa ggc acg gac att ccg gtg     547
Ile Val Ser Ala Ala Val Lys Ala Thr Glu Gly Thr Asp Ile Pro Val
    135                 140                 145

acg gtg aag ttc cgc gtt ggt att gat gat gag cac cat act cac ttg     595
Thr Val Lys Phe Arg Val Gly Ile Asp Asp Glu His His Thr His Leu
150                 155                 160                 165

gat gct gga cgc att gct gtc gac gcc ggc gcg aag tcc gta gcg ctt     643
Asp Ala Gly Arg Ile Ala Val Asp Ala Gly Ala Lys Ser Val Ala Leu
                170                 175                 180

cac gcc cgc act gcg gcg cag cgc tat tcc ggt gag gct gat tgg aac     691
His Ala Arg Thr Ala Ala Gln Arg Tyr Ser Gly Glu Ala Asp Trp Asn
            185                 190                 195

gag atc gcg cgc ctg aag gag cat ttg gca gat acc ggc atc cca gtt     739
Glu Ile Ala Arg Leu Lys Glu His Leu Ala Asp Thr Gly Ile Pro Val
        200                 205                 210

ttg ggc aat ggc gat att ttc gcg gca tcc gat gca acg cgc atg atg     787
Leu Gly Asn Gly Asp Ile Phe Ala Ala Ser Asp Ala Thr Arg Met Met
    215                 220                 225

gag caa act ggc tgc gat ggc gtc gtg gtt ggg cgt ggt tgc ctg ggc     835
Glu Gln Thr Gly Cys Asp Gly Val Val Val Gly Arg Gly Cys Leu Gly
230                 235                 240                 245

agg cct tgg ctc ttt gct gag ctg tct gct gct gtt cgt gga gaa gaa     883
Arg Pro Trp Leu Phe Ala Glu Leu Ser Ala Ala Val Arg Gly Glu Glu
                250                 255                 260

atc cca gag gag cct acc ttc ggc gaa gtt acc caa atc atc ctg cgc     931
Ile Pro Glu Glu Pro Thr Phe Gly Glu Val Thr Gln Ile Ile Leu Arg
            265                 270                 275

cac gca gaa ctc ctc atg cag cat gat ggc gaa acc aag ggg ctg cgc     979
His Ala Glu Leu Leu Met Gln His Asp Gly Glu Thr Lys Gly Leu Arg
        280                 285                 290

gat ctg cgt aag cac atg ggt tgg tac ctg cgc ggt ttc cct gtt ggc     1027
Asp Leu Arg Lys His Met Gly Trp Tyr Leu Arg Gly Phe Pro Val Gly

295                 300                 305

ggc gaa ttc cgc tcc aat ctg gcc aag gtt tcc acc tat gtg gag ctt     1075
Gly Glu Phe Arg Ser Asn Leu Ala Lys Val Ser Thr Tyr Val Glu Leu
310                 315                 320                 325

gag gat ctc cta gca cca tgg gct gac tcc acc gcc aag gca gag gac     1123
Glu Asp Leu Leu Ala Pro Trp Ala Asp Ser Thr Ala Lys Ala Glu Asp
                330                 335                 340

gcg gaa ggt gca cga ggt cga cag ggc gct cct gca aag gtg gca ctt     1171
Ala Glu Gly Ala Arg Gly Arg Gln Gly Ala Pro Ala Lys Val Ala Leu
            345                 350                 355

cca gat ggc tgg ttg gac gat cct gag gat gcc act gtt cct aaa ggc     1219
Pro Asp Gly Trp Leu Asp Asp Pro Glu Asp Ala Thr Val Pro Lys Gly
        360                 365                 370

gca gaa atg gaa aac tcc gga ggg tagttaattt aatacttacc ccc           1266
Ala Glu Met Glu Asn Ser Gly Gly
    375                 380


<210> 316
<211> 381
<212> PRT
<213> Corynebacterium glutamicum

<400> 316
Val Thr Leu Lys Ile Gly Pro Phe Asp Leu Ala Ser Pro Val Val Leu
1               5                   10                  15

Ala Pro Met Ala Gly Val Thr Asn Val Ala Phe Arg Thr Leu Cys Arg
            20                  25                  30

Glu Gln Glu Met Gln Arg Thr Gly Thr Ile Ser Gly Leu Tyr Val Cys
        35                  40                  45

Glu Met Val Thr Ala Arg Ala Leu Val Glu Arg Asn Glu Lys Thr Met
    50                  55                  60

His Met Thr Thr Phe Ala Pro Asp Glu Asn Pro Arg Ser Leu Gln Leu
65                  70                  75                  80

Tyr Thr Val Asp Pro Lys Tyr Thr Tyr Glu Ala Ala Lys Met Ile Val
                85                  90                  95

Asp Glu Asn Leu Ala Asp His Ile Asp Met Asn Phe Gly Cys Pro Val
            100                 105                 110

Pro Lys Val Thr Arg Arg Gly Gly Gly Ser Ala Ile Pro Tyr Lys Arg
        115                 120                 125

Arg Leu Phe Glu Asn Ile Val Ser Ala Ala Val Lys Ala Thr Glu Gly
    130                 135                 140

Thr Asp Ile Pro Val Thr Val Lys Phe Arg Val Gly Ile Asp Asp Glu
145                 150                 155                 160

His His Thr His Leu Asp Ala Gly Arg Ile Ala Val Asp Ala Gly Ala
                165                 170                 175

Lys Ser Val Ala Leu His Ala Arg Thr Ala Ala Gln Arg Tyr Ser Gly
            180                 185                 190

Glu Ala Asp Trp Asn Glu Ile Ala Arg Leu Lys Glu His Leu Ala Asp
        195                 200                 205

Thr Gly Ile Pro Val Leu Gly Asn Gly Asp Ile Phe Ala Ala Ser Asp
    210                 215                 220

Ala Thr Arg Met Met Glu Gln Thr Gly Cys Asp Gly Val Val Val Gly
225                 230                 235                 240

Arg Gly Cys Leu Gly Arg Pro Trp Leu Phe Ala Glu Leu Ser Ala Ala
                245                 250                 255

Val Arg Gly Glu Glu Ile Pro Glu Glu Pro Thr Phe Gly Glu Val Thr
            260                 265                 270

Gln Ile Ile Leu Arg His Ala Glu Leu Leu Met Gln His Asp Gly Glu
        275                 280                 285

Thr Lys Gly Leu Arg Asp Leu Arg Lys His Met Gly Trp Tyr Leu Arg
    290                 295                 300

Gly Phe Pro Val Gly Gly Glu Phe Arg Ser Asn Leu Ala Lys Val Ser
305                 310                 315                 320

Thr Tyr Val Glu Leu Glu Asp Leu Leu Ala Pro Trp Ala Asp Ser Thr
                325                 330                 335

Ala Lys Ala Glu Asp Ala Glu Gly Ala Arg Gly Arg Gln Gly Ala Pro
            340                 345                 350

Ala Lys Val Ala Leu Pro Asp Gly Trp Leu Asp Asp Pro Glu Asp Ala
        355                 360                 365

Thr Val Pro Lys Gly Ala Glu Met Glu Asn Ser Gly Gly
    370                 375                 380


<210> 317
<211> 290
<212> DNA
<213> Corynebacterium glutamicum

<220>
<221> CDS
<222> (1)..(267)
<223> RXA02746

<400> 317
ggt gtg cag ggc atg act gtc acc gaa acc caa ggc ttt ggc cag cag      48
Gly Val Gln Gly Met Thr Val Thr Glu Thr Gln Gly Phe Gly Gln Gln
  1               5                  10                  15

aaa ggc cac acc gag gtg tac cgt ggt gct gaa tac gct gtc gat ttt      96
Lys Gly His Thr Glu Val Tyr Arg Gly Ala Glu Tyr Ala Val Asp Phe
             20                  25                  30

gtg cct aag gtc aag att gaa gtt att atc tcc gat gct cag gct gag     144
Val Pro Lys Val Lys Ile Glu Val Ile Ile Ser Asp Ala Gln Ala Glu
         35                  40                  45

gaa gtc atc aac att atc gtc gag acc gca cgc acc ggc aaa gtc ggc     192
Glu Val Ile Asn Ile Ile Val Glu Thr Ala Arg Thr Gly Lys Val Gly
     50                  55                  60

gac ggc aaa gtg tgg atg act aac atc gaa gag ctg gtt cgt gtt cgt     240
Asp Gly Lys Val Trp Met Thr Asn Ile Glu Glu Leu Val Arg Val Arg
 65                  70                  75                  80

acc ggt gag cgc ggc gaa gca gcc ctt taaaaactta tgaataatcc           287
Thr Gly Glu Arg Gly Glu Ala Ala Leu
                 85

agc                                                                 290


<210> 318
<211> 89
<212> PRT
<213> Corynebacterium glutamicum

<400> 318
Gly Val Gln Gly Met Thr Val Thr Glu Thr Gln Gly Phe Gly Gln Gln
  1               5                  10                  15

Lys Gly His Thr Glu Val Tyr Arg Gly Ala Glu Tyr Ala Val Asp Phe
             20                  25                  30

Val Pro Lys Val Lys Ile Glu Val Ile Ile Ser Asp Ala Gln Ala Glu
         35                  40                  45

Glu Val Ile Asn Ile Ile Val Glu Thr Ala Arg Thr Gly Lys Val Gly
    50                  55                  60

Asp Gly Lys Val Trp Met Thr Asn Ile Glu Glu Leu Val Arg Val Arg
65                  70                  75                  80

Thr Gly Glu Arg Gly Glu Ala Ala Leu
                85

<210> 319
<211> 902
<212> DNA
<213> Corynebacterium glutamicum

<220>
<221> CDS
<222> (1)..(879)
<223> RXA02745

<400> 319
gcc gga atc tcc ttc gaa gtc ccc cgc ggt caa gtt ttg gcc ctc ctg      48
Ala Gly Ile Ser Phe Glu Val Pro Arg Gly Gln Val Leu Ala Leu Leu
1               5                   10                  15

gga cct aat ggc gca ggc aaa acc acc acc att gaa atg tgc gaa ggt      96
Gly Pro Asn Gly Ala Gly Lys Thr Thr Thr Ile Glu Met Cys Glu Gly
            20                  25                  30

ttt acc gcc ccc acc tct ggc agc atc cga gtc ttg ggc atc gat cca      144
Phe Thr Ala Pro Thr Ser Gly Ser Ile Arg Val Leu Gly Ile Asp Pro
        35                  40                  45

gcc aca gaa cca gac cag gtg cgc cga cgc atc ggc atc atg ctt caa      192
Ala Thr Glu Pro Asp Gln Val Arg Arg Arg Ile Gly Ile Met Leu Gln
    50                  55                  60

ggt ggc ggt tcc tac agc gga atc cgc gtg ttt gaa atg ctc aag ctt      240
Gly Gly Gly Ser Tyr Ser Gly Ile Arg Val Phe Glu Met Leu Lys Leu
65                  70                  75                  80

gcg gcg tcc tac aac gac aac cca cac gat cct gaa tgg ctg ctt gat      288
Ala Ala Ser Tyr Asn Asp Asn Pro His Asp Pro Glu Trp Leu Leu Asp
                85                  90                  95

ctt gta gga ctg cgt gaa caa cgc aaa acc acc tac cga cgt ctg tca      336
Leu Val Gly Leu Arg Glu Gln Arg Lys Thr Thr Tyr Arg Arg Leu Ser
            100                 105                 110

ggt ggc caa cag caa cgc ctt tct ttg gcc tta gca tta att ggt cgc      384
Gly Gly Gln Gln Gln Arg Leu Ser Leu Ala Leu Ala Leu Ile Gly Arg
        115                 120                 125

cct gag att atc ttc ctc gac gaa ccc acc gct ggc atg gat gcg caa      432
Pro Glu Ile Ile Phe Leu Asp Glu Pro Thr Ala Gly Met Asp Ala Gln
    130                 135                 140

tca cgc aac atg gtg tgg gag ctt gtc aac gat ctc cgc cgc gac ggc      480
Ser Arg Asn Met Val Trp Glu Leu Val Asn Asp Leu Arg Arg Asp Gly
145                 150                 155                 160

gtc acc atc gtg ctc acc acc cac ctg atg gat gag gcc gaa gca cta      528
Val Thr Ile Val Leu Thr Thr His Leu Met Asp Glu Ala Glu Ala Leu
                165                 170                 175

gct gac cac gtg atc atc gtt gcc aac ggt caa atc ctt gcc agt ggc      576
Ala Asp His Val Ile Ile Val Ala Asn Gly Gln Ile Leu Ala Ser Gly
            180                 185                 190

aca cct gat gaa ctc act gcg caa cgc gat cat ctt gaa att aat gtc      624
Thr Pro Asp Glu Leu Thr Ala Gln Arg Asp His Leu Glu Ile Asn Val
        195                 200                 205

tcc gta gag acc acg agc ccg ctt gat ctt gat cgc ttg gtg gat gat      672
Ser Val Glu Thr Thr Ser Pro Leu Asp Leu Asp Arg Leu Val Asp Asp
    210                 215                 220

ctc agc agc tta aac atc ggt gat gtg aaa gca cga gcc aac cgg cca     720
Leu Ser Ser Leu Asn Ile Gly Asp Val Lys Ala Arg Ala Asn Arg Pro
225                 230                 235                 240

ctg cat tat tca ctt cgg acg caa caa gcc acc ccg gat tcc ttg gcg     768
Leu His Tyr Ser Leu Arg Thr Gln Gln Ala Thr Pro Asp Ser Leu Ala
                245                 250                 255

cac atc gtc cag gct gtc gcc cgc caa aac gtc atg att cgc tct ttg     816
His Ile Val Gln Ala Val Ala Arg Gln Asn Val Met Ile Arg Ser Leu
            260                 265                 270

gat acg gga cac cgc tca ttg gaa gat gtc ttc ctg gac atc acc gga     864
Asp Thr Gly His Arg Ser Leu Glu Asp Val Phe Leu Asp Ile Thr Gly
        275                 280                 285

aaa gaa ctg agg agt taacgcacac catgtctaaa cct                       902
Lys Glu Leu Arg Ser
    290


<210> 320
<211> 293
<212> PRT
<213> Corynebacterium glutamicum

<400> 320
Ala Gly Ile Ser Phe Glu Val Pro Arg Gly Gln Val Leu Ala Leu Leu
1               5                   10                  15

Gly Pro Asn Gly Ala Gly Lys Thr Thr Thr Ile Glu Met Cys Glu Gly
            20                  25                  30

Phe Thr Ala Pro Thr Ser Gly Ser Ile Arg Val Leu Gly Ile Asp Pro
        35                  40                  45

Ala Thr Glu Pro Asp Gln Val Arg Arg Arg Ile Gly Ile Met Leu Gln
    50                  55                  60

Gly Gly Gly Ser Tyr Ser Gly Ile Arg Val Phe Glu Met Leu Lys Leu
65                  70                  75                  80

Ala Ala Ser Tyr Asn Asp Asn Pro His Asp Pro Glu Trp Leu Leu Asp
                85                  90                  95

Leu Val Gly Leu Arg Glu Gln Arg Lys Thr Thr Tyr Arg Arg Leu Ser
            100                 105                 110

Gly Gly Gln Gln Gln Arg Leu Ser Leu Ala Leu Ala Leu Ile Gly Arg
        115                 120                 125

Pro Glu Ile Ile Phe Leu Asp Glu Pro Thr Ala Gly Met Asp Ala Gln
    130                 135                 140

Ser Arg Asn Met Val Trp Glu Leu Val Asn Asp Leu Arg Arg Asp Gly
145                 150                 155                 160

Val Thr Ile Val Leu Thr Thr His Leu Met Asp Glu Ala Glu Ala Leu
                165                 170                 175

Ala Asp His Val Ile Ile Val Ala Asn Gly Gln Ile Leu Ala Ser Gly
            180                 185                 190

Thr Pro Asp Glu Leu Thr Ala Gln Arg Asp His Leu Glu Ile Asn Val
        195                 200                 205

Ser Val Glu Thr Thr Ser Pro Leu Asp Leu Asp Arg Leu Val Asp Asp
    210                 215                 220

Leu Ser Ser Leu Asn Ile Gly Asp Val Lys Ala Arg Ala Asn Arg Pro
225                 230                 235                 240

Leu His Tyr Ser Leu Arg Thr Gln Gln Ala Thr Pro Asp Ser Leu Ala
                245                 250                 255

His Ile Val Gln Ala Val Ala Arg Gln Asn Val Met Ile Arg Ser Leu

260                 265                 270

Asp Thr Gly His Arg Ser Leu Glu Asp Val Phe Leu Asp Ile Thr Gly
        275                 280                 285

Lys Glu Leu Arg Ser
    290


<210> 321
<211> 486
<212> DNA
<213> Corynebacterium glutamicum

<220>
<221> CDS
<222> (101)..(463)
<223> RXN00820

<400> 321
acttccacca ccccaaacca tcgtttcttt cgaagacgca ccaaccctca ccggccagga 60

cctgggcttt tcgcagtggc gcactgtcac ccaggagatg gtg aac acc ttg gcg   115
                                           Val Asn Thr Leu Ala
                                            1               5

gac gca act gat gat cag cag tgg att cac act gat cct gag cgc gcc   163
Asp Ala Thr Asp Asp Gln Gln Trp Ile His Thr Asp Pro Glu Arg Ala
                10                  15                  20

aag gac ggt cct ttt ggt ggc gca att gcc cac ggt ttc ctc acc ttg   211
Lys Asp Gly Pro Phe Gly Gly Ala Ile Ala His Gly Phe Leu Thr Leu
            25                  30                  35

tcc atg atc att ccg ttc tgg ggc gag ctt ctc gat gtc acc ggc gtg   259
Ser Met Ile Ile Pro Phe Trp Gly Glu Leu Leu Asp Val Thr Gly Val
        40                  45                  50

acc acc aag gtg aac tat ggc ctg gat aag gtg cgt ttc acc tct ccc   307
Thr Thr Lys Val Asn Tyr Gly Leu Asp Lys Val Arg Phe Thr Ser Pro
    55                  60                  65

gtc aag gtc ggt tcc cgc atc cgc atg ggc gct gtg gtc cgt gag atc   355
Val Lys Val Gly Ser Arg Ile Arg Met Gly Ala Val Val Arg Glu Ile
70                  75                  80                  85

tct gag gtg aag ggc aat ggc ctg cac ctg gtc gcc gat ggc act att   403
Ser Glu Val Lys Gly Asn Gly Leu His Leu Val Ala Asp Gly Thr Ile
                90                  95                  100

gag atc gaa ggg cag gag cgc ccg gcc gtc gta gct acc ttc ctc acc   451
Glu Ile Glu Gly Gln Glu Arg Pro Ala Val Val Ala Thr Phe Leu Thr
            105                 110                 115

cgc ttc tac gct taaaagcttg cttctcgacg caa                         486
Arg Phe Tyr Ala
        120


<210> 322
<211> 121
<212> PRT
<213> Corynebacterium glutamicum

<400> 322
Val Asn Thr Leu Ala Asp Ala Thr Asp Asp Gln Gln Trp Ile His Thr
1               5                   10                  15

Asp Pro Glu Arg Ala Lys Asp Gly Pro Phe Gly Gly Ala Ile Ala His
            20                  25                  30

Gly Phe Leu Thr Leu Ser Met Ile Ile Pro Phe Trp Gly Glu Leu Leu
        35                  40                  45

Asp Val Thr Gly Val Thr Thr Lys Val Asn Tyr Gly Leu Asp Lys Val
    50                  55                  60

Arg Phe Thr Ser Pro Val Lys Val Gly Ser Arg Ile Arg Met Gly Ala
65                  70                  75                  80

Val Val Arg Glu Ile Ser Glu Val Lys Gly Asn Gly Leu His Leu Val
                85                  90                  95

Ala Asp Gly Thr Ile Glu Ile Glu Gly Gln Glu Arg Pro Ala Val Val
            100                 105                 110

Ala Thr Phe Leu Thr Arg Phe Tyr Ala
        115                 120


<210> 323
<211> 486
<212> DNA
<213> Corynebacterium glutamicum

<220>
<221> CDS
<222> (101)..(463)
<223> FRXA00820

<400> 323
acttccacca ccccaaacca tcgtttcttt cgaagacgca ccaaccctca ccggccagga 60

cctgggcttt tcgcagtggc gcactgtcac ccaggagatg gtg aac acc ttc gcg   115
                                           Val Asn Thr Phe Ala
                                           1               5

gac gca act gat gat cag cag tgg att cac act gat cct gag cgc gcc   163
Asp Ala Thr Asp Asp Gln Gln Trp Ile His Thr Asp Pro Glu Arg Ala
                10                  15                  20

aag gac ggt cct ttt ggt ggc gca att gcc cac ggt ttc ctc acc ttg   211
Lys Asp Gly Pro Phe Gly Gly Ala Ile Ala His Gly Phe Leu Thr Leu
            25                  30                  35

tcc atg atc att ccg ttc tgg ggc gag ctt ctc gat gtc acc ggc gtg   259
Ser Met Ile Ile Pro Phe Trp Gly Glu Leu Leu Asp Val Thr Gly Val
        40                  45                  50

acc acc aag gtg aac tat ggc ctg gat aag gtg cgt ttc acc tct ccc   307
Thr Thr Lys Val Asn Tyr Gly Leu Asp Lys Val Arg Phe Thr Ser Pro
    55                  60                  65

gtc aag gtc ggt tcc cgc atc cgc atg ggc gct gtg gtc cgt gag atc   355
Val Lys Val Gly Ser Arg Ile Arg Met Gly Ala Val Val Arg Glu Ile
70                  75                  80                  85

tct gag gtg aag ggc aat ggc ctg cac ctg gtc gcc gat ggc act att   403
Ser Glu Val Lys Gly Asn Gly Leu His Leu Val Ala Asp Gly Thr Ile
                90                  95                  100

gag atc gaa ggg cag gag cgc ccg gcc gtc gta gct acc ttc ctc acc   451
Glu Ile Glu Gly Gln Glu Arg Pro Ala Val Val Ala Thr Phe Leu Thr
            105                 110                 115

cgc ttc tac gct taaaagcttg cttctcgacg caa                         486
Arg Phe Tyr Ala
        120


<210> 324
<211> 121
<212> PRT
<213> Corynebacterium glutamicum

<400> 324
Val Asn Thr Phe Ala Asp Ala Thr Asp Asp Gln Gln Trp Ile His Thr
1               5                   10                  15

Asp Pro Glu Arg Ala Lys Asp Gly Pro Phe Gly Gly Ala Ile Ala His
            20                  25                  30

Gly Phe Leu Thr Leu Ser Met Ile Ile Pro Phe Trp Gly Glu Leu Leu
        35                  40                  45

Asp Val Thr Gly Val Thr Thr Lys Val Asn Tyr Gly Leu Asp Lys Val
    50                  55                  60

Arg Phe Thr Ser Pro Val Lys Val Gly Ser Arg Ile Arg Met Gly Ala
65                  70                  75                  80

Val Val Arg Glu Ile Ser Glu Val Lys Gly Asn Gly Leu His Leu Val
                85                  90                  95

Ala Asp Gly Thr Ile Glu Ile Glu Gly Gln Glu Arg Pro Ala Val Val
            100                 105                 110

Ala Thr Phe Leu Thr Arg Phe Tyr Ala
        115                 120


<210> 325
<211> 732
<212> DNA
<213> Corynebacterium glutamicum

<220>
<221> CDS
<222> (101)..(709)
<223> RXA01059

<400> 325
aattattagc tttccaatac aaaatttaaa tcccagagcg atctgcccca cacttacttg 60

atgcgggaac aaatttgaag gtttttcagt tgctataggt atg act aca gtt act   115
                                           Met Thr Thr Val Thr
                                           1               5

caa gac ctt cta gca ctt gac gaa gac gca cag aac ctc ctt ttc cgt   163
Gln Asp Leu Leu Ala Leu Asp Glu Asp Ala Gln Asn Leu Leu Phe Arg
                10                  15                  20

gag gct cgc acc gca aat gct ttc act gat gaa cca atc tct gac gag   211
Glu Ala Arg Thr Ala Asn Ala Phe Thr Asp Glu Pro Ile Ser Asp Glu
            25                  30                  35

cag atc gaa gca atc ttc gac cta gtt aag tgg gca cca acc gca atg   259
Gln Ile Glu Ala Ile Phe Asp Leu Val Lys Trp Ala Pro Thr Ala Met
        40                  45                  50

aac tcc cag cct ctg cgc gtg gta att gtt cgt tcc gaa gaa gcc aaa   307
Asn Ser Gln Pro Leu Arg Val Val Ile Val Arg Ser Glu Glu Ala Lys
    55                  60                  65

gct cgc ctc gtg cca ttg atg gca gaa ggc aac cag gcc aag gtt gct   355
Ala Arg Leu Val Pro Leu Met Ala Glu Gly Asn Gln Ala Lys Val Ala
70                  75                  80                  85

gca gct cct gcg gtc gca ctt ctt gca gcc gac atc gac ttc cac gaa   403
Ala Ala Pro Ala Val Ala Leu Leu Ala Ala Asp Ile Asp Phe His Glu
                90                  95                  100

gaa atg ccc aag ctc ttc cca cct ttc cca ggc gca cgc gac atg ttc   451
Glu Met Pro Lys Leu Phe Pro Pro Phe Pro Gly Ala Arg Asp Met Phe
            105                 110                 115

gaa gcc gat gaa gct tca cgt gct tcc tcc gca gaa ctc aat gct ggc   499
Glu Ala Asp Glu Ala Ser Arg Ala Ser Ser Ala Glu Leu Asn Ala Gly
        120                 125                 130

ctt cag atc gga tac gcc atc atc ggt atc cgc gca gca ggt ctc gcc   547
Leu Gln Ile Gly Tyr Ala Ile Ile Gly Ile Arg Ala Ala Gly Leu Ala
    135                 140                 145

gct ggc cca atg acc ggc atg gat gca gac gct atc tcc aag gag ttc   595
Ala Gly Pro Met Thr Gly Met Asp Ala Asp Ala Ile Ser Lys Glu Phe
150                 155                 160                 165

ttc cca gac ggc cgc cac cgc gtt ctg gtt gcc atc aac atg ggt aag   643
Phe Pro Asp Gly Arg His Arg Val Leu Val Ala Ile Asn Met Gly Lys
                170                 175                 180

cca gct gac aat gct tgg tac gac cgc ctg cca cgc ctt gag cag gac 691
Pro Ala Asp Asn Ala Trp Tyr Asp Arg Leu Pro Arg Leu Glu Gln Asp
            185                 190                 195

gaa gtt gtc gaa acc ctc tagaaaccac tctagaaata gct 732
Glu Val Val Glu Thr Leu
        200

<210> 326
<211> 203
<212> PRT
<213> Corynebacterium glutamicum

<400> 326
Met Thr Thr Val Thr Gln Asp Leu Leu Ala Leu Asp Glu Asp Ala Gln
1               5                   10                  15

Asn Leu Leu Phe Arg Glu Ala Arg Thr Ala Asn Ala Phe Thr Asp Glu
            20                  25                  30

Pro Ile Ser Asp Glu Gln Ile Glu Ala Ile Phe Asp Leu Val Lys Trp
        35                  40                  45

Ala Pro Thr Ala Met Asn Ser Gln Pro Leu Arg Val Val Ile Val Arg
    50                  55                  60

Ser Glu Glu Ala Lys Ala Arg Leu Val Pro Leu Met Ala Glu Gly Asn
65                  70                  75                  80

Gln Ala Lys Val Ala Ala Ala Pro Ala Val Ala Leu Leu Ala Ala Asp
                85                  90                  95

Ile Asp Phe His Glu Glu Met Pro Lys Leu Phe Pro Pro Phe Pro Gly
            100                 105                 110

Ala Arg Asp Met Phe Glu Ala Asp Glu Ala Ser Arg Ala Ser Ser Ala
        115                 120                 125

Glu Leu Asn Ala Gly Leu Gln Ile Gly Tyr Ala Ile Ile Gly Ile Arg
    130                 135                 140

Ala Ala Gly Leu Ala Ala Gly Pro Met Thr Gly Met Asp Ala Asp Ala
145                 150                 155                 160

Ile Ser Lys Glu Phe Phe Pro Asp Gly Arg His Arg Val Leu Val Ala
                165                 170                 175

Ile Asn Met Gly Lys Pro Ala Asp Asn Ala Trp Tyr Asp Arg Leu Pro
            180                 185                 190

Arg Leu Glu Gln Asp Glu Val Val Glu Thr Leu
        195                 200

<210> 327
<211> 1053
<212> DNA
<213> Corynebacterium glutamicum

<220>
<221> CDS
<222> (101)..(1030)
<223> RXN01386

<400> 327
ctctattgtg gtacgcacca tgactgctca accagcccac gagccgccaa ggcatctccg 60

gttcagtacg agcgggatcc tcccggacaa tcgcgtccaa atg tgg gag ggc cac 115
Met Trp Glu Gly His
1               5

aat gct cgc gca ttg ctt ccg ctc gac att aga acc att gac gat cgc 163
Asn Ala Arg Ala Leu Leu Pro Leu Asp Ile Arg Thr Ile Asp Asp Arg
                10                  15                  20

ccc atg cag gcc tcc gaa acc aac ctg cac ctc cca tca atg cgg atg 211
Pro Met Gln Ala Ser Glu Thr Asn Leu His Leu Pro Ser Met Arg Met
            25                  30                  35

gcg agc gta ttc ggg act tcg caa ttt gtc gag cgt tca gag agt ttc 259
Ala Ser Val Phe Gly Thr Ser Gln Phe Val Glu Arg Ser Glu Ser Phe
        40                  45                  50

atc tca gaa aac ccc acg ggt gtg gtt gcg atc ttc ttt gcg act gaa 307
Ile Ser Glu Asn Pro Thr Gly Val Val Ala Ile Phe Phe Ala Thr Glu
    55                  60                  65

ggt gaa gca gtc ttc ttc cac cgt ggt gga cat gta gcg ctt cgg cca 355
Gly Glu Ala Val Phe Phe His Arg Gly Gly His Val Ala Leu Arg Pro
70                  75                  80                  85

ggt cag gcc att gtt tac gac gcc gat agg cca ttc ctc cgc gga ttc 403
Gly Gln Ala Ile Val Tyr Asp Ala Asp Arg Pro Phe Leu Arg Gly Phe
                90                  95                  100

aac aat cgc ttc cgc gag cta gtt ctc acc atc ccg aag cag cgc tac 451
Asn Asn Arg Phe Arg Glu Leu Val Leu Thr Ile Pro Lys Gln Arg Tyr
            105                 110                 115

ctt gaa att gtt ggc tca aaa ggc cct gag ctt ccc gct att ttt gag 499
Leu Glu Ile Val Gly Ser Lys Gly Pro Glu Leu Pro Ala Ile Phe Glu
        120                 125                 130

ttc gga gca aca gga acc gcc aat gaa caa gct tta gcg cga cta gtt 547
Phe Gly Ala Thr Gly Thr Ala Asn Glu Gln Ala Leu Ala Arg Leu Val
    135                 140                 145

cag gaa tct cta cac agg att gaa agt ggc gag ccg aag cat atc gat 595
Gln Glu Ser Leu His Arg Ile Glu Ser Gly Glu Pro Lys His Ile Asp
150                 155                 160                 165

tcc agt gga cct tta gga aaa ccg tgg agc gat atc gag cac gag gcc 643
Ser Ser Gly Pro Leu Gly Lys Pro Trp Ser Asp Ile Glu His Glu Ala
                170                 175                 180

cac gga ctt atc cgc aat gta ctt ggc gac gcc aca agt agc gaa gaa 691
His Gly Leu Ile Arg Asn Val Leu Gly Asp Ala Thr Ser Ser Glu Glu
            185                 190                 195

ggc tta att tct gca gcc cag aga ttt att gac atc aat att tcc gaa 739
Gly Leu Ile Ser Ala Ala Gln Arg Phe Ile Asp Ile Asn Ile Ser Glu
        200                 205                 210

agt gac tta caa gcg tcg cgg att gct gca gcc gtg gga atc agc gaa 787
Ser Asp Leu Gln Ala Ser Arg Ile Ala Ala Ala Val Gly Ile Ser Glu
    215                 220                 225

cgc caa cta agt cga atc ttc tca gac tca gga caa act atc gga cgc 835
Arg Gln Leu Ser Arg Ile Phe Ser Asp Ser Gly Gln Thr Ile Gly Arg
230                 235                 240                 245

tac gtc cta aac acc cga ctg gat ttt gca aag gaa gcg ctg tcg aca 883
Tyr Val Leu Asn Thr Arg Leu Asp Phe Ala Lys Glu Ala Leu Ser Thr
                250                 255                 260

ccg gag cga gac aag gtt tcg gtc agt gag atc ggt aag cgc ttt ggg 931
Pro Glu Arg Asp Lys Val Ser Val Ser Glu Ile Gly Lys Arg Phe Gly
            265                 270                 275

ttc gct tcc cca agt cat ttc agt cgc acc ttc cgc gag cgg ttt gaa 979
Phe Ala Ser Pro Ser His Phe Ser Arg Thr Phe Arg Glu Arg Phe Glu
        280                 285                 290

atg acg ccg ctt caa tgg agg aag gaa tcg cag cgt caa tcc ttt caa 1027
Met Thr Pro Leu Gln Trp Arg Lys Glu Ser Gln Arg Gln Ser Phe Gln
    295                 300                 305

gag tgaggttttt gttctcaggc gga 1053
Glu
310

<210> 328
<211> 310
<212> PRT
<213> Corynebacterium glutamicum

<400> 328
Met Trp Glu Gly His Asn Ala Arg Ala Leu Leu Pro Leu Asp Ile Arg
1               5                   10                  15

Thr Ile Asp Asp Arg Pro Met Gln Ala Ser Glu Thr Asn Leu His Leu
            20                  25                  30

Pro Ser Met Arg Met Ala Ser Val Phe Gly Thr Ser Gln Phe Val Glu
        35                  40                  45

Arg Ser Glu Ser Phe Ile Ser Glu Asn Pro Thr Gly Val Val Ala Ile
    50                  55                  60

Phe Phe Ala Thr Glu Gly Glu Ala Val Phe Phe His Arg Gly Gly His
65                  70                  75                  80

Val Ala Leu Arg Pro Gly Gln Ala Ile Val Tyr Asp Ala Asp Arg Pro
                85                  90                  95

Phe Leu Arg Gly Phe Asn Asn Arg Phe Arg Glu Leu Val Leu Thr Ile
            100                 105                 110

Pro Lys Gln Arg Tyr Leu Glu Ile Val Gly Ser Lys Gly Pro Glu Leu
        115                 120                 125

Pro Ala Ile Phe Glu Phe Gly Ala Thr Gly Thr Ala Asn Glu Gln Ala
    130                 135                 140

Leu Ala Arg Leu Val Gln Glu Ser Leu His Arg Ile Glu Ser Gly Glu
145                 150                 155                 160

Pro Lys His Ile Asp Ser Ser Gly Pro Leu Gly Lys Pro Trp Ser Asp
                165                 170                 175

Ile Glu His Glu Ala His Gly Leu Ile Arg Asn Val Leu Gly Asp Ala
            180                 185                 190

Thr Ser Ser Glu Glu Gly Leu Ile Ser Ala Ala Gln Arg Phe Ile Asp
        195                 200                 205

Ile Asn Ile Ser Glu Ser Asp Leu Gln Ala Ser Arg Ile Ala Ala Ala
    210                 215                 220

Val Gly Ile Ser Glu Arg Gln Leu Ser Arg Ile Phe Ser Asp Ser Gly
225                 230                 235                 240

Gln Thr Ile Gly Arg Tyr Val Leu Asn Thr Arg Leu Asp Phe Ala Lys
                245                 250                 255

Glu Ala Leu Ser Thr Pro Glu Arg Asp Lys Val Ser Val Ser Glu Ile
            260                 265                 270

Gly Lys Arg Phe Gly Phe Ala Ser Pro Ser His Phe Ser Arg Thr Phe
        275                 280                 285

Arg Glu Arg Phe Glu Met Thr Pro Leu Gln Trp Arg Lys Glu Ser Gln
    290                 295                 300

Arg Gln Ser Phe Gln Glu
305                 310


<210> 329
<211> 1806
<212> DNA
<213> Corynebacterium glutamicum

<220>
<221> CDS
<222> (101)..(1783)
<223> RXN00073

<400> 329
gaatctaatg gttggtctag acagagcggt acgtctaagt ttgcggatag atcaaaccga 60

gtgacatgta cttcactagc tctttaagga ttaactcccc atg aca aca acc acc 115
Met Thr Thr Thr Thr
1               5

gga agt gcc cgg cca gca cgt gcc gcc agg aag cct aag ccc gaa ggc 163
Gly Ser Ala Arg Pro Ala Arg Ala Ala Arg Lys Pro Lys Pro Glu Gly
                10                  15                  20

caa tgg aaa atc gac ggc acc gag ccg ctt aac cat gcc gag gaa att 211
Gln Trp Lys Ile Asp Gly Thr Glu Pro Leu Asn His Ala Glu Glu Ile
            25                  30                  35

aag caa gaa gaa ccc gct ttt gct gtc aag cag cgg gtc att gat att 259
Lys Gln Glu Glu Pro Ala Phe Ala Val Lys Gln Arg Val Ile Asp Ile
        40                  45                  50

tac tcc aag cag ggt ttt tct tcc att gca ccg gat gac att gcc cca 307
Tyr Ser Lys Gln Gly Phe Ser Ser Ile Ala Pro Asp Asp Ile Ala Pro
    55                  60                  65

cgc ttt aag tgg ttg ggc att tac acc cag cgt aag cag gat ctg ggc 355
Arg Phe Lys Trp Leu Gly Ile Tyr Thr Gln Arg Lys Gln Asp Leu Gly
70                  75                  80                  85

ggt gaa ctg acc ggt cag ctt cct gat gat gag ctg cag gat gag tac 403
Gly Glu Leu Thr Gly Gln Leu Pro Asp Asp Glu Leu Gln Asp Glu Tyr
                90                  95                  100

ttc atg atg cgt gtg cgt ttt gat ggc gga ctg gct tcc cct gag cgc 451
Phe Met Met Arg Val Arg Phe Asp Gly Gly Leu Ala Ser Pro Glu Arg
            105                 110                 115

ctg cgt gcc gtg ggt gaa att tct agg gat tat gct cgt tcc acc gcg 499
Leu Arg Ala Val Gly Glu Ile Ser Arg Asp Tyr Ala Arg Ser Thr Ala
        120                 125                 130

gac ttc acc gac cgc cag aac att cag ctg cac tgg att cgt att gaa 547
Asp Phe Thr Asp Arg Gln Asn Ile Gln Leu His Trp Ile Arg Ile Glu
    135                 140                 145

gat gtg cct gcg atc tgg gag aag cta gaa acc gtc gga ctg tcc acc 595
Asp Val Pro Ala Ile Trp Glu Lys Leu Glu Thr Val Gly Leu Ser Thr
150                 155                 160                 165

atg ctt ggt tgc ggt gac gtt cca cgt gtt atc ttg ggc tcc cca gtt 643
Met Leu Gly Cys Gly Asp Val Pro Arg Val Ile Leu Gly Ser Pro Val
                170                 175                 180

tct ggc gta gct gct gaa gag ctg atc gat gcc acc ccg gct atc gat 691
Ser Gly Val Ala Ala Glu Glu Leu Ile Asp Ala Thr Pro Ala Ile Asp
            185                 190                 195

gcg att cgt gag cgc tac cta gac aag gaa gag ttc cac aac ctt cct 739
Ala Ile Arg Glu Arg Tyr Leu Asp Lys Glu Glu Phe His Asn Leu Pro
        200                 205                 210

cgt aag ttt aag act gct atc act ggc aac cag cgc cag gat gtt acc 787
Arg Lys Phe Lys Thr Ala Ile Thr Gly Asn Gln Arg Gln Asp Val Thr
    215                 220                 225

cac gaa atc cag gac gtt tcc ttc gtt cct tcg att cac cca gaa ttc 835
His Glu Ile Gln Asp Val Ser Phe Val Pro Ser Ile His Pro Glu Phe
230                 235                 240                 245

ggc cca gga ttt gag tgc ttt gtg ggc ggt ggc ctg tcc acc aac cca 883
Gly Pro Gly Phe Glu Cys Phe Val Gly Gly Gly Leu Ser Thr Asn Pro
                250                 255                 260

atg ctt gct cag cca ctt ggt tct tgg att cca ctt gat gag gtt cca 931
Met Leu Ala Gln Pro Leu Gly Ser Trp Ile Pro Leu Asp Glu Val Pro
            265                 270                 275

gaa gtg tgg gct ggc gtc gcc gga att ttc cgc gac tac ggc ttc cga 979
Glu Val Trp Ala Gly Val Ala Gly Ile Phe Arg Asp Tyr Gly Phe Arg

280                 285                 290

cgc ctg cgt aac cgt gct cgc ctc aag ttc ttg gtg gca cag tgg ggt     1027
Arg Leu Arg Asn Arg Ala Arg Leu Lys Phe Leu Val Ala Gln Trp Gly
    295                 300                 305

att gag aag ttc cgt gaa gtt ctt gag acc gaa tac ctc gag cgc aag     1075
Ile Glu Lys Phe Arg Glu Val Leu Glu Thr Glu Tyr Leu Glu Arg Lys
310                 315                 320                 325

ctg atc gat ggc cca gtt gtt acc acc aac cct ggc tac cgt gac cac     1123
Leu Ile Asp Gly Pro Val Val Thr Thr Asn Pro Gly Tyr Arg Asp His
                330                 335                 340

att ggc att cac cca caa aag gac ggc aag ttc tac ctc ggt gtg aag     1171
Ile Gly Ile His Pro Gln Lys Asp Gly Lys Phe Tyr Leu Gly Val Lys
            345                 350                 355

cca acc gtt gga cac acc acc ggt gag cag ctc att gcc att gct gat     1219
Pro Thr Val Gly His Thr Thr Gly Glu Gln Leu Ile Ala Ile Ala Asp
        360                 365                 370

gtt gca gaa aag cac ggc atc acc agg att cgt acc acg gcg gaa aag     1267
Val Ala Glu Lys His Gly Ile Thr Arg Ile Arg Thr Thr Ala Glu Lys
    375                 380                 385

gaa ctg ctc ttc ctc gat att gag aga aag aac ctt act acc gtt gca     1315
Glu Leu Leu Phe Leu Asp Ile Glu Arg Lys Asn Leu Thr Thr Val Ala
390                 395                 400                 405

cgc gac ctg gat gaa atc gga ctg tac tct tca cct tcc gag ttc cgc     1363
Arg Asp Leu Asp Glu Ile Gly Leu Tyr Ser Ser Pro Ser Glu Phe Arg
                410                 415                 420

cgc ggc atc att tcc tgc acc ggc ttg gag ttc tgc aag ctt gcg cac     1411
Arg Gly Ile Ile Ser Cys Thr Gly Leu Glu Phe Cys Lys Leu Ala His
            425                 430                 435

gca acc acc aag tca cga gca att gag ctt gtc gac gaa ctg gaa gag     1459
Ala Thr Thr Lys Ser Arg Ala Ile Glu Leu Val Asp Glu Leu Glu Glu
        440                 445                 450

cgc ctc ggc gat ttg gat gtt ccc atc aag att gca ctg aac ggt tgc     1507
Arg Leu Gly Asp Leu Asp Val Pro Ile Lys Ile Ala Leu Asn Gly Cys
    455                 460                 465

cct aac tct tgt gca cgc acc cag gtt tcc gac atc gga ttc aag gga     1555
Pro Asn Ser Cys Ala Arg Thr Gln Val Ser Asp Ile Gly Phe Lys Gly
470                 475                 480                 485

cag acc gtc act gat gct gac ggc aac cgc gtt gaa ggt ttc cag gtt     1603
Gln Thr Val Thr Asp Ala Asp Gly Asn Arg Val Glu Gly Phe Gln Val
                490                 495                 500

cac ctg ggc ggt tcc atg aac ttg gat cca aac ttc gga cgc aag ctc     1651
His Leu Gly Gly Ser Met Asn Leu Asp Pro Asn Phe Gly Arg Lys Leu
            505                 510                 515

aag ggc cac aag gtt att gcc gat gaa gtg gga gag tac gtc act cgc     1699
Lys Gly His Lys Val Ile Ala Asp Glu Val Gly Glu Tyr Val Thr Arg
        520                 525                 530

gtt gtt acc cac ttc aag gaa cag cgc cac gag gac gag cac ttc cgc     1747
Val Val Thr His Phe Lys Glu Gln Arg His Glu Asp Glu His Phe Arg
    535                 540                 545

gat tgg gtc cag cgg gcc gct gag gaa gat ttg gtg tgagtcttcg          1793
Asp Trp Val Gln Arg Ala Ala Glu Glu Asp Leu Val
550                 555                 560

gaggaaaccc aat                                                      1806

<210> 330
<211> 561
<212> PRT
<213> Corynebacterium glutamicum

<400> 330
Met Thr Thr Thr Thr Gly Ser Ala Arg Pro Ala Arg Ala Ala Arg Lys
1               5                   10                  15

Pro Lys Pro Glu Gly Gln Trp Lys Ile Asp Gly Thr Glu Pro Leu Asn
            20                  25                  30

His Ala Glu Glu Ile Lys Gln Glu Glu Pro Ala Phe Ala Val Lys Gln
        35                  40                  45

Arg Val Ile Asp Ile Tyr Ser Lys Gln Gly Phe Ser Ser Ile Ala Pro
    50                  55                  60

Asp Asp Ile Ala Pro Arg Phe Lys Trp Leu Gly Ile Tyr Thr Gln Arg
65                  70                  75                  80

Lys Gln Asp Leu Gly Gly Glu Leu Thr Gly Gln Leu Pro Asp Asp Glu
                85                  90                  95

Leu Gln Asp Glu Tyr Phe Met Met Arg Val Arg Phe Asp Gly Gly Leu
            100                 105                 110

Ala Ser Pro Glu Arg Leu Arg Ala Val Gly Glu Ile Ser Arg Asp Tyr
        115                 120                 125

Ala Arg Ser Thr Ala Asp Phe Thr Asp Arg Gln Asn Ile Gln Leu His
    130                 135                 140

Trp Ile Arg Ile Glu Asp Val Pro Ala Ile Trp Glu Lys Leu Glu Thr
145                 150                 155                 160

Val Gly Leu Ser Thr Met Leu Gly Cys Gly Asp Val Pro Arg Val Ile
                165                 170                 175

Leu Gly Ser Pro Val Ser Gly Val Ala Ala Glu Glu Leu Ile Asp Ala
            180                 185                 190

Thr Pro Ala Ile Asp Ala Ile Arg Glu Arg Tyr Leu Asp Lys Glu Glu
        195                 200                 205

Phe His Asn Leu Pro Arg Lys Phe Lys Thr Ala Ile Thr Gly Asn Gln
    210                 215                 220

Arg Gln Asp Val Thr His Glu Ile Gln Asp Val Ser Phe Val Pro Ser
225                 230                 235                 240

Ile His Pro Glu Phe Gly Pro Gly Phe Glu Cys Phe Val Gly Gly Gly
                245                 250                 255

Leu Ser Thr Asn Pro Met Leu Ala Gln Pro Leu Gly Ser Trp Ile Pro
            260                 265                 270

Leu Asp Glu Val Pro Glu Val Trp Ala Gly Val Ala Gly Ile Phe Arg
        275                 280                 285

Asp Tyr Gly Phe Arg Arg Leu Arg Asn Arg Ala Arg Leu Lys Phe Leu
    290                 295                 300

Val Ala Gln Trp Gly Ile Glu Lys Phe Arg Glu Val Leu Glu Thr Glu
305                 310                 315                 320

Tyr Leu Glu Arg Lys Leu Ile Asp Gly Pro Val Val Thr Thr Asn Pro
                325                 330                 335

Gly Tyr Arg Asp His Ile Gly Ile His Pro Gln Lys Asp Gly Lys Phe
            340                 345                 350

Tyr Leu Gly Val Lys Pro Thr Val Gly His Thr Thr Gly Glu Gln Leu
        355                 360                 365

Ile Ala Ile Ala Asp Val Ala Glu Lys His Gly Ile Thr Arg Ile Arg
    370                 375                 380

Thr Thr Ala Glu Lys Glu Leu Leu Phe Leu Asp Ile Glu Arg Lys Asn
385                 390                 395                 400

Leu Thr Thr Val Ala Arg Asp Leu Asp Glu Ile Gly Leu Tyr Ser Ser
                405                 410                 415

Pro Ser Glu Phe Arg Arg Gly Ile Ile Ser Cys Thr Gly Leu Glu Phe
            420                 425                 430

Cys Lys Leu Ala His Ala Thr Thr Lys Ser Arg Ala Ile Glu Leu Val
        435                 440                 445

Asp Glu Leu Glu Glu Arg Leu Gly Asp Leu Asp Val Pro Ile Lys Ile
    450                 455                 460

Ala Leu Asn Gly Cys Pro Asn Ser Cys Ala Arg Thr Gln Val Ser Asp
465                 470                 475                 480

Ile Gly Phe Lys Gly Gln Thr Val Thr Asp Ala Asp Gly Asn Arg Val
                485                 490                 495

Glu Gly Phe Gln Val His Leu Gly Gly Ser Met Asn Leu Asp Pro Asn
            500                 505                 510

Phe Gly Arg Lys Leu Lys Gly His Lys Val Ile Ala Asp Glu Val Gly
        515                 520                 525

Glu Tyr Val Thr Arg Val Val Thr His Phe Lys Glu Gln Arg His Glu
    530                 535                 540

Asp Glu His Phe Arg Asp Trp Val Gln Arg Ala Ala Glu Glu Asp Leu
545                 550                 555                 560

Val

<210> 331
<211> 373
<212> DNA
<213> Corynebacterium glutamicum

<220>
<221> CDS
<222> (101)..(373)
<223> RXN03131

<400> 331
aacgcggctc gtgaagatta cctgaaaaac caagccctcc agcgtccgct gctcggctaa 60

accagttggc taaaccaaaa cgtatttaag gagaaacacc atg acc act tct gta    115
                                           Met Thr Thr Ser Val
                                           1               5

cct gca tcc acc aaa gct tta tct gtg gct ggc gaa aac cca ggc ctg    163
Pro Ala Ser Thr Lys Ala Leu Ser Val Ala Gly Glu Asn Pro Gly Leu
                10                  15                  20

cgc atc ggc acc gca cct gac tcg tgg ggc gtg tgg ttc cca gag gat    211
Arg Ile Gly Thr Ala Pro Asp Ser Trp Gly Val Trp Phe Pro Glu Asp
            25                  30                  35

cca aag cag atc cct tgg gag cgt ttt ctc tac gag gtc gtg aaa gct    259
Pro Lys Gln Ile Pro Trp Glu Arg Phe Leu Tyr Glu Val Val Lys Ala
        40                  45                  50

ggc tac acc tgg atc gag ctt ggc cca tac ggc tac ctg cca acc gat    307
Gly Tyr Thr Trp Ile Glu Leu Gly Pro Tyr Gly Tyr Leu Pro Thr Asp
    55                  60                  65

gcc aac cag ctt gaa gat gaa ctg ggc aag cgc ggc ctg aag ctg tcc    355
Ala Asn Gln Leu Glu Asp Glu Leu Gly Lys Arg Gly Leu Lys Leu Ser
70                  75                  80                  85

gct ggc acc gag ttc acc                                            373
Ala Gly Thr Glu Phe Thr
                90

<210> 332

<211> 91
<212> PRT
<213> Corynebacterium glutamicum

<400> 332
Met Thr Thr Ser Val Pro Ala Ser Thr Lys Ala Leu Ser Val Ala Gly
1               5                   10                  15

Glu Asn Pro Gly Leu Arg Ile Gly Thr Ala Pro Asp Ser Trp Gly Val
            20                  25                  30

Trp Phe Pro Glu Asp Pro Lys Gln Ile Pro Trp Glu Arg Phe Leu Tyr
        35                  40                  45

Glu Val Val Lys Ala Gly Tyr Thr Trp Ile Glu Leu Gly Pro Tyr Gly
    50                  55                  60

Tyr Leu Pro Thr Asp Ala Asn Gln Leu Glu Asp Glu Leu Gly Lys Arg
65                  70                  75                  80

Gly Leu Lys Leu Ser Ala Gly Thr Glu Phe Thr
                85                  90


<210> 333
<211> 549
<212> DNA
<213> Corynebacterium glutamicum

<220>
<221> CDS
<222> (101)..(526)
<223> RXS00153

<400> 333
cctcgatttg agtaaagagg acgtcctcgc ctgatttttc gggtgtgttt ttgcgtggcg 60

agccctgcct ggcccttcca aattatgtag ggtggcctgc gtg gga gca ata att    115
                                            Val Gly Ala Ile Ile
                                            1               5

tgg ttt atc gga gca ttg gtt ctt gct ggc ttg gaa ttg gca gta ggt    163
Trp Phe Ile Gly Ala Leu Val Leu Ala Gly Leu Glu Leu Ala Val Gly
                10                  15                  20

gag ttc acc tta ttg atg ctc ggc ggt gca gct ttg gca acc gcc ggc    211
Glu Phe Thr Leu Leu Met Leu Gly Gly Ala Ala Leu Ala Thr Ala Gly
            25                  30                  35

gtg gca ctc atc ggt gtc cca gta tgg gct gaa ttt gtc acc ttc gcg    259
Val Ala Leu Ile Gly Val Pro Val Trp Ala Glu Phe Val Thr Phe Ala
        40                  45                  50

gtg gcc tca gct gct cta ctg atg ttc att agg ccg gcc att aga aag    307
Val Ala Ser Ala Ala Leu Leu Met Phe Ile Arg Pro Ala Ile Arg Lys
    55                  60                  65

cgt ctg ctg aaa cca aag gtt ctg gac tct tca cca cga gca ctt gtt    355
Arg Leu Leu Lys Pro Lys Val Leu Asp Ser Ser Pro Arg Ala Leu Val
70                  75                  80                  85

ggc cac cgt gct gaa gtg ctc gaa gat gtc gga gcg acc agc ggg cag    403
Gly His Arg Ala Glu Val Leu Glu Asp Val Gly Ala Thr Ser Gly Gln
                90                  95                  100

gtc cgc ctg gat ggt tca att tgg tcc gcc cgc agc atg gat ccc aca    451
Val Arg Leu Asp Gly Ser Ile Trp Ser Ala Arg Ser Met Asp Pro Thr
            105                 110                 115

cac acc ttc gcg gaa ggt gaa att gtc agt gtc att gat atc caa ggc    499
His Thr Phe Ala Glu Gly Glu Ile Val Ser Val Ile Asp Ile Gln Gly
        120                 125                 130

acg acc gcg att gta tgg aaa gaa gcc taaattttta acaatcaaat          546
Thr Thr Ala Ile Val Trp Lys Glu Ala
    135                 140

agt                                                                    549

<210> 334
<211> 142
<212> PRT
<213> Corynebacterium glutamicum

<400> 334
Val Gly Ala Ile Ile Trp Phe Ile Gly Ala Leu Val Leu Ala Gly Leu
1               5                   10                  15

Glu Leu Ala Val Gly Glu Phe Thr Leu Leu Met Leu Gly Gly Ala Ala
            20                  25                  30

Leu Ala Thr Ala Gly Val Ala Leu Ile Gly Val Pro Val Trp Ala Glu
        35                  40                  45

Phe Val Thr Phe Ala Val Ala Ser Ala Ala Leu Leu Met Phe Ile Arg
    50                  55                  60

Pro Ala Ile Arg Lys Arg Leu Leu Lys Pro Lys Val Leu Asp Ser Ser
65                  70                  75                  80

Pro Arg Ala Leu Val Gly His Arg Ala Glu Val Leu Glu Asp Val Gly
                85                  90                  95

Ala Thr Ser Gly Gln Val Arg Leu Asp Gly Ser Ile Trp Ser Ala Arg
            100                 105                 110

Ser Met Asp Pro Thr His Thr Phe Ala Glu Gly Glu Ile Val Ser Val
        115                 120                 125

Ile Asp Ile Gln Gly Thr Thr Ala Ile Val Trp Lys Glu Ala
    130                 135                 140


<210> 335
<211> 509
<212> DNA
<213> Corynebacterium glutamicum

<220>
<221> CDS
<222> (1)..(486)
<223> RXN01716

<400> 335
gaa gtc act cct gag gga ttc aaa gag atc acc cgt gaa aac acc atc      48
Glu Val Thr Pro Glu Gly Phe Lys Glu Ile Thr Arg Glu Asn Thr Ile
1               5                   10                  15

gtt cgc ctg ggc aaa ggc gtc gac gcc acc ggt cag cta gac ccc gag      96
Val Arg Leu Gly Lys Gly Val Asp Ala Thr Gly Gln Leu Asp Pro Glu
            20                  25                  30

gca atc gag cgc act cgt gtc gct ttg gaa aac tac gtt gaa ctc atg      144
Ala Ile Glu Arg Thr Arg Val Ala Leu Glu Asn Tyr Val Glu Leu Met
        35                  40                  45

gaa acc cat ggg gta gag gcc gta cga atg gtt gcc acc tcc gca acc      192
Glu Thr His Gly Val Glu Ala Val Arg Met Val Ala Thr Ser Ala Thr
    50                  55                  60

cgc gat gcg tcc aac cgc gat gaa ttc ttt tcg atg acc cgc cag ctt      240
Arg Asp Ala Ser Asn Arg Asp Glu Phe Phe Ser Met Thr Arg Gln Leu
65                  70                  75                  80

ctg tcc aag atc cgt cct gga tac caa gct gaa gta att tcc ggc gaa      288
Leu Ser Lys Ile Arg Pro Gly Tyr Gln Ala Glu Val Ile Ser Gly Glu
                85                  90                  95

gag gaa gct ctg ctg tcc ttc cga ggt gca atc gtt gac ctg cct gaa      336
Glu Glu Ala Leu Leu Ser Phe Arg Gly Ala Ile Val Asp Leu Pro Glu
            100                 105                 110

gac caa ggt cct ttc tgt gtt atc gac ctt ggc ggt gga tcc act gag      384

Asp Gln Gly Pro Phe Cys Val Ile Asp Leu Gly Gly Gly Ser Thr Glu
        115                 120                 125

ttc atc gtt ggc acc tac gac ggt gaa atc cta ggc tcc cac tca acc 432
Phe Ile Val Gly Thr Tyr Asp Gly Glu Ile Leu Gly Ser His Ser Thr
    130                 135                 140

caa atg gga tgc gtg cgc ctg acc gaa cga atc atg cgc agc gac cca 480
Gln Met Gly Cys Val Arg Leu Thr Glu Arg Ile Met Arg Ser Asp Pro
145                 150                 155                 160

ccc gac tgaaaccgaa gtggaaatcg ccc 509
Pro Asp

<210> 336
<211> 162
<212> PRT
<213> Corynebacterium glutamicum

<400> 336
Glu Val Thr Pro Glu Gly Phe Lys Glu Ile Thr Arg Glu Asn Thr Ile
1               5                   10                  15

Val Arg Leu Gly Lys Gly Val Asp Ala Thr Gly Gln Leu Asp Pro Glu
            20                  25                  30

Ala Ile Glu Arg Thr Arg Val Ala Leu Glu Asn Tyr Val Glu Leu Met
        35                  40                  45

Glu Thr His Gly Val Glu Ala Val Arg Met Val Ala Thr Ser Ala Thr
    50                  55                  60

Arg Asp Ala Ser Asn Arg Asp Glu Phe Phe Ser Met Thr Arg Gln Leu
65                  70                  75                  80

Leu Ser Lys Ile Arg Pro Gly Tyr Gln Ala Glu Val Ile Ser Gly Glu
                85                  90                  95

Glu Glu Ala Leu Leu Ser Phe Arg Gly Ala Ile Val Asp Leu Pro Glu
            100                 105                 110

Asp Gln Gly Pro Phe Cys Val Ile Asp Leu Gly Gly Gly Ser Thr Glu
        115                 120                 125

Phe Ile Val Gly Thr Tyr Asp Gly Glu Ile Leu Gly Ser His Ser Thr
    130                 135                 140

Gln Met Gly Cys Val Arg Leu Thr Glu Arg Ile Met Arg Ser Asp Pro
145                 150                 155                 160

Pro Asp

<210> 337
<211> 534
<212> DNA
<213> Corynebacterium glutamicum

<220>
<221> CDS
<222> (101)..(511)
<223> RXN02972

<400> 337
acctacgacg gtgaaatcct aggctcccac tcaacccaaa tgggatgcgt gcgcctgacc 60

gaacgaatca tgcgcagcga cccacccgac tgaaaccgaa gtg gaa atc gcc cgc 115
Val Glu Ile Ala Arg
1               5

gac tac gtt gca gaa cgc atc cag gaa gta aaa gcc atc gtc cca att 163
Asp Tyr Val Ala Glu Arg Ile Gln Glu Val Lys Ala Ile Val Pro Ile
                10                  15                  20

tca aag gca aaa acc ttt gtg gga tgc gca ggc acc ttc acc aca atc     211
Ser Lys Ala Lys Thr Phe Val Gly Cys Ala Gly Thr Phe Thr Thr Ile
            25                  30                  35

tcc gcc tgg gtg caa ggc cta gaa agc tac gac cgc gac gcg atc cac     259
Ser Ala Trp Val Gln Gly Leu Glu Ser Tyr Asp Arg Asp Ala Ile His
        40                  45                  50

ctc tct gca ctc aac ttc gat gca ctg cga gtt gtc acc gat gag atc     307
Leu Ser Ala Leu Asn Phe Asp Ala Leu Arg Val Val Thr Asp Glu Ile
    55                  60                  65

att tca gaa tca tca tca cag cgc gcc agc aac cca gtt gtt gat cca     355
Ile Ser Glu Ser Ser Ser Gln Arg Ala Ser Asn Pro Val Val Asp Pro
70                  75                  80                  85

ggt cgc gcc gac gtc atc ggt ggc gga tcc gtt gtt gtc caa gca gcg     403
Gly Arg Ala Asp Val Ile Gly Gly Gly Ser Val Val Val Gln Ala Ala
                90                  95                  100

atc gac tta gcc tcc aaa gaa gcc ggt gta gac tac atc att att tcc     451
Ile Asp Leu Ala Ser Lys Glu Ala Gly Val Asp Tyr Ile Ile Ile Ser
            105                 110                 115

gaa aaa gac atc ctc gac ggc ctc atc ctt ggc ctg gta gaa gcc gac     499
Glu Lys Asp Ile Leu Asp Gly Leu Ile Leu Gly Leu Val Glu Ala Asp
        120                 125                 130

tct ttg aag aaa taggacccta gttttaaacc act                           534
Ser Leu Lys Lys
    135


<210> 338
<211> 137
<212> PRT
<213> Corynebacterium glutamicum

<400> 338
Val Glu Ile Ala Arg Asp Tyr Val Ala Glu Arg Ile Gln Glu Val Lys
1               5                   10                  15

Ala Ile Val Pro Ile Ser Lys Ala Lys Thr Phe Val Gly Cys Ala Gly
            20                  25                  30

Thr Phe Thr Thr Ile Ser Ala Trp Val Gln Gly Leu Glu Ser Tyr Asp
        35                  40                  45

Arg Asp Ala Ile His Leu Ser Ala Leu Asn Phe Asp Ala Leu Arg Val
    50                  55                  60

Val Thr Asp Glu Ile Ile Ser Glu Ser Ser Ser Gln Arg Ala Ser Asn
65                  70                  75                  80

Pro Val Val Asp Pro Gly Arg Ala Asp Val Ile Gly Gly Gly Ser Val
                85                  90                  95

Val Val Gln Ala Ala Ile Asp Leu Ala Ser Lys Glu Ala Gly Val Asp
            100                 105                 110

Tyr Ile Ile Ile Ser Glu Lys Asp Ile Leu Asp Gly Leu Ile Leu Gly
        115                 120                 125

Leu Val Glu Ala Asp Ser Leu Lys Lys
    130                 135


<210> 339
<211> 1497
<212> DNA
<213> Corynebacterium glutamicum

<220>
<221> CDS
<222> (101)..(1474)
<223> RXN00663

<400> 339
ctgaacgatt ggtgaccggc tcatgaaaac ttgacgagtc cccggtattc gccagcggtg 60

actactaccg tgggcgacaa gcccacttag aggaggactt gtg aca acc acc tat 115
Val Thr Thr Thr Tyr
1               5

cca gat ttc ctt gga aat tct tcg ctc caa aca gat acg gag cac tgg 163
Pro Asp Phe Leu Gly Asn Ser Ser Leu Gln Thr Asp Thr Glu His Trp
                10                  15                  20

gaa atg gaa gga ggt gcg cag gaa gtc tct gtt act tat gtt ttg gac 211
Glu Met Glu Gly Gly Ala Gln Glu Val Ser Val Thr Tyr Val Leu Asp
            25                  30                  35

acg tca gtg ttg ctg tct gat ccg ttg tcg ttg aca cgg ttc gcg gag 259
Thr Ser Val Leu Leu Ser Asp Pro Leu Ser Leu Thr Arg Phe Ala Glu
        40                  45                  50

cac gat gta gtt ctg cca att gtt gta att acg gaa tta gaa gcc aag 307
His Asp Val Val Leu Pro Ile Val Val Ile Thr Glu Leu Glu Ala Lys
    55                  60                  65

cgt cat cac ccg gac ctt ggc ttt ttt gct cgc caa gcg ctt cgg atg 355
Arg His His Pro Asp Leu Gly Phe Phe Ala Arg Gln Ala Leu Arg Met
70                  75                  80                  85

ctg gat gag ctg cgt gag atc cat ggg gat ttg tcc aag cca ctg cca 403
Leu Asp Glu Leu Arg Glu Ile His Gly Asp Leu Ser Lys Pro Leu Pro
                90                  95                  100

att ggc gat gaa ggc gga cac atc cat gtt gag ctg aat cac caa aac 451
Ile Gly Asp Glu Gly Gly His Ile His Val Glu Leu Asn His Gln Asn
            105                 110                 115

acg ggg tcc ttg ccc gtg gga ttc cgc ctt ggt gac aat gac acc cgc 499
Thr Gly Ser Leu Pro Val Gly Phe Arg Leu Gly Asp Asn Asp Thr Arg
        120                 125                 130

atc ctt gca gtg gcc aag aat ctg cag gaa gag ggc cac aat gtg gtt 547
Ile Leu Ala Val Ala Lys Asn Leu Gln Glu Glu Gly His Asn Val Val
    135                 140                 145

ctg gtg tcg aag gac ctg ccg atg cgg att aag gcg tcg gca agc gga 595
Leu Val Ser Lys Asp Leu Pro Met Arg Ile Lys Ala Ser Ala Ser Gly
150                 155                 160                 165

atc gcc gca cag gaa tac cgc gct gcc ctg gcg cgc gac cgt ggt tac 643
Ile Ala Ala Gln Glu Tyr Arg Ala Ala Leu Ala Arg Asp Arg Gly Tyr
                170                 175                 180

acc ggc atg acc cac gcc aat atc acc gat gac cag ctc agc gag ctc 691
Thr Gly Met Thr His Ala Asn Ile Thr Asp Asp Gln Leu Ser Glu Leu
            185                 190                 195

tac gac acc ggc gag gtg cgc att gag gag ctc gaa aag ctg ccc gtc 739
Tyr Asp Thr Gly Glu Val Arg Ile Glu Glu Leu Glu Lys Leu Pro Val
        200                 205                 210

aac cac ggc ttc acc ctc aaa tcc aac agc ggt tcg gcg ctt ggt cgt 787
Asn His Gly Phe Thr Leu Lys Ser Asn Ser Gly Ser Ala Leu Gly Arg
    215                 220                 225

atg aat tcc gac aag atc atc gag ctt gtc ccc ggc gac cag cag gta 835
Met Asn Ser Asp Lys Ile Ile Glu Leu Val Pro Gly Asp Gln Gln Val
230                 235                 240                 245

ttc ggt atc agc ggg cgt agc gct gag cag cgg gtt gcc att gat ttg 883
Phe Gly Ile Ser Gly Arg Ser Ala Glu Gln Arg Val Ala Ile Asp Leu
                250                 255                 260

ctt aac gac gac gcc gtc ggc atc gta tcc atc ggc ggc ccc gcg ggt 931
Leu Asn Asp Asp Ala Val Gly Ile Val Ser Ile Gly Gly Pro Ala Gly
            265                 270                 275

aca ggt aaa agc gca ctc gca ctg tgt gcc ggc ctg gaa gct gtg atg 979
Thr Gly Lys Ser Ala Leu Ala Leu Cys Ala Gly Leu Glu Ala Val Met

280                 285                 290

gag cgt cgc att cag cgc aag att atc gtg ttc cgc cca ctc ttt gcc      1027
Glu Arg Arg Ile Gln Arg Lys Ile Ile Val Phe Arg Pro Leu Phe Ala
    295                 300                 305

gtt ggc gga cag gaa ctt ggc tac ctg cct ggc gac caa gaa gaa aaa      1075
Val Gly Gly Gln Glu Leu Gly Tyr Leu Pro Gly Asp Gln Glu Glu Lys
310                 315                 320                 325

atg ggg cct tgg gcg caa gcg gtt ttt gac acc cta agc tcc atg gtc      1123
Met Gly Pro Trp Ala Gln Ala Val Phe Asp Thr Leu Ser Ser Met Val
                330                 335                 340

agc caa aac atc atc gat gaa gcc ctc tcc cgc ggc ctc atc gaa gtt      1171
Ser Gln Asn Ile Ile Asp Glu Ala Leu Ser Arg Gly Leu Ile Glu Val
            345                 350                 355

ctc cca ctt act cac atc cgc gga cgc tca ctc cac gat gct ttc gtc      1219
Leu Pro Leu Thr His Ile Arg Gly Arg Ser Leu His Asp Ala Phe Val
        360                 365                 370

atc gtc gac gag gcc caa tcc cta gaa cgc aac gtg ttg ctc acc atg      1267
Ile Val Asp Glu Ala Gln Ser Leu Glu Arg Asn Val Leu Leu Thr Met
    375                 380                 385

ctg tct cgc atc ggc cag aat tcc cga gta gtt ctc acc cat gac gta      1315
Leu Ser Arg Ile Gly Gln Asn Ser Arg Val Val Leu Thr His Asp Val
390                 395                 400                 405

gcg cag cgc gac aac ctg cgc gtt ggt cgc tac gac ggc atc gtc tct      1363
Ala Gln Arg Asp Asn Leu Arg Val Gly Arg Tyr Asp Gly Ile Val Ser
                410                 415                 420

gtg gtg gaa gca ctc aag gat cac gaa ctg ttt ggc cac atc acg ttg      1411
Val Val Glu Ala Leu Lys Asp His Glu Leu Phe Gly His Ile Thr Leu
            425                 430                 435

cag cgt tcc gaa cgc tcc cga atc gct gag ttg gtc acc caa gtt ttg      1459
Gln Arg Ser Glu Arg Ser Arg Ile Ala Glu Leu Val Thr Gln Val Leu
        440                 445                 450

gat gcg ccg tct ctg tagtcgcgca gtctgtggcg att                        1497
Asp Ala Pro Ser Leu
    455


<210> 340
<211> 458
<212> PRT
<213> Corynebacterium glutamicum

<400> 340
Val Thr Thr Thr Tyr Pro Asp Phe Leu Gly Asn Ser Ser Leu Gln Thr
1               5                   10                  15

Asp Thr Glu His Trp Glu Met Glu Gly Gly Ala Gln Glu Val Ser Val
            20                  25                  30

Thr Tyr Val Leu Asp Thr Ser Val Leu Leu Ser Asp Pro Leu Ser Leu
        35                  40                  45

Thr Arg Phe Ala Glu His Asp Val Val Leu Pro Ile Val Val Ile Thr
    50                  55                  60

Glu Leu Glu Ala Lys Arg His His Pro Asp Leu Gly Phe Phe Ala Arg
65                  70                  75                  80

Gln Ala Leu Arg Met Leu Asp Glu Leu Arg Glu Ile His Gly Asp Leu
                85                  90                  95

Ser Lys Pro Leu Pro Ile Gly Asp Glu Gly Gly His Ile His Val Glu
            100                 105                 110

Leu Asn His Gln Asn Thr Gly Ser Leu Pro Val Gly Phe Arg Leu Gly
        115                 120                 125

Asp Asn Asp Thr Arg Ile Leu Ala Val Ala Lys Asn Leu Gln Glu Glu
    130                 135                 140

Gly His Asn Val Val Leu Val Ser Lys Asp Leu Pro Met Arg Ile Lys
145                 150                 155                 160

Ala Ser Ala Ser Gly Ile Ala Ala Gln Glu Tyr Arg Ala Ala Leu Ala
                165                 170                 175

Arg Asp Arg Gly Tyr Thr Gly Met Thr His Ala Asn Ile Thr Asp Asp
            180                 185                 190

Gln Leu Ser Glu Leu Tyr Asp Thr Gly Glu Val Arg Ile Glu Glu Leu
        195                 200                 205

Glu Lys Leu Pro Val Asn His Gly Phe Thr Leu Lys Ser Asn Ser Gly
    210                 215                 220

Ser Ala Leu Gly Arg Met Asn Ser Asp Lys Ile Ile Glu Leu Val Pro
225                 230                 235                 240

Gly Asp Gln Gln Val Phe Gly Ile Ser Gly Arg Ser Ala Glu Gln Arg
                245                 250                 255

Val Ala Ile Asp Leu Leu Asn Asp Asp Ala Val Gly Ile Val Ser Ile
            260                 265                 270

Gly Gly Pro Ala Gly Thr Gly Lys Ser Ala Leu Ala Leu Cys Ala Gly
        275                 280                 285

Leu Glu Ala Val Met Glu Arg Arg Ile Gln Arg Lys Ile Ile Val Phe
    290                 295                 300

Arg Pro Leu Phe Ala Val Gly Gly Gln Glu Leu Gly Tyr Leu Pro Gly
305                 310                 315                 320

Asp Gln Glu Glu Lys Met Gly Pro Trp Ala Gln Ala Val Phe Asp Thr
                325                 330                 335

Leu Ser Ser Met Val Ser Gln Asn Ile Ile Asp Glu Ala Leu Ser Arg
            340                 345                 350

Gly Leu Ile Glu Val Leu Pro Leu Thr His Ile Arg Gly Arg Ser Leu
        355                 360                 365

His Asp Ala Phe Val Ile Val Asp Glu Ala Gln Ser Leu Glu Arg Asn
    370                 375                 380

Val Leu Leu Thr Met Leu Ser Arg Ile Gly Gln Asn Ser Arg Val Val
385                 390                 395                 400

Leu Thr His Asp Val Ala Gln Arg Asp Asn Leu Arg Val Gly Arg Tyr
                405                 410                 415

Asp Gly Ile Val Ser Val Val Glu Ala Leu Lys Asp His Glu Leu Phe
            420                 425                 430

Gly His Ile Thr Leu Gln Arg Ser Glu Arg Ser Arg Ile Ala Glu Leu
        435                 440                 445

Val Thr Gln Val Leu Asp Ala Pro Ser Leu
    450                 455


<210> 341
<211> 1248
<212> DNA
<213> Corynebacterium glutamicum

<220>
<221> CDS
<222> (101)..(1225)
<223> RXN00778

<400> 341
aggtcttagg tttttaagtc gtgagcaatc cggagggaaa ctagcccgcc tacaggatct 60

gctcagacga tgtcttcact taaaccggaa aggcttcccc gtg aac ctc act ctt 115
Val Asn Leu Thr Leu
1               5

aag cgc tcc atc gcc ctt gtg ggc gca gtt act gca ggc tcc ttc gct 163
Lys Arg Ser Ile Ala Leu Val Gly Ala Val Thr Ala Gly Ser Phe Ala
                10                  15                  20

ctt gta gct tgc tcc gac tcc aat gag tct gat tcc acc tcc tca tct 211
Leu Val Ala Cys Ser Asp Ser Asn Glu Ser Asp Ser Thr Ser Ser Ser
            25                  30                  35

gca gct tcc acc ggt tct tcc gat gct gca tcc att gag ggc ctt tcc 259
Ala Ala Ser Thr Gly Ser Ser Asp Ala Ala Ser Ile Glu Gly Leu Ser
        40                  45                  50

ggt gtt acc ggt cag ctc gtt gct gaa ggt gca tct tcc cag cag tcc 307
Gly Val Thr Gly Gln Leu Val Ala Glu Gly Ala Ser Ser Gln Gln Ser
    55                  60                  65

gca atg gac tac ttt ggt atc cgt tac tcc gag gct gtc agc ggt gca 355
Ala Met Asp Tyr Phe Gly Ile Arg Tyr Ser Glu Ala Val Ser Gly Ala
70                  75                  80                  85

tct ctg gct tac acc cct tca ggt tcc ggt tcc ggc cgc acc aac ttc 403
Ser Leu Ala Tyr Thr Pro Ser Gly Ser Gly Ser Gly Arg Thr Asn Phe
                90                  95                  100

gct gca ggc cag gtt gct ttc ggt ggc tcc gac tcc gca atg aag gac 451
Ala Ala Gly Gln Val Ala Phe Gly Gly Ser Asp Ser Ala Met Lys Asp
            105                 110                 115

gac cag gct gca gaa gca gaa gca cgt tgc aac ggc aac gaa gca tgg 499
Asp Gln Ala Ala Glu Ala Glu Ala Arg Cys Asn Gly Asn Glu Ala Trp
        120                 125                 130

cac ctg cca ttc gtt atc ggc cca gtt gca gtt gct tac aac ctg cct 547
His Leu Pro Phe Val Ile Gly Pro Val Ala Val Ala Tyr Asn Leu Pro
    135                 140                 145

ggc gtt gac acc ctg aac ctg gac acc aac atc atc gct cag atc ttc 595
Gly Val Asp Thr Leu Asn Leu Asp Thr Asn Ile Ile Ala Gln Ile Phe
150                 155                 160                 165

aag ggc gag atc acc aag tgg aac gac gaa gca atc gct tcc cag aac 643
Lys Gly Glu Ile Thr Lys Trp Asn Asp Glu Ala Ile Ala Ser Gln Asn
                170                 175                 180

gag ggc acc gac ctc cca gac cag gac atc tcc gtt ctg tac cgt tcc 691
Glu Gly Thr Asp Leu Pro Asp Gln Asp Ile Ser Val Leu Tyr Arg Ser
            185                 190                 195

gaa gag tcc ggt acc tcc gac aac ttc cag aag ttc ctc gga gct tcc 739
Glu Glu Ser Gly Thr Ser Asp Asn Phe Gln Lys Phe Leu Gly Ala Ser
        200                 205                 210

acc gac atc tgg gag acc gaa ggc cag cag ttc cca acc gag gtt ggc 787
Thr Asp Ile Trp Glu Thr Glu Gly Gln Gln Phe Pro Thr Glu Val Gly
    215                 220                 225

tcc ggt gcg cag ggc tcc aac ggt gta gct tct gag gct tcc aac atc 835
Ser Gly Ala Gln Gly Ser Asn Gly Val Ala Ser Glu Ala Ser Asn Ile
230                 235                 240                 245

gag ggt gca atc acc tac gtt gaa gct ggt ttc gct aac cag tcc ggc 883
Glu Gly Ala Ile Thr Tyr Val Glu Ala Gly Phe Ala Asn Gln Ser Gly
                250                 255                 260

ctg ggc gtt gca aac atc gac ttc ggt tcc ggc cca gtt gaa ctc aac 931
Leu Gly Val Ala Asn Ile Asp Phe Gly Ser Gly Pro Val Glu Leu Asn
            265                 270                 275

gct gag tcc gtt ggc gtt gca ctt ggt gca ctc gac ttc ctg act gag 979
Ala Glu Ser Val Gly Val Ala Leu Gly Ala Leu Asp Phe Leu Thr Glu
        280                 285                 290

ggc cac aac atg gtt gtt gac acc gac gct atg ttc gca atg aac gaa 1027

Gly His Asn Met Val Val Asp Thr Asp Ala Met Phe Ala Met Asn Glu
    295                 300                 305

gcc ggt gct tac cca ctg atc ctc acc acc tac gaa atc gtc tgc tcc     1075
Ala Gly Ala Tyr Pro Leu Ile Leu Thr Thr Tyr Glu Ile Val Cys Ser
310                 315                 320                 325

gca ggc tac gac gag acc acc cgc gac cag gtc aag gac ttc ctg acc     1123
Ala Gly Tyr Asp Glu Thr Thr Arg Asp Gln Val Lys Asp Phe Leu Thr
                330                 335                 340

gtt gca ctg gac tcc cag gat gac cag ctc gag gct ctc ggc tac atc     1171
Val Ala Leu Asp Ser Gln Asp Asp Gln Leu Glu Ala Leu Gly Tyr Ile
            345                 350                 355

cca gtt acc ggc gag cac tac gat cgc ctc gtt gca gca gtt gaa gca     1219
Pro Val Thr Gly Glu His Tyr Asp Arg Leu Val Ala Ala Val Glu Ala
        360                 365                 370

att cag taataaaccg ctgccgtagc ttc                                   1248
Ile Gln
    375


<210> 342
<211> 375
<212> PRT
<213> Corynebacterium glutamicum

<400> 342
Val Asn Leu Thr Leu Lys Arg Ser Ile Ala Leu Val Gly Ala Val Thr
  1               5                  10                  15

Ala Gly Ser Phe Ala Leu Val Ala Cys Ser Asp Ser Asn Glu Ser Asp
             20                  25                  30

Ser Thr Ser Ser Ser Ala Ala Ser Thr Gly Ser Ser Asp Ala Ala Ser
         35                  40                  45

Ile Glu Gly Leu Ser Gly Val Thr Gly Gln Leu Val Ala Glu Gly Ala
     50                  55                  60

Ser Ser Gln Gln Ser Ala Met Asp Tyr Phe Gly Ile Arg Tyr Ser Glu
 65                  70                  75                  80

Ala Val Ser Gly Ala Ser Leu Ala Tyr Thr Pro Ser Gly Ser Gly Ser
                 85                  90                  95

Gly Arg Thr Asn Phe Ala Ala Gly Gln Val Ala Phe Gly Gly Ser Asp
            100                 105                 110

Ser Ala Met Lys Asp Asp Gln Ala Ala Glu Ala Glu Ala Arg Cys Asn
        115                 120                 125

Gly Asn Glu Ala Trp His Leu Pro Phe Val Ile Gly Pro Val Ala Val
    130                 135                 140

Ala Tyr Asn Leu Pro Gly Val Asp Thr Leu Asn Leu Asp Thr Asn Ile
145                 150                 155                 160

Ile Ala Gln Ile Phe Lys Gly Glu Ile Thr Lys Trp Asn Asp Glu Ala
                165                 170                 175

Ile Ala Ser Gln Asn Glu Gly Thr Asp Leu Pro Asp Gln Asp Ile Ser
            180                 185                 190

Val Leu Tyr Arg Ser Glu Glu Ser Gly Thr Ser Asp Asn Phe Gln Lys
        195                 200                 205

Phe Leu Gly Ala Ser Thr Asp Ile Trp Glu Thr Glu Gly Gln Gln Phe
    210                 215                 220

Pro Thr Glu Val Gly Ser Gly Ala Gln Gly Ser Asn Gly Val Ala Ser
225                 230                 235                 240

Glu Ala Ser Asn Ile Glu Gly Ala Ile Thr Tyr Val Glu Ala Gly Phe
                245                 250                 255

Ala Asn Gln Ser Gly Leu Gly Val Ala Asn Ile Asp Phe Gly Ser Gly
            260                 265                 270

Pro Val Glu Leu Asn Ala Glu Ser Val Gly Val Ala Leu Gly Ala Leu
        275                 280                 285

Asp Phe Leu Thr Glu Gly His Asn Met Val Val Asp Thr Asp Ala Met
    290                 295                 300

Phe Ala Met Asn Glu Ala Gly Ala Tyr Pro Leu Ile Leu Thr Thr Tyr
305                 310                 315                 320

Glu Ile Val Cys Ser Ala Gly Tyr Asp Glu Thr Thr Arg Asp Gln Val
                325                 330                 335

Lys Asp Phe Leu Thr Val Ala Leu Asp Ser Gln Asp Asp Gln Leu Glu
            340                 345                 350

Ala Leu Gly Tyr Ile Pro Val Thr Gly Glu His Tyr Asp Arg Leu Val
        355                 360                 365

Ala Ala Val Glu Ala Ile Gln
    370                 375


<210> 343
<211> 870
<212> DNA
<213> Corynebacterium glutamicum

<220>
<221> CDS
<222> (101)..(847)
<223> RXN00250

<400> 343
acaacaccag accaccccaa ccctgaataa acccctattt ttctaaaaag tcacactttg 60

ccgtatagaa attcagtcaa ccaagagtac tctgtccacc atg gtt ttt act ctt    115
                                            Met Val Phe Thr Leu
                                            1               5

gcg gac tcc gtc tcc cag gtt gcg cta ggt ccg tcc tgg ctg gac cct    163
Ala Asp Ser Val Ser Gln Val Ala Leu Gly Pro Ser Trp Leu Asp Pro
                10                  15                  20

atg gaa ctt ctt tcc ggc tcc ggc ccg ttc ggt agc ttc att ctt ccg    211
Met Glu Leu Leu Ser Gly Ser Gly Pro Phe Gly Ser Phe Ile Leu Pro
            25                  30                  35

gcg atg ctt gcc att gtc ttt atc gaa tca ggc cta ctt ttc cca ctt    259
Ala Met Leu Ala Ile Val Phe Ile Glu Ser Gly Leu Leu Phe Pro Leu
        40                  45                  50

cta cca ggt gat tct ctc ctt ttc acc ggt ggt ctc cta gct aac cag    307
Leu Pro Gly Asp Ser Leu Leu Phe Thr Gly Gly Leu Leu Ala Asn Gln
    55                  60                  65

gct gac cct ttt gca ccg ctg tgg ctg gtg ctg atc ctc tgc cct atc    355
Ala Asp Pro Phe Ala Pro Leu Trp Leu Val Leu Ile Leu Cys Pro Ile
70                  75                  80                  85

gcc gca att ctt ggc gat cag gtg ggt tac tgg att ggc cac aag ttc    403
Ala Ala Ile Leu Gly Asp Gln Val Gly Tyr Trp Ile Gly His Lys Phe
                90                  95                  100

cac cct cgc ctg gtc aat cgt ccg gat ggc agg att ttc aag cag gaa    451
His Pro Arg Leu Val Asn Arg Pro Asp Gly Arg Ile Phe Lys Gln Glu
            105                 110                 115

tac ctc aag cag act gag gat ttc ttt gag aag cat ggc ccc gtg acg    499
Tyr Leu Lys Gln Thr Glu Asp Phe Phe Glu Lys His Gly Pro Val Thr
        120                 125                 130

atc att ttg tgc cgt ttc gtg ccc atc gtc cgt act tac gca cct ctg    547
Ile Ile Leu Cys Arg Phe Val Pro Ile Val Arg Thr Tyr Ala Pro Leu

135                 140                 145

gtc gca ggt atg gct ggc atg cgt tac cgc acg ttc att att tac aac     595
Val Ala Gly Met Ala Gly Met Arg Tyr Arg Thr Phe Ile Ile Tyr Asn
150                 155                 160                 165

atg atc ggt ggc att ttg tgg ggt tcc ggc gtg gtg gct ttg ggt gct     643
Met Ile Gly Gly Ile Leu Trp Gly Ser Gly Val Val Ala Leu Gly Ala
                170                 175                 180

gcg ttg ggt cag ttc gat ttc gtc cgc aac aat att gat ctg att ttc     691
Ala Leu Gly Gln Phe Asp Phe Val Arg Asn Asn Ile Asp Leu Ile Phe
            185                 190                 195

ttg ctg atc gtg ttc att tcg gtg gtt cct ggt ttg gtc ggc atg gcc     739
Leu Leu Ile Val Phe Ile Ser Val Val Pro Gly Leu Val Gly Met Ala
        200                 205                 210

cgc aag ctg gct gac ggc cac aag caa gcc aac acc gag cca caa gaa     787
Arg Lys Leu Ala Asp Gly His Lys Gln Ala Asn Thr Glu Pro Gln Glu
    215                 220                 225

aac ccc gca gtc cag aca gcc cca gta aaa acc cag gaa gcc cag gaa     835
Asn Pro Ala Val Gln Thr Ala Pro Val Lys Thr Gln Glu Ala Gln Glu
230                 235                 240                 245

gcc ccc cag aac taatctttcc ggtccgccag ttc                           870
Ala Pro Gln Asn

<210> 344
<211> 249
<212> PRT
<213> Corynebacterium glutamicum

<400> 344
Met Val Phe Thr Leu Ala Asp Ser Val Ser Gln Val Ala Leu Gly Pro
1               5                   10                  15

Ser Trp Leu Asp Pro Met Glu Leu Leu Ser Gly Ser Gly Pro Phe Gly
            20                  25                  30

Ser Phe Ile Leu Pro Ala Met Leu Ala Ile Val Phe Ile Glu Ser Gly
        35                  40                  45

Leu Leu Phe Pro Leu Leu Pro Gly Asp Ser Leu Leu Phe Thr Gly Gly
    50                  55                  60

Leu Leu Ala Asn Gln Ala Asp Pro Phe Ala Pro Leu Trp Leu Val Leu
65                  70                  75                  80

Ile Leu Cys Pro Ile Ala Ala Ile Leu Gly Asp Gln Val Gly Tyr Trp
                85                  90                  95

Ile Gly His Lys Phe His Pro Arg Leu Val Asn Arg Pro Asp Gly Arg
            100                 105                 110

Ile Phe Lys Gln Glu Tyr Leu Lys Gln Thr Glu Asp Phe Phe Glu Lys
        115                 120                 125

His Gly Pro Val Thr Ile Ile Leu Cys Arg Phe Val Pro Ile Val Arg
    130                 135                 140

Thr Tyr Ala Pro Leu Val Ala Gly Met Ala Gly Met Arg Tyr Arg Thr
145                 150                 155                 160

Phe Ile Ile Tyr Asn Met Ile Gly Gly Ile Leu Trp Gly Ser Gly Val
                165                 170                 175

Val Ala Leu Gly Ala Ala Leu Gly Gln Phe Asp Phe Val Arg Asn Asn
            180                 185                 190

Ile Asp Leu Ile Phe Leu Leu Ile Val Phe Ile Ser Val Val Pro Gly
        195                 200                 205

Leu Val Gly Met Ala Arg Lys Leu Ala Asp Gly His Lys Gln Ala Asn

210                 215                 220

Thr Glu Pro Gln Glu Asn Pro Ala Val Gln Thr Ala Pro Val Lys Thr
225                 230                 235                 240

Gln Glu Ala Gln Glu Ala Pro Gln Asn
                245


<210> 345
<211> 541
<212> DNA
<213> Corynebacterium glutamicum

<220>
<221> CDS
<222> (101)..(541)
<223> RXA00072

<400> 345
acggccagga cgatccagtg cacaggccag caccagcaaa gtccacatcg caagcattaa 60

aagaatctct cgaaagacac aaaagaggtg agtcgcaaca atg agc ttt caa cta   115
                                           Met Ser Phe Gln Leu
                                           1               5

gtt aac gcc ctg aaa aat act ggt tcg gta aaa gat ccc gag atc tca   163
Val Asn Ala Leu Lys Asn Thr Gly Ser Val Lys Asp Pro Glu Ile Ser
                10                  15                  20

ccc gaa gga cct cgc acg acc aca ccg ttg tca cca gag gta gca aaa   211
Pro Glu Gly Pro Arg Thr Thr Thr Pro Leu Ser Pro Glu Val Ala Lys
            25                  30                  35

cat aac gag gaa ctc gtc gaa aag cat gct gct gcg ttg tat gac gcc   259
His Asn Glu Glu Leu Val Glu Lys His Ala Ala Ala Leu Tyr Asp Ala
        40                  45                  50

agc gcg caa gag atc ctg gaa tgg aca gcc gag cac gcg ccg ggc gct   307
Ser Ala Gln Glu Ile Leu Glu Trp Thr Ala Glu His Ala Pro Gly Ala
    55                  60                  65

att gca gtg acc ttg agc atg gaa aac acc gtg ctg gcg gag ctg gct   355
Ile Ala Val Thr Leu Ser Met Glu Asn Thr Val Leu Ala Glu Leu Ala
70                  75                  80                  85

gcg cgg cac ctg ccg gaa gct gat ttc ctc ttt ttg gac acc ggt tac   403
Ala Arg His Leu Pro Glu Ala Asp Phe Leu Phe Leu Asp Thr Gly Tyr
                90                  95                  100

cac ttc aag gag acc ctt gaa gtt gcc cgt cag gta gat gag cgc tat   451
His Phe Lys Glu Thr Leu Glu Val Ala Arg Gln Val Asp Glu Arg Tyr
            105                 110                 115

tcc cag aag ctt gtc acc gcg ctg ccg atc ctc aag cgc acg gag cag   499
Ser Gln Lys Leu Val Thr Ala Leu Pro Ile Leu Lys Arg Thr Glu Gln
        120                 125                 130

gat tcc att tat ggt ctc aac ctg tac cgc agc aac cca gcg           541
Asp Ser Ile Tyr Gly Leu Asn Leu Tyr Arg Ser Asn Pro Ala
    135                 140                 145


<210> 346
<211> 147
<212> PRT
<213> Corynebacterium glutamicum

<400> 346
Met Ser Phe Gln Leu Val Asn Ala Leu Lys Asn Thr Gly Ser Val Lys
1               5                   10                  15

Asp Pro Glu Ile Ser Pro Glu Gly Pro Arg Thr Thr Thr Pro Leu Ser
            20                  25                  30

Pro Glu Val Ala Lys His Asn Glu Glu Leu Val Glu Lys His Ala Ala
        35                  40                  45

Ala Leu Tyr Asp Ala Ser Ala Gln Glu Ile Leu Glu Trp Thr Ala Glu
    50                  55                  60

His Ala Pro Gly Ala Ile Ala Val Thr Leu Ser Met Glu Asn Thr Val
65                  70                  75                  80

Leu Ala Glu Leu Ala Ala Arg His Leu Pro Glu Ala Asp Phe Leu Phe
                85                  90                  95

Leu Asp Thr Gly Tyr His Phe Lys Glu Thr Leu Glu Val Ala Arg Gln
            100                 105                 110

Val Asp Glu Arg Tyr Ser Gln Lys Leu Val Thr Ala Leu Pro Ile Leu
        115                 120                 125

Lys Arg Thr Glu Gln Asp Ser Ile Tyr Gly Leu Asn Leu Tyr Arg Ser
    130                 135                 140

Asn Pro Ala
145


<210> 347
<211> 1299
<212> DNA
<213> Corynebacterium glutamicum

<220>
<221> CDS
<222> (101)..(1276)
<223> RXA00793

<400> 347
tcgctggttt ttagatggtt ttcaagccag cgagaccaca ttagtttcac gctggttgaa 60

acctttgaga tcaatataga ccgtgtggtc tactcgagga atg agt gaa aac aat    115
                                           Met Ser Glu Asn Asn
                                           1               5

ccc act acc ttg cac tgg ttc cta ccc acc tat ggc gat tct cgc gga    163
Pro Thr Thr Leu His Trp Phe Leu Pro Thr Tyr Gly Asp Ser Arg Gly
                10                  15                  20

atc aca gcc ggc ggg cat ggc ttc ggc ttc cac tcc gga agc cgg aca    211
Ile Thr Ala Gly Gly His Gly Phe Gly Phe His Ser Gly Ser Arg Thr
            25                  30                  35

gca gac ctc gat tac ctc tcc caa att gcc ctg gcc gct gaa cga aac    259
Ala Asp Leu Asp Tyr Leu Ser Gln Ile Ala Leu Ala Ala Glu Arg Asn
        40                  45                  50

ggt ttt gaa tcc gtc ctg act ccc act gga ttg tgg tgc gaa gat gcg    307
Gly Phe Glu Ser Val Leu Thr Pro Thr Gly Leu Trp Cys Glu Asp Ala
    55                  60                  65

tgg atc acc acc gca gcg ctg ctg tct agg aca tca aaa ctg aaa ttc    355
Trp Ile Thr Thr Ala Ala Leu Leu Ser Arg Thr Ser Lys Leu Lys Phe
70                  75                  80                  85

ctc gtt gct att cga cca ggc caa gtt agc ccc acc atc atc gcg cag    403
Leu Val Ala Ile Arg Pro Gly Gln Val Ser Pro Thr Ile Ile Ala Gln
                90                  95                  100

cag ggt gct gcc ttc cag aaa ttc tca aat aac cgc ctg ctc atc aac    451
Gln Gly Ala Ala Phe Gln Lys Phe Ser Asn Asn Arg Leu Leu Ile Asn
            105                 110                 115

gtc gtg gtg ggt ggc gaa gac cat gaa cag cgc gct ttc gct gat tat    499
Val Val Val Gly Gly Glu Asp His Glu Gln Arg Ala Phe Ala Asp Tyr
        120                 125                 130

tct tcc aaa gag gag cgc tac cac aag gct gat gaa acc tta gag atc    547
Ser Ser Lys Glu Glu Arg Tyr His Lys Ala Asp Glu Thr Leu Glu Ile
    135                 140                 145

atc gat cac cta tgg aac agc gca gaa cct cta aat ttc cag ggt gaa    595

Ile Asp His Leu Trp Asn Ser Ala Glu Pro Leu Asn Phe Gln Gly Glu
150                 155                 160                 165

ttc ctc agt gtg gaa aac gcg gta ttg aag gaa cag ccc gag gtt tcc     643
Phe Leu Ser Val Glu Asn Ala Val Leu Lys Glu Gln Pro Glu Val Ser
                170                 175                 180

cca ccg att tac ttt ggc gga tcc tca caa ctc ggc atc gaa atc gca     691
Pro Pro Ile Tyr Phe Gly Gly Ser Ser Gln Leu Gly Ile Glu Ile Ala
            185                 190                 195

gcc caa cat tcc gat gtt tat ctc acc tgg ggt gaa cct gcg gaa aag     739
Ala Gln His Ser Asp Val Tyr Leu Thr Trp Gly Glu Pro Ala Glu Lys
        200                 205                 210

gta gag gag aag ctt gcc cgg gtg cgc gcc gaa gca gat aag cga aac     787
Val Glu Glu Lys Leu Ala Arg Val Arg Ala Glu Ala Asp Lys Arg Asn
    215                 220                 225

cgc gaa cta gac tat ggc atc cgc ctg cat gtc att gct cga cca act     835
Arg Glu Leu Asp Tyr Gly Ile Arg Leu His Val Ile Ala Arg Pro Thr
230                 235                 240                 245

gag gat gaa gcc tgg tca gtg gct caa aat ctt ctt gac caa ctt gat     883
Glu Asp Glu Ala Trp Ser Val Ala Gln Asn Leu Leu Asp Gln Leu Asp
                250                 255                 260

cag gaa gag gtt gcc cgc att cag gaa ggg ctt gcg cgt tct caa tcg     931
Gln Glu Glu Val Ala Arg Ile Gln Glu Gly Leu Ala Arg Ser Gln Ser
            265                 270                 275

gaa ggt cag cgt cgc atg acg gaa ctt cat gga caa ggg gca gca ttc     979
Glu Gly Gln Arg Arg Met Thr Glu Leu His Gly Gln Gly Ala Ala Phe
        280                 285                 290

aca gca gga gca gat gct cgc tcc ctt gaa att gca ccg aat ctc tgg     1027
Thr Ala Gly Ala Asp Ala Arg Ser Leu Glu Ile Ala Pro Asn Leu Trp
    295                 300                 305

gca ggt gtt ggg cta gtc cgc ggt ggc gcc ggc aca gcg ttg gtg ggt     1075
Ala Gly Val Gly Leu Val Arg Gly Gly Ala Gly Thr Ala Leu Val Gly
310                 315                 320                 325

tcc tat gag caa gtc gcg caa gca att ttg cga tac cgc gat att ggt     1123
Ser Tyr Glu Gln Val Ala Gln Ala Ile Leu Arg Tyr Arg Asp Ile Gly
                330                 335                 340

ctg agc cac ttc att ttc tcc ggc tat cca cat ttg gag gaa acc tat     1171
Leu Ser His Phe Ile Phe Ser Gly Tyr Pro His Leu Glu Glu Thr Tyr
            345                 350                 355

cac gtg ggc gaa gga gtg gta cct gag ctc ctc aaa ttg ggt gtt ccg     1219
His Val Gly Glu Gly Val Val Pro Glu Leu Leu Lys Leu Gly Val Pro
        360                 365                 370

gtg aac aac cat gaa gaa caa cgc aac gac gtg gta gcg act ccg ttt     1267
Val Asn Asn His Glu Glu Gln Arg Asn Asp Val Val Ala Thr Pro Phe
    375                 380                 385

att tcc aga tagatcacgg atcggctgct tta                               1299
Ile Ser Arg
390


<210> 348
<211> 392
<212> PRT
<213> Corynebacterium glutamicum

<400> 348
Met Ser Glu Asn Asn Pro Thr Thr Leu His Trp Phe Leu Pro Thr Tyr
  1               5                  10                  15

Gly Asp Ser Arg Gly Ile Thr Ala Gly Gly His Gly Phe Gly Phe His
             20                  25                  30

Ser Gly Ser Arg Thr Ala Asp Leu Asp Tyr Leu Ser Gln Ile Ala Leu

35                  40                  45

Ala Ala Glu Arg Asn Gly Phe Glu Ser Val Leu Thr Pro Thr Gly Leu
    50                  55                  60

Trp Cys Glu Asp Ala Trp Ile Thr Thr Ala Ala Leu Leu Ser Arg Thr
65                  70                  75                  80

Ser Lys Leu Lys Phe Leu Val Ala Ile Arg Pro Gly Gln Val Ser Pro
                85                  90                  95

Thr Ile Ile Ala Gln Gln Gly Ala Ala Phe Gln Lys Phe Ser Asn Asn
            100                 105                 110

Arg Leu Leu Ile Asn Val Val Val Gly Gly Glu Asp His Glu Gln Arg
        115                 120                 125

Ala Phe Ala Asp Tyr Ser Ser Lys Glu Glu Arg Tyr His Lys Ala Asp
    130                 135                 140

Glu Thr Leu Glu Ile Ile Asp His Leu Trp Asn Ser Ala Glu Pro Leu
145                 150                 155                 160

Asn Phe Gln Gly Glu Phe Leu Ser Val Glu Asn Ala Val Leu Lys Glu
                165                 170                 175

Gln Pro Glu Val Ser Pro Pro Ile Tyr Phe Gly Gly Ser Ser Gln Leu
            180                 185                 190

Gly Ile Glu Ile Ala Ala Gln His Ser Asp Val Tyr Leu Thr Trp Gly
        195                 200                 205

Glu Pro Ala Glu Lys Val Glu Glu Lys Leu Ala Arg Val Arg Ala Glu
    210                 215                 220

Ala Asp Lys Arg Asn Arg Glu Leu Asp Tyr Gly Ile Arg Leu His Val
225                 230                 235                 240

Ile Ala Arg Pro Thr Glu Asp Glu Ala Trp Ser Val Ala Gln Asn Leu
                245                 250                 255

Leu Asp Gln Leu Asp Gln Glu Glu Val Ala Arg Ile Gln Glu Gly Leu
            260                 265                 270

Ala Arg Ser Gln Ser Glu Gly Gln Arg Arg Met Thr Glu Leu His Gly
        275                 280                 285

Gln Gly Ala Ala Phe Thr Ala Gly Ala Asp Ala Arg Ser Leu Glu Ile
    290                 295                 300

Ala Pro Asn Leu Trp Ala Gly Val Gly Leu Val Arg Gly Gly Ala Gly
305                 310                 315                 320

Thr Ala Leu Val Gly Ser Tyr Glu Gln Val Ala Gln Ala Ile Leu Arg
                325                 330                 335

Tyr Arg Asp Ile Gly Leu Ser His Phe Ile Phe Ser Gly Tyr Pro His
            340                 345                 350

Leu Glu Glu Thr Tyr His Val Gly Glu Gly Val Val Pro Glu Leu Leu
        355                 360                 365

Lys Leu Gly Val Pro Val Asn Asn His Glu Glu Gln Arg Asn Asp Val
    370                 375                 380

Val Ala Thr Pro Phe Ile Ser Arg
385                 390

<210> 349
<211> 681
<212> DNA
<213> Corynebacterium glutamicum

<220>
<221> CDS
<222> (101)..(673)

<223> RXA01192

<400> 349
ccacggtgaa cacctgcagg tggagcaagc tagccttgcg catccgccag agattattcc 60

ggagattctt tttggtggat cgtcgccagc tgcaggtgag gtg gct gca cgt tat 115
Val Ala Ala Arg Tyr
1               5

gcg gac acc tat ctc acg tgg ggt gaa act ccc gat cag gtg gcg cag 163
Ala Asp Thr Tyr Leu Thr Trp Gly Glu Thr Pro Asp Gln Val Ala Gln
                10                  15                  20

aaa atc aac tgg atc aac gag cta gca gca cag cgc ggc cgg gaa ctg 211
Lys Ile Asn Trp Ile Asn Glu Leu Ala Ala Gln Arg Gly Arg Glu Leu
            25                  30                  35

cgc cat gga atc cgc ttc cat gtg atc acc cgc gat acg tct gaa gaa 259
Arg His Gly Ile Arg Phe His Val Ile Thr Arg Asp Thr Ser Glu Glu
        40                  45                  50

gca tgg gtg gtg gca gag aag ttg att agc ggg gtc act cca gaa cag 307
Ala Trp Val Val Ala Glu Lys Leu Ile Ser Gly Val Thr Pro Glu Gln
    55                  60                  65

gtc gct aag gct caa gcc ggg ttt gca acg tct aag tcg gag ggg cag 355
Val Ala Lys Ala Gln Ala Gly Phe Ala Thr Ser Lys Ser Glu Gly Gln
70                  75                  80                  85

cgc cgg atg gct gag ctg cac agc aag ggt cgt gcc ttt act agt ggc 403
Arg Arg Met Ala Glu Leu His Ser Lys Gly Arg Ala Phe Thr Ser Gly
                90                  95                  100

tca act gct cgt gat ctg gag gtg tat ccc aat gtg tgg gca ggc gtc 451
Ser Thr Ala Arg Asp Leu Glu Val Tyr Pro Asn Val Trp Ala Gly Val
            105                 110                 115

ggt ttg ctt cgc gga ggt gca gga aca gcc ctt gtg ggc tcg cat gaa 499
Gly Leu Leu Arg Gly Gly Ala Gly Thr Ala Leu Val Gly Ser His Glu
        120                 125                 130

gag gtc gcc gat cgc atc gaa gaa tac gca gca ctc ggc ttg gat cag 547
Glu Val Ala Asp Arg Ile Glu Glu Tyr Ala Ala Leu Gly Leu Asp Gln
    135                 140                 145

ttt gta ctg tcg ggt tat cca aac ttg gag gag gcc ttc cac ttc ggt 595
Phe Val Leu Ser Gly Tyr Pro Asn Leu Glu Glu Ala Phe His Phe Gly
150                 155                 160                 165

gag ggt gtg att ccg aaa ctg ctg cgc cgc ggt gtg gat atc aaa aat 643
Glu Gly Val Ile Pro Lys Leu Leu Arg Arg Gly Val Asp Ile Lys Asn
                170                 175                 180

caa gaa tca cga gtt ttg gaa cct gtt ggg taaacggg 681
Gln Glu Ser Arg Val Leu Glu Pro Val Gly
            185                 190

<210> 350
<211> 191
<212> PRT
<213> Corynebacterium glutamicum

<400> 350
Val Ala Ala Arg Tyr Ala Asp Thr Tyr Leu Thr Trp Gly Glu Thr Pro
1               5                   10                  15

Asp Gln Val Ala Gln Lys Ile Asn Trp Ile Asn Glu Leu Ala Ala Gln
            20                  25                  30

Arg Gly Arg Glu Leu Arg His Gly Ile Arg Phe His Val Ile Thr Arg
        35                  40                  45

Asp Thr Ser Glu Glu Ala Trp Val Val Ala Glu Lys Leu Ile Ser Gly
    50                  55                  60

Val Thr Pro Glu Gln Val Ala Lys Ala Gln Ala Gly Phe Ala Thr Ser

65                  70                  75                  80

Lys Ser Glu Gly Gln Arg Arg Met Ala Glu Leu His Ser Lys Gly Arg
                85                  90                  95

Ala Phe Thr Ser Gly Ser Thr Ala Arg Asp Leu Glu Val Tyr Pro Asn
            100                 105                 110

Val Trp Ala Gly Val Gly Leu Leu Arg Gly Gly Ala Gly Thr Ala Leu
        115                 120                 125

Val Gly Ser His Glu Glu Val Ala Asp Arg Ile Glu Glu Tyr Ala Ala
    130                 135                 140

Leu Gly Leu Asp Gln Phe Val Leu Ser Gly Tyr Pro Asn Leu Glu Glu
145                 150                 155                 160

Ala Phe His Phe Gly Glu Gly Val Ile Pro Lys Leu Leu Arg Arg Gly
                165                 170                 175

Val Asp Ile Lys Asn Gln Glu Ser Arg Val Leu Glu Pro Val Gly
            180                 185                 190


<210> 351
<211> 918
<212> DNA
<213> Corynebacterium glutamicum

<220>
<221> CDS
<222> (101)..(895)
<223> RXA00715

<400> 351
gtggtgttaa gcactaagat ggcaggttat gacttctctt aaagtaactt cgtccgcaga 60

tgcaaccaat aacaatgatg cccattttcc tgaaggtcca gtg gta acc gtt gac    115
                                           Val Val Thr Val Asp
                                           1               5

tgg ttg tca cac aac ctt gac cgg gat gat gtc atc gtg ttg tgt gcc    163
Trp Leu Ser His Asn Leu Asp Arg Asp Asp Val Ile Val Leu Cys Ala
                10                  15                  20

aca atg gag gat gat gaa att gca cgt caa gcg gga att ccg ggg gca    211
Thr Met Glu Asp Asp Glu Ile Ala Arg Gln Ala Gly Ile Pro Gly Ala
            25                  30                  35

ttt ctc gct gac ttg gaa gga gat ttc tca gat cca cat tcc gag ctt    259
Phe Leu Ala Asp Leu Glu Gly Asp Phe Ser Asp Pro His Ser Glu Leu
        40                  45                  50

cca cac acc gcg cca cca aat ttg gtg ggt ttg cta gaa agc tac ggc    307
Pro His Thr Ala Pro Pro Asn Leu Val Gly Leu Leu Glu Ser Tyr Gly
    55                  60                  65

att agc acc gat tcc acg gtg gtt gtt tat gat ctg cac ggc ctc atg    355
Ile Ser Thr Asp Ser Thr Val Val Val Tyr Asp Leu His Gly Leu Met
70                  75                  80                  85

gtt gca ccg cgg gtg tgg tgg ctt ctc cgt gtt gct gga tta agc agc    403
Val Ala Pro Arg Val Trp Trp Leu Leu Arg Val Ala Gly Leu Ser Ser
                90                  95                  100

att ggc gtg ctt gat ggc gga ttg cca gcc tgg gtt gat gct ggc ctt    451
Ile Gly Val Leu Asp Gly Gly Leu Pro Ala Trp Val Asp Ala Gly Leu
            105                 110                 115

cca acg gaa ccg ctg tcg cta cct aca agt ggt gga agg atc agc gca    499
Pro Thr Glu Pro Leu Ser Leu Pro Thr Ser Gly Gly Arg Ile Ser Ala
        120                 125                 130

gaa cca cag cca gat tta ctc gtt ggt gcc tcc ggc gtt gaa cgg gcg    547
Glu Pro Gln Pro Asp Leu Leu Val Gly Ala Ser Gly Val Glu Arg Ala
    135                 140                 145

atc gcg cgc tca agc aag gca gtg att gat gct cgt aat gcg agc cga     595
Ile Ala Arg Ser Ser Lys Ala Val Ile Asp Ala Arg Asn Ala Ser Arg
150                 155                 160                 165

ttc gct ggc gtt gaa gaa gag ccc cgt cca ggc ctt cga aaa ggg tcg     643
Phe Ala Gly Val Glu Glu Glu Pro Arg Pro Gly Leu Arg Lys Gly Ser
                170                 175                 180

atc cct gga agc gtc aac att ccc ttc act gac att tct gat gag cat     691
Ile Pro Gly Ser Val Asn Ile Pro Phe Thr Asp Ile Ser Asp Glu His
            185                 190                 195

ggt ttt gtc cgg cca gca gaa gaa ctg aag gaa ttg atc ttc agc cgc     739
Gly Phe Val Arg Pro Ala Glu Glu Leu Lys Glu Leu Ile Phe Ser Arg
        200                 205                 210

aca aat gga gcg cag tcg ttg gtc ttt agc tgt ggc tcc gga gtc acg     787
Thr Asn Gly Ala Gln Ser Leu Val Phe Ser Cys Gly Ser Gly Val Thr
    215                 220                 225

gca tgt gtt gat gcc tac gct gca gtt atc gca ggt tat gac gac gtt     835
Ala Cys Val Asp Ala Tyr Ala Ala Val Ile Ala Gly Tyr Asp Asp Val
230                 235                 240                 245

gta gtg tat gaa ggc tct tgg gcg gag tgg ggc aac ccg gca aac caa     883
Val Val Tyr Glu Gly Ser Trp Ala Glu Trp Gly Asn Pro Ala Asn Gln
                250                 255                 260

aag ccg att gct taacgcccgc tatgataacc act                           918
Lys Pro Ile Ala
            265


<210> 352
<211> 265
<212> PRT
<213> Corynebacterium glutamicum

<400> 352
Val Val Thr Val Asp Trp Leu Ser His Asn Leu Asp Arg Asp Asp Val
1               5                   10                  15

Ile Val Leu Cys Ala Thr Met Glu Asp Asp Glu Ile Ala Arg Gln Ala
            20                  25                  30

Gly Ile Pro Gly Ala Phe Leu Ala Asp Leu Glu Gly Asp Phe Ser Asp
        35                  40                  45

Pro His Ser Glu Leu Pro His Thr Ala Pro Pro Asn Leu Val Gly Leu
    50                  55                  60

Leu Glu Ser Tyr Gly Ile Ser Thr Asp Ser Thr Val Val Val Tyr Asp
65                  70                  75                  80

Leu His Gly Leu Met Val Ala Pro Arg Val Trp Trp Leu Leu Arg Val
                85                  90                  95

Ala Gly Leu Ser Ser Ile Gly Val Leu Asp Gly Gly Leu Pro Ala Trp
            100                 105                 110

Val Asp Ala Gly Leu Pro Thr Glu Pro Leu Ser Leu Pro Thr Ser Gly
        115                 120                 125

Gly Arg Ile Ser Ala Glu Pro Gln Pro Asp Leu Leu Val Gly Ala Ser
    130                 135                 140

Gly Val Glu Arg Ala Ile Ala Arg Ser Ser Lys Ala Val Ile Asp Ala
145                 150                 155                 160

Arg Asn Ala Ser Arg Phe Ala Gly Val Glu Glu Glu Pro Arg Pro Gly
                165                 170                 175

Leu Arg Lys Gly Ser Ile Pro Gly Ser Val Asn Ile Pro Phe Thr Asp
            180                 185                 190

Ile Ser Asp Glu His Gly Phe Val Arg Pro Ala Glu Glu Leu Lys Glu
        195                 200                 205

Leu Ile Phe Ser Arg Thr Asn Gly Ala Gln Ser Leu Val Phe Ser Cys
    210                 215                 220

Gly Ser Gly Val Thr Ala Cys Val Asp Ala Tyr Ala Ala Val Ile Ala
225                 230                 235                 240

Gly Tyr Asp Asp Val Val Val Tyr Glu Gly Ser Trp Ala Glu Trp Gly
                245                 250                 255

Asn Pro Ala Asn Gln Lys Pro Ile Ala
            260                 265


<210> 353
<211> 945
<212> DNA
<213> Corynebacterium glutamicum

<220>
<221> CDS
<222> (101)..(922)
<223> RXA01664

<400> 353
cgggttacca aagtgaatgg taggggaagt ttccgtgtct tataccggtt aggttttgcc 60

cgcgctgcgc ttggtcacat taacgcctag gctcggggct atg acc gtg ttg att    115
                                          Met Thr Val Leu Ile
                                          1               5

tct ccg tcc acc ctt gct gaa tca atc cac gct ggt aag aaa caa act    163
Ser Pro Ser Thr Leu Ala Glu Ser Ile His Ala Gly Lys Lys Gln Thr
                10                  15                  20

gtt ctc gct gct ttc tgg gct cca att gaa gga gca ggc cgc aca gtt    211
Val Leu Ala Ala Phe Trp Ala Pro Ile Glu Gly Ala Gly Arg Thr Val
            25                  30                  35

ttc tgc tct gag cac atc cca act tcc att ttc tgc gac cct gcc ctt    259
Phe Cys Ser Glu His Ile Pro Thr Ser Ile Phe Cys Asp Pro Ala Leu
        40                  45                  50

gag ctt tcc gga gtt cct tcc tct gaa gat ggc cgc aac cca ctg cca    307
Glu Leu Ser Gly Val Pro Ser Ser Glu Asp Gly Arg Asn Pro Leu Pro
    55                  60                  65

ccg ctg aat gtg ttg gca cgt tct ttc agg acc tgg ggt ttg aat acc    355
Pro Leu Asn Val Leu Ala Arg Ser Phe Arg Thr Trp Gly Leu Asn Thr
70                  75                  80                  85

gat cgt gaa atc gtg ttt tac gat cag gga cgt ggc ctt ttt gct gca    403
Asp Arg Glu Ile Val Phe Tyr Asp Gln Gly Arg Gly Leu Phe Ala Ala
                90                  95                  100

cgc gcc tgg tgg atc ctc cga tgg gcg ggc atg ccc aac gtt cgc atc    451
Arg Ala Trp Trp Ile Leu Arg Trp Ala Gly Met Pro Asn Val Arg Ile
            105                 110                 115

ctt gac ggt ggt ttc cag aag tgg gaa gac cat gag ctg gga cac gct    499
Leu Asp Gly Gly Phe Gln Lys Trp Glu Asp His Glu Leu Gly His Ala
        120                 125                 130

ggc ggg cct gga aac ttc ccg cac ttt tgc aat gtg cgt ccc aac cca    547
Gly Gly Pro Gly Asn Phe Pro His Phe Cys Asn Val Arg Pro Asn Pro
    135                 140                 145

ggt cag ctg tcg gta gcg acc atc gaa gat gtc aag gca cat cag ggc    595
Gly Gln Leu Ser Val Ala Thr Ile Glu Asp Val Lys Ala His Gln Gly
150                 155                 160                 165

att ttg att gat tct cgc gat gaa caa cga ttt gcg ggt cgc agt gaa    643
Ile Leu Ile Asp Ser Arg Asp Glu Gln Arg Phe Ala Gly Arg Ser Glu
                170                 175                 180

aag ctc gat ctg aaa gcc gga cac att cca ggc gct atc aac atc aac    691
Lys Leu Asp Leu Lys Ala Gly His Ile Pro Gly Ala Ile Asn Ile Asn

185                 190                 195

gct aaa tct ttg ctg gaa gat gat ttc acc ttc aaa tca cca gaa gaa     739
Ala Lys Ser Leu Leu Glu Asp Asp Phe Thr Phe Lys Ser Pro Glu Glu
        200                 205                 210

atc cgc cag att ttt gcg gac aag ggg gta acc agc gga gag aac gtc     787
Ile Arg Gln Ile Phe Ala Asp Lys Gly Val Thr Ser Gly Glu Asn Val
    215                 220                 225

atc gtt tat tcc ggt tcc ggt aac cac tcg tcc cag ttg ctg gct ggc     835
Ile Val Tyr Ser Gly Ser Gly Asn His Ser Ser Gln Leu Leu Ala Gly
230                 235                 240                 245

atg gag cac gcg ggg cta acc ggt gcg agc cat tat ttt gct ggt tgg     883
Met Glu His Ala Gly Leu Thr Gly Ala Ser His Tyr Phe Ala Gly Trp
                250                 255                 260

tca cag tgg agc gct aac ccc gag aat cct atc gag gcc taaaatcgtg     932
Ser Gln Trp Ser Ala Asn Pro Glu Asn Pro Ile Glu Ala
            265                 270

gcttgagtac gca                                                      945


<210> 354
<211> 274
<212> PRT
<213> Corynebacterium glutamicum

<400> 354
Met Thr Val Leu Ile Ser Pro Ser Thr Leu Ala Glu Ser Ile His Ala
1               5                   10                  15

Gly Lys Lys Gln Thr Val Leu Ala Ala Phe Trp Ala Pro Ile Glu Gly
            20                  25                  30

Ala Gly Arg Thr Val Phe Cys Ser Glu His Ile Pro Thr Ser Ile Phe
        35                  40                  45

Cys Asp Pro Ala Leu Glu Leu Ser Gly Val Pro Ser Ser Glu Asp Gly
    50                  55                  60

Arg Asn Pro Leu Pro Pro Leu Asn Val Leu Ala Arg Ser Phe Arg Thr
65                  70                  75                  80

Trp Gly Leu Asn Thr Asp Arg Glu Ile Val Phe Tyr Asp Gln Gly Arg
                85                  90                  95

Gly Leu Phe Ala Ala Arg Ala Trp Trp Ile Leu Arg Trp Ala Gly Met
            100                 105                 110

Pro Asn Val Arg Ile Leu Asp Gly Gly Phe Gln Lys Trp Glu Asp His
        115                 120                 125

Glu Leu Gly His Ala Gly Gly Pro Gly Asn Phe Pro His Phe Cys Asn
    130                 135                 140

Val Arg Pro Asn Pro Gly Gln Leu Ser Val Ala Thr Ile Glu Asp Val
145                 150                 155                 160

Lys Ala His Gln Gly Ile Leu Ile Asp Ser Arg Asp Glu Gln Arg Phe
                165                 170                 175

Ala Gly Arg Ser Glu Lys Leu Asp Leu Lys Ala Gly His Ile Pro Gly
            180                 185                 190

Ala Ile Asn Ile Asn Ala Lys Ser Leu Leu Glu Asp Asp Phe Thr Phe
        195                 200                 205

Lys Ser Pro Glu Glu Ile Arg Gln Ile Phe Ala Asp Lys Gly Val Thr
    210                 215                 220

Ser Gly Glu Asn Val Ile Val Tyr Ser Gly Ser Gly Asn His Ser Ser
225                 230                 235                 240

Gln Leu Leu Ala Gly Met Glu His Ala Gly Leu Thr Gly Ala Ser His

245                 250                 255

Tyr Phe Ala Gly Trp Ser Gln Trp Ser Ala Asn Pro Glu Asn Pro Ile
            260                 265                 270

Glu Ala

<210> 355
<211> 746
<212> DNA
<213> Corynebacterium glutamicum

<220>
<221> CDS
<222> (1)..(723)
<223> RXN02334

<400> 355
gtc aaa gac ctc aac gat ccc ctc acc cgc gat ttc att gac ggt gaa      48
Val Lys Asp Leu Asn Asp Pro Leu Thr Arg Asp Phe Ile Asp Gly Glu
  1               5                  10                  15

gct ttc gct gag ctg atg aac cgc aag ggc atc gct cgc gat gac acc      96
Ala Phe Ala Glu Leu Met Asn Arg Lys Gly Ile Ala Arg Asp Asp Thr
             20                  25                  30

gtt gtt gtc tac ggt gac aag tcc aac tgg tgg gct gcg ttc acc ctg     144
Val Val Val Tyr Gly Asp Lys Ser Asn Trp Trp Ala Ala Phe Thr Leu
         35                  40                  45

tgg gtc ttc gaa ctg ttc ggc cac tcc gat gtc cgc ctg ctc aac ggc     192
Trp Val Phe Glu Leu Phe Gly His Ser Asp Val Arg Leu Leu Asn Gly
     50                  55                  60

ggc cgc gac gcg tgg atg gct gaa gag cgc gac acc tcc tac gtg gtt     240
Gly Arg Asp Ala Trp Met Ala Glu Glu Arg Asp Thr Ser Tyr Val Val
 65                  70                  75                  80

ccg gag tac ccc tcc gcc aac tac ccc gtc gtg gag cgt gtc gac gaa     288
Pro Glu Tyr Pro Ser Ala Asn Tyr Pro Val Val Glu Arg Val Asp Glu
                 85                  90                  95

aac cag cgc gcg ttc gtg gct gag gtg ctc ggt tcg ctc acg caa tcc     336
Asn Gln Arg Ala Phe Val Ala Glu Val Leu Gly Ser Leu Thr Gln Ser
            100                 105                 110

ggt ggc atg acg ctt gtc gac gtc agg acc cct tcg gag ttc tcc gga     384
Gly Gly Met Thr Leu Val Asp Val Arg Thr Pro Ser Glu Phe Ser Gly
        115                 120                 125

ttg gat gag cac ggc aac cca acc tca aac acc ggc gtg ctt cgt ggt     432
Leu Asp Glu His Gly Asn Pro Thr Ser Asn Thr Gly Val Leu Arg Gly
    130                 135                 140

gga cac atc cca ggc gcg atc aac ctg gat tgg tcg gac gct gtt ctt     480
Gly His Ile Pro Gly Ala Ile Asn Leu Asp Trp Ser Asp Ala Val Leu
145                 150                 155                 160

ccc aac gga aac ttc cgc acc cgt gca gag ttg gac aag ctc tac gcc     528
Pro Asn Gly Asn Phe Arg Thr Arg Ala Glu Leu Asp Lys Leu Tyr Ala
                165                 170                 175

gat ctc aac cca gct gac gat acc gtt gtc tac tgc cag gtt ggc gac     576
Asp Leu Asn Pro Ala Asp Asp Thr Val Val Tyr Cys Gln Val Gly Asp
            180                 185                 190

cgc gcg gcc cac acc tgg ttc gtg ctg aag tat ctg ctc ggt ttc aac     624
Arg Ala Ala His Thr Trp Phe Val Leu Lys Tyr Leu Leu Gly Phe Asn
        195                 200                 205

aac gtc cga aac tat gac gga tcg tgg gca gaa tgg ggc aat atg gtt     672
Asn Val Arg Asn Tyr Asp Gly Ser Trp Ala Glu Trp Gly Asn Met Val
    210                 215                 220

cgc atg ccg atc gaa act ggc gaa aac acc aaa aat aac gtt tcg gtg     720

Arg Met Pro Ile Glu Thr Gly Glu Asn Thr Lys Asn Asn Val Ser Val
225                 230                 235                 240

tca tagaataggc gtatcccctt ttt                                    746
Ser

<210> 356
<211> 241
<212> PRT
<213> Corynebacterium glutamicum

<400> 356
Val Lys Asp Leu Asn Asp Pro Leu Thr Arg Asp Phe Ile Asp Gly Glu
1               5                   10                  15

Ala Phe Ala Glu Leu Met Asn Arg Lys Gly Ile Ala Arg Asp Asp Thr
            20                  25                  30

Val Val Val Tyr Gly Asp Lys Ser Asn Trp Trp Ala Ala Phe Thr Leu
        35                  40                  45

Trp Val Phe Glu Leu Phe Gly His Ser Asp Val Arg Leu Leu Asn Gly
    50                  55                  60

Gly Arg Asp Ala Trp Met Ala Glu Glu Arg Asp Thr Ser Tyr Val Val
65                  70                  75                  80

Pro Glu Tyr Pro Ser Ala Asn Tyr Pro Val Val Glu Arg Val Asp Glu
                85                  90                  95

Asn Gln Arg Ala Phe Val Ala Glu Val Leu Gly Ser Leu Thr Gln Ser
            100                 105                 110

Gly Gly Met Thr Leu Val Asp Val Arg Thr Pro Ser Glu Phe Ser Gly
        115                 120                 125

Leu Asp Glu His Gly Asn Pro Thr Ser Asn Thr Gly Val Leu Arg Gly
    130                 135                 140

Gly His Ile Pro Gly Ala Ile Asn Leu Asp Trp Ser Asp Ala Val Leu
145                 150                 155                 160

Pro Asn Gly Asn Phe Arg Thr Arg Ala Glu Leu Asp Lys Leu Tyr Ala
                165                 170                 175

Asp Leu Asn Pro Ala Asp Asp Thr Val Val Tyr Cys Gln Val Gly Asp
            180                 185                 190

Arg Ala Ala His Thr Trp Phe Val Leu Lys Tyr Leu Leu Gly Phe Asn
        195                 200                 205

Asn Val Arg Asn Tyr Asp Gly Ser Trp Ala Glu Trp Gly Asn Met Val
    210                 215                 220

Arg Met Pro Ile Glu Thr Gly Glu Asn Thr Lys Asn Asn Val Ser Val
225                 230                 235                 240

Ser

<210> 357
<211> 377
<212> DNA
<213> Corynebacterium glutamicum

<220>
<221> CDS
<222> (1)..(354)
<223> FRXA02334

<400> 357
gga gtt ctc ccg gat tgg atg agc acg gca acc caa ccc tca aac acc    48
Gly Val Leu Pro Asp Trp Met Ser Thr Ala Thr Gln Pro Ser Asn Thr
1               5                   10                  15

ggc gtg ctt cgt ggt gaa cac atc cca ggc gcg atc aac ctg gat tgg     96
Gly Val Leu Arg Gly Glu His Ile Pro Gly Ala Ile Asn Leu Asp Trp
            20                  25                  30

tcg gac gct gtt ctt ccc aac gga aac ttc cgc acc cgt gca gag ttg     144
Ser Asp Ala Val Leu Pro Asn Gly Asn Phe Arg Thr Arg Ala Glu Leu
        35                  40                  45

gac aag ctc tac gcc gat ctc aac cca gct gac gat acc gtt gtc tac     192
Asp Lys Leu Tyr Ala Asp Leu Asn Pro Ala Asp Asp Thr Val Val Tyr
    50                  55                  60

tgc cag gtt ggc gac cgc gcg gcc cac acc tgg ttc gtg ctg aag tat     240
Cys Gln Val Gly Asp Arg Ala Ala His Thr Trp Phe Val Leu Lys Tyr
65                  70                  75                  80

ctg ctc ggt ttc aac aac gtc cga aac tat gac gga tcg tgg gca gaa     288
Leu Leu Gly Phe Asn Asn Val Arg Asn Tyr Asp Gly Ser Trp Ala Glu
                85                  90                  95

tgg ggc aat atg gtt cgc atg ccg atc gaa act ggc gaa aac acc aaa     336
Trp Gly Asn Met Val Arg Met Pro Ile Glu Thr Gly Glu Asn Thr Lys
            100                 105                 110

aat aac gtt tcg gtg tca tagaataggc gtatcccctt ttt                   377
Asn Asn Val Ser Val Ser
        115


<210> 358
<211> 118
<212> PRT
<213> Corynebacterium glutamicum

<400> 358
Gly Val Leu Pro Asp Trp Met Ser Thr Ala Thr Gln Pro Ser Asn Thr
  1               5                  10                  15

Gly Val Leu Arg Gly Glu His Ile Pro Gly Ala Ile Asn Leu Asp Trp
            20                  25                  30

Ser Asp Ala Val Leu Pro Asn Gly Asn Phe Arg Thr Arg Ala Glu Leu
        35                  40                  45

Asp Lys Leu Tyr Ala Asp Leu Asn Pro Ala Asp Asp Thr Val Val Tyr
    50                  55                  60

Cys Gln Val Gly Asp Arg Ala Ala His Thr Trp Phe Val Leu Lys Tyr
 65                  70                  75                  80

Leu Leu Gly Phe Asn Asn Val Arg Asn Tyr Asp Gly Ser Trp Ala Glu
                85                  90                  95

Trp Gly Asn Met Val Arg Met Pro Ile Glu Thr Gly Glu Asn Thr Lys
            100                 105                 110

Asn Asn Val Ser Val Ser
        115


<210> 359
<211> 3945
<212> DNA
<213> Corynebacterium glutamicum

<220>
<221> CDS
<222> (101)..(3922)
<223> RXN01499

<400> 359
gcagcaatta tctccaccga agaggactaa atataacgtg gcattgagca gtgttccagc 60

acagttcctg agatccgccc aggcgccccc gaagcgtact ttg tgg gac gtc tta     115
                                           Leu Trp Asp Val Leu
                                             1               5

gaa tcc gtc gcc tct act tat cct gag gca gca gct att gac gat ggc    163
Glu Ser Val Ala Ser Thr Tyr Pro Glu Ala Ala Ala Ile Asp Asp Gly
                10                  15                  20

cag gtg ttg acc tac gca gag ttg atg gaa gaa gtc acc gcg ttg gct    211
Gln Val Leu Thr Tyr Ala Glu Leu Met Glu Glu Val Thr Ala Leu Ala
                25                  30                  35

gat tcc att cat gca cag ggc att cgc cgt ggt gat cgc atc ggt att    259
Asp Ser Ile His Ala Gln Gly Ile Arg Arg Gly Asp Arg Ile Gly Ile
        40                  45                  50

cgc atg ccg tct ggt acg cgt gac ctt tac atc gct att ttg gcc act    307
Arg Met Pro Ser Gly Thr Arg Asp Leu Tyr Ile Ala Ile Leu Ala Thr
    55                  60                  65

ctc gct gct ggt gct gct tac gtg cca gtt gat gca gat gat cct gaa    355
Leu Ala Ala Gly Ala Ala Tyr Val Pro Val Asp Ala Asp Asp Pro Glu
 70                  75                  80                  85

gag cgc gcc gag atg gtg ttt ggt gaa gca aat att aat gcg ctt ttc    403
Glu Arg Ala Glu Met Val Phe Gly Glu Ala Asn Ile Asn Ala Leu Phe
                90                  95                  100

gac gcc acc ggc ttc cat atg ctt cgc ccg acc gcg ggc ggc gat acc    451
Asp Ala Thr Gly Phe His Met Leu Arg Pro Thr Ala Gly Gly Asp Thr
            105                 110                 115

cgt aga cca cgc ttg gat gat acg gcg tgg att atc ttt act tcc ggt    499
Arg Arg Pro Arg Leu Asp Asp Thr Ala Trp Ile Ile Phe Thr Ser Gly
        120                 125                 130

tcc acc ggc aag cct aag ggt gtg gct gtg tcc cac cgt tca gct gcg    547
Ser Thr Gly Lys Pro Lys Gly Val Ala Val Ser His Arg Ser Ala Ala
    135                 140                 145

gct ttc gtg gat gcc gaa gca caa atg ttc ctt gtc gat cac cct tcc    595
Ala Phe Val Asp Ala Glu Ala Gln Met Phe Leu Val Asp His Pro Ser
150                 155                 160                 165

ggc ccc ctt ggc cca gaa gac cga gtc ctt gcg gga ttg tct gta gcc    643
Gly Pro Leu Gly Pro Glu Asp Arg Val Leu Ala Gly Leu Ser Val Ala
                170                 175                 180

ttt gac gca tct tgt gag gaa atg tgg ttg gcc tgg ggc cac ggc gcc    691
Phe Asp Ala Ser Cys Glu Glu Met Trp Leu Ala Trp Gly His Gly Ala
            185                 190                 195

tgc ttg gtg cca gca cca cgc tcc cta gtc cgt tcc ggt atg gac ttg    739
Cys Leu Val Pro Ala Pro Arg Ser Leu Val Arg Ser Gly Met Asp Leu
        200                 205                 210

ggc cca tgg ctg att cgc cgc gac atc agt gtc gtc tcc acc gtc cca    787
Gly Pro Trp Leu Ile Arg Arg Asp Ile Ser Val Val Ser Thr Val Pro
    215                 220                 225

act ctg gct ggt ctg tgg cca gca gaa gca ttg tca cag gtc cgc ttg    835
Thr Leu Ala Gly Leu Trp Pro Ala Glu Ala Leu Ser Gln Val Arg Leu
230                 235                 240                 245

ctc atc gtc ggc ggc gag gct tgc tcg cag gag ctc gtt gaa cgc tta    883
Leu Ile Val Gly Gly Glu Ala Cys Ser Gln Glu Leu Val Glu Arg Leu
                250                 255                 260

tcg acg cct gac cgc gag gtg tgg aac act tac ggc ccc acc gaa gca    931
Ser Thr Pro Asp Arg Glu Val Trp Asn Thr Tyr Gly Pro Thr Glu Ala
            265                 270                 275

acg gtg gtt gcc tgt ggc act caa ctc tat gct ggt cag cca gtg ggc    979
Thr Val Val Ala Cys Gly Thr Gln Leu Tyr Ala Gly Gln Pro Val Gly
        280                 285                 290

att ggt ttg cca ctt gct ggt tgg gat ctt gtt gtt gtc gac gat gcc    1027
Ile Gly Leu Pro Leu Ala Gly Trp Asp Leu Val Val Val Asp Asp Ala
    295                 300                 305

ggc gaa cct gtc gga atc ggc gag gtc ggc gaa ttg gtc atc ggt ggt 1075
Gly Glu Pro Val Gly Ile Gly Glu Val Gly Glu Leu Val Ile Gly Gly
310                 315                 320                 325

gtg ggt ctt gca cgc tac ctt gat cca gaa aaa gac cgc gag aag tat 1123
Val Gly Leu Ala Arg Tyr Leu Asp Pro Glu Lys Asp Arg Glu Lys Tyr
                330                 335                 340

gcg cca ctg aag tct gtt ggt tgg acc cgc gct tat cgt tcc ggt gac 1171
Ala Pro Leu Lys Ser Val Gly Trp Thr Arg Ala Tyr Arg Ser Gly Asp
            345                 350                 355

cac gtt cgt ctg gaa gaa gat ggc ctc tac ttt gtg ggc cgc gtt gat 1219
His Val Arg Leu Glu Glu Asp Gly Leu Tyr Phe Val Gly Arg Val Asp
        360                 365                 370

gat cag gtg aaa atc ggc ggt cga cgc atc gag ctc ggt gaa gtt gat 1267
Asp Gln Val Lys Ile Gly Gly Arg Arg Ile Glu Leu Gly Glu Val Asp
    375                 380                 385

gcc aat gtg gca gcg ctt tcc aac gtt cgt tcc tcc gca gtg gtt gtt 1315
Ala Asn Val Ala Ala Leu Ser Asn Val Arg Ser Ser Ala Val Val Val
390                 395                 400                 405

cag acc act ggt gcg gat caa aaa gtt ctg gtt gca tac gtt tct ttg 1363
Gln Thr Thr Gly Ala Asp Gln Lys Val Leu Val Ala Tyr Val Ser Leu
                410                 415                 420

gaa gat gct gca gct gga ttt gat cac aac gtc gcg act gcc cga ctc 1411
Glu Asp Ala Ala Ala Gly Phe Asp His Asn Val Ala Thr Ala Arg Leu
            425                 430                 435

acc gaa acc atg cct gct gct ttg gtt ccg cgc att cac gtg atg gat 1459
Thr Glu Thr Met Pro Ala Ala Leu Val Pro Arg Ile His Val Met Asp
        440                 445                 450

gat ctg cct gtc acc acc tcc ggc aag gtt gat aag aag tct ttg ccg 1507
Asp Leu Pro Val Thr Thr Ser Gly Lys Val Asp Lys Lys Ser Leu Pro
    455                 460                 465

tgg cct ctt cct ggc acc gtg gtg gaa gct aat gac ctc agc gca acg 1555
Trp Pro Leu Pro Gly Thr Val Val Glu Ala Asn Asp Leu Ser Ala Thr
470                 475                 480                 485

gaa gcg tgg att gct cag gaa tgg gtc gat atc ctc ggc act tct gtg 1603
Glu Ala Trp Ile Ala Gln Glu Trp Val Asp Ile Leu Gly Thr Ser Val
                490                 495                 500

agc agc aaa gac gcc gac ttc ttc tcc ctt ggc ggt acc tct ctc gcg 1651
Ser Ser Lys Asp Ala Asp Phe Phe Ser Leu Gly Gly Thr Ser Leu Ala
            505                 510                 515

gct gcg act ttg gtt ggc cgg gta cgc gca aag gtt ccc acc gct gcg 1699
Ala Ala Thr Leu Val Gly Arg Val Arg Ala Lys Val Pro Thr Ala Ala
        520                 525                 530

gtg cgt gat ctt tac gat cac cct cgc ttg gag aaa ttc gcc gag cgt 1747
Val Arg Asp Leu Tyr Asp His Pro Arg Leu Glu Lys Phe Ala Glu Arg
    535                 540                 545

gtc gag gct atc gcc gcc gac act ggc att tct ttg gag gcg cca aac 1795
Val Glu Ala Ile Ala Ala Asp Thr Gly Ile Ser Leu Glu Ala Pro Asn
550                 555                 560                 565

cag gtg gag gag cgc gtc gtc aag cct gtt tct ttt ggc act cgt gtg 1843
Gln Val Glu Glu Arg Val Val Lys Pro Val Ser Phe Gly Thr Arg Val
                570                 575                 580

atg cag acc ctc atc cag att ccg atc atg acg ctg caa gca gca cag 1891
Met Gln Thr Leu Ile Gln Ile Pro Ile Met Thr Leu Gln Ala Ala Gln
            585                 590                 595

tgg att gca tgg ttg ctg ttg ggc aac aac atc atg gca gcg ctt gat 1939
Trp Ile Ala Trp Leu Leu Leu Gly Asn Asn Ile Met Ala Ala Leu Asp
        600                 605                 610

ttc gat tgg gct gtt cat gtc tcc tgg tgg ctt gtc atc ggc atg att 1987

Phe Asp Trp Ala Val His Val Ser Trp Trp Leu Val Ile Gly Met Ile
    615                 620                 625

ttg gtg ttc gct acc ccg att ggt cgc ttg ccg atc ggc ggt tgg ggc     2035
Leu Val Phe Ala Thr Pro Ile Gly Arg Leu Pro Ile Gly Gly Trp Gly
630                 635                 640                 645

gcc cgc atc atc acc cgt ggc ata act cct ggc tcc tac cct cgt ggc     2083
Ala Arg Ile Ile Thr Arg Gly Ile Thr Pro Gly Ser Tyr Pro Arg Gly
                650                 655                 660

ggt tcc act cac ctg cgc att tgg tcc gcc gag cgc ctt gct gat gcc     2131
Gly Ser Thr His Leu Arg Ile Trp Ser Ala Glu Arg Leu Ala Asp Ala
            665                 670                 675

tct ggc tct cgc aat att tct ggc gca acc tgg gtg aac tac ttc gcg     2179
Ser Gly Ser Arg Asn Ile Ser Gly Ala Thr Trp Val Asn Tyr Phe Ala
        680                 685                 690

cgt tcc ctg ggt gtg aag atg ggc aag ggc gtg gat ctt cac tcc ctg     2227
Arg Ser Leu Gly Val Lys Met Gly Lys Gly Val Asp Leu His Ser Leu
    695                 700                 705

cca cca atc act ggc ctt ttg acc ttg ggc aac aat gtt tcc atc gag     2275
Pro Pro Ile Thr Gly Leu Leu Thr Leu Gly Asn Asn Val Ser Ile Glu
710                 715                 720                 725

caa gaa gtt gac ctt cgt ggc tac tgg ctc gac ggc gat atc ctg cgt     2323
Gln Glu Val Asp Leu Arg Gly Tyr Trp Leu Asp Gly Asp Ile Leu Arg
                730                 735                 740

gta ggc acc att gag gtc cat gac aac gct cgc atc ggc gct cgt tcc     2371
Val Gly Thr Ile Glu Val His Asp Asn Ala Arg Ile Gly Ala Arg Ser
            745                 750                 755

acc ctg ctt ccc ggc acc gtg gtg ggc acc ggc gct cac ctg ctg cct     2419
Thr Leu Leu Pro Gly Thr Val Val Gly Thr Gly Ala His Leu Leu Pro
        760                 765                 770

ggt tca aca gtg act ggt gat aag acc atc aag cct ggt tct cgt tgg     2467
Gly Ser Thr Val Thr Gly Asp Lys Thr Ile Lys Pro Gly Ser Arg Trp
    775                 780                 785

gct ggc tcc cct gca caa aag gtg ggt cgt gca aag cac cgg ttc cca     2515
Ala Gly Ser Pro Ala Gln Lys Val Gly Arg Ala Lys His Arg Phe Pro
790                 795                 800                 805

acc tcc cat cct cca cgc agg tcc cgg tgg gtt ccg gtg ttc ggc gcg     2563
Thr Ser His Pro Pro Arg Arg Ser Arg Trp Val Pro Val Phe Gly Ala
                810                 815                 820

acc tcc atc gtg ttg tcg ctg ctg cca ctt cag gct ctc gct att ggc     2611
Thr Ser Ile Val Leu Ser Leu Leu Pro Leu Gln Ala Leu Ala Ile Gly
            825                 830                 835

gct gct atc acc ttg tgg ctg gcc acg att agc ccg ctt cca ctg atc     2659
Ala Ala Ile Thr Leu Trp Leu Ala Thr Ile Ser Pro Leu Pro Leu Ile
        840                 845                 850

tgg ggt gtg ctg gtt ttt gct acc gtc ggc gcg ttg gct gcg ttc ttt     2707
Trp Gly Val Leu Val Phe Ala Thr Val Gly Ala Leu Ala Ala Phe Phe
    855                 860                 865

gct tac acc gtg acc atc tgg gtg ctt gtc cgt ttg atc cag atc ggc     2755
Ala Tyr Thr Val Thr Ile Trp Val Leu Val Arg Leu Ile Gln Ile Gly
870                 875                 880                 885

atc aag ggc ggc acc gca cca gtg agg tcc cgt ctt ggt tgg cag gtc     2803
Ile Lys Gly Gly Thr Ala Pro Val Arg Ser Arg Leu Gly Trp Gln Val
                890                 895                 900

tgg gca gtt caa cgc ctc atg gac gat gcc cgc acc tat ctc ttc ccg     2851
Trp Ala Val Gln Arg Leu Met Asp Asp Ala Arg Thr Tyr Leu Phe Pro
            905                 910                 915

ctc tac gca tcc caa ctg acc cca ctg tgg ttc cgc agc ttg ggc gcg     2899
Leu Tyr Ala Ser Gln Leu Thr Pro Leu Trp Phe Arg Ser Leu Gly Ala

920                 925                 930

aag atc ggc aag gat gtt gag atc tcc acc gcg gtg atg gtt cct aaa    2947
Lys Ile Gly Lys Asp Val Glu Ile Ser Thr Ala Val Met Val Pro Lys
    935                 940                 945

ctg gct gat atc cgc gaa ggc gca ttc ctg gcc gat gac acc ctc atc    2995
Leu Ala Asp Ile Arg Glu Gly Ala Phe Leu Ala Asp Asp Thr Leu Ile
950                 955                 960                 965

ggt ggc tat gag ctg ggt aat ggt tgg ctg ctc agt ggt gaa acc cgc    3043
Gly Gly Tyr Glu Leu Gly Asn Gly Trp Leu Leu Ser Gly Glu Thr Arg
                970                 975                 980

gtg ggt aag cgt tcc ttc att ggt aac tct ggc atc gca gga cct gag    3091
Val Gly Lys Arg Ser Phe Ile Gly Asn Ser Gly Ile Ala Gly Pro Glu
            985                 990                 995

cgc aag ctc  gct aag aac tcc ctg  gtt gca gtg ctc tcc  tcc acc ccg    3139
Arg Lys Leu  Ala Lys Asn Ser Leu  Val Ala Val Leu Ser  Ser Thr Pro
        1000                 1005                 1010

aag aag  gct aag gcc aac tcc  aac tgg tgg ggt tcc  cct cca gag cgc    3187
Lys Lys  Ala Lys Ala Asn Ser  Asn Trp Trp Gly Ser  Pro Pro Glu Arg
    1015                 1020                 1025

atg  cgt cgt gtc act gtc  gaa gtt gat gag ggc  gaa gca aag acc tac    3235
Met  Arg Arg Val Thr Val  Glu Val Asp Glu Gly  Glu Ala Lys Thr Tyr
1030                 1035                 1040                 1045

agc cct ggc ttt ggt  gtg aag ttt gca cgt  ggc gcg gtg gaa acc  gca    3283
Ser Pro Gly Phe Gly  Val Lys Phe Ala Arg  Gly Ala Val Glu Thr  Ala
                1050                 1055                 1060

cgt ctg ctt gct  cca ata acc tct ggt  gtg ttg gct gcg ctg  tca ctg    3331
Arg Leu Leu Ala  Pro Ile Thr Ser Gly  Val Leu Ala Ala Leu  Ser Leu
            1065                 1070                 1075

ctg ctc atg  cag tac ctg ctc act  gag ttc aac atg tgg  atc acc tgg    3379
Leu Leu Met  Gln Tyr Leu Leu Thr  Glu Phe Asn Met Trp  Ile Thr Trp
        1080                 1085                 1090

ttg ctt  ggc gga ctg atc ctc  atg acg gtt ggt gtg  ctc gcc atg ggc    3427
Leu Leu  Gly Gly Leu Ile Leu  Met Thr Val Gly Val  Leu Ala Met Gly
    1095                 1100                 1105

att  acg gtt gtg atg aag  tgg gtt tgc gtc ggc  aag cat aag ccg tct    3475
Ile  Thr Val Val Met Lys  Trp Val Cys Val Gly  Lys His Lys Pro Ser
1110                 1115                 1120                 1125

gag cac cct ctc ttc  agc cgc ttt gtg tgg  ctg aat gag ctg caa  gat    3523
Glu His Pro Leu Phe  Ser Arg Phe Val Trp  Leu Asn Glu Leu Gln  Asp
                1130                 1135                 1140

gcg ttc gtg gaa  tcc gtg gct ggc cca  tgg ttc ctc gtg ccc  aac ctg    3571
Ala Phe Val Glu  Ser Val Ala Gly Pro  Trp Phe Leu Val Pro  Asn Leu
            1145                 1150                 1155

ggc acc ggc  gcg ctg aac gcc ggc  atg agc gcg ctt ggc  gca cac atc    3619
Gly Thr Gly  Ala Leu Asn Ala Gly  Met Ser Ala Leu Gly  Ala His Ile
        1160                 1165                 1170

ggc cgt  ggc gca tgg atc gaa  tcc tac tgg ctg ccg  gaa acc gac ctc    3667
Gly Arg  Gly Ala Trp Ile Glu  Ser Tyr Trp Leu Pro  Glu Thr Asp Leu
    1175                 1180                 1185

tgc  tac atc ggc aag ggc  gca acc gtg ggc cct  ggc gtg gtc gtg cag    3715
Cys  Tyr Ile Gly Lys Gly  Ala Thr Val Gly Pro  Gly Val Val Val Gln
1190                 1195                 1200                 1205

acc cac ctc ttc cag  gac cgc gtg atg agc  cta gat acg gtg acc  gtc    3763
Thr His Leu Phe Gln  Asp Arg Val Met Ser  Leu Asp Thr Val Thr  Val
                1210                 1215                 1220

gct gac ggc gcc  acc cta gcg gac cac  tcc gtt gcc ctt cct  gct tcg    3811
Ala Asp Gly Ala  Thr Leu Ala Asp His  Ser Val Ala Leu Pro  Ala Ser
            1225                 1230                 1235

ctt atc gac gcc tcc gcc acc atc ggc cca ggc tcg ctg gtg atg cgc 3859
Leu Ile Asp Ala Ser Ala Thr Ile Gly Pro Gly Ser Leu Val Met Arg
        1240                1245                1250

ggc gac aag gta cca gcg cat acc cgc tgg caa ggc aac cca att gag 3907
Gly Asp Lys Val Pro Ala His Thr Arg Trp Gln Gly Asn Pro Ile Glu
    1255                1260                1265

ccg tgg agc aac tct taaataacaa caatcagccg gat 3945
Pro Trp Ser Asn Ser
1270

<210> 360
<211> 1274
<212> PRT
<213> Corynebacterium glutamicum

<400> 360
Leu Trp Asp Val Leu Glu Ser Val Ala Ser Thr Tyr Pro Glu Ala Ala
1               5                   10                  15

Ala Ile Asp Asp Gly Gln Val Leu Thr Tyr Ala Glu Leu Met Glu Glu
            20                  25                  30

Val Thr Ala Leu Ala Asp Ser Ile His Ala Gln Gly Ile Arg Arg Gly
        35                  40                  45

Asp Arg Ile Gly Ile Arg Met Pro Ser Gly Thr Arg Asp Leu Tyr Ile
    50                  55                  60

Ala Ile Leu Ala Thr Leu Ala Ala Gly Ala Ala Tyr Val Pro Val Asp
65                  70                  75                  80

Ala Asp Asp Pro Glu Glu Arg Ala Glu Met Val Phe Gly Glu Ala Asn
                85                  90                  95

Ile Asn Ala Leu Phe Asp Ala Thr Gly Phe His Met Leu Arg Pro Thr
            100                 105                 110

Ala Gly Gly Asp Thr Arg Arg Pro Arg Leu Asp Asp Thr Ala Trp Ile
        115                 120                 125

Ile Phe Thr Ser Gly Ser Thr Gly Lys Pro Lys Gly Val Ala Val Ser
    130                 135                 140

His Arg Ser Ala Ala Ala Phe Val Asp Ala Glu Ala Gln Met Phe Leu
145                 150                 155                 160

Val Asp His Pro Ser Gly Pro Leu Gly Pro Glu Asp Arg Val Leu Ala
                165                 170                 175

Gly Leu Ser Val Ala Phe Asp Ala Ser Cys Glu Glu Met Trp Leu Ala
            180                 185                 190

Trp Gly His Gly Ala Cys Leu Val Pro Ala Pro Arg Ser Leu Val Arg
        195                 200                 205

Ser Gly Met Asp Leu Gly Pro Trp Leu Ile Arg Arg Asp Ile Ser Val
    210                 215                 220

Val Ser Thr Val Pro Thr Leu Ala Gly Leu Trp Pro Ala Glu Ala Leu
225                 230                 235                 240

Ser Gln Val Arg Leu Leu Ile Val Gly Gly Glu Ala Cys Ser Gln Glu
                245                 250                 255

Leu Val Glu Arg Leu Ser Thr Pro Asp Arg Glu Val Trp Asn Thr Tyr
            260                 265                 270

Gly Pro Thr Glu Ala Thr Val Val Ala Cys Gly Thr Gln Leu Tyr Ala
        275                 280                 285

Gly Gln Pro Val Gly Ile Gly Leu Pro Leu Ala Gly Trp Asp Leu Val
    290                 295                 300

Val Val Asp Asp Ala Gly Glu Pro Val Gly Ile Gly Glu Val Gly Glu
305                 310                 315                 320

Leu Val Ile Gly Gly Val Gly Leu Ala Arg Tyr Leu Asp Pro Glu Lys
                325                 330                 335

Asp Arg Glu Lys Tyr Ala Pro Leu Lys Ser Val Gly Trp Thr Arg Ala
            340                 345                 350

Tyr Arg Ser Gly Asp His Val Arg Leu Glu Glu Asp Gly Leu Tyr Phe
        355                 360                 365

Val Gly Arg Val Asp Asp Gln Val Lys Ile Gly Gly Arg Arg Ile Glu
    370                 375                 380

Leu Gly Glu Val Asp Ala Asn Val Ala Ala Leu Ser Asn Val Arg Ser
385                 390                 395                 400

Ser Ala Val Val Val Gln Thr Thr Gly Ala Asp Gln Lys Val Leu Val
                405                 410                 415

Ala Tyr Val Ser Leu Glu Asp Ala Ala Ala Gly Phe Asp His Asn Val
            420                 425                 430

Ala Thr Ala Arg Leu Thr Glu Thr Met Pro Ala Ala Leu Val Pro Arg
        435                 440                 445

Ile His Val Met Asp Asp Leu Pro Val Thr Thr Ser Gly Lys Val Asp
    450                 455                 460

Lys Lys Ser Leu Pro Trp Pro Leu Pro Gly Thr Val Val Glu Ala Asn
465                 470                 475                 480

Asp Leu Ser Ala Thr Glu Ala Trp Ile Ala Gln Glu Trp Val Asp Ile
                485                 490                 495

Leu Gly Thr Ser Val Ser Ser Lys Asp Ala Asp Phe Phe Ser Leu Gly
            500                 505                 510

Gly Thr Ser Leu Ala Ala Ala Thr Leu Val Gly Arg Val Arg Ala Lys
        515                 520                 525

Val Pro Thr Ala Ala Val Arg Asp Leu Tyr Asp His Pro Arg Leu Glu
    530                 535                 540

Lys Phe Ala Glu Arg Val Glu Ala Ile Ala Ala Asp Thr Gly Ile Ser
545                 550                 555                 560

Leu Glu Ala Pro Asn Gln Val Glu Glu Arg Val Val Lys Pro Val Ser
                565                 570                 575

Phe Gly Thr Arg Val Met Gln Thr Leu Ile Gln Ile Pro Ile Met Thr
            580                 585                 590

Leu Gln Ala Ala Gln Trp Ile Ala Trp Leu Leu Leu Gly Asn Asn Ile
        595                 600                 605

Met Ala Ala Leu Asp Phe Asp Trp Ala Val His Val Ser Trp Trp Leu
    610                 615                 620

Val Ile Gly Met Ile Leu Val Phe Ala Thr Pro Ile Gly Arg Leu Pro
625                 630                 635                 640

Ile Gly Gly Trp Gly Ala Arg Ile Ile Thr Arg Gly Ile Thr Pro Gly
                645                 650                 655

Ser Tyr Pro Arg Gly Gly Ser Thr His Leu Arg Ile Trp Ser Ala Glu
            660                 665                 670

Arg Leu Ala Asp Ala Ser Gly Ser Arg Asn Ile Ser Gly Ala Thr Trp
        675                 680                 685

Val Asn Tyr Phe Ala Arg Ser Leu Gly Val Lys Met Gly Lys Gly Val
    690                 695                 700

Asp Leu His Ser Leu Pro Pro Ile Thr Gly Leu Leu Thr Leu Gly Asn
705                 710                 715                 720

Asn Val Ser Ile Glu Gln Glu Val Asp Leu Arg Gly Tyr Trp Leu Asp
                725                 730                 735

Gly Asp Ile Leu Arg Val Gly Thr Ile Glu Val His Asp Asn Ala Arg
            740                 745                 750

Ile Gly Ala Arg Ser Thr Leu Leu Pro Gly Thr Val Val Gly Thr Gly
        755                 760                 765

Ala His Leu Leu Pro Gly Ser Thr Val Thr Gly Asp Lys Thr Ile Lys
    770                 775                 780

Pro Gly Ser Arg Trp Ala Gly Ser Pro Ala Gln Lys Val Gly Arg Ala
785                 790                 795                 800

Lys His Arg Phe Pro Thr Ser His Pro Pro Arg Arg Ser Arg Trp Val
                805                 810                 815

Pro Val Phe Gly Ala Thr Ser Ile Val Leu Ser Leu Leu Pro Leu Gln
            820                 825                 830

Ala Leu Ala Ile Gly Ala Ala Ile Thr Leu Trp Leu Ala Thr Ile Ser
        835                 840                 845

Pro Leu Pro Leu Ile Trp Gly Val Leu Val Phe Ala Thr Val Gly Ala
    850                 855                 860

Leu Ala Ala Phe Phe Ala Tyr Thr Val Thr Ile Trp Val Leu Val Arg
865                 870                 875                 880

Leu Ile Gln Ile Gly Ile Lys Gly Gly Thr Ala Pro Val Arg Ser Arg
                885                 890                 895

Leu Gly Trp Gln Val Trp Ala Val Gln Arg Leu Met Asp Asp Ala Arg
            900                 905                 910

Thr Tyr Leu Phe Pro Leu Tyr Ala Ser Gln Leu Thr Pro Leu Trp Phe
        915                 920                 925

Arg Ser Leu Gly Ala Lys Ile Gly Lys Asp Val Glu Ile Ser Thr Ala
    930                 935                 940

Val Met Val Pro Lys Leu Ala Asp Ile Arg Glu Gly Ala Phe Leu Ala
945                 950                 955                 960

Asp Asp Thr Leu Ile Gly Gly Tyr Glu Leu Gly Asn Gly Trp Leu Leu
                965                 970                 975

Ser Gly Glu Thr Arg Val Gly Lys Arg Ser Phe Ile Gly Asn Ser Gly
            980                 985                 990

Ile Ala Gly Pro Glu Arg Lys Leu  Ala Lys Asn Ser Leu  Val Ala Val
        995                 1000                 1005

Leu Ser  Ser Thr Pro Lys Lys  Ala Lys Ala Asn Ser  Asn Trp Trp Gly
    1010                 1015                 1020

Ser  Pro Pro Glu Arg Met  Arg Arg Val Thr Val  Glu Val Asp Glu Gly
1025                 1030                 1035                 1040

Glu Ala Lys Thr Tyr  Ser Pro Gly Phe Gly  Val Lys Phe Ala Arg  Gly
                1045                 1050                 1055

Ala Val Glu Thr  Ala Arg Leu Leu Ala  Pro Ile Thr Ser Gly  Val Leu
            1060                 1065                 1070

Ala Ala Leu  Ser Leu Leu Leu Met  Gln Tyr Leu Leu Thr  Glu Phe Asn
        1075                 1080                 1085

Met Trp  Ile Thr Trp Leu Leu  Gly Gly Leu Ile Leu  Met Thr Val Gly
    1090                 1095                 1100

Val  Leu Ala Met Gly Ile  Thr Val Val Met Lys  Trp Val Cys Val Gly
1105                 1110                 1115                 1120

Lys His Lys Pro Ser  Glu His Pro Leu Phe  Ser Arg Phe Val Trp  Leu

1125                1130                1135

Asn Glu Leu Gln Asp Ala Phe Val Glu Ser Val Ala Gly Pro Trp Phe
        1140                1145                1150

Leu Val Pro Asn Leu Gly Thr Gly Ala Leu Asn Ala Gly Met Ser Ala
    1155                1160                1165

Leu Gly Ala His Ile Gly Arg Gly Ala Trp Ile Glu Ser Tyr Trp Leu
    1170                1175                1180

Pro Glu Thr Asp Leu Cys Tyr Ile Gly Lys Gly Ala Thr Val Gly Pro
1185                1190                1195                1200

Gly Val Val Val Gln Thr His Leu Phe Gln Asp Arg Val Met Ser Leu
                1205                1210                1215

Asp Thr Val Thr Val Ala Asp Gly Ala Thr Leu Ala Asp His Ser Val
            1220                1225                1230

Ala Leu Pro Ala Ser Leu Ile Asp Ala Ser Ala Thr Ile Gly Pro Gly
        1235                1240                1245

Ser Leu Val Met Arg Gly Asp Lys Val Pro Ala His Thr Arg Trp Gln
    1250                1255                1260

Gly Asn Pro Ile Glu Pro Trp Ser Asn Ser
1265                1270


<210> 361
<211> 609
<212> DNA
<213> Corynebacterium glutamicum

<220>
<221> CDS
<222> (101)..(586)
<223> RXN01997

<400> 361
aaaaaggtgg gaaacttagc caatccaaag cccaaaaatg cgggttatgc tgcgctaacc 60

tatgctgaca gccttgcgga agttgtgtac gttaggggcc atg aca atc aac gag   115
                                            Met Thr Ile Asn Glu
                                            1               5

aag atc gca tca gct ttc aac aac caa gtg act gca gag ctt gaa gct   163
Lys Ile Ala Ser Ala Phe Asn Asn Gln Val Thr Ala Glu Leu Glu Ala
                10                  15                  20

tca atg gtg tac ctt cag ctc tcc tac gtt cta gac gat ctg ggc ctc   211
Ser Met Val Tyr Leu Gln Leu Ser Tyr Val Leu Asp Asp Leu Gly Leu
            25                  30                  35

acc ggc atg cgc gac tgg atg aag gca cag agc aaa gaa gag ctc gaa   259
Thr Gly Met Arg Asp Trp Met Lys Ala Gln Ser Lys Glu Glu Leu Glu
        40                  45                  50

cac gca cag aag ttc gct cag cac ctt ctt gac cgt gac tac acc cca   307
His Ala Gln Lys Phe Ala Gln His Leu Leu Asp Arg Asp Tyr Thr Pro
    55                  60                  65

cag atc ggt gac att gca cca cca aag ctt gat gtc acc tcc gct atc   355
Gln Ile Gly Asp Ile Ala Pro Pro Lys Leu Asp Val Thr Ser Ala Ile
70                  75                  80                  85

gag gct ttc gag gct tcc ctg gca cac gag cag aag atc tcc ggc ctg   403
Glu Ala Phe Glu Ala Ser Leu Ala His Glu Gln Lys Ile Ser Gly Leu
                90                  95                  100

atc cgc gag ctc gct gcc atc cag gac gct gag aag gac tac gat tcc   451
Ile Arg Glu Leu Ala Ala Ile Gln Asp Ala Glu Lys Asp Tyr Asp Ser
            105                 110                 115

cgc gca ctg atc gac tgg ttc ctc aac gag cag atc gaa gaa gaa gca   499
Arg Ala Leu Ile Asp Trp Phe Leu Asn Glu Gln Ile Glu Glu Glu Ala

120                 125                 130

acc gtc ggc gag atc atc gac cgc ctc cgt atc gct ggt gat tcc ggt    547
Thr Val Gly Glu Ile Ile Asp Arg Leu Arg Ile Ala Gly Asp Ser Gly
    135                 140                 145

tcc gga atc ctg cgc atc gac ggc gaa ctc ggc tcc cgc taaattcccc    596
Ser Gly Ile Leu Arg Ile Asp Gly Glu Leu Gly Ser Arg
150                 155                 160

gcagttttta atg                                                    609

<210> 362
<211> 162
<212> PRT
<213> Corynebacterium glutamicum

<400> 362
Met Thr Ile Asn Glu Lys Ile Ala Ser Ala Phe Asn Asn Gln Val Thr
  1               5                  10                  15

Ala Glu Leu Glu Ala Ser Met Val Tyr Leu Gln Leu Ser Tyr Val Leu
             20                  25                  30

Asp Asp Leu Gly Leu Thr Gly Met Arg Asp Trp Met Lys Ala Gln Ser
         35                  40                  45

Lys Glu Glu Leu Glu His Ala Gln Lys Phe Ala Gln His Leu Leu Asp
     50                  55                  60

Arg Asp Tyr Thr Pro Gln Ile Gly Asp Ile Ala Pro Pro Lys Leu Asp
 65                  70                  75                  80

Val Thr Ser Ala Ile Glu Ala Phe Glu Ala Ser Leu Ala His Glu Gln
                 85                  90                  95

Lys Ile Ser Gly Leu Ile Arg Glu Leu Ala Ala Ile Gln Asp Ala Glu
            100                 105                 110

Lys Asp Tyr Asp Ser Arg Ala Leu Ile Asp Trp Phe Leu Asn Glu Gln
        115                 120                 125

Ile Glu Glu Glu Ala Thr Val Gly Glu Ile Ile Asp Arg Leu Arg Ile
    130                 135                 140

Ala Gly Asp Ser Gly Ser Gly Ile Leu Arg Ile Asp Gly Glu Leu Gly
145                 150                 155                 160

Ser Arg

<210> 363
<211> 867
<212> DNA
<213> Corynebacterium glutamicum

<220>
<221> CDS
<222> (101)..(844)
<223> RXA01848

<400> 363
ctgcaaggaa ctgcgcaggc gaaggcgcag actactggaa aggtaggtac tgccggatcc 60

ggcgacccct ttcgctccta ggcatttgcg cctggcgtcc atg ggg gag gag gac    115
                                            Met Gly Glu Glu Asp
                                              1               5

tcc acc cca ggt agg cgt tcc aag gcg tat tcg cgc cag ggc gct gat    163
Ser Thr Pro Gly Arg Arg Ser Lys Ala Tyr Ser Arg Gln Gly Ala Asp
                 10                  15                  20

gtc cgc ccc atg aag ggt gga cac ggc atc aac tta gtg ggc acg ctc    211
Val Arg Pro Met Lys Gly Gly His Gly Ile Asn Leu Val Gly Thr Leu
             25                  30                  35

atg gcg gct acg gaa cgc ggc gcc aac att gtt gaa ggc gtg gtc gat    259
Met Ala Ala Thr Glu Arg Gly Ala Asn Ile Val Glu Gly Val Val Asp
        40                  45                  50

ttc cgg ccc acg gac ctg cgg ggt tcg ctg cgc cgt ggg cgc gaa gcc    307
Phe Arg Pro Thr Asp Leu Arg Gly Ser Leu Arg Arg Gly Arg Glu Ala
    55                  60                  65

aac ctc atc gtg ttc gtc gtc gac aca tcg ggg tcg atg gct gcg cgt    355
Asn Leu Ile Val Phe Val Val Asp Thr Ser Gly Ser Met Ala Ala Arg
70                  75                  80                  85

tcc agg gtg cgt gcg gtc acc ggg act att acc tct atg ctt aac gac    403
Ser Arg Val Arg Ala Val Thr Gly Thr Ile Thr Ser Met Leu Asn Asp
                90                  95                  100

gcc tac cag cgc cgc gac aag gtt gcg gtt atc gcg gtc aac ggc aac    451
Ala Tyr Gln Arg Arg Asp Lys Val Ala Val Ile Ala Val Asn Gly Asn
            105                 110                 115

aag ccg aca ctg gtg ttg aat cca aca aat tct gtg gag caa gct cag    499
Lys Pro Thr Leu Val Leu Asn Pro Thr Asn Ser Val Glu Gln Ala Gln
        120                 125                 130

cag aaa tta aag gat atg ccg atg ggt ggt cgc act cca ctg gca gag    547
Gln Lys Leu Lys Asp Met Pro Met Gly Gly Arg Thr Pro Leu Ala Glu
    135                 140                 145

ggg ctg ctc atg gcc aag gat ctc atg gca agg gaa ctc cga aag gaa    595
Gly Leu Leu Met Ala Lys Asp Leu Met Ala Arg Glu Leu Arg Lys Glu
150                 155                 160                 165

ccc ggc cga cgc gcg atc ctc atg gtg atg acc gat ggc caa gac acc    643
Pro Gly Arg Arg Ala Ile Leu Met Val Met Thr Asp Gly Gln Asp Thr
                170                 175                 180

tcc gat gcc ggc gaa gca ggc att gcc acc gcg gcg gaa aca gtg gtg    691
Ser Asp Ala Gly Glu Ala Gly Ile Ala Thr Ala Ala Glu Thr Val Val
            185                 190                 195

aaa tca cga ctg tcc ggc aac gtg gtc atc gac tgc gaa ggc cga ctc    739
Lys Ser Arg Leu Ser Gly Asn Val Val Ile Asp Cys Glu Gly Arg Leu
        200                 205                 210

aaa gtg cgc aaa gag cgc gcc ggg gtg ttg gct gaa atg ctc ggt ggt    787
Lys Val Arg Lys Glu Arg Ala Gly Val Leu Ala Glu Met Leu Gly Gly
    215                 220                 225

gtg tgc gtg aga ttg cgt gat ctt aac tcc gag cac atc aaa atg gtg    835
Val Cys Val Arg Leu Arg Asp Leu Asn Ser Glu His Ile Lys Met Val
230                 235                 240                 245

att aac gcc tagacaacca gagtgagggt ttc                              867
Ile Asn Ala

<210> 364
<211> 248
<212> PRT
<213> Corynebacterium glutamicum

<400> 364
Met Gly Glu Glu Asp Ser Thr Pro Gly Arg Arg Ser Lys Ala Tyr Ser
1               5                   10                  15

Arg Gln Gly Ala Asp Val Arg Pro Met Lys Gly Gly His Gly Ile Asn
            20                  25                  30

Leu Val Gly Thr Leu Met Ala Ala Thr Glu Arg Gly Ala Asn Ile Val
        35                  40                  45

Glu Gly Val Val Asp Phe Arg Pro Thr Asp Leu Arg Gly Ser Leu Arg
    50                  55                  60

Arg Gly Arg Glu Ala Asn Leu Ile Val Phe Val Val Asp Thr Ser Gly

65                  70                  75                  80

Ser Met Ala Ala Arg Ser Arg Val Arg Ala Val Thr Gly Thr Ile Thr
                85                  90                  95

Ser Met Leu Asn Asp Ala Tyr Gln Arg Arg Asp Lys Val Ala Val Ile
            100                 105                 110

Ala Val Asn Gly Asn Lys Pro Thr Leu Val Leu Asn Pro Thr Asn Ser
        115                 120                 125

Val Glu Gln Ala Gln Gln Lys Leu Lys Asp Met Pro Met Gly Gly Arg
    130                 135                 140

Thr Pro Leu Ala Glu Gly Leu Leu Met Ala Lys Asp Leu Met Ala Arg
145                 150                 155                 160

Glu Leu Arg Lys Glu Pro Gly Arg Arg Ala Ile Leu Met Val Met Thr
                165                 170                 175

Asp Gly Gln Asp Thr Ser Asp Ala Gly Glu Ala Gly Ile Ala Thr Ala
            180                 185                 190

Ala Glu Thr Val Val Lys Ser Arg Leu Ser Gly Asn Val Val Ile Asp
        195                 200                 205

Cys Glu Gly Arg Leu Lys Val Arg Lys Glu Arg Ala Gly Val Leu Ala
    210                 215                 220

Glu Met Leu Gly Gly Val Cys Val Arg Leu Arg Asp Leu Asn Ser Glu
225                 230                 235                 240

His Ile Lys Met Val Ile Asn Ala
                245


<210> 365
<211> 1224
<212> DNA
<213> Corynebacterium glutamicum

<220>
<221> CDS
<222> (101)..(1201)
<223> RXN01849

<400> 365
aaaaccttaa gttgggtggt taaacccact aaggtctcac tttatggatg tgccaggtca 60

caccaaaaaa tctcaagaaa actcacatta aaggacagta atg gcg tca caa cag    115
                                          Met Ala Ser Gln Gln
                                          1               5

atc cgc tat cca ttc tcc gcg gtt gtg gga caa gac gag ctt cgg ctt    163
Ile Arg Tyr Pro Phe Ser Ala Val Val Gly Gln Asp Glu Leu Arg Leu
                10                  15                  20

gcg ttg atc ctc act gcg att tcc cca cgc att ggt ggc gtg gtg att    211
Ala Leu Ile Leu Thr Ala Ile Ser Pro Arg Ile Gly Gly Val Val Ile
            25                  30                  35

cga ggt gag aag ggt aca gcg aaa act acc act gtg cgt gct ttt gct    259
Arg Gly Glu Lys Gly Thr Ala Lys Thr Thr Thr Val Arg Ala Phe Ala
        40                  45                  50

ggt ctt tta ggt gat gcc cct ttg gtg aac ttg cct ctc gga tcc acg    307
Gly Leu Leu Gly Asp Ala Pro Leu Val Asn Leu Pro Leu Gly Ser Thr
    55                  60                  65

gag gat cgt gtg gtg ggt tcc ctc aac atg gaa act gtg ttg acc acc    355
Glu Asp Arg Val Val Gly Ser Leu Asn Met Glu Thr Val Leu Thr Thr
70                  75                  80                  85

ggc cgt gcg gaa tat cag cca ggt ttg ctc gcg cag gct gat ggc ggt    403
Gly Arg Ala Glu Tyr Gln Pro Gly Leu Leu Ala Gln Ala Asp Gly Gly
                90                  95                  100

gtg ctg tat gtc gat gag gtc aac ctc ttg gcg gat cac ctg gtg gat     451
Val Leu Tyr Val Asp Glu Val Asn Leu Leu Ala Asp His Leu Val Asp
            105                 110                 115

gct ctg ctc gat gca gct gca agc ggt cgc gtc agc att gag cgt gac     499
Ala Leu Leu Asp Ala Ala Ala Ser Gly Arg Val Ser Ile Glu Arg Asp
        120                 125                 130

ggt att tcg cat tct tca cca gca aac ttt gtg ttg gtg ggc acc atg     547
Gly Ile Ser His Ser Ser Pro Ala Asn Phe Val Leu Val Gly Thr Met
    135                 140                 145

aat ccg gag gaa ggc gag ctg cgc ccg cag ctg ctg gac cgt ttc ggt     595
Asn Pro Glu Glu Gly Glu Leu Arg Pro Gln Leu Leu Asp Arg Phe Gly
150                 155                 160                 165

ttg gct gtg gac gtt gct gcg tct acg aac cct gag gtg cgc gtg gag     643
Leu Ala Val Asp Val Ala Ala Ser Thr Asn Pro Glu Val Arg Val Glu
                170                 175                 180

atc att cgc cgc cgg ctt gat ttt gaa aac gct cct gag cag ttc atg     691
Ile Ile Arg Arg Arg Leu Asp Phe Glu Asn Ala Pro Glu Gln Phe Met
            185                 190                 195

gct aag tgg gct gag caa gat gcg gac acc tcc aac cgt att ttg gcg     739
Ala Lys Trp Ala Glu Gln Asp Ala Asp Thr Ser Asn Arg Ile Leu Ala
        200                 205                 210

gct aag gat ttg ctg cct ggt gtg gag ctg ccg gat ctg atc ttg tcg     787
Ala Lys Asp Leu Leu Pro Gly Val Glu Leu Pro Asp Leu Ile Leu Ser
    215                 220                 225

cag att gcg tgg ttg tgt gca cgt att gaa gtc gac ggt atg cgc gct     835
Gln Ile Ala Trp Leu Cys Ala Arg Ile Glu Val Asp Gly Met Arg Ala
230                 235                 240                 245

gac ctg gtg atc acg cgt acc gca ctt gct cac gcc gcg tgg gct gga     883
Asp Leu Val Ile Thr Arg Thr Ala Leu Ala His Ala Ala Trp Ala Gly
                250                 255                 260

cgc act gtg gtt acg gaa gaa gac gtg gag atc gca gct cgc cta gcg     931
Arg Thr Val Val Thr Glu Glu Asp Val Glu Ile Ala Ala Arg Leu Ala
            265                 270                 275

ttg ccg cac cgc cgt cgc cgt aat cct ttc gat gct cca gaa atg gag     979
Leu Pro His Arg Arg Arg Arg Asn Pro Phe Asp Ala Pro Glu Met Glu
        280                 285                 290

gag cgc aag ctt cag gaa acc ctg cag gaa gct cgg gac ttc ttc aaa     1027
Glu Arg Lys Leu Gln Glu Thr Leu Gln Glu Ala Arg Asp Phe Phe Lys
    295                 300                 305

gac aat gaa gat aaa gga cct gcc gcc aag atc acc gat gag gaa acc     1075
Asp Asn Glu Asp Lys Gly Pro Ala Ala Lys Ile Thr Asp Glu Glu Thr
310                 315                 320                 325

ggt gca gag gcc ttt acc gat acc gac aat ccc acc gag gaa gac ggt     1123
Gly Ala Glu Ala Phe Thr Asp Thr Asp Asn Pro Thr Glu Glu Asp Gly
                330                 335                 340

ctg caa gga act gcg cag gcg aag gcg cag act act gga aag gta ggt     1171
Leu Gln Gly Thr Ala Gln Ala Lys Ala Gln Thr Thr Gly Lys Val Gly
            345                 350                 355

act gcc gga tcc ggc gac ccc ttt cgc tcc taggcatttg cgcctggcgt     1221
Thr Ala Gly Ser Gly Asp Pro Phe Arg Ser
        360                 365

cca                                                                 1224

<210> 366
<211> 367
<212> PRT
<213> Corynebacterium glutamicum

<400> 366

Met Ala Ser Gln Gln Ile Arg Tyr Pro Phe Ser Ala Val Val Gly Gln
1               5                   10                  15

Asp Glu Leu Arg Leu Ala Leu Ile Leu Thr Ala Ile Ser Pro Arg Ile
            20                  25                  30

Gly Gly Val Val Ile Arg Gly Glu Lys Gly Thr Ala Lys Thr Thr Thr
        35                  40                  45

Val Arg Ala Phe Ala Gly Leu Leu Gly Asp Ala Pro Leu Val Asn Leu
    50                  55                  60

Pro Leu Gly Ser Thr Glu Asp Arg Val Val Gly Ser Leu Asn Met Glu
65                  70                  75                  80

Thr Val Leu Thr Thr Gly Arg Ala Glu Tyr Gln Pro Gly Leu Leu Ala
                85                  90                  95

Gln Ala Asp Gly Gly Val Leu Tyr Val Asp Glu Val Asn Leu Leu Ala
            100                 105                 110

Asp His Leu Val Asp Ala Leu Leu Asp Ala Ala Ala Ser Gly Arg Val
        115                 120                 125

Ser Ile Glu Arg Asp Gly Ile Ser His Ser Ser Pro Ala Asn Phe Val
    130                 135                 140

Leu Val Gly Thr Met Asn Pro Glu Glu Gly Glu Leu Arg Pro Gln Leu
145                 150                 155                 160

Leu Asp Arg Phe Gly Leu Ala Val Asp Val Ala Ala Ser Thr Asn Pro
                165                 170                 175

Glu Val Arg Val Glu Ile Ile Arg Arg Arg Leu Asp Phe Glu Asn Ala
            180                 185                 190

Pro Glu Gln Phe Met Ala Lys Trp Ala Glu Gln Asp Ala Asp Thr Ser
        195                 200                 205

Asn Arg Ile Leu Ala Ala Lys Asp Leu Leu Pro Gly Val Glu Leu Pro
    210                 215                 220

Asp Leu Ile Leu Ser Gln Ile Ala Trp Leu Cys Ala Arg Ile Glu Val
225                 230                 235                 240

Asp Gly Met Arg Ala Asp Leu Val Ile Thr Arg Thr Ala Leu Ala His
                245                 250                 255

Ala Ala Trp Ala Gly Arg Thr Val Val Thr Glu Glu Asp Val Glu Ile
            260                 265                 270

Ala Ala Arg Leu Ala Leu Pro His Arg Arg Arg Arg Asn Pro Phe Asp
        275                 280                 285

Ala Pro Glu Met Glu Glu Arg Lys Leu Gln Glu Thr Leu Gln Glu Ala
    290                 295                 300

Arg Asp Phe Phe Lys Asp Asn Glu Asp Lys Gly Pro Ala Ala Lys Ile
305                 310                 315                 320

Thr Asp Glu Glu Thr Gly Ala Glu Ala Phe Thr Asp Thr Asp Asn Pro
                325                 330                 335

Thr Glu Glu Asp Gly Leu Gln Gly Thr Ala Gln Ala Lys Ala Gln Thr
            340                 345                 350

Thr Gly Lys Val Gly Thr Ala Gly Ser Gly Asp Pro Phe Arg Ser
        355                 360                 365

<210> 367
<211> 473
<212> DNA
<213> Corynebacterium glutamicum

<220>
<221> CDS

<222> (1)..(450)
<223> FRXA01849

<400> 367
ctg cct ggt gtg gag ctg ccg gat ctg atc ttg tcg cag att gcg tgg     48
Leu Pro Gly Val Glu Leu Pro Asp Leu Ile Leu Ser Gln Ile Ala Trp
1               5                   10                  15

ttg tgt gca cgt att gaa gtc gac ggt atg cgc gct gac ctg gtg atc     96
Leu Cys Ala Arg Ile Glu Val Asp Gly Met Arg Ala Asp Leu Val Ile
            20                  25                  30

acg cgt acc gca ctt gct cac gcc gcg tgg gct gga cgc act gtg gtt     144
Thr Arg Thr Ala Leu Ala His Ala Ala Trp Ala Gly Arg Thr Val Val
        35                  40                  45

acg gaa gaa gac gtg gag atc gca gct cgc cta gcg ttg ccg cac cgc     192
Thr Glu Glu Asp Val Glu Ile Ala Ala Arg Leu Ala Leu Pro His Arg
    50                  55                  60

cgt cgc cgt aat cct ttc gat gct cca gaa atg gag gag cgc aag ctt     240
Arg Arg Arg Asn Pro Phe Asp Ala Pro Glu Met Glu Glu Arg Lys Leu
65                  70                  75                  80

cag gaa acc ctg cag gaa gct cgg gac ttc ttc aaa gac aat gaa gat     288
Gln Glu Thr Leu Gln Glu Ala Arg Asp Phe Phe Lys Asp Asn Glu Asp
                85                  90                  95

aaa gga cct gcc gcc aag atc acc gat gag gaa acc ggt gca gag gcc     336
Lys Gly Pro Ala Ala Lys Ile Thr Asp Glu Glu Thr Gly Ala Glu Ala
            100                 105                 110

ttt acc gat acc gac aat ccc acc gag gaa gac ggt ctg caa gga act     384
Phe Thr Asp Thr Asp Asn Pro Thr Glu Glu Asp Gly Leu Gln Gly Thr
        115                 120                 125

gcg cag gcg aag gcg cag act act gga aag gta ggt act gcc gga tcc     432
Ala Gln Ala Lys Ala Gln Thr Thr Gly Lys Val Gly Thr Ala Gly Ser
    130                 135                 140

ggc gac ccc ttt cgc tcc taggcatttg cgcctggcgt cca                   473
Gly Asp Pro Phe Arg Ser
145                 150


<210> 368
<211> 150
<212> PRT
<213> Corynebacterium glutamicum

<400> 368
Leu Pro Gly Val Glu Leu Pro Asp Leu Ile Leu Ser Gln Ile Ala Trp
1               5                   10                  15

Leu Cys Ala Arg Ile Glu Val Asp Gly Met Arg Ala Asp Leu Val Ile
            20                  25                  30

Thr Arg Thr Ala Leu Ala His Ala Ala Trp Ala Gly Arg Thr Val Val
        35                  40                  45

Thr Glu Glu Asp Val Glu Ile Ala Ala Arg Leu Ala Leu Pro His Arg
    50                  55                  60

Arg Arg Arg Asn Pro Phe Asp Ala Pro Glu Met Glu Glu Arg Lys Leu
65                  70                  75                  80

Gln Glu Thr Leu Gln Glu Ala Arg Asp Phe Phe Lys Asp Asn Glu Asp
                85                  90                  95

Lys Gly Pro Ala Ala Lys Ile Thr Asp Glu Glu Thr Gly Ala Glu Ala
            100                 105                 110

Phe Thr Asp Thr Asp Asn Pro Thr Glu Glu Asp Gly Leu Gln Gly Thr
        115                 120                 125

Ala Gln Ala Lys Ala Gln Thr Thr Gly Lys Val Gly Thr Ala Gly Ser
    130                 135                 140

Gly Asp Pro Phe Arg Ser
145                 150


<210> 369
<211> 667
<212> DNA
<213> Corynebacterium glutamicum

<220>
<221> CDS
<222> (101)..(667)
<223> FRXA01691

<400> 369
aaaaccttaa gttgggtggt taaacccact aaggtctcac tttatggatg tgccaggtca 60

caccaaaaaa tctcaagaaa actcacatta aaggacagta atg gcg tca caa cag   115
                                           Met Ala Ser Gln Gln
                                           1               5

atc cgc tat cca ttc tcc gcg gtt gtg gga caa gac gag ctt cgg ctt   163
Ile Arg Tyr Pro Phe Ser Ala Val Val Gly Gln Asp Glu Leu Arg Leu
                10                  15                  20

gcg ttg atc ctc act gcg att tcc cca cgc att ggt ggc gtg gtg att   211
Ala Leu Ile Leu Thr Ala Ile Ser Pro Arg Ile Gly Gly Val Val Ile
            25                  30                  35

cga ggt gag aag ggt aca gcg aaa act acc act gtg cgt gct ttt gct   259
Arg Gly Glu Lys Gly Thr Ala Lys Thr Thr Thr Val Arg Ala Phe Ala
        40                  45                  50

ggt ctt tta ggt gat gcc cct ttg gtg aac ttg cct ctc gga tcc acg   307
Gly Leu Leu Gly Asp Ala Pro Leu Val Asn Leu Pro Leu Gly Ser Thr
    55                  60                  65

gag gat cgt gtg gtg ggt tcc ctc aac atg gaa act gtg ttg acc acc   355
Glu Asp Arg Val Val Gly Ser Leu Asn Met Glu Thr Val Leu Thr Thr
70                  75                  80                  85

ggc cgt gcg gaa tat cag cca ggt ttg ctc gcg cag gct gat ggc ggt   403
Gly Arg Ala Glu Tyr Gln Pro Gly Leu Leu Ala Gln Ala Asp Gly Gly
                90                  95                  100

gtg ctg tat gtc gat gag gtc aac ctc ttg gcg gat cac ctg gtg gat   451
Val Leu Tyr Val Asp Glu Val Asn Leu Leu Ala Asp His Leu Val Asp
            105                 110                 115

gct ctg ctc gat gca gct gca agc ggt cgc gtc agc att gag cgt gac   499
Ala Leu Leu Asp Ala Ala Ala Ser Gly Arg Val Ser Ile Glu Arg Asp
        120                 125                 130

ggt att tcg cat tct tca cca gca aac ttt gtg ttg gtg ggc acc atg   547
Gly Ile Ser His Ser Ser Pro Ala Asn Phe Val Leu Val Gly Thr Met
    135                 140                 145

aat ccg gag gaa ggc gag ctg cgc ccg cag ctg ctg gac cgt ttc ggt   595
Asn Pro Glu Glu Gly Glu Leu Arg Pro Gln Leu Leu Asp Arg Phe Gly
150                 155                 160                 165

ttg gct gtg gac gtt gct gcg tct acg aac cct gag gtg cgc gtg gag   643
Leu Ala Val Asp Val Ala Ala Ser Thr Asn Pro Glu Val Arg Val Glu
                170                 175                 180

atc att cgc cgc cgg ctt gat ttt                                   667
Ile Ile Arg Arg Arg Leu Asp Phe
            185


<210> 370
<211> 189
<212> PRT
<213> Corynebacterium glutamicum

<400> 370

Met Ala Ser Gln Gln Ile Arg Tyr Pro Phe Ser Ala Val Val Gly Gln
1               5                   10                  15

Asp Glu Leu Arg Leu Ala Leu Ile Leu Thr Ala Ile Ser Pro Arg Ile
            20                  25                  30

Gly Gly Val Val Ile Arg Gly Glu Lys Gly Thr Ala Lys Thr Thr Thr
        35                  40                  45

Val Arg Ala Phe Ala Gly Leu Leu Gly Asp Ala Pro Leu Val Asn Leu
    50                  55                  60

Pro Leu Gly Ser Thr Glu Asp Arg Val Val Gly Ser Leu Asn Met Glu
65                  70                  75                  80

Thr Val Leu Thr Thr Gly Arg Ala Glu Tyr Gln Pro Gly Leu Leu Ala
                85                  90                  95

Gln Ala Asp Gly Gly Val Leu Tyr Val Asp Glu Val Asn Leu Leu Ala
            100                 105                 110

Asp His Leu Val Asp Ala Leu Leu Asp Ala Ala Ala Ser Gly Arg Val
        115                 120                 125

Ser Ile Glu Arg Asp Gly Ile Ser His Ser Ser Pro Ala Asn Phe Val
    130                 135                 140

Leu Val Gly Thr Met Asn Pro Glu Glu Gly Glu Leu Arg Pro Gln Leu
145                 150                 155                 160

Leu Asp Arg Phe Gly Leu Ala Val Asp Val Ala Ala Ser Thr Asn Pro
                165                 170                 175

Glu Val Arg Val Glu Ile Ile Arg Arg Arg Leu Asp Phe
            180                 185


<210> 371
<211> 601
<212> DNA
<213> Corynebacterium glutamicum

<220>
<221> CDS
<222> (101)..(601)
<223> RXN00665

<400> 371
accaaacact tctgtgcgtg acacgcgcca ccttatactc ccacaagcaa cacagaacac 60

tcgggatctc aaagtttcga gaaacacaga aagggcagca atg agc agc tca aca   115
                                           Met Ser Ser Ser Thr
                                           1               5

ctt ctc ctg gct tca gga caa gtc acg gca tta gcc gct gac tac acg   163
Leu Leu Leu Ala Ser Gly Gln Val Thr Ala Leu Ala Ala Asp Tyr Thr
                10                  15                  20

ctc agc cac acc ccc tca gat ggc atc ctg gta gtc ctt ggc ttc gcc   211
Leu Ser His Thr Pro Ser Asp Gly Ile Leu Val Val Leu Gly Phe Ala
            25                  30                  35

atg atc ctc acc ttc atg acc ctg atc atg ctg ggt cga ctc acc cca   259
Met Ile Leu Thr Phe Met Thr Leu Ile Met Leu Gly Arg Leu Thr Pro
        40                  45                  50

atg gtg gcc atg ctg ttg gtc ccc acc atc ttc ggt ctc atc gcc ggc   307
Met Val Ala Met Leu Leu Val Pro Thr Ile Phe Gly Leu Ile Ala Gly
    55                  60                  65

gca gga ctc ggc ctt ggt gac atg gcg ctt gac gcc atc aag gac atg   355
Ala Gly Leu Gly Leu Gly Asp Met Ala Leu Asp Ala Ile Lys Asp Met
70                  75                  80                  85

gcg cct acc gcg gca ctc ctg atg ttc gcg att atg ttc ttc gga atc   403
Ala Pro Thr Ala Ala Leu Leu Met Phe Ala Ile Met Phe Phe Gly Ile
                90                  95                  100

atg atc gac gtc gga ctc ttc gac ccc ctg atc cgc gtg atc acc cgc    451
Met Ile Asp Val Gly Leu Phe Asp Pro Leu Ile Arg Val Ile Thr Arg
            105                 110                 115

gtt ctt cac gat gac ccc gca aag gtc gtc atc ggc acc gca gta ctt    499
Val Leu His Asp Asp Pro Ala Lys Val Val Ile Gly Thr Ala Val Leu
        120                 125                 130

gca ggt gtt gtc tcc ctc gac ggc gac ggc tcc acc acc ttc atc att    547
Ala Gly Val Val Ser Leu Asp Gly Asp Gly Ser Thr Thr Phe Ile Ile
    135                 140                 145

acc acc ttc cgc gat gct gcc cat cta cct gcg cct tgg cat gag ccc    595
Thr Thr Phe Arg Asp Ala Ala His Leu Pro Ala Pro Trp His Glu Pro
150                 155                 160                 165

tgt ggt                                                            601
Cys Gly

<210> 372
<211> 167
<212> PRT
<213> Corynebacterium glutamicum

<400> 372
Met Ser Ser Ser Thr Leu Leu Leu Ala Ser Gly Gln Val Thr Ala Leu
  1               5                  10                  15

Ala Ala Asp Tyr Thr Leu Ser His Thr Pro Ser Asp Gly Ile Leu Val
             20                  25                  30

Val Leu Gly Phe Ala Met Ile Leu Thr Phe Met Thr Leu Ile Met Leu
         35                  40                  45

Gly Arg Leu Thr Pro Met Val Ala Met Leu Leu Val Pro Thr Ile Phe
     50                  55                  60

Gly Leu Ile Ala Gly Ala Gly Leu Gly Leu Gly Asp Met Ala Leu Asp
 65                  70                  75                  80

Ala Ile Lys Asp Met Ala Pro Thr Ala Ala Leu Leu Met Phe Ala Ile
                 85                  90                  95

Met Phe Phe Gly Ile Met Ile Asp Val Gly Leu Phe Asp Pro Leu Ile
            100                 105                 110

Arg Val Ile Thr Arg Val Leu His Asp Asp Pro Ala Lys Val Val Ile
        115                 120                 125

Gly Thr Ala Val Leu Ala Gly Val Val Ser Leu Asp Gly Asp Gly Ser
    130                 135                 140

Thr Thr Phe Ile Ile Thr Thr Phe Arg Asp Ala Ala His Leu Pro Ala
145                 150                 155                 160

Pro Trp His Glu Pro Cys Gly
                165

<210> 373
<211> 390
<212> DNA
<213> Corynebacterium glutamicum

<220>
<221> CDS
<222> (101)..(367)
<223> RXN03026

<400> 373
gttggcggcg cagtatcgtg aggtgcggga cctcgagcgg ggaatcccaa actagcatcc 60

cgaactagcc ccccaacaac aattagaaat ggaacctaaa atg cct gga aaa att    115
                                            Met Pro Gly Lys Ile

                                                1               5

ctc ctt ctc aac ggc cca aac ctg aac atg ctg ggc aaa cgc gag cct    163
Leu Leu Leu Asn Gly Pro Asn Leu Asn Met Leu Gly Lys Arg Glu Pro
                10                  15                  20

gac att tac gga cac gac acc ttg gaa gac gtc gtc gcg ctg gca acc    211
Asp Ile Tyr Gly His Asp Thr Leu Glu Asp Val Val Ala Leu Ala Thr
            25                  30                  35

gct gag gct gcg aaa cac ggc ctt gag gtt gag gcg ctg cag agc aat    259
Ala Glu Ala Ala Lys His Gly Leu Glu Val Glu Ala Leu Gln Ser Asn
        40                  45                  50

cac caa ggt gag cta atc gat gcg ctg cac aac gct cgc ggg acc cac    307
His Gln Gly Glu Leu Ile Asp Ala Leu His Asn Ala Arg Gly Thr His
    55                  60                  65

atc ggt tgc gtg att aac ccc ggc ggc ctg act aca ctt cgg tgg cgc    355
Ile Gly Cys Val Ile Asn Pro Gly Gly Leu Thr Thr Leu Arg Trp Arg
70                  75                  80                  85

ttt tgg atg ctg tgaaggcgtc tgagcttcct acc                          390
Phe Trp Met Leu

<210> 374
<211> 89
<212> PRT
<213> Corynebacterium glutamicum

<400> 374
Met Pro Gly Lys Ile Leu Leu Leu Asn Gly Pro Asn Leu Asn Met Leu
1               5                   10                  15

Gly Lys Arg Glu Pro Asp Ile Tyr Gly His Asp Thr Leu Glu Asp Val
            20                  25                  30

Val Ala Leu Ala Thr Ala Glu Ala Ala Lys His Gly Leu Glu Val Glu
        35                  40                  45

Ala Leu Gln Ser Asn His Gln Gly Glu Leu Ile Asp Ala Leu His Asn
    50                  55                  60

Ala Arg Gly Thr His Ile Gly Cys Val Ile Asn Pro Gly Gly Leu Thr
65                  70                  75                  80

Thr Leu Arg Trp Arg Phe Trp Met Leu
                85

<210> 375
<211> 384
<212> DNA
<213> Corynebacterium glutamicum

<220>
<221> CDS
<222> (101)..(361)
<223> RXN02908

<400> 375
gccaacgagg gttggtttac cacctctgat tcaggtgaac tccacgacgg gattctcacc 60

gtgactggtc gcgtggatac ccgtcattga ttccggtgga ttg aag ttg cac cca    115
                                           Leu Lys Leu His Pro
                                           1               5

gag gta ctg gaa cgt gcc atc gca gat att aaa ggt gtc acc gcg gcg    163
Glu Val Leu Glu Arg Ala Ile Ala Asp Ile Lys Gly Val Thr Ala Ala
                10                  15                  20

tgt gtt gtg ggt att ccc gat ccc cga tta ggc caa gca att gtg gcc    211
Cys Val Val Gly Ile Pro Asp Pro Arg Leu Gly Gln Ala Ile Val Ala
            25                  30                  35

gcg tac tcc gga tcg atc agt ccg tct gaa gtt att gaa ggc ctc gac    259
Ala Tyr Ser Gly Ser Ile Ser Pro Ser Glu Val Ile Glu Gly Leu Asp
        40                  45                  50

gat cta cct cgt tgg cag ctt ccc aaa cgg ctg aag cat ctg gaa tct    307
Asp Leu Pro Arg Trp Gln Leu Pro Lys Arg Leu Lys His Leu Glu Ser
    55                  60                  65

ttg ccc agc att ggt cct gga aaa gct gat cga cgt gct atc gcg aag    355
Leu Pro Ser Ile Gly Pro Gly Lys Ala Asp Arg Arg Ala Ile Ala Lys
70                  75                  80                  85

ctg ttt tagtcttcat tcttgctggc tgc                                  384
Leu Phe

<210> 376
<211> 87
<212> PRT
<213> Corynebacterium glutamicum

<400> 376
Leu Lys Leu His Pro Glu Val Leu Glu Arg Ala Ile Ala Asp Ile Lys
1               5                   10                  15

Gly Val Thr Ala Ala Cys Val Val Gly Ile Pro Asp Pro Arg Leu Gly
            20                  25                  30

Gln Ala Ile Val Ala Ala Tyr Ser Gly Ser Ile Ser Pro Ser Glu Val
        35                  40                  45

Ile Glu Gly Leu Asp Asp Leu Pro Arg Trp Gln Leu Pro Lys Arg Leu
    50                  55                  60

Lys His Leu Glu Ser Leu Pro Ser Ile Gly Pro Gly Lys Ala Asp Arg
65                  70                  75                  80

Arg Ala Ile Ala Lys Leu Phe
                85

<210> 377
<211> 667
<212> DNA
<213> Corynebacterium glutamicum

<220>
<221> CDS
<222> (101)..(667)
<223> RXN03000

<400> 377
cagcgtgttg ctgcgcctgc agaaaaccta cagcgtcacc cacaaagtcg aaatccaagg 60

ctaacccccct ttttcaactc acagttagga aacttcaccc atg tct ctt cca cat    115
                                            Met Ser Leu Pro His
                                            1               5

tct gat gaa ctc cgc ggc caa aag atc att att tcc ggt ggc gga att    163
Ser Asp Glu Leu Arg Gly Gln Lys Ile Ile Ile Ser Gly Gly Gly Ile
                10                  15                  20

ggt ggc gca gca ggt gca ctt gcg ctt gct ttg cgc ggt gcc gat gtc    211
Gly Gly Ala Ala Gly Ala Leu Ala Leu Ala Leu Arg Gly Ala Asp Val
            25                  30                  35

act ttg tac gaa cgc gca gct gag ttc aag gag gtc ggc gct ggc ctc    259
Thr Leu Tyr Glu Arg Ala Ala Glu Phe Lys Glu Val Gly Ala Gly Leu
        40                  45                  50

cag atc ggt ccg cac ggc tgg cga atg ctg gaa tcc tgg ggt ctg ctc    307
Gln Ile Gly Pro His Gly Trp Arg Met Leu Glu Ser Trp Gly Leu Leu
    55                  60                  65

gac caa att gtc gtg gcc ggc tac ctc cca gaa gac atg cag ttc cgc    355
Asp Gln Ile Val Val Ala Gly Tyr Leu Pro Glu Asp Met Gln Phe Arg

70                  75                  80                  85

gac gct gtc aac cgc gaa acc atc ctg acc atg cgt ttc gat gaa gaa     403
Asp Ala Val Asn Arg Glu Thr Ile Leu Thr Met Arg Phe Asp Glu Glu
                90                  95                  100

ttc cag cag cac tac ggc ggt cgc tac ctg gtg att cac cgc tct gac     451
Phe Gln Gln His Tyr Gly Gly Arg Tyr Leu Val Ile His Arg Ser Asp
            105                 110                 115

ctg ctc aac atc ctg gtc acc aac gcc gaa gca gcg ggc gcg aag ctc     499
Leu Leu Asn Ile Leu Val Thr Asn Ala Glu Ala Ala Gly Ala Lys Leu
        120                 125                 130

cac aat ggc gtc ctg gtc acc gat tcc cgc acc gtc gac ggc ggt atc     547
His Asn Gly Val Leu Val Thr Asp Ser Arg Thr Val Asp Gly Gly Ile
    135                 140                 145

gag gtg gac atc gaa tcc tcc atc aac aag ggc gaa gat aac aag act     595
Glu Val Asp Ile Glu Ser Ser Ile Asn Lys Gly Glu Asp Asn Lys Thr
150                 155                 160                 165

ttg ctt gtc gac gcc ttc ctc gcc ttc gac ggc atc cac tcg gtc atg     643
Leu Leu Val Asp Ala Phe Leu Ala Phe Asp Gly Ile His Ser Val Met
                170                 175                 180

cgc aaa aag ctt gtc gac gac gcc                                     667
Arg Lys Lys Leu Val Asp Asp Ala
            185

<210> 378
<211> 189
<212> PRT
<213> Corynebacterium glutamicum

<400> 378
Met Ser Leu Pro His Ser Asp Glu Leu Arg Gly Gln Lys Ile Ile Ile
1               5                   10                  15

Ser Gly Gly Gly Ile Gly Gly Ala Ala Gly Ala Leu Ala Leu Ala Leu
            20                  25                  30

Arg Gly Ala Asp Val Thr Leu Tyr Glu Arg Ala Ala Glu Phe Lys Glu
        35                  40                  45

Val Gly Ala Gly Leu Gln Ile Gly Pro His Gly Trp Arg Met Leu Glu
    50                  55                  60

Ser Trp Gly Leu Leu Asp Gln Ile Val Val Ala Gly Tyr Leu Pro Glu
65                  70                  75                  80

Asp Met Gln Phe Arg Asp Ala Val Asn Arg Glu Thr Ile Leu Thr Met
                85                  90                  95

Arg Phe Asp Glu Glu Phe Gln Gln His Tyr Gly Gly Arg Tyr Leu Val
            100                 105                 110

Ile His Arg Ser Asp Leu Leu Asn Ile Leu Val Thr Asn Ala Glu Ala
        115                 120                 125

Ala Gly Ala Lys Leu His Asn Gly Val Leu Val Thr Asp Ser Arg Thr
    130                 135                 140

Val Asp Gly Gly Ile Glu Val Asp Ile Glu Ser Ser Ile Asn Lys Gly
145                 150                 155                 160

Glu Asp Asn Lys Thr Leu Leu Val Asp Ala Phe Leu Ala Phe Asp Gly
                165                 170                 175

Ile His Ser Val Met Arg Lys Lys Leu Val Asp Asp Ala
            180                 185

<210> 379
<211> 766
<212> DNA

<213> Corynebacterium glutamicum

<220>
<221> CDS
<222> (101)..(766)
<223> RXN03036

<400> 379
tagaaaaatc tacccagtaa gcattcagga accattcaga atcttttctt agcatgtctc 60

tatcagcgta aacgtccgaa catgaaaggc tagaaaagcc atg gct gag cag ttg 115
Met Ala Glu Gln Leu
1               5

cgt caa ttt gaa ggc agg gtc ctc cct aat caa tcc gag gac ttg gaa 163
Arg Gln Phe Glu Gly Arg Val Leu Pro Asn Gln Ser Glu Asp Leu Glu
                10                  15                  20

gat cag ggt ttg gga ttt gac ctg gga acc gtt ttc tcc cgc agg aag 211
Asp Gln Gly Leu Gly Phe Asp Leu Gly Thr Val Phe Ser Arg Arg Lys
            25                  30                  35

gtt ttg gga ttc atc ggt gtt ggt gga gca ggt gtg gca ctt gct gct 259
Val Leu Gly Phe Ile Gly Val Gly Gly Ala Gly Val Ala Leu Ala Ala
        40                  45                  50

tgt tca cct tct ggt tct tcc gcg gca tcg agc acc tca agc gcg tcc 307
Cys Ser Pro Ser Gly Ser Ser Ala Ala Ser Ser Thr Ser Ser Ala Ser
    55                  60                  65

agc agc gca gct gca acc acc agt gca gca gca gag act ttg act gag 355
Ser Ser Ala Ala Ala Thr Thr Ser Ala Ala Ala Glu Thr Leu Thr Glu
70                  75                  80                  85

atg aag tcg gag act gct ggt ccg tac ccg ggc gat ggt tcg aat ggt 403
Met Lys Ser Glu Thr Ala Gly Pro Tyr Pro Gly Asp Gly Ser Asn Gly
                90                  95                  100

ccg gat gtg ttg gag gtc tcc ggt gtg gag cgc cag gac atc acc aag 451
Pro Asp Val Leu Glu Val Ser Gly Val Glu Arg Gln Asp Ile Thr Lys
            105                 110                 115

tcg att gat tct gac acc gtg gca gag ggc gta cct ctg acg ttg act 499
Ser Ile Asp Ser Asp Thr Val Ala Glu Gly Val Pro Leu Thr Leu Thr
        120                 125                 130

atg acc att ttg gac atg aac aac aac aat cag cca atg gag ggt gct 547
Met Thr Ile Leu Asp Met Asn Asn Asn Asn Gln Pro Met Glu Gly Ala
    135                 140                 145

gcg gtg tac gtg tgg cac tgt gat gcg ccg ggt cga tat tcg atg tac 595
Ala Val Tyr Val Trp His Cys Asp Ala Pro Gly Arg Tyr Ser Met Tyr
150                 155                 160                 165

gac tct gag ctg gaa gat gag acc tat tta cgc ggt gtg cag att acc 643
Asp Ser Glu Leu Glu Asp Glu Thr Tyr Leu Arg Gly Val Gln Ile Thr
                170                 175                 180

gat aag tat ggc cag gtc acg ttc gat acc att ttc cct ggt tgt tat 691
Asp Lys Tyr Gly Gln Val Thr Phe Asp Thr Ile Phe Pro Gly Cys Tyr
            185                 190                 195

gcg ggc cgt tgg gtg cat att cat ttc gag gtg ttc ccg gat cga gac 739
Ala Gly Arg Trp Val His Ile His Phe Glu Val Phe Pro Asp Arg Asp
        200                 205                 210

agc atc acg gat tcc acg aac aac att 766
Ser Ile Thr Asp Ser Thr Asn Asn Ile
    215                 220

<210> 380
<211> 222
<212> PRT
<213> Corynebacterium glutamicum

<400> 380

Met Ala Glu Gln Leu Arg Gln Phe Glu Gly Arg Val Leu Pro Asn Gln
1               5                   10                  15

Ser Glu Asp Leu Glu Asp Gln Gly Leu Gly Phe Asp Leu Gly Thr Val
            20                  25                  30

Phe Ser Arg Arg Lys Val Leu Gly Phe Ile Gly Val Gly Gly Ala Gly
        35                  40                  45

Val Ala Leu Ala Ala Cys Ser Pro Ser Gly Ser Ser Ala Ala Ser Ser
    50                  55                  60

Thr Ser Ser Ala Ser Ser Ser Ala Ala Ala Thr Thr Ser Ala Ala Ala
65                  70                  75                  80

Glu Thr Leu Thr Glu Met Lys Ser Glu Thr Ala Gly Pro Tyr Pro Gly
                85                  90                  95

Asp Gly Ser Asn Gly Pro Asp Val Leu Glu Val Ser Gly Val Glu Arg
            100                 105                 110

Gln Asp Ile Thr Lys Ser Ile Asp Ser Asp Thr Val Ala Glu Gly Val
        115                 120                 125

Pro Leu Thr Leu Thr Met Thr Ile Leu Asp Met Asn Asn Asn Asn Gln
    130                 135                 140

Pro Met Glu Gly Ala Ala Val Tyr Val Trp His Cys Asp Ala Pro Gly
145                 150                 155                 160

Arg Tyr Ser Met Tyr Asp Ser Glu Leu Glu Asp Glu Thr Tyr Leu Arg
                165                 170                 175

Gly Val Gln Ile Thr Asp Lys Tyr Gly Gln Val Thr Phe Asp Thr Ile
            180                 185                 190

Phe Pro Gly Cys Tyr Ala Gly Arg Trp Val His Ile His Phe Glu Val
        195                 200                 205

Phe Pro Asp Arg Asp Ser Ile Thr Asp Ser Thr Asn Asn Ile
    210                 215                 220


<210> 381
<211> 318
<212> DNA
<213> Corynebacterium glutamicum

<220>
<221> CDS
<222> (101)..(295)
<223> RXN02974

<400> 381
gcaggcatgg acacattcca ggtcctgacc ggcgtcagcg gctactacga tttggtgcgc 60

gccattccca gagcagcgcc ccacctatat cgccacctcg atg cag gat ctc tac    115
                                          Met Gln Asp Leu Tyr
                                          1               5

agc gat ccg ggc gag ctc aag cca ggt gcc cag ggc ggt ttt tca gcg    163
Ser Asp Pro Gly Glu Leu Lys Pro Gly Ala Gln Gly Gly Phe Ser Ala
                10                  15                  20

ctt atc gac ggc gac acc ctg gtc att tcc ggc ggc gat gcc ggc gca    211
Leu Ile Asp Gly Asp Thr Leu Val Ile Ser Gly Gly Asp Ala Gly Ala
            25                  30                  35

act ccg gtt gca gca ctc cgc act gcg ttg gat gtg gcc tgg gcg gcc    259
Thr Pro Val Ala Ala Leu Arg Thr Ala Leu Asp Val Ala Trp Ala Ala
        40                  45                  50

aca gag cag tca ccg agg tac gcg ctg att cag agg tagctgctac         305
Thr Glu Gln Ser Pro Arg Tyr Ala Leu Ile Gln Arg
    55                  60                  65

tgcattgcag agc                                                     318

<210> 382
<211> 65
<212> PRT
<213> Corynebacterium glutamicum

<400> 382
Met Gln Asp Leu Tyr Ser Asp Pro Gly Glu Leu Lys Pro Gly Ala Gln
1               5                   10                  15

Gly Gly Phe Ser Ala Leu Ile Asp Gly Asp Thr Leu Val Ile Ser Gly
            20                  25                  30

Gly Asp Ala Gly Ala Thr Pro Val Ala Ala Leu Arg Thr Ala Leu Asp
        35                  40                  45

Val Ala Trp Ala Ala Thr Glu Gln Ser Pro Arg Tyr Ala Leu Ile Gln
    50                  55                  60

Arg
65


<210> 383
<211> 1017
<212> DNA
<213> Corynebacterium glutamicum

<220>
<221> CDS
<222> (101)..(994)
<223> RXN00393

<400> 383
tctattcatt tcacaatagc gtttcacact cccccatagc ctgccgaacg tatttcaagc 60

aattgcgcga tcgagtatgt gatggggaaa gatagaggtt atg tct cac acg gaa   115
                                          Met Ser His Thr Glu
                                          1               5

ccc cag ccg aat tct gta act ttg tcc gat tgg att caa ggc gca cgc   163
Pro Gln Pro Asn Ser Val Thr Leu Ser Asp Trp Ile Gln Gly Ala Arg
                10                  15                  20

ccg cgt acc tgg gca aat gcg ttc gcg cct gtc att gcc ggt tca ggt   211
Pro Arg Thr Trp Ala Asn Ala Phe Ala Pro Val Ile Ala Gly Ser Gly
            25                  30                  35

gtc gcc gct ttt cat gat ggt ttt gtg tgg tgg aag gcc ttg ctg gcg   259
Val Ala Ala Phe His Asp Gly Phe Val Trp Trp Lys Ala Leu Leu Ala
        40                  45                  50

ctt gtc gtg gcg tgg gct ttg atc atc ggt gtg aat tac gcc aat gat   307
Leu Val Val Ala Trp Ala Leu Ile Ile Gly Val Asn Tyr Ala Asn Asp
    55                  60                  65

tac tct gat ggc att cgt ggc acc gat gaa gac cgc acc ggt cct ctg   355
Tyr Ser Asp Gly Ile Arg Gly Thr Asp Glu Asp Arg Thr Gly Pro Leu
70                  75                  80                  85

cga ctc act ggt tct ggg ttg gct gag ccg aag aaa gtg aaa gct gcg   403
Arg Leu Thr Gly Ser Gly Leu Ala Glu Pro Lys Lys Val Lys Ala Ala
                90                  95                  100

gcg ttt att tct ttc ggt atc gca ggt gtc gcc ggc acc gcg ctg agc   451
Ala Phe Ile Ser Phe Gly Ile Ala Gly Val Ala Gly Thr Ala Leu Ser
            105                 110                 115

ctg ttg agc gcg tgg tgg ctg atc ctc atc ggc atc ctg tgt gtg ctg   499
Leu Leu Ser Ala Trp Trp Leu Ile Leu Ile Gly Ile Leu Cys Val Leu
        120                 125                 130

ggc gcg tgg ttc tac acc ggc ggt aaa aat cct tat ggt tac cgc ggg   547
Gly Ala Trp Phe Tyr Thr Gly Gly Lys Asn Pro Tyr Gly Tyr Arg Gly
    135                 140                 145

ctc ggc gag att gct gtg ttc atc ttc ttc ggc ctc gtc gcg gtc atg     595
Leu Gly Glu Ile Ala Val Phe Ile Phe Phe Gly Leu Val Ala Val Met
150                 155                 160                 165

gga acg cag ttc acc caa acc ggt tcc gtc agc tgg gcc ggt ttg gcc     643
Gly Thr Gln Phe Thr Gln Thr Gly Ser Val Ser Trp Ala Gly Leu Ala
                170                 175                 180

gcc gca gtt ggc gtg ggg tcg atg tct gct ggc gtg aac ttg gcc aac     691
Ala Ala Val Gly Val Gly Ser Met Ser Ala Gly Val Asn Leu Ala Asn
            185                 190                 195

aat att cgc gat att cca acc gat agc aag acc gga aaa att acc ctc     739
Asn Ile Arg Asp Ile Pro Thr Asp Ser Lys Thr Gly Lys Ile Thr Leu
        200                 205                 210

gcg gtc cgc ctg ggc gat gcg ggt gct cgt aag ctg ttc ctc gcg ctg     787
Ala Val Arg Leu Gly Asp Ala Gly Ala Arg Lys Leu Phe Leu Ala Leu
    215                 220                 225

att tcc acg ccg ttc atc atg tcc atc tgc ctg gcg ttt gtc gcc tgg     835
Ile Ser Thr Pro Phe Ile Met Ser Ile Cys Leu Ala Phe Val Ala Trp
230                 235                 240                 245

cca gcg ctg atc gcg atc atc gtt ttc ccg ctg gca ctg aaa gcc gca     883
Pro Ala Leu Ile Ala Ile Ile Val Phe Pro Leu Ala Leu Lys Ala Ala
                250                 255                 260

ggg ccg atc cgc aac aac gcc acc ggc aag gat ctc atc ccc gtc atc     931
Gly Pro Ile Arg Asn Asn Ala Thr Gly Lys Asp Leu Ile Pro Val Ile
            265                 270                 275

ggc tca aca ggg cgc gcc atg gcg ttg tgg gcc gtg ctc acg ggc ctg     979
Gly Ser Thr Gly Arg Ala Met Ala Leu Trp Ala Val Leu Thr Gly Leu
        280                 285                 290

gca tta gcg ttt agc taaaacgctt ttcgacgctc ccc                       1017
Ala Leu Ala Phe Ser
    295


<210> 384
<211> 298
<212> PRT
<213> Corynebacterium glutamicum

<400> 384
Met Ser His Thr Glu Pro Gln Pro Asn Ser Val Thr Leu Ser Asp Trp
  1               5                  10                  15

Ile Gln Gly Ala Arg Pro Arg Thr Trp Ala Asn Ala Phe Ala Pro Val
             20                  25                  30

Ile Ala Gly Ser Gly Val Ala Ala Phe His Asp Gly Phe Val Trp Trp
         35                  40                  45

Lys Ala Leu Leu Ala Leu Val Val Ala Trp Ala Leu Ile Ile Gly Val
     50                  55                  60

Asn Tyr Ala Asn Asp Tyr Ser Asp Gly Ile Arg Gly Thr Asp Glu Asp
 65                  70                  75                  80

Arg Thr Gly Pro Leu Arg Leu Thr Gly Ser Gly Leu Ala Glu Pro Lys
                 85                  90                  95

Lys Val Lys Ala Ala Ala Phe Ile Ser Phe Gly Ile Ala Gly Val Ala
            100                 105                 110

Gly Thr Ala Leu Ser Leu Leu Ser Ala Trp Trp Leu Ile Leu Ile Gly
        115                 120                 125

Ile Leu Cys Val Leu Gly Ala Trp Phe Tyr Thr Gly Gly Lys Asn Pro
    130                 135                 140

Tyr Gly Tyr Arg Gly Leu Gly Glu Ile Ala Val Phe Ile Phe Phe Gly
145                 150                 155                 160

Leu Val Ala Val Met Gly Thr Gln Phe Thr Gln Thr Gly Ser Val Ser
                165                 170                 175

Trp Ala Gly Leu Ala Ala Ala Val Gly Val Gly Ser Met Ser Ala Gly
            180                 185                 190

Val Asn Leu Ala Asn Asn Ile Arg Asp Ile Pro Thr Asp Ser Lys Thr
        195                 200                 205

Gly Lys Ile Thr Leu Ala Val Arg Leu Gly Asp Ala Gly Ala Arg Lys
    210                 215                 220

Leu Phe Leu Ala Leu Ile Ser Thr Pro Phe Ile Met Ser Ile Cys Leu
225                 230                 235                 240

Ala Phe Val Ala Trp Pro Ala Leu Ile Ala Ile Ile Val Phe Pro Leu
                245                 250                 255

Ala Leu Lys Ala Ala Gly Pro Ile Arg Asn Asn Ala Thr Gly Lys Asp
            260                 265                 270

Leu Ile Pro Val Ile Gly Ser Thr Gly Arg Ala Met Ala Leu Trp Ala
        275                 280                 285

Val Leu Thr Gly Leu Ala Leu Ala Phe Ser
    290                 295


<210> 385
<211> 1242
<212> DNA
<213> Corynebacterium glutamicum

<220>
<221> CDS
<222> (101)..(1219)
<223> RXN00948

<400> 385
acaccctcca aatgatctcg taaaacagta ttgaatttag gtacgactct aatcgtacct 60

tgccctcaag ccaagctagt tgtacgatca aactcgttgt atg gca aac gtc gta   115
                                           Met Ala Asn Val Val
                                           1               5

cta gtc gat cga atg gag cct ttg gtg tcc aag ctg ttt acc cca att   163
Leu Val Asp Arg Met Glu Pro Leu Val Ser Lys Leu Phe Thr Pro Ile
                10                  15                  20

caa atc cgc gac atc acc atc ccc aac cgc gtg tgg atg tca ccg atg   211
Gln Ile Arg Asp Ile Thr Ile Pro Asn Arg Val Trp Met Ser Pro Met
            25                  30                  35

tgc acc tac tct gca gcc acc ggt tca ggt ctt ccc acc gat ttt cac   259
Cys Thr Tyr Ser Ala Ala Thr Gly Ser Gly Leu Pro Thr Asp Phe His
        40                  45                  50

cag gct cat tac gca gct cgc gca gca ggt ggt gtc gga tta gtc atg   307
Gln Ala His Tyr Ala Ala Arg Ala Ala Gly Gly Val Gly Leu Val Met
    55                  60                  65

gtt gaa gca act gga gtg aac ccc gta gct ccc atc tcc cca gtc gac   355
Val Glu Ala Thr Gly Val Asn Pro Val Ala Pro Ile Ser Pro Val Asp
70                  75                  80                  85

ctt gga ctt tgg agc cat gac caa att gaa cca ttc tcc cga gtg aca   403
Leu Gly Leu Trp Ser His Asp Gln Ile Glu Pro Phe Ser Arg Val Thr
                90                  95                  100

gca gct att cgc gcc ggt ggg gca gta ccg gcc gtt caa tta gcc cat   451
Ala Ala Ile Arg Ala Gly Gly Ala Val Pro Ala Val Gln Leu Ala His
            105                 110                 115

gct ggc cgc aag gca tcc acc gat gct ccg tgg aat ggt ggc gga tat   499
Ala Gly Arg Lys Ala Ser Thr Asp Ala Pro Trp Asn Gly Gly Gly Tyr
        120                 125                 130

gtt gga cca gaa acc aat gga tgg gag act gtc ggc ccc agc cct ctg     547
Val Gly Pro Glu Thr Asn Gly Trp Glu Thr Val Gly Pro Ser Pro Leu
    135                 140                 145

gca ttc cca ggt ttg cct gct ccg cgc gag ctg acg gtt tca gaa atc     595
Ala Phe Pro Gly Leu Pro Ala Pro Arg Glu Leu Thr Val Ser Glu Ile
150                 155                 160                 165

caa gag gtt gtg cag cag ttc gct ggc gcc gcc gtt cgt gcc gat cag     643
Gln Glu Val Val Gln Gln Phe Ala Gly Ala Ala Val Arg Ala Asp Gln
                170                 175                 180

gct ggt ttt gat gtc gtg gaa att cac gca gca cac ggc tac ctt ttg     691
Ala Gly Phe Asp Val Val Glu Ile His Ala Ala His Gly Tyr Leu Leu
            185                 190                 195

cat aac ttc ctt tct ccg atc tcc aac aag cgc acc gat tca tac ggc     739
His Asn Phe Leu Ser Pro Ile Ser Asn Lys Arg Thr Asp Ser Tyr Gly
        200                 205                 210

gga tct tta gaa aac cgc gct cgc atc gtg ctc gaa gtc att gat gca     787
Gly Ser Leu Glu Asn Arg Ala Arg Ile Val Leu Glu Val Ile Asp Ala
    215                 220                 225

atc cgc gca gtg tgg cca gag gaa aag cct gta ttc atg cgc att tcc     835
Ile Arg Ala Val Trp Pro Glu Glu Lys Pro Val Phe Met Arg Ile Ser
230                 235                 240                 245

acc acc gac tgg gtg gag gaa aac cca cag gat gat cgc gag tcc tgg     883
Thr Thr Asp Trp Val Glu Glu Asn Pro Gln Asp Asp Arg Glu Ser Trp
                250                 255                 260

acg ctg agc caa agc agg cag ctg gct ttg tgg gca tcc gag cac gga     931
Thr Leu Ser Gln Ser Arg Gln Leu Ala Leu Trp Ala Ser Glu His Gly
            265                 270                 275

gtt gat ttg atc gat gcc tct tct ggt ggc ctc gac atc gtc ccc att     979
Val Asp Leu Ile Asp Ala Ser Ser Gly Gly Leu Asp Ile Val Pro Ile
        280                 285                 290

ccg cat gac cgc gat tac caa acc gcg aag gcc gca gat ctt cac gca     1027
Pro His Asp Arg Asp Tyr Gln Thr Ala Lys Ala Ala Asp Leu His Ala
    295                 300                 305

agt acc gga gtg aca gtc gct gct gtg ggg cgc att gat gac gcc caa     1075
Ser Thr Gly Val Thr Val Ala Ala Val Gly Arg Ile Asp Asp Ala Gln
310                 315                 320                 325

act gcg cac aat ttg gtt gat tct ggc gat gtc aat gca gtt ttc ctc     1123
Thr Ala His Asn Leu Val Asp Ser Gly Asp Val Asn Ala Val Phe Leu
                330                 335                 340

ggc cgt cca ctg ctc aag gat cct tcc tgg gca aac caa gca gcc ctc     1171
Gly Arg Pro Leu Leu Lys Asp Pro Ser Trp Ala Asn Gln Ala Ala Leu
            345                 350                 355

gca cta ggt gcg gaa ccc agg tat gtt cac caa tac gac tac gta ctt     1219
Ala Leu Gly Ala Glu Pro Arg Tyr Val His Gln Tyr Asp Tyr Val Leu
        360                 365                 370

taaaggagag ttgacatgaa ggt                                           1242


<210> 386
<211> 373
<212> PRT
<213> Corynebacterium glutamicum

<400> 386
Met Ala Asn Val Val Leu Val Asp Arg Met Glu Pro Leu Val Ser Lys
1               5                   10                  15

Leu Phe Thr Pro Ile Gln Ile Arg Asp Ile Thr Ile Pro Asn Arg Val
            20                  25                  30

Trp Met Ser Pro Met Cys Thr Tyr Ser Ala Ala Thr Gly Ser Gly Leu
        35                  40                  45

Pro Thr Asp Phe His Gln Ala His Tyr Ala Ala Arg Ala Ala Gly Gly
    50                  55                  60

Val Gly Leu Val Met Val Glu Ala Thr Gly Val Asn Pro Val Ala Pro
65                  70                  75                  80

Ile Ser Pro Val Asp Leu Gly Leu Trp Ser His Asp Gln Ile Glu Pro
                85                  90                  95

Phe Ser Arg Val Thr Ala Ala Ile Arg Ala Gly Gly Ala Val Pro Ala
            100                 105                 110

Val Gln Leu Ala His Ala Gly Arg Lys Ala Ser Thr Asp Ala Pro Trp
        115                 120                 125

Asn Gly Gly Gly Tyr Val Gly Pro Glu Thr Asn Gly Trp Glu Thr Val
    130                 135                 140

Gly Pro Ser Pro Leu Ala Phe Pro Gly Leu Pro Ala Pro Arg Glu Leu
145                 150                 155                 160

Thr Val Ser Glu Ile Gln Glu Val Val Gln Gln Phe Ala Gly Ala Ala
                165                 170                 175

Val Arg Ala Asp Gln Ala Gly Phe Asp Val Val Glu Ile His Ala Ala
            180                 185                 190

His Gly Tyr Leu Leu His Asn Phe Leu Ser Pro Ile Ser Asn Lys Arg
        195                 200                 205

Thr Asp Ser Tyr Gly Gly Ser Leu Glu Asn Arg Ala Arg Ile Val Leu
    210                 215                 220

Glu Val Ile Asp Ala Ile Arg Ala Val Trp Pro Glu Glu Lys Pro Val
225                 230                 235                 240

Phe Met Arg Ile Ser Thr Thr Asp Trp Val Glu Glu Asn Pro Gln Asp
                245                 250                 255

Asp Arg Glu Ser Trp Thr Leu Ser Gln Ser Arg Gln Leu Ala Leu Trp
            260                 265                 270

Ala Ser Glu His Gly Val Asp Leu Ile Asp Ala Ser Ser Gly Gly Leu
        275                 280                 285

Asp Ile Val Pro Ile Pro His Asp Arg Asp Tyr Gln Thr Ala Lys Ala
    290                 295                 300

Ala Asp Leu His Ala Ser Thr Gly Val Thr Val Ala Ala Val Gly Arg
305                 310                 315                 320

Ile Asp Asp Ala Gln Thr Ala His Asn Leu Val Asp Ser Gly Asp Val
                325                 330                 335

Asn Ala Val Phe Leu Gly Arg Pro Leu Leu Lys Asp Pro Ser Trp Ala
            340                 345                 350

Asn Gln Ala Ala Leu Ala Leu Gly Ala Glu Pro Arg Tyr Val His Gln
        355                 360                 365

Tyr Asp Tyr Val Leu
    370


<210> 387
<211> 873
<212> DNA
<213> Corynebacterium glutamicum

<220>
<221> CDS
<222> (101)..(850)
<223> RXN01923

<400> 387
ccaaagtgaa taccccgact gcagcagcgc aaaagttcaa gtactttggg atgcaaatct 60

agtagcacgt cccatgtttc tcacactctc aggagctgac atg tct gca ctt att   115
                                           Met Ser Ala Leu Ile
                                           1               5

aaa ggt tca gga cct cat cat gtg gtt gtc tta aat ggt tgg ttt ggt   163
Lys Gly Ser Gly Pro His His Val Val Val Leu Asn Gly Trp Phe Gly
                10                  15                  20

cat gct gcg ggc tgg gga gct ttc gct gac tat ctt gac ctc ggc aac   211
His Ala Ala Gly Trp Gly Ala Phe Ala Asp Tyr Leu Asp Leu Gly Asn
            25                  30                  35

tac acc tgg cac ttt tgg gat tac cga ggt tac ggc aac aga aaa gac   259
Tyr Thr Trp His Phe Trp Asp Tyr Arg Gly Tyr Gly Asn Arg Lys Asp
        40                  45                  50

gac gca gga gaa ttt act ctg gag gaa att tca gcg gat atc gtt gca   307
Asp Ala Gly Glu Phe Thr Leu Glu Glu Ile Ser Ala Asp Ile Val Ala
    55                  60                  65

tac atc gac tcg att gag gca gaa aag gtt tcc atc ctg ggc cat tcc   355
Tyr Ile Asp Ser Ile Glu Ala Glu Lys Val Ser Ile Leu Gly His Ser
70                  75                  80                  85

atg ggt gga gtg ttc atg cag aaa gtc ctt gca gac agc gcc acc ccc   403
Met Gly Gly Val Phe Met Gln Lys Val Leu Ala Asp Ser Ala Thr Pro
                90                  95                  100

atc gct tca ctg gtt gga att tct gcc gtt gct gca gct gga aca cca   451
Ile Ala Ser Leu Val Gly Ile Ser Ala Val Ala Ala Ala Gly Thr Pro
            105                 110                 115

ttc gat gag gat tct cgg aag ctt ttc acc tca gca ggg cac aac ccg   499
Phe Asp Glu Asp Ser Arg Lys Leu Phe Thr Ser Ala Gly His Asn Pro
        120                 125                 130

gac tcg agg cga gcc atc atc gat ttc acc tca gga tct cgc caa cct   547
Asp Ser Arg Arg Ala Ile Ile Asp Phe Thr Ser Gly Ser Arg Gln Pro
    135                 140                 145

gcc gcg tgg ttg gat gat ctc acc gac tcg gcg gtg cag aat tcc act   595
Ala Ala Trp Leu Asp Asp Leu Thr Asp Ser Ala Val Gln Asn Ser Thr
150                 155                 160                 165

cca gag gcc gtt gaa aag tac ttt ttt gcg tgg gct gat tgt aat ttc   643
Pro Glu Ala Val Glu Lys Tyr Phe Phe Ala Trp Ala Asp Cys Asn Phe
                170                 175                 180

gca gcg gat tta ggc acc caa gat ttg ccc gtg gac att ctc acc ggc   691
Ala Ala Asp Leu Gly Thr Gln Asp Leu Pro Val Asp Ile Leu Thr Gly
            185                 190                 195

gat ctc gac ccc gcg gtc act aaa act gcc gtg gaa tcc gca ttc ggc   739
Asp Leu Asp Pro Ala Val Thr Lys Thr Ala Val Glu Ser Ala Phe Gly
        200                 205                 210

ccg atc tat caa aat ctg acc gtt gaa gaa ctc cac gat gtc gga cac   787
Pro Ile Tyr Gln Asn Leu Thr Val Glu Glu Leu His Asp Val Gly His
    215                 220                 225

tac gca att ttc gag cac ccc tta ggc ctt gcc gcc agg gtg ctt cga   835
Tyr Ala Ile Phe Glu His Pro Leu Gly Leu Ala Ala Arg Val Leu Arg
230                 235                 240                 245

ttt ctc gac gcc gtc tagtacttcc gcaaattcac cgg                    873
Phe Leu Asp Ala Val
                250


<210> 388
<211> 250
<212> PRT
<213> Corynebacterium glutamicum

<400> 388
Met Ser Ala Leu Ile Lys Gly Ser Gly Pro His His Val Val Val Leu

1               5                   10                  15

Asn Gly Trp Phe Gly His Ala Ala Gly Trp Gly Ala Phe Ala Asp Tyr
            20                  25                  30

Leu Asp Leu Gly Asn Tyr Thr Trp His Phe Trp Asp Tyr Arg Gly Tyr
        35                  40                  45

Gly Asn Arg Lys Asp Asp Ala Gly Glu Phe Thr Leu Glu Glu Ile Ser
    50                  55                  60

Ala Asp Ile Val Ala Tyr Ile Asp Ser Ile Glu Ala Glu Lys Val Ser
65                  70                  75                  80

Ile Leu Gly His Ser Met Gly Gly Val Phe Met Gln Lys Val Leu Ala
                85                  90                  95

Asp Ser Ala Thr Pro Ile Ala Ser Leu Val Gly Ile Ser Ala Val Ala
            100                 105                 110

Ala Ala Gly Thr Pro Phe Asp Glu Asp Ser Arg Lys Leu Phe Thr Ser
        115                 120                 125

Ala Gly His Asn Pro Asp Ser Arg Arg Ala Ile Ile Asp Phe Thr Ser
    130                 135                 140

Gly Ser Arg Gln Pro Ala Ala Trp Leu Asp Asp Leu Thr Asp Ser Ala
145                 150                 155                 160

Val Gln Asn Ser Thr Pro Glu Ala Val Glu Lys Tyr Phe Phe Ala Trp
                165                 170                 175

Ala Asp Cys Asn Phe Ala Ala Asp Leu Gly Thr Gln Asp Leu Pro Val
            180                 185                 190

Asp Ile Leu Thr Gly Asp Leu Asp Pro Ala Val Thr Lys Thr Ala Val
        195                 200                 205

Glu Ser Ala Phe Gly Pro Ile Tyr Gln Asn Leu Thr Val Glu Glu Leu
    210                 215                 220

His Asp Val Gly His Tyr Ala Ile Phe Glu His Pro Leu Gly Leu Ala
225                 230                 235                 240

Ala Arg Val Leu Arg Phe Leu Asp Ala Val
                245                 250


<210> 389
<211> 873
<212> DNA
<213> Corynebacterium glutamicum

<220>
<221> CDS
<222> (101)..(850)
<223> RXN00398

<400> 389
tgagttcgcc accatcagca ccggcaccca ccagcgcggt gtggttaacc gtgagaagtt 60

tgtctcccgt ctgcctgaag cacctaagga aaactaaatc atg gcc aag ttg ttt   115
                                            Met Ala Lys Leu Phe
                                            1               5

gat tcc cat ttc cat atc atc gat ccc cag cac cca ctg atc gaa aac   163
Asp Ser His Phe His Ile Ile Asp Pro Gln His Pro Leu Ile Glu Asn
                10                  15                  20

aac ggc tac ctc ccc gag cct ttc acc gtg gag gat tac act gcg cgt   211
Asn Gly Tyr Leu Pro Glu Pro Phe Thr Val Glu Asp Tyr Thr Ala Arg
            25                  30                  35

gtt gaa ggc ctc gaa gtt gct gcc gga gcg att gtt tcc ggt tct ttc   259
Val Glu Gly Leu Glu Val Ala Ala Gly Ala Ile Val Ser Gly Ser Phe
        40                  45                  50

cag gct ttc gac cag ggc tac ctc aaa gat gct ctc gca gtg ctt ggc    307
Gln Ala Phe Asp Gln Gly Tyr Leu Lys Asp Ala Leu Ala Val Leu Gly
     55                  60                  65

cca ggc tat gtc ggt gtc act cag atc ccc gca gat acc tct gat cag    355
Pro Gly Tyr Val Gly Val Thr Gln Ile Pro Ala Asp Thr Ser Asp Gln
 70                  75                  80                  85

gag att ctt gat ctg gac aaa gct ggc gtg aag gct gtg cgt tta aac    403
Glu Ile Leu Asp Leu Asp Lys Ala Gly Val Lys Ala Val Arg Leu Asn
                 90                  95                 100

ttg aag cgc ggt ggt tcg gca ggt ctt gac gat ctc gag acc ttg gca    451
Leu Lys Arg Gly Gly Ser Ala Gly Leu Asp Asp Leu Glu Thr Leu Ala
            105                 110                 115

cgc cga gtc cac gac cta gcc ggt tgg cac acc gaa ctc tat gtg gat    499
Arg Arg Val His Asp Leu Ala Gly Trp His Thr Glu Leu Tyr Val Asp
        120                 125                 130

gct cgc gaa cta gac gag ttg gaa tca acc ttg gcc tcc ctc cct gct    547
Ala Arg Glu Leu Asp Glu Leu Glu Ser Thr Leu Ala Ser Leu Pro Ala
    135                 140                 145

gtc agc att gat cac tta ggg ctc cac cgc gat gga ctt ccc gca ctt    595
Val Ser Ile Asp His Leu Gly Leu His Arg Asp Gly Leu Pro Ala Leu
150                 155                 160                 165

ctt cgc ttg gta gaa aat ggc att aaa gtc aaa gca acc gga ttc gga    643
Leu Arg Leu Val Glu Asn Gly Ile Lys Val Lys Ala Thr Gly Phe Gly
                170                 175                 180

cgg gta gaa cta gat cca act gaa gtc atc cag gca atc atg gct gtc    691
Arg Val Glu Leu Asp Pro Thr Glu Val Ile Gln Ala Ile Met Ala Val
            185                 190                 195

gat ccc act gct ttg atg atc gga act gat ctt cca tcc acc cgc act    739
Asp Pro Thr Ala Leu Met Ile Gly Thr Asp Leu Pro Ser Thr Arg Thr
        200                 205                 210

aag cga cct ttc gaa gac gct gac cta gat ttg atc gct gaa acg gtt    787
Lys Arg Pro Phe Glu Asp Ala Asp Leu Asp Leu Ile Ala Glu Thr Val
    215                 220                 225

ggc gaa gat cat gtc gac aac gtc ttc tgg aac aac gct gca gcg ttc    835
Gly Glu Asp His Val Asp Asn Val Phe Trp Asn Asn Ala Ala Ala Phe
230                 235                 240                 245

tac ctc gga gac cag tagttttaag acccgaaatg tct                      873
Tyr Leu Gly Asp Gln
                250


<210> 390
<211> 250
<212> PRT
<213> Corynebacterium glutamicum

<400> 390
Met Ala Lys Leu Phe Asp Ser His Phe His Ile Ile Asp Pro Gln His
  1               5                  10                  15

Pro Leu Ile Glu Asn Asn Gly Tyr Leu Pro Glu Pro Phe Thr Val Glu
             20                  25                  30

Asp Tyr Thr Ala Arg Val Glu Gly Leu Glu Val Ala Ala Gly Ala Ile
         35                  40                  45

Val Ser Gly Ser Phe Gln Ala Phe Asp Gln Gly Tyr Leu Lys Asp Ala
     50                  55                  60

Leu Ala Val Leu Gly Pro Gly Tyr Val Gly Val Thr Gln Ile Pro Ala
 65                  70                  75                  80

Asp Thr Ser Asp Gln Glu Ile Leu Asp Leu Asp Lys Ala Gly Val Lys
                 85                  90                  95

Ala Val Arg Leu Asn Leu Lys Arg Gly Gly Ser Ala Gly Leu Asp Asp
            100                 105                 110

Leu Glu Thr Leu Ala Arg Arg Val His Asp Leu Ala Gly Trp His Thr
        115                 120                 125

Glu Leu Tyr Val Asp Ala Arg Glu Leu Asp Glu Leu Glu Ser Thr Leu
    130                 135                 140

Ala Ser Leu Pro Ala Val Ser Ile Asp His Leu Gly Leu His Arg Asp
145                 150                 155                 160

Gly Leu Pro Ala Leu Leu Arg Leu Val Glu Asn Gly Ile Lys Val Lys
                165                 170                 175

Ala Thr Gly Phe Gly Arg Val Glu Leu Asp Pro Thr Glu Val Ile Gln
            180                 185                 190

Ala Ile Met Ala Val Asp Pro Thr Ala Leu Met Ile Gly Thr Asp Leu
        195                 200                 205

Pro Ser Thr Arg Thr Lys Arg Pro Phe Glu Asp Ala Asp Leu Asp Leu
    210                 215                 220

Ile Ala Glu Thr Val Gly Glu Asp His Val Asp Asn Val Phe Trp Asn
225                 230                 235                 240

Asn Ala Ala Ala Phe Tyr Leu Gly Asp Gln
                245                 250


<210> 391
<211> 1108
<212> DNA
<213> Corynebacterium glutamicum

<220>
<221> CDS
<222> (101)..(1099)
<223> RXN02813

<400> 391
ggactctaaa ttgacccgat ctttatactc cgaccttgct ggtagtggag aacacctcag 60

cagcctttcc gacgagactt tcctaaagaa tcttcttgtc gtg gag gcc gct ttg   115
                                           Val Glu Ala Ala Leu
                                           1               5

gcg gtt gca gct gcc ccc gag cac gca gca atg gcg aag gcc acc att   163
Ala Val Ala Ala Ala Pro Glu His Ala Ala Met Ala Lys Ala Thr Ile
                10                  15                  20

gat tct tat cag ttg gat gtg gag gag ctt tcc cgt cgc gca gcc gag   211
Asp Ser Tyr Gln Leu Asp Val Glu Glu Leu Ser Arg Arg Ala Ala Glu
            25                  30                  35

ggc ggt aat ccg ctc att ccg ctg gtc act gac ctc aag gcc atc aat   259
Gly Gly Asn Pro Leu Ile Pro Leu Val Thr Asp Leu Lys Ala Ile Asn
        40                  45                  50

ccg gca ggc atc cac att ggc gca acg agc cag gac atc att gat tct   307
Pro Ala Gly Ile His Ile Gly Ala Thr Ser Gln Asp Ile Ile Asp Ser
    55                  60                  65

gcg tta atg ctg tgc atg aag gaa ggg gtg ggg gag gtc gtc gac aag   355
Ala Leu Met Leu Cys Met Lys Glu Gly Val Gly Glu Val Val Asp Lys
70                  75                  80                  85

ctt aaa aag ctt gcg cga gat ttg gcc gag ctc acc gcg gag cat aaa   403
Leu Lys Lys Leu Ala Arg Asp Leu Ala Glu Leu Thr Ala Glu His Lys
                90                  95                  100

gca acc ccg atc atg ggg cgc acg ttg ggg cag atc gcg acg ccg acg   451
Ala Thr Pro Ile Met Gly Arg Thr Leu Gly Gln Ile Ala Thr Pro Thr
            105                 110                 115

acg ttc ggc gcg ctg acc ggc ggc tgg ctg gtt gcg gtg gac aat gcg   499

Thr Phe Gly Ala Leu Thr Gly Gly Trp Leu Val Ala Val Asp Asn Ala
        120                 125                 130

gca cgc gcc ctg gag gcg ctg gag ttt ccg gtg tcg tat ggc ggt gcc     547
Ala Arg Ala Leu Glu Ala Leu Glu Phe Pro Val Ser Tyr Gly Gly Ala
    135                 140                 145

agc gga aat atg acg gcg gtg cac ccg cgt ggc ttc gag att cag gcg     595
Ser Gly Asn Met Thr Ala Val His Pro Arg Gly Phe Glu Ile Gln Ala
150                 155                 160                 165

aag ctg gcc gag gag ttg ggc ctt ttt gat ccg cag tgg gtg tgg cat     643
Lys Leu Ala Glu Glu Leu Gly Leu Phe Asp Pro Gln Trp Val Trp His
                170                 175                 180

tcc gat cgc acg ccg atc act gcg atc gcg tcg gcg ctg gca acg gcc     691
Ser Asp Arg Thr Pro Ile Thr Ala Ile Ala Ser Ala Leu Ala Thr Ala
            185                 190                 195

gct ggt gtg gta cgc aaa att gct ggt gac gtg gtg ttt tac tca caa     739
Ala Gly Val Val Arg Lys Ile Ala Gly Asp Val Val Phe Tyr Ser Gln
        200                 205                 210

acc gag gtc ggc gag ttg cgg gag aaa tcc ccc ggc ggc agc tcc gcg     787
Thr Glu Val Gly Glu Leu Arg Glu Lys Ser Pro Gly Gly Ser Ser Ala
    215                 220                 225

atg ccc cac aaa gcc aat ccg gcc gct gcg att gcg tgc gac ggt tac     835
Met Pro His Lys Ala Asn Pro Ala Ala Ala Ile Ala Cys Asp Gly Tyr
230                 235                 240                 245

gcg cgc cgg gca cct ggc ctt ctt gca acg ctt ttc gac gcc ctc gac     883
Ala Arg Arg Ala Pro Gly Leu Leu Ala Thr Leu Phe Asp Ala Leu Asp
                250                 255                 260

tgc cgt ttg cag cgc ggc acc ggc agc tgg cac gcg gag tgg gca acg     931
Cys Arg Leu Gln Arg Gly Thr Gly Ser Trp His Ala Glu Trp Ala Thr
            265                 270                 275

ctg cgc gag ttg gct gct gtc act cac tca gca gtg agc agg gct gca     979
Leu Arg Glu Leu Ala Ala Val Thr His Ser Ala Val Ser Arg Ala Ala
        280                 285                 290

acc agc atc gat ggc atc acc gtc aac gtt gat gtg atg gca agt cgc     1027
Thr Ser Ile Asp Gly Ile Thr Val Asn Val Asp Val Met Ala Ser Arg
    295                 300                 305

gtc aat gga cca acc ggg cac gcc gaa gat ttg gcg gag cgg gca cta     1075
Val Asn Gly Pro Thr Gly His Ala Glu Asp Leu Ala Glu Arg Ala Leu
310                 315                 320                 325

gaa att tat gga aaa gga cgc agt taatggatc                           1108
Glu Ile Tyr Gly Lys Gly Arg Ser
                330


<210> 392
<211> 333
<212> PRT
<213> Corynebacterium glutamicum

<400> 392
Val Glu Ala Ala Leu Ala Val Ala Ala Ala Pro Glu His Ala Ala Met
1               5                   10                  15

Ala Lys Ala Thr Ile Asp Ser Tyr Gln Leu Asp Val Glu Glu Leu Ser
            20                  25                  30

Arg Arg Ala Ala Glu Gly Gly Asn Pro Leu Ile Pro Leu Val Thr Asp
        35                  40                  45

Leu Lys Ala Ile Asn Pro Ala Gly Ile His Ile Gly Ala Thr Ser Gln
    50                  55                  60

Asp Ile Ile Asp Ser Ala Leu Met Leu Cys Met Lys Glu Gly Val Gly
65                  70                  75                  80

Glu Val Val Asp Lys Leu Lys Lys Leu Ala Arg Asp Leu Ala Glu Leu
                85                  90                  95

Thr Ala Glu His Lys Ala Thr Pro Ile Met Gly Arg Thr Leu Gly Gln
            100                 105                 110

Ile Ala Thr Pro Thr Thr Phe Gly Ala Leu Thr Gly Gly Trp Leu Val
        115                 120                 125

Ala Val Asp Asn Ala Ala Arg Ala Leu Glu Ala Leu Glu Phe Pro Val
    130                 135                 140

Ser Tyr Gly Gly Ala Ser Gly Asn Met Thr Ala Val His Pro Arg Gly
145                 150                 155                 160

Phe Glu Ile Gln Ala Lys Leu Ala Glu Glu Leu Gly Leu Phe Asp Pro
                165                 170                 175

Gln Trp Val Trp His Ser Asp Arg Thr Pro Ile Thr Ala Ile Ala Ser
            180                 185                 190

Ala Leu Ala Thr Ala Ala Gly Val Val Arg Lys Ile Ala Gly Asp Val
        195                 200                 205

Val Phe Tyr Ser Gln Thr Glu Val Gly Glu Leu Arg Glu Lys Ser Pro
    210                 215                 220

Gly Gly Ser Ser Ala Met Pro His Lys Ala Asn Pro Ala Ala Ala Ile
225                 230                 235                 240

Ala Cys Asp Gly Tyr Ala Arg Arg Ala Pro Gly Leu Leu Ala Thr Leu
                245                 250                 255

Phe Asp Ala Leu Asp Cys Arg Leu Gln Arg Gly Thr Gly Ser Trp His
            260                 265                 270

Ala Glu Trp Ala Thr Leu Arg Glu Leu Ala Ala Val Thr His Ser Ala
        275                 280                 285

Val Ser Arg Ala Ala Thr Ser Ile Asp Gly Ile Thr Val Asn Val Asp
    290                 295                 300

Val Met Ala Ser Arg Val Asn Gly Pro Thr Gly His Ala Glu Asp Leu
305                 310                 315                 320

Ala Glu Arg Ala Leu Glu Ile Tyr Gly Lys Gly Arg Ser
                325                 330


<210> 393
<211> 1218
<212> DNA
<213> Corynebacterium glutamicum

<220>
<221> CDS
<222> (101)..(1195)
<223> RXN00136

<400> 393
cagtgttgca tcatctagaa atcgattaat taaaccgggc acctgattaa cattgggctg 60

cccggtttct tcctattaca agcgaaaggc aacgtgcccc atg agc gca gtg cag   115
                                           Met Ser Ala Val Gln
                                           1               5

att ttc aac acc gtc cac gtc aat gga tct tcc ccc tat gat gtc cac   163
Ile Phe Asn Thr Val His Val Asn Gly Ser Ser Pro Tyr Asp Val His
                10                  15                  20

att ggt tcc ggc ctc aac gag ctc att gtt cag cgc gca gcg gaa tca   211
Ile Gly Ser Gly Leu Asn Glu Leu Ile Val Gln Arg Ala Ala Glu Ser
            25                  30                  35

ggc gcg gag cag gta gcg att ttg cac cag ccc agc atg gat gac att   259
Gly Ala Glu Gln Val Ala Ile Leu His Gln Pro Ser Met Asp Asp Ile
        40                  45                  50

gca tcc gag ttg gat gca gca cta gtc gct gct ggt ttg aag gtc ctg    307
Ala Ser Glu Leu Asp Ala Ala Leu Val Ala Ala Gly Leu Lys Val Leu
     55                  60                  65

cac ctt aat gtt ccc gat gcg gaa aac ggc aag tcc ttg gaa gta gcg    355
His Leu Asn Val Pro Asp Ala Glu Asn Gly Lys Ser Leu Glu Val Ala
 70                  75                  80                  85

ggg cag tgc tgg gat gaa ttg ggt ggc gca gca ttc ggc cgc cgc gat    403
Gly Gln Cys Trp Asp Glu Leu Gly Gly Ala Ala Phe Gly Arg Arg Asp
                 90                  95                 100

atc gtc atc gga ctt ggt ggc ggt gct gcc aca gat ctc gcg gga ttc    451
Ile Val Ile Gly Leu Gly Gly Gly Ala Ala Thr Asp Leu Ala Gly Phe
            105                 110                 115

gtc gct gct gca tgg atg cgt ggc gtg cgc gtc att cag gtt cca acc    499
Val Ala Ala Ala Trp Met Arg Gly Val Arg Val Ile Gln Val Pro Thr
        120                 125                 130

acc ttg ttg gcc atg gtg gac gct gcg gtg ggc ggc aag act ggc atc    547
Thr Leu Leu Ala Met Val Asp Ala Ala Val Gly Gly Lys Thr Gly Ile
    135                 140                 145

aat acc gcc gca ggc aag aac ctt gtg ggc gcg ttc cac gag cct gac    595
Asn Thr Ala Ala Gly Lys Asn Leu Val Gly Ala Phe His Glu Pro Asp
150                 155                 160                 165

gca gta ttc att gac acc gat cgc cta gcc acc ctg cct gac gcg gaa    643
Ala Val Phe Ile Asp Thr Asp Arg Leu Ala Thr Leu Pro Asp Ala Glu
                170                 175                 180

atc atc gcg gga tcc gcc gaa atc atc aaa act ggt ttc atc gcc gac    691
Ile Ile Ala Gly Ser Ala Glu Ile Ile Lys Thr Gly Phe Ile Ala Asp
            185                 190                 195

cca gaa atc ctg cgc ctt tac gaa act gat ccc gca gcc tgc ctg aag    739
Pro Glu Ile Leu Arg Leu Tyr Glu Thr Asp Pro Ala Ala Cys Leu Lys
        200                 205                 210

aaa gaa gtc gaa ggc tcc cac cta cct gaa ctg att tgg cgc tcc gtc    787
Lys Glu Val Glu Gly Ser His Leu Pro Glu Leu Ile Trp Arg Ser Val
    215                 220                 225

acc gtc aag ggc tcc gtg gtc ggc caa gac ctc aaa gaa tct agc ctg    835
Thr Val Lys Gly Ser Val Val Gly Gln Asp Leu Lys Glu Ser Ser Leu
230                 235                 240                 245

cgc gaa atc ctc aac tac gga cac acc ttt gcc cac gcc gtc gaa ctc    883
Arg Glu Ile Leu Asn Tyr Gly His Thr Phe Ala His Ala Val Glu Leu
                250                 255                 260

cgc gaa aac ttc cgc tgg cgc cac ggc aat gcc gtt gca gtg ggc atg    931
Arg Glu Asn Phe Arg Trp Arg His Gly Asn Ala Val Ala Val Gly Met
            265                 270                 275

atg ttc atc gcc aac ctc tcc cac aag ctc ggg ctt atc gac gcg ccc    979
Met Phe Ile Ala Asn Leu Ser His Lys Leu Gly Leu Ile Asp Ala Pro
        280                 285                 290

ctc ctc gag cgc cac cgc tca atc ctg gcg gcc atc ggt ctg ccc act    1027
Leu Leu Glu Arg His Arg Ser Ile Leu Ala Ala Ile Gly Leu Pro Thr
    295                 300                 305

tcc tac gaa ggc gga gcc ttc gac gag ctt tac gac ggt atg acc cgc    1075
Ser Tyr Glu Gly Gly Ala Phe Asp Glu Leu Tyr Asp Gly Met Thr Arg
310                 315                 320                 325

gac aag aaa aac cgc gac ggc aac atc cgc ttc gtc gca ctg acc gcc    1123
Asp Lys Lys Asn Arg Asp Gly Asn Ile Arg Phe Val Ala Leu Thr Ala
                330                 335                 340

gtg ggc gag gtt acc cgc att gag ggg ccc tca aaa caa gat tta cag    1171
Val Gly Glu Val Thr Arg Ile Glu Gly Pro Ser Lys Gln Asp Leu Gln
            345                 350                 355

agt gct tat gag gca atc agc cac taagtgttga gtaatctact agt        1218
Ser Ala Tyr Glu Ala Ile Ser His
        360                 365

<210> 394
<211> 365
<212> PRT
<213> Corynebacterium glutamicum

<400> 394
Met Ser Ala Val Gln Ile Phe Asn Thr Val His Val Asn Gly Ser Ser
1               5                   10                  15

Pro Tyr Asp Val His Ile Gly Ser Gly Leu Asn Glu Leu Ile Val Gln
            20                  25                  30

Arg Ala Ala Glu Ser Gly Ala Glu Gln Val Ala Ile Leu His Gln Pro
        35                  40                  45

Ser Met Asp Asp Ile Ala Ser Glu Leu Asp Ala Ala Leu Val Ala Ala
    50                  55                  60

Gly Leu Lys Val Leu His Leu Asn Val Pro Asp Ala Glu Asn Gly Lys
65                  70                  75                  80

Ser Leu Glu Val Ala Gly Gln Cys Trp Asp Glu Leu Gly Gly Ala Ala
                85                  90                  95

Phe Gly Arg Arg Asp Ile Val Ile Gly Leu Gly Gly Gly Ala Ala Thr
            100                 105                 110

Asp Leu Ala Gly Phe Val Ala Ala Ala Trp Met Arg Gly Val Arg Val
        115                 120                 125

Ile Gln Val Pro Thr Thr Leu Leu Ala Met Val Asp Ala Ala Val Gly
    130                 135                 140

Gly Lys Thr Gly Ile Asn Thr Ala Ala Gly Lys Asn Leu Val Gly Ala
145                 150                 155                 160

Phe His Glu Pro Asp Ala Val Phe Ile Asp Thr Asp Arg Leu Ala Thr
                165                 170                 175

Leu Pro Asp Ala Glu Ile Ile Ala Gly Ser Ala Glu Ile Ile Lys Thr
            180                 185                 190

Gly Phe Ile Ala Asp Pro Glu Ile Leu Arg Leu Tyr Glu Thr Asp Pro
        195                 200                 205

Ala Ala Cys Leu Lys Lys Glu Val Glu Gly Ser His Leu Pro Glu Leu
    210                 215                 220

Ile Trp Arg Ser Val Thr Val Lys Gly Ser Val Val Gly Gln Asp Leu
225                 230                 235                 240

Lys Glu Ser Ser Leu Arg Glu Ile Leu Asn Tyr Gly His Thr Phe Ala
                245                 250                 255

His Ala Val Glu Leu Arg Glu Asn Phe Arg Trp Arg His Gly Asn Ala
            260                 265                 270

Val Ala Val Gly Met Met Phe Ile Ala Asn Leu Ser His Lys Leu Gly
        275                 280                 285

Leu Ile Asp Ala Pro Leu Leu Glu Arg His Arg Ser Ile Leu Ala Ala
    290                 295                 300

Ile Gly Leu Pro Thr Ser Tyr Glu Gly Gly Ala Phe Asp Glu Leu Tyr
305                 310                 315                 320

Asp Gly Met Thr Arg Asp Lys Lys Asn Arg Asp Gly Asn Ile Arg Phe
                325                 330                 335

Val Ala Leu Thr Ala Val Gly Glu Val Thr Arg Ile Glu Gly Pro Ser
            340                 345                 350

Lys Gln Asp Leu Gln Ser Ala Tyr Glu Ala Ile Ser His
        355                 360                 365

<210> 395
<211> 1977
<212> DNA
<213> Corynebacterium glutamicum

<220>
<221> CDS
<222> (101)..(1954)
<223> RXN02508

<400> 395
tgcacactgc tggtggtgag gccgcagacc tggcagccgc aagcaaagcc tccgaggccc 60

aactcgcggc tcagtaaaac caaaaggaat ctttgaccac atg cgt aca tcc att   115
                                           Met Arg Thr Ser Ile
                                           1               5

gcc act gtt tgt ttg tcc gga act ctt gct gaa aag ctg cgc gca gct   163
Ala Thr Val Cys Leu Ser Gly Thr Leu Ala Glu Lys Leu Arg Ala Ala
                10                  15                  20

gca gat gct gga ttt gat ggt gtg gaa atc ttc gag cag gac ttg gtg   211
Ala Asp Ala Gly Phe Asp Gly Val Glu Ile Phe Glu Gln Asp Leu Val
            25                  30                  35

gtt tcc ccg cat tcg gca gag cag att cgt cag cgg gct cag gat ttg   259
Val Ser Pro His Ser Ala Glu Gln Ile Arg Gln Arg Ala Gln Asp Leu
        40                  45                  50

gga tta acc ctg gat ctg ttc cag ccg ttt cga gat ttc gaa ggt gtg   307
Gly Leu Thr Leu Asp Leu Phe Gln Pro Phe Arg Asp Phe Glu Gly Val
    55                  60                  65

gaa gaa gag cag ttt ctg aag aat ctg cac cgc ttg gaa gag aag ttc   355
Glu Glu Glu Gln Phe Leu Lys Asn Leu His Arg Leu Glu Glu Lys Phe
70                  75                  80                  85

aag ctg atg aac agg ctt ggc att gag atg atc ttg ttg tgt tcc aat   403
Lys Leu Met Asn Arg Leu Gly Ile Glu Met Ile Leu Leu Cys Ser Asn
                90                  95                  100

gtg ggc acc gcg acc atc aat gat gat gac ctt ttc gtg gag cag ttg   451
Val Gly Thr Ala Thr Ile Asn Asp Asp Asp Leu Phe Val Glu Gln Leu
            105                 110                 115

cat cgt gca gca gat ttg gct gag aag tac aac gtc aag att gct tat   499
His Arg Ala Ala Asp Leu Ala Glu Lys Tyr Asn Val Lys Ile Ala Tyr
        120                 125                 130

gaa gcg ttg gcg tgg ggc aag ttt gtc aat gat ttt gag cat gcg cat   547
Glu Ala Leu Ala Trp Gly Lys Phe Val Asn Asp Phe Glu His Ala His
    135                 140                 145

gca ctt gtg gag aag gtg aat cac aag gcg ctg gga acc tgc ttg gat   595
Ala Leu Val Glu Lys Val Asn His Lys Ala Leu Gly Thr Cys Leu Asp
150                 155                 160                 165

acg ttc cat att ctt tcc cgt ggt tgg gaa acc gac gag gtg gag aac   643
Thr Phe His Ile Leu Ser Arg Gly Trp Glu Thr Asp Glu Val Glu Asn
                170                 175                 180

atc cct gcg gag aag atc ttc ttt gtt cag tta gcg gat gcg ccg aag   691
Ile Pro Ala Glu Lys Ile Phe Phe Val Gln Leu Ala Asp Ala Pro Lys
            185                 190                 195

ctg agc atg gac att ttg tcc tgg tcg cgt cac cac cgt gtt ttc cct   739
Leu Ser Met Asp Ile Leu Ser Trp Ser Arg His His Arg Val Phe Pro
        200                 205                 210

ggt gaa ggc gat ttc gat ctg gtg aaa ttc atg gtt cat ctg gcc aag   787
Gly Glu Gly Asp Phe Asp Leu Val Lys Phe Met Val His Leu Ala Lys
    215                 220                 225

acg ggt tat gat ggc ccg att tct ttg gag atc ttc aac gat tcc ttc    835
Thr Gly Tyr Asp Gly Pro Ile Ser Leu Glu Ile Phe Asn Asp Ser Phe
230                 235                 240                 245

cgc aag gcc gag gtt ggt cgc acc gcg att gat ggg ttg cgt tct ttg    883
Arg Lys Ala Glu Val Gly Arg Thr Ala Ile Asp Gly Leu Arg Ser Leu
                250                 255                 260

cgt tgg ttg gaa gat cag acc tgg cat gcg cta aat gct gag gat cgt    931
Arg Trp Leu Glu Asp Gln Thr Trp His Ala Leu Asn Ala Glu Asp Arg
            265                 270                 275

cca agc gct ctt gaa ctg cgt gca ctt cct gag gtc gcg gaa cct gag    979
Pro Ser Ala Leu Glu Leu Arg Ala Leu Pro Glu Val Ala Glu Pro Glu
        280                 285                 290

ggt gtt gat ttc att gag atc gcc act gga cgt ttg ggt gag acc att    1027
Gly Val Asp Phe Ile Glu Ile Ala Thr Gly Arg Leu Gly Glu Thr Ile
    295                 300                 305

cgg gtt ctt cat caa ttg ggt ttc cgc ttg ggt ggt cat cac tgc agt    1075
Arg Val Leu His Gln Leu Gly Phe Arg Leu Gly Gly His His Cys Ser
310                 315                 320                 325

aag cag gat tac cag gta tgg acc cag ggc gat gtg cgc att gtg gtg    1123
Lys Gln Asp Tyr Gln Val Trp Thr Gln Gly Asp Val Arg Ile Val Val
                330                 335                 340

tgt gat cgt ggg gtc acc ggg gct cca acc acg atc tct gcg atg ggc    1171
Cys Asp Arg Gly Val Thr Gly Ala Pro Thr Thr Ile Ser Ala Met Gly
            345                 350                 355

ttt gac acc ccc gat cca gaa gct gct cat gcc cgt gcg gaa ttg ctg    1219
Phe Asp Thr Pro Asp Pro Glu Ala Ala His Ala Arg Ala Glu Leu Leu
        360                 365                 370

cgg gct cag aca att gat cgt ccc cac atc gag ggc gaa gtt gac cta    1267
Arg Ala Gln Thr Ile Asp Arg Pro His Ile Glu Gly Glu Val Asp Leu
    375                 380                 385

aaa ggt gtg tac gca ccg gat ggg gtg gag ctg ttt ttc gcg ggg ccg    1315
Lys Gly Val Tyr Ala Pro Asp Gly Val Glu Leu Phe Phe Ala Gly Pro
390                 395                 400                 405

agc ccc gat gga atg ccc gag tgg ctg ccg gaa ttc ggc gtc gaa aag    1363
Ser Pro Asp Gly Met Pro Glu Trp Leu Pro Glu Phe Gly Val Glu Lys
                410                 415                 420

caa gaa gct ggt ctc att gaa gcc atc gac cac gtc aat ttc gcc cag    1411
Gln Glu Ala Gly Leu Ile Glu Ala Ile Asp His Val Asn Phe Ala Gln
            425                 430                 435

ccg tgg caa cat ttt gat gag gca gtg ctg ttt tac acc gcg ctg atg    1459
Pro Trp Gln His Phe Asp Glu Ala Val Leu Phe Tyr Thr Ala Leu Met
        440                 445                 450

gcg ttg gag act gtg cgt gag gat gag ttc ccg agc cca att ggt ttg    1507
Ala Leu Glu Thr Val Arg Glu Asp Glu Phe Pro Ser Pro Ile Gly Leu
    455                 460                 465

gtg cgc aat cag gtg atg cgt tcg ccg aat gat gcg gtg cgg ttg ctg    1555
Val Arg Asn Gln Val Met Arg Ser Pro Asn Asp Ala Val Arg Leu Leu
470                 475                 480                 485

ctc agc gtg gcg ccc gag gac ggt gag cag gga gat ttc ctc aac gcg    1603
Leu Ser Val Ala Pro Glu Asp Gly Glu Gln Gly Asp Phe Leu Asn Ala
                490                 495                 500

gcc tac ccg gag cac att gcg ttg gcc acg gcg gac atc gtg gcg gtg    1651
Ala Tyr Pro Glu His Ile Ala Leu Ala Thr Ala Asp Ile Val Ala Val
            505                 510                 515

gct gaa cgt gcg cgc aaa cga ggc ctg gat ttc ttg ccc gtc cca gag    1699
Ala Glu Arg Ala Arg Lys Arg Gly Leu Asp Phe Leu Pro Val Pro Glu
        520                 525                 530

aat tac tac gac gat gtg cag gcg cgt ttt gat ttg ccg cag gaa ttc    1747

Asn Tyr Tyr Asp Asp Val Gln Ala Arg Phe Asp Leu Pro Gln Glu Phe
    535                 540                 545

ttg gac aca ctc aag gaa aac cac ctg ctt tac gac cgc gac gag aac    1795
Leu Asp Thr Leu Lys Glu Asn His Leu Leu Tyr Asp Arg Asp Glu Asn
550                 555                 560                 565

ggc gaa ttc ctc cac ttt tac acc cgc acg ttg ggc acg ctg ttc ttc    1843
Gly Glu Phe Leu His Phe Tyr Thr Arg Thr Leu Gly Thr Leu Phe Phe
                570                 575                 580

gaa gtg gtg gaa cgc cgc ggc ggt ttt gca ggt tgg ggc gaa aca aac    1891
Glu Val Val Glu Arg Arg Gly Gly Phe Ala Gly Trp Gly Glu Thr Asn
            585                 590                 595

gct ccg gtg cgg ttg gcg gcg cag tat cgt gag gtg cgg gac ctc gag    1939
Ala Pro Val Arg Leu Ala Ala Gln Tyr Arg Glu Val Arg Asp Leu Glu
        600                 605                 610

cgg gga atc cca aac tagcatcccg aactagcccc cca                      1977
Arg Gly Ile Pro Asn
    615


<210> 396
<211> 618
<212> PRT
<213> Corynebacterium glutamicum

<400> 396
Met Arg Thr Ser Ile Ala Thr Val Cys Leu Ser Gly Thr Leu Ala Glu
  1               5                  10                  15

Lys Leu Arg Ala Ala Ala Asp Ala Gly Phe Asp Gly Val Glu Ile Phe
             20                  25                  30

Glu Gln Asp Leu Val Val Ser Pro His Ser Ala Glu Gln Ile Arg Gln
         35                  40                  45

Arg Ala Gln Asp Leu Gly Leu Thr Leu Asp Leu Phe Gln Pro Phe Arg
     50                  55                  60

Asp Phe Glu Gly Val Glu Glu Glu Gln Phe Leu Lys Asn Leu His Arg
 65                  70                  75                  80

Leu Glu Glu Lys Phe Lys Leu Met Asn Arg Leu Gly Ile Glu Met Ile
                 85                  90                  95

Leu Leu Cys Ser Asn Val Gly Thr Ala Thr Ile Asn Asp Asp Asp Leu
            100                 105                 110

Phe Val Glu Gln Leu His Arg Ala Ala Asp Leu Ala Glu Lys Tyr Asn
        115                 120                 125

Val Lys Ile Ala Tyr Glu Ala Leu Ala Trp Gly Lys Phe Val Asn Asp
    130                 135                 140

Phe Glu His Ala His Ala Leu Val Glu Lys Val Asn His Lys Ala Leu
145                 150                 155                 160

Gly Thr Cys Leu Asp Thr Phe His Ile Leu Ser Arg Gly Trp Glu Thr
                165                 170                 175

Asp Glu Val Glu Asn Ile Pro Ala Glu Lys Ile Phe Phe Val Gln Leu
            180                 185                 190

Ala Asp Ala Pro Lys Leu Ser Met Asp Ile Leu Ser Trp Ser Arg His
        195                 200                 205

His Arg Val Phe Pro Gly Glu Gly Asp Phe Asp Leu Val Lys Phe Met
    210                 215                 220

Val His Leu Ala Lys Thr Gly Tyr Asp Gly Pro Ile Ser Leu Glu Ile
225                 230                 235                 240

Phe Asn Asp Ser Phe Arg Lys Ala Glu Val Gly Arg Thr Ala Ile Asp
                245                 250                 255

Gly Leu Arg Ser Leu Arg Trp Leu Glu Asp Gln Thr Trp His Ala Leu
            260                 265                 270

Asn Ala Glu Asp Arg Pro Ser Ala Leu Glu Leu Arg Ala Leu Pro Glu
        275                 280                 285

Val Ala Glu Pro Glu Gly Val Asp Phe Ile Glu Ile Ala Thr Gly Arg
    290                 295                 300

Leu Gly Glu Thr Ile Arg Val Leu His Gln Leu Gly Phe Arg Leu Gly
305                 310                 315                 320

Gly His His Cys Ser Lys Gln Asp Tyr Gln Val Trp Thr Gln Gly Asp
                325                 330                 335

Val Arg Ile Val Val Cys Asp Arg Gly Val Thr Gly Ala Pro Thr Thr
            340                 345                 350

Ile Ser Ala Met Gly Phe Asp Thr Pro Asp Pro Glu Ala Ala His Ala
        355                 360                 365

Arg Ala Glu Leu Leu Arg Ala Gln Thr Ile Asp Arg Pro His Ile Glu
    370                 375                 380

Gly Glu Val Asp Leu Lys Gly Val Tyr Ala Pro Asp Gly Val Glu Leu
385                 390                 395                 400

Phe Phe Ala Gly Pro Ser Pro Asp Gly Met Pro Glu Trp Leu Pro Glu
                405                 410                 415

Phe Gly Val Glu Lys Gln Glu Ala Gly Leu Ile Glu Ala Ile Asp His
            420                 425                 430

Val Asn Phe Ala Gln Pro Trp Gln His Phe Asp Glu Ala Val Leu Phe
        435                 440                 445

Tyr Thr Ala Leu Met Ala Leu Glu Thr Val Arg Glu Asp Glu Phe Pro
    450                 455                 460

Ser Pro Ile Gly Leu Val Arg Asn Gln Val Met Arg Ser Pro Asn Asp
465                 470                 475                 480

Ala Val Arg Leu Leu Leu Ser Val Ala Pro Glu Asp Gly Glu Gln Gly
                485                 490                 495

Asp Phe Leu Asn Ala Ala Tyr Pro Glu His Ile Ala Leu Ala Thr Ala
            500                 505                 510

Asp Ile Val Ala Val Ala Glu Arg Ala Arg Lys Arg Gly Leu Asp Phe
        515                 520                 525

Leu Pro Val Pro Glu Asn Tyr Tyr Asp Asp Val Gln Ala Arg Phe Asp
    530                 535                 540

Leu Pro Gln Glu Phe Leu Asp Thr Leu Lys Glu Asn His Leu Leu Tyr
545                 550                 555                 560

Asp Arg Asp Glu Asn Gly Glu Phe Leu His Phe Tyr Thr Arg Thr Leu
                565                 570                 575

Gly Thr Leu Phe Phe Glu Val Val Glu Arg Arg Gly Gly Phe Ala Gly
            580                 585                 590

Trp Gly Glu Thr Asn Ala Pro Val Arg Leu Ala Ala Gln Tyr Arg Glu
        595                 600                 605

Val Arg Asp Leu Glu Arg Gly Ile Pro Asn
    610                 615

<210> 397
<211> 470
<212> DNA
<213> Corynebacterium glutamicum

<220>

<221> CDS
<222> (1)..(447)
<223> RXN02839

<400> 397
tgt gtg gtg aat gat tat gct gac cgc aag ttt gat ggt cat gtt aag     48
Cys Val Val Asn Asp Tyr Ala Asp Arg Lys Phe Asp Gly His Val Lys
  1               5                  10                  15

cgc acg gcg aac cga cca ctt ccc agc ggc gcg gta aca gag aaa gag     96
Arg Thr Ala Asn Arg Pro Leu Pro Ser Gly Ala Val Thr Glu Lys Glu
             20                  25                  30

gcg cgc gcg ctg ttt gtc gtg ctg gta ctg att tcg ttt tta ctg gtg    144
Ala Arg Ala Leu Phe Val Val Leu Val Leu Ile Ser Phe Leu Leu Val
         35                  40                  45

ctg acg ctg aat acg atg acc att ctg ttg tcg att gcc gcg cta gcg    192
Leu Thr Leu Asn Thr Met Thr Ile Leu Leu Ser Ile Ala Ala Leu Ala
     50                  55                  60

ctg gcg tgg gtg tac ccg ttt atg aag cgg tat acc cat cta ccg caa    240
Leu Ala Trp Val Tyr Pro Phe Met Lys Arg Tyr Thr His Leu Pro Gln
 65                  70                  75                  80

gtg gtg ctg ggc gcg gcg ttt ggc tgg tcg att cca atg gct ttt gcc    288
Val Val Leu Gly Ala Ala Phe Gly Trp Ser Ile Pro Met Ala Phe Ala
                 85                  90                  95

gct gtg agt gag tcg gtg cca ttg agt tgc tgg tta atg ttc ctc gcc    336
Ala Val Ser Glu Ser Val Pro Leu Ser Cys Trp Leu Met Phe Leu Ala
            100                 105                 110

aat att ctc tgg gcg gtg gct tac gac acg cag tat gcg atg gtt gac    384
Asn Ile Leu Trp Ala Val Ala Tyr Asp Thr Gln Tyr Ala Met Val Asp
        115                 120                 125

cgc gat gat gat gtg aag att ggc att aaa tcc acg gca atc ctg ttg    432
Arg Asp Asp Asp Val Lys Ile Gly Ile Lys Ser Thr Ala Ile Leu Leu
    130                 135                 140

gcc aat acg ata aat tgatattggg attttgcaga ttg                     470
Ala Asn Thr Ile Asn
145


<210> 398
<211> 149
<212> PRT
<213> Corynebacterium glutamicum

<400> 398
Cys Val Val Asn Asp Tyr Ala Asp Arg Lys Phe Asp Gly His Val Lys
  1               5                  10                  15

Arg Thr Ala Asn Arg Pro Leu Pro Ser Gly Ala Val Thr Glu Lys Glu
             20                  25                  30

Ala Arg Ala Leu Phe Val Val Leu Val Leu Ile Ser Phe Leu Leu Val
         35                  40                  45

Leu Thr Leu Asn Thr Met Thr Ile Leu Leu Ser Ile Ala Ala Leu Ala
     50                  55                  60

Leu Ala Trp Val Tyr Pro Phe Met Lys Arg Tyr Thr His Leu Pro Gln
 65                  70                  75                  80

Val Val Leu Gly Ala Ala Phe Gly Trp Ser Ile Pro Met Ala Phe Ala
                 85                  90                  95

Ala Val Ser Glu Ser Val Pro Leu Ser Cys Trp Leu Met Phe Leu Ala
            100                 105                 110

Asn Ile Leu Trp Ala Val Ala Tyr Asp Thr Gln Tyr Ala Met Val Asp
        115                 120                 125

Arg Asp Asp Asp Val Lys Ile Gly Ile Lys Ser Thr Ala Ile Leu Leu

130                 135                 140

Ala Asn Thr Ile Asn
145


<210> 399
<211> 978
<212> DNA
<213> Corynebacterium glutamicum

<220>
<221> CDS
<222> (101)..(955)
<223> RXN00639

<400> 399
agtgttgtta tcgagttcag ccgatcacaa agatttttcc gctaggcagt gatccgactc 60

gcacccccta cttcaccccc aaagtctcta ggagtatgac atg act tca gct gaa   115
                                           Met Thr Ser Ala Glu
                                           1               5

cag atc gtt gat cca aca gcc cac gat tcg ggc aac aag gca act gac   163
Gln Ile Val Asp Pro Thr Ala His Asp Ser Gly Asn Lys Ala Thr Asp
                10                  15                  20

aag ttc aag gca aac cgc gtt tcc tcc gat acc tcc aag gaa cgc gca   211
Lys Phe Lys Ala Asn Arg Val Ser Ser Asp Thr Ser Lys Glu Arg Ala
            25                  30                  35

aac gcg atc tac gta gat ctg ctc gcg gcg atc gcc cag gtt gct cac   259
Asn Ala Ile Tyr Val Asp Leu Leu Ala Ala Ile Ala Gln Val Ala His
        40                  45                  50

aag cac gaa gtc acc tac gaa gag tac gca gtg ctc aag cag tgg atg   307
Lys His Glu Val Thr Tyr Glu Glu Tyr Ala Val Leu Lys Gln Trp Met
    55                  60                  65

atc gac gtt gga gaa tac ggc gag tgg cca ctg tgg ttg gac gtt ttc   355
Ile Asp Val Gly Glu Tyr Gly Glu Trp Pro Leu Trp Leu Asp Val Phe
70                  75                  80                  85

gtt gag cat gag atc gaa gag atc aac tac aac cgc cac gac tac acc   403
Val Glu His Glu Ile Glu Glu Ile Asn Tyr Asn Arg His Asp Tyr Thr
                90                  95                  100

gga acc aag ggt tcc atc gaa ggc cct tat tac gta gag aac tct ccg   451
Gly Thr Lys Gly Ser Ile Glu Gly Pro Tyr Tyr Val Glu Asn Ser Pro
            105                 110                 115

aag ctc cct tgg gat gct gaa atg cca atg cgt gac aag gac cgc gca   499
Lys Leu Pro Trp Asp Ala Glu Met Pro Met Arg Asp Lys Asp Arg Ala
        120                 125                 130

tgc acc cca ctg atc ttc gag ggg cag gtt act gac ctc gac ggc aac   547
Cys Thr Pro Leu Ile Phe Glu Gly Gln Val Thr Asp Leu Asp Gly Asn
    135                 140                 145

ggt ctt gat gga gca gaa gtt gag ctc tgg cac gca gat gag gac gga   595
Gly Leu Asp Gly Ala Glu Val Glu Leu Trp His Ala Asp Glu Asp Gly
150                 155                 160                 165

tac tac tcc cag ttc gcg cct gga atc cca gag tgg aac ctg cgt ggc   643
Tyr Tyr Ser Gln Phe Ala Pro Gly Ile Pro Glu Trp Asn Leu Arg Gly
                170                 175                 180

acc atc gtt acc gat gag gaa ggc cgc tac aag atc aag acc ctg cag   691
Thr Ile Val Thr Asp Glu Glu Gly Arg Tyr Lys Ile Lys Thr Leu Gln
            185                 190                 195

cct gcg cct tac cag atc cct cat gat ggc cca acc ggt tgg ttc att   739
Pro Ala Pro Tyr Gln Ile Pro His Asp Gly Pro Thr Gly Trp Phe Ile
        200                 205                 210

gag tct tac ggt ggg cac cca tgg cgc cca gcc cac ctc cac ttg cgc   787
Glu Ser Tyr Gly Gly His Pro Trp Arg Pro Ala His Leu His Leu Arg

215                 220                 225

gtt tcc cac ccg ggc tac cgc acc atc acc acc cag ctt tac ttc gag     835
Val Ser His Pro Gly Tyr Arg Thr Ile Thr Thr Gln Leu Tyr Phe Glu
230                 235                 240                 245

ggt ggc gag tgg gtc gaa aac gac gtt gca acc gct gtg aag cca gaa     883
Gly Gly Glu Trp Val Glu Asn Asp Val Ala Thr Ala Val Lys Pro Glu
                250                 255                 260

ctg gtc ctg cac cct gag act ggc gag gat ggt aac cac gtt cac tac     931
Leu Val Leu His Pro Glu Thr Gly Glu Asp Gly Asn His Val His Tyr
            265                 270                 275

cca ttc gtc ctg gat aag gaa gac tagtttttct acctagctag cat           978
Pro Phe Val Leu Asp Lys Glu Asp
        280                 285


<210> 400
<211> 285
<212> PRT
<213> Corynebacterium glutamicum

<400> 400
Met Thr Ser Ala Glu Gln Ile Val Asp Pro Thr Ala His Asp Ser Gly
  1               5                  10                  15

Asn Lys Ala Thr Asp Lys Phe Lys Ala Asn Arg Val Ser Ser Asp Thr
             20                  25                  30

Ser Lys Glu Arg Ala Asn Ala Ile Tyr Val Asp Leu Leu Ala Ala Ile
         35                  40                  45

Ala Gln Val Ala His Lys His Glu Val Thr Tyr Glu Glu Tyr Ala Val
     50                  55                  60

Leu Lys Gln Trp Met Ile Asp Val Gly Glu Tyr Gly Glu Trp Pro Leu
 65                  70                  75                  80

Trp Leu Asp Val Phe Val Glu His Glu Ile Glu Glu Ile Asn Tyr Asn
                 85                  90                  95

Arg His Asp Tyr Thr Gly Thr Lys Gly Ser Ile Glu Gly Pro Tyr Tyr
            100                 105                 110

Val Glu Asn Ser Pro Lys Leu Pro Trp Asp Ala Glu Met Pro Met Arg
        115                 120                 125

Asp Lys Asp Arg Ala Cys Thr Pro Leu Ile Phe Glu Gly Gln Val Thr
    130                 135                 140

Asp Leu Asp Gly Asn Gly Leu Asp Gly Ala Glu Val Glu Leu Trp His
145                 150                 155                 160

Ala Asp Glu Asp Gly Tyr Tyr Ser Gln Phe Ala Pro Gly Ile Pro Glu
                165                 170                 175

Trp Asn Leu Arg Gly Thr Ile Val Thr Asp Glu Glu Gly Arg Tyr Lys
            180                 185                 190

Ile Lys Thr Leu Gln Pro Ala Pro Tyr Gln Ile Pro His Asp Gly Pro
        195                 200                 205

Thr Gly Trp Phe Ile Glu Ser Tyr Gly Gly His Pro Trp Arg Pro Ala
    210                 215                 220

His Leu His Leu Arg Val Ser His Pro Gly Tyr Arg Thr Ile Thr Thr
225                 230                 235                 240

Gln Leu Tyr Phe Glu Gly Gly Glu Trp Val Glu Asn Asp Val Ala Thr
                245                 250                 255

Ala Val Lys Pro Glu Leu Val Leu His Pro Glu Thr Gly Glu Asp Gly
            260                 265                 270

Asn His Val His Tyr Pro Phe Val Leu Asp Lys Glu Asp

275                 280                 285

<210> 401
<211> 780
<212> DNA
<213> Corynebacterium glutamicum

<220>
<221> CDS
<222> (101)..(757)
<223> RXN02530

<400> 401
gaggctgagc tttccgtcaa acaacatctt cccaatgtgg atgaaatgac tgtgaccatc 60

accccttcca aaccttgagt cccgtgatac aattgttgat atg tca aca aat tat   115
                                           Met Ser Thr Asn Tyr
                                           1               5

gaa gca atc atc att gga gca ggt cag gct gga ctc gcg gcg gcg cat   163
Glu Ala Ile Ile Ile Gly Ala Gly Gln Ala Gly Leu Ala Ala Ala His
                10                  15                  20

gaa ctt tcc cgc cgc ggt ttc act ccc gga aaa gat ttt ctc gtc ctc   211
Glu Leu Ser Arg Arg Gly Phe Thr Pro Gly Lys Asp Phe Leu Val Leu
            25                  30                  35

gat tcc aac gac ggg ccc ggt ggc gcc tgg cgg cat agg tgg gat tca   259
Asp Ser Asn Asp Gly Pro Gly Gly Ala Trp Arg His Arg Trp Asp Ser
        40                  45                  50

ctc aca tta ggt aaa gcc cac gga atc gcc gat ctc cca ggg ctt ccc   307
Leu Thr Leu Gly Lys Ala His Gly Ile Ala Asp Leu Pro Gly Leu Pro
    55                  60                  65

atg aat cgc ccc gat ccg aaa act ccg gct tcc aca ttg gtt gct ggt   355
Met Asn Arg Pro Asp Pro Lys Thr Pro Ala Ser Thr Leu Val Ala Gly
70                  75                  80                  85

tat tac ggc gct tac gag aac gag ttc tcc ttc gca gtt gtg cgc cca   403
Tyr Tyr Gly Ala Tyr Glu Asn Glu Phe Ser Phe Ala Val Val Arg Pro
                90                  95                  100

gtc aaa gtc tca cga gtt gag ccc act tcc gag gat cct tcg agc cca   451
Val Lys Val Ser Arg Val Glu Pro Thr Ser Glu Asp Pro Ser Ser Pro
            105                 110                 115

ttg cgc gtg agc agc gac gat ggt cga gag tgg att acc cgc atg gtt   499
Leu Arg Val Ser Ser Asp Asp Gly Arg Glu Trp Ile Thr Arg Met Val
        120                 125                 130

ctt aat gca aca ggt acg tgg aca aac cct tat gtt ccg tac att cct   547
Leu Asn Ala Thr Gly Thr Trp Thr Asn Pro Tyr Val Pro Tyr Ile Pro
    135                 140                 145

ggc atc gat aaa ttc cag ggc aag cag ctc cac acc gtt aat tac cgc   595
Gly Ile Asp Lys Phe Gln Gly Lys Gln Leu His Thr Val Asn Tyr Arg
150                 155                 160                 165

aag gcc gag gat ttc aaa ggt aag aaa gtc ctg gtc gtc ggc ggt ggt   643
Lys Ala Glu Asp Phe Lys Gly Lys Lys Val Leu Val Val Gly Gly Gly
                170                 175                 180

ttg agt gct gtg caa ttt ctg ctg gag ttg gaa ggc ttg gcg gaa acc   691
Leu Ser Ala Val Gln Phe Leu Leu Glu Leu Glu Gly Leu Ala Glu Thr
            185                 190                 195

acc tgg gcg acg cgt cgt ccg cga act tac gca gcg cga gtt cga cgc   739
Thr Trp Ala Thr Arg Arg Pro Arg Thr Tyr Ala Ala Arg Val Arg Arg
        200                 205                 210

cgg ctg ggg cat tgc ggt tgagcgcgcc gtccgcgaac gca                 780
Arg Leu Gly His Cys Gly
    215

<210> 402
<211> 219
<212> PRT
<213> Corynebacterium glutamicum

<400> 402
Met Ser Thr Asn Tyr Glu Ala Ile Ile Ile Gly Ala Gly Gln Ala Gly
1               5                   10                  15

Leu Ala Ala Ala His Glu Leu Ser Arg Arg Gly Phe Thr Pro Gly Lys
            20                  25                  30

Asp Phe Leu Val Leu Asp Ser Asn Asp Gly Pro Gly Gly Ala Trp Arg
        35                  40                  45

His Arg Trp Asp Ser Leu Thr Leu Gly Lys Ala His Gly Ile Ala Asp
    50                  55                  60

Leu Pro Gly Leu Pro Met Asn Arg Pro Asp Pro Lys Thr Pro Ala Ser
65                  70                  75                  80

Thr Leu Val Ala Gly Tyr Tyr Gly Ala Tyr Glu Asn Glu Phe Ser Phe
                85                  90                  95

Ala Val Val Arg Pro Val Lys Val Ser Arg Val Glu Pro Thr Ser Glu
            100                 105                 110

Asp Pro Ser Ser Pro Leu Arg Val Ser Ser Asp Asp Gly Arg Glu Trp
        115                 120                 125

Ile Thr Arg Met Val Leu Asn Ala Thr Gly Thr Trp Thr Asn Pro Tyr
    130                 135                 140

Val Pro Tyr Ile Pro Gly Ile Asp Lys Phe Gln Gly Lys Gln Leu His
145                 150                 155                 160

Thr Val Asn Tyr Arg Lys Ala Glu Asp Phe Lys Gly Lys Lys Val Leu
                165                 170                 175

Val Val Gly Gly Gly Leu Ser Ala Val Gln Phe Leu Leu Glu Leu Glu
            180                 185                 190

Gly Leu Ala Glu Thr Thr Trp Ala Thr Arg Arg Pro Arg Thr Tyr Ala
        195                 200                 205

Ala Arg Val Arg Arg Arg Leu Gly His Cys Gly
    210                 215


<210> 403
<211> 990
<212> DNA
<213> Corynebacterium glutamicum

<220>
<221> CDS
<222> (101)..(967)
<223> RXN00434

<400> 403
ccacgcgcca aaccatgcaa gccatcgtcc aaactgagga gaaggtcact gcttctctgg 60

aattacagga agtccccgtc ccgaccctga agccaggtag gtg ctg gtg aag gtg 115
                                           Val Leu Val Lys Val
                                           1               5

aag cct gcg ggc gtt aac cgt gcg gat ctt ttg cag acg cag gga aat 163
Lys Pro Ala Gly Val Asn Arg Ala Asp Leu Leu Gln Thr Gln Gly Asn
                10                  15                  20

tat cct gtg ccg gcg ggg gct tcg gag att ctc ggg ctg gaa tgt gcg 211
Tyr Pro Val Pro Ala Gly Ala Ser Glu Ile Leu Gly Leu Glu Cys Ala
            25                  30                  35

ggt gtg atc gtg aat gcc ggc gac act ggg caa aca gtg ggt cag gaa 259
Gly Val Ile Val Asn Ala Gly Asp Thr Gly Gln Thr Val Gly Gln Glu
        40                  45                  50

gtc gct tgc ctt ctc act ggc ggt gga tat gcg caa tat gtg gcg gtt    307
Val Ala Cys Leu Leu Thr Gly Gly Gly Tyr Ala Gln Tyr Val Ala Val
    55                  60                  65

ccg gaa ggt cag ttg atg cca att cca gag ggt tac agc ttt gtg gaa    355
Pro Glu Gly Gln Leu Met Pro Ile Pro Glu Gly Tyr Ser Phe Val Glu
70                  75                  80                  85

gcg gcc tcg atc gtg gag gtt gcg tgc acg gtg tgg tcg aat atc ggc    403
Ala Ala Ser Ile Val Glu Val Ala Cys Thr Val Trp Ser Asn Ile Gly
                90                  95                  100

atg ctg gcg ggc ttg cag aag gag gat act ttc ctt att cat ggt ggc    451
Met Leu Ala Gly Leu Gln Lys Glu Asp Thr Phe Leu Ile His Gly Gly
            105                 110                 115

gcg ggc ggt atc gga acg ttt gcc att cag atg ggc aag gct ctg ggt    499
Ala Gly Gly Ile Gly Thr Phe Ala Ile Gln Met Gly Lys Ala Leu Gly
        120                 125                 130

gtg acg gtt gcg gtg act gcc ggt tca act gaa aag tta aaa acc tgt    547
Val Thr Val Ala Val Thr Ala Gly Ser Thr Glu Lys Leu Lys Thr Cys
    135                 140                 145

aag aac tta ggg gcc gat atc ctc atc aat tac aag gag gaa gat ttc    595
Lys Asn Leu Gly Ala Asp Ile Leu Ile Asn Tyr Lys Glu Glu Asp Phe
150                 155                 160                 165

gcc gag gtt ttg aag aac aag gcg gat gtc att ctc gat att att ggt    643
Ala Glu Val Leu Lys Asn Lys Ala Asp Val Ile Leu Asp Ile Ile Gly
                170                 175                 180

gcg aag tat ttg tca cag aat gtg aag gcg atg gcc aag gac gcg cac    691
Ala Lys Tyr Leu Ser Gln Asn Val Lys Ala Met Ala Lys Asp Ala His
            185                 190                 195

atg gta gtc atc ggg atg cag ggt ggc gtg aaa ggg gag ctg aat ttg    739
Met Val Val Ile Gly Met Gln Gly Gly Val Lys Gly Glu Leu Asn Leu
        200                 205                 210

ggt cat ctt ttg gcc aag cga ggc acg att tct gcc act gcg ctg cgt    787
Gly His Leu Leu Ala Lys Arg Gly Thr Ile Ser Ala Thr Ala Leu Arg
    215                 220                 225

ggt cgc gat gag gcg gat aag gct cgg att gtc agc agc act gtc gaa    835
Gly Arg Asp Glu Ala Asp Lys Ala Arg Ile Val Ser Ser Thr Val Glu
230                 235                 240                 245

aat att tgg ccg ctg ctg caa tcg aag gaa att acc cct cac atc gac    883
Asn Ile Trp Pro Leu Leu Gln Ser Lys Glu Ile Thr Pro His Ile Asp
                250                 255                 260

cac acc ttg ccg cta gcc gaa gca gcc gcc gcc ttg cag aaa att caa    931
His Thr Leu Pro Leu Ala Glu Ala Ala Ala Ala Leu Gln Lys Ile Gln
            265                 270                 275

gac ggc acc atc acc ggc aag ctc gtg ctt gcg gtt taggcaagcg         977
Asp Gly Thr Ile Thr Gly Lys Leu Val Leu Ala Val
        280                 285

atgccagcac cct                                                     990


<210> 404
<211> 289
<212> PRT
<213> Corynebacterium glutamicum

<400> 404
Val Leu Val Lys Val Lys Pro Ala Gly Val Asn Arg Ala Asp Leu Leu
1               5                   10                  15

Gln Thr Gln Gly Asn Tyr Pro Val Pro Ala Gly Ala Ser Glu Ile Leu
            20                  25                  30

Gly Leu Glu Cys Ala Gly Val Ile Val Asn Ala Gly Asp Thr Gly Gln

35                  40                  45

Thr Val Gly Gln Glu Val Ala Cys Leu Leu Thr Gly Gly Gly Tyr Ala
    50                  55                  60

Gln Tyr Val Ala Val Pro Glu Gly Gln Leu Met Pro Ile Pro Glu Gly
65                  70                  75                  80

Tyr Ser Phe Val Glu Ala Ala Ser Ile Val Glu Val Ala Cys Thr Val
                85                  90                  95

Trp Ser Asn Ile Gly Met Leu Ala Gly Leu Gln Lys Glu Asp Thr Phe
            100                 105                 110

Leu Ile His Gly Gly Ala Gly Gly Ile Gly Thr Phe Ala Ile Gln Met
        115                 120                 125

Gly Lys Ala Leu Gly Val Thr Val Ala Val Thr Ala Gly Ser Thr Glu
    130                 135                 140

Lys Leu Lys Thr Cys Lys Asn Leu Gly Ala Asp Ile Leu Ile Asn Tyr
145                 150                 155                 160

Lys Glu Glu Asp Phe Ala Glu Val Leu Lys Asn Lys Ala Asp Val Ile
                165                 170                 175

Leu Asp Ile Ile Gly Ala Lys Tyr Leu Ser Gln Asn Val Lys Ala Met
            180                 185                 190

Ala Lys Asp Ala His Met Val Val Ile Gly Met Gln Gly Gly Val Lys
        195                 200                 205

Gly Glu Leu Asn Leu Gly His Leu Leu Ala Lys Arg Gly Thr Ile Ser
    210                 215                 220

Ala Thr Ala Leu Arg Gly Arg Asp Glu Ala Asp Lys Ala Arg Ile Val
225                 230                 235                 240

Ser Ser Thr Val Glu Asn Ile Trp Pro Leu Leu Gln Ser Lys Glu Ile
                245                 250                 255

Thr Pro His Ile Asp His Thr Leu Pro Leu Ala Glu Ala Ala Ala Ala
            260                 265                 270

Leu Gln Lys Ile Gln Asp Gly Thr Ile Thr Gly Lys Leu Val Leu Ala
        275                 280                 285

Val

<210> 405
<211> 1098
<212> DNA
<213> Corynebacterium glutamicum

<220>
<221> CDS
<222> (101)..(1075)
<223> RXN01619

<400> 405
cctgcaagtt tactgctcgg ccgtcacggg ggaatggaaa aagtacgctt ggtgttcata 60

tagcgaaccc attttctatt gcgatgagag gaacaccacc atg cgc gca atc act   115
                                            Met Arg Ala Ile Thr
                                            1               5

cac aac act ttc ggc gac ccc gcc gac gtc cta cag att acc gag aag   163
His Asn Thr Phe Gly Asp Pro Ala Asp Val Leu Gln Ile Thr Glu Lys
                10                  15                  20

gaa att ccc act ccc ggc cca ggt cag gtt cgt att caa gtg acg ctg   211
Glu Ile Pro Thr Pro Gly Pro Gly Gln Val Arg Ile Gln Val Thr Leu
            25                  30                  35

gca acc atc cac aac cat gat ttg tgg acc gtg aag ggc tct tac ggc   259

Ala Thr Ile His Asn His Asp Leu Trp Thr Val Lys Gly Ser Tyr Gly
        40                  45                  50

ttc gtc cca gat ctg ccg gcc gcc gca ggc acc gag gca gtc ggc atc     307
Phe Val Pro Asp Leu Pro Ala Ala Ala Gly Thr Glu Ala Val Gly Ile
    55                  60                  65

gtc gac gcc ctg ggc gag ggc gtc gaa ggt ttg cag gtc ggt cag cgt     355
Val Asp Ala Leu Gly Glu Gly Val Glu Gly Leu Gln Val Gly Gln Arg
 70                  75                  80                  85

gtt gcg tcc ggc acc agc ttt ggc atc tgg gcg gag tac gcg ctt gtc     403
Val Ala Ser Gly Thr Ser Phe Gly Ile Trp Ala Glu Tyr Ala Leu Val
                 90                  95                 100

gac gcc tcc ggc ctc att ccc gta cca gaa cag ctc tcc gac gaa agc     451
Asp Ala Ser Gly Leu Ile Pro Val Pro Glu Gln Leu Ser Asp Glu Ser
            105                 110                 115

gca gct cag ctc gtc gca atg cct ttc agc gcc atc agc ctt ctt gat     499
Ala Ala Gln Leu Val Ala Met Pro Phe Ser Ala Ile Ser Leu Leu Asp
        120                 125                 130

ttc ctg gat atg aaa cca ggg gag tgg ctg atc caa aac tcc gca aac     547
Phe Leu Asp Met Lys Pro Gly Glu Trp Leu Ile Gln Asn Ser Ala Asn
    135                 140                 145

ggt gcc gtc ggc cgc atg ctc gca cag ctg gca gaa tcc cgc ggc atc     595
Gly Ala Val Gly Arg Met Leu Ala Gln Leu Ala Glu Ser Arg Gly Ile
150                 155                 160                 165

cat gtc gtt ggt ctc gtc cgc cgt gac gcc ggt gtc caa gaa ctc gct     643
His Val Val Gly Leu Val Arg Arg Asp Ala Gly Val Gln Glu Leu Ala
                170                 175                 180

gct caa aac atc agc ggc gtc gtt tcc act gag acc cca ggc tgg gaa     691
Ala Gln Asn Ile Ser Gly Val Val Ser Thr Glu Thr Pro Gly Trp Glu
            185                 190                 195

aag cag gtc gaa gac atc acc ggt ggc gca agc atc gcc gtc gca ctt     739
Lys Gln Val Glu Asp Ile Thr Gly Gly Ala Ser Ile Ala Val Ala Leu
        200                 205                 210

gat tcc gtc ggt gga tcc tcc gca gct gac ctg gtg aaa ctg ctt ggc     787
Asp Ser Val Gly Gly Ser Ser Ala Ala Asp Leu Val Lys Leu Leu Gly
    215                 220                 225

gaa ggc ggc acc ctc gtc tcc ttc ggc gcc atg ggc aac cca atc atg     835
Glu Gly Gly Thr Leu Val Ser Phe Gly Ala Met Gly Asn Pro Ile Met
230                 235                 240                 245

gaa atc cca tcc ggc ccc gtc atc ttc aag cac atc acc gtc aag ggc     883
Glu Ile Pro Ser Gly Pro Val Ile Phe Lys His Ile Thr Val Lys Gly
                250                 255                 260

ttc tgg gga agc aaa gtc agc cgc gaa atg cca gca gag aag aaa acc     931
Phe Trp Gly Ser Lys Val Ser Arg Glu Met Pro Ala Glu Lys Lys Thr
            265                 270                 275

cag ttg ttc ggc gag ctc att gcg cgc ata ctt gat gga aca ttg acc     979
Gln Leu Phe Gly Glu Leu Ile Ala Arg Ile Leu Asp Gly Thr Leu Thr
        280                 285                 290

ctt cca gtt gat tcc acc ttt gat gcc gct gac atc gtc tcg gcc gtg    1027
Leu Pro Val Asp Ser Thr Phe Asp Ala Ala Asp Ile Val Ser Ala Val
    295                 300                 305

cgc gcc tcc agc gag cct ggc cgt gcc gga aaa gtg ctc att cgt ttc    1075
Arg Ala Ser Ser Glu Pro Gly Arg Ala Gly Lys Val Leu Ile Arg Phe
310                 315                 320                 325

taaacgttta aggcccatta gac                                          1098

<210> 406
<211> 325
<212> PRT

<213> Corynebacterium glutamicum

<400> 406
Met Arg Ala Ile Thr His Asn Thr Phe Gly Asp Pro Ala Asp Val Leu
1               5                   10                  15

Gln Ile Thr Glu Lys Glu Ile Pro Thr Pro Gly Pro Gly Gln Val Arg
            20                  25                  30

Ile Gln Val Thr Leu Ala Thr Ile His Asn His Asp Leu Trp Thr Val
        35                  40                  45

Lys Gly Ser Tyr Gly Phe Val Pro Asp Leu Pro Ala Ala Ala Gly Thr
    50                  55                  60

Glu Ala Val Gly Ile Val Asp Ala Leu Gly Glu Gly Val Glu Gly Leu
65                  70                  75                  80

Gln Val Gly Gln Arg Val Ala Ser Gly Thr Ser Phe Gly Ile Trp Ala
                85                  90                  95

Glu Tyr Ala Leu Val Asp Ala Ser Gly Leu Ile Pro Val Pro Glu Gln
            100                 105                 110

Leu Ser Asp Glu Ser Ala Ala Gln Leu Val Ala Met Pro Phe Ser Ala
        115                 120                 125

Ile Ser Leu Leu Asp Phe Leu Asp Met Lys Pro Gly Glu Trp Leu Ile
    130                 135                 140

Gln Asn Ser Ala Asn Gly Ala Val Gly Arg Met Leu Ala Gln Leu Ala
145                 150                 155                 160

Glu Ser Arg Gly Ile His Val Val Gly Leu Val Arg Arg Asp Ala Gly
                165                 170                 175

Val Gln Glu Leu Ala Ala Gln Asn Ile Ser Gly Val Val Ser Thr Glu
            180                 185                 190

Thr Pro Gly Trp Glu Lys Gln Val Glu Asp Ile Thr Gly Gly Ala Ser
        195                 200                 205

Ile Ala Val Ala Leu Asp Ser Val Gly Gly Ser Ser Ala Ala Asp Leu
    210                 215                 220

Val Lys Leu Leu Gly Glu Gly Gly Thr Leu Val Ser Phe Gly Ala Met
225                 230                 235                 240

Gly Asn Pro Ile Met Glu Ile Pro Ser Gly Pro Val Ile Phe Lys His
                245                 250                 255

Ile Thr Val Lys Gly Phe Trp Gly Ser Lys Val Ser Arg Glu Met Pro
            260                 265                 270

Ala Glu Lys Lys Thr Gln Leu Phe Gly Glu Leu Ile Ala Arg Ile Leu
        275                 280                 285

Asp Gly Thr Leu Thr Leu Pro Val Asp Ser Thr Phe Asp Ala Ala Asp
    290                 295                 300

Ile Val Ser Ala Val Arg Ala Ser Ser Glu Pro Gly Arg Ala Gly Lys
305                 310                 315                 320

Val Leu Ile Arg Phe
                325


<210> 407
<211> 1041
<212> DNA
<213> Corynebacterium glutamicum

<220>
<221> CDS
<222> (101)..(1018)
<223> RXN01842

<400> 407
cacgtcatgg tctcgcggtg atcatcttcc taccgtgaca ggcaaggccg caaacggccg 60

tgaccacagg aaagaaattc acgaggagag gaagcacacg atg tcg aag gta tac 115
                                            Met Ser Lys Val Tyr
                                            1               5

gtg tcc aac gag tac ggc ggc ccg gaa aac cag gaa ctg atc acc cgc 163
Val Ser Asn Glu Tyr Gly Gly Pro Glu Asn Gln Glu Leu Ile Thr Arg
                10                  15                  20

aac acc ccc cag cca ggc ccg gga gaa ctc ggg gtc aag gtc cac gcg 211
Asn Thr Pro Gln Pro Gly Pro Gly Glu Leu Gly Val Lys Val His Ala
            25                  30                  35

gcc ggg gtc aac ccg ctt gat tgg aag gtc cgt tcc ggg gtt gcc gga 259
Ala Gly Val Asn Pro Leu Asp Trp Lys Val Arg Ser Gly Val Ala Gly
        40                  45                  50

acc ccg cga gag ctt ccg gca ccc ctg ggc gag gag gcc tcc ggg atc 307
Thr Pro Arg Glu Leu Pro Ala Pro Leu Gly Glu Glu Ala Ser Gly Ile
    55                  60                  65

gtc acc gcc gtt gga gac ggt gtg gag ggc ttc gcg gtc ggc gat ccg 355
Val Thr Ala Val Gly Asp Gly Val Glu Gly Phe Ala Val Gly Asp Pro
70                  75                  80                  85

gtg ctc ggc ctg gtg gcc ccc ggc gtc ggc gga tat gcc gag gac acc 403
Val Leu Gly Leu Val Ala Pro Gly Val Gly Gly Tyr Ala Glu Asp Thr
                90                  95                  100

ctg ctg gtg gca gag agt acc gtg cta aag ccg gag gag atc tcg ttc 451
Leu Leu Val Ala Glu Ser Thr Val Leu Lys Pro Glu Glu Ile Ser Phe
            105                 110                 115

acc gac gcc gcc gcg atc ccg gtc gct ggg gcg agc gcc tac gcc ggc 499
Thr Asp Ala Ala Ala Ile Pro Val Ala Gly Ala Ser Ala Tyr Ala Gly
        120                 125                 130

act cac cag gtc gag ctt gaa cca ggc cag tcg ttg ctg atc aat ggg 547
Thr His Gln Val Glu Leu Glu Pro Gly Gln Ser Leu Leu Ile Asn Gly
    135                 140                 145

gcc ggt ggt ggg gtc ggg ctg atg gcc gcg cag atc gga cgg gtc cac 595
Ala Gly Gly Gly Val Gly Leu Met Ala Ala Gln Ile Gly Arg Val His
150                 155                 160                 165

aag ttc cag gtc gtc ggc gtt gac cac gag gac aag cgc gag ctc atc 643
Lys Phe Gln Val Val Gly Val Asp His Glu Asp Lys Arg Glu Leu Ile
                170                 175                 180

gaa tcc acc ggt gct atc ttc gtc gcc acc ggc gac gcc gtc gcg gag 691
Glu Ser Thr Gly Ala Ile Phe Val Ala Thr Gly Asp Ala Val Ala Glu
            185                 190                 195

cag gtg cgt gcg ctg ctc cct gac ggt gtg gac gta gtc ttc gac cta 739
Gln Val Arg Ala Leu Leu Pro Asp Gly Val Asp Val Val Phe Asp Leu
        200                 205                 210

gtc ggc ggg gag gcg ttg cgg gtg gtt gct ccc tta gcg aag aat ccg 787
Val Gly Gly Glu Ala Leu Arg Val Val Ala Pro Leu Ala Lys Asn Pro
    215                 220                 225

gcg cac gtg atc tcg gcg gct gat gct gcc acc gtg gga gaa ctc ggt 835
Ala His Val Ile Ser Ala Ala Asp Ala Ala Thr Val Gly Glu Leu Gly
230                 235                 240                 245

gga cag gtg ctg cgc cgc acc ccg gaa atg gtc gga cag atc acc ggg 883
Gly Gln Val Leu Arg Arg Thr Pro Glu Met Val Gly Gln Ile Thr Gly
                250                 255                 260

gtg gtc cag tac ggg ctg gtc gac ccg aag gtc gat acg acc tac ccg 931
Val Val Gln Tyr Gly Leu Val Asp Pro Lys Val Asp Thr Thr Tyr Pro
            265                 270                 275

ctg gaa cag gcc ggt aag gcc ctg gcc cac gtt gag cag ggc cac gcc 979
Leu Glu Gln Ala Gly Lys Ala Leu Ala His Val Glu Gln Gly His Ala

280                 285                 290

cgc ggc aag atc gtc ctc gag ctc atc acc tcc cag gac taaccagaca     1028
Arg Gly Lys Ile Val Leu Glu Leu Ile Thr Ser Gln Asp
    295                 300                 305

acgcggtgac ctc                                                     1041


<210> 408
<211> 306
<212> PRT
<213> Corynebacterium glutamicum

<400> 408
Met Ser Lys Val Tyr Val Ser Asn Glu Tyr Gly Gly Pro Glu Asn Gln
1               5                   10                  15

Glu Leu Ile Thr Arg Asn Thr Pro Gln Pro Gly Pro Gly Glu Leu Gly
            20                  25                  30

Val Lys Val His Ala Ala Gly Val Asn Pro Leu Asp Trp Lys Val Arg
        35                  40                  45

Ser Gly Val Ala Gly Thr Pro Arg Glu Leu Pro Ala Pro Leu Gly Glu
    50                  55                  60

Glu Ala Ser Gly Ile Val Thr Ala Val Gly Asp Gly Val Glu Gly Phe
65                  70                  75                  80

Ala Val Gly Asp Pro Val Leu Gly Leu Val Ala Pro Gly Val Gly Gly
                85                  90                  95

Tyr Ala Glu Asp Thr Leu Leu Val Ala Glu Ser Thr Val Leu Lys Pro
            100                 105                 110

Glu Glu Ile Ser Phe Thr Asp Ala Ala Ala Ile Pro Val Ala Gly Ala
        115                 120                 125

Ser Ala Tyr Ala Gly Thr His Gln Val Glu Leu Glu Pro Gly Gln Ser
    130                 135                 140

Leu Leu Ile Asn Gly Ala Gly Gly Gly Val Gly Leu Met Ala Ala Gln
145                 150                 155                 160

Ile Gly Arg Val His Lys Phe Gln Val Val Gly Val Asp His Glu Asp
                165                 170                 175

Lys Arg Glu Leu Ile Glu Ser Thr Gly Ala Ile Phe Val Ala Thr Gly
            180                 185                 190

Asp Ala Val Ala Glu Gln Val Arg Ala Leu Leu Pro Asp Gly Val Asp
        195                 200                 205

Val Val Phe Asp Leu Val Gly Gly Glu Ala Leu Arg Val Val Ala Pro
    210                 215                 220

Leu Ala Lys Asn Pro Ala His Val Ile Ser Ala Ala Asp Ala Ala Thr
225                 230                 235                 240

Val Gly Glu Leu Gly Gly Gln Val Leu Arg Arg Thr Pro Glu Met Val
                245                 250                 255

Gly Gln Ile Thr Gly Val Val Gln Tyr Gly Leu Val Asp Pro Lys Val
            260                 265                 270

Asp Thr Thr Tyr Pro Leu Glu Gln Ala Gly Lys Ala Leu Ala His Val
        275                 280                 285

Glu Gln Gly His Ala Arg Gly Lys Ile Val Leu Glu Leu Ile Thr Ser
    290                 295                 300

Gln Asp
305


<210> 409

<211> 1614
<212> DNA
<213> Corynebacterium glutamicum

<220>
<221> CDS
<222> (101)..(1591)
<223> RXN00641

<400> 409
tgcggaattg ctcgcaaatg tcacacaccg cttcaaagca aaaacgaaaa cgacatcgcg 60

gtggcaatac caacttcttt tcactctctt ggaggttcac atg tcc aca cca gtt 115
Met Ser Thr Pro Val
1               5

tca aat ttg gca agc gtt cag aaa act ctg gac cat gcg ctt gag gac 163
Ser Asn Leu Ala Ser Val Gln Lys Thr Leu Asp His Ala Leu Glu Asp
                10                  15                  20

cgc cct gaa gag gga atc gtc cgc gtc aac cgc aac atc ttc act gac 211
Arg Pro Glu Glu Gly Ile Val Arg Val Asn Arg Asn Ile Phe Thr Asp
            25                  30                  35

cct gag atc ttc gag ctg gag atg cgc cac atc ttc gaa ggc atc tgg 259
Pro Glu Ile Phe Glu Leu Glu Met Arg His Ile Phe Glu Gly Ile Trp
        40                  45                  50

atg gac atg gct cac gag tcc cag atc cct aac ggt gga gac tac ttc 307
Met Asp Met Ala His Glu Ser Gln Ile Pro Asn Gly Gly Asp Tyr Phe
    55                  60                  65

acc acc tac att ggc tgc cag cgg atc atg atc acc cgt tcc aag gaa 355
Thr Thr Tyr Ile Gly Cys Gln Arg Ile Met Ile Thr Arg Ser Lys Glu
70                  75                  80                  85

ggc aca ctc aac ggc ctg atc aac gcg tgt tct cac cgt ggc gcc atg 403
Gly Thr Leu Asn Gly Leu Ile Asn Ala Cys Ser His Arg Gly Ala Met
                90                  95                  100

ctc tgc cgt ggc aag agt gac aac cgc acc tcc ttg acc tgc cca ttc 451
Leu Cys Arg Gly Lys Ser Asp Asn Arg Thr Ser Leu Thr Cys Pro Phe
            105                 110                 115

cac ggc tgg cca ttc tgc aac ggc ggc gca ctg ctc aag gtc aag ggc 499
His Gly Trp Pro Phe Cys Asn Gly Gly Ala Leu Leu Lys Val Lys Gly
        120                 125                 130

gaa aaa gaa ggc gcc tac cca gag aat ttc cgc acc gac ggc tcc cac 547
Glu Lys Glu Gly Ala Tyr Pro Glu Asn Phe Arg Thr Asp Gly Ser His
    135                 140                 145

gat gtg cgt cgc gtt cct aag tta gag tcc tac cgt ggc ttc ctc ttc 595
Asp Val Arg Arg Val Pro Lys Leu Glu Ser Tyr Arg Gly Phe Leu Phe
150                 155                 160                 165

ggc tcc ctc aac gat gat gtc gtt tct ttg gaa gag cac ctc ggc gac 643
Gly Ser Leu Asn Asp Asp Val Val Ser Leu Glu Glu His Leu Gly Asp
                170                 175                 180

acc cgt acc gtc att gac atg ctg gtt gac caa tcc cca gaa ggc ctc 691
Thr Arg Thr Val Ile Asp Met Leu Val Asp Gln Ser Pro Glu Gly Leu
            185                 190                 195

gaa gta ctg cgc gga tcc tcc acc tac acc tac gac ggc aac tgg aag 739
Glu Val Leu Arg Gly Ser Ser Thr Tyr Thr Tyr Asp Gly Asn Trp Lys
        200                 205                 210

ctc cag acc gaa aac ggt gca gac ggc tac cac gtt tcc tcc acc cac 787
Leu Gln Thr Glu Asn Gly Ala Asp Gly Tyr His Val Ser Ser Thr His
    215                 220                 225

tgg aac tac gct gca acc acc tcc cgc cgt ggc act ggt gaa tcc gcg 835
Trp Asn Tyr Ala Ala Thr Thr Ser Arg Arg Gly Thr Gly Glu Ser Ala
230                 235                 240                 245

aac gaa acc aag gca atg gac gct ggt acc tgg ggc aag cag ggt ggc 883

Asn Glu Thr Lys Ala Met Asp Ala Gly Thr Trp Gly Lys Gln Gly Gly
                250                 255                 260

gga tac ttc tcc tac cct tac ggc cac atg ctg ctg tgg atg tgg tgg     931
Gly Tyr Phe Ser Tyr Pro Tyr Gly His Met Leu Leu Trp Met Trp Trp
            265                 270                 275

ggc aac cca gaa gac cgc cca ctg ttc gag cgc cgc gac gag ttc aag     979
Gly Asn Pro Glu Asp Arg Pro Leu Phe Glu Arg Arg Asp Glu Phe Lys
        280                 285                 290

aag gaa ttc ggc gaa gaa aag ggc gag ttc atg gtt ggt gct tcc cgc     1027
Lys Glu Phe Gly Glu Glu Lys Gly Glu Phe Met Val Gly Ala Ser Arg
    295                 300                 305

aat ctg tgc ctc tac ccc aat gtt tac ctg atg gat cag ttc tcc tca     1075
Asn Leu Cys Leu Tyr Pro Asn Val Tyr Leu Met Asp Gln Phe Ser Ser
310                 315                 320                 325

cag atc cgc cac atc cgc cca atc tca gtt gat cag acc gaa gtc acc     1123
Gln Ile Arg His Ile Arg Pro Ile Ser Val Asp Gln Thr Glu Val Thr
                330                 335                 340

atc tac tgc atc gca cct aag ggc gag tcc gcg gaa gca cgt gca aac     1171
Ile Tyr Cys Ile Ala Pro Lys Gly Glu Ser Ala Glu Ala Arg Ala Asn
            345                 350                 355

cgc atc cgc cag tac gaa gac ttc ttc aac gca acg ggc atg gca acc     1219
Arg Ile Arg Gln Tyr Glu Asp Phe Phe Asn Ala Thr Gly Met Ala Thr
        360                 365                 370

cca gat gac ctg gag gaa ttc cgc tcc tgc cag aag acc tac cag gca     1267
Pro Asp Asp Leu Glu Glu Phe Arg Ser Cys Gln Lys Thr Tyr Gln Ala
    375                 380                 385

tct gcc ttc cca tgg aat gac atg acc cgc ggt ttg ggc cac cag gta     1315
Ser Ala Phe Pro Trp Asn Asp Met Thr Arg Gly Leu Gly His Gln Val
390                 395                 400                 405

cag gga cca aac gag gtt gcc aag ggc cta ggc atg aac gaa gtt ctt     1363
Gln Gly Pro Asn Glu Val Ala Lys Gly Leu Gly Met Asn Glu Val Leu
                410                 415                 420

tcc tcc gga gca cgc acc gaa gat gaa ggc ctc tac cca atc cag cac     1411
Ser Ser Gly Ala Arg Thr Glu Asp Glu Gly Leu Tyr Pro Ile Gln His
            425                 430                 435

ggc ttc tgg cat gaa ctc atg cag gag gct gtg aat aag cag agc atc     1459
Gly Phe Trp His Glu Leu Met Gln Glu Ala Val Asn Lys Gln Ser Ile
        440                 445                 450

aag gaa aag gaa ttg gct gac gat acc gct tct tcc ctt gcc acc gta     1507
Lys Glu Lys Glu Leu Ala Asp Asp Thr Ala Ser Ser Leu Ala Thr Val
    455                 460                 465

gct gca gcc aaa atc cgt gag gaa gca aag gca gcc gcg aag tcc gac     1555
Ala Ala Ala Lys Ile Arg Glu Glu Ala Lys Ala Ala Ala Lys Ser Asp
470                 475                 480                 485

gct gga gag cct cgc cgc cgt cgt cgc acc cgc ggt tagtcgtcga          1601
Ala Gly Glu Pro Arg Arg Arg Arg Arg Thr Arg Gly
                490                 495

aaagcaaaaa atc                                                      1614


<210> 410
<211> 497
<212> PRT
<213> Corynebacterium glutamicum

<400> 410
Met Ser Thr Pro Val Ser Asn Leu Ala Ser Val Gln Lys Thr Leu Asp
1               5                   10                  15

His Ala Leu Glu Asp Arg Pro Glu Glu Gly Ile Val Arg Val Asn Arg
            20                  25                  30

Asn Ile Phe Thr Asp Pro Glu Ile Phe Glu Leu Glu Met Arg His Ile
            35                  40                  45

Phe Glu Gly Ile Trp Met Asp Met Ala His Glu Ser Gln Ile Pro Asn
        50                  55                  60

Gly Gly Asp Tyr Phe Thr Thr Tyr Ile Gly Cys Gln Arg Ile Met Ile
65                  70                  75                  80

Thr Arg Ser Lys Glu Gly Thr Leu Asn Gly Leu Ile Asn Ala Cys Ser
                85                  90                  95

His Arg Gly Ala Met Leu Cys Arg Gly Lys Ser Asp Asn Arg Thr Ser
            100                 105                 110

Leu Thr Cys Pro Phe His Gly Trp Pro Phe Cys Asn Gly Gly Ala Leu
        115                 120                 125

Leu Lys Val Lys Gly Glu Lys Glu Gly Ala Tyr Pro Glu Asn Phe Arg
    130                 135                 140

Thr Asp Gly Ser His Asp Val Arg Arg Val Pro Lys Leu Glu Ser Tyr
145                 150                 155                 160

Arg Gly Phe Leu Phe Gly Ser Leu Asn Asp Asp Val Val Ser Leu Glu
                165                 170                 175

Glu His Leu Gly Asp Thr Arg Thr Val Ile Asp Met Leu Val Asp Gln
            180                 185                 190

Ser Pro Glu Gly Leu Glu Val Leu Arg Gly Ser Ser Thr Tyr Thr Tyr
        195                 200                 205

Asp Gly Asn Trp Lys Leu Gln Thr Glu Asn Gly Ala Asp Gly Tyr His
    210                 215                 220

Val Ser Ser Thr His Trp Asn Tyr Ala Ala Thr Thr Ser Arg Arg Gly
225                 230                 235                 240

Thr Gly Glu Ser Ala Asn Glu Thr Lys Ala Met Asp Ala Gly Thr Trp
                245                 250                 255

Gly Lys Gln Gly Gly Gly Tyr Phe Ser Tyr Pro Tyr Gly His Met Leu
            260                 265                 270

Leu Trp Met Trp Trp Gly Asn Pro Glu Asp Arg Pro Leu Phe Glu Arg
        275                 280                 285

Arg Asp Glu Phe Lys Lys Glu Phe Gly Glu Glu Lys Gly Glu Phe Met
    290                 295                 300

Val Gly Ala Ser Arg Asn Leu Cys Leu Tyr Pro Asn Val Tyr Leu Met
305                 310                 315                 320

Asp Gln Phe Ser Ser Gln Ile Arg His Ile Arg Pro Ile Ser Val Asp
                325                 330                 335

Gln Thr Glu Val Thr Ile Tyr Cys Ile Ala Pro Lys Gly Glu Ser Ala
            340                 345                 350

Glu Ala Arg Ala Asn Arg Ile Arg Gln Tyr Glu Asp Phe Phe Asn Ala
        355                 360                 365

Thr Gly Met Ala Thr Pro Asp Asp Leu Glu Glu Phe Arg Ser Cys Gln
    370                 375                 380

Lys Thr Tyr Gln Ala Ser Ala Phe Pro Trp Asn Asp Met Thr Arg Gly
385                 390                 395                 400

Leu Gly His Gln Val Gln Gly Pro Asn Glu Val Ala Lys Gly Leu Gly
                405                 410                 415

Met Asn Glu Val Leu Ser Ser Gly Ala Arg Thr Glu Asp Glu Gly Leu
            420                 425                 430

Tyr Pro Ile Gln His Gly Phe Trp His Glu Leu Met Gln Glu Ala Val

435                 440                 445

Asn Lys Gln Ser Ile Lys Glu Lys Glu Leu Ala Asp Asp Thr Ala Ser
    450                 455                 460

Ser Leu Ala Thr Val Ala Ala Ala Lys Ile Arg Glu Glu Ala Lys Ala
465                 470                 475                 480

Ala Ala Lys Ser Asp Ala Gly Glu Pro Arg Arg Arg Arg Arg Thr Arg
                485                 490                 495

Gly

<210> 411
<211> 1166
<212> DNA
<213> Corynebacterium glutamicum

<220>
<221> CDS
<222> (16)..(1143)
<223> RXN01993

<400> 411
ctttaggagt tcacc atg aca ctg tcc gaa cgc aag ctc acc acc acc gcc    51
                 Met Thr Leu Ser Glu Arg Lys Leu Thr Thr Thr Ala
                 1               5                   10

aag att ctt ccc cac cca ctc aac gcc tgg tac gtc gcc gct tgg gat    99
Lys Ile Leu Pro His Pro Leu Asn Ala Trp Tyr Val Ala Ala Trp Asp
        15                  20                  25

tat gaa gtc aca tct aaa aag ccc atg gcc agg aca atc gcc aac aaa    147
Tyr Glu Val Thr Ser Lys Lys Pro Met Ala Arg Thr Ile Ala Asn Lys
    30                  35                  40

cca ctc gct ttg tac cgc acc aaa gat ggc cga gcc gtt gcc ctt gca    195
Pro Leu Ala Leu Tyr Arg Thr Lys Asp Gly Arg Ala Val Ala Leu Ala
45                  50                  55                  60

gac gcc tgc tgg cac cgc ctc gca ccg cta tcc aag gga aaa ctc gtg    243
Asp Ala Cys Trp His Arg Leu Ala Pro Leu Ser Lys Gly Lys Leu Val
                65                  70                  75

ggc aca gac gga atc caa tgc cct tat cac ggc ttg gag tac aac tcc    291
Gly Thr Asp Gly Ile Gln Cys Pro Tyr His Gly Leu Glu Tyr Asn Ser
            80                  85                  90

gcg ggc cgc tgc atg aaa atg ccc gcg cag gaa acc ctc aac ccg tca    339
Ala Gly Arg Cys Met Lys Met Pro Ala Gln Glu Thr Leu Asn Pro Ser
        95                  100                 105

gca gcc gtc aac tcc tac ccc gtg gtg gaa gcc cac cgc ttt gtg tgg    387
Ala Ala Val Asn Ser Tyr Pro Val Val Glu Ala His Arg Phe Val Trp
    110                 115                 120

gtg tgg ctg ggc gat ccc aca ttg gca gat ccc acc caa gta ccc gat    435
Val Trp Leu Gly Asp Pro Thr Leu Ala Asp Pro Thr Gln Val Pro Asp
125                 130                 135                 140

atg cac cag atg agc cac ccc gaa tgg gca ggc gat gga cgc acc atc    483
Met His Gln Met Ser His Pro Glu Trp Ala Gly Asp Gly Arg Thr Ile
                145                 150                 155

tcc gct gac tgc aac tac caa tta gtg ctg gac aac ttg atg gac ctc    531
Ser Ala Asp Cys Asn Tyr Gln Leu Val Leu Asp Asn Leu Met Asp Leu
            160                 165                 170

acc cac gaa gag ttc gtg cac tcc tcc agc atc gga caa gac gaa ctt    579
Thr His Glu Glu Phe Val His Ser Ser Ser Ile Gly Gln Asp Glu Leu
        175                 180                 185

agt gaa tca gag ttc gtg gtc acc cac act gaa gat tcc gtg acg gtc    627
Ser Glu Ser Glu Phe Val Val Thr His Thr Glu Asp Ser Val Thr Val
    190                 195                 200

acc cgc tgg atg cat gac ata gat gca cca ccg ttt tgg caa aag aac     675
Thr Arg Trp Met His Asp Ile Asp Ala Pro Pro Phe Trp Gln Lys Asn
205                 210                 215                 220

atg aat gat aag ttc cca gga ttt gaa ggc aag gtg gat cgt tgg cag     723
Met Asn Asp Lys Phe Pro Gly Phe Glu Gly Lys Val Asp Arg Trp Gln
                225                 230                 235

atc atc cac tac tac tac cct tcc acc atc tgc att gat gtt ggt gta     771
Ile Ile His Tyr Tyr Tyr Pro Ser Thr Ile Cys Ile Asp Val Gly Val
            240                 245                 250

gca aag gct gga act ggc gcg cag gaa ggc gac cgc agc cag ggc gtt     819
Ala Lys Ala Gly Thr Gly Ala Gln Glu Gly Asp Arg Ser Gln Gly Val
        255                 260                 265

aat ggg tat gtc atg aac acc att acc cca gat tca gat cgt tcc tct     867
Asn Gly Tyr Val Met Asn Thr Ile Thr Pro Asp Ser Asp Arg Ser Ser
    270                 275                 280

cat tac ttc tgg gca ttc atg cgc aac tac cgc ctg gaa agc caa acc     915
His Tyr Phe Trp Ala Phe Met Arg Asn Tyr Arg Leu Glu Ser Gln Thr
285                 290                 295                 300

atc acc acc cag ctg cgc gac ggt gta tcc ggt gta ttc aaa gaa gac     963
Ile Thr Thr Gln Leu Arg Asp Gly Val Ser Gly Val Phe Lys Glu Asp
                305                 310                 315

gaa gac atg ctg acc gct cag caa gat gcc atc gac gcc aac acc gac     1011
Glu Asp Met Leu Thr Ala Gln Gln Asp Ala Ile Asp Ala Asn Thr Asp
            320                 325                 330

tac gag ttt tac agc ctc aac att gat gcc ggt ggc atg tgg gtg cgc     1059
Tyr Glu Phe Tyr Ser Leu Asn Ile Asp Ala Gly Gly Met Trp Val Arg
        335                 340                 345

cga atc ctc gag gaa gca ctc tcc aag gaa ggc cga ctg gat atc ccc     1107
Arg Ile Leu Glu Glu Ala Leu Ser Lys Glu Gly Arg Leu Asp Ile Pro
    350                 355                 360

acc aca ttc ccc cgc gca aca ccg aag ccg gag gca taaaccatga          1153
Thr Thr Phe Pro Arg Ala Thr Pro Lys Pro Glu Ala
365                 370                 375

actcgcaatg gca                                                      1166


<210> 412
<211> 376
<212> PRT
<213> Corynebacterium glutamicum

<400> 412
Met Thr Leu Ser Glu Arg Lys Leu Thr Thr Thr Ala Lys Ile Leu Pro
  1               5                  10                  15

His Pro Leu Asn Ala Trp Tyr Val Ala Ala Trp Asp Tyr Glu Val Thr
             20                  25                  30

Ser Lys Lys Pro Met Ala Arg Thr Ile Ala Asn Lys Pro Leu Ala Leu
         35                  40                  45

Tyr Arg Thr Lys Asp Gly Arg Ala Val Ala Leu Ala Asp Ala Cys Trp
     50                  55                  60

His Arg Leu Ala Pro Leu Ser Lys Gly Lys Leu Val Gly Thr Asp Gly
 65                  70                  75                  80

Ile Gln Cys Pro Tyr His Gly Leu Glu Tyr Asn Ser Ala Gly Arg Cys
                 85                  90                  95

Met Lys Met Pro Ala Gln Glu Thr Leu Asn Pro Ser Ala Ala Val Asn
            100                 105                 110

Ser Tyr Pro Val Val Glu Ala His Arg Phe Val Trp Val Trp Leu Gly
        115                 120                 125

Asp Pro Thr Leu Ala Asp Pro Thr Gln Val Pro Asp Met His Gln Met
    130                 135                 140

Ser His Pro Glu Trp Ala Gly Asp Gly Arg Thr Ile Ser Ala Asp Cys
145                 150                 155                 160

Asn Tyr Gln Leu Val Leu Asp Asn Leu Met Asp Leu Thr His Glu Glu
                165                 170                 175

Phe Val His Ser Ser Ser Ile Gly Gln Asp Glu Leu Ser Glu Ser Glu
            180                 185                 190

Phe Val Val Thr His Thr Glu Asp Ser Val Thr Val Thr Arg Trp Met
        195                 200                 205

His Asp Ile Asp Ala Pro Pro Phe Trp Gln Lys Asn Met Asn Asp Lys
    210                 215                 220

Phe Pro Gly Phe Glu Gly Lys Val Asp Arg Trp Gln Ile Ile His Tyr
225                 230                 235                 240

Tyr Tyr Pro Ser Thr Ile Cys Ile Asp Val Gly Val Ala Lys Ala Gly
                245                 250                 255

Thr Gly Ala Gln Glu Gly Asp Arg Ser Gln Gly Val Asn Gly Tyr Val
            260                 265                 270

Met Asn Thr Ile Thr Pro Asp Ser Asp Arg Ser Ser His Tyr Phe Trp
        275                 280                 285

Ala Phe Met Arg Asn Tyr Arg Leu Glu Ser Gln Thr Ile Thr Thr Gln
    290                 295                 300

Leu Arg Asp Gly Val Ser Gly Val Phe Lys Glu Asp Glu Asp Met Leu
305                 310                 315                 320

Thr Ala Gln Gln Asp Ala Ile Asp Ala Asn Thr Asp Tyr Glu Phe Tyr
                325                 330                 335

Ser Leu Asn Ile Asp Ala Gly Gly Met Trp Val Arg Arg Ile Leu Glu
            340                 345                 350

Glu Ala Leu Ser Lys Glu Gly Arg Leu Asp Ile Pro Thr Thr Phe Pro
        355                 360                 365

Arg Ala Thr Pro Lys Pro Glu Ala
    370                 375


<210> 413
<211> 816
<212> DNA
<213> Corynebacterium glutamicum

<220>
<221> CDS
<222> (101)..(793)
<223> RXN00658

<400> 413
cattgacacc cacaggttta ccagcatcac ggaaagtttg gatggatttt tactccggcc 60

acaacgtctg gctggaagct cagccacgtg ctttctggtc gtg cgc cac gac gag    115
                                           Val Arg His Asp Glu
                                           1               5

cac tac cca gct gcg gca aac ctc att gct ttc gat aag gga tgg tcc    163
His Tyr Pro Ala Ala Ala Asn Leu Ile Ala Phe Asp Lys Gly Trp Ser
                10                  15                  20

acc ctc atc gcc cct cag ctg gaa gat cca gag gcg gag gag ttc acc    211
Thr Leu Ile Ala Pro Gln Leu Glu Asp Pro Glu Ala Glu Glu Phe Thr
            25                  30                  35

gcc gga ttc ctc acc gaa tac cag gac aat ctg atc act gcg ggc atg    259
Ala Gly Phe Leu Thr Glu Tyr Gln Asp Asn Leu Ile Thr Ala Gly Met

40                  45                  50

gag cac cag gcg ctc gcg agc ggc ttc ccg gtg ggg cgt cgc ttc aag     307
Glu His Gln Ala Leu Ala Ser Gly Phe Pro Val Gly Arg Arg Phe Lys
    55                  60                  65

tcc gat att gct tta cga cgc tgc gat gcg gtg acc acc cac atc ggc     355
Ser Asp Ile Ala Leu Arg Arg Cys Asp Ala Val Thr Thr His Ile Gly
70                  75                  80                  85

cac gaa cac tcc gcc gat ggt cac tgg agg atc tac gta ttc gct ggc     403
His Glu His Ser Ala Asp Gly His Trp Arg Ile Tyr Val Phe Ala Gly
                90                  95                  100

caa gcc acc cca caa gat tcc gag tct gca ctg aac aag tgg gcg cag     451
Gln Ala Thr Pro Gln Asp Ser Glu Ser Ala Leu Asn Lys Trp Ala Gln
            105                 110                 115

tgg atg gag gaa agc gaa gac tca cca ctc aac cgc ttc acc cca gaa     499
Trp Met Glu Glu Ser Glu Asp Ser Pro Leu Asn Arg Phe Thr Pro Glu
        120                 125                 130

gcc ggc gac cgc aac gca gtc ttc gat atc aag gct acc tac cag cag     547
Ala Gly Asp Arg Asn Ala Val Phe Asp Ile Lys Ala Thr Tyr Gln Gln
    135                 140                 145

cat tac cac tcc ttc gac ctg ttc gat gcg cca gag gtc ttc ttc cca     595
His Tyr His Ser Phe Asp Leu Phe Asp Ala Pro Glu Val Phe Phe Pro
150                 155                 160                 165

cga gtt gga cca tac aag ctg caa aac ctc gaa aac gtt tgg acc gca     643
Arg Val Gly Pro Tyr Lys Leu Gln Asn Leu Glu Asn Val Trp Thr Ala
                170                 175                 180

ctg gat tcc caa gac atc ttt gag tcc cgt ggc atc agt cgc gat ggc     691
Leu Asp Ser Gln Asp Ile Phe Glu Ser Arg Gly Ile Ser Arg Asp Gly
            185                 190                 195

gca att gtt gtc gtt cgc cca gac cag tac gtc gca gca gtc ctc cca     739
Ala Ile Val Val Val Arg Pro Asp Gln Tyr Val Ala Ala Val Leu Pro
        200                 205                 210

ctc gaa gac acc gca gca ctg gct gag ttc ttc aat ggc aat ctg ctt     787
Leu Glu Asp Thr Ala Ala Leu Ala Glu Phe Phe Asn Gly Asn Leu Leu
    215                 220                 225

gag cca taaaccctaa attctaggaa cga                                   816
Glu Pro
230


<210> 414
<211> 231
<212> PRT
<213> Corynebacterium glutamicum

<400> 414
Val Arg His Asp Glu His Tyr Pro Ala Ala Ala Asn Leu Ile Ala Phe
1               5                   10                  15

Asp Lys Gly Trp Ser Thr Leu Ile Ala Pro Gln Leu Glu Asp Pro Glu
            20                  25                  30

Ala Glu Glu Phe Thr Ala Gly Phe Leu Thr Glu Tyr Gln Asp Asn Leu
        35                  40                  45

Ile Thr Ala Gly Met Glu His Gln Ala Leu Ala Ser Gly Phe Pro Val
    50                  55                  60

Gly Arg Arg Phe Lys Ser Asp Ile Ala Leu Arg Arg Cys Asp Ala Val
65                  70                  75                  80

Thr Thr His Ile Gly His Glu His Ser Ala Asp Gly His Trp Arg Ile
                85                  90                  95

Tyr Val Phe Ala Gly Gln Ala Thr Pro Gln Asp Ser Glu Ser Ala Leu
            100                 105                 110

Asn Lys Trp Ala Gln Trp Met Glu Glu Ser Glu Asp Ser Pro Leu Asn
        115                 120                 125

Arg Phe Thr Pro Glu Ala Gly Asp Arg Asn Ala Val Phe Asp Ile Lys
    130                 135                 140

Ala Thr Tyr Gln Gln His Tyr His Ser Phe Asp Leu Phe Asp Ala Pro
145                 150                 155                 160

Glu Val Phe Phe Pro Arg Val Gly Pro Tyr Lys Leu Gln Asn Leu Glu
                165                 170                 175

Asn Val Trp Thr Ala Leu Asp Ser Gln Asp Ile Phe Glu Ser Arg Gly
        180                 185                 190

Ile Ser Arg Asp Gly Ala Ile Val Val Val Arg Pro Asp Gln Tyr Val
        195                 200                 205

Ala Ala Val Leu Pro Leu Glu Asp Thr Ala Ala Leu Ala Glu Phe Phe
    210                 215                 220

Asn Gly Asn Leu Leu Glu Pro
225                 230


<210> 415
<211> 1008
<212> DNA
<213> Corynebacterium glutamicum

<220>
<221> CDS
<222> (101)..(985)
<223> RXN00178

<400> 415
gcgaataatc cacggacggt cacagaagaa aacctcactg cgctgcttac cacagcgctc 60

aacggcgacg atccagcaac tttgaattaa ggagaccaac atg act att tca gca    115
                                           Met Thr Ile Ser Ala
                                           1               5

caa cag caa gca gtg gaa gaa gac ctt gta gag cgc gta ctc gca tct    163
Gln Gln Gln Ala Val Glu Glu Asp Leu Val Glu Arg Val Leu Ala Ser
                10                  15                  20

ttt gat tcg tgt gaa aac cct cgc ctc aaa cta gtg atg aaa tcc ctg    211
Phe Asp Ser Cys Glu Asn Pro Arg Leu Lys Leu Val Met Lys Ser Leu
            25                  30                  35

act gtg cat ctc cat gat ttc atc cgc gat gtt cga ctc act gaa gaa    259
Thr Val His Leu His Asp Phe Ile Arg Asp Val Arg Leu Thr Glu Glu
        40                  45                  50

gag tgg aac tac gcc att gat ttc ctc acc aag gtt ggg cat atc acc    307
Glu Trp Asn Tyr Ala Ile Asp Phe Leu Thr Lys Val Gly His Ile Thr
    55                  60                  65

gac gat aag cgc caa gaa ttc gtg ttg ctc tct gac acc ttg ggt gca    355
Asp Asp Lys Arg Gln Glu Phe Val Leu Leu Ser Asp Thr Leu Gly Ala
70                  75                  80                  85

tcc atg cag acc atc gct gtt aat aac gaa gca tat gaa gac gct acc    403
Ser Met Gln Thr Ile Ala Val Asn Asn Glu Ala Tyr Glu Asp Ala Thr
                90                  95                  100

gaa gca aca gtc ttt ggc ccc ttc ttt gtc gat gat gcg cca ctg gtc    451
Glu Ala Thr Val Phe Gly Pro Phe Phe Val Asp Asp Ala Pro Leu Val
            105                 110                 115

caa aac gga gat gac att gcc ttt ggc gca gtc ggc cag ccg gca tgg    499
Gln Asn Gly Asp Asp Ile Ala Phe Gly Ala Val Gly Gln Pro Ala Trp
        120                 125                 130

gtg gag gga acg gtc aaa gac act gaa gga aac ccc att ccc aat gca    547
Val Glu Gly Thr Val Lys Asp Thr Glu Gly Asn Pro Ile Pro Asn Ala

135                 140                 145

cgc att gaa gta tgg gaa tgc gat gaa gat gga ctt tat gat gtg caa     595
Arg Ile Glu Val Trp Glu Cys Asp Glu Asp Gly Leu Tyr Asp Val Gln
150                 155                 160                 165

tac gcc gat gag cgc agt gct gga cgc gca cac ctg tat tca gat gaa     643
Tyr Ala Asp Glu Arg Ser Ala Gly Arg Ala His Leu Tyr Ser Asp Glu
                170                 175                 180

aac ggc gaa tac cac ttc tgg gga cta act ccc gtg cca tat ccc atc     691
Asn Gly Glu Tyr His Phe Trp Gly Leu Thr Pro Val Pro Tyr Pro Ile
            185                 190                 195

cca cac gat ggt cca gta gga caa atg ctc caa gca gtt ggt cgt tcc     739
Pro His Asp Gly Pro Val Gly Gln Met Leu Gln Ala Val Gly Arg Ser
        200                 205                 210

ccc gtt cgt tgc gcg cac cta cac ttc atg gtg act gcg cca gag aag     787
Pro Val Arg Cys Ala His Leu His Phe Met Val Thr Ala Pro Glu Lys
    215                 220                 225

cga acc ttg gta acc cat atc ttc gtt gag ggc gat ccg cag cta gag     835
Arg Thr Leu Val Thr His Ile Phe Val Glu Gly Asp Pro Gln Leu Glu
230                 235                 240                 245

atc ggc gat tcc gtg ttt ggc gtg aag gac tca ctg att aag aaa ttc     883
Ile Gly Asp Ser Val Phe Gly Val Lys Asp Ser Leu Ile Lys Lys Phe
                250                 255                 260

gtt gag caa cct gca gga acc gca act cca gat ggt cgc gat gtg ggt     931
Val Glu Gln Pro Ala Gly Thr Ala Thr Pro Asp Gly Arg Asp Val Gly
            265                 270                 275

gat caa acc tgg gca cgc aca cgt ttt gat att gtg ctc gcc ccc ggc     979
Asp Gln Thr Trp Ala Arg Thr Arg Phe Asp Ile Val Leu Ala Pro Gly
        280                 285                 290

aat gtc taagtagaag cagcaaaaaa cca                                   1008
Asn Val
    295


<210> 416
<211> 295
<212> PRT
<213> Corynebacterium glutamicum

<400> 416
Met Thr Ile Ser Ala Gln Gln Gln Ala Val Glu Glu Asp Leu Val Glu
  1               5                  10                  15

Arg Val Leu Ala Ser Phe Asp Ser Cys Glu Asn Pro Arg Leu Lys Leu
                 20                  25                  30

Val Met Lys Ser Leu Thr Val His Leu His Asp Phe Ile Arg Asp Val
             35                  40                  45

Arg Leu Thr Glu Glu Glu Trp Asn Tyr Ala Ile Asp Phe Leu Thr Lys
     50                  55                  60

Val Gly His Ile Thr Asp Asp Lys Arg Gln Glu Phe Val Leu Leu Ser
 65                  70                  75                  80

Asp Thr Leu Gly Ala Ser Met Gln Thr Ile Ala Val Asn Asn Glu Ala
                 85                  90                  95

Tyr Glu Asp Ala Thr Glu Ala Thr Val Phe Gly Pro Phe Phe Val Asp
                100                 105                 110

Asp Ala Pro Leu Val Gln Asn Gly Asp Asp Ile Ala Phe Gly Ala Val
        115                 120                 125

Gly Gln Pro Ala Trp Val Glu Gly Thr Val Lys Asp Thr Glu Gly Asn
    130                 135                 140

Pro Ile Pro Asn Ala Arg Ile Glu Val Trp Glu Cys Asp Glu Asp Gly

145                 150                 155                 160

Leu Tyr Asp Val Gln Tyr Ala Asp Glu Arg Ser Ala Gly Arg Ala His
                165                 170                 175

Leu Tyr Ser Asp Glu Asn Gly Glu Tyr His Phe Trp Gly Leu Thr Pro
            180                 185                 190

Val Pro Tyr Pro Ile Pro His Asp Gly Pro Val Gly Gln Met Leu Gln
        195                 200                 205

Ala Val Gly Arg Ser Pro Val Arg Cys Ala His Leu His Phe Met Val
    210                 215                 220

Thr Ala Pro Glu Lys Arg Thr Leu Val Thr His Ile Phe Val Glu Gly
225                 230                 235                 240

Asp Pro Gln Leu Glu Ile Gly Asp Ser Val Phe Gly Val Lys Asp Ser
                245                 250                 255

Leu Ile Lys Lys Phe Val Glu Gln Pro Ala Gly Thr Ala Thr Pro Asp
            260                 265                 270

Gly Arg Asp Val Gly Asp Gln Thr Trp Ala Arg Thr Arg Phe Asp Ile
        275                 280                 285

Val Leu Ala Pro Gly Asn Val
    290                 295


<210> 417
<211> 735
<212> DNA
<213> Corynebacterium glutamicum

<220>
<221> CDS
<222> (101)..(712)
<223> RXN01461

<400> 417
tcgactatga cgagacccgt gaaaacttcg cgcttggtta caagttcgac atcgtccttc 60

gtggccgcaa cgccacccca tttgagtaaa gggttttgca atg att gat aca ggg   115
                                           Met Ile Asp Thr Gly
                                           1               5

aag aac ggc gag ttc cgc tac gag cag tcg aat atc atc gat cag aac   163
Lys Asn Gly Glu Phe Arg Tyr Glu Gln Ser Asn Ile Ile Asp Gln Asn
                10                  15                  20

gaa gcc gag ttc ggc atc act cct tca cag acc gtg ggc cct tac gtc   211
Glu Ala Glu Phe Gly Ile Thr Pro Ser Gln Thr Val Gly Pro Tyr Val
            25                  30                  35

cac atc ggt ttg acc ctt gaa ggt gcg gag cat ctc gtg gag cca ggt   259
His Ile Gly Leu Thr Leu Glu Gly Ala Glu His Leu Val Glu Pro Gly
        40                  45                  50

tcg gaa ggc gcg gtg tcc ttt act gtt tcc gca act gat ggc aac ggc   307
Ser Glu Gly Ala Val Ser Phe Thr Val Ser Ala Thr Asp Gly Asn Gly
    55                  60                  65

gac ccc atc gcg gat gcc atg ttt gaa ctg tgg cag gcc gat cca gag   355
Asp Pro Ile Ala Asp Ala Met Phe Glu Leu Trp Gln Ala Asp Pro Glu
70                  75                  80                  85

ggc atc cac aac tct gat ttg gat cca aac cgc aca gca cca gca acc   403
Gly Ile His Asn Ser Asp Leu Asp Pro Asn Arg Thr Ala Pro Ala Thr
                90                  95                  100

gca gat ggc ttc cgc ggg ctt ggt cgc gcg atg gca aac gcg cag ggt   451
Ala Asp Gly Phe Arg Gly Leu Gly Arg Ala Met Ala Asn Ala Gln Gly
            105                 110                 115

gag gca acg ttc acc act ttg gtt ccg gga gca ttc gca gat gag gca   499
Glu Ala Thr Phe Thr Thr Leu Val Pro Gly Ala Phe Ala Asp Glu Ala

        120                 125                 130

cca cac ttc aag gtt ggt gtg ttc gcc cgt ggc atg ctg gag cgt ctg    547
Pro His Phe Lys Val Gly Val Phe Ala Arg Gly Met Leu Glu Arg Leu
    135                 140                 145

tac act cgc gca tac ctg cca gac gcc gat ttg agc acc gac cca gtt    595
Tyr Thr Arg Ala Tyr Leu Pro Asp Ala Asp Leu Ser Thr Asp Pro Val
150                 155                 160                 165

ttg gct gtg gtc cca gct gat cga cgt gac ctc ctg gtg gct caa aag    643
Leu Ala Val Val Pro Ala Asp Arg Arg Asp Leu Leu Val Ala Gln Lys
                170                 175                 180

acc gat gat gga ttc cgc ttc gac atc act gtc cag gct gaa gac aat    691
Thr Asp Asp Gly Phe Arg Phe Asp Ile Thr Val Gln Ala Glu Asp Asn
            185                 190                 195

gaa acc cca ttt ttt gga ctc taaattgacc cgatctttat act              735
Glu Thr Pro Phe Phe Gly Leu
        200


<210> 418
<211> 204
<212> PRT
<213> Corynebacterium glutamicum

<400> 418
Met Ile Asp Thr Gly Lys Asn Gly Glu Phe Arg Tyr Glu Gln Ser Asn
  1               5                  10                  15

Ile Ile Asp Gln Asn Glu Ala Glu Phe Gly Ile Thr Pro Ser Gln Thr
             20                  25                  30

Val Gly Pro Tyr Val His Ile Gly Leu Thr Leu Glu Gly Ala Glu His
         35                  40                  45

Leu Val Glu Pro Gly Ser Glu Gly Ala Val Ser Phe Thr Val Ser Ala
     50                  55                  60

Thr Asp Gly Asn Gly Asp Pro Ile Ala Asp Ala Met Phe Glu Leu Trp
 65                  70                  75                  80

Gln Ala Asp Pro Glu Gly Ile His Asn Ser Asp Leu Asp Pro Asn Arg
                 85                  90                  95

Thr Ala Pro Ala Thr Ala Asp Gly Phe Arg Gly Leu Gly Arg Ala Met
            100                 105                 110

Ala Asn Ala Gln Gly Glu Ala Thr Phe Thr Thr Leu Val Pro Gly Ala
        115                 120                 125

Phe Ala Asp Glu Ala Pro His Phe Lys Val Gly Val Phe Ala Arg Gly
    130                 135                 140

Met Leu Glu Arg Leu Tyr Thr Arg Ala Tyr Leu Pro Asp Ala Asp Leu
145                 150                 155                 160

Ser Thr Asp Pro Val Leu Ala Val Val Pro Ala Asp Arg Arg Asp Leu
                165                 170                 175

Leu Val Ala Gln Lys Thr Asp Asp Gly Phe Arg Phe Asp Ile Thr Val
            180                 185                 190

Gln Ala Glu Asp Asn Glu Thr Pro Phe Phe Gly Leu
        195                 200


<210> 419
<211> 1584
<212> DNA
<213> Corynebacterium glutamicum

<220>
<221> CDS
<222> (101)..(1561)

<223> RXN01653

<400> 419
ttcattcagg gtgaatgctc tccttgtttc agatgttcaa cgctccataa agtagaccgc 60

aatctagaca aagatgtcta ttttaattaa ggagcagaac atg gcc acg gcc gag 115
                                           Met Ala Thr Ala Glu
                                           1               5

aac aca aca cag gag aat cgg aaa atc ctg ttc aac gca ttt gat atg 163
Asn Thr Thr Gln Glu Asn Arg Lys Ile Leu Phe Asn Ala Phe Asp Met
                10                  15                  20

aac tgc gtt gcg cat cag tcc cca gga ctg tgg aca cac ccg aag gat 211
Asn Cys Val Ala His Gln Ser Pro Gly Leu Trp Thr His Pro Lys Asp
            25                  30                  35

aag gcg cga gac tac aac act ctt gat tac tgg gtg cac ctt gcc aag 259
Lys Ala Arg Asp Tyr Asn Thr Leu Asp Tyr Trp Val His Leu Ala Lys
        40                  45                  50

act ttg gag aag ggc ctt ttc gac ggc ctt ttc atc gca gat gtg ctt 307
Thr Leu Glu Lys Gly Leu Phe Asp Gly Leu Phe Ile Ala Asp Val Leu
    55                  60                  65

gga act tac gat gtt tat ggt tct agt aat gaa gcg gcg ttg agc agt 355
Gly Thr Tyr Asp Val Tyr Gly Ser Ser Asn Glu Ala Ala Leu Ser Ser
70                  75                  80                  85

ggt gcg cag gtg cct gtc aat gat ccg atc ctt ctt gtt tct gcg atg 403
Gly Ala Gln Val Pro Val Asn Asp Pro Ile Leu Leu Val Ser Ala Met
                90                  95                  100

gcc tat gcc aca aag aac ctc ggg ttt ggc att act gca ggt act gcc 451
Ala Tyr Ala Thr Lys Asn Leu Gly Phe Gly Ile Thr Ala Gly Thr Ala
            105                 110                 115

tat gag cac ccg tat cct ttt gcg cgg cgt ctg gcc aca ctt gat cac 499
Tyr Glu His Pro Tyr Pro Phe Ala Arg Arg Leu Ala Thr Leu Asp His
        120                 125                 130

ctg act aat ggg cgt gtg ggg tgg aat gtg gtt act ggc tat ctt ccc 547
Leu Thr Asn Gly Arg Val Gly Trp Asn Val Val Thr Gly Tyr Leu Pro
    135                 140                 145

tct gct gct caa aac atg ggt gac acc gat cag ctg cca cat gat gag 595
Ser Ala Ala Gln Asn Met Gly Asp Thr Asp Gln Leu Pro His Asp Glu
150                 155                 160                 165

cgc tat gac aaa gca gat gaa tac ctg gaa gtg atc tac aag ctt ctc 643
Arg Tyr Asp Lys Ala Asp Glu Tyr Leu Glu Val Ile Tyr Lys Leu Leu
                170                 175                 180

gag ggc tcc tgg gaa gac gat gct gtt caa aac aat acg gag acg agt 691
Glu Gly Ser Trp Glu Asp Asp Ala Val Gln Asn Asn Thr Glu Thr Ser
            185                 190                 195

gtc ttt acg gac tcc tcc aaa gtg cac gcc att aat cat cat ggc aag 739
Val Phe Thr Asp Ser Ser Lys Val His Ala Ile Asn His His Gly Lys
        200                 205                 210

tac ttt gat gtg ccg ggc att gcc atc act gag ccg agt gtg cag cgt 787
Tyr Phe Asp Val Pro Gly Ile Ala Ile Thr Glu Pro Ser Val Gln Arg
    215                 220                 225

acg ccg gtg atc tac cag gcg ggt gca tcg ccg cgc gga ttg aaa ttc 835
Thr Pro Val Ile Tyr Gln Ala Gly Ala Ser Pro Arg Gly Leu Lys Phe
230                 235                 240                 245

gct ggt gag aat gca gaa gca gtg ttt atc aat tcc agc acc gtg gag 883
Ala Gly Glu Asn Ala Glu Ala Val Phe Ile Asn Ser Ser Thr Val Glu
                250                 255                 260

gca atc acc aag act gtc gca aaa att cgc gct gct gcg gtc gct gcg 931
Ala Ile Thr Lys Thr Val Ala Lys Ile Arg Ala Ala Ala Val Ala Ala
            265                 270                 275

gga cgt gat cca cat gcg gtg aag atc ttt gcg atg caa acc atc atc     979
Gly Arg Asp Pro His Ala Val Lys Ile Phe Ala Met Gln Thr Ile Ile
        280                 285                 290

act ggt gaa aca gaa gca gat gcg cag gca aag ctg gag gaa tac agt     1027
Thr Gly Glu Thr Glu Ala Asp Ala Gln Ala Lys Leu Glu Glu Tyr Ser
    295                 300                 305

cgc tat atc gat cct gtc ggt ggt ctg acc ttg atg tct gga tgg acc     1075
Arg Tyr Ile Asp Pro Val Gly Gly Leu Thr Leu Met Ser Gly Trp Thr
310                 315                 320                 325

ggc gcg gat ctg tcg cag tat gac ctg gat gaa ccg atc acc aat att     1123
Gly Ala Asp Leu Ser Gln Tyr Asp Leu Asp Glu Pro Ile Thr Asn Ile
                330                 335                 340

gag tca aac gct att cag tcc act gca gcc acc att agc aac ggc acc     1171
Glu Ser Asn Ala Ile Gln Ser Thr Ala Ala Thr Ile Ser Asn Gly Thr
            345                 350                 355

ggt gaa ggt gcg tgg acg gta cgc aaa ctg ggt gag gca acc ggc atc     1219
Gly Glu Gly Ala Trp Thr Val Arg Lys Leu Gly Glu Ala Thr Gly Ile
        360                 365                 370

ggc ggc ttc gga cca gtg ctt gtg gga tct ggc gct aac gtt gcc gcg     1267
Gly Gly Phe Gly Pro Val Leu Val Gly Ser Gly Ala Asn Val Ala Ala
    375                 380                 385

gaa ctt gca cgc atc cag gat ctc agc gat gtt gat ggt ttc aac ctt     1315
Glu Leu Ala Arg Ile Gln Asp Leu Ser Asp Val Asp Gly Phe Asn Leu
390                 395                 400                 405

gct tat gcc atc acc cca gga act ttt gaa gat gtc gtg gac ttt gtg     1363
Ala Tyr Ala Ile Thr Pro Gly Thr Phe Glu Asp Val Val Asp Phe Val
                410                 415                 420

gtg cct gag ctg caa aaa ctt agc cgc tac aag acg gaa tac gcg ccg     1411
Val Pro Glu Leu Gln Lys Leu Ser Arg Tyr Lys Thr Glu Tyr Ala Pro
            425                 430                 435

ggt tcc ttg cgc aac aaa ttg ctc ggt aaa ggt gat cgc ctg gac gat     1459
Gly Ser Leu Arg Asn Lys Leu Leu Gly Lys Gly Asp Arg Leu Asp Asp
        440                 445                 450

acc cac cgc ggc gca agc tac cgc cta ggc gct cgg aac tcc acc gcc     1507
Thr His Arg Gly Ala Ser Tyr Arg Leu Gly Ala Arg Asn Ser Thr Ala
    455                 460                 465

act att gat ctc agt tcc ata tcc gcc caa cta gtt tcc cag gga gcc     1555
Thr Ile Asp Leu Ser Ser Ile Ser Ala Gln Leu Val Ser Gln Gly Ala
470                 475                 480                 485

cac tca tgatctcacc gcaaacaatc atc                                   1584
His Ser

<210> 420
<211> 487
<212> PRT
<213> Corynebacterium glutamicum

<400> 420
Met Ala Thr Ala Glu Asn Thr Thr Gln Glu Asn Arg Lys Ile Leu Phe
1               5                   10                  15

Asn Ala Phe Asp Met Asn Cys Val Ala His Gln Ser Pro Gly Leu Trp
            20                  25                  30

Thr His Pro Lys Asp Lys Ala Arg Asp Tyr Asn Thr Leu Asp Tyr Trp
        35                  40                  45

Val His Leu Ala Lys Thr Leu Glu Lys Gly Leu Phe Asp Gly Leu Phe
    50                  55                  60

Ile Ala Asp Val Leu Gly Thr Tyr Asp Val Tyr Gly Ser Ser Asn Glu
65                  70                  75                  80

Ala Ala Leu Ser Ser Gly Ala Gln Val Pro Val Asn Asp Pro Ile Leu
                85                  90                  95

Leu Val Ser Ala Met Ala Tyr Ala Thr Lys Asn Leu Gly Phe Gly Ile
            100                 105                 110

Thr Ala Gly Thr Ala Tyr Glu His Pro Tyr Pro Phe Ala Arg Arg Leu
        115                 120                 125

Ala Thr Leu Asp His Leu Thr Asn Gly Arg Val Gly Trp Asn Val Val
    130                 135                 140

Thr Gly Tyr Leu Pro Ser Ala Ala Gln Asn Met Gly Asp Thr Asp Gln
145                 150                 155                 160

Leu Pro His Asp Glu Arg Tyr Asp Lys Ala Asp Glu Tyr Leu Glu Val
                165                 170                 175

Ile Tyr Lys Leu Leu Glu Gly Ser Trp Glu Asp Asp Ala Val Gln Asn
            180                 185                 190

Asn Thr Glu Thr Ser Val Phe Thr Asp Ser Ser Lys Val His Ala Ile
        195                 200                 205

Asn His His Gly Lys Tyr Phe Asp Val Pro Gly Ile Ala Ile Thr Glu
    210                 215                 220

Pro Ser Val Gln Arg Thr Pro Val Ile Tyr Gln Ala Gly Ala Ser Pro
225                 230                 235                 240

Arg Gly Leu Lys Phe Ala Gly Glu Asn Ala Glu Ala Val Phe Ile Asn
                245                 250                 255

Ser Ser Thr Val Glu Ala Ile Thr Lys Thr Val Ala Lys Ile Arg Ala
            260                 265                 270

Ala Ala Val Ala Ala Gly Arg Asp Pro His Ala Val Lys Ile Phe Ala
        275                 280                 285

Met Gln Thr Ile Ile Thr Gly Glu Thr Glu Ala Asp Ala Gln Ala Lys
    290                 295                 300

Leu Glu Glu Tyr Ser Arg Tyr Ile Asp Pro Val Gly Gly Leu Thr Leu
305                 310                 315                 320

Met Ser Gly Trp Thr Gly Ala Asp Leu Ser Gln Tyr Asp Leu Asp Glu
                325                 330                 335

Pro Ile Thr Asn Ile Glu Ser Asn Ala Ile Gln Ser Thr Ala Ala Thr
            340                 345                 350

Ile Ser Asn Gly Thr Gly Glu Gly Ala Trp Thr Val Arg Lys Leu Gly
        355                 360                 365

Glu Ala Thr Gly Ile Gly Gly Phe Gly Pro Val Leu Val Gly Ser Gly
    370                 375                 380

Ala Asn Val Ala Ala Glu Leu Ala Arg Ile Gln Asp Leu Ser Asp Val
385                 390                 395                 400

Asp Gly Phe Asn Leu Ala Tyr Ala Ile Thr Pro Gly Thr Phe Glu Asp
                405                 410                 415

Val Val Asp Phe Val Val Pro Glu Leu Gln Lys Leu Ser Arg Tyr Lys
            420                 425                 430

Thr Glu Tyr Ala Pro Gly Ser Leu Arg Asn Lys Leu Leu Gly Lys Gly
        435                 440                 445

Asp Arg Leu Asp Asp Thr His Arg Gly Ala Ser Tyr Arg Leu Gly Ala
    450                 455                 460

Arg Asn Ser Thr Ala Thr Ile Asp Leu Ser Ser Ile Ser Ala Gln Leu
465                 470                 475                 480

Val Ser Gln Gly Ala His Ser

                485

<210> 421
<211> 702
<212> DNA
<213> Corynebacterium glutamicum

<220>
<221> CDS
<222> (101)..(679)
<223> RXN02053

<400> 421
aaccagccag aaactatctc caaaagctaa taaaaccctt gcactgacaa ataaggcgac 60

ctaccatgac tctgtttcca acacataaaa aggataaaaa atg tca ctt tca gtc 115
Met Ser Leu Ser Val
1               5

gtc gag gcg att acc aac cgc cgc gcc acc cgc aaa tac acc gat gaa 163
Val Glu Ala Ile Thr Asn Arg Arg Ala Thr Arg Lys Tyr Thr Asp Glu
                10                  15                  20

gct cct acc cct gag ctg atc gac aaa atc gtt gac ctt gcc ctg gag 211
Ala Pro Thr Pro Glu Leu Ile Asp Lys Ile Val Asp Leu Ala Leu Glu
            25                  30                  35

gca ccc agt gcg ttc aat gcg cag caa cgt gaa att gtt gtg att act 259
Ala Pro Ser Ala Phe Asn Ala Gln Gln Arg Glu Ile Val Val Ile Thr
        40                  45                  50

gat ccc gca cag aag cag aag ctt tac gag gcc tcc cat cag aaa caa 307
Asp Pro Ala Gln Lys Gln Lys Leu Tyr Glu Ala Ser His Gln Lys Gln
    55                  60                  65

ttc ctc acc gca cct gta act ttc att gcg gtt gcc cgc gtg gaa aac 355
Phe Leu Thr Ala Pro Val Thr Phe Ile Ala Val Ala Arg Val Glu Asn
70                  75                  80                  85

gag cct gag gat ttg gaa gag att ctt ggt acg gaa agg gct gaa cgt 403
Glu Pro Glu Asp Leu Glu Glu Ile Leu Gly Thr Glu Arg Ala Glu Arg
                90                  95                  100

gtc gcg gga ttc atc aac ggt cgc agc att cag cag gca cgc gaa gca 451
Val Ala Gly Phe Ile Asn Gly Arg Ser Ile Gln Gln Ala Arg Glu Ala
            105                 110                 115

acg ttg agg gat gcc agc ctc gcg gcg gct ttt cta att ctg gct gcc 499
Thr Leu Arg Asp Ala Ser Leu Ala Ala Ala Phe Leu Ile Leu Ala Ala
        120                 125                 130

cag gcg gag ggt ttg agt acc agc ccg act act ggt tgg gat gag gaa 547
Gln Ala Glu Gly Leu Ser Thr Ser Pro Thr Thr Gly Trp Asp Glu Glu
    135                 140                 145

aaa gtg aag gaa gca atc ggt ctc ggc ggg cgt gag gat cgt gca atc 595
Lys Val Lys Glu Ala Ile Gly Leu Gly Gly Arg Glu Asp Arg Ala Ile
150                 155                 160                 165

gcc ctt gtt att gct acc gga ttc cct aat gaa cag ccg gag cac cct 643
Ala Leu Val Ile Ala Thr Gly Phe Pro Asn Glu Gln Pro Glu His Pro
                170                 175                 180

ggt cgt ttg cag aat agg cgc atc gac aac agc tac taactctgcc 689
Gly Arg Leu Gln Asn Arg Arg Ile Asp Asn Ser Tyr
            185                 190

agctcgcccg gac 702

<210> 422
<211> 193
<212> PRT
<213> Corynebacterium glutamicum

<400> 422

Met Ser Leu Ser Val Val Glu Ala Ile Thr Asn Arg Arg Ala Thr Arg
1               5                   10                  15

Lys Tyr Thr Asp Glu Ala Pro Thr Pro Glu Leu Ile Asp Lys Ile Val
            20                  25                  30

Asp Leu Ala Leu Glu Ala Pro Ser Ala Phe Asn Ala Gln Gln Arg Glu
        35                  40                  45

Ile Val Val Ile Thr Asp Pro Ala Gln Lys Gln Lys Leu Tyr Glu Ala
    50                  55                  60

Ser His Gln Lys Gln Phe Leu Thr Ala Pro Val Thr Phe Ile Ala Val
65                  70                  75                  80

Ala Arg Val Glu Asn Glu Pro Glu Asp Leu Glu Glu Ile Leu Gly Thr
                85                  90                  95

Glu Arg Ala Glu Arg Val Ala Gly Phe Ile Asn Gly Arg Ser Ile Gln
            100                 105                 110

Gln Ala Arg Glu Ala Thr Leu Arg Asp Ala Ser Leu Ala Ala Ala Phe
        115                 120                 125

Leu Ile Leu Ala Ala Gln Ala Glu Gly Leu Ser Thr Ser Pro Thr Thr
    130                 135                 140

Gly Trp Asp Glu Glu Lys Val Lys Glu Ala Ile Gly Leu Gly Gly Arg
145                 150                 155                 160

Glu Asp Arg Ala Ile Ala Leu Val Ile Ala Thr Gly Phe Pro Asn Glu
                165                 170                 175

Gln Pro Glu His Pro Gly Arg Leu Gln Asn Arg Arg Ile Asp Asn Ser
            180                 185                 190

Tyr

<210> 423
<211> 1191
<212> DNA
<213> Corynebacterium glutamicum

<220>
<221> CDS
<222> (101)..(1168)
<223> RXN00177

<400> 423
ctattccaca gcagtggacg ctgaacaact tcaaacacag attaagcagc tatcggatct 60

acttcacctc aactcagttg tcggagcata ggagctaaaa atg tct tta cag ttc   115
                                            Met Ser Leu Gln Phe
                                            1               5

gat cat gaa acc ctc ggt caa cga gtt ctg ttc ggt tca ggt gag gcg   163
Asp His Glu Thr Leu Gly Gln Arg Val Leu Phe Gly Ser Gly Glu Ala
                10                  15                  20

gcg caa aat ctc gcc gct gaa att agc cga ctc gat gcc aaa aac gtc   211
Ala Gln Asn Leu Ala Ala Glu Ile Ser Arg Leu Asp Ala Lys Asn Val
            25                  30                  35

atg gtg gtt gcc ggt gat ttc gag ctt ccc atg gca cgg caa gta gca   259
Met Val Val Ala Gly Asp Phe Glu Leu Pro Met Ala Arg Gln Val Ala
        40                  45                  50

gca gat att gat gtc aag gtg tgg cat tca aat gtc gtg atg cat gtg   307
Ala Asp Ile Asp Val Lys Val Trp His Ser Asn Val Val Met His Val
    55                  60                  65

ccc atc gaa aca gca gaa gaa gca cgc agt gtt gcg aaa gaa aac gac   355
Pro Ile Glu Thr Ala Glu Glu Ala Arg Ser Val Ala Lys Glu Asn Asp
70                  75                  80                  85

att gat gtt gtg gtg tgt gtg ggc ggt gga tcc aca aca ggt cta gct    403
Ile Asp Val Val Val Cys Val Gly Gly Gly Ser Thr Thr Gly Leu Ala
                    90                  95                  100

aaa gcg att gcc atg acc acc gca ttg ccg atc att gcg gta ccc act    451
Lys Ala Ile Ala Met Thr Thr Ala Leu Pro Ile Ile Ala Val Pro Thr
            105                 110                 115

act tat gca ggt tct gaa gca aca aat gtg tgg gga ttg acc gaa gcc    499
Thr Tyr Ala Gly Ser Glu Ala Thr Asn Val Trp Gly Leu Thr Glu Ala
        120                 125                 130

gcg cgc aaa aca act ggt gtt gat aac aaa gtg ctg cca gtg aca gtt    547
Ala Arg Lys Thr Thr Gly Val Asp Asn Lys Val Leu Pro Val Thr Val
    135                 140                 145

atc tac gat tca gcg tta acc atg tct ttg ccg gta gaa atg tcg gtt    595
Ile Tyr Asp Ser Ala Leu Thr Met Ser Leu Pro Val Glu Met Ser Val
150                 155                 160                 165

gct tct ggt ctc aat ggt ttg gct cac tgc att gat tct ttg tgg gga    643
Ala Ser Gly Leu Asn Gly Leu Ala His Cys Ile Asp Ser Leu Trp Gly
                170                 175                 180

ccg aag gcg gat ccc atc aat gcg gct atg gct gct gag gga att cga    691
Pro Lys Ala Asp Pro Ile Asn Ala Ala Met Ala Ala Glu Gly Ile Arg
            185                 190                 195

gca ctt tct gct ggc ctt ccc aag att gtg gca gat gct cag gac gta    739
Ala Leu Ser Ala Gly Leu Pro Lys Ile Val Ala Asp Ala Gln Asp Val
        200                 205                 210

gat ggt cgc gat gaa gcg ctc tac ggt gcc tac ctg gct gcg gtg tct    787
Asp Gly Arg Asp Glu Ala Leu Tyr Gly Ala Tyr Leu Ala Ala Val Ser
    215                 220                 225

ttt gcc tct gct ggc tct ggt ctc cac cac aag atc tgc cac gtg ttg    835
Phe Ala Ser Ala Gly Ser Gly Leu His His Lys Ile Cys His Val Leu
230                 235                 240                 245

ggt gga act ttt aac ctt cca cac gcg caa acc cat gca aca gta ctg    883
Gly Gly Thr Phe Asn Leu Pro His Ala Gln Thr His Ala Thr Val Leu
                250                 255                 260

cct tat gtt ctt gcc ttc aac gcg cca tat gcg cca cag gca gaa caa    931
Pro Tyr Val Leu Ala Phe Asn Ala Pro Tyr Ala Pro Gln Ala Glu Gln
            265                 270                 275

cgc gca gcg gca gct ttc ggt tct gcg aca gca ctt gaa gga ttg caa    979
Arg Ala Ala Ala Ala Phe Gly Ser Ala Thr Ala Leu Glu Gly Leu Gln
        280                 285                 290

cag ctg cgt gcc caa gtg gga gca cca cag cga cta tcc gat tac gga    1027
Gln Leu Arg Ala Gln Val Gly Ala Pro Gln Arg Leu Ser Asp Tyr Gly
    295                 300                 305

ttc acc gca gca gga atc cca gag gca gtg gaa atc atc ttg gag aaa    1075
Phe Thr Ala Ala Gly Ile Pro Glu Ala Val Glu Ile Ile Leu Glu Lys
310                 315                 320                 325

gta ccg gcg aat aat cca cgg acg gtc aca gaa gaa aac ctc act gcg    1123
Val Pro Ala Asn Asn Pro Arg Thr Val Thr Glu Glu Asn Leu Thr Ala
                330                 335                 340

ctg ctt acc aca gcg ctc aac ggc gac gat cca gca act ttg aat        1168
Leu Leu Thr Thr Ala Leu Asn Gly Asp Asp Pro Ala Thr Leu Asn
            345                 350                 355

taaggagacc aacatgacta ttt                                          1191

<210> 424
<211> 356
<212> PRT
<213> Corynebacterium glutamicum

<400> 424

Met Ser Leu Gln Phe Asp His Glu Thr Leu Gly Gln Arg Val Leu Phe
1               5                   10                  15

Gly Ser Gly Glu Ala Ala Gln Asn Leu Ala Ala Glu Ile Ser Arg Leu
            20                  25                  30

Asp Ala Lys Asn Val Met Val Val Ala Gly Asp Phe Glu Leu Pro Met
        35                  40                  45

Ala Arg Gln Val Ala Ala Asp Ile Asp Val Lys Val Trp His Ser Asn
    50                  55                  60

Val Val Met His Val Pro Ile Glu Thr Ala Glu Glu Ala Arg Ser Val
65                  70                  75                  80

Ala Lys Glu Asn Asp Ile Asp Val Val Val Cys Val Gly Gly Gly Ser
                85                  90                  95

Thr Thr Gly Leu Ala Lys Ala Ile Ala Met Thr Thr Ala Leu Pro Ile
            100                 105                 110

Ile Ala Val Pro Thr Thr Tyr Ala Gly Ser Glu Ala Thr Asn Val Trp
        115                 120                 125

Gly Leu Thr Glu Ala Ala Arg Lys Thr Thr Gly Val Asp Asn Lys Val
    130                 135                 140

Leu Pro Val Thr Val Ile Tyr Asp Ser Ala Leu Thr Met Ser Leu Pro
145                 150                 155                 160

Val Glu Met Ser Val Ala Ser Gly Leu Asn Gly Leu Ala His Cys Ile
                165                 170                 175

Asp Ser Leu Trp Gly Pro Lys Ala Asp Pro Ile Asn Ala Ala Met Ala
            180                 185                 190

Ala Glu Gly Ile Arg Ala Leu Ser Ala Gly Leu Pro Lys Ile Val Ala
        195                 200                 205

Asp Ala Gln Asp Val Asp Gly Arg Asp Glu Ala Leu Tyr Gly Ala Tyr
    210                 215                 220

Leu Ala Ala Val Ser Phe Ala Ser Ala Gly Ser Gly Leu His His Lys
225                 230                 235                 240

Ile Cys His Val Leu Gly Gly Thr Phe Asn Leu Pro His Ala Gln Thr
                245                 250                 255

His Ala Thr Val Leu Pro Tyr Val Leu Ala Phe Asn Ala Pro Tyr Ala
            260                 265                 270

Pro Gln Ala Glu Gln Arg Ala Ala Ala Ala Phe Gly Ser Ala Thr Ala
        275                 280                 285

Leu Glu Gly Leu Gln Gln Leu Arg Ala Gln Val Gly Ala Pro Gln Arg
    290                 295                 300

Leu Ser Asp Tyr Gly Phe Thr Ala Ala Gly Ile Pro Glu Ala Val Glu
305                 310                 315                 320

Ile Ile Leu Glu Lys Val Pro Ala Asn Asn Pro Arg Thr Val Thr Glu
                325                 330                 335

Glu Asn Leu Thr Ala Leu Leu Thr Thr Ala Leu Asn Gly Asp Asp Pro
            340                 345                 350

Ala Thr Leu Asn
        355

<210> 425
<211> 960
<212> DNA
<213> Corynebacterium glutamicum

<220>
<221> CDS

<222> (101)..(937)
<223> RXC00963

<400> 425
ctggctctga cggcgtcgac ttgttctgct tctctgatac accaattttc gaggccctta 60

acctcgcacg tacttttact ccggaaggaa tctagaactt atg cgt ctt gca aca 115
Met Arg Leu Ala Thr
1               5

atc cgc acc aac ggc acc acc att gct gct cgt gtt gaa tct gaa aac 163
Ile Arg Thr Asn Gly Thr Thr Ile Ala Ala Arg Val Glu Ser Glu Asn
                10                  15                  20

acc gct acc acc atc gag ggc ttt gcc aac gtc ggt gaa tta ctc cag 211
Thr Ala Thr Thr Ile Glu Gly Phe Ala Asn Val Gly Glu Leu Leu Gln
            25                  30                  35

gaa tcc aac tgg cgc gag ctg gca gaa aac gct gct ggt gag gct gtg 259
Glu Ser Asn Trp Arg Glu Leu Ala Glu Asn Ala Ala Gly Glu Ala Val
        40                  45                  50

acc ttt gaa aac aag gag cta gat gca gta gtt cca gca cct aag aag 307
Thr Phe Glu Asn Lys Glu Leu Asp Ala Val Val Pro Ala Pro Lys Lys
    55                  60                  65

att gtg tgc gtc ggc ctt aac tac gcc aac cac att aaa gaa atg ggc 355
Ile Val Cys Val Gly Leu Asn Tyr Ala Asn His Ile Lys Glu Met Gly
70                  75                  80                  85

cgc gac ctc cct gat acc cca acc ctt ttt gtt aag ttc cct gac gcg 403
Arg Asp Leu Pro Asp Thr Pro Thr Leu Phe Val Lys Phe Pro Asp Ala
                90                  95                  100

ctc atc gga cct ttc gat gat gtt gtc gtt cca gag tgg gct aac aag 451
Leu Ile Gly Pro Phe Asp Asp Val Val Val Pro Glu Trp Ala Asn Lys
            105                 110                 115

gct ctc gac tgg gaa ggc gag atg gca gtt atc att ggc aag cgc gca 499
Ala Leu Asp Trp Glu Gly Glu Met Ala Val Ile Ile Gly Lys Arg Ala
        120                 125                 130

cgc cgt gtc aag cag gcc gat gct gct gag tac atc gct ggc tac gca 547
Arg Arg Val Lys Gln Ala Asp Ala Ala Glu Tyr Ile Ala Gly Tyr Ala
    135                 140                 145

gtg atg aac gat tac acc acc cgc gat ttc cag tac gca gca cct gca 595
Val Met Asn Asp Tyr Thr Thr Arg Asp Phe Gln Tyr Ala Ala Pro Ala
150                 155                 160                 165

aag act cca cag tgg cac cag ggc aag tct ttg gaa aag tcc gct ggc 643
Lys Thr Pro Gln Trp His Gln Gly Lys Ser Leu Glu Lys Ser Ala Gly
                170                 175                 180

ttc ggg cct tgg atg act acc cca gat tct ttt gag ttc ggc ggc gag 691
Phe Gly Pro Trp Met Thr Thr Pro Asp Ser Phe Glu Phe Gly Gly Glu
            185                 190                 195

ctg gca acc tac ctc gag ggc gag aag gta cag tcc acc cct acc aat 739
Leu Ala Thr Tyr Leu Glu Gly Glu Lys Val Gln Ser Thr Pro Thr Asn
        200                 205                 210

gac ctg gtc ttt agc cca gaa aag ctc atc gaa tac atc acc cac atc 787
Asp Leu Val Phe Ser Pro Glu Lys Leu Ile Glu Tyr Ile Thr His Ile
    215                 220                 225

tac cca ttg gat gct ggc gac gtc att gtc acc ggt acc cca ggc ggc 835
Tyr Pro Leu Asp Ala Gly Asp Val Ile Val Thr Gly Thr Pro Gly Gly
230                 235                 240                 245

gtt ggc cac gca cgt aac cca cag cgc tac atc ggt gac ggc gaa acc 883
Val Gly His Ala Arg Asn Pro Gln Arg Tyr Ile Gly Asp Gly Glu Thr
                250                 255                 260

gta aag gtt gag att gcg ggc ctc ggc ttc att gaa aac aag acg gtg 931
Val Lys Val Glu Ile Ala Gly Leu Gly Phe Ile Glu Asn Lys Thr Val
            265                 270                 275

ttt gaa taaatgacaa ctttccacga tct                                    960
Phe Glu

<210> 426
<211> 279
<212> PRT
<213> Corynebacterium glutamicum

<400> 426
   Met Arg Leu Ala Thr Ile Arg Thr Asn Gly Thr Thr Ile Ala Ala Arg
   1 5 10 15
   Val Glu Ser Glu Asn Thr Ala Thr Thr Ile Glu Gly Phe Ala Asn Val
   20 25 30
   Gly Glu Leu Leu Gln Glu Ser Asn Trp Arg Glu Leu Ala Glu Asn Ala
   35 40 45
   Ala Gly Glu Ala Val Thr Phe Glu Asn Lys Glu Leu Asp Ala Val Val
   50 55 60
   Pro Ala Pro Lys Lys Ile Val Cys Val Gly Leu Asn Tyr Ala Asn His
   65 70 75 80
   Ile Lys Glu Met Gly Arg Asp Leu Pro Asp Thr Pro Thr Leu Phe Val
   85 90 95
   Lys Phe Pro Asp Ala Leu Ile Gly Pro Phe Asp Asp Val Val Val Pro
   100 105 110
   Glu Trp Ala Asn Lys Ala Leu Asp Trp Glu Gly Glu Met Ala Val Ile
   115 120 125
   Ile Gly Lys Arg Ala Arg Arg Val Lys Gln Ala Asp Ala Ala Glu Tyr
   130 135 140
   Ile Ala Gly Tyr Ala Val Met Asn Asp Tyr Thr Thr Arg Asp Phe Gln
   145 150 155 160
   Tyr Ala Ala Pro Ala Lys Thr Pro Gln Trp His Gln Gly Lys Ser Leu
   165 170 175
   Glu Lys Ser Ala Gly Phe Gly Pro Trp Met Thr Thr Pro Asp Ser Phe
   180 185 190
   Glu Phe Gly Gly Glu Leu Ala Thr Tyr Leu Glu Gly Glu Lys Val Gln
   195 200 205
   Ser Thr Pro Thr Asn Asp Leu Val Phe Ser Pro Glu Lys Leu Ile Glu
   210 215 220
   Tyr Ile Thr His Ile Tyr Pro Leu Asp Ala Gly Asp Val Ile Val Thr
   225 230 235 240
   Gly Thr Pro Gly Gly Val Gly His Ala Arg Asn Pro Gln Arg Tyr Ile
   245 250 255
   Gly Asp Gly Glu Thr Val Lys Val Glu Ile Ala Gly Leu Gly Phe Ile
   260 265 270
   Glu Asn Lys Thr Val Phe Glu
   275

<210> 427
<211> 1101
<212> DNA
<213> Corynebacterium glutamicum

<220>
<221> CDS
<222> (101)..(1078)
<223> RXN00299

<400> 427
tgccatcggt ttggctattg attggaacaa gaaaggtgcc cagtctgttg caaagaagga 60

atccatttcc gtctaatcgc taattgcgag gagtctttgc atg tct atc cca ctt 115
Met Ser Ile Pro Leu
1               5

tca ctg att gat ttt gcc acc att ttt gag ggc gaa agg cct ggt gac 163
Ser Leu Ile Asp Phe Ala Thr Ile Phe Glu Gly Glu Arg Pro Gly Asp
                10                  15                  20

agc ttc aaa cga tca gtg gca ttg gcg caa aaa gct gaa ggt tta ggc 211
Ser Phe Lys Arg Ser Val Ala Leu Ala Gln Lys Ala Glu Gly Leu Gly
            25                  30                  35

ttc aag cgc att tgg tac gca gag cat cac aac atg gag agc att tct 259
Phe Lys Arg Ile Trp Tyr Ala Glu His His Asn Met Glu Ser Ile Ser
        40                  45                  50

tca gct gct cct gca gtg ctt att tct cac atc ggt gcg aac acc aag 307
Ser Ala Ala Pro Ala Val Leu Ile Ser His Ile Gly Ala Asn Thr Lys
    55                  60                  65

act att cgt ctg ggt gcc ggc ggc gtc atg ctg ccc aac cac tcc cca 355
Thr Ile Arg Leu Gly Ala Gly Gly Val Met Leu Pro Asn His Ser Pro
70                  75                  80                  85

tat gtc atc gct gag cag ttc ggc acc ttg gcg gag ttg tac cca gac 403
Tyr Val Ile Ala Glu Gln Phe Gly Thr Leu Ala Glu Leu Tyr Pro Asp
                90                  95                  100

cgc atc gac ctc ggc ctg ggc cgt gcc cct ggc acg gac atg aat acc 451
Arg Ile Asp Leu Gly Leu Gly Arg Ala Pro Gly Thr Asp Met Asn Thr
            105                 110                 115

ttg cgc gct tta cga cgc gac cct cag tcc gcc gag aac ttc ccg tcc 499
Leu Arg Ala Leu Arg Arg Asp Pro Gln Ser Ala Glu Asn Phe Pro Ser
        120                 125                 130

gac gtt gtc gag ctg aac tct tac ctc acc ggc cgt tcc cgt ctc cca 547
Asp Val Val Glu Leu Asn Ser Tyr Leu Thr Gly Arg Ser Arg Leu Pro
    135                 140                 145

ggg gtt aac gca att cca ggc aag ggc acc aac gta ccg ctg tac atc 595
Gly Val Asn Ala Ile Pro Gly Lys Gly Thr Asn Val Pro Leu Tyr Ile
150                 155                 160                 165

ttg ggt tca tcc ctc ttt ggt gca caa ttg gca gca cag ttg ggt atg 643
Leu Gly Ser Ser Leu Phe Gly Ala Gln Leu Ala Ala Gln Leu Gly Met
                170                 175                 180

cct tat tcc ttc gca tcc cac ttc gca cca act cac ctt gag cac gcg 691
Pro Tyr Ser Phe Ala Ser His Phe Ala Pro Thr His Leu Glu His Ala
            185                 190                 195

gtg caa acc tac cgg gat aac tac cag cct tca gag cag cat cct gag 739
Val Gln Thr Tyr Arg Asp Asn Tyr Gln Pro Ser Glu Gln His Pro Glu
        200                 205                 210

cct tat gtc att gcg gcc gtc aat gtc acc gca tct gat tcc act gaa 787
Pro Tyr Val Ile Ala Ala Val Asn Val Thr Ala Ser Asp Ser Thr Glu
    215                 220                 225

caa gcc cac gat gat ttc tac aag gta gcg cgt gca cgc gtg aag aac 835
Gln Ala His Asp Asp Phe Tyr Lys Val Ala Arg Ala Arg Val Lys Asn
230                 235                 240                 245

atg gca ttg cgt ggc cga caa gtt act gat gag caa ctt gat gaa ctc 883
Met Ala Leu Arg Gly Arg Gln Val Thr Asp Glu Gln Leu Asp Glu Leu
                250                 255                 260

atg gat tca cca gct gct cgc caa att gtc gac atg ctt cac tac acc 931
Met Asp Ser Pro Ala Ala Arg Gln Ile Val Asp Met Leu His Tyr Thr
            265                 270                 275

gct ata ggc act gga tcc gaa gtt aaa gaa tac cta gac ggt ttt gta 979
Ala Ile Gly Thr Gly Ser Glu Val Lys Glu Tyr Leu Asp Gly Phe Val

280                 285                 290

aag acg gca cag gct gat gaa ctg atg atc tcc ctg caa tcc ccc aac     1027
Lys Thr Ala Gln Ala Asp Glu Leu Met Ile Ser Leu Gln Ser Pro Asn
    295                 300                 305

act gaa gca acc acg cgc aat atg gaa att ctt gcg gat gcg tgg att     1075
Thr Glu Ala Thr Thr Arg Asn Met Glu Ile Leu Ala Asp Ala Trp Ile
310                 315                 320                 325

aat tagtaccgat gggccggtag aca                                       1101
Asn

<210> 428
<211> 326
<212> PRT
<213> Corynebacterium glutamicum

<400> 428
Met Ser Ile Pro Leu Ser Leu Ile Asp Phe Ala Thr Ile Phe Glu Gly
1               5                   10                  15

Glu Arg Pro Gly Asp Ser Phe Lys Arg Ser Val Ala Leu Ala Gln Lys
            20                  25                  30

Ala Glu Gly Leu Gly Phe Lys Arg Ile Trp Tyr Ala Glu His His Asn
        35                  40                  45

Met Glu Ser Ile Ser Ser Ala Ala Pro Ala Val Leu Ile Ser His Ile
    50                  55                  60

Gly Ala Asn Thr Lys Thr Ile Arg Leu Gly Ala Gly Gly Val Met Leu
65                  70                  75                  80

Pro Asn His Ser Pro Tyr Val Ile Ala Glu Gln Phe Gly Thr Leu Ala
                85                  90                  95

Glu Leu Tyr Pro Asp Arg Ile Asp Leu Gly Leu Gly Arg Ala Pro Gly
            100                 105                 110

Thr Asp Met Asn Thr Leu Arg Ala Leu Arg Arg Asp Pro Gln Ser Ala
        115                 120                 125

Glu Asn Phe Pro Ser Asp Val Val Glu Leu Asn Ser Tyr Leu Thr Gly
    130                 135                 140

Arg Ser Arg Leu Pro Gly Val Asn Ala Ile Pro Gly Lys Gly Thr Asn
145                 150                 155                 160

Val Pro Leu Tyr Ile Leu Gly Ser Ser Leu Phe Gly Ala Gln Leu Ala
                165                 170                 175

Ala Gln Leu Gly Met Pro Tyr Ser Phe Ala Ser His Phe Ala Pro Thr
            180                 185                 190

His Leu Glu His Ala Val Gln Thr Tyr Arg Asp Asn Tyr Gln Pro Ser
        195                 200                 205

Glu Gln His Pro Glu Pro Tyr Val Ile Ala Ala Val Asn Val Thr Ala
    210                 215                 220

Ser Asp Ser Thr Glu Gln Ala His Asp Asp Phe Tyr Lys Val Ala Arg
225                 230                 235                 240

Ala Arg Val Lys Asn Met Ala Leu Arg Gly Arg Gln Val Thr Asp Glu
                245                 250                 255

Gln Leu Asp Glu Leu Met Asp Ser Pro Ala Ala Arg Gln Ile Val Asp
            260                 265                 270

Met Leu His Tyr Thr Ala Ile Gly Thr Gly Ser Glu Val Lys Glu Tyr
        275                 280                 285

Leu Asp Gly Phe Val Lys Thr Ala Gln Ala Asp Glu Leu Met Ile Ser
    290                 295                 300

Leu Gln Ser Pro Asn Thr Glu Ala Thr Thr Arg Asn Met Glu Ile Leu
305                 310                 315                 320

Ala Asp Ala Trp Ile Asn
                325

<210> 429
<211> 784
<212> DNA
<213> Corynebacterium glutamicum

<220>
<221> CDS
<222> (18)..(761)
<223> FRXA00299

<400> 429
gggtgccggc ggcggtc atg ctg ccc aac cac tcc cca tat gtc atc gct gag 53
                   Met Leu Pro Asn His Ser Pro Tyr Val Ile Ala Glu
                   1               5                   10

cag ttc ggc acc ttg gcg gag ttg tac cca gac cgc atc gac ctc ggc   101
Gln Phe Gly Thr Leu Ala Glu Leu Tyr Pro Asp Arg Ile Asp Leu Gly
        15                  20                  25

atg ggc cgt gcc cct ggc acg gac atg aat acc ttg cgc gct tta cga   149
Met Gly Arg Ala Pro Gly Thr Asp Met Asn Thr Leu Arg Ala Leu Arg
    30                  35                  40

cgc gac cct cag tcc gcc gag aac ttc ccg tcc gac gtt gtc gag ctg   197
Arg Asp Pro Gln Ser Ala Glu Asn Phe Pro Ser Asp Val Val Glu Leu
45                  50                  55                  60

aac tct tac ctc acc ggc cgt tcc cgt ctc cca ggg gtt aac gca att   245
Asn Ser Tyr Leu Thr Gly Arg Ser Arg Leu Pro Gly Val Asn Ala Ile
                65                  70                  75

cca ggc aag ggc acc aac gta ccg ctg tac atc ttg ggt tca tcc ctc   293
Pro Gly Lys Gly Thr Asn Val Pro Leu Tyr Ile Leu Gly Ser Ser Leu
            80                  85                  90

ttt ggt gca caa ttg gca gca cag ttg ggt atg cct tat tcc ttc gca   341
Phe Gly Ala Gln Leu Ala Ala Gln Leu Gly Met Pro Tyr Ser Phe Ala
        95                  100                 105

tcc cac ttc gca cca act cac ctt gag cac gcg gtg caa acc tac cgg   389
Ser His Phe Ala Pro Thr His Leu Glu His Ala Val Gln Thr Tyr Arg
    110                 115                 120

gat aac tac cag cct tca gag cag cat cct gag cct tat gtc att gcg   437
Asp Asn Tyr Gln Pro Ser Glu Gln His Pro Glu Pro Tyr Val Ile Ala
125                 130                 135                 140

gcc gtc aat gtc acc gca tct gat tcc act gaa caa gcc cac gat gat   485
Ala Val Asn Val Thr Ala Ser Asp Ser Thr Glu Gln Ala His Asp Asp
                145                 150                 155

ttc tac aag gta gcg cgt gca cgc gtg aag aac atg gca ttg cgt ggc   533
Phe Tyr Lys Val Ala Arg Ala Arg Val Lys Asn Met Ala Leu Arg Gly
            160                 165                 170

cga caa gtt act gat gag caa ctt gat gaa ctc atg gat tca cca gct   581
Arg Gln Val Thr Asp Glu Gln Leu Asp Glu Leu Met Asp Ser Pro Ala
        175                 180                 185

gct cgc caa att gtc gac atg ctt cac tac acc gct ata ggc act gga   629
Ala Arg Gln Ile Val Asp Met Leu His Tyr Thr Ala Ile Gly Thr Gly
    190                 195                 200

tcc gaa gtt aaa gaa tac cta gac ggt ttt gta aag acg gca cag gct   677
Ser Glu Val Lys Glu Tyr Leu Asp Gly Phe Val Lys Thr Ala Gln Ala
205                 210                 215                 220

gat gaa ctg atg atc tcc ctg caa tcc ccc aac act gaa gca acc acg   725
Asp Glu Leu Met Ile Ser Leu Gln Ser Pro Asn Thr Glu Ala Thr Thr

225                 230                 235

cgc aat atg gaa att ctt gcg gat gcg tgg att aat tagtaccgat        771
Arg Asn Met Glu Ile Leu Ala Asp Ala Trp Ile Asn
            240                 245

gggccggtag aca                                                    784


<210> 430
<211> 248
<212> PRT
<213> Corynebacterium glutamicum

<400> 430
Met Leu Pro Asn His Ser Pro Tyr Val Ile Ala Glu Gln Phe Gly Thr
 1               5                  10                  15

Leu Ala Glu Leu Tyr Pro Asp Arg Ile Asp Leu Gly Met Gly Arg Ala
            20                  25                  30

Pro Gly Thr Asp Met Asn Thr Leu Arg Ala Leu Arg Arg Asp Pro Gln
        35                  40                  45

Ser Ala Glu Asn Phe Pro Ser Asp Val Val Glu Leu Asn Ser Tyr Leu
    50                  55                  60

Thr Gly Arg Ser Arg Leu Pro Gly Val Asn Ala Ile Pro Gly Lys Gly
65                  70                  75                  80

Thr Asn Val Pro Leu Tyr Ile Leu Gly Ser Ser Leu Phe Gly Ala Gln
                85                  90                  95

Leu Ala Ala Gln Leu Gly Met Pro Tyr Ser Phe Ala Ser His Phe Ala
            100                 105                 110

Pro Thr His Leu Glu His Ala Val Gln Thr Tyr Arg Asp Asn Tyr Gln
        115                 120                 125

Pro Ser Glu Gln His Pro Glu Pro Tyr Val Ile Ala Ala Val Asn Val
    130                 135                 140

Thr Ala Ser Asp Ser Thr Glu Gln Ala His Asp Asp Phe Tyr Lys Val
145                 150                 155                 160

Ala Arg Ala Arg Val Lys Asn Met Ala Leu Arg Gly Arg Gln Val Thr
                165                 170                 175

Asp Glu Gln Leu Asp Glu Leu Met Asp Ser Pro Ala Ala Arg Gln Ile
            180                 185                 190

Val Asp Met Leu His Tyr Thr Ala Ile Gly Thr Gly Ser Glu Val Lys
        195                 200                 205

Glu Tyr Leu Asp Gly Phe Val Lys Thr Ala Gln Ala Asp Glu Leu Met
    210                 215                 220

Ile Ser Leu Gln Ser Pro Asn Thr Glu Ala Thr Thr Arg Asn Met Glu
225                 230                 235                 240

Ile Leu Ala Asp Ala Trp Ile Asn
                245


<210> 431
<211> 825
<212> DNA
<213> Corynebacterium glutamicum

<220>
<221> CDS
<222> (101)..(802)
<223> RXA00332

<400> 431
aatgaactct ggaaccgcca tgcagcaaaa cctctccaat tggtaatctt tgactcccag 60

gttacgccag ccctgcgaca ccaccatcta gggttagagt atg gcc ttc aac aaa   115
                                          Met Ala Phe Asn Lys
                                          1               5

gcg tac gat gca ctt cgc gcc cct caa atc acc ctc gga ctc atg aca   163
Ala Tyr Asp Ala Leu Arg Ala Pro Gln Ile Thr Leu Gly Leu Met Thr
                10                  15                  20

cca aac ggc cct gaa cta ggg cgc agt gaa atg gtt cca acc gaa aat   211
Pro Asn Gly Pro Glu Leu Gly Arg Ser Glu Met Val Pro Thr Glu Asn
            25                  30                  35

agc atc gaa cta gcc ata caa gca gaa gct caa gga ttc aga ggc atg   259
Ser Ile Glu Leu Ala Ile Gln Ala Glu Ala Gln Gly Phe Arg Gly Met
        40                  45                  50

tgg gtt cga gac gtt cca ctc gca gtt cct caa gga atc act gtt acc   307
Trp Val Arg Asp Val Pro Leu Ala Val Pro Gln Gly Ile Thr Val Thr
    55                  60                  65

gat aaa cag gct acg tat tta gat gat cca ttc tta atg ctc ggt gcg   355
Asp Lys Gln Ala Thr Tyr Leu Asp Asp Pro Phe Leu Met Leu Gly Ala
70                  75                  80                  85

atg gcc tct gtg acc tct aca atc gcg ctg ggc act gca gcg acc gtg   403
Met Ala Ser Val Thr Ser Thr Ile Ala Leu Gly Thr Ala Ala Thr Val
                90                  95                  100

ctt cca ctc aga cat ccg cta cat gtg gcg aaa tcc gcg ctc acc ctt   451
Leu Pro Leu Arg His Pro Leu His Val Ala Lys Ser Ala Leu Thr Leu
            105                 110                 115

gat cga ctc agc cac gga cgt ttc gtt tta ggc atc ggc tct ggc gac   499
Asp Arg Leu Ser His Gly Arg Phe Val Leu Gly Ile Gly Ser Gly Asp
        120                 125                 130

agg cct gaa gaa ttc gag att ttt ggc aaa agc tta gac aat cga cgc   547
Arg Pro Glu Glu Phe Glu Ile Phe Gly Lys Ser Leu Asp Asn Arg Arg
    135                 140                 145

gct gat att cag tct ggg tgg gca att ttg cgt gca gct ttg tcg ccg   595
Ala Asp Ile Gln Ser Gly Trp Ala Ile Leu Arg Ala Ala Leu Ser Pro
150                 155                 160                 165

gat cct gcg atg cgg gcc gac ctt gaa ttt gcg cca acc acg cca cct   643
Asp Pro Ala Met Arg Ala Asp Leu Glu Phe Ala Pro Thr Thr Pro Pro
                170                 175                 180

gaa gct cag atc ccc atg atc gct gta ggt tct gcc cga caa aca gtg   691
Glu Ala Gln Ile Pro Met Ile Ala Val Gly Ser Ala Arg Gln Thr Val
            185                 190                 195

caa tgg atc gcc cga aac gcc gac gga tgg gca acc tac tac cgc ccc   739
Gln Trp Ile Ala Arg Asn Ala Asp Gly Trp Ala Thr Tyr Tyr Arg Pro
        200                 205                 210

gct gaa gct caa gtc gga cgc ctc gat ctc tgg gac aaa gcc cgt ggt   787
Ala Glu Ala Gln Val Gly Arg Leu Asp Leu Trp Asp Lys Ala Arg Gly
    215                 220                 225

ggc acc cgc cct tgt tgatttcctc catggggctc aac                     825
Gly Thr Arg Pro Cys
230

<210> 432
<211> 234
<212> PRT
<213> Corynebacterium glutamicum

<400> 432
Met Ala Phe Asn Lys Ala Tyr Asp Ala Leu Arg Ala Pro Gln Ile Thr
1               5                   10                  15

Leu Gly Leu Met Thr Pro Asn Gly Pro Glu Leu Gly Arg Ser Glu Met
            20                  25                  30

Val Pro Thr Glu Asn Ser Ile Glu Leu Ala Ile Gln Ala Glu Ala Gln
        35                  40                  45

Gly Phe Arg Gly Met Trp Val Arg Asp Val Pro Leu Ala Val Pro Gln
    50                  55                  60

Gly Ile Thr Val Thr Asp Lys Gln Ala Thr Tyr Leu Asp Asp Pro Phe
65                  70                  75                  80

Leu Met Leu Gly Ala Met Ala Ser Val Thr Ser Thr Ile Ala Leu Gly
                85                  90                  95

Thr Ala Ala Thr Val Leu Pro Leu Arg His Pro Leu His Val Ala Lys
            100                 105                 110

Ser Ala Leu Thr Leu Asp Arg Leu Ser His Gly Arg Phe Val Leu Gly
        115                 120                 125

Ile Gly Ser Gly Asp Arg Pro Glu Glu Phe Glu Ile Phe Gly Lys Ser
    130                 135                 140

Leu Asp Asn Arg Arg Ala Asp Ile Gln Ser Gly Trp Ala Ile Leu Arg
145                 150                 155                 160

Ala Ala Leu Ser Pro Asp Pro Ala Met Arg Ala Asp Leu Glu Phe Ala
                165                 170                 175

Pro Thr Thr Pro Pro Glu Ala Gln Ile Pro Met Ile Ala Val Gly Ser
            180                 185                 190

Ala Arg Gln Thr Val Gln Trp Ile Ala Arg Asn Ala Asp Gly Trp Ala
        195                 200                 205

Thr Tyr Tyr Arg Pro Ala Glu Ala Gln Val Gly Arg Leu Asp Leu Trp
    210                 215                 220

Asp Lys Ala Arg Gly Gly Thr Arg Pro Cys
225                 230


<210> 433
<211> 842
<212> DNA
<213> Corynebacterium glutamicum

<220>
<221> CDS
<222> (1)..(819)
<223> RXA01838

<400> 433
cag cac ctc tcc ggc ggc cgt gtt gac ctt atg atg ggc cgt ggc aac      48
Gln His Leu Ser Gly Gly Arg Val Asp Leu Met Met Gly Arg Gly Asn
  1               5                  10                  15

acc gga ccc gtt tac cca tgg ttt ggc aaa gac atc cac caa ggc atc      96
Thr Gly Pro Val Tyr Pro Trp Phe Gly Lys Asp Ile His Gln Gly Ile
             20                  25                  30

cca cta gcg att gaa aac tac cac ctc ctg cgc cgc ctc tgg cgc gaa     144
Pro Leu Ala Ile Glu Asn Tyr His Leu Leu Arg Arg Leu Trp Arg Glu
         35                  40                  45

gac gta gtc aac tgg cag ggc aaa ttc cgc aca ccg ttg cag gga tac     192
Asp Val Val Asn Trp Gln Gly Lys Phe Arg Thr Pro Leu Gln Gly Tyr
     50                  55                  60

acc tct acc cca gca cca tta gac ggc gtt gca cca ttc gtc tgg cac     240
Thr Ser Thr Pro Ala Pro Leu Asp Gly Val Ala Pro Phe Val Trp His
 65                  70                  75                  80

ggc tcc atc cgc tcc acc gaa atc gca gag caa gca gcc ttc tat ggc     288
Gly Ser Ile Arg Ser Thr Glu Ile Ala Glu Gln Ala Ala Phe Tyr Gly
                 85                  90                  95

gac ggc ttc ttc cac aac aac atc ttc tgg aac aaa gag cac acc gcc     336
Asp Gly Phe Phe His Asn Asn Ile Phe Trp Asn Lys Glu His Thr Ala

100                 105                 110

caa atg gtc aac ctc tac cgc cag cgt ttc gaa cac tac gga cac ggc     384
Gln Met Val Asn Leu Tyr Arg Gln Arg Phe Glu His Tyr Gly His Gly
        115                 120                 125

caa gca gac cag gcc atc gtg gga ctc ggt ggc caa gtc ttc atc ggc     432
Gln Ala Asp Gln Ala Ile Val Gly Leu Gly Gly Gln Val Phe Ile Gly
    130                 135                 140

gat tct gaa gaa gaa gca aag aag acc ttc cgc ccc tac ttc gac aac     480
Asp Ser Glu Glu Glu Ala Lys Lys Thr Phe Arg Pro Tyr Phe Asp Asn
145                 150                 155                 160

gcc cct gtc tac gga cac gga cca tca ctt gaa gat ttc tcc cgc ctg     528
Ala Pro Val Tyr Gly His Gly Pro Ser Leu Glu Asp Phe Ser Arg Leu
                165                 170                 175

acc cca cta acc gtc ggt acc gct gag caa gtt atc gaa cgc acc atg     576
Thr Pro Leu Thr Val Gly Thr Ala Glu Gln Val Ile Glu Arg Thr Met
            180                 185                 190

gaa ttc gcc gac tgg gta ggc gat tac cag cgc cag ctc ttc ctc atc     624
Glu Phe Ala Asp Trp Val Gly Asp Tyr Gln Arg Gln Leu Phe Leu Ile
        195                 200                 205

gac cac gcc ggc ctg cca cta gaa atg gtc ctt gat cag atc gaa cgc     672
Asp His Ala Gly Leu Pro Leu Glu Met Val Leu Asp Gln Ile Glu Arg
    210                 215                 220

ctc ggc cac gat gtc gtc cca gag gta cgc cgc cgc atg gag gag cgt     720
Leu Gly His Asp Val Val Pro Glu Val Arg Arg Arg Met Glu Glu Arg
225                 230                 235                 240

cgc cca gac cac gtt ccc tcc aac cca cca acc cac cag agc ctg aag     768
Arg Pro Asp His Val Pro Ser Asn Pro Pro Thr His Gln Ser Leu Lys
                245                 250                 255

gcc aac cga aac agc cct tac ttt cag atc aac cct ggt cag cca act     816
Ala Asn Arg Asn Ser Pro Tyr Phe Gln Ile Asn Pro Gly Gln Pro Thr
            260                 265                 270

gag tagtttttct gaaactaagg aga                                       842
Glu



<210> 434
<211> 273
<212> PRT
<213> Corynebacterium glutamicum

<400> 434
Gln His Leu Ser Gly Gly Arg Val Asp Leu Met Met Gly Arg Gly Asn
1               5                   10                  15

Thr Gly Pro Val Tyr Pro Trp Phe Gly Lys Asp Ile His Gln Gly Ile
            20                  25                  30

Pro Leu Ala Ile Glu Asn Tyr His Leu Leu Arg Arg Leu Trp Arg Glu
        35                  40                  45

Asp Val Val Asn Trp Gln Gly Lys Phe Arg Thr Pro Leu Gln Gly Tyr
    50                  55                  60

Thr Ser Thr Pro Ala Pro Leu Asp Gly Val Ala Pro Phe Val Trp His
65                  70                  75                  80

Gly Ser Ile Arg Ser Thr Glu Ile Ala Glu Gln Ala Ala Phe Tyr Gly
                85                  90                  95

Asp Gly Phe Phe His Asn Asn Ile Phe Trp Asn Lys Glu His Thr Ala
            100                 105                 110

Gln Met Val Asn Leu Tyr Arg Gln Arg Phe Glu His Tyr Gly His Gly
        115                 120                 125

Gln Ala Asp Gln Ala Ile Val Gly Leu Gly Gly Gln Val Phe Ile Gly
    130                 135                 140

Asp Ser Glu Glu Glu Ala Lys Lys Thr Phe Arg Pro Tyr Phe Asp Asn
145                 150                 155                 160

Ala Pro Val Tyr Gly His Gly Pro Ser Leu Glu Asp Phe Ser Arg Leu
                165                 170                 175

Thr Pro Leu Thr Val Gly Thr Ala Glu Gln Val Ile Glu Arg Thr Met
            180                 185                 190

Glu Phe Ala Asp Trp Val Gly Asp Tyr Gln Arg Gln Leu Phe Leu Ile
        195                 200                 205

Asp His Ala Gly Leu Pro Leu Glu Met Val Leu Asp Gln Ile Glu Arg
    210                 215                 220

Leu Gly His Asp Val Val Pro Glu Val Arg Arg Arg Met Glu Glu Arg
225                 230                 235                 240

Arg Pro Asp His Val Pro Ser Asn Pro Pro Thr His Gln Ser Leu Lys
                245                 250                 255

Ala Asn Arg Asn Ser Pro Tyr Phe Gln Ile Asn Pro Gly Gln Pro Thr
            260                 265                 270

Glu

<210> 435
<211> 1167
<212> DNA
<213> Corynebacterium glutamicum

<220>
<221> CDS
<222> (101)..(1144)
<223> RXA02643

<400> 435
tgagaacacc taaaaacctt gcgggaatac caccaacccc atattgttga tatatctaca 60

aactctttta ataagtctga tcaacaacgt gaggaaagca atg aaa aac gtc tcc   115
                                           Met Lys Asn Val Ser
                                           1               5

ttc ggc ctc gac acc ttc ggc gac aac gcc atc gac ctg cag ggc aac   163
Phe Gly Leu Asp Thr Phe Gly Asp Asn Ala Ile Asp Leu Gln Gly Asn
                10                  15                  20

ccg gtc tcc cct gca caa aca ctt cga aac atc att gat gaa gcc aag   211
Pro Val Ser Pro Ala Gln Thr Leu Arg Asn Ile Ile Asp Glu Ala Lys
            25                  30                  35

atg gca gac aaa gtc ggg gtg gat atc atc ggc atc gga gag cac cac   259
Met Ala Asp Lys Val Gly Val Asp Ile Ile Gly Ile Gly Glu His His
        40                  45                  50

cgt gag gaa tac tca gtt tct gca cct gac atc gtc atg aca gct atc   307
Arg Glu Glu Tyr Ser Val Ser Ala Pro Asp Ile Val Met Thr Ala Ile
    55                  60                  65

ctc gca tcc act gag cga ctc aaa gtc acc tct tcc gtg act gtg ctg   355
Leu Ala Ser Thr Glu Arg Leu Lys Val Thr Ser Ser Val Thr Val Leu
70                  75                  80                  85

tcc tct gat gat cct gtt cgc ctg ttt gag cgt tat tcc acc atg aat   403
Ser Ser Asp Asp Pro Val Arg Leu Phe Glu Arg Tyr Ser Thr Met Asn
                90                  95                  100

gca ctg tcc aac ggt cgc gcc gaa atc acc ttg gga cgc ggt tcc ttc   451
Ala Leu Ser Asn Gly Arg Ala Glu Ile Thr Leu Gly Arg Gly Ser Phe
            105                 110                 115

att gag tct ttc cca ttg ttt ggt ttt gat ctt cag gac tac gag cag   499

Ile Glu Ser Phe Pro Leu Phe Gly Phe Asp Leu Gln Asp Tyr Glu Gln
        120                 125                 130

ctg ttt agt gaa cgc ctt gat ttg ttc gcg aag att ctt gag gcc gac      547
Leu Phe Ser Glu Arg Leu Asp Leu Phe Ala Lys Ile Leu Glu Ala Asp
    135                 140                 145

agc cgt ggt cag ggc gtg acc tgg cat ggt gag acc cgc tcg gcg ttg      595
Ser Arg Gly Gln Gly Val Thr Trp His Gly Glu Thr Arg Ser Ala Leu
150                 155                 160                 165

gaa aac cag atg ctt tac cca cca act gag aat ggc att cac gct tgg      643
Glu Asn Gln Met Leu Tyr Pro Pro Thr Glu Asn Gly Ile His Ala Trp
                170                 175                 180

gtt gca gtg ggt ggc agc cca gaa tca gtc gtg cgc gct gct aag tat      691
Val Ala Val Gly Gly Ser Pro Glu Ser Val Val Arg Ala Ala Lys Tyr
            185                 190                 195

cgt ttc ccg ttg atg ctt gcc atc atc ggc ggt gct cct gag cgt ttc      739
Arg Phe Pro Leu Met Leu Ala Ile Ile Gly Gly Ala Pro Glu Arg Phe
        200                 205                 210

cgc ccg tat gtg gat ctg tac aag cgt gcc aac gaa cag ttc ggg cag      787
Arg Pro Tyr Val Asp Leu Tyr Lys Arg Ala Asn Glu Gln Phe Gly Gln
    215                 220                 225

cct caa aag ccc att ggt gtg cac tcc cct gga ctc att gcg gca act      835
Pro Gln Lys Pro Ile Gly Val His Ser Pro Gly Leu Ile Ala Ala Thr
230                 235                 240                 245

gat gag gaa gcc cgt gag cta gca ctt aat gat tgg ttg gaa ctc caa      883
Asp Glu Glu Ala Arg Glu Leu Ala Leu Asn Asp Trp Leu Glu Leu Gln
                250                 255                 260

cgc aag atc ggt gct gaa cgc ggt tgg gct cct gcg gat gca atg cag      931
Arg Lys Ile Gly Ala Glu Arg Gly Trp Ala Pro Ala Asp Ala Met Gln
            265                 270                 275

ttt gaa cgc gaa atc gat cac ggt tcc tta tac atc ggt tcc cct gag      979
Phe Glu Arg Glu Ile Asp His Gly Ser Leu Tyr Ile Gly Ser Pro Glu
        280                 285                 290

acg gtc gca aag aag atc gcc aaa acc att tca gtg ctt gat ctt gat      1027
Thr Val Ala Lys Lys Ile Ala Lys Thr Ile Ser Val Leu Asp Leu Asp
    295                 300                 305

cgc ttt acc ctc aaa tac gcc agt ggc cag acc cct cat gag tac ttg      1075
Arg Phe Thr Leu Lys Tyr Ala Ser Gly Gln Thr Pro His Glu Tyr Leu
310                 315                 320                 325

ctg aag tcc att gag ttg tat ggc act gag gtt att ccg ctg gtg aag      1123
Leu Lys Ser Ile Glu Leu Tyr Gly Thr Glu Val Ile Pro Leu Val Lys
                330                 335                 340

gac atc ttg acc aag cag gct taagaaggtc ttaggacatt ccc                1167
Asp Ile Leu Thr Lys Gln Ala
            345


<210> 436
<211> 348
<212> PRT
<213> Corynebacterium glutamicum

<400> 436
Met Lys Asn Val Ser Phe Gly Leu Asp Thr Phe Gly Asp Asn Ala Ile
  1               5                  10                  15

Asp Leu Gln Gly Asn Pro Val Ser Pro Ala Gln Thr Leu Arg Asn Ile
            20                  25                  30

Ile Asp Glu Ala Lys Met Ala Asp Lys Val Gly Val Asp Ile Ile Gly
        35                  40                  45

Ile Gly Glu His His Arg Glu Glu Tyr Ser Val Ser Ala Pro Asp Ile
    50                  55                  60

Val Met Thr Ala Ile Leu Ala Ser Thr Glu Arg Leu Lys Val Thr Ser
65                  70                  75                  80

Ser Val Thr Val Leu Ser Ser Asp Asp Pro Val Arg Leu Phe Glu Arg
                85                  90                  95

Tyr Ser Thr Met Asn Ala Leu Ser Asn Gly Arg Ala Glu Ile Thr Leu
            100                 105                 110

Gly Arg Gly Ser Phe Ile Glu Ser Phe Pro Leu Phe Gly Phe Asp Leu
        115                 120                 125

Gln Asp Tyr Glu Gln Leu Phe Ser Glu Arg Leu Asp Leu Phe Ala Lys
    130                 135                 140

Ile Leu Glu Ala Asp Ser Arg Gly Gln Gly Val Thr Trp His Gly Glu
145                 150                 155                 160

Thr Arg Ser Ala Leu Glu Asn Gln Met Leu Tyr Pro Pro Thr Glu Asn
                165                 170                 175

Gly Ile His Ala Trp Val Ala Val Gly Gly Ser Pro Glu Ser Val Val
            180                 185                 190

Arg Ala Ala Lys Tyr Arg Phe Pro Leu Met Leu Ala Ile Ile Gly Gly
        195                 200                 205

Ala Pro Glu Arg Phe Arg Pro Tyr Val Asp Leu Tyr Lys Arg Ala Asn
    210                 215                 220

Glu Gln Phe Gly Gln Pro Gln Lys Pro Ile Gly Val His Ser Pro Gly
225                 230                 235                 240

Leu Ile Ala Ala Thr Asp Glu Glu Ala Arg Glu Leu Ala Leu Asn Asp
                245                 250                 255

Trp Leu Glu Leu Gln Arg Lys Ile Gly Ala Glu Arg Gly Trp Ala Pro
            260                 265                 270

Ala Asp Ala Met Gln Phe Glu Arg Glu Ile Asp His Gly Ser Leu Tyr
        275                 280                 285

Ile Gly Ser Pro Glu Thr Val Ala Lys Lys Ile Ala Lys Thr Ile Ser
    290                 295                 300

Val Leu Asp Leu Asp Arg Phe Thr Leu Lys Tyr Ala Ser Gly Gln Thr
305                 310                 315                 320

Pro His Glu Tyr Leu Leu Lys Ser Ile Glu Leu Tyr Gly Thr Glu Val
                325                 330                 335

Ile Pro Leu Val Lys Asp Ile Leu Thr Lys Gln Ala
            340                 345

<210> 437
<211> 726
<212> DNA
<213> Corynebacterium glutamicum

<220>
<221> CDS
<222> (101)..(703)
<223> RXA01933

<400> 437
ctagaagcct taggcaagaa atttggttga gttttcgatc tctacgacgc gtcatctcaa 60

ttccacctag gcttggatgc aggttagaaa ggagccttcg atg tct aag act cgt   115
                                            Met Ser Lys Thr Arg
                                            1               5

act ttt ctg ttt gat ctt tat ggt gtt ctc atc aag gag cat ggt gcg   163
Thr Phe Leu Phe Asp Leu Tyr Gly Val Leu Ile Lys Glu His Gly Ala
                10                  15                  20

gcg cag ttt gag cgg gtt gcg cgt gcg gtg ggg gag ccg tcc aag aac    211
Ala Gln Phe Glu Arg Val Ala Arg Ala Val Gly Glu Pro Ser Lys Asn
            25                  30                  35

gac aag ctg cat gag gtt tat gag tcg ctt cgt ctg gat ctg gat gcc    259
Asp Lys Leu His Glu Val Tyr Glu Ser Leu Arg Leu Asp Leu Asp Ala
        40                  45                  50

ggc cgc gtg agt gag gtg aat tat tgg aat cag atc aaa cta ttg gtg    307
Gly Arg Val Ser Glu Val Asn Tyr Trp Asn Gln Ile Lys Leu Leu Val
    55                  60                  65

ggt ttg gag ttt ttg gat atc cag gag gtc atc gcg gct gac tac agg    355
Gly Leu Glu Phe Leu Asp Ile Gln Glu Val Ile Ala Ala Asp Tyr Arg
70                  75                  80                  85

ggc ctt tat gag cgt gat cag gac atg gtt gat tat gtg ttg tcg ttg    403
Gly Leu Tyr Glu Arg Asp Gln Asp Met Val Asp Tyr Val Leu Ser Leu
                90                  95                  100

aag gcg aaa ggc cac cgc atc gga att ttg tcg aat att ccg gag ggg    451
Lys Ala Lys Gly His Arg Ile Gly Ile Leu Ser Asn Ile Pro Glu Gly
            105                 110                 115

ttg gcc aag ctg ttg aag gag cac aat tcg gag tgg ctt gat cag ctt    499
Leu Ala Lys Leu Leu Lys Glu His Asn Ser Glu Trp Leu Asp Gln Leu
        120                 125                 130

gat gcg gtg act ttg tcg tgc gat att ggc gcg gcg aag ccg gag ccg    547
Asp Ala Val Thr Leu Ser Cys Asp Ile Gly Ala Ala Lys Pro Glu Pro
    135                 140                 145

aag tct ttc cat gtg gca ctt gag gcc ctt ggt gaa aaa gct gag gat    595
Lys Ser Phe His Val Ala Leu Glu Ala Leu Gly Glu Lys Ala Glu Asp
150                 155                 160                 165

gtg acc ttt att gat gat cgc gtg cgt aac att gag gca gcg cgc gaa    643
Val Thr Phe Ile Asp Asp Arg Val Arg Asn Ile Glu Ala Ala Arg Glu
                170                 175                 180

gaa ggt ctc agc aca att cac ttc act ggc tta gat tcc tta aaa gaa    691
Glu Gly Leu Ser Thr Ile His Phe Thr Gly Leu Asp Ser Leu Lys Glu
            185                 190                 195

agc att cag gaa tgacacctca accactgatt ttg                          726
Ser Ile Gln Glu
        200


<210> 438
<211> 201
<212> PRT
<213> Corynebacterium glutamicum

<400> 438
Met Ser Lys Thr Arg Thr Phe Leu Phe Asp Leu Tyr Gly Val Leu Ile
1               5                   10                  15

Lys Glu His Gly Ala Ala Gln Phe Glu Arg Val Ala Arg Ala Val Gly
            20                  25                  30

Glu Pro Ser Lys Asn Asp Lys Leu His Glu Val Tyr Glu Ser Leu Arg
        35                  40                  45

Leu Asp Leu Asp Ala Gly Arg Val Ser Glu Val Asn Tyr Trp Asn Gln
    50                  55                  60

Ile Lys Leu Leu Val Gly Leu Glu Phe Leu Asp Ile Gln Glu Val Ile
65                  70                  75                  80

Ala Ala Asp Tyr Arg Gly Leu Tyr Glu Arg Asp Gln Asp Met Val Asp
                85                  90                  95

Tyr Val Leu Ser Leu Lys Ala Lys Gly His Arg Ile Gly Ile Leu Ser
            100                 105                 110

Asn Ile Pro Glu Gly Leu Ala Lys Leu Leu Lys Glu His Asn Ser Glu

        115                 120                 125

Trp Leu Asp Gln Leu Asp Ala Val Thr Leu Ser Cys Asp Ile Gly Ala
    130                 135                 140

Ala Lys Pro Glu Pro Lys Ser Phe His Val Ala Leu Glu Ala Leu Gly
145                 150                 155                 160

Glu Lys Ala Glu Asp Val Thr Phe Ile Asp Asp Arg Val Arg Asn Ile
                165                 170                 175

Glu Ala Ala Arg Glu Glu Gly Leu Ser Thr Ile His Phe Thr Gly Leu
            180                 185                 190

Asp Ser Leu Lys Glu Ser Ile Gln Glu
        195                 200


<210> 439
<211> 1039
<212> DNA
<213> Corynebacterium glutamicum

<220>
<221> CDS
<222> (101)..(1039)
<223> RXA02351

<400> 439
tgacacttta cagactggtt ttcaactaat gacaccgaaa gaaatacacc tcaacctttt 60

tgctttcggt gccgggcacc acgcggcggc gtggcgagcg gtg gag gga agc gtc   115
                                          Val Glu Gly Ser Val
                                          1               5

gaa aag ctg ggt tta att tcc tgg tgg gag gaa ctc gcg cgc acc gct   163
Glu Lys Leu Gly Leu Ile Ser Trp Trp Glu Glu Leu Ala Arg Thr Ala
                10                  15                  20

gag cgg ggc aag ctg gat gcg gtc ttt ttg gcc gat ggg cag gcg att   211
Glu Arg Gly Lys Leu Asp Ala Val Phe Leu Ala Asp Gly Gln Ala Ile
            25                  30                  35

aat ccg gtc ggt ctg gag aat ggg ccg ggc tgg ttt ttg gag ccg gtg   259
Asn Pro Val Gly Leu Glu Asn Gly Pro Gly Trp Phe Leu Glu Pro Val
        40                  45                  50

acc gcg ttg act gcg atg gcg cgg gcg acg aac aat att ggg ttg atc   307
Thr Ala Leu Thr Ala Met Ala Arg Ala Thr Asn Asn Ile Gly Leu Ile
    55                  60                  65

agc aca att tcc agt acg ttt tgg cag ccg ttt cat gcg gcg cgg atg   355
Ser Thr Ile Ser Ser Thr Phe Trp Gln Pro Phe His Ala Ala Arg Met
70                  75                  80                  85

atc gcc agc ttg gat cat att tcg ggt ggg cgt gct gga atc aat gtg   403
Ile Ala Ser Leu Asp His Ile Ser Gly Gly Arg Ala Gly Ile Asn Val
                90                  95                  100

gtg aca tcg atg acc gat gcg gag gcg cgt aac cac ggg atg gat gcg   451
Val Thr Ser Met Thr Asp Ala Glu Ala Arg Asn His Gly Met Asp Ala
            105                 110                 115

ttg ccg ggt cac gat gtt cgc tat gcg cgc gct gcg gaa ttt att gaa   499
Leu Pro Gly His Asp Val Arg Tyr Ala Arg Ala Ala Glu Phe Ile Glu
        120                 125                 130

acc atc act gcg ctg tgg gat tct tgg cct gcg gaa agt ttg gtg atg   547
Thr Ile Thr Ala Leu Trp Asp Ser Trp Pro Ala Glu Ser Leu Val Met
    135                 140                 145

gat cgt gct gga aaa ttt gcg gac tcc tcg ctc att aaa tct atc gat   595
Asp Arg Ala Gly Lys Phe Ala Asp Ser Ser Leu Ile Lys Ser Ile Asp
150                 155                 160                 165

cat gat ggt gag ttc ttc caa gtc gct ggt ccg ctg aat atc ccc agt   643
His Asp Gly Glu Phe Phe Gln Val Ala Gly Pro Leu Asn Ile Pro Ser

                    170                     175                     180

cct ccg cag ggt cga ccc gta ctt ttt cag gct gga tcc tca ccg caa    691
Pro Pro Gln Gly Arg Pro Val Leu Phe Gln Ala Gly Ser Ser Pro Gln
            185                 190                 195

gga cgg gaa atc gct gcg aaa tac gcc gag gca att tac tct gtg gcg    739
Gly Arg Glu Ile Ala Ala Lys Tyr Ala Glu Ala Ile Tyr Ser Val Ala
        200                 205                 210

tgg gat ttg gag caa gcg caa gat tat cgc tct gat att cat gct cgt    787
Trp Asp Leu Glu Gln Ala Gln Asp Tyr Arg Ser Asp Ile His Ala Arg
    215                 220                 225

gcc act gcc cag ggt cgc gag ccc atg ccg gtg ctt cct ggt ttg gtg    835
Ala Thr Ala Gln Gly Arg Glu Pro Met Pro Val Leu Pro Gly Leu Val
230                 235                 240                 245

act ttt gtt ggc acg acc gtg gaa gaa gcg cgt gca aaa cag cag gct    883
Thr Phe Val Gly Thr Thr Val Glu Glu Ala Arg Ala Lys Gln Gln Ala
                250                 255                 260

ctt aat gcg ttg ctg ccg gtc aaa gac tca cta aat cag ttg agt ttc    931
Leu Asn Ala Leu Leu Pro Val Lys Asp Ser Leu Asn Gln Leu Ser Phe
            265                 270                 275

ttt gtg ggt caa gat tgc tcg acg tgg gat ttg gat gca cct ccc cca    979
Phe Val Gly Gln Asp Cys Ser Thr Trp Asp Leu Asp Ala Pro Pro Pro
        280                 285                 290

cca ctg cca ccg cta gaa gag ttt tcc ggt cct aaa ggc agg tac gaa    1027
Pro Leu Pro Pro Leu Glu Glu Phe Ser Gly Pro Lys Gly Arg Tyr Glu
    295                 300                 305

acg gtc ctg cgg                                                    1039
Thr Val Leu Arg
310


<210> 440
<211> 313
<212> PRT
<213> Corynebacterium glutamicum

<400> 440
Val Glu Gly Ser Val Glu Lys Leu Gly Leu Ile Ser Trp Trp Glu Glu
1               5                   10                  15

Leu Ala Arg Thr Ala Glu Arg Gly Lys Leu Asp Ala Val Phe Leu Ala
            20                  25                  30

Asp Gly Gln Ala Ile Asn Pro Val Gly Leu Glu Asn Gly Pro Gly Trp
        35                  40                  45

Phe Leu Glu Pro Val Thr Ala Leu Thr Ala Met Ala Arg Ala Thr Asn
    50                  55                  60

Asn Ile Gly Leu Ile Ser Thr Ile Ser Ser Thr Phe Trp Gln Pro Phe
65                  70                  75                  80

His Ala Ala Arg Met Ile Ala Ser Leu Asp His Ile Ser Gly Gly Arg
                85                  90                  95

Ala Gly Ile Asn Val Val Thr Ser Met Thr Asp Ala Glu Ala Arg Asn
            100                 105                 110

His Gly Met Asp Ala Leu Pro Gly His Asp Val Arg Tyr Ala Arg Ala
        115                 120                 125

Ala Glu Phe Ile Glu Thr Ile Thr Ala Leu Trp Asp Ser Trp Pro Ala
    130                 135                 140

Glu Ser Leu Val Met Asp Arg Ala Gly Lys Phe Ala Asp Ser Ser Leu
145                 150                 155                 160

Ile Lys Ser Ile Asp His Asp Gly Glu Phe Phe Gln Val Ala Gly Pro
                165                 170                 175

Leu Asn Ile Pro Ser Pro Pro Gln Gly Arg Pro Val Leu Phe Gln Ala
            180                 185                 190

Gly Ser Ser Pro Gln Gly Arg Glu Ile Ala Ala Lys Tyr Ala Glu Ala
        195                 200                 205

Ile Tyr Ser Val Ala Trp Asp Leu Glu Gln Ala Gln Asp Tyr Arg Ser
    210                 215                 220

Asp Ile His Ala Arg Ala Thr Ala Gln Gly Arg Glu Pro Met Pro Val
225                 230                 235                 240

Leu Pro Gly Leu Val Thr Phe Val Gly Thr Thr Val Glu Glu Ala Arg
                245                 250                 255

Ala Lys Gln Gln Ala Leu Asn Ala Leu Leu Pro Val Lys Asp Ser Leu
            260                 265                 270

Asn Gln Leu Ser Phe Phe Val Gly Gln Asp Cys Ser Thr Trp Asp Leu
        275                 280                 285

Asp Ala Pro Pro Pro Pro Leu Pro Pro Leu Glu Glu Phe Ser Gly Pro
    290                 295                 300

Lys Gly Arg Tyr Glu Thr Val Leu Arg
305                 310

<210> 441
<211> 18
<212> DNA
<213> Artificial Sequence

<220>
<223> Description of Artificial Sequence: primer

<400> 441
ggaaacagta tgaccatg                                                18


<210> 442
<211> 17
<212> DNA
<213> Artificial Sequence

<220>
<223> Description of Artificial Sequence: primer

<400> 442
gtaaaacgac ggccagt                                                 17

## Description

### Related Applications

This application claims priority to prior filed U.S. Provisional Patent Application Serial No. 60/141031, filed June 25, 1999. This application also claims priority to prior filed German Patent Application No. 19931636.8, filed July 8, 1999, German Patent Application No. 19932125.6, filed July 9, 1999, German Patent Application No. 19932126.4, filed July 9, 1999, German Patent Application No. 19932127.2, filed July 9, 1999, German Patent Application No. 19932128.0, filed July 9, 1999, German Patent Application No. 19932129.9, filed July 9, 1999, German Patent Application No. 19932226.0, filed July 9, 1999, German Patent Application No. 19932920.6, filed July 14, 1999, German Patent Application No. 19932922.2, filed July 14, 1999, German Patent Application No. 19932924.9, filed July 14, 1999, German Patent Application No. 19932928.1, filed July 14, 1999, German Patent Application No. 19932930.3, filed July 14, 1999, German Patent Application No. 19932933.8, filed July 14, 1999, German Patent Application No. 19932935.4, filed July 14, 1999, German Patent Application No. 19932973.7, filed July 14, 1999, German Patent Application No. 19933002.6, filed July 14, 1999, German Patent Application No. 19933003.4, filed July 14, 1999, German Patent Application No. 19933005.0, filed July 14, 1999, German Patent Application No. 19933006.9, filed July 14, 1999, German Patent Application No. 19941378.9, filed August 31, 1999, German Patent Application No. 19941379.7, filed August 31, 1999, Gennan Patent Application No. 19941390.8, filed August 31, 1999, German Patent Application No. 19941391.6, filed August 31, 1999, and German Patent Application No. 19942088.2, filed September 3, 1999. The entire contents of all of the aforementioned applications are hereby expressly incorporated herein by this reference.

### Background of the Invention

Certain products and by-products of naturally-occurring metabolic processes in cells have utility in a wide array of industries, including the food, feed, cosmetics, and pharmaceutical industries. These molecules, collectively termed 'fine chemicals', include organic acids, both proteinogenic and non-proteinogenic amino acids, nucleotides and nucleosides, lipids and fatty acids, diols, carbohydrates, aromatic compounds, vitamins and cofactors, and enzymes. Their production is most conveniently performed through the large-scale culture of bacteria developed to produce and secrete large quantities of one or more desired molecules. One particularly useful organism for this purpose is *Corynebacterium glutamicum,* a gram positive, nonpathogenic bacterium. Through strain selection, a number of mutant strains have been developed which produce an array of desirable compounds. However, selection of strains improved for the production of a particular molecule is a time-consuming and difficult process.

### Summary of the Invention

The invention provides novel bacterial nucleic acid molecules which have a variety of uses. These uses include the identification of microorganisms which can be used to produce fine chemicals, the modulation of fine chemical production in *C. glutamicum* or related bacteria, the typing or identification of *C*. *glutamicum* or related bacteria, as reference points for mapping the *C. glutamicum* genome, and as markers for transformation. These novel nucleic acid molecules encode proteins, referred to herein as homeostasis and adaptation (HA) proteins.

*C. glutamicum* is a gram positive, aerobic bacterium which is commonly used in industry for the large-scale production of a variety of fine chemicals, and also for the degradation of hydrocarbons (such as in petroleum spills) and for the oxidation of terpenoids. The HA nucleic acid molecules of the invention, therefore, can be used to identify microorganisms which can be used to produce fine chemicals, e.g., by fermentation processes. Modulation of the expression of the HA nucleic acids of the invention, or modification of the sequence of the HA nucleic acid molecules of the invention, can be used to modulate the production of one or more fine chemicals from a microorganism (e.g., to improve the yield or production of one or more fine chemicals from a Corynebacterium or Brevibacterium species).

The HA nucleic acids of the invention may also be used to identify an organism as being *Corynebacterium glutamicum* or a close relative thereof, or to identify the presence of *C*. *glutamicum* or a relative thereof in a mixed population of microorganisms. The invention provides the nucleic acid sequences of a number of *C*. *glutamicum* genes; by probing the extracted genomic DNA of a culture of a unique or mixed population of microorganisms under stringent conditions with a probe spanning a region of a *C*. *glutamicum* gene which is unique to this organism, one can ascertain whether this organism is present. Although *Corynebacterium glutamicum* itself is nonpathogenic, it is related to species pathogenic in humans, such as *Corynebacterium diphtheriae* (the causative agent of diphtheria); the detection of such organisms is of significant clinical relevance.

The HA nucleic acid molecules of the invention may also serve as reference points for mapping of the *C*. *glutamicum* genome, or of genomes of related organisms. Similarly, these molecules, or variants or portions thereof, may serve as markers for genetically engineered Corynebacterium or Brevibacterium species.

The HA proteins encoded by the novel nucleic acid molecules of the invention are capable of, for example, performing a function involved in the maintenance of homeostasis in C. *glutamicum,* or in the ability of this microorganism to adapt to different environmental conditions. Given the availability of cloning vectors for use in *Corynebacterium glutamicum,* such as those disclosed in Sinskey *et al.,* U.S. Patent No. 4,649,119, and techniques for genetic manipulation of *C*. *glutamicum* and the related *Brevibacterium* species *(e.g., lactofermentum)* (Yoshihama et al, *J Bacteriol.* 162: 591-597 (1985); Katsumata *et al., J.Bacteriol.* 159: 306-311 (1984); and Santamaria *et al., J Gen. Microbiol.* 130: 2237-2246 (1984)), the nucleic acid molecules of the invention may be utilized in the genetic engineering of this organism to make it a better or more efficient producer of one or more fine chemicals. This improved production or efficiency of production of a fine chemical may be due to a direct effect of manipulation of a gene of the invention, or it may be due to an indirect effect of such manipulation.

There are a number of mechanisms by which the alteration of an HA protein of the invention may directly affect the yield, production, and/or efficiency of production of a fine chemical from a *C. glutamicum* strain incorporating such an altered protein. For example, by engineering enzymes which modify or degrade aromatic or aliphatic compounds such that these enzymes are increased or decreased in activity or number, it may be possible to modulate the production of one or more fine chemicals which are the modification or degradation products of these compounds. Similarly, enzymes involved in the metabolism of inorganic compounds provide key molecules (e.g. phosphorous, sulfur, and nitrogen molecules) for the biosynthesis of such fine chemicals as amino acids, vitamins, and nucleic acids. By altering the activity or number of these enzymes in *C. glutamicum,* it may be possible to increase the conversion of these inorganic compounds (or to use alternate inorganic compounds) to thus permit improved rates of incorporation of inorganic atoms into these fine chemicals. Genetic engineering of C. *glutamicum* enzymes involved in general cellular processes may also directly improve fine chemical production, since many of these enzymes directly modify fine chemicals (e.g., amino acids) or the enzymes which are involved in fine chemical synthesis or secretion. Modulation of the activity or number of cellular proteases may also have a direct effect on fine chemical production, since many proteases may degrade fine chemicals or enzymes involved in fine chemical production or breakdown.

Further, the aforementioned enzymes which participate in aromatic/aliphatic compound modification or degradation, general biocatalysis, inorganic compound metabolism or proteolysis are each themselves fine chemicals, desirable for their activity in *various in vitro* industrial applications. By altering the number of copies of the gene for one or more of these enzymes in C. *glutamicum* it may be possible to increase the number of these proteins produced by the cell, thereby increasing the potential yield or efficiency of production of these proteins from large-scale C. *glutamicum* or related bacterial cultures.

The alteration of an HA protein of the invention may also indirectly affect the yield, production, and/or efficiency of production of a fine chemical from a C. *glutamicum* strain incorporating such an altered protein. For example, by modulating the activity and/or number of those proteins involved in the construction or rearrangement of the cell wall, it may be possible to modify the structure of the cell wall itself such that the cell is able to better withstand the mechanical and other stresses present during large-scale fermentative culture. Also, large-scale growth of C. *glutamicum* requires significant cell wall production. Modulation of the activity or number of cell wall biosynthetic or degradative enzymes may allow more rapid rates of cell wall biosynthesis, which in turn may permit increased growth rates of this microorganism in culture and thereby increase the number of cells producing the desired fine chemical.

By modifying the HA enzymes of the invention, one may also indirectly impact the yield, production, or efficiency of production of one or more fine chemicals from C. *glutamicum.* For example, many of the general enzymes in C. *glutamicum* may have a significant impact on global cellular processes (e.g., regulatory processes) which in turn have a significant effect on fine chemical metabolism. Similarly, proteases, enzymes which modify or degrade possibly toxic aromatic or aliphatic compounds, and enzymes which promote the metabolism of inorganic compounds all serve to increase the viability of C. *glutamicum.* The proteases aid in the selective removal of misfolded or misregulated proteins, such as those that might occur under the relatively stressful environmental conditions encountered during large-scale fermentor culture. By altering these proteins, it may be possible to further enhance this activity and to improve the viability of C. *glutamicum* in culture. The aromatic/aliphatic modification or degradation proteins not only serve to detoxify these waste compounds (which may be encountered as impurities in culture medium or as waste products from cells themselves), but also to permit the cells to utilize alternate carbon sources if the optimal carbon source is limiting in the culture. By increasing their number and/or activity, the survival of *C*. *glutamicum* cells in culture may be enhanced. The inorganic metabolism proteins of the invention supply the cell with inorganic molecules required for all protein and nucleotide (among others) synthesis, and thus are critical for the overall viability of the cell. An increase in the number of viable cells producing one or more desired fine chemicals in large-scale culture should result in a concomitant increase in the yield, production, and/or efficiency of production of the fine chemical in the culture.

The invention provides novel nucleic acid molecules which encode proteins, referred to herein as HA proteins, which are capable of, for example, performing a function involved in the maintenance of homeostasis in C. *glutamicum,* or of participating in the ability of this microorganism to adapt to different environmental conditions. Nucleic acid molecules encoding an HA protein are referred to herein as HA nucleic acid molecules. In a preferred embodiment, an HA protein participates in C. *glutamicum* cell wall biosynthesis or rearrangements, metabolism of inorganic compounds, modification or degradation of aromatic or aliphatic compounds, or possesses a C. *glutamicum* enzymatic or proteolytic activity. Examples of such proteins include those encoded by the genes set forth in Table 1.

Accordingly, one aspect of the invention pertains to isolated nucleic acid molecules (e.g., cDNAs, DNAs, or RNAs) comprising a nucleotide sequence encoding an HA protein or biologically active portions thereof, as well as nucleic acid fragments suitable as primers or hybridization probes for the detection or amplification of HA-encoding nucleic acids (e.g., DNA or mRNA). In particularly preferred embodiments, the isolated nucleic acid molecule comprises one of the nucleotide sequences set forth as the odd-numbered SEQ ID NOs in the Sequence Listing (e.g., SEQ ID NO:1, SEQ ID NO:3, SEQ ID NO:5, SEQ ID NO:7....), or the coding region or a complement thereof of one of these nucleotide sequences. In other particularly preferred embodiments, the isolated nucleic acid molecule of the invention comprises a nucleotide sequence which hybridizes to or is at least about 50%, preferably at least about 60%, more preferably at least about 70%, 80% or 90%, and even more preferably at least about 95%, 96%, 97%, 98%, 99% or more homologous to a nucleotide sequence set forth as an odd-numbered SEQ ID NO in the Sequence Listing (e.g., SEQ ID NO:1, SEQ ID NO:3, SEQ ID NO: 5, SEQ ID NO:7....), or a portion thereof. In other preferred embodiments, the isolated nucleic acid molecule encodes one of the amino acid sequences set forth in as an even-numbered SEQ ID NO in the Sequence Listing (e.g., SEQ ID NO:2, SEQ ID NO:4, SEQ ID NO:6, SEQ ID NO:8....). The preferred HA proteins of the present invention also preferably possess at least one of the HA activities described herein.

In another embodiment, the isolated nucleic acid molecule encodes a protein or portion thereof wherein the protein or portion thereof includes an amino acid sequence which is sufficiently homologous to an amino acid sequence of the invention (e.g., a sequence having an even-numbered SEQ ID NO: in the Sequence Listing), e.g., sufficiently homologous to an amino acid sequence of the invention such that the protein or portion thereof maintains an HA activity. Preferably, the protein or portion thereof encoded by the nucleic acid molecule maintains the ability to participate in the maintenance of homeostasis in C. *glutamicum,* or to perform a function involved in the adaptation of this microorganism to different environmental conditions. In one embodiment, the protein encoded by the nucleic acid molecule is at least about 50%, preferably at least about 60%, and more preferably at least about 70%, 80%, or 90% and most preferably at least about 95%, 96%, 97%, 98%, or 99% or more homologous to an amino acid sequence of the invention (e.g., an entire amino acid sequence selected from those having an even-numbered SEQ ID NO in the Sequence Listing). In another preferred embodiment, the protein is a full length C. *glutamicum* protein which is substantially homologous to an entire amino acid sequence of the invention (encoded by an open reading frame shown in the corresponding odd-numbered SEQ ID NOs in the Sequence Listing (e.g., SEQ ID NO:1, SEQ ID NO:3, SEQ ID NO:5, SEQ ID NO:7....)

In another preferred embodiment, the isolated nucleic acid molecule is derived from C. *glutamicum* and encodes a protein (e.g., an HA fusion protein) which includes a biologically active domain which is at least about 50% or more homologous to one of the amino acid sequences of the invention (e.g., a sequence of one of the even-numbered SEQ ID NOs in the Sequence Listing) and is able to participate in the repair or recombination of DNA, in the transposition of genetic material, in gene expression (i.e., the processes of transcription or translation), in protein folding, or in protein secretion in *Corynebacterium glutamicum,* or has one or more of the activities set forth in Table 1, and which also includes heterologous nucleic acid sequences encoding a heterologous polypeptide or regulatory regions.

In another embodiment, the isolated nucleic acid molecule is at least 15 nucleotides in length and hybridizes under stringent conditions to a nucleic acid molecule comprising a nucleotide sequence of the invention (e.g., a sequence of an odd-numbered SEQ ID NO in the Sequence Listing). Preferably, the isolated nucleic acid molecule corresponds to a naturally-occurring nucleic acid molecule. More preferably, the isolated nucleic acid encodes a naturally-occurring C. *glutamicum* HA protein, or a biologically active portion thereof.

Another aspect of the invention pertains to vectors, e.g., recombinant expression vectors, containing the nucleic acid molecules of the invention, and host cells into which such vectors have been introduced. In one embodiment, such a host cell is used to produce an HA protein by culturing the host cell in a suitable medium. The HA protein can be then isolated from the medium or the host cell.

Yet another aspect of the invention pertains to a genetically altered microorganism in which an HA gene has been introduced or altered. In one embodiment, the genome of the microorganism has been altered by introduction of a nucleic acid molecule of the invention encoding wild-type or mutated HA sequence as a transgene. In another embodiment, an endogenous HA gene within the genome of the microorganism has been altered, e.g., functionally disrupted, by homologous recombination with an altered HA gene. In another embodiment, an endogenous or introduced HA gene in a microorganism has been altered by one or more point mutations, deletions, or inversions, but still encodes a functional HA protein. In still another embodiment, one or more of the regulatory regions (e.g., a promoter, repressor, or inducer) of an HA gene in a microorganism has been altered (e.g., by deletion, truncation, inversion, or point mutation) such that the expression of the HA gene is modulated. In a preferred embodiment, the microorganism belongs to the genus *Corynebacterium* or *Brevibacterium,* with *Corynebacterium glutamicum* being particularly preferred. In a preferred embodiment, the microorganism is also utilized for the production of a desired compound, such as an amino acid, with lysine being particularly preferred.

In another aspect, the invention provides a method of identifying the presence or activity of *Cornyebacterium diphtheriae* in a subject. This method includes detection of one or more of the nucleic acid or amino acid sequences of the invention (*e.g.*, the sequences set forth in the Sequence Listing as SEQ ID NOs 1 through 440) in a subject, thereby detecting the presence or activity of *Corynebacterium diphtheriae* in the subject.

Still another aspect of the invention pertains to an isolated HA protein or a portion, e.g., a biologically active portion, thereof. In a preferred embodiment, the isolated HA protein or portion thereof can participate in the maintenance of homeostasis in *C*. *glutamicum,* or can perform a function involved in the adaptation of this microorganism to different environmental conditions. In another preferred embodiment, the isolated HA protein or portion thereof is sufficiently homologous to an amino acid sequence of the invention (e.g., a sequence of an even-numbered SEQ ID NO: in the Sequence Listing) such that the protein or portion thereof maintains the ability to participate in the maintenance of homeostasis in *C glutamicum,* or to perform a function involved in the adaptation of this microorganism to different environmental conditions.

The invention also provides an isolated preparation of an HA protein. In preferred embodiments, the HA protein comprises an amino acid sequence of the invention (e.g., a sequence of an even-numbered SEQ ID NO: of the Sequence Listing). In another preferred embodiment, the invention pertains to an isolated full length protein which is substantially homologous to an entire amino acid sequence of the invention (e.g., a sequence of an even-numbered SEQ ID NO: of the Sequence Listing) (encoded by an open reading frame set forth in a corresponding odd-numbered SEQ ID NO: of the Sequence Listing). In yet another embodiment, the protein is at least about 50%, preferably at least about 60%, and more preferably at least about 70%, 80%, or 90%, and most preferably at least about 95%, 96%, 97%, 98%, or 99% or more homologous to an entire amino acid sequence of the invention (e.g., a sequence of an even-numbered SEQ ID NO: of the Sequence Listing). In other embodiments, the isolated HA protein comprises an amino acid sequence which is at least about 50% or more homologous to one of the amino acid sequences of the invention (e.g., a sequence of an even-numbered SEQ ID NO: of the Sequence Listing) and is able to participate in the maintenance of homeostasis in C. *glutamicum,* or to perform a function involved in the adaptation of this microorganism to different environmental conditions, or has one or more of the activities set forth in Table 1.

Alternatively, the isolated HA protein can comprise an amino acid sequence which is encoded by a nucleotide sequence which hybridizes, e.g., hybridizes under stringent conditions, or is at least about 50%, preferably at least about 60%, more preferably at least about 70%, 80%, or 90%, and even more preferably at least about 95%, 96%, 97%, 98,%, or 99% or more homologous, to a nucleotide sequence of one of the even-numbered SEQ ID NOs set forth in the Sequence Listing. It is also preferred that the preferred forms of HA proteins also have one or more of the HA bioactivities described herein.

The HA polypeptide, or a biologically active portion thereof, can be operatively linked to a non-HA polypeptide to form a fusion protein. In preferred embodiments, this fusion protein has an activity which differs from that of the HA protein alone. In other preferred embodiments, this fusion protein participates in the maintenance of homeostasis in C. *glutamicum,* or performs a function involved in the adaptation of this microorganism to different environmental conditions. In particularly preferred embodiments, integration of this fusion protein into a host cell modulates production of a desired compound from the cell.

In another aspect, the invention provides methods for screening molecules which modulate the activity of an HA protein, either by interacting with the protein itself or a substrate or binding partner of the HA protein, or by modulating the transcription or translation of an HA nucleic acid molecule of the invention.

Another aspect of the invention pertains to a method for producing a fine chemical. This method involves the culturing of a cell containing a vector directing the expression of an HA nucleic acid molecule of the invention, such that a fine chemical is produced. In a preferred embodiment, this method further includes the step of obtaining a cell containing such a vector, in which a cell is transfected with a vector directing the expression of an HA nucleic acid. In another preferred embodiment, this method further includes the step of recovering the fine chemical from the culture. In a particularly preferred embodiment, the cell is from the genus *Corynebacterium* or *Brevibacterium,* or is selected from those strains set forth in Table 3.

Another aspect of the invention pertains to methods for modulating production of a molecule from a microorganism. Such methods include contacting the cell with an agent which modulates HA protein activity or HA nucleic acid expression such that a cell associated activity is altered relative to this same activity in the absence of the agent. In a preferred embodiment, the cell is modulated for one or more C. *glutamicum* processes involved in cell wall biosynthesis or rearrangements, metabolism of inorganic compounds, modification or degradation of aromatic or aliphatic compounds, or enzymatic or proteolytic activities. The agent which modulates HA protein activity can be an agent which stimulates HA protein activity or HA nucleic acid expression. Examples of agents which stimulate HA protein activity or HA nucleic acid expression include small molecules, active HA proteins, and nucleic acids encoding HA proteins that have been introduced into the cell. Examples of agents which inhibit HA activity or expression include small molecules and antisense HA nucleic acid molecules.

Another aspect of the invention pertains to methods for modulating yields of a desired compound from a cell, involving the introduction of a wild-type or mutant HA gene into a cell, either maintained on a separate plasmid or integrated into the genome of the host cell. If integrated into the genome, such integration can be random, or it can take place by homologous recombination such that the native gene is replaced by the introduced copy, causing the production of the desired compound from the cell to be modulated. In a preferred embodiment, said yields are increased. In another preferred embodiment, said chemical is a fine chemical. In a particularly preferred embodiment, said fine chemical is an amino acid. In especially preferred embodiments, said amino acid is L-lysine.

### Detailed Description of the Invention

The present invention provides HA nucleic acid and protein molecules which are involved in *C*. *glutamicum* cell wall biosynthesis or rearrangements, metabolism of inorganic compounds, modification or degradation of aromatic or aliphatic compounds, or that have a *C*. *glutamicum* enzymatic or proteolytic activity. The molecules of the invention may be utilized in the modulation of production of fine chemicals from microorganisms, such as *C*. *glutamicum,* either directly (e.g., where overexpression or optimization of activity of a protein involved in the production of a fine chemical (e.g., an enzyme) has a direct impact on the yield, production, and/or efficiency of production of a fine chemical from the modified *C*. *glutamicum),* or an indirect impact which nonetheless results in an increase of yield, production, and/or efficiency of production of the desired compound (e.g., where modulation of the activity or number of copies of a *C*. *glutamicum* aromatic or aliphatic modification or degradation protein results in an increase in the viability of C. *glutamicum* cells, which in turn permits increased production in a large-scale culture setting). Aspects of the invention are further explicated below.

### I. Fine Chemicals

The term 'fine chemical' is art-recognized and includes molecules produced by an organism which have applications in various industries, such as, but not limited to, the pharmaceutical, agriculture, and cosmetics industries. Such compounds include organic acids, such as tartaric acid, itaconic acid, and diaminopimelic acid, both proteinogenic and non-proteinogenic amino acids, purine and pyrimidine bases, nucleosides, and nucleotides (as described e.g. in Kuninaka, A. (1996) Nucleotides and related compounds, p. 561-612, in Biotechnology vol. 6, *Rehm et al.,* eds. VCH: Weinheim, and references contained therein), lipids, both saturated and unsaturated fatty acids (e.g., arachidonic acid), diols (e.g., propane diol, and butane diol), carbohydrates (e.g., hyaluronic acid and trehalose), aromatic compounds (e.g., aromatic amines, vanillin, and indigo), vitamins and cofactors (as described in Ullmann's Encyclopedia of Industrial Chemistry, vol. A27, "Vitamins", p. 443-613 (1996) VCH: Weinheim and references therein; and Ong, A.S., Niki, E. & Packer, L. (1995) "Nutrition, Lipids, Health, and Disease" Proceedings of the UNESCO/Confederation of Scientific and Technological Associations in Malaysia, and the Society for Free Radical Research - Asia, held Sept. 1-3, 1994 at Penang, Malaysia, AOCS Press, (1995), enzymes, polyketides *(*Cane *et al.* (1998) *Science* 282: 63-68), and all other chemicals described in Gutcho (1983) Chemicals by Fermentation, Noyes Data Corporation, ISBN: 0818805086 and references therein. The metabolism and uses of certain of these fine chemicals are further explicated below.

### A. Amino Acid Metabolism and Uses

Amino acids comprise the basic structural units of all proteins, and as such are essential for normal cellular functioning in all organisms. The term "amino acid" is art-recognized. The proteinogenic amino acids, of which there are 20 species, serve as structural units for proteins, in which they are linked by peptide bonds, while the nonproteinogenic amino acids (hundreds of which are known) are not normally found in proteins (see Ulmann's Encyclopedia of Industrial Chemistry, vol. A2, p. 57-97 VCH: Weinheim (1985)). Amino acids may be in the D- or L- optical configuration, though L-amino acids are generally the only type found in naturally-occurring proteins. Biosynthetic and degradative pathways of each of the 20 proteinogenic amino acids have been well characterized in both prokaryotic and eukaryotic cells (see, for example, Stryer, L. Biochemistry, 3^{rd} edition, pages 578-590 (1988)). The 'essential' amino acids (histidine, isoleucine, leucine, lysine, methionine, phenylalanine, threonine, tryptophan, and valine), so named because they are generally a nutritional requirement due to the complexity of their biosyntheses, are readily converted by simple biosynthetic pathways to the remaining 11 'nonessential' amino acids (alanine, arginine, asparagine, aspartate, cysteine, glutamate, glutamine, glycine, proline, serine, and tyrosine). Higher animals do retain the ability to synthesize some of these amino acids, but the essential amino acids must be supplied from the diet in order for normal protein synthesis to occur.

Aside from their function in protein biosynthesis, these amino acids are interesting chemicals in their own right, and many have been found to have various applications in the food, feed, chemical, cosmetics, agriculture, and pharmaceutical industries. Lysine is an important amino acid in the nutrition not only of humans, but also of monogastric animals such as poultry and swine. Glutamate is most commonly used as a flavor additive (mono-sodium glutamate, MSG) and is widely used throughout the food industry, as are aspartate, phenylalanine, glycine, and cysteine. Glycine, L-methionine and tryptophan are all utilized in the pharmaceutical industry. Glutamine, valine, leucine, isoleucine, histidine, arginine, proline, serine and alanine are of use in both the pharmaceutical and cosmetics industries. Threonine, tryptophan, and D/ L-methionine are common feed additives. (Leuchtenberger, W. (1996) Amino aids ― technical production and use, p. 466-502 in Rehm *et al.* (eds.) Biotechnology vol. 6, chapter 14a, VCH: Weinheim). Additionally, these amino acids have been found to be useful as precursors for the synthesis of synthetic amino acids and proteins, such as N-acetylcysteine, S-carboxymethyl-L-cysteine, (S)-5-hydroxytryptophan, and others described in Ulmann's Encyclopedia of Industrial Chemistry, vol. A2, p. 57-97, VCH: Weinheim, 1985.

The biosynthesis of these natural amino acids in organisms capable of producing them, such as bacteria, has been well characterized (for review of bacterial amino acid biosynthesis and regulation thereof, see Umbarger, H.E.(1978) *Ann. Rev. Biochem.* 47: 533-606). Glutamate is synthesized by the reductive amination of α-ketoglutarate, an intermediate in the citric acid cycle. Glutamine, proline, and arginine are each subsequently produced from glutamate. The biosynthesis of serine is a three-step process beginning with 3-phosphoglycerate (an intermediate in glycolysis), and resulting in this amino acid after oxidation, transamination, and hydrolysis steps. Both cysteine and glycine are produced from serine; the former by the condensation of homocysteine with serine, and the latter by the transferal of the side-chain β-carbon atom to tetrahydrofolate, in a reaction catalyzed by serine transhydroxymethylase. Phenylalanine, and tyrosine are synthesized from the glycolytic and pentose phosphate pathway precursors erythrose 4-phosphate and phosphoenolpyruvate in a 9-step biosynthetic pathway that differ only at the final two steps after synthesis of prephenate. Tryptophan is also produced from these two initial molecules, but its synthesis is an 11-step pathway. Tyrosine may also be synthesized from phenylalanine, in a reaction catalyzed by phenylalanine hydroxylase. Alanine, valine, and leucine are all biosynthetic products of pyruvate, the final product of glycolysis. Aspartate is formed from oxaloacetate, an intermediate of the citric acid cycle. Asparagine, methionine, threonine, and lysine are each produced by the conversion of aspartate. Isoleucine is formed from threonine. A complex 9-step pathway results in the production of histidine from 5-phosphoribosyl-1-pyrophosphate, an activated sugar.

Amino acids in excess of the protein synthesis needs of the cell cannot be stored, and are instead degraded to provide intermediates for the major metabolic pathways of the cell (for review see Stryer, L. Biochemistry 3^{rd} ed. Ch. 21 "Amino Acid Degradation and the Urea Cycle" p. 495-516 (1988)). Although the cell is able to convert unwanted amino acids into useful metabolic intermediates, amino acid production is costly in terms of energy, precursor molecules, and the enzymes necessary to synthesize them. Thus it is not surprising that amino acid biosynthesis is regulated by feedback inhibition, in which the presence of a particular amino acid serves to slow or entirely stop its own production (for overview of feedback mechanisms in amino acid biosynthetic pathways, see Stryer, L. Biochemistry, 3^{rd} ed. Ch. 24: "Biosynthesis of Amino Acids and Heme" p. 575-600 (1988)). Thus, the output of any particular amino acid is limited by the amount of that amino acid present in the cell.

### B. Vitamin, Cofactor, and Nutraceutical Metabolism and Uses

Vitamins, cofactors, and nutraceuticals comprise another group of molecules which the higher animals have lost the ability to synthesize and so must ingest, although they are readily synthesized by other organisms such as bacteria. These molecules are either bioactive substances themselves, or are precursors of biologically active substances which may serve as electron carriers or intermediates in a variety of metabolic pathways. Aside from their nutritive value, these compounds also have significant industrial value as coloring agents, antioxidants, and catalysts or other processing aids. (For an overview of the structure, activity, and industrial applications of these compounds, see, for example, Ullman's Encyclopedia of Industrial Chemistry, "Vitamins" vol. A27, p. 443-613, VCH: Weinheim, 1996.) The term "vitamin" is art-recognized, and includes nutrients which are required by an organism for normal functioning, but which that organism cannot synthesize by itself. The group of vitamins may encompass cofactors and nutraceutical compounds. The language "cofactor" includes nonproteinaceous compounds required for a normal enzymatic activity to occur. Such compounds may be organic or inorganic; the cofactor molecules of the invention are preferably organic. The term "nutraceutical" includes dietary supplements having health benefits in plants and animals, particularly humans. Examples of such molecules are vitamins, antioxidants, and also certain lipids (e.g., polyunsaturated fatty acids).

The biosynthesis of these molecules in organisms capable of producing them, such as bacteria, has been largely characterized (Ullman's Encyclopedia of Industrial Chemistry, "Vitamins" vol. A27, p. 443-613, VCH: Weinheim, 1996; Michal, G. (1999) Biochemical Pathways: An Atlas of Biochemistry and Molecular Biology, John Wiley & Sons; Ong, A.S., Niki, E. & Packer, L. (1995) "Nutrition, Lipids, Health, and Disease" Proceedings of the UNESCO/Confederation of Scientific and Technological Associations in Malaysia, and the Society for Free Radical Research - Asia, held Sept. 1-3, 1994 at Penang, Malaysia, AOCS Press: Champaign, IL X, 374 S).

Thiamin (vitamin B₁) is produced by the chemical coupling of pyrimidine and thiazole moieties. Riboflavin (vitamin B₂) is synthesized from guanosine-5'-triphosphate (GTP) and ribose-5'-phosphate. Riboflavin, in turn, is utilized for the synthesis of flavin mononucleotide (FMN) and flavin adenine dinucleotide (FAD). The family of compounds collectively termed 'vitamin B₆' (e.g., pyridoxine, pyridoxamine, pyridoxa-5'-phosphate, and the commercially used pyridoxin hydrochloride) are all derivatives of the common structural unit, 5-hydroxy-6-methylpyridine. Pantothenate (pantothenic acid, (R)-(+)-N-(2,4-dihydroxy-3,3-dimethyl-1-oxobutyl)-β-alanine)can be produced either by chemical synthesis or by fermentation. The final steps in pantothenate biosynthesis consist of the ATP-driven condensation of β-alanine and pantoic acid. The enzymes responsible for the biosynthesis steps for the conversion to pantoic acid, to β-alanine and for the condensation to panthotenic acid are known. The metabolically active form of pantothenate is Coenzyme A, for which the biosynthesis proceeds in 5 enzymatic steps. Pantothenate, pyridoxal-5'-phosphate, cysteine and ATP are the precursors of Coenzyme A. These enzymes not only catalyze the formation of panthothante, but also the production of (R)-pantoic acid, (R)-pantolacton, (R)-panthenol (provitamin B₅), pantetheine (and its derivatives) and coenzyme A.

Biotin biosynthesis from the precursor molecule pimeloyl-CoA in microorganisms has been studied in detail and several of the genes involved have been identified. Many of the corresponding proteins have been found to also be involved in Fe-cluster synthesis and are members of the nifS class of proteins. Lipoic acid is derived from octanoic acid, and serves as a coenzyme in energy metabolism, where it becomes part of the pyruvate dehydrogenase complex and the α-ketoglutarate dehydrogenase complex. The folates are a group of substances which are all derivatives of folic acid, which is turn is derived from L-glutamic acid, p-amino-benzoic acid and 6-methylpterin. The biosynthesis of folic acid and its derivatives, starting from the metabolism intermediates guanosine-5'-triphosphate (GTP), L-glutamic acid and p-amino-benzoic acid has been studied in detail in certain microorganisms.

Corrinoids (such as the cobalamines and particularly vitamin B₁₂) and porphyrines belong to a group of chemicals characterized by a tetrapyrole ring system. The biosynthesis of vitamin B₁₂ is sufficiently complex that it has not yet been completely characterized, but many of the enzymes and substrates involved are now known. Nicotinic acid (nicotinate), and nicotinamide are pyridine derivatives which are also termed 'niacin'. Niacin is the precursor of the important coenzymes NAD (nicotinamide adenine dinucleotide) and NADP (nicotinamide adenine dinucleotide phosphate) and their reduced forms.

The large-scale production of these compounds has largely relied on cell-free chemical syntheses, though some of these chemicals have also been produced by large-scale culture of microorganisms, such as riboflavin, Vitamin B₆, pantothenate, and biotin. Only Vitamin B₁₂ is produced solely by fermentation, due to the complexity of its synthesis. *In vitro* methodologies require significant inputs of materials and time, often at great cost.

### C. Purine, Pyrimidine, Nucleoside and Nucleotide Metabolism and Uses

Purine and pyrimidine metabolism genes and their corresponding proteins are important targets for the therapy of tumor diseases and viral infections. The language "purine" or "pyrimidine" includes the nitrogenous bases which are constituents of nucleic acids, co-enzymes, and nucleotides. The term "nucleotide" includes the basic structural units of nucleic acid molecules, which are comprised of a nitrogenous base, a pentose sugar (in the case of RNA, the sugar is ribose; in the case of DNA, the sugar is D-deoxyribose), and phosphoric acid. The language "nucleoside" includes molecules which serve as precursors to nucleotides, but which are lacking the phosphoric acid moiety that nucleotides possess. By inhibiting the biosynthesis of these molecules, or their mobilization to form nucleic acid molecules, it is possible to inhibit RNA and DNA synthesis; by inhibiting this activity in a fashion targeted to cancerous cells, the ability of tumor cells to divide and replicate may be inhibited. Additionally, there are nucleotides which do not form nucleic acid molecules, but rather serve as energy stores (i.e., AMP) or as coenzymes *(i.e.,* FAD and NAD).

Several publications have described the use of these chemicals for these medical indications, by influencing purine and/or pyrimidine metabolism (e.g. Christopherson, R.I. and Lyons, S.D. (1990) "Potent inhibitors *of de novo* pyrimidine and purine biosynthesis as chemotherapeutic agents." *Med. Res. Reviews* 10: 505-548). Studies of enzymes involved in purine and pyrimidine metabolism have been focused on the development of new drugs which can be used, for example, as immunosuppressants or anti-proliferants (Smith, J.L., (1995) "Enzymes in nucleotide synthesis." *Curr. Opin. Struct. Biol.* 5: 752-757; (1995) *Biochem Soc. Transact.* 23: 877-902). However, purine and pyrimidine bases, nucleosides and nucleotides have other utilities: as intermediates in the biosynthesis of several fine chemicals (e.g., thiamine, S-adenosyl-methionine, folates, or riboflavin), as energy carriers for the cell (e.g., ATP or GTP), and for chemicals themselves, commonly used as flavor enhancers (e.g., IMP or GMP) or for several medicinal applications (see, for example, Kuninaka, A. (1996) Nucleotides and Related Compounds in Biotechnology vol. 6, *Rehm et al.,* eds. VCH: Weinheim, p. 561-612). Also, enzymes involved in purine, pyrimidine, nucleoside, or nucleotide metabolism are increasingly serving as targets against which chemicals for crop protection, including fungicides, herbicides and insecticides, are developed.

The metabolism of these compounds in bacteria has been characterized (for reviews see, for example, Zalkin, H. and Dixon, J.E. (1992) *"de novo* purine nucleotide biosynthesis", in: Progress in Nucleic Acid Research and Molecular Biology, vol. 42, Academic Press:, p. 259-287; and Michal, G. (1999) "Nucleotides and Nucleosides", Chapter 8 in: Biochemical Pathways: An Atlas of Biochemistry and Molecular Biology, Wiley: New York). Purine metabolism has been the subject of intensive research, and is essential to the normal functioning of the cell. Impaired purine metabolism in higher animals can cause severe disease, such as gout. Purine nucleotides are synthesized from ribose-5-phosphate, in a series of steps through the intermediate compound inosine-5'-phosphate (IMP), resulting in the production of guanosine-5'-monophosphate (GMP) or adenosine-5'-monophosphate (AMP), from which the triphosphate forms utilized as nucleotides are readily formed. These compounds are also utilized as energy stores, so their degradation provides energy for many different biochemical processes in the cell. Pyrimidine biosynthesis proceeds by the formation of uridine-5'-monophosphate (UMP) from ribose-5-phosphate. UMP, in turn, is converted to cytidine-5'-triphosphate (CTP). The deoxy- forms of all of these nucleotides are produced in a one step reduction reaction from the diphosphate ribose form of the nucleotide to the diphosphate deoxyribose form of the nucleotide. Upon phosphorylation, these molecules are able to participate in DNA synthesis.

### D. Trehalose Metabolism and Uses

Trehalose consists of two glucose molecules, bound in α, α-1,1 linkage. It is commonly used in the food industry as a sweetener, an additive for dried or frozen foods, and in beverages. However, it also has applications in the pharmaceutical, cosmetics and biotechnology industries (see, for example, Nishimoto *et al.,* (1998) U.S. Patent No. 5,759,610; Singer, M.A. and Lindquist, S. **(1998)** *Trends Biotech.* 16: 460-**467;** Paiva, C.L.A. and Panek, A.D. (1996) *Biotech. Ann. Rev.* 2: 293-314; and Shiosaka, M. (1997) J.Japan 172: 97-102). Trehalose is produced by enzymes from many microorganisms and is naturally released into the surrounding medium, from which it can be collected using methods known in the art.

### II. Maintenance of Homeostasis in C. glutamicum and Environmental Adaptation

The metabolic and other biochemical processes by which cells function are sensitive to environmental conditions such as temperature, pressure, solute concentration, and availability of oxygen. When one or more such environmental condition is perturbed or altered in a fashion that is incompatible with the normal functioning of these cellular processes, the cell must act to maintain an intracellular environment which will permit them to occur despite the hostile extracellular environment. Gram positive bacterial cells, such as C. *glutamicum* cells, have a number of mechanisms by which internal homeostasis may be maintained despite unfavorable extracellular conditions. These include a cell wall, proteins which are able to degrade possibly toxic aromatic and aliphatic compounds, mechanisms of proteolysis whereby misfolded or misregulated proteins may be rapidly destroyed, and catalysts which permit intracellular reactions to occur which would not normally take place under the conditions optimal for bacterial growth.

Aside from merely surviving in a hostile environment, bacterial cells *(e.g.* C. *glutamicum* cells) are also frequently able to adapt such that they are able to take advantage of such conditions. For example, cells in an environment lacking desired carbon sources may be able to adapt to growth on a less-suitable carbon source. Also, cells may be able to utilize less desirable inorganic compounds when the commonly utilized ones are unavailable. C. *glutamicum* cells possess a number of genes which permit them to adapt to utilize inorganic and organic molecules which they would normally not encounter under optimal growth conditions as nutrients and precursors for metabolism. Aspects of cellular processes involved in homeostasis and adaptation are further explicated below.

### A. Modification and Degradation ofAromatic and Aliphatic Compounds

Bacterial cells are routinely exposed to a variety of aromatic and aliphatic compounds in nature. Aromatic compounds are organic molecules having a cyclic ring structure, while aliphatic compounds are organic molecules having open chain structures rather than ring structures. Such compounds may arise as by-products of industrial processes (e.g., benzene or toluene), but may also be produced by certain microorganisms (e.g., alcohols). Many of these compounds are toxic to cells, particularly the aromatic compounds, which are highly reactive due to the high-energy ring structure. Thus, certain bacteria have developed mechanisms by which they are able to modify or degrade these compounds such that they are no longer hazardous to the cell. Cells may possess enzymes that are able to, for example, hydroxylate, isomerize, or methylate aromatic or aliphatic compounds such that they are either rendered less toxic, or such that the modified form is able to be processed by standard cellular waste and degradation pathways. Also, cells may possess enzymes which are able to specifically degrade one or more such potentially hazardous substance, thereby protecting the cell. Principles and examples of these types of modification and degradation processes in bacteria are described in several publications, e.g., Sahm, H. (1999) "Procaryotes in Industrial Production" in Lengeler, *J.W. et al.,* eds. Biology of the Procaryotes, Thieme Verlag: Stuttgart; and Schlegel, H.G. (1992) Allgemeine Mikrobiologie, Thieme: Stuttgart).

Aside from simply inactivating hazardous aromatic or aliphatic compounds, many bacteria have evolved to be able to utilize these compounds as carbon sources for continued metabolism when the preferred carbon sources of the cell are not available. For example, Pseudomonas strains able to utilize toluene, benzene, and 1,10-dichlorodecane as carbon sources are known (Chang, B.V. *et al.* (1997) *Chemosphere* 35(12): 2807-2815; Wischnak, *C. et al.* (1998) *Appl. Environ. Microbiol.* 64(9): 3507-3511; Churchill, S.A. *et al.* (1999) *Appl. Environ. Microbiol.* 65(2): 549-552). There are similar examples from many other bacterial species which are known in the art.

The ability of certain bacteria to modify or degrade aromatic and aliphatic compounds has begun to be exploited. Petroleum is a complex mixture of chemicals which includes aliphatic molecules and aromatic compounds. By applying bacteria having the ability to degrade or modify these toxic compounds to an oil spill, for example, it is possible to eliminate much of the environmental damage with high efficiency and low cost (see, for example, Smith, M.R. (1990) "The biodegradation of aromatic hydrocarbons by bacteria" *Biodegradation* 1(2-3): 191-206; and Suyama, T. *et al.* (1998) "Bacterial isolates degrading aliphatic polycarbonates," *FEMS Microbiol. Lett.* 161(2): 255-261).

### B. Metabolism of Inorganic Compounds

Cells (e.g., bacterial cells) contain large quantities of different molecules, such as water, inorganic ions, and organic substances (e.g., proteins, sugars, and other macromolecules). The bulk of the mass of a typical cell consists of only 4 types of atoms: carbon, oxygen, hydrogen, and nitrogen. Although they represent a smaller percentage of the content of a cell, inorganic substances are equally as important to the proper functioning of the cell. Such molecules include phosphorous, sulfur, calcium, magnesium, iron, zinc, manganese, copper, molybdenum, tungsten, and cobalt. Many of these compounds are critical for the construction of important molecules, such as nucleotides (phosphorous) and amino acids (nitrogen and sulfur). Others of these inorganic ions serve as cofactors for enzymic reactions or contribute to osmotic pressure. All such molecules must be taken up by the bacterium from the surrounding environment.

For each of these inorganic compounds it is desirable for the bacterium to take up the form which can be most readily used by the standard metabolic machinery of the cell. However, the bacterium may encounter environments in which these preferred forms are not readily available. In order to survive under these circumstances, it is important for bacteria to have additional biochemical mechanisms which are able to convert less metabolically active but readily available forms of these inorganic compounds to ones which may be used in cellular metabolism. Bacteria frequently possess a number of genes encoding enzymes for this purpose, which are not expressed unless the desired inorganic species are not available. Thus, these genes for the metabolism of various inorganic compounds serve as another tool which bacteria may use to adapt to suboptimal environmental conditions.

After carbon, the most important element in the cell is nitrogen. A typical bacterial cell contains between 12-15% nitrogen. It is a constituent of amino acids and nucleotides, as well as many other important molecules in the cell. Further, nitrogen may serve as a substitute for oxygen as a terminal electron acceptor in energy metabolism. Good sources of nitrogen include many organic and inorganic compounds, such ammonia gas or ammonia salts (e.g., NH₄Cl, (NH₄)₂SO₄, or NH₄OH), nitrates, urea, amino acids, or complex nitrogen sources like corn steep liquor, soy bean flour, soy bean protein, yeast extract, meat extract, etc. Ammonia nitrogen is fixed by the action of particular enzymes: glutamate dehydrogenase, glutamine synthase, and glutamine-2-oxoglutarate aminotransferase. The transfer of amino-nitrogen from one organic molecule to another is accomplished by the aminotransferases, a class of enzymes which transfer one amino group from an alpha-amino acid to an alpha-keto acid. Nitrate may be reduced via nitrate reductase, nitrite reductase, and further redox enzymes until it is converted to molecular nitrogen or ammonia, which may be readily utilized by the cell in standard metabolic pathways.

Phosphorous is typically found intracellularly in both organic and inorganic forms, and may be taken up by the cell in either of these forms as well, though most microorganisms preferentially take up inorganic phosphate. The conversion of organic phosphate to a form which the cell can utilize requires the action of phosphatases (e.g., phytases, which hydrolyze phyate-yielding phosphate and inositol derivatives). Phosphate is a key element in the synthesis of nucleic acids, and also has a significant role in cellular energy metabolism (e.g., in the synthesis of ATP, ADP, and AMP).

Sulfur is a requirement for the synthesis of amino acids (e.g., methionine and cysteine), vitamins (e.g., thiamine, biotin, and lipoic acid) and iron sulfur proteins. Bacteria obtain sulfur primarily from inorganic sulfate, though thiosulfate, sulfite, and sulfide are also commonly utilized. Under conditions where these compounds may not be readily available, many bacteria express genes which enable them to utilize sulfonate compounds such as 2-aminosulfonate (taurine) (Kertesz, M.A. (1993) "Proteins induced by sulfate limitation in *Escherichia coli, Pseudomonas putida,* or *Staphylococcus aureus." J. Bacteriol.* 175: 1187-1190).

Other inorganic atoms, e.g., metal or calcium ions, are also critical for the viability of cells. Iron, for example, plays a key role in redox reactions and is a cofactor of iron-sulfur proteins, heme proteins, and cytochromes. The uptake of iron into bacterial cells may be accomplished by the action of siderophores, chelating agents which bind extracellular iron ions and translocate them to the interior of the cell. For reference on the metabolism of iron and other inorganic compounds, see: Lengeler *et al.* (1999) Biology of Prokaryotes, Thieme Verlag: Stuttgart; Neidhardt, F.C. *et al.,* eds. *Escherichia coli* and *Salmonella.* ASM Press: Washington, D.C.; Sonenshein, A.L. *et al.,* eds. (199?) *Bacillus subtilis* and Other Gram-Positive Bacteria, ASM Press: Washington, D.C.; Voet, D. and Voet, J.G. (1992) Biochemie, VCH: Weinheim; Brock, T.D. and Madigan, M.T. (1991) Biology of Microorgansisms, 6^{th} ed. Prentice Hall: Englewood Cliffs, p. 267-269; Rhodes, P.M. and Stanbury, P.F. Applied Microbial Physiology - A Practical Approach, Oxford Univ. Press: Oxford.

### C. Enzymes and Proteolysis

The intracellular conditions for which bacteria such as C. *glutamicum* are optimized are frequently not conditions under which many biochemical reactions would normally take place. In order to make such reactions proceed under physiological conditions, cells utilize enzymes. Enzymes are proteinaceous biological catalysts, spatially orienting reacting molecules or providing a specialized environment such that the energy barrier to a biochemical reaction is lowered. Different enzymes catalyze different reactions, and each enzyme may be the subject of transcriptional, translational, or posttranslational regulation such that the reaction will only take place under appropriate conditions and at specified times. Enzymes may contribute to the degradation (e.g., the proteases), synthesis (e.g., the synthases), or modification *(e.g.,* transferases or isomerases) of compounds, all of which enable the production of necessary compounds within the cell. This, in turn, contributes to the maintenance of cellular homeostasis.

However, the fact that enzymes are optimized for activity under the physiological conditions at which the bacterium is most viable means that when environmental conditions are perturbed, there is a significant possibility that enzyme activity will also be perturbed. For example, changes in temperature may result in aberrantly folded proteins, and the same is true for changes of pH - protein folding is largely dependent on electrostatic and hydrophobic interactions of amino acids within the polypeptide chain, so any alteration to the charges on individual amino acids (as might be brought about by a change in cellular pH) may have a profound effect on the ability of the protein to correctly fold. Changes in temperature effectively change the amount of kinetic energy that the polypeptide molecule possesses, which affects the ability of the polypeptide to settle into a correctly folded, energetically stable configuration. Misfolded proteins may be harmful to the cell for two reasons. First, the aberrantly folded protein may have a similarly aberrant activity, or no activity whatsoever. Second, misfolded proteins may lack the conformational regions necessary for proper regulation by other cellular systems and thus may continue to be active but in an uncontrolled fashion.

The cell has a mechanism by which misfolded enzymes and regulatory proteins may be rapidly destroyed before any damage occurs to the cell: proteolysis. Proteins such as those of the Ia/Ion family and those of the Clp family specifically recognize and degrade misfolded proteins (see, e.g., Sherman, M.Y., Goldberg, A.L. (1999) *EXS* 77: 57-78 and references therein and Porankiewicz J. (1999) *Molec. Microbiol.* 32(3): 449-58, and references therein; Neidhardt, F.C., *et al.* (1996) *E. coli* and *Salmonella,* ASM Press: Washington, D.C. and references therein; and Pritchard, G.G., and Coolbear, T. (1993) *FEMS Microbiol.* Rev. 12(1-3): 179-206 and references therein). These enzymes bind to misfolded or unfolded proteins and degrade them in an ATP-dependent manner. Proteolysis thus serves as an important mechanism employed by the cell to prevent damage to normal cellular functions upon environmental changes, and it further permits cells to survive under conditions and in environments which would otherwise be toxic due to misregulated and/or aberrant enzyme or regulatory activity.

Proteolysis also has important functions in the cell under optimal environmental conditions. Within normal metabolic processes, proteases aid in the hydrolysis of peptide bonds, in the catabolism of complex molecules to provide necessary degradation products, and in protein modification. Secreted proteases play an important role in the catabolism of external nutrients even prior to the entry of these compounds into the cell. Further, proteolytic activity itself may serve regulatory functions; sporulation in B. *subtilis* and cell cycle progression in *Caulobacter* spp.are known to be regulated by key proteolytic events in each of these species (Gottesman, S. (1999) *Curr. Opin. Microbiol.* 2(2): 142-147). Thus, proteolytic processes are key for cellular survival under both suboptimal and optimal environmental conditions, and contribute to the overall maintenance of homeostasis in cells.

### D. Cell Wall Production and Rearrangements

While the biochemical machinery of the cell may be able to readily adapt to different and possibly unfavorable environments, cells still require a general mechanism by which they may be protected from the environment. For many bacteria, the cell wall affords such protection, and also plays roles in adhesion, cell growth and division, and transport of desired solutes and waste materials.

In order to function, cells require intracellular concentrations of metabolites and other molecules that are substantially higher than those of the surrounding media. Since these metabolites are largely prevented from leaving the cell due to the presence of the hydrophobic membrane, the tendency of the system is for water molecules to enter the cell from the external medium such that the interior concentrations of solutes match the exterior concentrations. Water molecules are readily able to cross the cellular membrane, and this membrane is not able to withstand the resulting swelling and pressure, which may lead to osmotic lysis of the cell. The rigidity of the cell wall greatly improves the ability of the cell to tolerate these pressures, and offers a further barrier to the unwanted diffusion of these metabolites and desired solutes from the cell. Similarly, the cell wall also serves to prevent unwanted material from entering the cell.

The cell wall also participates in a number of other cellular processes, such as adhesion and cell growth and division. Due to the fact that the cell wall completely surrounds the cell, any interaction of the cell with its surroundings must be mediated by the cell wall. Thus, the cell wall must participate in any adherence of the cell to other cells and to desired surfaces. Further, the cell cannot grow or divide without concomitant changes in the cell wall. Since the protection that the wall affords requires its presence during growth, morphogenesis and multiplication, one of the key steps in cell division is cell wall synthesis within the cell such that a new cell divides from the old. Thus, frequently cell wall biosynthesis is regulated in tandem with cell growth and cell division (see, e.g., Sonenshein, A.L. et al, eds. (1993) *Bacillus sublilis* and Other Gram-Positive Bacteria, ASM: Washington, D.C.).

The structure of the cell wall varies between gram-positive and gram-negative bacteria. However, in both types, the fundamental structural unit of the wall remains similar: an overlapping lattice of two polysaccharides, N-acetyl glucosamine (NAG) and N-acetyl muramic acid (NAM) which are cross-linked by amino acids (most commonly L-alanine, D-glutamate, diaminopimelic acid, and D-alanine), termed 'peptidoglycan'. The processes involved in the synthesis of the cell wall are known (see, e.g., Michal, G., ed. (1999) Biochemical Pathways: An Atlas of Biochemistry and Molecular Biology, Wiley: New York).

In gram-negative bacteria, the inner cellular membrane is coated by a single-layered peptidoglycan (approximately 10 nm thick), termed the murein-sacculus. This peptidoglycan structure is very rigid, and its structure determines the shape of the organism. The outer surface of the murein-sacculus is covered with an outer membrane, containing porins and other membrane proteins, phospholipids, and lipopolysaccharides. To maintain a tight association with the outer membrane, the gram-negative cell wall also has interspersed lipid molecules which serve to anchor it to the surrounding membrane.

In gram-positive bacteria, such as *Corynebacterium glutamicum,* the cytoplasmic membrane is covered by a multi-layered peptidoglycan, which ranges from 20-80 nm in thickness (see, *e.g.,* Lengeler *et al.* (1999) Biology of Prokaryotes Thieme Verlag: Stuttgart, p. 913-918, p. 875-899, and p. 88-109 and references therein). The gram-positive cell wall also contains teichoic acid, a polymer of glycerol or ribitol linked through phosphate groups. Teichoic acid is also able to associate with amino acids, and forms covalent bonds with muramic acid. Also present in the cell wall may be lipoteichoic acids and teichuronic acids. If present, cellular surface structures such as flagella or capsules will be anchored in this layer as well.

### III. Elements and Methods of the Invention

The present invention is based, at least in part, on the discovery of novel molecules, referred to herein as HA nucleic acid and protein molecules, which participate in the maintenance of homeostasis in C. *glutamicum,* or which perform a function involved in the adaptation of this microorganism to different environmental conditions. In one embodiment, the HA molecules participate in C. *glutamicum* cell wall biosynthesis or rearrangements, in the metabolism of inorganic compounds, in the modification or degradation of aromatic or aliphatic compounds, or have an enzymatic or proteolytic activity. In a preferred embodiment, the activity of the HA molecules of the present invention with regard to *C. glutamicum* cell wall biosynthesis or rearrangements, metabolism of inorganic compounds, modification or degradation of aromatic or aliphatic compounds, or enzymatic or proteolytic activity has an impact on the production of a desired fine chemical by this organism. In a particularly preferred embodiment, the HA molecules of the invention are modulated in activity, such that the *C. glutamicum* cellular processes in which the HA molecules participate *(e.g., C. glutamicum* cell wall biosynthesis or rearrangements, metabolism of inorganic compounds, modification or degradation of aromatic or aliphatic compounds, or enzymatic or proteolytic activity) are also altered in activity, resulting either directly or indirectly in a modulation of the yield, production, and/or efficiency of production of a desired fine chemical by *C. glutamicum.*

The language, "HA protein" or "HA polypeptide" includes proteins which participate in a number of cellular processes related to C. *glutamicum* homeostasis or the ability of C. *glutamicum* cells to adapt to unfavorable environmental conditions. For example, an HA protein may be involved in C. *glutamicum* cell wall biosynthesis or rearrangements, in the metabolism of inorganic compounds in *C*. *glutamicum,* in the modification or degradation of aromatic or aliphatic compounds in C. *glutamicum,* or have a C *glutamicum* enzymatic or proteolytic activity. Examples of HA proteins include those encoded by the HA genes set forth in Table 1 and by the odd-numbered SEQ ID NOs. The terms "HA gene" or "HA nucleic acid sequence" include nucleic acid sequences encoding an HA protein, which consist of a coding region and also corresponding untranslated 5' and 3' sequence regions. Examples of HA genes include those set forth in Table 1. The terms "production" or "productivity" are art-recognized and include the concentration of the fermentation product (for example, the desired fine chemical) formed within a given time and a given fermentation volume (e.g., kg product per hour per liter). The term "efficiency of production" includes the time required for a particular level of production to be achieved (for example, how long it takes for the cell to attain a particular rate of output of a fine chemical). The term "yield" or "product/carbon yield" is art-recognized and includes the efficiency of the conversion of the carbon source into the product *(i.e.,* fine chemical). This is generally written as, for example, kg product per kg carbon source. By increasing the yield or production of the compound, the quantity of recovered molecules, or of useful recovered molecules of that compound in a given amount of culture over a given amount of time is increased. The terms "biosynthesis" or a "biosynthetic pathway" are art-recognized and include the synthesis of a compound, preferably an organic compound, by a cell from intermediate compounds in what may be a multistep and highly regulated process. The terms "degradation" or a "degradation pathway" are art-recognized and include the breakdown of a compound, preferably an organic compound, by a cell to degradation products (generally speaking, smaller or less complex molecules) in what may be a multistep and highly regulated process. The language "metabolism" is art-recognized and includes the totality of the biochemical reactions that take place in an organism. The metabolism of a particular compound, then, (e.g., the metabolism of an amino acid such as glycine) comprises the overall biosynthetic, modification, and degradation pathways in the cell related to this compound. The term "homeostasis" is art-recognized and includes all of the mechanisms utilized by a cell to maintain a constant intracellular environment despite the prevailing extracellular environmental conditions. A non-limiting example of such processes is the utilization of a cell wall to prevent osmotic lysis due to high intracellular solute concentrations. The term "adaptation" or "adaptation to an environmental condition" is art-recognized and includes mechanisms utilized by the cell to render the cell able to survive under nonpreferred environmental conditions (generally speaking, those environmental conditions in which one or more favored nutrients are absent, or in which an environmental condition such as temperature, pH, osmolarity, oxygen percentage and the like fall outside of the optimal survival range of the cell). Many cells, including C. *glutamicum* cells, possess genes encoding proteins which are expressed under such environmental conditions and which permit continued growth in such suboptimal conditions.

In another embodiment, the HA molecules of the invention are capable of modulating the production of a desired molecule, such as a fine chemical, in a microorganism such as C. *glutamicum.* There are a number of mechanisms by which the alteration of an HA protein of the invention may directly affect the yield, production, and/or efficiency of production of a fine chemical from a C. *glutamicum* strain incorporating such an altered protein. For example, by engineering enzymes which modify or degrade aromatic or aliphatic compounds such that these enzymes are increased or decreased in activity or number, it may be possible to modulate the production of one or more fine chemicals which are the modification or degradation products of these compounds. Similarly, enzymes involved in the metabolism of inorganic compounds provide key molecules (e.g. phosphorous, sulfur, and nitrogen molecules) for the biosynthesis of such fine chemicals as amino acids, vitamins, and nucleic acids. By altering the activity or number of these enzymes in C. *glutamicum,* it may be possible to increase the conversion of these inorganic compounds (or to use alternate inorganic compounds) to thus permit improved rates of incorporation of inorganic atoms into these fine chemicals. Genetic engineering of C. *glutamicum* enzymes involved in general cellular processes may also directly improve fine chemical production, since many of these enzymes directly modify fine chemicals (e.g., amino acids) or the enzymes which are involved in fine chemical synthesis or secretion. Modulation of the activity or number of cellular proteases may also have a direct effect on fine chemical production, since many proteases may degrade fine chemicals or enzymes involved in fine chemical production or breakdown.

Further, the aforementioned enzymes which participate in aromatic/aliphatic compound modification or degradation, general biocatalysis, inorganic compound metabolism or proteolysis are each themselves fine chemicals, desirable for their activity in *various in vitro* industrial applications. By altering the number of copies of the gene for one or more of these enzymes in C. *glutamicum* it may be possible to increase the number of these proteins produced by the cell, thereby increasing the potential yield or efficiency of production of these proteins from large-scale C. *glutamicum* or related bacterial cultures.

The alteration of an HA protein of the invention may also indirectly affect the yield, production, and/or efficiency of production of a fine chemical from a C. *glutamicum* strain incorporating such an altered protein. For example, by modulating the activity and/or number of those proteins involved in the construction or rearrangement of the cell wall, it may be possible to modify the structure of the cell wall itself such that the cell is able to better withstand the mechanical and other stresses present during large-scale fermentative culture. Also, large-scale growth of C. *glutamicum* requires significant cell wall production. Modulation of the activity or number of cell wall biosynthetic or degradative enzymes may allow more rapid rates of cell wall biosynthesis, which in turn may permit increased growth rates of this microorganism in culture and thereby increase the number of cells producing the desired fine chemical.

By modifying the HA enzymes of the invention, one may also indirectly impact the yield, production, or efficiency of production of one or more fine chemicals from C. *glutamicum.* For example, many of the general enzymes in C. *glutamicum* may have a significant impact on global cellular processes (e.g., regulatory processes) which in turn have a significant effect on fine chemical metabolism. Similarly, proteases, enzymes which modify or degrade possibly toxic aromatic or aliphatic compounds, and enzymes which promote the metabolism of inorganic compounds all serve to increase the viability of C. *glutamicum.* The proteases aid in the selective removal of misfolded or misregulated proteins, such as those that might occur under the relatively stressful environmental conditions encountered during large-scale fermentor culture. By altering these proteins, it may be possible to further enhance this activity and to improve the viability of C*. glutamicum* in culture. The aromatic/aliphatic modification or degradation proteins not only serve to detoxify these waste compounds (which may be encountered as impurities in culture medium or as waste products from cells themselves), but also to permit the cells to utilize alternate carbon sources if the optimal carbon source is limiting in the culture. By increasing their number and/or activity, the survival of C. *glutamicum* cells in culture may be enhanced. The inorganic metabolism proteins of the invention supply the cell with inorganic molecules required for all protein and nucleotide (among others) synthesis, and thus are critical for the overall viability of the cell. An increase in the number of viable cells producing one or more desired fine chemicals in large-scale culture should result in a concomitant increase in the yield, production, and/or efficiency of production of the fine chemical in the culture.

The isolated nucleic acid sequences of the invention are contained within the genome of a *Corynebacterium glutamicum* strain available through the American Type Culture Collection, given designation ATCC 13032. The nucleotide sequence of the isolated C. *glutamicum* HA DNAs and the predicted amino acid sequences of the C. *glutamicum* HA proteins are shown in the Sequence Listing as odd-numbered SEQ ID NOs and even-numbered SEQ ID NOs, respectively, respectively. Computational analyses were performed which classified and/or identified these nucleotide sequences as sequences which encode proteins that participate in C. *glutamicum* cell wall biosynthesis or rearrangements, metabolism of inorganic compounds, modification or degradation of aromatic or aliphatic compounds, or that have a C. *glutamicum* enzymatic or proteolytic activity.

The present invention also pertains to proteins which have an amino acid sequence which is substantially homologous to an amino acid sequence of the invention (e.g., the sequence of an even-numbered SEQ ID NO of the Sequence Listing). As used herein, a protein which has an amino acid sequence which is substantially homologous to a selected amino acid sequence is least about 50% homologous to the selected amino acid sequence, e.g., the entire selected amino acid sequence. A protein which has an amino acid sequence which is substantially homologous to a selected amino acid sequence can also be least about 50-60%, preferably at least about 60-70%, and more preferably at least about 70-80%, 80-90%, or 90-95%, and most preferably at least about 96%, 97%, 98%, 99% or more homologous to the selected amino acid sequence.

The HA protein or a biologically active portion or fragment thereof of the invention can participate in the maintenance of homeostasis in C. *glutamicum,* or can perform a function involved in the adaptation of this microorganism to different environmental conditions, or have one or more of the activities set forth in Table 1.

Various aspects of the invention are described in further detail in the following subsections.

### A. Isolated Nucleic Acid Molecules

One aspect of the invention pertains to isolated nucleic acid molecules that encode HA polypeptides or biologically active portions thereof, as well as nucleic acid fragments sufficient for use as hybridization probes or primers for the identification or amplification of HA-encoding nucleic acid (e.g., HA DNA). As used herein, the term "nucleic acid molecule" is intended to include DNA molecules (e.g., cDNA or genomic DNA) and RNA molecules (e.g., mRNA) and analogs of the DNA or RNA generated using nucleotide analogs. This term also encompasses untranslated sequence located at both the 3' and 5' ends of the coding region of the gene: at least about 100 nucleotides of sequence upstream from the 5' end of the coding region and at least about 20 nucleotides of sequence downstream from the 3'end of the coding region of the gene. The nucleic acid molecule can be single-stranded or double-stranded, but preferably is double-stranded DNA. An "isolated" nucleic acid molecule is one which is separated from other nucleic acid molecules which are present in the natural source of the nucleic acid. Preferably, an "isolated" nucleic acid is free of sequences which naturally flank the nucleic acid (i.e., sequences located at the 5' and 3' ends of the nucleic acid) in the genomic DNA of the organism from which the nucleic acid is derived. For example, in various embodiments, the isolated HA nucleic acid molecule can contain less than about 5 kb, 4kb, 3kb, 2kb, 1 kb, 0.5 kb or 0.1 kb of nucleotide sequences which naturally flank the nucleic acid molecule in genomic DNA of the cell from which the nucleic acid is derived (e.g, a C. *glutamicum* cell). Moreover, an "isolated" nucleic acid molecule, such as a DNA molecule, can be substantially free of other cellular material, or culture medium when produced by recombinant techniques, or chemical precursors or other chemicals when chemically synthesized.

A nucleic acid molecule of the present invention, e.g., a nucleic acid molecule having a nucleotide sequence of an odd-numbered SEQ ID NO of the Sequence Listing, or a portion thereof, can be isolated using standard molecular biology techniques and the sequence information provided herein. For example, a C. *glutamicum* HA DNA can be isolated from a C. *glutamicum* library using all or portion of one of the odd-numbered SEQ ID NO sequences of the Sequence Listing as a hybridization probe and standard hybridization techniques (e.g., as described in Sambrook, J., Fritsh, E. F., and Maniatis, *T. Molecular Cloning: A Laboratory Manual. 2nd, ed., Cold Spring Harbor Laboratory,* Cold Spring Harbor Laboratory Press, Cold Spring Harbor, NY, 1989). Moreover, a nucleic acid molecule encompassing all or a portion of one of the nucleic acid sequences of the invention (e.g., an odd-numbered SEQ ID NO:) can be isolated by the polymerase chain reaction using oligonucleotide primers designed based upon this sequence (e.g., a nucleic acid molecule encompassing all or a portion of one of the nucleic acid sequences of the invention (*e.g.*, an odd-numbered SEQ ID NO of the Sequence Listing) can be isolated by the polymerase chain reaction using oligonucleotide primers designed based upon this same sequence). For example, mRNA can be isolated from normal endothelial cells (e.g., by the guanidinium-thiocyanate extraction procedure of Chirgwin *et al.* (1979) *Biochemistry* 18: 5294-5299) and DNA can be prepared using reverse transcriptase (e.g., Moloney MLV reverse transcriptase, available from Gibco/BRL, Bethesda, MD; or AMV reverse transcriptase, available from Seikagaku America, Inc., St. Petersburg, FL). Synthetic oligonucleotide primers for polymerase chain reaction amplification can be designed based upon one of the nucleotide sequences shown in the Sequence Listing. A nucleic acid of the invention can be amplified using cDNA or, alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to an HA nucleotide sequence can be prepared by standard synthetic techniques, e.g., using an automated DNA synthesizer.

In a preferred embodiment, an isolated nucleic acid molecule of the invention comprises one of the nucleotide sequences shown in the Sequence Listing. The nucleic acid sequences of the invention, as set forth in the Sequence Listing, correspond to the *Corynebacterium glutamicum* HA DNAs of the invention. This DNA comprises sequences encoding HA proteins (i.e., the "coding region", indicated in each odd-numbered SEQ ID NO: sequence in the Sequence Listing), as well as 5' untranslated sequences and 3' untranslated sequences, also indicated in each odd-numbered SEQ ID NO: in the Sequence Listing. Alternatively, the nucleic acid molecule can comprise only the coding region of any of the sequences in nucleic acid sequences of the Sequence Listing

For the purposes of this application, it will be understood that each of the nucleic acid and amino acid sequences set forth in the Sequence Listing has an identifying RXA, RXN, RXS, or RXC number having the designation "RXA," "RXN," "RXS, or "RXC" followed by 5 digits (i.e., RXA02458, RXN00249, RXS00153, or RXC00963). Each of the nucleic acid sequences comprises up to three parts: a 5' upstream region, a coding region, and a downstream region. Each of these three regions is identified by the same RXA, RXN, RXS, or RXC designation to eliminate confusion. The recitation "one of the odd-numbered sequences in of the Sequence Listing", then, refers to any of the nucleic acid sequences in the Sequence Listing, which may also be distinguished by their differing RXA, RXN, RXS, or RXC designations. The coding region of each of these sequences is translated into a corresponding amino acid sequence, which is also set forth in the Sequence Listing, as an even-numbered SEQ ID NO: immediately following the corresponding nucleic acid sequence . For example, the coding region for RXA02548 is set forth in SEQ ID NO:1, while the amino acid sequence which it encodes is set forth as SEQ ID NO:2. The sequences of the nucleic acid molecules of the invention are identified by the same RXA, RXN, RXS, or RXC designations as the amino acid molecules which they encode, such that they can be readily correlated. For example, the amino acid sequences designated RXA02458, RXN00249, RXS00153, and RXC00963 are translations of the coding regions of the nucleotide sequences of nucleic acid molecules RXA02458, RXN00249, RXS00153, and RXC00963, respectively. of the correspondence between the RXA, RXN, RXS, and RXC nucleotide and amino acid sequences of the invention and their assigned SEQ ID NOs is set forth in Table 1.

Several of the genes of the invention are "F-designated genes". An F-designated gene includes those genes set forth in Table 1 which have an 'F' in front of the RXA, RXN, RXS, or RXC designation. For example, SEQ ID NO: 5, designated, as indicated on Table 1, as "F RXA00249", is an F-designated gene, as are SEQ ID NOs: 11, 15, and 33 (designated on Table 1 as "F RXA02264", "F RXA02274", and "F RXA00675", respectively).

In one embodiment, the nucleic acid molecules of the present invention are not intended to include those compiled in Table 2. In the case of the dapD gene, a sequence for this gene was published in Wehrmann, A., *et al.* (1998) *J Bacteriol.* 180(12): 3159-3165. However, the sequence obtained by the inventors of the present application is significantly longer than the published version. It is believed that the published version relied on an incorrect start codon, and thus represents only a fragment of the actual coding region.

In another preferred embodiment, an isolated nucleic acid molecule of the invention comprises a nucleic acid molecule which is a complement of one of the nucleotide sequences of the invention (e.g., a sequence of an odd-numbered SEQ ID NO: of the Sequence Listing), or a portion thereof. A nucleic acid molecule which is complementary to one of the nucleotide sequences of the invention is one which is sufficiently complementary to one of the nucleotide sequences the Sequence Listing (e.g., the sequence of an odd-numbered SEQ ID NO:) such that it can hybridize to one of the nucleotide sequences of the invention, thereby forming a stable duplex.

In still another preferred embodiment, an isolated nucleic acid molecule of the invention comprises a nucleotide sequence which is at least about 50%, 51%, 52%, 53%, 54%, 55%, 56%, 57%, 58%, 59%, or 60%, preferably at least about 61%, 62%, 63%, 64%, 65%, 66%, 67%, 68%, 69%, or 70%%, more preferably at least about 71 %, 72%, 73%, 74%, 75%, 76%, 77%, 78%, 79%, or 80%, 81%, 82%, 83%, 84%, 85%, 86%, 87%, 88%, 89%, or 90%, or 91%, 92%, 93%, 94%, and even more preferably at least about 95%, 96%, 97%, 98%, 99% or more homologous to a nucleotide sequence of the invention (*e.g.*, a sequence of an odd-numbered SEQ ID NO: of the Sequence Listing), or a portion thereof. Ranges and identity values intermediate to the above-recited ranges, (e.g., 70-90% identical or 80-95% identical) are also intended to be encompassed by the present invention. For example, ranges of identity values using a combination of any of the above values recited as upper and/or lower limits are intended to be included. In an additional preferred embodiment, an isolated nucleic acid molecule of the invention comprises a nucleotide sequence which hybridizes, e.g., hybridizes under stringent conditions, to one of the nucleotide sequences of the invention, or a portion thereof.

Moreover, the nucleic acid molecule of the invention can comprise only a portion of the coding region of the sequence of one of the odd-numbered SEQ ID NOs of the Sequence Listing, for example a fragment which can be used as a probe or primer or a fragment encoding a biologically active portion of an HA protein. The nucleotide sequences determined from the cloning of the HA genes from C. *glutamicum* allows for the generation of probes and primers designed for use in identifying and/or cloning HA homologues in other cell types and organisms, as well as HA homologues from other *Corynebacteria* or related species. The probe/primer typically comprises substantially purified oligonucleotide. The oligonucleotide typically comprises a region of nucleotide sequence that hybridizes under stringent conditions to at least about 12, preferably about 25, more preferably about 40, 50 or 75 consecutive nucleotides of a sense strand of one of the nucleotide sequences of the invention (e.g., a sequence of one of the odd-numbered SEQ ID NOs of the Sequence Listing), an anti-sense sequence of one of these sequences , or naturally occurring mutants thereof. Primers based on a nucleotide sequence of the invention can be used in PCR reactions to clone HA homologues. Probes based on the HA nucleotide sequences can be used to detect transcripts or genomic sequences encoding the same or homologous proteins. In preferred embodiments, the probe further comprises a label group attached thereto, e.g. the label group can be a radioisotope, a fluorescent compound, an enzyme, or an enzyme cofactor. Such probes can be used as a part of a diagnostic test kit for identifying cells which misexpress an HA protein, such as by measuring a level of an HA-encoding nucleic acid in a sample of cells, e.g., detecting HA mRNA levels or determining whether a genomic HA gene has been mutated or deleted.

In one embodiment, the nucleic acid molecule of the invention encodes a protein or portion thereof which includes an amino acid sequence which is sufficiently homologous to an amino acid sequence of the invention (e.g., a sequence of an even-numbered SEQ ID NO of the Sequence Listing) such that the protein or portion thereof maintains the ability to participate in the maintenance of homeostasis in C. *glutamicum,* or to perform a function involved in the adaptation of this microorganism to different environmental conditions. As used herein, the language "sufficiently homologous" refers to proteins or portions thereof which have amino acid sequences which include a minimum number of identical or equivalent (e.g., an amino acid residue which has a similar side chain as an amino acid residue in a sequence of one of the even-numbered SEQ ID NOs of the Sequence Listing) amino acid residues to an amino acid sequence of the invention such that the protein or portion thereof is able to participate in the maintenance of homeostasis in C. *glutamicum,* or to perform a function involved in the adaptation of this microorganism to different environmental conditions. Proteins involved in C. *glutamicum* cell wall biosynthesis or rearrangements, metabolism of inorganic compounds, modification or degradation of aromatic or aliphatic compounds, or that have a *C*. *glutamicum* enzymatic or proteolytic activity, as described herein, may play a role in the production and secretion of one or more fine chemicals. Examples of such activities are also described herein. Thus, "the function of an HA protein" contributes either directly or indirectly to the yield, production, and/or efficiency of production of one or more fine chemicals. Examples of HA protein activities are set forth in Table 1.

In another embodiment, the protein is at least about 50-60%, preferably at least about 60-70%, and more preferably at least about 70-80%, 80-90%, 90-95%, and most preferably at least about 96%, 97%, 98%, 99% or more homologous to an entire amino acid sequence of the invention (e.g., a sequence of an even-numbered SEQ ID NO: of the Sequence Listing).

Portions of proteins encoded by the HA nucleic acid molecules of the invention are preferably biologically active portions of one of the HA proteins. As used herein, the term "biologically active portion of an HA protein" is intended to include a portion, e.g., a domain/motif, of an HA protein that can participate in the maintenance of homeostasis in *C glutamicum,* or that can perform a function involved in the adaptation of this microorganism to different environmental conditions, or has an activity as set forth in Table 1. To determine whether an HA protein or a biologically active portion thereof can participate in *C. glutamicum* cell wall biosynthesis or rearrangements, metabolism of inorganic compounds, modification or degradation of aromatic or aliphatic compounds, or has a *C*. *glutamicum* enzymatic or proteolytic activity, an assay of enzymatic activity may be performed. Such assay methods are well known to those of ordinary skill in the art, as detailed in Example 8 of the Exemplification.

Additional nucleic acid fragments encoding biologically active portions of an HA protein can be prepared by isolating a portion of one of the s amino acid sequences in of the invention (*e.g*., a sequence of an even-numbered SEQ ID NO: of the Sequence Listing), expressing the encoded portion of the HA protein or peptide (e.g., by recombinant expression *in vitro)* and assessing the activity of the encoded portion of the HA protein or peptide.

The invention further encompasses nucleic acid molecules that differ from one of the nucleotide sequences of the invention *(e.g.,* a sequence of an odd-numbered SEQ ID NO: of the Sequence Listing) (and portions thereof) due to degeneracy of the genetic code and thus encode the same HA protein as that encoded by the nucleotide sequences shown in of the invention. In another embodiment, an isolated nucleic acid molecule of the invention has a nucleotide sequence encoding a protein having an amino acid sequence shown in the Sequence Listing (e.g., an even-numbered SEQ ID NO:). In a still further embodiment, the nucleic acid molecule of the invention encodes a full length *C. glutamicum* protein which is substantially homologous to an amino acid sequence of the invention (encoded by an open reading frame shown in an odd-numbered SEQ ID NO: of the Sequence Listing).

It will be understood by one of ordinary skill in the art that in one embodiment the sequences of the invention are not meant to include the sequences of the prior art, such as those Genbank sequences set forth in Tables 2 or 4 which were available prior to the present invention. In one embodiment, the invention includes nucleotide and amino acid sequences having a percent identity to a nucleotide or amino acid sequence of the invention which is greater than that of a sequence of the prior art (e.g., a Genbank sequence (or the protein encoded by such a sequence) set forth in Tables 2 or 4). For example, the invention includes a nucleotide sequence which is greater than and/or at least 39% identical to the nucleotide sequence designated RXA00471(SEQ ID NO:293), a nucleotide sequence which is greater than and/or at least 41 % identical to the nucleotide sequence designated RXA00500 (SEQ ID NO: 143), and a nucleotide sequence which is greater than and/or at least 35% identical to the nucleotide sequence designated RXA00502(SEQ ID N0:147). One of ordinary skill in the art would be able to calculate the lower threshold of percent identity for any given sequence of the invention by examining the GAP-calculated percent identity scores set forth in Table 4 for each of the three top hits for the given sequence, and by subtracting the highest GAP-calculated percent identity from 100 percent. One of ordinary skill in the art will also appreciate that nucleic acid and amino acid sequences having percent identities greater than the lower threshold so calculated *(e.g.,* at least 50%, 51%, 52%, 53%, 54%, 55%, 56%, 57%, 58%, 59%, or 60%, preferably at least about 61%, 62%, 63%, 64%, 65%, 66%, 67%, 68%, 69%, or 70%, more preferably at least about 71 %, 72%, 73%, 74%, 75%, 76%, 77%, 78%, 79%, or 80%, 81%, 82%, 83%, 84%, 85%, 86%, 87%, 88%, 89%, or 90%, or 91%, 92%, 93%, 94%, and even more preferably at least about 95%, 96%, 97%, 98%, 99% or more identical) are also encompassed by the invention.

In addition to the *C*. *glutamicum* HA nucleotide set forth in the Sequence Listing as odd-numbered SEQ ID NOs, it will be appreciated by those of ordinary skill in the art that DNA sequence polymorphisms that lead to changes in the amino acid sequences of HA proteins may exist within a population *(e.g.,* the *C*. *glutamicum* population). Such genetic polymorphism in the HA gene may exist among individuals within a population due to natural variation. As used herein, the terms "gene" and "recombinant gene" refer to nucleic acid molecules comprising an open reading frame encoding an HA protein, preferably a *C. glutamicum* HA protein. Such natural variations can typically result in 1-5% variance in the nucleotide sequence of the HA gene. Any and all such nucleotide variations and resulting amino acid polymorphisms in HA that are the result of natural variation and that do not alter the functional activity of HA proteins are intended to be within the scope of the invention.

Nucleic acid molecules corresponding to natural variants and non-*C*. *glutamicum* homologues of the *C. glutamicum* HA DNA of the invention can be isolated based on their homology to the *C*. *glutamicum* HA nucleic acid disclosed herein using the *C*. *glutamicum* DNA, or a portion thereof, as a hybridization probe according to standard hybridization techniques under stringent hybridization conditions. Accordingly, in another embodiment, an isolated nucleic acid molecule of the invention is at least 15 nucleotides in length and hybridizes under stringent conditions to the nucleic acid molecule comprising a nucleotide sequence of an odd-numbered SEQ ID NO: of the Sequence Listing. In other embodiments, the nucleic acid is at least 30, 50, 100, 250 or more nucleotides in length. As used herein, the term "hybridizes under stringent conditions" is intended to describe conditions for hybridization and washing under which nucleotide sequences at least 60% homologous to each other typically remain hybridized to each other. Preferably, the conditions are such that sequences at least about 65%, more preferably at least about 70%, and even more preferably at least about 75% or more homologous to each other typically remain hybridized to each other. Such stringent conditions are known to those of ordinary skill in the art and can be found in *Current Protocols in Molecular Biology,* John Wiley & Sons, N.Y. (1989), 6.3.1-6.3.6. A preferred, non-limiting example of stringent hybridization conditions are hybridization in 6X sodium chloride/sodium citrate (SSC) at about 45°C, followed by one or more washes in 0.2 X SSC, 0.1% SDS at 50-65°C. Preferably, an isolated nucleic acid molecule of the invention that hybridizes under stringent conditions to a nucleotide sequence of the invention corresponds to a naturally-occurring nucleic acid molecule. As used herein, a "naturally-occurring" nucleic acid molecule refers to an RNA or DNA molecule having a nucleotide sequence that occurs in nature (e.g., encodes a natural protein). In one embodiment, the nucleic acid encodes a natural *C*. *glutamicum* HA protein.

In addition to naturally-occurring variants of the HA sequence that may exist in the population, one of ordinary skill in the art will further appreciate that changes can be introduced by mutation into a nucleotide sequence of the invention, thereby leading to changes in the amino acid sequence of the encoded HA protein, without altering the functional ability of the HA protein. For example, nucleotide substitutions leading to amino acid substitutions at "non-essential" amino acid residues can be made in a nucleotide sequence of the invention. A "non-essential" amino acid residue is a residue that can be altered from the wild-type sequence of one of the HA proteins (e.g., an even-numbered SEQ ID NO: of the Sequence Listing) without altering the activity of said HA protein, whereas an "essential" amino acid residue is required for HA protein activity. Other amino acid residues, however, (*e.g*., those that are not conserved or only semi-conserved in the domain having HA activity) may not be essential for activity and thus are likely to be amenable to alteration without altering HA activity.

Accordingly, another aspect of the invention pertains to nucleic acid molecules encoding HA proteins that contain changes in amino acid residues that are not essential for HA activity. Such HA proteins differ in amino acid sequence from a sequence of an even-numbered SEQ ID NO: of the Sequence Listing yet retain at least one of the HA activities described herein. In one embodiment, the isolated nucleic acid molecule comprises a nucleotide sequence encoding a protein, wherein the protein comprises an amino acid sequence at least about 50% homologous to an amino acid sequence of the invention and is capable of participating in the maintenance of homeostasis in *C*. *glutamicum,* or of performing a function involved in the adaptation of this microorganism to different environmental conditions, or has one or more of the activities set forth in Table 1. Preferably, the protein encoded by the nucleic acid molecule is at least about 50-60% homologous to the amino acid sequence of one of the odd-numbered SEQ ID NOs of the Sequence Listing, more preferably at least about 60-70% homologous to one of these sequences in, even more preferably at least about 70-80%, 80-90%, 90-95% homologous to one of these sequences, and most preferably at least about 96%, 97%, 98%, or 99% homologous to one of the amino acid sequences of the invention

To determine the percent homology of two amino acid sequences (e.g., one of the amino acid sequences of the invention and a mutant form thereof) or of two nucleic acids, the sequences are aligned for optimal comparison purposes (e.g., gaps can be introduced in the sequence of one protein or nucleic acid for optimal alignment with the other protein or nucleic acid). The amino acid residues or nucleotides at corresponding amino acid positions or nucleotide positions are then compared. When a position in one sequence (e.g., one of the amino acid sequences of the invention) is occupied by the same amino acid residue or nucleotide as the corresponding position in the other sequence (e.g., a mutant form of the amino acid sequence), then the molecules are homologous at that position (i.e., as used herein amino acid or nucleic acid "homology" is equivalent to amino acid or nucleic acid "identity"). The percent homology between the two sequences is a function of the number of identical positions shared by the sequences *(i.e.,* % homology = # of identical positions/total # of positions x 100).

An isolated nucleic acid molecule encoding an HA protein homologous to a protein sequence of the invention (e.g., a sequence of an even-numbered SEQ ID NO: of the Sequence Listing) can be created by introducing one or more nucleotide substitutions, additions or deletions into a nucleotide sequence of the invention such that one or more amino acid substitutions, additions or deletions are introduced into the encoded protein. Mutations can be introduced into one of the nucleotide sequences of the invention by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in an HA protein is preferably replaced with another amino acid residue from the same side chain family. Alternatively, in another embodiment, mutations can be introduced randomly along all or part of an HA coding sequence, such as by saturation mutagenesis, and the resultant mutants can be screened for an HA activity described herein to identify mutants that retain HA activity. Following mutagenesis of the nucleotide sequence of one of the odd-numbered SEQ ID NOs of the Sequence Listing, the encoded protein can be expressed recombinantly and the activity of the protein can be determined using, for example, assays described herein (see Example 8 of the Exemplification).

In addition to the nucleic acid molecules encoding HA proteins described above, another aspect of the invention pertains to isolated nucleic acid molecules which are antisense thereto. An "antisense" nucleic acid comprises a nucleotide sequence which is complementary to a "sense" nucleic acid encoding a protein, e.g., complementary to the coding strand of a double-stranded DNA molecule or complementary to an mRNA sequence. Accordingly, an antisense nucleic acid can hydrogen bond to a sense nucleic acid. The antisense nucleic acid can be complementary to an entire HA coding strand, or to only a portion thereof In one embodiment, an antisense nucleic acid molecule is antisense to a "coding region" of the coding strand of a nucleotide sequence encoding an HA protein. The term "coding region" refers to the region of the nucleotide sequence comprising codons which are translated into amino acid residues (e.g., the entire coding region of SEQ ID NO. 3 (RXN00249) comprises nucleotides 1 to 957). In another embodiment, the antisense nucleic acid molecule is antisense to a "noncoding region" of the coding strand of a nucleotide sequence encoding HA. The term "noncoding region" refers to 5' and 3' sequences which flank the coding region that are not translated into amino acids *(i.e.,* also referred to as 5' and 3' untranslated regions).

Given the coding strand sequences encoding HA disclosed herein (e.g., the sequences set forth as odd-numbered SEQ ID NOs in the Sequence Listing), antisense nucleic acids of the invention can be designed according to the rules of Watson and Crick base pairing. The antisense nucleic acid molecule can be complementary to the entire coding region of HA mRNA, but more preferably is an oligonucleotide which is antisense to only a portion of the coding or noncoding region of HA mRNA. For example, the antisense oligonucleotide can be complementary to the region surrounding the translation start site of HA mRNA. An antisense oligonucleotide can be, for example, about 5, 10, 15, 20, 25, 30, 35, 40, 45 or 50 nucleotides in length. An antisense nucleic acid of the invention can be constructed using chemical synthesis and enzymatic ligation reactions using procedures known in the art. For example, an antisense nucleic acid *(e.g.,* an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation *(i.e.,* RNA transcribed from the inserted nucleic acid will be of an antisense orientation to a target nucleic acid of interest, described further in the following subsection).

The antisense nucleic acid molecules of the invention are typically administered to a cell or generated *in situ* such that they hybridize with or bind to cellular mRNA and/or genomic DNA encoding an HA protein to thereby inhibit expression of the protein, e.g., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. The antisense molecule can be modified such that it specifically binds to a receptor or an antigen expressed on a selected cell surface, e.g., by linking the antisense nucleic acid molecule to a peptide or an antibody which binds to a cell surface receptor or antigen. The antisense nucleic acid molecule can also be delivered to cells using the vectors described herein. To achieve sufficient intracellular concentrations of the antisense molecules, vector constructs in which the antisense nucleic acid molecule is placed under the control of a strong prokaryotic, viral, or eukaryotic promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier *et al.* (1987) *Nucleic Acids. Res.* 15:6625-6641). The antisense nucleic acid molecule can also comprise a 2'-o-methylribonucleotide (Inoue *et al.* (1987) *Nucleic Acids Res.* 15:6131-6148) or a chimeric RNA-DNA analogue (Inoue *et al.* (1987) *FEBS Lett.* 215:327-330).

In still another embodiment, an antisense nucleic acid of the invention is a ribozyme. Ribozymes are catalytic RNA molecules with ribonuclease activity which are capable of cleaving a single-stranded nucleic acid, such as an mRNA, to which they have a complementary region. Thus, ribozymes (e.g., hammerhead ribozymes (described in Haselhoff and Gerlach (1988) *Nature* 334:585-591)) can be used to catalytically cleave HA mRNA transcripts to thereby inhibit translation of HA mRNA. A ribozyme having specificity for an HA-encoding nucleic acid can be designed based upon the nucleotide sequence of an HA DNA molecule disclosed herein (i.e., SEQ ID NO. 3 (RXN00249)). For example, a derivative of a *Tetrahymena* L-19 IVS RNA can be constructed in which the nucleotide sequence of the active site is complementary to the nucleotide sequence to be cleaved in an HA-encoding mRNA. See, *e.g.,* Cech *et al.* U.S. Patent No. 4,987,071 and Cech *et al.* U.S. Patent No. 5,116,742. Alternatively, HA mRNA can be used to select a catalytic RNA having a specific ribonuclease activity from a pool of RNA molecules. See, *e.g.,* Bartel, D. and Szostak, J.W. (1993) *Science* 261:1411-1418.

Alternatively, HA gene expression can be inhibited by targeting nucleotide sequences complementary to the regulatory region of an HA nucleotide sequence (e.g., an HA promoter and/or enhancers) to form triple helical structures that prevent transcription of an HA gene in target cells. See generally, Helene, C. (1991) *Anticancer Drug Des.* 6(6):569-84; Helene, C. *et al.* (1992) *Ann. N.Y. Acad. Sci.* 660:27-36; and Maher, L.J. (1992) *Bioassays* 14(12):807-15.

### B. Recombinant Expression Vectors and Host Cells

Another aspect of the invention pertains to vectors, preferably expression vectors, containing a nucleic acid encoding an HA protein (or a portion thereof). As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e.g*., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, which are operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (e.g., in an *in vitro* transcription/translation system or in a host cell when the vector is introduced into the host cell). The term "regulatory sequence" is intended to include promoters, enhancers and other expression control elements (e.g., polyadenylation signals). Such regulatory sequences are described, for example, in Goeddel; *Gene Expression Technology: Methods in Enzymology* 185, Academic Press, San Diego, CA (1990). Regulatory sequences include those which direct constitutive expression of a nucleotide sequence in many types of host cell and those which direct expression of the nucleotide sequence only in certain host cells. Preferred regulatory sequences are, for example, promoters such as cos-, tac-, trp-, tet-, trp-tet-, lpp-, lac-, lpp-lac-, lacI^{q}-, T7-, T5-, T3-, gal-, trc-, ara-, SP6-, arny, SP02, λ-P_{R-}or λ P_{L}, which are used preferably in bacteria. Additional regulatory sequences are, for example, promoters from yeasts and fungi, such as ADC1, MFα, AC, P-60, CYC1, GAPDH, TEF, rp28, ADH, promoters from plants such as CaMV/35S, SSU, OCS, lib4, usp, STLS1, B33, nos or ubiquitin- or phaseolin-promoters. It is also possible to use artificial promoters. It will be appreciated by those of ordinary skill in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (e.g., HA proteins, mutant forms of HA proteins, fusion proteins, etc.).

The recombinant expression vectors of the invention can be designed for expression of HA proteins in prokaryotic or eukaryotic cells. For example, HA genes can be expressed in bacterial cells such as C. *glutamicum,* insect cells (using baculovirus expression vectors), yeast and other fungal cells (see Romanos, M.A. *et al.* (1992) "Foreign gene expression in yeast: a review", *Yeast* 8: 423-488; van den Hondel, C.A.M.J.J. *et al.* (1991) "Heterologous gene expression in filamentous fungi" in: More Gene Manipulations in Fungi, J.W. Bennet & L.L. Lasure, eds., p. 396-428: Academic Press: San Diego; and van den Hondel, C.A.M.J.J. & Punt, P.J. (1991) "Gene transfer systems and vector development for filamentous fungi, in: Applied Molecular Genetics of Fungi, Peberdy, J.F. *et al.,* eds., p. 1-28, Cambridge University Press: Cambridge), algae and multicellular plant cells (see Schmidt, R. and Willmitzer, L. (1988) High efficiency *Agrobacterium tumefaciens* -mediated transformation *of Arabidopsis thaliana* leaf and cotyledon explants" *Plant Cell Rep.:* 583-586), or mammalian cells. Suitable host cells are discussed further in Goeddel, *Gene Expression Technology: Methods in Enzymology* 185, Academic Press, San Diego, CA (1990). Alternatively, the recombinant expression vector can be transcribed and translated *in vitro,* for example using T7 promoter regulatory sequences and T7 polymerase.

Expression of proteins in prokaryotes is most often carried out with vectors containing constitutive or inducible promoters directing the expression of either fusion or non-fusion proteins. Fusion vectors add a number of amino acids to a protein encoded therein, usually to the amino terminus of the recombinant protein but also to the C-terminus or fused within suitable regions in the proteins. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase.

Typical fusion expression vectors include pGEX (Pharmacia Biotech Inc; Smith, D.B. and Johnson, K.S. (1988) *Gene* 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein. In one embodiment, the coding sequence of the HA protein is cloned into a pGEX expression vector to create a vector encoding a fusion protein comprising, from the N-terminus to the C-terminus, GST-thrombin cleavage site-X protein. The fusion protein can be purified by affinity chromatography using glutathione-agarose resin. Recombinant HA protein unfused to GST can be recovered by cleavage of the fusion protein with thrombin.

Examples of suitable inducible non-fusion *E. coli* expression vectors include pTrc *(Amann et al.,* (1988) *Gene* 69:301-315) pLG338, pACYC184, pBR322, pUC18, pUC19, pKC30, pRep4, pHS1, pHS2, pPLc236, pMBL24, pLG200, pUR290, pIN-III113-B1, λgt11, pBdCl, and pET 11d (Studier *et al., Gene Expression Technology: Methods in Enzymology* 185, Academic Press, San Diego, California (1990) 60-89 ; and *Pouwels et al.,* eds. (1985) Cloning Vectors. Elsevier: New York IBSN 0 444 904018). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a coexpressed viral RNA polymerase (T7 gnl). This viral polymerase is supplied by host strains BL21(DE3) or HMS174(DE3) from a resident λ prophage harboring a T7 gn1 gene under the transcriptional control of the lacUV 5 promoter. For transformation of other varieties of bacteria, appropriate vectors may be selected. For example, the plasmids pIJ101, pIJ364, pIJ702 and pIJ361 are known to be useful in transforming Streptomyces, while plasmids pUB 110, pC 194, or pBD214 are suited for transformation of Bacillus species. Several plasmids of use in the transfer of genetic information into Corynebacterium include pHM1519, pBL1, pSA77, or pAJ667 *(Pouwels et al.,* eds. (1985) Cloning Vectors. Elsevier: New York IBSN 0 444 904018).

One strategy to maximize recombinant protein expression is to express the protein in a host bacteria with an impaired capacity to proteolytically cleave the recombinant protein (Gottesman, S., *Gene Expression Technology: Methods in Enzymology* 185, Academic Press, San Diego, California (1990) 119-128). Another strategy is to alter the nucleic acid sequence of the nucleic acid to be inserted into an expression vector so that the individual codons for each amino acid are those preferentially utilized in the bacterium chosen for expression, such as *C*. *glutamicum (Wada et al.* (1992) *Nucleic Acids Res.* 20: 2111-2118). Such alteration of nucleic acid sequences of the invention can be carried out by standard DNA synthesis techniques.

In another embodiment, the HA protein expression vector is a yeast expression vector. Examples of vectors for expression in yeast S. *cerevisiae* include pYepSec1 (Baldari, *et al.,* (1987) *Embo* J 6:229-234), 2 µ, pAG-1, Yep6, Yep13, pEMBLYe23, pMFa (Kurjan and Herskowitz, (1982) *Cell* 30:933-943), pJRY88 *(Schultz et al.,* (1987) *Gene* 54:113-123), and pYES2 (Invitrogen Corporation, San Diego, CA). Vectors and methods for the construction of vectors appropriate for use in other fungi, such as the filamentous fungi, include those detailed in: van den Hondel, C.A.M.J.J. & Punt, P.J. (1991) "Gene transfer systems and vector development for filamentous fungi, in: Applied Molecular Genetics of Fungi, J.F. *Peberdy, et al.,* eds., p. 1-28, Cambridge University Press: Cambridge, and *Pouwels et al.,* eds. (1985) Cloning Vectors. Elsevier: New York (IBSN 0 444 904018).

Alternatively, the HA proteins of the invention can be expressed in insect cells using baculovirus expression vectors. Baculovirus vectors available for expression of proteins in cultured insect cells *(e.g.,* Sf 9 cells) include the pAc series *(Smith et al.* (1983) *Mol. Cell Biol.* 3:2156-2165) and the pVL series (Lucklow and Summers (1989) *Virology* 170:31-39).

In another embodiment, the HA proteins of the invention may be expressed in unicellular plant cells (such as algae) or in plant cells from higher plants (e.g., the spermatophytes, such as crop plants). Examples of plant expression vectors include those detailed in: Becker, D., Kemper, E., Schell, J. and Masterson, R. (1992) "New plant binary vectors with selectable markers located proximal to the left border", *Plant Mol. Biol.* 20: 1195-1197; and Bevan, M.W. (1984) "Binary *Agrobacterium* vectors for plant transformation", *Nucl. Acid. Res.* 12: 8711-8721, and include pLGV23, pGHlac+, pBIN19, pAK2004, and pDH51 *(Pouwels et al.,* eds. (1985) Cloning Vectors. Elsevier: New York IBSN 0 444 904018).

In yet another embodiment, a nucleic acid of the invention is expressed in mammalian cells using a mammalian expression vector. Examples of mammalian expression vectors include pCDM8 (Seed, B. (1987) *Nature* 329:840) and pMT2PC (Kaufman *et al.* (1987) *EMBO J.* 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook, J., Fritsh, E. F., and Maniatis, T. *Molecular Cloning: A Laboratory Manual. 2nd, ed., Cold Spring Harbor Laboratory,* Cold Spring Harbor Laboratory Press, Cold Spring Harbor, NY, 1989.

In another embodiment, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert *et al.* (1987) *Genes Dev.* 1:268-277), lymphoid-specific promoters (Calame and Eaton (1988) *Adv. Immunol.* 43:235-275), in particular promoters of T cell receptors (Winoto and Baltimore (1989) *EMBO J.* 8:729-733) and immunoglobulins (Banerji *et al.* (1983) *Cell* 33:729-740; Queen and Baltimore (1983) *Cell* 33:741-748), neuron-specific promoters *(e.g.,* the neurofilament promoter; Byrne and Ruddle (1989) *PNAS* 86:5473-5477), pancreas-specific promoters (Edlund *et al.* (1985) *Science* 230:912-916), and mammary gland-specific promoters (e.g., milk whey promoter; U.S. Patent No. 4,873,316 and European Application Publication No. 264,166). Developmentally-regulated promoters are also encompassed, for example the murine hox promoters (Kessel and Gruss (1990) *Science* 249:374-379) and the α-fetoprotein promoter (Campes and Tilghman (1989) *Genes Dev.* 3:537-546).

The invention further provides a recombinant expression vector comprising a DNA molecule of the invention cloned into the expression vector in an antisense orientation. That is, the DNA molecule is operatively linked to a regulatory sequence in a manner which allows for expression (by transcription of the DNA molecule) of an RNA molecule which is antisense to HA mRNA. Regulatory sequences operatively linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see Weintraub, *H. et al.,* Antisense RNA as a molecular tool for genetic analysis, *Reviews - Trends in Genetics,* Vol. 1(1) (1986).

Another aspect of the invention pertains to host cells into which a recombinant expression vector of the invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, an HA protein can be expressed in bacterial cells such as *C*. *glutamicum,* insect cells, yeast or mammalian cells (such as Chinese hamster ovary cells (CHO) or COS cells). Other suitable host cells are known to those of ordinary skill in the art. Microorganisms related to *Corynebacterium glutamicum* which may be conveniently used as host cells for the nucleic acid and protein molecules of the invention are set forth in Table 3.

Vector DNA can be introduced into prokaryotic or eukaryotic cells via conventional transformation or transfection techniques. As used herein, the terms "transformation" and "transfection", "conjugation" and "transduction" are intended to refer to a variety of art-recognized techniques for introducing foreign nucleic acid *(e.g.,* linear DNA or RNA (e.g., a linearized vector or a gene construct alone without a vector) or nucleic acid in the form of a vector (e.g., a plasmid, phage, phasmid, phagemid, transposon or other DNA) into a host cell, including calcium phosphate or calcium chloride co-precipitation, DEAE-dextran-mediated transfection, lipofection, natural competence, chemical-mediated transfer, or electroporation. Suitable methods for transforming or transfecting host cells can be found in Sambrook, *et al. (Molecular Cloning: A Laboratory Manual. 2nd, ed., Cold Spring Harbor Laboratory,* Cold Spring Harbor Laboratory Press, Cold Spring Harbor, NY, 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same vector as that encoding an HA protein or can be introduced on a separate vector. Cells stably transfected with the introduced nucleic acid can be identified by, for example, drug selection (*e.g.,* cells that have incorporated the selectable marker gene will survive, while the other cells die).

To create a homologous recombinant microorganism, a vector is prepared which contains at least a portion of an HA gene into which a deletion, addition or substitution has been introduced to thereby alter, e.g., functionally disrupt, the HA gene. Preferably, this HA gene is a *Corynebacterium glutamicum* HA gene, but it can be a homologue from a related bacterium or even from a mammalian, yeast, or insect source. In a preferred embodiment, the vector is designed such that, upon homologous recombination, the endogenous HA gene is functionally disrupted (i.e., no longer encodes a functional protein; also referred to as a "knock out" vector). Alternatively, the vector can be designed such that, upon homologous recombination, the endogenous HA gene is mutated or otherwise altered but still encodes functional protein (e.g., the upstream regulatory region can be altered to thereby alter the expression of the endogenous HA protein). In the homologous recombination vector, the altered portion of the HA gene is flanked at its 5' and 3' ends by additional nucleic acid of the HA gene to allow for homologous recombination to occur between the exogenous HA gene carried by the vector and an endogenous HA gene in a microorganism. The additional flanking HA nucleic acid is of sufficient length for successful homologous recombination with the endogenous gene. Typically, several kilobases of flanking DNA (both at the 5' and 3' ends) are included in the vector (see e.g., Thomas, K.R., and Capecchi, M.R. (1987) Cell 51: 503 for a description of homologous recombination vectors). The vector is introduced into a microorganism (e.g., by electroporation) and cells in which the introduced HA gene has homologously recombined with the endogenous HA gene are selected, using art-known techniques.

In another embodiment, recombinant microorganisms can be produced which contain selected systems which allow for regulated expression of the introduced gene. For example, inclusion of an HA gene on a vector placing it under control of the lac operon permits expression of the HA gene only in the presence of IPTG. Such regulatory systems are well known in the art.

In another embodiment, an endogenous HA gene in a host cell is disrupted (e.g., by homologous recombination or other genetic means known in the art) such that expression of its protein product does not occur. In another embodiment, an endogenous or introduced HA gene in a host cell has been altered by one or more point mutations, deletions, or inversions, but still encodes a functional HA protein. In still another embodiment, one or more of the regulatory regions (e.g., a promoter, repressor, or inducer) of an HA gene in a microorganism has been altered (e.g., by deletion, truncation, inversion, or point mutation) such that the expression of the HA gene is modulated. One of ordinary skill in the art will appreciate that host cells containing more than one of the described HA gene and protein modifications may be readily produced using the methods of the invention, and are meant to be included in the present invention.

A host cell of the invention, such as a prokaryotic or eukaryotic host cell in culture, can be used to produce *(i.e.,* express) an HA protein. Accordingly, the invention further provides methods for producing HA proteins using the host cells of the invention. In one embodiment, the method comprises culturing the host cell of invention (into which a recombinant expression vector encoding an HA protein has been introduced, or into which genome has been introduced a gene encoding a wild-type or altered HA protein) in a suitable medium until HA protein is produced. In another embodiment, the method further comprises isolating HA proteins from the medium or the host cell.

### C. Isolated HA Proteins

Another aspect of the invention pertains to isolated HA proteins, and biologically active portions thereof. An "isolated" or "purified" protein or biologically active portion thereof is substantially free of cellular material when produced by recombinant DNA techniques, or chemical precursors or other chemicals when chemically synthesized. The language "substantially free of cellular material" includes preparations of HA protein in which the protein is separated from cellular components of the cells in which it is naturally or recombinantly produced. In one embodiment, the language "substantially free of cellular material" includes preparations of HA protein having less than about 30% (by dry weight) of non-HA protein (also referred to herein as a "contaminating protein"), more preferably less than about 20% of non-HA protein, still more preferably less than about 10% of non-HA protein, and most preferably less than about 5% non-HA protein. When the HA protein or biologically active portion thereof is recombinantly produced, it is also preferably substantially free of culture medium, i.e., culture medium represents less than about 20%, more preferably less than about 10%, and most preferably less than about 5% of the volume of the protein preparation. The language "substantially free of chemical precursors or other chemicals" includes preparations of HA protein in which the protein is separated from chemical precursors or other chemicals which are involved in the synthesis of the protein. In one embodiment, the language "substantially free of chemical precursors or other chemicals" includes preparations of HA protein having less than about 30% (by dry weight) of chemical precursors or non-HA chemicals, more preferably less than about 20% chemical precursors or non-HA chemicals, still more preferably less than about 10% chemical precursors or non-HA chemicals, and most preferably less than about 5% chemical precursors or non-HA chemicals. In preferred embodiments, isolated proteins or biologically active portions thereof lack contaminating proteins from the same organism from which the HA protein is derived. Typically, such proteins are produced by recombinant expression of, for example, a *C*. *glutamicum* HA protein in a microorganism such as *C. glutamicum.*

An isolated HA protein or a portion thereof of the invention can participate in the repair or recombination of DNA, in the transposition of genetic material, in gene expression *(i.e.,* the processes of transcription or translation), in protein folding, or in protein secretion in *Corynebacterium glutamicum,* or has one or more of the activities set forth in Table 1. In preferred embodiments, the protein or portion thereof comprises an amino acid sequence which is sufficiently homologous to an amino acid sequence of the invention *(e.g.,* a sequence of an even-numbered SEQ ID NO: of the Sequence Listing) such that the protein or portion thereof maintains the ability to participate in the maintenance of homeostasis in *C*. *glutamicum,* or to perform a function involved in the adaptation of this microorganism to different environmental conditions. The portion of the protein is preferably a biologically active portion as described herein. In another preferred embodiment, an HA protein of the invention has an amino acid sequence set forth as an even-numbered SEQ ID NO: of the Sequence Listing. In yet another preferred embodiment, the HA protein has an amino acid sequence which is encoded by a nucleotide sequence which hybridizes, e.g., hybridizes under stringent conditions, to a nucleotide sequence the invention (e.g., a sequence of an odd-numbered SEQ ID NO: of the Sequence Listing). In still another preferred embodiment, the HA protein has an amino acid sequence which is encoded by a nucleotide sequence that is at least about 50%, 51%, 52%, 53%, 54%, 55%, 56%, 57%, 58%, 59%, or 60%, preferably at least about 61%, 62%, 63%, 64%, 65%, 66%, 67%, 68%, 69%, or 70%, more preferably at least about 71%, 72%, 73%, 74%, 75%, 76%, 77%, 78%, 79%, or 80%, 81%, 82%, 83%, 84%, 85%, 86%, 87%, 88%, 89%, or 90%, or 91%, 92%, 93%, 94%, and even more preferably at least about 95%, 96%, 97%, 98%, 99% % or more homologous to one of the nucleic acid sequences of the invention, or a portion thereof. Ranges and identity values intermediate to the above-recited values, (e.g., 70-90% identical or 80-95% identical) are also intended to be encompassed by the present invention. For example, ranges of identity values using a combination of any of the above values recited as upper and/or lower limits are intended to be included. The preferred HA proteins of the present invention also preferably possess at least one of the HA activities described herein. For example, a preferred HA protein of the present invention includes an amino acid sequence encoded by a nucleotide sequence which hybridizes, e.g., hybridizes under stringent conditions, to a nucleotide sequence of the invention, and which can participate in the maintenance of homeostasis in C. *glutamicum,* or can perform a function involved in the adaptation of this microorganism to different environmental conditions, or which has one or more of the activities set forth in Table 1.

In other embodiments, the HA protein is substantially homologous to an amino acid sequence of the invention (e.g., a sequence of an even-numbered SEQ ID NO: of the Sequence Listing) and retains the functional activity of the protein of one of the amino acid sequences of the invention yet differs in amino acid sequence due to natural variation or mutagenesis, as described in detail in subsection I above. Accordingly, in another embodiment, the HA protein is a protein which comprises an amino acid sequence which is at least about 50%, 51%, 52%, 53%, 54%, 55%, 56%, 57%, 58%, 59%, or 60%, preferably at least about 61%, 62%, 63%, 64%, 65%, 66%, 67%, 68%, 69%, or 70%, more preferably at least about 71%, 72%, 73%, 74%, 75%, 76%, 77%, 78%, 79%, or 80%, 81%, 82%, 83%, 84%, 85%, 86%, 87%, 88%, 89%, or 90%, or 91%, 92%, 93%, 94%, and even more preferably at least about 95%, 96%, 97%, 98%, 99% or more homologous to an entire amino acid sequence of the invention and which has at least one of the HA activities described herein. Ranges and identity values intermediate to the above-recited values, (e.g., 70-90% identical or 80-95% identical) are also intended to be encompassed by the present invention. For example, ranges of identity values using a combination of any of the above values recited as upper and/or lower limits are intended to be included. In another embodiment, the invention pertains to a full length *C*. *glutamicum* protein which is substantially homologous to an entire amino acid sequence of the invention.

Biologically active portions of an HA protein include peptides comprising amino acid sequences derived from the amino acid sequence of an HA protein, e.g., the amino acid sequence of an even-numbered SEQ ID NO: of the Sequence Listing, the amino acid sequence of a protein homologous to an HA protein, which include fewer amino acids than a full length HA protein or the full length protein which is homologous to an HA protein, and exhibit at least one activity of an HA protein. Typically, biologically active portions (peptides, e.g., peptides which are, for example, 5, 10, 15, 20, 30, 35, 36, 37, 38, 39, 40, 50, 100 or more amino acids in length) comprise a domain or motif with at least one activity of an HA protein. Moreover, other biologically active portions, in which other regions of the protein are deleted, can be prepared by recombinant techniques and evaluated for one or more of the activities described herein. Preferably, the biologically active portions of an HA protein include one or more selected domains/motifs or portions thereof having biological activity.

HA proteins are preferably produced by recombinant DNA techniques. For example, a nucleic acid molecule encoding the protein is cloned into an expression vector (as described above), the expression vector is introduced into a host cell (as described above) and the HA protein is expressed in the host cell. The HA protein can then be isolated from the cells by an appropriate purification scheme using standard protein purification techniques. Alternative to recombinant expression, an HA protein, polypeptide, or peptide can be synthesized chemically using standard peptide synthesis techniques. Moreover, native HA protein can be isolated from cells (e.g., endothelial cells), for example using an anti-HA antibody, which can be produced by standard techniques utilizing an HA protein or fragment thereof of this invention.

The invention also provides HA chimeric or fusion proteins. As used herein, an HA "chimeric protein" or "fusion protein" comprises an HA polypeptide operatively linked to a non-HA polypeptide. An "HA polypeptide" refers to a polypeptide having an amino acid sequence corresponding to an HA protein, whereas a "non-HA polypeptide" refers to a polypeptide having an amino acid sequence corresponding to a protein which is not substantially homologous to the HA protein, e.g., a protein which is different from the HA protein and which is derived from the same or a different organism. Within the fusion protein, the term "operatively linked" is intended to indicate that the HA polypeptide and the non-HA polypeptide are fused in-frame to each other. The non-HA polypeptide can be fused to the N-terminus or C-terminus of the HA polypeptide. For example, in one embodiment the fusion protein is a GST-HA fusion protein in which the HA sequences are fused to the C-terminus of the GST sequences. Such fusion proteins can facilitate the purification of recombinant HA proteins. In another embodiment, the fusion protein is an HA protein containing a heterologous signal sequence at its N-terminus. In certain host cells (e.g., mammalian host cells), expression and/or secretion of an HA protein can be increased through use of a heterologous signal sequence.

Preferably, an HA chimeric or fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, *Current Protocols in Molecular Biology,* eds. Ausubel *et al.* John Wiley & Sons: 1992). Moreover, many expression vectors are commercially available that already encode a fusion moiety (e.g., a GST polypeptide). An HA-encoding nucleic acid can be cloned into such an expression vector such that the fusion moiety is linked in-frame to the HA protein.

Homologues of the HA protein can be generated by mutagenesis, e.g., discrete point mutation or truncation of the HA protein. As used herein, the term "homologue" refers to a variant form of the HA protein which acts as an agonist or antagonist of the activity of the HA protein. An agonist of the HA protein can retain substantially the same, or a subset, of the biological activities of the HA protein. An antagonist of the HA protein can inhibit one or more of the activities of the naturally occurring form of the HA protein, by, for example, competitively binding to a downstream or upstream member of a biochemical cascade which includes the HA protein, by binding to a target molecule with which the HA protein interacts, such that no functional interaction is possible, or by binding directly to the HA protein and inhibiting its normal activity.

In an alternative embodiment, homologues of the HA protein can be identified by screening combinatorial libraries of mutants, e.g., truncation mutants, of the HA protein for HA protein agonist or antagonist activity. In one embodiment, a variegated library of HA variants is generated by combinatorial mutagenesis at the nucleic acid level and is encoded by a variegated gene library. A variegated library of HA variants can be produced by, for example, enzymatically ligating a mixture of synthetic oligonucleotides into gene sequences such that a degenerate set of potential HA sequences is expressible as individual polypeptides, or alternatively, as a set of larger fusion proteins (e.g., for phage display) containing the set of HA sequences therein. There are a variety of methods which can be used to produce libraries of potential HA homologues from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential HA sequences. Methods for synthesizing degenerate oligonucleotides are known in the art (see, e.g., Narang, S.A. (1983) *Tetrahedron 39:3;* Itakura *et al.* (1984) *Annu. Rev. Biochem.* 53:323; Itakura *et al.* (1984) *Science* 198:1056; Ike *et al.* (1983) *Nucleic Acid Res. 11:477.*

In addition, libraries of fragments of the HA protein coding can be used to generate a variegated population of HA fragments for screening and subsequent selection of homologues of an HA protein. In one embodiment, a library of coding sequence fragments can be generated by treating a double stranded PCR fragment of an HA coding sequence with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S 1 nuclease, and ligating the resulting fragment library into an expression vector. By this method, an expression library can be derived which encodes N-terminal, C-terminal and internal fragments of various sizes of the HA protein.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of HA homologues. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique which enhances the frequency of functional mutants in the libraries, can be used in combination with the screening assays to identify HA homologues (Arkin and Yourvan (1992) *PNAS* 89:7811-7815; Delgrave *et al.* (1993) *Protein Engineering* 6(3):327-331).

In another embodiment, cell based assays can be exploited to analyze a variegated HA library, using methods well known in the art.

### D. Uses and Methods of the Invention

The nucleic acid molecules, proteins, protein homologues, fusion proteins, primers, vectors, and host cells described herein can be used in one or more of the following methods: identification of *C*. *glutamicum* and related organisms; mapping of genomes of organisms related to *C*. *glutamicum;* identification and localization of *C. glutamicum* sequences of interest; evolutionary studies; determination of HA protein regions required for function; modulation of an HA protein activity; modulation of the metabolism of one or more inorganic compounds; modulation of the modification or degradation of one or more aromatic or aliphatic compounds; modulation of cell wall synthesis or rearrangements; modulation of enzyme activity or proteolysis; and modulation of cellular production of a desired compound, such as a fine chemical.

The HA nucleic acid molecules of the invention have a variety of uses. First, they may be used to identify an organism as being *Corynebacterium glutamicum* or a close relative thereof. Also, they may be used to identify the presence of *C*. *glutamicum* or a relative thereof in a mixed population of microorganisms. The invention provides the nucleic acid sequences of a number of *C*. *glutamicum* genes; by probing the extracted genomic DNA of a culture of a unique or mixed population of microorganisms under stringent conditions with a probe spanning a region of a *C*. *glutamicum* gene which is unique to this organism, one can ascertain whether this organism is present. Although *Corynebacterium glutamicum* itself is nonpathogenic, it is related to pathogenic species, such as *Corynebacterium diphtheriae. Corynebacterium diphtheriae* is the causative agent of diphtheria, a rapidly developing, acute, febrile infection which involves both local and systemic pathology. In this disease, a local lesion develops in the upper respiratory tract and involves necrotic injury to epithelial cells; the bacilli secrete toxin which is disseminated through this lesion to distal susceptible tissues of the body. Degenerative changes brought about by the inhibition of protein synthesis in these tissues, which include heart, muscle, peripheral nerves, adrenals, kidneys, liver and spleen, result in the systemic pathology of the disease. Diphtheria continues to have high incidence in many parts of the world, including Africa, Asia, Eastern Europe and the independent states of the former Soviet Union. An ongoing epidemic of diphtheria in the latter two regions has resulted in at least 5,000 deaths since 1990.

In one embodiment, the invention provides a method of identifying the presence or activity of *Cornyebacterium diphtheriae* in a subject. This method includes detection of one or more of the nucleic acid or amino acid sequences of the invention (e.g., the sequences set forth in as odd-numbered or even-numbered SEQ ID NOs, respectively, in the Sequence Listing) in a subject, thereby detecting the presence or activity of *Corynebacterium diphtheriae* in the subject. *C. glutamicum* and *C*. *diphtheriae* are related bacteria, and many of the nucleic acid and protein molecules in *C*. *glutamicum* are homologous to *C*. *diphtheriae* nucleic acid and protein molecules, and can therefore be used to detect *C*. *diphtheriae* in a subject.

The nucleic acid and protein molecules of the invention may also serve as markers for specific regions of the genome. This has utility not only in the mapping of the genome, but also for functional studies of *C. glutamicum* proteins. For example, to identify the region of the genome to which a particular *C*. *glulamicum* DNA-binding protein binds, the *C glutamicum* genome could be digested, and the fragments incubated with the DNA-binding protein. Those which bind the protein may be additionally probed with the nucleic acid molecules of the invention, preferably with readily detectable labels; binding of such a nucleic acid molecule to the genome fragment enables the localization of the fragment to the genome map of *C. glutamicum,* and, when performed multiple times with different enzymes, facilitates a rapid determination of the nucleic acid sequence to which the protein binds. Further, the nucleic acid molecules of the invention may be sufficiently homologous to the sequences of related species such that these nucleic acid molecules may serve as markers for the construction of a genomic map in related bacteria, such as *Brevibacterium lactofermentum.*

The HA nucleic acid molecules of the invention are also useful for evolutionary and protein structural studies. The processes involved in adaptation and the maintenance of homeostasis in which the molecules of the invention participate are utilized by a wide variety of species; by comparing the sequences of the nucleic acid molecules of the present invention to those encoding similar enzymes from other organisms, the evolutionary relatedness of the organisms can be assessed. Similarly, such a comparison permits an assessment of which regions of the sequence are conserved and which are not, which may aid in determining those regions of the protein which are essential for the functioning of the enzyme. This type of determination is of value for protein engineering studies and may give an indication of what the protein can tolerate in terms of mutagenesis without losing function.

Manipulation of the HA nucleic acid molecules of the invention may result in the production of HA proteins having functional differences from the wild-type HA proteins. These proteins may be improved in efficiency or activity, may be present in greater numbers in the cell than is usual, or may be decreased in efficiency or activity.

The invention provides methods for screening molecules which modulate the activity of an HA protein, either by interacting with the protein itself or a substrate or binding partner of the HA protein, or by modulating the transcription or translation of an HA nucleic acid molecule of the invention. In such methods, a microorganism expressing one or more HA proteins of the invention is contacted with one or more test compounds, and the effect of each test compound on the activity or level of expression of the HA protein is assessed.

The modulation of activity or number of HA proteins involved in cell wall biosynthesis or rearrangements may impact the production, yield, and/or efficiency of production of one or more fine chemicals from *C*. *glutamicum* cells. For example, by altering the activity of these proteins, it may be possible to modulate the structure or thickness of the cell wall. The cell wall serves in large measure as a protective device against osmotic lysis and external sources of injury; by modifying the cell wall it may be possible to increase the ability of *C*. *glutamicum* to withstand the mechanical and shear force stresses encountered by this microorganism during large-scale fermentor culture. Further, each *C. glutamicum* cell is surrounded by a thick cell wall, and thus, a significant portion of the biomass present in large scale culture consists of cell wall. By increasing the rate at which the cell wall is synthesized or by activating cell wall synthesis (through genetic engineering of the HA cell wall proteins of the invention) it may be possible to improve the growth rate of the microorganism. Similarly, by decreasing the activity or number of proteins involved in the degradation of cell wall or by decreasing the repression of cell wall biosynthesis, an overall increase in cell wall production may be achieved. An increase in the number of viable *C*. *glutamicum* cells (as may be accomplished by any of the foregoing described protein alterations) should result in increased numbers of cells producing the desired fine chemical in large-scale fermentor culture, which should permit increased yields or efficiency of production of these compounds from the culture.

The modulation of activity or number of *C*. *glutamicum* HA proteins that participate in the modification or degradation of aromatic or aliphatic compounds may also have direct or indirect impacts on the production of one or more fine chemicals from these cells. Certain aromatic or aliphatic modification or degradation products are desirable fine chemicals (e.g., organic acids or modified aromatic and aliphatic compounds); thus, by modifying the enzymes which perform these modifications (e.g., hydroxylation, methylation, or isomerization) or degradation reactions, it may be possible to increase the yields of these desired compounds. Similarly, by decreasing the activity or number of proteins involved in pathways which further degrade the modified or breakdown products of the aforementioned reactions it may be possible to improve the yields of these fine chemicals from *C*. *glutamicum* cells in culture.

These aromatic and aliphatic modification and degradative enzymes are themselves fine chemicals. In purified form, these enzymes may be used to degrade aromatic and aliphatic compounds (e.g., toxic chemicals such as petroleum products), either for the bioremediation of polluted sites, for the engineered decomposition of wastes, or for the large-scale and economically feasible production of desired modified aromatic or aliphatic compounds or their breakdown products, some of which may be conveniently used as carbon or energy sources for other fine chemical-producing compounds in culture (see, e.g., Faber, K. (1995) Biotransformations in Organic Chemistry, Springer: Berlin and references therein; and Roberts, S.M., ed. (1992-1996) Preparative Biotransformations, Wiley: Chichester, and references therein). By genetically altering these proteins such that their regulation by other cellular mechanisms is lessened or abolished, it may be possible to increase the overall number or activity of these proteins, thereby improving not only the yield of these fine chemicals but also the activity of these harvested proteins.

The modification of these aromatic and aliphatic modifying and degradation enzymes may also have an indirect effect on the production of one or more fine chemical. Many aromatic and aliphatic compounds (such as those that may be encountered as impurities in culture media or as waste products from cellular metabolism) are toxic to cells; by modifying and/or degrading these compounds such that they may be readily removed or destroyed, cellular viability should be increased. Further, these enzymes may modify or degrade these compounds in such a manner that the resulting products may enter the normal carbon metabolism pathways of the cell, thus rendering the cell able to use these compounds as alternate carbon or energy sources. In large-scale culture situations, when there may be limiting amounts of optimal carbon sources, these enzymes provide a method by which cells may continue to grow and divide using aromatic or aliphatic compounds as nutrients. In either case, the resulting increase in the number of *C*. *glutamicum* cells in the culture producing the desired fine chemical should in turn result in increased yields or efficiency of production of the fine chemical(s).

Modifications in activity or number of HA proteins involved in the metabolism of inorganic compounds may also directly or indirectly affect the production of one or more fine chemicals from *C. glutamicum* or related bacterial cultures. For example, many desirable fine chemicals, such as nucleic acids, amino acids, cofactors and vitamins (e.g., thiamine, biotin, and lipoic acid) cannot be synthesized without inorganic molecules such as phosphorous, nitrate, sulfate, and iron. The inorganic metabolism proteins of the invention permit the cell to obtain these molecules from a variety of inorganic compounds and to divert them into various fine chemical biosynthetic pathways. Therefore, by increasing the activity or number of enzymes involved in the metabolism of these inorganic compounds, it may be possible to increase the supply of these possibly limiting inorganic molecules, thereby directly increasing the production or efficiency of production of various fine chemicals from *C*. *glutamicum* cells containing such altered proteins. Modification of the activity or number of inorganic metabolism enzymes of the invention may also render *C*. *glutamicum* able to better utilize limited inorganic compound supplies, or to utilize nonoptimal inorganic compounds to synthesize amino acids, vitamins, cofactors, or nucleic acids, all of which are necessary for continued growth and replication of the cell. By improving the viability of these cells in large-scale culture, the number of *C*. *glutamicum* cells producing one or more fine chemicals in the culture may also be increased, in turn increasing the yields or efficiency of production of one or more fine chemicals.

*C. glutamicum* enzymes for general processes are themselves desirable fine chemicals. The specific properties of enzymes *(i.e.,* regio- and stereospecificity, among others) make them useful catalysts for chemical reactions *in vitro.* Either whole *C*. *glutamicum* cells may be incubated with an appropriate substrate such that the desired product is produced by enzymes in the cell, or the desired enzymes may be overproduced and purified from *C*. *glutamicum* cultures (or those of a related bacterium) and subsequently utilized in *in vitro* reactions in an industrial setting (either in solution or immobilized on a suitable immobile phase). In either situation, the enzyme can either be a natural *C*. *glutamicum* protein, or it may be mutagenized to have an altered activity; typical industrial uses for such enzymes include as catalysts in the chemical industry (e.g., for synthetic organic chemistry) as food additives, as feed components, for fruit processing, for leather preparation, in detergents, in analysis and medicine, and in the textile industry (see, e.g.,Yamada, H. (1993) "Microbial reactions for the production of useful organic compounds," *Chimica* 47: 5-10; Roberts, S.M. (1998) Preparative biotransformations: the employment of enzymes and whole-cells in synthetic chemistry," *J. Chem. Soc. Perkin Trans. 1:* 157-169; Zaks, A. and Dodds, D.R. (1997) "Application of biocatalysis and biotransformations to the synthesis of pharmaceuticals," *DDT 2:* 513-531; Roberts, S.M. and Williamson, N.M. (1997) "The use of enzymes for the preparation of biologically active natural products and analogues in optically active form," *Curr. Organ. Chemistry 1:* 1-20; Faber, K. (1995) Biotransformations in Organic Chemistry, Springer: Berlin; Roberts, S.M., ed. (1992-96) Preparative Biotransformations, Wiley: Chichester; Cheetham, P.S.J. (1995) "The applications of enzymes in industry" in : Handbook of Enzyme Biotechnology, 3^{rd} ed., Wiseman, A., ed., Elis: Horwood, p. 419-552; and Ullmann's Encyclopedia of Industrial Chemistry (1987), vol. A9, Enzymes, p. 390-457. Thus, by increasing the activity or number of these enzymes, it may be possible to also increase the ability of the cell to convert supplied substrates to desired products, or to overproduce these enzymes for increased yields in large-scale culture. Further, by mutagenizing these proteins it may be possible to remove feedback inhibition or other repressive cellular regulatory controls such that greater numbers of these enzymes may be produced and activated by the cell, thereby leading to greater yields, production, or efficiency of production of these fine chemical proteins from large-scale cultures. Further, manipulation of these enzymes may alter the activity of one or more *C*. *glutamicum* metabolic pathways, such as those for the biosynthesis or secretion of one or more fine chemicals.

Mutagenesis of the proteolytic enzymes of the invention such that they are altered in activity or number may also directly or indirectly affect the yield, production, and/or efficiency of production of one or more fine chemicals from *C. glutamicum.* For example, by increasing the activity or number of these proteins, it may be possible to increase the ability of the bacterium to survive in large-scale culture, due to an increased ability of the cell to rapidly degrade proteins misfolded in response to the high temperatures, nonoptimal pH, and other stresses encountered during fermentor culture. Increased numbers of cells in these cultures may result in increased yields or efficiency of production of one or more desired fine chemicals, due to the relatively larger number of cells producing these compounds in the culture. Also, *C*. *glutamicum* cells possess multiple cell-surface proteases which serve to break down external nutrients into molecules which may be more readily incorporated by the cells as carbon/energy sources or nutrients of other kinds. An increase in activity or number of these enzymes may improve this turnover and increase the levels of available nutrients, thereby improving cell growth or production. Thus, modifications of the proteases of the invention may indirectly impact *C*. *glutamicum* fine chemical production.

A more direct impact on fine chemical production in response to the modification of one or more of the proteases of the invention may occur when these proteases are involved in the production or degradation of a desired fine chemical. By decreasing the activity of a protease which degrades a fine chemical or a protein involved in the synthesis of a fine chemical it may be possible to increase the levels of that fine chemical (due to the decreased degradation or increased synthesis of the compound). Similarly, by increasing the activity of a protease which degrades a compound to result in a fine chemical or a protein involved in the degradation of a fine chemical, a similar result should be achieved: increased levels of the desired fine chemical from *C*. *glutamicum* cells containing these engineered proteins.

The aforementioned mutagenesis strategies for HA proteins to result in increased yields of a fine chemical from *C*. *glutamicum* are not meant to be limiting; variations on these strategies will be readily apparent to one of ordinary skill in the art. Using such strategies, and incorporating the mechanisms disclosed herein, the nucleic acid and protein molecules of the invention may be utilized to generate *C*. *glutamicum* or related strains of bacteria expressing mutated HA nucleic acid and protein molecules such that the yield, production, and/or efficiency of production of a desired compound is improved. This desired compound may be any product produced by *C*. *glutamicum,* which includes the final products of biosynthesis pathways and intermediates of naturally-occurring metabolic pathways, as well as molecules which do not naturally occur in the metabolism of *C*. *glutamicum,* but which are produced by a *C*. *glutamicum* strain of the invention.

This invention is further illustrated by the following examples which should not be construed as limiting. The contents of all references, patent applications, patents, published patent applications, Tables, and the sequence listing cited throughout this application are hereby incorporated by reference.

**Table 1: Genes in the Application**

| Nucleic Acid SEQ ID NO | Amino Acid SEQ ID NO | Identification Code | Contig. | NT Start | NT Stop | Function |
|---|---|---|---|---|---|---|
| 1 | 2 | RXA02548 | GR00727 | 3 | 293 | SULFATE ADENYLATE TRANSFERASE SUBUNIT 2 (EC 2.7.7.4) |
| 3 | 4 | RXN00249 | W0057 | 36825 | 35869 | ADENYLYLSULFATE KINASE (EC 2.7.1.25) |
| 5 | 6 | F RXA00249 | GR00037 | 8837 | 7884 | ADENYLYLSULFATE KINASE (EC 2.7.1.25) |
| 7 | 8 | RXA01073 | GR00300 | 1274 | 2104 | NH(3)-DEPENDENT NAD(+) SYNTHETASE (EC 6.3.5.1) |

| **Urease** | | | | | | |
|---|---|---|---|---|---|---|
| Nucleic Acid SEQ ID NO | Amino Acid SEQ ID NO | Identification Code | Contig. | NT Start | NT Stop | Function |
| 9 | 10 | RXN02913 | W0020 | 8998 | 8513 | UREASE BETA SUBUNIT (EC 3.5.1.5) |
| 11 | 12 | F RXA02264 | GR00655 | 123 | 4 | UREASE ALPHA SUBUNIT (EC 3.5.1.5) |
| 13 | 14 | RXN02274 | W0020 | 8509 | 6800 | UREASE ALPHA SUBUNIT (EC 3.5.1.5) |
| 15 | 16 | F RXA02274 | GR00656 | 3 | 1604 | UREASE ALPHA SUBUNIT (EC 3.5.1.5) |
| 17 | 18 | RXA02265 | GR00655 | 452 | 153 | UREASE GAMMA SUBUNIT (EC 3.5.1.5) |
| 19 | 20 | RXA02278 | GR00656 | 3420 | 4268 | UREASE OPERON URED PROTEIN |
| 21 | 22 | RXA02275 | GR00656 | 1632 | 2102 | UREASE ACCESSORY PROTEIN UREE |
| 23 | 24 | RXA02276 | GR00656 | 2105 | 2782 | UREASE ACCESSORY PROTEIN UREF |
| 25 | 26 | RXA02277 | GR00656 | 2802 | 3416 | UREASE ACCESSORY PROTEIN UREG |
| 27 | 28 | RXA02603 | GR00742 | 7742 | 8737 | 4-HYDROXYBENZOATE OCTAPRENYLTRANSFERASE (EC 2.5.1.-) |
| 29 | 30 | RXA01385 | GR00406 | 5320 | 3440 | PHENOL 2 MONOOXYGENASE (EC 1.14.13.7) |

| **Proteolysis** | | | | | | |
|---|---|---|---|---|---|---|
| Nucleic Acid SEQ ID NO | Amino Acid SEQ ID NO | Identification Code | Contig. | NT Start | NT Stop | Function |
| 31 | 32 | RXN00675 | W0005 | 33258 | 34049 | METHIONINE AMINOPEPTIDASE (EC 3.4.11.18) |
| 33 | 34 | F RXA00675 | GR00178 | 2 | 484 | METHIONINE AMINOPEPTIDASE (EC 3.4.11.18) |
| 35 | 36 | RXA01609 | GR00449 | 2740 | 3612 | METHIONINE AMINOPEPTIDASE (EC 3.4.11.18) |
| 37 | 38 | RXA01358 | GR00393 | 5337 | 6857 | ATP-DEPENDENT PROTEASE LA (EC 3.4.21.53) |
| 39 | 40 | RXA01458 | GR00420 | 3225 | 2176 | ATP-DEPENDENT PROTEASE LA (EC 3.4.21.53) |
| 41 | 42 | RXA01654 | GR00459 | 986 | 1981 | (AL022121) putative alkaline serine protease [Mycobacterium tuberculosis] |
| 43 | 44 | RXN01868 | W0127 | 9980 | 11905 | ZINC METALLOPROTEASE (EC 3.4.24.-) |
| 45 | 46 | F RXA01868 | GR00534 | 1640 | 30 | ZINC METALLOPROTEASE (EC 3.4.24.-) |
| 47 | 48 | F RXA01869 | GR00534 | 1954 | 1652 | ZINC METALLOPROTEASE (EC 3.4.24.-) |
| 49 | 50 | RXN03028 | W0008 | 41156 | 43930 | ATP-DEPENDENT CLP PROTEASE ATP-BINDING SUBUNIT CLPA |
| 51 | 52 | F RXA02470 | GR00715 | 2216 | 3196 | ATP-DEPENDENT CLP PROTEASE ATP-BINDING SUBUNIT CLPA |
| 53 | 54 | F RXA02471 | GR00715 | 3159 | 4991 | ATP-DEPENDENT CLP PROTEASE ATP-BINDING SUBUNIT CLPA |
| 55 | 56 | RXA02630 | GR00748 | 2654 | 1332 | (AL021999) putative serine protease [Mycobacterium tuberculosis] |
| 57 | 58 | RXA02834 | GR00823 | 3 | 497 | ATPases with chaperone activity, ATP-dependent protease subunit |
| 59 | 60 | RXA00112 | GR00016 | 3687 | 2497 | PROBABLE PERIPLASMIC SERINE PROTEASE DO-LIKE PRECURSOR |
| 61 | 62 | RXA00566 | GR00152 | 742 | 137 | ATP-DEPENDENT CLP PROTEASE PROTEOLYTIC SUBUNIT (EC 3.4.21.92) |
| 63 | 64 | RXA00567 | GR00152 | 1388 | 798 | ATP-DEPENDENT CLP PROTEASE PROTEOLYTIC SUBUNIT (EC 3.4.21.92) |
| 65 | 66 | RXN03094 | W0057 | 1794 | 43 | CLPB PROTEIN |
| 67 | 68 | FRXA01668 | GR00464 | 2205 | 3920 | CLPB PROTEIN |
| 69 | 70 | RXN01120 | W0182 | 5678 | 4401 | ATP-DEPENDENT CLP PROTEASE ATP-BINDING SUBUNIT CLPX |
| 71 | 72 | F RXA01120 | GR00310 | 2349 | 1072 | ATP-DEPENDENT CLP PROTEASE ATP-BINDING SUBUNIT CLPX |
| 73 | 74 | RXA00744 | GR00202 | 10722 | 9781 | Periplasmic serine proteases |
| 75 | 76 | RXA00844 | GR00228 | 3620 | 4453 | Hypothetical Secretory Serine Protease (EC 3.4.21.-) |
| 77 | 78 | RXA01151 | GR00324 | 862 | 5 | ATP-dependent Zn proteases |
| 79 | 80 | RXA02317 | GR00665 | 9664 | 9053 | PEPTIDASE E (EC 3.4.-.-) |
| 81 | 82 | RXA02644 | GR00751 | 767 | 117 | XAA-PRO DIPEPTIDASE (EC 3.4.13.9) |
| 83 | 84 | RXN02820 | W0131 | 4799 | 6109 | GAMMA-GLUTAMYLTRANSPEPTIDASE (EC 2.3.2.2) |
| 85 | 86 | F RXA02820 | GR00801 | 1 | 507 | GAMMA-GLUTAMYLTRANSPEPTIDASE (EC 2.3.2.2) |
| 87 | 88 | F RXA02000 | GR00589 | 3430 | 3933 | GAMMA-GLUTAMYLTRANSPEPTIDASE (EC 2.3.2.2) |
| 89 | 90 | RXN03178 | W0334 | 921 | 121 | PENICILLIN-BINDING PROTEIN 5* PRECURSOR (D-ALANYL-D-ALANINE CARBOXYPEPTIDASE) (EC 3.4.16.4) |
| 91 | 92 | F RXA02859 | GR10005 | 846 | 121 | PENICILLIN-BINDING PROTEIN 5* PRECURSOR (D-ALANYL-D-ALANINE CARBOXYPEPTIDASE) (EC 3.4.16.4) |
| 93 | 94 | RXA00137 | GR00022 | 738 | 1826 | XAA-PRO AMINOPEPTIDASE (EC 3.4.11.9) |
| 95 | 96 | RXN00499 | W0086 | 8158 | 9438 | PROLINE IMINOPEPTIDASE (EC 3.4.11.5) |
| 97 | 98 | F RXA00499 | GR00125 | 3 | 959 | PROLINE IMINOPEPTIDASE |
| 99 | 100 | RXN00877 | W0099 | 2221 | 3885 | PEPTIDYL-DIPEPTIDASE DCP (EC 3.4.15.5) |
| 101 | 102 | F RXA00877 | GR00242 | 3 | 1067 | PEPTIDYL-DIPEPTIDASE DCP (EC 3.4.15.5) |
| 103 | 104 | RXN01014 | W0209 | 13328 | 10728 | AMINOPEPTIDASE N (EC 3.4.11.2) |
| 105 | 106 | F RXA01014 | GR00289 | 3 | 1580 | AMINOPEPTIDASE N (EC 3.4.11.2) |
| 107 | 108 | F RXA01018 | GR00290 | 2289 | 3152 | AMINOPEPTIDASE N (EC 3.4.11.2) |
| 109 | 110 | RXA01147 | GR00323 | 1353 | 94 | VACUOLAR AMINOPEPTIDASE I PRECURSOR (EC 3.4.11.1) |
| 111 | 112 | RXA01161 | GR00329 | 1253 | 117 | XAA-PRO AMINOPEPTIDASE (EC 3.4.11.9) |
| 113 | 114 | RXN01181 | W0065 | 1 | 957 | AMINOPEPTIDASE A/I (EC 3.4.11.1) |
| 115 | 116 | F RXA01181 | GR00337 | 1 | 957 | AMINOPEPTIDASE |
| 117 | 118 | RXN01277 | W0009 | 32155 | 34158 | PROLYL ENDOPEPTIDASE (EC 3.4.21.26) |
| 119 | 120 | F RXA01277 | GR00368 | 1738 | 50 | PROLYL ENDOPEPTIDASE (EC 3.4.21.26) |
| 121 | 122 | RXA01914 | GR00548 | 125 | 550 | AMINOPEPTIDASE |
| 123 | 124 | RXA02048 | GR00624 | 207 | 1580 | AMINOPEPTIDASE N (EC 3.4.11.2) |
| 125 | 126 | RXN00621 | W0135 | 5853 | 5071 | PROTEASE II (EC 3.4.21.83) |
| 127 | 128 | F RXA00621 | GR00163 | 4075 | 4857 | PTRB periplasmic protease |
| 129 | 130 | RXN00622 | W0135 | 5150 | 3735 | PROTEASE II (EC 3.4.21.83) |
| 131 | 132 | F RXA00622 | GR00163 | 4778 | 6193 | PTRB periplasmic protease |
| 133 | 134 | RXN00982 | W0149 | 7596 | 6091 | (L42758) proteinase [Streptomyces lividans] |
| 135 | 136 | F RXA00977 | GR00275 | 1647 | 2660 | (L42758) proteinase [Streptomyces lividans] |
| 137 | 138 | F RXA00982 | GR00276 | 5194 | 4949 | (L42758) proteinase [Streptomyces lividans] |
| 139 | 140 | RXA00152 | GR00023 | 7175 | 5880 | HFLC PROTEIN (EC 3.4.-.-) |
| 141 | 142 | RXA02558 | GR00731 | 4939 | 3965 | HFLC PROTEIN (EC 3.4.-.-) |
| 143 | 144 | RXA00500 | GR00125 | 969 | 1643 | O-SIALOGLYCOPROTEIN ENDOPEPTIDASE (EC 3.4.24.57) |
| 145 | 146 | RXA00501 | GR00125 | 1643 | 2149 | O-SIALOGLYCOPROTEIN ENDOPEPTIDASE (EC 3.4.24.57) |
| 147 | 148 | RXA00502 | GR00125 | 2156 | 3187 | O-SIALOGLYCOPROTEIN ENDOPEPTIDASE (EC 3.4.24.57) |

| **Enzymes in general** | | | | | | |
|---|---|---|---|---|---|---|
| Nucleic Acid SEQ ID NO | Amino Acid SEQ ID NO | Identification Code | Contig. | NT Start | NT Stop | Function |
| 149 | 150 | RXN02589 | W0098 | 16346 | 17110 | Hypothetical Methyltransferase (EC 2.1.1.-) |
| 151 | 152 | F RXA02589 | GR00741 | 13804 | 13040 | Predicted S-adenosylmethionine-dependent methyltransferase |
| 153 | 154 | RXA00226 | GR00032 | 26836 | 26012 | SAM-dependent methyltransferases |
| 155 | 156 | RXN01885 | W0184 | 2004 | 2804 | Hypothetical Methyltransferase (EC 2.1.1.-) |
| 157 | 158 | F RXA01885 | GR00539 | 1589 | 2389 | SAM-dependent methyltransferases |
| 159 | 160 | RXA02592 | GR00741 | 18477 | 17707 | SAM-dependent methyltransferases |
| 161 | 162 | RXN01795 | W0093 | 722 | 1318 | MODIFIKATION METHYLASE (EC 2.1.1.73) |
| 163 | 164 | F RXA01795 | GR00507 | 706 | 1140 | MODIFICATION METHYLASE (EC 2.1.1.73) |
| 165 | 166 | RXA01214 | GR00351 | 1640 | 3130 | LACCASE 1 PRECURSOR (EC 1.10.3.2) |
| 167 | 168 | RXA01250 | GR00364 | 592 | 5 | LACCASE 1 PRECURSOR (EC 1.10.3.2) |
| 169 | 170 | RXA02477 | GR00715 | 10581 | 11201 | CARBONIC ANHYDRASE (EC 4.2.1.1) |
| 171 | 172 | RXN00833 | GR00225 | 374 | 6 | THIOL PEROXIDASE (EC 1.11.1.-) |
| 173 | 174 | F RXA00833 | GR00225 | 374 | 6 | THIOL PEROXIDASE (EC 1.11.1.-) |
| 175 | 176 | RXA01224 | GR00354 | 4186 | 5208 | 2-NITROPROPANE DIOXYGENASE (EC 1.13.11.32) |
| 177 | 178 | RXA01182 | GR00337 | 1363 | 971 | Hypothetical Oxidoreductase |
| 179 | 180 | RXA02531 | GR00726 | 1226 | 1936 | Hypothetical Oxidoreductase |
| 181 | 182 | RXN00689 | W0005 | 22416 | 20926 | BETAINE-ALDEHYDE DEHYDROGENASE PRECURSOR (EC 1.2.1.8) |
| 183 | 184 | F RXA00689 | GR00180 | 1401 | 775 | BETAINE-ALDEHYDE DEHYDROGENASE PRECURSOR (EC 1.2.1.8) |
| 185 | 186 | RXN03128 | W0120 | 3 | 857 | MORPHINE 6-DEHYDROGENASE (EC 1.1.1.218) |
| 187 | 188 | F RXA02192 | GR00643 | 2 | 523 | MORPHINE 6-DEHYDROGENASE (EC 1.1.1.218) |
| 189 | 190 | RXA02351 | GR00679 | 132 | 1070 | NITRILOTRIACETATE MONOOXYGENASE COMPONENT A (EC 1.14.13.-) |
| 191 | 192 | RXN00905 | W0238 | 8075 | 8875 | N-ACYL-L-AMINO ACID AMIDOHYDROLASE (EC 3.5.1.14) |
| 193 | 194 | F RXA00905 | GR00247 | 2 | 694 | N-ACYL-L-AMINO ACID AMIDOHYDROLASE (EC 3.5.1.14) |
| 195 | 196 | RXA00906 | GR00247 | 630 | 1133 | N-ACYL-L-AMINO ACID AMIDOHYDROLASE (EC 3.5.1.14) |
| 197 | 198 | RXA00907 | GR00247 | 1143 | 1265 | N-ACYL-L-AMINO ACID AMIDOHYDROLASE (EC 3.5.1.14) |
| 199 | 200 | RXA02101 | GR00631 | 3104 | 1842 | N-ACYL-L-AMINO ACID AMIDOHYDROLASE (EC 3.5.1.14) |
| 201 | 202 | RXN02565 | W0154 | 14299 | 13034 | N-ACYL-L-AMINO ACID AMIDOHYDROLASE (EC 3.5.1.14) |
| 203 | 204 | F RXA02565 | GR00733 | 1 | 342 | N-ACYL-L-AMINO ACID AMIDOHYDROLASE (EC 3.5.1.14) |
| 205 | 206 | F RXA02567 | GR00734 | 3 | 740 | N-ACYL-L-AMINO ACID AMIDOHYDROLASE (EC 3.5.1.14) |
| 207 | 208 | RXN03077 | W0043 | 1729 | 2913 | N-ACYL-L-AMINO ACID AMIDOHYDROLASE (EC 3.5.1.14) |
| 209 | 210 | F RXA02855 | GR10002 | 1693 | 2877 | N-ACYL-L-AMINO ACID AMIDOHYDROLASE (EC 3.5.1.14), hippurate hydrolase |
| 211 | 212 | RXA00026 | GR00003 | 3657 | 5042 | Hypothetical Amidohydrolase (EC 3.5.1.-) |
| 213 | 214 | RXA01971 | GR00569 | 963 | 133 | Hypothetical Metal-Dependent Hydrolase |
| 215 | 216 | RXA01802 | GR00509 | 3461 | 4291 | Predicted hydrolases (HAD superfamily) |
| 217 | 218 | RXN00866 | W0258 | 3557 | 4522 | Predicted Zn-dependent hydrolases |
| 219 | 220 | F RXA00866 | GR00236 | 3555 | 4499 | Predicted Zn-dependent hydrolases |
| 221 | 222 | RXA02410 | GR00703 | 792 | 127 | Predicted Zn-dependent hydrolases |
| 223 | 224 | RXA00961 | GR00267 | 2 | 433 | SALICYLATE HYDROXYLASE (EC 1.14.13.1) |
| 225 | 226 | RXA00111 | GR00016 | 930 | 1922 | SOLUBLE EPOXIDE HYDROLASE (SEH) (EC 3.3.2.3) |
| 227 | 228 | RXA01932 | GR00555 | 6479 | 5583 | ACETYL-HYDROLASE (EC 3.1,1.-) |
| 229 | 230 | RXA02574 | GR00739 | 833 | 1840 | PUTATIVE SECRETED HYDROLASE |
| 231 | 232 | RXN00983 | W0231 | 1796 | 321 | SIALIDASE PRECURSOR (EC 3.2.1.18) |
| 233 | 234 | F RXA00983 | GR00278 | 1200 | 4 | SIALIDASE PRECURSOR (EC 3.2.1.18) |
| 235 | 236 | RXA00984 | GR00278 | 1716 | 1300 | SIALIDASE PRECURSOR (EC 3.2.1.18) |
| 237 | 238 | RXN02513 | W0193 | 737 | 6 | SIALIDASE PRECURSOR (EC 3.2.1.18) |
| 239 | 240 | F RXA02513 | GR00722 | 93 | 824 | SIALIDASE PRECURSOR (EC 3.2.1.18) |
| 241 | 242 | RXA00903 | GR00246 | 637 | 5 | Putative epimerase |
| 243 | 244 | RXA01224 | GR00354 | 4186 | 5208 | 2-NITROPROPANE DIOXYGENASE (EC 1.13.11.32) |
| 245 | 246 | RXA01571 | GR00438 | 1360 | 1959 | ALCOHOL DEHYDROGENASE (EC 1.1.1.1) |
| 247 | 248 | RXN02478 | W0119 | 7564 | 6350 | SIALIDASE PRECURSOR (EC 3.2.1.18) |
| 249 | 250 | RXN00343 | W0125 | 1118 | 6 | 3-OXOSTEROID 1-DEHYDROGENASE (EC 1.3.99.4) |
| 251 | 252 | RXN01555 | W0135 | 29820 | 28861 | 3-OXOSTEROID 1-DEHYDROGENASE (EC 1.3.99.4) |
| 253 | 254 | RXN01166 | W0117 | 18142 | 16838 | EXTRACELLULAR LIPASE PRECURSOR (EC 3.1.1.3) |
| 255 | 256 | RXN02001 | W0326 | 630 | 1787 | N-ACYL-L-AMINO ACID AMIDOHYDROLASE (EC 3.5.1.14) |
| 257 | 258 | RXN03145 | W0142 | 7561 | 7115 | 4-OXALOCROTONATE TAUTOMERASE (EC 5.3.2.-) |
| 259 | 260 | RXN01466 | W0019 | 7050 | 6091 | ARYLESTERASE (EC 3.1.1.2) |
| 261 | 262 | RXN01145 | W0077 | 7538 | 6525 | KETOL-ACID REDUCTOISOMERASE (EC 1.1.1.86) |
| 263 | 264 | RXN03088 | W0052 | 3431 | 3817 | Hypothetical Methyltransferase (EC 2.1.1.-) |
| 265 | 266 | RXN02952 | W0320 | 1032 | 1547 | PUTATIVE REDUCTASE |
| 267 | 268 | RXN00513 | W0092 | 1573 | 653 | CARBOXYVINYL-CARBOXYPHOSPHONATE PHOSPHORYLMUTASE (EC 2.7.8.23) |
| 269 | 270 | RXN01152 | W0136 | 1740 | 907 | PROTEIN-L-ISOASPARTATE O-METHYLTRANSFERASE (EC 2.1.1.77) |
| 271 | 272 | RXN00787 | W0321 | 3736 | 5637 | D-AMINO ACID DEHYDROGENASE LARGE SUBUNIT (EC 1.4.99.1) |

| **N-metabolism** | | | | | | |
|---|---|---|---|---|---|---|
| Nucleic Acid SEQ ID NO | Amino Acid SEQ ID NO | Identification Code | Contig. | NT Start | NT Stop | Function |
| 273 | 274 | RXN01302 | W0148 | 2837 | 2385 | NITRATE REDUCTASE ALPHA CHAIN (EC 1.7.99.4) |
| 275 | 276 | F RXA01302 | GR00376 | 370 | 5 | NITRATE REDUCTASE ALPHA CHAIN (EC 1.7.99.4) |
| 277 | 278 | RXN01308 | W0148 | 2406 | 4 | NITRATE REDUCTASE ALPHA CHAIN (EC 1.7.99.4) |
| 279 | 280 | F RXA01307 | GR00377 | 686 | 6 | NITRATE REDUCTASE ALPHA CHAIN (EC 1.7.99.4) |
| 281 | 282 | F RXA01308 | GR00378 | 1211 | 6 | NITRATE REDUCTASE ALPHA CHAIN (EC 1.7.99.4) |
| 283 | 284 | RXN01309 | W0158 | 1 | 801 | NITRATE REDUCTASE ALPHA CHAIN (EC 1.7.99.4) |
| 285 | 286 | F RXA01309 | GR00379 | 719 | 51 | NITRATE REDUCTASE ALPHA CHAIN (EC 1.7.99.4) |
| 287 | 288 | RXA02017 | GR00610 | 1731 | 1048 | NITRATE REDUCTASE ALPHA CHAIN (EC 1.7.99.4) |
| 289 | 290 | RXA02018 | GR00610 | 2788 | 1739 | NITRATE REDUCTASE BETA CHAIN (EC 1.7.99.4) |
| 291 | 292 | RXA02016 | GR00610 | 1036 | 260 | NITRATE REDUCTASE GAMMA CHAIN (EC 1.7.99.4) |
| 293 | 294 | RXA00471 | GR00119 | 2997 | 3686 | NITRATE/NITRITE RESPONSE REGULATOR PROTEIN NARL |
| 295 | 296 | RXA00133 | GR00021 | 201 | 1013 | NITRATE/NITRITE RESPONSE REGULATOR PROTEIN NARP |
| 297 | 298 | RXA00650 | GR00169 | 4017 | 3382 | NITRATE/NITRITE RESPONSE REGULATOR PROTEIN NARP |
| 299 | 300 | RXA01189 | GR00339 | 2545 | 1937 | NITRATE/NITRITE RESPONSE REGULATOR PROTEIN NARP |
| 301 | 302 | RXA01607 | GR00449 | 123 | 752 | NITRATE/NITRITE RESPONSE REGULATOR PROTEIN NARP |
| 303 | 304 | RXN00470 | W0086 | 27401 | 28669 | NITRATE/NITRITE SENSOR PROTEIN NARX (EC 2.7.3.-) |
| 305 | 306 | F RXA00470 | GR00119 | 1752 | 2951 | NITRATE/NITRITE SENSOR PROTEIN NARX (EC 2.7.3.-) |
| 307 | 308 | RXA00756 | GR00203 | 2932 | 1937 | N UTILIZATION SUBSTANCE PROTEIN A |
| 309 | 310 | RXA00139 | GR00022 | 2514 | 3224 | N UTILIZATION SUBSTANCE PROTEIN B |
| 311 | 312 | RXA01303 | GR00376 | 1724 | 390 | NITRITE EXTRUSION PROTEIN |
| 313 | 314 | RXA01412 | GR00412 | 620 | 417 | NITROGEN FIXATION PROTEIN FIXI (PROBABLE E1-E2 TYPE CATION ATPASE) (EC 3.6.1.-) |
| 315 | 316 | RXA00773 | GR00205 | 3208 | 4350 | NITROGEN REGULATION PROTEIN NIFR3 |
| 317 | 318 | RXA02746 | GR00764 | 1 | 267 | NITROGEN REGULATORY PROTEIN P-II |
| 319 | 320 | RXA02745 | GR00763 | 15350 | 14472 | NODULATION ATP-BINDING PROTEIN |
| 321 | 322 | RXN00820 | W0054 | 19455 | 19817 | NODULATION PROTEIN N |
| 323 | 324 | F RXA00820 | GR00221 | 1007 | 1369 | NODULATION PROTEIN N |
| 325 | 326 | RXA01059 | GR00296 | 8782 | 9390 | OXYGEN-INSENSITIVE NAD(P)H NITROREDUCTASE (EC 1.-.-.-) |
| 327 | 328 | RXN01386 | W0008 | 39246 | 38317 | NITRILASE REGULATOR |
| 329 | 330 | RXN00073 | W0154 | 2369 | 687 | FERREDOXIN--NITRITE REDUCTASE (EC 1.7.7.1) |
| 331 | 332 | RXN03131 | W0127 | 276 | 4 | RHIZOPINE CATABOLISM PROTEIN MOCC |
| 333 | 334 | RXS00153 | W0167 | 4195 | 4620 | NODULATION PROTEIN |

| **Urease** | | | | | | |
|---|---|---|---|---|---|---|
| Nucleic Acid SEQ ID NO | Amino Acid SEQ ID NO | Identification Code | Contig. | NT Start | NT Stop | Function |
| **Phosphate and Phosphonate metabolism** | | | | | | |
| Nucleic Acid SEQ ID NO | Amino Acid SEQ ID NO | Identification Code | Contig. | NT Start | NT Stop | Function |
| 335 | 336 | RXN01716 | VV0319 | 3259 | 2774 | EXOPOLYPHOSPHATASE (EC 3.6.1.11) |
| 337 | 338 | RXN02972 | W0319 | 2763 | 2353 | EXOPOLYPHOSPHATASE (EC 3.6.1.11) |
| 339 | 340 | RXN00663 | W0142 | 10120 | 11493 | PHOH PROTEIN HOMOLOG |
| 341 | 342 | RXN00778 | VV0103 | 18126 | 19250 | PHOSPHATE-BINDING PERIPLASMIC PROTEIN PRECURSOR |
| 343 | 344 | RXN00250 | W0189 | 286 | 1032 | DEDA PROTEIN - ALKALINE PHOSPHATASE LIKE PROTEIN |

| **Sulfate metabolism** | | | | | | |
|---|---|---|---|---|---|---|
| Nucleic Acid SEQ ID NO | Amino Acid SEQ ID NO | Identification Code | Contig. | NT Start | NT Stop | Function |
| 345 | 346 | RXA00072 | GR00012 | 446 | 6 | PHOSPHOADENOSINE PHOSPHOSULFATE REDUCTASE (EC 1.8.99.4) |
| 347 | 348 | RXA00793 | GR00211 | 1469 | 2644 | SULFATE STARVATION-INDUCED PROTEIN 6 |
| 349 | 350 | RXA01192 | GR00342 | 161 | 733 | SULFATE STARVATION-INDUCED PROTEIN 6 |
| 351 | 352 | RXA00715 | GR00188 | 2120 | 2914 | THIOSULFATE SULFURTRANSFERASE (EC 2.8.1.1) |
| 353 | 354 | RXA01664 | GR00463 | 1306 | 485 | THIOSULFATE SULFURTRANSFERASE (EC 2.8.1.1) |
| 355 | 356 | RXN02334 | W0141 | 7939 | 7217 | THIOSULFATE SULFURTRANSFERASE (EC 2.8.1.1) |
| 357 | 358 | F RXA02334 | GR00672 | 2 | 355 | THIOSULFATE SULFURTRANSFERASE (EC 2.8.1.1) |

| **Fe-Metabolism** | | | | | | |
|---|---|---|---|---|---|---|
| Nucleic Acid SEQ ID NO | Amino Acid SEQ ID NO | Identification Code | Contig. | NT Start | NT Stop | Function |
| 359 | 360 | RXN01499 | W0008 | 7034 | 3213 | ENTEROBACTIN SYNTHETASE COMPONENT F |
| 361 | 362 | RXN01997 | W0084 | 33308 | 33793 | FERRITIN |

| **Mg Metabolism** | | | | | | |
|---|---|---|---|---|---|---|
| Nucleic Acid SEQ ID NO | Amino Acid SEQ ID NO | Identification Code | Contig. | NT Start | NT Stop | Function |
| 363 | 364 | RXA01848 | GR00524 | 1532 | 789 | MAGNESIUM-CHELATASE SUBUNIT CHLI |
| 365 | 366 | RXN01849 | W0139 | 16415 | 17515 | MAGNESIUM-CHELATASE SUBUNIT CHLI |
| 367 | 368 | F RXA01849 | GR00524 | 2004 | 1555 | MAGNESIUM-CHELATASE SUBUNIT CHLI |
| 369 | 370 | F RXA01691 | GR00474 | 570 | 4 | MAGNESIUM-CHELATASE SUBUNIT CHLI |
| 371 | 372 | RXN00665 | W0252 | 135 | 635 | MG2+/CITRATE COMPLEX SECONDARY TRANSPORTER |

| **Modification and degradation of aromatic compounds** | | | | | | |
|---|---|---|---|---|---|---|
| Nucleic Acid SEQ ID NO | Amino Acid SEQ ID NO | Identification Code | Contig. | NT Start | NT Stop | Function |
| 373 | 374 | RXN03026 | W0007 | 28635 | 28901 | 3-DEHYDROQUINATE DEHYDRATASE (EC 4.2.1.10) |
| 375 | 376 | RXN02908 | W0025 | 8507 | 8247 | O-SUCCINYLBENZOIC ACID--COA LIGASE (EC 6.2.1.26) |
| 377 | 378 | RXN03000 | W0235 | 570 | 4 | SALICYLATE HYDROXYLASE (EC 1.14.13.1) |
| 379 | 380 | RXN03036 | W0014 | 671 | 6 | PROTOCATECHUATE 3,4-DIOXYGENASE BETA CHAIN (EC 1.13.11.3) |
| 381 | 382 | RXN02974 | W0229 | 12631 | 12437 | 4-NITROPHENYLPHOSPHATASE (EC 3.1.3.41) |
| 383 | 384 | RXN00393 | W0025 | 7241 | 6348 | 1,4-DIHYDROXY-2-NAPHTHOATE OCTAPRENYLTRANSFERASE (EC 2.5.-.-) |
| 385 | 386 | RXN00948 | W0107 | 4266 | 5384 | 12-oxophytodienoate reductase (EC 1.3.1.42) |
| 387 | 388 | RXN01923 | W0020 | 3384 | 4133 | 2-HYDROXY-6-OXO-6-PHENYLHEXA-2,4-DIENOATE HYDROLASE (EC 3.7.1.-) |
| 389 | 390 | RXN00398 | W0025 | 14633 | 13884 | 2-PYRONE-4,6-DICARBOXYLATE LACTONASE (EC 3.1.1.57) |
| 391 | 392 | RXN02813 | W0128 | 13120 | 14118 | 3-CARBOXY-CIS,CIS-MUCONATE CYCLOISOMERASE HOMOLOG (EC 5.5.1.2) |
| 393 | 394 | RXN00136 | W0134 | 13373 | 14467 | 3-DEHYDROQUINATE SYNTHASE (EC 4.6.1.3) |
| 395 | 396 | RXN02508 | W0007 | 26733 | 28586 | 3-DEHYDROSHIKIMATE DEHYDRATASE (EC 4.2.1.-) |
| 397 | 398 | RXN02839 | W0362 | 3 | 449 | 4-HYDROXYBENZOATE OCTAPRENYLTRANSFERASE (EC 2.5.1.-) |
| 399 | 400 | RXN00639 | W0128 | 7858 | 8712 | CATECHOL 1,2-DIOXYGENASE (EC 1.13.11.1) |
| 401 | 402 | RXN02530 | W0057 | 5469 | 6125 | DIMETHYLANILINE MONOOXYGENASE (N-OXIDE FORMING) 1 (EC 1.14.13.8) |
| 403 | 404 | RXN00434 | W0112 | 12078 | 11212 | QUINONE-OXIDOREDUCTASE (EC 1.6.5.5) |
| 405 | 406 | RXN01619 | W0050 | 24649 | 23675 | QUINONE OXIDOREDUCTASE (EC 1.6.5.5) |
| 407 | 408 | RXN01842 | W0234 | 1615 | 2532 | QUINONE OXIDOREDUCTASE (EC 1.6.5.5) |
| 409 | 410 | RXN00641 | W0128 | 7440 | 5950 | TOLUATE 1,2-DIOXYGENASE ALPHA SUBUNIT (EC 1.14.12.-) |
| 411 | 412 | RXN01993 | W0182 | 16 | 1143 | VANILLATE DEMETHYLASE (EC 1.14.-.-) |
| 413 | 414 | RXN00658 | W0083 | 15705 | 16397 | PHENOL 2-MONOOXYGENASE (EC 1.14.13.7) |
| 415 | 416 | RXN00178 | W0174 | 14670 | 15554 | hydroxyquinol 1,2-dioxygenase (EC 1.13.11.37) |
| 417 | 418 | RXN01461 | W0128 | 12414 | 13025 | PROTOCATECHUATE 3,4-DIOXYGENASE ALPHA CHAIN (EC 1.13.11.3) |
| 419 | 420 | RXN01653 | W0321 | 12867 | 11407 | DIBENZOTHIOPHENE DESULFURIZATION ENZYME A |
| 421 | 422 | RXN02053 | W0009 | 39448 | 40026 | DRGA PROTEIN |
| 423 | 424 | RXN00177 | W0174 | 13589 | 14656 | MALEYLACETATE REDUCTASE (EC 1.3.1.32) |
| 425 | 426 | RXC00963 | W0249 | 1816 | 2652 | PROTEIN involved in degradation of aromatic compounds |

| **Modification and degradation of aliphatic compounds** | | | | | | |
|---|---|---|---|---|---|---|
| Nucleic Acid SEQ ID NO | Amino Acid SEQ ID NO | Identification Code | Contig. | NT Start | NT Stop | Function |
| 427 | 428 | RXN00299 | W0176 | 43379 | 42402 | ALKANAL MONOOXYGENASE ALPHA CHAIN (EC 1.14.14.3) |
| 429 | 430 | F RXA00299 | GR00048 | 7376 | 6633 | ALKANAL MONOOXYGENASE ALPHA CHAIN (EC 1.14.14.3) |
| 431 | 432 | RXA00332 | GR00057 | 16086 | 15385 | ALKANAL MONOOXYGENASE ALPHA CHAIN (EC 1.14.14.3) |
| 433 | 434 | RXA01838 | GR00519 | 2 | 820 | ALKANAL MONOOXYGENASE ALPHA CHAIN (EC 1.14.14.3) |
| 435 | 436 | RXA02643 | GR00750 | 1603 | 560 | ALKANAL MONOOXYGENASE ALPHA CHAIN (EC 1.14.14.3) |
| 437 | 438 | RXA01933 | GR00555 | 6590 | 7192 | 2-HALOALKANOIC ACID DEHALOGENASE I (EC 3.8.1.2) |
| 439 | 440 | RXA02351 | GR00679 | 132 | 1070 | NITRILOTRIACETATE MONOOXYGENASE COMPONENT A (EC 1.14.13.-) |

| **TABLE 2 - Excluded Genes** | | | |
|---|---|---|---|
| **GenBank™ Accession No.** | **Gene Name** | **Gene Function** | **Reference** |
| A09073 | ppg | Phosphoenol pyruvate carboxylase | Bachmann, B. et al. "DNA fragment coding for phosphoenolpyruvat corboxylase, recombinant DNA carrying said fragment, strains carrying the recombinant DNA and method for producing L-aminino acids using said strains," Patent: EP 0358940-A 3 03121/90 |
| A45579, A45581, A45583, A45585 A45587 | | Threonine dehydratase | Moeckel, B. et al. "Production of L-isoleucine by means of recombinant micro-organisms with deregulated threonine dehydratase," Patent: WO 9519442-A 5 07/20/95 |
| AB003132 | murC; ftsQ; ftsZ | | Kobayashi, M. et al. "Cloning, sequencing, and characterization of the ftsZ gene from coryneform bacteria," *Biochem. Biophys. Res. Commun.,* 236(2):383-388 (1997) |
| AB015023 | murC; ftsQ | | Wachi, M. et al. "A murC gene from Coryneform bacteria," *Appl. Microbiol. Biotechnol.,* 51 (2):223-228 (1999) |
| AB018530 | dtsR | | Kimura, E. et al. "Molecular cloning of a novel gene, dtsR, which rescues the detergent sensitivity of a mutant derived from *Brevibacterium lactofermentum," Biosci. Biotechnol. Biochem.,* 60(10):1565-1570 (1996) |
| AB018531 | dtsR1; dtsR2 | | |
| AB020624 | murI | D-glutamate racemase | |
| AB023377 | tkt | transketolase | |
| AB024708 | gltB; gltD | Glutamine 2-oxoglutarate aminotransferase large and small subunits | |
| AB025424 | acn | aconitase | |
| AB027714 | rep | Replication protein | |
| AB027715 | rep; aad | Replication protein; aminoglycoside adenyltransferase | |
| AF005242 | argC | N-acetylglutamate-5-semialdehyde dehydrogenase | |
| AF005635 | glnA | Glutamine synthetase | |
| AF030405 | hisF | cyclase | |
| AF030520 | argG | Argininosuccinate synthetase | |
| AF031518 | argF | Ornithine carbamolytransferase | |
| AF036932 | aroD | 3-dehydroquinate dehydratase | |
| AF038548 | pyc | Pyruvate carboxylase | |
| | | | |
| AF038651 | dciAE; apt; rel | Dipeptide-binding protein; adenine phosphoribosyltransferase; GTP pyrophosphokinase | Wehmeier, L. et al. "The role of the Corynebacterium glutamicum rel gene in (p)ppGpp metabolism," *Microbiology,* 144:1853-1862 (1998) |
| AF041436 | argR | Arginine repressor | |
| AF045998 | impA | Inositol monophosphate phosphatase | |
| AF048764 | argH | Argininosuccinate lyase | |
| AF049897 | argC; argJ; argB; argD; argF; argR; argG; argH | N-acetylglutamylphosphate reductase; ornithine acetyltransferase; N-acetylglutamate kinase; acetylornithine transminase; ornithine carbamoyltransferase; arginine repressor; argininosuccinate synthase; argininosuccinate lyase | |
| AF050109 | inhA | Enoyl-acyl carrier protein reductase | |
| AF050166 | hisG | ATP phosphoribosyltransferase | |
| AF051846 | hisA | Phosphoribosylformimino-5-amino-1-phosphoribosyl-4-imidazolecarboxamide isomerase | |
| AF052652 | metA | Homoserine O-acetyltransferase | Park, S. et al. "Isolation and analysis of metA, a methionine biosynthetic gene encoding homoserine acetyltransferase in Corynebacterium glutamicum," *Mol. Cells.,* 8(3):286-294 (1998) |
| AF053071 | aroB | Dehydroquinate synthetase | |
| AF060558 | hisH | Glutamine amidotransferase | |
| AF086704 | hisE | Phosphoribosyl-ATP-pyrophosphohydrolase | |
| AF114233 | aroA | 5-enolpyruvylshikimate 3-phosphate synthase | |
| AF116184 | panD | L-aspartate-alpha-decarboxylase precursor | Dusch, N. et al. "Expression of the Corynebacterium glutamicum panD gene encoding L-aspartate-alpha-decarboxylase leads to pantothenate overproduction in Escherichia coli," *Appl. Environ. Microbiol.,* 65(4)1530-1539(1999) |
| AF124518 | aroD; aroE | 3-dehydroquinase; shikimate dehydrogenase | |
| AF124600 | aroC; aroK; aroB; pepQ | Chorismate synthase; shikimate kinase; 3-dehydroquinate synthase; putative cytoplasmic peptidase | |
| AF145897 | inhA | | |
| AF145898 | inhA | | |
| AJ001436 | ectP | Transport of ectoine, glycine betaine, proline | Peter, H. et al. "Corynebacterium glutamicum is equipped with four secondary carriers for compatible solutes: Identification, sequencing, and characterization of the proline/ectoine uptake system, ProP, and the ectoine/proline/glycine betaine carrier, EctP," *J. Bacterial.,* 180(22):6005-6012 (1998) |
| AJ004934 | dapD | Tetrahydrodipicolinate succinylase (incompleteⁱ) | Wehrmann, A. et al. "Different modes of diaminopimelate synthesis and their role in cell wall integrity: A study with Corynebacterium glutamicum," J. *Bacteriol,* 180(12):3159-3165 (1998) |
| AJ007732 | ppc; secG; amt; ocd; soxA | Phosphoenolpyruvate-carboxylase; ?; high affinity ammonium uptake protein; putative omithine-cyclodecarboxylase; sarcosine oxidase | |
| AJ010319 | ftsY, glnB, glnD; srp; amtP | Involved in cell division; PII protein; uridylyltransferase (uridylyl-removing enzmye); signal recognition particle; low affinity ammonium uptake protein | Jakoby, M. et al. "Nitrogen regulation in Corynebacterium glutamicum; Isolation of genes involved in biochemical characterization of corresponding proteins," *FEMS Microbiol,* 173(2):303-310 (1999) |
| AJ132968 | cat | Chloramphenicol aceteyl transferase | |
| AJ224946 | mqo | L-malate: quinone oxidoreductase | Molenaar, D. et al. "Biochemical and genetic characterization of the membrane-associated malate dehydrogenase (acceptor) from Corynebacterium glutamicum," *Eur. J. Biochem.,* 254(2):395-403 (1998) |
| AJ238250 | ndh | NADH dehydrogenase | |
| AJ238703 | porA | Porin | Lichtinger, T. et al. "Biochemical and biophysical characterization of the cell wall porin of Corynebacterium glutamicum: The channel is formed by a low molecular mass polypeptide," *Biochemistry,* 37(43):15024-15032 (1998) |
| D17429 | | Transposable element IS31831 | Vertes et al."Isolation and characterization of IS31831, a transposable element from Corynebacterium glutamicum," *Mol. Microbiol.,* 11(4):739-746 (1994) |
| D84102 | odhA | 2-oxoglutarate dehydrogenase | Usuda, Y. et al. "Molecular cloning of the Corynebacterium glutamicum (Brevibacterium lactofermentum AJ 12036) odhA gene encoding a novel type of 2-oxoglutarate dehydrogenase," *Microbiology,* 142:3347-3354 (1996) |
| E01358 | hdh; hk | Homoserine dehydrogenase; homoserine kinase | Katsumata, R. et al. "Production of L-thereonine and L-isoleucine," Patent: JP 1987232392-A 1 10/12/87 |
| E01359 | | Upstream of the start codon of homoserine kinase gene | Katsumata, R. et al. "Production of L-thereonine and L-isoleucine," Patent: JP 1987232392-A 2 10/12/87 |
| E01375 | | Tryptophan operon | |
| E01376 | trpL; trpE | Leader peptide; anthranilate synthase | Matsui, K. et al. "Tryptophan operon, peptide and protein coded thereby, utilization of tryptophan operon gene expression and production of tryptophan," Patent: JP 1987244382-A 1 10/24/87 |
| | | | |
| | | | |
| E01377 | | Promoter and operator regions of tryptophan operon | Matsui, K. et al. "Tryptophan operon, peptide and protein coded thereby, utilization of tryptophan operon gene expression and production of tryptophan," Patent: JP 1987244382-A 1 10/24/87 |
| E03937 | | Biotin-synthase | Hatakeyama, K. et al. "DNA fragment containing gene capable of coding biotin synthetase and its utilization," Patent: JP 1992278088-A 1 10/02/92 |
| E04040 | | Diamino pelargonic acid aminotransferase | Kohama, K. et al. "Gene coding diaminopelargonic acid aminotransferase and desthiobiotin synthetase and its utilization," Patent: JP 1992330284-A 11/18/92 |
| E04041 | | Desthiobiotinsynthetase | Kohama, K. et al. "Gene coding diaminopelargonic acid aminotransferase and desthiobiotin synthetase and its utilization," Patent: JP 1992330284-A 1 11/18/92 |
| E04307 | | Flavum aspartase | Kurusu, Y. et al. "Gene DNA coding aspartase and utilization thereof," Patent: JP 1993030977-A 1 02/09/93 |
| E04376 | | Isocitric acid lyase | Katsumata, R. et al. "Gene manifestation controlling DNA," Patent: JP 1993056782-A 3 03/09193 |
| E04377 | | Isocitric acid lyase N-terminal fragment | Katsumata, R. et al. "Gene manifestation controlling DNA," Patent: JP 1993056782-A 3 03/09/93 |
| E04484 | | Prephenate dehydratase | Sotouchi, N. et al. "Production of L-phenylalanine by fermentation," Patent: JP 1993076352-A 2 03/30/93 |
| E05108 | | Aspartokinase | Fugono, N. et al. "Gene DNA coding Aspartokinase and its use," Patent: JP 1993184366-A 1 07/27/93 |
| E05112 | | Dihydro-dipichorinate synthetase | Hatakeyama, K. et al. "Gene DNA coding dihydrodipicolinic acid synthetase and its use," Patent: JP 1993184371-A 1 07/27/93 |
| E05776 | | Diaminopimelic acid dehydrogenase | Kobayashi, M. et al. "Gene DNA coding Diaminopimelic acid dehydrogenase and its use," Patent: JP 1993284970-A 1 11/02/93 |
| E05779 | | Threonine synthase | Kohama, K. et al. "Gene DNA coding threonine synthase and its use," Patent: JP 1993284972-A 1 11/02/93 |
| E06110 | | Prephenate dehydratase | Kikuchi, T. et al. "Production of L-phenylalanine by fermentation method," Patent: JP 1993344881-A 1 12/27/93 |
| E06111 | | Mutated Prephenate dehydratase | Kikuchi, T. et al. "Production of L-phenylalanine by fermentation method," Patent: JP 1993344881-A 1 12/27/93 |
| E06146 | | Acetohydroxy acid synthetase | Inui, M. et al. "Gene capable of coding Acetohydroxy acid synthetase and its use," Patent: JP 1993344893-A 1 12/27/93 |
| E06825 | | Aspartokinase | Sugimoto, M. et al. "Mutant aspartokinase gene," patent: JP 1994062866-A 1 03/08/94 |
| E06826 | | Mutated aspartokinase alpha subunit | Sugimoto, M. et al. "Mutant aspartokinase gene," patent: JP 1994062866-A 1 03/08/94 |
| | | | |
| E06827 | | Mutated aspartokinase alpha subunit | Sugimoto, M. et al. "Mutant aspartokinase gene," patent: JP 1994062866-A1 03/08/94 |
| E07701 | secY | | Honno, N. et al. "Gene DNA participating in integration of membraneous protein to membrane," Patent: JP 1994169780-A 1 06/21/94 |
| E08177 | | Aspartokinase | Sato, Y. et al. ''Genetic DNA capable of coding Aspartokinase released from feedback inhibition and its utilization," Patent: JP 1994261766-A 1 09/20/94 |
| E08178, E08179, E08180, E08181, E08182 | | Feedback inhibition-released Aspartokinase | Sato, Y. et al. "Genetic DNA capable of coding Aspartokinase released from feedback inhibition and its utilization," Patent: JP 1994261766-A 1 09/20/94 |
| E08232 | | Acetohydroxy-acid isomeroreductase | Inui, M. et al. "Gene DNA coding acetohydroxy acid isomeroreductase," Patent: JP 1994277067-A 1 10/04/94 |
| E08234 | secE | | Asai, Y. et al. "Gene DNA coding for translocation machinery of protein," Patent: JP 1994277073-A1 10/04/94 |
| E08643 | | FT aminotransferase and desthiobiotin synthetase promoter region | Hatakeyama, K. et al. "DNA fragment having promoter function in coryneform bacterium," Patent: JP 1995031476-A1 02/03/95 |
| E08646 | | Biotin synthetase | Hatakeyama, K. et al. "DNA fragment having promoter function in coryneform bacterium," Patent: JP 1995031476-A 1 02/03/95 |
| E08649 | | Aspartase | Kohama, K. et al "DNA fragment having promoter function in coryneform bacterium," Patent: JP 1995031478-A 1 02/03/95 |
| E08900 | | Dihydrodipicolinate reductase | Madori, M. et al. "DNA fragment containing gene coding Dihydrodipicolinate acid reductase and utilization thereof," Patent: JP 1995075578-A 1 03/20/95 |
| E08901 | | Diaminopimelic acid decarboxylase | Madori, M. et al. "DNA fragment containing gene coding Diaminopimelic acid decarboxylase and utilization thereof," Patent: JP 1995075579-A 1 03/20/95 |
| E12594 | | Serine hydroxymethyltransferase | Hatakeyama, K. et al. "Production of L-trypophan," Patent: JP 1997028391-A 1 02/04/97 |
| E12760, E12759, E12758 | | transposase | Moriya, M. et al. "Amplification of gene using artificial transposon," Patent: JP 1997070291-A 03/18/97 |
| E12764 | | Arginyl-tRNA synthetase; diaminopimelic acid decarboxylase | Moriya, M. et al. "Amplification of gene using artificial transposon," Patent: JP 1997070291-A 03/18/97 |
| E 12767 | | Dihydrodipicolinic acid synthetase | Moriya, M. et al. "Amplification of gene using artificial transposon," Patent: JP 1997070291-A 03/18/97 |
| E12770 | | aspartokinase | Moriya, M. et al. "Amplification of gene using artificial transposon," Patent: JP 1997070291-A 03/18/97 |
| E12773 | | Dihydrodipicolinic acid reductase | Moriya, M. et al. "Amplification of gene using artificial transposon," Patent: JP 1997070291-A 03/18/97 |
| E13655 | | Glucose-6-phosphate dehydrogenase | Hatakeyama, K. et al. "Glucose-6-phosphate dehydrogenase and DNA capable of coding the same," Patent: JP 1997224661-A 1 09/02/97 |
| L01508 | 11vA | Threonine dehydratase | Moeckel, B. et al. "Functional and structural analysis of the threonine dehydratase of Corynebacterium glutamicum," *J*. *Bacteriol.,* 174:8065-8072 (1992) |
| L07603 | EC 4.2.1.15 | 3-deoxy-D-arabinoheptulosonate-7-phosphate synthase | Chen, C. et al. "The cloning and nucleotide sequence of Corynebacterium glutamicum 3-deoxy-D-arabinoheptulosonate-7-phosphate synthase gene," *FEMS Microbiol. Lett.,* 107:223-230 (1993) |
| L09232 | llvB; ilvN; ilvc | Acetohydroxy acid synthase large subunit; Acetohydroxy acid synthase small subunit; Acetohydroxy acid isomeroreductase | Keilhauer, C. et al. "Isoleucine synthesis in Corynebacterium glutamicum: molecular analysis of the ilvB-ilvN-ilvC operon," *J. Bacteriol.,* 175(17):5595-5603 (1993) |
| L18874 | PtsM | Phosphoenolpyruvate sugar phosphotransferase | Fouet, A et al. "Bacillus subtilis sucrose-specific enzyme II of the phosphotransferase system: expression in Escherichia coli and homology to enzymes II from enteric bacteria," *PNAS USA,* 84(24):8773-8777 (1987); Lee, J.K. et al. "Nucleotide sequence of the gene encoding the Corynebacterium glutamicum mannose enzyme II and analyses of the deduced protein sequence," *FEMS Microbiol. Lett.,* 119(1-2):137-145 (1994) |
| L27123 | aceB | Malate synthase | Lee, H-S. et al. "Molecular characterization of aceB, a gene encoding malate synthase in Corynebacterium glutamicum," *J. Microbiol. Biotechnol.,* 4(4):256-263 (1994) |
| L27126 | | Pyruvate kinase | Jetten, M. S. et al. "Structural and functional analysis of pyruvate kinase from Corynebacterium glutamicum," *Appl. Environ. Microbiol.,* 60(7):2501-2507 (1994) |
| L28760 | aceA | Isocitrate lyase | |
| L35906 | dtxr | Diphtheria toxin repressor | Oguiza, J.A. et al. "Molecular cloning, DNA sequence analysis, and characterization of the Corynebacterium diphtheriae dtxR from Brevibacterium lactofermentum," *J. Bacteriol.,* 177(2):465-467 (1995) |
| M 13774 | | Prephenate dehydratase | Follettie, M.T. et al. "Molecular cloning and nucleotide sequence of the Corynebacterium glutamicum pheA gene," *J. Bacteriol.,* 167:695-702 (1986) |
| M16175 | 5S rRNA | | Park, Y-H. et al. "Phylogenetic analysis of the coryneform bacteria by 56 rRNA sequences," *J. Bacterial.,* 169:1801-1806 (1987) |
| M 16663 | trpE | Anthranilate synthase, 5' end | Sano, K. et al. "Structure and function of the trp operon control regions of Brevibacterium lactofermentum, a glutamic-acid-producing bacterium," *Gene,* 52:191-200 (1987) |
| M16664 | trpA | Tryptophan synthase, 3'end | Sano, K. et al. "Structure and function of the trp operon control regions of Brevibacterium lactofermentum, a glutamic-acid-producing bacterium," *Gene,* 52:191-200 (1987) |
| | | | |
| M25819 | | Phosphoenolpyruvate carboxylase | O'Regan, M. et al. "Cloning and nucleotide sequence of the Phosphoenolpyruvate carboxylase-coding gene of Corynebacterium glutamicum ATCC13032," *Gene,* 77(2):237-251 (1989) |
| M85106 | | 23S rRNA gene insertion sequence | Roller, C. et al. "Gram-positive bacteria with a high DNA G+C content are characterized by a common insertion within their 23S rRNA genes," *J. Gen. Microbiol.,* 138:1167-1175 (1992) |
| M85107, M85108 | | 23S rRNA gene insertion sequence | Roller, C. et al. "Gram-positive bacteria with a high DNA G+C content are characterized by a common insertion within their 23S rRNA genes," *J. Gen. Microbiol.,* 138:1167-1175 (1992) |
| M89931 | aecD; brnQ; yhbw | Beta C-S lyase; branched-chain amino acid uptake carrier; hypothetical protein yhbw | Rossol, I. et al. "The Corynebacterium glutamicum aecD gene encodes a C-S lyase with alpha, beta-elimination activity that degrades aminoethylcysteine," *J. Bacteriol.,* 174(9):2968-2977 (1992); Tauch, A. et al. "Isoleucine uptake in Corynebacterium glutamicum ATCC 13032 is directed by the brnQ gene product," *Arch. Microbiol.,* 169(4):303-312 (1998) |
| S59299 | trp | Leader gene (promoter) | Herry, D.M. et al. "Cloning of the trp gene cluster from a tryptophan-hyperproducing strain of Corynebacterium glutamicum: identification of a mutation in the trp leader sequence," *Appl. Environ. Microbiol.,* 59(3):791-799 (1993) |
| U11545 | trpD | Anthranilate phosphoribosyltransferase | O'Gara, J.P. and Dunican, L.K. (1994) Complete nucleotide sequence of the Corynebacterium glutamicum ATCC 21850 tpD gene." Thesis, Microbiology Department, University College Galway, Ireland. |
| U 13922 | cgIIM; cgIIR; eigIIR | Putative type II 5-cytosoine methyltransferase; putative type II restriction endonuclease; putative type I or type III restriction endonuclease | Schafer, A. et al. "Cloning and characterization of a DNA region encoding a stress-sensitive restriction system from Corynebacterium glutamicum ATCC 13032 and analysis of its role in intergeneric conjugation with Escherichia coli," *J*. *Bacteriol.,* 176(23):7309-7319 (1994); Schafer, A. et al. "The Corynebacterium glutamicum cglIM gene encoding a 5-cytosine in an McrBC-deficient Escherichia coli strain," *Gene,* 203(2):95-101 (1997) |
| U 14965 | recA | | |
| U31224 | ppx | | Ankri, S. et al. "Mutations in the Corynebacterium glutamicumproline biosynthetic pathway: A natural bypass of the proA step," *J. Bacteriol.,* 178(15):4412-4419 (1996) |
| U31225 | proC | L-proline: NADP+ 5-oxidoreductase | Ankri, S. et al. "Mutations in the Corynebacterium glutamicumproline biosynthetic pathway: A natural bypass of the proA step," *J. Bacteriol.,* 178(15):4412-4419 (1996) |
| U31230 | obg; proB; unkdh | ?;gamma glutamyl kinase;similar to D-isomer specific 2-hydroxyacid dehydrogenases | Ankri, S. et al. "Mutations in the Corynebacterium glutamicumproline biosynthetic pathway: A natural bypass of the proA step," *J. Bacteriol.,* 178(15):4412-4419 (1996) |
| | | | |
| U31281 | bioB | Biotin synthase | Serebriiskii, I.G., "Two new members of the bio B superfamily: Cloning, sequencing and expression of bio B genes of Methylobacillus flagellatum and Corynebacterium glutamicum," *Gene,* 175:15-22 (1996) |
| U35023 | thtR; accBC | Thiosulfate sulfurtransferase; acyl CoA carboxylase | Jager, W. et al. "A Corynebacterium glutamicum gene encoding a two-domain protein similar to biotin carboxylases and biotin-carboxyl-carrier proteins," *Arch. Microbiol.,* 166(2);76-82 (1996) |
| U43535 | cmr | Multidrug resistance protein | Jager, W. et al. "A Corynebacterium glutamicum gene conferring multidrug resistance in the heterologous host Escherichia coli," *J. Bacteriol.,* 179(7):2449-2451 (1997) |
| U43536 | clpB | Heat shock ATP-binding protein | |
| U53587 | aphA-3 | 3'5" -aminoglycoside phosphotransferase | |
| U89648 | | Corynebacterium glutamicum unidentified sequence involved in histidine biosynthesis, partial sequence | |
| X04960 | trpA; trpB; trpC; trpD; trpE; trpG; trpL | Tryptophan operon | Matsui, K. et al. "Complete nucleotide and deduced amino acid sequences of the Brevibacterium lactofermentum tryptophan operon," *Nucleic Acids Res.,* 14(24):10113-10114 (1986) |
| X07563 | lys A | DAP decarboxylase (meso-diaminopimelate decarboxylase, EC 4.1.1.20) | Yeh, P. et al. "Nucleic sequence of the lysA gene of Corynebacterium glutamicum and possible mechanisms for modulation of its expression," *Mol. Gen. Genet.,* 212(1):112-119 (1988) |
| X14234 | EC 4.1.1.31 | Phosphoenolpyruvate carboxylase | Eikmanns, B.J. et al. "The Phosphoenolpyruvate carboxylase gene of Corynebacterium glutamicum: Molecular cloning, nucleotide sequence, and expression," *Mol. Gen. Genet.,* 218(2):330-339 (1989); Lepiniec, L. et al. "Sorghum Phosphoenolpyruvate carboxylase gene family: structure, function and molecular evolution," *Plant. Mol. Biol.,* 21 (3):487-502 (1993) |
| X17313 | fda | Fructose-bisphosphate aldolase | Von der Osten, C.H. et al. "Molecular cloning, nucleotide sequence and fine-structural analysis of the Corynebacterium glutamicum fda gene: structural comparison of C. glutamicum fructose-1, 6-biphosphate aldolase to class I and class II aldolases," *Mol. Microbiol.,* |
| X53993 | dapA | L-2, 3-dihydrodipicolinate synthetase (EC 4.2.1.52) | Bonnassie, S. et al. "Nucleic sequence of the dapA gene from Corynebacterium glutamicum," *Nucleic Acids Res.,* 18(21):6421 (1990) |
| X54223 | | AttB-related site | Cianciotto, N. et al. "DNA sequence homology between att B-related sites of Corynebacterium diphtheriae, Corynebacterium ulcerans, Corynebacterium glutamicum , and the attP site of lambdacorynephage," *FEMS. Microbiol, Lett.,* 66:299-302 (1990) |
| X54740 | argS; lysA | Arginyl-tRNA synthetase; Diaminopimelate decarboxylase | Marcel, T. et al. "Nucleotide sequence and organization of the upstream region of the Corynebacterium glutamicum lysA gene," *Mol. Microbiol.*, 4(11):1819-1830 (1990) |
| X55994 | trpL; trpE | Putative leader peptide; anthranilate synthase component I | Heery, D.M. et al. "Nucleotide sequence of the Corynebacterium glutamicum trpE gene," *Nucleic Acids Res.,* 18(23):7138 (1990) |
| X56037 | thrC | Threonine synthase | Han, K.S. et al. "The molecular structure of the Corynebacterium glutamicum threonine synthase gene," *Mol. Microbiol.,* 4(10):1693-1702 (1990) |
| X56075 | attB-related site | Attachment site | Cianciotto, N. et al. "DNA sequence homology between att B-related sites of Corynebacterium diphtheriae, Corynebacterium ulcerans, Corynebacterium glutamicum , and the attP site of lambdacorynephage," *FEMS Microbiol, Lett.,* 66:299-302 (1990) |
| X57226 | lysc-alpha; lysc-beta; asd | Aspartokinase-alpha subunit; Aspartokinase-beta subunit; aspartate beta semialdehyde dehydrogenase | Kalinowski, J. et al. "Genetic and biochemical analysis of the Aspartokinase from Corynebacterium glutamicum," *Mol. Microbiol.,* 5(5):1197-1204 (1991); Kalinowski, J. et al. "Aspartokinase genes lysc alpha and lysc beta overlap and are adjacent to the aspertate beta-semialdehyde dehydrogenase gene asd in Corynebacterium glutamicum," *Mol. Gen. Genet.,* 224(3):317-324 (1990) |
| X59403 | gap;pgk; tpi | Glyceraldehyde-3-phosphate; phosphoglycerate kinase; triosephosphate isomerase | Eikmanns, B.J. "Identification, sequence analysis, and expression of a Corynebacterium glutamicum gene cluster encoding the three glycolytic enzymes glyceraldehyde-3-phosphate dehydrogenase, 3-phosphoglycerate kinase, and triosephosphate isomeras," *J. Bacteriol.,* 174(19):6076-6086 (1992) |
| X59404 | gdh | Glutamate dehydrogenase | Bormann, E.R. et al. "Molecular analysis of the Corynebacterium glutamicum gdh gene encoding glutamate dehydrogenase," *Mol. Microbiol.,* 6(3):317-326 (1992) |
| X60312 | lysI | L-lysine permease | Seep-Feldhaus, A.H. et al. "Molecular analysis of the Corynebacterium glutamicum lysI gene involved in lysine uptake," *Mol. Microbiol.,* 5(12):2995-3005 (1991) |
| X66078 | cop1 | Ps1 protein | Joliff, G. et al. "Cloning and nucleotide sequence of the csp1 gene encoding PS1 one of the two major secreted proteins of Corynebacterium glutamicum: The deduced N-terminal region of PS1 is similar to the Mycobacterium antigen 85 complex," *Mol. Microbiol.,* 6(16):2349-2362 (1992) |
| X66112 | glt | Citrate synthase | Eikmanns, B.J. et al. "Cloning sequence, expression and transcriptional analysis of the Corynebacterium glutamicum gltA gene encoding citrate synthase," *Microbiol.,* 140:1817-1828 (1994) |
| X67737 | dapB | Dihydrodipicolinate reductase | |
| X69103 | csp2 | Surface layer protein PS2 | Peyret, J.L. et al. "Characterization of the cspB gene encoding PS2, an ordered surface-layer protein in Corynebacterium glutamicum," *Mol. Microbiol.,* 9(1):97-109 (1993) |
| X69104 | | IS3 related insertion element | Bonamy, C. et al. "Identification of IS1206, a Corynebacterium glutamicum IS3-related insertion sequence and phylogenetic analysis," *Mol. Microbiol.,* 14(3):571-581 (1994) |
| X70959 | leuA | Isopropylmalate synthase | Patek, M. et al. "Leucine synthesis in Corynebacterium glutamicum: enzyme activities, structure of leuA, and effect of leuA inactivation on lysine synthesis," *Appl. Environ. Microbiol.,* 60(1):133-140 (1994) |
| X71489 | icd | Isocitrate dehydrogenase (NADP+) | Eikmanns, B.J. et al. "Cloning sequence analysis, expression, and inactivation of the Corynebacterium glutamicum icd gene encoding isocitrate dehydrogenase and biochemical characterization of the enzyme," *J. Bacteriol.,* 177(3):774-782 (1995) |
| X72855 | GDHA | Glutamate dehydrogenase (NADP+) | |
| X75083, X70584 | mtrA | 5-methyltryptophan resistance | Heery, D.M. et al. "A sequence from a tryptophan-hyperproducing strain of Corynebacterium glutamicum encoding resistance to 5-methyltryptophan," *Biochem. Biophys. Res. Commun.,* 201(3):1255-1262 (1994) |
| X75085 | recA | | Fitzpatrick, R. et al. "Construction and characterization of recA mutant strains of Corynebacterium glutamicum and Brevibacterium lactofermentum," *Appl. Microbiol. Biotechnol.,* 42(4):575-580 (1994) |
| X75504 | aceA; thiX | Partial Isocitrate lyase; ? | Reinscheid, D.J. et al. "Characterization of the isocitrate lyase gene from Corynebacterium glutamicum and biochemical analysis of the enzyme," J. *Bacteriol.,* 176(12):3474-3483 (1994) |
| X76875 | | ATPase beta-subunit | Ludwig, W. et al. "Phylogenetic relationships of bacteria based on comparative sequence analysis of elongation factor Tu and ATP-synthase beta-subunit genes," *Antonie Van Leeuwenhoek,* 64:285-305 (1993) |
| X77034 | tuf | Elongation factor Tu | Ludwig, W. et al. "Phylogenetic relationships of bacteria based on comparative sequence analysis of elongation factor Tu and ATP-synthase beta-subunit genes," *Antonie Van Leeuwenhoek,* 64:285-305 (1993) |
| X77384 | recA | | Billman-Jacobe, H. "Nucleotide sequence of a recA gene from Corynebacterium glutamicum," *DNA Seq.,* 4(6):403-404 (1994) |
| X78491 | aceB | Malate synthase | Reinscheid, D.J. et al. "Malate synthase from Corynebacterium glutamicum pta-ack operon encoding phosphotransacetylase: sequence analysis," *Microbiology,* 140:3099-3108 (1994) |
| X80629 | 16S rDNA | 16S ribosomal RNA | Rainey, F.A. et al. "Phylogenetic analysis of the genera Rhodococcus and Norcardia and evidence for the evolutionary origin of the genus Norcardia from within the radiation of Rhodococcus species," *Microbiol.,* 141:523-528 (1995) |
| X81191 | gluA; gluB; gluC; gluD | Glutamate uptake system | Kronemeyer, W. et al. "Structure of the gluABCD cluster encoding the glutamate uptake system of Corynebacterium glutamicum," *J. Bacteriol.,* 177(5):1152-1158 (1995) |
| X81379 | dapE | Succinyldiaminopimelate desuccinylase | Wehrmann, A. et al. "Analysis of different DNA fragments of Corynebacterium glutamicum complementing dapE of Escherichia coli," *Microbiology,* 40:3349-56 (1994) |
| X82061 | 16S rDNA | 16S ribosomal RNA | Ruimy, R. et al. "Phylogeny of the genus Corynebacterium deduced from analyses of small-subunit ribosomal DNA sequences," *Int. J. Syst. Bacteriol.,* 45(4):740-746 (1995) |
| X82928 | asd; lysC | Aspartate-semialdehyde dehydrogenase; ? | Serebrijski, I. et al. "Multicopy suppression by asd gene and osmotic stress-dependent complementation by heterologous proA in proA mutants," *J*. *Bacteriol.,* 177(24):7255-7260 (1995) |
| X82929 | proA | Gamma-glutamyl phosphate reductase | Serebrijski, I. et al. "Multicopy suppression by asd gene and osmotic stress-dependent complementation by heterologous proA in proA mutants," *J*. *Bacteriol.,* 177(24):7255-7260 (1995) |
| X84257 | 16S rDNA | 16S ribosomal RNA | Pascual, C. et al. "Phylogenetic analysis of the genus Corynebacterium based on 16S rRNA gene sequences," *Int. J. Syst. Bacteriol.,* 45(4):724-728 (1995) |
| X85965 | aroP; dapE | Aromatic amino acid permease; ? | Wehrmann et al. "Functional analysis of sequences adjacent to dapE of C. glutamicum proline reveals the presence of aroP, which encodes the aromatic amino acid transporter," *J. Bacterial.,* 177(20):5991-5993 (1995) |
| X86157 | argB; argC; argD; argF; argJ | Acetylglutamate kinase; N-acetyl-gamma-glutamyl-phosphate reductase; acetylornithine aminotransferase; ornithine carbamoyltransferase; glutamate N-acetyltransferase | Sakanyan, V. et al. "Genes and enzymes of the acetyl cycle of arginine biosynthesis in Corynebacterium glutamicum: enzyme evolution in the early steps of the arginine pathway," *Microbiology,* 142:99-108 (1996) |
| X89084 | pta; ackA | Phosphate acetyltransferase; acetate kinase | Reinscheid, D.J. et al. "Cloning, sequence analysis, expression and inactivation of the Corynebacterium glutamicum pta-ack operon encoding phosphotransacetylase and acetate kinase," *Microbiology,* 145:503-513 (1999) |
| X89850 | attB | Attachment site | Le Marrec, C. et al. "Genetic characterization of site-specific integration functions of phi AAU2 infecting "Arthrobacter aureus C70," *J. Bacteriol.,* 178(7):1996-2004 (1996) |
| X90356 | | Promoter fragment F1 | Patek, M. et al. "Promoters from Corynebacterium glutamicum: cloning, molecular analysis and search for a consensus motif," *Microbiology,* 142:1297-1309 (1996) |
| X90357 | | Promoter fragment F2 | Patek, M. et al. "Promoters from Corynebacterium glutamicum: cloning, molecular analysis and search for a consensus motif," *Microbiology,* 142:1297-1309 (1996) |
| X90358 | | Promoter fragment F10 | Patek, M. et al. "Promoters from Corynebacterium glutamicum: cloning, molecular analysis and search for a consensus motif," *Microbiology,* 142:1297-1309 (1996) |
| X90359 | | Promoter fragment F13 | Patek, M. et al. "Promoters from Corynebacterium glutamicum: cloning, molecular analysis and search for a consensus motif," *Microbiology,* 142:1297-1309 (1996) |
| | | | |
| X90360 | | Promoter fragment F22 | Patek, M. et al. "Promoters from Corynebacterium glutamicum: cloning, molecular analysis and search for a consensus motif," *Microbiology,* 142:1297-1309(1996) |
| X90361 | | Promoter fragment F34 | Patek, M. et al. "Promoters from Corynebacterium glutamicum: cloning, molecular analysis and search for a consensus motif," *Microbiology,* 142:1297-1309 (1996) |
| X90362 | | Promoter fragment F37 | Patek, M. et al. "Promoters from C. glutamicum: cloning, molecular analysis and search for a consensus motif," *Microbiology,* 142:1297-1309 (1996) |
| X90363 | | Promoter fragment F45 | Patek, M. et al. "Promoters from Corynebacterium glutamicum: cloning, molecular analysis and search for a consensus motif," *Microbiology,* 142:1297-1309(1996) |
| X90364 | | Promoter fragment F64 | Patek, M. et al. "Promoters from Corynebacterium glutamicum: cloning, molecular analysis and search for a consensus motif," *Microbiology,* 142:1297-1309 (1996) |
| X90365 | | Promoter fragment F75 | Patek, M. et al. "Promoters from Corynebacterium glutamicum: cloning, molecular analysis and search for a consensus motif," *Microbiology,* 142:1297-1309(1996) |
| X90366 | | Promoter fragment PF101 | Patek, M. et al. "Promoters from Corynebacterium glutamicum: cloning, molecular analysis and search for a consensus motif," *Microbiology,* 142:1297-1309 (1996) |
| X90367 | | Promoter fragment PF104 | Patek, M. et al. "Promoters from Corynebacterium glutamicum: cloning, molecular analysis and search for a consensus motif," *Microbiology,* 142:1297-1309 (1996) |
| X90368 | | Promoter fragment PF109 | Patek, M. et al. "Promoters from Corynebacterium glutamicum: cloning, molecular analysis and search for a consensus motif," *Microbiology,* 142:1297-1309 (1996) |
| X93513 | amt | Ammonium transport system | Siewe, R.M. et al. "Functional and genetic characterization of the (methyl) ammonium uptake carrier of Corynebacterium glutamicum," *J. Biol. Chem.,* 271(10):5398-5403(1996) |
| X93514 | betP | Glycine betaine transport system | Peter, H. et al. "Isolation, characterization, and expression of the Corynebacterium glutamicum betP gene, encoding the transport system for the compatible solute glycine betaine," *J. Bacteriol.,* 178(17):5229-5234 (1996) |
| X95649 | orf4 | | Patek, M. et al. "Identification and transcriptional analysis of the dapB-ORF2-dapA-ORF4 operon of Corynebacterium glutamicum, encoding two enzymes involved in L-lysine synthesis," *Biotechnol. Lett.,* 19:1113-1117 (1997) |
| X96471 | lysE; lysG | Lysine exporter protein; Lysine export regulator protein | Vrljic, M. et al. "A new type of transporter with a new type of cellular function: L-lysine export from Corynebacterium glutamicum," *Mol. Microbiol.,* 22(5):815-826 (1996) |
| X96580 | panB; panC; xylB | 3-methyl-2-oxobutanoate hydroxymethyltransferase; pantoate-beta-alanine ligase; xylulokinase | Sahm, H. et al. "D-pantothenate synthesis in Corynebacterium glutamicum and use of panBC and genes encoding L-valine synthesis for D-pantothenate overproduction," *Appl. Environ. Microbiol.,* 65(5):1973-1979 (1999) |
| X96962 | | Insertion sequence IS1207 and transposase | |
| X99289 | | Elongation factor P | Ramos, A. et al. "Cloning, sequencing and expression of the gene encoding elongation factor P in the amino-acid producer Brevibacterium lactofermentum (Corynebacterium glutamicum ATCC 13869)," *Gene,* 198:217-222 (1997) |
| Y00140 | thrB | Homoserine kinase | Mateos, L.M. et al. "Nucleotide sequence of the homoserine kinase (thrB) gene of the Brevibacterium lactofermentum," *Nucleic Acids Res.,* 15(9):3922 (1987) |
| Y00151 | ddh | Meso-diaminopimelate D-dehydrogenase (EC 1.4.1.16) | Ishino, S. et al. "Nucleotide sequence of the meso-diaminopimelate D-dehydrogenase gene from Corynebacterium glutamicum," *Nucleic Acids Res.,* 15(9):3917 (1987) |
| Y00476 | thrA | Homoserine dehydrogenase | Mateos, L.M. et al. "Nucleotide sequence of the homoserine dehydrogenase (thrA) gene of the Brevibacterium lactofermentum," *Nucleic Acids Res.,* 15(24):10598 (1987) |
| Y00546 | hom; thrB | Homoserine dehydrogenase; homoserine kinase | Peoples, O.P. et al. "Nucleotide sequence and fine structural analysis of the Corynebacterium glutamicum hom-thrB operon," *Mol. Microbiol.,* 2(1):63-72 (1988) |
| Y08964 | murC; ftsQ/divD; ftsZ | UPD-N-acetylmuramate-alanine ligase; division initiation protein or cell division protein; cell division protein | Honrubia, M.P. et al. "Identification, characterization, and chromosomal organization of the ftsZ gene from Brevibacterium lactofermentum," *Mol. Gen. Genet.,* 259(1):97-104 (1998) |
| Y09163 | putP | High affinity proline transport system | Peter, H. et al. "Isolation of the putP gene of Corynebacterium glutamicumproline and characterization of a low-affinity uptake system for compatible *solutes," Arch. Microbiol,* 168(2):143-151 (1997) |
| Y09548 | pyc | Pyruvate carboxylase | Peters-Wendisch, P.G. et al. "Pyruvate carboxylase from Corynebacterium glutamicum: characterization, expression and inactivation of the pyc gene," *Microbiology,* 144:915-927 (1998) |
| Y09578 | leuB | 3-isopropylmalate dehydrogenase | Patek, M. et al. "Analysis of the leuB gene from Corynebacterium glutamicum,"*Appl. Microbial. Biotechnol.,* 50(1):42-47 (1998) |
| Y12472 | | Attachment site bacteriophage Phi-16 | Moreau, S. et al. "Site-specific integration of corynephage Phi-16: The construction of an integration vector," *Microbiol.,* 145:539-548 (1999) |
| Y12537 | proP | Proline/ectoine uptake system protein | Peter, H. et al. "Corynebacterium glutamicum is equipped with four secondary carriers for compatible solutes: Identification, sequencing, and characterization of the proline/ectoine uptake system, ProP, and the ectoine/proline/glycine betaine carrier, EctP," *J. Bacterial.,* 180(22):6005-6012 (1998) |
| Y13221 | glnA | Glutamine synthetase I | Jakoby, M. et al. "Isolation of Corynebacterium glutamicum glnA gene encoding glutamine synthetase I," *FEMS Microbiol. Lett.,* 154(1):81-88 (1997) |
| Y16642 | 1pd | Dihydrolipoamide dehydrogenase | |
| Y18059 | | Attachment site Corynephage 304L | Moreau, S. et al. "Analysis of the integration functions of φ304L: An integrase module among corynephages," *Virology,* 255(1):150-159 (1999) |
| Z21501 | argS; lysA | Arginyl-tRNA synthetase; diaminopimelate decarboxylase (partial) | Oguiza, J.A. et al. "A gene encoding arginyl-tRNA synthetase is located in the upstream region of the lysA gene in Brevibacterium lactofermentum: Regulation of argS-lysA cluster expression by arginine," *J*. *Bacteriol,* 175(22):7356-7362 (1993) |
| Z21502 | dapA; dapB | Dihydrodipicolinate synthase; dihydrodipicolinate reductase | Pisabarro, A. et al. "A cluster of three genes (dapA, orf2, and dapB) of Brevibacterium lactofermentum encodes dihydrodipicolinate reductase, and a third polypeptide of unknown function," *J. Bacteriol.,* 175(9):2743-2749 (1993) |
| Z29563 | thrC | Threonine synthase | Malumbres, M. et al. "Analysis and expression of the thrC gene of the encoded threonine synthase," *Appl. Environ. Microbiol.,* 60(7)2209-2219 (1994) |
| Z46753 | 16S rDNA | Gene for 16S ribosomal RNA | |
| Z49822 | sigA | SigA sigma factor | Oguiza, J.A. et al "Multiple sigma factor genes in Brevibacterium lactofermentum: Characterization of sigA and sigB," *J Bacteriol.,* 178(2):550-553 (1996) |
| Z49823 | galE; dtxR | Catalytic activity UDP-galactose 4-epimerase; diphtheria toxin regulatory protein | Oguiza, J.A. et al "The galE gene encoding the UDP-galactose 4-epimerase of Brevibacterium lactofermentum is coupled transcriptionally to the dmdR gene," *Gene,* 177:103-107 (1996) |
| Z49824 | orfl ; sigB | ?; SigB sigma factor | Oguiza, J.A. et at "Multiple sigma factor genes in Brevibacterium lactofermentum: Characterization of sigA and sigB," *J. Bacteriol.,* 178(2):550-553 (1996) |
| Z66534 | | Transposase | Correia, A. et al. "Cloning and characterization of an IS-like element present in the genome of Brevibacterium lactofermentum ATCC 13869," *Gene,* 170(1):91-94 (1996) |
| ¹ A sequence for this gene was published in the indicated reference. However, the sequence obtained by the inventors of the present application is significantly longer than the published version. It is believed that the published version relied on an incorrect start codon, and thus represents only a fragment of the actual coding region. | | | |

**TABLE 3: Corynebacterium and Brevibacterium Strains Which May be Used in the Practice of the Invention**

| **Genus** | **species** | **ATCC** | **FERM** | **NRRL** | **CECT** | **NCIMB** | **CBS** | **NCTC** | **DSMZ** |
|---|---|---|---|---|---|---|---|---|---|
| Brevibacterium | ammoniagenes | 21054 | | | | | | | |
| Brevibacterium | ammoniagenes | 19350 | | | | | | | |
| Brevibacterium | ammoniagenes | 19351 | | | | | | | |
| Brevibacterium | ammoniagenes | 19352 | | | | | | | |
| Brevibacterium | ammoniagenes | 19353 | | | | | | | |
| Brevibacterium | ammoniagenes | 19354 | | | | | | | |
| Brevibacterium | ammoniagenes | 19355 | | | | | | | |
| Brevibacterium | ammoniagenes | 19356 | | | | | | | |
| Brevibacterium | ammoniagenes | 21055 | | | | | | | |
| Brevibacterium | ammoniagenes | 21077 | | | | | | | |
| Brevibacterium | ammoniagenes | 21553 | | | | | | | |
| Brevibacterium | ammoniagenes | 21580 | | | | | | | |
| Brevibacterium | ammoniagenes | 39101 | | | | | | | |
| Brevibacterium | butanicum | 21196 | | | | | | | |
| Brevibacterium | divaricatum | 21792 | P928 | | | | | | |
| Brevibacterium | flavum | 21474 | | | | | | | |
| Brevibacterium | flavum | 21129 | | | | | | | |
| Brevibacterium | flavum | 21518 | | | | | | | |
| Brevibacterium | flavum | | | B11474 | | | | | |
| Brevibacterium | flavum | | | B11472 | | | | | |
| Brevibacterium | flavum | 21127 | | | | | | | |
| Brevibacterium | flavum | 21128 | | | | | | | |
| Brevibacterium | flavum | 21427 | | | | | | | |
| Brevibacterium | flavum | 21475 | | | | | | | |
| Brevibacterium | flavum | 21517 | | | | | | | |
| Brevibacterium | flavum | 21528 | | | | | | | |
| Brevibacterium | flawm | 21529 | | | | | | | |
| Brevibacterium | flawm | | | B11477 | | | | | |
| Brevibacterium | flavum | | | B11478 | | | | | |
| Brevibacterium | flavum | 21127 | | | | | | | |
| Brevibacterium | flavum | | | B11474 | | | | | |
| Brevibacterium | healii | 15527 | | | | | | | |
| Brevibacterium | ketoglutamicum | 21004 | | | | | | | |
| Brevibacterium | ketoglutamicum | 21089 | | | | | | | |
| Brevibacterium | ketosoreductum | 21914 | | | | | | | |
| Brevibacterium | lactofermentum | | | | 70 | | | | |
| Brevibacterium | lactofermentum | | | | 74 | | | | |
| Brevibacterium | lactofermentum | | | | 77 | | | | |
| Brevibacterium | lactofermentum | 21798 | | | | | | | |
| Brevibacterium | lactofermentum | 21799 | | | | | | | |
| Brevibacterium | lactofermentum | 21800 | | | | | | | |
| Brevibacterium | lactofermentum | 21801 | | | | | | | |
| Brevibacterium | lactofermentum | | | B11470 | | | | | |
| Brevibacterium | lactofermentum | | | B 11471 | | | | | |
| Brevibacterium | lactofermentum | 21086 | | | | | | | |
| Brevibacterium | lactofermentum | 21420 | | | | | | | |
| Brevibacterium | lactofermentum | 21086 | | | | | | | |
| Brevibacterium | lactofermentum | 31269 | | | | | | | |
| Brevibacterium | linens | 9174 | | | | | | | |
| Brevibacterium | linens | 19391 | | | | | | | |
| Brevibacterium | linens | 8377 | | | | | | | |
| Brevibacterium | paraffinolyticum | | | | | 11160 | | | |
| Brevibacterium | spec. | | | | | | 717.73 | | |
| Brevibacterium | spec. | | | | | | 717.73 | | |
| Brevibacterium | spec. | 14604 | | | | | | | |
| Brevibacterium | spec. | 21860 | | | | | | | |
| Brevibacterium | spec. | 21864 | | | | | | | |
| Brevibacterium | spec. | 21865 | | | | | | | |
| Brevibacterium | spec. | 21866 | | | | | | | |
| Brevibacterium | spec. | 19240 | | | | | | | |
| Corynebacterium | acetoacidophilum | 21476 | | | | | | | |
| Corynebacterium | acetoacidophilum | 13870 | | | | | | | |
| Corynebacterium | acetoglutamicum | | | B11473 | | | | | |
| Corynebacterium | acetoglutamicum | | | B11475 | | | | | |
| Corynebacterium | acetoglutamicum | 15806 | | | | | | | |
| Corynebacterium | acetoglutamicum | 21491 | | | | | | | |
| Corynebacterium | acetoglutamicum | 31270 | | | | | | | |
| Corynebacterium | acetophilum | | | B3671 | | | | | |
| Corynebacterium | ammoniagenes | 6872 | | | | | | 2399 | |
| Corynebacterium | ammoniagenes | 15511 | | | | | | | |
| Corynebacterium | fujiokense | 21496 | | | | | | | |
| Corynebacterium | glutamicum | 14067 | | | | | | | |
| Corynebacterium | glutamicum | 39137 | | | | | | | |
| Corynebacterium | glutamicum | 21254 | | | | | | | |
| Corynebacterium | glutamicum | 21255 | | | | | | | |
| Corynebacterium | glutamicum | 31830 | | | | | | | |
| Corynebacterium | glutamicum | 13032 | | | | | | | |
| Corynebacterium | glutamicum | 14305 | | | | | | | |
| Corynebacterium | glutamicum | 15455 | | | | | | | |
| Corynebacterium | glutamicum | 13058 | | | | | | | |
| Corynebacterium | glutamicum | 13059 | | | | | | | |
| Corynebacterium | glutamicum | 13060 | | | | | | | |
| Corynebacterium | glutamicum | 21492 | | | | | | | |
| Corynebacterium | glutamicum | 21513 | | | | | | | |
| Corynebacterium | glutamicum | 21526 | | | | | | | |
| Corynebacterium | glutamicum | 21543 | | | | | | | |
| Corynebacterium | glutamicum | 13287 | | | | | | | |
| Corynebacterium | glutamicum | 21851 | | | | | | | |
| Corynebacterium | glutamicum | 21253 | | | | | | | |
| Corynebacterium | glutamicum | 21514 | | | | | | | |
| Corynebacterium | glutamicum | 21516 | | | | | | | |
| Corynebacterium | glutamicum | 21299 | | | | | | | |
| Corynebacterium | glutamicum | 21300 | | | | | | | |
| Corynebacterium | glutamicum | 39684 | | | | | | | |
| Corynebacterium | glutamicum | 21488 | | | | | | | |
| Corynebacterium | glutamicum | 21649 | | | | | | | |
| Corynebacterium | glutamicum | 21650 | | | | | | | |
| Corynebacterium | glutamicum | 19223 | | | | | | | |
| Corynebacterium | glutamicum | 13869 | | | | | | | |
| Corynebacterium | glutamicum | 21157 | | | | | | | |
| Corynebacterium | glutamicum | 21158 | | | | | | | |
| Corynebacterium | glutamicum | 21159 | | | | | | | |
| Corynebacterium | glutamicum | 21355 | | | | | | | |
| Corynebacterium | glutamicum | 31808 | | | | | | | |
| Corynebacterium | glutamicum | 21674 | | | | | | | |
| Corynebacterium | glutamicum | 21562 | | | | | | | |
| Corynebacterium | glutamicum | 21563 | | | | | | | |
| Corynebacterium | glutamicum | 21564 | | | | | | | |
| Corynebacterium | glutamicum | 21565 | | | | | | | |
| Corynebacterium | glutamicum | 21566 | | | | | | | |
| Corynebacterium | glutamicum | 21567 | | | | | | | |
| Corynebacterium | glutamicum | 21568 | | | | | | | |
| Corynebacterium | glutamicum | 21569 | | | | | | | |
| Corynebacterium | glutamicum | 21570 | | | | | | | |
| Corynebacterium | glutamicum | 21571 | | | | | | | |
| Corynebacterium | glutamicum | 21572 | | | | | | | |
| Corynebacterium | glutamicum | 21573 | | | | | | | |
| Corynebacterium | glutamicum | 21579 | | | | | | | |
| Corynebacterium | glutamicum | 19049 | | | | | | | |
| Corynebacterium | glutamicum | 19050 | | | | | | | |
| Corynebacterium | glutamicum | 19051 | | | | | | | |
| Corynebacterium | glutamicum | 19052 | | | | | | | |
| Corynebacterium | glutamicum | 19053 | | | | | | | |
| Corynebacterium | glutamicum | 19054 | | | | | | | |
| Corynebacterium | glutamicum | 19055 | | | | | | | |
| Corynebacterium | glutamicum | 19056 | | | | | | | |
| Corynebacterium | glutamicum | 19057 | | | | | | | |
| Corynebacterium | glutamicum | 19058 | | | | | | | |
| Corynebacterium | glutamicum | 19059 | | | | | | | |
| Corynebacterium | glutamicum | 19060 | | | | | | | |
| Corynebacterium | glutamicum | 19185 | | | | | | | |
| Corynebacterium | glutamicum | 13286 | | | | | | | |
| Corynebacterium | glutamicum | 21515 | | | | | | | |
| Corynebacterium | glutamicum | 21527 | | | | | | | |
| Corynebacterium | glutamicum | 21544 | | | | | | | |
| Corynebacterium | glutamicum | 21492 | | | | | | | |
| Corynebacterium | glutamicum | | | B8183 | | | | | |
| Corynebacterium | glutamicum | | | B8182 | | | | | |
| Corynebacterium | glutamicum | | | B12416 | | | | | |
| Corynebacterium | glutamicum | | | B12417 | | | | | |
| Corynebacterium | glutamicum | | | B12418 | | | | | |
| Corynebacterium | glutamicum | | | B11476 | | | | | |
| Corynebacterium | glutamicum | 21608 | | | | | | | |
| Corynebacterium | lilium | | P973 | | | | | | |
| Corynebacterium | nitrilophilus | 21419 | | | | 11594 | | | |
| Corynebacterium | spec. | | P4445 | | | | | | |
| Corynebacterium | spec. | | P4446 | | | | | | |
| Corynebacterium | spec. | 31088 | | | | | | | |
| Corynebacterium | spec. | 31089 | | | | | | | |
| Corynebacterium | spec. | 31090 | | | | | | | |
| Corynebacterium | spec. | 31090 | | | | | | | |
| Corynebacterium | spec. | 31090 | | | | | | | |
| Corynebacterium | spec. | 15954 | | | | | | | 20145 |
| Corynebacterium | spec. | 21857 | | | | | | | |
| Corynebacterium | spec. | 21862 | | | | | | | |
| Corynebacterium | spec. | 21863 | | | | | | | |

| | | | | | | | | | |
|---|---|---|---|---|---|---|---|---|---|
| ATCC: American Type Culture Collection, Rockville, MD, USA | | | | | | | | | |
| FERM: Fermentation Research Institute, Chiba, Japan | | | | | | | | | |
| NRRL: ARS Culture Collection, Northern Regional Research Laboratory, Peoria, IL, USA | | | | | | | | | |
| CECT: Coleccion Espanola de Cultivos Tipo, Valencia, Spain | | | | | | | | | |
| NCIMB: National Collection of Industrial and Marine Bacteria Ltd., Aberdeen, UK | | | | | | | | | |
| CBS: Centraalbureau voor Schimmelcultures, Baam, NL | | | | | | | | | |
| NCTC: National Collection of Type Cultures, London, UK | | | | | | | | | |
| DSMZ: Deutsche Sammlung von Mikroorganismen und Zellkulturen, Braunschweig, Germany | | | | | | | | | |

For reference see Sugawara, H. et al. (1993) World directory of collections of cultures of microorganisms: Bacteria, fungi and yeasts (4^{th} edn), World federation for culture collections world data center on microorganisms, Saimata, Japen.

**Table 4: Alignment Results**

| **ID #** | **length (NT)** | **Genbank Hit** | **Length** | **Accession** | **Name of Genbank Hit** | **Source of Genbank Hit** | **% homology (GAP)** | **Date of Deposit** |
|---|---|---|---|---|---|---|---|---|
| rxa00026 | 1509 | GB_RO:MMHC310M6 | 158405 | AF109906 | Mus musculus MHC class III region RD gene, partial cds; Bf, C2, G9A, NG22, G9, HSP70, HSP70, HSC70t, and smRNP genes, complete cds; G7A gene, partial cds; and unknown genes. | Mus musculus | 38,003 | 10-DEC-1998 |
| | | GB_HTG2:AC007029 | 119007 | AC007029 | Homo sapiens clone DJ0855F16, *** SEQUENCING IN PROGRESS ***, 1 unordered pieces. | Homo sapiens | 37,943 | 7-Apr-99 |
| rxa00072 | | GB_HTG2:AC007029 | 119007 | AC007029 | Homo sapiens clone DJ0855F16, *** SEQUENCING IN PROGRESS ***, 1 unordered pieces. | Homo sapiens | 37,943 | 7-Apr-99 |
| rxa00111 | 1116 | GB_BA1:SAUSIGA | 2748 | M94370 | Stigmatella aurantiaca sigma factor (sigA) gene, complete cds. | Stigmatella aurantiaca | 40,435 | 16-Aug-94 |
| | | GB_BA1:SC5B8 | 28500 | AL022374 | Streptomyces coelicolor cosmid 5B8. | Streptomyces coelicolor | 40,090 | 22-Apr-98 |
| | | GB_BA2:AE001767 | 9086 | AE001767 | Thermotoga maritima section 79 of 136 of the complete genome. | Thermotoga maritima | 35,091 | 2-Jun-99 |
| rxa00112 | 1314 | GB_EST35:AU075536 | 418 | AU075536 | AU075536 Rice shoot Oryza sativa cDNA clone S0028_22, mRNA sequence. | Oryza sativa | 39,423 | 7-Jul-99 |
| | | GB_GSS9:AQ157585 | 647 | AQ157585 | nbxb0009B16r CUGI Rice BAC Library Oryza sativa genomic clone nbxb0009B16r, genomic survey sequence. | Oryza sativa | 40,867 | 12-Sep-98 |
| | | GB_GSS14:AQ510314 | 542 | AQ510314 | nbxb0095005f CUGI Rice BAC Library Oryza sativa genomic clone nbxbOO95005f, genomic survey sequence. | Oryza sativa | 39,372 | 04-MAY-1 999 |
| rxa00133 | 936 | GB_BA 1 :SC2G5 | 38404 | AL035478 | Streptomyces coelicolor cosmid 2G5. | Streptomyces coelicolor | 41,170 | 11-Jun-99 |
| | | GB_EST7:W64291 | 515 | W64291 | md98h12.r1 Soares mouse embryo NbME13.5 14.5 Mus musculus cDNA clone IMAGE:386087 5' similar to gb:L26528 Mus musculus Rab11b mRNA, complete cds (MOUSE);, mRNA sequence. | Mus musculus | 35,306 | 10-Jun-96 |
| | | GB_PR3:AC005624 | 39594 | AC005624 | Homo sapiens chromosome 19, cosmid R30017, complete sequence. | Homo sapiens | 39,054 | 6-Sep-98 |
| rxa00137 | 1212 | GB_BA2:AF124600 | 4115 | AF124600 | Corynebacterium glutamicum chorismate synthase (aroC), shikimate kinase (aroK), and 3-dehydroquinate synthase (aroB) genes, complete cds; and putative cytoplasmic peptidase (pepQ) gene, partial cds. | Corynebacterium glutamicum | 99,867 | 04-MAY-1999 |
| | | GB_BA1:MTCY159 | 33818 | Z83863 | Mycobacterium tuberculosis H37Rv complete genome; segment 111/162. | Mycobacterium tuberculosis | 40,959 | 17-Jun-98 |
| | | GB_BA1:MT3DEHQ | 3437 | X59509 | M.tuberculosis, genes for 3-dehydroquinate synthase and 3-dehydroquinase. | Mycobacterium tuberculosis | 52,583 | 30-Jun-93 |
| rxa00139 | 834 | GB_BA1:BLELONP | 738 | X99289 | B.lactofermentum gene encoding elongation factor P. | Corynebacterium glutamicum | 100,000 | 1-Nov-97 |
| | | GB_PL1:SPAC24C9 | 38666 | Z98601 | S.pombe chromosome I cosmid c24C9. | Schizosaccharomyces pombe | 35,230 | 24-Feb-99 |
| | | GB_HTG1 :CEY102A5_1 | 110000 | Z99711 | Caenorhabditis elegans chromosome V clone Y102A5, *** SEQUENCING IN PROGRESS ***, in unordered pieces. | Caenorhabditis elegans | 37,775 | Z99711 |
| rxa00152 | 1419 | GB_BA1:MTCY277 | 38300 | Z79701 | Mycobacterium tuberculosis H37Rv complete genome; segment 65/162. | Mycobacterium tuberculosis | 58,500 | 17-Jun-98 |
| | | GB_BA1:MSGY456 | 37316 | AD000001 | Mycobacterium tuberculosis sequence from clone y456. | Mycobacterium tuberculosis | 38,913 | 03-DEC-1996 |
| | | GB_BA2:AF002133 | 15437 | AF002133 | Mycobacterium avium strain GIR10 transcriptional regulator (mav81) gene, partial cds, aconitase (acn), invasin 1 (inv1), invasin 2 (inv2), transcriptional regulator (moxR), ketoacyl-reductase (fabG), enoyl-reductase (inhA) and ferrochelatase (mav272) genes, complete cds. | Mycobacterium avium | 64,009 | 26-MAR-1998 |
| rxa00226 | 948 | GB_PR3:AC005756 | 43299 | AC005756 | Homo sapiens chromosome 19, fosmid 39347, complete sequence. | Homo sapiens | 36,209 | 02-OCT-1998 |
| | | GB_GSS5:AQ818463 | 413 | AQ818463 | HS_5250_A2_B08_SP6E RPCI-11 1 Human Male BAC Library Homo sapiens genomic clone Plate=826 Col=16 Row=C, genomic survey sequence. | Homo sapiens | 37,288 | 26-Aug-99 |
| | | GB_GSS5:AQ782337 | 832 | AQ782337 | HS_3184_B1 H12_T7C CIT Approved Human Genomic Sperm Library D Homo sapiens genomic clone Plate=3184 Col=23 Row=P, genomic survey sequence. | Homo sapiens | 35,917 | 2-Aug-99 |
| rxa00249 | 980 | GB_BA2:AF035608 | 3614 | AF035608 | Pseudomonas aeruginosa ATP sulfurylase small subunit (cysD) and ATP sulfurylase GTP-binding subunit/APS kinase (cysN) genes, complete cds. | Pseudomonas aeruginosa | 50,205 | 1-Jun-98 |
| | | GB_BA1:AB017641 | 17101 | AB017641 | Micromonospora griseorubida gene for polyketide synthase, complete cds. | Micromonospora griseorubida | 40,266 | 2-Apr-99 |
| | | GB_BA2:AF002133 | 15437 | AF002133 | Mycobacterium avium strain GIR10 transcriptional regulator (mav81) gene, partial cds, aconitase (acn), invasin 1 (inv1), invasin 2 (inv2), transcriptional regulator (moxR), ketoacyl-reductase (fabG), enoyl-reductase (inhA) and ferrochelatase (mav272) genes, complete cds. | Mycobacterium avium | 38,429 | 26-MAR-1998 |
| rxa00299 | 1101 | GB_BA2:CORCSLYS | 2821 | M89931 | Corynebacterium glutamicum beta C-S lyase (aecD) and branched-chain amino acid uptake carrier (brnQ) genes, complete cds, and hypothetical protein Yhbw (yhbw) gene, partial cds. | Corynebacterium glutamicum | 100,000 | 4-Jun-98 |
| | | GB_BA1:CGECTP | 2719 | AJ001436 | Corynebacterium glutamicum ectP gene. | Corynebacterium glutamicum | 41,143 | 20-Nov-98 |
| | | GB_BA2:AF181035 | 5922 | AF181035 | Rhodobacter sphaeroides glycogen utilization operon, complete sequence. | Rhodobacter sphaeroides | 36,701 | 7-Sep-99 |
| rxa00332 | 825 | GB_BA1:CGTHRC | 3120 | X56037 | Corynebacterium glutamicum thrC gene for threonine synthase (EC 4.2.99.2). | Corynebacterium glutamicum | 37,730 | 17-Jun-97 |
| | | GB_PAT:109078 | 3146 | 109078 | Sequence 4 from Patent WO 8809819. | Unknown. | 38,700 | 02-DEC-1994 |
| | | GB_PR3:HSJ333B15 | 73666 | AL109954 | Human DNA sequence from clone 333B15 on chromosome 20, complete sequence. | Homo sapiens | 37,203 | 23-Nov-99 |
| rxa00470 | 1392 | GB_PL2:DCPCNAM | 865 | X62977 | D.carota mRNA for proliferating cell nuclear antigen (PCNA). | Daucus carota | 37,914 | 30-Sep-99 |
| | | GB_PL2:AC006267 | 101644 | AC006267 | Arabidopsis thaliana BAC F9M13 from chromosome IV near 21.5 cM, complete sequence. | Arabidopsis thaliana | 36,158 | 27-Apr-99 |
| | | GB_BA1:TT10SARNA | 721 | Y15063 | Thermus thermophilus 10Sa RNA gene. | Thermus thermophilus | 39,494 | 18-Aug-98 |
| rxa00471 | 813 | GB_BA1:SERERYAA | 11219 | M63676 | S.erythraea first ORF of eryA gene, complete cds. | Saccharopolyspora erythraea | 38,781 | 26-Apr-93 |
| | | GB_PAT:AR049367 | 11219 | AR049367 | Sequence 1 from patent US 5824513. | Unknown. | 38,781 | 29-Sep-99 |
| | | GB_BA1:SERERYAA | 11219 | M63676 | S.erythraea first ORF of eryA gene, complete cds. | Saccharopolyspora erythraea | 38,205 | 26-Apr-93 |
| rxa00499 | 1404 | GB_PR4:AC007206 | 42732 | AC007206 | Homo sapiens chromosome 19, cosmid R27370, complete sequence. | Homo sapiens | 34,982 | 4-Apr-99 |
| | | GB_EST26:Al344735 | 462 | A1344735 | qp05a10.x1 NCI_CGAP_Kid5 Homo sapiens cDNA clone IMAGE:1917114 3' similar to gb:M15800 T-LYMPHOCYTE MATURATION-ASSOCIATED PROTEIN (HUMAN);, mRNA sequence. | Homo sapiens | 42,675 | 2-Feb-99 |
| | | GB_PR4:AC006479 | 161837 | AC006479 | Homo sapiens clone DJ1051J04, complete sequence. | Homo sapiens | 38,462 | 11-Nov-99 |
| rxa00500 | 798 | GB_PR4:AC006111 | 190825 | AC006111 | Homo sapiens chromosome 16 clone RPCI-11_461A8, complete sequence. | Homo sapiens | 40,736 | 3-Jul-99 |
| | | GB_HTG2:AF128834 | 196589 | AF128834 | Homo sapiens chromosome 8 clone BAC 57G24 map 8p12, *** SEQUENCING IN PROGRESS ***, in unordered pieces. | Homo sapiens | 34,062 | 28-Feb-99 |
| | | GB_HTG2:AF128834 | 196589 | AF128834 | Homo sapiens chromosome 8 clone BAC 57G24 map 8p12, *** SEQUENCING IN PROGRESS ***, in unordered pieces. | Homo sapiens | 34,062 | 28-Feb-99 |
| rxa00501 | 630 | GB_BA1:086429 | 5925 | D86429 | Saccharopolyspora rectivirgula gene for beta-galactosidase, complete cds. | Saccharopolyspora rectivirgula | 53,871 | 09-DEC-1998 |
| | | GB_HTG1:HS1099D15 | 1301 | AL035456 | Homo sapiens chromosome 20 clone RP5-1099D15, *** SEQUENCING IN PROGRESS ***, in unordered pieces. | Homo sapiens | 33,546 | 23-Nov-99 |
| | | GB_HTG1:HS1099D15 | 1301 | AL035456 | Homo sapiens chromosome 20 clone RP5-1099D15, *** SEQUENCING IN PROGRESS ***, in unordered pieces. | Homo sapiens | 33,546 | 23-Nov-99 |
| rxa00502 | 1155 | GB_BA2:U00015 | 42325 | U00015 | Mycobacterium leprae cosmid B1620. | Mycobacterium leprae | 34,783 | 01-MAR-1994 |
| | | GB_BA1:U00020 | 36947 | U00020 | Mycobacterium leprae cosmid B229. | Mycobacterium leprae | 34,900 | 01-MAR-1994 |
| | | GB_HTG1:HS179I15 | 210672 | Z84464 | Homo sapiens chromosome 13 clone 179115, *** SEQUENCING IN PROGRESS ***, in unordered pieces. | Homo sapiens | 32,898 | 22-Jan-97 |
| rxa00566 | 729 | GB_BA1:MTV008 | 63033 | AL021246 | Mycobacterium tuberculosis H37Rv complete genome; segment 108/162. | Mycobacterium tuberculosis | 37,011 | 17-Jun-98 |
| | | GB_BA2:AF071885 | 2188 | AF071885 | Streptomyces coelicolor ATP-dependent Clp protease proteolytic subunit 1 (clpP1) and ATP-dependent Clp protease proteolytic subunit 2 (clpP2) genes, complete cds; and ATP-dependent Clp protease ATP-binding subunit Clpx (clpX) gene, partial cds. | Streptomyces coelicolor | 62,963 | 29-Jun-99 |
| | | GB_BA2:AF013216 | 15742 | AF013216 | Myxococcus xanthus Dog (dog), isocitrate lyase (icl), Mls (mls), Ufo (ufo), fumarate hydratase (fhy), and proteosome major subunit (clpP) genes, complete cds; and acyl-CoA oxidase (aco) gene, partial cds. | Myxococcus xanthus | 54,683 | 28-Jan-98 |
| rxa00567 | 714 | GB_BA1:MTV008 | 63033 | AL021246 | Mycobacterium tuberculosis H37Rv complete genome, segment 108/162. | Mycobacterium tuberculosis | 42,090 | 17-Jun-98 |
| | | GB_BA1:CGBPHI16 | 962 | Y12472 | C.glutamicum DNA, attachment site bacteriophage Phi-16. | Corynebacterium glutamicum | 40,000 | 05-MAR-1999 |
| | | GB_BA1:ECOCLPPA | 1236 | J05534 | Escherichia coli ATP-dependent clp protease proteolytic component (clpP) gene, complete cds. | Escherichia coli | 52,119 | 26-Apr-93 |
| rxa00621 | 906 | GB_EST1:D36491 | 360 | D36491 | CELK033GYF Yuji Kohara unpublished cDNA Caenorhabditis elegans cDNA clone yk33g11 5', mRNA sequence. | Caenorhabditis elegans | 40,390 | 8-Aug-94 |
| | | GB_IN2:CELC16A3 | 34968 | U41534 | Caenorhabditis elegans cosmid C16A3. | Caenorhabditis elegans | 35,477 | 18-MAY-1999 |
| | | GB_HTG3:AC009311 | 160198 | AC009311 | Homo sapiens clone NH0311 L03, *** SEQUENCING IN PROGRESS ***, 3 unordered pieces. | Homo sapiens | 38,636 | 13-Aug-99 |
| rxa00622 | 1539 | GB_BA1:AB004795 | 3039 | AB004795 | Pseudomonas sp. gene for dipeptidyl aminopeptidase, complete cds. | Pseudomonas sp. | 54,721 | 5-Feb-99 |
| | | GB_BA 1 :MBOPII | 2392 | D38405 | Moraxella lacunata gene for protease II, complete cds. | Moraxella lacunata | 50,167 | 8-Feb-99 |
| | | GB_IN2:AF078916 | 2960 | AF078916 | Trypanosoma brucei brucei oligopeptidase B (opb) gene, complete cds. | Trypanosoma brucei brucei | 48,076 | 08-OCT-1 999 |
| rxa00650 | 759 | GB_BA2:AF161327 | 2021 | AF161327 | Corynebacterium diphtheriae histidine kinase ChrS (chrs) and response regulator ChrA (chrA) genes, complete cds. | Corynebacterium diphtheriae | 51,319 | 9-Sep-99 |
| | | GB_PL2:ATAC006533 | 99188 | AC006533 | Arabidopsis thaliana chromosome II BAC F20M17 genomic sequence, complete sequence. | Arabidopsis thaliana | 38,051 | 26-MAY-1999 |
| | | GB_PL2:ATAC006533 | 99188 | AC006533 | Arabidopsis thaliana chromosome II BAC F20M17 genomic sequence, complete sequence. | Arabidopsis thaliana | 35,403 | 26-MAY-1999 |
| rxa00675 | 915 | GB_BA1:SC3C8 | 33095 | AL023861 | Streptomyces coelicolor cosmid 3C8. | Streptomyces coelicolor | 36,836 | 15-Jan-99 |
| | | GB_PR3:AC005736 | 215441 | AC005736 | Homo sapiens chromosome 16, BAC clone 462G18 (LANL), complete sequence. | Homo sapiens | 42,027 | 01-OCT-1998 |
| | | GB_IN2:AC005719 | 188357 | AC005719 | Drosophila melanogaster, chromosome 2L, region 38A5-38B4, BAC clone BACR48M05, complete sequence. | Drosophila melanogaster | 35,531 | 27-OCT-1 999 |
| rxa00689 | 1614 | GB_PAT:E07294 | 2975 | E07294 | genomic DNA encoding dehydrogenase of Bacillus stearothermophilus. | Bacillus stearothermophilus | 45,677 | 29-Sep-97 |
| | | GB_BA1:BACALDHT | 1975 | D13846 | B. stearothermophilus aldhT gene for aldehyde dehydrogenase, complete cds. | Bacillus stearothermophilus | 45,677 | 20-Feb-99 |
| | | GB_BA2:PPU96338 | 5276 | U96338 | Pseudomonas putida NCIMB 9866 plasmid pRA4000 p-cresol degradative pathway genes, p-hydroxybenzaldehyde dehydrogenase (pchA), p-cresol melhylhydroxylase, cytochrome subunit precursor (pchC), unknown (pchX) and p-cresol methylhydroxylase, flavoprotein subunit (pchF) genes, complete cds. | Pseudomonas putida | 44,317 | 13-MAY-1999 |
| rxa00715 | 918 | GB_EST30:Al647104 | 218 | Al647104 | vn15c01.y1 Stratagene mouse heart (#937316) Mus musculus cDNA clone IMAGE:1021248 5', mRNA sequence. | Mus musculus | 58,511 | 29-Apr-99 |
| | | GB_EST17:AA636159 | 447, | AA636159 | vn15c01.r1 Stratagene mouse heart (#937316) Mus musculus cDNA clone IMAGE:1021248 5', mRNA sequence. | Mus musculus | 41,195 | 22-OCT-1997 |
| | | GB_EST10:AA184468 | 583 | AA184468 | mt52h05.r1 Stratagene mouse embryonic carcinoma (#937317) Mus musculus cDNA clone IMAGE:633561 5' similar to gb:D10918 Mouse mRNA for ubiquitin like protein, partial sequence (MOUSE);, mRNA sequence. | Mus musculus | 40,426 | 12-Feb-97 |
| rxa00744 | 1065 | GB_HTG3:AC009855 | 167592 | AC009855 | Homo sapiens clone 1_C_5,*** SEQUENCING IN PROGRESS ***, 13 unordered pieces. | Homo sapiens | 36,673 | 3-Sep-99 |
| | | GB_HTG3:AC009855 | 167592 | AC009855 | Homo sapiens clone 1_C_5,*** SEQUENCING IN PROGRESS ***, 13 unordered pieces. | Homo sapiens | 36,673 | 3-Sep-99 |
| | | GB_PR4:AC005082 | 169739 | AC005082 | Homo sapiens clone RG271G13. complete sequence. | Homo sapiens | 39,557 | 8-Sep-99 |
| rxa00756 | 1119 | GB_BA1 :MLCB596 | 38426 | AL035472 | Mycobacterium leprae cosmid B596. | Mycobacterium leprae | 54,562 | 27-Aug-99 |
| | | GB_GSS12:AQ368028 | 652 | AQ368028 | toxb0001N11r CUGI Tomato BAC Library Lycopersicon esculentum genomic clone toxb0001 N11r, genomic survey sequence. | Lycopersicon esculentum | 42,657 | 5-Feb-99 |
| | | GB_HTG3:AC008067 | 151242 | AC008067 | Homo sapiens clone NH0303104, *** SEQUENCING IN PROGRESS ***, 2 unordered pieces. | Homo sapiens | 37,239 | 8-Sep-99 |
| rxa00773 | 1266 | GB_BA1:MLU15182 | 40123 | U15182 | Mycobacterium leprae cosmid B2266. | Mycobacterium leprae | 36,616 | 09-MAR-1995 |
| | | GB_BA1:MSGL611CS | 37769 | L78822 | Mycobacterium leprae cosmid L611 DNA sequence. | Mycobacterium leprae | 35,714 | 15-Jun-96 |
| | | GB_GSS14:AQ578181 | 728 | AQ578181 | nbxb0083P08r CUGI Rice BAC Library Oryza sativa genomic clone nbxb00B3P08r, genomic survey sequence. | Oryza sativa | 39,246 | 2-Jun-99 |
| rxa00793 | 1299 | GB_GSS5:AQ769737 | 519 | AQ769737 | HS_3160_A2_G04_T7C CIT Approved Human Genomic Sperm Library D Homo sapiens genomic clone Plate=3160 Col=8 Row=M, genomic survey sequence. | Homo sapiens | 37,765 | 28-Jul-99 |
| | | GB_BA1:RTU08434 | 2400 | U08434 | Rhizobium trifolii orotate phosphoribosyltransferase (pyrE) and fructokinase (frk) genes, complete cds. | Rhizobium trifolii | 40,700 | 16-Apr-97 |
| | | GB_EST31:F33810 0 | 243 | F33810 | HSPD27491 HM3 Homo sapiens cDNA clone s3000041 E12, mRNA sequence. | Homo sapiens | 41,564 | 13-MAY-1999 |
| rxa00820 | 486 | GB_PR4:AC005868 | 96180 | AC005868 | Homo sapiens 12q24.2 PAC RPCI5-944M2 (Roswell Park Cancer Institute Human PAC Library) complete sequence. | Homo sapiens | 32,298 | 27-Feb-99 |
| | | GB_EST8:AA000903 | 396 | AA000903 | mg38b04.r1 Soares mouse embryo NbME13.514.5 Mus musculus cDNA clone IMAGE:426031 5', mRNA sequence. | Mus musculus | 42,045 | 18-Jul-96 |
| | | GB_EST25:AI317789 | 696 | AI317789 | uj20g09.y1 Sugano mouse embryo mewa Mus musculus cDNA clone IMAGE:1920544 5' similar to WP:C13C4.5 CE08130 SUGAR TRANSPORTER mRNA sequence. | Mus musculus | 38,557 | 17-DEC-1998 |
| rxa00833 | 618 | GB_PH:BPH6589 | 41489 | AJ006589 | Bacteriophage phi-C31 complete genome. | Bacteriophage phi-C31 | 41,806 | 29-Apr-99 |
| | | GB_HTG2:AC006887 | 215801 | AC006887 | Caenorhabditis elegans clone Y59H11, *** SEQUENCING IN PROGRESS ***, 3 unordered pieces. | Caenorhabditis elegans | 35,798 | 24-Feb-99 |
| | | GB_HTG2:AC006887 | 215801 | AC006887 | Caenorhabditis elegans clone Y59H11, *** SEQUENCING IN PROGRESS ***, 3 unordered pieces. | Caenorhabditis elegans | 35,798 | 24-Feb-99 |
| rxa00844 | 957 | GB_GSS15:AQ605195 | 459 | AQ605195 | HS_2136_B1 C12 T7C CIT Approved Human Genomic Sperm Library D Homo sapiens genomic clone Plate=2136 Col=23 Row=F, genomic survey sequence. | Homo sapiens | 38,074 | 10-Jun-99 |
| | | GB_HTG1:CNS00M8S | 214599 | AL079302 | Homo sapiens chromosome 14 clone R-1089B7, *** SEQUENCING IN PROGRESS ***, in ordered pieces. | Homo sapiens | 38,120 | 15-OCT-1999 |
| | | G8_HTG1:CNS00M8S | 214599 | AL079302 | Homo sapiens chromosome 14 clone R-1089B7,*** SEQUENCING IN PROGRESS ***, in ordered pieces. | Homo sapiens | 38,120 | 15-OCT-1999 |
| rxa00866 | 1066 | GB_BA1:CGORF4GEN | 2398 | X95649 | C.glutamicum ORF4 gene. | Corynebacterium glutamicum | 99,273 | 10-MAR-1998 |
| | | GB_BA1:BLDAPAB | 3572 | Z21502 | B.lactofermentum dapA and dapB genes for dihydrodipicolinate synthase and dihydrodipicolinate reductase. | Corynebacterium glutamicum | 99,301 | 16-Aug-93 |
| | | GB_PAT:E14517 | 1411 | E14517 | DNA encoding Brevibacterium dihydrodipicolinic acid reductase. | Corynebacterium glutamicum | 99,659 | 28-Jul-99 |
| rxa00877 | 1788 | GB_PAT:192050 | 567 | 192050 | Sequence 17 from patent US 5726299. | Unknown. | 62,787 | 01-DEC-1998 |
| | | GB_PAT:178760 | 567 | 178760 | Sequence 16 from patent US 5693781. | Unknown. | 62,787 | 3-Apr-98 |
| | | GB_BA2:AE000426 | 10240 | AE000426 | Escherichia coli K-12 MG1655 section 316 of 400 of the complete genome. | Escherichia coli | 36,456 | 12-Nov-98 |
| rxa00903 | 733 | GB_BA2:AE001598 | 11136 | AE001598 | Chlamydia pneumoniae section 14 of 103 of the complete genome. | Chlamydophila pneumoniae | 32,782 | 08-MAR-1999 |
| | | GB_PL2:AF079370 | 2897 | AF079370 | Kluyveromyces lactis invertase (INV1) gene, complete cds. | Kluyveromyces lactis | 35,849 | 4-Aug-99 |
| | | GB_BA2:AE001598 | 11136 | AE001598 | Chlamydia pneumoniae section 14 of 103 of the complete genome. | Chlamydophila pneumoniae | 40,138 | 08-MAR-1999 |
| rxa00905 | 924 | GB_PR2:HSQ15C24 | 73192 | AJ239325 | Homo sapiens chromosome 21 from cosmids LLNLc116 1C16 and LLNLc116 15C24 map 21q22.3 region D21S171-LA161, complete sequence. | Homo sapiens | 35,076 | 28-Sep-99 |
| | | GB_GSS4:AQ691923 | 446 | AQ691923 | HS_5400_B2_G04_SP6E RPCI-11 Human Male BAC Library Homo sapiens genomic clone Plate=976 Col=8 Row=N, genomic survey sequence. | Homo sapiens | 33,500 | 6-Jul-99 |
| | | GB_EST37:AI967802 | 479 | AI967802 | Ljirnpest12-930-d6 Ljirnp Lambda HybriZap two-hybrid library Lotus japonicus cDNA clone LP930-12-d6 5' similar to 60S ribosomal protein L7A, mRNA sequence. | Lotus japonicus | 41,127 | 24-Aug-99 |
| rxa00906 | 627 | GB_PAT:I78750 | 588 | 178750 | Sequence 6 from patent US 5693781. | Unknown. | 97,071 | 3-Apr-98 |
| | | GB_PAT:I92039 | 588 | 192039 | Sequence 6 from patent US 5726299. | Unknown. | 97,071 | 01-DEC-1998 |
| | | GB_PR3:HS929C8 | 139190 | AL020994 | Human DNA sequence from clone 929C8 on chromosome 22q12.1-12.3 Contains CA repeat, GSS, STS, complete sequence. | Homo sapiens | 39,016 | 23-Nov-99 |
| rxa00907 | 246 | GB_PAT:178750 | 588 | 178750 | Sequence 6 from patent US 5693781. | Unknown. | 97,561 | 3-Apr-98 |
| | | GB_PAT:192039 | 588 | 192039 | Sequence 6 from patent US 5726299. | Unknown. | 97,561 | 01-DEC-1998 |
| | | GB_PAT:178750 | 588 | 178750 | Sequence 6 from patent US 5693781. | Unknown. | 37,222 | 3-Apr-98 |
| rxa00961 | 455 | GB_BA1:AB032799 | 9077 | AB032799 | Chromobacterium violaceum violacein biosynthetic gene cluster (vio A, vio B, vio C, vio D), complete cds. | Chromobacterium violaceum | 39,868 | 02-OCT-1999 |
| | | GB_BA2:AF172851 | 10094 | AF172851 | Chromobacterium violaceum violacein biosynthetic gene cluster, complete sequence. | Chromobacterium violaceum | 42,760 | 30-Aug-99 |
| | | GB_BA1:AB032799 | 9077 | AB032799 | Chromobacterium violaceum violacein biosynthetic gene cluster (vio A, vio B, vio C, vio D), complete cds. | Chromobacterium violaceum | 39,551 | 02-OCT-1999 |
| rxa00982 | 1629 | GB_BA1:BLARGS | 2501 | Z21501 | B.lactofermentum argS and lysA genes for arginyl-tRNA synthetase and diaminopimelate decarboxylase (partial). | Corynebacterium glutamicum | 39,003 | 28-DEC-1993 |
| | | GB_BA1:CGXLYSA | 2344 | X54740 | Corynebacterium glutamicum argS-lysA operon gene for the upstream region of the arginyl-tRNA synthetase and diaminopimelate decarboxylase (EC 4.1.1.20). | Corynebacterium glutamicum | 41,435 | 30-Jun-93 |
| | | GB_PAT:E14508 | 3579 | E14508 | DNA encoding Brevibacterium diaminopimelic acid decarboxylase and arginyl-tRNA synthase. | Corynebacterium glutamicum | 40,566 | 28-Jul-99 |
| rxa00983 | 1599 | GB_HTG2:AC008152 | 24000 | AC008152 | Leishmania major chromosome 35 clone L7936 strain Friedlin, *** SEQUENCING IN PROGRESS ***, 4 unordered pieces. | Leishmania major | 38,658 | 28-Jul-99 |
| | | GB_HTG2:AC008152 | 24000 | AC008152 | Leishmania major chromosome 35 clone L7936 strain Friedlin, *** SEQUENCING IN PROGRESS ***, 4 unordered pieces. | Leishmania major | 38,658 | 28-Jul-99 |
| | | GB_HTG3:AC008648 | 87249 | AC008648 | Homo sapiens chromosome 5 clone CIT978SKB_186E14, *** SEQUENCING IN PROGRESS ***, 22 unordered pieces. | Homo sapiens | 36,102 | 3-Aug-99 |
| rxa00984 | 440 | GB_BA1:MVINED | 3098 | D01045 | Micromonospora viridifaciens DNA for nedR protein and neuraminidase, complete cds. | Micromonospora viridifaciens | 59,226 | 2-Feb-99 |
| | | GB_PAT:E02375 | 1881 | E02375 | Neuraminidase gene. | Micromonospora viridifaciens | 59,226 | 29-Sep-97 |
| | | GB_PR4:HUAC004513 | 101311 | AC004513 | Homo sapiens Chromosome 16 BAC clone CIT987SK-A-926E7, complete sequence. | Homo sapiens | 41,204 | 23-Nov-99 |
| rxa01014 | 2724 | GB_BA1:MTV008 | 63033 | AL021246 | Mycobacterium tuberculosis H37Rv complete genome; segment 108/162. | Mycobacterium tuberculosis | 56,167 | 17-Jun-98 |
| | | GB_BA1:STMAMPEPN | 2849 | L23172 | Streptomyces lividans aminopeptidase N gene, complete cds. | Streptomyces lividans | 57,067 | 18-MAY-1994 |
| | | GB_BA1:SC7H2 | 42655 | AL109732 | Streptomyces coelicolor cosmid 7H2. | Streptomyces coelicolor A3(2) | 37,551 | 2-Aug-99 |
| rxa01059 | 732 | GB_HTG3:AC008154 | 172241 | AC008154 | Homo sapiens chromosome 7, *** SEQUENCING IN PROGRESS ***, 26 unordered pieces. | Homo sapiens | 39,499 | 8-Sep-99 |
| | | GB_HTG3:AC008154 | 172241 | AC008154 | Homo sapiens chromosome 7, *** SEQUENCING IN PROGRESS ***, 26 unordered pieces. | Homo sapiens | 39,499 | 8-Sep-99 |
| | | GB_EST32:Al756574 | 299 | Al756574 | ea02f10.y1 Eimeria M5-6 Merozoite stage Eimeria tenella cDNA 5', mRNA sequence. | Eimeria tenella | 37,793 | 23-Jun-99 |
| rxa01073 | 954 | GB_BA1:BACOUTB | 1004 | M15811 | Bacillus subtilis outB gene encoding a sporulation protein, complete cds. | Bacillus subtilis | 53,723 | 26-Apr-93 |
| | | GB_PR4:AC007938 | 167237 | AC007938 | Homo sapiens clone UWGC:djs201 from 7q31, complete sequence. | Homo sapiens | 34,322 | 1-Jul-99 |
| | | GB_PL2:ATAC006282 | 92577 | AC006282 | Arabidopsis thaliana chromosome II BAC F13K3 genomic sequence, complete sequence. | Arabidopsis thaliana | 36,181 | 13-MAR-1999 |
| rxa01120 | 1401 | GB_BA1:MTV008 | 63033 | AL021246 | Mycobacterium tuberculosis H37Rv complete genome; segment 108/162. | Mycobacterium tuberculosis | 36,715 | 17-Jun-98 |
| | | GB_BA1:CAJ10321 | 6710 | AJ010321 | Caulobacter crescentus partial tig gene and clpP, cicA, clpX, Ion genes. | Caulobacter crescentus | 63,311 | 01-OCT-1998 |
| | | GB_BA2:AF150357 | 4440 | AF150957 | Azospirillum brasilense trigger factor (tig), heat-shock protein ClpP (clpP), and heat-shock protein ClpX (clpX) genes, complete cds; and Lon protease (Ion) gene, partial cds. | Azospirillum brasilense | 60,613 | 7-Jun-99 |
| rxa01147 | 1383 | GB_PR3:HS408N23 | 97916 | Z98048 | Human DNA sequence from PAC 408N23 on chromosome 22q13. Contains HIP, HSC70-INTERACTING PROTEIN (PROGESTERONE RECEPTOR-ASSOCIATED P48 PROTEIN), ESTs and STS. | Homo sapiens | 34,567 | 23-Nov-99 |
| | | GB_BA2:AE001227 | 26849 | AE001227 | Treponema pallidum section 43 of 87 of the complete genome. | Treponema pallidum | 37,564 | 16-Jul-98 |
| | | GB_PR3:HS408N23 | 97916 | Z98048 | Human DNA sequence from PAC 408N23 on chromosome 22q13. Contains HIP, HSC70-INTERACTING PROTEIN (PROGESTERONE RECEPTOR-ASSOCIATED P48 PROTEIN), ESTs and STS. | Homo sapiens | 34,911 | 23-Nov-99 |
| rxa01151 | 958 | GB_BA1:MTCY261 | 27322 | Z97559 | Mycobacterium tuberculosis H37Rv complete genome; segment 95/162. | Mycobacterium tuberculosis | 38,789 | 17-Jun-98 |
| | | GB_HTG4:AC009849 | 114993 | AC009849 | Drosophila melanogaster chromosome 2 clone BACR07H08 (D864) RPCI-98 07.H.8 map 31B-31C strain y; cn bw sp, *** SEQUENCING IN PROGRESS ***, 55 unordered pieces. | Drosophila melanogaster | 39,213 | 25-OCT-1 999 |
| | | GB_HTG4:AC009849 | 114993 | AC009849 | Drosophila melanogaster chromosome 2 clone BACR07H08 (D864) RPCI-98 07.H.8 map 31B-31C strain y cn bw sp, *** SEQUENCING IN PROGRESS ***, 55 unordered pieces. | Drosophila melanogaster | 39,213 | 25-OCT-1999 |
| rxa01161 | 1260 | GB_BA2:AF176799 | 2943 | AF176799 | Lactobacillus pentosus PepQ (pepQ) and catabolite control protein A (ccpA) genes, complete cds. | Lactobacillus pentosus | 37,043 | 5-Sep-99 |
| | | GB BA2:AF012084 | 3082 | AF012084 | Lactobacillus helveticus prolidase (pepQ) gene, complete cds. | Lactobacillus helveticus | 46,796 | 1-Jul-98 |
| | | GB_EST32:AR28955 | 611 | A1728955 | BNLGHi12114 Six-day Cotton fiber Gossypium hirsutum cDNA 5' similar to (AC004481) putative permease [Arabidopsis thaliana], mRNA sequence. | Gossypium hirsutum | 37,647 | 11-Jun-99 |
| rxa01181 | 980 | GB_BA1:MLCB22 | 40281 | Z98741 | Mycobacterium leprae cosmid B22. | Mycobacterium leprae | 61,570 | 22-Aug-97 |
| | | GB_BA1:MTCY190 | 34150 | Z70283 | Mycobacterium tuberculosis H37Rv complete genome; segment 98/162. | Mycobacterium tuberculosis | 60,434 | 17-Jun-98 |
| | | GB_BA1:SC5F7 | 40024 | AL096872 | Streptomyces coelicolor cosmid 5F7. | Streptomyces coelicolor A3(2) | 57,011 | 22-Jul-99 |
| rxa01182 | 516 | GB_HTG1 :CEY116A8_2 | 110000 | Z98858 | Caenorhabditis elegans chromosome IV clone Y116A8, *** SEQUENCING IN PROGRESS ***, in unordered pieces. | Caenorhabditis elegans | 34,843 | 26-Oct-99 |
| | | GB_HTG1 :CEY116A8_2 | 110000 | Z98858 | Caenorhabditis elegans chromosome IV clone Y116A8, *** SEQUENCING IN PROGRESS ***, in unordered pieces. | Caenorhabditis elegans | 34,843 | 26-Oct-99 |
| | | GB_IN1 :CEY116A8C | 260341 | AL117204 | Caenorhabditis elegans cosmid Y116A8C, complete sequence. | Caenorhabditis elegans | 34,843 | 19-Nov-99 |
| rxa01189 | 732 | GB_BA1:D90915 | 130001 | D90915 | Synechocystis sp. PCC6803 complete genome, 17/27, 2137259-2267259. | Synechocystis sp. | 36,538 | 7-Feb-99 |
| | | GB_BA1 :D90915 | 130001 | D90915 | Synechocystis sp. PCC6803 complete genome, 17/27, 2137259-2267259. | Synechocystis sp. | 34,512 | 7-Feb-99 |
| | | GB_HTG3:AC010515 | 41038 | AC010515 | Homo sapiens chromosome 19 clone LLNL-R_249H9, *** SEQUENCING IN PROGRESS ***, 31 unordered pieces. | Homo sapiens | 33,564 | 15-Sep-99 |
| rxa01192 | 681 | GB_OM:CFP180RRC | 5425 | X87224 | Canis familiaris mRNA for ribosome receptor, p180. | Canis familiaris | 41,229 | 22-Jan-99 |
| | | GB_OM:CFP180RRC | 5425 | X87224 | Canis familiaris mRNA for ribosome receptor, p180. | Canis familiaris | 38,187 | 22-Jan-99 |
| rxa01214 | 1614 | GB_IN1:CEY47D3A | 199814 | AL117202 | Caenorhabditis elegans cosmid Y47D3A, complete sequence. | Caenorhabditis elegans | 36,604 | 19-Nov-99 |
| | | GB_PR4:AC006039 | 176257 | AC006039 | Homo sapiens clone NH0319F03, complete sequence. | Homo sapiens | 34,984 | 05-MAY-1999 |
| | | GB_PR4:AC006039 | 176257 | AC006039 | Homo sapiens clone NH0319F03, complete sequence. | Homo sapiens | 35,951 | 05-MAY-1999 |
| rxa01224 | 1146 | GB_EST22:AI070047 | 479 | AI070047 | UI-R-C1-In-f-08-0-UI.s1 UI-R-C1 Rattus norvegicus cDNA clone UI-R-C1-In-f-08-0-UI 3', mRNA sequence. | Rattus norvegicus | 36,975 | 5-Jul-99 |
| | | GB_RO:S75965 | 625 | S75965 | THP=Tamm-Horsfall protein {promoter} [rats, Genomic, 625 nt]. | Rattus sp. | 34,400 | 27-Jul-95 |
| | | GB_EST5:H96951 | 459 | H96951 | yu01g03.r1 Soares_pineal_gland_N3HPG Homo sapiens cDNA clone IMAGE:232564 5', mRNA sequence. | Homo sapiens | 32,969 | 11-DEC-1995 |
| rxa01250 | 588 | GB_PL1:NEULCCB | 2656 | M18334 | N.crassa (strain TS) laccase gene, complete cds. | Neurospora crassa | 44,330 | 03-MAY-1994 |
| | | GB_OV:MTRACOMPL | 16714 | Y16884 | Rhea americana complete mitochondrial genome. | Mitochondrion Rhea americana | 35,094 | 19-Jul-99 |
| | | GB_OV:AF090339 | 16704 | AF090339 | Rhea americana mitochondrion, complete genome. | Mitochondrion Rhea americana | 35,094 | 27-MAY-1999 |
| rxa01277 | 2127 | GB_PL2:AF111709 | 52684 | AF111709 | Oryza sativa subsp. indica Retrosat 1 retrotransposon and Ty3-Gypsy type Retrosat 2 retrotransposon, complete sequences; and unknown genes. | Oryza sativa subsp. indica | 37,410 | 26-Apr-99 |
| | | GB_IN1:CELZC250 | 34372 | AF003383 | Caenorhabditis elegans cosmid ZC250. | Caenorhabditis elegans | 35,506 | 14-MAY-1997 |
| | | GB_EST1:Z14808 | 331 | Z14808 | CEL5E4 Chris Martin sorted cDNA library Caenorhabditis elegans cDNA clone cm5e4 5', mRNA sequence. | Caenorhabditis elegans | 36,890 | 19-Jun-97 |
| rxa01302 | 576 | GB_BA1:MTCI65 | 34331 | Z95584 | Mycobacterium tuberculosis H37Rv complete genome; segment 50/162. | Mycobacterium tuberculosis | 59,298 | 17-Jun-98 |
| | | GB_BA1:MSGY348 | 40056 | AD000020 | Mycobacterium tuberculosis sequence from clone y348. | Mycobacterium tuberculosis | 59,227 | 10-DEC-1996 |
| | | GB_BA1:SC5C7 | 41906 | AL031515 | Streptomyces coelicolor cosmid 5C7. | Streptomyces coelicolor | 39,261 | 7-Sep-98 |
| rxa01303 | 1458 | GB_BA1:TTAJ5043 | 837 | AJ225043 | Thermus thermophilus partial narK gene. | Thermus thermophilus | 55,245 | 18-Jun-98 |
| | | GB_PL2:AC010675 | 84723 | AC010675 | Arabidopsis thaliana chromosome I BAC T17F3 genomic sequence, complete sequence. | Arabidopsis thaliana | 37,058 | 11-Nov-99 |
| | | GB_GSS9:AQ170862 | 518 | AQ170862 | HS_3165_B2_F03_T7 CIT Approved Human Genomic Sperm Library D Homo sapiens genomic clone Plate=3165 Col=6 Row=L, genomic survey sequence. | Homo sapiens | 38,610 | 17-OCT-1998 |
| rxa01308 | 2503 | GB_BA1:D90757 | 17621 | D90757 | Escherichia coli genomic DNA. (27.3-27.7 min). | Escherichia coli | 55,445 | 7-Feb-99 |
| | | GB_BA1:D90787 | 15942 | D90787 | E.coli genomic DNA, Kohara clone #276(33.0-33.3 min.). | Escherichia coli | 36,815 | 29-MAY-1 997 |
| | | GB_BA1:D90758 | 13860 | D90758 | Escherichia coli genomic DNA. (27.6-27.9 min). | Escherichia coli | 54,942 | 7-Feb-99 |
| rxa01309 | 824 | GB_BA1:SCJ12 | 35302 | AL109989 | Streptomyces coelicolor cosmid J12. | Streptomyces coelicolor A3(2) | 62,423 | 24-Aug-99 |
| | | GB_BA1:BSNARYWI | 12450 | Z49884 | B.subtilis nar[G,H,I,J,K], ywi[C,D,E] and argS genes. | Bacillus subtilis | 57,447 | 24-Jun-98 |
| | | GB_BA1:BSUB0020 | 212150 | Z99123 | Bacillus subtilis complete genome (section 20 of 21): from 3798401 to 4010550. | Bacillus subtilis | 37,129 | 26-Nov-97 |
| rxa01358 | 1644 | GB_GSS11:AQ260413 | 453 | AQ260413 | CITBI-E1-251 OB12.TF CITBI-E1 Homo sapiens genomic clone 2510B12, genomic survey sequence. | Homo sapiens | 41,531 | 24-OCT-1998 |
| | | GB_EST20:AA840582 | 326 | AA840582 | vw77h07.r1 Stratagene mouse heart (#937316) Mus musculus cDNA clone IMAGE:1261021 5' similar to gb:J04181 Mouse A-X actin mRNA, complete cds (MOUSE);, mRNA sequence. | Mus musculus | 42,901 | 27-Feb-98 |
| | | GB_PAT:A39944 | 3836 | A39944 | Sequence 1 from Patent WO9421807. | unidentified | 38,764 | 05-MAR-1997 |
| rxa01385 | 2004 | GB_BA1:FVBPENTA | 2519 | M98557 | Flavobacterium sp. pentachlorophenol 4-monooxygenase gene, complete mRNA. | Flavobacterium sp. | 40,855 | 26-Apr-93 |
| | | GB_PAT:119994 | 2516 | 119994 | Sequence 2 from patent US 5512478. | Unknown. | 40,855 | 07-OCT-1996 |
| | | GB_BA2:AF059680 | 2410 | AF059680 | Sphingomonas sp. UG30 pentachlorophenol 4-monooxygenase (pcpB) gene, complete cds; and pentachlorophenol 4-monooxygenase reductase (pcpD) gene, partial cds. | Sphingomonas sp. UG30 | 42,993 | 27-Apr-99 |
| rxa01412 | 327 | GB-GSS12:AQ332469 | 459 | AQ332469 | HS_5003_A1_H08_SP6E RPCI11 Human Male BAC Library Homo sapiens genomic clone Plate=579 Col=15 Row=O, genomic survey sequence. | Homo sapiens | 38,208 | 06-MAR-1999 |
| | | GB_EST27:AA998532 | 453 | AA998532 | UI-R-CO-ic-d-11-0-ULs1 UI-R-CO Rattus norvegicus cDNA clone Ul-R-CO-ic-d-11-0-UI 3', mRNA sequence. | Rattus norvegicus | 39,336 | 09-MAR-1999 |
| | | GB_HTG1:HSA342D11 | 178183 | AL121748 | Homo sapiens chromosome 10 clone RP11-342D11, *** SEQUENCING IN PROGRESS ***, in unordered pieces. | Homo sapiens | 40,550 | 23-Nov-99 |
| rxa01458 | 1173 | GB_BA2:AE000745 | 15085 | AE000745 | Aquifex aeolicus section 77 of 109 of the complete genome. | Aquifex aeolicus | 37,694 | 25-MAR-1998 |
| | | GB_BA2:AE000745 | 15085 | AE000745 | Aquifex aeolicus section 77 of 109 of the complete genome. | Aquifex aeolicus | 35,567 | 25-MAR-1998 |
| rxa01571 | 723 | GB_BA1 :AB011413 | 12070 | AB011413 | Streptomyces griseus genes for Orf2, Orf3, Orf4, Orf5, AfsA, Orf8, partial and complete cds. | Streptomyces griseus | 57,500 | 7-Aug-98 |
| | | GB_BA1:AB011413 | 12070 | AB011413 | Streptomyces griseus genes for Orf2, Orf3, Orf4, Orf5, AfsA, Orf8, partial and complete cds. | Streptomyces griseus | 35,655 | 7-Aug-98 |
| rxa01607 | 753 | GB_PR4:AC005005 | 133893 | AC005005 | Homo sapiens PAC clone DJ412A9 from 22, complete sequence. | Homo sapiens | 38,399 | 02-MAR-1999 |
| | | GB_HTG3:AC008257 | 109187 | AC008257 | Drosophila melanogaster chromosome 2 clone BACR08A11 (D916) RPCI-98 08.A.11 map 42A-42A strain y; cn bw sp, *** SEQUENCING IN PROGRESS ***, 93 unordered pieces. | Drosophila melanogaster | 33,741 | 08-OCT-1999 |
| | | GB_HTG3:AC008257 | 109187 | AC008257 | Drosophila melanogaster chromosome 2 clone BACR08A11 (D916) RPCI-98 08.A.11 map 42A-42A strain y; cn bw sp, *** SEQUENCING IN PROGRESS ***, 93 unordered pieces. | Drosophila melanogaster | 33,741 | 08-OCT-1999 |
| rxa01609 | 996 | GB_BA1:MTV003 | 13246 | AL008883 | Mycobacterium tuberculosis H37Rv complete genome; segment 125/162. | Mycobacterium tuberculosis | 39,369 | 17-Jun-98 |
| | | GB_BA1:MSGB1529CS | 36985 | L78824 | Mycobacterium leprae cosmid B1529 DNA sequence. | Mycobacterium leprae | 60,624 | 15-Jun-96 |
| | | GB_BA1:AB024601 | 14807 | AB024601 | Pseudomonas aeruginosa dapD gene for tetrahydrodipicolinate N-succinyletransferase, complete cds, strain PAO1. | Pseudomonas aeruginosa | 41,603 | 12-MAR-1999 |
| rxa01654 | 1119 | GB_GSS4:AQ704352 | 532 | AQ704352 | HS_2147 _A2_H04_MR CIT Approved Human Genomic Sperm Library D Homo sapiens genomic clone Plate=2147 Col=8 Row=O, genomic survey sequence. | Homo sapiens | 37,838 | 7-Jul-99 |
| | | GB_RO:MMAE000663 | 250611 | AE000663 | Mus musculus TCR beta locus from bases 1 to 250611 (section 1 of 3) of the complete sequence. | Mus musculus | 35,799 | 4-Sep-97 |
| | | GB_EST23:Al158428 | 511 | AI158428 | ud24f12.r1 Soares 2NbMT Mus musculus cDNA clone IMAGE:1446863 5', mRNA sequence. | Mus musculus | 41,337 | 30-Sep-98 |
| rxa01664 | 945 | GB_OV:AF026198 | 63155 | AF026198 | Fugu rubripes neural cell adhesion molecule L1 homolog (L1-CAM) gene, complete cds putative protein 1 (PUT1) gene, partial cds; mitosis-specific chromosome segregation protein SMC1 homolog (SMC1) gene, complete cds and calcium channel alpha-1 subunit homolog (CCA1) and putative protein 2 (PUT2) genes, partial cds, complete sequence. | Fugu rubripes | 35,187 | 02-MAY-1998 |
| | | GB_PR3:AC004466 | 122186 | AC004466 | Homo sapiens 12q13.1 PAC RPCI5-1057I20 (Roswell Park Cancer Institute Human PAC library) complete sequence. | Homo sapiens | 37,382 | 17-Sep-98 |
| | | GB_PR3:AC004466 | 122186 | AC004466 | Homo sapiens 12q13.1 PAC RPCI5-1057I20 (Roswell Park Cancer Institute Human PAC library) complete sequence. | Homo sapiens | 37,325 | 17-Sep-98 |
| rxa01795 | 720 | GB BA2:CGU13922 | 4412 | U13922 | Corynebacterium glutamicum putative type II 5-cytosoine methyltransferase (cgIIM) and putative type II restriction endonuclease (cgIIR) and putative type I or type III restriction endonuclease (cIgllR) genes, complete cds. | Corynebacterium glutamicum | 99,444 | 3-Feb-98 |
| | | GB_BA1:S86113 | 1044 | S86113 | ORF 1 [Neisseria gonorrhoeae, Genomic, 1044 nt]. | Neisseria gonorrhoeae | 58,320 | 07-MAY-1993 |
| | | GB_PAT:I22080 | 850 | 122080 | Sequence 1 from patent US 5525717. | Unknown. | 57,722 | 07-OCT-1996 |
| rxa01802 | 954 | GB_BA2:AE001519 | 14062 | AE001519 | Helicobacter pylori, strain J99 section 80 of 132 of the complete genome. | Helicobacter pylori J99 | 33,510 | 20-Jan-99 |
| | | GB_GSS5:AQ774071 | 552 | AQ774071 | HS_2269_B1_C10_T7C CIT Approved Human Genomic Sperm Library D Homo sapiens genomic clone Plate=2269 Col=19 Row=F, genomic survey sequence. | Homo sapiens | 37,967 | 29-Jul-99 |
| | | GB_PR4:AC007459 | 40907 | AC007459 | Homo sapiens chromosome 16 clone 306C6, complete sequence. | Homo sapiens | 39,140 | 04-MAY-1999 |
| rxa01838 | 842 | GB_BA1:SCE15 | 26440 | AL049707 | Streptomyces coelicolor cosmid E15. | Streptomyces coelicolor | 36,297 | 22-Apr-99 |
| | | GB_HTG3:AC009545 | 165042 | AC009545 | Homo sapiens chromosome 11 clone 131_J_04 map 11, *** SEQUENCING IN PROGRESS ***, 8 unordered pieces. | Homo sapiens | 37,651 | 01-OCT-1999 |
| | | GB_HTG3:AC009545 | 165042 | AC009545 | Homo sapiens chromosome 11 clone 131_J_04 map 11,*** SEQUENCING IN PROGRESS ***, 8 unordered pieces. | Homo sapiens | 37,651 | 01-OCT-1999 |
| rxa01848 | 867 | GB_BA1:MTCY24A1 | 20270 | Z95207 | Mycobacterium tuberculosis H37Rv complete genome; segment 124/162. | Mycobacterium tuberculosis | 38,270 | 17-Jun-98 |
| | | GB_EST21 :C89252 | 587 | C89252 | C89252 Mouse early blastocyst cDNA Mus musculus cDNA clone 01B00061JC08, mRNA sequence. | Mus musculus | 37,219 | 28-MAY-1 998 |
| | | GB_EST14:AA423340 | 457 | AA423340 | ve39d04.r1 Soares mouse mammary gland NbMMG Mus musculus cDNA clone IMAGE:820519 5', mRNA sequence. | Mus musculus | 38,377 | 16-OCT-1997 |
| rxa01849 | 1224 | GB_BA1:MTCY24A1 | 20270 | Z95207 | Mycobacterium tuberculosis H37Rv complete genome; segment 124/162. | Mycobacterium tuberculosis | 39,950 | 17-Jun-98 |
| | | GB_BA2:RCPHSYNG | 45959 | Z11165 | R capsulatus complete photosynthesis gene cluster. | Rhodobacter capsulatus | 37,344 | 2-Sep-99 |
| | | GB_BA1:RSP010302 | 40707 | AJ010302 | Rhodobacter sphaeroides photosynthetic gene cluster. | Rhodobacter sphaeroides | 40,898 | 27-Aug-99 |
| rxa01868 | 2049 | GB_BA1:MTV033 | 21620 | AL021928 | Mycobacterium tuberculosis H37Rv complete genome; segment 11/162. | Mycobacterium tuberculosis | 38,679 | 17-Jun-98 |
| | | GB_BA1:MLCL622 | 42498 | Z95398 | Mycobacterium leprae cosmid L622. | Mycobacterium leprae | 38,911 | 24-Jun-97 |
| | | GB_BA1:MSGB983CS | 36788 | L78828 | Mycobacterium leprae cosmid B983 DNA sequence. | Mycobacterium leprae | 38,933 | 15-Jun-96 |
| rxa01885 | 924 | GB_BA1:MTCY1A10 | 25949 | Z95387 | Mycobacterium tuberculosis H37Rv complete genome; segment 117/162. | Mycobacterium tuberculosis | 51,094 | 17-Jun-98 |
| | | GB_PR3:HSU220B11 | 41247 | Z69908 | Human DNA sequence from cosmid cU220B11, between markers DXS6791 and DXS8038 on chromosome X. | Homo sapiens | 39,038 | 23-Nov-99 |
| | | GB_BA1:PDU17435 | 993 | U17435 | Paracoccus denitrificans Fnr-like transcriptional activator (nnr) gene, complete cds. | Paracoccus denitrificans | 39,390 | 19-Jul-95 |
| rxa01914 | 526 | GB_PR3:AC005796 | 43843 | AC005796 | Homo sapiens chromosome 19, cosmid R31408, complete sequence. | Homo sapiens | 34,961 | 06-OCT-1 998 |
| | | GB_PR3:HS390C10 | 114231 | AL008721 | Homo sapiens DNA sequence from BAC 390C10 on chromosome 22q11.21-12.1. Contains an Immunoglobulin LIKE gene and a pseudogene similar to Beta Crystallin. Contains ESTs, STSs, GSSs and taga and tat repeat polymorphisms, complete sequence. | Homo sapiens | 39,600 | 23-Nov-99 |
| | | GB_PR3:AC005796 | 43843 | AC005796 | Homo sapiens chromosome 19, cosmid R31408, complete sequence. | Homo sapiens | 37,725 | 06-OCT-1998 |
| rxa01932 | 1020 | GB_PR3:AC003025 | 112309 | AC003025 | Human Chromosome 11p12.2 PAC clone pDJ466a11, complete sequence. | Homo sapiens | 35,585 | 23-Jul-98 |
| | | GB_GSS3:B78728 | 312 | B78728 | CIT-HSP-431 E3.TV CIT-HSP Homo sapiens genomic clone 431 E3, genomic survey sequence. | Homo sapiens | 38,907 | 25-Jun-98 |
| | | GB_PR3:AC003025 | 112309 | AC003025 | Human Chromosome 11p12.2 PAC clone pDJ466a11, complete sequence. | Homo sapiens | 35,859 | 23-Jul-98 |
| rxa01933 | 726 | GB_HTG1:HS74016 | 169401 | AL110119 | Homo sapiens chromosome 21 clone RPCIP70401674 map 21q21, *** SEQUENCING IN PROGRESS ***, in unordered pieces. | Homo sapiens | 35,302 | 27-Aug-99 |
| | | GB_HTG1:HS74016 | 169401 | AL110119 | Homo sapiens chromosome 21 clone RPCIP70401674 map 21q21, *** SEQUENCING IN PROGRESS ***, in unordered pieces. | Homo sapiens | 35,302 | 27-Aug-99 |
| | | GB_PR4:AC006032 | 170282 | AC006032 | Homo sapiens BAC clone NH0115E20 from Y, complete sequence. | Homo sapiens | 37,640 | 27-Feb-99 |
| rxa01971 | 954 | GB_HTG3:AC008230 | 108469 | AC008230 | Drosophila melanogaster chromosome 2 clone BACR17117 (D934) RPCI-98 17.1.17 map 53A-53C strain y; cn bw sp, *** SEQUENCING IN PROGRESS ***, 108 unordered pieces. | Drosophila melanogaster | 35,466 | 10-Aug-99 |
| | | GB_HTG3:AC008230 | 108469 | AC008230 | Drosophila melanogaster chromosome 2 clone BACR17117 (D934) RPCI-98 17.1.17 map 53A-53C strain y; cn bw sp, *** SEQUENCING IN PROGRESS***, 108 unordered pieces. | Drosophila melanogaster | 35,466 | 10-Aug-99 |
| | | GB_PR3:AF064860 | 165382 | AF064860 | Homo sapiens chromosome 21q22.3 PAC 70124, complete sequence. | Homo sapiens | 39,716 | 2-Jun-98 |
| rxa02016 | 900 | GB_EST2:D48846 | 459 | D48846 | RICS15292A Rice green shoot Oryza sativa cDNA, mRNA sequence. | Oryza sativa | 37,118 | 2-Aug-95 |
| | | GB_GSS10:AQ195886 | 595 | AQ195886 | RPCI11-66013.TJ RPCI-11 Homo sapiens genomic clone RPCI-11-66013, genomic survey sequence. | Homo sapiens | 41,000 | 20-Apr-99 |
| | | GB_GSS10:AQ195886 | 595 | AQ195886 | RPCI11-66013.TJ RPCI-11 Homo sapiens genomic clone RPCI-11-66013, genomic survey sequence. | Homo sapiens | 34,790 | 20-Apr-99 |
| rxa02017 | 807 | GB_EST20:AA855266 | 406 | AA855266 | vw70b08.r1 Stratagene mouse heart (#937316) Mus musculus cDNA clone IMAGE:1260279 5', mRNA sequence. | Mus musculus | 42,638 | 06-MAR-1998 |
| | | GB_EST20:AA855266 | 406 | AA855266 | vw70b08.r1 Stratagene mouse heart (#937316) Mus musculus cDNA clone IMAGE:1260279 5', mRNA sequence. | Mus musculus | 37,183 | 06-MAR-1998 |
| nca02018 | 1073 | GB_BA1:SC5C7 | 41906 | AL031515 | Streptomyces coelicolor cosmid 5C7. | Streptomyces coelicolor | 41,732 | 7-Sep-98 |
| | | GB-BA1:MTC165 | 34331 | Z95584 | Mycobacterium tuberculosis H37Rv complete genome; segment 50/162. | Mycobacterium tuberculosis | 62,395 | 17-Jun-98 |
| | | GB_BA1:SCJ12 | 35302 | AL109989 | Streptomyces coelicolor cosmid J12. | Streptomyces coelicolor A3(2) | 61,603 | 24-Aug-99 |
| rxa02048 | 1497 | GB_PAT:E15823 | 2323 | E15823 | DNA encoding cell surface protein from Corynebacterium ammoniagenes. | Corynebacterium ammoniagenes | 53,942 | 28-Jul-99 |
| | | GB_OM:SSAMPTDN | 3387 | Z29522 | S.scrofa mRNA for aminopeptidase N. | Sus scrofa | 42,672 | 26-Sep-94 |
| | | GB_OV:D87992 | 3181 | D87992 | Gallus gallus mRNA for aminopeptidase Ey, complete cds. | Gallus gallus | 41,554 | 5-Jun-99 |
| rxa02101 | 1386 | GB_BA1:AP000064 | 247695 | AP000064 | Aeropyrum pernix genomic DNA, section 7/7. | Aeropyrum pernix | 39,882 | 22-Jun-99 |
| | | GB_PL2:ATAC006587 | 79262 | AC006587 | Arabidopsis thaliana chromosome II BAC T17D12 genomic sequence, complete sequence. | Arabidopsis thaliana | 38,490 | 23-MAR-1999 |
| | | GB_PL2:ATAC006587 | 79262 | AC006587 | Arabidopsis thaliana chromosome II BAC T17D12 genomic sequence, complete sequence. | Arabidopsis thaliana | 34,863 | 23-MAR-1999 |
| rxa02265 | 423 | GB_BA2:AF120718 | 4137 | AF120718 | Lactobacillus fermentum urease operon, partial sequence. | Lactobacillus fermentum | 56,265 | 31-MAR-1999 |
| | | GB_PAT:E03531 | 2896 | E03531 | DNA sequence coding for acid urease. | Lactobacillus fermentum | 56,265 | 29-Sep-97 |
| | | GB_BA1:LBAAURE | 2896 | D10605 | L.fermentum gene for acid urease. | Lactobacillus fermentum | 56,265 | 2-Feb-99 |
| rxa02276 | 801 | GB_GSS10:AQ242920 | 451 | AQ242920 | HS_2061_A1_E08_MR CIT Approved Human Genomic Sperm Library D Homo sapiens genomic clone Plate=2061 Col=15 Row=I, genomic survey sequence. | Homo sapiens | 37,916 | 03-OCT-1 998 |
| | | GB_IN1:SLMMTPMF | 14503 | D29637 | Physarum polycephalum mitochondrial DNA. | Mitochondrion Physarum polycephalum | 40,335 | 12-MAY-1999 |
| | | GB_IN2:AF012249 | 5542 | AF012249 | Physarum polycephalum strain aux2-S region of mitochondria derived from mF plasmid, including URFA', URFC, URFD, URFE, URFF, and URFG genes, complete cds, and URFH gene, partial cds. | Mitochondrion Physarum polycephalum | 40,335 | 08-MAY-1 998 |
| rxa02277 | 738 | GB_BA2:AF048784 | 681 | AF048784 | Actinomyces naeslundii urease accessory protein (ureG) gene, complete cds. | Actinomyces naeslundii | 66,814 | 9-Feb-99 |
| | | GB_BA2:AF056321 | 5482 | AF056321 | Actinomyces naeslundii urease gamma subunit UreA (ureA), urease beta subunit UreB (ureB), urease alpha subunit UreC (ureC), urease accessory protein UreE (ureE), urease accessory protein UreF (ureF), urease accessory protein UreG (ureG), and urease accessory protein UreD (ureD) genes, complete cds. | Actinomyces naeslundii | 63,686 | 9-Feb-99 |
| | | GB_BA2:SSU35248 | 5773 | U35248 | Streptococcus salivarius ure cluster nickel transporter homolog (urel) gene, partial cds, and urease beta subunit (ureA), gamma subunit (ureB), alpha subunit (ureC), and accessory proteins (ureE), (ureF), (ureG), and (ureD) genes, complete cds. | Streptococcus salivarius | 61,931 | 26-Jan-96 |
| rxa02278 | 972 | GB_GSS3:B49054 | 543 | B49054 | RPCI11-4I13.TV RPCI-11 Homo sapiens genomic clone RPCI-11-4113, genomic survey sequence. | Homo sapiens | 39,161 | 8-Apr-99 |
| | | GB_PL1:PMCMSGI | 3363 | L27092 | Pneumocystis carinii B-cell receptor (msgl) gene, 3' end. | Pneumocystis carinii | 39,819 | 26-Sep-94 |
| | | GB_PL2:AF038556 | 12792 | AF038556 | Pneumocystis carinii f. sp.hominis variant regions of major surface glycoproteins (msg1, msg3, msg4) genes, partial cds . | Pneumocystis carinii f. sp. hominis | 33,832 | 10-Sep-98 |
| rxa02317 | 735 | GB_GSS8:AQ051031 | 914 | AQ051031 | nbxb0004dG10r CUGI Rice BAC Library Oryza sativa genomic clone nbxb0004N20r, genomic survey sequence. | Oryza sativa | 32,299 | 24-MAR-1999 |
| | | GB_GSS8:AQ051031 | 914 | AQ051031 | nbxb0004dG10r CUGI Rice BAC Library Oryza sativa genomic clone nbxb0004N20r, genomic survey sequence. | Oryza sativa | 34,573 | 24-MAR-1999 |
| rxa02334 | 746 | GB_BA1:CGU35023 | 3195 | U35023 | Corynebacterium glutamicum thiosulfate sulfurtransferase (thtR) gene, partial cds, acyl CoA carboxylase (accBC) gene, complete cds. | Corynebacterium glutamicum | 100,000 | 16-Jan-97 |
| | | GB_BA1:MTCY71 | 42729 | Z92771 | Mycobacterium tuberculosis H37Rv complete genome, segment 141/162. | Mycobacterium tuberculosis | 60,380 | 10-Feb-99 |
| | | GB_BA1:U00012 | 33312 | U00012 | Mycobacterium leprae cosmid B1308. | Mycobacterium leprae | 37,660 | 30-Jan-96 |
| rxa02351 | 1039 | GB_HTG2:HS225E12 | 126464 | AL031772 | Homo sapiens chromosome 6 clone RP1-225E12 map q24, *** SEQUENCING IN PROGRESS ***, in unordered pieces. | Homo sapiens | 35,973 | 03-DEC-1999 |
| | | GB_HTG2:HS225E12 | 126464 | AL031772 | Homo sapiens chromosome 6 clone RP1-225E12 map q24, *** SEQUENCING IN PROGRESS ***, in unordered pieces. | Homo sapiens | 35,973 | 03-DEC-1999 |
| | | GB_HTG2:HS225E12 | 126464 | AL031772 | Homo sapiens chromosome 6 clone RP1-225E12 map q24, *** SEQUENCING IN PROGRESS ***, in unordered pieces. | Homo sapiens | 36,992 | 03-DEC-1999 |
| rxa02410 | 789 | GB_BA1:AB020624 | 1605 | AB020624 | Corynebacterium glutamicum murl gene for D-glutamate racemase, complete cds. | Corynebacterium glutamicum | 99,227 | 24-Jul-99 |
| | | GB_EST4:H51527 | 294 | H51527 | yo33b09.s1 Soares adult brain N2b4HB55Y Homo sapiens cDNA clone IMAGE:179705 3', mRNA sequence. | Homo sapiens | 40,411 | 18-Sep-95 |
| | | GB_GSS1:CNS003CM | 1101 | AL064136 | Drosophila melanogaster genome survey sequence T7 end of BAC # BACR08C19 of RPCI-98 library from Drosophila melanogaster (fruit fly), genomic survey sequence. | Drosophila melanogaster | 37,674 | 3-Jun-99 |
| rxa02477 | 744 | GB_HTG4:AC010054 | 130191 | AC010054 | Drosophila melanogaster chromosome 3L/74E2 clone RPC198-15E10, *** SEQUENCING IN PROGRESS ***, 70 unordered pieces. | Drosophila melanogaster | 37,466 | 16-OCT-1999 |
| | | GB_HTG4:AC010054 | 130191 | AC010054 | Drosophila melanogaster chromosome 3U74E2 clone RPCI98-15E10, *** SEQUENCING IN PROGRESS ***, 70 unordered pieces. | Drosophila melanogaster | 37,466 | 16-OCT-1999 |
| | | GB_HTG4:AC009375 | 137069 | AC009375 | Drosophila melanogaster chromosome 3L/75A1 clone RPCI98-44L18, *** SEQUENCING IN PROGRESS ***, 59 unordered pieces. | Drosophila melanogaster | 39,118 | 16-OCT-1 999 |
| rxa02513 | 832 | GB_BA1:MTER260 | 373 | X92572 | M.terrae gene for 32 kDa protein (partial). | Mycobacterium terrae | 42,895 | 15-Jan-98 |
| | | G8_PL1:AB019229 | 84294 | A8019229 | Arabidopsis thaliana genomic DNA, chromosome 3, P1 clone: MDC16, complete sequence. | Arabidopsis thaliana | 36,084 | 20-Nov-99 |
| | | GB_PL1:AB019229 | 84294 | AB019229 | Arabidopsis thaliana genomic DNA, chromosome 3, P1 clone: MDC16, complete sequence. | Arabidopsis thaliana | 35,244 | 20-Nov-99 |
| rxa02531 | 834 | GB_BA1:CGLATTB | 271 | X89850 | C.glutamicum DNA for attB region. | Corynebacterium glutamicum | 40,590 | 8-Aug-96 |
| | | GB_EST11:AA239557 | 423 | AA239557 | mv25f04.r1 GuayWoodford Beier mouse kidney day 0 Mus musculus cDNA clone IMAGE:656095 5' similar to gb:X52634 Murine tlm oncogene for tlm protein (MOUSE);, mRNA sequence. | Mus musculus | 38,760 | 12-MAR-1997 |
| | | GB_BA1:RSPYPPCl | 6500 | AJ002398 | Rhodobacter sphaeroides pyp and pcl genes, and orfA, orfB, orfC, orfD, orfE, orfF. | Rhodobacter sphaeroides | 37,091 | 17-DEC-1998 |
| rxa02548 | 314 | GB_BA2:AF127374 | 63734 | AF127374 | Streptomyces lavendulae LinA homolog, cytochrome P450 hydroxylase ORF4, cytochrome P450 hydroxylase ORF3, MitT (mitT), MitS (mitS), MitR (mitR), MitQ (mitQ), MitP (mitP), MitO (mitO), MitN (mitN), MitM (mitM), MitL (mitL), MitK (mitK), MitJ (mitJ), Mitl (mitl), MitH (mitH), MitG (mitG), MitF (mitF), MitE (mitE), MitD (mitD), MitC (mitC), MitB (mitB), MitA (mitA), MmcA (mmcA), MmcB (mmcB), MmcC (mmcC), MmcD (mmcD), MmcE (mmcE), MmcF (mmcF), MmcG (mmcG), MmcH (mmcH), Mmcl (mmcl), MmcJ (mmcJ), MmcK (mmcK), MmcL (mmcL), MmcM (mmcM), MmcN (mmcN), MmcO (mmcO), Mrd (mrd), MmcP (mmcP), MmcQ (mmcQ), MmcR (mmcR), MmcS (mmcS), MmcT (mmcT), MmcU (mmcU), MmcV (mmcV), Mct (mct), MmcW (mmcW), MmcX (mmcX), and MmcY (mmcY) genes, complete cds; and unknown genes. | Streptomyces lavendulae | 66,242 | 27-MAY-1999 |
| | | GB BA2:AF127374 | 63734 | AF127374 | Streptomyces lavendulae LinA homolog, cytochrome P450 hydroxylase ORF4, cytochrome P450 hydroxylase ORF3, MitT (mitT), MitS (mitS), MitR (mitR), MitQ (mitQ), MitP (mitP), MitO (mitO), MitN (mitN), MitM (mitM), MitL (mitL), MitK (mitK), MitJ (mitJ), Mitl (mitl), MitH (mitH), MitG (mitG), MitF (mitF), MitE (mitE), MitD (mitD), MitC (mitC), MitB (mitB), MitA (mitA), MmcA (mmcA), MmcB (mmcB), MmcC (mmcC), MmcD (mmcD), MmcE (mmcE), MmcF (mmcF), MmcG (mmcG), MmcH (mmcH), Mmcl (mmcl), MmcJ (mmcJ), MmcK (mmcK), MmcL (mmcL), MmcM (mmcM), MmcN (mmcN), MmcO (mmcO), Mrd (mrd), MmcP (mmcP), MmcQ (mmcQ). MmcR (mmcR), MmcS (mmcS), MmcT (mmcT), MmcU (mmcU), MmcV (mmcV), Mct (mct), MmcW (mmcW), MmcX (mmcX), and MmcY (mmcY) genes, complete cds; and unknown genes. | Streptomyces lavendulae | 38,411 | 27-MAY-1999 |
| | | GB_GSS4:AQ741886 | 742 | AQ741886 | HS_5569_B2_B02_SP6 RPCI-11 Human Male BAC Library Homo sapiens genomic clone Plate=1145 Col=4 Row=D, genomic survey sequence. | Homo sapiens | 38,907 | 16-Jul-99 |
| rxa02558 | 1098 | GB_EST18:AA567307 | 741 | AA567307 | HL01004.5prime HL Drosophila melanogaster head BlueScript Drosophila melanogaster cDNA clone HL01004 5prime, mRNA sequence. | Drosophila melanogaster | 38,736 | 28-Nov-98 |
| | | GB_EST27:Al402394 | 630 | Al402394 | GH21610.5prime GH Drosophila melanogaster head pOT2 Drosophila melanogaster cDNA clone GH21610 5prime, mRNA sequence. | Drosophila melanogaster | 41,308 | 8-Feb-99 |
| | | GB_GSS10:AQ237646 | 715 | AQ237646 | RPCI11-6119.TJB RPCI-11 Homo sapiens genomic clone RPCI-11-6119, genomic survey sequence. | Homo sapiens | 44,340 | 21-Apr-99 |
| rxa02565 | 1389 | GB_EST32:AI726448 | 562 | AI726448 | BNLGHi5854 Six-day Cotton fiber Gossypium hirsutum cDNA 5' similar to (U53418) UDP-glucose dehydrogenase [Glycine max], mRNA sequence. | Gossypium hirsutum | 37,003 | 11-Jun-99 |
| | | GB_EST32:AI726198 | 608 | AI726198 | BNLGHi5243 Six-day Cotton fiber Gossypium hirsutum cDNA 5' similar to (U53418) UDP-glucose dehydrogenase [Glycine max], mRNA sequence. | Gossypium hirsutum | 40,925 | 11-Jun-99 |
| | | GB_PR4:AC002992 | 154848 | AC002992 | Homo sapiens chromosome Y, clone 203M13, complete sequence. | Homo sapiens | 38,039 | 13-OCT-1999 |
| rxa02574 | 1131 | GB_EST4:H29653 | 415 | H29653 | ym58f01.r1 Soares infant brain 1 NIB Homo sapiens cDNA clone IMAGE:52678 5' similar to SP:OXDD_BOVIN P31228 D-ASPARTATE OXIDASE mRNA sequence. | Homo sapiens | 39,036 | 17-Jul-95 |
| | | GB_PR3:HSDJ261K5 | 131974 | AL050350 | Human DNA sequence from clone 261K5 on chromosome 6q21-22.1. Contains the 3' part of the gene for a novel organic cation transporter (BAC ORF RG331 P03), the DDO gene for D-aspartate oxidase (EC 1.4.3.1), ESTs. STSs, GSSs and two putative CpG islands, complete sequence. | Homo sapiens | 35,957 | 23-Nov-99 |
| | | GB_EST2:R20147 | 494 | R20147 | yg18h02.r1 Soares infant brain 1NIB Homo sapiens cDNA clone IMAGE:32866 5' similar to SP:OXDD_BOVIN P31228 D-ASPARTATE OXIDASE;, mRNA sequence. | Homo sapiens | 36,437 | 17-Apr-95 |
| rxa02589 | 888 | GB_HTG1:CEY6E2 | 186306 | Z96799 | Caenorhabditis elegans chromosome V clone Y6E2, *** SEQUENCING IN PROGRESS ***, in unordered pieces. | Caenorhabditis elegans | 37,979 | 02-OCT-1 997 |
| | | GB_HTG1:CEY6E2 | 186306 | Z96799 | Caenorhabditis elegans chromosome V clone Y6E2, *** SEQUENCING IN PROGRESS ***, in unordered pieces. | Caenorhabditis elegans | 37,979 | 02-OCT-1997 |
| | | GB_HTG3:AC011690 | 72277 | AC011690 | Homo sapiens clone 17_E_13, LOW-PASS SEQUENCE SAMPLING. | Homo sapiens | 35,814 | 10-OCT-1999 |
| rxa02592 | 894 | GB_BA1:MSGB983CS | 36788 | L78828 | Mycobacterium leprae cosmid B983 DNA sequence. | Mycobacterium leprae | 53,235 | 15-Jun-96 |
| | | GB_GSS9:AQ170723 | 487 | AQ170723 | HS_2270_B2_F05_MR CIT Approved Human Genomic Sperm Library D Homo sapiens genomic clone Plate=2270 Col=10 Row=L, genomic survey sequence. | Homo sapiens | 39,666 | 16-OCT-1998 |
| | | GB_GSS12:AQ349397 | 791 | AQ349397 | RPCI11-118H16.TJ RPCI-11 Homo sapiens genomic clone RPCI-11-118H16, genomic survey sequence. | Homo sapiens | 34,204 | 07-MAY-1999 |
| rxa02603 | 1119 | GB_BA1:MTV026 | 23740 | AL022076 | Mycobacterium tuberculosis H37Rv complete genome; segment 1571162. | Mycobacterium tuberculosis | 37,975 | 24-Jun-99 |
| | | GB_IN2:AC005714 | 177740 | AC005714 | Drosophila melanogaster, chromosome 2R, region 58D4-58E2, BAC clone BACR48M13, complete sequence. | Drosophila melanogaster | 41,226 | 01-MAY-1999 |
| | | GB_EST19:AA775050 | 218 | AA775050 | ac76e10.s1 Stratagene lung (#937210) Homo sapiens cDNA clone IMAGE:868554 3' similar to gb:Y00371_rna1 HEAT SHOCK COGNATE 71 KD PROTEIN (HUMAN); mRNA sequence. | Homo sapiens | 40,826 | 5-Feb-98 |
| rxa02630 | 1446 | GB_BA1:MLCL373 | 37304 | AL035500 | Mycobacterium leprae cosmid L373. | Mycobacterium leprae | 49,015 | 27-Aug-99 |
| | | GB_BA1:MTV044 | 16150 | AL021999 | Mycobacterium tuberculosis H37Rv complete genome; segment 45/162. | Mycobacterium tuberculosis | 49,192 | 17-Jun-98 |
| | | GB_BA1:MLU15180 | 38675 | U15180 | Mycobacterium leprae cosmid B1756. | Mycobacterium leprae | 45,621 | 09-MAR-1995 |
| rxa02643 | 1167 | GB_EST37:AI950576 | 308 | AI950576 | wx52e08.x1 NCI_CGAP_Lu28 Homo sapiens cDNA clone IMAGE:2547302 3', mRNA sequence. | Homo sapiens | 40,909 | 6-Sep-99 |
| | | GB_EST37:AI950576 | 308 | AI950576 | wx52e08.x1 NCI_CGAP_Lu28 Homo sapiens cDNA clone IMAGE:2547302 3', mRNA sequence. | Homo sapiens | 40,288 | 6-Sep-99 |
| rxa02644 | 774 | GB_EST34:AV149547 | 302 | AV149547 | AV149547 Mus musculus C57BU6J 10-11 day embryo Mus musculus cDNA clone 2810489D03, mRNA sequence. | Mus musculus | 38,627 | 5-Jul-99 |
| | | GB_EST35:AV156221 | 271 | AV156221 | AV156221 Mus musculus head C57BL/6J 12-day embryo Mus musculus cDNA clone 3000001 C24, mRNA sequence. | Mus musculus | 33,990 | 7-Jul-99 |
| | | GB_EST32:AV054919 | 274 | AV054919 | AV054919 Mus musculus pancreas C57BL/6J adult Mus musculus cDNA clone 1810033C08, mRNA sequence. | Mus musculus | 36,585 | 23-Jun-99 |
| rxa02745 | 902 | GB_BA1:MTV007 | 32806 | AL021184 | Mycobacterium tuberculosis H37Rv complete genome; segment 641162. | Mycobacterium tuberculosis | 39,298 | 17-Jun-98 |
| | | GB_BA2:AF027770 | 30683 | AF027770 | Mycobacterium smegmatis FxbA (fxbA) gene, partial cds; FxbB (fxbB), FxbC (fxbC), and FxuD (fxtD) genes, complete cds; and unknown genes. | Mycobacterium smegmatis | 55,125 | 03-DEC-1998 |
| | | GB_BA2:SAU43537 | 3938 | U43537 | Streptomyces argillaceus mithramycin resistance determinant, ATP-binding protein (mtrA) and membrane protein (mtrB) genes, complete cds. | Streptomyces argillaceus | 46,868 | 5-Sep-96 |
| rxa02746 | 290 | GB_BA1:CAJ10319 | 5368 | AJ010319 | Corynebacterium glutamicum amtP, glnB, glnD genes and partial ftsY and srp genes. | Corynebacterium glutamicum | 100,000 | 14-MAY-1999 |
| | | GB_BA1:MTCY338 | 29372 | Z74697 | Mycobacterium tuberculosis H37Rv complete genome; segment 1271162. | Mycobacterium tuberculosis | 39,785 | 17-Jun-98 |
| | | GB_HTG3:AC008733 | 216140 | AC008733 | Homo sapiens chromosome 19 clone CITB-E1_2525J15, *** SEQUENCING IN PROGRESS ***, 72 unordered pieces. | Homo sapiens | 35,688 | 3-Aug-99 |
| rxa02820 | 1411 | GB_BA1:BFU64514 | 3837 | U64514 | Bacillus firmus dppABC operon, dipeptide transporter protein dppA gene, partial cds, and dipeptide transporter proteins dppB and dppC genes, complete cds. | Bacillus firmus | 36,859 | 1-Feb-97 |
| | | GB_IN1:CET04C10 | 20958 | Z69885 | Caenorhabditis elegans cosmid T04C10, complete sequence. | Caenorhabditis elegans | 35,934 | 2-Sep-99 |
| | | GB_EST35:AI823090 | 720 | AI823090 | L30-944T3 Ice plant Lambda Uni-Zap XR expression library, 30 hours NaCl treatment Mesembryanthemum crystallinum cDNA clone L30-944 5' similar to 60S ribosomal protein L36 (AC004684)[Arabidopsis thaliana], mRNA sequence. | Mesembryanthemum crystallinum | 35,770 | 21-Jul-99 |
| rxa02834 | 518 | GB_BA1:CJY13333 | 3315 | Y13333 | Campylobacter jejuni clpB gene. | Campylobacterjejuni | 53,400 | 12-Apr-99 |
| | | GB_BA2:AF065404 | 181654 | AF065404 | Bacillus anthracis virulence plasmid PX01, complete sequence. | Bacillus anthracis | 45,168 | 20-OCT-1 999 |
| | | GB_PL2:AC006601 | 110684 | AC006601 | Arabidopsis thaliana chromosome V map near 60.5 cM, complete sequence. | Arabidopsis thaliana | 36,680 | 22-Feb-99 |

### Exemplification

### Example 1: Preparation of total genomic DNA of Corynebacterium glutamicum ATCC 13032

A culture of *Corynebacterium glutamicum* (ATCC 13032) was grown overnight at 30°C with vigorous shaking in BHI medium (Difco). The cells were harvested by centrifugation, the supernatant was discarded and the cells were resuspended in 5 ml buffer-1 (5% of the original volume of the culture ― all indicated volumes have been calculated for 100 ml of culture volume). Composition of buffer-I: 140.34 g/l sucrose, 2.46 g/l MgSO₄ x 7H₂O, 10 ml/l KH₂PO₄ solution (100 g/l, adjusted to pH 6.7 with KOH), 50 ml/l M12 concentrate (10 g/l (NH₄)₂SO₄, 1 g/l NaCl, 2 g/l MgSO₄ x 7H₂O, 0.2 g/l CaCl₂, 0.5 g/l yeast extract (Difco), 10 ml/l trace-elements-mix (200 mg/l FeSO₄ x H₂O, 10 mg/l ZnSO₄ x 7 H₂O, 3 mg/l MnCl₂ x 4 H₂O, 30 mg/l H₃BO₃ 20 mg/l CoCl₂ x 6 H₂O, 1 mg/l NiCl₂ x 6 H₂O, 3 mg/l Na₂MoO₄ x 2 H₂O, 500 mg/l complexing agent (EDTA or critic acid), 100 ml/l vitamins-mix (0.2 mg/l biotin, 0.2 mg/l folic acid, 20 mg/l p-amino benzoic acid, 20 mg/l riboflavin, 40 mg/l ca-panthothenate, 140 mg/l nicotinic acid, 40 mg/l pyridoxole hydrochloride, 200 mg/l myo-inositol). Lysozyme was added to the suspension to a final concentration of 2.5 mg/ml. After an approximately 4 h incubation at 37°C, the cell wall was degraded and the resulting protoplasts are harvested by centrifugation. The pellet was washed once with 5 ml buffer-I and once with 5 ml TE-buffer (10 mM Tris-HCl, 1 mM EDTA, pH 8). The pellet was resuspended in 4 ml TE-buffer and 0.5 ml SDS solution (10%) and 0.5 ml NaCl solution (5 M) are added. After adding of proteinase K to a final concentration of 200 µg/ml, the suspension is incubated for ca.18 h at 37°C. The DNA was purified by extraction with phenol, phenol-chloroform-isoamylalcohol and chloroform-isoamylalcohol using standard procedures. Then, the DNA was precipitated by adding 1/50 volume of 3 M sodium acetate and 2 volumes of ethanol, followed by a 30 min incubation at -20°C and a 30 min centrifugation at 12,000 rpm in a high speed centrifuge using a SS34 rotor (Sorvall). The DNA was dissolved in 1 ml TE-buffer containing 20 µg/ml RNaseA and dialysed at 4°C against 1000 ml TE-buffer for at least 3 hours. During this time, the buffer was exchanged 3 times. To aliquots of 0.4 ml of the dialysed DNA solution, 0.4 ml of 2 M LiC1 and 0.8 ml of ethanol are added. After a 30 min incubation at -20°C, the DNA was collected by centrifugation (13,000 rpm, Biofuge Fresco, Heraeus, Hanau, Germany). The DNA pellet was dissolved in TE-buffer. DNA prepared by this procedure could be used for all purposes, including southern blotting or construction of genomic libraries.

### Example 2: Construction of genomic libraries in Escherichia coli of Corynebacterium glutamicum ATCC13032.

Using DNA prepared as described in Example 1, cosmid and plasmid libraries were constructed according to known and well established methods *(see e.g.,* Sambrook, J. *et al.* (1989) "Molecular Cloning : A Laboratory Manual", Cold Spring Harbor Laboratory Press, or Ausubel, *F.M. et al.* (1994) "Current Protocols in Molecular Biology", John Wiley & Sons.)

Any plasmid or cosmid could be used. Of particular use were the plasmids pBR322 (Sutcliffe, J.G. (1979) *Proc. Natl. Acad. Sci. USA,* 75:3737-3741); pACYC177 (Change & Cohen (1978) *J. Bacteriol* 134:1141-1156), plasmids of the pBS series (pBSSK+, pBSSK- and others; Stratagene, LaJolla, USA), or cosmids as SuperCosl (Stratagene, LaJolla, USA) or Lorist6 (Gibson, T.J., Rosenthal A. and Waterson, R.H. (1987) *Gene* 53:283-286. Gene libraries specifically for use in *C*. *glutamicum* may be constructed using plasmid pSL109 (Lee, H.-S. and A. J. Sinskey (1994) *J. Microbiol. Biotechnol.* 4: 256-263).

### Example 3: DNA Sequencing and Computational Functional Analysis

Genomic libraries as described in Example 2 were used for DNA sequencing according to standard methods, in particular by the chain termination method using ABI377 sequencing machines (see e.g., Fleischman, *R.D. et al.* (1995) "Whole-genome Random Sequencing and Assembly of Haemophilus Influenzae Rd., Science, 269:496-512). Sequencing primers with the following nucleotide sequences were used: 5'-GGAAACAGTATGACCATG-3'or 5'-GTAAAACGACGGCCAGT-3'.

### Example 4: In vivo Mutagenesis

*In vivo* mutagenesis of *Corynebacterium glutamicum* can be performed by passage of plasmid (or other vector) DNA through E. *coli* or other microorganisms *(e.g. Bacillus* spp. or yeasts such as *Saccharomyces cerevisiae)* which are impaired in their capabilities to maintain the integrity of their genetic information. Typical mutator strains have mutations in the genes for the DNA repair system (e.g., mutHLS, mutD, mutT, etc.; for reference, see Rupp, W.D. (1996) DNA repair mechanisms, in: *Escherichia coli* and *Salmonella,* p. 2277-2294, ASM: Washington.) Such strains are well known to those of ordinary skill in the art. The use of such strains is illustrated, for example, in Greener, A. and Callahan, M. (1994) *Strategies* 7: 32-34.

### Example 5: DNA Transfer Between Escherichia coli and Corynebacterium glutamicum

Several *Corynebacterium* and *Brevibacterium* species contain endogenous plasmids (as *e.g.,* pHM1519 or pBL1) which replicate autonomously (for review see, *e.g.,* Martin, J.F. *et at.* (1987) *Biotechnology,* 5:137-146). Shuttle vectors for *Escherichia coli* and *Corynebacterium glutamicum* can be readily constructed by using standard vectors for *E. coli* (Sambrook, J. *et al.* (1989), "Molecular Cloning: A Laboratory Manual", Cold Spring Harbor Laboratory Press or Ausubel, F.M. *et al.* (1994) "Current Protocols in Molecular Biology", John Wiley & Sons) to which a origin or replication for and a suitable marker from *Corynebacterium glutamicum* is added. Such origins of replication are preferably taken from endogenous plasmids isolated from *Corynebacterium* and *Brevibacterium* species. Of particular use as transformation markers for these species are genes for kanamycin resistance (such as those derived from the Tn5 or Tn903 transposons) or chloramphenicol (Winnacker, E.L. (1987) "From Genes to Clones - Introduction to Gene Technology, VCH, Weinheim). There are numerous examples in the literature of the construction of a wide variety of shuttle vectors which replicate in both E. *coli and C. glutamicum,* and which can be used for several purposes, including gene overexpression (for reference, see e.g., Yoshihama, M. *et al.* (1985) *J. Bacteriol.* 162:591-597, Martin J.F. *et al.* (1987) *Biotechnology,* 5:137-146 and Eikmanns, B.J. *et al.* (1991) *Gene,* 102:93-98).

Using standard methods, it is possible to clone a gene of interest into one of the shuttle vectors described above and to introduce such a hybrid vectors into strains of *Corynebacterium glutamicum.* Transformation of C. *glutamicum* can be achieved by protoplast transformation (Kastsumata, R*. et al.* (1984) *J. Bacteriol.* 159306-311), electroporation (Liebl, E. *et al.* (1989) *FEMS Microbiol. Letters,* 53:399-303) and in cases where special vectors are used, also by conjugation (as described e.g. in Schäfer, *A et al. (*1990) J. *Bacteriol.* 172:1663-1666). It is also possible to transfer the shuttle vectors for *C. glutamicum* to *E. coli* by preparing plasmid DNA from *C*. *glutamicum* (using standard methods well-known in the art) and transforming it into *E. coli.* This transformation step can be performed using standard methods, but it is advantageous to use an Mcr-deficient *E. coli* strain, such as NM522 (Gough & Murray (1983) *J Mol. Biol.* 166:1-19).

Genes may be overexpressed in *C*. *glutamicum* strains using plasmids which comprise pCG1 (U.S. Patent No. 4,617,267) or fragments thereof, and optionally the gene for kanamycin resistance from TN903 (Grindley, N.D. and Joyce, C.M. (1980) *Proc. Natl. Acad. Sci. USA* 77(12): 7176-7180). In addition, genes may be overexpressed in C. *glutamicum* strains using plasmid pSL109 (Lee, H.-S. and A. J. Sinskey (1994) *J. Microbiol. Biotechnol.* 4: 256-263).

Aside from the use of replicative plasmids, gene overexpression can also be achieved by integration into the genome. Genomic integration in *C*. *glutamicum* or other Corynebacterium or Brevibacterium species may be accomplished by well-known methods, such as homologous recombination with genomic region(s), restriction endonuclease mediated integration (REMI) (see, e.g., DE Patent 19823834), or through the use of transposons. It is also possible to modulate the activity of a gene of interest by modifying the regulatory regions (e.g., a promoter, a repressor, and/or an enhancer) by sequence modification, insertion, or deletion using site-directed methods (such as homologous recombination) or methods based on random events (such as transposon mutagenesis or REMI). Nucleic acid sequences which function as transcriptional terminators may also be inserted 3' to the coding region of one or more genes of the invention; such terminators are well-known in the art and are described, for example, in Winnacker, E.L. (1987) From Genes to Clones - Introduction to Gene Technology. VCH: Weinheim.

### Example 6: Assessment of the Expression of the Mutant Protein

Observations of the activity of a mutated protein in a transformed host cell rely on the fact that the mutant protein is expressed in a similar fashion and in a similar quantity to that of the wild-type protein. A useful method to ascertain the level of transcription of the mutant gene (an indicator of the amount of mRNA available for translation to the gene product) is to perform a Northern blot (for reference see, for example, Ausubel *et al.* (1988) Current Protocols in Molecular Biology, Wiley: New York), in which a primer designed to bind to the gene of interest is labeled with a detectable tag (usually radioactive or chemiluminescent), such that when the total RNA of a culture of the organism is extracted, run on gel, transferred to a stable matrix and incubated with this probe, the binding and quantity of binding of the probe indicates the presence and also the quantity of mRNA for this gene. This information is evidence of the degree of transcription of the mutant gene. Total cellular RNA can be prepared from *Corynebacterium glutamicum* by several methods, all well-known in the art, such as that described in Bormann, *E.R. et al.* (1992) *Mol. Microbiol.* 6: 317-326.

To assess the presence or relative quantity of protein translated from this mRNA, standard techniques, such as a Western blot, may be employed (see, for example, Ausubel *et al.* (1988) Current Protocols in Molecular Biology, Wiley: New York). In this process, total cellular proteins are extracted, separated by gel electrophoresis, transferred to a matrix such as nitrocellulose, and incubated with a probe, such as an antibody, which specifically binds to the desired protein. This probe is generally tagged with a chemiluminescent or colorimetric label which may be readily detected. The presence and quantity of label observed indicates the presence and quantity of the desired mutant protein present in the cell.

### Example 7: Growth of Genetically Modified Corynebacterium glutamicum - Media and Culture Conditions

Genetically modified *Corynebacteria* are cultured in synthetic or natural growth media. A number of different growth media for Corynebacteria are both well-known and readily available (Lieb *et al.* (1989) *Appl. Microbiol. Biotechnol.,* 32:205-210; von der Osten *et al.* (1998) *Biotechnology Letters,* 11:11-16; Patent DE 4,120,867; Liebl (1992) "The Genus *Corynebacterium,* in: The Procaryotes, Volume II, Balows, A. *et al.,* eds. Springer-Verlag). These media consist of one or more carbon sources, nitrogen sources, inorganic salts, vitamins and trace elements. Preferred carbon sources are sugars, such as mono-, di-, or polysaccharides. For example, glucose, fructose, mannose, galactose, ribose, sorbose, ribulose, lactose, maltose, sucrose, raffinose, starch or cellulose serve as very good carbon sources. It is also possible to supply sugar to the media via complex compounds such as molasses or other by-products from sugar refinement. It can also be advantageous to supply mixtures of different carbon sources. Other possible carbon sources are alcohols and organic acids, such as methanol, ethanol, acetic acid or lactic acid. Nitrogen sources are usually organic or inorganic nitrogen compounds, or materials which contain these compounds. Exemplary nitrogen sources include ammonia gas or ammonia salts, such as NH₄Cl or (NH₄)₂SO₄, NH₄OH, nitrates, urea, amino acids or complex nitrogen sources like corn steep liquor, soy bean flour, soy bean protein, yeast extract, meat extract and others.

Inorganic salt compounds which may be included in the media include the chloride-, phosphorous- or sulfate- salts of calcium, magnesium, sodium, cobalt, molybdenum, potassium, manganese, zinc, copper and iron. Chelating compounds can be added to the medium to keep the metal ions in solution. Particularly useful chelating compounds include dihydroxyphenols, like catechol or protocatechuate, or organic acids, such as citric acid. It is typical for the media to also contain other growth factors, such as vitamins or growth promoters, examples of which include biotin, riboflavin, thiamin, folic acid, nicotinic acid, pantothenate and pyridoxin. Growth factors and salts frequently originate from complex media components such as yeast extract, molasses, corn steep liquor and others. The exact composition of the media compounds depends strongly on the immediate experiment and is individually decided for each specific case. Information about media optimization is available in the textbook "Applied Microbiol. Physiology, A Practical Approach *(eds.* P.M. Rhodes, P.F. Stanbury, IRL Press (1997) pp. 53-73, ISBN 0 19 963577 3). It is also possible to select growth media from commercial suppliers, like standard 1 (Merck) or BHI (grain heart infusion, DIFCO) or others.

All medium components are sterilized, either by heat (20 minutes at 1.5 bar and 121 °C) or by sterile filtration. The components can either be sterilized together or, if necessary, separately. All media components can be present at the beginning of growth, or they can optionally be added continuously or batchwise.

Culture conditions are defined separately for each experiment. The temperature should be in a range between 15°C and 45°C. The temperature can be kept constant or can be altered during the experiment. The pH of the medium should be in the range of 5 to 8.5, preferably around 7.0, and can be maintained by the addition of buffers to the media. An exemplary buffer for this purpose is a potassium phosphate buffer. Synthetic buffers such as MOPS, HEPES, ACES and others can alternatively or simultaneously be used. It is also possible to maintain a constant culture pH through the addition of NaOH or NH₄OH during growth. If complex medium components such as yeast extract are utilized, the necessity for additional buffers may be reduced, due to the fact that many complex compounds have high buffer capacities. If a fermentor is utilized for culturing the micro-organisms, the pH can also be controlled using gaseous ammonia.

The incubation time is usually in a range from several hours to several days. This time is selected in order to permit the maximal amount of product to accumulate in the broth. The disclosed growth experiments can be carried out in a variety of vessels, such as microtiter plates, glass tubes, glass flasks or glass or metal fermentors of different sizes. For screening a large number of clones, the microorganisms should be cultured in microtiter plates, glass tubes or shake flasks, either with or without baffles. Preferably 100 ml shake flasks are used, filled with 10% (by volume) of the required growth medium. The flasks should be shaken on a rotary shaker (amplitude 25 mm) using a speed-range of 100 - 300 rpm. Evaporation losses can be diminished by the maintenance of a humid atmosphere; alternatively, a mathematical correction for evaporation losses should be performed.

If genetically modified clones are tested, an unmodified control clone or a control clone containing the basic plasmid without any insert should also be tested. The medium is inoculated to an OD₆₀₀ of 0.5 - 1.5 using cells grown on agar plates, such as CM plates (10 g/l glucose, 2,5 g/l NaCl, 2 g/l urea, 10 g/l polypeptone, 5 g/l yeast extract, 5 g/l meat extract, 22 g/l NaCl, 2 g/l urea, 10 g/l polypeptone, 5 g/l yeast extract, 5 g/l meat extract, 22 g/l agar, pH 6.8 with 2M NaOH) that had been incubated at 30°C. Inoculation of the media is accomplished by either introduction of a saline suspension of C. *glutamicum* cells from CM plates or addition of a liquid preculture of this bacterium.

### Example 8 -In vitro Analysis of the Function of Mutant Proteins

The determination of activities and kinetic parameters of enzymes is well established in the art. Experiments to determine the activity of any given altered enzyme must be tailored to the specific activity of the wild-type enzyme, which is well within the ability of one of ordinary skill in the art. Overviews about enzymes in general, as well as specific details concerning structure, kinetics, principles, methods, applications and examples for the determination of many enzyme activities may be found, for example, in the following references: Dixon, M., and Webb, E.C., (1979) Enzymes. Longmans: London; Fersht, (1985) Enzyme Structure and Mechanism. Freeman: New York; Walsh, (1979) Enzymatic Reaction Mechanisms. Freeman: San Francisco; Price, N.C., Stevens, L. (1982) Fundamentals of Enzymology. Oxford Univ. Press: Oxford; Boyer, P.D., ed. (1983) The Enzymes, 3^{rd} ed. Academic Press: New York; Bisswanger, H., (1994) Enzymkinetik, 2^{nd} ed. VCH: Weinheim (ISBN 3527300325); Bergmeyer, H.U., Bergmeyer, J., Graßl, M., eds. (1983-1986) Methods of Enzymatic Analysis, 3^{rd} ed., vol. I-XII, Verlag Chemie: Weinheim; and Ullmann's Encyclopedia of Industrial Chemistry (1987) vol. A9, "Enzymes". VCH: Weinheim, p. 352-363.

The activity of proteins which bind to DNA can be measured by several well-established methods, such as DNA band-shift assays (also called gel retardation assays). The effect of such proteins on the expression of other molecules can be measured using reporter gene assays (such as that described in Kolmar, *H. et al.* (1995) *EMBO J. 14:* 3895-3904 and references cited therein). Reporter gene test systems are well known and established for applications in both pro- and eukaryotic cells, using enzymes such as beta-galactosidase, green fluorescent protein, and several others.

The determination of activity of membrane-transport proteins can be performed according to techniques such as those described in Gennis, R.B. (1989) "Pores, Channels and Transporters", in Biomembranes, Molecular Structure and Function, Springer: Heidelberg, p. 85-137; 199-234; and 270-322.

### Example 9: Analysis of Impact of Mutant Protein on the Production of the Desired Product

The effect of the genetic modification in C. *glutamicum* on production of a desired compound (such as an amino acid) can be assessed by growing the modified microorganism under suitable conditions (such as those described above) and analyzing the medium and/or the cellular component for increased production of the desired product *(i. e. ,* an amino acid). Such analysis techniques are well known to one of ordinary skill in the art, and include spectroscopy, thin layer chromatography, staining methods of various kinds, enzymatic and microbiological methods, and analytical chromatography such as high performance liquid chromatography see, for example, Ullman, Encyclopedia of Industrial Chemistry, vol. A2, p. 89-90 and p. 443-613, VCH: Weinheim (1985); Fallon, *A. et al.,* (1987) "Applications of HPLC in Biochemistry" in: Laboratory Techniques in Biochemistry and Molecular Biology, vol. 17; *Rehm et al.* (1993) Biotechnology, vol. 3, Chapter III: "Product recovery and purification", page 469-714, VCH: Weinheim; Belter, *P.A. et al.* (1988) Bioseparations: downstream processing for biotechnology, John Wiley and Sons; Kennedy, J.F. and Cabral, J.M.S. (1992) Recovery processes for biological materials, John Wiley and Sons; Shaeiwitz, J.A. and Henry, J.D. (1988) Biochemical separations, in: Ulmann's Encyclopedia of Industrial Chemistry, vol. B3, Chapter 11, page 1-27, VCH: Weinheim; and Dechow, F.J. (1989) Separation and purification techniques in biotechnology, Noyes Publications.

In addition to the measurement of the final product of fermentation, it is also possible to analyze other components of the metabolic pathways utilized for the production of the desired compound, such as intermediates and side-products, to determine the overall efficiency of production of the compound. Analysis methods include measurements of nutrient levels in the medium *(e.g.,* sugars, hydrocarbons, nitrogen sources, phosphate, and other ions), measurements of biomass composition and growth, analysis of the production of common metabolites of biosynthetic pathways, and measurement of gasses produced during fermentation. Standard methods for these measurements are outlined in Applied Microbial Physiology, A Practical Approach, P.M. Rhodes and P.F. Stanbury, eds., IRL Press, p. 103-129; 131-163; and 165-192 (ISBN: 0199635773) and references cited therein.

### Example 10: Purification of the Desired Product from C. glutamicum Culture

Recovery of the desired product from the *C*. *glutamicum* cells or supernatant of the above-described culture can be performed by various methods well known in the art. If the desired product is not secreted from the cells, the cells can be harvested from the culture by low-speed centrifugation, the cells can be lysed by standard techniques, such as mechanical force or sonication. The cellular debris is removed by centrifugation, and the supernatant fraction containing the soluble proteins is retained for further purification of the desired compound. If the product is secreted from the *C*. *glutamicum* cells, then the cells are removed from the culture by low-speed centrifugation, and the supernate fraction is retained for further purification.

The supernatant fraction from either purification method is subjected to chromatography with a suitable resin, in which the desired molecule is either retained on a chromatography resin while many of the impurities in the sample are not, or where the impurities are retained by the resin while the sample is not. Such chromatography steps may be repeated as necessary, using the same or different chromatography resins. One of ordinary skill in the art would be well-versed in the selection of appropriate chromatography resins and in their most efficacious application for a particular molecule to be purified. The purified product may be concentrated by filtration or ultrafiltration, and stored at a temperature at which the stability of the product is maximized.

There are a wide array of purification methods known to the art and the preceding method of purification is not meant to be limiting. Such purification techniques are described, for example, in Bailey, J.E. & Ollis, D.F. Biochemical Engineering Fundamentals, McGraw-Hill: New York (1986).

The identity and purity of the isolated compounds may be assessed by techniques standard in the art. These include high-performance liquid chromatography (HPLC), spectroscopic methods, staining methods, thin layer chromatography, NIRS, enzymatic assay, or microbiologically. Such analysis methods are reviewed in: *Patek et al.* (1994) *Appl. Environ. Microbiol.* 60: 133-140; Malakhova *et al.* (1996) *Biotekhnologiya* 11: 27-32; and Schmidt *et al.* (1998) *Bioprocess Engineer.* 19: 67-70. Ulmann's Encyclopedia of Industrial Chemistry, (1996) vol. A27, VCH: Weinheim, p. 89-90, p. 521-540, p. 540-547, p. 559-566, 575-581 and p. 581-587; Michal, G. (1999) Biochemical Pathways: An Atlas of Biochemistry and Molecular Biology, John Wiley and Sons; Fallon, A. *et al.* (1987) Applications of HPLC in Biochemistry in: Laboratory Techniques in Biochemistry and Molecular Biology, vol. 17.

### Example 11: Analysis of the Gene Sequences of the Invention

The comparison of sequences and determination of percent homology between two sequences are art-known techniques, and can be accomplished using a mathematical algorithm, such as the algorithm of Karlin and Altschul (1990) *Proc. Natl. Acad. Sci.* USA 87:2264-68, modified as in Karlin and Altschul (1993) *Proc. Natl. Acad. Sci.* USA 90:5873-77. Such an algorithm is incorporated into the NBLAST and XBLAST programs (version 2.0) of Altschul, *et al.* (1990) *J Mol. Biol.* 215:403-10. BLAST nucleotide searches can be performed with the NBLAST program, score = 100, wordlength = 12 to obtain nucleotide sequences homologous to HA nucleic acid molecules of the invention. BLAST protein searches can be performed with the XBLAST program, score = 50, wordlength = 3 to obtain amino acid sequences homologous to HA protein molecules of the invention. To obtain gapped alignments for comparison purposes, Gapped BLAST can be utilized as described in *Altschul et al.,* (1997) *Nucleic Acids Res.* 25(17):3389-3402. When utilizing BLAST and Gapped BLAST programs, one of ordinary skill in the art will know how to optimize the parameters of the program (e.g., XBLAST and NBLAST) for the specific sequence being analyzed.

Another example of a mathematical algorithm utilized for the comparison of sequences is the algorithm of Meyers and Miller ((1988) *Comput. Appl. Biosci. 4:* 11-17). Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Additional algorithms for sequence analysis are known in the art, and include ADVANCE and ADAM. described in Torelli and Robotti (1994) *Comput. Appl. Biosci.* 10:3-5; and FASTA, described in Pearson and Lipman (1988) *P.N.A.S.* 85:2444-8.

The percent homology between two amino acid sequences can also be accomplished using the GAP program in the GCG software package (available at http://www.gcg.com), using either a Blosum 62 matrix or a PAM250 matrix, and a gap weight of 12, 10, 8, 6, or 4 and a length weight of 2, 3, or 4. The percent homology between two nucleic acid sequences can be accomplished using the GAP program in the GCG software package, using standard parameters, such as a gap weight of 50 and a length weight of 3.

A comparative analysis of the gene sequences of the invention with those present in Genbank has been performed using techniques known in the art (see, e.g., Bexevanis and Ouellette, eds. (1998) Bioinformatics: A Practical Guide to the Analysis of Genes and Proteins. John Wiley and Sons: New York). The gene sequences of the invention were compared to genes present in Genbank in a three-step process. In a first step, a BLASTN analysis (e.g., a local alignment analysis) was performed for each of the sequences of the invention against the nucleotide sequences present in Genbank, and the top 500 hits were retained for further analysis. A subsequent FASTA search (e.g., a combined local and global alignment analysis, in which limited regions of the sequences are aligned) was performed on these 500 hits. Each gene sequence of the invention was subsequently globally aligned to each of the top three FASTA hits, using the GAP program in the GCG software package (using standard parameters). In order to obtain correct results, the length of the sequences extracted from Genbank were adjusted to the length of the query sequences by methods well-known in the art. The results of this analysis are set forth in Table 4. The resulting data is identical to that which would have been obtained had a GAP (global) analysis alone been performed on each of the genes of the invention in comparison with each of the references in Genbank, but required significantly reduced computational time as compared to such a database-wide GAP (global) analysis. Sequences of the invention for which no alignments above the cutoff values were obtained are indicated on Table 4 by the absence of alignment information. It will further be understood by one of ordinary skill in the art that the GAP alignment homology percentages set forth in Table 4 under the heading "% homology (GAP)" are listed in the European numerical format, wherein a ',' represents a decimal point. For example, a value of "40,345" in this column represents "40.345%".

### Example 12: Construction and Operation of DNA Microarrays

The sequences of the invention may additionally be used in the construction and application of DNA microarrays (the design, methodology, and uses of DNA arrays are well known in the art, and are described, for example, in Schena, M. *et al.* (1995) *Science* 270: 467-470; Wodicka, L. *et al.* (1997) *Nature Biotechnology* 15: 1359-1367; DeSaizieu, *A. et al.* (1998) *Nature Biotechnology* 16: 45-48; and DeRisi, J.L. *et al.* (1997) *Science* 278: 680-686).

DNA microarrays are solid or flexible supports consisting of nitrocellulose, nylon, glass, silicone, or other materials. Nucleic acid molecules may be attached to the surface in an ordered manner. After appropriate labeling, other nucleic acids or nucleic acid mixtures can be hybridized to the immobilized nucleic acid molecules, and the label may be used to monitor and measure the individual signal intensities of the hybridized molecules at defined regions. This methodology allows the simultaneous quantification of the relative or absolute amount of all or selected nucleic acids in the applied nucleic acid sample or mixture. DNA microarrays, therefore, permit an analysis of the expression of multiple (as many as 6800 or more) nucleic acids in parallel (see, e.g., Schena, M. (1996) *BioEssays* 18(5): 427-431).

The sequences of the invention may be used to design oligonucleotide primers which are able to amplify defined regions of one or more C. *glutamicum* genes by a nucleic acid amplification reaction such as the polymerase chain reaction. The choice and design of the 5' or 3' oligonucleotide primers or of appropriate linkers allows the covalent attachment of the resulting PCR products to the surface of a support medium described above (and also described, for example, Schena, *M. et al.* (1995) *Science 270:* 467-470).

Nucleic acid microarrays may also be constructed *by in situ* oligonucleotide synthesis as described by Wodicka, L. *et al.* (1997) *Nature Biotechnology* 15: 1359-1367. By photolithographic methods, precisely defined regions of the matrix are exposed to light. Protective groups which are photolabile are thereby activated and undergo nucleotide addition, whereas regions that are masked from light do not undergo any modification. Subsequent cycles of protection and light activation permit the synthesis of different oligonucleotides at defined positions. Small, defined regions of the genes of the invention may be synthesized on microarrays by solid phase oligonucleotide synthesis.

The nucleic acid molecules of the invention present in a sample or mixture of nucleotides may be hybridized to the microarrays. These nucleic acid molecules can be labeled according to standard methods. In brief, nucleic acid molecules (e.g., mRNA molecules or DNA molecules) are labeled by the incorporation of isotopically or fluorescently labeled nucleotides, e.g., during reverse transcription or DNA synthesis. Hybridization of labeled nucleic acids to microarrays is described (e.g., in Schena, M. *et al.* (1995) *supra;* Wodicka, L. *et al.* (1997), *supra;* and DeSaizieu A. *et al.* (1998), *supra).* The detection and quantification of the hybridized molecule are tailored to the specific incorporated label. Radioactive labels can be detected, for example, as described in Schena, *M. et al.* (1995) *supra)* and fluorescent labels may be detected, for example, by the method of Shalon *et al.* (1996) *Genome Research* 6: 639-645.

The application of the sequences of the invention to DNA microarray technology, as described above, permits comparative analyses of different strains of *C*. *glutamicum* or other Corynebacteria. For example, studies of inter-strain variations based on individual transcript profiles and the identification of genes that are important for specific and/or desired strain properties such as pathogenicity, productivity and stress tolerance are facilitated by nucleic acid array methodologies. Also, comparisons of the profile of expression of genes of the invention during the course of a fermentation reaction are possible using nucleic acid array technology.

### Example 13: Analysis of the Dynamics of Cellular Protein Populations (Proteomics)

The genes, compositions, and methods of the invention may be applied to study the interactions and dynamics of populations of proteins, termed 'proteomics' . Protein populations of interest include, but are not limited to, the total protein population of *C*. *glutamicum (e.g.,* in comparison with the protein populations of other organisms), those proteins which are active under specific environmental or metabolic conditions (e.g., during fermentation, at high or low temperature, or at high or low pH), or those proteins which are active during specific phases of growth and development.

Protein populations can be analyzed by various well-known techniques, such as gel electrophoresis. Cellular proteins may be obtained, for example, by lysis or extraction, and may be separated from one another using a variety of electrophoretic techniques. Sodium dodecyl sulfate polyacrylamide gel electrophoresis (SDS-PAGE) separates proteins largely on the basis of their molecular weight. Isoelectric focusing polyacrylamide gel electrophoresis (IEF-PAGE) separates proteins by their isoelectric point (which reflects not only the amino acid sequence but also posttranslatiorial modifications of the protein). Another, more preferred method of protein analysis is the consecutive combination of both IEF-PAGE and SDS-PAGE, known as 2-D-gel electrophoresis (described, for example, in Hermann *et al.* (1998) *Electrophoresis 19:* 3217-3221; Fountoulakis *et al.* (1998) *Electrophoresis* 19: 1193-1202; Langen *et al.* (1997) *Electrophoresis* 18: 1184-1192; Antelmann *et al.* (1997) *Electrophoresis 18:* 1451-1463). Other separation techniques may also be utilized for protein separation, such as capillary gel electrophoresis; such techniques are well known in the art.

Proteins separated by these methodologies can be visualized by standard techniques, such as by staining or labeling. Suitable stains are known in the art, and include Coomassie Brilliant Blue, silver stain, or fluorescent dyes such as Sypro Ruby (Molecular Probes). The inclusion of radioactively labeled amino acids or other protein precursors (e.g., ³⁵S-methionine, ³⁵S-cysteine, ¹⁴C-labelled amino acids, ¹⁵N-amino acids, ¹⁵NO₃ or ¹⁵NH₄⁺ or ¹³C-labelled amino acids) in the medium of C. *glutamicum* permits the labeling of proteins from these cells prior to their separation. Similarly, fluorescent labels may be employed. These labeled proteins can be extracted, isolated and separated according to the previously described techniques.

Proteins visualized by these techniques can be further analyzed by measuring the amount of dye or label used. The amount of a given protein can be determined quantitatively using, for example, optical methods and can be compared to the amount of other proteins in the same gel or in other gels. Comparisons of proteins on gels can be made, for example, by optical comparison, by spectroscopy, by image scanning and analysis of gels, or through the use of photographic films and screens. Such techniques are well-known in the art.

To determine the identity of any given protein, direct sequencing or other standard techniques may be employed. For example, N- and/or C-terminal amino acid sequencing (such as Edman degradation) may be used, as may mass spectrometry (in particular MALDI or ESI techniques (see, e.g., *Langen et al.* (1997) *Electrophoresis 18:* 1184-1192)). The protein sequences provided herein can be used for the identification of *C*. *glutamicum* proteins by these techniques.

The information obtained by these methods can be used to compare patterns of protein presence, activity, or modification between different samples from various biological conditions (e.g., different organisms, time points of fermentation, media conditions, or different biotopes, among others). Data obtained from such experiments alone, or in combination with other techniques, can be used for various applications, such as to compare the behavior of various organisms in a given (e.g., metabolic) situation, to increase the productivity of strains which produce fine chemicals or to increase the efficiency of the production of fine chemicals.

### Equivalents

Those of ordinary skill in the art will recognize, or will be able to ascertain using no more than routine experimentation, many equivalents to the specific embodiments of the invention described herein. Such equivalents are intended to be encompassed by the following claims.

## Claims

1. An isolated Corynebacterium glutamicum nucleic acid molecule comprising the nucleotide sequence set forth in SEQ ID NO: 399, or a complement thereof.

2. An isolated nucleic acid molecule which encodes a polypeptide comprising the amino sequence set forth in SEQ ID NO: 400, or a compliment thereof.

3. An isolated nucleic acid molecule which encodes a naturally occurring allelic variant of a polypeptide comprising the amino sequence set forth in SEQ ID NO: 400, or a compliment thereof.

4. An isolated nucleic acid molecule comprising a nucleotide sequence which is at least 50% homologous to the nucleotide sequence set forth in SEQ ID NO: 399 or a compliment thereof.

5. An isolated nucleic acid molecule comprising a fragment of at least 15 contignous nucleotides of the nucleotide sequence set forth in SEQ ID NO: 399, or a complement thereof.

6. An isolated nucleic acid molecule which hybridizes to the nucleic acid molecule of any one of claims 1-5 under stringent conditions.

7. An isolated nucleic acid molecule comprising the nucleic acid molecule of any one of claims 1-6 or a portion thereof and a nucleotide sequence encoding a heterologous polypeptide.

8. A vector comprising the nucleic acid molecule of any one of claims 1-7.

9. The vector of claim 8, which is an expression vector.

10. A host cell transfected with the expression vector of claim 9.

11. The host cell of Claim 10, wherein said cell is a microorganism.

12. The host cell of Claim 11, wherein said cell belongs to the genes *Corynebacterium* or *Brevibacterium.*

13. The host cell of Claim 10, wherein the expression of said nucleic acid molecule results in the modulation in production of a fine chemical from said cell.

14. The host cell of Claim 13, wherein said fine chemical is selected from the group consisting of organic acids, proteinogenic and nonproteinogenic amino acids, purine and pyrimidine bases, nucleotides, nucleotides, lipids, saturated and unsaturated fatty acids, diols, carbohydrates, aromatic compounds, vitamins, cofactors, polyketides, and enzymes.

15. A method of producing a polypeptide comprising culturing the host cell of claim 12 in an appropriate culture medium to, thereby, produce the polypeptide.

16. An isolated polypeptide comprising the amino acid sequence set forth in SEQ ID NO: 400.

17. An isolated polypeptide comprising a naturally occurring allelic variant of a polypeptide comprising the amino acid sequence set forth in SEQ ID NO: 400.

18. The isolated polypeptide of any of claims 16-17, further comprising heterologous amino acid sequences.

19. An isolated polypeptide which is encoded by a nucleic acid molecule comprising a nucleotide sequence which is at least 50% homologous to a nucleic acid set forth in SEQ ID NO: 399, wherein said polypeptide has Phosphoenolpyrurat carboxikinase activity.

20. An isolated polypeptide comprising an amino acid sequence which is at least 50% homologous to an amino acid sequence set forth in SEQ ID NO: 400.

21. A method for producing a fine chemical, comprising culturing the cell of Claim 10 such that the fine chemical is produced.

22. The method of Claim 21, wherein said method further comprises the step of recovering the fine chemical from said culture.

23. The method of Claim 21, wherein said method further comprises the step of transfecting said cell with the vector of Claim 9 to result in a cell containing said vector.

24. The method of Claim 21, wherein said cell belongs to the genes *Corynebacterium* or *Brevibacterium.*

25. The method of Claim 21, wherein said cell is selected from the group consisting of: *Corynebacterium glutamicum, Corynebacterium herculis, Corynebacterium, lilium, Corynebacterium aceloacidophilum, Corynebacterium acetoglutamicum, Corynebacterium acetophilum, Corynebacterium ammoniagenes, Corynebacterium fujiokense, Corynebacterium nitrilophilus, Brevibacterium ammoniagenes, Brevibacterium butanicum, Bre vibacterium divaricatum, Brevibacterium fa vum, Brevibacterium healii, Bre vibacterium ketoglutamicum, Bre vibacterium ketosoreductum, Bre vibacterium lactofermentum, Brevibacterium linens, Brevibacterium paraffinolyticum, and* those strains set forth in Table 3.

26. The method of Claim 21, wherein expression of the nucleic acid molecule from said vector results in modulation of production of said fine chemical.

27. The method of Claim 21, wherein said fine chemical is selected from the group consisting of: organic acids, proteinogenic and nonproteinogenic amino acids, purine and pyrimidine bases, nucleosides, nucleotides, lipids, saturated and unsaturated fatty acids, diols, carbohydrates, aromatic compounds, vitamins, cofactors, polyketides, and enzymes.

28. The method of Claim 21, wherein said fine chemical is an amino acid.

29. The method of Claim 28, wherein said amino acid is drawn from the group consisting of: lysine, glutamate, glutamine, alanine, aspartate, glycine, serine, threonine, methionine, cysteine, valine, leucine, isoleucine, arginine, proline, histidine, tyrosine, phenylalanine, and tryptophan.

30. A method for producing a fine chemical, comprising culturing a cell whose genomic DNA has been altered by the inclusion of a nucleic acid molecule of any one of Claims 1-7.

31. A method of detecting the presence or activity of *Corynebacterium diphtheriae* in a subject, comprising detecting the presence of at least one of the nucleic acid molecules of Claims 1 - 7 or the polypeptide molecules of Claims 16 - 20, thereby detecting the presence or activity of *Corynebacterium diphtheriae* in the subject.

32. A host cell comprising a nucleic acid molecule comprising the nucleotide sequence set forth in SEQ ID NO: 399, wherein the nucleic acid molecule is disrupted.

33. A host cell comprising a nucleic acid molecule comprising the nucleotide sequence set forth in SEQ ID NO: 399, wherein the nucleic acid molecule comprises one or more nucleic acid modifications as compared to the sequence set forth in-SEQ ID NO: 399.

34. A host cell comprising a nucleic acid molecule comprising the nucleotide sequence set forth in SEQ ID NO: 399, wherein the regulatory region of the nucleic acid molecule is modified relative to the wild-type regulatory region of the molecule.

35. An isolated polypeptide comprising a fragment of a polypeptide comprising the aminoacid sequence set forth in SEQ ID NO: 400, wherein said polypeptide fragment maintains a biological activity of the polypeptide comprising the amino-acid sequence set forth in SEQ ID NO: 400.
